# EUROPEAN PATENT APPLICATION

(11) **EP 4 763 839 A1**
(43) Date of publication of application: **24.06.2026**
(21) Application number: 24853857.1
(22) Date of filing: 15.08.2024
(51) Int. Cl.: C07D 401/14, C07D 487/04, C07D 495/04, C07D 519/00, A61K 39/395, A61K 47/64, A61K 47/68, A61K 31/513, A61K 31/519, A61K 31/496

(54) **COMPOUND TARGETING IRAK PROTEIN INHIBITION OR DEGRADATION AND USE THEREOF**

(30) Priority: 15.08.2023 CN 202311025660; 07.02.2024 CN 202410175807
(71) Applicant: Pamplona Therapeutics (Shenzhen) Co., Ltd., Shenzhen, Guangdong 518107 (CN); Guangzhou IMD Pharmaceutical Co., Ltd., Guangzhou, Guangdong 510000 (CN)
(72) Inventor: LIU, Jinsong, Shenzhen, Guangdong 518107 (CN); ZHU, Jiabin, Shenzhen, Guangdong 518107 (CN); ZHAO, Mohan, Shenzhen, Guangdong 518107 (CN); GAN, Qingqing, Shenzhen, Guangdong 518107 (CN); LIN, Jialu, Shenzhen, Guangdong 518107 (CN); CHEN, Rui, Shenzhen, Guangdong 518107 (CN); QI, Huaiyuan, Shenzhen, Guangdong 518107 (CN)
(74) Representative: Ipsilon Belgium
(86) International application number: PCT/CN2024/112342
(87) International publication number: WO 2025/036451

(57) **Abstract**

The present disclosure relates to a compound targeting IRAK protein inhibition or degradation, a preparation method therefor, and use thereof in the treatment or prevention of IRAK protein-mediated related diseases.

## Description

### TECHNICAL FIELD

The present disclosure belongs to the field of pharmaceuticals, and specifically relates to compounds that inhibit or degrade IRAK proteins, preparation methods therefor, and the use thereof in the treatment or prevention of diseases mediated by IRAK proteins.

### BACKGROUND

The interleukin-1 receptor-associated kinase (IRAK) family is a class of serine/threonine protein kinases that play pivotal roles in innate immune regulation and adaptive immune responses mediated by the toll-like receptor (TLR) family and the interleukin-1 receptor (IL-1R) family. The IRAK protein family comprises four isoenzymes: IRAK1, IRAK2, IRAK3 (also referred to as IRAK-M), and IRAK4. These enzymes share similar domains, including a conserved N-terminal death domain (DD), a proline/serine/threonine-rich (ProST) domain, a kinase or pseudokinase domain (KD), and, with the exception of IRAK4, a long C-terminal domain (CTD). During inflammatory responses and immune signal transduction, activated TLR/IL-1R receptors recruit the adaptor protein MyD88 via their receptor intracellular TIR domains, leading to the assembly of the Myddosome, a multiprotein complex composed of MyD88, IRAK4, and IRAK2. In the Myddosome complex, IRAK4 is activated by trans-autophosphorylation, which then phosphorylates and activates IRAK1 and promotes its dissociation from the complex. Activated IRAK1 forms a complex with the E3 ubiquitin ligase TNF receptor-associated factor 6 (TRAF6), which in turn activates TGF-β-activated kinase 1-binding protein 1 (TAK1). TAK1 ultimately activates downstream NF-κB and MAPK/JNK/p38 signaling pathways through a series of intracellular cascades, resulting in the secretion of pro-inflammatory cytokines and the proliferation and differentiation of immune cells. In monocytes and macrophages, IRAK3 functions as a negative regulator of IRAK signaling by inhibiting the activation of IRAK4 and IRAK1.

Mutations in MYD88, IRAK4, and IRAK1, currently identified in infected patients, highlight the critical roles of these proteins in host defense. Studies have shown that patients with autosomal recessive deficiencies in IRAK4 or MyD88 are highly susceptible to pyogenic bacterial infections, yet remain resistant to other classes of pathogens, including most bacteria, viruses, fungi, and parasites. As the central kinase converging the two major signaling pathways of the TLR family and the IL-1R family, IRAK4 has kinase activity that is essential for the activation of downstream TLR/IL-1R pathways and thus represents a candidate therapeutic target for a variety of inflammatory diseases. IRAK4 with kinase domain mutations has demonstrated protective effects in mice across multiple preclinical inflammatory disease models, including septic shock, systemic lupus erythematosus, acute liver injury, cardiovascular disease, and Alzheimer's disease, etc.

According to incomplete statistics, there are currently about 40 IRAK4-targeting drug candidates under investigation worldwide, most of which are in early clinical stages. To date, no drug targeting IRAK4 has been approved for marketing. Among these candidates, six are in Phase II clinical trials, two in Phase I/II, ten in Phase I, and twenty-five in preclinical stages. The most extensively pursued indications are autoimmune diseases, including rheumatoid arthritis, hidradenitis suppurativa, atopic dermatitis, lupus erythematosus, etc. Cancer indications follow, such as acute myeloid leukemia, myelodysplastic syndromes, and B cell lymphomas. The most advanced agents in the field include Pfizer's potent IRAK4 inhibitor PF-06650833, which has completed Phase II clinical studies in rheumatoid arthritis indications and hidradenitis suppurativa. Results indicated that PF-06650833 did not produce significant efficacy in these indications. Curis has developed the inhibitor CA-4948, which is currently in Phase II clinical trials across multiple oncology indications. Notably, Kymera has developed the IRAK4 degrader KT-474 using targeted protein degradation (TPD) technology. KT-474 has completed Phase I clinical studies in hidradenitis suppurativa and atopic dermatitis, demonstrating strong anti-inflammatory activity as well as favorable safety and tolerability. TPD represents an emerging therapeutic strategy that employs proteolysis-targeting chimeras (PROTACs) to directly target proteins that are otherwise difficult to modulate with conventional small molecules. PROTACs are heterobifunctional molecules composed of two connected ligands: one ligand recruits and binds the protein of interest (POI), while the other ligand recruits and binds an E3 ubiquitin ligase. By inducing the binding between the protein of interest and E3 ligase, PROTACs enable the attachment of a ubiquitination tag to a lysine of the protein of interest through E3 catalysis. The ubiquitinated protein is subsequently recognized and degraded by the 26S proteasome subunit within the cell. Currently, due to the lack of available binding pockets and suitable chemical matter, approximately 80%-85% of the human proteome is considered "undruggable." Meanwhile, druggable targets such as EGFR-TKIs are prone to resistance mutations, leading to diminished efficacy. Therefore, making undruggable targets druggable through targeted protein degradation, avoiding resistance caused by point mutations, and thereby enabling drug development has become a major hotspot in new drug research and development. Compared with traditional inhibitors, KT-474 is the first effective, highly selective, orally bioavailable IRAK4 degrader, offering incomparable and unique advantages in suppressing TLR/IL-1R signaling pathways. IRAK4 mediates IL-1R/TLR downstream signaling and participates in immune surveillance through two mechanisms: (i) its kinase activity, which phosphorylates downstream substrates such as IRAK1 and IRF5/7; and (ii) its scaffolding function, which participates in the assembly of the multiprotein complex myddosome. Vollmer et al. reported that pharmacological inhibition of IRAK4 autophosphorylation failed to block IL-1 induced activation of IRAK1 and NF-κB/P38/JNK signaling pathways in IL-1R cells. Qin and Song demonstrated that in IRAK4-deficient cells, induction of kinase domain mutant inactive IRAK4 restored IL-1 induced stress responses, including increased IRAK1 phosphorylation under IL-1 stimulation and activation of downstream inflammatory signaling. Conventional small molecule inhibitors that target only kinase activity exhibit moderate potency and cannot disrupt complex formation to fully block inflammatory signaling. In contrast, KT-474 simultaneously abrogates both kinase activity and scaffolding function, showing stronger pathway inhibition and anti-inflammatory activity in studies.

There remains an urgent need to develop additional inhibitors and degraders targeting IRAK4.

### SUMMARY

The present disclosure aims to provide a compound targeting IRAK protein inhibition or degradation, a preparation method therefor, and use thereof in the treatment or prevention of IRAK protein-mediated related diseases.

In one aspect, the present disclosure provides a compound of formula (I) or a pharmaceutically acceptable salt, ester, solvate, or stereoisomer thereof: 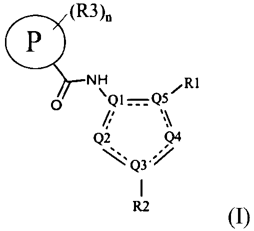 wherein:
Q1, Q2, Q3, Q4, and Q5 are independently selected from C, CH, CH₂, N, NH, S, or O;
--- means that a double bond is present or absent;
ring P is aryl or heteroaryl;
R1 is selected from hydrogen, deuterium, halogen, hydroxyl, sulfhydryl, amino, nitro, cyano, -OR^{1a}, -SR^{1a}, -NR^{1a}R^{1b}, -COR^{1a}, -SOR^{1a}, -SO₂R^{1a}, -COOR^{1a}, -CONR^{1a}R^{1b}, -NR^{1a}COR^{1b}, -OCOR^{1a}, -S(O)(NR^{1a})R^{1b}, -SONR^{1a}R^{1b}, -SO₂NR^{1a}R^{1b}, -NR^{1a}SOR^{1b}, -NR^{1a}SO₂R^{1b}, optionally substituted alkyl, optionally substituted alkoxy, optionally substituted cycloalkyl, and optionally substituted heterocyclyl, wherein R^{1a} and R^{1b} are independently selected from hydrogen, deuterium, optionally substituted alkyl, optionally substituted cycloalkyl, and optionally substituted heterocyclyl;
R2 is selected from optionally substituted cycloalkyl, optionally substituted heterocyclyl, optionally substituted aryl, and optionally substituted heteroaryl;
R3 is selected from hydrogen, deuterium, halogen, hydroxyl, sulfhydryl, amino, nitro, cyano, -OR^{3a}, -SR^{3a}, -NR^{3a}R^{3b}, -COR^{3a}, -SOR^{3a}, -SO₂R^{3a}, -COOR^{3a}, -CONR^{3a}R^{3b}, -NR^{3a}COR^{3b}, -OCOR^{3a}, -S(O)(NR^{3a})R^{3b}, -SONR^{3a}R^{3b}, -SO₂NR^{3a}R^{3b}, -NR^{3a}SOR^{3b}, -NR^{3a}SO₂R^{3b}, optionally substituted alkyl, optionally substituted alkoxy, optionally substituted cycloalkyl, optionally substituted heterocyclyl, optionally substituted aryl, and optionally substituted heteroaryl, wherein R^{3a} and R^{3b} are independently selected from hydrogen, deuterium, optionally substituted alkyl, optionally substituted cycloalkyl, optionally substituted heterocyclyl, optionally substituted aryl, and optionally substituted heteroaryl; and
n is 0, 1, 2, 3, 4, or 5.

In another aspect, the present disclosure provides a compound of formula (II) or a pharmaceutically acceptable salt, ester, solvate, or stereoisomer thereof:

A-L-E (II)

wherein moiety A has the following structure: 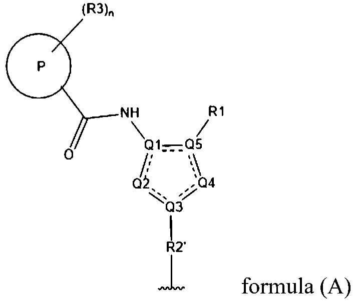 wherein:
Q1, Q2, Q3, Q4, and Q5 are independently selected from C, CH, CH₂, N, NH, S, or O;
--- means that a double bond is present or absent;
ring P is aryl or heteroaryl;
R1 is selected from hydrogen, deuterium, halogen, hydroxyl, sulfhydryl, amino, nitro, cyano, -OR^{1a}, -SR^{1a}, -NR^{1a}R^{1b}, -COR^{1a}, -SOR^{1a}, -SO₂R^{1a}, -COOR^{1a}, -CONR^{1a}R^{1b}, -NR^{1a}COR^{1b}, -OCOR^{1a}, -S(O)(NR^{1a})R^{1b}, -SONR^{1a}R^{1b}, -SO₂NR^{1a}R^{1b}, -NR^{1a}SOR^{1b}, -NR^{1a}SO₂R^{1b}, optionally substituted alkyl, optionally substituted alkoxy, optionally substituted cycloalkyl, and optionally substituted heterocyclyl, wherein R^{1a} and R^{1b} are independently selected from hydrogen, deuterium, optionally substituted alkyl, optionally substituted cycloalkyl, and optionally substituted heterocyclyl;
R3 is selected from hydrogen, deuterium, halogen, hydroxyl, sulfhydryl, amino, nitro, cyano, -OR^{3a}, -SR^{3a}, -NR^{3a}R^{3b}, -COR^{3a}, -SOR^{3a}, -SO₂R^{3a}, -COOR^{3a}, -CONR^{3a}R^{3b}, -NR^{3a}COR^{3b}, -OCOR^{3a}, -S(O)(NR^{3a})R^{3b}, -SONR^{3a}R^{3b}, -SO₂NR^{3a}R^{3b}, -NR^{3a}SOR^{3b}, -NR^{3a}SO₂R^{3b}, optionally substituted alkyl, optionally substituted alkoxy, optionally substituted cycloalkyl, optionally substituted heterocyclyl, optionally substituted aryl, and optionally substituted heteroaryl, wherein R^{3a} and R^{3b} are independently selected from hydrogen, deuterium, optionally substituted alkyl, optionally substituted cycloalkyl, optionally substituted heterocyclyl, optionally substituted aryl, and optionally substituted heteroaryl; and
n is 0, 1, 2, 3, 4, or 5;
R2' is selected from optionally substituted cycloalkylene, optionally substituted heterocyclylene, optionally substituted arylene, and optionally substituted heteroarylene; and
moiety A is attached to moiety L via a ring atom of R2';
moiety L has the following structure:

   -L1-L2-L3-L4-L5- formula (L)

   wherein:
   L1, L3, and L5 are independently absent or selected from -Rm-, -Rm-Rn-, -Rm-O-Rn-, -Rm-C(O)-Rn-, -Rm-C(O)O-Rn-, -Rm-OC(O)-Rn-, -Rm-NRx-Rn-, -Rm-C(O)NRx-Rn-, -Rm-NRxC(O)-Rn-, -Rm-S(O)-Rn-, -Rm-S(O)NRx-Rn-, -Rm-NRxS(O)-Rn-, -Rm-S(O)₂-Rn-, -Rm-S(O)₂NRx-Rn-, -Rm-NRxS(O)₂-Rn-, -Rm-NRxC(O)NRy-Rn-, -Rm-OC(O)NRx-Rn-, -Rm-NRxC(O)O-Rn-, 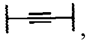 -(CH₂CH₂O)_{g}-, or - (OCH₂CH₂)_{g}-, wherein Rm and Rn are independently selected from a bond, optionally substituted alkylene, optionally substituted alkyleneoxy, optionally substituted cycloalkylene, or optionally substituted heterocyclylene; Rx and Ry are independently selected from hydrogen, deuterium, optionally substituted alkyl, optionally substituted cycloalkyl, or optionally substituted heterocyclyl; and g is an integer from 1 to 20;
   L2 and L4 are independently absent or selected from optionally substituted cycloalkylene, optionally substituted heterocyclylene, optionally substituted arylene, or optionally substituted heteroarylene; and
   moiety E has the following structure: 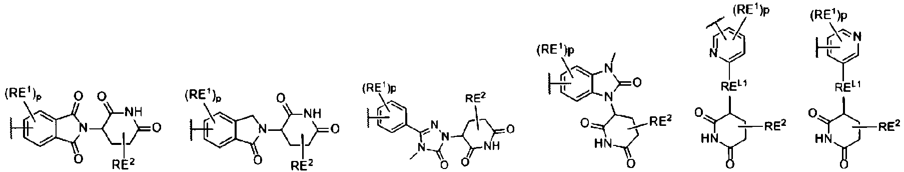 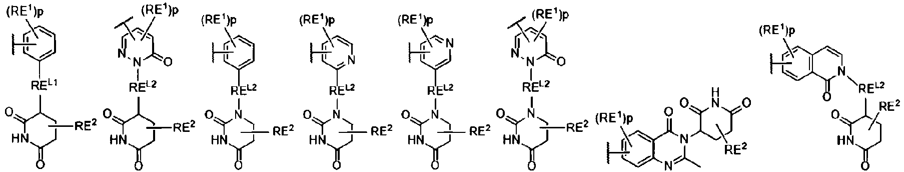 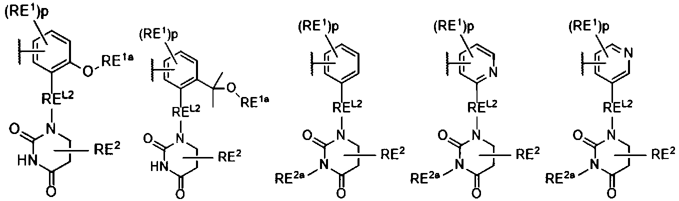
   wherein RE¹ is selected from hydrogen, deuterium, halogen, hydroxyl, sulfhydryl, amino, nitro, cyano, optionally substituted alkyl, optionally substituted alkoxy, optionally substituted cycloalkyl, or optionally substituted heterocyclyl; RE² is selected from hydrogen, deuterium, or halogen; RE^{L1} is selected from a bond, -CH₂-, -NH-, -NCH₃-, -O-, -CONH-, or -CONCH₃-; RE^{L2} is selected from a bond or -CH₂-; and p is 1, 2, 3, 4, or 5;
   RE^{1a} is selected from -COCH(CH₃)OH, -COCH₃, -CON(CH₃)₂, -CO(CH₂)₂CH(NH₂)COOH, or - COCH(NH₂)(CH₂)₂COOH, and RE^{2a} is selected from -CH₂OCOOC(CH₃)₃ or -CH₂OCOC(CH₃)₃.

In another aspect, the present disclosure provides a method for treating or preventing an IRAK protein-mediated disease, and the method comprises administering to a patient in need thereof the compound of formula (I) or formula (II).

In another aspect, the present disclosure provides use of the compound of formula (I) or formula (II) in the manufacture of a medicament for treating or preventing an IRAK protein-mediated disease.

### DETAILED DESCRIPTION

### Definitions

As used herein, "substituted" refers to all permissible substituents of the compounds or functional groups described herein. In the broadest sense, the permissible substituents include acyclic and cyclic, branched and unbranched, carbocyclic and heterocyclic, and aromatic and non-aromatic substituents of organic compounds. Exemplary substituents include, but are not limited to, halogen, hydroxyl, or any other organic group containing any number of carbon atoms (e.g., 1-20 carbon atoms), and optionally include one or more heteroatoms in the form of a linear, branched, or cyclic structure, such as oxygen, sulfur, or nitrogen groups. Representative substituents include deuterium, alkyl, substituted alkyl, alkenyl, substituted alkenyl, alkynyl, substituted alkynyl, phenyl, substituted phenyl, aryl, substituted aryl, heteroaryl, substituted heteroaryl, halogen, nitro, hydroxyl, alkoxy, substituted alkoxy, phenoxy, substituted phenoxy, aryloxy, substituted aryloxy, sulfhydryl, substituted sulfhydryl, alkylthio, substituted alkylthio, phenylthio, substituted phenylthio, arylthio, substituted arylthio, cyano, substituted cyano, isocyano, substituted isocyano, carbonyl, substituted carbonyl, carboxyl, substituted carboxyl, amino, substituted amino, amido, substituted amido, sulfonyl, substituted sulfonyl, sulfonic acid, phosphoryl, substituted phosphoryl, phosphonyl, substituted phosphonyl, polyaryl, substituted polyaryl, C₃-C₂₀ cyclic groups, substituted C₃-C₂₀ cyclic groups, heterocycles, substituted heterocycles, amino acids, poly(lactic-co-glycolic acid), and peptide and polypeptide groups. Such alkyl, substituted alkyl, alkenyl, substituted alkenyl, alkynyl, substituted alkynyl, phenyl, substituted phenyl, aryl, substituted aryl, heteroaryl, substituted heteroaryl, halogen, nitro, hydroxyl, alkoxy, substituted alkoxy, phenoxy, substituted phenoxy, aryloxy, substituted aryloxy, sulfhydryl, substituted sulfhydryl, alkylthio, substituted alkylthio, phenylthio, substituted phenylthio, arylthio, substituted arylthio, cyano, substituted cyano, isocyano, substituted isocyano, carbonyl, substituted carbonyl, carboxyl, substituted carboxyl, amino, substituted amino, amido, substituted amido, sulfonyl, substituted sulfonyl, sulfonic acid, phosphoryl, substituted phosphoryl, phosphonyl, substituted phosphonyl, polyaryl, substituted polyaryl, C₃-C₂₀ cyclic groups, substituted C₃-C₂₀ cyclic groups, heterocycles, substituted heterocycles, amino acids, poly(lactic-co-glycolic acid), and peptide and polypeptide groups may be further substituted.

Heteroatoms such as nitrogen may have hydrogen substituents and/or any permissible substituents of the organic compounds described herein that satisfy the valences of the heteroatoms.

It will be appreciated that "substitution" or "substituted" includes the implicit proviso that such substitution is in accordance with the permitted valence of the substituted atom and the substituent, and that the substitution results in a stable compound, i.e., a compound that does not spontaneously undergo transformation, e.g., by rearrangement, cyclization, elimination, etc.

Unless otherwise specifically and explicitly stated, the term "substituted" refers to a structure, e.g., a moiety of a compound or a larger compound, regardless of how the structure is formed. The structure is not limited to structures made by any particular method.

As used herein, "aryl" refers to a C₅-C₂₆ membered aromatic, fused aromatic, or multi-aromatic ring system. As used herein, broadly defined "aryl" includes 5-, 6-, 7-, 8-, 9-, 10-, 14-, 18-, and 24-membered monocyclic or polycyclic aromatic groups such as benzene, naphthalene, anthracene, phenanthrene, chrysene, pyrene, corannulene, and coronene.

"Aryl" also includes polycyclic ring systems having two or more rings, wherein two or more carbon atoms are shared between two adjacent rings (i.e., "fused rings"), and at least one of the rings is aromatic; for example, the other cyclic ring or rings may be cycloalkyl groups, cycloalkenyl groups, cycloalkynyl groups, aryl groups, heteroaryl groups, and/or heterocycles.

The term "substituted aryl" refers to an aryl group in which one or more hydrogen atoms on one or more aromatic rings are substituted with one or more substituents including, but not limited to, deuterium, halogen, azide, alkyl, aralkyl, alkenyl, alkynyl, cycloalkyl, hydroxyl, alkoxy, carbonyl (e.g., ketone, aldehyde, carboxyl, alkoxycarbonyl, formyl, or acyl), silyl, ether, ester, thiocarbonyl (e.g., thioester, thioacetate, or thioformate), alkoxy, phosphoryl, phosphate, phosphonate, phosphinate, amino (or quaternized amino), amido, amidine, imine, cyano, nitro, azido, sulfhydryl, imino, alkylthio, sulfate, sulfonate, sulfamoyl, sulfoxide, sulfonamido, sulfonyl, heterocyclyl, alkylaryl, haloalkyl (e.g., CF₃, -CH₂-CF₃, and -CCl₃), -CN, aryl, heteroaryl, and combinations thereof, and the substituents may be further substituted.

"Heterocycle" and "heterocyclyl" are used interchangeably and refer to a monocyclic or polycyclic (e.g., bicyclic such as spiro-ring or fused-ring) non-aromatic cyclic group containing 3-20 ring atoms (e.g., 3-16, 3-14, 3-12, or 3-10 ring atoms, such as 3, 4, 5, 6, 7, 8, 9, 10, 11, or 12 ring atoms). The group consists of carbon and one to six (e.g., one, two, three, or four) heteroatoms, and each of the heteroatoms may be selected from oxygen, sulfur, and nitrogen. Moreover, the group optionally comprises 1-3 double bonds and is optionally substituted with one or more substituents. Heterocyclyl is, by definition, different from heteroaryl. Examples of heterocycles include, but are not limited to, piperazinyl, piperidinyl, piperidinonyl, 4-piperidinonyl, tetrahydrofuranyl, dihydrofuro[2,3-b]tetrahydrofuran, morpholinyl, thiomorpholinyl, piperazinyl, piperidinyl, piperidinonyl, 4-piperidinonyl, piperonyl, indolinyl, pyranyl, tetrahydropyranyl, pyrrolidinyl, pyrrolinyl, 2H-pyrrolyl, imidazolidinyl, imidazolinyl, pyrazolidinyl, 4H-quinolyl, quinuclidinyl, tetrahydrofuranyl, 6H-1,2,5-thiadiazinyl, tetrahydrothienyl, isoxazolidinyl, isothiazolidinyl, oxazolidinyl, thiazolidinyl, azepanyl, and benzoazepine. Heterocyclic groups may be optionally substituted with one or more substituents as defined herein (e.g., defined for alkyl, aryl, etc.).

The term "heteroaryl" refers to C₅-C₂₆ membered monocyclic or polycyclic aromatic ring systems or combinations thereof (e.g., 5-24, 5-20, 5-16, 5-12, or 5-10 ring atoms, such as 5, 6, 7, 8, 9, 10, 11, or 12 ring atoms), wherein one or more carbon atoms on one or more aromatic ring structures have been substituted with heteroatoms (e.g., one to six, such as one, two, three, or four heteroatoms). Suitable heteroatoms include, but are not limited to, oxygen, sulfur, and nitrogen. As used herein, broadly defined "heteroaryl" includes 5-, 6-, 7-, 8-, 9-, 10-, 14-, 18-, and 24-membered monocyclic aromatic groups that may comprise one to four heteroatoms, such as pyrrole, furan, thiophene, imidazole, oxazole, thiazole, triazole, tetrazole, pyrazole, pyridine, pyrazine, pyridazine, pyrimidine, oxadiazolyl, thiadiazolyl, triazinyl, isoxazolyl, isothiazolyl, azepinyl, and diazepinyl. Heteroaryl groups may also be referred to as "aryl heterocycles" or "heteroaromatics". "Heteroaryl" also includes polycyclic ring systems having two or more rings, wherein two or more carbon atoms are shared between two adjacent rings (e.g., "fused rings"); at least one of the rings is heteroaromatic, and the other ring or rings may be cycloalkyl groups, cycloalkenyl groups, cycloalkynyl groups, aryl groups, heterocycles, heteroaromatic rings, or combinations thereof. Examples of heteroaromatic rings include, but are not limited to, benzimidazolyl, benzofuranyl, benzothiofuranyl, benzothienyl, benzoxazolyl, benzoxazolinyl, benzothiazolyl, benzotriazolyl, benzotetrazolyl, benzisoxazolyl, benzisothiazolyl, benzimidazolinyl, benzoxadiazolyl, benzothiadiazolyl, benzoquinolyl, benzisoquinolyl, carbazolyl, 4aH-carbazolyl, carbolinyl, chromanyl, benzopyranyl, cinnolinyl, decahydroquinolyl, 2H,6H-1,5,2-dithiazinyl, furanyl, furazanyl, imidazolidinyl, imidazolinyl, imidazolyl, 1H-indazolyl, indolenyl, indolinyl, indolizinyl, indolyl, 3H-indolyl, isatinoyl, isobenzofuranyl, isochromanyl, isoindazolyl, isoindolinyl, isoindolyl, isoquinolyl, isothiazolyl, isoxazolyl, methylenedioxyphenyl, naphthyridinyl, octahydroisoquinolyl, 1,2,3-oxadiazolyl, 1,2,4-oxadiazolyl, 1,2,5-oxadiazolyl, 1,3,4-oxadiazolyl, oxazolidinyl, oxazolyl, oxindolyl, pyrimidinyl, phenanthridinyl, phenanthrolinyl, phenazinyl, phenothiazinyl, phenoxathinyl, phenoxazinyl, phthalazinyl, pteridinyl, purinyl, pyrazinyl, pyrazolidinyl, pyrazolinyl, pyrazolyl, pyridazinyl, pyridooxazole, pyridoimidazole, pyridothiazole, pyrazolopyrimidine, pyridinyl, pyridyl, pyrimidinyl, pyrrolidinyl, pyrrolinyl, pyrrolyl, pyrrolopyridazine, quinazolinyl, quinolyl, quinoxalinyl, tetrahydroisoquinolyl, tetrahydroquinolyl, tetrazolyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, phenylthio, and xanthenyl. One or more rings of heteroaryl may be substituted, as defined herein.

The term "substituted heteroaryl" refers to a heteroaryl group in which one or more hydrogen atoms on one or more heteroaryl rings are substituted with one or more substituents including, but not limited to, deuterium, halogen, azide, alkyl, aralkyl, alkenyl, alkynyl, cycloalkyl, hydroxyl, alkoxy, carbonyl (e.g., ketone, aldehyde, carboxyl, alkoxycarbonyl, formyl, or acyl), silyl, ether, ester, thiocarbonyl (e.g., thioester, thioacetate, or thioformate), alkoxy, phosphoryl, phosphate, phosphonate, phosphinate, amino (or quaternized amino), amido, amidine, imine, cyano, nitro, azido, sulfhydryl, imino, alkylthio, sulfate, sulfonate, sulfamoyl, sulfoxide, sulfonamido, sulfonyl, heterocyclyl, alkylaryl, haloalkyl (e.g., CF₃, -CH₂-CF₃, and -CCl₃), -CN, aryl, heteroaryl, and combinations thereof, and the substituents may be further substituted.

As used herein, "alkyl" refers to a linear or branched, saturated, aliphatic group. It may comprise 1-30, 1-26, 1-20, 1-16, 1-12, 1-8, or 1-6 carbon atoms. Alkyl may be, for example, methyl, ethyl, n-propyl, isopropyl, n-butyl, sec-butyl, isobutyl, tert-butyl, n-pentyl, 2-methylbutyl, 3-methylbutyl, n-hexyl, 4-methylpentyl, neopentyl, n-hexyl, 1,2-dimethylpropyl, 2,2-dimethylpropyl, or 1,2,2-trimethylpropyl. The term "alkyl" (or "lower alkyl") used throughout the specification, examples, and claims is intended to include "unsubstituted alkyl" and "substituted alkyl", the latter of which refers to an alkyl group in which one or more hydrogen atoms on carbon atoms of the hydrocarbon chain are replaced with one or more substituents. Such substituents include, but are not limited to, deuterium, halogen, hydroxyl, carbonyl (e.g., carboxyl, alkoxycarbonyl, formyl, or acyl), thiocarbonyl (e.g., thioester, thioacetate, or thioformate), alkoxy, phosphoryl, phosphate, phosphonate, phosphinate, amino, amido, amidine, imine, cyano, nitro, azido, sulfhydryl, alkylthio, sulfate, sulfonate, sulfamoyl, sulfoxide, sulfonamido, sulfonyl, cycloalkyl, heterocyclyl, aralkyl, or aromatic or heteroaromatic moieties, and the substituents may be further substituted.

Unless the number of carbon atoms is otherwise specified, the term "lower alkyl" used herein refers to an alkyl group as defined above, but its backbone structure has 1 to 10 carbon atoms, more preferably 1 to 6 carbon atoms.

As used herein, the term "cycloalkyl" refers to a saturated, non-aromatic, monocyclic or polycyclic hydrocarbon group. It may have 3-30, 3-26, 3-20, 3-16, 3-12, 3-8, or 3-6 carbon atoms, such as cyclopropyl, cyclobutyl, cyclopentyl, or cyclohexyl.

"Alkyl" may include one or more substitutions at one or more carbon atoms of hydrocarbyl. Suitable substituents include, but are not limited to, halogen, e.g., fluorine, chlorine, bromine, or iodine; hydroxyl; - NRR', wherein R and R' are independently hydrogen, alkyl, cycloalkyl, aryl, or heteroaryl, and the nitrogen atom is optionally quaternized; -SR, wherein R is hydrogen, alkyl, cycloalkyl, aryl, or heteroaryl; -CN; - NO₂; -COOH; carboxylate; -COR, -COOR, or CON(R)₂, wherein R is hydrogen, alkyl, or aryl; azide, aralkyl, alkoxy, imino, phosphonate, phosphinate, silyl, ether, sulfonyl, sulfonamido, heterocyclyl, aromatic or heteroaromatic moieties, and haloalkyl (e.g., -CF₃, -CH₂-CF₃, or -CCl₃); CN; -NCOCOCH₂CH₂; NCOCOCH; -NCS; and combinations thereof.

Those skilled in the art will appreciate that the substituted moiety on the hydrocarbon chain may itself be substituted, as appropriate. For example, the substituents of substituted alkyl may include deuterium, halogen, hydroxyl, sulfhydryl, nitro, thiol, amino, azido, imino, amido, phosphoryl (including phosphonate and phosphinate), sulfonyl (including sulfate, sulfonamido, sulfamoyl, sulfoxide, and sulfonate), and silyl, as well as ether, alkylthio, carbonyl (including ketone, aldehyde, carboxylate, and ester), haloalkyl, -CN, etc. Cycloalkyl, heterocyclyl, aryl, heteroaryl, etc., may be substituted in the same way.

As used herein, the term "halogen" refers to fluorine, chlorine, bromine, or iodine.

As used herein, the term "hydroxyl" refers to the -OH group.

As used herein, the term "amino" refers to the -NH₂ group.

As used herein, the term "cyano" refers to the -CN group.

As used herein, the term "nitro" refers to the -NO₂ group.

As used herein, the term "carbonyl" refers to the -COR group (R is hydrogen or alkyl), particularly the -CHO group.

As used herein, the terms "alkylene", etc., refer to divalent groups in which two hydrogen atoms in the corresponding group, etc., (e.g., alkyl) are substituted.

As used herein, the term "stereoisomer" refers to compounds composed of the same atoms bonded by the same bonds but having different three-dimensional structures, and the three-dimensional structures are not interchangeable. "Stereoisomer" includes enantiomers, diastereomers, and other stereoisomeric forms defined according to absolute stereochemistry. The present disclosure is meant to include all such possible isomers, as well as their racemic and optically pure forms.

As used herein, the term "pharmaceutically acceptable salt" refers to a derivative of the disclosed compound, wherein the parent compound is modified by converting an existing acid or base moiety to its salt form. Examples of pharmaceutically acceptable salts include, but are not limited to, salts with inorganic acids such as hydrochloric acid, hydrobromic acid, phosphoric acid, and sulfuric acid, and salts with organic carboxylic acids and sulfonic acids such as acetic acid, trifluoroacetic acid, propionic acid, maleic acid, fumaric acid, malic acid, citric acid, tartaric acid, lactic acid, benzoic acid, methanesulfonic acid, ethanesulfonic acid, benzenesulfonic acid, toluenesulfonic acid, naphthalenesulfonic acid, and naphthalenedisulfonic acid; or salts with conventional bases, such as alkali metal salts (e.g., sodium salts and potassium salts), alkaline earth metal salts (e.g., calcium salts and magnesium salts), and ammonium salts derived from ammonia and organic amines (e.g., diethylamine, triethylamine, ethyl diisopropylamine, procaine, dibenzylamine, N-methylmorpholine, dihydroabiethylamine, methylpiperidine, L-arginine, creatine, choline, L-lysine, ethylenediamine, N,N-dibenzylethylenediamine (benzathine), ethanolamine, meglumine, and tromethamine).

As used herein, the term "solvate" refers to a coordination complex formed by coordination with a solvent molecule. When the solvent molecule is water, the solvate is a hydrate.

The compounds described herein may be isotopically labeled; that is, one or more atoms therein are replaced with atoms having a different atomic mass or mass number. Such isotopically labeled compounds are considered to be within the scope of the present disclosure. Examples of isotopes that can be incorporated into compounds include isotopes of hydrogen, carbon, nitrogen, oxygen, phosphorus, sulfur, fluorine, chlorine, and iodine, such as but not limited to ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I.

The present disclosure provides a compound of formula (I) or a pharmaceutically acceptable salt, ester, solvate, or stereoisomer thereof: 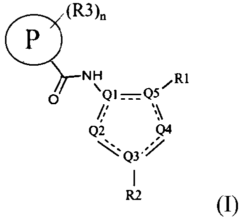 wherein:
Q1, Q2, Q3, Q4, and Q5 are independently selected from C, CH, CH₂, N, NH, S, or O;
--- means that a double bond is present or absent;
ring P is aryl or heteroaryl;
R1 is selected from hydrogen, deuterium, halogen, hydroxyl, sulfhydryl, amino, nitro, cyano, -OR^{1a}, -SR^{1a}, -NR^{1a}R^{1b}, -COR^{1a}, -SOR^{1a}, -SO₂R^{1a}, -COOR^{1a}, -CONR^{1a}R^{1b}, -NR^{1a}COR^{1b}, -OCOR^{1a}, -S(O)(NR^{1a})R^{1b}, -SONR^{1a}R^{1b}, -SO₂NR^{1a}R^{1b}, -NR^{1a}SOR^{1b}, -NR^{1a}SO₂R^{1b}, optionally substituted alkyl, optionally substituted alkoxy, optionally substituted cycloalkyl, and optionally substituted heterocyclyl, wherein R^{1a} and R^{1b} are independently selected from hydrogen, deuterium, optionally substituted alkyl, optionally substituted cycloalkyl, and optionally substituted heterocyclyl;
R2 is selected from optionally substituted cycloalkyl, optionally substituted heterocyclyl, optionally substituted aryl, and optionally substituted heteroaryl;
R3 is selected from hydrogen, deuterium, halogen, hydroxyl, sulfhydryl, amino, nitro, cyano, -OR^{3a}, -SR^{3a}, -NR^{3a}R^{3b}, -COR^{3a} , -SOR^{3a}, -SO₂R^{3a}, -COOR^{3a}, -CONR^{3a}R^{3b}, -NR^{3a}COR^{3b}, -OCOR^{3a}, -S(O)(NR^{3a})R^{3b}, -SONR^{3a}R^{3b}, -SO₂NR^{3a}R^{3b}, -NR^{3a}SOR^{3b}, -NR^{3a}SO₂R^{3b}, optionally substituted alkyl, optionally substituted alkoxy, optionally substituted cycloalkyl, optionally substituted heterocyclyl, optionally substituted aryl, and optionally substituted heteroaryl, wherein R^{3a} and R^{3b} are independently selected from hydrogen, deuterium, optionally substituted alkyl, optionally substituted cycloalkyl, optionally substituted heterocyclyl, optionally substituted aryl, and optionally substituted heteroaryl; and
n is 0, 1, 2, 3, 4, or 5.

In some embodiments, the ring structure where Q1-Q5 are present is selected from the following structures:

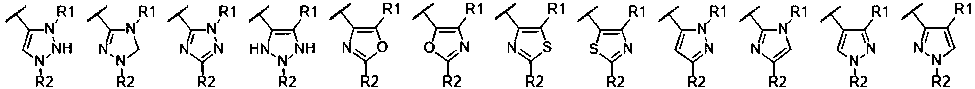

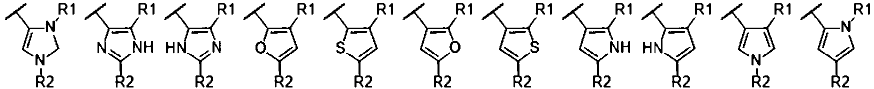

In some embodiments, ring P is 6-10 membered aryl or heteroaryl. In some embodiments, ring P is 6-membered aryl or heteroaryl. In some embodiments, ring P is 9-10 membered bicyclic heteroaryl.

In some embodiments, ring P is selected from phenyl, pyridinyl, pyrazinyl, pyridazinyl, pyrimidinyl, triazinyl, pyrazolopyrimidinyl (e.g., pyrazolo[2,3-A]pyrimidinyl derived from a structure of 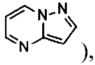 or pyrrolopyridazinyl (e.g., pyrrolo[1,2-B]pyridazinyl derived from a structure of 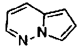 ).

In some embodiments, R1 is selected from hydrogen, deuterium, halogen, hydroxyl, sulfhydryl, amino, nitro, cyano, -OR^{1a}, -SR^{1a}, -NR^{1a}R^{1b}, -COR^{1a}, -SOR^{1a}, -SO₂R^{1a}, -COOR^{1a}, -CONR^{1a}R^{1b}, -NR^{1a}COR^{1b}, -OCOR^{1a}, -S(O)(NR^{1a})R^{1b}, -SONR^{1a}R^{1b}, -SO₂NR^{1a}R^{1b}, -NR^{1a}SOR^{1b}, -NR^{1a}SO₂R^{1b}, optionally substituted C₁₋₁₂ alkyl (e.g., C₁₋₆ alkyl), optionally substituted C₁₋₁₂ alkoxy (e.g., C₁₋₆ alkoxy), optionally substituted C₃₋₁₂ cycloalkyl (e.g., C₃₋₆ cycloalkyl), and optionally substituted 3-12 membered heterocyclyl (e.g., 3-6 membered heterocyclyl), wherein R^{1a} and R^{1b} are independently selected from hydrogen, deuterium, optionally substituted C₁₋₁₂ alkyl (e.g., C₁₋₆ alkyl), optionally substituted C₃₋₁₂ cycloalkyl (e.g., C₃₋₆ cycloalkyl), and optionally substituted 3-12 membered heterocyclyl (e.g., 3-6 membered heterocyclyl).

In some embodiments, R^{1a} and R^{1b} are independently selected from: hydrogen; deuterium; unsubstituted alkyl, cycloalkyl, or heterocyclyl; and alkyl, cycloalkyl, or heterocyclyl substituted with one or more substituents independently selected from: deuterium; halogen; hydroxyl; sulfhydryl; amino; cyano; nitro; an aldehyde group; carboxyl; -OCO-alkyl; -OCOO-alkyl; and alkyl, alkoxy, cycloalkyl, or heterocyclyl optionally substituted with one or more substituents selected from deuterium, halogen, hydroxyl, sulfhydryl, amino, cyano, nitro, an aldehyde group, carboxyl, alkyl, haloalkyl, alkoxy, haloalkoxy, cycloalkyl, halocycloalkyl, heterocyclyl, and/or haloheterocyclyl.

In some embodiments, R1 is selected from unsubstituted alkyl, alkoxy, cycloalkyl, or heterocyclyl; and alkyl, alkoxy, cycloalkyl, or heterocyclyl substituted with one or more substituents independently selected from: deuterium, halogen, hydroxyl, sulfhydryl, amino, cyano, nitro, -OR^{1c}, -SR^{1c}, -NR^{1c}R^{1d}, - COR^{1c}, -SOR^{1c}, -SO₂R^{1c}, -COOR^{1c}, -CONR^{1c}R^{1d}, -NR^{1c}COR^{1d}, -OCOR^{1c}, -S(O)(NR^{1c})R^{1d}, -SONR^{1c}R^{1d}, - SO₂NR^{1c}R^{1d}, -NR^{1c}SOR^{1d}, -NR^{1c}SO₂R^{1d}, optionally substituted alkyl, optionally substituted alkoxy, optionally substituted cycloalkyl, and optionally substituted heterocyclyl, wherein R^{1c} and R^{1d} are independently selected from hydrogen, deuterium, optionally substituted alkyl, optionally substituted cycloalkyl, and optionally substituted heterocyclyl.

In some embodiments, R1 is selected from alkyl, alkoxy, cycloalkyl, or heterocyclyl substituted with one or more substituents independently selected from:
deuterium; halogen; hydroxyl; sulfhydryl; amino; cyano; nitro;
-OR^{1c}, -SR^{1c}, -NR^{1c}R^{1d}, -COR^{1c}, -SOR^{1c}, -SO₂R^{1c}, -COOR^{1c}, -CONR^{1c}R^{1d}, -NR^{1c}COR^{1d}, -OCOR^{1c}, -S(O)(NR^{1c})R^{1d}, -SONR^{1c}R^{1d}, -SO₂NR^{1c}R^{1d}, -NR^{1c}SOR^{1d}, and -NR^{1c}SO₂R^{1d}, wherein R^{1c} and R^{1d} are independently selected from: hydrogen; deuterium; unsubstituted alkyl, cycloalkyl, or heterocyclyl; and alkyl, cycloalkyl, or heterocyclyl substituted with one or more substituents independently selected from: deuterium; halogen; hydroxyl; sulfhydryl; amino; cyano; nitro; an aldehyde group; carboxyl; and alkyl, alkoxy, cycloalkyl, or heterocyclyl optionally substituted with one or more substituents selected from deuterium, halogen, hydroxyl, sulfhydryl, amino, cyano, nitro, an aldehyde group, carboxyl, alkyl, haloalkyl, alkoxy, haloalkoxy, cycloalkyl, halocycloalkyl, heterocyclyl, and/or haloheterocyclyl; and
alkyl, alkoxy, cycloalkyl, or heterocyclyl optionally substituted with one or more substituents independently selected from: deuterium; halogen; hydroxyl; sulfhydryl; amino; cyano; nitro; an aldehyde group; carboxyl; alkyl; alkoxy; cycloalkyl; heterocyclyl; and alkyl, alkoxy, cycloalkyl, or heterocyclyl substituted with one or more substituents selected from deuterium, halogen, hydroxyl, sulfhydryl, amino, cyano, nitro, an aldehyde group, and/or carboxyl.

In some embodiments, R1 is selected from hydrogen; deuterium; halogen; hydroxyl; sulfhydryl; amino; nitro; cyano; alkyl; alkyl substituted with one or more substituents selected from deuterium, halogen, hydroxyl, sulfhydryl, amino, cyano, nitro, an aldehyde group, and/or carboxyl; alkoxy; alkoxy substituted with one or more substituents selected from deuterium, halogen, hydroxyl, sulfhydryl, amino, cyano, nitro, an aldehyde group, and/or carboxyl; cycloalkyl; cycloalkyl substituted with one or more substituents selected from deuterium, halogen, hydroxyl, sulfhydryl, amino, cyano, nitro, an aldehyde group, carboxyl, -OR^{1c}, and/or -NR^{1c}R^{1d}; heterocyclyl; heterocyclyl substituted with one or more substituents selected from deuterium, halogen, hydroxyl, sulfhydryl, amino, cyano, nitro, an aldehyde group, carboxyl, -OR^{1c}, and/or -NR^{1c}R^{1d}; and -OR^{1a}, -SR^{1a}, -NR^{1a}R^{1b}, -COR^{1a}, -SOR^{1a}, -SO₂R^{1a}, -COOR^{1a}, -CONR^{1a}R^{1b}, -NR^{1a}COR^{1b}, - OCOR^{1a}, -S(O)(NR^{1a})R^{1b}, -SONR^{1a}R^{1b}, -SO₂NR^{1a}R^{1b}, -NR^{1a}SOR^{1b}, and -NR^{1a}SO₂R^{1b}, wherein R^{1a} and R^{1b} are independently selected from hydrogen; deuterium; alkyl; cycloalkyl; heterocyclyl; and alkyl, cycloalkyl, or heterocyclyl substituted with one or more substituents selected from deuterium, halogen, hydroxyl, sulfhydryl, amino, cyano, nitro, an aldehyde group, and/or carboxyl, and R^{1c} and R^{1d} are independently selected from hydrogen; deuterium; alkyl; cycloalkyl; heterocyclyl; and alkyl, cycloalkyl, or heterocyclyl substituted with one or more substituents selected from deuterium, halogen, hydroxyl, sulfhydryl, amino, cyano, nitro, an aldehyde group, and/or carboxyl.

In some embodiments, R1 is selected from hydrogen; hydroxyl; cyano; alkyl; haloalkyl; hydroxyalkyl; alkoxy; haloalkoxy; cycloalkyl; cycloalkyl substituted with cyano, -OR^{1c}, and/or -NR^{1c}R^{1d}; heterocyclyl; -NR^{1a}R^{1b}; -COR^{1a}; -CONR^{1a}R^{1b}; -S(O)(NR^{1a})R^{1b}; and -SO₂NR^{1a}R^{1b}, wherein R^{1a} and R^{1b} are independently selected from hydrogen; alkyl; cycloalkyl; and alkyl or cycloalkyl substituted with one or more substituents selected from deuterium, halogen, cyano, -OCO-alkyl, and/or -OCOO-alkyl, and R^{1c} and R^{1d} are independently selected from hydrogen and alkyl.

In some embodiments, R1 is selected from hydroxyl; cyano; methyl; ethyl; propyl (e.g., n-propyl and sec-propyl); tert-butyl; difluoromethyl (-CHF₂); hydroxymethyl; hydroxyethyl; hydroxypropyl (e.g., hydroxyisopropyl); methoxy; ethoxy; isopropoxy; tert-butoxy; trifluoromethoxy; trifluoroethoxy; cyclopropyl; azetidinyl; cyclobutyl; cyclobutyl substituted with cyano, hydroxyl, or -NHCH₃; - S(O)(NH)CH₃; -SO₂NR^{1a}R^{1b}; -NR^{1a}R^{1b}; -COR^{1a}; and -CONR^{1a}R^{1b}, wherein R^{1a} and R^{1b} are independently selected from hydrogen, methyl, isopropyl, -CH₂CN, -CD₃, -CH₂CF₃, difluoromethyl (-CHF₂), -CF₃, cyclopropyl, difluorocyclopropyl, -CH₂OCOOC(CH₃)₃, and -CH₂OCOC(CH₃)₃.

In some embodiments, R2 is selected from optionally substituted C₃₋₁₂ cycloalkyl, optionally substituted 3-12 membered heterocyclyl, optionally substituted 6-10 membered aryl, and optionally substituted 5-10 membered heteroaryl.

In some embodiments, R2 is selected from optionally substituted cyclopropyl, cyclobutyl, cyclopentyl, or cyclohexyl, bicyclo[1.1.1]pentyl (e.g., 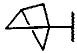 ), bicyclo[2.2.2]octyl (e.g., 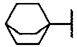 ), and cubanyl (e.g., 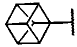 ).

In some embodiments, R2 is selected from optionally substituted 3-12 membered heterocyclyl comprising one to six (e.g., one, two, three, or four) heteroatoms independently selected from N, O, and S.

In some embodiments, R2 is selected from optionally substituted tetrahydropyrrolyl, piperidinyl, piperazinyl, azetidinyl, 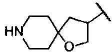 (1-oxa-8-azaspiro[4.5]decyl), and 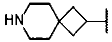 (7-azaspiro[3.5]nonyl).

In some embodiments, R2 is selected from optionally substituted phenyl.

In some embodiments, R2 is selected from optionally substituted 5-6 membered heteroaryl. In some embodiments, R2 is selected from optionally substituted pyridinyl, pyrazinyl, pyridazinyl, pyrimidinyl, or triazinyl.

In some embodiments, R2 is selected from unsubstituted cycloalkyl, heterocyclyl, aryl, or heteroaryl; and cycloalkyl, heterocyclyl, aryl, or heteroaryl substituted with one or more substituents independently selected from: deuterium, halogen, hydroxyl, sulfhydryl, amino, cyano, nitro, -OR^{2a}, -SR^{2a}, -NR^{2a}R^{2b}, - COR^{2a}, -SOR^{2a}, -SO₂R^{2a}, -COOR^{2a}, -CONR^{2a}R^{2b}, -NR^{2a}COR^{2b}, -OCOR^{2a}, -S(O)(NR^{2a})R^{2b}, -SONR^{2a}R^{2b}, - SO₂NR^{2a}R^{2b}, -NR^{2a}SOR^{2b}, -NR^{2a}SO₂R^{2b}, optionally substituted alkyl, optionally substituted alkoxy, optionally substituted cycloalkyl, optionally substituted heterocyclyl, optionally substituted aryl, and optionally substituted heteroaryl, wherein R^{2a} and R^{2b} are independently selected from hydrogen, deuterium, optionally substituted alkyl, optionally substituted cycloalkyl, optionally substituted heterocyclyl, optionally substituted aryl, and optionally substituted heteroaryl.

In some embodiments, R^{2a} and R^{2b} are independently selected from: hydrogen; deuterium; unsubstituted alkyl, cycloalkyl, heterocyclyl, aryl, and heteroaryl; and alkyl, cycloalkyl, heterocyclyl, aryl, and heteroaryl substituted with one or more substituents independently selected from: deuterium; halogen; hydroxyl; sulfhydryl; amino; cyano; nitro; an aldehyde group; carboxyl; and alkyl, alkoxy, cycloalkyl, heterocyclyl, aryl, and heteroaryl optionally substituted with one or more substituents selected from deuterium, halogen, hydroxyl, sulfhydryl, amino, cyano, nitro, an aldehyde group, carboxyl, alkyl, haloalkyl, alkoxy, haloalkoxy, cycloalkyl, halocycloalkyl, heterocyclyl, and/or haloheterocyclyl.

In some embodiments, R2 is selected from cycloalkyl, heterocyclyl, aryl, or heteroaryl substituted with one or more substituents independently selected from:
deuterium; halogen; hydroxyl; sulfhydryl; amino; cyano; nitro;
-OR^{2a}, -SR^{2a}, -NR^{2a}R^{2b}, -COR^{2a}, -SOR^{2a}, -SO₂R^{2a}, -COOR^{2a}, -CONR^{2a}R^{2b}, -NR^{2a}COR^{2b}, -OCOR^{2a}, -S(O)(NR^{2a})R^{2b}, -SONR^{2a}R^{2b}, -SO₂NR^{2a}R^{2b}, -NR^{2a}SOR^{2b}, and -NR^{2a}SO₂R^{2b}, wherein R^{2a} and R^{2b} are independently selected from hydrogen, deuterium, optionally substituted alkyl, optionally substituted cycloalkyl, optionally substituted heterocyclyl, optionally substituted aryl, and optionally substituted heteroaryl; and
alkyl, alkoxy, cycloalkyl, heterocyclyl, aryl, or heteroaryl optionally substituted with one or more substituents independently selected from: deuterium; halogen; hydroxyl; sulfhydryl; amino; cyano; nitro; - OR^{2c}; -SR^{2c}; -NR^{2c}R^{2d}; -COR^{2c}; -SOR^{2c}; -SO₂R^{2c}; -COOR^{2c}; -CONR^{2c}R^{2d}; -NR^{2c}COR^{2d}; -OCOR^{2c}; - S(O)(NR^{2c})R^{2d}; -SONR^{2c}R^{2d}; -SO₂NR^{2c}R^{2d}; -NR^{2c}SOR^{2d}; -NR^{2c}SO₂R^{2d}, alkyl; alkoxy; cycloalkyl; heterocyclyl; aryl; heteroaryl; and alkyl, alkoxy, cycloalkyl, heterocyclyl, aryl, or heteroaryl substituted with one or more substituents selected from deuterium, halogen, hydroxyl, sulfhydryl, amino, cyano, nitro, an aldehyde group, and/or carboxyl, wherein R^{2c} and R^{2d} are independently selected from hydrogen; deuterium; unsubstituted alkyl, cycloalkyl, heterocyclyl, aryl, or heteroaryl; and alkyl, cycloalkyl, heterocyclyl, aryl, or heteroaryl substituted with one or more substituents independently selected from: deuterium; halogen; hydroxyl; sulfhydryl; amino; cyano; nitro; an aldehyde group; carboxyl; and alkyl, alkoxy, cycloalkyl, heterocyclyl, aryl, or heteroaryl optionally substituted with one or more substituents selected from deuterium, halogen, hydroxyl, sulfhydryl, amino, cyano, nitro, an aldehyde group, carboxyl, alkyl, haloalkyl, alkoxy, haloalkoxy, cycloalkyl, halocycloalkyl, heterocyclyl, and/or haloheterocyclyl.

In some embodiments, R2 is selected from:
cycloalkyl; heterocyclyl; aryl; heteroaryl;
cycloalkyl, heterocyclyl, aryl, or heteroaryl substituted with one or more substituents selected from deuterium, halogen, hydroxyl, sulfhydryl, amino, nitro, cyano, -OR^{2a}, -NR^{2a}R^{2b}, -COR^{2a}, -COOR^{2a}, - CONR^{2a}R^{2b}, and/or -NR^{2a}COR^{2b}, wherein R^{2a} and R^{2b} are independently selected from hydrogen; alkyl; cycloalkyl; heterocyclyl; aryl; heteroaryl; and alkyl, cycloalkyl, heterocyclyl, aryl, or heteroaryl substituted with one or more substituents selected from deuterium, halogen, hydroxyl, sulfhydryl, amino, cyano, nitro, an aldehyde group, carboxyl, alkyl, and/or haloalkyl;
cycloalkyl, heterocyclyl, aryl, or heteroaryl substituted with one or more substituents selected from: alkyl, cycloalkyl, or heterocyclyl substituted with one or more substituents selected from deuterium, halogen, hydroxyl, sulfhydryl, amino, cyano, nitro, -OR^{2c}, -NR^{2c}R^{2d}, -COR^{2c}, -COOR^{2c}, -CONR^{2c}R^{2d}, and/or-NR^{2c}COR^{2d}, wherein R^{2c} and R^{2d} are independently selected from hydrogen; alkyl; cycloalkyl; heterocyclyl; aryl; heteroaryl; and alkyl, cycloalkyl, heterocyclyl, aryl, or heteroaryl substituted with one or more substituents of deuterium, halogen, hydroxyl, sulfhydryl, amino, cyano, nitro, an aldehyde group, carboxyl, alkyl, and/or haloalkyl; and
cycloalkyl, heterocyclyl, aryl, or heteroaryl substituted with one or more substituents selected from: alkyl; cycloalkyl; heterocyclyl; and alkyl, cycloalkyl, or heterocyclyl substituted with alkyl, cycloalkyl, and/or heterocyclyl optionally substituted with one or more substituents selected from deuterium, halogen, hydroxyl, sulfhydryl, amino, cyano, nitro, an aldehyde group, carboxyl, alkyl, and/or haloalkyl.

In some embodiments, R2 is selected from: cycloalkyl; heterocyclyl; aryl; heteroaryl; and cycloalkyl, heterocyclyl, aryl, and heteroaryl substituted with one or more substituents independently selected from: halogen; -OR^{2a}; -NR^{2a}R^{2b}; -COR^{2a}; -COOR^{2a}; -CONR^{2a}R^{2b}; -NR^{2a}COR^{2b}; alkyl; cycloalkyl; heterocyclyl; and alkyl, cycloalkyl, or heterocyclyl substituted with one or more substituents selected from halogen, -OR^{2c}, -NR^{2c}R^{2d}, -COR^{2c}, -COOR^{2c}, -CONR^{2c}R^{2d}, -NR^{2c}COR^{2d}, and/or alkyl, wherein R^{2a} and R^{2b} are independently selected from hydrogen, alkyl, cycloalkyl, heterocyclyl, aryl, and alkyl substituted with the following substituent: alkyl-substituted cycloalkyl or heterocyclyl, and R^{2c} and R^{2d} are independently selected from hydrogen, alkyl, cycloalkyl, heterocyclyl, aryl, and alkyl substituted with the following substituent: alkyl-substituted cycloalkyl or heterocyclyl.

In some embodiments, R2 is selected from phenyl; pyridinyl; pyrazinyl; pyridazinyl; pyrimidinyl; piperidinyl; cyclobutyl and cyclohexyl; azetidinyl; 1-oxa-8-azaspiro[4.5]decyl; 7-azaspiro[3.5]nonyl; phenyl, pyridinyl, pyrazinyl, pyridazinyl, pyrimidinyl, piperidinyl, cyclobutyl, and cyclohexyl substituted with one or more substituents selected from fluorine, bromine, -OR^{2a}, -NR^{2a}R^{2b}, -COR^{2a}, -COOR^{2a}, - CONR^{2a}R^{2b}, -NR^{2a}COR^{2b}, and/or piperazinyl, wherein R^{2a} and R^{2b} are selected from hydrogen, methyl, tert-butyl, piperidinyl, and methylpiperidinyl-substituted methyl; and phenyl substituted with one or more substituents selected from: methyl or piperazinyl substituted with one or more substituents selected from methyl, -OR^{2c}, -NR^{2c}R^{2d}, -COR^{2c}, -COOR^{2c}, -CONR^{2c}R^{2d}, and/or -NR^{2c}COR^{2d}, wherein R^{2c} and R^{2d} are independently selected from hydrogen, methyl, phenyl, or methylpiperidinyl-substituted methyl.

In some embodiments, R3 is selected from hydrogen, deuterium, halogen, hydroxyl, sulfhydryl, amino, nitro, cyano, -OR^{3a}, -SR^{3a}, -NR^{3a}R^{3b}, -COR^{3a}, -SOR^{3a}, -SO₂R^{3a}, -COOR^{3a}, -CONR^{3a}R^{3b}, -NR^{3a}COR^{3b}, -OCOR^{3a}, -S(O)(NR^{3a})R^{3b}, -SONR^{3a}R^{3b}, -SO₂NR^{3a}R^{3b}, -NR^{3a}SOR^{3b}, -NR^{3a}SO₂R^{3b}, optionally substituted C₁₋₁₂ alkyl (e.g., C₁₋₆ alkyl), optionally substituted C₁₋₁₂ alkoxy (e.g., C₁₋₆ alkoxy), optionally substituted C₃₋₁₂ cycloalkyl (e.g., C₃₋₆ cycloalkyl), optionally substituted 3-12 membered heterocyclyl (e.g., 3-10 membered heterocyclyl), optionally substituted 6-10 membered aryl (e.g., phenyl), and optionally substituted 5-10 membered heteroaryl (e.g., 5-6 membered heteroaryl), wherein R^{3a} and R^{3b} are independently selected from hydrogen, deuterium, optionally substituted C₁₋₁₂ alkyl (e.g., C₁₋₆ alkyl), optionally substituted C₃₋₁₂ cycloalkyl (e.g., C₃₋₆ cycloalkyl), optionally substituted 3-12 membered heterocyclyl (e.g., 3-10 membered heterocyclyl), optionally substituted 6-10 membered aryl (e.g., phenyl), and optionally substituted 5-10 membered heteroaryl (e.g., 5-6 membered heteroaryl).

In some embodiments, R^{3a} and R^{3b} are independently selected from: hydrogen; deuterium; unsubstituted alkyl, cycloalkyl, heterocyclyl, aryl, or heteroaryl; and alkyl, cycloalkyl, heterocyclyl, aryl, or heteroaryl substituted with one or more substituents independently selected from: deuterium; halogen; hydroxyl; sulfhydryl; amino; cyano; nitro; an aldehyde group; carboxyl; and alkyl, alkoxy, cycloalkyl, or heterocyclyl optionally substituted with one or more substituents selected from deuterium, halogen, hydroxyl, sulfhydryl, amino, cyano, nitro, an aldehyde group, carboxyl, alkyl, haloalkyl, alkoxy, haloalkoxy, cycloalkyl, halocycloalkyl, heterocyclyl, and/or haloheterocyclyl.

In some embodiments, R3 is selected from unsubstituted alkyl, alkoxy, cycloalkyl, heterocyclyl, aryl, or heteroaryl; and alkyl, alkoxy, cycloalkyl, heterocyclyl, aryl, or heteroaryl substituted with one or more substituents independently selected from: deuterium, halogen, hydroxyl, sulfhydryl, amino, cyano, nitro, =O, =S, -OR^{3c}, -SR^{3c}, -NR^{3c}R^{3d}, -COR^{3c}, -SOR^{3c}, -SO₂R^{3c}, -COOR^{3c}, -CONR^{3c}R^{3d}, -NR^{3c}COR^{3d}, - OCOR^{3c}, -S(O)(NR^{3c})R^{3d}, -SONR^{3c}R^{3d}, -SO₂NR^{3c}R^{3d}, -NR^{3c}SOR^{3d}, -NR^{3c}SO₂R^{3d}, optionally substituted alkyl, optionally substituted alkoxy, optionally substituted cycloalkyl, optionally substituted heterocyclyl, optionally substituted aryl, or optionally substituted heteroaryl, wherein R^{3c} and R^{3d} are independently selected from hydrogen, deuterium, optionally substituted alkyl, optionally substituted cycloalkyl, optionally substituted heterocyclyl, optionally substituted aryl, and optionally substituted heteroaryl.

In some embodiments, R3 is selected from alkyl, alkoxy, cycloalkyl, heterocyclyl, aryl, or heteroaryl substituted with one or more substituents independently selected from:
deuterium; halogen; hydroxyl; sulfhydryl; amino; cyano; nitro; =O; =S;
-OR^{3c}, -SR^{3c}, -NR^{3c}R^{3d}, -COR^{3c}, -SOR^{3c}, -SO₂R^{3c}, -COOR^{3c}, -CONR^{3c}R^{3d}, -NR^{3c}COR^{3d}, -OCOR^{3c}, -S(O)(NR^{3c})R^{3d}, -SONR^{3c}R^{3d}, -SO₂NR^{3c}R^{3d} -NR^{3c}SOR^{3d}, and -NR^{3c}SO₂R^{3d}, wherein R^{3c} and R^{3d} are independently selected from hydrogen; deuterium; unsubstituted alkyl, cycloalkyl, heterocyclyl, aryl, or heteroaryl; and alkyl, cycloalkyl, heterocyclyl, aryl, or heteroaryl substituted with one or more substituents independently selected from: deuterium; halogen; hydroxyl; sulfhydryl; amino; cyano; nitro; an aldehyde group; carboxyl; and alkyl, alkoxy, cycloalkyl, or heterocyclyl optionally substituted with one or more substituents selected from deuterium, halogen, hydroxyl, sulfhydryl, amino, cyano, nitro, an aldehyde group, carboxyl, alkyl, haloalkyl, alkoxy, haloalkoxy, cycloalkyl, halocycloalkyl, heterocyclyl, or haloheterocyclyl; and
alkyl, alkoxy, cycloalkyl, heterocyclyl, aryl, or heteroaryl optionally substituted with one or more substituents independently selected from deuterium; halogen; hydroxyl; sulfhydryl; amino; cyano; nitro; an aldehyde group; carboxyl; =O; =S; -OR^{3e}, -SR^{3e}, -NR^{3e}R^{3f}, -COR^{3e}, -SOR^{3e}, -SO₂R^{3e}, -COOR^{3e}, -CONR^{3e}R^{3f}, -NR^{3e}COR^{3f}, -OCOR^{3e}, -S(O)(NR^{3e})R^{3f}, -SONR^{3e}R^{3f}, -SO₂NR^{3e}R^{3f}, -NR^{3e}SOR^{3f}, and -NR^{3e}SO₂R^{3f}, wherein R^{3e} and R^{3f} are independently selected from hydrogen, deuterium, alkyl, haloalkyl, cycloalkyl, halocycloalkyl, heterocyclyl, and haloheterocyclyl; alkyl; alkoxy; cycloalkyl; heterocyclyl; and alkyl, alkoxy, cycloalkyl, or heterocyclyl substituted with one or more substituents selected from deuterium, halogen, hydroxyl, sulfhydryl, amino, cyano, nitro, an aldehyde group, and/or carboxyl.

In some embodiments, R3 is selected from:
hydrogen; deuterium; halogen; hydroxyl; sulfhydryl; amino; nitro; cyano;
-OR^{3a}, -SR^{3a}, -NR^{3a}R^{3b}, -COR^{3a}, -SOR^{3a}, -SO₂R^{3a}, -COOR^{3a}, -CONR^{3a}R^{3b}, -NR^{3a}COR^{3b}, -OCOR^{3a}, -S(O)(NR^{3a})R^{3b}, -SONR^{3a}R^{3b}, -SO₂NR^{3a}R^{3b}, -NR^{3a}SOR^{3b}, and -NR^{3a}SO₂R^{3b}, wherein R^{3a} and R^{3b} are independently selected from hydrogen and alkyl;
alkyl substituted with one or more substituents selected from deuterium, halogen, hydroxyl, sulfhydryl, amino, cyano, nitro, -OR^{3c}, -SR^{3c}, -NR^{3c}R^{3d}, -COR^{3c}, -SOR^{3c}, -SO₂R^{3c}, -COOR^{3c}, -CONR^{3c}R^{3d}, -NR^{3c}COR^{3d}, -OCOR^{3c}, -S(O)(NR^{3c})R^{3d}, -SONR^{3c}R^{3d}, -SO₂NR^{3c}R^{3d} -NR^{3c}SOR^{3d}, and/or -NR^{3c}SO₂R^{3d}, wherein R^{3c} and R^{3d} are independently selected from hydrogen and alkyl;
alkyl substituted with one or more substituents selected from: heterocyclyl substituted with one or more substituents selected from deuterium, halogen, hydroxyl, sulfhydryl, amino, cyano, nitro, =O, =S, - OR^{3e}, -SR^{3e}, -NR^{3e}R^{3f}, -COR^{3e}, -SOR^{3e}, -SO₂R^{3e}, -COOR^{3e}, -CONR^{3e}R^{3f}, -NR^{3e}COR^{3f}, -OCOR^{3e}, - S(O)(NR^{3e})R^{3f}, -SONR^{3e}R^{3f}, -SO₂NR^{3e}R^{3f}, -NR^{3e}SOR^{3f}, -NR^{3e}SO₂R^{3f}, and/or alkyl, wherein R^{3e} and R^{3f} are independently selected from hydrogen and alkyl;
heterocyclyl;
heterocyclyl substituted with one or more substituents selected from deuterium, halogen, hydroxyl, sulfhydryl, amino, cyano, nitro, =O, =S, -OR^{3c}, -SR^{3c}, -NR^{3c}R^{3d}, -COR^{3c}, -SOR^{3c}, -SO₂R^{3c}, -COOR^{3c}, - CONR^{3c}R^{3d}, -NR^{3c}COR^{3d}, -OCOR^{3c}, -S(O)(NR^{3c})R^{3d}, -SONR^{3c}R^{3d}, -SO₂NR^{3c}R^{3d} -NR^{3c}SOR^{3d}, - NR^{3c}SO₂R^{3d}, and/or alkyl;
heterocyclyl substituted with one or more substituents selected from: alkyl substituted with one or more substituents selected from deuterium, halogen, hydroxyl, sulfhydryl, amino, cyano, nitro, -OR^{3e}, -SR^{3e},
-NR^{3e}R^{3f}; -COR^{3e}, -SOR^{3e}, -SO₂R^{3e}, -COOR^{3e}, -CONR^{3e}R^{3f}, -NR^{3e}COR^{3f}, -OCOR^{3e}, -S(O)(NR^{3e})R^{3f}, - SONR^{3e}R^{3f}, -SO₂NR^{3e}R^{3f}, -NR^{3e}SOR^{3f}, and/or -NR^{3e}SO₂R^{3f};
heteroaryl;
heteroaryl substituted with one or more substituents selected from deuterium, halogen, hydroxyl, sulfhydryl, amino, cyano, nitro, -OR^{3c}, -SR^{3c}, -NR^{3c}R^{3d}, -COR^{3c}, -SOR^{3c}, -SO₂R^{3c}, -COOR^{3c}, -CONR^{3c}R^{3d}, -NR^{3c}COR^{3d}, -OCOR^{3c}, -S(O)(NR^{3c})R^{3d}, -SONR^{3c}R^{3d}, -SO₂NR^{3c}R^{3d}, -NR^{3c}SOR^{3d}, -NR^{3c}SO₂R^{3d}, and/or alkyl; and
heteroaryl substituted with one or more substituents selected from: alkyl substituted with one or more substituents selected from deuterium, halogen, hydroxyl, sulfhydryl, amino, cyano, nitro, -OR^{3e}, -SR^{3e}, -NR^{3e}R^{3f}; -COR^{3e}, -SOR^{3e}, -SO₂R^{3e}, -COOR^{3e}, -CONR^{3e}R^{3f}, -NR^{3e}COR^{3f}, -OCOR^{3e}, -S(O)(NR^{3e})R^{3f}; - SONR^{3e}R^{3f}, -SO₂NR^{3e}R^{3f}, -NR^{3e}SOR^{3f}, and/or -NR^{3e}SO₂R^{3f};
wherein R^{3a}, R^{3b}, R^{3c}, R^{3d}, R^{3e}, and R^{3f} are independently selected from hydrogen and alkyl.

In some embodiments, R3 is selected from hydrogen; deuterium; halogen; hydroxyl; sulfhydryl; amino; nitro; cyano; -OR^{3a}, -NR^{3a}R^{3a}, and -CONR^{3a}R^{3b}; alkyl substituted with one or more substituents selected from halogen, -OR^{3c}, -NR^{3c}R^{3d}, and/or -CONR^{3c}R^{3d}; alkyl substituted with one or more substituents selected from: heterocyclyl substituted with one or more substituents selected from halogen, =O, =S, -OR^{3e}, -NR^{3e}R^{3f}, -CONR^{3e}R^{3f}, and/or alkyl; heterocyclyl; heterocyclyl substituted with one or more substituents selected from halogen, =O, =S, -OR^{3c}, -NR^{3c}R^{3d}, -CONR^{3c}R^{3d}, and/or alkyl; heterocyclyl substituted with one or more substituents selected from: alkyl substituted with one or more substituents selected from halogen, -OR^{3e}, -NR^{3e}R^{3f}, and/or -CONR^{3e}R^{3f}; heteroaryl; heteroaryl substituted with one or more substituents selected from halogen, -OR^{3c}, -NR^{3c}R^{3d}, -CONR^{3c}R^{3d}, and/or alkyl; and heteroaryl substituted with one or more substituents selected from: alkyl substituted with one or more substituents selected from halogen, -OR^{3e}, -NR^{3e}R^{3f}, and/or -CONR^{3e}R^{3f};
wherein R^{3a}, R^{3b}, R^{3c}, R^{3d}, R^{3e}, and R^{3f} are independently selected from hydrogen and alkyl.

In some embodiments, R3 is selected from: nitro; -CONH₂; trifluoromethyl; difluoroethyl;
methyl substituted with the following substituent: tetrahydropyrimidinyl, triazacyclopentyl, morpholinyl, imidazolidinyl, octahydropyrrolo[3,4-B][1,4]oxazinyl, 2-oxa-5-azabicyclo[2.2.1]heptyl, 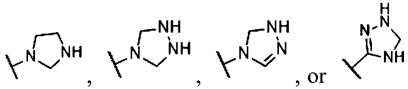 optionally substituted with one or more substituents selected from =O, =S, alkyl (e.g., methyl), and/or substituted alkyl (e.g., difluoromethyl, hydroxymethyl, aminomethyl, or dimethylaminomethyl);
tetrahydropyrimidinyl; triazacyclopentyl; morpholinyl; imidazolidinyl; octahydropyrrolo[3,4-B][1,4]oxazinyl; 2-oxa-5-azabicyclo[2.2.1]heptyl; 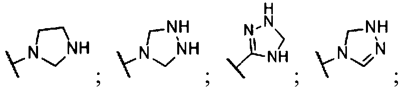
the following group substituted with one or more substituents selected from =O, =S, alkyl (e.g., methyl), and/or substituted alkyl (e.g., difluoromethyl, hydroxymethyl, aminomethyl, or dimethylaminomethyl): tetrahydropyrimidinyl, morpholinyl, imidazolidinyl, triazacyclopentyl, octahydropyrrolo[3,4-B][1,4]oxazinyl, 2-oxa-5-azabicyclo[2.2.1]heptyl, 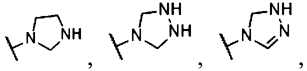 or 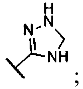 and
pyrrolyl, imidazolyl, pyrazolyl, triazolyl, tetrazolyl, thiadiazolyl, and amino-substituted thiadiazole.

In some embodiments, R3 is selected from the following structures:

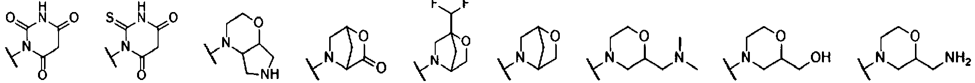

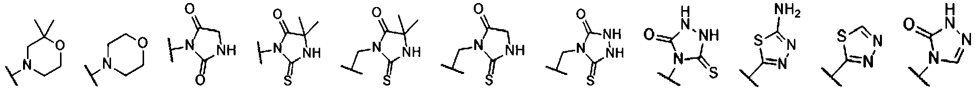

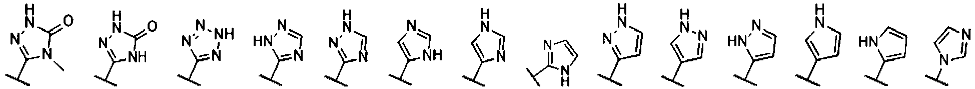

In some embodiments, when an R3 substituent is present, the substitution with the R3 substituent may occur at the ortho-, meta-, or para-position (e.g., meta-position) of the position where ring P is attached to the rest of the molecule.

In some embodiments, the compound of formula (I) of the present disclosure has formula (III): 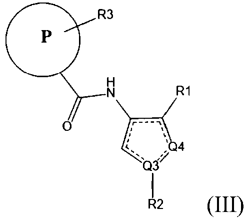
wherein the ring structure where Q3 and Q4 are present is selected from the following structures: 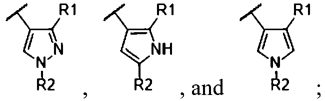
wherein the definitions of ring P, R1, R2, and R3 in formula (III) are the same as those in formula (I).

In some embodiments, the present disclosure relates to a compound of formula (III) or a pharmaceutically acceptable salt, ester, solvate, or stereoisomer thereof: 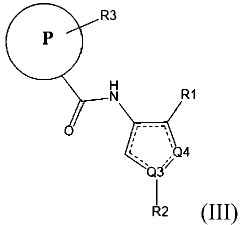 wherein:
the ring structure where Q3 and Q4 are present is selected from the following structures: 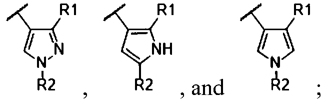
ring P is pyridinyl, pyrazolopyrimidinyl, or pyrrolopyridazinyl;
R1 is selected from -OR^{1a}, -COR^{1a}, -CONR^{1a}R^{1b}, -S(O)(NR^{1a})R^{1b}, -SO₂NR^{1a}R^{1b}, and optionally substituted alkyl, wherein R^{1a} and R^{1b} are independently selected from hydrogen, deuterium, optionally substituted alkyl, and optionally substituted cycloalkyl;
R2 is selected from optionally substituted cycloalkyl, optionally substituted heterocyclyl, and optionally substituted phenyl, pyridinyl, and pyrimidinyl;
R3 is selected from cyano, optionally substituted alkyl, and optionally substituted heteroaryl.

In some embodiments, R1 is selected from alkyl (e.g., methyl, ethyl, or propyl (e.g., isopropyl)) substituted with one or more substituents independently selected from halogen and hydroxyl:
In some embodiments, R^{1a} and R^{1b} are independently selected from: hydrogen; deuterium; alkyl (e.g., methyl, ethyl, or propyl (e.g., isopropyl)); cycloalkyl (e.g., cyclopropyl); and alkyl (e.g., methyl, ethyl, or propyl (e.g., isopropyl)) and cycloalkyl (e.g., cyclopropyl) substituted with one or more substituents independently selected from: deuterium, halogen (e.g., fluorine), hydroxyl, sulfhydryl, amino, cyano, and nitro.

In some embodiments, R1 is selected from -CONH₂, hydroxyisopropyl, hydroxymethyl, hydroxyethyl, methoxy, difluoromethyl, -CONH-(cyclopropyl), -SO₂NH₂, -S(O)(NH)CH₃, -OCH₂CF₃, - COCH₂CF₃, -COCH₃, -COCD₂H, -COCF₃, -COCHF₂, -CONHCH₃, -CONHCH(CH₃)₂, -SO₂NHCH₃, - SO₂NHCH(CH₃)₂, and -CONH-(difluorocyclopropyl).

In some embodiments, R2 is selected from optionally substituted cyclobutyl, cyclopentyl, cyclohexyl, bicyclo[1.1.1]pentyl, or bicyclo[2.2.2]octyl; optionally substituted piperidinyl, azetidinyl, azacyclopentyl, 1-oxa-8-azaspiro[4.5]decyl, or 7-azaspiro[3.5]nonyl; and optionally substituted phenyl, pyridinyl, or pyrimidinyl.

In some embodiments, R2 is selected from unsubstituted cycloalkyl, heterocyclyl, phenyl, and pyridinyl; and cycloalkyl, heterocyclyl, phenyl, and pyridinyl substituted with one or more substituents independently selected from: deuterium, halogen (e.g., fluorine or bromine), hydroxyl, sulfhydryl, amino, cyano, nitro, -OR^{2a}, -NR^{2a}R^{2b}, -COR^{2a}, -COOR^{2a}, -CONR^{2a}R^{2b}, -NR^{2a}COR^{2b}, -OCOR^{2a}, optionally substituted alkyl, and optionally substituted heterocyclyl, wherein R^{2a} and R^{2b} are independently selected from hydrogen, deuterium, optionally substituted alkyl, and optionally substituted heterocyclyl.

In some embodiments, R^{2a} and R^{2b} are independently selected from hydrogen, deuterium, alkyl (e.g., methyl), halogen-substituted alkyl, heterocyclyl (e.g., piperazinyl), and alkyl-substituted heterocyclyl.

In some embodiments, R2 is selected from cycloalkyl, heterocyclyl, phenyl, and pyridinyl substituted with one or more substituents independently selected from:
alkyl;
heterocyclyl;
alkyl (e.g., methyl) or heterocyclyl (e.g., piperazinyl) substituted with one or more substituents selected from -OR^{2e}, -NR^{2c}R^{2d}, -COR^{2c}, -COOR^{2c}, -CONR^{2c}R^{2d}, and/or -NR^{2c}COR^{2d}, wherein R^{2c} and R^{2d} are independently selected from hydrogen, deuterium, alkyl (e.g., methyl), and phenyl; and
heterocyclyl (e.g., piperazinyl) substituted with one or more substituents selected from alkyl (e.g., methyl).

In some embodiments, R2 is selected from phenyl; and phenyl substituted with one or more substituents independently selected from: deuterium; halogen (e.g., fluorine or bromine); -OR^{2a}, -NR^{2a}R^{2b}, -COR^{2a}, -COOR^{2a}, -CONR^{2a}R^{2b}, -NR^{2a}COR^{2b}, and -OCOR^{2a}, wherein R^{2a} and R^{2b} are independently selected from hydrogen, deuterium, alkyl (e.g., methyl), and heterocyclyl (e.g., piperazinyl); and alkyl (e.g., methyl), heterocyclyl (e.g., piperazinyl), and alkyl (e.g., methyl) or heterocyclyl (e.g., piperazinyl) substituted with one or more substituents independently selected from: -OR^{2c}, -NR^{2c}R^{2d}, -COR^{2c}, -COOR^{2c}, -CONR^{2c}R^{2d}, and/or -NR^{2c}COR^{2d}, wherein R^{2c} and R^{2d} are independently selected from hydrogen, deuterium, alkyl (e.g., methyl), and phenyl.

In some embodiments, R2 is selected from phenyl; and phenyl substituted with one or more substituents independently selected from: fluorine, bromine, hydroxyl, methoxy, amino, -NHCH₃, -N(CH₃)₂, -NHCOCH₃, -COOH, -CONHCH₃, -CON(CH₃)₂, -CO-(N-piperazinyl), -CH₂OH, -CH₂OCH₃, -CH₂NH₂, - CH₂NHCH₃, -CH₂N(CH₃)₂, -CH₂NHCOCH₃, -CH₂NCH₃COCH₃, piperazinyl, 4-methylpiperazinyl, 4-acetylpiperazinyl, and 4-benzoylpiperazinyl.

In some embodiments, R3 is selected from halogen (e.g., fluorine)-substituted alkyl (e.g., methyl), such as trifluoromethyl.

In some embodiments, R3 is selected from 5-membered heteroaryl comprising one or more nitrogen ring atoms.

In some embodiments, R3 is selected from:

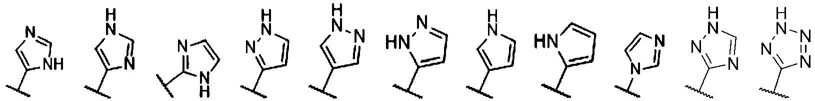

In some embodiments, ring P is pyridinyl, and R3 is selected from -CF₃.

In some embodiments, ring P is pyridinyl or pyrazolopyrimidinyl, and R3 is selected from:

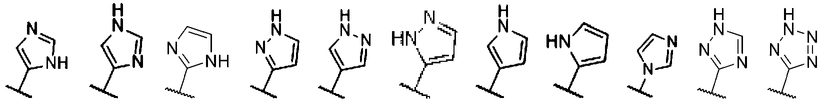

In some embodiments, ring P is pyrrolopyridazinyl, and R3 is cyano.

In some embodiments, ring P is pyridinyl, R1 is -CONH₂, R2 is optionally substituted phenyl, and R3 is selected from -CF₃.

In some embodiments, ring P is pyridinyl, R2 is optionally substituted phenyl, and R3 is selected from:

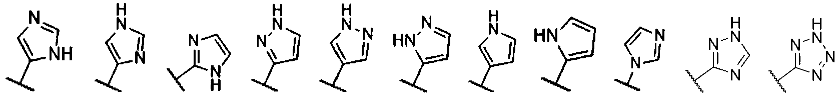

In some embodiments, ring P is pyridinyl, R1 is selected from -CONH₂, hydroxyisopropyl, hydroxymethyl, hydroxyethyl, methoxy, difluoromethyl, -CONH-(cyclopropyl), -SO₂NH₂, -S(O)(NH)CH₃, -OCH₂CF₃, -COCH₂CF₃, -COCH₃, -COCD₂H, -COCF₃, -COCHF₂, -CONHCH₃, -CONHCH(CH₃)₂, - SO₂NHCH₃, -SO₂NHCH(CH₃)₂, and -CONH-(difluorocyclopropyl), R2 is phenyl, and R3 is selected from:

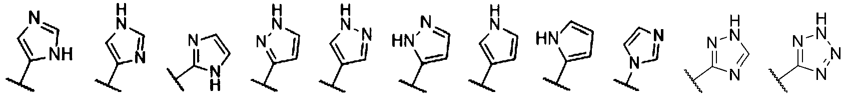

The present disclosure further provides a compound of formula (II) or a pharmaceutically acceptable salt, ester, solvate, or stereoisomer thereof:

A-L-E (II)

wherein moiety A has the following structure: 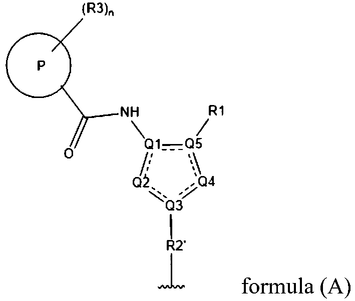 wherein:
Q1, Q2, Q3, Q4, and Q5 are independently selected from C, CH, CH₂, N, NH, S, or O;
--- means that a double bond is present or absent;
ring P is aryl or heteroaryl;
R1 is selected from hydrogen, deuterium, halogen, hydroxyl, sulfhydryl, amino, nitro, cyano, -OR^{1a}, -SR^{1a}, -NR^{1a}R^{1b}, -COR^{1a}, -SOR^{1a}, -SO₂R^{1a}, -COOR^{1a}, -CONR^{1a}R^{1b}, -NR^{1a}COR^{1b}, -OCOR^{1a}, -S(O)(NR^{1a})R^{1b}, -SONR^{1a}R^{1b}, -SO₂NR^{1a}R^{1b}, -NR^{1a}SOR^{1b}, -NR^{1a}SO₂R^{1b}, optionally substituted alkyl, optionally substituted alkoxy, optionally substituted cycloalkyl, and optionally substituted heterocyclyl, wherein R^{1a} and R^{1b} are independently selected from hydrogen, deuterium, optionally substituted alkyl, optionally substituted cycloalkyl, and optionally substituted heterocyclyl;
R3 is selected from hydrogen, deuterium, halogen, hydroxyl, sulfhydryl, amino, nitro, cyano, -OR^{3a}, -SR^{3a}, -NR^{3a}R^{3b}, -COR^{3a}, -SOR^{3a}, -SO₂R^{3a}, -COOR^{3a}, -CONR^{3a}R^{3b}, -NR^{3a}COR^{3b}, -OCOR^{3a}, -S(O)(NR^{3a})R^{3b}, -SONR^{3a}R^{3b}, -SO₂NR^{3a}R^{3b}, -NR^{3a}SOR^{3b}, -NR^{3a}SO₂R^{3b}, optionally substituted alkyl, optionally substituted alkoxy, optionally substituted cycloalkyl, optionally substituted heterocyclyl, optionally substituted aryl, and optionally substituted heteroaryl, wherein R^{3a} and R^{3b} are independently selected from hydrogen, deuterium, optionally substituted alkyl, optionally substituted cycloalkyl, optionally substituted heterocyclyl, optionally substituted aryl, and optionally substituted heteroaryl;
n is 0, 1, 2, 3, 4, or 5;
R2' is selected from optionally substituted cycloalkylene, optionally substituted heterocyclylene, optionally substituted arylene, and optionally substituted heteroarylene; and
moiety A is attached to moiety L via a ring atom of R2';
moiety L has the following structure:

   -L1-L2-L3-L4-L5- formula (L)
wherein:
L1, L3, and L5 are independently absent or selected from -Rm-, -Rm-Rn-, -Rm-O-Rn-, -Rm-C(O)-Rn-, -Rm-C(O)O-Rn-, -Rm-OC(O)-Rn-, -Rm-NRx-Rn-, -Rm-C(O)NRx-Rn-, -Rm-NRxC(O)-Rn-, -Rm-S(O)-Rn-, -Rm-S(O)NRx-Rn-, -Rm-NRxS(O)-Rn-, -Rm-S(O)₂-Rn-, -Rm-S(O)₂NRx-Rn-, -Rm-NRxS(O)₂-Rn-, -Rm-NRxC(O)NRy-Rn-, -Rm-OC(O)NRx-Rn-, -Rm-NRxC(O)O-Rn-, 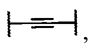 -(CH₂CH₂O)_{g}-, or - (OCH₂CH₂)_{g}-, wherein Rm and Rn are independently selected from a bond, optionally substituted alkylene, optionally substituted alkyleneoxy, optionally substituted cycloalkylene, or optionally substituted heterocyclylene; Rx and Ry are independently selected from hydrogen, deuterium, optionally substituted alkyl, optionally substituted cycloalkyl, or optionally substituted heterocyclyl; and g is an integer from 1 to 20;
L2 and L4 are independently absent or selected from optionally substituted cycloalkylene, optionally substituted heterocyclylene, optionally substituted arylene, or optionally substituted heteroarylene; and
moiety E has the following structure: 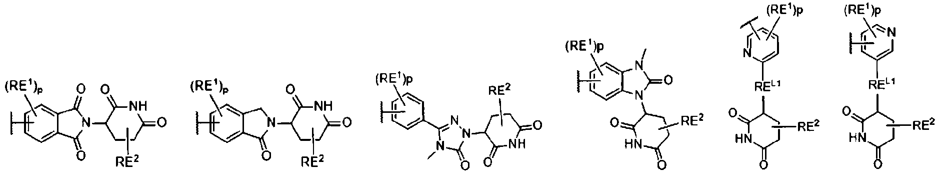 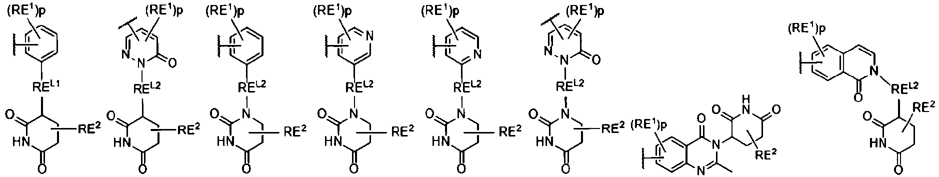 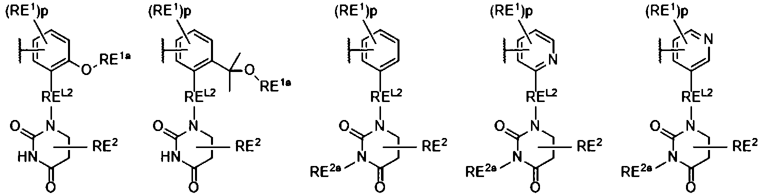
wherein RE¹ is selected from hydrogen, deuterium, halogen, hydroxyl, sulfhydryl, amino, nitro, cyano, optionally substituted alkyl, optionally substituted alkoxy, optionally substituted cycloalkyl, or optionally substituted heterocyclyl; RE² is selected from hydrogen, deuterium, or halogen; RE^{L1} is selected from a bond, -CH₂-, -NH-, -NCH₃-, -O-, -CONH-, or -CONCH₃-; RE^{L2} is selected from a bond or -CH₂-; and p is 1, 2, 3, 4, or 5.
RE^{1a} is selected from -COCH(CH₃)OH, -COCH₃, -CON(CH₃)₂, -CO(CH₂)₂CH(NH₂)COOH, or - COCH(NH₂)(CH₂)₂COOH, and RE^{2a} is selected from -CH₂OCOOC(CH₃)₃ or -CH₂OCOC(CH₃)₃.

### Moiety A

In some embodiments, the definitions of Q1-Q5, the ring where Q1-Q5 are present, ring P, R1, and R3 in formula (II) are the same as those in formula (I) (and formula (III)).

In some embodiments, moiety A has the following structure:

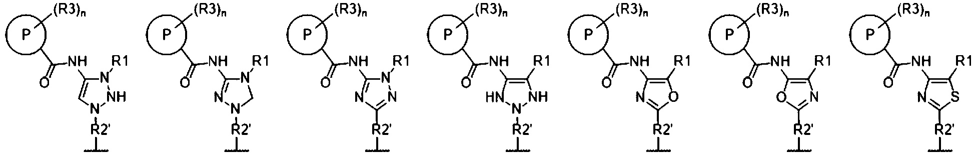

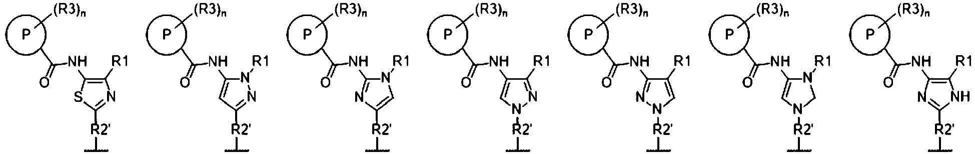

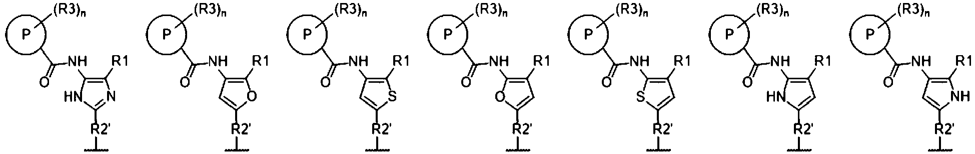

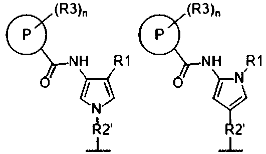

In some embodiments, R2' is selected from optionally substituted C₃₋₁₂ cycloalkylene, optionally substituted 3-12 membered heterocyclylene, optionally substituted 6-10 membered arylene, and optionally substituted 5-10 membered heteroarylene.

In some embodiments, R2' is selected from optionally substituted cyclopropylene, cyclobutylene, cyclopentylene, or cyclohexylene, bicyclo[1.1.1]pentylene (e.g., 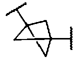 ), bicyclo[2.2.2]octylene (e.g., 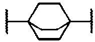 ), and cubanylene (e.g., 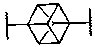 ).

In some embodiments, R2' is selected from optionally substituted 3-12 membered heterocyclylene comprising one to six (e.g., one, two, three, or four) heteroatoms independently selected from N, O, and S.

In some embodiments, R2' is selected from optionally substituted tetrahydropyrrolylene, piperidinylene, piperazinylene, azetidinylene, 1-oxa-8-azaspiro[4.5]decylene, and 7-azaspiro[3.5]nonylene.

In some embodiments, R2' is selected from optionally substituted phenylene.

In some embodiments, R2' is selected from optionally substituted 5-6 membered heteroarylene. In some embodiments, R2' is selected from optionally substituted pyridinylene, pyrazinylene, pyridazinylene, pyrimidinylene, or triazinylene.

In some embodiments, R2' is selected from cycloalkylene, heterocyclylene, arylene, or heteroarylene optionally substituted with one or more substituents selected from: deuterium; halogen; hydroxyl; sulfhydryl; amino; cyano; nitro; an aldehyde group; carboxyl; alkyl; alkoxy; and alkyl or alkoxy substituted with one or more substituents selected from deuterium, halogen, hydroxyl, sulfhydryl, amino, cyano, nitro, an aldehyde group, and/or carboxyl.

In some embodiments, R2' is selected from phenylene, pyridinylene, piperidinylene, cyclohexylene, bicyclo[1.1.1]pentylene, bicyclo[2.2.2]octylene, or cubanylene.

### Moiety L

In some embodiments, Rm and Rn are independently selected from a bond, optionally substituted C₁₋₂₀ alkylene (e.g., C₁₋₁₂ alkylene or C₁₋₆ alkylene), optionally substituted C₁₋₂₀ alkyleneoxy (e.g., C₁₋₁₂ alkyleneoxy or C₁₋₆ alkyleneoxy), optionally substituted C₃₋₆ cycloalkylene, or optionally substituted 3-6 membered heterocyclylene.

In some embodiments, Rx and Ry are independently selected from hydrogen, deuterium, optionally substituted C₁₋₂₀ alkyl (e.g., C₁₋₁₂ alkyl or C₁₋₆ alkyl), optionally substituted C₃₋₆ cycloalkyl, or optionally substituted 3-6 membered heterocyclyl.

In some embodiments, g is an integer from 1 to 20, or an integer from 1 to 16, or an integer from 1 to 12, or an integer from 1 to 8, or an integer from 1 to 6, or 1, 2, 3, 4, or 5.

In some embodiments, Rm and Rn are independently selected from a bond; alkylene; alkyleneoxy; cycloalkylene; heterocyclylene; and alkylene, alkyleneoxy, cycloalkylene, or heterocyclylene substituted with one or more substituents selected from: deuterium; halogen; hydroxyl; sulfhydryl; amino; nitro; cyano; alkyl; alkoxy; and alkyl or alkoxy substituted with one or more substituents selected from deuterium, halogen, hydroxyl, sulfhydryl, amino, cyano, nitro, an aldehyde group, and/or carboxyl.

In some embodiments, Rx and Ry are independently selected from hydrogen; deuterium; alkyl; cycloalkyl; heterocyclyl; and alkyl, cycloalkyl, or heterocyclyl substituted with one or more substituents selected from: deuterium; halogen; hydroxyl; sulfhydryl; amino; nitro; cyano; alkyl; alkoxy; and alkyl or alkoxy substituted with one or more substituents selected from deuterium, halogen, hydroxyl, sulfhydryl, amino, cyano, nitro, an aldehyde group, and/or carboxyl.

In some embodiments, Rm and Rn are independently selected from a bond, methylene, ethylene, propylene, butylene, cyclopropylene, and C₁₋₄ fluoroalkylene.

In some embodiments, Rx and Ry are independently selected from hydrogen, methyl, or cyclopropyl.

In some embodiments, L1, L3, and L5 are independently selected from: 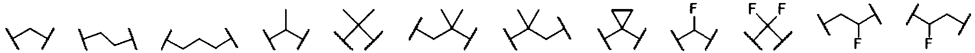 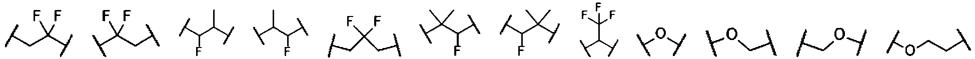 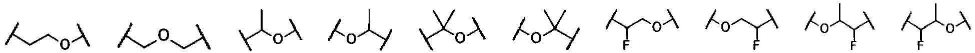 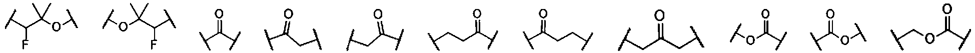 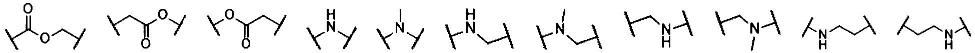 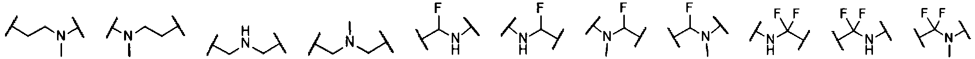 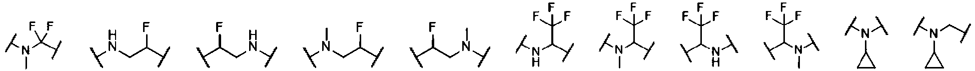 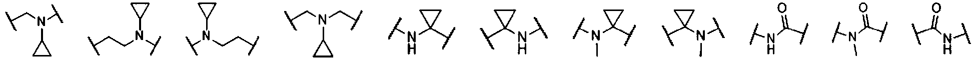 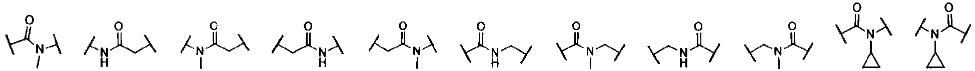 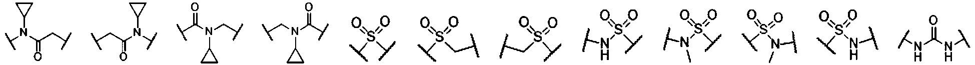 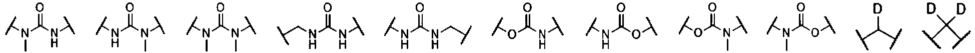 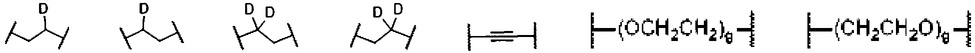 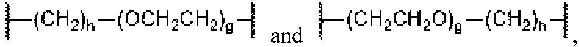 wherein h is 1, 2, 3, 4, or 5.

In some embodiments, L2 and L4 are independently absent or selected from optionally substituted C₃₋₁₂ cycloalkylene, optionally substituted 3-12 membered heterocyclylene, optionally substituted 6-10 membered arylene, or optionally substituted 5-10 membered heteroarylene.

In some embodiments, L2 and L4 are independently selected from cycloalkylene; heterocyclylene; arylene; heteroarylene; and cycloalkylene, heterocyclylene, arylene, or heteroarylene substituted with one or more substituents selected from: deuterium; halogen; hydroxyl; sulfhydryl; amino; nitro; cyano; =O; =S; alkyl; alkoxy; and alkyl or alkoxy substituted with one or more substituents selected from deuterium, halogen, hydroxyl, sulfhydryl, amino, cyano, nitro, an aldehyde group, and/or carboxyl.

In some embodiments, L2 and L4 are independently selected from cycloalkylene or halocycloalkylene (e.g., fluorocycloalkylene).

In some embodiments, L2 and L4 are independently selected from: 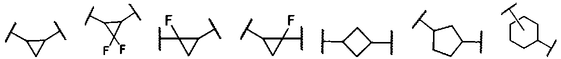 (e.g.,

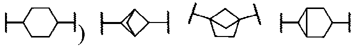

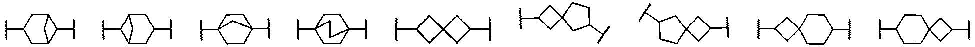

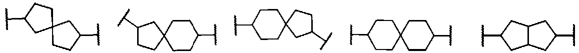

In some embodiments, L2 and L4 are independently selected from 3-12 membered heterocyclylene that comprises one to six (preferably one, two, three, or four) heteroatoms independently selected from N, O, and S and is optionally substituted with one or more substituents selected from halogen (e.g., fluorine), =O, and/or alkyl (e.g., methyl).

In some embodiments, L2 and L4 are independently selected from: 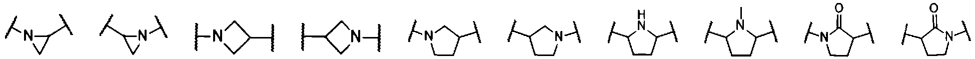 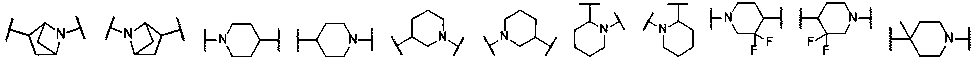 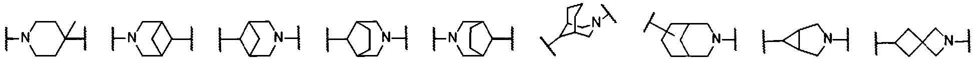 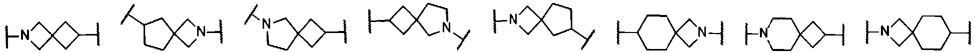 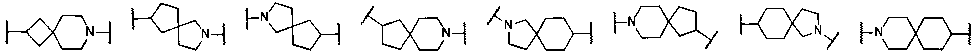 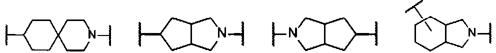 (e.g., 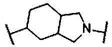 ) 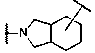 (e.g., 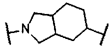 ) 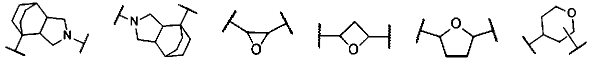 (e.g., 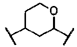 ) 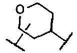 (e.g., 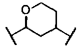 )

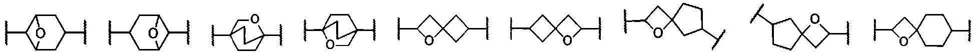

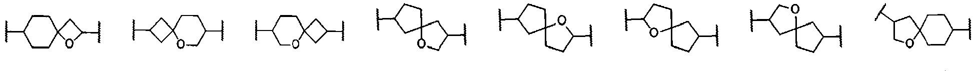

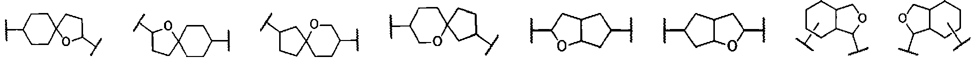

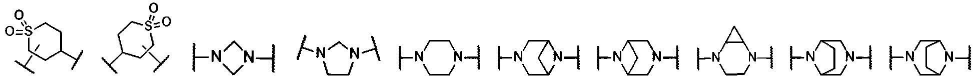

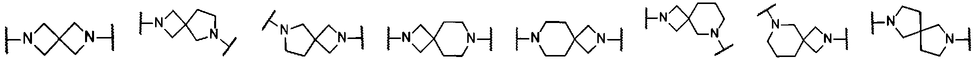

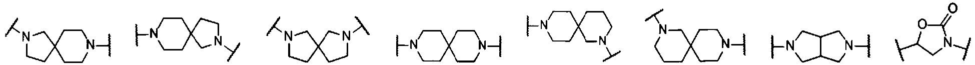

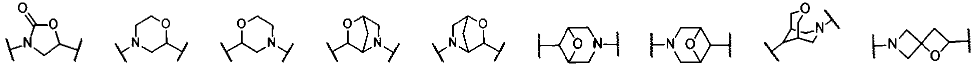

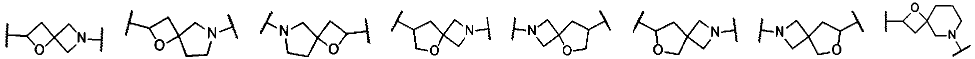

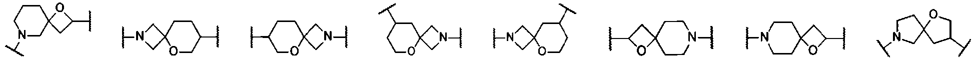

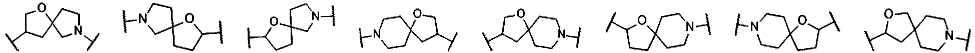

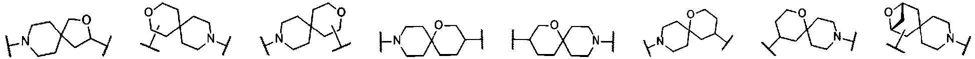

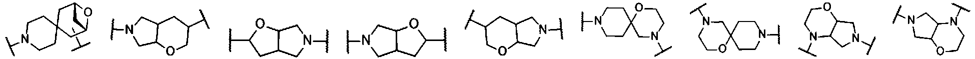

In some embodiments, L2 and L4 are independently selected from phenylene optionally substituted with one or more substituents selected from halogen (e.g., fluorine) and/or alkyl (e.g., methyl).

In some embodiments, L2 and L4 are independently selected from

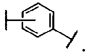

In some embodiments, L2 and L4 are independently selected from 5-6 membered heteroarylene that comprises one, two, three, or four heteroatoms independently selected from N, O, and S and is optionally substituted with one or more substituents selected from halogen (e.g., fluorine) and/or alkyl (e.g., methyl). In some embodiments, L2 and L4 are independently selected from pyridinylene, pyrazinylene, pyridazinylene, pyrimidinylene, triazinylene, pyrrolylene, imidazolylene, pyrazolylene, triazolylene, tetrazolylene, oxazolylene, thiazolylene, oxadiazolylene, or thiadiazolylene optionally substituted with one or more substituents selected from halogen (e.g., fluorine) and/or alkyl (e.g., methyl).

In some embodiments, L2 and L4 are independently selected from:

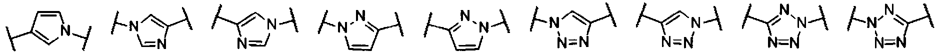

In some embodiments, a combination of L2, L3, and L4 (with L1 and/or L5 being present or absent) in moiety L or moiety L is selected from:

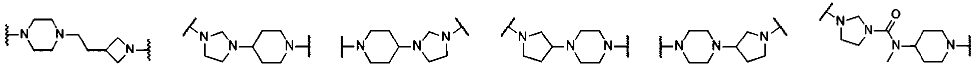

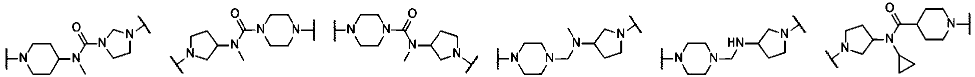

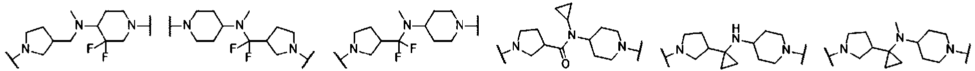

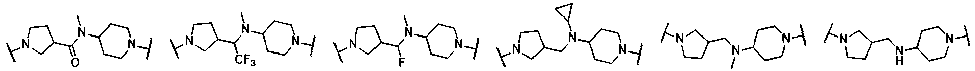

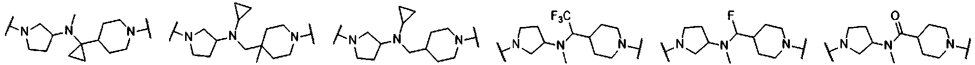

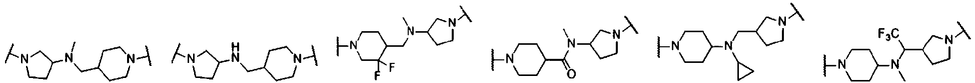

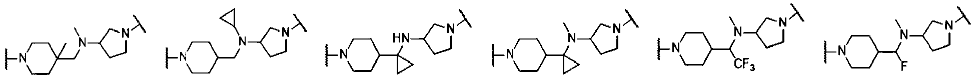

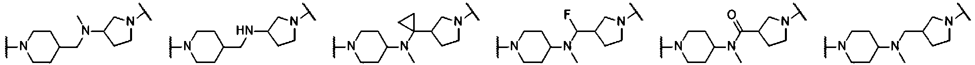

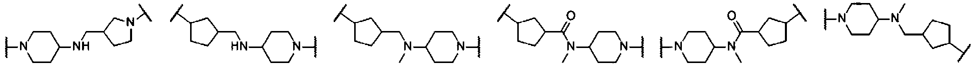

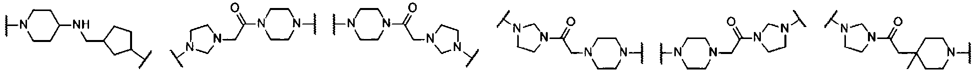

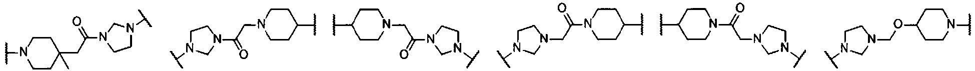

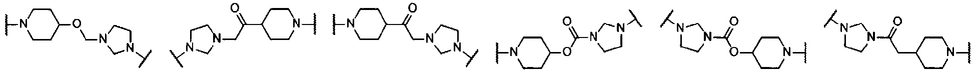

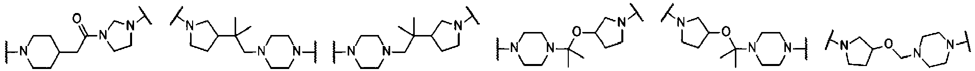

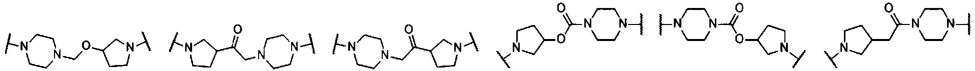

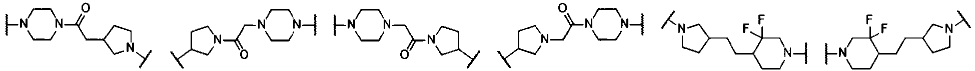

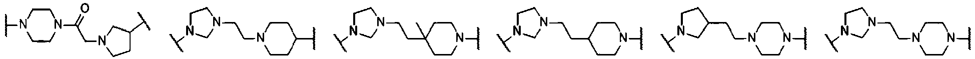

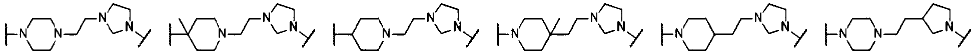

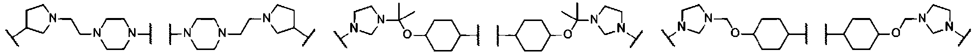

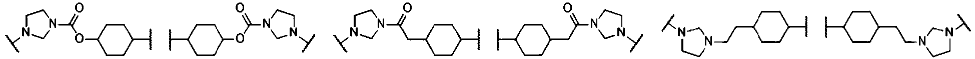

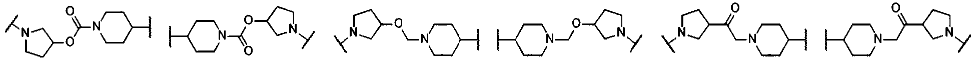

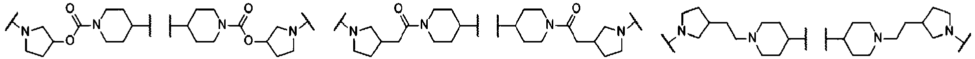

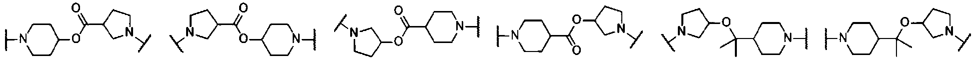

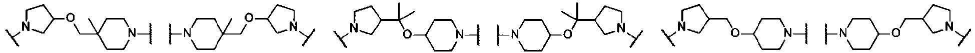

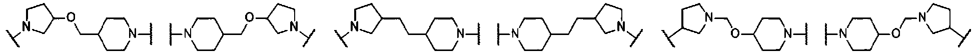

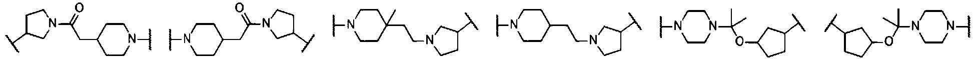

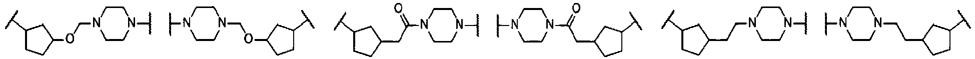

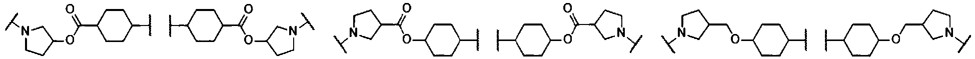

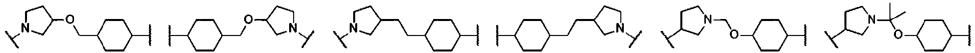

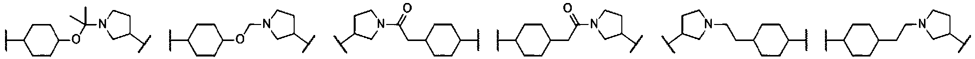

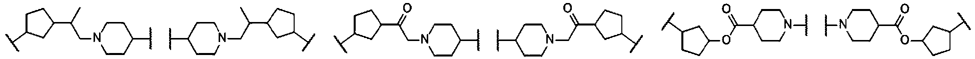

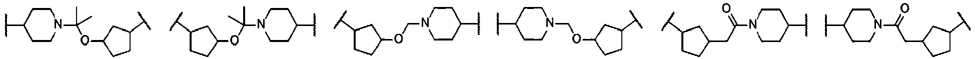

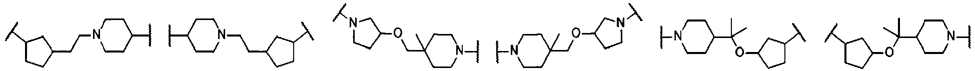

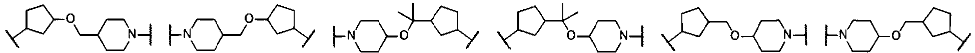

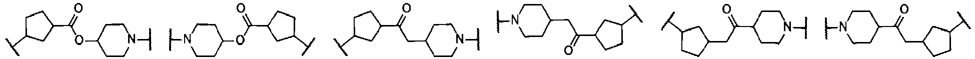

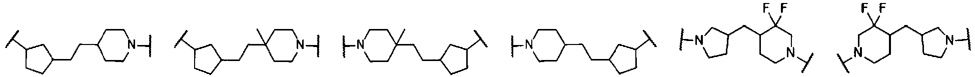

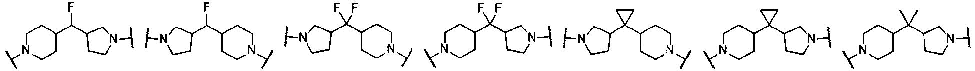

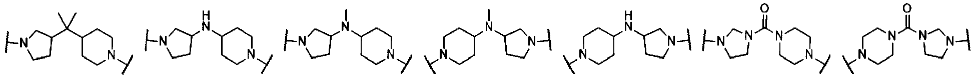

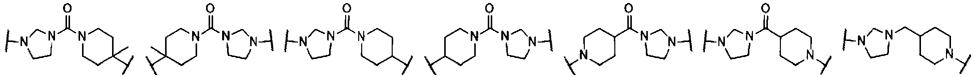

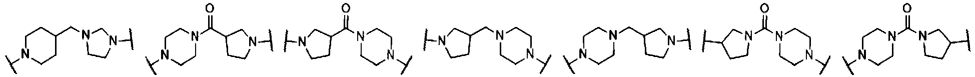

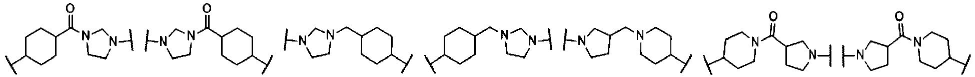

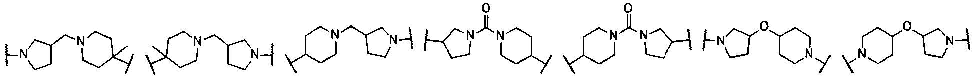

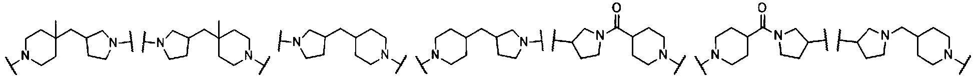

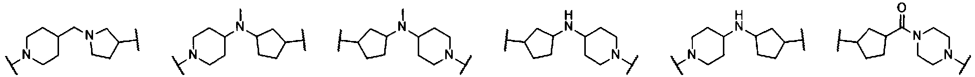

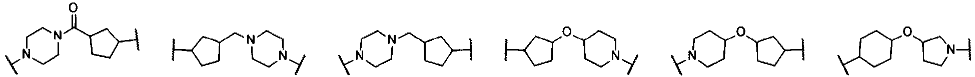

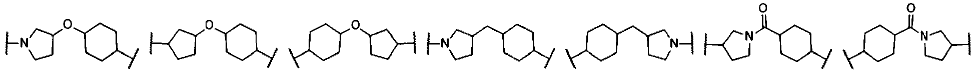

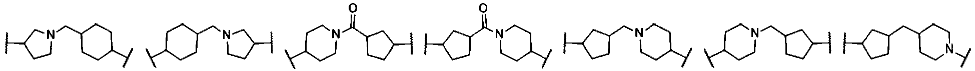

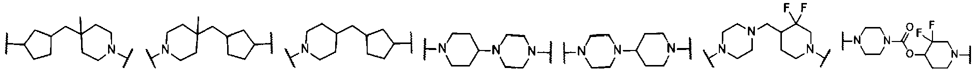

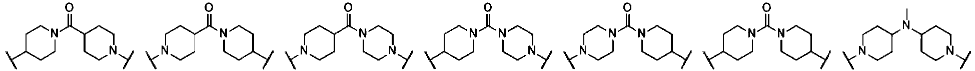

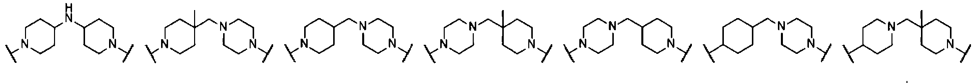

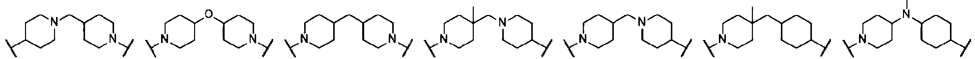

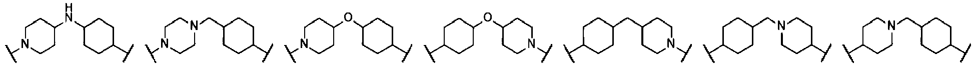

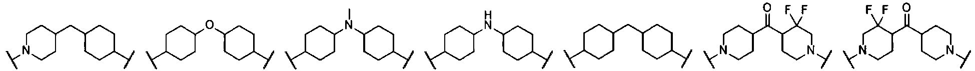

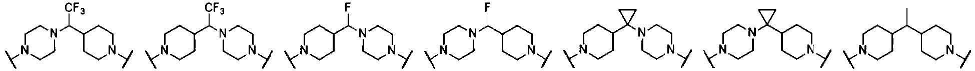

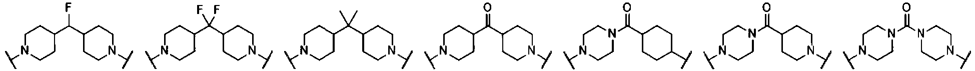

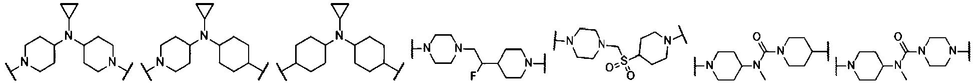

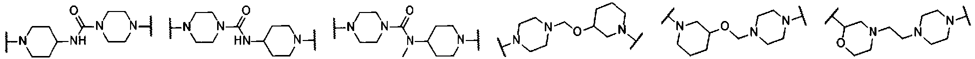

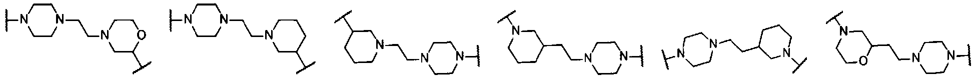

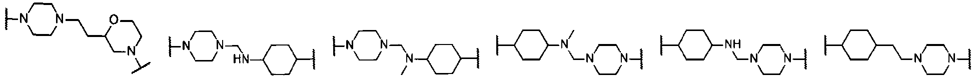

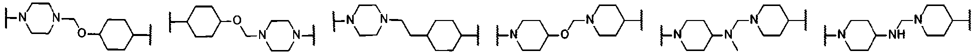

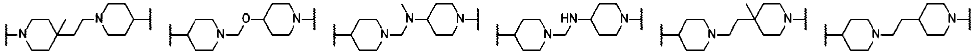

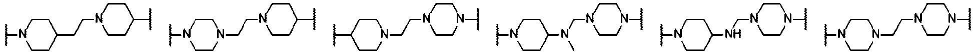

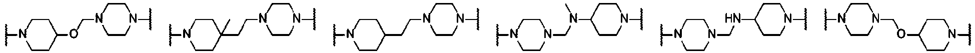

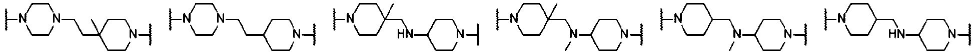

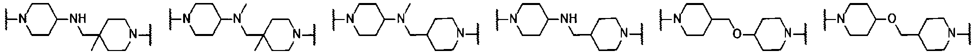

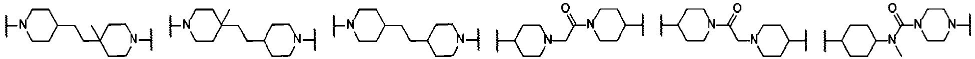

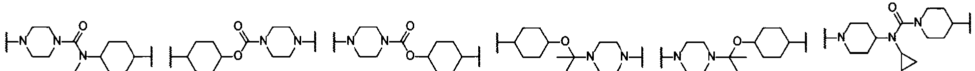

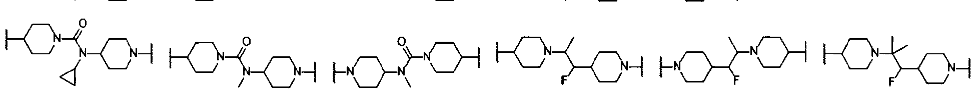

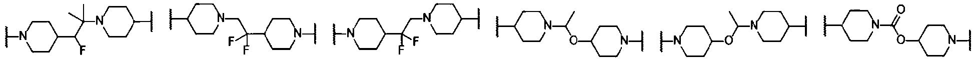

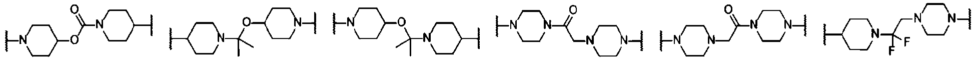

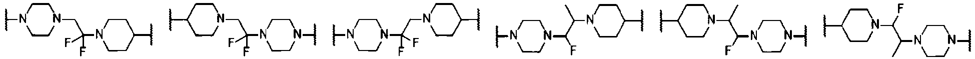

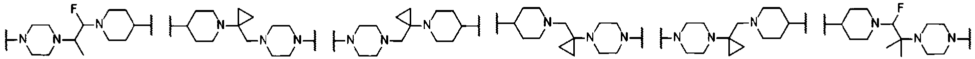

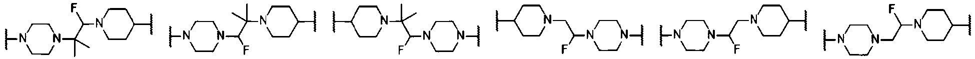

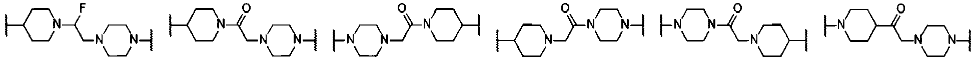

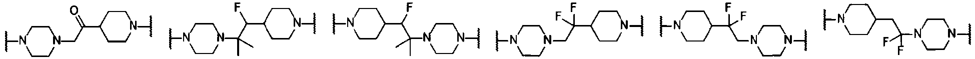

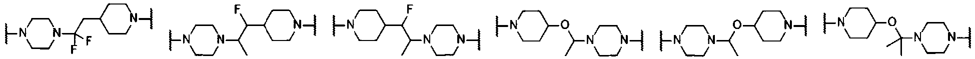

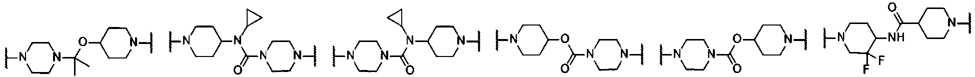

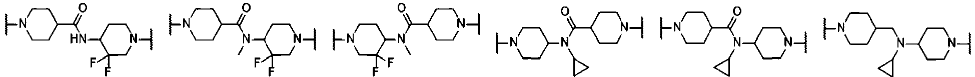

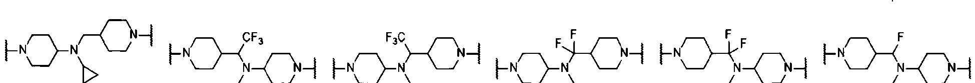

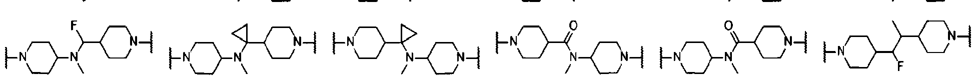

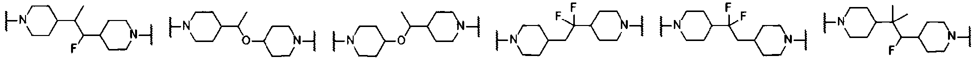

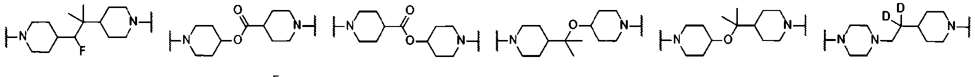

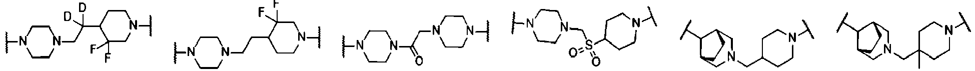

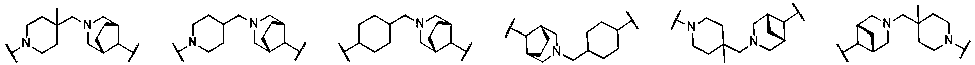

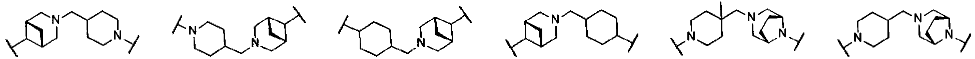

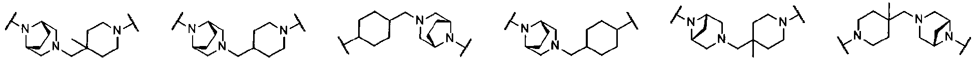

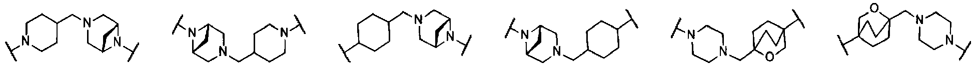

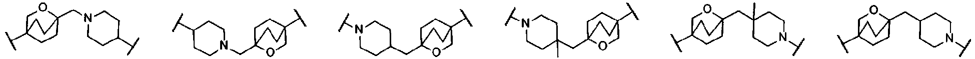

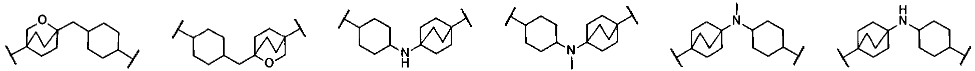

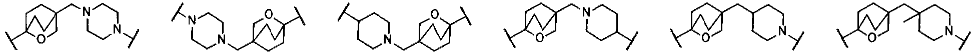

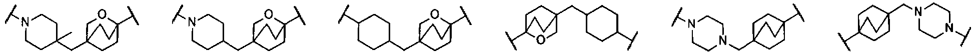

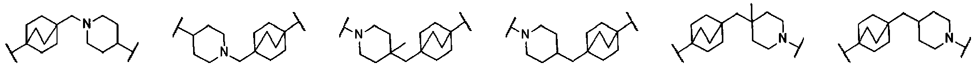

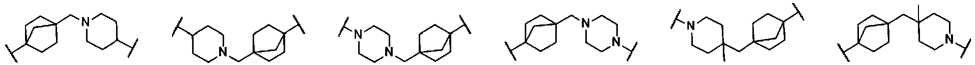

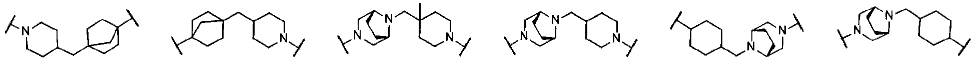

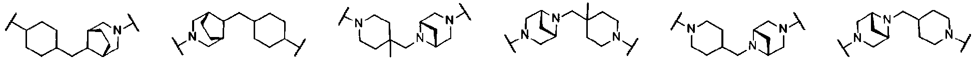

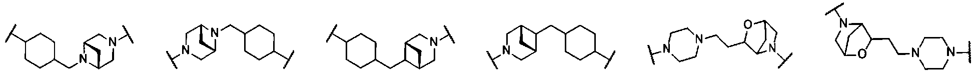

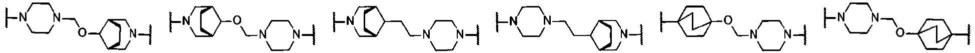

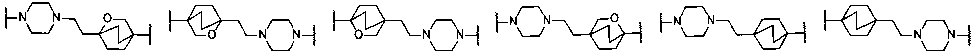

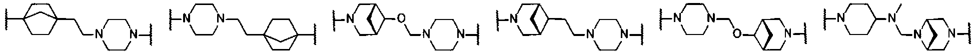

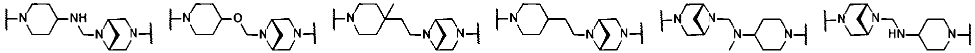

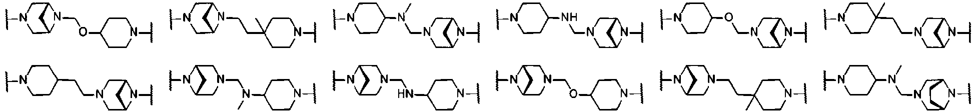

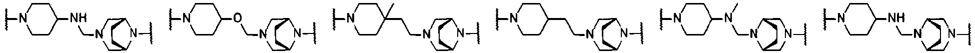

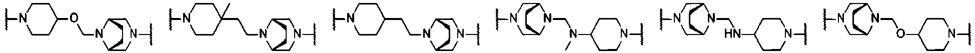

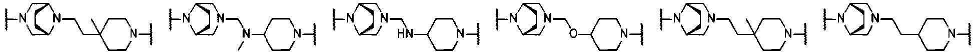

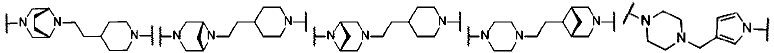

In some embodiments, moiety L is selected from:

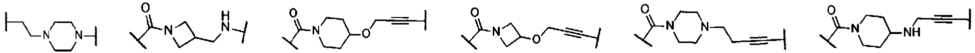

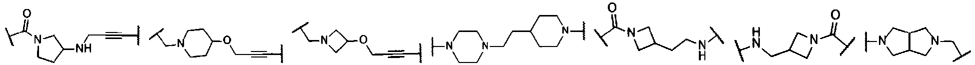

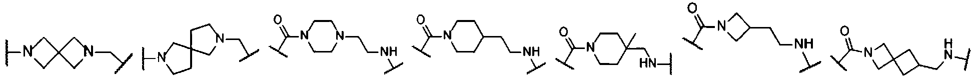

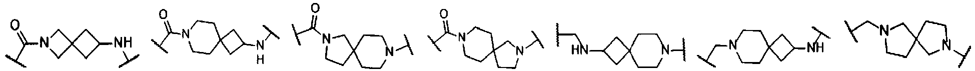

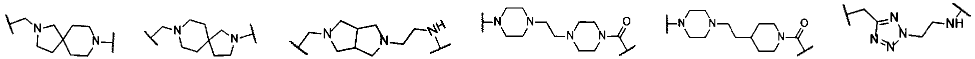

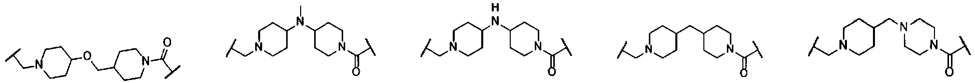

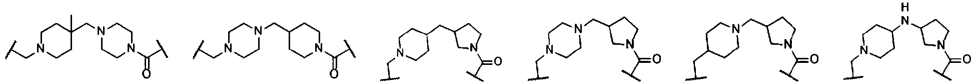

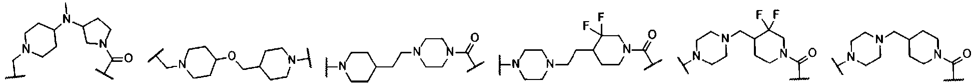

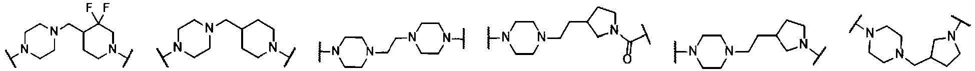

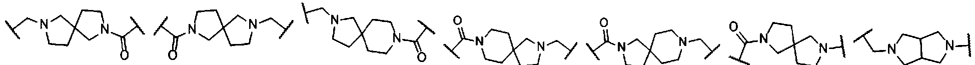

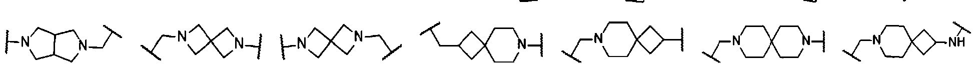

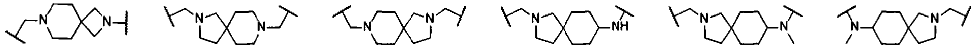

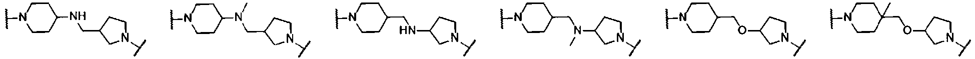

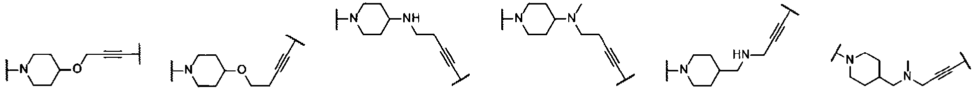

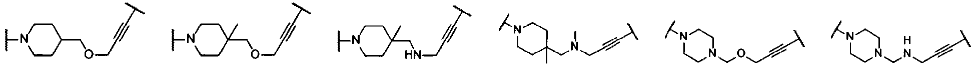

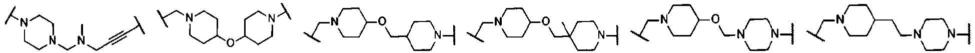

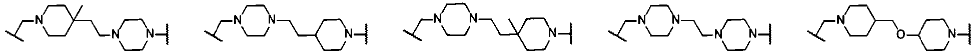

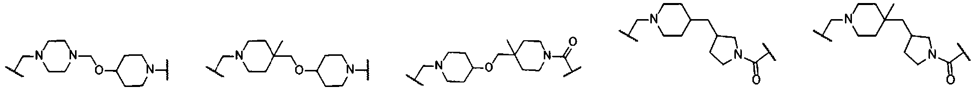

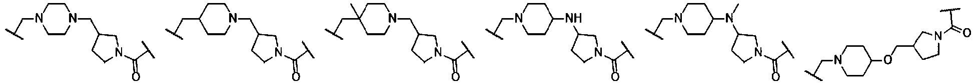

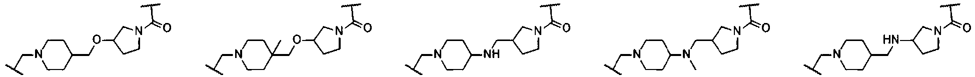

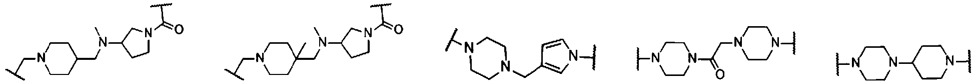

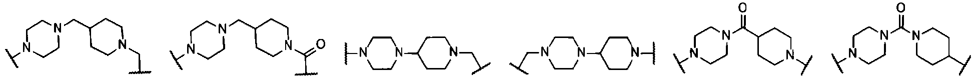

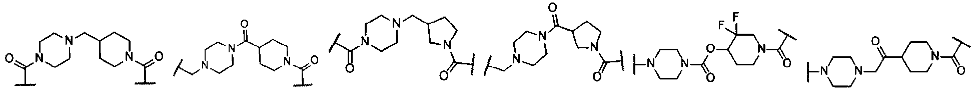

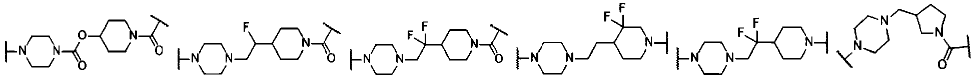

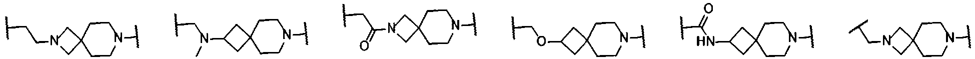

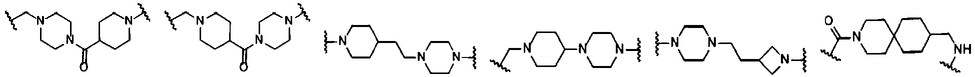

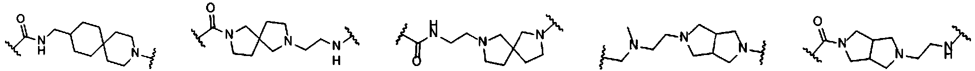

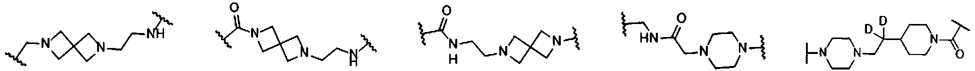

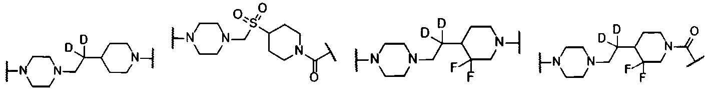

In general, for moiety L and the groups in moiety L, the point of attachment on the left is attached to the remaining molecular moiety from moiety A or in the direction of moiety A, and the point of attachment on the right is attached to the remaining molecular moiety from moiety E or in the direction of moiety E.

### Moiety E

In some embodiments, moiety E has the following structure:

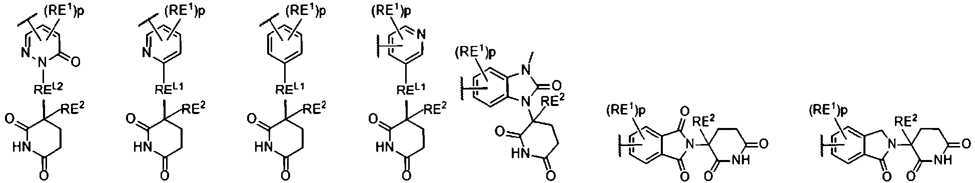

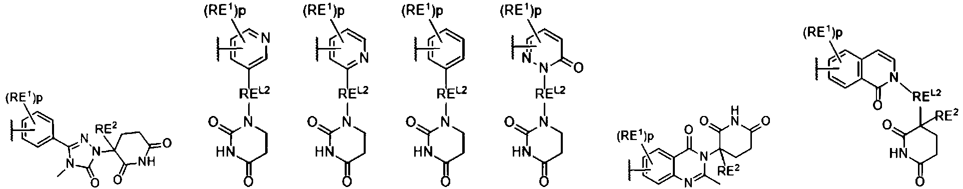

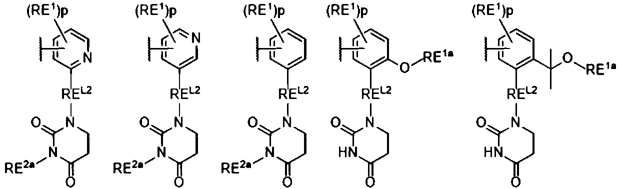

In some embodiments, RE¹ is selected from hydrogen, deuterium, halogen, hydroxyl, sulfhydryl, amino, nitro, cyano, optionally substituted C₁₋₂₀ alkyl (e.g., C₁₋₁₂ alkyl or C₁₋₆ alkyl), optionally substituted C₁₋₂₀ alkoxy (e.g., C₁₋₁₂ alkoxy or C₁₋₆ alkoxy), optionally substituted C₃₋₆ cycloalkyl, or optionally substituted 3-6 membered heterocyclyl; RE² is selected from hydrogen, deuterium, or halogen; RE^{L1} is selected from a bond, -CH₂-, -NH-, -NCH₃-, -O-, -CONH-, or -CONCH₃-; RE^{L2} is selected from a bond or -CH₂-; and p is 1, 2, 3, 4, or 5.

In some embodiments, RE¹ is selected from hydrogen; deuterium; halogen; hydroxyl; sulfhydryl; amino; nitro; cyano; alkyl; alkoxy; cycloalkyl; heterocyclyl; and alkyl, alkoxy, cycloalkyl, or heterocyclyl substituted with one or more substituents selected from deuterium, halogen, hydroxyl, sulfhydryl, amino, nitro, cyano, alkyl, haloalkyl, alkoxy, and/or haloalkoxy.

In some embodiments, RE¹ is selected from hydrogen, deuterium, fluorine, chlorine, hydroxyl, methyl, methoxy, ethoxy, hydroxymethyl, hydroxypropyl (e.g., hydroxyisopropyl), trifluoromethyl, or trifluoromethoxy.

In some embodiments, RE² is selected from hydrogen, deuterium, or fluorine.

In some embodiments, RE^{1a} is selected from -COCH(CH₃)OH, -COCH₃, -CON(CH₃)₂, - CO(CH₂)₂CH(NH₂)COOH, or -COCH(NH₂)(CH₂)₂COOH.

In some embodiments, RE^{2a} is selected from -CH₂OCOOC(CH₃)₃ or -CH₂OCOC(CH₃)₃.

In some embodiments, moiety E is selected from the following structures: 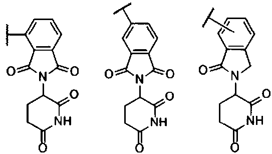 (e.g. 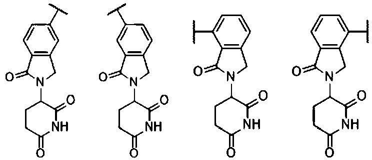 ) 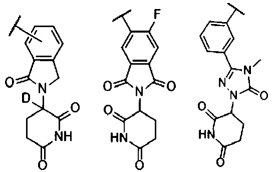 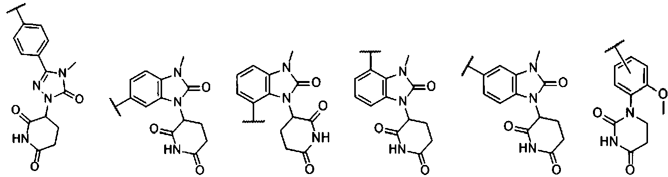 (e.g. 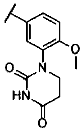 ) 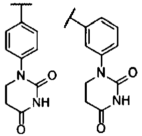 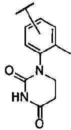 (e.g. 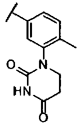 ) 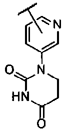 (e.g. 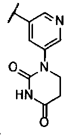 ) 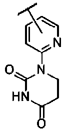 (e.g. 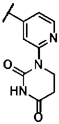 ) 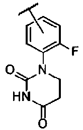 (e.g. 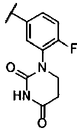 ) 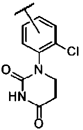 (e.g. 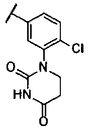 ) 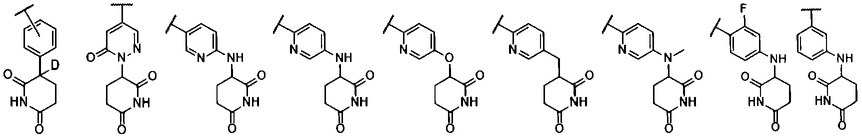 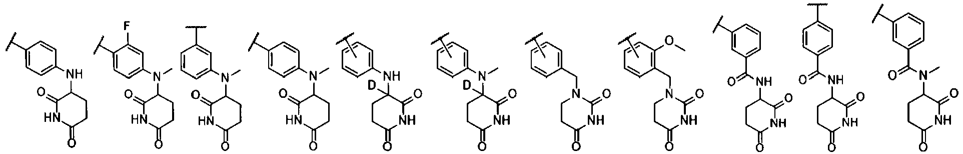 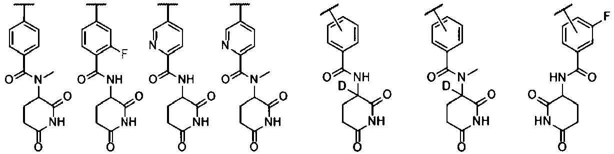 (e.g. 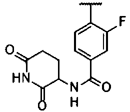 ) 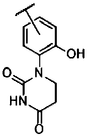 (e.g. 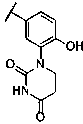 ) 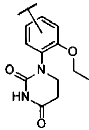 (e.g. 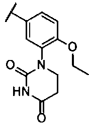 ) 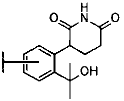 (e.g. 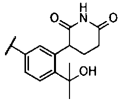 ) 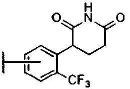 (e.g. 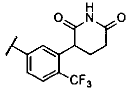 ) 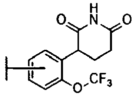 (e.g. 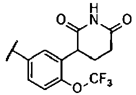 ) 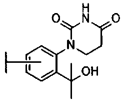 (e.g. 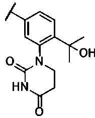 ) 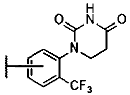 (e.g. 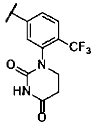 ) 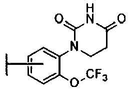 (e.g. 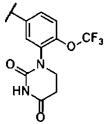 ) 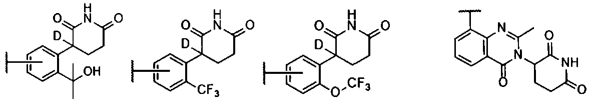 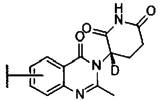 (e.g. 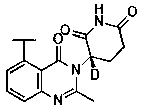 ) 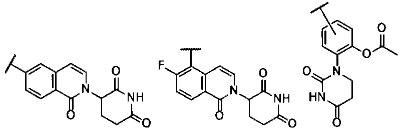 (e.g. 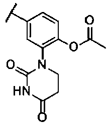 ) 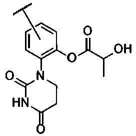 (e.g. 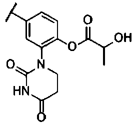 ) 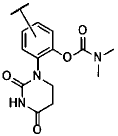 (e.g. 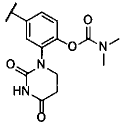 ) 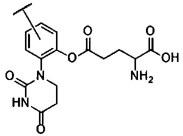 (e.g. 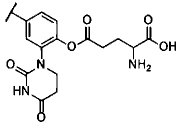 ) 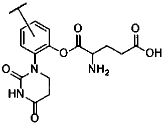 (e.g. 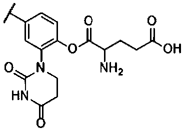 ) 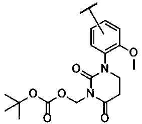 (e.g. 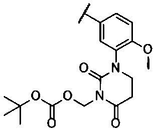 )

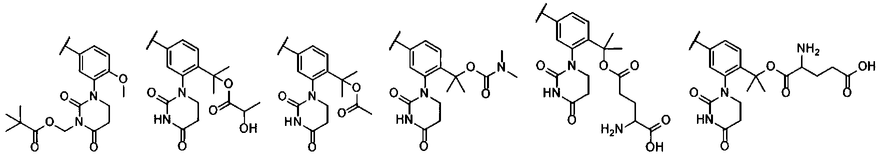

In some embodiments, moiety A of the compound of formula (II) has the following structure: 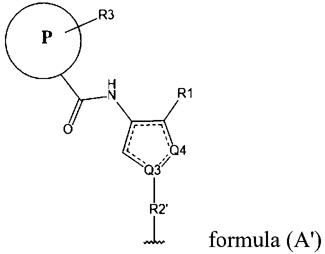 wherein the ring structure where Q3 and Q4 are present is selected from the following structures: 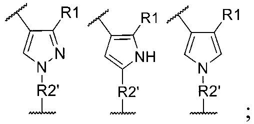 wherein the definitions of ring P, R1, R2', and R3 in formula (II) are the same as those described above.

In some embodiments, the present disclosure relates to a compound of formula (II) or a pharmaceutically acceptable salt, ester, solvate, or stereoisomer thereof:

A-L-E (II)
wherein moiety A has the following structure: 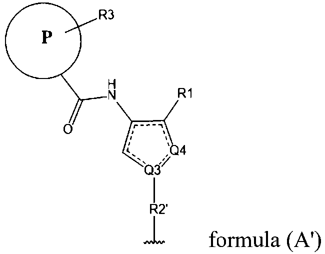
wherein the ring structure where Q3 and Q4 are present is selected from the following structures: 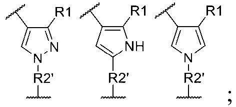
ring P is pyridinyl, pyrazolopyrimidinyl, or pyrrolopyridazinyl;
R1 is selected from -OR^{1a}, -COR^{1a}, -CONR^{1a}R^{1b}, -S(O)(NR^{1a})R^{1b}, -SO₂NR^{1a}R^{1b}, and optionally substituted alkyl, wherein R^{1a} and R^{1b} are independently selected from hydrogen, deuterium, optionally substituted alkyl, and optionally substituted cycloalkyl;
R2' is selected from optionally substituted cycloalkylene, optionally substituted heterocyclylene, optionally substituted phenylene, and pyridinylene;
R3 is selected from cyano, optionally substituted alkyl, and optionally substituted heteroaryl;
and the definitions of L and E are the same as those described above (e.g., formula (II)).

In some embodiments, the definitions of ring P, R1, and R3 in formula (A') are the same as those described above (e.g., formula (III)).

In some embodiments, R2' is selected from cycloalkylene, heterocyclylene, phenylene, and pyridinylene optionally substituted with one or more substituents selected from: deuterium, halogen, hydroxyl, sulfhydryl, amino, cyano, nitro, an aldehyde group, carboxyl, and alkyl.

In some embodiments, R2' is selected from optionally substituted cyclobutylene, cyclopentylene, cyclohexylene, bicyclo[1.1.1]pentylene, or bicyclo[2.2.2]octylene; optionally substituted piperidinylene, azetidinylene, azacyclopentylene, 1-oxa-8-azaspiro[4.5]decylene, or 7-azaspiro[3.5]nonylene; and optionally substituted phenylene or pyridinylene.

In some embodiments, L1, L3, and L5 are independently absent or selected from -Rm-, -Rm-O-Rn-, -Rm-C(O)-Rn-, -Rm-C(O)O-Rn-, -Rm-OC(O)-Rn-, -Rm-NRx-Rn-, -Rm-C(O)NRx-Rn-, -Rm-NRxC(O)-Rn-, or 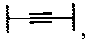 wherein Rm and Rn are independently selected from a bond or optionally substituted alkylene, and Rx is independently selected from hydrogen, deuterium, or optionally substituted alkyl.

In some embodiments, Rm and Rn are independently selected from a bond or alkylene (e.g., C₁₋₄ alkylene, such as methylene or ethylene) optionally substituted with halogen (e.g., fluorine).

In some embodiments, Rx is independently selected from hydrogen, deuterium, or alkyl (e.g., C₁₋₄ alkylene, such as methylene and ethylene).

In some embodiments, L2 is selected from optionally substituted heterocyclylene.

In some embodiments, L2 and L4 are independently selected from 3-12 membered heterocyclylene that comprises one or two nitrogen heteroatoms and is optionally substituted with one or more substituents selected from halogen (e.g., fluorine) and/or alkyl (e.g., methyl).

In some embodiments, L2 and L4 are independently selected from:

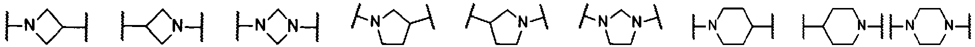

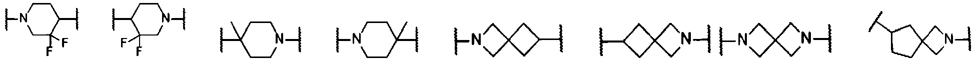

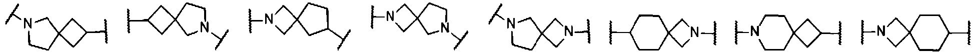

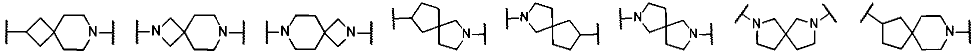

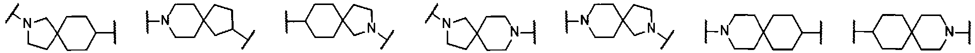

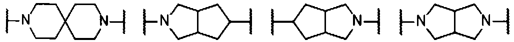

In some embodiments, L1 is absent or selected from -Rm-, -Rm-O-Rn-, -Rm-C(O)-Rn-, -Rm-C(O)O-Rn-, -Rm-OC(O)-Rn-, -Rm-NRx-Rn-, -Rm-C(O)NRx-Rn-, or -Rm-NRxC(O)-Rn-, wherein Rm and Rn are independently selected from a bond or alkylene (e.g., methylene or ethylene), and Rx is independently selected from hydrogen, deuterium, or alkyl (e.g., methyl).

In some embodiments, L1 is absent or selected from -CH₂-, -CH₂CH₂-, -CH₂O-, -C(O)-, -CH₂C(O)-, -CH₂NH-, -NHCH₂-, -CH₂N(CH₃)-, and -C(O)NH-.

In some embodiments, L2 and L4 are independently selected from:

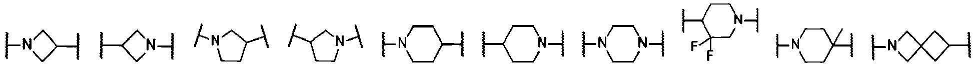

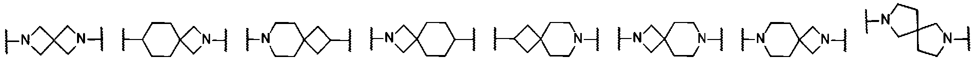

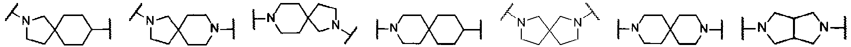

In some embodiments, L3 is absent or selected from -Rm-, -Rm-O-Rn-, -Rm-C(O)-Rn-, -Rm-C(O)O-Rn-, -Rm-OC(O)-Rn-, -Rm-NRx-Rn-, -Rm-C(O)NRx-Rn-, or -Rm-NRxC(O)-Rn-, wherein Rm and Rn are independently selected from a bond or alkylene (e.g., methylene or ethylene), and Rx is independently selected from hydrogen, deuterium, or alkyl (e.g., methyl).

In some embodiments, L3 is absent or selected from -CH₂-, -CH₂CH₂-, -OCH₂-, -C(O)-, -C(O)CH₂-, -C(O)O-, -NH-, -NHCH₂-, and -N(CH₃)-.

In some embodiments, L5 is absent or selected from -Rm-, -Rm-C(O)-Rn-, -Rm-NRx-Rn-, or 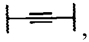 wherein Rm and Rn are independently selected from a bond or alkylene (e.g., methylene or ethylene), and Rx is independently selected from hydrogen, deuterium, or alkyl (e.g., methyl).

In some embodiments, L5 is absent or selected from -CH₂-, -C(O)-, -NH-, or

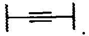

In some embodiments, L1 is absent or selected from -CH₂-, -CH₂CH₂-, -C(O)-, -NHCH₂-, - CH₂N(CH₃)-, -CH₂O-, -C(O)NH-, -CH₂C(O)-, and -CH₂NH-;
L2 is absent, and L4 is selected from the following groups; or L2 is selected from the following groups, and L4 is absent: 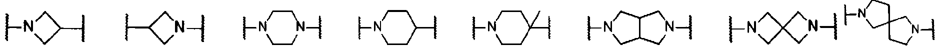 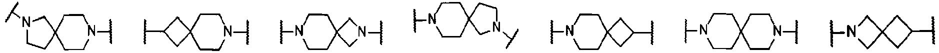 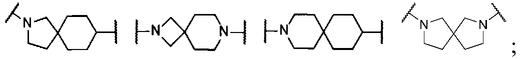
L3 is absent or selected from -CH₂-, -CH₂CH₂-, or -C(O)-;
L5 is absent or selected from -CH₂-, -NH-, and -C(O)-.

In some embodiments, L1 is selected from -CH₂- and -C(O)-;
L2 is selected from 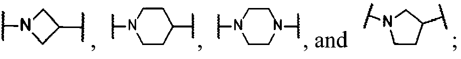
L3 is absent or selected from -OCH₂-, -CH₂CH₂-, and -NHCH₂-;
L4 is absent;
L5 is selected from

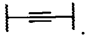

In some embodiments, L1 is absent or selected from -CH₂-, -C(O)-, and -CH₂O-;
L2 is selected from 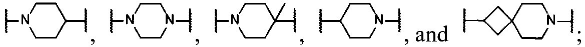
L3 is absent or selected from -CH₂-, -CH₂CH₂-, -NHCH₂-, -C(O)O-, -OCH₂-, -NH-, -N(CH₃)-, - C(O)-, and -C(O)CH₂-;
L4 is selected from 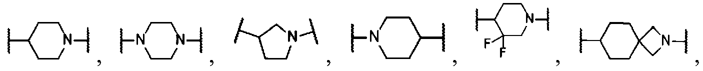 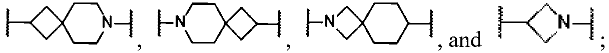
L5 is absent or selected from -C(O)- and -CH₂-.

In some embodiments, L1 is selected from absent;
L2 is selected from 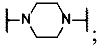
L3 is absent or selected from -CH₂-, -CH₂CH₂-, and -C(O)-;
L4 is selected from 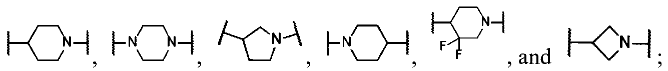
L5 is absent or selected from -C(O)-.

In some embodiments, L1 is selected from -CH₂-, -CH₂CH₂-, -C(O)-, -C(O)NH-, -CH₂NH-, - CH₂C(O)-, -CH₂O-, and -CH₂N(CH₃)-;
L2 is selected from 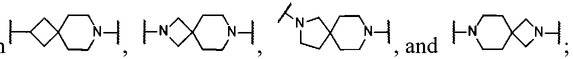
L3 is absent or selected from -CH₂- and -C(O)-;
L4 is absent or selected from 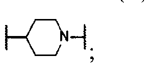
L5 is absent.

In a specific embodiment of L, L is selected from:

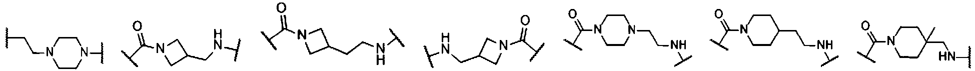

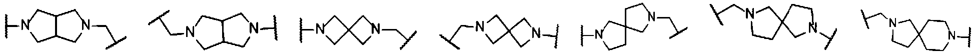

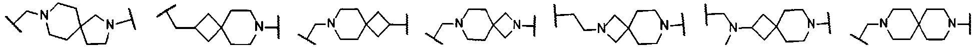

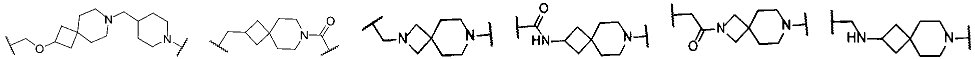

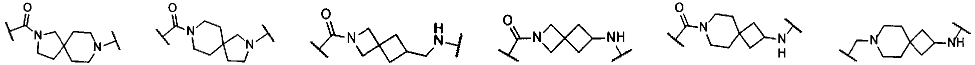

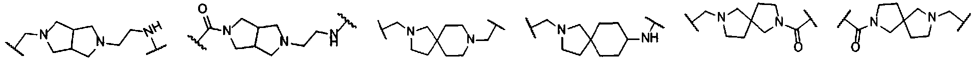

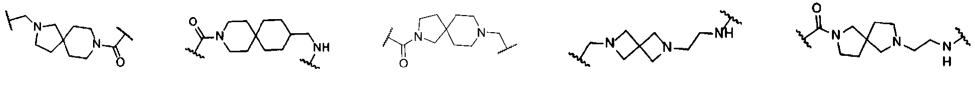

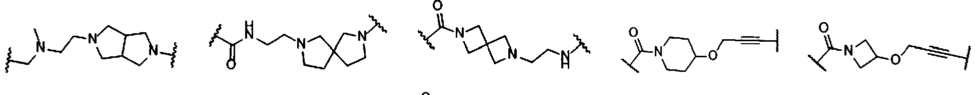

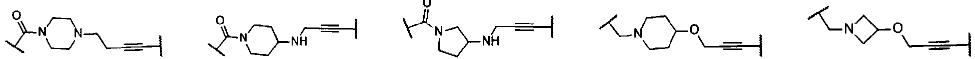

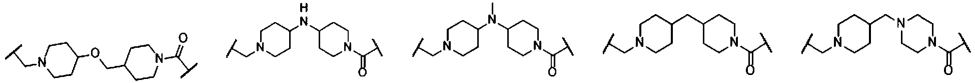

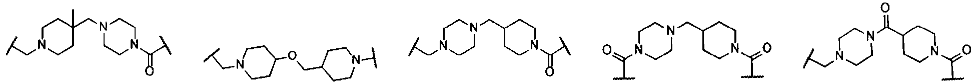

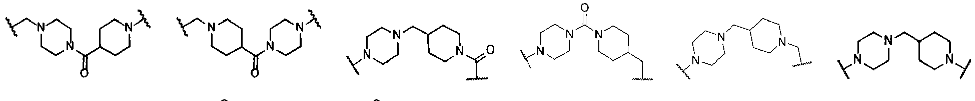

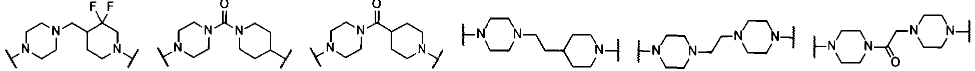

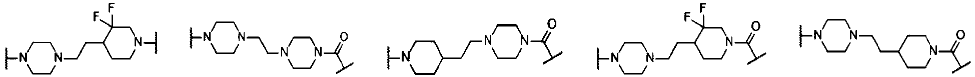

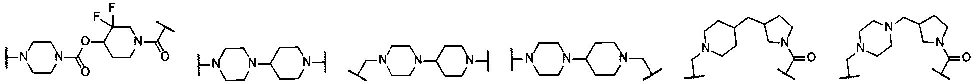

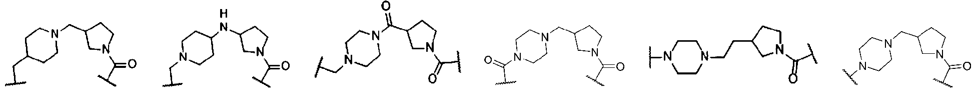

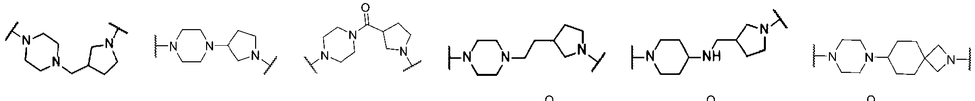

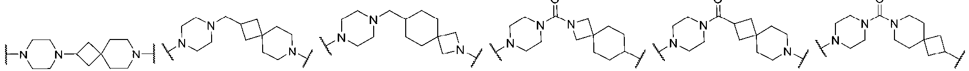

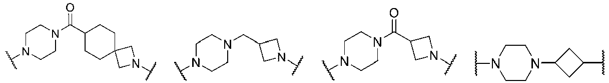

In some embodiments, moiety E has the following structure:

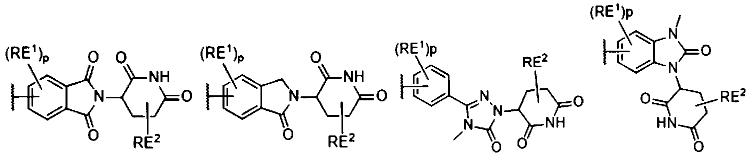

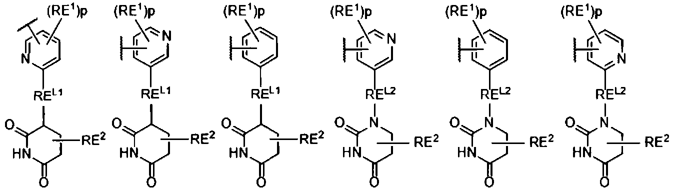

In some embodiments, RE¹ is selected from hydrogen, deuterium, halogen, hydroxyl, sulfhydryl, amino, nitro, cyano, alkyl, and alkoxy; RE² is selected from hydrogen, deuterium, or halogen; RE^{L1} is selected from a bond, -CH₂-, -NH-, -NCH₃-, -O-, -CONH-, or -CONCH₃-; RE^{L2} is selected from a bond, - CH₂-, or cyclopropylene; and p is 1 or 2.

In some embodiments, RE¹ is selected from hydrogen, deuterium, halogen (e.g., fluorine), and methoxy.

In some embodiments, RE² is selected from hydrogen or deuterium.

In some embodiments, moiety E has the following structure: 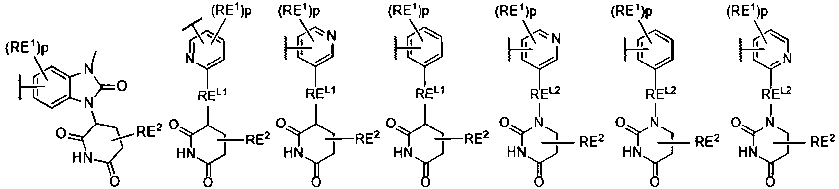 wherein RE¹ is selected from hydrogen, deuterium, halogen (e.g., fluorine), and methoxy; RE² is selected from hydrogen, deuterium, or halogen; RE^{L1} is selected from a bond, -CH₂-, -NH-, -NCH₃-, -O-, - CONH-, or -CONCH₃-; RE^{L2} is selected from a bond and -CH₂-; and p is 1 or 2.

In some embodiments, moiety E has the following structure:

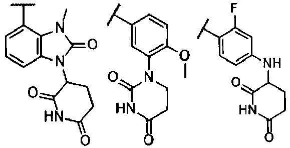

In a specific embodiment of E, E is selected from:

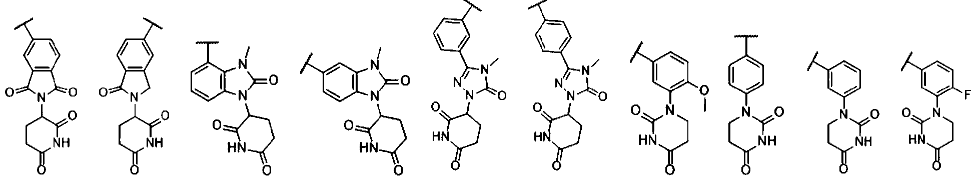

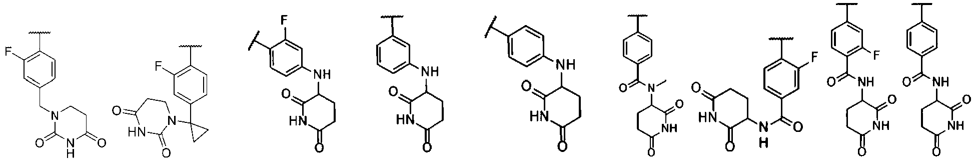

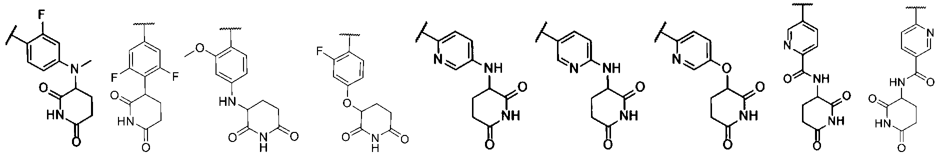

In some embodiments, the compound of formula (II) has the following structure: A-L-E (II), wherein:
moiety A has a structure of formula (A'), wherein:
the ring structure where Q3 and Q4 are present is selected from: 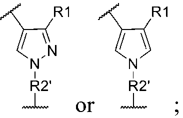
ring P is pyridinyl;
R1 is selected from -CONH₂, hydroxyisopropyl, difluoromethyl, and -CONH-(cyclopropyl);
R2' is selected from phenylene, cyclohexylene, and bicyclo[1.1.1]pentylene;
R3 is selected from -CF₃.
moiety L has the following structure: -L1-L2-L3-L4-L5- formula (L)
wherein L1 is absent or selected from -CH₂-, -C(O)-, and -CH₂O-;
L2 is selected from 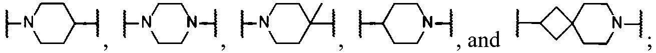
L3 is absent or selected from -CH₂-, -CH₂CH₂-, -NHCH₂-, -C(O)O-, -OCH₂-, -NH-, -N(CH₃)-, - C(O)-, and -C(O)CH₂-;
L4 is selected from 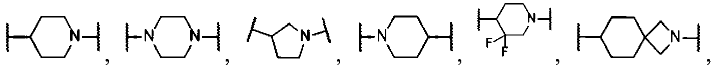 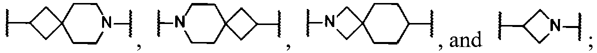
L5 is absent or selected from -C(O)- and -CH₂-; or
specifically, L1 is selected from absent; L2 is selected from 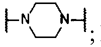 L3 is absent or selected from -CH₂-, -CH₂CH₂-, and -C(O)-; L4 is selected from 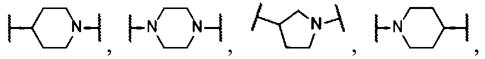 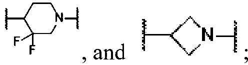 and L5 is absent or selected from -C(O)-.
moiety E has the following structure: 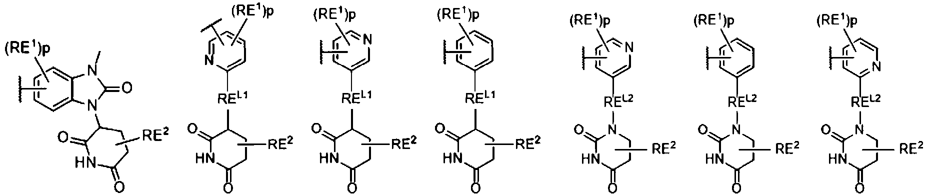
wherein RE¹ is selected from hydrogen, deuterium, halogen (e.g., fluorine), and methoxy; RE² is selected from hydrogen, deuterium, or halogen; RE^{L1} is selected from a bond, -CH₂-, -NH-, -NCH₃-, -O-, - CONH-, or -CONCH₃-; RE^{L2} is selected from a bond and -CH₂-; and p is 1 or 2; or
specifically, E is selected from:

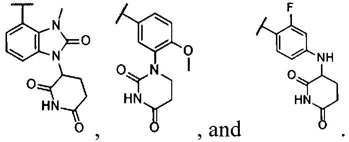

In some embodiments, the compound of formula (II) has the following structure: A-L-E (II), wherein:
moiety A has the following structure: 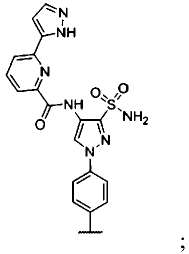
moiety L has the following structure: -L1-L2-L3-L4-L5- formula (L)
wherein L1 is absent or selected from -CH₂-, -CH₂CH₂-, -C(O)-, -NHCH₂-, -CH₂N(CH₃)-, -CH₂O-, -C(O)NH-, -CH₂C(O)-, and -CH₂NH-;
L2 is absent, and L4 is selected from the following groups; or L2 is selected from the following groups, and L4 is absent: 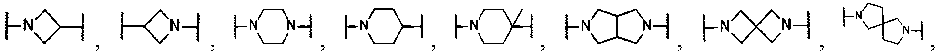 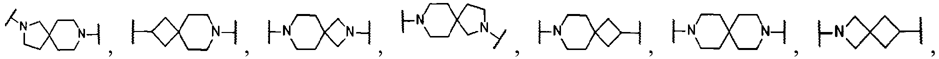 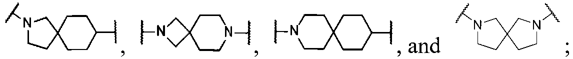
L3 is absent or selected from -CH₂-, -CH₂CH₂-, or -C(O)-;
L5 is absent or selected from -CH₂-, -NH-, and -C(O)-.
E is selected from the following structures:

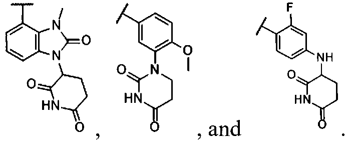

In some embodiments, the compound of formula (II) has the following structure: A-L-E (II), wherein:
moiety A has the following structure: 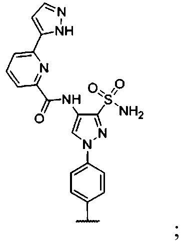
moiety L has the following structure: -L1-L2-L3-L4-L5- formula (L)
wherein L1 is selected from -CH₂- and -C(O)-;
L2 is selected from 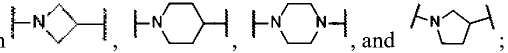
L3 is absent or selected from -OCH₂-, -CH₂CH₂-, and -NHCH₂-;
L4 is absent;
L5 is selected from 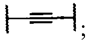
E is selected from the following structures:

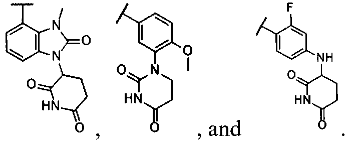

In some embodiments, the compound of formula (II) has the following structure: A-L-E (II), wherein:
moiety A has the following structure: 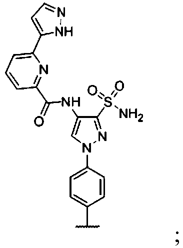
moiety L has the following structure: -L1-L2-L3-L4-L5- formula (L)
wherein L1 is absent or selected from -CH₂-, -C(O)-, and -CH₂O-;
L2 is selected from 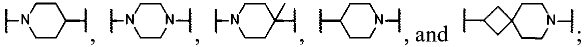
L3 is absent or selected from -CH₂-, -CH₂CH₂-, -NHCH₂-, -C(O)O-, -OCH₂-, -NH-, -N(CH₃)-, - C(O)-, and -C(O)CH₂-;
L4 is selected from 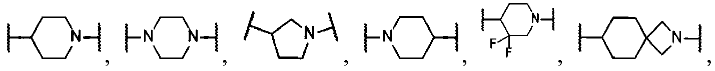 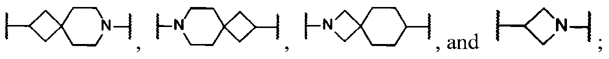
L5 is absent or selected from -C(O)- and -CH₂-;
E is selected from the following structures:

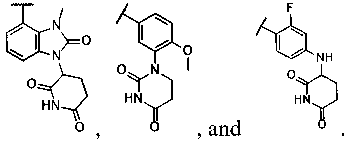

In some embodiments, the compound of formula (II) has the following structure: A-L-E (II), wherein:
moiety A has the following structure: 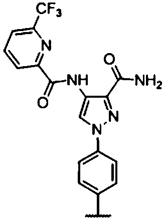
moiety L has the following structure: -L1-L2-L3-L4-L5- formula (L)
wherein L1 is selected from -CH₂-, -CH₂CH₂-, -C(O)-, -C(O)NH-, -CH₂NH-, -CH₂C(O)-, -CH₂O-, and -CH₂N(CH₃)-;
L2 is selected from 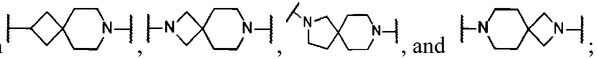
L3 is absent or selected from -CH₂- and -C(O)-;
L4 is absent or selected from 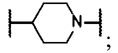
L5 is absent.
E is selected from the following structures:

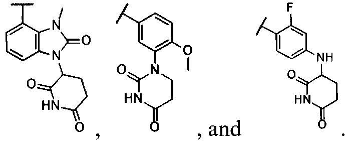

In some embodiments, the compound of formula I is selected from:
A1, A2, A3, A4, A5, A6, A7, A8, A9, A10, A11, A12, A13, A14, A15, A16, A17, A18, A19, A20, A21, A22, A23, A24, A25, A26, A27, A28, A29, A30, A31, A32, A33, A34, A35, A36, A37, A38, A39, A40, A41, A42, A43, A44, A45, A46, A47, A48, A49, A50, A51, A52, A53, A54, A55, A56, A57, A58, A59, A60, A61, A62, A63, A64, A65, A66, A67, A68, A69, A70, A71, A72, A73, A74, A75, A76, A77, A78, A79, A80, A81, A82, A83, A84, A85, A86, A87, A88, A89, A90, A91, A92, A93, A94, A95, A96, A97, A98, A99, A100, A101, A102, A103, A104, A105, A106, A107, A108, A229, A230, A231, A232, A233, A234, A235, A236, A237, A238, A239, A240, A241, A242, A243, A244, A245, A246, A247, A248, A249, A250, A251, A252, A253, A254, A255, A256, A257, A258, A259, A260, A261, A262, A263, A264, A265, A266, A267, A268, A269, A270, A271, A272, A273, A274, A275, A276, A277, A278, A279, A280, A281, A282, A283, A284, A285, A286, A287, A288, A289, A290, A291, A292, A293, A294, A295, A296, A297, A455, A456, A457, A458, A459, A460, A461, A462, A463, A464, A465, A466, A467, A468, A469, A470, A471, A472, and A473.

In some embodiments, the compound of formula (II) is selected from:
A109, A110, A111, A112,A113, A114,A115, A116,A117, A118, A119, A120, A121, A122, A123, A124, A125, A126, A127, A128, A129, A130, A131, A132, A133, A134, A135, A136, A137, A138, A139, A140, A141, A142, A143, A144, A145, A146, A147, A148, A149, A150, A151, A152, A153, A154, A155, A156, A157, A158, A159, A160, A161, A162, A163, A164, A165, A166, A167, A168, A169, A170, A171, A172, A173, A174, A175, A176, A177, A178, A179, A180, A181, A182, A183, A184, A185, A186, A187, A188, A189, A190, A191, A192, A193, A194, A195, A196, A197, A198, A199, A200, A201, A202, A203, A204, A205, A206, A207, A208, A209, A210, A211, A212, A213, A214, A215, A216, A217, A218, A219, A220, A221, A222, A223, A224, A225, A226, A227, A228, A298, A299, A300, A301, A302, A303, A304, A305, A307, A308, A309, A310, A311, A312, A313, A314, A315, A316, A317, A318, A319, A320, A321, A322, A324, A325, A326, A327, A328, A329, A330, A331, A332, A333, A334, A335, A336, A337, A338, A339, A340, A341, A343, A348, A349, A350, A351, A352, A353, A354, A355, A356, A357, A358, A359, A360, A361, A362, A363, A364, A365, A366, A367, A368, A369, A370, A371, A372, A373, A374, A377, A378, A379, A380, A383, A384, A385, A386, A387, A388, A389, A390, A391, A392, A393, A394, A395, A396, A397, A398, A399, A400, A401, A402, A403, A404, A405, A406, A407, A408, A409, A410, A411, A412, A413, A414, A415, A416, A417, A418, A419, A420, A421, A422, A423, A424, A425, A426, A427, A428, A429, A430, A431, A432, A433, A434, A435, A436, A437, A438, A439, A440, A441, A442, A443, A444, A445, A446, A447, A448, A449, A450, A451, A452, A453, and A454.

The present disclosure further provides a pharmaceutical composition comprising the compound or the pharmaceutically acceptable salt, ester, solvate, or stereoisomer thereof of the present disclosure, and a pharmaceutically acceptable excipient.

The present disclosure provides a method for treating or preventing an IRAK protein-mediated disease, and the method comprises administering to a patient in need thereof the compound of formula (I) or formula (II) or the composition of the present disclosure.

The present disclosure provides use of the compound of formula (I) or formula (II) or the composition of the present disclosure in the manufacture of a medicament for treating or preventing an IRAK protein-mediated disease.

In some embodiments, the IRAK protein-mediated disease is selected from: brain cancer, kidney cancer, liver cancer, adrenal cancer, bladder cancer, breast cancer, gastric cancer, ovarian cancer, colon cancer, rectal cancer, prostate cancer, pancreatic cancer, lung cancer, vaginal cancer, cervical cancer, testicular cancer, genitourinary cancers, esophageal cancer, laryngeal cancer, skin cancer, bone cancer, thyroid cancer, sarcoma, mesothelioma, glioblastoma, neuroblastoma, head and neck cancer, adenoma, adenocarcinoma, melanoma, lymphoma, Hodgkin's lymphoma, non-Hodgkin's lymphoma, follicular lymphoma, plasmacytoma or intravascular large B cell lymphoma, diffuse large B cell lymphoma (DLBCL), Burkitt lymphoma, acute myeloid leukemia (AML), chronic lymphocytic leukemia (CLL), myelodysplastic syndromes (MDS), multiple myeloma, Alzheimer's disease, Parkinson's disease, amyotrophic lateral sclerosis, Huntington's disease, cerebral ischemia or injury, hypoxemia, epilepsy, metabolic syndrome, catabolic syndrome, hypertension, type 1 diabetes, type 2 diabetes, obesity, gout, hypercholesterolemia, hypertriglyceridemia, dyslipidemia, heart disease, chronic heart failure, cardiac hypertrophy, myocarditis, ocular diseases, ocular allergy, conjunctivitis, dry eye, cataract, glaucoma, retinal diseases, endocrine related eye diseases, nasal diseases, sinusitis, allergic rhinitis, viral myocarditis, autoimmune blood disorders, hemolytic anemia, aplastic anemia, pure red cell aplasia, idiopathic thrombocytopenia, myasthenia gravis, Stevens-Johnson syndrome, gastrointestinal diseases, gastritis, enteritis, colitis, proctitis, irritable bowel syndrome, Crohn's disease, necrotizing enterocolitis, inflammatory bowel disease, small intestinal colitis, gastrointestinal infection related inflammation (including Clostridium difficile infection), ulcerative colitis, pulmonary diseases, pneumonia, acute lung injury, acute respiratory distress syndrome, hyaline membrane disease, chronic obstructive pulmonary disease (COPD), cystic fibrosis, acid-induced lung injury, pulmonary hypertension, asthma, allergy, bronchiolitis, bronchitis, interstitial pulmonary fibrosis, interstitial lung disease, renal diseases, glomerular diseases, chronic kidney disease, diabetic nephropathy, nephritis, glomerulonephritis, leptospiral nephropathy, IgA nephropathy, renal fibrosis, pyelonephritis, hepatic diseases, hepatitis, chronic hepatitis, alcoholic liver disease, alcoholic fatty liver, alcoholic hepatitis, nonalcoholic fatty liver disease, nonalcoholic steatohepatitis (NASH), arthritis, systemic juvenile idiopathic arthritis, chronic gouty arthritis, psoriatic arthritis, rheumatoid arthritis, juvenile rheumatoid arthritis, polychondritis, other inflammatory diseases, cutaneous lupus erythematosus (CLE), systemic lupus erythematosus (SLE), lupus nephritis, atopic dermatitis, hidradenitis suppurativa, psoriasis, multiple sclerosis, scleroderma, vitiligo, Sjögren's syndrome, uveitis, organ transplantation or graft versus host disease, vasculitis, cystitis, chronic granulomatous disease, muscular atrophy, pancreatitis, hereditary periodic fever syndromes, allergic reactions, systemic anaphylaxis, polyneuropathy, inclusion body myositis, thyroiditis, Addison's disease, appendicitis, bursitis, cervicitis, cholangitis, cholecystitis, chronic transplant rejection, dacryoadenitis, dermatitis, dermatomyositis, polymyositis, encephalitis, endocarditis, endometritis, epididymitis, fasciitis, fibrositis, laryngitis, mastitis, meningitis, myelitis, myositis, hepatic fibrosis, cardiac fibrosis, oophoritis, orchitis, osteitis, otitis, parotitis, pericarditis, peritonitis, pharyngitis, pleuritis, phlebitis, prostatitis, salpingitis, stomatitis, synovitis, tonsillitis, vaginitis, vulvitis, alopecia areata, erythema multiforme, dermatitis herpetiformis, sclerotic diseases, leukoderma, urticaria, pemphigus, cryopyrin-associated periodic syndromes, Lyme disease, pelvic inflammatory disease, and acute and chronic tissue injury.

### Detailed Description of Embodiments

### Examples

The present invention will be further described below with reference to specific examples. It should be understood that these examples are for illustrative purposes only and should not be construed as limiting the invention in any way. Experimental methods without specific conditions described herein are generally carried out under conventional conditions or the manufacturer's recommended conditions.

### Preparation Example 1. Synthesis of Compound A1

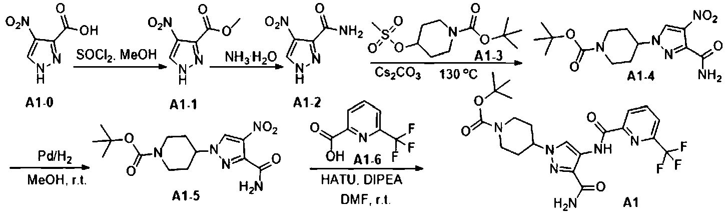

Compound A1-0 (10 g, 54.013 mmol, 1 eq) was dissolved in 150 ml of MeOH, and thionyl chloride (8 ml, 110.145 mmol, 2 eq) was added dropwise in an ice bath. The mixture was stirred at room temperature overnight. Upon completion, the solvent was removed by rotary evaporation to afford compound A1-1 (9 g, 52.598 mmol, yield 98%).

Compound A1-1 (9 g, 52.598 mmol, 1 eq) was diluted in 28% aqueous ammonia (100 ml, 799 mmol, 15 eq) and refluxed in an oil bath at 100°C for 24 h. Upon completion of the reaction (at which point the solution appeared yellow and turbid), the solvent was removed by rotary evaporation to afford compound A1-2 (7.4 g, 47.406 mmol, yield 90%) as a yellow solid.

Compound A1-2 (4 g, 25.625 mmol, 1 eq) and compound A1-3 (7.16 g, 25.631 mmol, 1 eq) were dissolved in 50 ml of DMF, and cesium carbonate (16.7 g, 51.255 mmol, 2 eq) was added. The mixture was reacted in an oil bath at 130°C for 16 h. Upon completion, the reaction mixture was diluted in EtOAc and filtered through Celite to remove solids. The filtrate was extracted sequentially with water and saturated aqueous NaCl, dried over anhydrous Na₂SO₄, loaded onto silica gel while wet, and purified by silica gel column chromatography (petroleum ether : ethyl acetate = 1 : 2) to afford compound A1-4 (2.4 g, 7.072 mmol, yield 28%).

Compound A1-4 (2.4 g, 7.072 mmol, 1 eq) was dissolved in 25 ml of MeOH, and 5% palladium on carbon (800 mg, 1:0.33) was added. The mixture was stirred under H₂ (balloon) for 8 h. Upon completion, the reaction mixture was suction-filtered through a Celite pad with a thin layer of silica gel and subjected to rotary evaporation to dryness to afford compound A1-5 (830 mg, 2.683 mmol, yield 38%) as a white solid.

Compound A1-6 (518 mg, 2.710 mmol, 1.01 eq) was dissolved in 10 ml of dry DMF, and DIPEA (2.3 ml, 13.204 mmol, 5 eq) was added. After stirring, HATU (1.224 g, 3.219 mmol, 1.2 eq) was added, and the mixture was stirred at rt for 1 h. Compound A1-5 (830 mg, 2.683 mmol, 1 eq, in 5 ml of DMF) was added, and the mixture was reacted at rt for 3 h. Upon completion, the mixture was diluted in a large amount of EtOAc, extracted with saturated aqueous NaCl, dried over anhydrous Na₂SO₄, loaded onto silica gel while wet, and purified by silica gel column chromatography (DCM : MeOH = 250 : 1) to afford compound A1 (750 mg, 1.555 mmol, yield 58%). **¹H NMR:** (500 MHz, DMSO) *δ* 11.70 (s, 1H), 8.47 (s, 1H), 8.41 (d, 1H, *J =* 7.6 Hz), 8.37 (t, 1H, *J* = 7.7 Hz), 8.18 (d, 1H, *J =* 7.6 Hz), 7.68 (s, 1H), 7.55 (s, 1H), 4.44-4.53 (m, 1H), 4.00-4.14 (m, 2H), 2.81-3.03 (m, 2H), 2.00-2.08 (m, 2H), 1.81-1.92 (m, 2H), 1.42 (s, 9H).

### Preparation Example 2. Synthesis of Compound A2

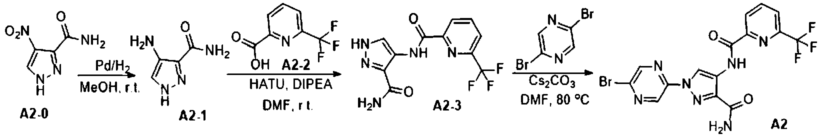

Compound A2-0 (6.1 g, 39.078 mmol, 1 eq) was dissolved in 30 ml of MeOH, and 5% palladium on carbon (900 mg, 1:0.3) was added. The mixture was stirred under H₂ (balloon) for 8 h. Upon completion, the mixture was suction-filtered through Celite, subjected to rotary evaporation to dryness, dissolved in a large amount of MeOH/DCM, and purified by flash column chromatography (DCM : MeOH = 4 : 1) to afford compound A2-1 (4.4 g, 34.887 mmol, yield 89%).

Compound A2-2 (6.7 g, 35.058 mmol, 1.005 eq) was dissolved in 30 ml of dry DMF, and DIPEA (30 ml, 172.224 mmol, 5 eq) was added. After stirring, HATU (16 g, 42.079 mmol, 1.2 eq) was added, and the mixture was stirred at rt for 1 h. Compound A2-1 (4.4 g, 34.887 mmol, 1 eq, in 10 ml of DMF) was added, and the mixture was reacted at rt for 3 h. Upon completion, the mixture was diluted in a large amount of EtOAc, extracted with saturated aqueous NaCl, dried over anhydrous Na₂SO₄, and subjected to rotary evaporation to dryness. DCM was added to precipitate the solid product, and the mixture was suction-filtered to afford compound A2-3 (6.6 g, 22.058 mmol, yield 63%).

2,5-Dibromopyrazine (890 mg, 3.741 mmol, 1.12 eq) was dissolved in 10 ml of DMF in a sealed tube, followed by the addition of cesium carbonate (2.7 g, 8.287 mmol, 2.5 eq). Separately, compound A2-3 (1 g, 3.342 mmol, 1 eq) was dissolved in 5 ml of DMF and added dropwise to the reaction system. The reaction was conducted in an oil bath at 80°C for 5 h. Upon completion, the mixture was diluted in EtOAc and suction-filtered through Celite. The filtrate was extracted with saturated aqueous NaCl, dried over anhydrous Na₂SO₄, and purified by silica gel column chromatography (DCM : TEA = 500 : 10, followed by DCM : MeOH : TEA = 500 : 5 : 10) to afford compound A2 (350 mg, 0.767 mmol, yield 22%). **¹H NMR:** (500 MHz, DMSO) *δ* 11.74 (s, 1H), 9.16 (s, 1H), 9.08 (s, 1H), 8.82 (s, 1H), 8.43 (d, 1H, *J* = 7.6 Hz), 8.38 (t, 1H, *J =* 7.6 Hz), 8.23 (s, 1H), 8.20 (d, 1H, *J =* 7.6 Hz), 7.94 (s, 1H).

### Preparation Example 3. Synthesis of Compound A3

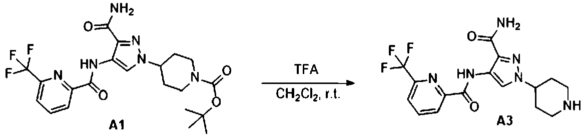

Compound A1 (120 mg, 0.249 mmol, 1 eq) was dissolved in 20 ml of DCM, and TFA (boiling point: 72.4°C) (0.25 ml, 3.366 mmol, 12 eq) was added. The mixture was reacted at rt for 3 h. Upon completion, the mixture was diluted in EtOAc and extracted sequentially with aqueous NaOH and saturated aqueous NaCl. The organic layer was dried over anhydrous Na₂SO₄ and subjected to rotary evaporation to afford compound A3 (93 mg, 0.243 mmol, yield 97.7%) as a white solid. **¹H NMR:** (500 MHz, DMSO) *δ* 11.72 (s, 1H), 8.41-8.46 (m, 2H), 8.38 (t, 1H, *J* = 7.6 Hz), 8.19 (d, 1H, *J* = 7.6 Hz), 7.66 (s, 1H), 7.57 (s, 1H), 4.29-4.38 (m, 1H), 3.03-3.13 (m, 2H), 2.59-2.64 (m, 2H), 1.97-2.04 (m, 2H), 1.81-1.91 (m, 2H).

### Preparation Example 4. Synthesis of Compound A4

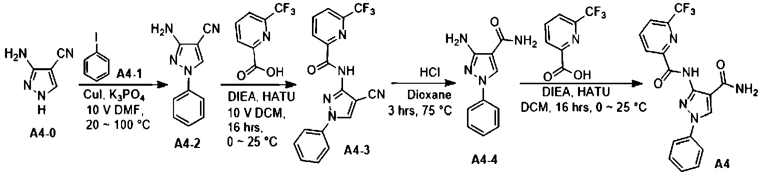

Compound A4-0 (3.30 g, 30.5 mmol, 1.00 eq) and DMF (33.0 mL) were added to a 100 mL three-necked flask, followed by the addition of compound A4-1 (6.23 g, 30.5 mmol, 3.40 mL, 1.00 eq), DMEDA (2.69 g, 30.5 mmol, 3.29 mL, 1.00 eq), CuI (2.91 g, 15.2 mmol, 0.50 eq), and K₃PO₄ (19.4 g, 91.5 mmol, 3.00 eq) under a nitrogen atmosphere at 20-25°C. The reaction was carried out at 100°C for 3 h. LCMS indicated complete consumption of compound A4-0, with the target mass spectrum detected (RT = 1.748 min). The mixture was filtered, and the filtrate was collected and concentrated. The crude was purified by preparative HPLC (TFA conditions; column: Phenomenex Luna C18 200×40 mm×10 µm; mobile phase: [water (TFA)-ACN]; gradient: 20-50% B, 10 min). The mobile phase was extracted with EtOAc (20 mL×2), dried over Na₂SO₄, filtered, and concentrated to afford compound A4-2 (970 mg, 5.16 mmol, yield 16.9%, purity 98.0%) as a yellow solid.

6-(Trifluoromethyl)pyridine-2-carboxylic acid (986 mg, 5.16 mmol, 1.00 eq), HATU (2.94 g, 7.74 mmol, 1.50 eq), and DCM (10.0 mL) were added to a 100 mL three-necked flask, followed by the addition of DIEA (2.00 g, 15.4 mmol, 2.70 mL, 3.00 eq). The mixture was reacted at 0-10°C for 0.5 h, and then compound A4-2 (970 mg, 5.16 mmol, 1.00 eq) was added. The mixture was reacted at 20-25°C for 15.5 h. LCMS indicated 17.7% of compound A4-2 remained (RT = 0.319 min), with the target mass spectrum detected (RT = 0.478 min). The reaction solution was concentrated to afford the crude, which was separated by column chromatography (SiO₂, DCM : MeOH = 50 : 1 to 10 : 1, product: Rf = 0.75) to afford compound A4-3 (1.01 g, 2.82 mmol, yield 54.6%, purity 99.7%) as a white solid.

At 20-25°C, compound A4-3 (1.00 g, 2.79 mmol, 1.00 eq) and dioxane (2.00 mL) were added to a 100 mL three-necked flask, followed by the addition of HCl (12.0 M, 20.0 mL, 86.0 eq). The mixture was heated to 75°C and reacted for 3 h. LCMS indicated 11.2% of compound A4-3 remained (RT = 0.452 min), with the target mass spectrum detected (RT = 0.253 min). NaHCO₃ was added to adjust pH to 7-8, and the mixture was extracted with EtOAc (×3) (50.0 mL). The combined organic phases were washed with saturated aqueous NaCl (50.0 mL), dried over Na₂SO₄, filtered, and concentrated to afford compound A4-4 (300 mg, 937 µmol, yield 33.6%, purity 63.2%) as a yellow solid.

6-(Trifluoromethyl)pyridine-2-carboxylic acid (179 mg, 937 µmol, 1.20 eq), HATU (445 mg, 1.17 mmol, 1.50 eq), and THF (2.00 mL) were added to a 10 mL single-necked flask, followed by the addition of DIEA (302 mg, 2.34 mmol, 408 µL, 3.00 eq). The mixture was reacted at 0-10°C for 0.5 h, and then compound A4-4 (250 mg, 781 µmol, 1.00 eq) was added. The reaction was carried out at 25°C for 15.5 h. TLC (DCM : MeOH = 8 : 1) indicated complete consumption of compound A4-4 (Rf = 0.70) and appearance of a new spot (Rf = 0.45). The mixture was filtered, and the filter cake was collected and dried in vacuo. The solid was slurried with THF (5.00 mL) and filtered. The filter cake was dissolved in water (20.0 mL) and acetonitrile (5.00 mL), and lyophilized to afford compound A4 (114 mg, 262 µmol, yield 33.5%, purity 98.5%) as a white solid. LCMS: m/z = 376.1 (M+H)⁺. **¹H NMR:** (400 MHz, DMSO-*d₆*) *δ* 12.22 (s, 1H) 9.00 (s, 1H) 8.36-8.49 (m, 2H) 8.21 (d, J = 7.4 Hz, 1H) 7.74-7.84 (m, 3H) 7.54-7.62 (m, 3H) 7.38 (t, J = 7.4 Hz, 1H).

### Preparation Example 5. Synthesis of Compound A5

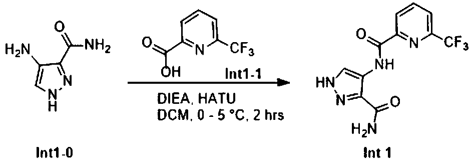

Compound Int1-1 (7.73 g, 40.4 mmol, 1.02 eq) and DCM (75.0 mL) were added to a 250 mL three-necked flask. At 0-5°C, DIEA (10.2 g, 79.2 mmol, 13.8 mL, 2.00 eq) and HATU (16.5 g, 43.6 mmol, 1.10 eq) were added, and the mixture was stirred at 0-5°C for 1 h. Compound Int1-0 (5.00 g, 39.6 mmol, 1.00 eq) was then added, and the mixture was stirred at 0-5°C for another 1 h. LCMS indicated complete consumption of Int1-0, with the main peak of the product detected. The reaction mixture was concentrated under reduced pressure, and EtOAc (500 mL) was added. The organic phase was washed with saturated aqueous NaCl (500 mL×3), dried over Na₂SO₄, and concentrated. The crude was slurried with EtOAc/PE = 1/5 (200 mL) and filtered, and the filter cake was washed with petroleum ether (200 mL) and dried under reduced pressure to afford compound Int1 (12.5 g, 38.4 mmol, yield 80.7%, purity 92%) as a yellow solid. LCMS: m/z = 322.1 (M+Na)⁺. **¹H NMR:** (400 MHz, DMSO) δ 13.31 (s, 1H), 11.72 (s, 1H), 8.42-8.39 (m, 3H), 8.43-8.34 (m, 1H), 8.18 (d, J = 6.4 Hz, 2H), 7.78 (d, J = 100 Hz, 1H).

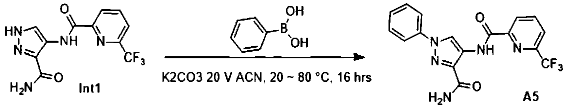

At 20-25°C, ACN (4.00 mL), compound Int1 (200 mg, 668 µmol, 1.00 eq), and phenylboronic acid (81.5 mg, 668 µmol, 1.00 eq) were added to a three-necked round-bottom flask. Under an oxygen atmosphere, chloro(hydroxy)copper;N,N,N',N'-tetramethylethylenediamine (93.1 mg, 200 µmol, 0.300 eq) and K₂CO₃ (184 mg, 1.34 mmol, 2.00 eq) were added. Under O₂, the temperature was raised to 75°C -80°C and the reaction was carried out for 16 h. LCMS indicated complete consumption of compound Int1, with the target mass spectrum detected. The reaction solution was filtered, and the filter cake was washed twice with ACN (0.5 mL). The filtrate was purified by preparative HPLC (TFA conditions), and the collected fractions were concentrated in vacuo at 45°C. NaHCO₃ (2.00 g) was added to the concentrate, and the mixture was extracted with EtOAc (×3) (20.0 mL). The combined organic phases were washed with saturated aqueous NaCl (10.0 mL), dried over Na₂SO₄, filtered, and concentrated. The crude was further purified by preparative TLC (ethyl acetate : petroleum ether = 1 : 1, Rf = 0.44). The silica gel powder scraped from the plate was stirred with ACN (30.0 mL) for 30 min and concentrated to afford compound A5 (66.0 mg, 171 µmol, yield 12.8%, purity 97.6%) as a white solid. LCMS: m/z = 376.1 (M+H)⁺. **¹H NMR:** (400 MHz, DMSO-*d₆*) *δ* 11.79 (s, 1H), 9.03 (s, 1H), 8.42-8.36 (m, 1H), 8.49-8.36 (m, 1H), 8.25-8.18 (m, 1H), 8.03 (br s, 1H), 7.99 (br d, J = 8.0 Hz, 2H), 7.77 (br s, 1H), 7.56 (t, J = 8.0 Hz, 2H), 7.46-7.36 (m, 1H).

### Preparation Example 6. Synthesis of Compound A6

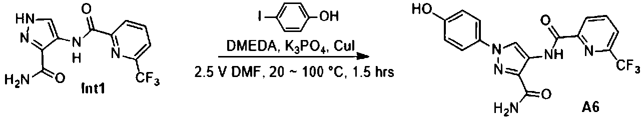

At 20°C-25°C, compound Int1 (400 mg, 1.34 mmol, 1.00 eq), 4-iodophenol (294 mg, 1.34 mmol, 1.00 eq), DMEDA (118 mg, 1.34 mmol, 144 µL, 1.00 eq), K₃PO₄ (851 mg, 4.01 mmol, 3.00 eq), DMF (1.00 mL) and CuI (127 mg, 668 µmol, 0.50 eq) were added to a 10 mL single-necked round-bottom flask. Under N₂, the temperature was raised to 100°C and the reaction was carried out for 1.5 h. LCMS indicated 1.76% of compound Int1 remained, with the target mass spectrum detected. The reaction solution was filtered, and the filter cake was washed (×3) with DMF (0.5 mL). The mixture was concentrated in vacuo at 50°C-55°C, and the resulting crude was purified by preparative HPLC (TFA conditions), concentrated in vacuo at 45°C, and lyophilized to afford compound A6 (207 mg, 523 µmol, yield 39.1%, purity 98.8%) as a pink solid. LCMS: m/z = 392.0 (M+H)⁺. **¹H NMR:** (400 MHz, DMSO-*d₆*) *δ* 11.79 (s, 1H) 9.79 (s, 1H) 8.85 (s, 1H) 8.48-8.38 (m, 2H) 8.21 (dd, J = 7.6, 1.0 Hz, 1H) 7.93 (s, 1H) 7.78-7.68 (m, 3H) 6.95-6.89 (m, 2H).

### Preparation Example 7. Synthesis of Compound A7

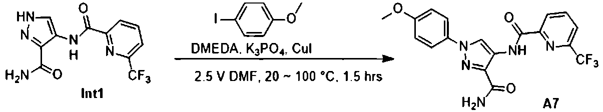

At 20°C-25°C, compound Intl (400 mg, 1.34 mmol, 1.00 eq), 4-iodoanisole (313 mg, 1.34 mmol, 1.00 eq), DMEDA (118 mg, 1.34 mmol, 144 µL, 1.00 eq), K₃PO₄ (851 mg, 4.01 mmol, 3.00 eq), DMF (1.00 mL) and CuI (127 mg, 668 µmol, 0.50 eq) were added to a 10 mL single-necked round-bottom flask. Under N₂, the temperature was raised to 100°C and the reaction was carried out for 1.5 h. LCMS indicated 1.41% of compound Int1 remained, with the target mass spectrum detected. The reaction solution was filtered, and the filter cake was washed (×3) with DMF (0.5 mL). The mixture was concentrated in vacuo at 50°C-55°C, and the resulting crude was purified by preparative HPLC (TFA conditions), concentrated in vacuo at 45°C, and lyophilized to afford compound A7 (128 mg, 310 µmol, yield 23.2%, purity 97.8%) as an off-white solid. LCMS: m/z = 406.1 (M+H)⁺. **¹H NMR:** (400 MHz, DMSO-*d₆*) *δ* 11.79 (s, 1H). 8.93 (s, 1H) 8.48-8.37 (m, 2H) 8.22 (dd, J = 7.6, 0.8 Hz, 1H) 7.98 (s, 1H) 7.94-7.86 (m, 2H) 7.73 (s, 1H) 7.15-7.01 (m, 2H) 3.83 (s, 3H).

### Preparation Example 8. Synthesis of Compound A8

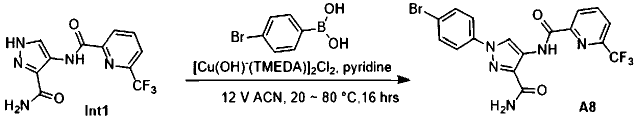

At 20°C-25°C, compound Intl (923 mg, 2.49 mmol, 1.00 eq), pyridine (394 mg, 4.98 mmol, 402 µL, 2.00 eq) and 4-bromophenylboronic acid (500 mg, 2.49 mmol, 1.00 eq) were added to a 40 mL reaction flask. Under O₂, [Cu(OH)-(TMEDA)]₂Cl₂ (347 mg, 747 µmol, 0.30 eq) and ACN (12.0 mL) were added. Under O₂, the temperature was raised to 80°C and the reaction was carried out for 16 h. LCMS indicated 32.8% of compound Int1 remained, with the target mass spectrum detected. The mixture was concentrated in vacuo at 45°C. The crude was purified by column chromatography (SiO₂, DCM : MeOH = 10 : 1, Rf = 0.5), then purified by flash silica gel column chromatography (Welch Ultimate XB-SiOH 250×70×10 µm; mobile phase: [hexane-EtOH]; B%: 6%-30%, 25 min) and lyophilized to afford compound A8 (55.5 mg, 119 µmol, yield 4.80%, purity 97.7%) as a white solid. LCMS: m/z = 455.7 (M+H)⁺. **¹H NMR:** (400 MHz, DMSO-*d₆*) *δ* 11.78 (s, 1H) 9.07 (s, 1H) 8.47-8.37 (m, 2H) 8.13-8.26 (m, 1H) 8.08 (s, 1H) 7.99 (d, J = 9.0 Hz, 2H) 7.67-7.85 (m, 3H) 3.60 (br t, J = 6.6 Hz, 1H).

### Preparation Example 9. Synthesis of Compound A9

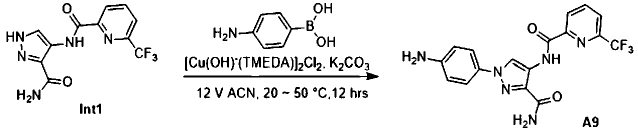

At 20°C-25°C, compound Int1 (2.85 g, 7.69 mmol, 1.00 eq), K₂CO₃ (2.12 g, 15.4 mmol, 2.00 eq) and [Cu(OH)-(TMEDA)]₂Cl₂ (428 mg, 922 µmol, 0.30 eq) were added to a 100 mL three-necked reaction flask. Under O₂, 4-bromophenylboronic acid (1.05 g, 7.69 mmol, 1.00 eq) and ACN (35.0 mL) were added. Under O₂, the temperature was raised to 50°C and the reaction was carried out for 16 h. LCMS indicated 42.7% of compound Int1 remained, with the target mass spectrum (49.1%) detected. After filtration, the mixture was concentrated in vacuo at 45°C. The crude was purified by preparative HPLC (column: Daisogel C18 250×70 mm×10 µm; mobile phase: [water (NH₄HCO₃)-ACN]; B%: 26%-66%, 15 min) and lyophilized to afford compound A9 (83.5 mg, 205 µmol, yield 2.68%, purity 96.2%) as a gray solid. LCMS: m/z = 390.9 (M+H)⁺. **¹H NMR:** (400 MHz, DMSO-*d₆*) *δ* 12.11-11.48 (m, 1H) 8.76 (s, 1H) 8.47-8.33 (m, 2H) 8.19 (d, J = 7.4 Hz, 1H) 7.88 (br s, 1H) 7.67 (br s, 1H) 7.56 (d, J = 8.8 Hz, 2H) 6.68 (d, J = 8.2 Hz, 2H) 5.38 (br s, 2H).

### Preparation Example 10. Synthesis of Compound A10

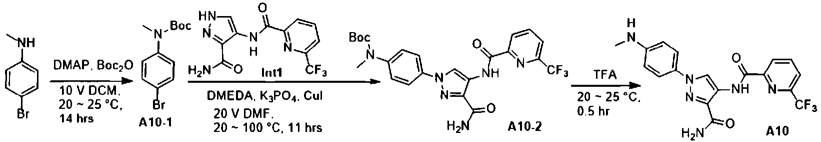

N-methyl-4-bromoaniline (4-bromo-N-methylaniline) (500 mg, 2.69 mmol, 1.00 eq), DMAP (32.8 mg, 269 µmol, 0.10 eq), Boc₂O (880 mg, 4.03 mmol, 926 µL, 1.50 eq), and DCM (5.00 mL) were added to a 40 mL parallel reaction flask at 20°C-25°C. The reaction was allowed to proceed at 20°C-25°C for 14 h. LCMS indicated 15.5% of N-methyl-4-bromoaniline (4-bromo-N-methylaniline) remained, with the target mass spectrum detected (RT = 0.520 min). The reaction mixture was poured into water (10.0 mL) and extracted four times with DCM (15.0 mL). The combined organic phases were washed with saturated aqueous NaCl (50.0 mL), dried over Na₂SO₄, filtered, and concentrated to afford compound A10-1 (800 mg, 2.10 mmol, yield 78.0%, purity 75.0%) as a dark brown liquid.

At 20°C-25°C, a 100 mL three-necked round-bottom flask was charged with compound Int1 (500 mg, 1.67 mmol, 1.00 eq), compound A10-1 (638 mg, 1.67 mmol, 1.00 eq), DMEDA (147 mg, 1.67 mmol, 180 µL, 1.00 eq), K₃PO₄ (1.06 g, 5.01 mmol, 3.00 eq), DMF (10.0 mL), and CuI (159 mg, 836 µmol, 0.50 eq). The atmosphere was purged with N₂ (×3), the temperature was raised to 100°C, and the mixture was reacted for 11 h. LCMS indicated 5.02% of compound Int1 remained, with the target mass spectrum detected. The mixture was filtered and extracted with EtOAc (×3) (5.00 mL). The crude was purified by preparative HPLC (TFA conditions), concentrated at 40°C-45°C, and lyophilized to afford compound A10-2 (50.0 mg, 97.0 µmol, yield 5.81%, purity 97.9%) as a brown solid.

Compound A10-2 (40.0 mg, 79.3 µmol, 1.00 eq) and TFA (271 mg, 2.38 mmol, 177 µL, 30.0 eq) were added to a 10 mL parallel reaction flask at 20°C-25°C and reacted at 20°C-25°C for 0.5 h. LCMS indicated complete consumption of compound A10-2, with the target mass spectrum detected. After concentration, MeOH (100 mL) and 4.00 g of basic resin were added, reacted at 20°C-25°C for 1 h, filtered, and concentrated to afford compound A10 (29.6 mg, 68.5 µmol, yield 86.4%, purity 93.5%) as a yellow solid. LCMS: m/z = 405.1 (M+H)⁺. **¹H NMR:** (400 MHz, DMSO-*d₆*) *δ* 11.80 (s, 1H) 8.76 (s, 1H) 8.46-8.29 (m, 2H) 8.19 (br d, J = 3.6 Hz, 1H) 7.87 (s, 1H) 7.58 (m, 3H) 6.59 (d, J = 9.0 Hz, 2H) 5.97-5.84 (m, 1H) 2.66 (d, J = 5.0 Hz, 3H).

### Preparation Example 11. Synthesis of Compound A11

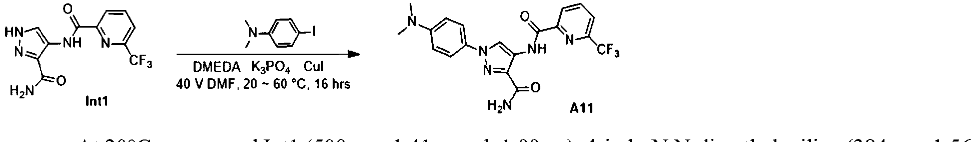

At 20°C, compound Int1 (500 mg, 1.41 mmol, 1.00 eq), 4-iodo-N,N-dimethylaniline (384 mg, 1.56 mmol, 1.10 eq), DMEDA (124 mg, 1.41 mmol, 152 µL, 1.00 eq), K₃PO₄ (900 mg, 4.24 mmol, 3.00 eq), CuI (134 mg, 706 µmol, 0.50 eq) and DMF (20.0 mL) were added to a 10 mL reaction flask. Under O₂, the temperature was raised to 60°C and the reaction was carried out for 16 h. LCMS indicated 12.6% of compound Int1 remained, with the target mass spectrum detected. The reaction solution was concentrated in vacuo. The crude was purified by preparative HPLC (column: Welch Ultimate C18 150×25 mm×5 µm; mobile phase: [water (FA)-ACN]; gradient: 42%-72% B, 12 min) and lyophilized to give compound A11 (31.7 mg, 75.2 µmol, yield 5.33%, purity 99.3%) as a gray solid. LCMS: m/z = 419.1 (M+H)⁺. **¹H NMR:** (400 MHz, DMSO-*d₆*) *δ* 11.80 (s, 1H), 8.84 (s, 1H), 8.54-8.32 (m, 2H), 8.24-8.18 (m, 1H), 7.90 (s, 1H), 7.74 (d, J = 9.0 Hz, 2H), 7.68 (s, 1H), 6.68 (d, J = 9.2 Hz, 2H), 2.96 (s, 6H).

### Preparation Example 12. Synthesis of Compound A12

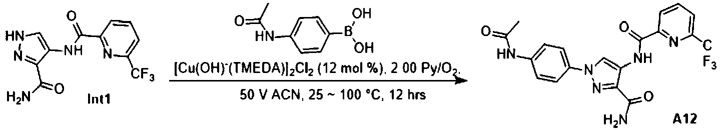

4-Acetamidophenylboronic acid (300 mg, 848 µmol, 1.00 eq), compound Intl (152 mg, 848 µmol, 1.00 eq), pyridine (134 mg, 1.70 mmol, 137 µL, 2.00 eq), and [Cu(OH)-(TMEDA)]₂Cl₂ (12 mol%) (78.8 mg, 170 µmol, 0.20 eq) were added to ACN (15.0 mL), heated to 100°C at 25°C, and stirred at 100°C under O₂ for 12 h. LCMS indicated 23.0% of 4-acetamidophenylboronic acid remained, with the desired mass spectrum detected. The reaction mixture was poured into water (100 mL), and the aqueous phase was extracted with EtOAc (100 mL×2). The combined organic phases were washed with aqueous NaCl (100 mL×2), dried over anhydrous Na₂SO₄, filtered, and concentrated in vacuo to afford the crude. The crude was purified by reverse-phase high performance liquid chromatography (column: Welch Xtimate C18 150×25 mm×5 µm; mobile phase: [water (TFA)-ACN]; gradient: 30%-60% B over 10 min). The eluent was concentrated in vacuo to remove the ACN, and the aqueous phase was lyophilized to afford compound A12 (54.6 mg, 124 µmol, yield 14.6%, 97.9% purity) as a yellow solid. LCMS: m/z = 433.1 (M+H)⁺. **¹H NMR:** (400 MHz, DMSO-*d₆*) *δ* 11.8 (s, 1H), 10.1 (s, 1H), 8.95 (s, 1H), 8.46-8.38 (m, 2H), 8.21 (d, *J=* 7.4 Hz, 1H), 7.97 (s, 1H), 7.90 (d, *J* = 8.6 Hz, 2H), 7.74 (d, *J=* 7.8 Hz, 3H), 2.08 (s, 3H).

### Preparation Example 13. Synthesis of Compound A13

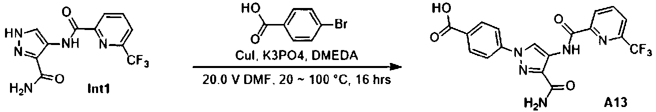

Compound Int1 (500 mg, 1.41 mmol, 1.00 eq) and DMF (10.0 mL) were added to a 100 mL flask, and 4-bromobenzoic acid (283 mg, 1.41 mmol, 1.00 eq), CuI (134 mg, 706 µmol, 0.50 eq) and DMEDA (124 mg, 1.41 mmol, 151 µL, 1.00 eq) were injected into the flask at 20°C-25°C under N₂. K₃PO₄ (899 mg, 4.24 mmol, 3.00 eq) was then added to the flask at 20°C-25°C under N₂, and stirred at 100°C under N₂ for 16 h. LCMS indicated 2.01% of compound Int1 remained, with the desired mass spectrum detected. The crude was stirred with ACN (5.00 mL) at 20°C-25°C for 10 min and filtered. The filter cake was dissolved in 5 ml of water, then adjusted to pH 5-6 with HCl (2.00 M), and filtered to collect the filter cake. The filter cake was dissolved in ACN (2.00 mL), and water (10.0 mL) was added. Then, the mixture was lyophilized to afford compound A13 (266 mg, 634 µmol, yield 44.9%, purity 100%) as a blue solid. LCMS: m/z = 242.0 (M+Na) ⁺. **¹H NMR:** (400 MHz, DMSO-*d₆*) *δ* 11.80 (s, 1H) 9.08 (s, 1H) 8.47-8.38 (m, 2H) 8.28-8.01 (m, 6H) 7.74 (br s, 1H).

### Preparation Example 14. Synthesis of Compound A14

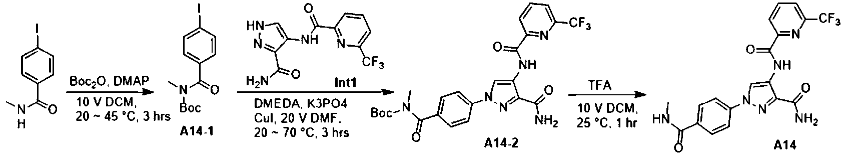

Boc₂O (836.01 mg, 3.83 mmol, 880 µL, 2.00 eq), DMAP (46.8 mg, 383 µmol, 0.200 eq), and 4-iodo-N-methylbenzamide (500 mg, 1.92 mmol, 1.00 eq) were added to DCM (5.00 mL), and the mixture was stirred at 40°C-45°C for 3 h. Thin layer chromatography (ethyl acetate : petroleum ether = 1 : 1) indicated complete consumption of 4-iodo-N-methylbenzamide (Rf = 0.300), with the formation of a new spot (Rf = 0.750). The reaction mixture was added to water (50.0 mL), extracted with METB (50.0 mL×3), and washed with aqueous NaCl (50.0 mL) to afford compound A14-1 (700 mg, crude) as a yellow solid.

To a solution of compound A14-1 (408 mg, 1.13 mmol, 1.00 eq) in DMF (8.00 mL) were added compound Int1 (400 mg, 1.13 mmol, 1.00 eq), DMEDA (99.5 mg, 1.13 mmol, 121 µL, 1.00 eq), K₃PO₄ (719 mg, 3.39 mmol, 3.00 eq) and CuI (107 mg, 564 µmol, 0.500 eq), and the mixture was heated at 20°C-25°C under N₂. The mixture was stirred at 70°C under N₂ for 3 h. Thin layer chromatography (petroleum ether : ethyl acetate = 8 : 1) indicated complete consumption of compound A14-1, with the formation of 1 new spot (Rf = 0.600). The mixture was washed with water (10 ml) and NaHCO₃ (10 ml). The precipitate was collected. The solid was dissolved in 10 ml of ACN and 20 ml of water. The solution was lyophilized under neutral conditions to afford crude A14-2 (400 mg, 751 µmol, yield 66.5%) as a yellow solid, which was used directly in the next step without purification.

TFA (2.57 g, 22.5 mmol, 1.67 mL, 60.0 eq) and compound A14-2 (200 mg, 375 µmol, 1.00 eq) were added to DCM (2.00 mL). The mixture was stirred at 25°C for 1 h. Thin layer chromatography (petroleum ether : ethyl acetate = 8 : 1) indicated complete consumption of compound A14-2 (Rf = 0.600), with the formation of 1 new spot (Rf = 0.500). Thin layer chromatography (TLC) indicated that the reaction was complete. NaHCO₃ (10 mL) was added, and the mixture was extracted with DCM (50.0 mL×3). The combined organic phases were washed with aqueous NaCl (50.0 mL). The mixture was dried over anhydrous Na₂SO₄ and filtered. The filtrate was concentrated under reduced pressure at 40°C to afford compound A14 (54.7 mg, 125 µmol, yield 33.4%, 99.2% purity) as an off-white solid. LCMS: m/z = 432.9 (M+H)⁺. **¹H NMR:** (400 MHz, DMSO-*d₆*) *δ* 11.79 (s, 1H), 9.13 (s, 1H), 8.59-8.51 (m, 1H), 8.43 (br d, *J* = 18.6 Hz, 2H), 8.22 (br d, *J=* 7.6 Hz, 1H), 8.15-8.07 (m, 3H), 8.01 (br d, *J=* 8.6 Hz, 2H), 7.81 (br s, 1H), 2.81 (d, *J =* 4.4 Hz, 3H).

### Preparation Example 15. Synthesis of Compound A15

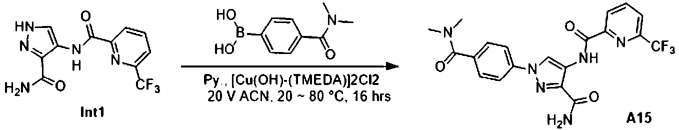

Compound Int1 (700 mg, 1.98 mmol, 1.00 eq), 4-(N,N-dimethyl carbamoyl)phenylboronic acid (419 mg, 2.17 mmol, 1.10 eq), pyridine (312 mg, 3.95 mmol, 319.1 µL, 2 eq), and [Cu(OH)-(TMEDA)]₂Cl₂ (275 mg, 593 µmol, 0.300 eq) were added to ACN (14.0 mL). The mixture was stirred at 80°C for 16 h. LCMS indicated approximately 24.4% of compound Int1 remained (RT = 0.317 min). Several new peaks appeared on the LCMS, and approximately 22.2% of the target compound was detected (RT = 0.400 min). The mixture was filtered and concentrated in vacuo at 40°C to obtain a residue. The residue was purified by column chromatography (SiO₂, petroleum ether: ethyl acetate = 0 : 1, Rf = 0.600). The crude was fermented with THF (2.00 mL) at 25°C for 20 min to afford compound A15 (91.3 mg, 201 µmol, yield 10.1%, 98.3% purity) as a white solid. LCMS: m/z = 447.1 (M+H)⁺. **¹H NMR:** (400 MHz, DMSO-*d₆*) *δ* 11.80 (s, 1H), 9.09 (s, 1H), 8.43 (br d, *J=* 19.0 Hz, 2H), 8.22 (d, *J=* 7.6 Hz, 1H), 8.07 (d, *J=* 8.4 Hz, 3H), 7.81 (s, 1H), 7.59 (d, *J =* 8.6 Hz, 2H), 3.05-2.89 (m, 6H).

### Preparation Example 16. Synthesis of Compound A16

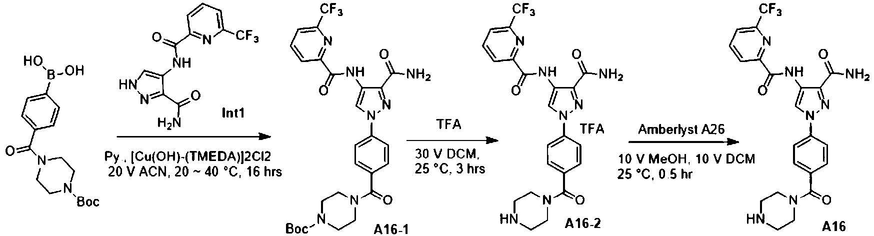

At 25°C under O₂, 4-(4-(tert-butoxycarbonyl)piperazine-1-carbonyl)phenylboronic acid (726 mg, 2.17 mmol, 1.10 eq), pyridine (312 mg, 3.95 mmol, 319 µL, 2.00 eq), [Cu(OH)-(TMEDA)]₂Cl₂ (275 mg, 593 µmol, 0.300 eq), and compound Int1 (700 mg, 1.98 mmol, 1.00 eq) were added to ACN (14.0 mL). The mixture was stirred at 40°C for 16 h. LCMS indicated approximately 19.6% of compound Int1 remained (RT= 0.319 min). Several new peaks appeared on the LCMS, and approximately 23.9% of the target compound was detected (RT = 0.464 min). The mixture was filtered and concentrated in vacuo at 40°C to obtain a residue. The residue was purified by column chromatography (SiO₂, petroleum ether/ethyl acetate=1/1 to 1/5), followed by secondary purification by thin layer chromatography (petroleum ether : ethyl acetate=0 : 1, Rf=0.500). The crude was fermented with THF (5.00 mL) at 25°C for 10 min to afford compound A16-1 (180 mg, 296 µmol, yield 15.0%, 96.8% purity) as a white solid.

TFA (601 mg, 5.27 mmol, 391 µL, 20.0 eq) and compound A16-1 (160 mg, 263 µmol, 1.00 eq) were added to DCM (6.50 mL), and the mixture was stirred at 25°C for 3 h. Thin layer chromatography (petroleum ether : ethyl acetate = 0 : 1) indicated complete consumption of compound A16-1 (Rf = 0.500), with the formation of a new spot (Rf = 0.150). Thin layer chromatography (TLC) indicated that the reaction was complete. The filtrate was concentrated under reduced pressure at 40°C to afford crude A16-2 (160 mg, 260 µmol, yield 98.9%, purity 98.0%) as a white solid, which was used directly without purification.

Compound A16-2 (130 mg, 216 µmol, 1.00 eq, TFA) was dissolved in MeOH (10.0 mL) and DCM (10.0 mL). Amberlyst A26 (4.00 g, 216 µmol, 1.00 eq) was added at 25°C, and the mixture was stirred at 25°C for 0.5 h. Thin layer chromatography (DCM : MeOH = 1 : 2) indicated complete consumption of compound A16-2 (Rf = 0.00), with the formation of a new spot (Rf = 0.110). Thin layer chromatography (TLC) indicated that the reaction was complete. The mixture was filtered with MeOH (10 ml) at 25°C. The mixture was filtered and concentrated in vacuo at 40°C to obtain a residue. The residue was lyophilized under neutral conditions to afford compound A16 (79.7 mg, 154 µmol, yield 71.4%, purity 94.3%) as an off-white solid. LCMS: m/z = 488.1 (M+H)⁺. **¹H NMR:** (400 MHz, DMSO-*d₆*) *δ* 11.79 (s, 1H), 9.08 (s, 1H), 8.43 (dd, J = 7.2, 18.6 Hz, 2H), 8.22 (d, J = 7.0 Hz, 1H), 8.07-8.06 (m, 3H), 7.81 (s, 1H), 7.56 (d, J = 7.8 Hz, 2H), 3.74-3.46 (m, 4H), 2.80-2.60 (m, 4H).

### Preparation Example 17. Synthesis of Compound A17

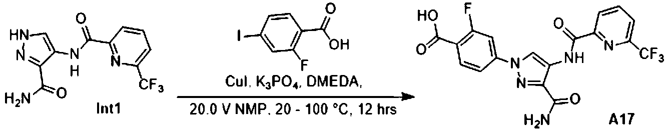

At 20°C-25°C, compound Intl (591 mg, 1.67 mmol, 1.00 eq), 2-fluoro-4-iodobenzoic acid (444 mg, 1.67 mmol, 1.00 eq) and NMP (6.00 mL) were added to a 100 mL three-necked round-bottom flask. At 20°C-25°C under N₂, CuI (159 mg, 835 µmol, 0.50 eq), DMEDA (147 mg, 1.67 mmol, 179 µL, 1.00 eq) and K₃PO₄ (1.06 g, 5.01 mmol, 3.00 eq) were added. The mixture was purged with N₂ (×3), and stirred at 100°C for 12 h. LCMS indicated 7.87% of compound Int1 remained (RT = 0.429 min), with the target mass spectrum (75.7%) detected (RT = 0.549 min).

ACN (20.0 mL) was added, and the mixture was filtered. 20.0 mL of water was added to the filter cake, and the mixture was adjusted to pH 5-6 with HCl (2N). After filtration, the filter cake was concentrated. The residue was purified by high performance liquid chromatography (column: Waters xbridge 150×25 mm 10 µm; mobile phase: [water (NH₄HCO₃)-ACN]; B%: 11%-41%, 10 min). The eluate was then lyophilized to yield compound A17 (103 mg, 227 µmol, yield 13.6%, purity 96.6%). LCMS: m/z = 438.0 (M+H)⁺. **¹H NMR:** (400 MHz, DMSO-*d₆*) *δ* 11.8 (s, 1H), 9.10 (s, 1H), 8.48-8.45 (m, 1H), 8.43-8.37 (m, 1H), 8.22 (d, *J* = 8.0 Hz, 1H), 8.12 (s, 1H), 7.92-7.74 (m, 4H).

### Preparation Example 18. Synthesis of Compound A18

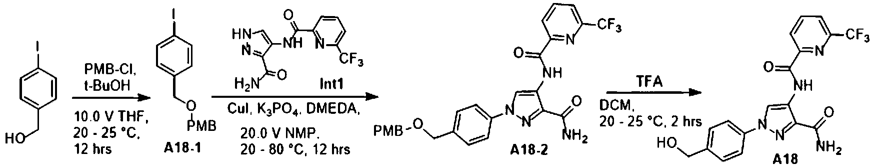

4-Iodobenzyl alcohol (500 mg, 2.14 mmol, 1.00 eq) and THF (5.00 mL) were added to a 100 mL three-necked round-bottom flask at 20°C-25°C. PMB-Cl (669 mg, 4.27 mmol, 579 µL, 2.00 eq) and t-BuOK (479 mg, 4.27 mmol, 2.00 eq) were added at 20°C-25°C. The mixture was purged with N₂ (×3) and stirred at 25°C for 12 h. Thin layer chromatography (petroleum ether : ethyl acetate =1 : 1) indicated complete consumption of 4-iodobenzyl alcohol (Rf = 0.2), with the formation of a new spot (Rf = 0.5). The reaction solution was poured into 10.0 ml of water, and the aqueous phase was extracted with EtOAc (10.0 mL×3). The combined organic layers were washed with aqueous NaCl (10.0 mL), dried over Na₂SO₄, and concentrated to yield compound A18-1 (700 mg, crude) as a white solid.

Compound Int1 (591 mg, 1.67 mmol, 1.00 eq), compound A18-1 (591 mg, 1.67 mmol, 1.00 eq), and NMP (10.0 mL) were added to a 100 mL three-necked flask at 20°C-25°C. CuI (159 mg, 835 µmol, 0.50 eq), DMEDA (147 mg, 1.67 mmol, 179 µL, 1.00 eq), and K₃PO₄ (1.06 g, 5.01 mmol, 3.00 eq) were then added. The mixture was purged with N₂ (×3), heated to 80°C and reacted for 12 h. LCMS indicated 15.9% of compound A18-1 remained (RT = 0.426 min), with the target mass spectrum (25.9%) detected. The product was poured into water and filtered, and the filtrate was concentrated. The crude was then purified by preparative HPLC (column: Welch Xtimate C18 150×25 mm×5 µm; mobile phase: [water (TFA)-ACN]; gradient: 45%-75% B over 10 min). The eluate was lyophilized to afford compound A18-2 (100 mg, 162 µmol, yield 9.71%, purity 85.3%) as a yellow solid.

At 20°C-25°C, compound A18-2 (100 mg, 190 µmol, 1.00 eq) and DCM (4.00 mL) were added to a 100 mL three-necked flask, and TFA (433 mg, 3.81 mmol, 282 µL, 20.0 eq) was added. The mixture was purged with N₂ (×3), and reacted at 25°C for 2 h. LCMS indicated complete consumption of compound A18-2, with the target mass spectrum detected (RT = 0.395 min). After concentration, the crude was purified by thin layer chromatography (SiO₂, petroleum ether : ethyl acetate = 1/1, Rf = 0.2). The eluent was concentrated to afford compound A18 (30.0 mg, 73.6 µmol, yield 38.7%, purity 99.5%) as an off-white solid. LCMS: m/z = 406.1 (M+H)⁺. **¹H NMR:** (400 MHz, DMSO-*d₆*) *δ* 11.8 (s, 1H), 9.01 (s, 1H), 8.51-8.35 (m, 2H), 8.22 (dd, *J* = 1.2, 7.6 Hz, 1H), 8.02 (s, 1H), 7.95 (d, *J =* 8.4 Hz, 2H), 7.76 (s, 1H), 7.49 (d, *J =* 8.4 Hz, 2H), 5.30 (t, *J* = 5.6 Hz, 1H), 4.56 (d, *J* = 5.6 Hz, 2H).

### Preparation Example 19. Synthesis of Compound A19

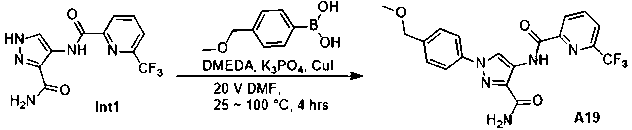

At 25°C, compound Int1 (500 mg, 1.41 mmol, 2.00 eq), 4-(methoxymethoxy)phenylboronic acid (142 mg, 707 µmol, 1.00 eq), DMF (10.0 mL), DMEDA (62.3 mg, 707 µmol, 76.1 µL, 1.00 eq), K₃PO₄ (450 mg, 2.12 mmol, 3.00 eq) and CuI (67.3 mg, 353 µmol, 0.50 eq) were added to the reaction flask. The temperature was raised to 100°C, and the mixture was stirred under a N₂ atmosphere for 4 h. LCMS indicated 49.5% of compound Int1 remained, with the target product mass spectrum detected. The reaction solution was poured into 50.0 ml of water and filtered. The filter cake was concentrated in vacuo, and DMF was added. The mixture was slurried at 100°C for 30 min to obtain a crude. The crude was added to CAN and slurried at 80°C for 30 min to afford compound A19 (45.9 mg, 106 µmol, yield 15.1%, purity 97.2%). LCMS: m/z = 420.1 (M+H)⁺. **¹H NMR:** (400 MHz, DMSO-*d₆*) *δ* 11.8 (s, 1H), 9.06 (s, 1H), 8.46-8.40 (m, 2H), 8.21 (d, J = 7.0 Hz, 1H), 8.03-7.97 (m, 3H), 7.79 (s, 1H), 7.49 (d, J = 7.6 Hz, 2H), 4.47 (s, 2H), 3.34-3.34 (m, 3H).

### Preparation Example 20. Synthesis of Compound A20

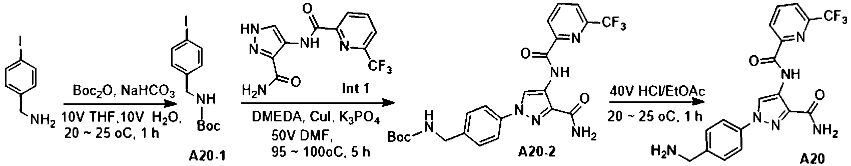

At 20°C-25°C, THF (20.0 mL) was added to a three-necked reaction flask, followed by the addition of (4-iodophenyl)methanamine. NaHCO₃ (2.88 g, 34.3 mmol, 1.34 mL, 4.00 eq) was dissolved in water (20.0 mL) and added to the reaction flask. Boc₂O (2.43 g, 11.2 mmol, 2.56 mL, 1.30 eq) was then added. The reaction solution was stirred at 20°C-25°C for 1 h. LCMS indicated complete consumption of (4-iodophenyl)methanamine, with the target mass spectrum detected. 20.0 ml of water was then added, and the mixture was extracted with EtOAc (×3) (40 ml). The organic phase was washed with 15% aqueous citric acid solution (100 ml), dried over anhydrous Na₂SO₄, filtered, and concentrated to afford compound A20-1 (4.74 g, crude).

At 20°C-25°C, DMF (35.0 mL), compound Int1 (650 mg, 2.00 mmol, 1.00 eq), compound A20-1 (705 mg, 2.00 mmol, 1.00 eq), DMEDA (528 mg, 6.00 mmol, 645 µL, 3.00 eq), CuI (761 mg, 4.00 mmol, 2.00 eq) and K₃PO₄ (1.27 g, 6.00 mmol, 3.00 eq) were sequentially added to a 100 ml three-necked reaction flask and stirred at 95°C-100°C under N₂ for 5 h. LCMS indicated 31.0% of compound A20-1 remained, with the target product mass spectrum detected. 100 ml of water was then added and filtered to obtain a filter cake. The crude was purified by preparative HPLC to afford compound A20-2 (246 mg, 454 µmol, yield 22.7%, purity 93.2%).

HCl/EtOAc (10.0 mL) and compound A20-2 (246 mg, 454 µmol, 1.00 eq) were added to a 100-ml three-necked reaction flask at 20°C-25°C and stirred for 1 h. HPLC indicated complete consumption of compound A20-2, with a main peak detected. The mixture was filtered to afford a filter cake. The filter cake was dissolved in 10 ml of water and the pH was adjusted to 7-8 by adding saturated aqueous NaHCO₃. The mixture was filtered to afford a filter cake, i.e., compound A20. LCMS: m/z = 405.0 (M+H)⁺. **¹H NMR:** (400 MHz, DMSO-*d₆*) *δ* 11.79 (br s, 1H), 9.00 (s, 1H), 8.49-8.36 (m, 2H), 8.21 (br d, *J =* 7.4 Hz, 1H), 8.02-7.87 (m, 3H), 7.75 (br s, 1H), 7.50 (br d, *J =* 8.2 Hz, 2H), 3.78 (s, 2H).

### Preparation Example 21. Synthesis of Compound A21

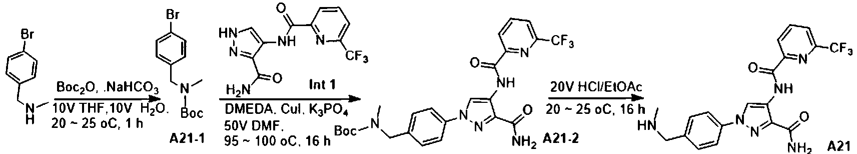

At 20°C-25°C, THF (20.0 mL) and N-methyl-4-bromobenzylamine (2.00 g, 10.0 mmol, 2.00 mL, 1.00 eq) were added to a 100 ml three-necked reaction flask. NaHCO₃ (3.36 g, 39.9 mmol, 1.56 mL, 4.00 eq) was dissolved in water (20.0 mL) and added to the reaction flask. Boc₂O (2.84 g, 13.0 mmol, 2.99 mL, 1.30 eq) was then added to the reaction flask. The mixture was stirred at 20°C-25°C for 1 h. LCMS indicated complete consumption of N-methyl-4-bromobenzylamine, with the target mass spectrum detected. water (20.0 mL) was then added, and the mixture was extracted with EtOAc (×3) (50.0 mL), washed with 15% aqueous citric acid solution (100 mL), dried over anhydrous Na₂SO₄, filtered, and concentrated. After n-heptane (10.0 mL) was added, the mixture was stirred at 20°C-25°C for 2 h and filtered to afford compound A21-1 (3.2 g, crude).

At 20°C-25°C, DMF (35.0 mL), compound Int1 (750 mg, 2.31 mmol, 1.00 eq), compound A21-1 (699 mg, 2.31 mmol, 1.00 eq), DMEDA (406 mg, 4.61 mmol, 496 µL, 2.00 eq), CuI (878 mg, 4.61 mmol, 2.00 eq), and K₃PO₄ (1.47 g, 6.92 mmol, 3.00 eq) were added to a 100 ml three-necked reaction flask. The mixture was stirred at 95°C-100°C under N₂ for 16 h. LCMS indicated 27.3% of compound A21-1 remained, with the target mass spectrum detected. water (100 ml) was added to the reaction, and the mixture was filtered to afford a filter cake. The crude was purified by preparative HPLC to afford compound A21-2.

HCl/EtOAc (10.0 mL) and compound A21-2 (494 mg, 921 µmol, 1.00 eq) were added to a 100 ml three-necked reaction flask at 20°C-25°C and stirred for 16 h. LCMS indicated complete consumption of compound A21-2, with the target mass spectrum detected. The mixture was filtered to afford a filter cake, which was dissolved in water (10.0 ml). The mixture was adjusted to pH 7-8 with saturated aqueous NaHCO₃. The mixture was filtered to afford a filter cake, i.e., compound A21. LCMS: m/z = 419.0 (M+H)⁺. **¹H NMR:** (400 MHz, DMSO-*d₆*) *δ* 11.78 (s, 1H), 9.00 (s, 1H), 8.48-8.36 (m, 2H), 8.21 (d, *J* = 7.4 Hz, 1H), 8.03-7.87 (m, 3H), 7.75 (br s, 1H), 7.48 (br d, *J* = 8.4 Hz, 2H), 3.69 (s, 2H), 2.28 (s, 3H).

### Preparation Example 22. Synthesis of Compound A22

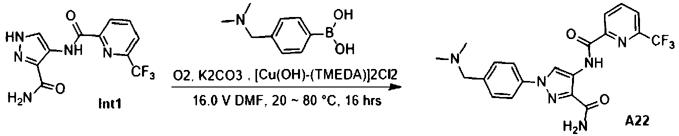

At 20°C-25°C and under O₂, compound Int1 (500 mg, 1.41 mmol, 1.00 eq), DMF (8.00 mL), {4-[(N,N'-dimethyl)methylene]}phenylboronic acid (278 mg, 1.55 mmol, 1.10 eq) and K₂CO₃ (390 mg, 2.82 mmol, 2.00 eq) were added to the reactor, followed by the addition of chloro(hydroxy)copper;N,N,N',N'-tetramethylethylenediamine (196 mg, 423 µmol, 0.30 eq) to the reaction. The mixture was stirred at 80°C for 16 h. LCMS indicated 17.4% of compound Int1 remained, with the target mass spectrum detected. The mixture was concentrated at 50°C-55°C. The crude was purified by column chromatography and then purified by preparative HPLC (TFA conditions) to obtain compound A22. LCMS: m/z = 433.0 (M+H) ⁺. **¹H NMR:** (400 MHz, DMSO-*d₆*) *δ* 11.79 (s, 1H) 9.00 (s, 1H) 8.36-8.49 (m, 2H) 8.21 (d, *J =* 7.6 Hz, 1H) 8.01 (br s, 1H) 7.93 (d, *J* = 8.4 Hz, 2H) 7.76 (br s, 1H) 7.45 (d, *J* = 8.2 Hz, 2H) 3.44 (s, 2H) 2.17 (s, 6H).

### Preparation Example 23. Synthesis of Compound A23

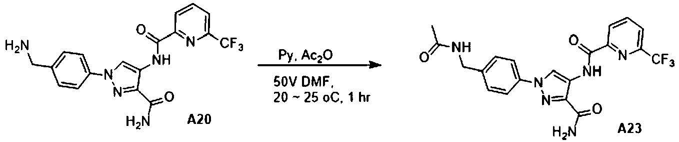

DMF (5.00 mL), compound A20 (100 mg, 233 µmol, 1.00 eq), pyridine (55.4 mg, 701 µmol, 56.5 µL, 3.00 eq), and Ac₂O (35.7 mg, 350 µmol, 32.9 µL, 1.50 eq) were added to a 40.0 ml reaction flask at 20°C-25°C and stirred for 1 h. LCMS indicated complete consumption of compound A20, with the target mass spectrum detected. Saturated aqueous NaHCO₃ was added to adjust the pH to 7-8, and the mixture was filtered to obtain compound A23 (56.5 mg, 121 µmol, 51.9% yield, 95.9% purity). LCMS: m/z = 447.2 (M+H)⁺. **¹H NMR:** (400 MHz, DMSO-*d₆*) *δ* 11.79 (s, 1H), 9.01 (s, 1H), 8.54-8.36 (m, 3H), 8.21 (br d, *J* = 7.6 Hz, 1H), 8.03-7.90 (m, 3H), 7.76 (br s, 1H), 7.42 (br d, *J =* 8.0 Hz, 2H), 4.31 (br d, *J =* 5.2 Hz, 2H), 1.90 (s, 3H).

### Preparation Example 24. Synthesis of Compound A24

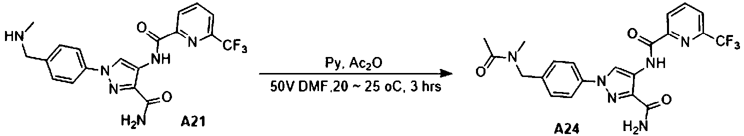

At 20°C-25°C, DMF (5.00 mL), compound A21 (100 mg, 236 µmol, 1.00 eq), pyridine (56.0 mg, 708 µmol, 57.1 µL, 3.00 eq), and Ac₂O (36.1 mg, 354 µmol, 33.2 µL, 1.50 eq) were added to a 40.0 ml reaction flask. After stirring for 3 h, LCMS indicated complete consumption of compound A21, with the target mass spectrum detected. Saturated aqueous NaHCO₃ was added to adjust the pH to 7-8, and the mixture was filtered to obtain compound A24 (96.0 mg, 206 µmol, yield 87.3%, purity 98.9%). LCMS: m/z = 461.1 (M+H)⁺. **¹H NMR:** (400 MHz, DMSO-*d₆*) *δ* 11.79 (s, 1H), 9.04-8.98 (m, 1H), 8.48-8.37 (m, 2H), 8.21 (d, *J =* 7.2 Hz, 1H), 8.04-7.91 (m, 3H), 7.77 (br s, 1H), 7.43-7.35 (m, 2H), 4.64-4.53 (m, 2H), 2.97-2.80 (m, 3H), 2.11-2.06 (m, 3H).

### Preparation Example 25. Synthesis of Compound A25

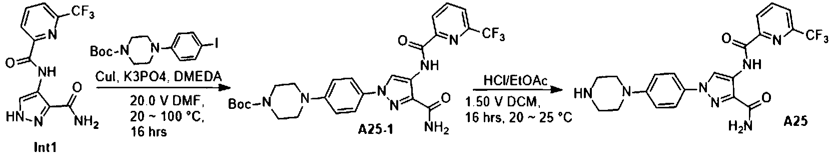

At 20°C-25°C under N₂, compound Int1 (1.00 g, 2.82 mmol, 1.00 eq), DMF (10 mL), tert-butyl 4-(4-iodophenyl)tetrahydro-1(2H)-pyrazinecarboxylate (1.10 g, 2.82 mmol, 1.00 eq), DMEDA (248 mg, 2.82 mmol, 303 µL, 1.00 eq), K₃PO₄ (1.80 g, 8.47 mmol, 3.00 eq) and CuI (268 mg, 1.41 mmol, 0.50 eq) were added to a reaction flask and stirred at 100°C for 16 h. LCMS indicated 14.7% of compound Int 1 remained, with the target mass spectrum detected. The mixture was filtered and concentrated, and the crude was purified by preparative HPLC to yield compound A25-1 (400 mg, 706 µmol, yield 25.0%, purity 98.8%).

Compound A25-1 (400 mg, 706 µmol, 1.00 eq), DCM (0.50 mL), and HCl/EtOAc (4.00 M, 4.94 mL, 28.0 eq) were added to a 10 ml reaction flask at 20°C-25°C and stirred for 16 h. LCMS indicated 63.8% of compound A25-1 remained, with the target mass spectrum detected. The crude was concentrated and dissolved in water (10.0 mL). Saturated aqueous NaHCO₃ was added to adjust the pH to 7-8 and filtered, and the filter cake was washed with water (×3) (4.00 mL). The filter cake was dissolved in water (20.0 mL) and ACN (3.00 mL) and lyophilized to afford compound A25 (285 mg, 620 µmol, yield 87.8%, purity 100%). LCMS: m/z = 460.2 (M+H)⁺. **¹H NMR:** (400 MHz, DMSO-*d₆*) *δ* 11.79 (s, 1H) 8.87 (s, 1H) 8.34-8.52 (m, 2H) 8.14-8.26 (m, 1H) 7.94 (br s, 1H) 7.77 (d, *J* = 9.0 Hz, 2H) 7.70 (s, 1H) 7.05 (d, *J =* 9.2 Hz, 2H) 3.06-3.15 (m, 4H) 2.80-2.89 (m, 4H).

### Preparation Example 26. Synthesis of Compound A26

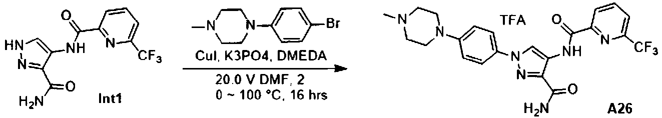

At 20°C-25°C under N₂, compound Int1 (500 mg, 1.41 mmol, 1.00 eq), 1-(4-bromophenyl)-4-methylpiperazine (360 mg, 1.41 mmol, 1.00 eq), DMEDA (124 mg, 1.41 mmol, 151 µL, 1.00 eq) and DMF (10.0 mL) were added to a 100 mL three-necked round-bottom flask, and then CuI (134 mg, 706 µmol, 0.5 eq) and K₃PO₄ (899 mg, 4.24 mmol, 3.00 eq) were added under N₂. The temperature was raised to 100°C, and the reaction was carried out under N₂ for 16 h. LCMS indicated 25.0% of compound Int1 remained, with the target mass spectrum detected (RT=0.343 min). The reaction solution was filtered, and the filtrate was collected and concentrated in vacuo. The crude was purified by reverse-phase high performance liquid chromatography and lyophilized to afford compound A26 (155 mg, 304 µmol, yield 21.5%, purity 93.1%, TFA) as a white solid. LCMS: m/z = 474.2 (M+H)⁺. **¹H NMR:** (400 MHz, DMSO-*d₆*) *δ* 11.79 (s, 1H) 9.72-9.98 (m, 1H) 8.93 (s, 1H) 8.32-8.50 (m, 2H) 8.21 (d, *J =* 7.6 Hz, 1H) 7.95 (br s, 1H) 7.87 (br d, *J* = 8.8 Hz, 2H) 7.73 (br s, 1H) 7.17 (br d, *J =* 9.0 Hz, 2H) 3.95 (br d, *J* = 11.2 Hz, 2H) 3.56-3.60 (m, 2H) 3.18 (br s, 2H) 2.94-3.10 (m, 2H) 2.88 (s, 3H).

### Preparation Example 27. Synthesis of Compound A27

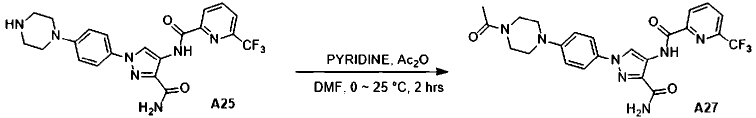

Compound A25 (80.0 mg, 174 µmol, 1.00 eq), pyridine (27.5 mg, 348 µmol, 28.1 µL, 2.00 eq), and DMF (4.00 mL) were added to a 10 mL parallel reaction flask at 20°C-25°C. Ac₂O (26.6 mg, 261 µmol, 24.5 µL, 1.50 eq) was then added at 0°C-5°C. The reaction was allowed to proceed at 20°C-25°C for 2 h. LCMS indicated complete consumption of compound A25, with the target mass spectrum detected (RT = 0.441 min). The crude was dissolved in water (10.0 mL), and the pH was adjusted to 7-8 with saturated NaHCO₃. The reaction solution was filtered and washed with water (4 mL×3). The filter cake was dissolved in water (20.0 mL) and ACN (3.00 mL). The mixture was directly lyophilized to afford compound A27 (73.9 mg, 147 µmol, yield 84.6%, purity 100%) as a white solid. LCMS: m/z = 502.1 (M+H)⁺. **¹H NMR:** (400 MHz, DMSO-*d₆*) *δ* 11.79 (s, 1H) 8.90 (s, 1H) 8.36-8.51 (m, 2H) 8.21 (d, *J =* 7.6 Hz, 1H) 7.95 (br s, 1H) 7.82 (d, *J =* 9.0 Hz, 2H) 7.71 (br s, 1H) 7.11 (d, *J* = 9.2 Hz, 2H) 3.60 (br s, 4H) 3.23-3.29 (m, 2H) 3.15-3.22 (m, 2H) 2.06 (s, 3H).

### Preparation Example 28. Synthesis of Compound A28

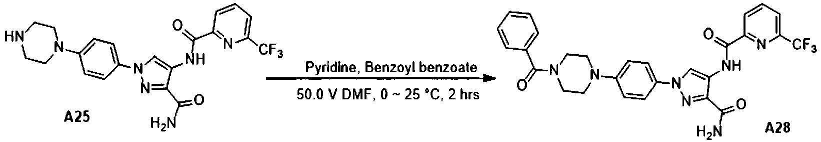

Compound A25 (140 mg, 304 µmol, 1.00 eq), pyridine (48.2 mg, 609 µmol, 49.1 µL, 2.00 eq), and DMF (4.00 mL) were added to a 10 mL parallel reaction flask at 20°C-25°C. Benzoylbenzoate (103 mg, 457 µmol, 86.2 µL, 1.50 eq) was then added at 0°C-5°C, and the mixture was reacted at 20°C-25°C for 2 h. LCMS indicated complete consumption of compound A25, with the target mass spectrum detected (RT = 0.496 min). The crude was dissolved in water (10.0 mL), and the pH was adjusted to 7-8 with saturated NaHCO₃. The mixture was filtered and washed with water (4.00 mL×3). The filter cake was dissolved in water (20.0 mL) and ACN (3.00 mL). The mixture was directly lyophilized to obtain the crude. The crude was stirred with ACN (10.0 mL) at 20°C-25°C for 10 min, and the filter cake was dissolved in water (20.0 mL) and ACN (3.00 mL). The mixture was directly lyophilized to afford compound A28 (74.1 mg, 131 µmol, yield 43.1%, purity 100%) as a white solid. LCMS: m/z = 564.3 (M+H)⁺. **¹H NMR:** (400 MHz, DMSO-*d₆*) *δ* 11.78 (s, 1H) 8.89 (s, 1H) 8.36-8.49 (m, 2H) 8.20 (d, J = 7.6 Hz, 1H) 7.94 (s, 1H) 7.82 (d, J = 9.0 Hz, 2H) 7.70 (s, 1H) 7.38-7.53 (m, 5H)7.11 (d, J = 9.0 Hz, 2H) 3.44-3.86 (m, 4H) 3.31-3.27 (m, 4H).

### Preparation Example 29. Synthesis of compound A29

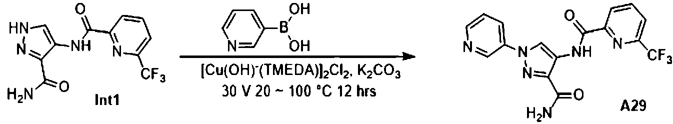

At 20°C-25°C under O₂, compound Int1 (500 mg, 1.41 mmol, 1.00 eq), pyridine-3-boronic acid (190 mg, 1.55 mmol, 1.10 eq), [Cu(OH)-(TMEDA)]₂Cl₂ (196 mg, 423 µmol, 0.30 eq), K₂CO₃ (585 mg, 4.24 mmol, 3.00 eq) and DMF (15.0 mL) were added to a 10 mL parallel reaction flask, heated to 100°C, and reacted under O₂ for 12 h. LCMS indicated 42.8% of compound Int1 remained (RT=1.468 min), with the target mass spectrum detected (RT=1.386 min). The reaction solution was concentrated in vacuo. The crude was purified by reverse-phase high performance liquid chromatography and lyophilized to afford compound A29 (104 mg, 272 µmol, yield 19.2%, purity 97.7%) as a yellow solid. LCMS: m/z = 376.9 (M+H)⁺. **¹H NMR:** (400 MHz, DMSO-*d₆*) *δ* 14.02-13.60 (m, 1H), 11.38 (s, 1H), 11.16 (s, 1H), 9.32 (br s, 1H), 8.72 (br d, *J =* 8.6 Hz, 1H), 8.64-8.52 (m, 2H), 8.50-8.36 (m, 2H), 8.22 (d, *J =* 7.6 Hz, 1H), 7.92 (dd, *J =* 5.4, 8.4 Hz, 1H).

### Preparation Example 30. Synthesis of Compound A30

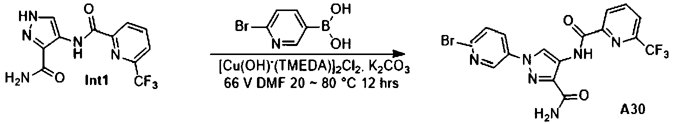

At 20°C-25°C under O₂, compound Int1 (300 mg, 848 µmol, 1.00 eq), 2-bromopyridine-5-boronic acid (188 mg, 933 µmol, 1.10 eq), K₂CO₃ (351 mg, 2.54 mmol, 3.00 eq), [Cu(OH)-(TMEDA)]₂Cl₂ (118 mg, 254 µmol, 0.30 eq) and DMF (20.0 mL) were added to a 40 mL parallel reaction flask, heated to 80°C, and reacted under O₂ for 12 h. LCMS indicated 23.7% of compound Int1 remained (RT = 0.424 min), with the target mass spectrum detected (RT = 0.585 min). The reaction solution was concentrated in vacuo. The crude was purified by reverse-phase high performance liquid chromatography and lyophilized to afford compound A30 (86.0 mg, 179 µmol, yield 21.1%, purity 94.9%) as a yellow solid. LCMS: m/z = 456.9 (M+H)⁺. **¹H NMR:** (400 MHz, DMSO-*d₆*) *δ* 13.68 (s, 1H), 11.44 (s, 1H), 10.74 (s, 1H), 8.90 (d, *J =* 2.4 Hz, 1H), 8.54 (s, 1H), 8.46-8.36 (m, 2H), 8.22 (br d, *J* = 7.8 Hz, 2H), 7.68 (d, *J* = 8.8 Hz, 1H).

### Preparation Example 31. Synthesis of Compound A31

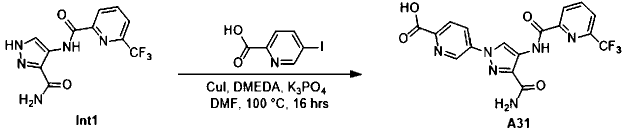

At 20°C-25°C under N₂, compound Int1 (500 mg, 1.54 mmol, 1.00 eq), 5-iodopyridine-2-carboxylic acid (382 mg, 1.54 mmol, 1.00 eq), DMEDA (135 mg, 1.54 mmol, 165 µL, 1.00 eq), K₃PO₄ (979 mg, 4.61 mmol, 3.00 eq), CuI (146 mg, 768 µmol, 0.50 eq) and DMF (10.0 mL) were added to a 40 mL parallel reaction flask, heated to 100°C, and reacted under N₂ for 16 h. LCMS indicated 61% of compound Int1 remained (RT = 0.438 min), with the target mass spectrum detected (RT = 0.391 min). The reaction solution was cooled to 25°C, ACN (50.0 mL) was added, and the mixture was filtered. The filter cake was collected. The filter cake was added to water (50.0 mL), and 2.00 M HCl was added to adjust the pH to 5-6. The mixture was filtered and the filter cake was collected. The filter cake was dissolved in DMF (50.0 mL) and filtered through celite. After filtration and concentration, the mixture was lyophilized to obtain compound A31 (187 mg, 439 µmol, yield 28.5%, purity 98.7%) as an off-white solid. LCMS: m/z = 421.1 (M+H)⁺. **¹H NMR:** (400 MHz, DMSO-*d₆*) *δ* 11.82 (s, 1H), 9.43-9.18 (m, 2H), 8.60 (s, 1H), 8.47-8.39 (m, 2H), 8.23-8.17 (m, 3H), 7.88 (s, 1H).

### Preparation Example 32. Synthesis of Compound A32

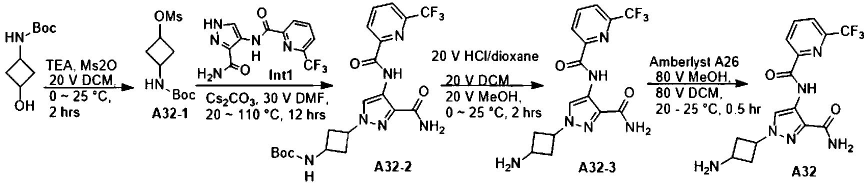

At 0°C-25°C, tert-butyl (3-hydroxycyclobutyl)carbamate (1.00 g, 5.34 mmol, 1.00 eq), TEA (1.62 g, 16.0 mmol, 2.23 mL, 3.00 eq), and DCM (10.0 mL) were added to a 100 mL three-necked round-bottom flask. MS₂O (1.30 g, 7.48 mmol, 1.40 eq) and DCM (10 mL) were slowly added. The mixture was purged with N₂ (×3) and reacted at 0°C-25°C for 2 h. LCMS indicated complete consumption of the compound tert-butyl (3-hydroxycyclobutyl)carbamate, with the target mass spectrum (53.0%) detected. The product was concentrated at 45°C to obtain compound A32-1 (1.42 g, crude) as a pale yellow solid.

Compound A32-1 (532 mg, 2.01 mmol, 1.20 eq), compound Int1 (500 mg, 1.67 mmol, 1.00 eq), Cs₂CO₃ (2.18 g, 6.68 mmol, 4.00 eq), and DMF (15.0 mL) were added to a 100 mL three-necked round-bottom flask at 20°C-25°C. The mixture was heated to 110°C and reacted for 12 h. LCMS indicated 4.25% of compound A32-1 remained, with the target mass spectrum (51.5%) detected (RT=0.609 min). The mixture was poured into water (30.0 mL) and extracted with EtOAc (×3) (60.0 mL). The combined organic phases were washed with saturated aqueous NaCl (30.0 mL), dried over Na₂SO₄, filtered, and concentrated to yield compound A32-2 (1.30 g, crude) as a yellow oil.

Compound A32-2 (170 mg, 363 µmol, 1.00 eq), DCM (3.40 mL), and MeOH (3.40 mL) were added to a 40 mL parallel reaction flask at 20°C-25°C. After cooling to 0°C-5°C, HCl/dioxane (4.00 M, 3.40 mL, 37.5 eq) was added. The temperature was raised to 20°C-25°C, and the mixture was reacted for 2 h. TLC (DCM : MeOH = 10 : 1) showed the formation of a new spot (Rf = 0.30). The mixture was filtered and concentrated to afford compound A32-3 (130 mg, 348 µmol, yield 91.4%, purity 98.7%) as a white solid.

Compound A32-3 (130 mg, 353 µmol, 1.00 eq), DCM (10.0 mL), and MeOH (10.0 mL) were added to a 100 mL three-necked round-bottom flask at 20°C -25°C. Amberlyst A26 (4.00 g, 1.00 eq) was then added, and the mixture was reacted at 20°C-25°C for 0.5 h. TLC (DCM : MeOH = 1 : 1) showed the formation of a new spot (Rf = 0.00). The product was filtered, concentrated, diluted in water (30.0 mL), and lyophilized to afford compound A32 (84.9 mg, 226 µmol, yield 64.1%, purity 98.0%) as a white solid. LCMS: m/z = 368.9 (M+H)⁺. **¹H NMR:** (400 MHz, DMSO-*d₆*) *δ* 11.70 (s, 1H) 8.44 (s, 1H) 8.42-8.36 (m, 2H) 8.18 (d, J = 7.4Hz, 1H) 7.73-7.52 (m, 2H) 5.15-4.95 (m, 1H) 3.73-3.64 (m, 1H) 2.71-2.60 (m, 2H) 2.25-2.15 (m, 2H).

### Preparation Example 33. Synthesis of Compound A33

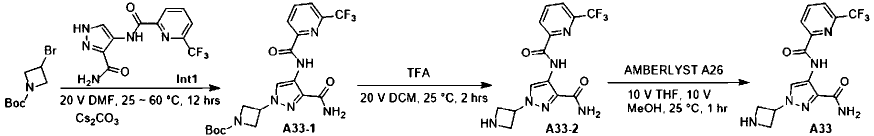

Compound Int1 (500 mg, 1.67 mmol, 1.00 eq), N-BOC-3-bromocyclobutane (473 mg, 2.01 mmol, 1.20 eq), Cs₂CO₃ (1.09 g, 3.34 mmol, 2.00 eq), and DMF (10.0 mL) were added to a 100 mL three-necked round-bottom flask at 20°C-25°C. The mixture was heated to 60°C and reacted for 12 h. LCMS indicated complete consumption of compound Int1, with the target mass spectrum detected (RT=0.456 min). The mixture was poured into water (50.0 mL) and extracted twice with EtOAc (50.0 mL). The combined organic phases were washed twice with saturated aqueous NaCl (50.0 mL), dried over Na₂SO₄ and filtered. The crude was purified by column chromatography and concentrated to yield compound A33-1 (480 mg, 1.01 mmol, yield 60.3%, purity 95.4%) as a white solid.

Compound A33-1 (480 mg, 1.01 mmol, 1.00 eq), DCM (10.0 mL), and TFA (1.72 g, 15.12 mmol, 1.12 mL, 15.0 eq) were added to a 40 mL parallel reaction flask at 20°C-25°C and reacted at 20°C-25°C for 2 h. LCMS indicated complete consumption of compound A33-1, with the target mass spectrum detected (RT=0.289 min). The mixture was filtered and concentrated to afford compound A33-2 (490 mg, 990 µmol, yield 98.2%, purity 94.6%, TFA) as a white solid.

Compound A33-2 (494 mg, 998 µmol, 1.00 eq, TFA), THF (5.00 mL), and MeOH (5.00 mL) were added to a 100 mL three-necked round-bottom flask at 20°C-25°C. AMBERLYST A26 (5.00 g, 30.9 mmol) was then added and reacted at 20°C-25°C for 0.5 h. TLC (DCM : MeOH = 1 : 1) showed the formation of a new spot (Rf=0.10). The mixture was filtered and concentrated to afford compound A33 (201 mg, 544 µmol, yield 54.5%, purity 95.7%) as a bright yellow solid. LCMS: m/z = 354.9 (M+H)⁺. **¹H NMR:** (400 MHz, DMSO-*d₆*) *δ* 11.7 (s, 1H), 8.58 (s, 1H), 8.43-8.35 (m, 2H), 8.19 (d, *J =* 7.4 Hz, 1H), 7.76 (s, 1H), 7.60 (s, 1H), 5.32-5.25 (m, 1H), 3.93 (t, *J =* 7.6 Hz, 2H), 3.81-3.77 (m, 2H), 1.35 (s, 1H).

### Preparation Example 34. Synthesis of Compound A34

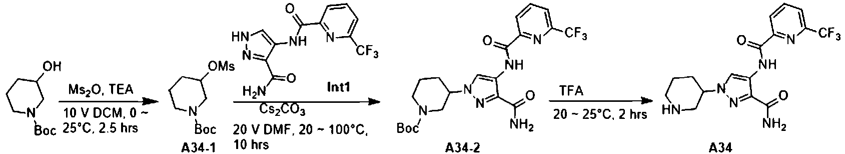

At 20°C-25°C, N-BOC-3-hydroxypiperidine (1.00 g, 4.97 mmol, 1.00 eq), TEA (1.51 g, 14.9 mmol, 2.07 mL, 3.00 eq), and DCM (5.00 mL) were added to a 100 mL three-necked round-bottom flask. Ms₂O (1.13 g, 6.46 mmol, 1.30 eq) dissolved in DCM (5.00 mL) was slowly added, and the reaction was carried out at 0°C-5°C for 2.5 h. LCMS indicated 16.3% of the compound N-BOC-3-hydroxypiperidine remained, with the target mass spectrum detected (RT=0.388 min). The mixture was poured into water (100.0 mL) and extracted with DCM (×3) (30.0 mL). The combined organic phases were washed with saturated aqueous NaCl (90.0 mL), dried over Na₂SO₄, filtered, and concentrated to yield compound A34-1 (1.37 g, 2.40 mmol, yield 48.4%, purity 49.0%) as a yellow oil.

Compound Int1 (856 mg, 2.40 mmol, 1.00 eq), compound A34-1 (1.37 g, 2.40 mmol, 1.00 eq), Cs₂CO₃ (1.57 g, 4.81 mmol, 2.00 eq), and DMF (27.4 mL) were added to a 100 mL three-necked round-bottom flask at 20°C-25°C. The mixture was heated to 100°C and reacted for 10 h. LCMS indicated 29.7% of compound Int1 remained (RT = 0.340 min), with the target mass spectrum detected (RT = 0.466 min). The mixture was poured into water (50.0 mL) and extracted four times with EtOAc (30.0 mL). The combined organic phases were washed with saturated aqueous NaCl (100.0 mL), dried over Na₂SO₄, and filtered. The crude was purified by column chromatography and concentrated to obtain compound A34-2 (532 mg, 1.10 mmol, 45.6%, purity 99.4%) as a white solid.

Compound A34-2 (150 mg, 309 µmol, 1.00 eq) and TFA (1.06 g, 9.27 mmol, 689 µL, 30.0 eq) were added to a 10 mL parallel reaction flask at 20°C-25°C and reacted at 20°C-25°C for 2 h. LCMS indicated complete consumption of compound A34-2, with the target mass spectrum detected (RT=0.305 min). 5.00 g of basic resin was added, and the mixture was reacted at 20°C-25°C for 1 h, filtered and concentrated to afford compound A34 (125 mg, 311 µmol, yield 77.3%, purity 94.9%) as a white solid. LCMS: m/z = 383.1 (M+H)⁺. **¹H NMR:** (400 MHz, DMSO-*d₆*) *δ* 11.75-11.69 (m, 1H) 8.49 (s, 1H) 8.43-8.36 (m, 2H) 8.19 (d, J = 7.4 Hz, 1H) 7.74-7.50 (m, 2H) 4.31-4.19 (m, 1H) 3.23-3.15 (m, 1H) 2.91-2.81 (m, 2H) 2.44-2.32 (m, 1H) 2.20-2.09 (m, 1H) 2.00-1.90 (m, 1H) 1.76-1.69 (m, 1H) 1.59 (m, 1H).

### Preparation Example 35. Synthesis of Compound A35

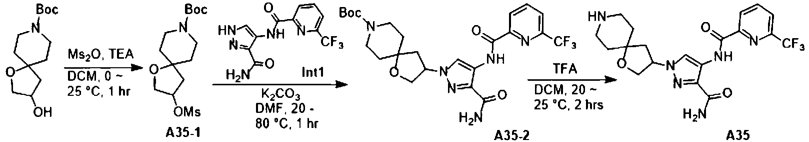

At 20°C-25°C, N-tert-butoxycarbonyl-1-oxa-8-azaspiro[4.5]decan-3-ol (500 mg, 1.94 mmol, 1.00 eq), TEA (589 mg, 5.83 mmol, 811 µL, 3.00 eq), and DCM (2.50 mL) were added to a 100 mL three-necked round-bottom flask. Ms₂O (473 mg, 2.72 mmol, 1.40 eq) dissolved in DCM (2.50 mL) was slowly added, and the mixture was reacted at 0°C-25°C for 1 h. LCMS indicated complete consumption of starting materials, with the target mass spectrum detected (RT = 0.372 min). After concentration, compound A35-1 (500 mg, crude) was obtained as a yellow solid.

At 20°C-25°C, compound Int1 (483 mg, 1.61 mmol, 1.00 eq), compound A35-1 (500 mg, 1.49 mmol, 1.00 eq), K2CO3 (669 mg, 4.84 mmol, 3.00 eq) and DMF (210.0 mL) were added to a 100 mL three-necked round-bottom flask. The mixture was heated to 80°C and reacted for 1 h. LCMS indicated complete consumption of compound Int1, with the target mass spectrum detected (RT = 0.463 min). The mixture was poured into water (25.0 mL) and extracted with EtOAc (×3) (25.0 mL), and the combined organic phases were washed with NaHCO₃ (25.0 mL), dried over Na₂SO₄ and filtered. The crude was purified by preparative HPLC and concentrated to obtain compound A35-2 (400 mg, 736 µmol, yield 45.5%, purity 99.1%) as a yellow solid.

At 20°C-25°C, compound A35-2 (200 mg, 368.05 µmol, 1.00 eq), TFA (839 mg, 7.36 mmol, 546 µL, 20.0 eq), and DCM (2.00 mL) were added to a 10 mL parallel reaction flask. The mixture was purged with N₂ (×3), and reacted at 20°C-25°C for 2 h. LCMS indicated complete consumption of compound A35-2, with the target mass spectrum detected (RT = 0.302 min). The pH was adjusted to 9-10 with NaHCO₃, and the mixture was extracted with DCM (×3) (10.0 mL), dried over Na₂SO₄, filtered, and concentrated to afford compound A35 (85.3 mg, 186 µmol, yield 50.5%, purity 95.6%) as a yellow solid. LCMS: m/z = 439.2 (M+H)⁺. **¹H NMR:** (400 MHz, DMSO-*d₆*) *δ* 11.71 (s, 1H), 8.49 (s, 1H), 8.44-8.34 (m, 2H), 8.19 (dd, J = 1.2, 7.6 Hz, 1H), 7.73-7.55 (m, 2H), 5.24-5.09 (m, 1H), 4.23-4.00 (m, 2H), 2.85 (dd, J = 3.2, 9.2 Hz, 2H), 2.72-2.59 (m, 2H), 2.38 (dd, J = 8.8, 13.2 Hz, 1H), 2.20 (dd, J = 5.6, 13.2 Hz, 1H), 1.66 (t, J = 5.2 Hz, 2H), 1.57 (t, J = 5.2 Hz, 2H).

### Preparation Example 36. Synthesis of Compound A36

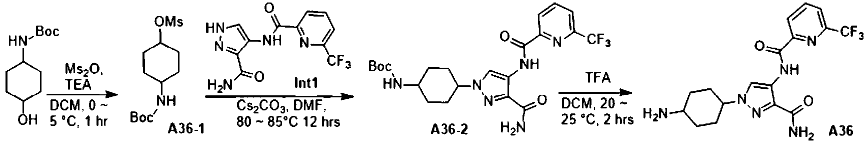

Trans-N-4-BOC-aminocyclohexanol (750 mg, 3.48 mmol, 1.00 eq), TEA (1.06 g, 10.4 mmol, 1.45 mL, 3.00 eq), and DCM (5.00 mL) were added to a 100 mL three-necked round-bottom flask at 0°C-5°C. Ms₂O (910 mg, 5.23 mmol, 1.50 eq) was then added to DCM (5.00 mL) and the mixture was reacted at 0°C-5°C for 1 h. LCMS indicated that 51.2% of trans-N-4-BOC-aminocyclohexanol (RT=0.434 min) remained unreacted, with the target mass spectrum detected (RT=0.515 min). The mixture was poured into water (30.0 mL) and extracted twice with EtOAc (40.0 mL). The combined organic phases were washed with saturated aqueous NaCl (40.0 mL), dried over Na₂SO₄, filtered, and concentrated to yield compound A36-1 (1.06 g, 3.38 mmol, yield 96.9%, purity 93.5%) as a yellow solid.

Compound A36-1 (1.12 g, 3.72 mmol, 1.10 eq), compound Int1 (1.06 g, 3.38 mmol, 1.00 eq), Cs₂CO₃ (3.30 g, 10.1 mmol, 3.00 eq), and DCM (5.00 mL) were added to a 100 mL three-necked round-bottom flask at 20°C-25°C. The mixture was heated to 80°C-85°C and reacted for 12 h. LCMS indicated that 50.1% of compound A36-1 (RT=0.316 min) remained unreacted, with the target mass spectrum detected (RT=0.456 min). The reaction solution was concentrated, and the crude was purified by preparative HPLC and lyophilized to yield compound A36-2 (0.303 g, 604 µmol, yield 17.8%, purity 99.0%) as a white solid.

Compound A36-2 (288 mg, 574 µmol, 1.00 eq), TFA (1.83 g, 16.0 mmol, 1.19 mL, 28.0 eq), and DCM (5.00 mL) were added to a 100 mL three-necked round-bottom flask at 20°C-25°C. The mixture was reacted at 20°C-25°C for 2 h. LCMS indicated complete consumption of compound A36-2, with the target mass spectrum detected (RT = 0.289 min). 10 ml of saturated aqueous NaHCO₃ was added and stirred at 20°C for 10 min. The mixture was extracted twice with EtOAc (20.0 mL). The combined organic phases were washed with saturated aqueous NaCl (15.0 mL), dried over Na₂SO₄, filtered, and concentrated. The crude was purified by preparative HPLC and lyophilized to obtain compound A36 (39.2 mg, 97.7 µmol, yield 17.0%, purity 98.8%) as a white solid. LCMS: m/z = 397.1 (M+H)⁺. **¹H NMR:** (400 MHz, DMSO-*d₆*) *δ* 11.71 (s, 1H), 8.56 (s, 1H), 8.49-8.35 (m, 2H), 8.19 (d, J = 7.4 Hz, 1H), 7.65-7.54 (m, 2H), 4.27-4.21 (m, 1H), 3.05-3.03 (m, 1H), 2.25-2.23 (m, 2H), 1.85-1.76 (m, 3H), 1.64-1.61 (m, 3H).

### Preparation Example 37. Synthesis of Compound A37

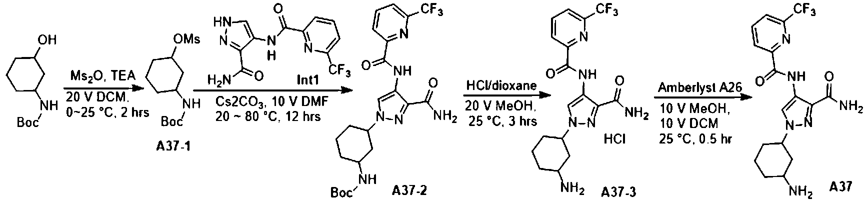

At 25°C under N₂, tert-butyl 3-hydroxycyclohexylcarbamate (650 mg, 3.02 mmol, 1.00 eq), TEA (916 mg, 9.06 mmol, 1.26 mL, 3.00 eq), and DCM (15.0 mL) were added to a 100 mL three-necked round-bottom flask. The temperature was lowered to 0°C-5°C, and Ms₂O (788 mg, 4.53 mmol, 1.50 eq) was added under N₂. LCMS indicated complete consumption of tert-butyl 3-hydroxycyclohexylcarbamate, with the target mass spectrum detected (RT=0.488 min). The mixture was concentrated in vacuo. 100 ml of aqueous solution was added, and the mixture was extracted with EtOAc (200 mL×3). The combined organic phases were washed with saturated aqueous NaCl, dried over Na₂SO₄, and filtered. The reaction solution was concentrated to afford compound A37-1 (890 mg, 2.96 mmol, yield 97.9%, purity 97.5%) as a yellow solid.

Compound Int1 (1.20 g, 3.41 mmol, 1.00 eq), compound A37-1 (1.00 g, 3.41 mmol, 1.00 eq), pyridine (312 mg, 3.95 mmol, 319 µL, 2.00 eq), Cs₂CO₃ (3.33 g, 10.2 mmol, 3.00 eq), and DMF (10.0 mL) were added to a 100 mL three-necked round-bottom flask at 25°C. The mixture was heated to 80°C and reacted for 12 h. LCMS indicated 57.8% of compound A37-1 remained (RT = 0.317 min), with the target mass spectrum (24.57%) detected (RT = 0.454 min). The mixture was concentrated in vacuo, and water (50 mL×3) was added. The mixture was extracted with EtOAc (100 mL×3). The combined organic phases were washed with saturated aqueous NaCl (100 mL×2), dried over Na₂SO₄, and filtered. The reaction solution was concentrated to yield compound A37-2 (1.50 g, crude) as a yellow oil.

Compound A37-2 (100 mg, 199 µmol, 1.00 eq), HCl/dioxane (4.00 M, 1.10 mL, 22.1 eq), and MeOH (2.00 mL) were added to a 100 mL three-necked round-bottom flask at 25°C and reacted at 25°C for 3 h. TLC (petroleum ether : ethyl acetate =0 : 1) indicated complete consumption of compound A37-2 (Rf=0.600), with a new spot detected (Rf=0.200). The reaction solution was concentrated to yield compound A37-3 (87.0 mg, 197 µmol, yield 99.0%, purity 98.0%, HCl) as a white solid.

Compound A37-3 (87.0 mg, 197 µmol, 1.00 eq, HCl) was added to MeOH (10.0 mL) and DCM (10.0 mL) at 25°C. Amberlyst A26 (4.00 g, 197 µmol, 1.00 eq) was then added to a 100 mL three-necked round-bottom flask and reacted at 25°C for 0.5 h. TLC (DCM : MeOH = 1 : 2) indicated complete consumption of compound A37-3 (Rf = 0.100), with a new spot (Rf = 0.12) detected. MeOH (10.0 ml) was then added, and the mixture was filtered at 25°C. The mixture was filtered and concentrated in vacuo at 40°C to obtain a crude. The crude was lyophilized under neutral conditions to afford compound A37 (61.2 mg, 154 µmol, yield 78.4%, purity 100%) as an off-white solid. LCMS: m/z = 397.0 (M+H)⁺. **¹H NMR:** (400 MHz, DMSO-*d₆*) *δ* 11.71 (s, 1H), 8.41 (s, 3H), 8.18 (d, J = 6.4 Hz, 1H), 7.64-7.53 (m, 2H), 4.79-4.19 (m, 1H), 2.71-2.66 (m, 1H), 2.18-1.95 (m, 2H), 1.93-1.75 (m, 2H), 1.56-1.49 (m, 4H).

### Preparation Example 38. Synthesis of Compound A38

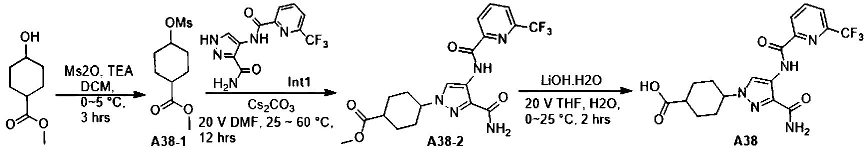

Methyl 4-hydroxycyclohexanecarboxylate (1.00 g, 6.32 mmol, 1.00 eq), TEA (959 mg, 9.48 mmol, 1.32 mL, 1.50 eq), and DCM (10.0 mL) were added to a 100 mL three-necked round-bottom flask at 20°C-25°C. Ms₂O (1.43 g, 8.22 mmol, 1.30 eq) dissolved in DCM (10.0 mL) was slowly added and the mixture was reacted at 0°C for 3 h. TLC (petroleum ether : ethyl acetate=1 : 1) indicated complete consumption of methyl 4-hydroxycyclohexanecarboxylate (Rf=0.40), with the formation of a new spot (Rf=0.50). The mixture was concentrated to yield compound A38-1 (1.30 g, crude) as a yellow oil.

Compound Int1 (1.00 g, 2.83 mmol, 1.00 eq), Cs₂CO₃ (1.84 g, 5.65 mmol, 2.00 eq), A38-1 (802 mg, 3.39 mmol, 1.20 eq), and DMF (15.0 mL) were added to a 100 mL three-necked round-bottom flask at 20°C-25°C. The temperature was raised to 80°C and the reaction was carried out for 12 h. LCMS indicated 21.8% of compound A38-1 remained, with the target mass spectrum detected (RT=0.427 min). The mixture was poured into water (50.0 mL) and extracted twice with EtOAc (50.0 mL). The solution was washed with aqueous NaCl (×3) (50.0 mL), dried over Na₂SO₄ and filtered. The crude was purified by silica gel column chromatography and concentrated to yield compound A38-2 (680 mg, 1.42 mmol, yield 50.3%, purity 91.8%) as a white solid.

Compound A38-2 (160 mg, 364.14 µmol, 1.00 eq), THF (2.40 mL), and water (0.80 mL) were added to a 100 mL three-necked round-bottom flask at 20°C-25°C. The mixture was cooled to 0°C, and LiOH water (15.3 mg, 364 µmol, 1.00 eq) and THF (0.30 mL) were added. The mixture was reacted at 0°C for 2 h. LCMS indicated complete consumption of compound A38-2, with the target mass spectrum detected (RT = 0.376 min). The mixture was poured into water (20.0 mL), adjusted to pH 4-5, and extracted twice with EtOAc (20.0 mL). The solution was washed twice with aqueous NaCl (20.0 mL), dried over Na₂SO₄ and filtered. The crude was purified by preparative HPLC and concentrated to obtain compound A38 (46.3 mg, 104 µmol, yield 28.5%, purity 95.3%) as a white solid. LCMS: m/z = 426.1 (M+H)⁺. **¹H NMR:** (400 MHz, DMSO-*d₆*) *δ* 11.7 (s, 1H), 8.42-8.35 (m, 3H), 8.18 (d, *J* = 7.2 Hz, 1H), 7.64 (s, 1H), 7.52 (s, 1H), 4.35-4.31 (m, 1H), 2.60 (d, *J =* 4.4 Hz, 1H), 2.08-1.95 (m, 7H), 1.70-1.63 (m, 2H).

### Preparation Example 39. Synthesis of Compound A39

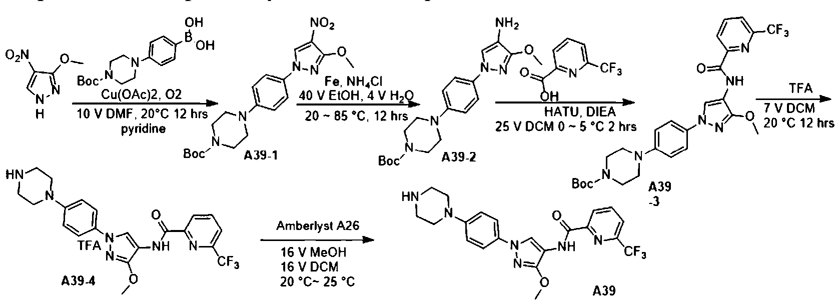

3-Methoxy-4-nitro-1H-pyrazole (1 g, 6.99 mmol, 1.00 eq), 4-(4-BOC-1-piperazinyl)phenylboronic acid (2.35 g, 7.69 mmol, 1.10 eq), pyridine (1.66 g, 20.9 mmol, 1.69 mL, 3.00 eq), and Cu(OAc)₂ (152 mg, 838 µmol, 0.12 eq) were added to a 10 mL single-necked flask at 20°C-25°C. DMF (10.0 mL) was then added, and the mixture was purged with O₂ (×3). The reaction was allowed to proceed at 20°C-25°C for 12 h. LCMS indicated 57.3% of 3-methoxy-4-nitro-1H-pyrazole remained, with the target mass spectrum detected. The reaction solution was directly concentrated and slurried with EtOAc to obtain compound A39-1 (1.05 g, 2.59 mmol, yield 37.0%, purity 99.5%) as a yellow solid.

Compound A39-1 (500 mg, 1.23 mmol, 1.00 eq), NH4C1 (329 mg, 6.17 mmol, 5.00 eq), EtOH (20.0 mL), and water (2.00 mL) were added to a 100 mL single-necked flask at 20°C-25°C. The temperature was raised to 50°C and the reaction was allowed to proceed for 0.5 h. Fe (206 mg, 3.70 mmol, 3.00 eq) was then added, and the mixture was reacted at 85°C for 12 h. LCMS indicated complete consumption of compound A39-1, with the target mass spectrum detected. The reaction solution was cooled to 25°C, and Celite was added. The mixture was stirred for 10 min, and filtered. The filtrate was added with water, extracted with EtOAc, dried over anhydrous Na₂SO₄, and concentrated to yield compound A39-2 (510 mg, 1.07 mmol, yield 87.0%, purity 78.6%) as a black solid.

6-(Trifluoromethyl)pyridine-2-carboxylic acid (193 mg, 1.01 mmol, 1.20 eq), HATU (384 mg, 1.01 mmol, 1.20 eq), DIPEA (217 mg, 1.68 mmol, 293 µL, 2.00 eq), and DCM (10.0 mL) were added to a 100 mL single-necked flask at 20°C-25°C and reacted at 0°C-5°C for 0.5 h. Compound A39-2 (400 mg, 841 µmol, 1.00 eq) was then added, and the mixture was reacted at 0°C-5°C for 1 h. LCMS indicated 7.76% of compound A39-2 remained, with the target mass spectrum detected. The reaction solution was concentrated, and the crude was purified by preparative HPLC to yield compound A39-3 (288 mg, 310 µmol, yield 36.8%, purity 58.9%) as a black solid.

Compound A39-3 (288 mg, 310 µmol, 1.00 eq), TFA (990 mg, 8.69 mmol, 645 µL, 28.0 eq), and DCM (2.00 mL) were added to a 100 mL single-necked flask at 20°C-25°C and reacted at 20°C for 12 h. LCMS indicated complete consumption of compound A39-3, with the target mass spectrum detected. The reaction solution was concentrated to yield compound A39-4 (180 mg, crude) as a yellow solid.

Compound A39-4 (180 mg, 403 µmol, 1.00 eq), MeOH (3.00 mL), and DCM (3.0 mL) were added to a 50 mL single-necked flask at 20°C-25°C. Amberlyst A26 (1.00 g, 403 µmol, 1.00 eq) was then added and reacted at 20°C-25°C for 0.5 h. A spot plate showed residual compound A39-4, and new spots were detected. The reaction solution was filtered, concentrated, and lyophilized to afford compound A39 (120 mg, 267 µmol, yield 66.2%, purity 98.7%) as a yellow solid. LCMS: m/z = 447 (M+H)⁺. **¹H NMR:** (400 MHz, DMSO-*d₆*) *δ* 9.72 (s, 1H), 8.56-8.46 (m, 1H), 8.42-8.30 (m, 2H), 8.18 (dd, *J =* 2.2*,* 6.6 Hz, 1H), 7.68-7.46 (m, 2H), 7.12-6.94 (m, 2H), 3.98 (s, 3H), 3.16 (br d, *J =* 4.6 Hz, 1H), 3.06-3.02 (m, 3H), 2.86-2.80 (m, 3H), 2.62 (br d, *J* = 3.2 Hz, 1H).

### Preparation Example 40. Synthesis of Compound A40

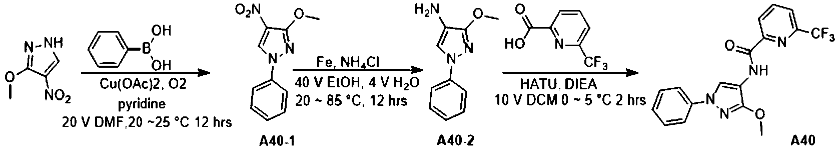

3-Methoxy-4-nitro-1H-pyrazole (1.00 g, 6.99 mmol, 1.00 eq), phenylboronic acid (937 mg, 7.69 mmol, 1.10 eq), pyridine (1.66 g, 20.9 mmol, 1.69 mL, 3.00 eq), and DMF (17.0 mL) were added to a 100 mL three-necked round-bottom flask at 20°C-25°C. Cu(OAc)₂ (152 mg, 838 µmol, 0.12 eq) was then added. The mixture was purged with N₂ (×3), heated to 25°C and reacted for 12 h. LCMS indicated 11.4% of 3-methoxy-4-nitro-1H-pyrazole remained, with the target mass spectrum detected. The reaction mixture was directly concentrated, and the crude was purified by preparative HPLC and lyophilized to yield compound A40-1 (800 mg, 3.65 mmol, yield 52.2%, purity 100%) as a yellow solid.

Compound A40-1 (500 mg, 2.28 mmol, 1.00 eq), EtOH (20.0 mL), and water (2.00 mL) were added to a 100 mL three-necked round-bottom flask at 20°C-25°C. NH₄Cl (610 mg, 11.4 mmol, 5.00 eq) was then added, and the mixture was heated to 50°C and reacted for 0.5 h. Fe (382 mg, 6.84 mmol, 3.00 eq) was then added, and the mixture was heated to 85°C and reacted for 12 h. LCMS indicated complete consumption of compound A40-1, with the target mass spectrum detected. The reaction was cooled to 25°C, and Celite was then added. The mixture was stirred for 10 min and filtered. The filtrate was poured into water, extracted with EtOAc, dried over anhydrous Na₂SO₄, and concentrated to yield compound A40-2 (510 mg, 1.92 mmol, yield 84.0%, purity 71.1%) as a black solid.

6-(Trifluoromethyl)pyridine-2-carboxylic acid (344 mg, 1.80 mmol, 1.20 eq), HATU (628 mg, 1.65 mmol, 1.10 eq), DIPEA (388 mg, 3.01 mmol, 523 µL, 2.00 eq), and DCM (10.0 mL) were added to a 100 mL three-necked round-bottom flask at 0°C-5°C. Compound A40-2 (400 mg, 1.50 mmol, 1.00 eq) was then added and reacted at 0°C-5°C for 2 h. The target mass spectrum was detected by LCMS. The reaction solution was directly concentrated, and the crude was purified by preparative HPLC and lyophilized to obtain compound A40 (182 mg, 495 µmol, yield 33.0%, purity 98.6%) as a yellow solid. LCMS: m/z = 363 (M+H)⁺. **¹H NMR:** (400 MHz, DMSO-*d₆*) *δ* 9.76 (s, 1H), 8.68 (s, 1H), 8.42-8.32 (m, 2H), 8.18 (dd, *J =* 1.8, 6.8 Hz, 1H), 7.76 (d, *J* = 7.8 Hz, 2H), 7.46 (dd, *J* = 7.6, 8.4 Hz, 2H), 7.26-7.18 (m, 1H), 4.02 (s, 3H).

### Preparation Example 41. Synthesis of Compound A41

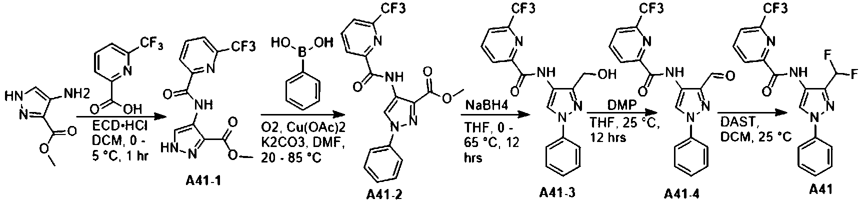

At 20°C-25°C, methyl 4-amino-1H-pyrazole-3-carboxylate (8.50 g, 60.2 mmol, 1.00 eq) and DCM (130 mL) were added to a 250 mL three-necked round-bottom flask. The temperature was lowered to 0°C-5°C, followed by the addition of 6-(trifluoromethyl)pyridine-2-carboxylic acid (11.7 g, 61.4 mmol, 1.02 eq) and ECD•HCl (13.8 g, 72.2 mmol, 1.20 eq). The mixture was reacted at 0°C for 1 h. LCMS indicated complete consumption of methyl 4-amino-1H-pyrazole-3-carboxylate, with the target mass spectrum detected. The reaction solution was concentrated, and water was added. The mixture was extracted with EtOAc, dried over anhydrous Na₂SO₄, and concentrated to yield compound A41-1 (18.3 g, 57.3 mmol, yield 95.1%, purity 98.4%) as a black solid.

At 20°C-25°C, compound A41-1 (5.00 g, 15.6 mmol, 1.00 eq), phenylboronic acid (1.91 g, 15.6 mmol, 1 eq) and DMF (50.0 mL) were added to a 250 mL three-necked round-bottom flask, and Cu(OAc)₂ (341 mg, 1.88 mmol, 0.12 eq) and K₂CO₃ (4.33 g, 31.3 mmol, 2.00 eq) were added. The mixture was purged with O₂ (×3), heated to 85°C and reacted for 12 h. LCMS indicated 2.33% of compound A41-1 remained, with the target mass spectrum detected. The reaction solution was cooled to 20°C-25°C and filtered through Celite. The filter cake was washed with EtOAc, and water was added to the filtrate. The mixture was extracted with EtOAc, dried over anhydrous Na₂SO₄, and concentrated. The crude was purified by preparative HPLC to yield compound A41-2 (2.00 g, 5.01 mmol, yield 31.9%, purity 97.7%) as a yellow solid.

Compound A41-2 (1.00 g, 2.50 mmol, 1.00 eq) and THF (15.0 mL) were added to a 100 mL three-necked round-bottom flask at 20°C-25°C. The temperature was lowered to 0°C-5°C, and the mixture was purged with N₂ (×3). NaBH₄ (1.23 g, 32.5 mmol, 13.0 eq) was then added, and the mixture was reacted at 65°C for 12 h. LCMS indicated compound A41-2 remained, with the target mass spectrum detected. The reaction solution was poured into aqueous NH₄Cl, extracted with EtOAc, washed with aqueous NaCl, dried over anhydrous Na₂SO₄, and concentrated. The crude was purified by column chromatography to obtain compound A41-3 (400 mg, 1.10 mmol, yield 43.8%, purity 99.4%) as a white solid.

Compound A41-3 (338 mg, 927 µmol, 1.00 eq) and DCM (5.00 mL) were added to a 100 mL three-necked round-bottom flask at 20°C-25°C. DMP (786 mg, 1.85 mmol, 574 µL, 2.00 eq) was then added and reacted at 25°C for 12 h. LCMS indicated complete consumption of compound A41-3, with the target mass spectrum detected. The reaction solution was poured into aqueous Na₂S₂O₃, extracted with DCM, washed with NaHCO₃ and aqueous NaCl, dried over anhydrous Na₂SO₄, and concentrated to obtain compound A41-3 (400 mg, 1.10 mmol, yield 43.8%, purity 99.4%) as a white solid.

At 20°C-25°C, compound A41-3 (325 mg, 733 µmol, 1.00 eq) and DCM (10 mL) were added to a 100 mL three-necked round-bottom flask, and DAST (2.36 g, 14.6 mmol, 1.94 mL, 20.0 eq) was added and reacted at 25°C for 12 h. LCMS indicated complete consumption of compound A41-3, with the target mass spectrum detected. The reaction solution was poured into an aqueous NaHCO₃, extracted with DCM, washed with NaHCO₃ and aqueous NaCl, dried over anhydrous Na₂SO₄, and concentrated. The resultant was slurried with methyl tert-butyl ether to obtain compound A41 (96.9 mg, 247 µmol, yield 33.7%, purity 97.6%) as a white solid. LCMS: m/z = 383.1 (M+H)⁺. **¹H NMR:** (400 MHz, DMSO-*d₆*) *δ* 10.30 (s, 1H), 8.94 (s, 1H), 8.52-8.36 (m, 2H), 8.23 (d, *J =* 7.6 Hz, 1H), 7.89 (d, *J* = 8.0 Hz, 2H), 7.56 (t*, J =* 8.0 Hz, 2H), 7.47-7.17 (m, 2H).

### Preparation Example 42. Synthesis of Compound A42

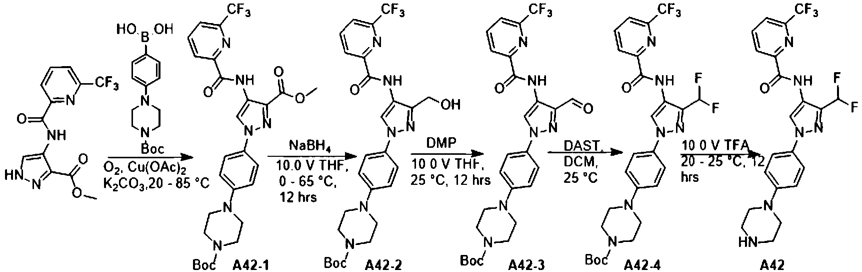

At 20°C-25°C, compound A41-1 (5.00 g, 15.6 mmol, 1.00 eq), 4-(4-BOC-1-piperazinyl)phenylboronic acid (4.79 g, 15.6 mmol, 1.00 eq) and DMF (50.0 mL) were added to a 500 mL three-necked round-bottom flask, and Cu(OAc)₂ (341.27 mg, 1.88 mmol, 0.12 eq) and K₂CO₃ (4.33 g, 31.3 mmol, 2.00 eq) were added and reacted at 85°C for 12 h. LCMS indicated 9.90% of compound A41-1 remained, with the target mass spectrum detected. The reaction solution was poured into water, extracted with EtOAc, washed with NaHCO₃ and aqueous NaCl, dried over anhydrous Na₂SO₄, and concentrated. The crude was purified by preparative HPLC to yield compound A42-1 (1.30 g, 2.26 mmol, yield 14.4%, purity 100%) as a white solid.

Compound A42-1 (1.00 g, 1.74 mmol, 1.00 eq) and THF (15.0 mL) were added to a 100 mL three-necked round-bottom flask at 20°C-25°C. The temperature was lowered to 0°C-5°C, and the mixture was purged with N₂ (×3). NaBH₄ (856 mg, 22.6 mmol, 13.0 eq) was then added, and the mixture was reacted at 65°C for 12 h. LCMS indicated complete consumption of compound A42-1, with the target mass spectrum detected. The reaction solution was poured into aqueous NH₄Cl, extracted with EtOAc, washed with aqueous NaCl, dried over anhydrous Na₂SO₄, and concentrated. The crude was separated by column chromatography to obtain compound A42-2 (400 mg, 726 µmol, yield 41.7%, purity 99.3%) as a white solid.

Compound A42-2 (334 mg, 606 µmol, 1.00 eq) and DCM (1.00 mL) were added to a 100 mL three-necked flask at 25°C. The mixture was purged with N₂ (×3), and DMP (514 mg, 1.21 mmol, 376 µL, 2.00 eq) was added. The reaction was allowed to proceed at 25°C for 12 h. LCMS indicated complete consumption of compound A42-2, with the target mass spectrum detected (RT = 0.553 min). Saturated ammonium chloride (20.0 mL) and saturated NaHCO₃ (20.0 mL) were added, and the mixture was extracted with EtOAc (20.0 mL×3). The organic phase was washed with saturated aqueous NaCl (20.0 mL), dried over Na₂SO₄, filtered, and concentrated to yield compound A42-3 (400 mg, 539 µmol, yield 88.9%, purity 73.5%) as a yellow solid.

Compound A42-3 (430 mg, 580 µmol, 1.00 eq) and DCM (10 mL) were added to a 100 mL three-necked round-bottom flask at 20°C-25°C. DAST (1.87 g, 11.6 mmol, 1.53 mL, 20.0 eq) was then added and reacted at 25°C for 12 h. LCMS indicated complete consumption of compound A42-3, with the target mass spectrum detected. The reaction solution was poured into aqueous NaHCO₃, extracted with DCM, washed with NaHCO₃ and aqueous NaCl, dried over anhydrous Na₂SO₄, and concentrated. The resultant was slurried with methyl tert-butyl ether to obtain compound A42-4 (65.0 mg, 114 µmol, yield 21.2%, purity 100%) as a white solid.

Compound A42-4 (65.0 mg, 114 µmol, 1.00 eq) and DCM (2 mL) were added to a 100 mL three-necked round-bottom flask at 20°C-25°C. TFA (261 mg, 2.29 mmol, 170 µL, 20.0 eq) was then added and reacted at 25°C for 12 h. LCMS indicated complete consumption of compound A42-4, with the target mass spectrum detected. The reaction solution was poured into aqueous NaHCO₃, stirred for 10 min, filtered, and concentrated to obtain compound A42 (65.0 mg, 114 µmol, yield 21.2%, purity 100%) as a white solid. LCMS: m/z = 467.1 (M+H)⁺. **¹H NMR:** (400 MHz, DMSO-*d₆*) *δ* 10.26 (s, 1H), 8.78 (s, 1H), 8.46-8.37 (m, 2H), 8.23 (dd, *J =* 1.2*,* 7.6 Hz, 1H), 7.68 (d, *J =* 9.2 Hz, 2H), 7.29 (t, *J* = 54.0 Hz, 1H), 7.05 (d, *J =* 9.2 Hz, 2H), 3.14-3.07 (m, 4H), 2.87-2.81 (m, 4H).

### Preparation Example 43. Synthesis of Compound A43

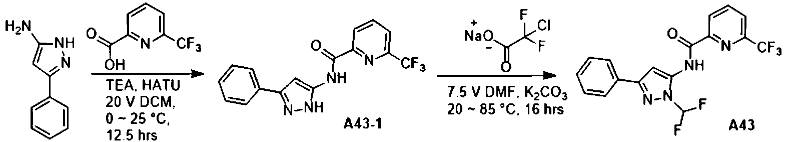

At 20°C-25°C, DCM (40.0 mL), 6-(trifluoromethyl)pyridine-2-carboxylic acid (2.40 g, 12.5 mmol, 1.00 eq), TEA (7.63 g, 75.3 mmol, 10.4 mL, 6.00 eq), and HATU (4.78 g, 12.5 mmol, 1.00 eq) were added to a 100 mL three-necked round-bottom flask and reacted at 0°C-5°C for 0.5 h. 3-phenyl-1H-pyrazol-5-amine (2.00 g, 12.5 mmol, 1.00 eq) was added, and the mixture was reacted at 20°C-25°C for 12 h. LCMS indicated complete consumption of 3-phenyl-1H-pyrazol-5-amine, with the target mass spectrum detected. The mixture was filtered and slurried with DCM to yield compound A43-1 (3.10 g, 9.09 mmol, yield 72.3%, purity 97.4%) as a white solid.

Compound A43-1 (200 mg, 586 µmol, 1.00 eq) and DMF (1.50 mL) were added to a 100 mL three-necked round-bottom flask at 20°C-25°C. Sodium difluorochloroacetate (268 mg, 1.76 mmol, 3.00 eq) and K₂CO₃ (243 mg, 1.76 mmol, 3.00 eq) were then added and reacted at 80°C-85°C for 16 h. LCMS indicated approximately 6.2% of compound A43-1 remained, with the target mass spectrum detected. The reaction solution was poured into water, extracted with EtOAc, dried over anhydrous Na₂SO₄, and concentrated. The crude was purified by preparative HPLC to afford compound A43 (146 mg, 392 µmol, yield 66.9%, purity 100%) as a white solid. LCMS: m/z = 383.1 (M+H)⁺. **¹H NMR:** (400 MHz, DMSO-*d₆*) *δ* 10.94 (s, 1H) 8.42-8.39 (m, 2H) 7.90 (d, J = 6.8 Hz, 1H) 7.78 (t, J = 42.4 Hz, 1H) 7.48-7.42 (m, 3H) 7.03 (s, 1H).

### Preparation Example 44. Synthesis of Compound A44

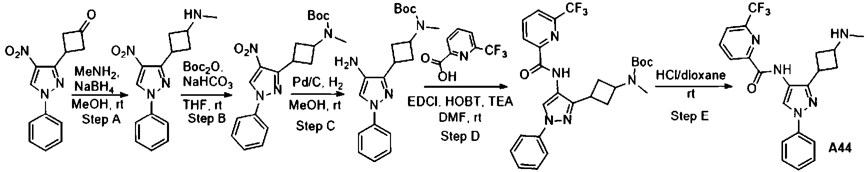

### Step A: N-Methyl-3-(4-nitro-1-phenyl-1H-pyrazol-3-yl)cyclobutan-1-amine

At rt, 3-(4-nitro-1-phenyl-1H-pyrazol-3-yl)cyclobutan-1-one (200 mg, 0.8 mmol, 1.0 eq) was stirred in a solution of methylamine/MeOH (20 mL) for 15 h. Sodium borohydride (58.8 mg, 1.6 mmol, 2.0 eq) was then added. The reaction solution was stirred at 25°C under N₂ for 1 h. Upon completion, the reaction solution was quenched with water and extracted with EtOAc (×3). The combined organic phases were washed sequentially with water and saturated aqueous NaCl, dried over anhydrous Na₂SO₄, filtered, and concentrated under reduced pressure to afford the title compound (yellow solid, 218 mg, crude), which was used directly without purification.

### Step B: Tert-butyl methyl(3-(4-nitro-1-phenyl-1H-pyrazol-3-yl)cyclobutyl)carbamate

At rt, to a solution of N-methyl-3-(4-nitro-1-phenyl-1H-pyrazol-3-yl)cyclobutan-1-amine (218 mg, 0.8 mmol, 1.0 eq) in tetrahydrofuran/water (2 : 1, 30 mL) were added NaHCO₃ (160 mg, 1.9 mmol, 2.4 eq) and di-tert-butyl dicarbonate (218.6 mg, 1.0 mmol, 1.25 eq). The reaction was stirred at rt for 3 h. Upon completion, the reaction solution was quenched with water and extracted with EtOAc (×3). The combined organic phases were washed twice with saturated aqueous NaCl, dried over anhydrous Na₂SO₄, filtered, and concentrated under reduced pressure. The residue was purified by column chromatography to afford the title compound (yellow solid, 230 mg, yield 65%).

### Step C: Tert-butyl (3-(4-amino-1-phenyl-1H-pyrazol-3-yl)cyclobutyl)(methyl)carbamate

At rt, to a solution of tert-butyl methyl(3-(4-nitro-1-phenyl-1H-pyrazol-3-yl)cyclobutyl)carbamate (230 mg, 0.6 mmol, 1.0 eq) in MeOH (25 mL) was added 10% palladium on carbon (32.9 mg, 0.3 mmol) was added. The mixture was purged with H₂ (using a hydrogen balloon) (×3) and stirred under H₂ at rt for 2 h. Upon completion, the mixture was filtered, and the filtrate was concentrated under reduced pressure to afford the title compound (yellow oil, 170 mg, crude), which was used directly without purification.

### Step D: Tert-butyl methyl(3-(1-phenyl-4-(6-(trifluoromethyl)picolinamido)-1H-pyrazol-3-yl)cyclobutyl)carbamate

At rt, to a solution of tert-butyl (3-(4-amino-1-phenyl-1H-pyrazol-3-yl)cyclobutyl)(methyl)carbamate (170 mg, 0.5 mmol, 1.0 eq) and 6-(trifluoromethyl)pyridine-2-carboxylic acid (95 mg, 0.5 mmol, 1.0 eq) in DMF (15 mL) were added EDCI (142.9 mg, 0.7 mmol, 1.5 eq), HOBt (100.8 mg, 0.7 mmol, 1.5 eq), and TEA (0.2 mL, 1.5 mmol, 3.0 eq). The reaction solution was stirred at rt under N₂ for 1 h. Upon completion, the reaction solution was diluted in EtOAc, washed sequentially with water and saturated aqueous NaCl, dried over anhydrous Na₂SO₄, filtered, and concentrated under reduced pressure. The residue was purified by column chromatography to afford the title compound (pale yellow solid, 256 mg, crude).

### Step E: N-(3-(3-(Methylamino)cyclobutyl)-1-phenyl-1H-pyrazol-4-yl)-6-(trifluoromethyl)picolinamide

At rt, tert-butyl methyl(3-(1-phenyl-4-(6-(trifluoromethyl)picolinamido)-1H-pyrazol-3-yl)cyclobutyl)carbamate (256 mg, 0.6 mmol, 1.0 eq) was stirred in a solution of HCl/1,4-dioxane (4 N, 10 mL) for 1 h. Upon completion, the reaction solution was concentrated under reduced pressure. The residue was purified by HPLC to afford the title compound (pale yellow solid, 9.6 mg, yield 4%). **¹H NMR** (400 MHz, DMSO-*d*₆) δ 8.68 (s, 1H), 8.43-8.34 (m, 2H), 8.31 (s, 1H), 8.24-8.13 (m, 1H), 7.82 (d, *J =* 7.8 Hz, 2H), 7.56-7.45 (m, 2H), 7.36-7.25 (m, 1H), 2.69-2.59 (m, 2H), 2.29 (s, 3H), 2.22-2.11 (m, 2H), 2.09-1.92 (m, 2H). LCMS (ESI): m/z = 416.3 [M+H]⁺.

### Preparation Example 45. Synthesis of Compound A45

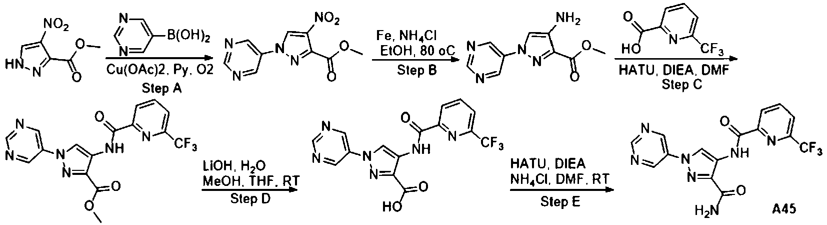

### Step A: Methyl 4-nitro-1-(pyrimidin-5-yl)-1H-pyrazole-3-carboxylate

At rt, pyrimidin-5-ylboronic acid (1.09 g, 8.77 mmol, 1.5 eq), copper acetate (1.17 g, 5.84 mmol, 1.0 eq), and pyridine (1.89 mL, 23.4 mmol, 4.0 eq) were added to a solution of methyl 4-nitro-1H-pyrazole-3-carboxylate (1.00 g, 5.84 mmol, 1.0 eq) in DCM (20 mL). The reaction solution was stirred under O₂ at rt for 18 h. Upon completion, the reaction solution was filtered, and the filtrate was diluted in water and extracted with DCM (×3). The combined organic phases were washed with water and saturated aqueous NaCl, dried over anhydrous Na₂SO₄, and concentrated under reduced pressure. The residue was purified by column chromatography to afford the title compound (white solid, 580 mg, yield 40%).

### Step B: Methyl 4-amino-1-(pyrimidin-5-yl)-1H-pyrazole-3-carboxylate

At rt, iron powder (313 mg, 5.62 mmol, 5.0 eq) and ammonium chloride (60.1 mg, 1.12 mmol, 1.0 eq) were added to a solution of methyl 4-nitro-1-(pyrimidin-5-yl)-1H-pyrazole-3-carboxylate (280 mg, 1.12 mmol, 1.0 eq) in ethanol (9 mL) and water (3 mL). The mixture was stirred at 80°C for 1 h. Upon completion, the reaction mixture was filtered, and the filtrate was concentrated under reduced pressure to afford the title compound (yellow solid, 190 mg, yield 77%, crude), which was used directly without purification.

### Step C: Methyl 1-(pyrimidin-5-yl)-4-(6-(trifluoromethyl)picolinamido)-1H-pyrazole-3-carboxylate

At rt, to a solution of methyl 4-amino-1-(pyrimidin-5-yl)-1H-pyrazole-3-carboxylate (190 mg, 0.88 mmol, 1.0 eq) in DMF (7 mL) were added 6-(trifluoromethyl)picolinic acid (165.7 mg, 0.88 mmol, 1.0 eq), DIEA (336 mg, 2.6 mmol, 3.0 eq), and HATU (329 mg, 0.88 mmol, 1.0 eq). The reaction solution was stirred at rt under N₂ for 18 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 70 mg, yield 20%).

### Step D: 1-(Pyrimidin-5-yl)-4-(6-(trifluoromethyl)picolinamido)-1H-pyrazole-3-carboxylic acid

At 0°C, lithium hydroxide monohydrate (15 mg, 0.36 mmol, 2.0 eq) was added to a mixed solution of methyl 1-(pyrimidin-5-yl)-4-(6-(trifluoromethyl)picolinamido)-1H-pyrazole-3-carboxylate (70 mg, 0.18 mmol, 1.0 eq) in MeOH (9 mL), THF (9 mL), and water (3 mL). The reaction solution was stirred at rt under N₂ for 18 h. Upon completion, the reaction solution was adjusted to pH < 5 using 1 N HCl. The mixture was filtered and dried to afford the title compound (yellow solid, 50 mg, yield 74%, crude), which was used directly without purification.

### Step E: N-(3-amino-1-(pyrimidin-5-yl)-1H-pyrazol-4-yl)-6-(trifluoromethyl)picolinamide

At rt, ammonium chloride (21.2 mg, 0.40 mmol, 3.0 eq), DIEA (51.3 mg, 0.40 mmol, 3.0 eq), and HATU (75.4 mg, 0.20 mmol, 1.5 eq) were added to a solution of 1-(pyrimidin-5-yl)-4-(6-(trifluoromethyl)picolinamido)-1H-pyrazole-3-carboxylic acid (50 mg, 0.13 mmol, 1.0 eq) in DMF (3 mL). The reaction solution was stirred at rt under N₂ for 0.5 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 15 mg, yield 30%). **¹H NMR** (400 MHz, DMSO-*d*₆) δ 11.81 (s, 1H), 9.50 (s, 2H), 9.28 (s, 1H), 9.20 (s, 1H), 8.47 (d, *J* = 7.7 Hz, 1H), 8.41 (t, *J* = 7.8 Hz, 1H), 8.23 (d, *J* = 7.6 Hz, 1H), 8.17 (s, 1H), 7.89 (s, 1H). LCMS (ESI): m/z 378 [M+H]⁺.

### Preparation Example 46. Synthesis of Compound A46

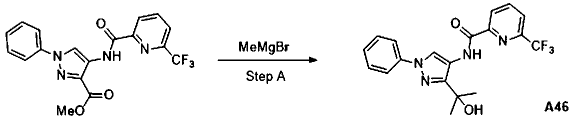

### Step A: N-(3-(2-hydroxypropan-2-yl)-1-phenyl-1H-pyrazol-4-yl)-6-(trifluoromethyl)picolinamide

Under N₂ at 0°C, a 3 M solution of methylmagnesium bromide in diethyl ether (0.85 mL, 2.56 mmol, 10 eq) was slowly added to a solution of methyl 1-phenyl-4-(6-(trifluoromethyl)picolinamido)-1H-pyrazole-3-carboxylate (100 mg, 0.256 mmol, 1.0 eq) in THF (5 mL). The reaction solution was stirred at rt under N₂ for 18 h. Upon completion, the reaction was quenched with saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by HPLC to afford the title compound (white solid, 16.1 mg, yield 16%). **¹H NMR** (400 MHz, DMSO-*d*₆) δ 11.22 (s, 1H), 8.89 (s, 1H), 8.45 (d, *J=* 7.6 Hz, 1H), 8.39 (t, *J* = 7.8 Hz, 1H), 8.19 (d, *J* = 7.7 Hz, 1H), 7.80 (d, *J* = 7.8 Hz, 2H), 7.49 (t, *J =* 7.9 Hz, 2H), 7.29 (t, *J* = 7.4 Hz, 1H), 5.98 (s, 1H), 1.59 (s, 6H). LC/MS (ESI) m/z: 391 [M+H]⁺.

### Preparation Example 47. Synthesis of Compound A47

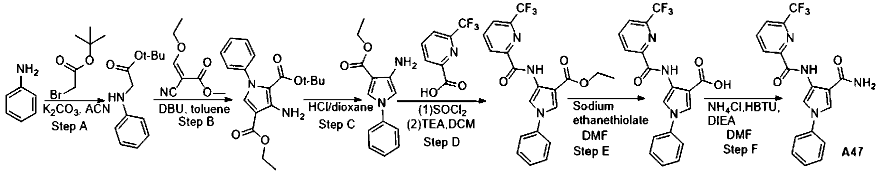

### Step A: Tert-butyl phenylglycinate

At rt, potassium carbonate (10.6 g, 76.9 mmol, 1.5 eq) was added to a solution of aniline (4.8 g, 51.3 mmol, 1.0 eq) and tert-butyl 2-bromoacetate (10.0 g, 51.3 mmol, 1.0 eq) in acetonitrile (50.0 mL). The reaction solution was stirred at rt under N₂ for 16 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (green oil, 10.2 g, yield 96%).

### Step B: 2-(Tert-butyl)-4-ethyl 3-amino-1-phenyl-1H-pyrrole-2,4-dicarboxylic acid

At rt, DBU (21.2 g, 84.4 mmol, 3.5 eq) was added to a solution of tert-butyl phenylglycinate (5.0 g, 24.1 mmol, 1.0 eq) and (Z)-methyl 2-cyano-3-ethoxyacrylate (7.5 g, 48.3 mmol, 2.0 eq) in toluene (50.0 mL). The reaction solution was stirred at 120°C under N₂ for 16 h. Upon completion, the reaction solution was cooled to rt, quenched with water, and extracted with EtOAc (×3). The combined organic phases were washed with water and saturated aqueous NaCl, dried over anhydrous Na₂SO₄, and concentrated under reduced pressure. The residue was purified by column chromatography to afford the title compound (white solid, 1.0 g, yield 13%).

### Step C: Ethyl 4-amino-1-phenyl-1H-pyrrole-3-carboxylate

At 0°C, 4 M HCl in 1,4-dioxane (10 mL) was added to 2-(tert-butyl)-4-ethyl 3-amino-1-phenyl-1H-pyrrole-2,4-dicarboxylic acid (500.0 mg, 1.5 mmol, 1.0 eq). The reaction solution was stirred at rt under N₂ for 2 h. Upon completion, the mixture was concentrated under reduced pressure to afford the title compound (white solid, 300 mg, yield 86%, crude), which was used directly without purification.

### Step D: Ethyl 1-phenyl-4-(6-(trifluoromethyl)picolinamido)-1H-pyrrole-3-carboxylate

At 0°C, thionyl chloride (5 mL) was added to 6-(trifluoromethyl)picolinic acid (750 mg, 3.9 mmol, 3.0 eq). The reaction solution was stirred at 80°C under N₂ for 2 h. Upon completion, the mixture was concentrated under reduced pressure to afford 6-(trifluoromethyl)picolinoyl chloride (3.0 eq), which was dissolved in DCM (2.0 mL). At 0°C, a solution of 6-(trifluoromethyl)picolinoyl chloride (3.0 eq) in DCM was added dropwise to a solution of ethyl 4-amino-1-phenyl-1H-pyrrole-3-carboxylate (300.0 mg, 1.3 mmol, 1.0 eq) and TEA (660.0 mg, 6.5 mmol, 5.0 eq) in DCM (3.0 mL). The mixture was stirred at rt under N₂ for 1 h. Upon completion, the mixture was diluted in DCM, washed sequentially with water and saturated aqueous NaCl, dried over anhydrous Na₂SO₄, and concentrated under reduced pressure. The residue was purified by column chromatography to afford the title compound (white solid, 300 mg, yield 57%).

### Step E: 1-Phenyl-4-(6-(trifluoromethyl)picolinamido)-1H-pyrrole-3-carboxylic acid

At rt, sodium ethanethiolate (625.6 mg, 7.4 mmol, 10.0 eq) was added to a solution of ethyl 1-phenyl-4-(6-(trifluoromethyl)picolinamido)-1H-pyrrole-3-carboxylate (300.0 mg, 743.8 µmol, 1.0 eq) in DMF (5.0 mL). The reaction solution was stirred at 100°C under N₂ for 2 h. Upon completion, the reaction solution was cooled to rt, adjusted to pH 6-7 with 1 N HCl, and extracted with EtOAc (×3). The combined organic phases were washed with water and saturated aqueous NaCl, dried over anhydrous Na₂SO₄, and concentrated under reduced pressure. The residue was purified by column chromatography to afford the title compound (white solid, 200.0 mg, yield 72%).

### Step F: N-(4-amino-1-phenyl-1H-pyrrol-3-yl)-6-(trifluoromethyl)picolinamide

At rt, ammonium chloride (43.0 mg, 800.0 µmol, 1.5 eq), DIEA (344.4 mg, 2.7 mmol, 5.0 eq), and HATU (304.0 mg, 800.0 µmol, 1.5 eq) were added to a solution of 1-phenyl-4-(6-(trifluoromethyl)picolinamido)-1H-pyrrole-3-carboxylic acid (200.0 mg, 532.9 µmol, 1.0 eq) in DMF (5 mL). The reaction solution was stirred at rt under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 52.6 mg, yield 26%). **¹H NMR** (400 MHz, DMSO-*d*₆) δ 12.25 (s, 1H), 8.45-8.35 (m, 2H), 8.17 (d, *J* = 7.4 Hz, 1H), 8.11 (d, *J* = 2.4 Hz, 1H), 8.02 (d, *J* = 2.4 Hz, 1H), 7.75 (d, *J* = 26.4 Hz, 1H), 7.62-7.54 (m, 4H), 7.36 (dd, *J* = 13.8, 6.7 Hz, 2H). LCMS (ESI): m/z 375 [M+H]⁺.

### Preparation Example 48. Synthesis of Compound A48

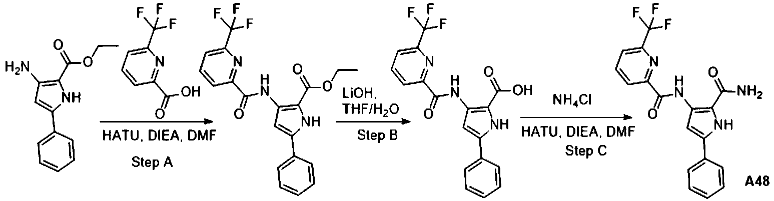

### Step A: Ethyl 5-phenyl-3-(6-(trifluoromethyl)picolinamido)-1H-pyrrole-2-carboxylate

At rt, 6-(trifluoromethyl)picolinic acid (83.0 mg, 0.434 mmol, 1.0 eq), DIEA (168 mg, 1.30 mmol, 3.0 eq), and HATU (165 mg, 0.434 mmol, 1.0 eq) were added to a solution of ethyl 3-amino-5-phenyl-1H-pyrrole-2-carboxylate (100 mg, 0.434 mmol, 1.0 eq) in DMF (10 mL). The reaction solution was stirred at rt under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 130 mg, yield 74%).

### Step B: 5-Phenyl-3-(6-(trifluoromethyl)picolinamido)-1H-pyrrole-2-carboxylic acid

At 0°C, lithium hydroxide monohydrate (15.4 mg, 0.645 mmol, 2.0 eq) was added to a mixed solution of ethyl 5-phenyl-3-(6-(trifluoromethyl)picolinamido)-1H-pyrrole-2-carboxylate (130 mg, 0.322 mmol, 1.0 eq) in THF (10 mL) and water (10 mL). The reaction solution was stirred at 40°C for 24 h. Upon completion, the reaction solution was adjusted to pH 5-6 using 1 N HCl. The mixture was extracted with EtOAc (×3), and the combined organic phases were washed with water and saturated aqueous NaCl, dried over anhydrous Na₂SO₄, and concentrated under reduced pressure to afford the title compound (white solid, 100 mg, yield 83%, crude), which was used directly without purification.

### Step C: N-(2-amino-5-phenyl-1H-pyrrol-3-yl)-6-(trifluoromethyl)picolinamide

At rt, ammonium chloride (42.8 mg, 0.799 mmol, 3.0 eq), DIEA (103 mg, 0.799 mmol, 3.0 eq), and HATU (152 mg, 0.400 mmol, 1.5 eq) were added to a solution of 5-phenyl-3-(6-(trifluoromethyl)picolinamido)-1H-pyrrole-2-carboxylic acid (100 mg, 0.266 mmol, 1.0 eq) in DMF (10 mL). The reaction solution was stirred at rt under N₂ for 12 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (yellow solid, 30.0 mg, yield 30%). **¹H NMR** (400 MHz, CD₃OD) δ 8.45 (d, *J* = 7.8 Hz, 1H), 8.28 (t, *J =* 7.8 Hz, 1H), 8.02 (d, *J =* 7.2 Hz, 1H), 7.73-7.68 (m, 2H), 7.48 (s, 1H), 7.44 (t, *J =* 7.7 Hz, 2H), 7.32 (t, *J=* 7.4 Hz, 1H). LCMS (ESI) m/z: 375 [M+H]⁺.

### Preparation Example 49. Synthesis of Compound A49

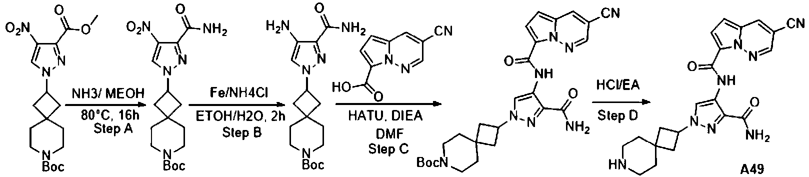

### Step A: Tert-butyl 2-(3-carbamoyl-4-nitro-1H-pyrazol-1-yl)-7-azaspiro[3.5]nonane-7-carboxylate

At rt, tert-butyl 2-(3-(methoxycarbonyl)-4-nitro-1H-pyrazol-1-yl)-7-azaspiro[3.5]nonane-7-carboxylate (2 g, 3.04 mmol, 1.0 eq) was added to a solution of ammonia in MeOH (7 N, 50 mL, 12.0 eq). The mixture was stirred in a sealed tube at 80°C under N₂ for 18 h. The mixture was cooled and concentrated in vacuo. The residue was purified by silica gel column chromatography to afford tert-butyl 2-(3-carbamoyl-4-nitro-1H-pyrazol-1-yl)-7-azaspiro[3.5]nonane-7-carboxylate as a white solid (0.8 g, 69.30%).

### Step B: Tert-butyl 2-(4-amino-3-carbamoyl-1H-pyrazol-1-yl)-7-azaspiro[3.5]nonane-7-carboxylate

At rt, tert-butyl 2-(3-carbamoyl-4-nitro-1H-pyrazol-1-yl)-7-azaspiro[3.5]nonane-7-carboxylate (300 mg, 0.79 mmol, 1 eq), ethanol (10 mL), water (2 mL), iron powder (442 mg, 7.91 mmol, 10 eq), and ammonium chloride (423 mg, 7.91 mmol, 10 eq) were mixed and stirred at 80°C for 2 h. The mixture was cooled and then concentrated in vacuo. The residue was purified by silica gel column chromatography to afford tert-butyl 2-(4-amino-3-carbamoyl-1H-pyrazol-1-yl)-7-azaspiro[3.5]nonane-7-carboxylate as a white solid (200 mg, 72.39%).

### Step C: Tert-butyl 2-(3-carbamoyl-4-(3-isocyanopyrrolo[1,2-b]pyridazine-7-carboxamido)-1H-pyrazol-1-yl)-7-azaspiro[3.5]nonane-7-carboxylate

At 0°C under N₂, HATU (326.44 mg, 0.859 mmol, 1.5 eq) was added to a solution of 3-isocyanopyrrolo[1,2-b]pyridazine-7-carboxylic acid (107 mg, 0.57 mmol, 1 eq), tert-butyl 2-(4-amino-3-carbamoyl-1H-pyrazol-1-yl)-7-azaspiro[3.5]nonane-7-carboxylate (200 mg, 0.57 mmol, 1 eq), and DIEA (222 mg, 1.72 mmol, 3 eq) in DMF (5 mL). The reaction was stirred under N₂ for 1 h. Water was then added, and the mixture was extracted, dried, and concentrated in vacuo. The residue was purified by flash column chromatography to afford tert-butyl 2-(3-carbamoyl-4-(3-isocyanopyrrolo[1,2-b]pyridazine-7-carboxamido)-1H-pyrazol-1-yl)-7-azaspiro[3.5]nonane-7-carboxylate (colorless oil, 200 mg, yield 67.38%).

### Step D: N-(3-carbamoyl-1-(7-azaspiro[3.5]nonan-2-yl)-1H-pyrazol-4-yl)-3-isocyanopyrrolo[1,2-b]pyridazine-7-carboxamide

Under N₂ and with stirring in an ice bath, tert-butyl 2-(3-carbamoyl-4-(3-isocyanopyrrolo[1,2-b]pyridazine-7-carboxamido)-1H-pyrazol-1-yl)-7-azaspiro[3.5]nonane-7-carboxylate (150 mg, 0.412 mmol, 1 eq) was added to an HCl/EA solution (4 N, 5 mL). The mixture was stirred under N₂ for 1 h. The reaction mixture was concentrated in vacuo, The residue was purified by preparative high performance liquid chromatography to afford N-(3-carbamoyl-1-(7-azaspiro[3.5]nonan-2-yl)-1H-pyrazol-4-yl)-3-isocyanopyrrolo[1,2-b]pyridazine-7-carboxamide as a yellow solid (98.7 mg, yield 61.16%). **¹H NMR** (400 MHz, DMSO) δ 12.12 (s, 1H), 8.97 (s, 1H), 8.71 (d, *J =* 2.1 Hz, 1H), 8.48 (s, 1H), 8.39 (s, 1H), 7.74 (d, *J =* 4.8 Hz, 1H), 7.60 (s, 1H), 7.49 (s, 1H), 7.10 (d, *J* = 4.8 Hz, 1H), 4.98 (p*, J =* 8.3 Hz, 1H), 2.87 (dd, *J* = 30.5, 4.0 Hz, 4H), 2.42 (dd, *J* = 15.3, 5.0 Hz, 2H), 2.34-2.28 (m, 2H), 1.74 (s, 4H).

### Preparation Example 50. Synthesis of Compound A50

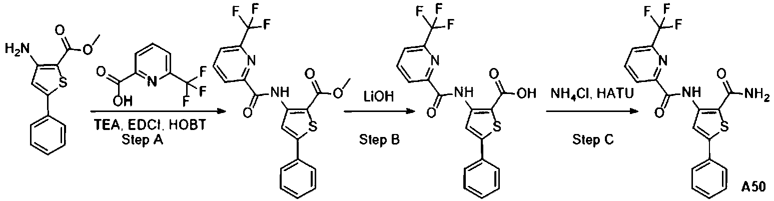

### Step A: Methyl 5-phenyl-3-(6-(trifluoromethyl)picolinamido)thiophene-2-carboxylate

At rt, 6-(trifluoromethyl)picolinic acid (615.0 mg, 3.2 mmol, 1.5 eq), HOBT (652.0 mg, 4.3 mmol, 2.0 eq), EDCI (823.0 mg, 4.3 mmol, 2.0 eq), and TEA (1.1 g, 10.7 mmol, 5.0 eq) were added to a solution of methyl 3-amino-5-phenylthiophene-2-carboxylate (500.0 mg, 2.1 mmol, 1.0 eq) in DMF (5 mL). The reaction solution was stirred at rt under N₂ for 18 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 600 mg, yield 70%).

### Step B: 5-Phenyl-3-(6-(trifluoromethyl)picolinamido)thiophene-2-carboxylic acid

At 0°C, lithium hydroxide (176.4 mg, 7.4 mmol, 5.0 eq) was added to a mixed solution of methyl 5-phenyl-3-(6-(trifluoromethyl)picolinamido)thiophene-2-carboxylate (600.0 mg, 1.47 mmol, 1.0 eq) in THF (4 mL) and water (4 mL). The reaction solution was stirred at 50°C for 18 h. Upon completion, the reaction solution was adjusted to pH=4 using 1 N HCl. The mixture was extracted with EtOAc (×3), and the combined organic phases were washed with water and saturated aqueous NaCl, dried over anhydrous Na₂SO₄, and concentrated under reduced pressure to afford the title compound (white solid, 300 mg, yield 69%, crude), which was used directly without purification.

### Step C: N-(2-amino-5-phenylthiophen-3-yl)-6-(trifluoromethyl)picolinamide

At rt, ammonium chloride (36.4 mg, 680.2 µmol, 2.0 eq) and TEA (172.1 mg, 1.7 mmol, 5.0 eq) were added to a solution of 5-phenyl-3-(6-(trifluoromethyl)picolinamido)thiophene-2-carboxylic acid (100.0 mg, 340.1 µmol, 1.0 eq) and HATU (193.9 mg, 510.2 µmol, 1.5 eq) in DMF (3 mL). The reaction solution was stirred at rt under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 8.7 mg, yield 7%). **¹H NMR** (400 MHz, DMSO-*d*₆) δ 13.18 (s, 1H), 8.55 (s, 1H), 8.47 (d, *J =* 7.7 Hz, 1H), 8.40 (t, *J =* 7.8 Hz, 1H), 8.22 (d, *J =* 7.1 Hz, 1H), 7.81 (s, 2H), 7.76-7.69 (m, 2H), 7.52 (t, *J =* 7.5 Hz, 2H), 7.45 (t, *J =* 7.3 Hz, 1H). LCMS (ESI): m/z 392 [M+H]⁺.

### Preparation Example 51. Synthesis of Compound A51

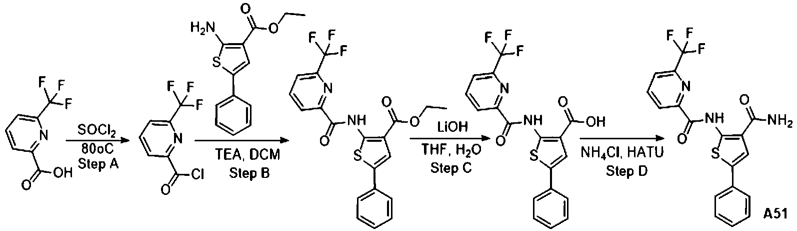

### Step A: 6-(Trifluoromethyl)picolinoyl chloride

At 0°C, thionyl chloride (20 mL) was added to 6-(trifluoromethyl)picolinic acid (2.0 g, 2.1 mmol, 1.0 eq). The reaction solution was stirred at 80°C under N₂ for 2 h. Upon completion, the mixture was concentrated under reduced pressure to afford the title compound (yellow solid, 2.0 g, crude), which was used directly without purification.

### Step B: Ethyl 5-phenyl-2-(6-(trifluoromethyl)picolinamido)thiophene-3-carboxylate

At 0°C, a solution of 6-(trifluoromethyl)picolinoyl chloride (500.0 mg, crude) in DCM was added dropwise to a solution of ethyl 2-amino-5-phenylthiophene-3-carboxylate (172.95 mg, 0.7 mmol, 1.0 eq) and TEA (48.7 mg, 2.0 mmol, 3.0 eq) in DCM (8.0 mL). The reaction solution was stirred at rt under N₂ for 1 h. Upon completion, the mixture was diluted in DCM, washed sequentially with water and saturated aqueous NaCl, dried over anhydrous Na₂SO₄, and concentrated under reduced pressure. The residue was purified by column chromatography to afford the title compound (colorless oil, 100 mg, yield 29%).

### Step C: 5-Phenyl-2-(6-(trifluoromethyl)picolinamido)thiophene-3-carboxylic acid

At 0°C, lithium hydroxide (75.6 mg, 0.6 mmol, 3.0 eq) was added to a mixed solution of ethyl 5-phenyl-2-(6-(trifluoromethyl)picolinamido)thiophene-3-carboxylate (100.0 mg, 0.2 mmol, 1.0 eq) in THF (5 mL) and water (1 mL). The reaction solution was stirred at 80°C for 16 h. Upon completion, the reaction solution was adjusted to pH=4 using 1 N HCl. The mixture was extracted with EtOAc (×3), and the combined organic phases were washed with water and saturated aqueous NaCl, dried over anhydrous Na₂SO₄, and concentrated under reduced pressure to afford the title compound (colorless oil, 100 mg, crude), which was used directly without purification.

### Step D: N-(3-amino-3-phenylthiophen-2-yl)-6-(trifluoromethyl)picolinamide

At rt, ammonium chloride (200.0 mg, 0.02 mmol, 1.0 eq) and TEA (1 mL) were added to a solution of 5-phenyl-2-(6-(trifluoromethyl)picolinamido)thiophene-3-carboxylic acid (100.0 mg, 0.02 mmol, 1.0 eq) and HATU (1 mL) in DMF (2 mL). The reaction solution was stirred at rt under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 11.4 mg, yield 11%). **¹H NMR** (400 MHz, DMSO-*d*₆) δ 13.93 (s, 1H), 8.51-8.38 (m, 2H), 8.23 (d, *J =* 7.5 Hz, 1H), 8.00 (s, 1H), 7.96 (s, 1H), 7.76 (s, 1H), 7.63 (d, *J* = 7.5 Hz, 2H), 7.47 (t, *J =* 7.7 Hz, 2H), 7.33 (t, *J =* 7.3 Hz, 1H). LCMS (ESI): m/z 392 [M+H]⁺.

### Preparation Example 52. Synthesis of Compound A52

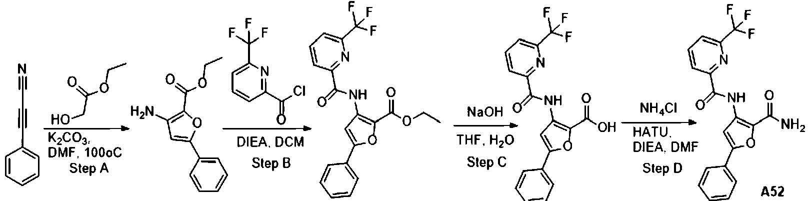

### Step A: Ethyl 3-amino-5-phenylfuran-2-carboxylate

At rt, to a solution of 3-phenylpropanenitrile (500.0 mg, 2.1 mmol, 1.0 eq) and ethyl 2-hydroxyacetate (218.63 mg, 2.1 mmol, 1.0 eq) in DMF (20 mL) was added potassium carbonate (438.0 mg, 3.1 mmol, 1.5 eq). The reaction solution was stirred at 100°C under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (colorless oil, 450.0 mg, yield 48%).

### Step B: Ethyl 5-phenyl-3-(6-(trifluoromethyl)picolinamido)furan-2-carboxylate

At 0°C, 6-(trifluoromethyl)picolinoyl chloride (100.0 mg, crude) was added dropwise to a solution of ethyl 3-amino-5-phenylfuran-2-carboxylate (162.4 mg, 0.7 mmol, 1.0 eq) and DIEA (48.7 mg, 2.0 mmol, 3.0 eq) in DCM (8.0 mL). The reaction solution was stirred at rt under N₂ for 1 h. Upon completion, the mixture was diluted in DCM, washed sequentially with water and saturated aqueous NaCl, dried over anhydrous Na₂SO₄, and concentrated under reduced pressure. The residue was purified by column chromatography to afford the title compound (colorless oil, 80 mg, yield 29%).

### Step C: 5-Phenyl-3-(6-(trifluoromethyl)picolinamido)furan-2-carboxylic acid

At 0°C, NaOH (75.6 mg, 0.6 mmol, 3.0 eq) was added to a mixed solution of ethyl 5-phenyl-3-(6-(trifluoromethyl)picolinamido)furan-2-carboxylate (80.0 mg, 0.2 mmol, 1.0 eq) in THF (5 mL) and water (1 mL). The reaction solution was stirred at 80°C for 16 h. Upon completion, the reaction solution was adjusted to pH=4 using 1 N HCl. The mixture was extracted with EtOAc (×3), and the combined organic phases were washed with water and saturated aqueous NaCl, dried over anhydrous Na₂SO₄, and concentrated under reduced pressure to afford the title compound (colorless oil, 80.0 mg, crude), which was used directly without purification.

### Step D: N-(2-amino-5-phenylfuran-3-yl)-6-(trifluoromethyl)picolinamide

At rt, to a solution of 5-phenyl-3-(6-(trifluoromethyl)picolinamido)furan-2-carboxylic acid (80.0 mg, 20.0 µmol, 1.0 eq) and HATU (11.4 mg, 30.0 µmol, 1.5 eq) in DMF (2.0 mL) were added ammonium chloride (90.0 mg, 0.1 mmol, 1.5 eq) and DIEA (2.0 mL). The reaction solution was stirred at rt under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 26.6 mg, yield 33%). **¹H NMR** (400 MHz, DMSO-*d*₆) δ 11.81 (s, 1H), 8.47 (d, *J* = 7.7 Hz, 1H), 8.41 (t, *J* = 7.8 Hz, 1H), 8.23 (d, *J* = 7.6 Hz, 1H), 8.11 (s, 1H), 8.03 (d, *J* = 7.4 Hz, 2H), 7.87 (s, 1H), 7.71 (s, 1H), 7.51 (t, *J* = 7.5 Hz, 2H), 7.42 (t, *J* = 7.3 Hz, 1H). LCMS (ESI): m/z 376 [M+H]⁺.

### Preparation Example 53. Synthesis of Compound A53

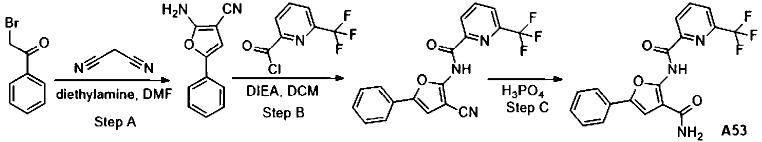

### Step A: 2-Amino-5-phenylfuran-3-carbonitrile

At rt, to a solution of 2-bromo-1-phenylethan-1-one (2.0 g, 10.0 mmol, 1.0 eq) in DMF (30.0 mL) were added malononitrile (0.66 g, 10.0 mmol, 1.0 eq) and diethylamine (2.2 g, 30.1 mmol, 3.0 eq). The reaction solution was stirred at rt under N₂ for 1 h. Upon completion, water (100 mL) was added and the mixture was stirred for 20 min. The mixture was filtered, and the filter cake was washed with water twice, and dried completely to afford the title compound (yellow solid, 1.0 g, yield 54%, crude), which was used directly without purification.

### Step B: N-(3-Cyano-5-phenylfuran-2-yl)-6-(trifluoromethyl)picolinamide

At 0°C, 6-(trifluoromethyl)pyridine-2-carbonyl chloride (1.7 g, 8.1 mmol, 1.5 eq) was added dropwise to a solution of 2-amino-5-phenylfuran-3-carbonitrile (1.0 g, 5.4 mmol, 1.0 eq) and DIEA (2.1 g, 16.3 mmol, 3.0 eq) in DCM (10.0 mL). The reaction solution was stirred at rt under N₂ for 0.5 h. Upon completion, the mixture was diluted in DCM, washed sequentially with water and saturated aqueous NaCl, dried over anhydrous Na₂SO₄, and concentrated under reduced pressure. The residue was purified by column chromatography to afford the title compound (yellow solid, 500.0 mg, yield 26%).

### Step C: N-(3-Amino-5-phenylfuran-2-yl)-6-(trifluoromethyl)picolinamide

Phosphoric acid (20.0 mL) was added to N-(3-cyano-5-phenylfuran-2-yl)-6-(trifluoromethyl)picolinamide (500.0 mg, 1.4 mmol, 1.0 eq). The reaction solution was stirred at 100°C for 1 h. Upon completion, the mixture was cooled to rt, diluted in water, and extracted with DCM (×3). The combined organic phases were washed with water and saturated aqueous NaCl, dried over anhydrous Na₂SO₄, and concentrated under reduced pressure. The residue was purified by HPLC to afford the title compound (yellow solid, 31.5 mg, yield 6%). **¹H NMR** (400 MHz, DMSO-*d*₆) δ 12.45 (s, 1H), 8.50-8.38 (m, 2H), 8.24 (d, *J =* 7.5 Hz, 1H), 7.85 (s, 1H), 7.65 (d, *J =* 7.5 Hz, 2H), 7.56 (s, 1H), 7.49 (t, *J =* 7.8 Hz, 2H), 7.40 (s, 1H), 7.34 (t, *J =* 7.4 Hz, 1H). LCMS (ESI): m/z 376 [M+H]⁺.

### Preparation Example 54. Synthesis of Compound A54

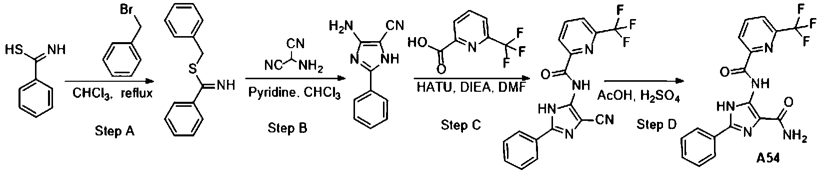

### Step A: Benzoylbenzylcarbamothioate

At rt, a solution of bromomethylbenzene (0.44 mL, 3.6 mmol, 1.0 eq) in chloroform (3 mL) was slowly added to a stirred solution of thiobenzoic acid (500 mg, 3.6 mmol, 1.0 eq) in chloroform (2 mL). The mixture was stirred under reflux for 12 h. Upon completion, the mixture was concentrated under reduced pressure. The residue was diluted in diethyl ether until a solid precipitated. The mixture was filtered, and the filter cake was washed with cold diethyl ether, and dried completely to afford the title compound (white solid, 600 mg, yield 53%).

### Step B: 4-Amino-2-phenyl-1H-imidazole-5-carbonitrile

At 0°C, to a solution of benzoylbenzylcarbamothioate (500 mg, 2.2 mmol, 1.0 eq) in chloroform (5 mL) were added 2-aminopropanedinitrile (535 mg, 6.6 mmol, 3.0 eq) and pyridine (0.54 mL, 6.6 mmol, 3.0 eq). The mixture was stirred at rt for 16 h. Upon completion, the reaction was quenched with saturated aqueous NH₄Cl and extracted with DCM (×3). The combined organic phases were washed with water and saturated aqueous NaCl, dried over anhydrous Na₂SO₄, and concentrated under reduced pressure. The residue was purified by column chromatography to afford the title compound (yellow solid, 320 mg, yield 78%).

### Step C: N-(4-Cyano-2-phenyl-1H-imidazol-5-yl)-6-(trifluoromethyl)picolinamide

At rt, to a solution of 4-amino-2-phenyl-1H-imidazole-5-carbonitrile (320 mg, 1.7 mmol, 1.0 eq) in DCM (5 mL) were added 6-(trifluoromethyl)pyridine-2-carboxylic acid (498.0 mg, 2.6 mmol, 1.5 eq), HATU (990.9 mg, 2.6 mmol, 1.5 eq), and DIEA (673.52 mg, 5.211 mmol, 3.0 eq). The mixture was stirred at 40°C for 16 h. Upon completion, the reaction solution was diluted in saturated aqueous NH₄Cl and extracted with DCM (×3). The combined organic phases were washed sequentially with water and saturated aqueous NaCl, dried over anhydrous Na₂SO₄, and concentrated under reduced pressure. The residue was purified by column chromatography to afford the title compound (yellow solid, 200 mg, yield 32%).

### Step D: N-(4-amino-2-phenyl-1H-imidazol-5-yl)-6-(trifluoromethyl)picolinamide

At rt, to a solution of N-(4-cyano-2-phenyl-1H-imidazol-5-yl)-6-(trifluoromethyl)picolinamide (200 mg, 0.56 mmol, 1.0 eq) in AcOH (10 mL) was added concentrated sulfuric acid (2 mL). The mixture was stirred at 40°C for 16 h. Upon completion, the mixture was cooled to rt, diluted in water, and extracted with DCM (×3). The combined organic phases were washed with water and saturated aqueous NaCl, dried over anhydrous Na₂SO₄, and concentrated under reduced pressure. The residue was purified by column chromatography to afford the title compound (yellow solid, 77 mg, yield 36%). **¹H NMR** (400 MHz, DMSO-*d*₆) δ 12.85 (brs, 1H), 12.06 (brs, 1H), 8.48 (d, *J* = 7.7 Hz, 1H), 8.42 (t, *J =* 7.7 Hz, 1H), 8.24 (d, *J =* 7.6 Hz, 1H), 8.08 (d, *J =* 6.4 Hz, 2H), 7.63-7.27 (m, 5H). LCMS (ESI): m/z 376 [M+H]⁺.

### Preparation Example 55. Synthesis of Compound A55

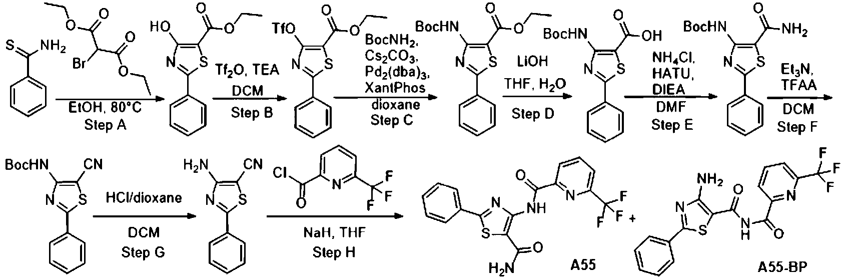

### Step A: Ethyl 4-hydroxy-2-phenylthiazole-5-carboxylate

At rt, to a solution of thiobenzamide (1 g, 7.29 mmol, 1.0 eq) in ethanol (20 mL) was added ethyl 2-bromo-3-ethoxy-3-oxopropanoate (1.92 g, 8.02 mmol, 1.1 eq). The mixture was stirred at 80°C for 2 h. Upon completion, the reaction solution was concentrated under reduced pressure. The residue was purified by column chromatography to afford the title compound (yellow solid, 1.6 g, yield 88%).

### Step B: Ethyl 2-phenyl-4-(((trifluoromethyl)sulfonyl)oxy)thiazole-5-carboxylate

At 0°C, to a solution of ethyl 4-hydroxy-2-phenylthiazole-5-carboxylate (1.6 g, 6.42 mmol, 1.0 eq) and TEA (1.80 mL, 12.84 mmol, 2.0 eq) in DCM (20 mL) was slowly added trifluoromethanesulfonic anhydride (2.17 g, 7.70 mmol, 1.2 eq). The mixture was stirred at rt for 2 h. Upon completion, the reaction was quenched with saturated aqueous NH₄Cl and extracted with DCM (×3). The combined organic phases were washed with water and saturated aqueous NaCl, dried over anhydrous Na₂SO₄, and concentrated under reduced pressure. The residue was purified by column chromatography to afford the title compound (white solid, 1.3 g, yield 53%).

### Step C: Ethyl 4-((tert-butoxycarbonyl)amino)-2-phenylthiazole-5-carboxylate

At rt, to a solution of ethyl 2-phenyl-4-(((trifluoromethyl)sulfonyl)oxy)thiazole-5-carboxylate (1.3 g, 3.409 mmol, 1.0 eq) and tert-butyl carbamate (0.80 g, 6.82 mmol, 2.0 eq) in 1,4-dioxane (20 mL) were added Pd₂(dba)₃ (0.31 g, 0.34 mmol, 0.1 eq), XantPhos (0.30 g, 0.51 mmol, 0.15 eq), and cesium carbonate (4.44 g, 13.64 mmol, 4.0 eq). The mixture was stirred at 80°C under N₂ for 2 h. Upon completion, the mixture was cooled to rt, diluted in water, extracted with EtOAc, washed with saturated aqueous NaCl, dried over anhydrous Na₂SO₄, and concentrated under reduced pressure. The residue was purified by column chromatography to afford the title compound (yellow solid, 1 g, yield 84%).

### Step D: 4-((Tert-butoxycarbonyl)amino)-2-phenylthiazole-5-carboxylic acid

At 0°C, to a mixed solution of ethyl 4-((tert-butoxycarbonyl)amino)-2-phenylthiazole-5-carboxylate (0.8 g, 2.30 mmol, 1.0 eq) in THF (5 mL) and water (5 mL) was added lithium hydroxide (0.19 g, 4.59 mmol, 2.0 eq). The reaction solution was stirred at rt under N₂ for 2 h. Upon completion, the reaction solution was adjusted to pH=4 using 4 N HCl. The mixture was extracted with EtOAc (×3), and the combined organic phases were washed with water and saturated aqueous NaCl, dried over anhydrous Na₂SO₄, and concentrated under reduced pressure to afford the title compound (white solid, 600 mg, crude), which was used directly without purification.

### Step E: Tert-butyl (5-carbamoyl-2-phenylthiazol-4-yl)carbamate

At rt, to a solution of 4-((tert-butoxycarbonyl)amino)-2-phenylthiazole-5-carboxylic acid (400 mg, 1.25 mmol, 1.0 eq) and ammonium chloride (334 mg, 6.24 mmol, 5.0 eq) in DCM (8 mL) were added HATU (569.7 mg, 1.50 mmol, 1.2 eq) and DIEA (484.1 mg, 3.75 mmol, 3.0 eq). The mixture was stirred at rt for 3 h. Upon completion, the reaction solution was concentrated under reduced pressure. The residue was purified by column chromatography to afford the title compound (white solid, 230 mg, yield 58%).

### Step F: Tert-butyl (5-cyano-2-phenylthiazol-4-yl)carbamate

At 0°C, to a solution of tert-butyl (5-carbamoyl-2-phenylthiazol-4-yl)carbamate (160 mg, 0.50 mmol, 1.0 eq) and TEA (0.20 mL, 1.50 mmol, 3.0 eq) in DCM (5 mL) was slowly added trifluoroacetic anhydride (157.8 mg, 0.75 mmol, 1.5 eq). The mixture was stirred at rt for 2 h. Upon completion, the mixture was diluted in water and extracted with DCM (×3). The combined organic phases were washed with water and saturated aqueous NaCl, dried over anhydrous Na₂SO₄, and concentrated under reduced pressure. The residue was purified by column chromatography to afford the title compound (white solid, 100 mg, yield 66%).

### Step G: 4-Amino-2-phenylthiazole-5-carbonitrile

At 0°C, to tert-butyl (5-cyano-2-phenylthiazol-4-yl)carbamate (100 mg, 0.33 mmol, 1.0 eq) was added 4 M HCl in 1,4-dioxane (1 mL, 4 mmol, 12 eq). The reaction solution was stirred at rt under N₂ for 1 h. The reaction solution was concentrated under reduced pressure to afford the title compound (white solid, 60 mg, crude), which was used directly without purification.

### Step H: 2-Phenyl-4-(6-(trifluoromethyl)picolinamido)thiazole-5-carboxamide and 4-amino-2-phenyl-N-(6-(trifluoromethyl)picolinoyl)thiazole-5-carboxamide

At 0°C under N₂, to a solution of 4-amino-2-phenylthiazole-5-carbonitrile (60 mg, 0.30 mmol, 1.0 eq) in THF (3 mL) was added 60 wt% sodium hydride (21.46 mg, 0.89 mmol, 3.0 eq) in portions. The mixture was stirred at rt for 0.5 h. To the above mixture was then added 6-(trifluoromethyl)picolinoyl chloride (75 mg, 0.36 mmol, 1.2 eq). The mixture was stirred at rt under N₂ for 2 h. Upon completion, the reaction was quenched with saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by HPLC to afford 2-phenyl-4-(6-(trifluoromethyl)picolinamido)thiazole-5-carboxamide (yellow solid, 15.3 mg, yield 13%). **¹H NMR** (400 MHz, DMSO-*d*₆): δ 10.69 (s, 1H), 8.42-8.36 (m, 2H), 8.24 (dd, *J =* 6.4, 2.4 Hz, 1H), 7.97-7.94 (m, 2H), 7.64 (s, 2H), 7.60-7.53 (m, 3H). LCMS (ESI): m/z 393 [M+H]⁺. 4-amino-2-phenyl-N-(6-(trifluoromethyl)picolinoyl)thiazole-5-carboxamide (white solid, 2.7 mg, yield 2%). **¹H NMR** (400 MHz, DMSO-*d*₆): δ 12.74 (s, 1H), 8.46 (d, *J =* 7.6 Hz, 1H), 8.40 (t, *J* = 7.7 Hz, 1H), 8.22 (d, *J =* 7.7 Hz, 1H), 8.03-7.99 (m, 2H), 7.92 (brs, 2H), 7.60-7.57 (m, 3H). LCMS (ESI): m/z 393 [M+H]⁺.

### Preparation Example 56. Synthesis of Compound A56

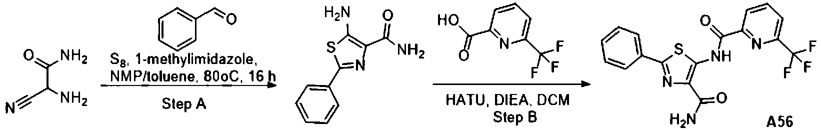

### Step A: 5-Amino-2-phenylthiazole-4-carboxamide

At rt, to a mixed solution of 2-amino-2-cyanoacetamide (1.0 g, 10.1 mmol, 1.0 eq) in toluene (20 mL) and N-methylpyrrolidone (20 mL) were added benzaldehyde (1.0 mL, 10.1 mmol, 1.0 eq), cyclooctasulfur (520.0 mg, 2.0 mmol, 0.2 eq), and 1-methylimidazole (830.0 mg, 10.1 mmol, 1.0 eq). The mixture was stirred at 80°C for 16 h. Upon completion, the reaction solution was cooled to rt, poured into water, and extracted with EtOAc (×3). The combined organic phases were washed with water and saturated aqueous NaCl, dried over anhydrous Na₂SO₄, and concentrated under reduced pressure. The residue was purified by column chromatography to afford the title compound (white solid, 450 mg, yield 20%).

### Step B: 2-Phenyl-5-(6-(trifluoromethyl)picolinamido)thiazole-4-carboxamide

At rt, to a solution of 6-(trifluoromethyl)pyridine-2-carboxylic acid (217.9 mg, 1.14 mmol, 1.0 eq) in DCM (10 mL) were added HATU (433.5 mg, 1.14 mmol, 1.0 eq) and DIEA (294.7 mg, 2.3 mmol, 2.0 eq). The mixture was stirred at rt for 0.5 h. 5-amino-2-phenylthiazole-4-carboxamide (250.0 mg, 1.14 mmol, 1.0 eq) was then added. The mixture was stirred at 40°C for 12 h. Upon completion, the reaction solution was diluted in DCM, washed sequentially with water and saturated aqueous NaCl, dried over anhydrous Na₂SO₄, and concentrated under reduced pressure. The residue was purified by column chromatography to afford the title compound (yellow solid, 70 mg, yield 16%). **¹H NMR** (400 MHz, DMSO-*d*₆) δ 13.42 (s, 1H), 8.50-8.41 (m, 2H), 8.26 (d, *J* = 7.6 Hz, 1H), 8.05 (dd, *J =* 7.8, 1.3 Hz, 3H), 7.96 (s, 1H), 7.56-7.50 (m, 3H). LCMS(ESI): m/z 393 [M+H]⁺.

### Preparation Example 57. Synthesis of Compound A57

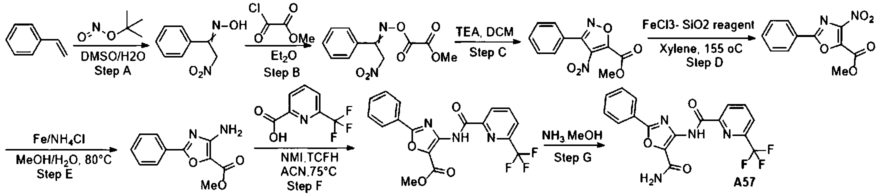

### Step A: 2-Nitro-1-phenylethan-1-one oxime

At rt, to a mixed solution of styrene (5.549 mL, 48.008 mmol, 1.0 eq) in dimethyl sulfoxide (100 mL) and water (50 mL) was added tert-butyl nitrite (9.90 g, 96.015 mmol, 2.0 eq). The reaction solution was stirred at rt under N₂ for 12 h. Upon completion, the reaction solution was diluted in DCM, washed sequentially with water and saturated aqueous NaCl, dried over anhydrous Na₂SO₄, filtered, and concentrated under reduced pressure. The residue was purified by column chromatography to afford the title compound (yellow oil, 7.0 g, yield 81%).

### Step B: Methyl 2-(((2-nitro-1-phenylethylidene)amino)oxy)-2-oxoacetate

At rt, to a solution of 2-nitro-1-phenylethan-1-one oxime (3.0 g, 16.6 mmol, 1.0 eq) in diethyl ether (100 mL) was added methyl 2-chloro-2-oxoacetate (2.04 g, 16.6 mmol, 1.0 eq). The reaction solution was stirred at rt for 24 h. Upon completion, the reaction solution was diluted in DCM, washed sequentially with water and saturated aqueous NaCl, dried over anhydrous Na₂SO₄, filtered, and concentrated under reduced pressure. The residue was purified by column chromatography to afford the title compound (yellow oil, 4.0 g, yield 90%).

### Step C: Methyl 4-nitro-3-phenylisoxazole-5-carboxylate

At rt, to a solution of methyl 2-(((2-nitro-1-phenylethylidene)amino)oxy)-2-oxoacetate (4.0 g, 15.0 mmol, 1.0 eq) in DCM (60 mL) was added TEA (2.1 mL, 15.0 mmol, 1.0 eq). The reaction solution was stirred at 40°C for 48 h. Upon completion, the mixture was cooled to rt. The reaction solution was diluted in DCM, washed sequentially with water and saturated aqueous NaCl, dried over anhydrous Na₂SO₄, filtered, and concentrated under reduced pressure. The residue was purified by column chromatography to afford the title compound (yellow solid, 1.0 g, yield 27%).

### Step D: Methyl 4-nitro-2-phenyloxazole-5-carboxylate

At rt, to a solution of methyl 4-nitro-3-phenylisoxazole-5-carboxylate (50 mg, 0.201 mmol, 1.0 eq) in xylene (5 mL) was added anhydrous FeCl₃-SiO₂ reagent (2 mg, 0.201 mmol, 1.0 eq). The reaction solution was stirred in a sealed tube at 155°C until completion of the reaction. Upon completion, the mixture was cooled to rt. The reaction solution was diluted in DCM and filtered, and the filtrate was concentrated under reduced pressure. The residue was purified by column chromatography to afford the title compound (yellow solid, 30 mg, yield 60%).

### Step E: Methyl 4-amino-2-phenyloxazole-5-carboxylate

At rt, to a solution of methyl 4-nitro-2-phenyloxazole-5-carboxylate (300 mg, 1.21 mmol, 1.0 eq) in MeOH (10 mL) and water (2 mL) were added iron powder (337 mg, 6.04 mmol, 5.0 eq) and ammonium chloride (323 mg, 6.04 mmol, 5.0 eq). The reaction solution was stirred at 80°C for 2 h. Upon completion, the mixture was filtered, and the filtrate was concentrated under reduced pressure. The residue was purified by column chromatography to afford the title compound (white solid, 180 mg, yield 68%).

### Step F: Methyl 2-phenyl-4-(6-(trifluoromethyl)picolinamido)oxazole-5-carboxylate

At rt, to a solution of methyl 4-amino-2-phenyloxazole-5-carboxylate (170 mg, 0.779 mmol, 1.0 eq) in acetonitrile (15 mL) were added 6-(trifluoromethyl)picolinic acid (1.49 g, 7.79 mmol, 10 eq) and N-methylimidazole (1.28 g, 15.6 mmol, 20 eq). The mixture was stirred at 80°C for 5 min. N,N,N',N'-tetramethylchloroformamidinium hexafluorophosphate (2.19 g, 7.79 mmol, 10 eq) was then added. The reaction solution was stirred at 80°C for 5 h. Upon completion, the mixture was cooled to rt, poured into ice water, and extracted with EtOAc (×3). The combined organic phases were washed with water and saturated aqueous NaCl, dried over anhydrous Na₂SO₄, filtered, and concentrated under reduced pressure. The residue was purified by column chromatography to afford the title compound (white solid, 200 mg, yield 46%).

### Step G: 2-Phenyl-4-(6-(trifluoromethyl)picolinamido)oxazole-5-carboxamide

At rt, to methyl 2-phenyl-4-(6-(trifluoromethyl)picolinamido)oxazole-5-carboxylate (150 mg, 0.383 mmol, 1.0 eq) was added 2 N amine solution in MeOH (15 mL). The reaction solution was stirred at rt for 8 h. Upon completion, the mixture was concentrated under reduced pressure. The residue was purified by HPLC to afford the title compound (white solid, 15.7 mg, yield 11%). **¹H NMR** (400 MHz, CDCl₃) & 11.46 (s, 1H), 8.53 (d, *J* = 7.8 Hz, 1H), 8.20 (d, *J =* 6.7 Hz, 2H), 8.14 (t, *J =* 7.8 Hz, 1H), 7.91 (d, *J =* 7.8 Hz, 1H), 7.58-7.47 (m, 3H), 5.97 (s, 2H). LCMS (ESI): m/z 377 [M+H]⁺.

### Preparation Example 58. Synthesis of Compound A58

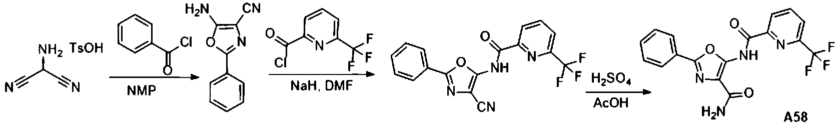

### Step A: 5-Amino-2-phenyloxazole-4-carbonitrile

At 0°C, to a solution of aminomalononitrile p-toluenesulfonate (2 g, 7.896 mmol, 1.0 eq) in N-methylpyrrolidone (20 mL) was added benzoyl chloride (1.22 g, 8.686 mmol, 1.1 eq). The mixture was stirred at rt for 18 h. Upon completion, the reaction was quenched with saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow solid, 1 g, yield 68%).

### Step B: N-(4-cyano-2-phenyloxazol-5-yl)-6-(trifluoromethyl)picolinamide

At 0°C under N₂, to a solution of 5-amino-2-phenyloxazole-4-carbonitrile (300 mg, 1.620 mmol, 1.0 eq) in THF (5 mL) was added 60 wt% sodium hydride (129.60 mg, 3.240 mmol, 2.0 eq) in portions. The mixture was stirred at rt for 0.5 h. To the above mixture was then added 6-(trifluoromethyl)picolinoyl chloride (373.41 mg, 1.782 mmol, 1.1 eq). The mixture was stirred at rt under N₂ for 2 h. Upon completion, the reaction was quenched with saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow solid, 100 mg, yield 17%).

### Step C: 2-Phenyl-5-(6-(trifluoromethyl)picolinamido)oxazole-4-carboxamide

At 0°C, to N-(4-cyano-2-phenyloxazol-5-yl)-6-(trifluoromethyl)picolinamide (100 mg, 0.279 mmol, 1.0 eq) were sequentially added AcOH (4 mL) and concentrated sulfuric acid (2 mL). The mixture was stirred at rt for 18 h. Upon completion, the reaction solution was diluted in ice water and extracted with EtOAc (×3). The combined organic phases were washed with saturated aqueous NaHCO₃ and saturated aqueous NaCl, dried over anhydrous Na₂SO₄, and concentrated under reduced pressure. The residue was purified by HPLC to afford the title compound (yellow solid, 47.9 mg, yield 45%). **¹H NMR** (400 MHz, DMSO-*d*₆): δ 11.90 (s, 1H), 8.45 (dt, *J =* 15.4, 7.7 Hz, 2H), 8.27 (d, *J =* 7.4 Hz, 1H), 8.06-7.97 (m, 2H), 7.76 (d, *J =* 38.6 Hz, 2H), 7.63-7.55 (m, 3H). LCMS (ESI): m/z 377 [M+H]⁺.

### Preparation Example 59. Synthesis of Compound A59

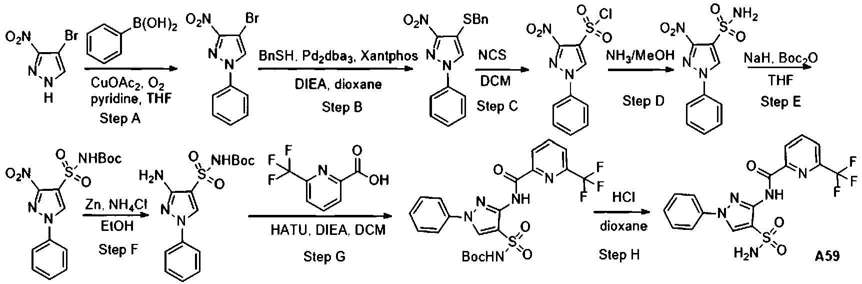

### Step A: 4-Bromo-3-nitro-1-phenyl-1H-pyrazole

At rt, to a solution of 4-bromo-3-nitro-1H-pyrazole (1.5 g, 7.8 mmol, 1.0 eq) in THF (50 mL) were added phenylboronic acid (1.05 g, 8.6 mmol, 1.1 eq), copper acetate (2.34 g, 11.7 mmol, 1.5 eq), and pyridine (3.2 mL, 39.1 mmol, 5.0 eq). The reaction solution was stirred at 40°C under O₂ for 18 h. Upon completion, the reaction solution was filtered, and the filtrate was diluted in water and extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow solid, 800 mg, yield 38%).

### Step B: 4-(Benzylthio)-3-nitro-1-phenyl-1H-pyrazole

To a solution of 4-bromo-3-nitro-1-phenyl-1H-pyrazole (500 mg, 1.9 mmol, 1.0 eq) and benzyl mercaptan (278.0 mg, 2.2 mmol, 1.2 eq) in 1,4-dioxane (10 mL) were sequentially added diisopropylethylamine (482.2 mg, 3.8 mmol, 2.0 eq), 4,5-bis(diphenylphosphino)-9,9-dimethylxanthene (215.8 mg, 0.4 mmol, 0.2 eq), and tris(dibenzylideneacetone)dipalladium (170.8 mg, 0.20 mmol, 0.1 eq). The reaction solution was purged with N₂ (×3), and the reaction was stirred at 100°C under N₂ for 3 h. Upon completion, the mixture was cooled to rt, diluted in EtOAc, washed sequentially with water and saturated aqueous NaCl, dried over anhydrous Na₂SO₄, filtered, and concentrated under reduced pressure. The residue was purified by column chromatography to afford the title compound (yellow solid, 350 mg, yield 60%).

### Step C: 3-Nitro-1-phenylpyrazole-4-sulfonyl chloride

At 0°C, to a solution of 4-(benzylthio)-3-nitro-1-phenyl-1H-pyrazole (350 mg, 1.125 mmol, 1.0 eq) in DCM (5 mL) was added N-chlorosuccinimide (375.3 mg, 2.8 mmol, 2.5 eq). The reaction solution was stirred at rt under N₂ for 2 h. Upon completion, the mixture was concentrated under reduced pressure. The residue was purified by column chromatography to afford the title compound (yellow solid, 210 mg, yield 65%).

### Step D: 3-Nitro-1-phenylpyrazole-4-sulfonamide

At 0°C, to a solution of 3-nitro-1-phenylpyrazole-4-sulfonyl chloride (210 mg, 0.73 mmol, 1.0 eq) in DCM (5 mL) was added 7 M ammonia in MeOH (0.3 mL). The reaction solution was stirred at 30°C for 2 h. Upon completion, the mixture was concentrated under reduced pressure to afford the title compound (yellow solid, 195 mg, yield 99%, crude), which was used directly without purification.

### Step E: Tert-butyl (3-nitro-1-phenyl-1H-pyrazol-4-yl)sulfonyl)carbamate

At 0°C under N₂, to a solution of 3-nitro-1-phenylpyrazole-4-sulfonamide (100 mg, 0.37 mmol, 1.0 eq) in THF (3 mL) was added 60 wt% sodium hydride (29.9 mg, 0.75 mmol, 2.0 eq) in portions. The mixture was stirred at rt for 0.5 h. To the above mixture was then added di-tert-butyl dicarbonate (122.0 mg, 0.56 mmol, 1.5 eq). The mixture was stirred at 50°C under N₂ for 2 h. Upon completion, the mixture was cooled to rt, quenched with saturated aqueous NH₄Cl, and extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow liquid, 90 mg, yield 65%).

### Step F: Tert-butyl (3-amino-1-phenyl-1H-pyrazol-4-yl)sulfonyl)carbamate

At rt, to a solution of tert-butyl (3-nitro-1-phenyl-1H-pyrazol-4-yl)sulfonyl)carbamate (90.0 mg, 0.25 mmol, 1.0 eq) in ethanol (5 mL) were added zinc powder (159.74 mg, 2.5 mmol, 10 eq) and ammonium chloride (26.14 mg, 0.5 mmol, 2.0 eq). The mixture was stirred at 80°C for 3 h. Upon completion, the mixture was filtered, and the filtrate was concentrated under reduced pressure to afford the title compound (colorless liquid, 70 mg, yield 84%, crude), which was used directly without purification.

### Step G: Tert-butyl (1-phenyl-3-((6-(trifluoromethyl)picolinamido)-1H-pyrazol-4-yl)sulfonyl)carbamate

At rt, to a solution of 6-(trifluoromethyl)pyridine-2-carboxylic acid (47.44 mg, 0.25 mmol, 1.2 eq) in DCM (5 mL) were added HATU (118.0 mg, 0.31 mmol, 1.5 eq) and DIEA (80.2 mg, 0.6 mmol, 2.4 eq). The mixture was stirred at rt for 0.5 h. To the above mixture was then added tert-butyl (3-amino-1-phenyl-1H-pyrazol-4-yl)sulfonyl)carbamate (70 mg, 0.21 mmol, 1.0 eq). The mixture was stirred at 40°C for 12 h. Upon completion, the reaction solution was diluted in DCM, washed sequentially with water and saturated aqueous NaCl, dried over anhydrous Na₂SO₄, and concentrated under reduced pressure. The residue was purified by column chromatography to afford the title compound (yellow solid, 60 mg, yield 57%).

### Step H: N-(1-phenyl-4-sulfamoyl-1H-pyrazol-3-yl)-6-(trifluoromethyl)picolinamide

At 0°C, to a solution of tert-butyl (1-phenyl-3-((6-(trifluoromethyl)picolinamido)-1H-pyrazol-4-yl)sulfonyl)carbamate (60 mg, 0.117 mmol, 1.0 eq) in 1,4-dioxane (1 mL) was added 4 M HCl in 1,4-dioxane (1 mL). The mixture was stirred at 30°C for 1 h. Upon completion, the mixture was concentrated under reduced pressure. The residue was purified by HPLC to afford the title compound (white solid, 35 mg, yield 72%). **¹H NMR** (400 MHz, DMSO-*d₆*) δ 10.88 (s, 1H), 8.98 (s, 1H), 8.52-8.32 (m, 2H), 8.24 (d, *J* = 6.6 Hz, 1H), 7.92 (d, *J* = 7.8 Hz, 2H), 7.56 (t, *J =* 7.9 Hz, 2H), 7.48-7.30 (m, 3H). LCMS (ESI): m/z 412 [M+H]⁺.

### Preparation Example 60. Synthesis of Compound A60

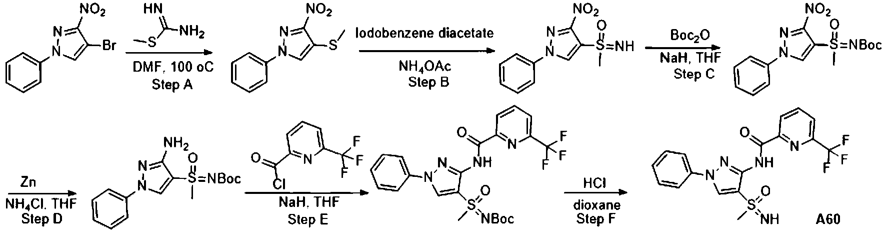

### Step A: 4-(methylthio)-3-nitro-1-phenyl-1H-pyrazole

At rt, to a solution of 4-bromo-3-nitro-1-phenylpyrazole (850.0 mg, 3.2 mmol, 1.0 eq) in DMF (5 mL) was added methyl carbamimidothioate (857.5 mg, 9.6 mmol, 3.0 eq). The mixture was stirred at 100°C for 16 h. Upon completion, the reaction solution was cooled to rt, diluted in water, and extracted with EtOAc (×3). The combined organic phases were washed with water and saturated aqueous NaCl, dried over anhydrous Na₂SO₄, filtered, and concentrated under reduced pressure. The residue was purified by column chromatography to afford the title compound (yellow solid, 420 mg, yield 56%).

### Step B: Methyl (3-nitro-1-phenylpyrazol-4-yl)(oxo)-λ⁶-sulfanylaminate

At rt, to a solution of 4-(methylthio)-3-nitro-1-phenyl-1H-pyrazole (200.0 mg, 0.85 mmol, 1.0 eq) in MeOH (5 mL) were added (diacetoxyiodo)benzene (551.1 mg, 1.7 mmol, 2.0 eq) and ammonium acetate (131.1 mg, 1.7 mmol, 2.0 eq). The mixture was stirred at rt for 2 h. Upon completion, the reaction solution was cooled to rt, poured into ice water, and extracted with EtOAc (×3). The combined organic phases were washed with water and saturated aqueous NaCl, dried over anhydrous Na₂SO₄, filtered, and concentrated under reduced pressure. The residue was purified by column chromatography to afford the title compound (yellow solid, 96.0 mg, yield 42%).

### Step C: Tert-butyl (3-nitro-1-phenyl-1H-pyrazol-4-yl)(oxo)-λ⁶-sulfinyl)carbamate

At 0°C under N₂, to a solution of methyl (3-nitro-1-phenylpyrazol-4-yl)(oxo)-λ⁶-sulfanylaminate (90.0 mg, 0.34 mmol, 1.0 eq) in THF (5 mL) was added 60 wt% sodium hydride (40.6 mg, 1.0 mmol, 3.0 eq) in portions. The mixture was stirred at rt for 0.5 h. To the above mixture was then added di-tert-butyl dicarbonate (147.5 mg, 0.68 mmol, 2.0 eq). The mixture was stirred at 50°C under N₂ for 2 h. Upon completion, the reaction was quenched with saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow solid, 95 mg, yield 76%).

### Step D: Tert-butyl (3-amino-1-phenyl-1H-pyrazol-4-yl)(methyl)(oxo)-λ⁶-sulfinyl)carbamate

At rt, to a solution of tert-butyl (3-nitro-1-phenyl-1H-pyrazol-4-yl)(oxo)-λ⁶-sulfinyl)carbamate (95.0 mg, 0.26 mmol, 1.0 eq) in THF (5 mL) and water (5 mL) were added zinc powder (169.3 mg, 2.6 mmol, 10.0 eq) and ammonium chloride (13.8 mg, 0.26 mmol, 1.0 eq). The mixture was stirred at 80°C for 3 h. Upon completion, the mixture was filtered, and the filtrate was concentrated under reduced pressure to afford the title compound (yellow solid, 70 mg, crude), which was used directly without purification.

### Step E: Tert-butyl (methyl(oxo)(1-phenyl-3-(6-(trifluoromethyl)picolinamido)-1H-pyrazol-4-yl)-λ⁶-sulfinyl)carbamate

At 0°C under N₂, to a solution of tert-butyl ((3-amino-1-phenyl-1H-pyrazol-4-yl)(methyl)(oxo)-λ⁶-sulfinyl)carbamate (70.0 mg, 0.21 mmol, 1.0 eq) in THF (3 mL) was added 60 wt% sodium hydride (15.0 mg, 0.63 mmol, 3.0 eq) in portions. The mixture was stirred at rt for 0.5 h. To the above mixture was then added 6-(trifluoromethyl)pyridine-2-carbonyl chloride (52.3 mg, 0.25 mmol, 1.2 eq) The mixture was stirred at 30°C under N₂ for 2 h. Upon completion, the mixture was cooled to rt, quenched with saturated aqueous NH₄Cl, and extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow solid, 105 mg, yield 99%).

### Step F: N-(4-(S-methylsulfimino)-1-phenyl-1H-pyrazol-3-yl)-6-(trifluoromethyl)picolinamide

At 0°C, to a solution of tert-butyl (methyl(oxo)(1-phenyl-3-(6-(trifluoromethyl)picolinamido)-1H-pyrazol-4-yl)-λ⁶-sulfinyl)carbamate (105.0 mg, 0.098 mmol) in 1,4-dioxane (2 mL) was added 4 M HCl in 1,4-dioxane (1 mL). The mixture was stirred at rt for 1 h. Upon completion, the mixture was concentrated under reduced pressure. The residue was purified by HPLC to afford the title compound (off-white solid, 46.0 mg, yield 55%). **¹H NMR** (400 MHz, DMSO-*d*₆) δ 11.69 (s, 1H), 9.13 (s, 1H), 8.47-8.36 (m, 2H), 8.22 (dd, *J* = 7.5, 1.2 Hz, 1H), 7.93 (dd, *J* = 8.6, 0.9 Hz, 2H), 7.57 (m, 2H), 7.40 (t, *J =* 7.4 Hz, 1H), 4.76 (s, 1H), 3.29 (s, 3H). LCMS (ESI): m/z 410 [M+H]⁺.

### Preparation Example 61. Synthesis of Compound A61

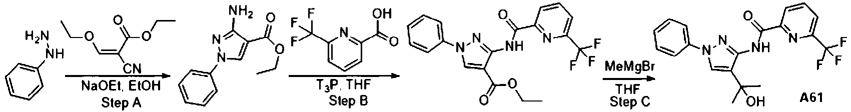

### Step A: Ethyl 3-amino-1-phenyl-1H-pyrazole-4-carboxylate

At 0°C, to a solution of ethyl (2Z)-2-cyano-3-ethoxyprop-2-enoate (6.26 g, 36.98 mmol, 1.0 eq) and phenylhydrazine (4 g, 36.98 mmol, 1.0 eq) in ethanol (20 mL) was added sodium ethoxide (5.04 g, 73.97 mmol, 2.0 eq). The mixture was stirred at rt for 3 h. Upon completion, the reaction was quenched with 4 N HCl, diluted in water, and extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow solid, 700 mg, yield 8%).

### Step B: Ethyl 1-phenyl-3-(6-(trifluoromethyl)picolinamido)-1H-pyrazole-4-carboxylate

At rt, to a solution of ethyl 3-amino-1-phenyl-1H-pyrazole-4-carboxylate (100 mg, 0.43 mmol, 1.0 eq) and 6-(trifluoromethyl)pyridine-2-carboxylic acid (123.9 mg, 0.65 mmol, 1.5 eq) in THF (5 mL) was added T₃P (50 wt% in EtOAc, 550.4 mg, 0.87 mmol, 2.0 eq). The mixture was stirred at 50°C for 18 h. Upon completion, the mixture was concentrated under reduced pressure. The residue was purified by column chromatography to afford the title compound (white solid, 30 mg, yield 17%).

### Step C: N-(4-(2-hydroxypropan-2-yl)-1-phenyl-1H-pyrazol-3-yl)-6-(trifluoromethyl)picolinamide

At 0°C under N₂, to a solution of ethyl 1-phenyl-3-(6-(trifluoromethyl) picolinamido)-1H-pyrazole-4-carboxylate (30 mg, 0.07 mmol, 1.0 eq) in THF (5 mL) was slowly added 3 M methylmagnesium bromide in diethyl ether (0.1 mL, 0.30 mmol, 4.0 eq). The reaction solution was stirred at rt under N₂ for 3 h. Upon completion, the reaction was quenched with saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by HPLC to afford the title compound (white solid, 20.5 mg, yield 71%). **¹H NMR** (400 MHz, CD₃OD) δ 8.49 (d, *J =* 7.6 Hz, 1H), 8.31 (t, *J =* 7.6 Hz, 1H), 8.13 (s, 1H), 8.06 (d, *J* = 7.6 Hz, 1H), 7.82 (d, *J =* 8.0 Hz, 2H), 7.47 (t, *J =* 8.0 Hz, 2H), 7.29 (t, *J =* 7.2 Hz, 1H), 1.63 (s, 6H). LC/MS (ESI) m/z: 391 [M+H]⁺.

### Preparation Example 62. Synthesis of Compound A62

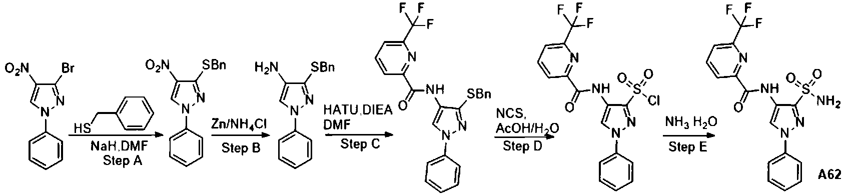

### Step A: 3-(benzylthio)-4-nitro-1-phenyl-1H-pyrazole

At 0°C under N₂, to a solution of 3-bromo-4-nitro-1-phenylpyrazole (400 mg, 1.49 mmol, 1.0 eq) in DMF (10 mL) was added sodium hydride (60 wt%, 89.5 mg, 2.24 mmol, 1.5 eq) in portions. The mixture was stirred at rt for 0.5 h. To the above mixture was then added benzyl mercaptan (204 mg, 1.64 mmol, 1.1 eq). The mixture was stirred at 70°C under N₂ for 1 h. Upon completion, the mixture was cooled to rt, quenched with saturated aqueous NH₄Cl, and extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow liquid, 390 mg, yield 84%).

### Step B: 3-(benzylthio)-1-phenyl-1H-pyrazol-4-amine

At rt, to a solution of 3-(benzylthio)-4-nitro-1-phenyl-1H-pyrazole (390 mg, 1.253 mmol, 1.0 eq) in MeOH (10 mL) was added zinc powder (655 mg, 10.0 mmol, 8.0 eq) and ammonium chloride (670 mg, 12.5 mmol, 10 eq) in water (5 mL). The mixture was stirred at rt for 1 h. Upon completion, the mixture was filtered and concentrated under reduced pressure. The residue was purified by column chromatography to afford the title compound (white solid, 240 mg, yield 68%).

### Step C: N-(3-(benzylthio)-1-phenyl-1H-pyrazol-4-yl)-6-(trifluoromethyl)picolinamide

At rt, to a solution of 6-(trifluoromethyl)pyridine-2-carboxylic acid (179 mg, 0.938 mmol, 1.0 eq) in DMF (5 mL) were added HATU (422 mg, 1.11 mmol, 1.2 eq) and DIEA (331 mg, 2.56 mmol, 2.7 eq). The mixture was stirred at rt for 0.5 h. 3-(benzylthio)-1-phenyl-1H-pyrazol-4-amine (240 mg, 0.853 mmol, 0.9 eq) was then added to the above reaction solution, and the mixture was stirred at rt for 1 h. Upon completion, the reaction solution was diluted in saturated aqueous NH₄Cl, and extracted with EtOAc (×3). The combined organic phases were washed sequentially with water and saturated aqueous NaCl, dried over anhydrous Na₂SO₄, and concentrated under reduced pressure. The residue was purified by column chromatography to afford the title compound (yellow solid, 240 mg, yield 62%).

### Step D: 1-Phenyl-4-(6-(trifluoromethyl)picolinamido)-1H-pyrazole-3-sulfonyl chloride

At 0°C, to a mixed solution of N-(3-(benzylthio)-1-phenyl-1H-pyrazol-4-yl)-6-(trifluoromethyl)picolinamide (240 mg, 0.528 mmol, 1.0 eq) in acetonitrile (3 mL), water (1 mL), and AcOH (3 mL) was added N-chlorosuccinimide (564 mg, 4.22 mmol, 8.0 eq). The reaction solution was stirred at rt under N₂ for 12 h. Upon completion, the mixture was diluted in EtOAc, washed with water and saturated aqueous NaCl, dried over anhydrous Na₂SO₄, and concentrated under reduced pressure. The residue was purified by column chromatography to afford the title compound (yellow solid, 220 mg, yield 96%).

### Step E: N-(1-phenyl-3-sulfamoyl-1H-pyrazol-4-yl)-6-(trifluoromethyl)picolinamide

At 0°C, to a solution of 1-phenyl-4-(6-(trifluoromethyl)picolinamido)-1H-pyrazole-3-sulfonyl chloride (170 mg, 0.395 mmol, 1.0 eq) in acetonitrile (3 mL) was added 18% (w/w) aqueous ammonia (1 mL). The reaction solution was stirred at rt for 2 h. Upon completion, the reaction solution was poured into water and extracted with EtOAc (×3). The mixture was dried over anhydrous Na₂SO₄ and concentrated under reduced pressure. The residue was purified by HPLC to afford the title compound (white solid, 48 mg, yield 30%). **¹H NMR** (400 MHz, DMSO-*d*₆) δ 10.85 (s, 1H), 9.11 (s, 1H), 8.48 (d, *J =* 7.7 Hz, 1H), 8.42 (t, *J =* 7.8 Hz, 1H), 8.24 (d, *J =* 7.7 Hz, 1H), 8.02 (s, 2H), 7.92 (d, *J =* 8.2 Hz, 2H), 7.59 (t, *J =* 7.9 Hz, 2H), 7.45 (t, *J =* 7.4 Hz, 1H). LCMS (ESI): m/z 412 [M+H]⁺.

### Preparation Example 63. Synthesis of Compound A63

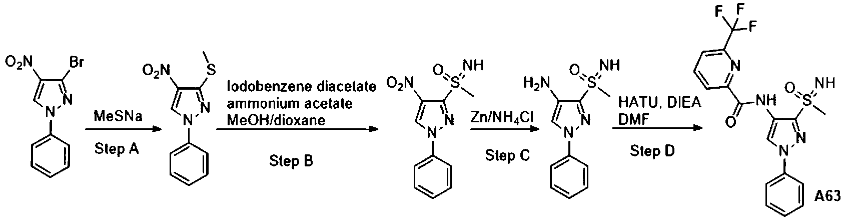

### Step A: 3-(methylthio)-4-nitro-1-phenyl-1H-pyrazole

At 0°C, sodium methanethiolate (261 mg, 3.73 mmol, 2.0 eq) was added portionwise to a solution of 3-bromo-4-nitro-1-phenylpyrazole (500 mg, 1.87 mmol, 1.0 eq) in THF (20 mL). The mixture was stirred at rt for 1 h. Upon completion, the reaction was quenched with water, and extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow solid, 240 mg, yield 54%).

### Step B: Imino(methyl)(4-nitro-1-phenyl-1H-pyrazol-3-yl)-λ⁶-sulfanone

At rt, (diacetoxyiodo)benzene (661 mg, 2.04 mmol, 2.0 eq) and ammonium acetate (314 mg, 4.08 mmol, 4.0 eq) were added to a solution of 3-(methylthio)-4-nitro-1-phenyl-1H-pyrazole (240 mg, 1.02 mmol, 1.0 eq) in 1,4-dioxane (3 mL) and MeOH (3 mL). The mixture was stirred at rt for 12 h. Upon completion, the reaction was quenched with water, and extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow solid, 160 mg, yield 59%).

### Step C: (4-amino-1-phenyl-1H-pyrazol-3-yl)(imino)(methyl)-λ⁶-sulfanone

At rt, zinc powder (314 mg, 4.81 mmol, 8.0 eq) and ammonium chloride (321 mg, 6.01 mmol, 10.0 eq) in water (2 mL) were added to a solution of imino(methyl)(4-nitro-1-phenyl-1H-pyrazol-3-yl)-λ⁶-sulfanone (160 mg, 0.601 mmol, 1.0 eq) in MeOH (5 mL). The mixture was stirred at rt for 1 h. Upon completion, the mixture was filtered and concentrated under reduced pressure. The residue was purified by column chromatography to afford the title compound (white solid, 120 mg, yield 68%).

### Step D: N-(3-(S-methylsulfonimidoyl)-1-phenyl-1H-pyrazol-4-yl)-6-(trifluoromethyl) picolinamide

At rt, to a solution of 6-(trifluoromethyl)pyridine-2-carboxylic acid (89.0 mg, 0.466 mmol, 1.0 eq) in DMF (5 mL) were added HATU (209 mg, 0.550 mmol, 1.2 eq) and DIEA (164 mg, 1.27 mmol, 2.7 eq). The mixture was stirred at rt for 0.5 h. (4-amino-1-phenyl-1H-pyrazol-3-yl)(imino)(methyl)-λ⁶-sulfanone (100 mg, 0.423 mmol, 0.9 eq) was then added to the above reaction solution, and the mixture was stirred at rt for 1 h. Upon completion, the reaction solution was diluted in saturated aqueous NH₄Cl, and extracted with EtOAc (×3). The combined organic phases were washed sequentially with water and saturated aqueous NaCl, dried over anhydrous Na₂SO₄, and concentrated under reduced pressure. The residue was purified by HPLC to afford the title compound (white solid, 23 mg, yield 13%). **¹H NMR** (400 MHz, DMSO-*d*₆) δ 11.83 (s, 1H), 9.12 (s, 1H), 8.46 (d, *J* = 7.6 Hz, 1H), 8.40 *(t, J=* 7.8 Hz, 1H), 8.22 (d, *J =* 7.6 Hz, 1H), 7.95 (d, *J =* 8.0 Hz, 2H), 7.58 (t, *J =* 7.9 Hz, 2H), 7.45 (t, *J =* 7.4 Hz, 1H), 5.22 (s, 1H), 3.36 (s, 3H). LCMS (ESI): m/z 410 [M+H]⁺.

### Preparation Example 64. Synthesis of Compound A64

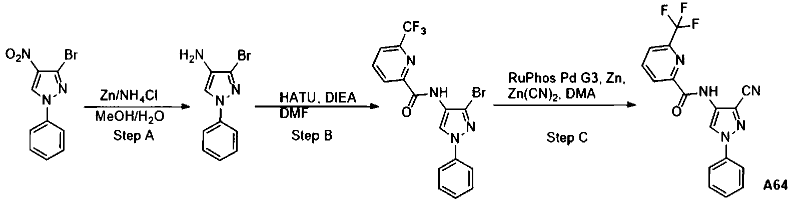

### Step A: 3-bromo-1-phenyl-1H-pyrazol-4-amine

At rt, zinc powder (390 mg, 5.97 mmol, 8.0 eq) and ammonium chloride (399 mg, 7.46 mmol, 10.0 eq) in water (4 mL) were added to a solution of 3-bromo-4-nitro-1-phenylpyrazole (200 mg, 0.746 mmol, 1.0 eq) in MeOH (8 mL). The mixture was stirred at 70°C for 1 h. Upon completion, the mixture was filtered and concentrated under reduced pressure. The residue was purified by column chromatography to afford the title compound (white solid, 125 mg, yield 70%).

### Step B: N-(3-bromo-1-phenyl-1H-pyrazol-4-yl)-6-(trifluoromethyl)picolinamide

At rt, to a solution of 6-(trifluoromethyl)pyridine-2-carboxylic acid (125 mg, 0.525 mmol, 1.0 eq) in DMF (5 mL) were added HATU (220 mg, 0.578 mmol, 1.1 eq) and DIEA (204 mg, 1.58 mmol, 3.0 eq). The mixture was stirred at rt for 0.5 h. 3-bromo-1-phenyl-1H-pyrazol-4-amine (125 mg, 0.525 mmol, 1.0 eq) was then added to the above reaction solution, and the mixture was stirred at rt for 1 h. Upon completion, the reaction solution was diluted in saturated aqueous NH₄Cl, and extracted with EtOAc (×3). The combined organic phases were washed sequentially with water and saturated aqueous NaCl, dried over anhydrous Na₂SO₄, and concentrated under reduced pressure. The residue was purified by column chromatography to afford the title compound (yellow solid, 180 mg, yield 83%).

### Step C: N-(3-cyano-1-phenyl-1H-pyrazol-4-yl)-6-(trifluoromethyl)picolinamide

At rt, zinc powder (85.9 mg, 1.31 mmol, 5.0 eq), zinc cyanide (257 mg, 2.19 mmol, 10.0 eq), and RuPhos-Pd-G₃ (55 mg, 0.066 mmol, 0.3 eq) were added to a solution of N-(3-bromo-1-phenyl-1H-pyrazol-4-yl)-6-(trifluoromethyl)picolinamide (90 mg, 0.219 mmol, 1.0 eq) in N,N-dimethylacetamide (10 mL). The mixture was stirred at 120°C under N₂ for 12 h. Upon completion, the mixture was cooled to rt, diluted in water, extracted with EtOAc, washed with saturated aqueous NaCl, dried over anhydrous Na₂SO₄, and concentrated under reduced pressure. The residue was purified by HPLC to afford the title compound (white solid, 19.4 mg, yield 25%). **¹H NMR** (400 MHz, DMSO-*d*₆) δ 10.82 (s, 1H), 8.93 (s, 1H), 8.41 (dt, *J =* 15.5, 7.8 Hz, 2H), 8.23 (d, *J* = 7.5 Hz, 1H), 7.92 (d, *J =* 8.1 Hz, 2H), 7.59 (t, *J =* 7.8 Hz, 2H), 7.47 (t, *J =* 7.4 Hz, 1H). LCMS (ESI): m/z 358 [M+H]⁺.

### Preparation Example 65. Synthesis of Compound A65

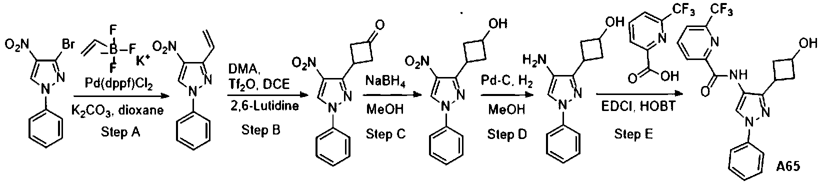

### Step A: 4-nitro-1-phenyl-3-vinyl-1H-pyrazole

To a solution of 3-bromo-4-nitro-1-phenyl-1H-pyrazole (2 g, 7.46 mmol, 1 eq) and potassium carbonate (3.1 g, 22.4 mmol, 3 eq) in 1,4-dioxane (10 mL) and water (3 mL) were added Pd(dppf)Cl₂ (545 mg, 0.746 mmol, 0.1 eq) and potassium trifluoro(vinyl)borate (2 g, 14.9 mmol, 2 eq) at rt. The reaction solution was stirred at 90°C under N₂ for 3 h. Upon completion, the reaction solution was cooled to rt, quenched with water, and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 1.5 g, yield 93%).

### Step B: 3-(4-nitro-1-phenyl-1H-pyrazol-3-yl)cyclobutan-1-one

To a Schlenk tube containing N,N-dimethylacetamide (1.2 mL, 13.4 mmol, 1.2 eq) and 1,2-dichloroethane (30 mL) was added trifluoromethanesulfonic anhydride (9.83 g, 34.85 mmol, 3.1 eq) dropwise at rt in a water bath, and the mixture was stirred for 10 min at rt. A solution of 4-nitro-1-phenyl-3-vinyl-1H-pyrazole (2.4 g, 11.2 mmol, 1.0 eq) and 2,6-dimethylpyridine (4.05 mL, 34.85 mmol, 3.1 eq) in 1,2-dichloroethane (2 mL) was then added. The reaction solution was stirred at 90°C under N₂ for 8 h. After the reaction solution was cooled to rt, water (40 mL) was added. The reaction solution was stirred at 90°C for 16 h. Upon completion, the reaction solution was cooled to rt and extracted with DCM (×3). The combined organic phases were washed with saturated aqueous NaCl, dried over anhydrous Na₂SO₄, filtered, and concentrated under reduced pressure. The residue was purified by column chromatography to afford the title compound (yellow solid, 403 mg, yield 14%).

### Step C: 3-(4-nitro-1-phenyl-1H-pyrazol-3-yl)cyclobutan-1-ol

To a solution of 3-(4-nitro-1-phenyl-1H-pyrazol-3-yl)cyclobutan-1-one (150 mg, 0.6 mmol, 1.0 eq) in MeOH (2 mL) was added sodium borohydride (44.1 mg, 1.2 mmol, 2.0 eq) at rt. The reaction solution was stirred at 25°C under N₂ for 1 h. Upon completion, the reaction solution was quenched with water and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (yellow solid, 120 mg, yield 79%).

### Step D: 3-(4-amino-1-phenyl-1H-pyrazol-3-yl)cyclobutan-1-ol

To a solution of 3-(4-nitro-1-phenyl-1H-pyrazol-3-yl)cyclobutan-1-ol (120 mg, 0.5 mmol, 1.0 eq) in MeOH (2 mL) was added 10% palladium on carbon (49.3 mg, 0.5 mmol, 1.0 eq) at rt under N₂. The reaction solution was purged with H₂ (×3) and stirred at 25°C under H₂ for 1 h. Upon completion, the reaction solution was filtered and concentrated under reduced pressure to afford the title compound (colorless oil, 100 mg, crude), which was used directly without purification.

### Step E: N-(3-(3-hydroxycyclobutyl)-1-phenyl-1H-pyrazol-4-yl)-6-(trifluoromethyl)picolinamide

To a solution of 3-(4-amino-1-phenyl-1H-pyrazol-3-yl)cyclobutan-1-ol (100 mg, 0.4 mmol, 1.0 eq) and 6-(trifluoromethyl)pyridine-2-carboxylic acid (83.4 mg, 0.4 mmol, 1.0 eq) in DMF (2 mL) were added EDCI (125.4 mg, 0.6 mmol, 1.5 eq), HOBt (88.4 mg, 0.6 mmol, 1.5 eq), and TEA (0.2 mL, 1.3 mmol, 3.0 eq) at rt. The reaction solution was stirred at rt under N₂ for 1 h. Upon completion, the reaction solution was diluted in EtOAc, washed sequentially with water and saturated aqueous NaCl, dried over anhydrous Na₂SO₄, filtered, and concentrated under reduced pressure. The residue was purified by HPLC to afford the title compound (white solid, 30 mg, yield 17%). **¹H NMR** (400 MHz, DMSO-*d*₆) δ 9.95 (s, 1H), 8.66 (s, 1H), 8.42-8.33 (m, 2H), 8.20 (dd, *J =* 6.8, 1.9 Hz, 1H), 7.85-7.78 (m, 2H), 7.51 (t, *J =* 8.0 Hz, 2H), 7.30 (t, *J =* 7.4 Hz, 1H), 5.22 (d, *J =* 6.8 Hz, 1H), 4.16-4.06 (m, 1H), 3.10-3.02 (m, 1H), 2.69-2.60 (m, 2H), 2.20-2.09 (m, 2H). LCMS (ESI): m/z 403 [M+H]⁺.

### Preparation Example 66. Synthesis of Compound A66

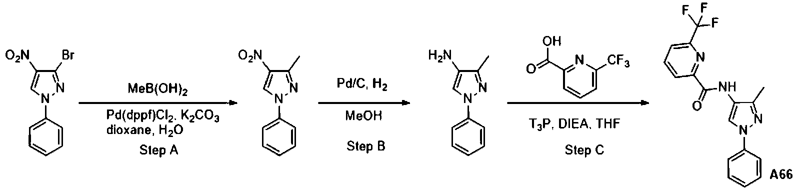

### Step A: 3-methyl-4-nitro-1-phenyl-1H-pyrazole

To a solution of 3-bromo-4-nitro-1-phenylpyrazole (500 mg, 1.865 mmol, 1.0 eq) and methylboronic acid (167.47 mg, 2.798 mmol, 1.5 eq) in 1,4-dioxane (5 mL) and water (1 mL) were added potassium carbonate (773.31 mg, 5.596 mmol, 3.0 eq) and Pd(dppf)Cl₂ (136.48 mg, 0.187 mmol, 0.1 eq) at rt. The mixture was stirred at 100°C under N₂ for 4 h. Upon completion, the mixture was cooled to rt, diluted in water, extracted with EtOAc, washed with saturated aqueous NaCl, dried over anhydrous Na₂SO₄, and concentrated under reduced pressure. The residue was purified by column chromatography to afford the title compound (yellow solid, 150 mg, yield 39%).

### Step B: 3-methyl-1-phenyl-1H-pyrazol-4-amine

To a solution of 3-methyl-4-nitro-1-phenyl-1H-pyrazole (150 mg, 0.738 mmol, 1.0 eq) in MeOH (3 mL) was added 10% palladium on carbon (20 mg) at rt. The reaction solution was purged with H₂ (using a hydrogen balloon) (×3) and stirred under H₂ at rt for 2 h. Upon completion, the mixture was filtered, and the filtrate was concentrated under reduced pressure to afford the title compound (yellow solid, 80 mg, yield 62%, crude), which was used directly without purification.

### Step C: N-(3-methyl-1-phenyl-1H-pyrazol-4-yl)-6-(trifluoromethyl)picolinamide

To a solution of 3-methyl-1-phenyl-1H-pyrazol-4-amine (80 mg, 0.462 mmol, 1.0 eq) and 6-(trifluoromethyl)pyridine-2-carboxylic acid (132.39 mg, 0.693 mmol, 1.5 eq) in THF (3 mL) were added 50 wt% T₃P in EtOAc (440.85 mg, 1.386 mmol, 3.0 eq) and DIEA (179.08 mg, 1.386 mmol, 3.0 eq) at rt. The mixture was stirred at 50°C for 12 h. Upon completion, the mixture was concentrated under reduced pressure. The residue was purified by HPLC to afford the title compound (pink solid, 79.5 mg, yield 49%). **¹H NMR** (400 MHz, CD₃OD) δ 8.55 (s, 1H), 8.45 (d, *J =* 8.0 Hz, 1H), 8.30 (t, *J =* 8.0 Hz, 1H), 8.06 (d, *J =* 8.0 Hz, 1H), 7.74-7.65 (m, 2H), 7.48 (t, *J =* 8.0 Hz, 2H), 7.31 (d, *J* = 7.2 Hz, 1H), 2.39 (s, 3H). LCMS (ESI): m/z 347 [M+H]⁺.

### Preparation Example 67. Synthesis of Compound A67

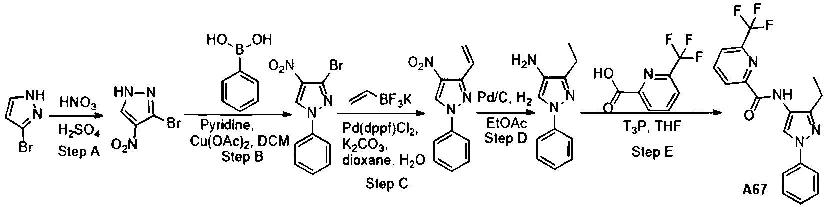

### Step A: 3-Bromo-4-nitro-1H-pyrazole

At 0°C, to 3-bromo-1H-pyrazole (5 g, 34.2 mmol, 1.0 eq) was added concentrated sulfuric acid (10 mL), followed by dropwise addition of concentrated nitric acid (2 mL). The reaction solution was stirred at 0°C for 1 h. Upon completion, the reaction solution was poured into ice water and filtered. The filter cake was dried completely to afford the title compound (yellow solid, 3 g, yield 46%, crude), which was used directly without purification.

### Step B: 3-Bromo-4-nitro-1-phenyl-1H-pyrazole

To a solution of 3-bromo-4-nitro-1H-pyrazole (500 mg, 2.61 mmol, 1.0 eq) in DCM (10 mL) were added phenylboronic acid (413 mg, 3.39 mmol, 1.30 eq), copper acetate (260 mg, 1.302 mmol, 0.50 eq) and pyridine (0.105 mL, 1.30 mmol, 0.50 eq) at rt. The reaction solution was stirred under O₂ at rt for 16 h. Upon completion, the reaction solution was filtered, and the filtrate was concentrated under reduced pressure. The residue was purified by column chromatography to afford the title compound (yellow solid, 400 mg, yield 57%).

### Step C: 4-nitro-1-phenyl-3-vinyl-1H-pyrazole

To a solution of 3-bromo-4-nitro-1-phenyl-1H-pyrazole (200 mg, 0.746 mmol, 1.0 eq) and potassium vinyltrifluoroborate (85 mg, 0.97 mmol, 1.3 eq) in 1,4-dioxane (4 mL) and water (1 mL) were added potassium carbonate (309 mg, 2.24 mmol, 3.0 eq) and Pd(dppf)Cl₂ (55 mg, 0.075 mmol, 0.1 eq) at rt. The mixture was stirred at 100°C under N₂ for 2 h. Upon completion, the mixture was cooled to rt, diluted in water, extracted with EtOAc, washed with saturated aqueous NaCl, dried over anhydrous Na₂SO₄, and concentrated under reduced pressure. The residue was purified by column chromatography to afford the title compound (yellow solid, 110 mg, yield 39%).

### Step D: 3-ethyl-1-phenyl-1H-pyrazol-4-amine

To a solution of 4-nitro-1-phenyl-3-vinyl-1H-pyrazole (110 mg, 0.511 mmol, 1.0 eq) in EtOAc (10 mL) was added 10% palladium on carbon (10 mg) at rt. The reaction solution was purged with H₂ (using a hydrogen balloon) (×3) and stirred under H₂ at rt for 2 h. Upon completion, the mixture was filtered, and the filtrate was concentrated under reduced pressure to afford the title compound (yellow solid, 90 mg, yield 94%, crude), which was used directly without purification.

### Step E: N-(1-phenyl-3-propyl-1H-pyrazol-4-yl)-6-(trifluoromethyl)picolinamide

To a solution of 3-ethyl-1-phenyl-1H-pyrazol-4-amine (90 mg, 0.481 mmol, 1.0 eq) and 6-(trifluoromethyl)pyridine-2-carboxylic acid (110 mg, 0.577 mmol, 1.5 eq) in THF (10 mL) was added 50 wt% T₃P in EtOAc (379 mg, 1.19 mmol, 3.0 eq) at rt. The mixture was stirred at 50°C for 3 h. Upon completion, the mixture was concentrated under reduced pressure. The residue was purified by column chromatography to afford the title compound (white solid, 54 mg, yield 30%). **¹H NMR** (400 MHz, CDCl₃) δ 9.68 (s, 1H), 8.68 (s, 1H), 8.48 (d, *J =* 7.8 Hz, 1H), 8.14 (s, 1H), 7.89 (d, *J* = 7.8 Hz, 1H), 7.72 (d, *J* = 8.0 Hz, 2H), 7.44 (t, *J =* 7.9 Hz, 2H), 7.26 (d, *J =* 4.4 Hz, 1H), 2.85 (q, *J =* 7.6 Hz, 2H), 1.43 (t, *J =* 7.6 Hz, 3H). LCMS (ESI): m/z 361 [M+H]⁺.

### Preparation Example 68. Synthesis of Compound A68

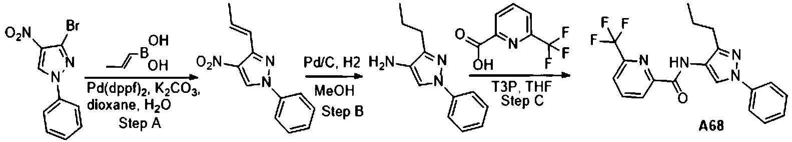

### Step A: (E)-4-nitro-1-phenyl-3-(prop-1-en-1-yl)-1H-pyrazole

To a solution of 3-bromo-4-nitro-1-phenyl-1H-pyrazole (100 mg, 0.373 mmol, 1.0 eq) and (E)-prop-1-en-1-ylboronic acid (48 mg, 0.559 mmol, 1.5 eq) in 1,4-dioxane (4 mL) and water (1 mL) were added potassium carbonate (103 mg, 0.746 mmol, 2.0 eq) and Pd(dppf)Cl₂ (27 mg, 0.037 mmol, 0.1 eq) at rt. The mixture was stirred at 100°C under N₂ for 10 h. Upon completion, the mixture was cooled to rt, diluted in water, extracted with EtOAc, washed with saturated aqueous NaCl, dried over anhydrous Na₂SO₄, and concentrated under reduced pressure. The residue was purified by column chromatography to afford the title compound (white solid, 70 mg, yield 82%).

### Step B: 1-phenyl-3-propyl-1H-pyrazol-4-amine

To a solution of (E)-4-nitro-1-phenyl-3-(prop-1-en-1-yl)-1H-pyrazole (70 mg, 0.305 mmol, 1.0 eq) in MeOH (5 mL) was added 10% palladium on carbon (10 mg) at rt. The reaction solution was purged with H₂ (using a hydrogen balloon) (×3) and stirred under H₂ at rt for 5 h. Upon completion, the mixture was filtered, and the filtrate was concentrated under reduced pressure to afford the title compound (brown oil, 50 mg, yield 81%, crude), which was used directly without purification.

### Step C: N-(1-phenyl-3-propyl-1H-pyrazol-4-yl)-6-(trifluoromethyl)picolinamide

To a solution of 1-phenyl-3-propyl-1H-pyrazol-4-amine (50 mg, 0.248 mmol, 1.0 eq) and 6-(trifluoromethyl)pyridine-2-carboxylic acid (57 mg, 0.298 mmol, 1.20 eq) in THF (10 mL) was added 50 wt% T₃P in EtOAc (379 mg, 0.745 mmol, 3.0 eq) at rt. The mixture was stirred at 50°C for 3 h. Upon completion, the mixture was concentrated under reduced pressure. The residue was purified by column chromatography to afford the title compound (white solid, 26.4 mg, yield 28%). **¹H NMR** (400 MHz, CDCl₃) δ 9.73 (s, 1H), 8.70 (s, 1H), 8.47 (d, *J* = 7.8 Hz, 1H), 8.15 (t, *J* = 7.8 Hz, 1H), 7.89 (d, *J* = 7.8 Hz, 1H), 7.72 *(d, J =* 7.7 Hz, 2H), 7.45 (t, *J =* 7.9 Hz, 2H), 7.32-7.21 (t, *J =* 7.7 Hz, 1H), 2.82 (t, *J =* 7.5 Hz, 2H), 1.85 (dd, *J* = 14.9, 7.4 Hz, 2H), 1.09 (t, *J =* 7.4 Hz, 3H). LCMS (ESI): m/z 375 [M+H]⁺.

### Preparation Example 69. Synthesis of Compound A69

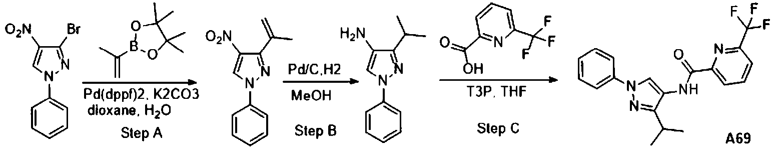

### Step A: 4-nitro-1-phenyl-3-(prop-1-en-2-yl)-1H-pyrazole

To a solution of 3-bromo-4-nitro-1-phenyl-1H-pyrazole (200 mg, 0.746 mmol, 1.0 eq) and 4,4,5,5-tetramethyl-2-(prop-1-en-2-yl)-1,3,2-dioxaborolane (163 mg, 0.97 mmol, 1.3 eq) in 1,4-dioxane (4 mL) and water (1 mL) were added potassium carbonate (309 mg, 2.238 mmol, 3.0 eq) and Pd(dppf)Cl₂ (55 mg, 0.075 mmol, 0.1 eq) at rt. The mixture was stirred at 100°C under N₂ for 2 h. Upon completion, the mixture was cooled to rt, diluted in water, extracted with EtOAc, washed with saturated aqueous NaCl, dried over anhydrous Na₂SO₄, and concentrated under reduced pressure. The residue was purified by column chromatography to afford the title compound (yellow solid, 100 mg, yield 58%).

### Step B: 3-isopropyl-1-phenyl-1H-pyrazol-4-amine

To a solution of 4-nitro-1-phenyl-3-(prop-1-en-2-yl)-1H-pyrazole (100 mg, 0.436 mmol, 1.0 eq) in MeOH (10 mL) was added 10% palladium on carbon (10 mg) at rt. The reaction solution was purged with H₂ (using a hydrogen balloon) (×3) and stirred under H₂ at rt for 2 h. Upon completion, the mixture was filtered, and the filtrate was concentrated under reduced pressure to afford the title compound (brown oil, 80 mg, yield 91%, crude), which was used directly without purification.

### Step C: N-(3-isopropyl-1-phenyl-1H-pyrazol-4-yl)-6-(trifluoromethyl)picolinamide

To a solution of 3-isopropyl-1-phenyl-1H-pyrazol-4-amine (80 mg, 0.397 mmol, 1.0 eq) and 6-(trifluoromethyl)pyridine-2-carboxylic acid (91 mg, 0.477 mmol, 1.20 eq) in THF (10 mL) was added 50 wt% T₃P in EtOAc (379 mg, 1.192 mmol, 3.0 eq) at rt. The mixture was stirred at 50°C for 3 h. Upon completion, the mixture was concentrated under reduced pressure. The residue was purified by column chromatography to afford the title compound (white solid, 26.4 mg, yield 28%). **¹H NMR** (400 MHz, CD₃OD) *δ* 8.59 (s, 1H), 8.46 (d, *J* = 8.0 Hz, 1H), 8.31 (t, *J =* 8.0 Hz, 1H), 8.07 (d, *J =* 8.0 Hz, 1H), 7.74 (d, *J =* 8.4 Hz, 2H), 7.48 (t, *J=* 8.0 Hz, 2H), 7.30 (t, *J =* 7.6 Hz, 1H), 3.23-3.16 (m, 1H), 1.42 (d, *J =* 6.8 Hz, 6H). LCMS (ESI): m/z 375 [M+H]⁺.

### Preparation Example 70. Synthesis of Compound A70

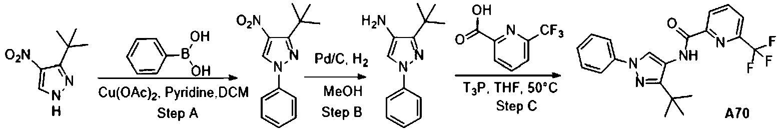

### Step A: 3-(tert-butyl)-4-nitro-1-phenyl-1H-pyrazole

At rt, to a solution of 3-(tert-butyl)-4-nitro-1H-pyrazole (160 mg, 0.946 mmol, 1.00 eq) and phenylboronic acid (173 mg, 1.419 mmol, 1.50 eq) in DCM (4 mL) were added copper acetate (94.41 mg, 0.473 mmol, 0.50 eq) and pyridine (74.81 mg, 0.946 mmol, 1.00 eq). The reaction solution was stirred at 30°C under O₂ for 12 h. Upon completion, the reaction solution was filtered, and the filtrate was diluted in water and extracted with DCM (×3). The combined organic phases were washed with water and saturated aqueous NaCl, dried over anhydrous Na₂SO₄, and concentrated under reduced pressure. The residue was purified by column chromatography to afford the title compound (white solid, 200 mg, yield 86%).

### Step B: 3-(tert-butyl)-1-phenyl-1H-pyrazol-4-amine

At rt, to a solution of 3-(tert-butyl)-4-nitro-1-phenyl-1H-pyrazole (120 mg, 0.489 mmol, 1.00 eq) in MeOH (5 mL) was added 10% palladium on carbon (20 mg). The reaction solution was purged with H₂ (using a hydrogen balloon) (×3) and stirred under H₂ at rt for 1 h. Upon completion, the mixture was filtered, and the filtrate was concentrated under reduced pressure to afford the title compound (yellow solid, 100 mg, crude), which was used directly without purification.

### Step C: N-(3-(tert-butyl)-1-phenyl-1H-pyrazol-4-yl)-6-(trifluoromethyl)picolinamide

At rt, to a solution of 3-(tert-butyl)-1-phenyl-1H-pyrazol-4-amine (100 mg, 0.464 mmol, 1.00 eq) and 6-(trifluoromethyl)pyridine-2-carboxylic acid (133.15 mg, 0.697 mmol, 1.50 eq) in THF (2 mL) was added 50 wt% T₃P in EtOAc (886.71 mg, 1.393 mmol, 3.00 eq). The mixture was stirred at 50°C for 12 h. Upon completion, the mixture was concentrated under reduced pressure. The residue was purified by HPLC to afford the title compound (white solid, 106.4 mg, yield 59%). **¹H NMR** (400 MHz, DMSO-*d*₆) δ 9.96 (s, 1H), 8.78 (s, 1H), 8.47-8.34 (m, 2H), 8.23 (dd, *J =* 6.8, 2.4 Hz, 1H), 7.82 (dd, *J =* 8.4, 1.2 Hz, 2H), 7.54-7.45 (m, 2H), 7.29 (t, *J =* 7.6 Hz, 1H), 1.43 (s, 9H). LCMS (ESI): m/z 389 [M+H]⁺.

### Preparation Example 71. Synthesis of Compound A71

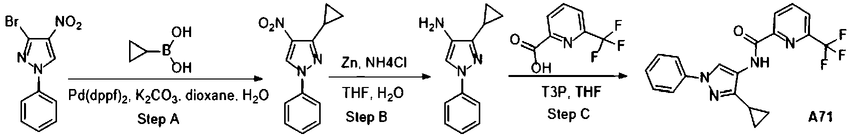

### Step A: 3-cyclopropyl-4-nitro-1-phenyl-1H-pyrazole

At rt, to a solution of 3-bromo-4-nitro-1-phenyl-1H-pyrazole (200 mg, 0.746 mmol, 1.0 eq) and cyclopropylboronic acid (128 mg, 1.492 mmol, 2.0 eq) in 1,4-dioxane (4 mL) and water (1 mL) were added potassium carbonate (309 mg, 2.238 mmol, 3.0 eq) and Pd(dppf)Cl₂ (55 mg, 0.075 mmol, 0.1 eq). The mixture was stirred at 100°C under N₂ for 2 h. Upon completion, the mixture was cooled to rt, diluted in water, extracted with EtOAc, washed with saturated aqueous NaCl, dried over anhydrous Na₂SO₄, and concentrated under reduced pressure. The residue was purified by column chromatography to afford the title compound (white solid, 100 mg, yield 58%).

### Step B: 3-cyclopropyl-1-phenyl-1H-pyrazol-4-amine

At rt, to a solution of 3-cyclopropyl-4-nitro-1-phenyl-1H-pyrazole (100 mg, 0.436 mmol, 1.0 eq) in THF (3 mL) and water (1 mL) were added zinc powder (56 mg, 0.872 mmol, 2.0 eq) and ammonium chloride (47 mg, 0.872 mmol, 2.0 eq). The mixture was stirred at 50°C for 2 h. Upon completion, the mixture was filtered, and the filtrate was concentrated under reduced pressure to afford the title compound (red oil, 80 mg, yield 92%, crude), which was used directly without purification.

### Step C: N-(3-cyclopropyl-1-phenyl-1H-pyrazol-4-yl)-6-(trifluoromethyl)picolinamide

To a solution of 3-cyclopropyl-1-phenyl-1H-pyrazol-4-amine (80 mg, 0.401 mmol, 1.0 eq) and 6-(trifluoromethyl)pyridine-2-carboxylic acid (92 mg, 0.481 mmol, 1.20 eq) in THF (10 mL) was added 50 wt% T₃P in EtOAc (383 mg, 1.203 mmol, 3.0 eq) at rt. The mixture was stirred at 50°C for 12 h. Upon completion, the mixture was concentrated under reduced pressure. The residue was purified by column chromatography to afford the title compound (white solid, 40.1 mg, yield 27%). **¹H NMR** (400 MHz, DMSO-*d*₆) δ 10.14 (s, 1H), 8.67 (s, 1H), 8.39 (q, *J =* 8.0 Hz, 2H), 8.20 (d, *J =* 7.1 Hz, 1H), 7.78 (d, *J* = 8.1 Hz, 2H), 7.47 (t, *J =* 7.8 Hz, 2H), 7.27 (t, *J* = 7.3 Hz, 1H), 2.01 (td, *J =* 8.4, 4.2 Hz, 1H), 0.94 (dd, *J =* 8.2, 2.9 Hz, 2H), 0.88 (dd, *J =* 9.0, 6.2 Hz, 2H). LCMS (ESI): m/z 373 [M+H]⁺.

### Preparation Example 72. Synthesis of Compound A72

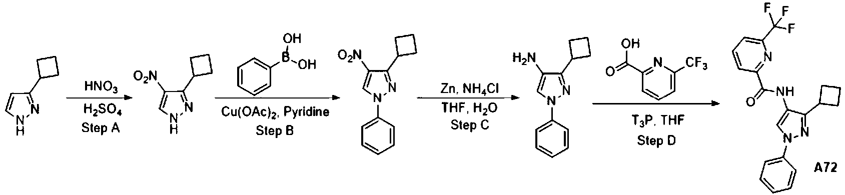

### Step A: 3-cyclobutyl-4-nitro-1H-pyrazole

At 0°C, concentrated sulfuric acid (2 mL) was added to 3-cyclobutyl-1H-pyrazole (120 mg, 0.984 mmol, 1.0 eq), followed by dropwise addition of concentrated nitric acid (1.5 mL). The reaction solution was stirred at 0°C for 2 h. Upon completion, the reaction solution was poured into ice water and extracted with EtOAc (×3). The combined organic phases were washed with water and saturated aqueous NaCl, dried over anhydrous Na₂SO₄, and concentrated under reduced pressure. The residue was purified by reverse-phase column chromatography to afford the title compound (yellow oil, 120 mg, yield 73%).

### Step B: 3-cyclobutyl-4-nitro-1-phenyl-1H-pyrazole

At rt, to a solution of 3-cyclobutyl-4-nitro-1H-pyrazole (120 mg, 0.732 mmol, 1.0 eq) and phenylboronic acid (178.6 mg, 1.464 mmol, 2.0 eq) in DCM (3 mL) were added copper acetate (73.1 mg, 0.366 mmol, 0.5 eq) and pyridine (28 mg, 0.366 mmol, 0.5 eq). The reaction solution was stirred under O₂ at rt for 12 h. Upon completion, the reaction solution was filtered, and the filtrate was diluted in water and extracted with DCM (×3). The combined organic phases were washed with water and saturated aqueous NaCl, dried over anhydrous Na₂SO₄, and concentrated under reduced pressure. The residue was purified by column chromatography to afford the title compound (white solid, 130 mg, yield 74%).

### Step C: 3-cyclobutyl-1-phenyl-1H-pyrazol-4-amine

At rt, to a solution of 3-cyclobutyl-4-nitro-1-phenyl-1H-pyrazole (130 mg, 0.534 mmol, 1.0 eq) in THF (3 mL) and water (1 mL) were added zinc powder (342 mg, 5.34 mmol, 10.0 eq) and ammonium chloride (572 mg, 10.7 mmol, 20.0 eq). The mixture was stirred at 50°C for 3 h. Upon completion, the reaction solution was filtered, and the filtrate was concentrated under reduced pressure to afford the title compound (red oil, 130 mg, crude), which was used directly without purification.

### Step D: N-(3-cyclobutyl-1-phenyl-1H-pyrazol-4-yl)-6-(trifluoromethyl)picolinamide

To a solution of 3-cyclobutyl-1-phenyl-1H-pyrazol-4-amine (130 mg, 0.610 mmol, 1.0 eq) and 6-(trifluoromethyl)pyridine-2-carboxylic acid (140 mg, 0.732 mmol, 1.2 eq) in THF (10 mL) was added 50 wt% T₃P in EtOAc (582 mg, 0.915 mmol, 1.5 eq) at rt. The mixture was stirred at 50°C for 4 h. Upon completion, the mixture was concentrated under reduced pressure. The residue was purified by column chromatography to afford the title compound (white solid, 56.2 mg, yield 24%). **¹H NMR** (400 MHz, DMSO-*d*₆) δ 9.93 (s, 1H), 8.69 (s, 1H), 8.42-8.34 (m, 2H), 8.20 (dd, *J* = 6.8, 2.0 Hz, 1H), 7.83 (d, *J =* 7.7 Hz, 2H), 7.49 (t, *J =* 8.0 Hz, 2H), 7.28 (t, *J=* 7.4 Hz, 1H), 3.77-3.67 (m, 1H), 2.36 (td, *J =* 8.8, 6.4 Hz, 4H), 2.12-2.01 (m, 1H), 1.97-1.86 (m, 1H). LCMS (ESI): m/z 387 [M+H]⁺.

### Preparation Example 73. Synthesis of Compound A73

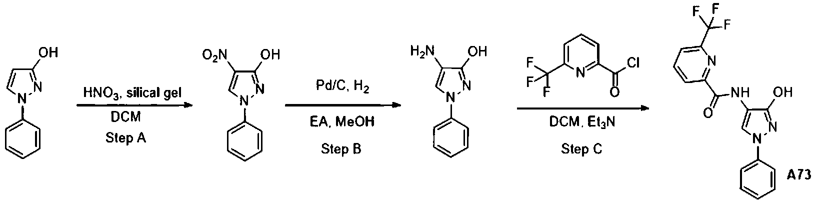

### Step A: 4-nitro-1-phenyl-1H-pyrazol-3-ol

Under N₂ at rt, to a solution of 1-phenylpyrazol-3-ol (500 mg, 3.12 mmol, 1.0 eq) and silica gel powder (1.00 g) in DCM (10 mL) was added 68% concentrated nitric acid (0.5 mL, 3.12 mmol, 1.0 eq). The mixture was stirred at 40°C for 18 h. Upon completion, the mixture was filtered, and the filtrate was quenched with water and extracted with DCM (×3). The combined organic phases were washed with water and saturated aqueous NaCl, dried over anhydrous Na₂SO₄, and concentrated under reduced pressure to afford the title compound (yellow solid, 400 mg, yield 62%, crude), which was used directly without purification.

### Step B: 4-amino-1-phenyl-1H-pyrazol-3-ol

To a solution of 4-nitro-1-phenyl-1H-pyrazol-3-ol (400 mg, 1.95 mmol, 1.0 eq) in MeOH (6 mL) and EtOAc (6 mL) was added 10% palladium on carbon (180 mg) at rt. The mixture was purged with H₂ (using a hydrogen balloon) (×3) and stirred under H₂ at rt for 6 h. Upon completion, the mixture was filtered, and the filtrate was concentrated under reduced pressure to afford the title compound (brown solid, 220 mg, yield 64%, crude), which was used directly without purification.

### Step C: N-(3-hydroxy-1-phenyl-1H-pyrazol-4-yl)-6-(trifluoromethyl)picolinamide

Under N₂ at 0°C, to a solution of 4-amino-1-phenyl-1H-pyrazol-3-ol (220 mg, 1.26 mmol, 1.0 eq) and TEA (0.698 mL, 5.02 mmol, 4.0 eq) in DCM (10 mL) was added 6-(trifluoromethyl)picolinoyl chloride (289 mg, 1.38 mmol, 1.1 eq). The mixture was stirred at 0°C for 1 h. Upon completion, the reaction solution was quenched with saturated aqueous Na₂CO₃, and extracted with DCM (×3). The combined organic phases were washed with water and saturated aqueous NaCl, and dried over anhydrous Na₂SO₄. The residue was purified by column chromatography to afford the title compound (yellow solid, 69.4 mg, yield 16%). **¹H NMR** (400 MHz, DMSO-*d*₆) δ 10.94 (s, 1H), 9.91 (s, 1H), 8.65 (s, 1H), 8.43-8.36 (m, 2H), 8.20 (d, *J =* 7.3 Hz, 1H), 7.71 (d, *J =* 8.4 Hz, 2H), 7.45 (t, *J =* 7.9 Hz, 2H), 7.20 (t, *J =* 7.3 Hz, 1H). LCMS (ESI): m/z 349 [M+H]⁺.

### Preparation Example 74. Synthesis of Compound A74

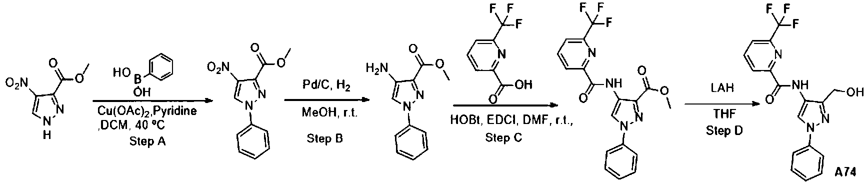

### Step A: Methyl 4-nitro-1-phenyl-1H-pyrazole-3-carboxylate

To a solution of methyl 4-nitro-1H-pyrazole-3-carboxylate (10.0 g, 58.44 mmol, 1.0 eq), phenylboronic acid (14.3 g, 116.88 mmol, 2.0 eq) and copper acetate (15.9 g, 87.66 mmol, 1.5 eq) in DCM (200 mL) was added pyridine (18.8 mL, 233.77 mmol, 4.0 eq) at rt. The reaction solution was stirred at 40°C under O₂ for 18 h. Upon completion, the reaction solution was filtered, and the filtrate was diluted in water and extracted with DCM (×3). The combined organic phases were washed with water and saturated aqueous NaCl, dried over anhydrous Na₂SO₄, and concentrated under reduced pressure. The residue was purified by column chromatography to afford the title compound (yellow solid, 8.2 g, yield 57%).

### Step B: Methyl 4-amino-1-phenyl-1H-pyrazole-3-carboxylate

To a solution of methyl 4-nitro-1-phenyl-1H-pyrazole-3-carboxylate (6.0 g, 24.27 mmol, 1.0 eq) in MeOH (120 mL) was added 10% palladium on carbon (2.6 g, 2.43 mmol, 0.1 eq) at rt. The reaction solution was purged with H₂ (using a hydrogen balloon) (×3) and stirred under H₂ at rt for 18 h. Upon completion, the mixture was filtered, and the filtrate was concentrated under reduced pressure to afford the title compound (yellow oil, 5.3 g, yield 100%, crude), which was used directly without purification.

### Step C: Methyl 1-phenyl-4-(6-(trifluoromethyl)picolinamido)-1H-pyrazole-3-carboxylate

To a solution of methyl 4-amino-1-phenyl-1H-pyrazole-3-carboxylate (5.3 g, 24.40 mmol, 1.0 eq), 6-(trifluoromethyl)picolinic acid (4.7 g, 24.40 mmol, 1.0 eq) and HOBT (4.0 g, 29.28 mmol, 1.2 eq) in DMF (120 mL) was added EDCI (5.6 g, 29.28 mmol, 1.2 eq) at rt. The reaction solution was stirred at rt under N₂ for 18 h. Upon completion, the reaction solution was diluted in water, with the crude product precipitated. The mixture was filtered, and the filter cake was washed with water, slurried with EtOAc, filtered, and dried completely to afford the title compound (gray solid, 6.3 g, yield 66%, crude), which was used directly without purification.

### Step D: N-(3-(hydroxymethyl)-1-phenyl-1H-pyrazol-4-yl)-6-(trifluoromethyl)picolinamide

Under N₂ at 0°C, lithium diisopropylamide (1.0 mL, 1.0 mmol, 2.0 eq) was slowly added to a solution of methyl 1-phenyl-4-(6-(trifluoromethyl)picolinamido)-1H-pyrazole-3-carboxylate (200.0 mg, 0.5 mmol, 1.0 eq) in THF (3 mL). The reaction solution was stirred at 0°C under N₂ for 1 h. Upon completion, the reaction was quenched with water (0.2 mL) and 30% aqueous NaOH (0.2 mL), diluted in water, and extracted with EtOAc (×3). The combined organic phases were washed with water and saturated aqueous NaCl, dried over anhydrous Na₂SO₄, and concentrated under reduced pressure. The residue was purified by HPLC to afford the title compound (white solid, 40.2 mg, yield 22%). **¹H NMR** (400 MHz, DMSO-*d*₆) δ 10.76 (s, 1H), 8.88 (s, 1H), 8.46-8.36 (m, 2H), 8.20 (d, *J =* 6.8 Hz, 1H), 7.81 (d, *J =* 7.8 Hz, 2H), 7.49 (t, *J* = 8.0 Hz, 2H), 7.29 (t, *J* = 7.4 Hz, 1H), 5.87 (t, *J* = 4.9 Hz, 1H), 4.82 (d, *J =* 4.9 Hz, 2H). LCMS (ESI): m/z 363 [M+H]⁺.

### Preparation Example 75. Synthesis of Compound A75

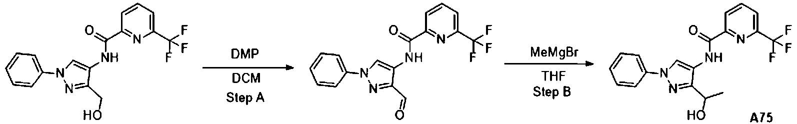

### Step A: N-(3-formyl-1-phenyl-1H-pyrazol-4-yl)-6-(trifluoromethyl)picolinamide

At 0°C, DMP (373.1 mg, 0.8 mmol, 2.0 eq) was added to a solution of N-(3-(hydroxymethyl)-1-phenyl-1H-pyrazol-4-yl)-6-(trifluoromethyl)picolinamide (160.0 mg, 0.4 mmol, 1.0 eq) in DCM (2 mL). The mixture was stirred at rt under N₂ for 1 h, Upon completion, the reaction was quenched with saturated NaHCO₃ solution and stirred for 0.5 h. The mixture was extracted with DCM (×3), and the combined organic phases were washed with water and saturated sodium thiosulfate, dried over anhydrous Na₂SO₄, filtered, and concentrated under reduced pressure to afford the title compound (white solid, 120.0 mg, yield 75%, crude), which was used directly without purification.

### Step B: N-(3-(1-hydroxyethyl)-1-phenyl-1H-pyrazol-4-yl)-6-(trifluoromethyl)picolinamide

Under N₂ at -78°C, a 2 M solution of methylmagnesium bromide in diethyl ether (1.0 mL, 2.0 mmol, 6.0 eq) was slowly added to a solution of N-(3-formyl-1-phenyl-1H-pyrazol-4-yl)-6-(trifluoromethyl)picolinamide (120.0 mg, 0.3 mmol, 1.0 eq) in THF (5 mL). The reaction solution was stirred at -78°C under N₂ for 2 h. Upon completion, the reaction was quenched with saturated aqueous NH₄Cl and extracted with EtOAc (×3). The combined organic phases were washed with water and saturated aqueous NaCl, dried over anhydrous Na₂SO₄, filtered, and concentrated under reduced pressure. The residue was purified by HPLC to afford the title compound (white solid, 50.6 mg, yield 40%). **¹H NMR** (400 MHz, DMSO-*d*₆) δ 11.02 (s, 1H), 8.89 (s, 1H), 8.46-8.34 (m, 2H), 8.19 (d, *J =* 7.6 Hz, 1H), 7.81 (d, *J* = 7.9 Hz, 2H), 7.49 (t, *J =* 7.9 Hz, 2H), 7.29 (t, *J =* 7.4 Hz, 1H), 6.09 (d, *J =* 4.3 Hz, 1H), 5.18-5.06 (m, 1H), 1.54 (d, *J* = 6.5 Hz, 3H). LCMS (ESI): m/z 377 [M+H]⁺.

### Preparation Example 76. Synthesis of Compound A76

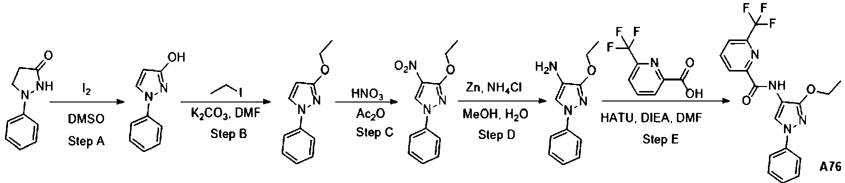

### Step A: 1-Phenyl-1H-pyrazol-3-ol

To a solution of 1-phenylpyrazolin-3-one (10 g, 61.6 mmol, 1.0 eq) in dimethyl sulfoxide (100 mL) was added iodine (0.8 g, 3.1 mmol, 0.05 eq) at rt. The reaction solution was stirred at 100°C for 16 h. Upon completion, the reaction solution was cooled to rt, quenched with saturated sodium thiosulfate, extracted with EtOAc twice. The combined organic phases were washed with water and saturated aqueous NaCl, dried over anhydrous Na₂SO₄, filtered, and concentrated under reduced pressure. The residue was purified by column chromatography to afford the title compound (yellow solid, 8.5 g, yield 86%).

### Step B: 3-ethoxy-1-phenyl-1H-pyrazole

To a solution of 1-phenyl-1H-pyrazol-3-ol (0.7 g, 4.4 mmol, 1.0 eq) in DMF (10 mL) were added iodoethane (1.0 g, 6.5 mmol, 1.5 eq) and potassium carbonate (1.2 g, 8.7 mmol, 2.0 eq) at rt. The reaction solution was stirred at rt under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (off-white solid, 660 mg, yield 80%).

### Step C: 3-ethoxy-4-nitro-1-phenyl-1H-pyrazole

At -20°C, 60% nitric acid (0.1 mL, 1.7 mmol, 2.0 eq) was added dropwise to a solution of 3-ethoxy-1-phenyl-1H-pyrazole (160 mg, 0.8 mmol, 1.0 eq) in acetic anhydride (5 mL). The reaction solution was stirred at -20°C for 0.5 h. Upon completion, the reaction solution was poured into ice water and extracted with EtOAc (×3). The combined organic phases were washed with water and saturated aqueous NaCl, dried over anhydrous Na₂SO₄, filtered, and concentrated under reduced pressure. The residue was purified by column chromatography to afford the title compound (colorless oil, 90 mg, yield 45%).

### Step D: 3-ethoxy-1-phenyl-1H-pyrazol-4-amine

To a solution of 3-ethoxy-4-nitro-1-phenyl-1H-pyrazole (600 mg, 2.6 mmol, 1.0 eq) in MeOH (6 mL) were added zinc powder (841 mg, 12.9 mmol, 5.0 eq) and ammonium chloride (1.4 g, 25.7 mmol, 10 eq) in water (3 mL) at rt. The mixture was stirred at 70°C for 1 h. Upon completion, the mixture was filtered and extracted with EtOAc. The organic phases were washed with water and saturated aqueous NaCl, dried over anhydrous Na₂SO₄ and filtered. The filtrate was concentrated under reduced pressure to afford the title compound (yellow solid, 600 mg, crude), which was used directly without purification.

### Step E: N-(3-ethoxy-1-phenyl-1H-pyrazol-4-yl)-6-(trifluoromethyl)picolinamide

To a solution of 6-(trifluoromethyl)picolinic acid (180 mg, 0.9 mmol, 1.0 eq) and 3-ethoxy-1-phenyl-1H-pyrazol-4-amine (229.7 mg, 1.1 mmol, 1.2 eq) in DMF (5 mL) were added DIEA (365.2 mg, 2.8 mmol, 3.0 eq) and HATU (429.8 mg, 1.1 mmol, 1.2 eq) at rt. The reaction solution was stirred at rt under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (off-white solid, 63 mg, yield 18%). **¹H NMR** (400 MHz, DMSO-*d*₆) δ 9.72 (s, 1H), 8.67 (s, 1H), 8.44-8.30 (m, 2H), 8.20 (dd, *J =* 6.9, 1.9 Hz, 1H), 7.75 (d, *J* = 7.8 Hz, 2H), 7.46 (t, *J =* 8.0 Hz, 2H), 7.22 (t, *J =* 7.4 Hz, 1H), 4.39 (q, *J =* 7.0 Hz, 2H), 1.41 (t, *J =* 7.0 Hz, 3H). LCMS (ESI): m/z 377 [M+H]⁺.

### Preparation Example 77. Synthesis of Compound A77

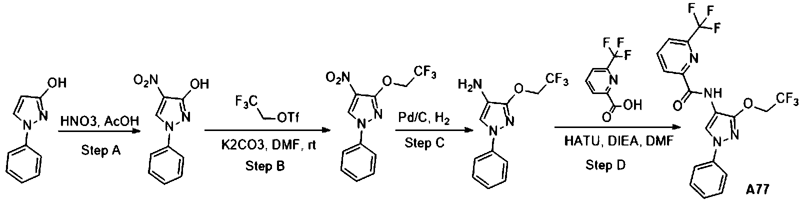

### Step A: 4-nitro-1-phenyl-1H-pyrazol-3-ol

To a solution of 1-phenyl-1H-pyrazol-3-ol (300 mg, 1.87 mmol, 1.0 eq) in AcOH (10 mL) was added 60% nitric acid (983 mg, 4.68 mmol, 2.5 eq) at rt. The mixture was stirred at rt for 1 h. Upon completion, the reaction was quenched with saturated aqueous NaHCO₃ and extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow liquid, 260 mg, yield 68%).

### Step B: 4-nitro-1-phenyl-3-(2,2,2-trifluoroethoxy)-1H-pyrazole

To a solution of 4-nitro-1-phenyl-1H-pyrazol-3-ol (800 mg, 3.899 mmol, 1.0 eq) in DMF (10 mL) were added potassium carbonate (1077 mg, 7.80 mmol, 2.0 eq) and 2,2,2-trifluoroethyl trifluoromethanesulfonate (1.36 g, 5.85 mmol, 1.5 eq) at rt. The mixture was stirred at rt for 2 h. Upon completion, the mixture was cooled to rt, quenched with water and extracted with EtOAc (×3). The combined organic phases were washed with water and saturated aqueous NaCl, dried over anhydrous Na₂SO₄, and concentrated under reduced pressure to afford the title compound (white liquid, 1.0 g, crude), which was used directly without purification.

### Step C: 1-phenyl-3-(2,2,2-trifluoroethoxy)-1H-pyrazol-4-amine

To a solution of 4-nitro-1-phenyl-3-(2,2,2-trifluoroethoxy)-1H-pyrazole (500 mg, 1.74 mmol, 1.0 eq) in MeOH (5 mL) was added 5% palladium on carbon (185 mg, 0.174 mmol, 0.1 eq) at rt. The reaction solution was purged with H₂ (using a hydrogen balloon) (×3) and stirred under H₂ at rt for 18 h. Upon completion, the mixture was filtered, and the filtrate was concentrated under reduced pressure to afford the title compound (yellow solid, 370 mg, crude), which was used directly without purification.

### Step D: N-(1-phenyl-3-(2,2,2-trifluoroethoxy)-1H-pyrazol-4-yl)-6-(trifluoromethyl)picolinamide

To a solution of 1-phenyl-3-(2,2,2-trifluoroethoxy)-1H-pyrazol-4-amine (150 mg, 0.583 mmol, 1.0 eq) and 6-(trifluoromethyl)picolinic acid (111 mg, 0.583 mmol, 1.0 eq) in DMF (5 mL) were added DIEA (226 mg, 1.75 mmol, 3.0 eq) and HATU (244 mg, 0.641 mmol, 1.1 eq) at rt. The reaction solution was stirred at rt under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by preparative column chromatography to afford the title compound (white solid, 200 mg, yield 80%). **¹H NMR** (400 MHz, DMSO-*d*₆) δ 9.89 (s, 1H), 8.72 (s, 1H), 8.44-8.33 (m, 2H), 8.20 (dd, *J* = 6.8, 1.8 Hz, 1H), 7.79 (d, *J =* 7.9 Hz, 2H), 7.49 (t, *J =* 7.9 Hz, 2H), 7.27 (t, *J* = 7.4 Hz, 1H), 5.05 (q, *J =* 8.9 Hz, 2H). LCMS (ESI): m/z 431 [M+H]⁺.

### Preparation Example 78. Synthesis of Compound A78

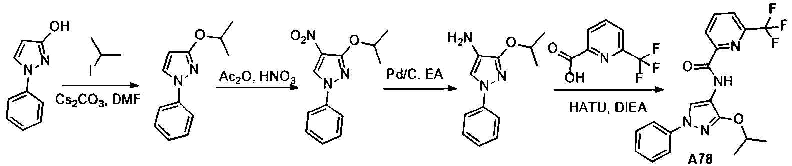

### Step A: 3-isopropoxy-1-phenyl-1H-pyrazole

To a solution of 1-phenyl-1H-pyrazol-3-ol (1.0 g, 6.24 mmol, 1.0 eq) in DMF (30 mL) were added cesium carbonate (4.07 g, 12.5 mmol, 2.0 eq) and 2-iodopropane (3.12 mL, 31.2 mmol, 5.0 eq) at rt. The mixture was stirred at 50°C for 18 h. Upon completion, the mixture was cooled to rt, quenched with water and extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow liquid, 1.1 g, yield 87%).

### Step B: 3-isopropoxy-4-nitro-1-phenyl-1H-pyrazole

At 0°C, concentrated nitric acid (1.58 g, 16.3 mmol, 3.0 eq) was added to a solution of 3-isopropoxy-1-phenyl-1H-pyrazole (1.1 g, 5.44 mmol, 1.0 eq) in acetic anhydride (10 mL). The mixture was stirred at rt for 1 h. Upon completion, the reaction solution was poured into ice water and extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow solid, 700 mg, yield 52%).

### Step C: 3-isopropoxy-1-phenyl-1H-pyrazol-4-amine

To a solution of 3-isopropoxy-4-nitro-1-phenyl-1H-pyrazole (200 mg, 0.81 mmol, 1.0 eq) in EtOAc (15 mL) was added 5% palladium on carbon (120 mg, 1.13 mmol, 1.4 eq) at rt. The reaction solution was purged with H₂ (using a hydrogen balloon) (×3) and stirred under H₂ at rt for 2 h. Upon completion, the mixture was filtered and concentrated under reduced pressure. The residue was purified by column chromatography to afford the title compound (yellow solid, 100 mg, yield 57%).

### Step D: N-(3-isopropoxy-1-phenyl-1H-pyrazol-4-yl)-6-(trifluoromethyl)picolinamide

To a solution of 3-isopropoxy-1-phenyl-1H-pyrazol-4-amine (100 mg, 0.46 mmol, 1.0 eq) and 6-(trifluoromethyl)picolinic acid (88 mg, 0.46 mmol, 1.0 eq) in DMF (5 mL) were added DIEA (178 mg, 1.38 mmol, 3.0 eq) and HATU (175 mg, 0.46 mmol, 1.0 eq) at rt. The reaction solution was stirred at rt under N₂ for 0.5 h. Upon completion, the reaction solution was diluted in EtOAc, washed sequentially with water and saturated aqueous NaCl, dried over anhydrous Na₂SO₄, and concentrated under reduced pressure. The residue was purified by preparative HPLC to afford the title compound (yellow solid, 68 mg, yield 38%). The residue was purified by column chromatography to afford the title compound (yellow solid, 70 mg, yield 20%). **¹H NMR** (400 MHz, DMSO-*d*₆) δ 9.66 (s, 1H), 8.67 (s, 1H), 8.44-8.34 (m, 2H), 8.20 (dd, *J* = 6.9, 1.9 Hz, 1H), 7.75 (d, *J =* 7.9 Hz, 2H), 7.47 (t, *J =* 7.9 Hz, 2H), 7.23 (t, *J =* 7.4 Hz, 1H), 5.03-4.94 (m, 1H), 1.41 (d, *J =* 6.1 Hz, 6H). LCMS (ESI): m/z 391 [M+H]⁺.

### Preparation Example 79. Synthesis of Compound A79

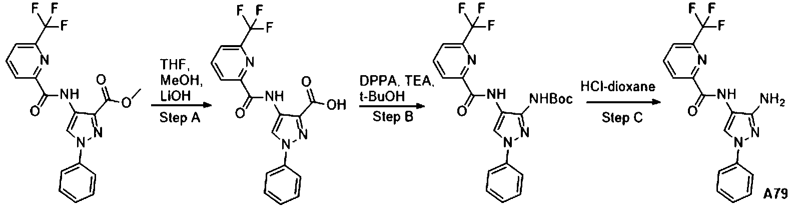

### Step A: 1-phenyl-4-(6-(trifluoromethyl)picolinamido)-1H-pyrazole-3-carboxylic acid

At 0°C, to a mixed solution of methyl 1-phenyl-4-(6-(trifluoromethyl)picolinamido)-1H-pyrazole-3-carboxylate (1.2 g, 3.07 mmol, 1.0 eq) in MeOH (50 mL) and water (20 mL) was added lithium hydroxide (220 mg, 9.22 mmol, 3.0 eq). The reaction solution was stirred at rt for 4 h. Upon completion, the mixture was concentrated under reduced pressure to remove MeOH, diluted in water, and adjusted to pH=4 with 1 N HCl until the product fully precipitated. The mixture was filtered, and the filter cake was dried completely to afford the title compound (white solid, 1.1 g, yield 95%, crude), which was used directly without purification.

### Step B: Tert-butyl (1-phenyl-4-(6-(trifluoromethyl)picolinamido)-1H-pyrazol-3-yl)carbamate

At rt, to a solution of 1-phenyl-4-(6-(trifluoromethyl)picolinamido)-1H-pyrazole-3-carboxylic acid (900 mg, 2.39 mmol, 1.0 eq) and TEA (1.66 mL, 12.0 mmol, 5.0 eq) in tert-butanol (50 mL) was added diphenylphosphoryl azide (1,316 mg, 4.78 mmol, 2.0 eq). The reaction solution was stirred at 90°C under N₂ for 18 h. Upon completion, the mixture was concentrated under reduced pressure to remove tert-butanol, diluted in water, and extracted with EtOAc (×3). The combined organic phases were washed with water and saturated aqueous NaCl, dried over anhydrous Na₂SO₄, filtered, and concentrated under reduced pressure. The residue was purified by column chromatography and slurried with petroleum ether/ethyl acetate (10 mL, 1/20) to afford the title compound (white solid, 230 mg, yield 21%).

### Step C: N-(3-amino-1-phenyl-1H-pyrazol-4-yl)-6-(trifluoromethyl)picolinamide

At 0°C, 4 M HCl in 1,4-dioxane (5 mL) was added to a solution of tert-butyl (1-phenyl-4-(6-(trifluoromethyl)picolinamido)-1H-pyrazol-3-yl)carbamate (230 mg, 0.51 mmol, 1.0 eq) in 1,4-dioxane (1 mL). The reaction solution was stirred at rt under N₂ for 2 h. Upon completion, the reaction solution was concentrated under reduced pressure, diluted in saturated NaHCO₃ solution and extracted with DCM (×3). The combined organic phases were washed with water and saturated aqueous NaCl, dried over anhydrous Na₂SO₄, filtered, and concentrated under reduced pressure. The residue was purified by preparative HPLC to afford the title compound (yellow solid, 80 mg, yield 45%). **¹H NMR** (400 MHz, DMSO-*d*₆) δ 10.14 (s, 1H), 8.57 (s, 1H), 8.42-8.29 (m, 2H), 8.17 (dd, *J =* 7.2, 1.6 Hz, 1H), 7.67-7.65 (m, 2H), 7.46-7.36 (m, 2H), 7.16-7.13 (m, 1H), 5.34 (s, 2H). LCMS (ESI): m/z 348 [M+H]⁺.

### Preparation Example 80. Synthesis of Compound A80

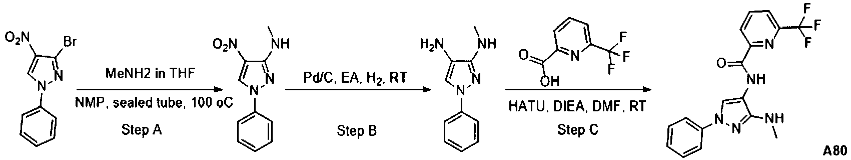

### Step A: N-methyl-4-nitro-1-phenyl-1H-pyrazol-3-amine

At 0°C, to a solution of 3-bromo-4-nitro-1-phenyl-1H-pyrazole (500 mg, 1.87 mmol, 1.0 eq) in N-methylpyrrolidone (10 mL) were added DIEA (1,205 mg, 9.4 mmol, 5.0 eq) and 2 M methylamine in THF (5 mL, 10.0 mmol, 5.4 eq). The mixture was stirred in a sealed tube at 100°C for 18 h. Upon completion, the mixture was cooled to rt, extracted with EtOAc, washed with saturated aqueous NaCl, dried over anhydrous Na₂SO₄, and concentrated under reduced pressure. The residue was purified by column chromatography to afford the title compound (yellow solid, 160 mg, yield 39%).

### Step B: N³-methyl-1-phenyl-1H-pyrazole-3,4-diamine

At rt, to a solution of N-methyl-4-nitro-1-phenyl-1H-pyrazol-3-amine (160 mg, 0.73 mmol, 1.0 eq) in EtOAc (25 mL) was added 10% palladium on carbon (80 mg, 0.75 mmol, 1.0 eq). The reaction solution was purged with H₂ (using a hydrogen balloon) (×3) and stirred under H₂ at rt for 18 h. Upon completion, the mixture was filtered, and the filtrate was concentrated under reduced pressure to afford the title compound (yellow oil, 150 mg, yield 98%, crude), which was used directly without purification.

### Step C: N-(3-(methylamino)-1-phenyl-1H-pyrazol-4-yl)-6-(trifluoromethyl)picolinamide

At rt, to a solution of N³-methyl-1-phenyl-1H-pyrazole-3,4-diamine (150 mg, 0.72 mmol, 1.0 eq) and 6-(trifluoromethyl)picolinic acid (137.1 mg, 0.72 mmol, 1.0 eq) in DMF (10 mL) were added DIEA (278.1 mg, 2.2 mmol, 3.0 eq) and HATU (272.7 mg, 0.72 mmol, 1.0 eq). The reaction solution was stirred at rt under N₂ for 0.5 h. Upon completion, the reaction solution was diluted in EtOAc, washed sequentially with water and saturated aqueous NaCl, dried over anhydrous Na₂SO₄, and concentrated under reduced pressure. The residue was purified by preparative HPLC to afford the title compound (yellow solid, 150 mg, yield 58%). **¹H NMR** (400 MHz, DMSO-*d*₆) δ 10.11 (s, 1H), 8.58 (s, 1H), 8.40-8.33 (m, 2H), 8.22-8.09 (m, 1H), 7.70 (d, *J =* 8.1 Hz, 2H), 7.42 (t, *J* = 7.9 Hz, 2H), 7.14 (t, *J =* 7.3 Hz, 1H), 5.77-5.74 (m, 1H), 2.86 (d, *J* = 5.0 Hz, 3H). LCMS (ESI): m/z 362 [M+H]⁺.

### Preparation Example 81. Synthesis of Compound A81

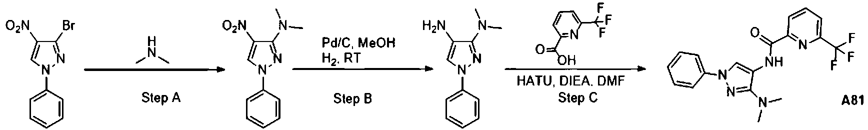

### Step A: N,N-dimethyl-4-nitro-1-phenyl-1H-pyrazol-3-amine

At 0°C, to a solution of 3-bromo-4-nitro-1-phenyl-1H-pyrazole (200 mg, 0.746 mmol, 1.0 eq) in N-methylpyrrolidone (5 mL) was added dimethylamine (343 mg, 7.46 mmol, 10 eq). The mixture was stirred in a sealed tube at 80°C for 1 h. Upon completion, the mixture was cooled to rt, extracted with EtOAc, washed with saturated aqueous NaCl, dried over anhydrous Na₂SO₄, and concentrated under reduced pressure. The residue was purified by column chromatography to afford the title compound (yellow solid, 60 mg, yield 35%).

### Step B: N³,N³-dimethyl-1-phenyl-1H-pyrazole-3,4-diamine

At rt, to a solution of N,N-dimethyl-4-nitro-1-phenyl-1H-pyrazol-3-amine (70 mg, 0.30 mmol, 1.0 eq) in MeOH (10 mL) was added 10% palladium on carbon (40 mg, 0.38 mmol, 1.3 eq). The reaction solution was purged with H₂ (using a hydrogen balloon) (×3) and stirred under H₂ at rt for 18 h. Upon completion, the mixture was filtered, and the filtrate was concentrated under reduced pressure to afford the title compound (yellow oil, 60 mg, yield 98%, crude), which was used directly without purification.

### Step C: N-(3-(dimethylamino)-1-phenyl-1H-pyrazol-4-yl)-6-(trifluoromethyl)picolinamide

At rt, to a solution of N³,N³-dimethyl-1-phenyl-1H-pyrazole-3,4-diamine (60 mg, 0.30 mmol, 1.0 eq) and 6-(trifluoromethyl)picolinic acid (56.7 mg, 0.30 mmol, 1.0 eq) in DMF (4 mL) were added DIEA (115 mg, 0.89 mmol, 3.0 eq) and HATU (112.8 mg, 0.30 mmol, 3.0 eq). The reaction solution was stirred at rt under N₂ for 0.5 h. Upon completion, the reaction solution was diluted in EtOAc, washed sequentially with water and saturated aqueous NaCl, dried over anhydrous Na₂SO₄, and concentrated under reduced pressure. The residue was purified by preparative HPLC to afford the title compound (yellow solid, 68 mg, yield 28%). **¹H NMR** (400 MHz, DMSO-*d*₆) δ 9.90 (s, 1H), 8.59 (s, 1H), 8.42-8.34 (m, 2H), 8.22-8.17 (m, 1H), 7.75 (d, *J =* 8.2 Hz, 2H), 7.45 (t, J = 7.9 Hz, 2H), 7.20 (t, *J =* 7.4 Hz, 1H), 2.86 (s, 6H). LCMS (ESI): m/z 376 [M+H]⁺.

### Preparation Example 82. Synthesis of Compound A82

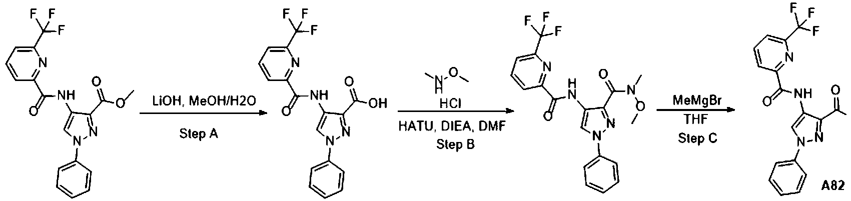

### Step A: 1-phenyl-4-(6-(trifluoromethyl)picolinamido)-1H-pyrazole-3-carboxylic acid

At 0°C, to a mixed solution of methyl 1-phenyl-4-(6-(trifluoromethyl)picolinamido)-1H-pyrazole-3-carboxylate (200 mg, 0.51 mmol, 1.0 eq) in MeOH (5 mL) and water (5 mL) was added lithium hydroxide (24.5 mg, 1.02 mmol, 2.0 eq). The reaction solution was stirred at rt under N₂ for 2 h. Upon completion, the reaction solution was adjusted to pH 5-6 using 1 N HCl. The mixture was extracted with EtOAc (×3), and the combined organic phases were washed with water and saturated aqueous NaCl, dried over anhydrous Na₂SO₄, and concentrated under reduced pressure to afford the title compound (white solid, 150 mg, yield 78%, crude), which was used directly without purification.

### Step B: N-(3-(methoxy(methyl)carbamoyl)-1-phenyl-1H-pyrazol-4-yl)-6-(trifluoromethyl)picolinamide

At rt, to a solution of 1-phenyl-4-(6-(trifluoromethyl)picolinamido)-1H-pyrazole-3-carboxylic acid (150 mg, 0.40 mmol, 1.0 eq) and N,O-dimethylhydroxylamine hydrochloride (43 mg, 0.44 mmol, 1.1 eq) in DMF (5 mL) were added DIEA (155 mg, 1.20 mmol, 3.0 eq) and HATU (152 mg, 0.40 mmol, 1.0 eq). The reaction solution was stirred at rt under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 130 mg, yield 78%).

### Step C: N-(3-acetyl-1-phenyl-1H-pyrazol-4-yl)-6-(trifluoromethyl)picolinamide

At 0°C under N₂, a solution of methylmagnesium bromide in diethyl ether (3 M, 0.41 mL, 1.24 mmol, 4.0 eq) was slowly added to a solution of N-(3-(methoxy(methyl)carbamoyl)-1-phenyl-1H-pyrazol-4-yl)-6-(trifluoromethyl)picolinamide (130 mg, 0.31 mmol, 1.0 eq) in THF (5 mL). The reaction solution was stirred at rt under N₂ for 3 h. Upon completion, the reaction was quenched with saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by HPLC to afford the title compound (white solid, 56.2 mg, yield 48%). **¹H NMR** (400 MHz, DMSO-*d*₆) δ 11.48 (s, 1H), 9.06 (s, 1H), 8.47-8.36 (m, 2H), 8.23 (d, *J =* 7.4 Hz, 1H), 7.98 (d, *J =* 8.0 Hz, 2H), 7.57 (t, *J =* 7.8 Hz, 2H), 7.44 (t, *J =* 7.3 Hz, 1H), 2.66 (s, 3H). LCMS (ESI) m/z: 375 [M+H]⁺.

### Preparation Example 83. Synthesis of Compound A83

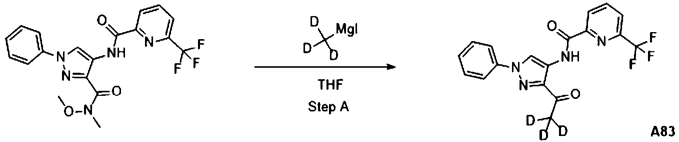

### Step A: N-(3-(acetyl-d3)-1-phenyl-1H-pyrazol-4-yl)-6-(trifluoromethyl)picolinamide

At 0°C under N₂, a solution of (methyl-d3) magnesium iodide in diethyl ether (1 M, 1.6 mL, 1.60 mmol, 4.0 eq) was slowly added to a solution of N-(3-(methoxy(methyl)carbamoyl)-1-phenyl-1H-pyrazol-4-yl)-6-(trifluoromethyl)picolinamide (170 mg, 0.40 mmol, 1.0 eq) in THF (10 mL). The reaction solution was stirred at rt under N₂ for 3 h. Upon completion, the reaction was quenched with saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by HPLC to afford the title compound (white solid, 79.8 mg, yield 52%). **¹H NMR** (400 MHz, DMSO-*d*₆) δ 11.48 (s, 1H), 9.06 (s, 1H), 8.50-8.37 (m, 2H), 8.24 (d, *J* = 7.5 Hz, 1H), 7.98 (d, *J =* 7.8 Hz, 2H), 7.58 (t, *J =* 7.7 Hz, 2H), 7.44 (t, *J =* 7.3 Hz, 1H). LCMS (ESI): m/z 378 [M+H]⁺.

### Preparation Example 84. Synthesis of Compound A84

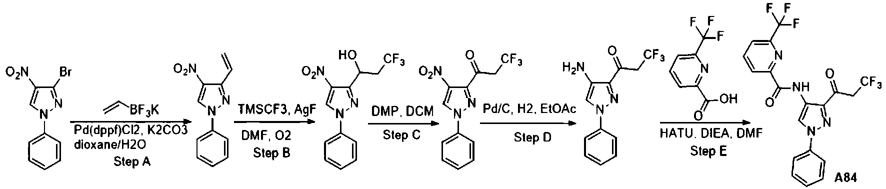

### Step A: 4-nitro-1-phenyl-3-vinyl-1H-pyrazole

At rt, to a solution of 3-bromo-4-nitro-1-phenyl-1H-pyrazole (2 g, 7.46 mmol, 1.0 eq) and potassium vinyltrifluoroborate (2.0 g, 14.92 mmol, 2.0 eq) in 1,4-dioxane (20 mL) and water (5 mL) were added potassium carbonate (2.0 g, 14.92 mmol, 2.0 eq) and Pd(dppf)Cl₂ (545.3 mg, 0.746 mmol, 0.1 eq). The mixture was stirred at 90°C under N₂ for 2 h. Upon completion, the mixture was cooled to rt, diluted in water, extracted with EtOAc, washed with saturated aqueous NaCl, dried over anhydrous Na₂SO₄, and concentrated under reduced pressure. The residue was purified by column chromatography to afford the title compound (yellow solid, 1.5 g, yield 93%).

### Step B: 3,3,3-trifluoro-1-(4-nitro-1-phenyl-1H-pyrazol-3-yl)propan-1-ol

Silver fluoride (1.32 g, 10.4 mmol, 1.5 eq) was added to a dry Schlenk tube, sealed with a septum, and exchanged with oxygen (5×). A solution of 4-nitro-1-phenyl-3-vinyl-1H-pyrazole (1.5 g, 6.94 mmol, 1.0 eq) and (trifluoromethyl)trimethylsilane (1.97 g, 13.88 mmol, 2.0 eq) was dissolved in anhydrous DMF (20 mL) and quickly added to the above Schlenk tube. The mixture was stirred at rt for 12 h. Upon completion, the mixture was diluted in EtOAc, and filtered. The filtrate was washed with saturated aqueous NaCl, dried over anhydrous Na₂SO₄, and concentrated under reduced pressure. The residue was purified by column chromatography to afford the title compound (yellow solid, 500 mg, yield 24%).

### Step C: 3,3,3-Trifluoro-1-(4-nitro-1-phenyl-1H-pyrazol-3-yl)propan-1-one

At 0°C, Dess-Martin periodinane (840 mg, 1.98 mmol, 1.2 eq) was added in portions to a solution of 3,3,3-trifluoro-1-(4-nitro-1-phenyl-1H-pyrazol-3-yl)propan-1-ol (500 mg, 1.65 mmol, 1.0 eq) in DCM (10 mL) . The mixture was stirred at rt for 2 h. Upon completion, the mixture was filtered, and the filtrate was concentrated under reduced pressure. The residue was purified by column chromatography to afford the title compound (yellow solid, 400 mg, yield 81%).

### Step D: 1-(4-amino-1-phenyl-1H-pyrazol-3-yl)-3,3-trifluoropropan-1-one

At rt, to a solution of 3,3,3-trifluoro-1-(4-nitro-1-phenyl-1H-pyrazol-3-yl)propan-1-one (400 mg, 1.34 mmol, 1.0 eq) in EtOAc (20 mL) was added 10% palladium on carbon (285 mg, 0.267 mmol, 0.2 eq). The reaction solution was purged with H₂ (using a hydrogen balloon) (×3) and stirred under H₂ at rt for 12 h. Upon completion, the mixture was filtered, and the filtrate was concentrated under reduced pressure to afford the title compound (yellow solid, 300 mg, yield 83%, crude), which was used directly without purification.

### Step E: N-(1-phenyl-3-(3,3,3-trifluoropropionyl)-1H-pyrazol-4-yl)-6-(trifluoromethyl)picolinamide

At rt, to a DMF (5 mL) solution of 1-(4-amino-1-phenyl-1H-pyrazol-3-yl)-3,3-trifluoropropan-1-one (100 mg, 0.371 mmol, 1.0 eq) were added 6-(trifluoromethyl)picolinic acid (71.0 mg, 0.371 mmol, 1.0 eq), DIEA (144 mg, 1.11 mmol, 3.0 eq), and HATU (212 mg, 0.557 mmol, 1.5 eq). The reaction solution was stirred at rt under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 53.4 mg, yield 33%). **¹H NMR** (400 MHz, DMSO-*d*₆) δ 11.23 (s, 1H), 9.11 (s, 1H), 8.43 (dt, *J =* 15.4, 7.6 Hz, 2H), 8.24 (d, *J =* 7.4 Hz, 1H), 8.02 (d, *J* = 7.9 Hz, 2H), 7.58 (t, *J =* 7.8 Hz, 2H), 7.46 (t, *J =* 7.4 Hz, 1H), 4.39 (q, *J* = 11.0 Hz, 2H). LCMS (ESI): m/z 443 [M+H]⁺.

### Preparation Example 85. Synthesis of Compound A85

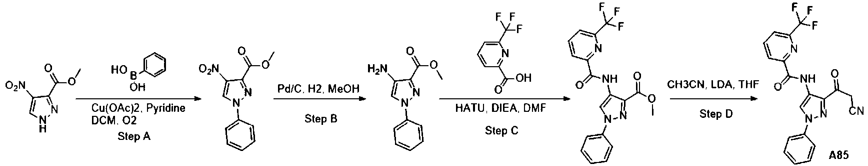

### Step A: Methyl 4-nitro-1-phenyl-1H-pyrazole-3-carboxylate

At rt, to a DCM (20 mL) solution of methyl 4-nitro-1H-pyrazole-3-carboxylate (3.0 g, 17.5 mmol, 1.0 eq) were added phenylboronic acid (4.28 g, 35.1 mmol, 2.0 eq), copper acetate (5.25 g, 26.3 mmol, 1.5 eq), and pyridine (5.7 mL, 70.1 mmol, 4.0 eq). The reaction solution was stirred under O₂ (1 atm) at rt for 16 h. Upon completion, the reaction solution was filtered, and the filtrate was diluted in water and extracted with DCM (×3). The combined organic phases were washed with water and saturated aqueous NaCl, dried over anhydrous Na₂SO₄, and concentrated under reduced pressure. The residue was purified by column chromatography to afford the title compound (yellow solid, 3.5 g, yield 81%).

### Step B: Methyl 4-amino-1-phenyl-1H-pyrazole-3-carboxylate

To a solution of methyl 4-nitro-1-phenyl-1H-pyrazole-3-carboxylate (1 g, 4.05 mmol, 1.0 eq) in MeOH (30 mL) was added 10% palladium on carbon (861 mg, 0.81 mmol, 0.2 eq) at rt. The reaction solution was purged with H₂ (using a hydrogen balloon) (×3) and stirred under H₂ (1 atm) at rt for 12 h. Upon completion, the mixture was filtered, and the filtrate was concentrated under reduced pressure to afford the title compound (yellow solid, 700 mg, yield 80%, crude), which was used directly without purification.

### Step C: Methyl 1-phenyl-4-(6-(trifluoromethyl)picolinamido)-1H-pyrazole-3-carboxylate

At rt, to a DMF (20 mL) solution of methyl 4-amino-1-phenyl-1H-pyrazole-3-carboxylate (500 mg, 2.30 mmol, 1.0 eq) and 6-(trifluoromethyl)picolinic acid (440 mg, 2.30 mmol, 1.0 eq) were added DIEA (892 mg, 6.91 mmol, 3.0 eq) and HATU (875 mg, 2.30 mmol, 1.0 eq). Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 680 mg, yield 76%).

### Step D: N-(3-(2-cyanoacetyl)-1-phenyl-1H-pyrazol-4-yl)-6-(trifluoromethyl)picolinamide

At -78°C under N₂, a solution of lithium diisopropylamide in THF (2 M, 0.51 mL, 1.02 mmol, 4.0 eq) was added dropwise over 20 min to acetonitrile (42 mg, 1.02 mmol, 4.0 eq). After the addition, the mixture was stirred at -78°C under N₂ for 0.5 h. To the above mixture was then added a solution of methyl 1-phenyl-4-(6-(trifluoromethyl)picolinamido)-1H-pyrazole-3-carboxylate (100 mg, 0.26 mmol, 1.0 eq) dissolved in THF (5.0 mL). The reaction solution was stirred under N₂ for 2 h. Upon completion, the reaction was quenched with saturated aqueous NH₄Cl, and extracted with EtOAc (×3). The combined organic phases were washed with water and saturated aqueous NaCl, dried over anhydrous Na₂SO₄, and concentrated under reduced pressure. The residue was purified by HPLC to afford the title compound (yellow solid, 45.3 mg, yield 44%). **¹H NMR** (400 MHz, DMSO-*d*₆) δ 11.21 (s, 1H), 9.12 (s, 1H), 8.49-8.39 (m, 2H), 8.26 (d, *J* = 7.5 Hz, 1H), 8.00 (d, *J =* 8.0 Hz, 2H), 7.59 (t, *J =* 7.8 Hz, 2H), 7.46 (t, *J =* 7.3 Hz, 1H), 4.73 (s, 2H). LCMS (ESI): m/z 400 [M+H]⁺.

### Preparation Example 86. Synthesis of Compound A86

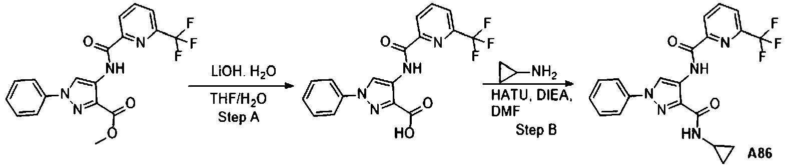

### Step A: 1-phenyl-4-(6-(trifluoromethyl)picolinamido)-1H-pyrazole-3-carboxylic acid

At 0°C, to a mixed solution of methyl 1-phenyl-4-(6-(trifluoromethyl)picolinamido)-1H-pyrazole-3-carboxylate (300.0 mg, 0.8 mmol, 1.0 eq) in THF (3 mL) and water (0.6 mL) was added lithium hydroxide monohydrate (65.0 mg, 1.6 mmol, 2.0 eq). The reaction solution was stirred at rt under N₂ for 2 h. Upon completion, the reaction solution was adjusted to pH 5-6 using 1 N HCl. The mixture was extracted with EtOAc (×3). The combined organic phases were washed with water and saturated aqueous NaCl, dried over anhydrous Na₂SO₄, filtered, and concentrated under reduced pressure to afford the title compound (yellow oil, 260 mg, yield 89%, crude), which was used directly without purification.

### Step B: N-(3-(cyclopropylcarbamoyl)-1-phenyl-1H-pyrazol-4-yl)-6-(trifluoromethyl)picolinamide

At rt, to a DMF (1 mL) solution of 1-phenyl-4-(6-(trifluoromethyl)picolinamido)-1H-pyrazole-3-carboxylic acid (120.0 mg, 0.3 mmol, 1.0 eq) and cyclopropylamine (21.9 mg, 0.4 mmol, 1.2 eq) were added DIEA (116.1 mg, 0.9 mmol, 3.0 eq) and HATU (182.5 mg, 0.5 mmol, 1.5 eq). The reaction solution was stirred at rt under N₂ for 16 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 50.5 mg, yield 38%). **¹H NMR** (400 MHz, DMSO-*d*₆) δ 11.61 (s, 1H), 9.05 (s, 1H), 8.60 (d, *J =* 4.5 Hz, 1H), 8.49-8.38 (m, 2H), 8.23 (d, *J =* 7.6 Hz, 1H), 8.00 (d, *J* = 7.9 Hz, 2H), 7.55 (t, *J =* 7.9 Hz, 2H), 7.40 (t, *J =* 7.4 Hz, 1H), 3.01-2.92 (m, 1H), 0.78-0.68 (m, 4H). LCMS (ESI): m/z 416 [M+H]⁺.

### Preparation Example 87. Synthesis of Compound A87

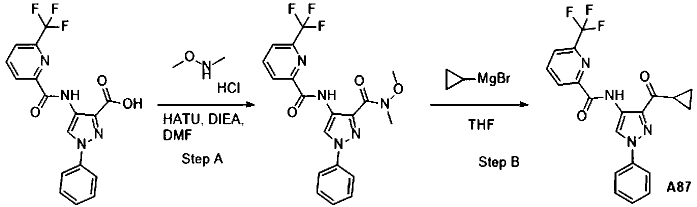

### Step A: N-(3-(methoxy(methyl)carbamoyl)-1-phenyl-1H-pyrazol-4-yl)-6-(trifluoromethyl)picolinamide

At rt, to a solution of 1-phenyl-4-(6-(trifluoromethyl)picolinamido)-1H-pyrazole-3-carboxylic acid (160.0 mg, 0.4 mmol, 1.0 eq) and N,O-dimethylhydroxylamine hydrochloride (41.9 mg, 0.4 mmol, 1.0 eq) in DMF (2 mL) were added HATU (245.3 mg, 0.6 mmol, 1.5 eq) and DIEA (154.8 mg, 1.2 mmol, 3.0 eq). The mixture was stirred at rt under N₂ for 16 h. Upon completion, the reaction solution was diluted in saturated aqueous NH₄Cl, and extracted with EtOAc (×3). The combined organic phases were washed sequentially with water and saturated aqueous NaCl, dried over anhydrous Na₂SO₄, and concentrated under reduced pressure. The residue was purified by column chromatography to afford the title compound (white solid, 150.0 mg, yield 83%).

### Step B: N-(3-(Cyclopropanecarbonyl)-1-phenyl-1H-pyrazol-4-yl)-6-(trifluoromethyl)picolinamide

Under N₂ at 0°C, to a solution of N-(3-(methoxy(methyl)carbamoyl)-1-phenyl-1H-pyrazol-4-yl)-6-(trifluoromethyl)picolinamide (150.0 mg, 0.4 mmol, 1.0 eq) in THF (1.5 mL) was slowly added 1 M cyclopropylmagnesium bromide THF solution (1.8 mL, 1.8 mmol, 5.0 eq). The reaction solution was stirred at rt under N₂ for 1 h. Upon completion, the reaction was quenched with saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by HPLC to afford the title compound (white solid, 48.9 mg, yield 34%). **¹H NMR** (400 MHz, DMSO-*d*₆) δ 11.45 (s, 1H), 9.12 (s, 1H), 8.49-8.38 (m, 2H), 8.24 (d, *J =* 6.9 Hz, 1H), 8.03 (d, *J =* 7.8 Hz, 2H), 7.59 (t, *J* = 7.9 Hz, 2H), 7.45 (t, *J =* 7.4 Hz, 1H), 3.21-3.12 (m, 1H), 1.22-1.12 (m, 4H). LCMS (ESI): m/z 401 [M+H]⁺.

### Preparation Example 88. Synthesis of Compound A88

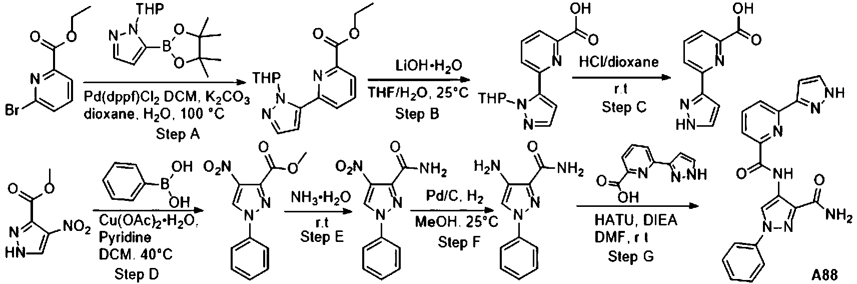

### Step A: Ethyl 6-(1-(tetrahydro-2H-pyran-2-yl)-1H-pyrazol-5-yl)picolinate

At rt, to a solution of ethyl 6-bromopicolinate (3.0 g, 13.1 mmol, 1.0 eq) and 1-(tetrahydro-2H-pyran-2-yl)-5-(4,4,5,5-tetramethyl-1,3,2-dioxaborolan-2-yl)-1H-pyrazole (4.37 g, 15.7 mmol, 1.2 eq) in 1,4-dioxane (50 mL) and water (10 mL) were sequentially added potassium carbonate (4.52 g, 32.8 mmol, 2.5 eq) and Pd(dppf)Cl₂·CH₂Cl₂ (1.06 g, 1.31 mmol, 0.1 eq). The reaction solution was purged with N₂ (×3), and the reaction was stirred at 100°C under N₂ for 2 h. Upon completion, the mixture was cooled to rt, diluted in EtOAc, washed sequentially with water and saturated aqueous NaCl, dried over anhydrous Na₂SO₄, filtered, and concentrated under reduced pressure. The residue was purified by column chromatography to afford the title compound (white solid, 3.5 g, yield 88%).

### Step B: 6-(1-(tetrahydro-2H-pyran-2-yl)-1H-pyrazol-5-yl)picolinic acid

At 0°C, to a mixed solution of ethyl 6-(1-(tetrahydro-2H-pyran-2-yl)-1H-pyrazol-5-yl)picolinate (3.5 g, 11.6 mmol, 1.0 eq) in THF (40 mL) and water (8 mL) was added lithium hydroxide (1.46 g, 34.8 mmol, 3.0 eq). The reaction solution was stirred at rt under N₂ for 12 h. Upon completion, the reaction solution was adjusted to pH 5-6 using 1 N HCl. The mixture was extracted with EtOAc (×3), and the combined organic phases were washed with water and saturated aqueous NaCl, dried over anhydrous Na₂SO₄, and concentrated under reduced pressure to afford the title compound (yellow solid, 3.5 g, crude), which was used directly without purification.

### Step C: 6-(1H-pyrazol-3-yl)picolinic acid

At 0°C, 4 M HCl in 1,4-dioxane (35 mL) was added to 6-(1-(tetrahydro-2H-pyran-2-yl)-1H-pyrazol-5-yl)picolinic acid (3.5 g, crude). The reaction solution was stirred at rt under N₂ for 2 h. The filtrate was concentrated under reduced pressure to afford the title compound (white solid, 3 g, crude), which was used directly without purification.

### Step D: Methyl 4-nitro-1-phenyl-1H-pyrazole-3-carboxylate

At rt, to a solution of methyl 4-nitro-1H-pyrazole-3-carboxylate (1 g, 5.84 mmol, 1.0 eq) in DCM (20 mL) were added phenylboronic acid (0.71 g, 5.84 mmol, 1.0 eq), copper acetate monohydrate (1.75 g, 8.76 mmol, 1.5 eq), and pyridine (2.3 g, 29.2 mmol, 5.0 eq). The reaction solution was stirred at 40°C under O₂ for 14 h. Upon completion, the reaction solution was filtered, and the filtrate was diluted in water and extracted with DCM (×3). The combined organic phases were washed with water and saturated aqueous NaCl, dried over anhydrous Na₂SO₄, and concentrated under reduced pressure. The residue was purified by column chromatography to afford the title compound (colorless liquid, 400 mg, yield 28%).

### Step E: 4-nitro-1-phenyl-1H-pyrazole-3-carboxamide

A solution of methyl 4-nitro-1-phenyl-1H-pyrazole-3-carboxylate (100 mg, 0.41 mmol, 1.0 eq) in aqueous ammonia (5 mL) was stirred at rt for 6 h. Upon completion, the mixture was filtered, and the filter cake was washed with water and dried completely to afford the title compound (white solid, 80 mg, crude), which was used directly without purification.

### Step F: 4-Amino-1-phenyl-1H-pyrazole-3-carboxamide

At rt, to a solution of 4-nitro-1-phenyl-1H-pyrazole-3-carboxamide (80 mg, 0.34 mmol, 1.0 eq) in MeOH (10 mL) was added 10% palladium on carbon (40 mg). The reaction solution was purged with H₂ (using a hydrogen balloon) (×3) and stirred under H₂ at rt for 14 h. Upon completion, the mixture was filtered, and the filtrate was concentrated under reduced pressure to afford the title compound (pale brown solid, 60 mg, crude), which was used directly without purification.

### Step G: N-(3-amino-1-(pyridin-3-yl)-1H-pyrazol-4-yl)-6-(trifluoromethyl)picolinamide

At rt, to a solution of 6-(1H-pyrazol-3-yl)picolinic acid (97 mg, 0.51 mmol, 1.3 eq) and HATU (194 mg, 0.51 mmol, 1.3 eq) in DMF (4 mL) were added DIEA (258 mg, 2.00 mmol, 5.0 eq) and 4-amino-1-phenyl-1H-pyrazole-3-carboxamide (80 mg, 0.40 mmol, 1.0 eq). The reaction solution was stirred at rt under N₂ for 2.5 h. Upon completion, the reaction solution was diluted in EtOAc, washed sequentially with water and saturated aqueous NaCl, dried over anhydrous Na₂SO₄, and concentrated under reduced pressure. The residue was purified by preparative HPLC to afford the title compound (white solid, 49 mg, yield 35%). **¹H NMR** (400 MHz, DMSO-*d₆*) δ 13.21 (s, 1H), 12.19 (s, 1H), 9.02 (s, 1H), 8.23 (d, *J* = 7.8 Hz, 1H), 8.10 (d, *J =* 7.7 Hz, 1H), 8.06 (d, *J =* 6.6 Hz, 2H), 8.01 (d, *J =* 7.8 Hz, 2H), 7.92 (d, *J =* 11.5 Hz, 2H), 7.57 (t, *J =* 7.9 Hz, 2H), 7.40 (t, *J =* 7.4 Hz, 1H), 7.33 (s, 1H). LCMS (ESI): m/z 374.1 [M+H]⁺.

### Preparation Example 89. Synthesis of Compound A89

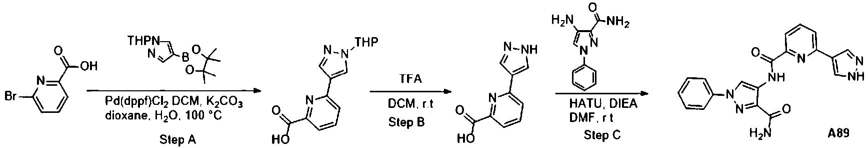

### Step A: 6-(1-(tetrahydro-2H-pyran-2-yl)-1H-pyrazol-4-yl)picolinic acid

At rt, to a solution of 6-bromopyridinecarboxylic acid(2.5 g, 12.4 mmol, 1.0 eq) and 1-(tetrahydro-2H-pyran-2-yl)-4-(4,4,5,5-tetramethyl-1,3,2-dioxaborolan-2-yl)-1H-pyrazole (4.14 g, 14.9 mmol, 1.2 eq) in 1,4-dioxane (30 mL) and water (6 mL) were sequentially added potassium carbonate (4.28 g, 31.0 mmol, 2.5 eq) and Pd(dppf)Cl₂·CH₂Cl, (2.51 g, 3.10 mmol, 0.1 eq). The reaction solution was purged with N₂ (×3), and the reaction was stirred at 100°C under N₂ for 2 h. Upon completion, the mixture was cooled to rt, diluted in EtOAc, washed sequentially with water and saturated aqueous NaCl, dried over anhydrous Na₂SO₄, filtered, and concentrated under reduced pressure. The residue was purified by column chromatography to afford the title compound (white solid, 2.6 g, yield 77%).

### Step B: 6-(1H-pyrazol-4-yl)picolinic acid

At 0°C, trifluoroAcOH (3 mL) was added to a solution of 6-(1-(tetrahydro-2H-pyran-2-yl)-1H-pyrazol-4-yl)picolinic acid (1 g, 3.66 mmol, 1.0 eq) in DCM (10 mL). The mixture was stirred at rt for 2 h. Upon completion, the mixture was concentrated under reduced pressure to afford the title compound (white solid, 380 mg, crude), which was used directly without purification.

### Step C: N-(3-amino-1-phenyl-1H-pyrazol-4-yl)-6-(1H-pyrazole-4-carbonyl)picolinamide

At rt, to a solution of 6-(1H-pyrazol-4-yl)picolinic acid (140 mg, 0.74 mmol, 1.0 eq) and HATU (366 mg, 0.96 mmol, 1.2 eq) in DMF (5 mL) were added DIEA (477 mg, 3.70 mmol, 5.0 eq) and 4-amino-1-phenyl-1H-pyrazole-3-carboxamide (150 mg, 0.74 mmol, 1.0 eq). The reaction solution was stirred at rt under N₂ for 2.5 h. Upon completion, the reaction solution was diluted in EtOAc, washed sequentially with water and saturated aqueous NaCl, dried over anhydrous Na₂SO₄, and concentrated under reduced pressure. The residue was purified by preparative HPLC to afford the title compound (white solid, 19 mg, yield 7%). **¹H NMR** (400 MHz, DMSO-*d₆*) δ 13.22 (s, 1H), 12.20 (s, 1H), 9.00 (s, 1H), 8.61-8.35 (m, 2H), 8.13-8.06 (m, 1H), 8.06-7.96 (m, 4H), 7.95-7.88 (m, 2H), 7.57 (t, *J =* 7.8 Hz, 2H), 7.40 (t, *J =* 7.3 Hz, 1H). LCMS (ESI): m/z 374.1 [M+H]⁺.

### Preparation Example 90. Synthesis of Compound A90

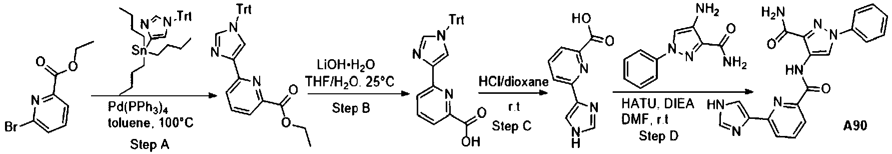

### Step A: Ethyl 6-(1-(triisopropylsilyl)-1H-pyrrol-3-yl)picolinate

At rt, to a solution of ethyl 6-bromopicolinate (520 mg, 2.27 mmol, 1.0 eq) and 4-(tributylstannyl)-1-trimethylsilyl-1H-imidazole (1.50 g, 2.50 mmol, 1.1 eq) in toluene (10 mL) was added tetrakis(triphenylphosphine)palladium (131 mg, 0.11 mmol, 0.05 eq). The reaction solution was purged with N₂ (×3), and the reaction was stirred at 100°C under N₂ for 16 h. Upon completion, the mixture was cooled to rt, diluted in EtOAc, washed sequentially with water and saturated aqueous NaCl, dried over anhydrous Na₂SO₄, filtered, and concentrated under reduced pressure. The residue was purified by column chromatography to afford the title compound (white solid, 700 mg, yield 67%).

### Step B: 6-(1-trimethylsilyl-1H-imidazol-4-yl)picolinic acid

At 0°C, to a mixed solution of ethyl 6-(1-(triisopropylsilyl)-1H-pyrrol-3-yl)picolinate (700 mg, 1.53 mmol, 1.0 eq) in THF (10 mL) and water (2 mL) was added lithium hydroxide monohydrate (193 mg, 4.59 mmol, 3.0 eq). The reaction solution was stirred at rt under N₂ for 12 h. Upon completion, the reaction solution was adjusted to pH 5-6 using 1 N HCl. The mixture was extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow solid, 500 mg, yield 76%).

### Step C: 6-(1H-imidazol-4-yl)picolinic acid

At 0°C, 4 M HCl in 1,4-dioxane (5 mL) was added to 6-(1-trimethylsilyl-1H-imidazol-4-yl)picolinic acid (110 mg, 0.26 mmol, 1.0 eq). The reaction solution was stirred at rt under N₂ for 2 h. The filtrate was concentrated under reduced pressure to afford the title compound (yellow solid, 50 mg, crude), which was used directly without purification.

### Step D: N-(3-amino-1-phenyl-1H-pyrazol-4-yl)-6-(1H-imidazol-4-yl)picolinamide

At rt, to a solution of 6-(1H-imidazol-4-yl)picolinic acid (50 mg, 0.26 mmol, 1.0 eq) and HATU (128 mg, 0.34 mmol, 1.3 eq) in DMF (3 mL) were added DIEA (168 mg, 1.30 mmol, 5 eq) and 4-amino-1-phenyl-1H-pyrazole-3-carboxamide (53 mg, 0.26 mmol, 1.0 eq). The reaction solution was stirred at rt under N₂ for 0.5 h. Upon completion, the reaction solution was diluted in EtOAc, washed sequentially with water and saturated aqueous NaCl, dried over anhydrous Na₂SO₄, and concentrated under reduced pressure. The residue was purified by preparative HPLC to afford the title compound (white solid, 23.4 mg, yield 24%). **¹H NMR** (400 MHz, DMSO-*d₆*) δ 12.58 (s, 1H), 12.15 (s, 1H), 9.01 (s, 1H), 8.19-7.92 (m, 7H), 7.88-7.78 (m, 2H), 7.57 (t, *J* = 7.9 Hz, 2H), 7.41 (t, *J =* 7.4 Hz, 1H). LCMS (ESI): m/z 374.1 [M+H]⁺.

### Preparation Example 91. Synthesis of Compound A91

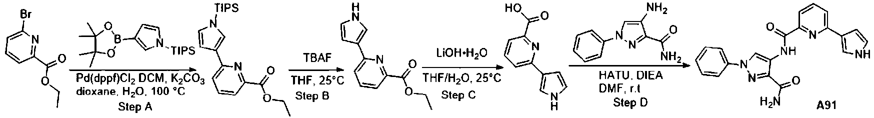

### Step A: Ethyl 6-(1-(triisopropylsilyl)-1H-pyrrol-3-yl)picolinate

At rt, to a solution of ethyl 6-bromopicolinate (2.5 g, 10.9 mmol, 1.0 eq) and 3-(4,4,5,5-tetramethyl-1,3,2-dioxaborolan-2-yl)-1-(triisopropylsilyl)-1H-pyrrole (4.56 g, 13.0 mmol, 1.2 eq) in 1,4-dioxane (50 mL) and water (10 mL) were added potassium carbonate (3.75 g, 27.2 mmol, 2.5 eq) and Pd(dppf)Cl₂·CH₂Cl₂ (0.89 g, 1.09 mmol, 0.1 eq). The reaction solution was purged with N₂ (×3), and the reaction was stirred at 100°C under N₂ for 2 h. Upon completion, the mixture was cooled to rt, diluted in EtOAc, washed sequentially with water and saturated aqueous NaCl, dried over anhydrous Na₂SO₄, filtered, and concentrated under reduced pressure. The residue was purified by column chromatography to afford the title compound (white solid, 2.9 g, yield 72%).

### Step B: Ethyl 6-(1H-pyrrol-3-yl)picolinate

At rt, to a solution of ethyl 6-(1-(triisopropylsilyl)-1H-pyrrol-3-yl)picolinate (2.9 g, 7.78 mmol, 1.0 eq) in THF (60 mL) was added 1 M TBAF in THF (15.6 mL, 15.6 mmol, 2.0 eq). The mixture was stirred at rt under N₂ for 1 h. Upon completion, the mixture was concentrated under reduced pressure. The residue was purified by column chromatography to afford the title compound (white solid, 1.3 g, yield 77%).

### Step C: 6-(1H-pyrrol-3-yl)picolinic acid

At 0°C, to a mixed solution of ethyl 6-(1H-pyrrol-3-yl)picolinate (800 mg, 3.70 mmol, 1.0 eq) in THF (20 mL) and water (4 mL) was added lithium hydroxide monohydrate (466 mg, 11.1 mmol, 3.0 eq). The reaction solution was stirred at rt under N₂ for 12 h. Upon completion, the reaction solution was adjusted to pH=3 using 1 N HCl. The mixture was extracted with EtOAc (×3). The combined organic phases were washed with water and saturated aqueous NaCl, dried over anhydrous Na₂SO₄, and concentrated under reduced pressure. The residue was purified by reverse-phase column chromatography to afford the title compound (yellow solid, 600 mg, yield 86%).

### Step D: N-(3-amino-1-phenyl-1H-pyrazol-4-yl)-6-(1H-pyrrol-3-yl)picolinamide

At rt, to a solution of 6-(1H-pyrrol-3-yl)picolinic acid (100 mg, 0.53 mmol, 1.0 eq) and HATU (262 mg, 0.69 mmol, 1.3 eq) in DMF (3 mL) were added DIEA (343 mg, 2.66 mmol, 5 eq) and 4-amino-1-phenyl-1H-pyrazole-3-carboxamide (107 mg, 0.53 mmol, 1.0 eq). The reaction solution was stirred at rt under N₂ for 0.5 h. Upon completion, the reaction solution was diluted in EtOAc, washed sequentially with water and saturated aqueous NaCl, dried over anhydrous Na₂SO₄, and concentrated under reduced pressure. The residue was purified by preparative HPLC to afford the title compound (white solid, 15 mg, yield 8%). **¹H NMR** (400 MHz, DMSO-*d₆*) δ 12.09 (s, 1H), 11.23 (s, 1H), 9.01 (s, 1H), 8.06-7.98 (m, 3H), 7.97-7.91 (m, 1H), 7.90-7.81 (m, 3H), 7.75-7.71 (m, 1H), 7.56 (t, *J =* 8.0 Hz, 2H), 7.40 (t, *J =* 7.4 Hz, 1H), 6.99-6.96 (m, 1H), 6.91-6.87 (m, 1H). LCMS (ESI): m/z 373.1 [M+H]⁺.

### Preparation Example 92. Synthesis of Compounds A92, A93, A94, A95, A102 and A103

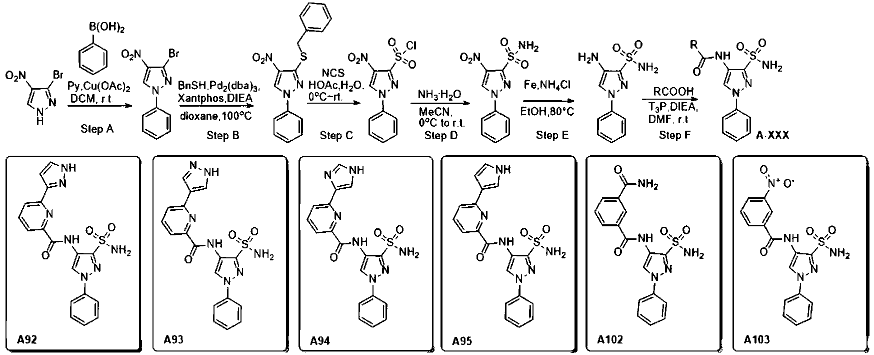

### Step A: 3-Bromo-4-nitro-1-phenylpyrazole

At rt, to a solution of 3-bromo-4-nitro-1H-pyrazole (8.50 g, 44.3 mmol, 1.0 eq) and phenylboronic acid (7.02 g, 57.6 mmol, 1.3 eq) in DCM (15 mL) were added copper acetate (13.3 g, 66.4 mmol, 1.5 eq) and pyridine (14 mL, 177 mmol, 4.0 eq). The reaction solution was stirred under O₂ at rt for 18 h. Upon completion, the reaction solution was filtered, and the filtrate was diluted in water and extracted with DCM (×3). The combined organic phases were washed with water and saturated aqueous NaCl, dried over anhydrous Na₂SO₄, and concentrated under reduced pressure. The residue was purified by column chromatography to afford the title compound (white solid, 11 g, yield 93%).

### Step B: 3-(Benzylthio)-4-nitro-1-phenylpyrazole

At rt, to a solution of 3-bromo-4-nitro-1-phenylpyrazole (10.7 g, 39.9 mmol, 1.05 eq) and benzyl mercaptan (4.71 g, 37.9 mmol, 1.0 eq) in 1,4-dioxane (150 mL) were sequentially added diisopropylethylamine (5.67 g, 43.9 mmol, 1.2 eq), 4,5-bis(diphenylphosphino)-9,9-dimethylxanthene (4.62 g, 7.98 mmol, 0.2 eq) and tris(dibenzylideneacetone)dipalladium (3.66 g, 3.99 mmol, 0.1 eq). The reaction solution was purged with N₂ (×3), and the reaction was stirred at 100°C under N₂ for 18 h. Upon completion, the mixture was cooled to rt, diluted in EtOAc, washed sequentially with water and saturated aqueous NaCl, dried over anhydrous Na₂SO₄, filtered, and concentrated under reduced pressure. The residue was purified by column chromatography to afford the title compound (yellow solid, 9 g, yield 72%).

### Step C: 4-Nitro-1-phenylpyrazole-3-sulfonyl chloride

At 0°C, N-chlorosuccinimide (11.6 g, 86.7 mmol, 3.0 eq) was added to a solution of 3-(benzylthio)-4-nitro-1-phenylpyrazole (9 g, 28.9 mmol, 1.0 eq) in AcOH (150 mL) and water (25 mL). The reaction solution was stirred at rt under N₂ for 1 h. Upon completion, the reaction solution was extracted with EtOAc. The combined organic phases were washed with water and saturated aqueous NaCl, dried over anhydrous Na₂SO₄, filtered, and concentrated under reduced pressure. The residue was purified by column chromatography to afford the title compound (white solid, 8 g, yield 96%).

### Step D: 4-Nitro-1-phenylpyrazole-3-sulfonamide

At 0°C, 25 wt% aqueous ammonia (25 mL) was added to a solution of 4-nitro-1-phenylpyrazole-3-sulfonyl chloride (8 g, 27.8 mmol, 1.0 eq) in acetonitrile (100 mL). The reaction solution was stirred at rt for 1 h. Upon completion, the mixture was concentrated under reduced pressure. The residue was slurried with water (50 mL) and purified to afford the title compound (white solid, 6 g, yield 80%, crude), which was used directly without purification.

### Step E: 4-Amino-1-phenyl-1H-pyrazole-3-sulfonamide

At rt, to a solution of 4-nitro-1-phenylpyrazole-3-sulfonamide (1 g, 3.73 mmol, 1.0 eq) in ethanol (20 mL) and water (10 mL) were added iron powder (1.04 g, 18.6 mmol, 5.0 eq) and ammonium chloride (997 mg, 18.6 mmol, 5.0 eq). The mixture was stirred at 80°C for 1 h. Upon completion, the mixture was filtered, and the filtrate was concentrated under reduced pressure. The residue was purified by column chromatography to afford the title compound (white solid, 600 mg, yield 68%).

### Step F: N-(1-phenyl-3-sulfamoyl-1H-pyrazol-4-yl)-6-(1H-pyrazol-3-yl)picolinamide (A92)

At rt, to a solution of 4-amino-1-phenyl-1H-pyrazole-3-sulfonamide (100 mg, 0.42 mmol, 1.0 eq) and 6-(1H-pyrazol-3-yl)pyridine-2-carboxylic acid (79 mg, 0.42 mmol, 1.0 eq) in DMF (2 mL) were added DIEA (54 mg, 0.42 mmol, 1.0 eq) and 50 wt% T₃P in EtOAc (134 mg, 0.42 mmol, 1.0 eq). The mixture was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by preparative HPLC to afford the title compound (white solid, 54 mg, yield 31%). **¹H NMR** (400 MHz, DMSO-*d₆*) δ 13.20 (s, 1H), 11.33 (s, 1H), 9.10 (s, 1H), 8.25 (d, *J =* 7.6 Hz, 1H), 8.09 (ddd, *J* = 18.5, 13.6, 9.3 Hz, 4H), 7.98-7.87 (m, 3H), 7.59 (t, *J =* 8.0 Hz, 2H), 7.45 (t, *J =* 7.4 Hz, 1H), 7.23 (s, 1H). LCMS(ESI): m/z 410.1 [M+H]⁺.

### N-(1-phenyl-3-sulfamoyl-1H-pyrazol-4-yl)-6-(1H-pyrazole-4-carbonyl)picolinamide (A93)

At rt, to a solution of 4-amino-1-phenyl-1H-pyrazole-3-sulfonamide (100 mg, 0.42 mmol, 1.0 eq) and 6-(1H-pyrazol-4-yl)pyridine-2-carboxylic acid (238 mg, 1.26 mmol, 3.0 eq) in DMF (3 mL) were added DIEA (434 mg, 3.36 mmol, 8.0 eq) and 50 wt% T₃P in EtOAc (1.07 g, 1.68 mmol, 4.0 eq). The mixture was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by preparative HPLC to afford the title compound (white solid, 20.3 mg, yield 12%). **¹H NMR** (400 MHz, DMSO-*d₆*) δ 13.23 (s, 1H), 11.35 (s, 1H), 9.09 (s, 1H), 8.53 (s, 1H), 8.33 (s, 1H), 8.13-7.97 (m, 4H), 7.94 (dd, *J =* 9.5, 4.3 Hz, 3H), 7.59 (t, *J =* 8.0 Hz, 2H), 7.44 (t, *J =* 7.4 Hz, 1H). LCMS (ESI): m/z 410.1 [M+H]⁺.

### 6-(1H-imidazol-4-yl)-N-(1-phenyl-3-sulfamoyl-1H-pyrazol-4-yl)picolinamide (A94)

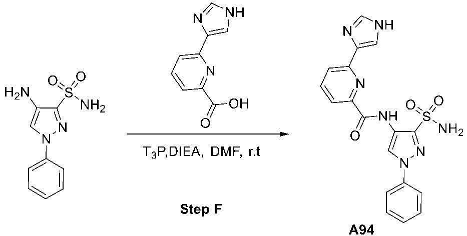

At rt, to a solution of 4-amino-1-phenyl-1H-pyrazole-3-sulfonamide (100 mg, 0.42 mmol, 1.0 eq) and 6-(3H-imidazol-5-yl)pyridine-2-carboxylic acid (238 mg, 1.26 mmol, 3.0 eq) in DMF (5 mL) were added pyridine (0.5 mL, 6.18 mmol, 14.7 eq), HOBt (170 mg, 1.26 mmol, 3.0 eq) and EDCI (242 mg, 1.26 mmol, 3.0 eq). The mixture was stirred at rt for 3 h. Upon completion, the reaction solution was diluted in EtOAc, washed sequentially with water and saturated aqueous NaCl, dried over anhydrous Na₂SO₄, and concentrated under reduced pressure. The residue was purified by preparative HPLC to afford the title compound (white solid, 19.1 mg, yield 11%). **¹H NMR** (400 MHz, DMSO-*d₆*) δ 12.52 (s, 1H), 11.37 (s, 1H), 9.09 (s, 1H), 8.15-8.00 (m, 4H), 8.00-7.89 (m, 4H), 7.85 (s, 1H), 7.59 (t, *J =* 7.9 Hz, 2H), 7.44 (t, *J =* 7.4 Hz, 1H). LCMS(ESI): m/z 410.1 [M+H]⁺.

### N-(1-phenyl-3-sulfamoyl-1H-pyrazol-4-yl)-6-(1H-pyrrol-3-yl)picolinamide (A95)

At rt, to a solution of 4-amino-1-phenyl-1H-pyrazole-3-sulfonamide (100 mg, 0.42 mmol, 1.0 eq) and 6-(1H-pyrrol-3-yl)pyridine-2-carboxylic acid (237 mg, 1.26 mmol, 3.0 eq) in DMF (3 mL) were added DIEA (434 mg, 3.36 mmol, 8.0 eq) and 50 wt% T₃P in EtOAc (1.07 g, 1.68 mmol, 4.0 eq). The mixture was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by preparative HPLC to afford the title compound (white solid, 23.4 mg, yield 14%). **¹H NMR** (400 MHz, DMSO-*d₆*) δ 11.33 (s, 1H), 11.26 (s, 1H), 9.09 (s, 1H), 8.03 (s, 2H), 7.98-7.90 (m, 3H), 7.90-7.82 (m, 2H), 7.73 (dd, *J* = 2.8, 1.5 Hz, 1H), 7.59 (t, *J =* 8.0 Hz, 2H), 7.44 (t, *J =* 7.4 Hz, 1H), 6.89 (dt, *J =* 3.5, 2.1 Hz, 2H). LCMS(ESI): m/z 409.1 [M+H]⁺.

### N-(1-phenyl-3-sulfamoyl-1H-pyrazol-4-yl)isophthalamide (A102)

At rt, to a solution of 4-amino-1-phenyl-1H-pyrazole-3-sulfonamide (100 mg, 0.42 mmol, 1.0 eq) and 3-aminobenzoic acid (208 mg, 1.26 mmol, 3.0 eq) in DMF (3 mL) were added DIEA (434 mg, 3.36 mmol, 8.0 eq) and 50 wt% T₃P in EtOAc (1.07 g, 1.68 mmol, 4.0 eq). The mixture was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by preparative HPLC to afford the title compound (white solid, 52.9 mg, yield 33%). **¹H NMR** (400 MHz, DMSO-*d₆*) δ 9.71 (s, 1H), 9.01 (s, 1H), 8.42 (t, *J =* 1.5 Hz, 1H), 8.18 (s, 1H), 8.12 (d, *J =* 7.8 Hz, 1H), 8.03 (d, *J =* 8.2 Hz, 1H), 7.91 (dd, *J =* 14.2, 6.5 Hz, 4H), 7.69 (t, *J =* 7.7 Hz, 1H), 7.59 (dd, *J =* 10.7, 5.2 Hz, 3H), 7.44 (t, *J* = 7.4 Hz, 1H). LCMS(ESI): m/z 386.1 [M+H]⁺.

### 3-Nitro-N-(1-phenyl-3-sulfamoyl-1H-pyrazol-4-yl)benzamide (A103)

At rt, to a solution of 4-amino-1-phenylpyrazole-3-sulfonamide (250 mg, 1.05 mmol, 1.0 eq) and 3-nitrobenzoic acid (526 mg, 3.15 mmol, 3.0 eq) in DMF (4 mL) were added DIEA (1085 mg, 8.39 mmol, 8.0 eq) and 50 wt% T₃P in EtOAc (2.67 g, 4.20 mmol, 4.0 eq). The mixture was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by preparative HPLC to afford the title compound (white solid, 18.3 mg, yield 4%). **¹H NMR** (400 MHz, DMSO-*d₆*) δ 9.96 (brs, 1H), 9.00 (s, 1H), 8.70 (s, 1H), 8.50 (dd, *J* = 7.9, 1.7 Hz, 1H), 8.34 (d, *J* = 7.8 Hz, 1H), 8.14-7.67 (m, 5H), 7.58 (t, *J* = 7.9 Hz, 2H), 7.44 (t, *J* = 7.4 Hz, 1H). LCMS(ESI): m/z 388.0 [M+H]⁺.

### Preparation Example 93. Synthesis of Compound A96

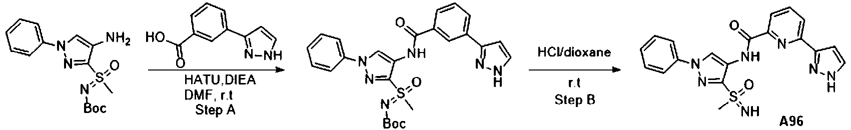

### Step A: Tert-butyl ((4-(3-(1H-pyrazol-3-yl)benzamido)-1-phenyl-1H-pyrazole-3-carbonyl)(methyl)(oxo)-λ⁶-sulfinyl)carbamate

At rt, to a solution of tert-butyl (4-amino-1-phenyl-1H-pyrazol-3-yl)(methyl)(oxo)-λ⁶-sulfinyl)carbamate (100 mg, 0.30 mmol, 1.0 eq) and 3-(1H-pyrazol-3-yl)benzoic acid (84 mg, 0.45 mmol, 1.5 eq) in DMF (4 mL) were added DIEA (115 mg, 0.89 mmol, 3.0 eq) and HATU (169 mg, 0.45 mmol, 1.5 eq). The reaction solution was stirred at rt under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 100 mg, yield 66%).

### Step B: N-(3-(S-methylsulfonimidoyl)-1-phenyl-1H-pyrazol-4-yl)-6-(1H-pyrazol-3-yl)picolinamide

At 0°C, 4 M HCl in 1,4-dioxane (4 mL) was added to tert-butyl ((4-(3-(1H-pyrazol-3-yl)benzamido)-1-phenyl-1H-pyrazole-3-carbonyl)(methyl)(oxo)-λ⁶-sulfinyl)carbamate (100 mg, 0.20 mmol, 1.0 eq). The mixture was stirred at rt under N₂ for 2 h. Upon completion, the filtrate was concentrated under reduced pressure. The residue was purified by preparative HPLC to afford the title compound (white solid, 42.8 mg, yield 53%). **¹H NMR** (400 MHz, DMSO-*d₆*) δ 13.19 (s, 1H), 11.92 (s, 1H), 9.13 (s, 1H), 8.24 (d, *J* = 7.5 Hz, 1H), 8.16-8.04 (m, 2H), 7.94 (t, *J =* 10.3 Hz, 3H), 7.59 (t, *J* = 7.9 Hz, 2H), 7.45 (t, *J =* 7.4 Hz, 1H), 7.25 (d, *J* = 1.9 Hz, 1H), 5.33 (s, 1H), 3.38 (s, 3H). LCMS (ESI): m/z 408.1 [M+H]⁺.

### Preparation Example 94. Synthesis of Compound A97

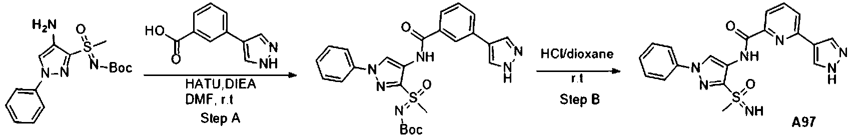

### Step A: Tert-butyl (4-(3-(1H-pyrazol-4-yl)benzamido)-1-phenyl-1H-pyrazol-3-yl)(methyl)(oxo)-λ⁶-sulfinyl)carbamate

At rt, to a solution of tert-butyl (4-amino-1-phenyl-1H-pyrazol-3-yl)(methyl)(oxo)-λ⁶-sulfinyl)carbamate (100 mg, 0.30 mmol, 1.0 eq) and 3-(1H-pyrazol-4-yl)benzoic acid (84 mg, 0.45 mmol, 1.5 eq) in DMF (4 mL) were added DIEA (115 mg, 0.89 mmol, 3.0 eq) and HATU (169 mg, 0.45 mmol, 1.5 eq). The reaction solution was stirred at rt under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 100 mg, yield 66%).

### Step B: N-(3-(S-methylsulfonimidoyl)-1-phenyl-1H-pyrazol-4-yl)-6-(1H-pyrazol-4-yl)picolinamide

At 0°C, 4 M HCl in 1,4-dioxane (4 mL) was added to tert-butyl (4-(3-(1H-pyrazol-4-yl)benzamido)-1-phenyl-1H-pyrazol-3-yl)(methyl)(oxo)-λ⁶-sulfinyl)carbamate (100 mg, 0.20 mmol, 1.0 eq). The reaction solution was stirred at rt under N₂ for 2 h. Upon completion, the filtrate was concentrated under reduced pressure. The residue was purified by preparative HPLC to afford the title compound (white solid, 30.7 mg, yield 38%). **¹H NMR** (400 MHz, DMSO-*d₆*) δ 13.20 (s, 1H), 11.92 (s, 1H), 9.12 (s, 1H), 8.52 (s, 1H), 8.35 (s, 1H), 8.05 (t, *J =* 7.6 Hz, 1H), 8.01-7.89 (m, 4H), 7.59 (t, *J =* 7.8 Hz, 2H), 7.45 (t, *J =* 7.3 Hz, 1H), 5.39 (s, 1H), 3.38 (s, 3H). LCMS (ESI): m/z 408.2 [M+H]⁺.

### Preparation Example 95. Synthesis of Compound A98

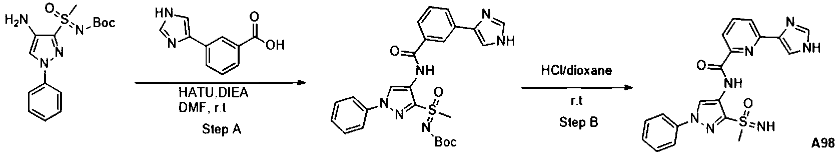

### Step A: Tert-butyl (4-(3-(1H-imidazol-4-yl)benzamido)-1-phenyl-1H-pyrazol-3-yl)(methyl)(oxo)-λ⁶-sulfinyl)carbamate

At rt, to a solution of tert-butyl (4-amino-1-phenyl-1H-pyrazol-3-yl)(methyl)(oxo)-λ⁶-sulfinyl)carbamate (100 mg, 0.30 mmol, 1.0 eq) and 3-(1H-imidazol-4-yl)benzoic acid (84 mg, 0.45 mmol, 1.5 eq) in DMF (4 mL) were added DIEA (115 mg, 0.89 mmol, 3.0 eq) and HATU (169 mg, 0.45 mmol, 1.5 eq). The reaction solution was stirred at rt under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 100 mg, yield 66%).

### Step B: 6-(1H-imidazol-4-yl)-N-(3-(S-methylsulfonimidoyl)-1-phenyl-1H-pyrazol-4-yl)picolinamide

At 0°C, 4 M HCl in 1,4-dioxane (4 mL) was added to tert-butyl (4-(3-(1H-imidazol-4-yl)benzamido)-1-phenyl-1H-pyrazol-3-yl)(methyl)(oxo)-λ6-sulfinyl)carbamate (100 mg, 0.20 mmol, 1.0 eq). The reaction solution was stirred at rt under N₂ for 2 h. Upon completion, the filtrate was concentrated under reduced pressure. The residue was purified by preparative HPLC to afford the title compound (white solid, 31.0 mg, yield 39%). **¹H NMR** (400 MHz, DMSO-*d₆*) δ 12.47 (s, 1H), 11.91 (s, 1H), 9.12 (s, 1H), 8.12 (dd, *J* = 7.9, 1.1 Hz, 1H), 8.07 (t, *J =* 7.7 Hz, 1H), 8.03 (s, 1H), 7.98-7.93 (m, 3H), 7.84 (s, 1H), 7.59 (t, *J =* 7.9 Hz, 2H), 7.45 (t, *J =* 7.4 Hz, 1H), 5.39 (s, 1H), 3.37 (s, 3H). LCMS (ESI): m/z 408.2 [M+H]⁺.

### Preparation Example 96. Synthesis of Compound A99

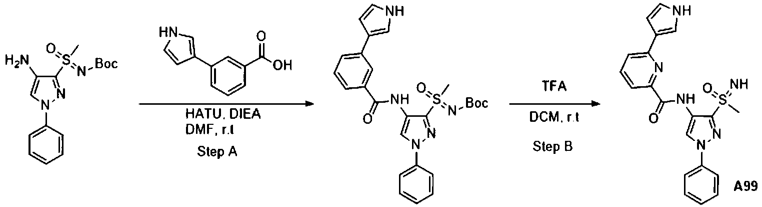

### Step A: Tert-butyl (4-(3-(1H-pyrrol-3-yl)benzamido)-1-phenyl-1H-pyrazol-3-yl)(methyl)(oxo)-λ⁶-sulfinyl)carbamate

At rt, to a solution of tert-butyl (4-amino-1-phenyl-1H-pyrazol-3-yl)(methyl)(oxo)-λ⁶-sulfinyl)carbamate (100 mg, 0.30 mmol, 1.0 eq) and 3-(1H-pyrrol-3-yl)benzoic acid (84 mg, 0.45 mmol, 1.5 eq) in DMF (4 mL) were added DIEA (115 mg, 0.89 mmol, 3.0 eq) and HATU (169 mg, 0.45 mmol, 1.5 eq). The reaction solution was stirred at rt under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 100 mg, yield 66%).

### Step B: N-(3-(S-methylsulfonimidoyl)-1-phenyl-1H-pyrazol-4-yl)-6-(1H-pyrrol-3-yl)picolinamide

At 0°C, TFA (1 mL) was added to a solution of tert-butyl (4-(3-(1H-pyrrol-3-yl)benzamido)-1-phenyl-1H-pyrazol-3-yl)(methyl)(oxo)-λ⁶-sulfinyl)carbamate (100 mg, 0.20 mmol, 1.0 eq) in DCM (3 mL). The reaction solution was stirred at rt under N₂ for 2 h. Upon completion, the filtrate was concentrated under reduced pressure. The residue was purified by preparative HPLC to afford the title compound (white solid, 33.2 mg, yield 41%). **¹H NMR** (400 MHz, DMSO-*d₆*) δ 11.84 (s, 1H), 11.20 (s, 1H), 9.12 (s, 1H), 7.94 (dd, *J =* 7.5, 5.1 Hz, 3H), 7.85 (t, *J =* 8.3 Hz, 2H), 7.73 (s, 1H), 7.59 (t, *J =* 7.9 Hz, 2H), 7.44 (t, *J =* 7.4 Hz, 1H), 6.88 (d, *J* = 2.1 Hz, 2H), 5.27 (s, 1H), 3.36 (s, 3H). LCMS (ESI): m/z 407.2 [M+H]⁺.

### Preparation Example 97. Synthesis of Compound A100

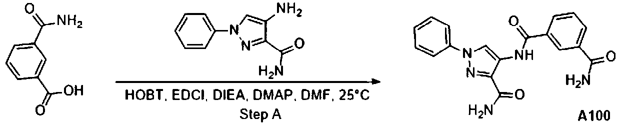

### Step A: N-(3-amino-1-phenyl-1H-pyrazol-4-yl)isophthalamide

At rt, to a solution of 3-aminobenzoic acid (200 mg, 1.21 mmol, 1.0 eq) and EDCI (348 mg, 1.82 mmol, 1.5 eq) in DMF (5 mL) were added HOBt (245 mg, 1.82 mmol, 1.5 eq), 4-dimethylaminopyridine (15 mg, 0.12 mmol, 0.1 eq), and DIEA (783 mg, 6.06 mmol, 5.0 eq). The mixture was stirred at rt for 0.5 h. To the above mixture was then added 4-amino-1-phenylpyrazole-3-carboxamide (245 mg, 1.21 mmol, 1.0 eq). The mixture was stirred at rt under N₂ for 12 h. Upon completion, the reaction solution was diluted in EtOAc, washed sequentially with water and saturated aqueous NaCl, dried over anhydrous Na₂SO₄, and concentrated under reduced pressure. The residue was purified by preparative HPLC to afford the title compound (pink solid, 36 mg, yield 9%). **¹H NMR** (400 MHz, DMSO-*d₆*) δ 10.84 (s, 1H), 8.97 (s, 1H), 8.42 (s, 1H), 8.20 (s, 1H), 8.15-8.09 (m, 2H), 8.04 (d, *J =* 8.0 Hz, 1H), 7.99 (d, *J =* 8.0 Hz, 2H), 7.83 (s, 1H), 7.69 (t, *J =* 8.0 Hz, 1H), 7.56 (t, *J =* 8.0 Hz, 3H), 7.40 (t, *J =* 8.0 Hz, 1H). LCMS (ESI): m/z 350.2 [M+H]⁺.

### Preparation Example 98. Synthesis of Compound A101

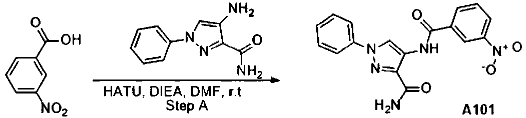

### Step A: 4-(3-nitrobenzamido)-1-phenyl-1H-pyrazole-3-carboxamide

At rt, to a solution of 3-nitrobenzoic acid (66 mg, 0.40 mmol, 1.0 eq) and HATU (180 mg, 0.48 mmol, 1.2 eq) in DMF (4 mL) were added DIEA (102 mg, 0.79 mmol, 2.0 eq) and 4-amino-1-phenylpyrazole-3-carboxamide (80 mg, 0.40 mmol, 1.0 eq). The reaction solution was stirred at rt under N₂ for 2.5 h. Upon completion, the reaction solution was diluted in EtOAc, washed sequentially with water and saturated aqueous NaCl, dried over anhydrous Na₂SO₄, and concentrated under reduced pressure. The residue was purified by preparative HPLC to afford the title compound (white solid, 70 g, yield 50%). ¹H MR (400 MHz, DMSO-*d₆*) δ 10.98 (s, 1H), 8.97 (s, 1H), 8.67 (t, *J =* 1.8 Hz, 1H), 8.49 (dd, *J =* 8.2, 1.5 Hz, 1H), 8.32 (d, *J* = 7.9 Hz, 1H), 8.13 (s, 1H), 8.00 (d, *J =* 7.7 Hz, 2H), 7.90 (dd, *J* = 18.7, 10.7 Hz, 2H), 7.56 (t, *J =* 8.0 Hz, 2H), 7.41 (d, *J =* 7.4 Hz, 1H). LCMS (ESI): m/z 352.1 [M+H]⁺.

### Preparation Example 99. Synthesis of Compound A104

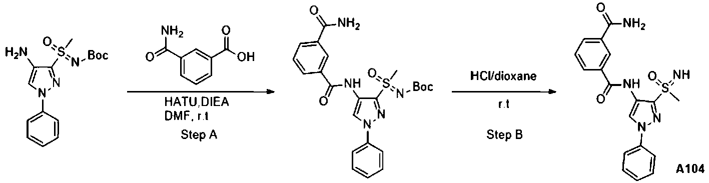

### Step A: Tert-butyl (4-(3-carbamoylbenzamido)-1-phenyl-1H-pyrazol-3-yl)(methyl)(oxo)-λ⁶-sulfinyl)carbamate

At rt, to a solution of tert-butyl (4-amino-1-phenyl-1H-pyrazol-3-yl)(methyl)(oxo)-λ⁶-sulfinyl)carbamate (100 mg, 0.30 mmol, 1.0 eq) and 3-aminobenzoic acid (74 mg, 0.45 mmol, 1.5 eq) in DMF (4 mL) were added HATU (169 mg, 0.45 mmol, 1.5 eq) and DIEA (115 mg, 0.89 mmol, 3.0 eq). The reaction solution was stirred at rt under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 100 mg, yield 70%).

### Step B: N-(3-(S-methylsulfonimidoyl)-1-phenyl-1H-pyrazol-4-yl)isophthalamide

At 0°C, 4 M HCl in 1,4-dioxane (4 mL) was added to tert-butyl (4-(3-carbamoylbenzamido)-1-phenyl-1H-pyrazol-3-yl)(methyl)(oxo)-λ⁶-sulfinyl)carbamate (100 mg, 0.21 mmol, 1.0 eq). The reaction solution was stirred at rt under N₂ for 2 h. Upon completion, the filtrate was concentrated under reduced pressure. The residue was purified by preparative HPLC to afford the title compound (white solid, 56.7 mg, yield 71%). **¹H NMR** (400 MHz, DMSO-*d₆*) δ 11.01 (s, 1H), 9.05 (s, 1H), 8.42 (s, 1H), 8.17 (s, 1H), 8.12 (d, *J* = 7.7 Hz, 1H), 8.01 (d, *J* = 7.9 Hz, 1H), 7.94 (d, *J =* 8.1 Hz, 2H), 7.69 (t, *J =* 7.7 Hz, 1H), 7.62-7.51 (m, 3H), 7.45 (t, *J* = 7.4 Hz, 1H), 5.35 (s, 1H), 3.39 (s, 3H). LCMS (ESI): m/z 384.2 [M+H]⁺.

### Preparation Example 100. Synthesis of Compound A105

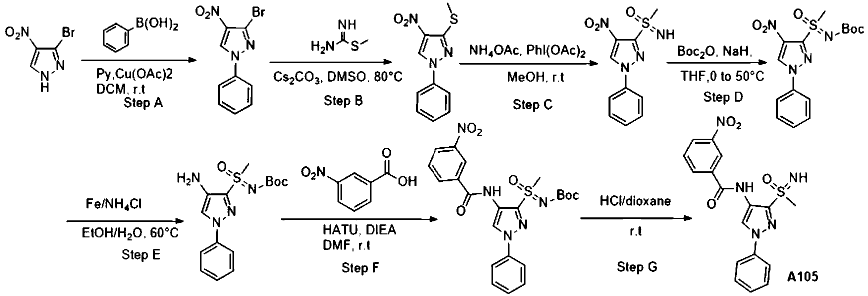

### Step A: 3-Bromo-4-nitro-1-phenyl-1H-pyrazole

At rt, to a solution of 3-bromo-4-nitro-1H-pyrazole (300 mg, 1.56 mmol, 1.0 eq) and phenylboronic acid (381 mg, 3.1 mmol, 2.0 eq) in DCM (20 mL) were added copper acetate (468 mg, 2.3 mmol, 1.5 eq) and pyridine (494 mg, 6.2 mmol, 4.0 eq). The reaction solution was stirred under O₂ at rt for 2 h. Upon completion, the reaction solution was filtered, and the filtrate was diluted in water and extracted with DCM (×3). The combined organic phases were washed with water and saturated aqueous NaCl, dried over anhydrous Na₂SO₄, and concentrated under reduced pressure. The residue was purified by column chromatography to afford the title compound (white solid, 370 mg, yield 88%).

### Step B: 3-(methylthio)-4-nitro-1-phenyl-1H-pyrazole

At rt, to a solution of 3-bromo-4-nitro-1-phenyl-1H-pyrazole (370 mg, 1.38 mmol, 1.0 eq) and azacyclic(methylthio)methanamine (187 mg, 2.1 mmol, 1.5 eq) in dimethyl sulfoxide (15 mL) was added cesium carbonate (1.79 g, 5.5 mmol, 4.0 eq). The mixture was stirred at 80°C under N₂ for 1 h. Upon completion, the reaction was quenched with water, and extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow solid, 290 mg, yield 89%).

### Step C: Imino(methyl)(4-nitro-1-phenyl-1H-pyrazol-3-yl)-λ⁶-sulfanone

At rt, to a solution of 3-(methylthio)-4-nitro-1-phenyl-1H-pyrazole (290 mg, 1.23 mmol, 1.0 eq) and ammonium acetate (950 mg, 12.3 mmol, 10.0 eq) in MeOH (5 mL) was added (diacetoxyiodo)benzene (1.19 g, 3.7 mmol, 3.0 eq). The mixture was stirred at rt under N₂ for 2 h, Upon completion, the mixture was concentrated under reduced pressure. The residue was purified by column chromatography to afford the title compound (yellow solid, 280 mg, yield 85%).

### Step D: Tert-butyl (4-nitro-1-phenyl-1H-pyrazol-3-yl)(oxo)-λ⁶-sulfinyl)carbamate

At 0°C under N₂, to a solution of imino(methyl)(4-nitro-1-phenyl-1H-pyrazol-3-yl)-λ⁶-sulfanone (260 mg, 0.98 mmol, 1.0 eq) in THF (10 mL) was added 60 wt% sodium hydride (78 mg, 1.95 mmol, 2.0 eq) in portions. The mixture was stirred at rt for 1 h. To the above mixture was then added di-tert-butyl dicarbonate (426 mg, 1.95 mmol, 2.0 eq). The mixture was stirred at 50°C under N₂ for 3 h. Upon completion, the mixture was cooled to rt, quenched with saturated aqueous NH₄Cl, and extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow solid, 310 mg, yield 86%).

### Step E: Tert-butyl (4-amino-1-phenyl-1H-pyrazol-3-yl)(methyl)(oxo)-λ⁶-sulfinyl)carbamate

At rt, to a solution of tert-butyl (4-nitro-1-phenyl-1H-pyrazol-3-yl)(oxo)-λ⁶-sulfinyl)carbamate (280 mg, 0.76 mmol, 1.0 eq) in ethanol (5 mL) and water (5 mL) were added iron powder (213 mg, 3.8 mmol, 5.0 eq) and ammonium chloride (408 mg, 7.6 mmol, 10.0 eq). The mixture was stirred at 60°C for 0.5 h. Upon completion, the mixture was filtered, and the filtrate was concentrated under reduced pressure to remove ethanol. The mixture was diluted in water and extracted twice with EtOAc. The combined organic phases were washed with water and saturated aqueous NaCl, dried over anhydrous Na₂SO₄, and concentrated under reduced pressure. The residue was purified by column chromatography to afford the title compound (yellow solid, 180 mg, yield 70%).

### Step F: Tert-butyl (4-(3-nitrobenzamido)-1-phenyl-1H-pyrazol-3-yl)(oxo)-λ⁶-sulfinyl)carbamate

At rt, to a solution of 3-nitrobenzoic acid (67 mg, 0.40 mmol, 1.5 eq) and HATU (152 mg, 0.40 mmol, 1.5 eq) in DMF (4 mL) were added DIEA (103 mg, 0.80 mmol, 3.0 eq) and tert-butyl (4-amino-1-phenyl-1H-pyrazol-3-yl)(methyl)(oxo)-λ⁶-sulfinyl)carbamate (90 mg, 0.27 mmol, 1.0 eq). The reaction solution was stirred at rt under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 100 mg, yield 77%).

### Step G: N-(3-(S-methylsulfonimidoyl)-1-phenyl-1H-pyrazol-4-yl)-3-nitrobenzamide

At 0°C, 4 M HCl in 1,4-dioxane (4 mL) was added to tert-butyl (4-(3-nitrobenzamido)-1-phenyl-1H-pyrazol-3-yl)(oxo)-λ⁶-sulfinyl)carbamate (90 mg, 0.18 mmol, 1.0 eq). The reaction solution was stirred at rt under N₂ for 2 h. Upon completion, the filtrate was concentrated under reduced pressure. The residue was purified by preparative HPLC to afford the title compound (white solid, 30.4 mg, yield 42%). **¹H NMR** (400 MHz, DMSO-*d₆*) δ 11.17 (s, 1H), 9.06 (s, 1H), 8.67 (s, 1H), 8.50 (dd, *J =* 8.1, 1.9 Hz, 1H), 8.31 (d, *J* = 7.8 Hz, 1H), 7.93 (dd, *J =* 14.8, 7.7 Hz, 3H), 7.59 (t, *J =* 7.9 Hz, 2H), 7.46 (t, *J =* 7.4 Hz, 1H), 5.35 (s, 1H), 3.40 (s, 3H). LCMS (ESI): m/z 386 [M+H]⁺.

### Preparation Example 101. Synthesis of Compound A106

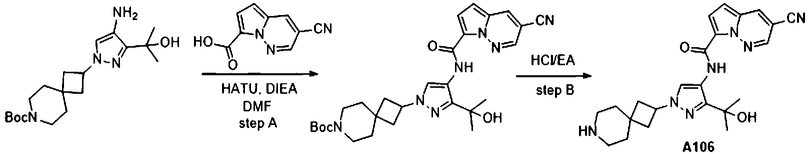

### Step A: Tert-butyl 2-(4-(3-cyanopyrrolo[1,2-b]pyridazine-7-carboxamido)-3-(2-hydroxypropan-2-yl)-1H-pyrazol-1-yl)-7-azaspiro[3.5]nonane-7-carboxylate

To a solution of 3-isocyanopyrrolo[1,2-b]pyridazine-7-carboxylic acid (77 mg, 0.41 mmol, 1.0 eq), tert-butyl 2-(4-amino-3-(2-hydroxypropan-2-yl)-1H-pyrazol-1-yl)-7-azaspiro[3.5]nonane-7-carboxylate (150 mg, 0.41 mmol, 1.0 eq), and DIEA (160 mg, 1.24 mmol, 3.0 eq) in DMF (5 mL) was added HATU (235 mg, 0.62 mmol, 1.5 eq) at 0°C under N₂. The reaction was stirred under N₂ for 1 h. Water was then added, and the mixture was extracted, dried, and concentrated in vacuo. The residue was purified by flash column chromatography to afford tert-butyl 2-(4-(3-cyanopyrrolo[1,2-b]pyridazine-7-carboxamido)-3-(2-hydroxypropan-2-yl)-1H-pyrazol-1-yl)-7-azaspiro[3.5]nonane-7-carboxylate (white solid, 150 mg, yield 68.30%).

### Step B: 3-Cyano-N-(3-(2-hydroxypropan-2-yl)-1-(7-azaspiro[3.5]nonan-2-yl)-1H-pyrazol-4-yl)pyrrolo[1,2-b]pyridazine-7-carboxamide

A solution of tert-butyl 2-(4-(3-cyanopyrrolo[1,2-b]pyridazine-7-carboxamido)-3-(2-hydroxypropan-2-yl)-1H-pyrazol-1-yl)-7-azaspiro[3.5]nonane-7-carboxylate (150 mg, 0.28 mmol, 1.0 eq) in HCl/EA (4 N, 5 mL) was stirred under N₂ at 0°C for 1 h. The reaction mixture was concentrated in vacuo, The residue was purified by preparative high performance liquid chromatography to afford 3-cyano-N-(3-(2-hydroxypropan-2-yl)-1-(7-azaspiro[3.5]nonan-2-yl)-1H-pyrazol-4-yl)pyrrolo[1,2-b]pyridazine-7-carboxamide (white solid, 38.7 mg, yield 31.76%). LCMS (ESI) m/z: 434 [M+H]⁺. **¹HNMR** (400 MHz, DMSO) δ 11.75 (s, 1H), 8.95 (d, *J* = 2.2 Hz, 1H), 8.73 (d, *J=* 2.2 Hz, 1H), 8.45-8.28 (m, 2H), 7.70 (d, *J =* 4.8 Hz, 1H), 7.09 (d, *J =* 4.8 Hz, 1H), 4.86-4.78 (m, 1H), 2.90-2.80 (m, 4H), 2.40-2.35 (m, 2H), 2.20-2.15 (m, 2H), 1.74-1.66 (m, 4H), 1.55 (s, 6H).

### Preparation Example 102. Synthesis of Compound A107

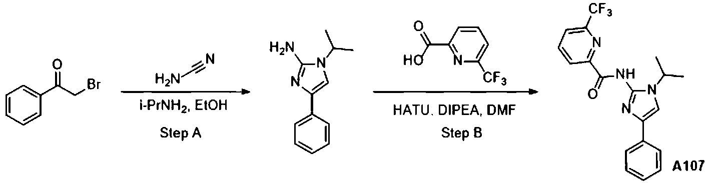

### Step A: 1-Isopropyl-4-phenyl-1H-imidazol-2-amine

To a solution of 2-bromo-1-phenylethan-1-one (2 g, 10.0 mmol, 1.0 eq) in ethanol (40 mL) was added propan-2-amine (0.820 mL, 9.55 mmol, 1.0 eq) at rt. The mixture was stirred for 0.5 h. To the above mixture was added cyanamide (1.27 g, 30.1 mmol, 3.0 eq), and the pH was adjusted to 4 with 1 N HCl solution. The reaction solution was stirred at 95°C under N₂ for 18 h. Upon completion, the mixture was concentrated under reduced pressure. The residue was purified by column chromatography to afford the title compound (yellow solid, 500 mg, yield 25%).

### Step B: N-(1-isopropyl-4-phenyl-1H-imidazol-2-yl)-6-(trifluoromethyl)picolinamide

To a solution of 6-(trifluoromethyl)picolinic acid (228 mg, 1.19 mmol, 1.2 eq) in DMF (8 mL) were added DIEA (256 mg, 1.99 mmol, 2.0 eq) and HATU (567 mg, 1.49 mmol, 1.5 eq) at rt. The mixture was stirred at 50°C for 0.5 h. To the above mixture was added 1-isopropyl-4-phenyl-1H-imidazol-2-amine (200 mg, 0.994 mmol, 1.0 eq). The reaction solution was stirred at 50°C for 18 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 50.5 mg, yield 14%). **¹H NMR** (400 MHz, DMSO-*d₆*) δ 10.70 (s, 1H), 8.42-8.34 (m, 2H), 8.21 (d, *J =* 7.3 Hz, 1H), 7.87 (s, 1H), 7.77 (d, *J =* 7.6 Hz, 2H), 7.36 (t, *J =* 7.6 Hz, 2H), 7.20 (t, *J =* 7.3 Hz, 1H), 4.41-4.17 (m, 1H), 1.40 (d, *J =* 6.7 Hz, 6H). LCMS (ESI): m/z 375 [M+H]⁺.

### Preparation Example 103. Synthesis of Compound A108

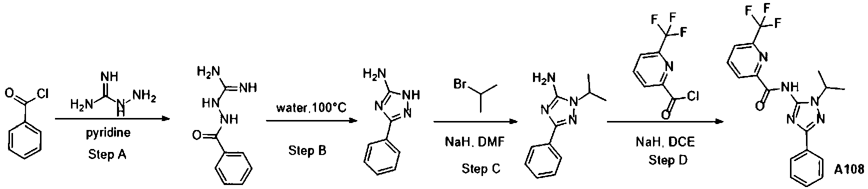

### Step A: 2-benzoylhydrazine-1-carboximide

At 0°C, to a solution of benzoyl chloride (2 g, 14.228 mmol, 1.0 eq) in pyridine (20 mL) was added portionwise carbohydrazide (1.05 g, 14.228 mmol, 1.0 eq). The mixture was stirred at 0°C for 0.5 h, then warmed to rt and stirred for 12 h. Upon completion, the mixture was concentrated under reduced pressure. The residue was dissolved in water (20 mL) and the pH was adjusted to 12 using 2N NaOH solution until the product precipitated. The precipitate was filtered, and the filter cake was dried completely to afford the title compound (red solid, 1.3 g, yield 51%).

### Step B: 3-phenyl-1H-1,2,4-triazol-5-amine

A solution of 2-benzoylhydrazine-1-carboximide (280 mg, 1.12 mmol, 1.0 eq) in water (5 mL) was stirred in a sealed tube at 80°C for 2 h. Upon completion, the mixture was cooled to rt, and the precipitate was filtered. The filter cake was washed with ice water, and dried completely to afford the title compound (white solid, 215 mg, yield 60%).

### Step C: 1-isopropyl-3-phenyl-1H-1,2,4-triazol-5-amine

At 0°C under N₂, to a solution of 60 wt% sodium hydride (75.22 mg, 3.134 mmol, 2.0 eq) in DMF (3 mL) was added 3-phenyl-1H-1,2,4-triazol-5-amine (251 mg, 1.567 mmol, 1.0 eq). The mixture was stirred at rt for 0.5 h. To the above mixture was then added 2-bromopropane (0.148 mL, 1.567 mmol, 1.0 eq). The mixture was stirred at rt under N₂ for 3 h. Upon completion, the reaction was quenched with saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by column chromatography to afford the title compound (green solid, 106 mg, yield 33%).

### Step D: N-(1-Isopropyl-3-phenyl-1H-1,2,4-triazol-5-yl)-6-(trifluoromethyl)picolinamide

To a solution of sodium hydride (60 wt%, 14.24 mg, 0.593 mmol, 1.5 eq) in dichloroethane (3 mL) was added 1-isopropyl-3-phenyl-1H-1,2,4-triazol-5-amine (80 mg, 0.396 mmol, 1.0 eq) at 0°C under N₂. The mixture was stirred at rt for 0.5 h. To the above mixture was then added 6-(trifluoromethyl)picolinoyl chloride (165.77 mg, 0.791 mmol, 2.0 eq). The mixture was stirred at rt under N₂ for 3 h. Upon completion, the reaction was quenched with saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by column chromatography to afford the title compound (white solid, 56 mg , yield 38%). **¹H NMR** (400 MHz, CD₃OD) δ 8.46 (d, *J =* 7.8 Hz, 1H), 8.31 (t, *J =* 7.9 Hz, 1H), 8.11 (d, *J =* 7.7 Hz, 1H), 8.03 (dd, *J =* 7.9, 1.5 Hz, 2H), 7.52-7.36 (m, 3H), 4.67-4.56 (m, 1H), 1.55 (d, *J =* 6.6 Hz, 6H). LCMS (ESI): m/z 376 [M+H]⁺.

### Preparation Example 104. Synthesis of Compound A229

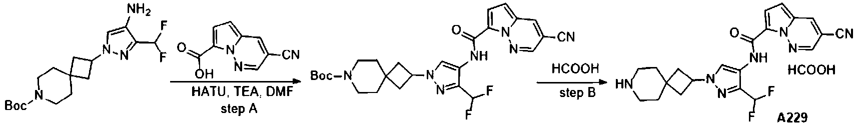

### Step A: Tert-butyl 2-(4-(3-cyanopyrrolo[1,2-b]pyridazine-7-carboxamido)-3-(difluoromethyl)-1H-pyrazol-1-yl)-7-azaspiro[3.5]nonane-7-carboxylate

To a solution of tert-butyl 2-(4-amino-3-(difluoromethyl)-1H-pyrazol-1-yl)-7-azaspiro[3.5]nonane-7-carboxylate (322.8 mg, 0.9 mmol, 1.0 eq) and 3-cyanopyrrolo[1,2-b]pyridazine-7-carboxylic acid (186.4 mg, 1.0 mmol, 1.1 eq) in DMF (5 mL) were added HATU (513.0 mg, 1.4 mmol, 1.5 eq) and TEA (181.8 mg, 1.8 mmol, 2.0 eq) at rt. The reaction solution was stirred at rt under N₂ for 1 h. Upon completion, the reaction solution was poured into water (50 mL) and extracted with EtOAc (50 mL×3). The combined organic phases were washed sequentially with water and saturated aqueous NaCl, dried over anhydrous Na₂SO₄, and concentrated under reduced pressure. The residue was purified by column chromatography to afford the title compound (yellow solid, 410.0 mg, yield 86%).

### Step B: 3-Cyano-N-(3-(difluoromethyl)-1-(7-azaspiro[3.5]nonan-2-yl)-1H-pyrazol-4-yl)pyrrolo[1,2-b]pyridazine-7-carboxamide

A solution of tert-butyl 2-(4-(3-cyanopyrrolo[1,2-b]pyridazine-7-carboxamido)-3-(difluoromethyl)-1H-pyrazol-1-yl)-7-azaspiro[3.5]nonane-7-carboxylate (140.0 mg, 0.3 mmol, 1.0 eq) was dissolved in formic acid (2 mL) at rt, and the mixture was stirred at rt for 2 h. Upon completion, the mixture was concentrated under reduced pressure to remove the solvent. The residue was purified by HPLC to afford the title compound (yellow solid, formate, 109.7 mg, yield 87%). **¹H NMR** (400 MHz, DMSO-*d₆*) δ 10.92 (s, 1H), 8.99 (d, *J =* 2.2 Hz, 1H), 8.85 (d, *J =* 2.2 Hz, 1H), 8.45 (s, 1H), 8.40 (s, 1H), 7.74 (d, *J =* 4.8 Hz, 1H), 7.36-7.08 (m, 2H), 5.00 (p, *J =* 8.3 Hz, 1H), 2.94 (dt, *J =* 34.1, 5.5 Hz, 4H), 2.48-2.37 (m, 2H), 2.33-2.21 (m, 2H), 1.78 (dt, *J =* 20.6, 4.9 Hz, 4H). LCMS (ESI): m/z 426 [M+H]⁺.

### Preparation Example 105. Synthesis of Compound A230

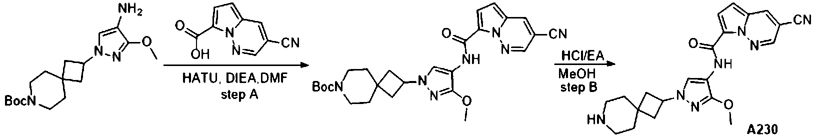

### Step A: Tert-butyl 2-(4-(3-cyanopyrrolo[1,2-b]pyridazine-7-carboxamido)-3-methoxy-1H-pyrazol-1-yl)-7-azaspiro[3.5]nonane-7-carboxylate

Tert-butyl 2-(4-amino-3-methoxy-1H-pyrazol-1-yl)-7-azaspiro[3.5]nonane-7-carboxylate (78.0 mg, 0.23 mmol, 1.0 eq) and DIEA (93.1 mg, 0.72 mmol, 3.0 eq) was added to anhydrous DMF (2 mL), and HATU (137.0 mg, 0.36 mmol, 1.5 eq) and 3-cyanopyrrolo[1,2-b]pyridazine-7-carboxylic acid (58.4 mg, 0.31 mmol, 1.3 eq) were added to anhydrous DMF (2 mL). The two mixtures were separately stirred under N₂ for 30 min, and then combined. The reaction solution was stirred at rt under N₂ for 16 h. The reaction solution was diluted in water (20 mL) and extracted with EtOAc (20 mL×3). The organic phases were combined, washed with aqueous NaCl, dried over anhydrous Na₂SO₄, filtered, and concentrated under reduced pressure to obtain the crude. The crude was purified by column chromatography (41% ethyl acetate/petroleum ether) to afford the title compound (yellow solid, 105.0 mg, yield 87.5%).

### Step B: 3-Cyano-N-(3-methoxy-1-(7-azaspiro[3.5]nonan-2-yl)-1H-pyrazol-4-yl)pyrrolo[1,2-b]pyridazine-7-carboxamide

To a solution of tert-butyl 2-(4-(3-cyanopyrrolo[1,2-b]pyridazine-7-carboxamido)-3-methoxy-1H-pyrazol-1-yl)-7-azaspiro[3.5]nonane-7-carboxylate (105.0 mg, 0.21 mmol, 1.0 eq) in MeOH (5.0 mL) was added 2 M HCl in EtOAc (10 mL). The mixture was stirred at rt for 2 h. LCMS analysis indicated the reaction was complete. The reaction solution was concentrated under reduced pressure to obtain the crude, which was purified by HPLC to afford the title compound (white solid, 35.1 mg, yield 41.3%). **¹H NMR** (400 MHz, DMSO) δ 10.25 (s, 1H), 8.97 (d, *J* = 2.1 Hz, 1H), 8.89 (d, *J* = 2.1 Hz, 1H), 8.40 (s, 1H), 8.09 (s, 1H), 7.70 (d, *J* = 4.8 Hz, 1H), 7.10 (d, *J =* 4.8 Hz, 1H), 4.80-4.68 (m, 1H), 3.93 (s, 3H), 2.94-2.87 (m, 2H), 2.87-2.80 (m, 2H), 2.37-2.29 (m, 2H), 2.23-2.13 (m, 2H), 1.75-1.67 (m, 4H). LCMS (ESI): m/z 406 [M+H]⁺.

### Preparation Example 106. Synthesis of Compound A231

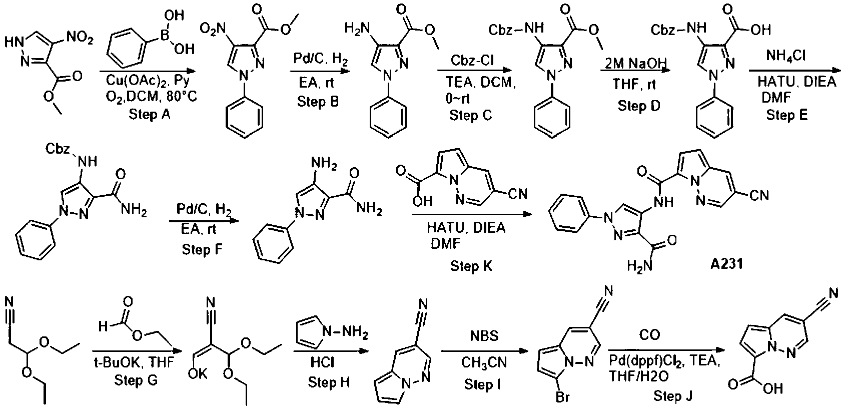

### Step A: Methyl 4-nitro-1-phenyl-1H-pyrazole-3-carboxylate

To a solution of methyl 4-nitro-1H-pyrazole-3-carboxylate (2.5 g, 14.61 mmol, 1.0 eq) in DCM (100 mL) were added copper acetate (3.9 g, 21.91 mmol, 1.5 eq), pyridine (4.72 mL, 58.44 mmol, 4.0 eq), and phenylboronic acid (3.5 g, 29.22 mmol, 2.0 eq) at rt. The reaction solution was reacted at rt for 16 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 2.3 g, yield 63.68%).

### Step B: Methyl 4-amino-1-phenyl-1H-pyrazole-3-carboxylate

To a solution of methyl 4-nitro-1-phenyl-1H-pyrazole-3-carboxylate (2.5 g, 10.11 mmol, 1.0 eq) in MeOH (100 mL) was added palladium on carbon (10%, 0.32 g) at rt. The reaction solution was purged with H₂ (×3), and then reacted at rt for 16 h. Upon completion, the reaction solution was filtered, and the filtrate was subjected to rotary evaporation to dryness to afford the title compound (white solid, 2.0 g, crude).

### Step C: Methyl 4-(((benzyloxy)carbonyl)amino)-1-phenyl-1H-pyrazole-3-carboxylate

To a solution of methyl 4-amino-1-phenyl-1H-pyrazole-3-carboxylate (750 mg, 3.45 mmol, 1.0 eq) and DIEA (1.7 g, 10.35 mmol, 3.0 eq) in THF (30 mL) were added benzyl chloroformate (647 mg, 3.79 mmol, 1.1 eq) and DMAP (85 mg, 0.69 mmol, 0.2 eq) at rt. The reaction solution was reacted at rt for 2 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 1.2 g, yield 98.92%).

### Step D: 4-(((Benzyloxy)carbonyl)amino)-1-phenyl-1H-pyrazole-3-carboxylic acid

To a mixed solution of methyl 4-(((benzyloxy)carbonyl)amino)-1-phenyl-1H-pyrazole-3-carboxylate (330 mg, 0.94 mmol, 1.0 eq) in MeOH (5.0 mL) and water (2.0 mL) was added lithium hydroxide (75 mg, 1.88 mmol, 2.0 eq) at rt. The reaction solution was reacted at 40°C for 8 h. Upon completion, the reaction solution was adjusted to pH 4-5 with 1 N HCl and concentrated under reduced pressure to afford the title compound (white solid, 300 mg, crude).

### Step E: Benzyl (3-carbamoyl-1-phenyl-1H-pyrazol-4-yl)carbamate

To a solution of 4-(((benzyloxy)carbonyl)amino)-1-phenyl-1H-pyrazole-3-carboxylic acid (300 mg, 0.88 mmol, 1.0 eq) and ammonium chloride (238 mg, 4.44 mmol, 5.0 eq) in DMF (5.0 mL) were added DIEA (574 mg, 4.44 mmol, 5.0 eq) and HATU (507 mg, 1.33 mmol, 1.5 eq) at rt. The reaction solution was reacted at rt for 4 h. Upon completion, the reaction solution was poured into NaHCO₃ solution and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 200 mg, yield 66.86%).

### Step F: 4-Amino-1-phenyl-1H-pyrazole-3-carboxamide

To a solution of benzyl (3-carbamoyl-1-phenyl-1H-pyrazol-4-yl)carbamate (200 mg, 0.59 mmol, 1.0 eq) in MeOH (5 mL) was added palladium on carbon (10%, 63 mg) at rt. The mixture was purged with H₂ (×3), and then reacted at rt for 16 h. Upon completion, the mixture was filtered, and the filtrate was subjected to rotary evaporation to dryness to afford the title compound (white solid, 100 mg, crude).

### Step G: Potassium 3,3-diethoxy-2-formylpropanenitrile

To a solution of 3,3-diethoxypropanenitrile (24.5 g, 171.1 mmol, 1.0 eq) and ethyl formate (15.2 g, 205.3 mmol, 1.2 eq) in THF (500 mL) was added potassium tert-butoxide (21.1 g, 188.2 mmol, 1.1 eq) portionwise at 10°C. The mixture was stirred at rt under N₂ for 2 h. Upon completion, n-hexane (800 mL) was then added, and the mixture was stirred for 20 min and filtered. The filter cake was washed once with n-hexane, and dried to afford the title compound (pale yellow solid, 30 g, yield 83%).

### Step H: Pyrrolo[1,2-b]pyridazine-3-carbonitrile

At 0°C, to a solution of potassium 3,3-diethoxy-2-formylpropanenitrile (29 g, 138.1 mmol, 1.0 eq) in MeOH (270 mL) was added concentrated HCl (35 mL, 414.3 mmol, 3.0 eq). The reaction solution was stirred at rt for 30 min. 1H-pyrrol-1-amine (11.3 g, 138.1 mmol, 1.0 eq) was added. The reaction solution was stirred at 90°C for 2 h. After cooling to rt, the reaction solution was concentrated to remove half of the solvent, and the pH was adjusted to 7 with saturated NaHCO₃ solution. The mixture was extracted with EtOAc (×3), and the combined organic phases were washed with water and saturated aqueous NaCl, dried over anhydrous Na₂SO₄, and concentrated under reduced pressure. The residue was purified by column chromatography (PE : EA=3 : 1) to afford the title compound (yellow solid, 15.7 g, yield 79%).

### Step I: 7-Bromopyrrolo[1,2-b]pyridazine-3-carbonitrile

To a solution of pyrrolo[1,2-b]pyridazine-3-carbonitrile (15.7 g, 109.7 mmol, 1.0 eq) in anhydrous acetonitrile (300 mL) was added N-bromosuccinimide (19.5 g, 109.7 mmol, 1.0 eq) at 0°C. The reaction solution was stirred at 0°C under N₂ for 30 min. Upon completion, the reaction solution was poured into saturated NaHCO₃ solution. The mixture was extracted with EtOAc twice, washed with saturated aqueous NaCl, dried over anhydrous Na₂SO₄, and concentrated under reduced pressure. The residue was purified by column chromatography (PE : DCM = 7 : 3) to afford the title compound (yellow solid, 17.7 g, yield 72%).

### Step J: 3-Cyanopyrrolo[1,2-b]pyridazine-7-carboxylic acid

To a 2000 mL reaction flask were added 7-bromopyrrolo[1,2-b]pyridazine-3-carbonitrile (10 g, 45.0 mmol, 1.0 eq) and then THF/H₂O (600 mL/200 mL). The flask was purged with N₂ (×3). Then, TEA (37.5 mL, 270.2 mmol, 6.0 eq), Xant-Phos (2.6 g, 4.5 mmol, 0.1 eq), palladium acetate (505 mg, 2.25 mmol, 0.05 eq), and Pd(dppf)Cl₂ (1.6 g, 2.25 mmol, 0.05 eq) were added. The reaction solution was purged with carbon monoxide and stirred at 100°C under vigorous reflux for 18 h. Upon completion, the reaction solution was cooled to rt and concentrated under reduced pressure. The crude was washed once with water and ethyl acetate, and then dried to afford the title compound (yellow-green solid, 5.7 g, yield 67%).

### Step K: N-(3-amino-1-phenyl-1H-pyrazol-4-yl)-3-cyanopyrrolo[1,2-b]pyridazine-7-carboxamide

To a solution of 4-amino-1-phenyl-1H-pyrazole-3-carboxamide (60 mg, 0.30 mmol, 1.0 eq), DIEA (95 mg, 0.74 mmol, 2.5 eq), and 3-cyanopyrrolo[1,2-b]pyridazine-7-carboxylic acid (61 mg, 0.33 mmol, 1.1 eq) in DMF (3.0 mL) was added HATU (124 mg, 0.33 mmol, 1.1 eq) at rt. The resulting mixture was stirred for 1 h. The mixture was then diluted in water and extracted with EA twice. The combined organic phases were washed with aqueous NaCl, dried over anhydrous Na₂SO₄, filtered, and concentrated to dryness. The residue was purified by preparative HPLC to afford the title compound (yellow solid, 17.4 mg, yield 15.79%). **¹H NMR** (400 MHz, DMSO) δ 12.12 (s, 1H), 8.96 (d, *J =* 2.1 Hz, 1H), 8.70 (d, *J =* 2.1 Hz, 1H), 8.45 (s, 1H), 7.73 (d, *J* = 4.8 Hz, 1H), 7.56 (s, 1H), 7.43 (s, 1H), 7.09 (d, *J* = 4.8 Hz, 1H), 4.24 (m, 1H), 2.06 (m, 2H), 1.88-1.64 (m, 5H), 1.51-1.35 (m, 2H), 1.30-1.17 (m, 1H).

### Preparation Example 107. Synthesis of Compound A232

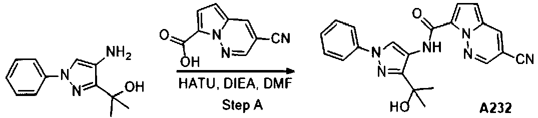

### Step A: 3-Cyano-N-(3-(2-hydroxypropan-2-yl)-1-phenyl-1H-pyrazol-4-yl)pyrrolo[1,2-b]pyridazine-7-carboxamide

At rt, to a solution of 2-(4-amino-1-phenyl-1H-pyrazol-3-yl)propan-2-ol (60 mg, 0.28 mmol, 1.0 eq), DIEA (89 mg, 0.69 mmol, 2.5 eq), and 3-cyanopyrrolo[1,2-b]pyridazine-7-carboxylic acid (57 mg, 0.30 mmol, 1.1 eq) in DMF (3.0 mL) was added HATU (115 mg, 0.30 mmol, 1.1 eq). The resulting mixture was stirred for 1 h. The mixture was then diluted in water and extracted with EA twice. The combined organic phases were washed with aqueous NaCl, dried over anhydrous Na₂SO₄, filtered, and concentrated to dryness. The residue was purified by preparative HPLC to afford the title compound (yellow solid, 15.2 mg, yield 14.24%). LCMS (ESI): m/z 369 [M+H-18]⁺/387 [M+H]⁺. **¹H NMR** (400 MHz, DMSO) δ 11.89 (s, 1H), 8.96 (d, *J* = 2.0 Hz, 1H), 8.88 (s, 1H), 8.75 (d, *J* = 2.0 Hz, 1H), 7.77 (dd, *J =* 12.2, 6.5 Hz, 3H), 7.49 (t, *J =* 7.9 Hz, 2H), 7.27 (t, *J* = 7.3 Hz, 1H), 7.11 (d, *J* = 4.8 Hz, 1H), 5.80 (s, 1H), 1.63 (s, 6H).

### Preparation Example 108. Synthesis of Compound A233

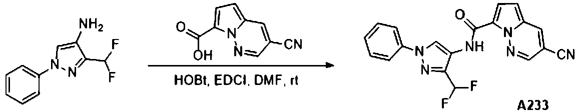

### Step A: 3-Cyano-N-(3-(difluoromethyl)-1-phenyl-1H-pyrazol-4-yl)pyrrolo[1,2-b]pyridazine-7-carboxamide

To a solution of 3-(difluoromethyl)-1-phenyl-1H-pyrazol-4-amine (50.0 mg, 0.24 mmol, 1.0 eq), 3-cyanopyrrolo[1,2-b]pyridazine-7-carboxylic acid (44.7 mg, 0.24 mmol, 1.0 eq), and HOBt (38.8 mg, 0.29 mmol, 1.2 eq) in DMF (2 mL) was added EDCI (55.0 mg, 0.29 mmol, 1.2 eq). The reaction solution was stirred at rt under N₂ for 18 h. Upon completion, the reaction solution was quenched with water and extracted with DCM. The organic phase was washed with saturated aqueous NaCl, dried over anhydrous Na₂SO₄, and concentrated under reduced pressure. The residue was purified by column chromatography to afford the title compound (yellow solid, 43.8 mg, yield 48.4%). LCMS (ESI): m/z 379.3 [M+H]⁺. **¹H NMR** (400 MHz, CDCl₃) δ 11.25 (s, 1H), 8.91 (s, 1H), 8.47 (d, *J* = 2.1 Hz, 1H), 8.32 (d, *J* = 2.1 Hz, 1H), 7.92 (d, *J* = 4.9 Hz, 1H), 7.78-7.70 (m, 2H), 7.49 (t, *J =* 8.0 Hz, 2H), 7.35 (t, *J* = 7.4 Hz, 1H), 7.12-6.77 (m, 2H).

### Preparation Example 109. Synthesis of Compound A234

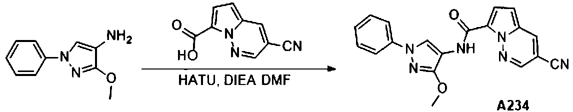

### Step A: 3-Isocyano-N-(3-methoxy-1-phenyl-1H-pyrazol-4-yl)pyrrolo[1,2-b]pyridazine-7-carboxamide

3-methoxy-1-phenyl-1H-pyrazol-4-amine (49.0 mg, 0.26 mmol, 1.0 eq) was dissolved in anhydrous DMF (2 mL), and DIEA (100.6 mg, 0.78 mmol, 3.0 eq) was added. Separately, 3-cyanopyrrolo[1,2-b]pyridazine-7-carboxylic acid (63.6 mg, 0.34 mmol, 1.3 eq) and HATU (148.2 mg, 0.39 mmol, 1.5 eq) were dissolved in anhydrous DMF (2 mL). Each solution was stirred under N₂ for 30 min, then combined and stirred at rt under N₂ for 16 h. The reaction solution was diluted in water (20 mL) and extracted with EtOAc (20 mL×3). The combined organic phases were washed with aqueous NaCl, dried over anhydrous Na₂SO₄, filtered, and concentrated under reduced pressure. The crude was purified by HPLC to afford the title compound (yellow solid, 31.5 mg, yield 33.8%). **¹H NMR** (400 MHz, DMSO) δ 10.42 (s, 1H), 8.99 (d, *J =* 2.0 Hz, 1H), 8.92 (d, *J =* 2.0 Hz, 1H), 8.71 (s, 1H), 7.78-7.73 (m, 3H), 7.46 (t, *J =* 7.9 Hz, 2H), 7.22 (t, *J =* 7.4 Hz, 1H), 7.12 (d, *J =* 4.8 Hz, 1H), 4.06 (s, 3H). LCMS (ESI): m/z 359 [M+H]⁺.

### Preparation Example 110. Synthesis of Compound A235

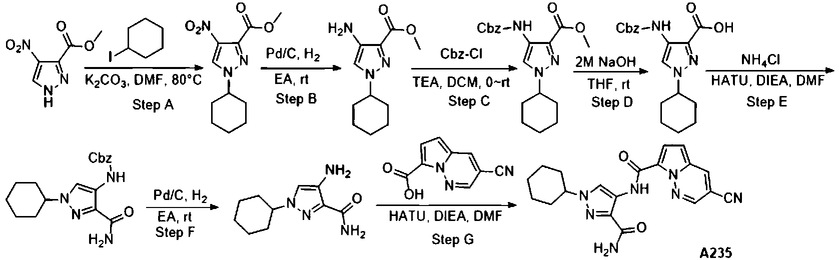

### Step A: Methyl 1-cyclohexyl-4-nitro-1H-pyrazole-3-carboxylate

To a solution of methyl 4-nitro-1H-pyrazole-3-carboxylate (1.72 g, 10.05 mmol, 1.0 eq) and K₂CO₃ (6.95 g, 50.26 mmol, 5.0 eq) in DMF (30 mL) was added iodocyclohexane (3.17 g, 15.08 mmol, 1.5 eq) at rt. The mixture was heated to 80°C and stirred for 18 h. The reaction solution was then diluted in water and extracted twice with EA (50 mL). The combined organic layers were washed with aqueous NaCl, dried over anhydrous Na₂SO₄, filtered, and concentrated under reduced pressure. The residue was purified by column chromatography to afford the title compound (light yellow oil, 0.7 g, yield 27.45%).

### Step B: Methyl 4-amino-1-cyclohexyl-1H-pyrazole-3-carboxylate

To a solution of methyl 1-cyclohexyl-4-nitro-1H-pyrazole-3-carboxylate (700 mg, 2.76 mmol, 1.0 eq) in EA (10 mL) was added Pd/C (10%, 70 mg, loaded on activated carbon). The mixture was stirred at rt under a H₂ balloon for 18 h. The mixture was filtered, and the filtrate was concentrated to afford the title compound (550 mg, yield 89.12%).

### Step C: Methyl 4-(((benzyloxy)carbonyl)amino)-1-cyclohexyl-1H-pyrazole-3-carboxylate

To a mixture of methyl 4-amino-1-cyclohexyl-1H-pyrazole-3-carboxylate (550 mg, 2.46 mmol, 1.0 eq) and TEA (1.03 mL, 7.39 mmol, 3.0 eq) in DCM (10 mL) was added CbzCl (0.44 mL, 3.08 mmol, 1.25 eq) at 0°C. The mixture was warmed to rt and stirred for 6 h. The mixture was poured into water and extracted with EA twice. The combined organic phases were washed with aqueous NaCl, dried over anhydrous Na₂SO₄, filtered, and concentrated to dryness. The residue was purified by column chromatography to afford the title compound (420 mg, yield 47.71%).

### Step D: 4-(((Benzyloxy)carbonyl)amino)-1-cyclohexyl-1H-pyrazole-3-carboxylic acid

To a solution of methyl 4-(((benzyloxy)carbonyl)amino)-1-cyclohexyl-1H-pyrazole-3-carboxylate (150 mg, 0.42 mmol, 1.0 eq) in THF (2.0 mL) was added 2 M NaOH (0.53 mL, 1.05 mmol, 2.5 eq). The mixture was stirred at 50°C for 3 h. After cooling, the mixture was diluted in EA and adjusted to pH 2-3 with 2 M HCl. The aqueous layer was extracted with EA twice. The combined organic layers were washed with aqueous NaCl, dried over anhydrous Na₂SO₄, filtered, and concentrated to dryness to afford the title compound (white solid, 130 mg, yield 90.21%).

### Step E: Benzyl (3-carbamoyl-1-cyclohexyl-1H-pyrazol-4-yl)carbamate

To a solution of 4-(((benzyloxy)carbonyl)amino)-1-cyclohexyl-1H-pyrazole-3-carboxylic acid (130 mg, 0.38 mmol, 1.0 eq), DIEA (122 mg, 0.95 mmol, 2.5 eq), and ammonium chloride (24 mg, 0.45 mmol, 1.2 eq) in DMF (5 mL) was added HATU (173 mg, 0.45 mmol, 1.5 eq) at rt. The resulting mixture was stirred for 1 h. The reaction solution was then diluted in water and extracted with EA twice. The combined organic phases were washed with aqueous NaCl, dried over anhydrous Na₂SO₄, filtered, and concentrated to dryness. The residue was purified by column chromatography to afford the title compound (white solid, 90 mg, yield 69.43%).

### Step F: 4-Amino-1-cyclohexyl-1H-pyrazole-3-carboxamide

To a solution of benzyl (3-carbamoyl-1-cyclohexyl-1H-pyrazol-4-yl)carbamate (90 mg, 0.26 mmol, 1.0 eq) in MeOH (3 mL) was added Pd/C (10%, 45 mg, loaded on activated carbon) at rt. The mixture was stirred at rt under a H₂ balloon for 18 h. The mixture was filtered, and the filtrate was concentrated to afford the title compound (45 mg, yield 82.20%).

### Step G: N-(3-amino-1-cyclohexyl-1H-pyrazol-4-yl)-3-cyanopyrrolo[1,2-b]pyridazine-7-carboxamide

To a solution of 4-amino-1-cyclohexyl-1H-pyrazole-3-carboxamide (45 mg, 0.22 mmol, 1.0 eq), DIEA (69 mg, 0.54 mmol, 2.5 eq), and 3-cyanopyrrolo[1,2-b]pyridazine-7-carboxylic acid (44 mg, 0.24 mmol, 1.1 eq) in DMF (3 mL) was added HATU (90 mg, 0.24 mmol, 1.1 eq) at rt. The resulting mixture was stirred for 1 h. The reaction solution was then diluted in water and extracted with EA twice. The combined organic phases were washed with aqueous NaCl, dried over anhydrous Na₂SO₄, filtered, and concentrated to dryness. The residue was purified by preparative HPLC to afford the title compound (yellow solid, 20.1 mg, yield 22.18%). **¹H NMR** (400 MHz, DMSO) δ 12.12 (s, 1H), 8.96 (d, *J =* 2.1 Hz, 1H), 8.70 (d, *J* = 2.1 Hz, 1H), 8.45 (s, 1H), 7.73 (d, *J =* 4.8 Hz, 1H), 7.56 (s, 1H), 7.43 (s, 1H), 7.09 (d, *J =* 4.8 Hz, 1H), 4.24 (m, 1H), 2.06 (m, 2H), 1.88-1.64 (m, 5H), 1.51-1.35 (m, 2H), 1.30-1.17 (m, 1H).

### Preparation Example 111. Synthesis of Compound A236

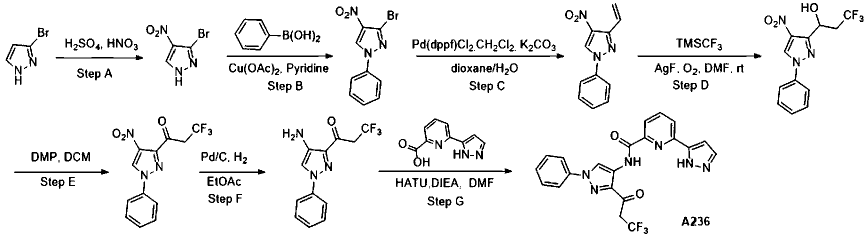

### Step A: 3-Bromo-4-nitro-1H-pyrazole

At rt, 3-bromo-1H-pyrazole (5.0 g, 34.0 mmol, 1.0 eq) was dissolved in concentrated sulfuric acid (10 mL). To this solution, concentrated nitric acid (5 mL, 34.0 mmol, 1.0 eq) was added dropwise. The reaction solution was reacted at 50°C for 3 h. Upon completion, the reaction solution was poured into saturated NaHCO₃ solution (50 mL) and extracted with EtOAc (50 mL×3). The organic phases were combined, washed with saturated aqueous NaCl (50 mL), dried over anhydrous Na₂SO₄, filtered, and subjected to rotary evaporation to dryness to afford the title compound (yellow solid, 5.0 g, yield 77%).

### Step B: 3-Bromo-4-nitro-1-phenyl-1H-pyrazole

To a solution of 3-bromo-4-nitro-1H-pyrazole (2.0 g, 10.4 mmol, 1.0 eq) in DCM (20 mL) were added phenylboronic acid (2.54 g, 20.8 mmol, 2.0 eq), copper acetate (4.16 g, 20.8 mmol, 1.0 eq), and pyridine (1.7 mL, 20.8 mmol, 2.0 eq). The reaction was stirred at rt under O₂ overnight. Upon completion, the reaction solution was diluted in water (20 mL) and extracted with DCM (50 mL×3). The organic phases were combined, washed with saturated aqueous NaCl (20 mL), dried over anhydrous Na₂SO₄, filtered, and subjected to rotary evaporation to dryness. The crude was separated by column chromatography to afford the title compound (yellow solid, 1.5 g, yield 54%).

### Step C: 4-nitro-1-phenyl-3-vinyl-1H-pyrazole

To a solution of 3-bromo-4-nitro-1-phenyl-1H-pyrazole (1.0 g, 3.73 mmol, 1.0 eq) in 1,4-dioxane (10 mL) and water (2 mL) were added 4,4,5,5-tetramethyl-2-vinyl-1,3,2-dioxaborolane (0.86 g, 5.60 mmol, 1.5 eq), potassium carbonate (1.03 g, 7.46 mmol, 2.0 eq), and Pd(dppf)Cl₂ (3.05 g, 3.73 mmol, 1.0 eq). The reaction solution was reacted at 80°C under N₂ for 2 h. Upon completion, the reaction solution was diluted in water (20 mL) and extracted with EtOAc (20 mL×3). The organic phases were combined, washed with saturated aqueous NaCl (20 mL), dried over anhydrous Na₂SO₄, filtered, and subjected to rotary evaporation to dryness. The crude was separated by column chromatography to afford the title compound (yellow solid, 700 mg, yield 87%).

### Step D: 3,3,3-trifluoro-1-(4-nitro-1-phenyl-1H-pyrazol-3-yl)propan-1-ol

To a solution of silver fluoride (1.18 g, 9.30 mmol, 2.0 eq) in DMF (10 mL) were added 4-nitro-1-phenyl-3-vinyl-1H-pyrazole (1.0 g, 4.65 mmol, 1.0 eq) and (trifluoromethyl)trimethylsilane (1.32 g, 9.30 mmol, 2.0 eq). The reaction solution was reacted at 35°C under O₂ for 12 h. Upon completion, ethyl acetate (30 mL) was added to the reaction system, and the mixture was filtered to remove solid impurities. The mother liquor was diluted in water (30 mL) and extracted with EtOAc (30 mL×3). The organic phases were combined, washed with saturated aqueous NaCl (20 mL), dried over anhydrous Na₂SO₄, filtered, and subjected to rotary evaporation to dryness. The crude was separated by column chromatography to afford the title compound (yellow solid, 280 mg, yield 20%).

### Step E: 3,3,3-Trifluoro-1-(4-nitro-1-phenyl-1H-pyrazol-3-yl)propan-1-one

To a solution of 3,3,3-trifluoro-1-(4-nitro-1-phenyl-1H-pyrazol-3-yl)propan-1-ol (250 mg, 0.83 mmol, 1.0 eq) in DCM (10 mL) was added Dess-Martin periodinane (458 mg, 1.08 mmol, 1.3 eq). The reaction solution was reacted at rt for 1 h. Upon completion, the reaction solution was subjected to rotary evaporation to dryness, and the residue was purified by column chromatography to afford the title compound (yellow solid, 200 mg, yield 81%).

### Step F: 1-(4-amino-1-phenyl-1H-pyrazol-3-yl)-3,3-trifluoropropan-1-one

To a solution of 3,3,3-trifluoro-1-(4-nitro-1-phenyl-1H-pyrazol-3-yl)propan-1-one (200 mg, 0.67 mmol, 1.0 eq) in EtOAc (10 mL) was added wet Pd/C (71 mg). The reaction solution was reacted at rt under H₂ for 14 h. Upon completion, the mixture was filtered to remove Pd/C, and the reaction solution was subjected to rotary evaporation to dryness to afford the title compound (yellow solid, 150 mg, yield 83%).

### Step G: N-(1-Phenyl-3-(3,3,3-trifluoropropanoyl)-1H-pyrazol-4-yl)-6-(1H-pyrazol-5-yl)picolinamide

To a solution of 6-(1H-pyrazol-5-yl)picolinic acid (500 mg, 0.26 mmol, 1.0 eq) in DMF (3 mL) were added HATU (101 mg, 0.26 mmol, 1.0 eq), DIEA (68 mg, 0.53 mmol, 2.0 eq), and 1-(4-amino-1-phenyl-1H-pyrazol-3-yl)-3,3-trifluoropropan-1-one (71 mg, 0.26 mmol, 1.0 eq). The reaction solution was reacted at rt for 2 h. Upon completion, the crude was purified by reverse-phase column chromatography to afford the title compound (yellow solid, 15.1 mg, yield 13%). **¹H NMR** (400 MHz, DMSO-*d₆*) δ 13.26 (s, 1H), 11.75 (s, 1H), 9.14 (s, 1H), 8.27 (d, *J =* 7.6 Hz, 1H), 8.18-7.98 (m, 5H), 7.61 (t, *J =* 7.9 Hz, 2H), 7.48 (t, *J =* 7.4 Hz, 1H), 7.32 (s, 1H), 4.44 (q, *J =* 11.1 Hz, 2H). LCMS(ESI): m/z 441.2 [M+H]⁺.

### Preparation Example 112. Synthesis of Compound A237

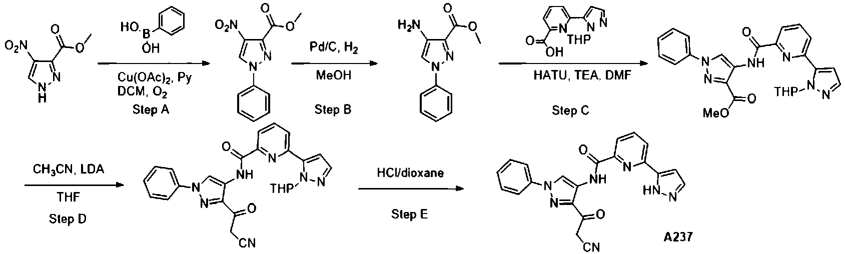

### Step A: Methyl 4-nitro-1-phenyl-1H-pyrazole-3-carboxylate

At rt, to a solution of methyl 4-nitro-1H-pyrazole-3-carboxylate (10 g, 58.4 mmol, 1.0 eq) and phenylboronic acid (14.3 g, 117 mmol, 2.0 eq) in DCM (150.0 mL) were added copper acetate (17.5 g, 87.7 mmol, 1.5 eq) and pyridine (14.2 mL, 175 mmol, 3.0 eq). The reaction solution was reacted under O₂ at rt for 12 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 8 g, yield 55%).

### Step B: Methyl 4-amino-1-phenyl-1H-pyrazole-3-carboxylate

Under N₂, to a solution of methyl 4-nitro-1-phenyl-1H-pyrazole-3-carboxylate (2.5 g, 10.1 mmol, 1.0 eq) in MeOH (50.0 mL) was added palladium on carbon (0.22 g, 10% purity). The reaction solution was purged with H₂ (×3), and reacted at rt under H₂ for 12 h. Upon completion, the reaction solution was filtered, and the filter cake was washed with MeOH (×3). The filtrate was concentrated under reduced pressure to afford the title compound (yellow solid, 2.1 g, yield 95%).

### Step C: Methyl 1-phenyl-4-(6-(1-(tetrahydro-2H-pyran-2-yl)-1H-pyrazol-5-yl)picolinamido)-1H-pyrazole-3-carboxylate

At rt, to a solution of 6-[2-(3,4,5,6-tetrahydro-2H-pyran-2-yl)pyrazol-3-yl]pyridine-2-carboxylic acid (1 g, 3.66 mmol, 1.0 eq) and HATU (1.67 g, 4.39 mmol, 1.2 eq) in DMF (3.0 mL) were added methyl 4-amino-1-phenyl-1H-pyrazole-3-carboxylate (0.87 g, 4.03 mmol, 1.1 eq) and TEA (1.5 mL, 11.0 mmol, 3.0 eq). The reaction solution was reacted at rt for 12 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 1 g, yield 58%).

### Step D: N-(3-(2-cyanoacetyl)-1-phenyl-1H-pyrazol-4-yl)-6-(1-(tetrahydro-2H-pyrazol-2-yl)-1H-pyrazin-5-yl)picolinamide

At -78°C, to a solution of acetonitrile (260 mg, 6.35 mmol, 10.0 eq) in THF (3.0 mL) was added lithium diisopropylamide (2.54 mL, 6.35 mmol, 10.0 eq, 1 M in tetrahydrofuran). The mixture was reacted at -78°C for 1 h. A solution of methyl 1-phenyl-4-(6-(1-(tetrahydro-2H-pyran-2-yl)-1H-pyrazol-5-yl)picolinamido)-1H-pyrazole-3-carboxylate (300 mg, 0.64 mmol, 1.0 eq) was then added. The reaction solution was reacted at -78°C for 0.5 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by reverse-phase column chromatography to afford the title compound (yellow solid, 71 mg, yield 23%).

### Step E: N-(3-(2-cyanoacetyl)-1-phenyl-1H-pyrazol-4-yl)-6-(1H-pyrazol-5-yl)picolinamide

At rt, to a solution of *N*-(3-(2-cyanoacetyl)-1-phenyl-1H-pyrazol-4-yl)-6-(1-(tetrahydro-2H-pyrazol-2-yl)-1H-pyrazin-5-yl)picolinamide (50 mg, 0.10 mmol, 1.0 eq) in 1,4-dioxane (1.0 mL) was added 4 M HCl/dioxane (1.0 mL). The reaction solution was reacted at rt for 0.5 h. Upon completion, the reaction solution was concentrated under reduced pressure. The residue was purified by HPLC to afford the title compound (white solid, 15.0 mg, yield 36%). **¹H NMR(400** MHz, DMSO-*d₆*) δ 13.27 (s, 1H), 11.67 (s, 1H), 9.13 (s, 1H), 8.27 (d, *J =* 7.2 Hz, 1H), 8.18-7.99 (m, 5H), 7.60 (t, *J =* 7.7 Hz, 2H), 7.47 (t, *J =* 7.2 Hz, 1H), 7.33 (s, 1H), 4.77 (s, 2H). LCMS (ESI): m/z 398 [M+H]⁺.

### Preparation Example 113. Synthesis of Compound A238

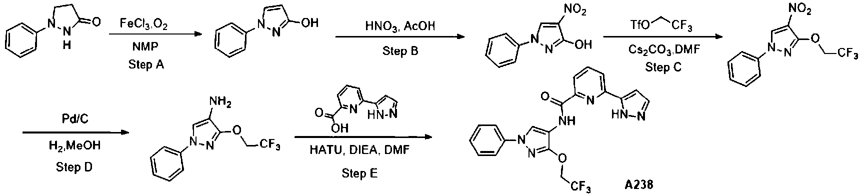

### Step A: 1-Phenyl-1H-pyrazol-3-ol

At rt, to a solution of 1-phenylpyrazolidin-3-one (10 g, 61.7 mmol, 1.0 eq) in N-methylpyrrolidone (50.0 mL) was added iron(III) chloride (0.2 g, 1.23 mmol, 0.02 eq). The reaction solution was reacted under O₂ at 100°C for 16 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 9 g, yield 91%).

### Step B: 4-nitro-1-phenyl-1H-pyrazol-3-ol

At rt, to a solution of 1-phenyl-1H-pyrazol-3-ol (9 g, 56.2 mmol, 1.0 eq) in AcOH (100.0 mL) was slowly added nitric acid (20 mL). The reaction solution was reacted at rt for 16 h. Upon completion, the reaction solution was poured into water and filtered. The filter cake was dried under reduced pressure to afford the title compound (white solid, 9.5 g, yield 82%).

### Step C: 4-nitro-1-phenyl-3-(2,2,2-trifluoroethoxy)-1H-pyrazole

At rt, to a solution of 4-nitro-1-phenyl-1H-pyrazol-3-ol (500 mg, 2.44 mmol, 1.0 eq) and 2,2,2-trifluoroethyl triflate (679 mg, 2.92 mmol, 1.2 eq) in DMF (10.0 mL) was added cesium carbonate (1.59 g, 4.87 mmol, 2.0 eq). The mixture was reacted at 20°C for 2 h. Upon completion, the mixture was purified by reverse-phase column chromatography to afford the title compound (white solid, 0.5 g, yield 71%).

### Step D: 1-phenyl-3-(2,2,2-trifluoroethoxy)-1H-pyrazol-4-amine

Under N₂, to a solution of 4-nitro-1-phenyl-3-(2,2,2-trifluoroethoxy)-1H-pyrazole (50 mg, 0.17 mmol, 1.0 eq) in MeOH (5.0 mL) was added palladium on carbon (5 mg, 10% purity). The reaction solution was purged with H₂ (×3), and reacted at rt under H₂ for 0.5 h. Upon completion, the reaction solution was filtered, and the filter cake was washed with MeOH (×3). The filtrate was concentrated under reduced pressure to afford the title compound (colorless oil, 35 mg, yield 78%).

### Step E: N-(1-Phenyl-3-(2,2,2-trifluoroethoxy)-1H-pyrazol-4-yl)-6-(1H-pyrazol-5-yl)picolinamide

At rt, to a solution of 6-(2H-pyrazol-3-yl)pyridine-2-carboxylic acid (26 mg, 0.14 mmol, 1.0 eq) and HATU (62 mg, 0.16 mmol, 1.1 eq) in DMF (1.0 mL) were added 1-phenyl-3-(2,2,2-trifluoroethoxy)-1H-pyrazol-4-amine (35 mg, 0.14 mmol, 1.0 eq) and DIEA (53 mg, 0.41 mmol, 2.9 eq). The reaction solution was reacted at rt for 0.5 h. Upon completion, the reaction solution was purified by HPLC to afford the title compound (white solid, 20.0 mg, yield 34%). **¹H NMR** (400 MHz, MeOD-*d₄*) δ 8.65-8.49 (m, 1H), 8.19-8.02 (m, 3H), 7.84-7.65 (m, 3H), 7.47 (t, *J =* 8.0 Hz, 2H), 7.26 (t, *J* = 7.4 Hz, 1H), 7.03 (s, 1H), 4.94 (q, *J =* 8.6 Hz, 2H). LCMS (ESI): m/z 429.2 [M+H]⁺.

### Preparation Example 114. Synthesis of Compound A239

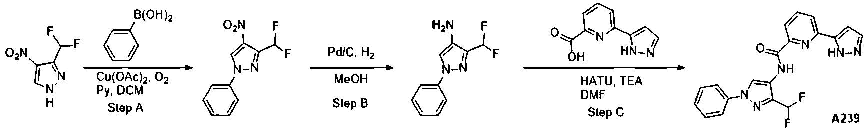

### Step A: 3-(Difluoromethyl)-4-nitro-1-phenyl-1H-pyrazole

At rt, to a solution of 3-(difluoromethyl)-4-nitro-1H-pyrazole (500 mg, 3.07 mmol, 1.0 eq) and phenylboronic acid (748 mg, 6.13 mmol, 2.0 eq) in DCM (30.0 mL) were added copper acetate (918 mg, 4.60 mmol, 1.5 eq) and pyridine (0.25 mL, 3.07 mmol, 1.0 eq). The reaction solution was reacted under O₂ at rt for 0.5 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 0.7 g, yield 95%).

### Step B: 3-(Difluoromethyl)-1-phenyl-1H-pyrazol-4-amine

At rt, to a solution of 3-(difluoromethyl)-4-nitro-1-phenyl-1H-pyrazole (300 mg, 1.25 mmol, 1.0 eq) in MeOH (15.0 mL) was added palladium on carbon (60 mg, 10% purity). The reaction solution was reacted at rt for 12 h under H₂. Upon completion, the reaction solution was filtered, and the filter cake was washed with MeOH (×3). The filtrate was concentrated under reduced pressure to afford the title compound (colorless oil, 250 mg, crude).

### Step C: N-(3-(Difluoromethyl)-1-phenyl-1H-pyrazol-4-yl)-6-(1H-pyrazol-5-yl)picolinamide

At rt, to a solution of 6-(1H-pyrazol-5-yl)picolinic acid (45 mg, 0.24 mmol, 1.0 eq) and HATU (100 mg, 0.26 mmol, 1.1 eq) in DMF (2.0 mL) were added 3-(difluoromethyl)-1-phenyl-1H-pyrazol-4-amine (50 mg, 0.24 mmol, 1.0 eq) and TEA (0.2 mL, 1.19 mmol, 5.0 eq). The mixture was reacted at rt for 0.5 h. Upon completion, the mixture was purified by HPLC to afford the title compound (white solid, 46 mg, yield 51%). **¹H NMR** (400 MHz, MeOD-*d₄*) δ 8.88 (s, 1H), 8.22-8.04 (m, 3H), 7.85-7.79 (m, 3H), 7.54 (m, 2H), 7.39 (t, *J =* 7.4 Hz, 1H), 7.15 (t, *J =* 54.4 Hz, 1H), 7.09-7.03 (m, 1H). LCMS (ESI): m/z 381 [M+H]⁺.

### Preparation Example 115. Synthesis of Compound A240

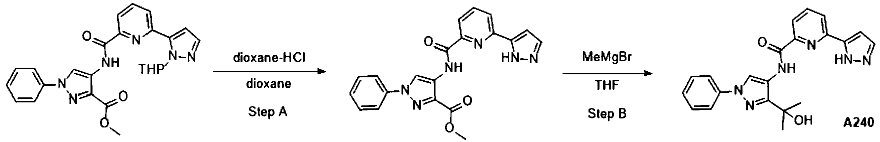

### Step A: Methyl 4-(6-(1H-pyrazol-5-yl)picolinamido)-1-phenyl-1H-pyrazole-3-carboxylate

At rt, to a solution of methyl 1-phenyl-4-(6-(1-(tetrahydro-2H-pyran-2-yl)-1H-pyrazol-5-yl)picolinamido)-1H-pyrazole-3-carboxylate (100 mg, 0.21 mmol, 1.0 eq) in dioxane (5.0 mL) was added HCl/dioxane (3.0 mL, 4 M). The mixture was reacted at rt for 1 h. Upon completion, the reaction solution was concentrated under reduced pressure to afford the title compound (white solid, 90 mg, crude).

### Step B: N-(3-(2-Hydroxypropan-2-yl)-1-phenyl-1H-pyrazol-4-yl)-6-(1H-pyrazol-5-yl)picolinamide

Under N₂, to a solution of methyl 4-(6-(1H-pyrazol-5-yl)picolinamido)-1-phenyl-1H-pyrazole-3-carboxylate (90 mg, 0.23 mmol, 1.0 eq) in THF (5.0 mL) was added methylmagnesium bromide (1.2 mL, 1.16 mmol, 5.0 eq, 1 M) dropwise. The reaction solution was reacted at rt for 16 h. Upon completion, the reaction solution was poured into aqueous NH₄Cl and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (white solid, 46.9 mg, yield 52%). **¹H NMR** (400 MHz, DMSO-*d₆*) δ 11.51 (s, 1H), 8.89 (s, 1H), 8.23-8.17 (m, 1H), 8.10 (t, *J* = 7.7 Hz, 1H), 8.07-8.02 (m, 1H), 7.91 (s, 1H), 7.81 (d, *J* = 7.7 Hz, 2H), 7.49 (t, *J =* 8.0 Hz, 2H), 7.28 (t, *J* = 7.4 Hz, 1H), 7.15 (d, *J =* 2.2 Hz, 1H), 1.64 (s, 6H). LCMS (ESI): m/z 371.2 [M-17]⁺.

### Preparation Example 116. Synthesis of Compound A241

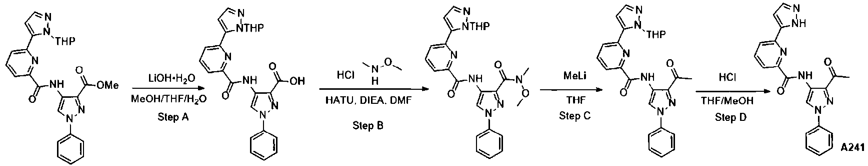

### Step A: 1-Phenyl-4-(6-(1-(tetrahydro-2H-pyran-2-yl)-1H-pyrazol-5-yl)picolinamido)-1H-pyrazole-3-carboxylic acid

At rt, to a mixed solution of methyl 1-phenyl-4-(6-(1-(tetrahydro-2H-pyran-2-yl)-1H-pyrazol-5-yl)picolinamido)-1H-pyrazole-3-carboxylate (1.13 g, 2.39 mmol, 1.0 eq) in MeOH (30.0 mL), THF (10 mL), and water (10 mL) was added lithium hydroxide monohydrate (0.40 g, 9.57 mmol, 4.0 eq). The reaction solution was reacted at 60°C for 2 h. Upon completion, the reaction solution was concentrated under reduced pressure. The residue was poured into water and adjusted to neutral pH with dilute HCl, forming a suspension. The suspension was filtered, and the filter cake was washed with water (×3) and dried under reduced pressure to afford the title compound (yellow solid, 897 mg, crude).

### Step B: N-(3-(Methoxy(methyl)carbamoyl)-1-phenyl-1H-pyrazol-4-yl)-6-(1-(tetrahydro-2H-pyrazol-2-yl)-1H-pyrazin-5-yl)picolinamide

At rt, to a solution of 1-phenyl-4-(6-(1-(tetrahydro-2H-pyran-2-yl)-1H-pyrazol-5-yl)picolinamido)-1H-pyrazole-3-carboxylic acid (0.86 g, 1.88 mmol, 1.0 eq) and HATU (1.07 g, 2.81 mmol, 1.5 eq) in DMF (3.0 mL) were added N,O-dimethylhydroxylamine hydrochloride (0.22 g, 2.25 mmol, 1.2 eq) and DIEA (0.97 g, 7.50 mmol, 4.0 eq). The reaction solution was reacted at rt for 18 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure to afford the title compound (yellow solid, 1.02 g, crude).

### Step C: N-(3-Acetyl-1-phenyl-1H-pyrazol-4-yl)-6-(1-(tetrahydro-2H-pyrazol-2-yl)-1H-pyrazin-5-yl)picolinamide

At 0°C, to a solution of *N*-(3-(methoxy(methyl)carbamoyl)-1-phenyl-1H-pyrazol-4-yl)-6-(1-(tetrahydro-2H-pyrazol-2-yl)-1H-pyrazin-5-yl)picolinamide (0.2 g, 0.40 mmol, 1.0 eq) in THF (10.0 mL) was added methyllithium (1.6 mL, 1.60 mmol, 4.0 eq). The reaction solution was reacted at rt for 3 h. Upon completion, the reaction solution was poured into saturated aqueous NH₄Cl and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 132 mg, yield 72.5%).

### Step D: N-(3-Acetyl-1-phenyl-1H-pyrazol-4-yl)-6-(1H-pyrazol-5-yl)picolinamide

At rt, to a solution of *N*-(3-acetyl-1-phenyl-1H-pyrazol-4-yl)-6-(1-(tetrahydro-2H-pyrazol-2-yl)-1H-pyrazin-5-yl)picolinamide (50 mg, 0.10 mmol, 1.0 eq) in THF (5.0 mL) and MeOH (2.5 mL) was added HCl/dioxane (1.0 mL, 4 M). The reaction solution was reacted at rt for 0.5 h. Upon completion, the reaction solution was concentrated under reduced pressure. The residue was poured into water, adjusted to pH=8 with NaHCO₃, and extracted with DCM (×3). The combined organic phases were washed with saturated aqueous NaCl, dried over anhydrous Na₂SO₄, filtered, and concentrated under reduced pressure. The residue was purified by HPLC to afford the title compound (pale yellow solid, 46.4 mg, yield 44%). **¹H NMR** (400 MHz, DMSO-*d₆*) δ 13.24 (s, 1H), 11.94 (s, 1H), 9.07 (s, 1H), 8.25 (d, *J =* 8.0 Hz, 1H), 8.13 (t, *J =* 7.6 Hz, 1H), 8.07 (dd, *J =* 7.6, 1.2 Hz, 1H), 8.01 (d, *J =* 7.6 Hz, 3H), 7.59 (t, *J =* 7.6 Hz, 2H), 7.45 (t, *J =* 7.6 Hz, 1H), 7.38 (s, 1H), 2.71 (s, 3H). LCMS (ESI): m/z 373.2 [M+H]⁺.

### Preparation Example 117. Synthesis of Compound A242

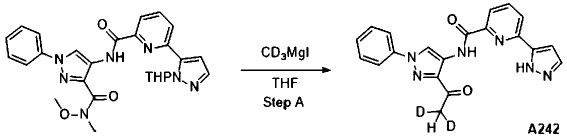

### Step A: N-(3-(Acetyl-2,2-d₂)-1-phenyl-1H-pyrazol-4-yl)-6-(1H-pyrazol-5-yl)picolinamide

At 0°C, to a solution of *N*-(3-(methoxy(methyl)carbamoyl)-1-phenyl-1H-pyrazol-4-yl)-6-(1-(tetrahydro-2H-pyrazol-2-yl)-1H-pyrazin-5-yl)picolinamide (300 mg, 0.60 mmol, 1.0 eq) in THF (30.0 mL) was added CD₃MgI (2.4 mL, 2.40 mmol, 4.0 eq, 1 M in tetrahydrofuran). The reaction solution was reacted at rt for 3 h. Upon completion, the reaction solution was poured into saturated NaHCO₃ solution and extracted with EtOAc (×3). The combined organic phases were washed with saturated aqueous NaCl, dried over anhydrous Na₂SO₄, filtered, and concentrated under reduced pressure. The residue was purified by two rounds of HPLC to afford the title compound (pale yellow solid, 37.4 mg, yield 17%). **¹H** NMR (400 MHz, DMSO-*d₆*) δ 13.25 (s, 1H), 11.94 (s, 1H), 9.08 (s, 1H), 8.25 (d, *J =* 7.6 Hz, 1H), 8.14 (t, *J =* 7.6 Hz, 1H), 8.08 (dd, *J* = 7.6, 1.2 Hz, 1H), 8.01 (d, *J =* 7.6 Hz, 3H), 7.60 (t, *J =* 7.6 Hz, 2H), 7.46 (t, *J =* 7.6 Hz, 1H), 7.38 (s, 1H), 2.74-2.67 (m, 1H). LCMS (ESI): m/z 375.2 [M+H]⁺.

### Preparation Example 118. Synthesis of Compound A243

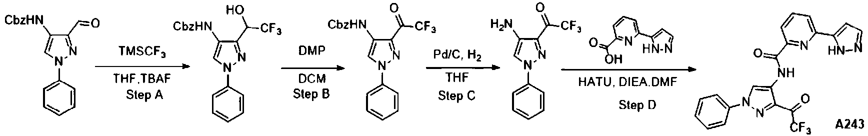

### Step A: Benzyl (1-phenyl-3-(2,2,2-trifluoro-1-hydroxyethyl)-1H-pyrazol-4-yl)carbamate

At 0°C, trimethyl(trifluoromethyl)silane (256 mg, 1.8 mmol, 2.0 eq) and TBAF (1 N in THF, 0.09 mL, 0.09 mmol, 0.1 eq) were added to a solution of benzyl (3-formyl-1-phenyl-1H-pyrazol-4-yl)carbamate (300 mg, 0.9 mmol, 1.0 eq) in anhydrous THF (20 mL). The mixture was stirred at rt overnight under N₂. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 130 mg, yield 37%).

### Step B: Benzyl (1-phenyl-3-(2,2,2-trifluoroacetyl)-1H-pyrazol-4-yl)carbamate

At 0°C, DMP (255 mg, 0.6 mmol, 2.0 eq) was slowly added to a solution of benzyl (1-phenyl-3-(2,2,2-trifluoro-1-hydroxyethyl)-1H-pyrazol-4-yl)carbamate (130 mg, 0.3 mmol, 1.0 eq) in anhydrous DCM (10 mL). The reaction solution was stirred at rt under N₂ for 2 h. Upon completion, the reaction solution was diluted in EtOAc, washed sequentially with saturated sodium bisulfite and saturated aqueous NaCl, dried over anhydrous Na₂SO₄, and concentrated under reduced pressure. The residue was purified by column chromatography to afford the title compound (yellow solid, 105 mg, yield 90%).

### Step C: 1-(4-Amino-1-phenyl-1H-pyrazol-3-yl)-2,2,2-trifluoroethan-1-one

At rt, Pd/C (15 mg, 10% on carbon, ~55% water wet) was added to a solution of benzyl (1-phenyl-3-(2,2,2-trifluoroacetyl)-1H-pyrazol-4-yl)carbamate (105 mg, 0.3 mmol, 1.0 eq) in THF (20 mL). The reaction solution was stirred overnight at rt under H₂. Upon completion, the reaction solution was filtered, and the filter cake was washed with MeOH (20 mL). The combined filtrates were concentrated to afford the title compound (52 mg, quantitative), which was used directly in the next step without further purification.

### Step D: N-(1-Phenyl-3-(2,2,2-trifluoroacetyl)-1H-pyrazol-4-yl)-6-(1H-pyrazol-5-yl)picolinamide

To a mixture of 6-(1H-pyrazol-5-yl)picolinic acid (57 mg, 0.3 mmol, 1.5 eq) and HATU (114 mg, 0.3 mmol, 1.5 eq) in anhydrous DMF (100 mL) were added DIEA (77 mg, 0.6 mmol, 3.0 eq) and 1-(4-amino-1-phenyl-1H-pyrazol-3-yl)-2,2,2-trifluoroethan-1-one (52 mg, 0.2 mmol, 1.0 eq). The reaction solution was stirred overnight under N₂. Upon completion, the reaction solution was diluted in water (10 mL) and extracted with EtOAc (15 mL×3). The combined organic layers were washed with saturated aqueous NaCl, dried over anhydrous Na₂SO₄, and concentrated under reduced pressure. The residue was purified by preparative chromatography to afford the title compound (white solid, 10.1 mg, yield 12%). LCMS (ESI): m/z 427 [M+H]⁺. **¹H NMR** (400 MHz, MeOD-*d₄*) δ 9.00 (s, 1H), 8.22 (d, *J =* 7.3 Hz, 1H), 8.13 (d, *J =* 7.5 Hz, 1H), 8.05 (t, *J* = 7.6 Hz, 1H), 7.84 (d, *J* = 7.7 Hz, 2H), 7.79 (s, 1H), 7.53 (t, *J =* 8.0 Hz, 2H), 7.37 (t, *J =* 7.4 Hz, 1H), 7.20 (s, 1H).

### Preparation Example 119. Synthesis of Compound A244

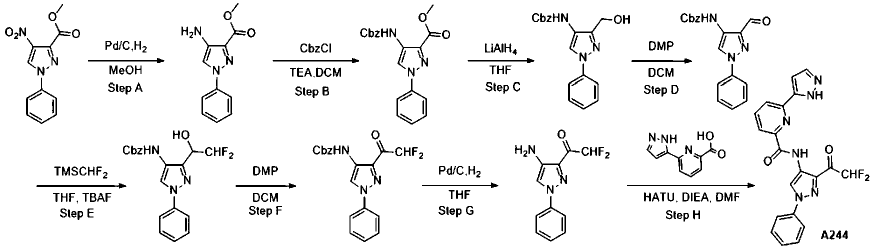

### Step A: Methyl 4-amino-1-phenyl-1H-pyrazole-3-carboxylate

Pd/C (200 mg, 10% on carbon, ~55% water wet) was added to a solution of methyl 4-nitro-1-phenyl-1H-pyrazole-3-carboxylate (2.0 g, 8.1 mmol, 1.0 eq) in MeOH (50 mL). The reaction solution was stirred overnight at rt under H₂. Upon completion, the reaction solution was filtered, and the filter cake was washed with MeOH (20 mL). The combined filtrates were concentrated to afford the title compound (1.6 g, quantitative, crude), which was used directly in the next step without further purification.

### Step B: Methyl 4-(((benzyloxy)carbonyl)amino)-1-phenyl-1H-pyrazole-3-carboxylate

At 0°C, CbzCl (2.5 g, 14.6 mmol, 2.0 eq) was added to a solution of methyl 4-amino-1-phenyl-1H-pyrazole-3-carboxylate (1.6 g, 7.3 mmol, 1.0 eq) and TEA (2.2 g, 21.9 mmol, 3.0 eq) in DCM (30 mL). The solution was stirred overnight at rt under N₂. The reaction solution was diluted in water (20 ml) and extracted with EtOAc (20 mL×3). The combined organic layers were washed with aqueous NaCl (20 mL×3), dried over anhydrous Na₂SO₄, and concentrated under reduced pressure. The residue was purified by flash column chromatography on silica gel to afford the title compound (white solid, 1.5 g, yield 58%).

### Step C: Benzyl (3-(hydroxymethyl)-1-phenyl-1H-pyrazol-4-yl)carbamate

At 0°C, lithium aluminium hydride (1 N in THF, 6.5 mL, 6.5 mmol, 1.5 eq) was added dropwise to a solution of methyl 4-(((benzyloxy)carbonyl)amino)-1-phenyl-1H-pyrazole-3-carboxylate (1.5 g, 4.3 mmol, 1.0 eq) in anhydrous THF (30 mL). The reaction solution was stirred at 0°C for 30 min under N₂. The mixture was quenched with water (0.2 mL), and then 15% NaOH (0.2 mL) and water (0.6 mL) were added. The suspension was diluted in EtOAc (30 mL) and filtered. The filtrate was dried over anhydrous Na₂SO₄, filtered, and concentrated under reduced pressure to dryness. The residue was purified by flash column chromatography on silica gel to afford the title compound (pale yellow solid, 950 mg, yield 68%).

### Step D: Benzyl (3-formyl-1-phenyl-1H-pyrazol-4-yl)carbamate

At 0°C, Dess-Martin periodinane (2.5 g, 5.8 mmol, 2.0 eq) was added slowly to a solution of benzyl (3-(hydroxymethyl)-1-phenyl-1H-pyrazol-4-yl)carbamate (950 mg, 2.9 mmol, 1.0 eq) in anhydrous DCM (30 mL). The reaction solution was stirred at rt for 2 h under N₂. The reaction mixture was quenched with saturated aqueous NaHSO3 solution (10 mL) and extracted with EtOAc (20 mL×3). The combined organic layers were washed with NaHCO₃ (15 mL×2), dried over anhydrous Na₂SO₄, and concentrated under reduced pressure. The residue was purified by flash column chromatography on silica gel to afford the title compound (yellow solid, 610 mg, yield 66%).

### Step E: Benzyl (3-(2,2-difluoro-1-hydroxyethyl)-1-phenyl-1H-pyrazol-4-yl)carbamate

At 0°C, trimethylsilyldifluoromethane (223 mg, 1.8 mmol, 2.0 eq) and tetrabutylammonium fluoride (1 N in THF, 0.09 mL, 0.09 mmol, 0.1 eq) were added to a solution of benzyl (3-formyl-1-phenyl-1H-pyrazol-4-yl)carbamate (310 mg, 0.9 mmol, 1.0 eq) in anhydrous THF (20 mL). The reaction solution was stirred overnight at rt under N₂. The reaction mixture was diluted in water (15 mL) and extracted with EtOAc (15 mL×3). The combined organic layers were washed with aqueous NaCl (15 mL), dried over anhydrous Na₂SO₄, and concentrated under reduced pressure. The residue was purified by flash column chromatography on silica gel to afford the title compound (yellow solid, 180 mg, yield 53%).

### Step F: Benzyl (3-(2,2-difluoroacetyl)-1-phenyl-1H-pyrazol-4-yl)carbamate

At 0°C, Dess-Martin periodinane (424 mg, 1.0 mmol, 2.0 eq) was added slowly to a solution of benzyl (3-(2,2-difluoro-1-hydroxyethyl)-1-phenyl-1H-pyrazol-4-yl)carbamate (180 mg, 0.5 mmol, 1.0 eq) in anhydrous DCM (20 mL). The reaction solution was stirred at rt under N₂ for 2 h. The reaction solution was quenched with saturated aqueous NaHSO₃ solution and extracted with EtOAc (20 mL×3). The combined organic layers were washed with NaHCO₃ (15 mL×2), dried over anhydrous Na₂SO₄, and concentrated under reduced pressure. The residue was purified by flash column chromatography on silica gel to afford the title compound (yellow solid, 140 mg, yield 75%).

### Step G: 1-(4-amino-1-phenyl-1H-pyrazol-3-yl)-2,2-difluoroethan-1-one

Pd/C (20 mg, 10% on carbon, ~55% water wet) was added to a solution of benzyl (3-(2,2-difluoroacetyl)-1-phenyl-1H-pyrazol-4-yl)carbamate (140 mg, 0.4 mmol, 1.0 eq) in THF (20 mL). The reaction solution was stirred overnight at rt under H₂. Upon completion, the reaction solution was filtered through a funnel, and the filter cake was washed with MeOH (20 mL). The combined filtrates were concentrated to dryness to afford the title compound (65 mg, quantitative) as a white solid, which was used directly in the next step without further purification.

### Step H: N-(3-(2,2-difluoroacetyl)-1-phenyl-1H-pyrazol-4-yl)-6-(1H-pyrazol-5-yl)picolinamide

To a solution of 6-(1H-pyrazol-5-yl)picolinic acid (76 mg, 0.4 mmol, 1.2 eq) and HATU (152 mg, 0.4 mmol, 1.2 eq) in anhydrous DMF (100 mL) were added DIEA (116 mg, 0.9 mmol, 3.0 eq) and 1-(4-amino-1-phenyl-1H-pyrazol-3-yl)-2,2-difluoroethan-1-one (65 mg, 0.3 mmol, 1.0 eq). The reaction solution was stirred overnight at rt under N₂. The reaction mixture was diluted in water (15 mL) and extracted with EtOAc (15 mL×3). The combined organic layers were washed with aqueous NaCl, dried over anhydrous Na₂SO₄, and concentrated under reduced pressure. The residue was purified by preparative chromatography to afford the title compound (white solid, 18.1 mg, yield 15%). **¹H** NMR (400 MHz, DMSO-*d₆*) δ 13.27 (s, 1H), 11.69 (s, 1H), 9.18 (s, 1H), 8.28 (d, *J* = 7.5 Hz, 1H), 8.19-8.09 (m, 2H), 8.08-7.99 (m, 3H), 7.62 (t, *J =* 7.9 Hz, 2H), 7.50 (t, *J* = 7.4 Hz, 1H), 7.32 (d, *J* = 9.4 Hz, 1H), 7.10 (t, *J =* 53.0 Hz, 1H). LCMS (ESI): m/z 409 [M+H]⁺.

### Preparation Example 120. Synthesis of Compound A245

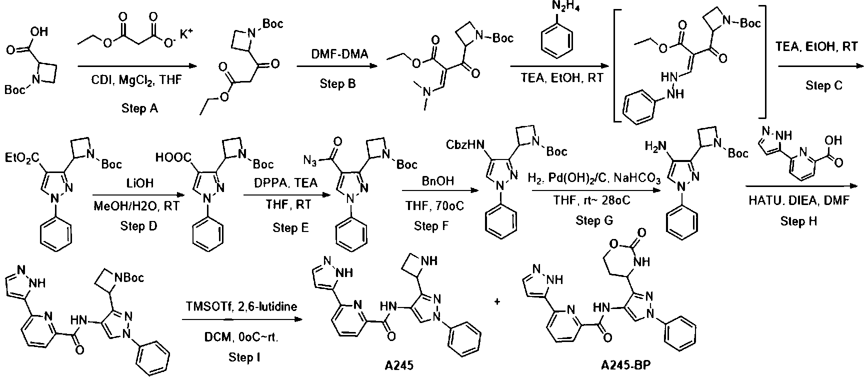

### Step A: Tert-butyl 2-(3-ethoxy-3-oxopropanoyl)azetidine-1-carboxylate

1-{[(2-methylpropan-2-yl)oxy]carbonyl}azetidine-2-carboxylic acid (2 g, 9.939 mmol, 1.0 eq), bis(imidazol-1-yl)methanone (1.93 g, 11.927 mmol, 1.2 eq) and THF (20 mL) were added to a pre-dried flask. The mixture was purged with N₂ (×3) and stirred at 25°C for 2 h. Magnesium chloride (1.18 g, 12.424 mmol, 1.25 eq) and Potassium monoethyl malonate (2.03 g, 11.927 mmol, 1.2 eq) were added, and the mixture was stirred at 25°C for another 4 h. Upon completion, the reaction mixture was diluted in water (30 mL) and extracted with EtOAc (50 mL×3). The combined organic phases were washed with saturated aqueous NaCl (15 mL), dried over anhydrous Na₂SO₄, and filtered, and the filtrate was concentrated under reduced pressure. The residue was purified by flash column chromatography to afford the title compound (colorless oil, 1.1 g, yield 39%).

### Step B: Tert-butyl (Z)-2-(3-(dimethylamino)-2-(ethoxycarbonyl)acryloyl)azetidine-1-carboxylate

To a solution of tert-butyl 2-(3-ethoxy-3-oxopropanoyl)azetidine-1-carboxylate (1.06 g, 3.712 mmol, 1.0 eq) in dioxane (10 mL) was added (dimethylamino)dimethoxymethane (0.614 mL, 4.825 mmol, 1.3 eq). The mixture was stirred at 25°C for 3 h. The resulting mixture was concentrated under reduced pressure, and the residue was purified by flash column chromatography to afford the title compound (pale yellow oil, 1.2 g, yield 97%).

### Step C: Ethyl 3-(1-(tert-butoxycarbonyl)azetidin-2-yl)-1-phenyl-1H-pyrazole-4-carboxylate

To a solution of tert-butyl (Z)-2-(3-(dimethylamino)-2-(ethoxycarbonyl)acryloyl)azetidine-1-carboxylate (1.1 g, 3.303 mmol, 1.0 eq) and TEA (1.377 mL, 9.908 mmol, 3.0 eq) in dioxane (20 mL) was added phenylhydrazine (0.357 mL, 3.303 mmol, 3.0 eq). The mixture was stirred at rt overnight. Upon completion, the resulting mixture was concentrated under reduced pressure, and the residue was purified by reverse-phase preparative chromatography to afford the title compound (pale yellow solid, 1.2 g, yield 88%).

### Step D: 3-(1-(tert-Butoxycarbonyl)azetidin-2-yl)-1-phenyl-1H-pyrazole-4-carboxylic acid

To a solution of ethyl 3-(1-(tert-butoxycarbonyl)azetidin-2-yl)-1-phenyl-1H-pyrazole-4-carboxylate (1.2 g, 3.166 mmol, 1.0 eq) in MeOH (15 mL) were added water (5 mL) and LiOH (0.20 g, 4.749 mmol, 1.5 eq). The mixture was stirred at rt overnight. Upon completion, the reaction mixture was concentrated under reduced pressure. The residue was dissolved in water (20 mL) and extracted with DCM (40 mL). The aqueous layer was adjusted to pH 6 with concentrated HCl and then extracted with EtOAc (50 mL). The combined organic phases were washed with water (30 mL×3), dried over anhydrous Na₂SO₄, and filtered, and the filtrate was concentrated under reduced pressure to afford the title compound (yellow oil, 1.05 g, yield 92%).

### Step E: Tert-butyl 2-(4-(azidocarbonyl)-1-phenyl-1H-pyrazol-3-yl)azetidine-1-carboxylate

To a solution of 3-(1-(tert-butoxycarbonyl)azetidin-2-yl)-1-phenyl-1H-pyrazole-4-carboxylic acid (1.1 g, 3.043 mmol, 1.0 eq) in THF (15 mL) were added TEA (1.1 mL, 7.608 mmol, 2.5 eq) and DPPA (1.09 g, 3.956 mmol, 1.3 eq). The mixture was stirred at rt for 1 h, then heated to 70°C and stirred overnight. Upon completion, the reaction mixture was diluted in water (30 mL) and extracted with EtOAc (50 mL×3). The combined organic phases were washed with saturated aqueous NaCl (15 mL), dried over anhydrous Na₂SO₄, and filtered, and the filtrate was concentrated under reduced pressure. The residue was purified by flash column chromatography to afford the title compound (yellow oil, 1.1 g, yield 93%).

### Step F: Tert-butyl 2-(4-(((benzyloxy)carbonyl)amino)-1-phenyl-1H-pyrazol-3-yl)azetidine-1-carboxylate

To a solution of tert-butyl 2-(4-(azidocarbonyl)-1-phenyl-1H-pyrazol-3-yl)azetidine-1-carboxylate (1 g, 2.714 mmol, 1.0 eq) in THF (20 mL) was added benzyl alcohol (0.842 mL, 8.143 mmol, 3.0 eq). The mixture was stirred at 70°C for 3 h. Upon completion, the reaction mixture was diluted in water (20 mL) and extracted with EtOAc (40 mL×3). The combined organic layers were washed with saturated aqueous NaCl (15 mL), dried over anhydrous Na₂SO₄, and filtered, and the filtrate was concentrated under reduced pressure. The residue was purified by flash column chromatography to afford the title compound (colorless oil, 1.1 g, yield 86%).

### Step G: Tert-butyl 2-(4-amino-1-phenyl-1H-pyrazol-3-yl)azetidine-1-carboxylate

To a solution of tert-butyl 2-(4-(((benzyloxy)carbonyl)amino)-1-phenyl-1H-pyrazol-3-yl)azetidine-1-carboxylate (400 mg, 0.847 mmol, 1.0 eq) in THF (5 mL) were added NaHCO₃ (142 mg, 1.694 mmol, 2.0 eq) and Pd(OH)₂/C (12 mg). The mixture was stirred at 28°C under H₂ (1 atm) for 12 h. The reaction mixture was filtered through Celite and concentrated under reduced pressure to afford the title compound (yellow solid, 250 mg, yield 94%), which was used directly in the next step without further purification.

### Step H: Tert-butyl 2-(4-(6-(1H-pyrazol-5-yl)picolinamido)-1-phenyl-1H-pyrazol-3-yl)azetidine-1-carboxylate

To a solution of tert-butyl 2-(4-amino-1-phenyl-1H-pyrazol-3-yl)azetidine-1-carboxylate (340 mg, 1.081 mmol, 1.0 eq), 6-(1H-pyrazol-5-yl)picolinic acid (225 mg, 1.1891 mmol, 1.1 eq), and DIEA (280 mg, 2.163 mmol, 2.0 eq) in DMF (6 mL) was added HATU (617 mg, 1.622 mmol, 1.5 eq). The mixture was stirred at rt for 2 h. The reaction mixture was diluted in water (10 mL) and extracted with EtOAc (40 mL×3). The combined organic layers were washed with saturated aqueous NaCl (20 mL), dried over anhydrous Na₂SO₄, and filtered, and the filtrate was concentrated under reduced pressure. The residue was purified by pre-TLC (EA/EtOH = 20:1) to afford the title compound (white solid, 60 mg, yield 11%).

### Step I: N-(3-(Azetidin-2-yl)-1-phenyl-1H-pyrazol-4-yl)-6-(1H-pyrazol-5-yl)picolinamide and N-(3-(2-oxo-1,3-oxazinan-4-yl)-1-phenyl-1H-pyrazol-4-yl)-6-(1H-pyrazol-5-yl)picolinamide

To a solution of tert-butyl 2-(4-(6-(1H-pyrazol-5-yl)picolinamido)-1-phenyl-1H-pyrazol-3-yl)azetidine-1-carboxylate (60 mg, 0.124 mmol, 1.0 eq) and 2,6-dimethylpyridine (0.029 mL, 0.248 mmol, 2.0 eq) in DCM (1 mL) was added trimethylsilyl trifluoromethanesulfonate (83 mg, 0.372 mmol, 3.0 eq). The mixture was stirred at rt for 1 h. Upon completion, the mixture was concentrated under reduced pressure, and the residue was purified by reverse-phase preparative chromatography to afford N-(3-(azetidin-2-yl)-1-phenyl-1H-pyrazol-4-yl)-6-(1H-pyrazol-5-yl)picolinamide (white solid, 4.2 mg, yield 9%) and the byproduct N-(3-(2-oxo-1,3-oxazinan-4-yl)-1-phenyl-1H-pyrazol-4-yl)-6-(1H-pyrazol-5-yl)picolinamide (white solid, 8.6 mg, yield 16%). **¹H NMR** (400 MHz, DMSO-*d₆*) δ 13.18 (s, 1H), 12.70 (s, 1H), 8.40 (s, 1H), 8.33 (s, 1H), 8.18 (d, *J* = 7.3 Hz, 1H), 8.13-8.04 (m, 2H), 7.85 (s, 1H), 7.56 (t, *J* = 7.6 Hz, 2H), 7.47 (t, *J =* 7.4 Hz, 1H), 7.39 (d, *J* = 7.3 Hz, 2H), 6.96 (d, *J =* 2.2 Hz, 1H), 5.10 (t, *J =* 8.1 Hz, 1H), 3.83-3.61 (m, 2H), 2.60 (dd, *J =* 12.2, 5.4 Hz, 2H). LCMS (ESI): m/z = 386.3 [M+H]⁺.

### Preparation Example 121. Synthesis of Compound A246

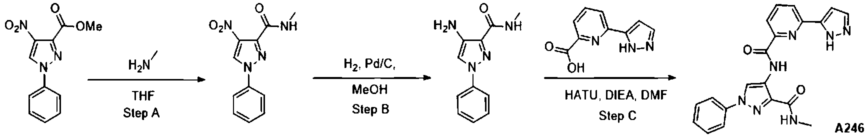

### Step A: N-Methyl-4-nitro-1-phenyl-1H-pyrazole-3-carboxamide

A mixture of methyl 4-nitro-1-phenylpyrazole-3-carboxylate (100 mg, 0.41 mmol, 1.0 eq) and methylamine in THF (1.0 M, 5.0 mL, 12.4 eq) was reacted at 70°C in a sealed tube for 16 h. Upon completion, the reaction solution was concentrated under reduced pressure to afford the title compound (yellow solid, 95 mg, crude).

### Step B: 4-Amino-N-methyl-1-phenyl-1H-pyrazole-3-carboxamide

At rt, palladium on carbon (10 mg, 10%) was added to a solution of *N*-methyl-4-nitro-1-phenyl-1H-pyrazole-3-carboxamide (95 mg, crude) in MeOH (10 mL). The reaction solution was purged with H₂ (×3) and then reacted at 25°C for 1.5 h. Upon completion, the reaction solution was filtered through Celite, and the filter cake was washed with MeOH (×3). The filtrate was concentrated under reduced pressure to afford the title compound (yellow solid, 80 mg, crude).

### Step C: N-(3-(Methylcarbamoyl)-1-phenyl-1H-pyrazol-4-yl)-6-(1H-pyrazol-5-yl)picolinamide

At 0°C, to a solution of 6-(1H-pyrazol-5-yl)picolinic acid (58 mg, 0.31 mmol, 1.1 eq) and 4-amino-N-methyl-1-phenyl-1H-pyrazole-3-carboxamide (60 mg, crude) in DMF (1.5 mL) were added DIEA (108 mg, 0.83 mmol, 3.0 eq) and HATU (127 mg, 0.33 mmol, 1.2 eq). The reaction solution was reacted at rt for 1 h. Upon completion, the reaction solution was purified by HPLC to afford the title compound (white solid, 61.7 mg, yield 57%). **¹H NMR** (400 MHz, DMSO-*d₆*) δ 13.20 (s, 1H), 12.09 (s, 1H), 9.03 (s, 1H), 8.60 (d, *J* = 4.8 Hz, 1H), 8.23-8.22 (m, 1H), 8.13-8.06 (m, 2H), 8.01-7.97 (m, 3H), 7.57 (t, *J =* 7.6 Hz, 2H), 7.42-7.34 (m, 2H), 2.92 (d, *J =* 4.4 Hz, 3H). LCMS (ESI): m/z 388 [M+H]⁺.

### Preparation Example 122. Synthesis of Compound A247

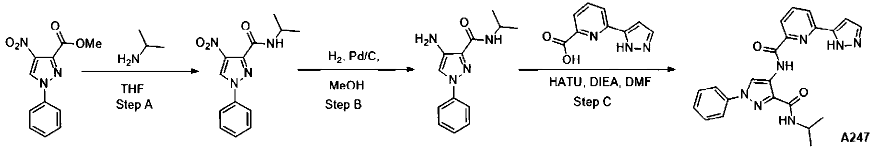

### Step A: N-Isopropyl-4-nitro-1-phenyl-1H-pyrazole-3-carboxamide

At rt, to a solution of methyl 4-nitro-1-phenyl-1H-pyrazole-3-carboxylate (100 mg, 0.40 mmol, 1.0 eq) in THF (2.0 mL) was added isopropylamine (238 mg, 4.04 mmol, 10.0 eq). The reaction solution was reacted at 70°C in a sealed tube for 16 h. Upon completion, the reaction solution was concentrated under reduced pressure to afford the title compound (yellow solid, 95 mg, crude).

### Step B: 4-Amino-N-isopropyl-1-phenyl-1H-pyrazole-3-carboxamide

At rt, to a solution of *N*-isopropyl-4-nitro-1-phenyl-1H-pyrazole-3-carboxamide (90 mg, crude) in MeOH (5 mL) was added 10% palladium on carbon (10 mg). The reaction solution was purged with H₂ (×3) and then reacted at 25°C for 1.5 h. Upon completion, the reaction solution was filtered through Celite, and the filter cake was washed with MeOH (×3). The filtrate was concentrated under reduced pressure to afford the title compound (yellow solid, 35 mg, crude).

### Step C: N-(3-(Isopropylcarbamoyl)-1-phenyl-1H-pyrazol-4-yl)-6-(1H-pyrazol-5-yl)picolinamide

At 0°C, to a solution of 6-(1H-pyrazol-5-yl)picolinic acid (30 mg, 0.16 mmol, 1.1 eq) and 4-amino-N-isopropyl-1-phenyl-1H-pyrazole-3-carboxamide (35 mg, crude) in DMF (1.5 mL) were added DIEA (54 mg, 0.42 mmol, 3.0 eq) and HATU (65 mg, 0.17 mmol, 1.2 eq). The mixture was reacted at rt for 1 h. Upon completion, the mixture was purified by HPLC to afford the title compound (white solid, 15.0 mg, yield 26%). **¹H NMR** (400 MHz, DMSO-*d₆*) *δ* 13.20 (s, 1H), 12.10 (s, 1H), 9.03 (s, 1H), 8.37 (d, *J =* 8.4 Hz, 1H), 8.24 (d, *J =* 7.6 Hz, 1H), 8.14-8.01 (m, 4H), 7.97 (s, 1H), 7.57 (t, *J =* 8.0 Hz, 2H), 7.41 (t, *J* = 7.2 Hz, 1H), 7.32 (s, 1H), 4.31 (dd, *J =* 14.4, 6.8 Hz, 1H), 1.27 (d, *J =* 6.8 Hz, 6H). LCMS (ESI): m/z 416 [M+H]⁺.

### Preparation Example 123. Synthesis of Compound A248

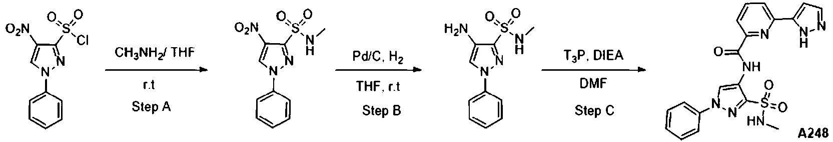

### Step A: N-Methyl-4-nitro-1-phenyl-1H-pyrazole-3-sulfonamide

At 0°C, methylamine (2 mL, 2.10 mmol, 3.0 eq) was added to a solution of 4-nitro-1-phenylpyrazole-3-sulfonyl chloride (200 mg, 0.70 mmol, 1.0 eq) in THF (1 mL). The reaction solution was reacted at rt for 1 h. Upon completion, the reaction solution was concentrated under reduced pressure. The residue was slurried with water (5 mL) to afford the title compound (white solid, 150 mg, yield 76%).

### Step B: 4-Amino-N-methyl-1-phenyl-1H-pyrazole-3-sulfonamide

At rt, palladium on carbon (57 mg, 10%) was added to a solution of *N*-methyl-4-nitro-1-phenyl-1H-pyrazole-3-sulfonamide (150 mg, 0.53 mmol, 1.0 eq) in THF (3 mL). The reaction solution was purged with H₂ (×3) and then reacted at 25°C for 2 h. Upon completion, the reaction solution was filtered through Celite, and the filter cake was washed with MeOH (×3). The filtrate was concentrated under reduced pressure to afford the title compound (yellow oil, 110 mg, yield 82%, crude).

### Step C: N-(3-(N-Methylsulfamoyl)-1-phenyl-1H-pyrazol-4-yl)-6-(1H-pyrazol-5-yl)picolinamide

At 0°C, to a solution of 6-(2H-pyrazol-3-yl)pyridine-2-carboxylic acid (75 mg, 0.40 mmol, 1 eq) and 4-amino-N-methyl-1-phenyl-1H-pyrazole-3-sulfonamide (100 mg, 0.40 mmol, 1.0 eq) in DMF (2.0 mL) were added DIEA (153.69 mg, 1.19 mmol, 3.0 eq) and T₃P (378 mg, 0.60 mmol, 1.5 eq, 50% in EtOAc). The mixture was reacted at rt for 1 h. Upon completion, the mixture was purified by HPLC to afford the title compound (white solid, 30 mg, yield 18%). **¹H NMR** (400 MHz, DMSO-*d₆*) δ 13.21 (s, 1H), 11.29 (s, 1H), 9.15 (s, 1H), 8.26 (d, *J =* 8.0 Hz, 1H), 8.17-8.01 (m, 3H), 7.95 (d, *J =* 8.0 Hz, 3H), 7.59 (t, *J =* 7.6 Hz, 2H), 7.46 (t, *J =* 7.6 Hz, 1H), 7.22 (s, 1H), 2.69 (d, *J =* 4.8 Hz, 3H). LCMS(ESI): m/z 424.2 [M+H]⁺.

### Preparation Example 124. Synthesis of Compound A249

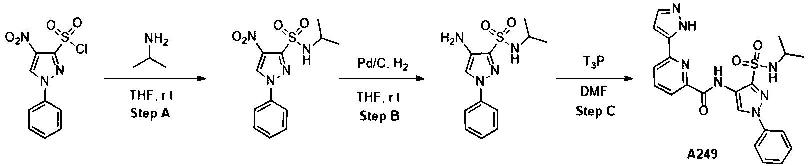

### Step A: N-Isopropyl-4-nitro-1-phenyl-1H-pyrazole-3-sulfonamide

At 0°C, to a solution of 4-nitro-1-phenylpyrazole-3-sulfonyl chloride (200 mg, 0.70 mmol, 1.0 eq) in THF (5 mL) was added isopropylamine (0.18 mL, 2.10 mmol, 3.0 eq). The reaction solution was reacted at rt for 1 h. Upon completion, the reaction solution was concentrated under reduced pressure. The residue was slurried with water (5 mL) to afford the title compound (white solid,180 mg, yield 83%).

### Step B: 4-Amino-N-isopropyl-1-phenyl-1H-pyrazole-3-sulfonamide

At rt, to a solution of *N*-isopropyl-4-nitro-1-phenyl-1H-pyrazole-3-sulfonamide (150 mg, 0.48 mmol, 1.0 eq) in THF (3 mL) was added 10% palladium on carbon (51 mg). The reaction solution was purged with H₂ (×3) and then reacted at 25°C for 2 h. Upon completion, the reaction solution was filtered through Celite, and the filter cake was washed with MeOH (×3). The filtrate was concentrated under reduced pressure to afford the title compound (yellow oil, 105 mg, yield 77%, crude).

### Step C: N-(3-(N-Isopropylsulfamoyl)-1-phenyl-1H-pyrazol-4-yl)-6-(1H-pyrazol-5-yl)picolinamide

At 0°C, to a solution of 6-(2H-pyrazol-3-yl)pyridine-2-carboxylic acid (68 mg, 0.36 mmol, 1 eq) and 4-amino-*N*-isopropyl-1-phenyl-1*H*-pyrazole-3-sulfonamide (100 mg, 0.40 mmol, 1.0 eq) in DMF (2.0 mL) were added DIEA (138 mg, 1.07 mmol, 3 eq) and T₃P (340 mg, 0.54 mmol, 1.5 eq, 50% in EtOAc). The reaction solution was reacted at rt for 1 h. Upon completion, the reaction solution was purified by HPLC to afford the title compound (white solid, 30 mg, yield 18%). **¹H NMR** (400 MHz, DMSO-*d₆*) δ 13.21 (s, 1H), 11.32 (s, 1H), 9.13 (s, 1H), 8.35-8.22 (m, 2H), 8.13-8.07 (m, 2H), 7.99-7.86 (m, 3H), 7.59 (t, *J =* 8.0 Hz, 2H), 7.45 (t, *J =* 7.2 Hz, 1H), 7.22 (s, 1H), 3.70-3.60 (m, 1H), 1.09 (d, *J =* 6.8 Hz, 6H). LCMS(ESI): m/z 452.2 [M+H]⁺.

### Preparation Example 125. Synthesis of Compound A250

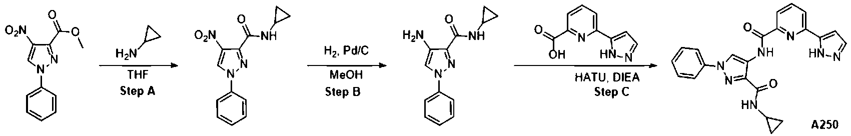

### Step A: N-cyclopropyl-4-nitro-1-phenyl-1H-pyrazole-3-carboxamide

At rt, to a solution of methyl 4-nitro-1-phenylpyrazole-3-carboxylate (300 mg, 1.21 mmol, 1.0 eq) in THF (3 mL) was added cyclopropylamine (1385.9 mg, 24.27 mmol, 20.0 eq). The reaction solution was reacted at 70°C in a sealed tube for 16 h. Upon completion, the reaction solution was concentrated under reduced pressure to afford the title compound (white solid, 210 mg, yield 84%).

### Step B: 4-Amino-N-cyclopropyl-1-phenyl-1H-pyrazole-3-carboxamide

At rt, to a solution of *N*-cyclopropyl-4-nitro-1-phenyl-1H-pyrazole-3-carboxamide (280 mg, 1.03 mmol, 1.0 eq) in MeOH (10 mL) was added palladium on carbon (22 mg, 10%). The reaction solution was purged with H₂ (×3) and then reacted at 25°C for 2 h. Upon completion, the reaction solution was filtered through Celite, and the filter cake was washed with MeOH (×3). The filtrate was concentrated under reduced pressure to afford the title compound (yellow oil, 210 mg, yield 84%, crude).

### Step C: N-(3-(Cyclopropylcarbamoyl)-1-phenyl-1H-pyrazol-4-yl)-6-(1H-pyrazol-5-yl)picolinamide

At 0°C, to a solution of 6-(1H-pyrazol-5-yl)picolinic acid (47 mg, 0.25 mmol, 1.0 eq) and 4-amino-N-cyclopropyl-1-phenyl-1H-pyrazole-3-carboxamide (60 mg, 0.25 mmol, 1.0 eq) in DMF (2.0 mL) were added DIEA (96.0 mg, 0.74 mmol, 3.0 eq) and HATU (113 mg, 0.30 mmol, 1.2 eq). The reaction solution was reacted at rt for 1 h. Upon completion, the reaction solution was purified by reverse-phase column chromatography to afford the title compound (yellow solid, 33 mg, yield 32%). **¹H NMR** (400 MHz, DMSO DMSO-*d₆*) δ 13.21 (s, 1H), 12.04 (s, 1H), 9.03 (s, 1H), 8.61 (d, *J =* 4.8 Hz, 1H), 8.24 (d, *J =* 7.6 Hz, 1H), 8.09 (dd, *J =* 15.6, 7.6 Hz, 2H), 8.03-7.95 (m, 3H), 7.56 (t, *J =* 8.0 Hz, 2H), 7.42-7.33 (m, 2H), 3.04 (d, *J =* 4.4 Hz, 1H), 0.79-0.74 (m, 4H). LCMS (ESI): m/z 414 [M+H]⁺.

### Preparation Example 126. Synthesis of Compound A251

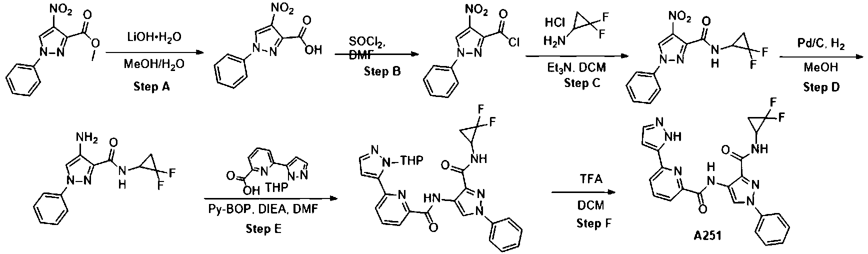

### Step A: 4-Nitro-1-phenyl-1H-pyrazole-3-carboxylic acid

At rt, to a mixed solution of methyl 4-nitro-1-phenyl-1H-pyrazole-3-carboxylate (500 mg, 2.02 mmol, 1.0 eq) in THF (3.0 mL) and water (3.0 mL) was added lithium hydroxide monohydrate (254 mg, 6.07 mmol, 3.0 eq). The reaction solution was reacted at rt for 2 h. Upon completion, the reaction solution was adjusted to pH 2-3 with 1 M HCl. The suspension was filtered, and the filter cake was washed with water (×3) and dried under reduced pressure to afford the title compound (white solid, 350 mg, yield 74%).

### Step B: 4-Nitro-1-phenyl-1H-pyrazole-3-carbonyl chloride

At rt, a solution of 4-nitro-1-phenyl-1H-pyrazole-3-carboxylic acid (350 mg, 1.50 mmol, 1.0 eq) in thionyl chloride (5.0 mL) was reacted at 80°C for 2 h. Upon completion, the reaction solution was concentrated under reduced pressure to afford the title compound (white solid, 350 mg, crude).

### Step C: N-(2,2-Difluorocyclopropyl)-4-nitro-1-phenyl-1H-pyrazole-3-carboxamide

At rt, to a solution of 2,2-difluorocyclopropan-1-amine hydrochloride (180 mg, 1.39 mmol, 1.0 eq) and TEA (0.6 mL, 4.17 mmol, 3.0 eq) in DCM (5.0 mL) was added 4-nitro-1-phenyl-1H-pyrazole-3-carbonyl chloride (17.5 g, 87.7 mmol, 1.5 eq). The reaction solution was reacted at rt for 1 h. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by column chromatography to afford the title compound (white solid, 180 mg, yield 42%).

### Step D: 4-Amino-N-(2,2-difluorocyclopropyl)-1-phenyl-1H-pyrazole-3-carboxamide

At rt, to a solution of *N*-(2,2-difluorocyclopropyl)-4-nitro-1-phenyl-1H-pyrazole-3-carboxamide (180 mg, 0.58 mmol, 1.0 eq) in MeOH (5.0 mL) was added palladium on carbon (63 mg, 10%). The reaction solution was reacted at rt for 2 h under H₂. Upon completion, the reaction solution was filtered, and the filter cake was washed with MeOH (×3). The filtrate was concentrated under reduced pressure to afford the title compound (yellow solid, 140 mg, crude).

### Step E: N-(3-((2,2-Difluorocyclopropyl)carbamoyl)-1-phenyl-1H-pyrazol-4-yl)-6-(1-(tetrahydro-2H-pyrazol-2-yl)-1H-pyrazin-5-yl)picolinamide

At rt, to a solution of 4-amino-N-(2,2-difluorocyclopropyl)-1-phenyl-1H-pyrazole-3-carboxamide (140 mg, 0.50 mmol, 1.0 eq) and 6-(1-(tetrahydro-2H-pyran-2-yl)-1H-pyrazol-5-yl)picolinic acid (138 mg, 0.50 mmol, 1.0 eq) in DMF (3.0 mL) were added 1H-benzotriazol-1-yloxytripyrrolidinophosphonium hexafluorophosphate (393 mg, 0.76 mmol, 1.5 eq) and DIEA (195 mg, 1.51 mmol, 3.0 eq). The reaction solution was reacted at rt for 2 h under N₂. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by column chromatography to afford the title compound (yellow solid, 170 mg, yield 63%).

### Step F: N-(3-((2,2-Difluorocyclopropyl)carbamoyl)-1-phenyl-1H-pyrazol-4-yl)-6-(1H-pyrazol-5-yl)picolinamide

At rt, to a solution of N-(3-((2,2-difluorocyclopropyl)carbamoyl)-1-phenyl-1H-pyrazol-4-yl)-6-(1-(tetrahydro-2H-pyrazol-2-yl)-1H-pyrazin-5-yl)picolinamide (170 mg, 0.32 mmol, 1.0 eq) in DCM (3.0 mL) was added TFA (1.0 mL). The reaction solution was reacted at rt for 16 h under N₂. Upon completion, the reaction solution was concentrated under reduced pressure. The residue was purified by HPLC to afford the title compound (white solid, 17.5 mg, yield 12%). **¹H NMR** (400 MHz, DMSO-*d₆*) δ 13.22 (s, 1H), 11.97 (s, 1H), 9.06 (s, 1H), 9.05-9.02 (m, 1H), 8.28-8.20 (m, 1H), 8.12 (t, *J* = 7.6 Hz, 1H), 8.09-8.06 (m, 1H), 8.03 (d, *J* = 7.8 Hz, 2H), 7.94 (s, 1H), 7.58 (t, *J =* 7.9 Hz, 2H), 7.42 (t, *J =* 7.4 Hz, 1H), 7.32 (s, 1H), 3.68-3.56 (m, 1H), 2.08-1.88 (m, 2H). LCMS (ESI): m/z 450.3 [M+H]⁺.

### Preparation Example 127. Synthesis of Compound A252

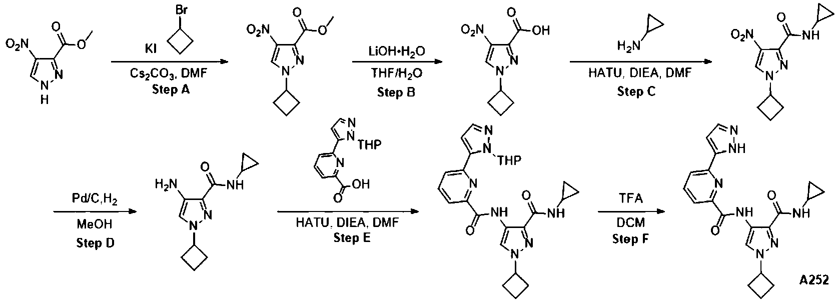

### Step A: Methyl 1-cyclobutyl-4-nitro-1H-pyrazole-3-carboxylate

At rt, cesium carbonate (11.4 g, 35.1 mmol, 3.0 eq) and potassium iodide (1.9 g, 11.6 mmol, 1.0 eq) were added to a solution of methyl 4-nitro-1H-pyrazole-3-carboxylate (2 g, 11.7 mmol, 1.0 eq) and bromocyclobutane (1.9 g, 14.0 mmol, 1.2 eq) in DMF (30.0 mL). The reaction solution was reacted at 100°C for 5 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 750 mg, yield 29%).

### Step B: 1-Cyclobutyl-4-nitro-1H-pyrazole-3-carboxylic acid

At rt, lithium hydroxide monohydrate (1.3 g, 31.0 mmol, 1.0 eq) was added to a mixed solution of methyl 1-cyclobutyl-4-nitro-1H-pyrazole-3-carboxylate (700 mg, 3.1 mmol, 1.0 eq) in THF (10.0 mL) and water (5.0 mL). The reaction solution was reacted at rt for 3 h. Upon completion, the pH was adjusted to 4-5 with 2 M HCl, and the reaction solution was extracted with EtOAc (×3). The combined organic phases were washed with saturated aqueous NaCl, dried over anhydrous Na₂SO₄, filtered, and concentrated under reduced pressure to afford the title compound (white solid, 670 mg, crude).

### Step C: 1-Cyclobutyl-N-cyclopropyl-4-nitro-1H-pyrazole-3-carboxamide

At rt, cyclopropylamine (207 mg, 3.6 mmol, 1.2 eq) and DIEA (1.56 g, 12.1 mmol, 4.0 eq) were added to a solution of 1-cyclobutyl-4-nitro-1H-pyrazole-3-carboxylic acid (640 mg, 3.03 mmol, 1.0 eq) and HATU (1.73 g, 4.5 mmol, 1.5 eq) in DMF (3.0 mL). The reaction solution was reacted at rt for 15 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 380 mg, yield 50%).

### Step D: 4-Amino-1-cyclobutyl-N-cyclopropyl-1H-pyrazole-3-carboxamide

At rt, palladium on carbon (50 mg, 10%) was added to a solution of 1-cyclobutyl-N-cyclopropyl-4-nitro-1H-pyrazole-3-carboxamide (360 mg, 1.4 mmol, 1.0 eq) in MeOH (10.0 mL). The reaction solution was reacted at rt for 2 h under H₂. Upon completion, the reaction solution was filtered, and the filter cake was washed with MeOH (×3). The filtrate was concentrated under reduced pressure to afford the title compound (white solid, 240 mg, yield 76%).

### Step E: N-(1-Cyclobutyl-3-(cyclopropylcarbamoyl)-1H-pyrazol-4-yl)-6-(1-(tetrahydro-2H-pyrazol-2-yl)-1H-pyrazol-5-yl)picolinamide

At rt, to a solution of 6-(1-(tetrahydro-2H-pyran-2-yl)-1H-pyrazol-5-yl)picolinic acid (300 mg, 1.1 mmol, 1.0 eq) and HATU (570 mg, 1.5 mmol, 1.5 eq) in DMF (5.0 mL) were added 4-amino-1-cyclobutyl-N-cyclopropyl-1H-pyrazole-3-carboxamide (220 mg, 1.0 mmol, 1.0 eq) and DIEA (387 mg, 3.0 mmol, 3.0 eq). The reaction solution was reacted at rt for 4 h. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by column chromatography to afford the title compound (white solid, 210 mg, yield 44%).

### Step F: N-(1-Cyclobutyl-3-(cyclopropylcarbamoyl)-1H-pyrazol-4-yl)-6-(1H-pyrazol-5-yl)picolinamide

At rt, to a solution of N-(1-cyclobutyl-3-(cyclopropylcarbamoyl)-1H-pyrazol-4-yl)-6-(1-(tetrahydro-2H-pyrazol-2-yl)-1H-pyrazol-5-yl)picolinamide (210 mg, 0.4 mmol, 1.0 eq) in DCM (6.0 mL) was added TFA (2.0 mL). The reaction solution was reacted at rt for 5 h. Upon completion, the reaction solution was concentrated under reduced pressure. The residue was purified by HPLC to afford the title compound (white solid, 22.7 mg, yield 13%). **¹H NMR** (400 MHz, DMSO-*d₆*) δ 13.19 (s, 1H), 11.96 (s, 1H), 8.48 (s, 1H), 8.33 (d, *J* = 4.8 Hz, 1H), 8.21 (d, *J =* 7.6 Hz, 1H), 8.09 (t, *J =* 7.6 Hz, 1H), 8.02 (d, *J =* 7.6 Hz, 1H), 7.95 (s, 1H), 7.31 (s, 1H), 4.95 (t, *J* = 8.4 Hz, 1H), 2.99 (d, *J* = 5.2 Hz, 1H), 2.66-2.53 (m, 2H), 2.41 (d, *J =* 7.6 Hz, 2H), 1.91-1.71 (m, 2H), 0.72 (d, *J =* 6.0 Hz, 4H). LCMS (ESI): m/z 392 [M+H]⁺.

### Preparation Example 128. Synthesis of Compound A253

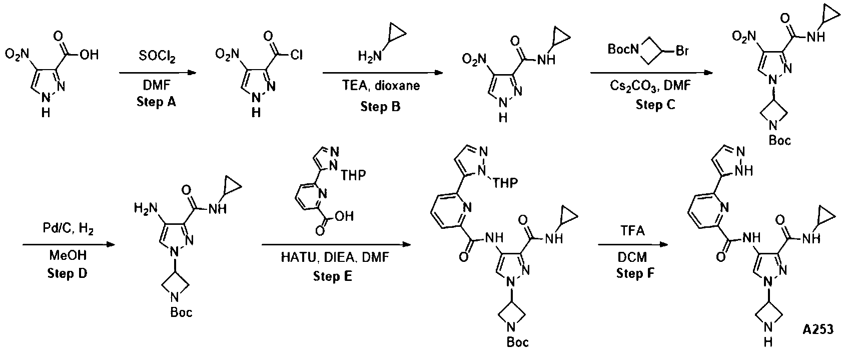

### Step A: 4-nitro-1H-pyrazole-3-carbonyl chloride

At rt, thionyl chloride (20.0 mL) was added to a solution of 4-nitro-1H-pyrazole-3-carboxylic acid (2 g, 12.732 mmol, 1.0 eq). The mixture was reacted at 80°C for 2 h. Upon completion, the reaction solution was concentrated under reduced pressure to afford the title compound (white solid, 1.5 g, crude).

### Step B: N-cyclopropyl-4-nitro-1H-pyrazole-3-carboxamide

At rt, 4-nitro-1H-pyrazole-3-carbonyl chloride (1.5 g, 8.546 mmol, 1.0 eq) was added to a solution of cyclopropylamine (0.49 g, 8.55 mmol, 1.0 eq) and TEA (3.6 mL, 25.6 mmol, 3.0 eq) in 1,4-dioxane (20.0 mL). The reaction solution was reacted at rt for 1 h. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by column chromatography to afford the title compound (white solid, 1.2 g, yield 72%).

### Step C: Tert-butyl 3-(3-(cyclopropylcarbamoyl)-4-nitro-1H-pyrazol-1-yl)azetidine-1-carboxylate

At rt, cesium carbonate (3.32 g, 10.2 mmol, 3.0 eq) was added to a solution of *N*-cyclopropyl-4-nitro-1H-pyrazole-3-carboxamide (1 g, 5.10 mmol, 1.0 eq) and tert-butyl 3-bromoazetidine-1-carboxylate (1.20 g, 5.10 mmol, 1.0 eq) in DMF (10.0 mL). The reaction solution was reacted at 100°C for 2 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 400 mg, yield 22%).

### Step D: Tert-butyl 3-(4-amino-3-(cyclopropylcarbamoyl)-1H-pyrazol-1-yl)azetidine-1-carboxylate

At rt, palladium on carbon (121 mg, 10%) was added to a solution of tert-butyl 3-(3-(cyclopropylcarbamoyl)-4-nitro-1H-pyrazol-1-yl)azetidine-1-carboxylate (400 mg, 1.14 mmol, 1.0 eq) in MeOH (5.0 mL). The reaction solution was reacted at rt for 2 h under H₂. Upon completion, the reaction solution was filtered, and the filter cake was washed with MeOH (×3). The filtrate was concentrated under reduced pressure to afford the title compound (yellow solid, 380 mg, crude).

### Step E: Tert-butyl 3-(3-(cyclopropylcarbamoyl)-4-(6-(1-(tetrahydro-2H-pyran-2-yl)-1H-pyrazol-5-yl)picolinamido)-1H-pyrazol-1-yl)azetidine-1-carboxylate

At rt, to a solution of 6-(1-(tetrahydro-2H-pyran-2-yl)-1H-pyrazol-5-yl)picolinic acid (170 mg, 0.62 mmol, 1.0 eq) and HATU (281 mg, 0.74 mmol, 1.2 eq) in DMF (10.0 mL) were added tert-butyl 3-(4-amino-3-(cyclopropylcarbamoyl)-1H-pyrazol-1-yl)azetidine-1-carboxylate (200 mg, 0.62 mmol, 1.0 eq) and DIEA (241 mg, 1.88 mmol, 2.0 eq). The reaction solution was reacted at rt for 15 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 250 mg, yield 70%).

### Step F: N-(1-(Azetidin-3-yl)-3-(cyclopropylcarbamoyl)-1H-pyrazol-4-yl)-6-(1H-pyrazol-5-yl)picolinamide

At rt, to a solution of tert-butyl 3-(3-(cyclopropylcarbamoyl)-4-(6-(1-(tetrahydro-2H-pyran-2-yl)-1H-pyrazol-5-yl)picolinamido)-1H-pyrazol-1-yl)azetidine-1-carboxylate (240 mg, 0.42 mmol, 1.0 eq) in DCM (3.0 mL) was added TFA (1.0 mL). The reaction solution was reacted at rt for 16 h. Upon completion, the reaction solution was concentrated under reduced pressure. The residue was purified by HPLC to afford the title compound (white solid, 27.0 mg, yield 17%). **¹H NMR** (400 MHz, DMSO-*d₆*) δ 13.23 (s, 1H), 11.97 (s, 1H), 8.59 (s, 1H), 8.39 (d, *J* = 4.2 Hz, 1H), 8.21 (d, *J =* 7.8 Hz, 1H), 8.10 (t, *J* = 7.6 Hz, 1H), 8.03 (d, *J =* 7.5 Hz, 1H), 7.93 (s, 1H), 7.30 (s, 1H), 5.35-5.20 (m, 1H), 3.94 (t, *J* = 7.3 Hz, 2H), 3.79 (t, *J =* 7.7 Hz, 2H), 3.05-2.95 (m, 1H), 0.76-0.68 (m, 4H). LCMS (ESI): m/z 393.2 [M+H]⁺.

### Preparation Example 129. Synthesis of Compound A254

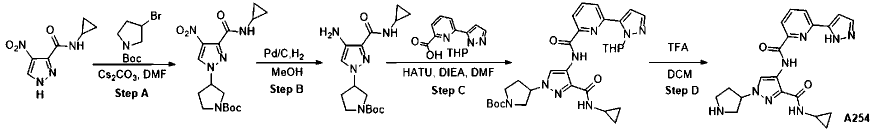

### Step A: Tert-butyl 3-(3-(cyclopropylcarbamoyl)-4-nitro-1H-pyrazol-1-yl)pyrrolidine-1-carboxylate

At rt, cesium carbonate (5.0 g, 15.3 mmol, 3.0 eq) was added to a solution of*N*-cyclopropyl-4-nitro-1H-pyrazole-3-carboxamide (1 g, 5.10 mmol, 1.0 eq) and tert-butyl 3-bromopyrrolidine-1-carboxylate (1.28 g, 5.1 mmol, 1.0 eq) in DMF (10.0 mL). The reaction solution was reacted at 100°C for 2 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 420 mg, yield 23%).

### Step B: Tert-butyl 3-(4-amino-3-(cyclopropylcarbamoyl)-1H-pyrazol-1-yl)pyrrolidine-1-carboxylate

At rt, palladium on carbon (50 mg, 10%) was added to a solution of tert-butyl 3-(3-(cyclopropylcarbamoyl)-4-nitro-1H-pyrazol-1-yl)pyrrolidine-1-carboxylate (420 mg, 1.1 mmol, 1.0 eq) in MeOH (10.0 mL). The reaction solution was reacted at rt for 2 h under H₂. Upon completion, the reaction solution was filtered, and the filter cake was washed with MeOH (×3). The filtrate was concentrated under reduced pressure to afford the title compound (white solid, 240 mg, crude).

### Step C: Tert-butyl 3-(3-(cyclopropylcarbamoyl)-4-(6-(1-(tetrahydro-2H-pyran-2-yl)-1H-pyrazol-5-yl)picolinamido)-1H-pyrazol-1-yl)pyrrolidine-1-carboxylate

At rt, to a solution of 6-(1-(tetrahydro-2H-pyran-2-yl)-1H-pyrazol-5-yl)picolinic acid (197 mg, 0.77 mmol, 1.1 eq) and HATU (399 mg, 1.05 mmol, 1.5 eq) in DMF (10.0 mL) were added tert-butyl 3-(4-amino-3-(cyclopropylcarbamoyl)-1H-pyrazol-1-yl)pyrrolidine-1-carboxylate (220 mg, 0.7 mmol, 1.0 eq) and DIEA (271 mg, 2.1 mmol, 3.0 eq). The reaction solution was reacted at rt for 4 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 190 mg, yield 49%).

### Step D: N-(3-(Cyclopropylcarbamoyl)-1-(pyrrolidin-3-yl)-1H-pyrazol-4-yl)-6-(1H-pyrazol-5-yl)picolinamide

At rt, to a solution of tert-butyl 3-(3-(cyclopropylcarbamoyl)-4-(6-(1-(tetrahydro-2H-pyran-2-yl)-1H-pyrazol-5-yl)picolinamido)-1H-pyrazol-1-yl)pyrrolidine-1-carboxylate (190 mg, 0.3 mmol, 1.0 eq) in DCM (6.0 mL) was added TFA (2.0 mL). The reaction solution was reacted at rt for 16 h. Upon completion, the mixture was concentrated under reduced pressure. The residue was purified by HPLC to afford the title compound (white solid, 22.6 mg, yield 17%). **¹H NMR** (400 MHz, DMSO-*d₆*) δ 13.21 (s, 1H), 11.95 (s, 1H), 8.48 (s, 1H), 8.32 (s, 1H), 8.21 (d, *J =* 7.6 Hz, 1H), 8.10 (t, *J =* 7.6 Hz, 1H), 8.03 (d, *J* = 7.6 Hz, 1H), 7.94 (s, 1H), 7.30 (s, 1H), 4.95 (s, 1H), 3.11 (s, 3H), 2.97 (d, *J =* 3.6 Hz, 1H), 2.87 (s, 1H), 2.25 (dd, *J =* 13.6, 5.6 Hz, 1H), 2.01 (s, 1H), 0.88-0.60 (m, 4H). LCMS (ESI): m/z 407 [M+H]⁺.

### Preparation Example 130. Synthesis of Compound A255

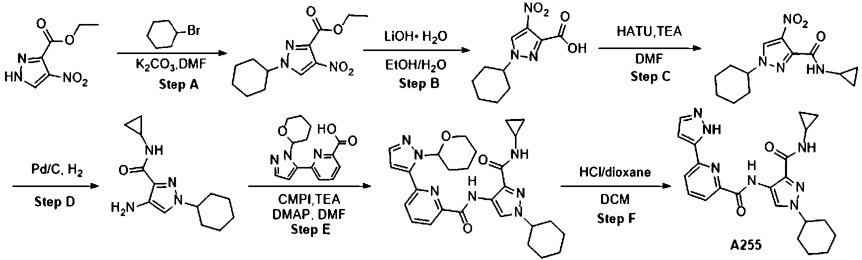

### Step A: Ethyl 1-cyclohexyl-4-nitro-1H-pyrazole-3-carboxylate

Potassium carbonate (1343 mg, 9.7 mmol, 2.0 eq) was added to a solution of ethyl 4-nitro-1H-pyrazole-3-carboxylate (900 mg, 4.9 mmol, 1.0 eq) and cyclohexyl bromide (951 mg, 5.8 mmol, 1.2 eq) in DMF (50 mL). The mixture was stirred at 100°C overnight. Upon completion, the mixture was diluted in water (15 mL) and extracted with EtOAc (15 mL×2). The organic layer was washed with saturated aqueous NaCl, dried over anhydrous Na₂SO₄, and concentrated under reduced pressure. The residue was purified by normal-phase column chromatography to afford the title compound (colorless oil, 320 mg, yield 24%).

### Step B: 1-Cyclohexyl-4-nitro-1H-pyrazole-3-carboxylic acid

Lithium hydroxide monohydrate (151 mg, 3.6 mmol, 3.0 eq) was added to a solution of ethyl 1-cyclohexyl-4-nitro-1H-pyrazole-3-carboxylate (320 mg, 1.2 mmol, 1.0 eq) in ethanol (5 mL) and water (1 mL). The mixture was stirred at rt for 2 h. Upon completion, the mixture was adjusted to pH 4-5 with 2 N HCl, The mixture was diluted in water (10 mL), and extracted with EtOAc (3×20 mL). The organic layer was washed with saturated aqueous NaCl (2×15 mL), dried over anhydrous Na₂SO₄, and concentrated under reduced pressure to afford the title compound (white solid, 230 mg, quantitative).

### Step C: 1-cyclohexyl-N-cyclopropyl-4-nitro-1H-pyrazole-3-carboxamide

To a solution of 1-cyclohexyl-4-nitropyrazole-3-carboxylic acid (230 mg, 1.0 mmol, 1.0 eq) and HATU (439 mg, 1.2 mmol, 1.2 eq) in DMF (5 mL) were added TEA (0.5 mL, 3.85 mmol, 4.0 eq) and cyclopropylamine (82 mg, 1.5 mmol, 1.5 eq). The mixture was stirred at 25°C for 30 min. Upon completion, the reaction mixture was diluted in water (15 mL) and extracted with EtOAc (3×15 mL). The organic layer was washed with saturated aqueous NaCl (15 mL), dried over anhydrous Na₂SO₄, and concentrated under reduced pressure. The residue was purified by normal-phase column chromatography to afford the title compound (white solid, 220 mg, yield 82%).

### Step D: 4-amino-1-cyclohexyl-N-cyclopropyl-1H-pyrazole-3-carboxamide

Palladium on carbon (25 mg, 10%) was added to a solution of 1-cyclohexyl-N-cyclopropyl-4-nitropyrazole-3-carboxamide (220 mg, 0.8 mmol, 1.0 eq) in MeOH (10 mL). The mixture was purged with H₂ (using a hydrogen balloon) (×3) and stirred at rt for 1 h. Upon completion, the mixture was filtered through Celite, and the filter cake was washed with MeOH (20 mL). The combined filtrates were concentrated to dryness to afford the title compound (brown solid, 190 mg, quantitative), which was used directly in the next step without further purification.

### Step E: N-(1-Cyclohexyl-3-(cyclopropylcarbamoyl)-1H-pyrazol-4-yl)-6-(1-(tetrahydro-2H-pyran-2-yl)-1H-pyrazol-5-yl)picolinamide

To a solution of 4-amino-1-cyclohexyl-N-cyclopropyl-1H-pyrazole-3-carboxamide (190 mg, 0.8 mmol, 1.0 eq), CMPI (587 mg, 2.3 mmol, 3.0 eq), TEA (0.35 mL, 3.1 mmol, 4.0 eq), and 6-(1-(tetrahydro-2H-pyran-2-yl)-1H-pyrazin-5-yl)picolinic acid (250 mg, 0.92 mmol, 1.2 eq) in DMF (5 mL) was added DMAP (9 mg, 0.076 mmol, 0.1 eq). The mixture was stirred at rt for 30 min. Upon completion, the reaction mixture was diluted in water (15 mL) and extracted with EtOAc (20 mL×3). The organic layer was washed with saturated aqueous NaCl (15 mL×2), dried over anhydrous Na₂SO₄, and concentrated under reduced pressure. The residue was purified by normal-phase column chromatography to afford the title compound (white solid, 160 mg, yield 42%).

### Step F: N-(1-Cyclohexyl-3-(cyclopropylcarbamoyl)-1H-pyrazol-4-yl)-6-(1H-pyrazol-5-yl)picolinamide

To a solution of N-(1-cyclohexyl-3-(cyclopropylcarbamoyl)-1H-pyrazol-4-yl)-6-(1-(tetrahydro-2H-pyrazol-2-yl)-1H-pyrazol-5-yl)picolinamide (130 mg, 0.22 mmol, 1.0 eq) in DCM (2 mL) was added HCl/1,4-dioxane (1 mL, 4 M). The mixture was stirred at 25°C for 10 min. Upon completion, the reaction mixture was concentrated under reduced pressure. The residue was purified by HPLC to afford the title compound (white solid, 37.2 mg, yield 34%). **¹H NMR** (400 MHz, DMSO-*d₆*) δ 13.20 (s, 1H), 11.96 (s, 1H), 8.43 (s, 1H), 8.26 (d, *J* = 4.8 Hz, 1H),8.21 (d, *J* = 7.8 Hz, 1H), 8.09 (t, *J =* 7.7 Hz, 1H), 8.03 (d, *J =* 7.6 Hz, 1H), 7.95 (s, 1H),7.31 (s, 1H), 4.42-4.09 (m, 1H), 3.03-2.93 (m, 1H), 2.11-2.01 (m, 2H), 1.81 (dt, *J =* 12.2, 8.7 Hz, 4H), 1.68 (d, *J =* 12.1 Hz, 1H), 1.41 (q, *J =* 13.0 Hz, 2H), 1.25 (dd, *J =* 25.1, 12.7 Hz, 1H), 0.79-0.65 (m, 4H). LCMS (ESI): m/z 420 [M+H]⁺.

### Preparation Example 131. Synthesis of Compound A256

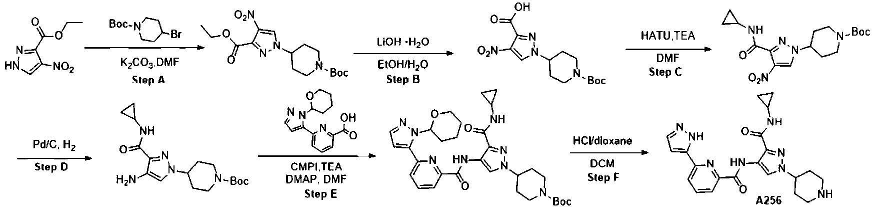

### Step A: Tert-butyl 4-(3-(ethoxycarbonyl)-4-nitro-1H-pyrazol-1-yl)piperidine-1-carboxylate

To a solution of ethyl 4-nitro-1H-pyrazole-3-carboxylate (900 mg, 4.9 mmol, 1.0 eq) and tert-butyl 4-bromopiperidine-1-carboxylate (1.54 g, 5.8 mmol, 1.2 eq) in DMF (50 mL) was added potassium carbonate (1343 mg, 9.7 mmol, 2.0 eq). The solution was stirred at 100°C overnight. Upon completion, the reaction mixture was diluted in water (15 mL) and extracted with EtOAc (2×25 mL). The organic layer was washed with saturated aqueous NaCl, dried over anhydrous Na₂SO₄, and concentrated under reduced pressure. The residue was purified by normal-phase column chromatography to afford the title compound (colorless oil, 390 mg, yield 21%).

### Step B: 1-(1-(tert-butoxycarbonyl)piperidin-4-yl)-4-nitro-1H-pyrazole-3-carboxylic acid

Lithium hydroxide monohydrate (133 mg, 3.2 mmol, 3.0 eq) was added to a solution of tert-butyl 4-(3-(ethoxycarbonyl)-4-nitro-1H-pyrazol-1-yl)piperidine-1-carboxylate (390 mg, 1.06 mmol, 1.0 eq) in ethanol (5 mL) and water (1 mL). The resulting mixture was stirred at rt for 2 h. Upon completion, the mixture was adjusted to pH 4-5 with 2 N HCl, The resulting mixture was diluted in water (10 mL), and extracted with EtOAc (3×20 mL). The organic layer was washed with saturated aqueous NaCl (2×15 mL), dried over anhydrous Na₂SO₄, and concentrated under reduced pressure to afford the title compound (white solid, 320 mg, quantitative).

### Step C: Tert-butyl 4-(3-(cyclopropylcarbamoyl)-4-nitro-1H-pyrazol-1-yl)piperidine-1-carboxylate

To a solution of 1-(1-(tert-butoxycarbonyl)piperidin-4-yl)-4-nitro-1H-pyrazole-3-carboxylic acid (320 mg, 0.94 mmol, 1.0 eq) and HATU (429 mg, 1.1 mmol, 1.2 eq) in DMF (5 mL) were added TEA (0.5 mL, 3.85 mmol, 4.0 eq) and cyclopropylamine (107 mg, 1.9 mmol, 2.0 eq). The mixture was stirred at 25°C for 30 min. Upon completion, the reaction mixture was diluted in water (15 mL) and extracted with EtOAc (3×15 mL). The organic layer was washed with saturated aqueous NaCl (15 mL), dried over anhydrous Na₂SO₄, and concentrated under reduced pressure. The residue was purified by normal-phase column chromatography to afford the title compound (colorless oil, 290 mg, yield 82%).

### Step D: Tert-butyl 4-(4-amino-3-(cyclopropylcarbamoyl)-1H-pyrazol-1-yl)piperidine-1-carboxylate

Palladium on carbon (25 mg, 10%) was added to a solution of tert-butyl 4-(3-(cyclopropylcarbamoyl)-4-nitro-1H-pyrazol-1-yl)piperidine-1-carboxylate (290 mg, 0.53 mmol, 1.0 eq) in MeOH (10 mL). The mixture was purged with H₂ (using a hydrogen balloon) (×3) and stirred at rt for 1 h. Upon completion, the mixture was filtered through Celite, and the filter cake was washed with MeOH (20 mL). The combined filtrates were concentrated to dryness to afford the title compound (brown solid, 260 mg, quantitative), which was used directly in the next step without further purification.

### Step E: Tert-butyl 4-(3-(cyclopropylcarbamoyl)-4-(6-(1-(tetrahydro-2H-pyran-2-yl)-1H-pyrazol-5-yl)picolinamido)-1H-pyrazol-1-yl)piperidine-1-carboxylate

At rt, to a solution of tert-butyl 4-(4-amino-3-(cyclopropylcarbamoyl)-1H-pyrazol-1-yl)piperidine-1-carboxylate (260 mg, 0.75 mmol, 1.0 eq), CMPI (571 mg, 2.2 mmol, 3.0 eq), TEA (0.35 mL, 3.0 mmol, 4.0 eq), and 6-(1-(tetrahydro-2H-pyran-2-yl)-1H-pyrazol-5-yl)picolinic acid (244 mg, 0.90 mmol, 1.2 eq) in DMF (5 mL) was added DMAP (9 mg, 0.076 mmol, 0.1 eq). The mixture was stirred at rt for 30 min. Upon completion, the reaction mixture was diluted in water (15 mL) and extracted with EtOAc (20 mL×3). The organic layer was washed with saturated aqueous NaCl (15 mL×2), dried over anhydrous Na₂SO₄, and concentrated under reduced pressure. The residue was purified by normal-phase column chromatography to afford the title compound (white solid, 220 mg, yield 49%).

### Step F: N-(3-(Cyclopropylcarbamoyl)-1-(piperidin-4-yl)-1H-pyrazol-4-yl)-6-(1H-pyrazol-5-yl)picolinamide

At rt, to a solution of tert-butyl 4-(3-(cyclopropylcarbamoyl)-4-(6-(1-(tetrahydro-2H-pyran-2-yl)-1H-pyrazol-5-yl)picolinamido)-1H-pyrazol-1-yl)piperidine-1-carboxylate (130 mg, 0.22 mmol, 1.0 eq) in DCM (2 mL) was added HCl/1,4-dioxane (1 mL, 4 M). The mixture was stirred at 25°C for 10 min. Upon completion, the reaction mixture was concentrated under reduced pressure. The residue was purified by preparative HPLC to afford the title compound (white solid, 35.3 mg, yield 38%). **¹H NMR** (400 MHz, DMSO-*d₆*) δ 13.26 (s, 1H), 11.96 (s, 1H), 8.44 (s, 1H), 8.32-8.24 (m, 2H),8.21 (d, *J =* 7.7 Hz, 1H), 8.10 (t, *J* = 7.7 Hz, 1H), 8.03 (d, *J =* 7.6 Hz, 1H), 7.93 (s, 1H),7.30 (d, *J* = 2.1 Hz, 1H), 4.41 (dd, *J =* 15.5, 11.9 Hz, 1H), 3.19 (d, *J =* 12.6 Hz, 2H), 3.02-2.94 (m, 1H), 2.75 (t, *J =* 12.0 Hz, 2H), 2.14-2.03 (m, 2H), 1.96 (dd, *J* = 21.5, 11.9 Hz, 2H), 0.78-0.64 (m, 4H). LCMS (ESI): m/z 421 [M+H]⁺.

### Preparation Example 132. Synthesis of Compound A257

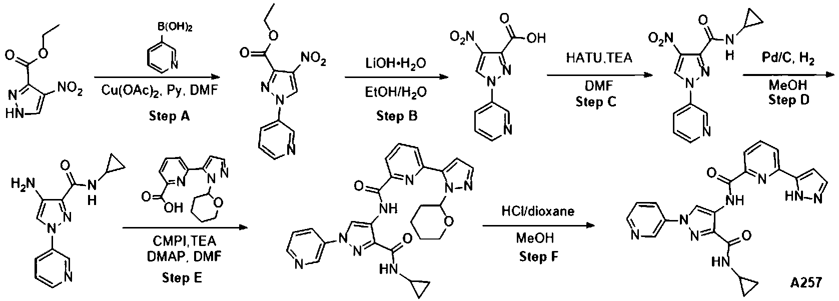

### Step A: Ethyl 4-nitro-1-(pyridin-3-yl)-1H-pyrazole-3-carboxylate

To a mixed solution of ethyl 4-nitro-1H-pyrazole-3-carboxylate (500 mg, 2.7 mmol, 1.0 eq) and pyridin-3-ylboronic acid (399 mg, 3.24 mmol, 1.2 eq) in DMF (20 mL) were added Cu(OAc)2 (488 mg, 2.7 mmol, 1 eq) and pyridine (43 mg, 0.54 mmol, 0.2 eq). The mixture was stirred at 100°C for 4 h. Upon completion, the reaction mixture was diluted in water (15 ml) and extracted with EtOAc (15 mL×3). The combined organic layers were dried over anhydrous Na₂SO₄ and concentrated under reduced pressure. The residue was purified by silica gel column chromatography to afford the title compound (white solid, 180 mg, yield 25%).

### Step B: 4-nitro-1-(pyridin-3-yl)-1H-pyrazole-3-carboxylic acid

Ethyl 4-nitro-1-(pyridine-3-carbonyl)-1H-pyrazole-3-carboxylate (180 mg, 0.68 mmol, 1.0 eq) was dissolved in diethyl ether (5 mL) and water (1 mL), followed by the addition of lithium hydroxide monohydrate (137 mg, 3.44 mmol, 5.0 eq). The mixture was stirred at 25°C for 4 h. Upon completion, the pH was adjusted to 4-5 with 1 N HCl. The mixture was diluted in water (10 mL) and extracted with EtOAc (20 mL×3). The organic layer was washed with aqueous NaCl (15 mL×2), dried over anhydrous Na₂SO₄, and concentrated under reduced pressure to afford the title compound (white solid, 150 mg, quantitative), which was used directly in the next step without further purification.

### Step C: N-cyclopropyl 4-nitro-1-(pyridin-3-yl)-1H-pyrazole-3-carboxamide

4-nitro-1-(pyridine-3-carbonyl)-1H-pyrazole-3-carboxylic acid (160 mg, 0.68 mmol, 1.0 eq) and HATU (310 mg, 0.82 mmol, 1.2 eq) were dissolved in DMF (5 mL), followed by the addition of TEA (141 mg, 1.4 mmol, 2.0 eq) and cyclopropylamine (47 mg, 0.82 mmol, 1.2 eq). Upon completion, the mixture was diluted in water (15 ml) and extracted with EtOAc (15 ml×3). The organic layer was washed with aqueous NaCl (15 ml), dried over anhydrous Na₂SO₄, and concentrated under reduced pressure. The residue was purified by silica gel chromatography to afford the title compound (white solid, 70 mg, yield 38%).

### Step D: 4-amino-N-cyclopropyl-1-(pyridin-3-yl)-1H-pyrazole-3-carboxamide

Pd/C (10%, 25 mg) was added to a solution of N-cyclopropyl 4-nitro-1-(pyridin-3-yl)-1H-pyrazole-3-carboxamide (70 mg, 0.26 mmol, 1.0 eq) in MeOH (10 mL). The mixture was degassed and purged with H₂ (×3) and stirred at rt for 1 h. The mixture was filtered through a Celite^{®} pad and the filter cake was washed with MeOH (20 mL). The filtrate was concentrated and dried to afford the title compound (colorless oil, 55 mg, quantitative), which was used directly in the next step without further purification.

### Step E: N-(3-(Cyclopropylcarbamoyl)-1-(pyridin-3-yl)-1H-pyrazol-4-yl)-6-(1-(tetrahydro-2H-pyran-2-yl)-1H-pyrazol-5-yl)picolinamide

To a solution of 4-amino-N-cyclopropyl-1-(pyridine-3-carbonyl)-1H-pyrazole-3-carboxamide (55 mg, 0.23 mmol, 1.0 eq), CMPI (70 mg, 0.28 mmol, 1.2 eq), TEA (46 mg, 0.46 mmol, 2.0 eq), and 6-(1-(tetrahydro-2H-pyran-2-carbonyl)-1H-pyrazole-5-carbonyl)pyridinecarboxylic acid (75 mg, 0.28 mmol, 1.2 eq) in DMF (5 mL) was added DMAP (3 mg, 0.02 mmol, 0.1 eq). The mixture was reacted at rt. Upon completion, the reaction mixture was diluted in water (15 ml) and extracted with EtOAc (20 mL×3). The organic layers were washed with aqueous NaCl (15 ml×2), dried over anhydrous Na₂SO₄, and concentrated under reduced pressure. The residue was purified by silica gel column chromatography to afford the title compound (white solid, 70 mg, yield 38%).

### Step F: N-(3-(Cyclopropylcarbamoyl)-1-(pyridin-3-yl)-1H-pyrazol-4-yl)-6-(1H-pyrazol-5-yl)picolinamide

To a solution of N-(3-(cyclopropylcarbamoyl)-1-(pyridin-3-yl)-1H-pyrazol-4-yl)-6-(1-(tetrahydro-2H-pyran-2-yl)-1H-pyrazol-5-yl)picolinamide (70 mg, 0.14 mmol, 1.0 eq) in MeOH (5 mL) was added HCl/dioxane (1 mL, 4 M) at 25°C. The mixture was stirred at 25°C for 10 min. Upon completion, the mixture was concentrated under reduced pressure. The residue was purified by reverse-phase column chromatography to afford the title compound (white solid, 30.2 mg, yield 52%). **¹H NMR** (400 MHz, DMSO-*d₆*) δ 13.22 (s, 1H), 12.04 (s, 1H), 9.29 (d, *J =* 2.6 Hz, 1H), 9.14 (s, 1H), 8.68 (d, *J =* 4.8 Hz, 1H), 8.59 (dd, *J* = 4.7, 1.4 Hz, 1H), 8.45-8.40 (m, 1H), 8.24 (d, *J* = 7.8 Hz, 1H), 8.15-8.05 (m, 2H), 7.96 (s, 1H), 7.60 (dd, *J =* 8.4, 4.7 Hz, 1H), 7.33 (s, 1H), 3.03 (d, *J =* 1.2 Hz, 1H), 0.79-0.72 (m, 4H). LCMS (ESI): m/z 415 [M+H]⁺.

### Preparation Example 133. Synthesis of Compound A258

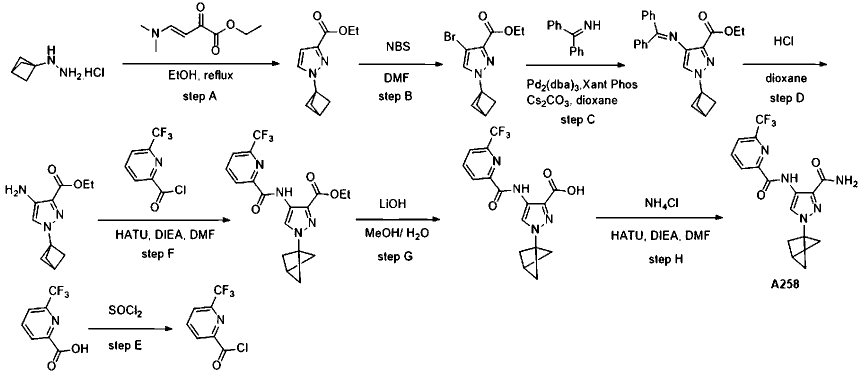

### Step A: Ethyl 1-(bicyclo[1.1.1]pentan-1-yl)-1H-pyrazole-3-carboxylate

At rt, to a solution of bicyclo[1.1.1]pentan-1-yl hydrazine hydrochloride (250 mg, 1.46 mmol, 1.0 eq) in ethanol (5.0 mL) was added ethyl 4-(dimethylamino)-2-oxobut-3-enoate (325 mg, 1.9 mmol, 1.3 eq). The reaction solution was refluxed at 60°C under N₂ for 16 h. Upon completion, the reaction solution was cooled to rt and concentrated under reduced pressure. The residue was purified by column chromatography to afford the title compound (yellow oil, 85 mg, yield 28.3%).

### Step B: Ethyl 1-(bicyclo[1.1.1]pentan-1-yl)-4-bromo-1H-pyrazole-3-carboxylate

At rt, to a solution of ethyl 1-(bicyclo[1.1.1]pentan-1-yl)-1H-pyrazole-3-carboxylate (80 mg, 0.38 mmol, 1.0 eq) in DMF (3.0 mL) was added N-bromosuccinimide (203 mg, 1.14 mmol, 3.0 eq). The reaction solution was reacted for 4 h under N₂ at rt. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow oil, 50 mg, yield 45.5%).

### Step C: Ethyl 1-(bicyclo[1.1.1]pentan-1-yl)-4-((diphenylmethylene)amino)-1H-pyrazole-3-carboxylate

At rt, to a solution of ethyl 1-(bicyclo[1.1.1]pentan-1-yl)-4-bromo-1H-pyrazole-3-carboxylate (50 mg, 0.18 mmol, 1.0 eq) in 1,4-dioxane (2.0 mL) were added benzophenone imine (36 mg, 0.2 mmol, 1.1 eq), Pd₂(dba)₃ (9 mg, 0.01 mmol, 0.1 eq), Xant-phos (6 mg, 0.01 mmol, 0.2 eq), and cesium carbonate (174 mg, 0.5 mmol, 3.0 eq). The reaction solution was reacted at 100°C under N₂ for 3 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow oil, 35 mg, yield 52.4%).

### Step D: Ethyl 4-amino-1-(bicyclo[1.1.1]pentan-1-yl)-1H-pyrazole-3-carboxylate

At rt, to a solution of ethyl 1-(bicyclo[1.1.1]pentan-1-yl)-4-((diphenylmethylene)amino)-1H-pyrazole-3-carboxylate (35 mg, 0.09 mmol, 1.0 eq) in 1,4-dioxane (1.0 mL) was added concentrated HCl (0.25 mL). The mixture was reacted for 1 h under N₂ at rt. Upon completion, the mixture was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 18 mg, yield 90.6%).

### Step E: 6-(Trifluoromethyl)picolinoyl chloride

Thionyl chloride (1.0 mL) was added to 6-(trifluoromethyl)picolinic acid (50 mg, 0.26 mmol, 1.0 eq) at rt, The reaction solution was refluxed at 60°C under N₂ for 2 h. Upon completion, the reaction solution was concentrated under reduced pressure to afford the title compound (yellow oil, 50 mg, crude).

### Step F: Ethyl 1-(bicyclo[1.1.1]pentan-1-yl)-4-(6-(trifluoromethyl)picolinamido)-1H-pyrazole-3-carboxylate

At 0°C, TEA (25 mg, 0.24 mmol, 3.0 eq) was added to a solution of ethyl 4-amino-1-(bicyclo[1.1.1]pentan-1-yl)-1H-pyrazole-3-carboxylate (18 mg, 0.08 mmol, 1.0 eq) and 6-(trifluoromethyl)picolinoyl chloride (50 mg, 0.24 mmol, 1.5 eq) in DCM (3.0 mL). The reaction solution was reacted for 1 h under N₂ at rt. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by column chromatography to afford the title compound (white solid, 15 mg, yield 46.8%).

### Step G: 1-(Bicyclo[1.1.1]pentan-1-yl)-4-(6-(trifluoromethyl)picolinamido)-1H-pyrazole-3-carboxylic acid

Lithium hydroxide (8 mg, 0.2 mmol, 5.0 eq) was added to a mixed solution of ethyl 1-(bicyclo[1.1.1]pentan-1-yl)-4-(6-(trifluoromethyl)picolinamido)-1H-pyrazole-3-carboxylate (15 mg, 0.04 mmol, 1.0 eq) in MeOH (2.0 mL) and water (0.5 mL) at rt. The reaction solution was reacted at 40°C under N₂ for 1 h. Upon completion, the reaction solution was poured into water, adjusted to pH 3-4 with 1 M HCl, and extracted with DCM (×3). The combined organic phases were washed with saturated aqueous NaCl, dried over anhydrous Na₂SO₄, filtered, and concentrated under reduced pressure to afford the title compound (white solid, 15 mg, crude).

### Step H: N-(1-(Bicyclo[1.1.1]pentan-1-yl)-3-formamido-1H-pyrazol-4-yl)-6-(trifluoromethyl)picolinamide

At rt, to a solution of 1-(bicyclo[1.1.1]pentan-1-yl)-4-(6-(trifluoromethyl)picolinamido)-1H-pyrazole-3-carboxylic acid (15 mg, crude) in DMF (2.0 mL) were added ammonium chloride (3 mg, 0.08 mmol, 1.1 eq), DIEA (10.5 mg, 0.12 mmol, 3.0 eq), and HATU (46.5 mg, 0.12 mmol, 2 eq). The reaction solution was reacted for 1 h under N₂ at rt. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (white solid, 3.3 mg, yield 22.6%). **¹H NMR** (400 MHz, MeOD) δ 8.48-8.38 (m, 2H), 8.30 (t, *J =* 7.8 Hz, 1H), 8.05 (d, *J* = 7.8 Hz, 1H), 2.69 (s, 1H), 2.39 (s, 6H).

### Preparation Example 134. Synthesis of Compound A259

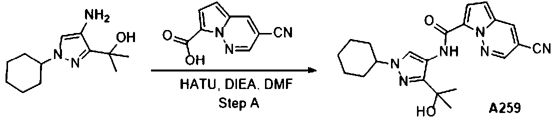

### Step A: 3-cyano-N-(1-cyclohexyl-3-(2-hydroxypropan-2-yl)-1H-pyrazol-4-yl)pyrrolo[1,2-b]pyridazine-7-carboxamide

At rt, to a solution of 2-(4-amino-1-cyclohexyl-1H-pyrazol-3-yl)propan-2-ol (30 mg, 0.13 mmol, 1.0 eq), DIEA (43 mg, 0.34 mmol, 2.5 eq), and 3-cyanopyrrolo[1,2-b]pyridazine-7-carboxylic acid (27 mg, 0.15 mmol, 1.1 eq) in DMF (2.0 mL) was added HATU (56 mg, 0.15 mmol, 1.1 eq). The resulting mixture was stirred at rt for 1 h. The mixture was diluted in water and extracted with EA (×2). The combined organic phases were washed with aqueous NaCl, dried over anhydrous Na₂SO₄, filtered, and concentrated to dryness. The residue was purified by preparative HPLC to afford the title compound (yellow solid, 16.3 mg, yield 30.92%). **¹H NMR** (400 MHz, DMSO) δ 11.73 (s, 1H), 8.94 (d, *J =* 2.2 Hz, 1H), 8.71 (d, *J =* 2.2 Hz, 1H), 8.25 (s, 1H), 7.70 (d, *J* = 4.8 Hz, 1H), 7.08 (d, *J* = 4.8 Hz, 1H), 5.52 (s, 1H), 4.14-3.99 (m, 1H), 2.05-1.95 (m, 2H), 1.87-1.75 (m, 2H), 1.72-1.60 (m, 3H), 1.53 (s, 6H), 1.46-1.32 (m, 2H), 1.30-1.17 (m, 1H).

### Preparation Example 135. Synthesis of Compound A260

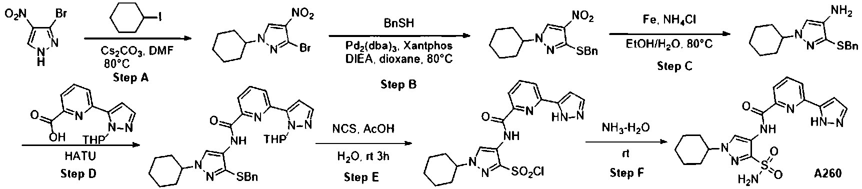

### Step A: 3-Bromo-1-cyclohexyl-4-nitro-1H-pyrazole

At rt, to a solution of 3-bromo-4-nitro-1H-pyrazole (900 mg, 4.7 mmol, 1.0 eq) and iodocyclohexane (1.97 g, 9.4 mmol, 2.0 eq) in DMF (2.0 mL) was added cesium carbonate (4.6 g, 14.1 mmol, 3.0 eq). The reaction solution was reacted at 80°C for 16 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 400 mg, yield 31%).

### Step B: 3-(benzylthio)-1-cyclohexyl-4-nitro-1H-pyrazole carboxylate

At rt, to a solution of 3-bromo-1-cyclohexyl-4-nitro-1H-pyrazole (400 mg, 1.5 mmol, 1.0 eq) and benzyl mercaptan (272 mg, 2.19 mmol, 1.5 eq) in 1,4-dioxane (8.00 mL) were added Pd₂(dba)₃ (134 mg, 0.15 mmol, 0.1 eq), Xantphos (169 mg, 0.3 mmol, 0.2 eq) and DIEA (566 mg, 4.4 mmol, 3.0 eq). The reaction solution was reacted at 80°C for 16 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 330 mg, yield 72%).

### Step C: 3-(benzylthio)-1-cyclohexyl-1H-pyrazole-4-amine

At rt, iron powder (349 mg, 6.3 mmol, 6.0 eq) and ammonium chloride (557 mg, 11.1 mmol, 10.0 eq) were added to a mixed solution of 3-(benzylthio)-1-cyclohexyl-4-nitro-1H-pyrazole carboxylate (330 mg, 1.1 mmol, 1.0 eq) in ethanol (5.0 mL) and water (1.0 ml). The reaction solution was reacted at 80°C for 2 h. Upon completion, the reaction solution was cooled to rt and then filtered. The filtrate was poured into water and extracted with EtOAc (×3). The combined organic phases were washed with saturated aqueous NaCl, dried over anhydrous Na₂SO₄, filtered, and concentrated under reduced pressure to afford the title compound (brown solid, 160 mg, yield 54%).

### Step D: N-(3-(Benzylthio)-1-cyclohexyl-1H-pyrazol-4-yl)-6-(1-(tetrahydro-2H-pyrazol-2-yl)-1H-pyrazin-5-yl)picolinamide

At rt, to a solution of 3-(benzylthio)-1-cyclohexyl-1H-pyrazol-4-amine (160 mg, 0.56 mmol, 1.0 eq) and 6-(1-(tetrahydro-2H-pyran-2-yl)-1H-pyrazol-5-yl)picolinic acid (305 mg, 1.1 mmol, 2.0 eq) in DMF (5.0 ml) were added HATU (424 mg, 1.1 mmol, 2.0 eq) and DIEA (216 mg, 1.67 mmol, 3.0 eq). The reaction solution was reacted at rt for 16 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (brown solid, 130 mg, yield 43%).

### Step E: 4-(6-(1H-Pyrazol-5-yl)picolinamido)-1-cyclohexyl-1H-pyrazole-3-sulfonyl chloride

At rt, to a mixture of N-(3-(benzylthio)-1-cyclohexyl-1H-pyrazol-4-yl)-6-(1-(tetrahydro-2H-pyrazol-2-yl)-1H-pyrazin-5-yl)picolinamide (130 mg, 0.24 mmol, 1.0 eq) in AcOH (1.0 ml) and water (0.3 ml) was added N-chlorosuccinimide (96 mg, 0.72 mmol, 3.0 eq). The reaction solution was reacted at rt for 5 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure to afford the title compound (brown solid, 80 mg, crude).

### Step F: N-(1-Cyclohexyl-3-sulfamoyl-1H-pyrazol-4-yl)-6-(1H-pyrazol-5-yl)picolinamide

At rt, to a solution of 4-(6-(1H-pyrazol-5-yl)picolinamido)-1-cyclohexyl-1H-pyrazole-3-sulfonyl chloride (80 mg, crude) in THF (1.0 ml) was added aqueous ammonia (0.5 mL). The reaction solution was reacted at rt for 10 min. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (brown solid, 19.0 mg, yield 25%). **¹H NMR** (400 MHz, DMSO-*d₆*) δ 13.19 (s, 1H), 11.24 (s, 1H), 8.52 (s, 1H), 8.21 (d, *J =* 7.8 Hz, 1H), 8.10 (t, *J* = 7.7 Hz, 1H), 8.03 (d, *J* = 7.7 Hz, 1H), 7.92 (s, 1H), 7.78 (s, 2H), 7.19 (d, *J =* 2.0 Hz, 1H), 4.37-4.25 (m, 1H), 2.05 (d, *J* = 12.3 Hz, 2H), 1.86-1.64 (m, 5H), 1.42 (q, *J =* 12.8 Hz, 2H), 1.24 (dd, *J =* 8.8, 3.6 Hz, 1H). LCMS (ESI): m/z 416 [M+H]⁺.

### Preparation Example 136. Synthesis of Compound A261

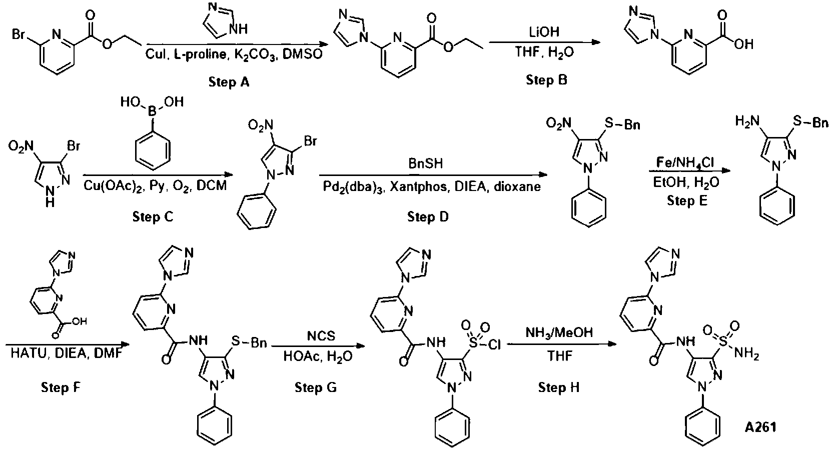

### Step A: Ethyl 6-(1H-imidazol-1-yl)picolinate

At rt, to a solution of ethyl 6-bromopyridine-2-carboxylate (2.50 g, 10.86 mmol, 1.0 eq) and imidazole (1.11 g, 16.30 mmol, 1.5 eq) in dimethyl sulfoxide (25.0 mL) was added cuprous iodide (0.62 g, 3.26 mmol, 0.3 eq), L-proline (0.50 g, 4.34 mmol, 0.4 eq) and potassium carbonate (3.00 g, 21.73 mmol, 2.0 eq). The reaction solution was reacted under N₂ at 100°C for 16 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow oil, 1.4 g, yield 59.3%).

### Step B: 6-(1H-imidazol-1-yl)picolinic acid

At rt, to a mixed solution of ethyl 6-(1H-imidazol-1-yl)picolinate (1.40 g, 6.44 mmol, 1.0 eq) in THF (20.0 mL) and MeOH (10.0 mL) was added lithium hydroxide (0.23 g, 9.66 mmol, 1.5 eq). The reaction solution was reacted at rt for 16 h. Upon completion, the reaction solution was adjusted to pH 6 with 2 N HCl and concentrated under reduced pressure to afford the title compound (yellow oil, 1.0 g, crude).

### Step C: 3-Bromo-4-nitro-1-phenyl-1H-pyrazole

At rt, to a solution of 3-bromo-4-nitro-1H-pyrazole (2.50 g, 13.02 mmol, 1.0 eq) and phenylboronic acid (3.18 g, 26.04 mmol, 2.0 eq) in DCM (50.0 mL) was added copper acetate (3.53 g, 19.53 mmol, 1.5 eq) and pyridine (2.10 mL, 26.04 mmol, 2.0 eq). The reaction solution was reacted under O₂ at rt for 48 h. Upon completion, the reaction solution was filtered and concentrated under reduced pressure. The residue was purified by column chromatography to afford the title compound (white solid, 2.8 g, yield 80.2%).

### Step D: 3-(benzylthio)-4-nitro-1-phenyl-1H-pyrazole

At rt, to a solution of 3-bromo-4-nitro-1-phenyl-1H-pyrazole (1.0 g, 3.73 mmol, 1.0 eq) and benzyl mercaptan (0.69 g, 5.59 mmol, 1.5 eq) in 1,4-dioxane (30.0 mL) was added Xantphos (0.22 g, 0.37 mmol, 0.1 eq), Pd₂(dba)₃ (0.17 g, 0.18 mmol, 0.05 eq) and DIEA (0.96 g, 7.46 mmol, 2.0 eq). The reaction solution was reacted under N₂ at 100°C for 16 h. Upon completion, the reaction solution was cooled to rt and concentrated under reduced pressure. The residue was purified by column chromatography to afford the title compound (orange solid, 1.1 g, yield 94.7%).

### Step E: 3-(benzylthio)-1-phenyl-1H-pyrazol-4-amine

At rt, to a mixed solution of 3-(benzylthio)-4-nitro-1-phenyl-1H-pyrazole (500 mg, 1.60 mmol, 1.0 eq) in ethanol (30.0 mL) and water (2.0 mL) was added iron powder (448.36 mg, 8.03 mmol, 5.0 eq) and ammonium chloride (858.97 mg, 16.06 mmol, 10.0 eq). The reaction solution was reacted at 80°C for 3 h. Upon completion, the reaction solution was cooled to rt, and then filtered. The filtrate was concentrated under reduced pressure. The residue was purified by column chromatography to afford the title compound (brown solid, 300 mg, yield 66.4%).

### Step F: N-(3-(Benzylthio)-1-phenyl-1H-pyrazol-4-yl)-6-(1H-imidazol-1-yl)picolinamide

At rt, to a solution of 3-(benzylthio)-1-phenyl-1H-pyrazol-4-amine (300 mg, 1.06 mmol, 1.0 eq) and 6-(1H-imidazol-1-yl)picolinic acid (125 mg, crude) in DMF (10.0 mL) were added DIEA (551.21 mg, 4.26 mmol, 2.0 eq) and HATU (486.48 mg, 1.28 mmol, 1.2 eq). The mixture was reacted at rt for 1 h. Upon completion, the mixture was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (gray solid, 470 mg, yield 97.4%).

### Step G: 4-(6-(1H-Imidazol-1-yl)picolinamido)-1-phenyl-1H-pyrazole-3-sulfonyl chloride

At 0°C, to *N*-(3-(benzylthio)-1-phenyl-1H-pyrazol-4-yl)-6-(1H-imidazol-1-yl)picolinamide (120 mg, 0.27 mmol, 1.0 eq) was added AcOH (1.0 mL). Then, water (0.1 mL) and N-chlorosuccinimide (141.63 mg, 1.06 mmol, 4.0 eq) were added under ice bath conditions. The reaction solution was reacted at 0°C for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure to afford the title compound (gray solid, 110 mg, crude).

### Step H: 6-(1H-Imidazol-1-yl)-N-(1-phenyl-3-sulfamoyl-1H-pyrazol-4-yl)picolinamide

At 0°C, to a solution of 4-(6-(1H-imidazol-1-yl)picolinamido)-1-phenyl-1H-pyrazole-3-sulfonyl chloride (110 mg, crude) in THF (1.0 mL) was added ammonia in MeOH (0.29 mL, 7 M in MeOH, 8.0 eq). The reaction solution was reacted at 0°C for 2 h. Upon completion, the reaction solution was concentrated under reduced pressure. The residue was purified by HPLC to afford the title compound (off-white solid, 38.0 mg, yield 35.7%). **¹H NMR** (400 MHz, DMSO-*d₆*) δ 11.08 (s, 1H), 9.08 (s, 1H), 8.78 (s, 1H), 8.31 (t, *J* = 7.8 Hz, 1H), 8.18 (d, *J* = 9.0 Hz, 2H), 8.14-8.04 (m, 3H), 7.93 (d, *J =* 7.9 Hz, 2H), 7.59 (t, *J* = 7.8 Hz, 2H), 7.44 (t, *J =* 7.3 Hz, 1H), 7.25 (s, 1H). LCMS (ESI): m/z 410 (M+H)⁺.

### Preparation Example 137. Synthesis of Compound A262

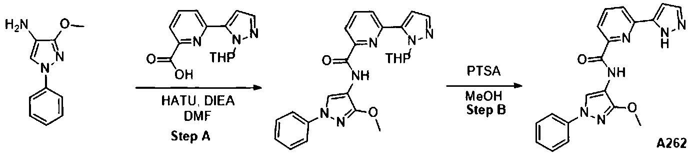

### Step A: N-((3-methoxy-1-phenyl-1H-pyrazol-4-yl)methyl)-6-(1-(tetrahydro-2H-pyrazol-2-yl)-1H-pyrazin-4-yl)picolinamide

At 0°C, to a solution of (3-methoxy-1-phenyl-1H-pyrazol-4-yl)methanamine (50 mg, 0.2 mmol, 1.5 eq) and 6-(1-(tetrahydro-2H-pyran-2-yl)-1H-pyrazol-4-yl)picolinic acid (100 mg, 0.4 mmol, 1.0 eq) in DMF (10.0 mL) were added DIEA (95 mg, 0.7 mmol, 3.0 eq) and HATU (140 mg, 0.4 mmol, 1.2 eq). The reaction solution was reacted at rt for 2 h. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by column chromatography to afford the title compound (yellow solid, 50 mg, yield 44.3%).

### Step B: N-(3-methoxy-1-phenyl-1H-pyrazol-4-yl)-6-(1H-pyrazole-4-carbonyl)picolinamide

At rt, to a solution of N-((3-methoxy-1-phenyl-1H-pyrazol-4-yl)methyl)-6-(1-(tetrahydro-2H-pyrazol-2-yl)-1H-pyrazin-4-yl)picolinamide (200 mg, 0.4 mmol, 1.0 eq) in MeOH (3.0 mL) was added p-toluenesulfonic acid (50 mg, 0.3 mmol, 1.2 eq). The reaction solution was reacted at rt for 1 h under N₂. Upon completion, the reaction solution was filtered, and the filtrate was concentrated under reduced pressure. The residue was purified by HPLC to afford the title compound (yellow solid, 50.0 mg, yield 47.8%). **¹H NMR** (400 MHz, DMSO-*d₆*) δ 13.62 (d, *J =* 343.7 Hz, 1H), 10.23 (d, *J =* 212.0 Hz, 1H), 8.69 (s, 1H), 8.05 (t, *J =* 13.9 Hz, 3H), 7.74 (t, *J* = 28.8 Hz, 3H), 7.48 (t, *J =* 8.0 Hz, 2H), 7.24 (t, *J =* 7.4 Hz, 2H), 4.03 (s, 3H). LC/MS (ESI) m/z: 361 [M+H]⁺.

### Preparation Example 138. Synthesis of Compound A263

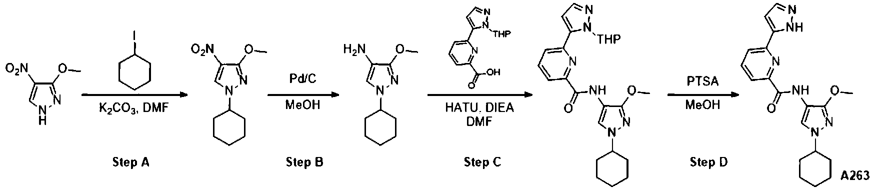

### Step A: 1-Cyclohexyl-3-methoxy-4-nitro-1H-pyrazole

At rt, to a solution of 3-methoxy-4-nitro-1H-pyrazole (150 mg, 1.0 mmol, 1.0 eq) and iodocyclohexane (440 mg, 2.1 mmol, 2 eq) in DMF (25.0 mL) was added potassium carbonate (434 mg, 3.1 mmol, 3 eq). The reaction solution was reacted at 80°C for 12 h. Upon completion, the reaction solution was cooled to rt, poured into ice water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (brown solid, 150 mg, yield 63.5%).

### Step B: 1-Cyclohexyl-3-methoxy-1H-pyrazol-4-amine

At rt, to a solution of 1-cyclohexyl-3-methoxy-4-nitro-1H-pyrazole (150 mg, 0.6 mmol, 1.0 eq) in MeOH (10.0 mL) was added palladium on carbon (21 mg, 10%, 0.2 eq). The mixture was purged with H₂ (×3), and then reacted at rt for 16 h. Upon completion, the reaction solution was filtered, and the filtrate was concentrated under reduced pressure to afford the title compound (yellow oil, 100 mg, yield 76.9%).

### Step C: N-(1-Cyclohexyl-3-methoxy-1H-pyrazol-4-yl)-6-(1-(tetrahydro-2H-pyrazol-2-yl)-1H-pyrazin-5-yl)picolinamide

At rt, to a solution of 1-cyclohexyl-3-methoxy-1H-pyrazol-4-amine (60 mg, 0.3 mmol, 1.0 eq) and 6-(1-(tetrahydro-2H-pyran-2-yl)-1H-pyrazol-5-yl)picolinic acid (84 mg, 0.3 mmol, 1.2 eq) in DMF (10.0 mL) were added DIEA (119 mg, 0.9 mmol, 3.0 eq) and HATU (175 mg, 0.45 mmol, 1.5 eq). The reaction solution was reacted at rt for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 100 mg, yield 72.2%).

### Step D: N-(1-Cyclohexyl-3-methoxy-1H-pyrazol-4-yl)-6-(1H-pyrazol-5-yl)picolinamide

At rt, to a solution of *N*-(1-cyclohexyl-3-methoxy-1H-pyrazol-4-yl)-6-(1-(tetrahydro-2H-pyrazol-2-yl)-1H-pyrazin-5-yl)picolinamide (60 mg, 0.1 mmol, 1.0 eq) in MeOH (10.0 mL) was added p-toluenesulfonic acid (60 mg, 0.3 mmol, 3 eq). The mixture was reacted at rt for 16 h. Upon completion, the mixture was concentrated under reduced pressure. The residue was purified by HPLC to afford the title compound (yellow solid, 15.0 mg, yield 30.7%). **¹H NMR** (400 MHz, DMSO-*d₆*) δ 13.58 (d, *J =* 329.4 Hz, 1H), 10.01 (d, *J =* 209.9 Hz, 1H), 8.20-8.01 (m, 2H), 7.98 (d, *J* = 7.4 Hz, 1H), 7.96-7.59 (m, 2H), 7.08 (d, *J* = 39.5 Hz, 1H), 4.03-3.91 (m, 1H), 3.87 (d, *J =* 8.4 Hz, 3H), 2.00 (d, *J* = 12.0 Hz, 2H), 1.81 (d, *J =* 13.0 Hz, 2H), 1.74-1.59 (m, 3H), 1.39 (q, *J =* 12.9 Hz, 2H), 1.30-1.16 (m, 1H). LC/MS (ESI) m/z: 367 [M+H]⁺.

### Preparation Example 139. Synthesis of Compound A264

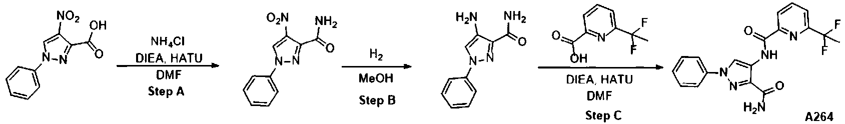

### Step A: 4-nitro-1-phenyl-1H-pyrazole-3-carboxamide

At rt, to a solution of 4-nitro-1-phenyl-1H-pyrazole-3-carboxylic acid (200 mg, 0.9 mmol, 1.0 eq) and ammonium chloride (55 mg, 1.1 mmol, 1.5 eq) in DMF (4.0 mL) were added DIEA (332 mg, 2.5 mmol, 3.0 eq) and HATU (489 mg, 1.3 mmol, 1.2 eq). The reaction solution was reacted at rt for 2 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 150 mg, yield 75.3%).

### Step B: 4-Amino-1-phenyl-1H-pyrazole-3-carboxamide

At rt, to a solution of 4-nitro-1-phenyl-1H-pyrazole-3-carboxamide (150 mg, 0.7 mmol, 1.0 eq) in MeOH (8.0 mL) was added palladium on carbon (80 mg, 0.8 mmol, 10% purity, 1.1 eq). The reaction solution was purged with H₂ (×3), and then stirred at rt for 1 h. Upon completion, the reaction solution was filtered, and the filtrate was concentrated under reduced pressure to afford the title compound (yellow solid, 100 mg, yield 76.5%).

### Step C: N-(3-amino-1-phenyl-1H-pyrazol-4-yl)-6-(1,1-difluoroethyl)picolinamide

At rt, to a solution of 4-amino-N-cyclopropyl-1-phenylpyrazole-3-carboxamide (97 mg, 0.4 mmol, 1.5 eq) and 6-(1,1-difluoroethyl)pyridine-2-carboxylic acid (50 mg, 0.3 mmol, 1.0 eq) in DMF (3.0 mL) were added DIEA (104 mg, 0.8 mmol, 3.0 eq) and HATU (122 mg, 0.3 mmol, 1.2 eq). The reaction solution was reacted at rt for 2 h. Upon completion, the mixture was poured into water and extracted with EtOAc (×3). The combined organic phases were washed with saturated aqueous NaCl, dried over anhydrous Na₂SO₄, filtered, and concentrated under reduced pressure. The residue was purified by prep-HPLC to afford the title compound (white solid, 50 mg, yield 45.5%). **¹H NMR** (400 MHz, DMSO) δ 11.98 (s, 1H), 9.01 (s, 1H), 8.30 (dd, *J* = 8.0*,* 5.5 Hz, 2H), 8.12-7.95 (m, 4H), 7.85 (s, 1H), 7.56 (t, *J =* 8.0 Hz, 2H), 7.40 (t, *J* = 7.4 Hz, 1H), 2.23 (t, *J =* 19.3 Hz, 3H).

### Preparation Example 140. Synthesis of Compound A265

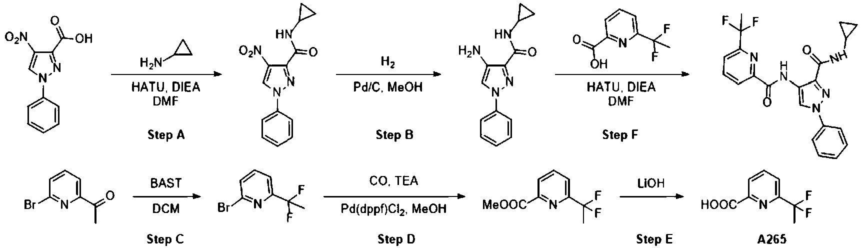

### Step A: N-cyclopropyl-4-nitro-1-phenyl-1H-pyrazole-3-carboxamide

At 0°C, to a solution of 4-nitro-1-phenyl-1H-pyrazole-3-carboxylic acid (150 mg, 0.7 mmol, 1.0 eq) and cyclopropylamine (55 mg, 1.0 mmol, 1.5 eq) in DMF (5.0 mL) were added DIEA (249 mg, 1.9 mmol, 3.0 eq) and HATU (367 mg, 1.0 mmol, 1.5 eq). The reaction solution was reacted at rt for 2 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (brown solid, 100 mg, yield 57.1%).

### Step B: 4-amino-N-cyclopropyl-1-phenyl-1H-pyrazole-3-carboxamide

At 0°C, to a solution of *N*-cyclopropyl-4-nitro-1-phenyl-1H-pyrazole-3-carboxamide (100 mg, 0.4 mmol, 1.0 eq) in MeOH (4.0 mL) was added palladium on carbon (60 mg, 0.6 mmol, 10%, 1.2 eq). The reaction solution was purged with H₂ (×3), and then stirred at rt for 1 h. Upon completion, the reaction solution was filtered, and the filtrate was concentrated under reduced pressure to afford the title compound (white solid, 50 mg, yield 56.2%).

### Step C: 2-bromo-6-(1,1-difluoroethyl)pyridine

At 0°C, to a solution of 1-(6-bromopyridin-2-yl)ethan-1-one (2.0 g, 10.0 mmol, 1.0 eq) in DCM (25.0 mL) was added bis(2-methoxyethyl)aminosulfur trifluoride (18.4 mL, 100.0 mmol, 10.0 eq). The mixture was purged with N₂ (×3), and then reacted at rt for 16 h. Upon completion, the mixture was poured into aqueous NaHCO₃, and extracted with DCM (×3). The combined organic phases were washed with saturated aqueous NaCl, dried over anhydrous Na₂SO₄, filtered, and concentrated under reduced pressure. The residue was purified by column chromatography to afford the title compound (colorless oil, 1.5 g, yield 67%).

### Step D: Methyl 6-(1,1-difluoroethyl)picolinate

At 0°C, to a solution of 2-bromo-6-(1,1-difluoroethyl)pyridine (1.5 g, 6.8 mmol, 1.0 eq) in MeOH (25.0 mL) were added Pd(dppf)Cl₂ (490 mg, 0.7 mmol, 0.1 eq) and TEA (1.9 mL, 13.5 mmol, 2.0 eq). The reaction solution was reacted under a carbon monoxide atmosphere at 80°C for 16 h. Upon completion, the reaction solution was cooled to rt and concentrated under reduced pressure. The residue was purified by column chromatography to afford the title compound (colorless oil, 150 mg, yield 11%).

### Step E: 6-(1,1-difluoroethyl)picolinic acid

At rt, to a mixed solution of methyl 6-(1,1-difluoroethyl)picolinate (150 mg, 0.7 mmol, 1.0 eq) in THF (4.0 mL) and water (1.0 mL) was added lithium hydroxide (36 mg, 1.5 mmol, 2.0 eq). The reaction solution was reacted at rt for 2 h. Upon completion, the reaction solution was adjusted to pH 4-5 with 1 N HCl, and concentrated under reduced pressure to afford the title compound (white solid, 140 mg, crude).

### Step F: N-(3-(Cyclopropylcarbamoyl)-1-phenyl-1H-pyrazol-4-yl)-6-(1,1-difluoroethyl)picolinamide

At 0°C, to a solution of 4-amino-N-cyclopropyl-1-phenyl-1H-pyrazole-3-carboxamide (97 mg, 0.4 mmol, 1.5 eq) and 6-(1,1-difluoroethyl)picolinic acid (50 mg, crude) in DMF (3.0 mL) were added DIEA (104 mg, 0.8 mmol, 3.0 eq) and HATU (122 mg, 0.3 mmol, 1.5 eq). The reaction solution was reacted at rt for 2 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (white solid, 50.0 mg, yield 45.5%). **¹H NMR** (400 MHz, DMSO-*d₆*) δ 11.82 (s, 1H), 9.03 (s, 1H), 8.60 (d, *J* = 4.8 Hz, 1H), 8.34-8.27 (m, 2H), 8.07-7.98 (m, 3H), 7.56 (t, *J =* 8.0 Hz, 2H), 7.40 (t, *J =* 7.4 Hz, 1H), 3.05-2.93 (m, 1H), 2.25 (t, *J =* 19.3 Hz, 3H), 0.79-0.65 (m, 4H). LCMS (ESI): m/z 412 [M+H]⁺.

### Preparation Example 141. Synthesis of Compound A266

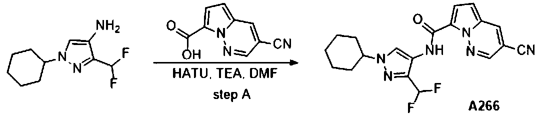

### Step A: 3-cyano-N-(1-cyclohexyl-3-(difluoromethyl)-1H-pyrazol-4-yl)pyrrolo[1,2-b]pyridazine-7-carboxamide

At rt, to a solution of 1-cyclohexyl-3-(difluoromethyl)-1H-pyrazol-4-amine (80.0 mg, 0.4 mmol, 1.0 eq) and 3-cyanopyrrolo[1,2-b]pyridazine-7-carboxylic acid (76.5 mg, 0.4 mmol, 1.1 eq) in DMF (2 mL) were added HATU (211.9 mg, 0.6 mmol, 1.5 eq) and TEA (75.1 mg, 0.7 mmol, 2.0 eq). The reaction solution was stirred at rt under N₂ for 1 h. Upon completion, the reaction solution was poured into water (20 mL) and extracted with EtOAc (20 mL×3). The combined organic phases were washed sequentially with water and saturated aqueous NaCl, dried over anhydrous Na₂SO₄, and concentrated under reduced pressure. The residue was purified by column chromatography, followed by HPLC, to afford the title compound (white solid, 82.1 mg, yield 57%). **¹H NMR** (400 MHz, DMSO-*d₆*) δ 10.90 (s, 1H), 8.99 (d, *J =* 2.1 Hz, 1H), 8.85 (d, *J* = 2.2 Hz, 1H), 8.41 (s, 1H), 7.75 (d, *J* = 4.8 Hz, 1H), 7.38-7.02 (m, 2H), 4.31-4.17 (m, 1H), 2.08-1.96 (m, 2H), 1.90-1.57 (m, 5H), 1.48-1.33 (m, 2H), 1.24 (tt, *J* = 12.7, 3.5 Hz, 1H). LCMS (ESI): m/z 385 [M+H]⁺.

### Preparation Example 142. Synthesis of Compound A267

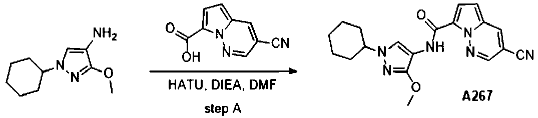

### Step A: N-(1-cyclohexyl-3-methoxy-1H-pyrazol-4-yl)-3-isocyanopyrrolo[1,2-b]pyridazine-7-carboxamide

1-cyclohexyl-3-methoxy-1H-pyrazol-4-amine (55.0 mg, 0.28 mmol, 1.0 eq) was dissolved in anhydrous DMF (2 mL), and DIEA (108.4 mg, 0.84 mmol, 3.0 eq) was added. HATU (159.6 mg, 0.42 mmol, 1.5 eq) and 3-cyanopyrrolo[1,2-b]pyridazine-7-carboxylic acid (67.5 mg, 0.36 mmol, 1.2 eq) were dissolved in anhydrous DMF (2 mL). The two mixtures were stirred separately under N₂ for 30 min, and then combined. The reaction solution was stirred at rt under N₂ for 16 h. The reaction solution was diluted in water (20 mL) and extracted with EtOAc (20 mL×3). The combined organic phases were washed with aqueous NaCl, dried over anhydrous Na₂SO₄, filtered, and concentrated under reduced pressure. The crude was purified by HPLC to afford the title compound (55.7 mg, yield 54.7%) as a yellow solid. **¹H NMR** (400 MHz, DMSO) δ 10.23 (s, 1H), 8.96 (d, *J =* 2.1 Hz, 1H), 8.88 (d, *J* = 2.1 Hz, 1H), 8.02 (s, 1H), 7.70 (d, *J =* 4.8 Hz, 1H), 7.09 (d, *J =* 4.8 Hz, 1H), 4.00-3.93 (m, 1H), 3.91 (s, 3H), 1.99 (d, *J =* 10.3 Hz, 2H), 1.80 (d, *J* = 13.3 Hz, 2H), 1.72-1.61 (m, 3H), 1.45-1.33 (m, 2H), 1.27-1.18 (m, 1H). LCMS (ESI): m/z 365 [M+H]⁺.

### Preparation Example 143. Synthesis of Compound A268

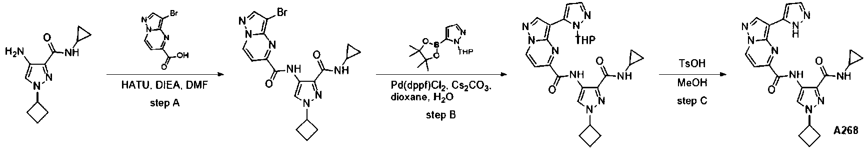

### Step A: 3-bromo-N-(1-cyclobutyl-3-(cyclopropylcarbamoyl)-1H-pyrazol-4-yl)pyrazolo[1,5-a]pyrimidine-5-carboxamide

At rt, to a solution of 4-amino-1-cyclobutyl-N-cyclopropyl-1H-pyrazole-3-carboxamide (160 mg, 0.7 mmol, 1.0 eq) and 3-bromopyrazolo[1,5-a]pyrimidine-5-carboxylic acid (175 mg, 0.7 mmol, 1.0 eq) in DMF (20.0 ml) were added HATU (552 mg, 1.4 mmol, 2.0 eq) and DIEA (468 mg, 3.6 mmol, 5.0 eq). The reaction solution was reacted at rt for 16 h. Upon completion, the reaction solution was poured into aqueous NaHCO₃ and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 240 mg, yield 74%).

### Step B: N-(1-cyclobutyl-3-(cyclopropylcarbamoyl)-1H-pyrazol-4-yl)-3-(1-(tetrahydro-2H-pyrazol-2-yl)-1H-pyrazol-5-yl)pyrazolo[1,5-a]pyrimidine-5-carboxamide

At rt, to a solution of 3-bromo-N-(1-cyclobutyl-3-(cyclopropylcarbamoyl)-1H-pyrazol-4-yl)pyrazolo[1,5-a]pyrimidine-5-carboxamide (240 mg, 0.5 mmol, 1.0 eq) and 1-(tetrahydro-2H-pyran-2-yl)-5-(4,4,5,5-tetramethyl-1,3,2-dioxaborolan-2-yl)-1H-pyrazole (300 mg, 1.1 mmol, 2.0 eq) in 1,4-dioxane (20.0 ml and water (4 mL) were added Pd(dppf)Cl₂ (39 mg, 0.1 mmol, 0.1 eq) and cesium carbonate (528 mg, 1.6 mmol, 3.0 eq). The reaction solution was reacted at 90°C for 48 h. Upon completion, the reaction solution was cooled to rt and concentrated under reduced pressure. The residue was purified by column chromatography to afford the title compound (yellow oil, 66 mg, yield 24%).

### Step C: N-(1-cyclobutyl-3-(cyclopropylcarbamoyl)-1H-pyrazol-4-yl)-3-(1H-pyrazol-5-yl)pyrazolo[1,5-a]pyrimidine-5-carboxamide

At rt, to a solution of *N*-(1-cyclobutyl-3-(cyclopropylcarbamoyl)-1H-pyrazol-4-yl)-3-(1-(tetrahydro-2H-pyrazol-2-yl)-1H-pyrazol-5-yl)pyrazolo[1,5-a]pyrimidine-5-carboxamide (66 mg, 0.1 mmol, 1.0 eq) in MeOH (10.0 ml) was added 4-methylbenzenesulfonic acid (66 mg, 0.4 mmol, 4.0 eq). The reaction solution was reacted at rt for 1 h. Upon completion, the reaction solution was poured into aqueous NaHCO₃, and extracted with DCM (×3). The combined organic phases were washed with saturated aqueous NaCl, dried over anhydrous Na₂SO₄, filtered, and concentrated under reduced pressure. The residue was purified by HPLC to afford the title compound (yellow oil, 54.5 mg, yield 98%). **¹H NMR** (400 MHz, DMSO-*d₆*) δ 13.36-12.74 (m, 1H), 12.07 (s, 1H), 9.36 (d, *J* = 7.2 Hz, 1H), 8.77 (s, 1H), 8.48 (d, *J* = 4.7 Hz, 2H), 7.78 (s, 1H), 7.68-7.57 (m, 2H), 5.03-4.91 (m, 1H), 3.06-2.97 (m, 1H), 2.64-2.53 (m, 2H), 2.47-2.39 (m, 2H), 1.88-1.78 (m, 2H), 0.80-0.72 (m, 4H). LCMS (ESI): m/z 432 [M+H]⁺.

### Preparation Example 144. Synthesis of Compound A269

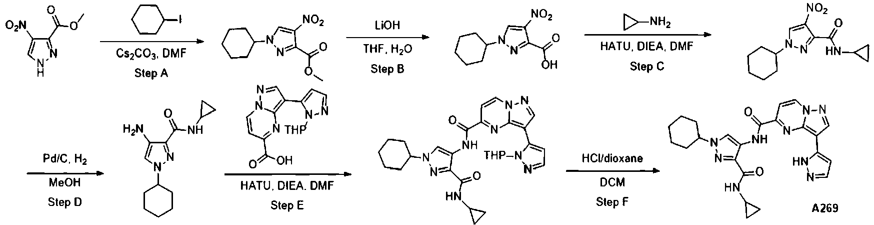

### Step A: Methyl 1-cyclohexyl-4-nitro-1H-pyrazole-3-carboxylate

At rt, to a solution of methyl 4-nitro-1H-pyrazole-3-carboxylate (7 g, 40.9 mmol, 1.0 eq) in DMF (2.0 mL) were added iodocyclohexane (17.2 g, 81.8 mmol, 2.0 eq) and cesium carbonate (26.7 g, 81.8 mmol, 2.0 eq). The reaction solution was reacted at 90°C for 16 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow oil, 2.04 g, yield 19%).

### Step B: 1-Cyclohexyl-4-nitro-1H-pyrazole-3-carboxylic acid

To a mixed solution of methyl 1-cyclohexyl-4-nitro-1H-pyrazole-3-carboxylate (670 mg, 2.6 mmol, 1.0 eq) in THF (15.0 mL) and water (3.0 mL) was added lithium hydroxide (222 mg, 5.2 mmol, 2.0 eq) at rt. The reaction solution was reacted at rt for 16 h. Upon completion, the reaction solution was adjusted to pH 4-5 with 1 N HCl and concentrated under reduced pressure to afford the title compound (white solid, 620 mg, crude).

### Step C: 1-cyclohexyl-N-cyclopropyl-4-nitro-1H-pyrazole-3-carboxamide

To a solution of 1-cyclohexyl-4-nitro-1H-pyrazole-3-carboxylic acid (620 mg, crude) and cyclopropylamine (222 mg, 3.9 mmol, 1.5 eq) in DMF (15.0 mL) were added DIEA (1.4 mL, 7.8 mmol, 3.0 eq) and HATU (1.9 g, 5.2 mmol, 1.0 eq) at rt. The reaction solution was reacted at rt for 4 h. Upon completion, the reaction solution was poured into NaHCO₃ solution and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (colorless oil, 700 mg, yield 97%).

### Step D: 4-amino-1-cyclohexyl-N-cyclopropyl-1H-pyrazole-3-carboxamide

To a solution of 1-cyclohexyl-N-cyclopropyl-4-nitro-1H-pyrazole-3-carboxamide (700 mg, 2.5 mmol, 1.0 eq) in MeOH (50.0 mL) was added palladium on carbon (70 mg, 10%) at rt. The reaction solution was purged with H₂ (×3), and then reacted at rt for 16 h. Upon completion, the reaction solution was filtered and the filtrate was subjected to rotary evaporation to dryness to afford the title compound (brown oil, 560 mg, crude).

### Step E: N-(1-cyclohexyl-3-(cyclopropylcarbamoyl)-1H-pyrazol-4-yl)-3-(1-(tetrahydro-2H-pyrazol-2-yl)-1H-pyrazol-5-yl)pyrazolo[1,5-a]pyrimidine-5-carboxamide

At rt, to a solution of 4-amino-N-cyclopropyl-1-cyclohexyl-1H-pyrazole-3-carboxamide (60 mg, crude) and 3-(1-(tetrahydro-2H-pyran-2-yl)-1H-pyrazol-5-yl)pyrazolo[1,5-a]pyrimidine-5-carboxylic acid (91 mg, 0.3 mmol, 1.2 eq) in DMF (10.0 mL) were added DIEA (94 mg, 0.7 mmol, 3.0 eq) and HATU (184 mg, 0.5 mmol, 2.0 eq). The reaction solution was reacted at rt for 3 h. Upon completion, the reaction solution was poured into NaHCO₃ solution and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow oil, 70 mg, yield 53%).

### Step F: N-(1-cyclohexyl-3-(cyclopropylcarbamoyl)-1H-pyrazol-4-yl)-3-(1H-pyrazol-5-yl)pyrazolo[1,5-a]pyrimidine-5-carboxamide

At rt, to a solution of N-(1-cyclohexyl-3-(cyclopropylcarbamoyl)-1H-pyrazol-4-yl)-3-(1-(tetrahydro-2H-pyrazol-2-yl)-1H-pyrazol-5-yl)pyrazolo[1,5-a]pyrimidine-5-carboxamide (70 mg, 0.1 mmol, 1.0 eq) in DCM (4.0 mL) was added HCl/dioxane (2 mL, 4 M). The reaction solution was reacted at rt for 1 h. Upon completion, the reaction solution was concentrated under reduced pressure. The residue was purified by HPLC to afford the title compound (yellow solid, 21 mg, yield 35%). **¹H NMR** (400 MHz, DMSO-*d*₆) δ 13.07 (d, *J* = 143.2 Hz, 1H), 12.08 (d, *J* = 27.4 Hz, 1H), 9.36 (dd, *J* = 17.4, 7.0 Hz, 1H), 8.78 (d, *J* = 41.3 Hz, 1H), 8.45-8.35 (m, 2H), 7.84 (s, 0.6H), 7.71-7.58 (m, 2H), 7.53 (s, 0.3H), 4.30-4.21 (m, 1H), 3.04-2.96 (m, 1H), 2.10-2.02 (m, 2H), 1.88-1.75 (m, 4H), 1.72-1.64 (m, 1H), 1.47-1.35 (m, 2H), 1.30-1.19 (m, 1H), 0.79-0.69 (m, 4H). LCMS (ESI): m/z 460 [M+H]⁺.

### Preparation Example 145. Synthesis of Compound A270

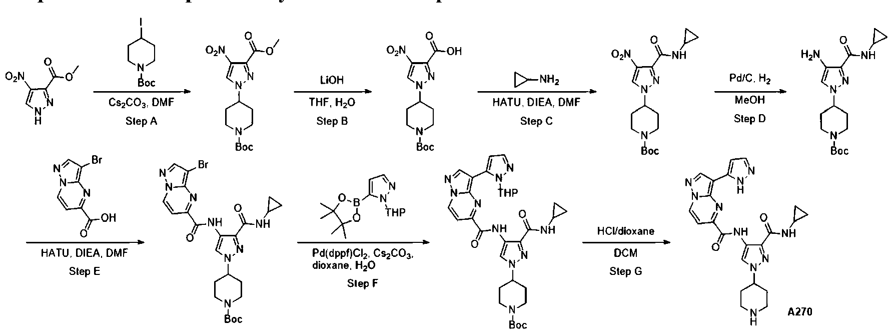

### Step A: Tert-butyl 4-(3-(methoxycarbonyl)-4-nitro-1H-pyrazol-1-yl)piperidine-1-carboxylate

At rt, to a solution of methyl 4-nitro-1H-pyrazole-3-carboxylate (1 g, 5.8 mmol, 1.0 eq) and tert-butyl 4-iodopiperidine-1-carboxylate (2.7 g, 8.8 mmol, 1.5 eq) in DMF (25.0 mL) was added cesium carbonate (3.8 g, 11.7 mmol, 2.0 eq). The reaction solution was reacted at 90°C for 3 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (colorless oil, 350 mg, yield 17%).

### Step B: 1-(1-(tert-butoxycarbonyl)piperidin-4-yl)-4-nitro-1H-pyrazole-3-carboxylic acid

At rt, to a mixed solution of tert-butyl 4-(3-(methoxycarbonyl)-4-nitro-1H-pyrazol-1-yl)piperidine-1-carboxylate (350 mg, 1.0 mmol, 1.0 eq) in THF (15.0 mL) and water (3.0 mL) was added lithium hydroxide (47 mg, 2.0 mmol, 2.0 eq). The reaction solution was reacted at rt for 3 h. Upon completion, the reaction solution was adjusted to pH 6-7 with 1 N HCl and concentrated under reduced pressure to afford the title compound (white solid, 340 mg, crude).

### Step C: Tert-butyl 4-(3-(cyclopropylcarbamoyl)-4-nitro-1H-pyrazol-1-yl)piperidine-1-carboxylate

At rt, to a solution of 1-(1-(tert-butoxycarbonyl)piperidin-4-yl)-4-nitro-1H-pyrazole-3-carboxylic acid (340 mg, crude) and cyclopropylamine (114 mg, 2.0 mmol, 2.0 eq) in DMF (15.0 mL) were added DIEA (0.9 mL, 5.0 mmol, 5.0 eq) and HATU (760 mg, 2.0 mmol, 2.0 eq). The reaction solution was reacted at rt for 16 h. Upon completion, the reaction solution was poured into NaHCO₃ solution and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow oil, 370 mg, yield 98%).

### Step D: Tert-butyl 4-(4-amino-3-(cyclopropylcarbamoyl)-1H-pyrazol-1-yl)piperidine-1-carboxylate

At rt, to a solution of tert-butyl 4-(3-(cyclopropylcarbamoyl)-4-nitro-1H-pyrazol-1-yl)piperidine-1-carboxylate (370 mg, 0.9 mmol, 1.0 eq) in MeOH (50.0 mL) was added palladium on carbon (40 mg, 10%). The reaction solution was purged with H₂ (×3), and then stirred at rt for 16 h. Upon completion, the reaction solution was filtered, and the filtrate was concentrated under reduced pressure to afford the title compound (brown oil, 320 mg, crude).

### Step E: Tert-butyl 4-(4-(3-bromopyrazolo[1,5-a]pyrimidine-5-carboxamido)-3-(cyclopropylcarbamoyl)-1H-pyrazol-1-yl)piperidine-1-carboxylate

At rt, to a solution of tert-butyl 4-(4-amino-3-(cyclopropylcarbamoyl)-1H-pyrazol-1-yl)piperidine-1-carboxylate (120 mg, crude) and 3-bromopyrazolo[1,5-a]pyrimidine-5-carboxylic acid (99 mg, 0.4 mmol, 1.2 eq) in DMF (10.0 mL) were added DIEA (0.2 mL, 1.0 mmol, 3.0 eq) and HATU (260 mg, 0.6 mmol, 2.0 eq). The reaction solution was reacted at rt for 16 h. Upon completion, the reaction solution was poured into NaHCO₃ solution and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 50 mg, yield 25%).

### Step F: Tert-butyl 4-(3-(cyclopropylcarbamoyl)-4-(3-(1-(tetrahydro-2H-pyran-2-yl)-1H-pyrazol-5-yl)pyrazolo[1,5-a]pyrimidine-S-carboxamido)-1H-pyrazol-1-yl)piperidine-1-carboxylate

At rt, to a solution of tert-butyl 4-(4-(3-bromopyrazolo[1,5-a]pyrimidine-5-carboxamido)-3-(cyclopropylcarbamoyl)-1H-pyrazol-1-yl)piperidine-1-carboxylate (50 mg, 0.1 mmol, 1.0 eq) and 1-(tetrahydro-2H-pyran-2-yl)-5-(4,4,5,5-tetramethyl-1,3,2-dioxaborolan-2-yl)-1H-pyrazole (48 mg, 0.2 mmol, 2.0 eq) in 1,4-dioxane (10.0 mL) and water (3.0 mL) were added Pd(dppf)Cl₂ (6 mg, 0.01 mmol, 0.1 eq) and cesium carbonate (85 mg, 0.3 mmol, 3.0 eq). The reaction solution was reacted at 90°C for 48 h. Upon completion, the reaction solution was concentrated under reduced pressure. The residue was purified by column chromatography to afford the title compound (yellow solid, 40 mg, yield 71%).

### Step G: N-(3-(cyclopropylcarbamoyl)-1-(piperidin-4-yl)-1H-pyrazol-4-yl)-3-(1H-pyrazol-5-yl)pyrazolo[1,5-a]pyrimidine-5-carboxamide

At rt, to a solution of tert-butyl 4-(3-(cyclopropylcarbamoyl)-4-(3-(1-(tetrahydro-2H-pyran-2-yl)-1H-pyrazol-5-yl)pyrazolo[1,5-a]pyrimidine-5-carboxamido-1H-pyrazol-1-yl)piperidine-1-carboxylate (50 mg, 0.1 mmol, 1.0 eq) in DCM (4.0 mL) was added HCl/dioxane (2 mL, 4 M). The reaction solution was reacted at rt for 1 h. Upon completion, the reaction solution was concentrated under reduced pressure. The residue was purified by HPLC to afford the title compound (yellow solid, 20.8 mg, yield 58%). **¹H NMR** (400 MHz, DMSO-*d*₆) δ 13.07 (d, *J* = 142.5 Hz, 1H), 12.06 (s, 1H), 9.36 (d, *J* = 7.2 Hz, 1H), 8.75 (s, 1H), 8.42 (s, 2H), 7.74 (d, *J* = 75.4 Hz, 3H), 4.40-4.26 (m, 1H), 3.11-3.04 (m, 2H), 3.03-2.97 (m, 1H), 2.67-2.54 (m, 2H), 2.03-1.96 (m, 2H), 1.92-1.81 (m, 2H), 0.81-0.70 (m, 4H). LCMS (ESI): m/z 461 [M+H]⁺.

### Preparation Example 146. Synthesis of Compound A271

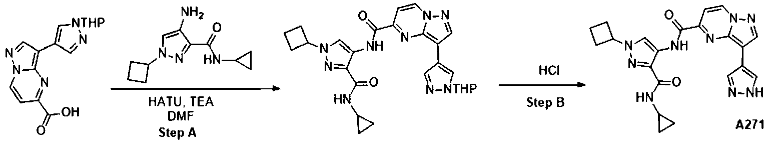

### Step A: N-(1-cyclobutyl-3-(cyclopropylcarbamoyl)-1H-pyrazol-4-yl)-3-(1-(tetrahydro-2H-pyrazol-2-yl)-1H-pyridin-4-yl)pyrazolo[1,5-a]pyrimidine-5-carboxamide

At rt, to a solution of 3-(1-(tetrahydro-2H-pyran-2-yl)-1H-pyrazol-4-yl)pyrazolo[1,5-a]pyrimidine-5-carboxylic acid (100 mg, 0.32 mmol, 1.0 eq) and 4-amino-1-cyclobutyl-N-cyclopropyl-1H-pyrazole-3-carboxamide (71 mg, 0.32 mmol, 1.0 eq) in DMF (5.0 mL) were added TEA (0.13 mL, 0.96 mmol, 3.0 eq) and HATU (242 mg, 0.64 mmol, 2.0 eq). The reaction solution was reacted at rt for 16 h. Upon completion, the reaction solution was poured into aqueous NaCl and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 125 mg, yield 76%).

### Step B: N-(1-cyclobutyl-3-(cyclopropylcarbamoyl)-1H-pyrazol-4-yl)-3-(1H-pyrazol-4-yl)pyrazolo[1,5-a]pyrimidine-5-carboxamide

At rt, to a solution of *N*-(1-cyclobutyl-3-(cyclopropylcarbamoyl)-1H-pyrazol-4-yl)-3-(1-(tetrahydro-2H-pyrazol-2-yl)-1H-pyridin-4-yl)pyrazolo[1,5-a]pyrimidine-5-carboxamide (125 mg, 0.24 mmol, 1.0 eq) in DCM (5.0 mL) was added HCl/dioxane (5 mL, 4 M). The reaction solution was reacted at rt for 1 h. Upon completion, the reaction solution was concentrated under reduced pressure. The residue was purified by HPLC to afford the title compound (yellow solid, 25 mg, yield 23.9%). **¹H NMR** (400 MHz, DMSO-*d*₆) δ 13.05 (s, 1H), 12.12 (s, 1H), 9.28 (d, *J* = 7.2 Hz, 1H), 8.76 (s, 2H), 8.46 (d, *J* = 4.9 Hz, 2H), 8.29 (s, 1H), 7.59 (d, *J* = 7.2 Hz, 1H), 4.96 (t, *J* = 8.3 Hz, 1H), 3.22 (td, *J* = 7.1, 3.0 Hz, 1H), 2.67-2.53 (m, 2H), 2.46-2.38 (m, 2H), 1.82 (qd, *J* = 10.3, 7.0 Hz, 2H), 0.81-0.70 (m, 4H). LCMS (ESI) m/z: 432 [M+H]⁺.

### Preparation Example 147. Synthesis of Compound A272

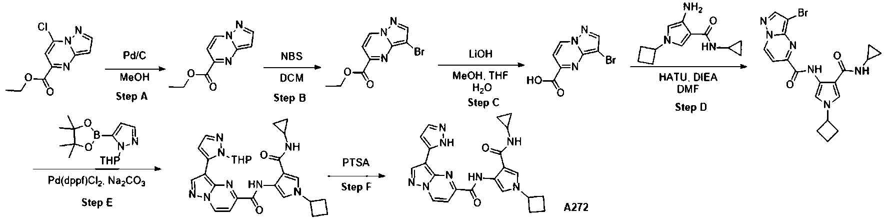

### Step A: Ethyl pyrazolo[1,5-a]pyrimidine-5-carboxylate

At rt, to a solution of ethyl 7-chloropyrazolo[1,5-a]pyrimidine-5-carboxylate (600 mg, 2.7 mmol, 1.0 eq) in MeOH (6.0 mL) was added palladium on carbon (300 mg, 2.8 mmol, 10% purity, 0.2 eq). The reaction solution was purged with H₂ (×3), and then stirred at rt for 2 h. Upon completion, the reaction solution was filtered, and the filtrate was concentrated under reduced pressure to afford the title compound (yellow oil, 460 mg, yield 90%).

### Step B: Ethyl 3-bromopyrazolo[1,5-a]pyrimidine-5-carboxylate

At rt, to a solution of ethyl pyrazolo[1,5-a]pyrimidine-5-carboxylate (500 mg, 2.6 mmol, 1.0 eq) in DCM (5.0 mL) was added NBS (1.4 g, 7.8 mmol, 3.0 eq). The reaction solution was reacted at rt for 2 h. Upon completion, the reaction solution was concentrated under reduced pressure. The residue was purified by column chromatography to afford the title compound (yellow solid, 600 mg, yield 85%).

### Step C: 3-Bromopyrazolo[1,5-a]pyrimidine-5-carboxylic acid

At rt, to a mixed solution of ethyl 3-bromopyrazolo[1,5-a]pyrimidine-5-carboxylate (500 mg, 1.8 mmol, 1.0 eq) in MeOH (3.0 mL), THF (3.0 mL), and water (3.0 mL) was added lithium hydroxide (133 mg, 5.6 mmol, 3.0 eq). The reaction solution was reacted at rt for 2 h. Upon completion, the reaction solution was adjusted to pH 4-5 with 1 N HCl, and extracted with EtOAc (×3). The combined organic phases were washed with saturated aqueous NaCl, dried over anhydrous Na₂SO₄, filtered, and concentrated under reduced pressure to afford the title compound (white solid, 300 mg, yield 67%).

### Step D: 3-bromo-N-(1-cyclobutyl-4-(cyclopropylcarbamoyl)-1H-pyrrol-3-yl)pyrazolo[1,5-a]pyrimidine-5-carboxamide

At rt, to a solution of 3-bromopyrazolo[1,5-a]pyrimidine-5-carboxylic acid (300 mg, 1.2 mmol, 1.0 eq) and 4-amino-1-cyclobutyl-N-cyclopropyl-1H-pyrrole-3-carboxamide (326 mg, 1.5 mmol, 1.2 eq) in DMF (5.0 mL) were added DIEA (480 mg, 3.7 mmol, 3.0 eq) and HATU (707 mg, 1.8 mmol, 1.2 eq). The reaction solution was reacted at rt for 2 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 400 mg, yield 73%).

### Step E: N-(1-cyclobutyl-4-(cyclopropylcarbamoyl)-1H-pyrrol-3-yl)-3-(1-(tetrahydro-2H-pyran-2-yl)-1H-pyrazol-5-yl)pyrazolo[1,5-a]pyrimidine-5-carboxamide

At rt, to a mixed solution of 3-bromo-N-(1-cyclobutyl-4-(cyclopropylcarbamoyl)-1H-pyrrol-3-yl)pyrazolo[1,5-a]pyrimidine-5-carboxamide (200 mg, 0.4 mmol, 1.0 eq) and 1-(tetrahydro-2H-pyran-2-yl)-5-(4,4,5,5-tetramethyl-1,3,2-dioxaborolan-2-yl)-1H-pyrazole (188 mg, 0.7 mmol, 1.5 eq) in THF (5.0 mL) and water (1.0 mL) were added Pd(dppf)Cl₂ (33 mg, 0.04 mmol, 0.1 eq) and potassium carbonate (187 mg, 1.3 mmol, 3.0 eq). The reaction solution was reacted at 80°C for 16 h. Upon completion, the reaction solution was concentrated under reduced pressure. The residue was purified by column chromatography to afford the title compound (yellow solid, 180.0 mg, yield 78%).

### Step F: N-(1-cyclobutyl-4-(cyclopropylcarbamoyl)-1H-pyrrol-3-yl)-3-(1H-pyrazol-5-yl)pyrazolo[1,5-a]pyrimidine-5-carboxamide

At rt, to a solution of N-(1-cyclobutyl-4-(cyclopropylcarbamoyl)-1H-pyrrol-3-yl)-3-(1-(tetrahydro-2H-pyran-2-yl)-1H-pyrazol-5-yl)pyrazolo[1,5-a]pyrimidine-5-carboxamide (100 mg, 0.2 mmol, 1.0 eq) in MeOH (4.0 mL) was added p-toluenesulfonic acid (50 mg, 0.3 mmol, 1.2 eq). The reaction solution was reacted at rt for 1 h under N₂. Upon completion, the reaction solution was poured into aqueous NaHCO₃, and extracted with DCM (×3). The combined organic phases were washed with saturated aqueous NaCl, dried over anhydrous Na₂SO₄, filtered, and concentrated under reduced pressure. The residue was purified by HPLC to afford the title compound (white solid, 50 mg, yield 60%). **¹H NMR** (400 MHz, DMSO) δ 11.98 (s, 1H), 9.32 (d, *J* = 7.2 Hz, 1H), 8.41 (d, *J* = 2.4 Hz, 1H), 8.04 (d, *J* = 3.7 Hz, 1H), 7.61 (dd, *J* = 25.8, 10.9 Hz, 4H), 7.02 (d*, J* = 1.7 Hz, 1H), 4.74-4.54 (m, 1H), 2.82 (td, *J* = 7.2, 3.7 Hz, 1H), 2.47-2.35 (m, 3H), 2.29 (ddd, *J* = 11.8, 9.5, 2.7 Hz, 3H), 1.87-1.71 (m, 3H), 0.78-0.66 (m, 2H), 0.59-0.48 (m, 2H).

### Preparation Example 148. Synthesis of Compound A273

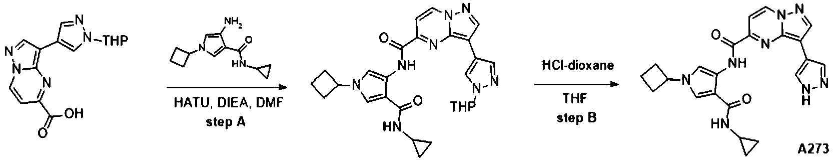

### Step A: N-(1-cyclobutyl-4-(cyclopropylcarbamoyl)-1H-pyrrol-3-yl)-3-(1-(tetrahydro-2H-pyran-2-yl)-1H-pyrazol-4-yl)pyrazolo[1,5-a]pyrimidine-5-carboxamide

At rt, to a solution of 3-(1-(tetrahydro-2H-pyran-2-yl)-1H-pyrazol-4-yl)pyrazolo[1,5-a]pyrimidine-5-carboxylic acid (200 mg, 0.6 mmol, 1.0 eq) and 4-amino-1-cyclobutyl-N-cyclopropyl-1H-pyrrole-3-carboxamide (155 mg, 0.7 mmol, 1.1 eq) in DMF (5.0 mL) were added DIEA (247 mg, 1.9 mmol, 3 eq) and HATU (485 mg, 1.3 mmol, 2 eq). The reaction solution was reacted for 16 h under N₂ at rt. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow oil, 275 mg, yield 83.5%).

### Step B: N-(1-cyclobutyl-4-(cyclopropylcarbamoyl)-1H-pyrrol-3-yl)-3-(1H-pyrazol-4-yl)pyrazolo[1,5-a]pyrimidine-5-carboxamide

At rt, to a solution of N-(1-cyclobutyl-4-(cyclopropylcarbamoyl)-1H-pyrrol-3-yl)-3-(1-(tetrahydro-2H-pyran-2-yl)-1H-pyrazol-4-yl)pyrazolo[1,5-a]pyrimidine-5-carboxamide (275 mg, 0.54 mmol, 1.0 eq) in THF (5.0 mL) was added hydrogen chloride in dioxane (1.0 mL). The reaction solution was reacted for 4 h under N₂ at rt. Upon completion, the reaction solution was cooled to rt and concentrated under reduced pressure. The residue was purified by HPLC to afford the title compound (yellow solid, 13 mg, yield 5.65%). **¹H NMR** (400 MHz, DMSO-*d*₆) δ 12.59 (s, 1H), 9.26 (d, *J* = 7.2 Hz, 1H), 8.74 (s, 1H), 8.60 (s, 2H), 8.08 (d, *J* = 4.7 Hz, 1H), 7.58 (d, *J* = 7.2 Hz, 2H), 7.54 (d, *J* = 2.1 Hz, 1H), 4.67-4.61 (m, 1H), 2.47-2.42 (m, 2H), 2.29 (dd, *J* = 15.4, 6.1 Hz, 2H), 1.79 (dd, *J* = 11.4, 3.8 Hz, 2H), 1.46 (s, 1H), 0.86 (t, *J* = 6.8 Hz, 1H), 0.81-0.77 (m, 2H), 0.55 (dd, *J* = 7.2, 3.5 Hz, 2H).

### Preparation Example 149. Synthesis of Compound A274

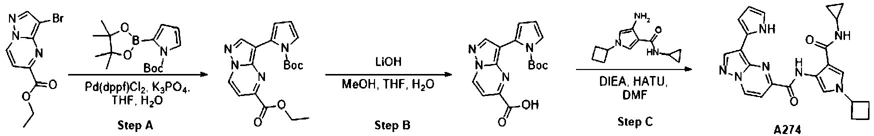

### Step A: Ethyl 3-(1-(tert-butoxycarbonyl)-1H-pyrrol-2-yl)pyrazolo[1,5-a]pyrimidine-5-carboxylate

At rt, to a mixed solution of ethyl 3-bromopyrazolo[1,5-a]pyrimidine-5-carboxylate (400 mg, 1.5 mmol, 1.0 eq) and tert-butyl 2-(4,4,5,5-tetramethyl-1,3,2-dioxaborolan-2-yl)-1H-pyrrole-1-carboxylate (521 mg, 1.8 mmol, 1.5 eq) in THF (5.0 mL) and water (1.0 mL) were added Pd(dppf)Cl₂ (108 mg, 0.1 mmol, 0.1 eq) and potassium phosphate (943 mg, 4.4 mmol, 3.0 eq). The reaction solution was reacted at 80°C for 16 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 400.0 mg, yield 76%).

### Step B: 3-(1-(tert-butoxycarbonyl)-1H-pyrrol-2-yl)pyrazolo[1,5-a]pyrimidine-5-carboxylic acid

At rt, to a mixed solution of ethyl 3-(1-(tert-butoxycarbonyl)-1H-pyrrol-2-yl)pyrazolo[1,5-a]pyrimidine-5-carboxylate (400 mg, 1.1 mmol, 1.0 eq) in MeOH (5.0 mL), THF (5.0 mL), and water (5.0 mL) was added lithium hydroxide (80 mg, 3.4 mmol, 3.0 eq). The reaction solution was reacted at rt for 2 h. Upon completion, the reaction solution was concentrated under reduced pressure, poured into water, and extracted with EtOAc (×3). The combined organic phases were washed with dilute HCl, dried over anhydrous Na₂SO₄, filtered, and concentrated under reduced pressure to afford the title compound (red solid, 300 mg, yield 82%).

### Step C: N-(1-cyclobutyl-4-(cyclopropylcarbamoyl)-1H-pyrrol-3-yl)-3-(1H-pyrrol-2-yl)pyrazolo[1,5-a]pyrimidine-5-carboxamide

At rt, to a solution of 3-(1-(tert-butoxycarbonyl)-1H-pyrrol-2-yl)pyrazolo[1,5-a]pyrimidine-5-carboxylic acid (100 mg, 0.3 mmol, 1.0 eq) and 4-amino-1-cyclobutyl-N-cyclopropyl-1H-pyrrole-3-carboxamide (67 mg, 0.3 mmol, 1.5 eq) in DMF (5.0 mL) were added DIEA (39 mg, 0.3 mmol, 3.0 eq) and HATU (115 mg, 0.3 mmol, 2.0 eq). The reaction solution was reacted at rt for 2 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (red solid, 60 mg, yield 46%). **¹H NMR** (400 MHz, DMSO) δ 12.72 (s, 1H), 11.00 (s, 1H), 9.27 (d, *J* = 7.2 Hz, 1H), 8.71 (s, 1H), 8.24 (d, *J* = 3.7 Hz, 1H), 7.72-7.40 (m, 3H), 7.02 (s, 1H), 6.94 (s, 1H), 6.20 (dd, *J* = 5.7, 2.5 Hz, 1H), 4.66 (p, *J* = 8.2 Hz, 1H), 2.94 (dt, *J* = 10.9, 3.7 Hz, 1H), 2.43 (dd, *J* = 9.6, 6.8 Hz, 2H), 2.37-2.24 (m, 2H), 1.80 (ddd, *J* = 14.4, 10.9, 8.0 Hz, 2H), 0.87-0.75 (m, 2H), 0.64-0.54 (m, 2H).

### Preparation Example 150. Synthesis of Compound A275

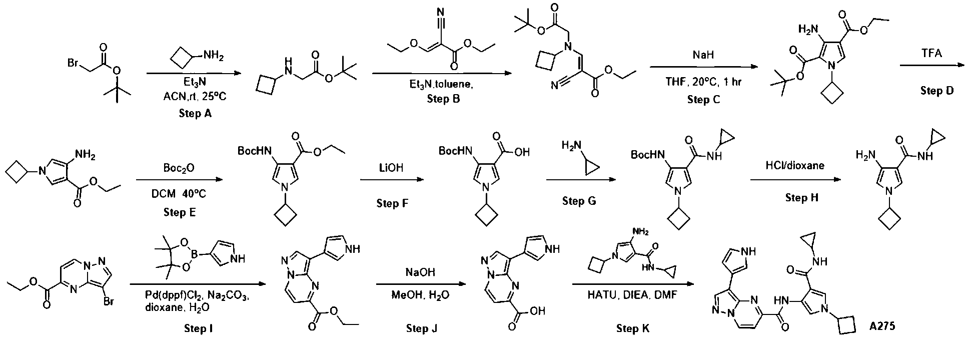

### Step A: Tert-butyl cyclobutylglycinate

At rt, to a solution of tert-butyl 2-bromoacetate (10.0 g, 0.05 mol, 1.0 eq) in acetonitrile (50.0 mL) were added cyclobutylamine (3.7 g, 0.05 mol, 1.0 eq) and TEA (15.7 g, 0.15 mol, 3.0 eq). The reaction solution was reacted at rt for 16 h. Upon completion, the reaction solution was concentrated under reduced pressure, poured into water, and extracted with DCM (×3). The combined organic phases were washed with saturated aqueous NaCl, dried over anhydrous Na₂SO₄, filtered, and concentrated under reduced pressure to afford the title compound (brown oil, 7.0 g, yield 72.9%).

### Step B: (E)-Ethyl 3-((2-(tert-butoxy)-2-oxoethyl)(cyclobutyl)amino)-2-cyanoacrylate

At rt, to a solution of tert-butyl cyclobutylglycinate (7.0 g, 0.04 mol, 1.0 eq) in toluene (30.0 mL) were added (*E*)-ethyl 2-cyano-3-ethoxyacrylate (7.7 g, 0.04 mol, 1.0 eq) and TEA (11.5 g, 0.15 mol, 2.75 eq). The reaction solution was reacted at 110°C for 16 h. Upon completion, the reaction solution was concentrated under reduced pressure, poured into water, and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (brown oil, 10.2 g, yield 87.6%).

### Step C: 2-(tert-butyl)-4-ethyl-3-amino-1-cyclobutyl-1H-pyrrole-2,4-dicarboxylate

At 0°C, to a solution of (E)-ethyl 3-((2-(tert-butoxy)-2-oxoethyl)(cyclobutyl)amino)-2-cyanoacrylate (10.2 g, 0.03 mol, 1.0 eq) in THF (20.0 mL) was added sodium hydride (5.2 g, 0.13 mol, 60% purity, 4.3 eq). The reaction solution was reacted at rt for 0.5 h. Upon completion, the reaction solution was poured into aqueous NH₄Cl and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (brown oil, 7.2 g, yield 70.5%).

### Step D: Ethyl 4-amino-1-cyclobutyl-1H-pyrrole-3-carboxylate

At rt, to a solution of 2-(tert-butyl)-4-ethyl-3-amino-1-cyclobutyl-1H-pyrrole-2,4-dicarboxylate (7.2 g, 0.02 mol, 1.0 eq) in DCM (5.0 mL) was added TFA (5.0 mL). The reaction solution was reacted at rt for 2 h. Upon completion, the reaction solution was concentrated under reduced pressure to afford the title compound (yellow solid, 4.6 g, yield 95.8%).

### Step E: Ethyl 4-((tert-butoxycarbonyl)amino)-1-cyclobutyl-1H-pyrrole-3-carboxylate

At rt, to a solution of ethyl 4-amino-1-cyclobutyl-1H-pyrrole-3-carboxylate (4.6 g, 0.22 mol, 1.0 eq) in DCM (20.0 mL) was added di-tert-butyl dicarbonate (4.8 g, 0.22 mol, 1.0 eq). The reaction solution was reacted at rt for 12 h. Upon completion, the reaction solution was concentrated under reduced pressure. The residue was purified by column chromatography to afford the title compound (white solid, 6.5 g, yield 95.6%).

### Step F: 4-((tert-butoxycarbonyl)amino)-1-cyclobutyl-1H-pyrrole-3-carboxylic acid

At rt, to a mixed solution of ethyl 4-((tert-butoxycarbonyl)amino)-1-cyclobutyl-1H-pyrrole-3-carboxylate (6.5 g, 0.02 mol, 1.0 eq) in MeOH (20.0 mL) and water (20.0 mL) was added lithium hydroxide (5.0 g, 0.21 mol, 10.0 eq). The reaction solution was reacted at 40°C for 8 h. Upon completion, the reaction solution was concentrated under reduced pressure, poured into water, and extracted with EtOAc (×3). The combined organic phases were washed with dilute HCl, dried over anhydrous Na₂SO₄, filtered, and concentrated under reduced pressure to afford the title compound (white solid, 5.4 g, yield 91.5%).

### Step G: tert-Butyl (1-cyclobutyl-4-(cyclopropylcarbamoyl)-1H-pyrrol-3-yl)carbamate

At rt, to a solution of 4-((tert-butoxycarbonyl)amino)-1-cyclobutyl-1H-pyrrole-3-carboxylic acid (5.4 g, 0.02 mol, 1.0 eq) and cyclopropylamine (2.2 g, 0.04 mol, 1.0 eq) in DMF (20.0 mL) were added DIEA (7.5 g, 0.06 mol, 3.0 eq) and HATU (14.7 g, 0.04 mol, 2.0 eq). The reaction solution was reacted at 30°C for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure to afford the title compound (yellow oil, 5.5 g, yield 89.4%).

### Step H: 4-amino-1-cyclobutyl-N-cyclopropyl-1H-pyrrole-3-carboxamide

At rt, to a solution of tert-butyl (1-cyclobutyl-4-(cyclopropylcarbamoyl)-1H-pyrrol-3-yl)carbamate (5.5 g, 0.02 mol, 1.0 eq) in DCM (20.0 mL) was added HCl/dioxane (5.0 mL). The reaction solution was reacted at 30°C for 1 h. Upon completion, the reaction solution was concentrated under reduced pressure to afford the title compound (white oil, 5.0 g, crude).

### Step I: Ethyl 3-(1H-pyrrol-3-yl)pyrazolo[1,5-a]pyrimidine-5-carboxylate

At rt, to a mixed solution of ethyl 3-bromopyrazolo[1,5-a]pyrimidine-5-carboxylate (100 mg, 0.4 mmol, 1.0 eq) and 3-(4,4,5,5-tetramethyl-1,3,2-dioxaborolan-2-yl)-1H-pyrrole (107 mg, 0.6 mmol, 1.5 eq) in 1,4-dioxane (20.0 mL) and water (4.0 mL) were added Pd(dppf)Cl₂ (27 mg, 0.03 mmol, 0.1 eq) and sodium carbonate (117 mg, 1.1 mmol, 3.0 eq). The reaction solution was reacted at 80°C for 2 h. Upon completion, the reaction solution was concentrated under reduced pressure. The residue was purified by column chromatography to afford the title compound (red solid, 75.0 mg, yield 79%).

### Step J: 3-(1H-pyrrol-3-yl)pyrazolo[1,5-a]pyrimidine-5-carboxylic acid

At rt, to a mixed solution of ethyl 3-(1H-pyrrol-3-yl)pyrazolo[1,5-a]pyrimidine-5-carboxylate (75 mg, 0.3 mmol, 1.0 eq) in MeOH (20.0 mL) and water (10.0 mL) was added NaOH (23 mg, 0.6 mmol, 10.0 eq). The reaction solution was reacted at rt for 16 h. Upon completion, the reaction solution was adjusted to pH 4-5 with 1 N HCl, and extracted with EtOAc (×3). The combined organic phases were washed with saturated aqueous NaCl, dried over anhydrous Na₂SO₄, filtered, and concentrated under reduced pressure to afford the title compound (yellow solid, 66 mg, yield 98%).

### Step K: N-(1-cyclobutyl-4-(cyclopropylcarbamoyl)-1H-pyrrol-3-yl)-3-(1H-pyrrol-3-yl)pyrazolo[1,5-a]pyrimidine-5-carboxamide

At rt, to a solution of 3-(1H-pyrrol-3-yl)pyrazolo[1,5-a]pyrimidine-5-carboxylic acid (70 mg, 0.3 mmol, 1.0 eq) and 4-amino-1-cyclobutyl-N-cyclopropyl-1H-pyrrole-3-carboxamide (67 mg, 0.3 mmol, 1.0 eq) in DMF (5.0 mL) were added DIEA (198 mg, 1.5 mmol, 5.0 eq) and HATU (233 mg, 0.6 mmol, 2.0 eq). The reaction solution was reacted at rt for 16 h. Upon completion, the reaction solution was poured into NaHCO₃ solution and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (red solid, 24.5 mg, yield 19%). **¹H NMR** (400 MHz, DMSO-*d*₆) δ 12.44 (s, 1H), 10.83 (s, 1H), 9.19 (d, *J* = 7.2 Hz, 1H), 8.61 (s, 1H), 8.04 (d, *J* = 4.4 Hz, 1H), 7.98-7.92 (m, 1H), 7.59-7.50 (m, 3H), 6.94-6.87 (m, 1H), 6.85-6.81 (m, 1H), 4.70-4.59 (m, 1H), 3.12-3.04 (m, 1H), 2.48-2.40 (m, 2H), 2.35-2.25 (m, 2H), 1.85-1.74 (m, 2H), 0.82-0.74 (m, 2H), 0.60-0.52 (m, 2H). LCMS (ESI): m/z 430 [M+H]⁺.

### Preparation Example 151. Synthesis of Compound A276

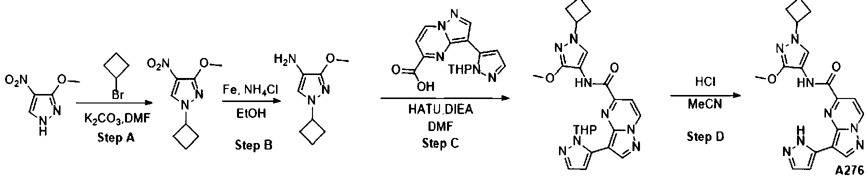

### Step A: 1-cyclobutyl-3-methoxy-4-nitro-1H-pyrazole

At rt, to a solution of 3-methoxy-4-nitro-1H-pyrazole (500 mg, 3.4 mmol, 1.0 eq) and bromocyclobutane (465 mg, 3.4 mmol, 1.0 eq) in DMF (5.0 mL) was added potassium carbonate (1.4 g, 10.2 mmol, 3.0 eq). The reaction solution was reacted at 80°C for 3 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 0.5 g, yield 72.0%).

### Step B: 1-cyclobutyl-3-methoxy-1H-pyrazol-4-amine

To a mixed solution of 1-cyclobutyl-3-methoxy-4-nitro-1H-pyrazole (500 mg, 2.5 mol, 1.0 eq) in ethanol (5.0 mL) and water (1.0 mL) at rt were added iron powder (1.4 g, 25.0 mmol, 10.0 eq) and ammonium chloride (1.3 g, 25.0 mmol, 10.0 eq). The reaction solution was reacted at 80°C for 3 h. Upon completion, the reaction solution was cooled to rt and then filtered. The filtrate was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 350 mg, yield 82.0%).

### Step C: N-(1-cyclobutyl-3-methoxy-1H-pyrazol-4-yl)-3-(1-(tetrahydro-2H-pyrazol-2-yl)-1H-pyrazin-5-yl)pyrazolo[1,5-a]pyrimidine-5-carboxamide

At rt, to a solution of 1-cyclobutyl-3-methoxy-1H-pyrazol-4-amine (50 mg, 0.3 mmol, 1.0 eq) and 3-(1-(tetrahydro-2H-pyran-2-yl)-1H-pyrazol-5-yl)pyrazolo[1,5-a]pyrimidine-5-carboxylic acid (103 mg, 0.3 mmol, 1.1 eq) in DMF (5.0 mL) were added DIEA (116 mg, 0.9 mmol, 3.0 eq) and HATU (170 mg, 0.45 mmol, 1.5 eq). The reaction solution was reacted at rt for 3 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 70 mg, yield 50.0%).

### Step D: N-(1-cyclobutyl-3-methoxy-1H-pyrazol-4-yl)-3-(1H-pyrazol-5-yl)pyrazolo[1,5-a]pyrimidine-5-carboxamide

At rt, to a solution of *N-*(1-cyclobutyl-3-methoxy-1H-pyrazol-4-yl)-3-(1-(tetrahydro-2H-pyrazol-2-yl)-1H-pyrazin-5-yl)pyrazolo[1,5-a]pyrimidine-5-carboxamide (70 mg, 0.15 mmol, 1.0 eq) in acetonitrile (5.0 mL) was added HCl/dioxane (2 mL, 4 M in dioxane). The reaction solution was reacted at rt for 1 h. Upon completion, the reaction solution was concentrated under reduced pressure. The residue was purified by HPLC to afford the title compound (white solid, 15.0 mg, yield 26%). **¹H NMR** (400 MHz, DMSO-*d*₆) δ 13.16 (d, *J=* 194.0 Hz, 1H), 10.19 (d, *J* = 333.3 Hz, 1H), 9.32 (d, *J* = 7.1 Hz, 1H), 8.76 (d, *J* = 45.6 Hz, 1H), 8.00 (s, 1H), 7.75 (d, *J* = 97.7 Hz, 2H), 7.02 (d, *J* = 133.0 Hz, 1H), 4.70 (p, *J* = 8.3 Hz, 1H), 3.91 (s, 3H), 2.49-2.39 (m, 2H), 2.39-2.31 (m, 2H), 1.83-1.70 (m, 2H). LCMS (ESI): m/z 379 [M+H]⁺.

### Preparation Example 152. Synthesis of Compound A277

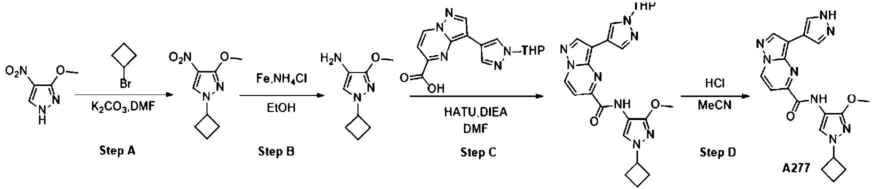

### Step A: 1-cyclobutyl-3-methoxy-4-nitro-1H-pyrazole

At rt, to a solution of 3-methoxy-4-nitro-1H-pyrazole (500 mg, 3.4 mmol, 1.0 eq) and bromocyclobutane (465 mg, 3.4 mmol, 1.0 eq) in DMF (5.0 mL) was added potassium carbonate (1.4 g, 10.2 mmol, 3.0 eq). The reaction solution was reacted at 80°C for 3 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 0.5 g, yield 72.0%).

### Step B: 1-cyclobutyl-3-methoxy-1H-pyrazol-4-amine

To a mixed solution of 1-cyclobutyl-3-methoxy-4-nitro-1H-pyrazole (500 mg, 2.5 mol, 1.0 eq) in ethanol (5.0 mL) and water (1.0 mL) at rt were added iron powder (1.4 g, 25.0 mmol, 10.0 eq) and ammonium chloride (1.3 g, 25.0 mmol, 10.0 eq). The reaction solution was reacted at 80°C for 3 h. Upon completion, the reaction solution was cooled to rt and then filtered. The filtrate was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 350 mg, yield 82.0%).

### Step C: N-(1-cyclobutyl-3-methoxy-1H-pyrazol-4-yl)-3-(1-(tetrahydro-2H-pyrazol-2-yl)-1H-pyrazin-4-yl)pyrazolo[1,5-a]pyrimidine-5-carboxamide

At rt, to a solution of 1-cyclobutyl-3-methoxy-1H-pyrazol-4-amine (50 mg, 0.3 mmol, 1.0 eq) and 3-(1-(tetrahydro-2H-pyran-2-yl)-1H-pyrazol-4-yl)pyrazolo[1,5-a]pyrimidine-5-carboxylic acid (103 mg, 0.3 mmol, 1.1 eq) in DMF (5.0 mL) were added DIEA (116 mg, 0.9 mmol, 3.0 eq) and HATU (170 mg, 0.45 mmol, 1.5 eq). The reaction solution was reacted at rt for 3 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 70 mg, yield 50.0%).

### Step D: N-(1-cyclobutyl-3-methoxy-1H-pyrazol-4-yl)-3-(1H-pyrazol-4-yl)pyrazolo[1,5-a]pyrimidine-5-carboxamide

At rt, to a solution of *N-*(1-cyclobutyl-3-methoxy-1H-pyrazol-4-yl)-3-(1-(tetrahydro-2H-pyrazol-2-yl)-1H-pyrazin-4-yl)pyrazolo[1,5-a]pyrimidine-5-carboxamide (70 mg, 0.15 mmol, 1.0 eq) in acetonitrile (5.0 mL) was added HCl/dioxane (2 mL, 4 M). The reaction solution was reacted at rt for 1 h. Upon completion, the reaction solution was concentrated under reduced pressure. The residue was purified by HPLC to afford the title compound (white solid, 15.0 mg, yield 26%). **¹H NMR** (400 MHz, DMSO-*d*₆) δ 9.90 (s, 1H), 9.24 (d, *J* = 7.2 Hz, 1H), 8.69 (s, 1H), 8.39 (s, 2H), 7.96 (s, 1H), 7.54 (d, *J* = 7.2 Hz, 1H), 4.75-4.66 (m, 1H), 3.90 (s, 3H), 2.48-2.42 (m, 2H), 2.35 (dd, *J* = 9.2, 6.5 Hz, 2H), 1.80-1.73 (m, 2H). LCMS (ESI): m/z 379 [M+H]⁺.

### Preparation Example 153. Synthesis of Compound A278

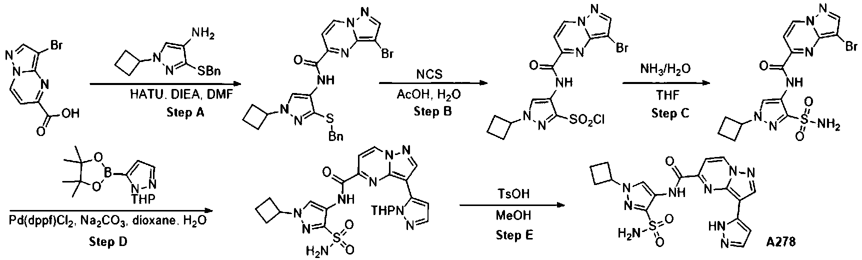

### Step A: N-(3-(benzylthio)-1-cyclobutyl-1H-pyrazol-4-yl)-3-bromopyrazolo[1,5-a]pyrimidine-5-carboxamide

At rt, to a solution of 3-bromopyrazolo[1,5-a]pyrimidine-5-carboxylic acid (100 mg, 0.41 mmol, 1.0 eq) and 3-(benzylthio)-1-cyclobutyl-1H-pyrazol-4-amine (107 mg, 0.41 mmol, 1.0 eq) in DMF (5.0 mL) was added DIEA (160 mg, 1.23 mmol, 3.0 eq). After stirring at rt for 10 min, HATU (235 mg, 0.62 mmol, 1.5 eq) was then added, and the mixture was further reacted at rt. After 1 h, the reaction solution was poured into water and extracted with EtOAc (×3). The resulting organic phases were combined, washed with saturated aqueous NaCl, dried over anhydrous Na₂SO₄, filtered, and concentrated under reduced pressure. The residue was purified by column chromatography to afford the title compound (white solid, 170 mg, yield 85.15%).

### Step B: 4-(3-bromopyrazolo[1,5-a]pyrimidine-5-carboxamide)-1-cyclobutyl-1H-pyrazole-3-sulfonyl chloride

Under ice bath conditions, to a solution of N-(3-(benzylthio)-1-cyclobutyl-1H-pyrazol-4-yl)-3-bromopyrazolo[1,5-a]pyrimidine-5-carboxamide (100 mg, 0.21 mmol, 1.0 eq) in AcOH (2.0 mL) were successively added water (0.2 mL) and NCS (110.49 mg, 0.83 mmol, 4.0 eq). After 18 h, the reaction solution was poured into water and extracted with EtOAc twice. The resulting organic phases were combined, washed with saturated aqueous NaCl, dried over anhydrous Na₂SO₄, and filtered, and the filtrate was concentrated under reduced pressure to afford the title compound (130 mg, crude).

### Step C: 3-bromo-N-(1-cyclobutyl-3-sulfonamide-1H-pyrazol-4-yl)pyrazolo[1,5-a]pyrimidine-5-carboxamide

At rt, to a solution of 4-(3-bromopyrazolo[1,5-a]pyrimidine-5-carboxamide)-1-cyclobutyl-1H-pyrazole-3-sulfonyl chloride (90 mg, 0.20 mmol, 1.0 eq) in THF (2.0 mL) was added aqueous ammonia (1 mL). After stirring at rt for 1 h, the reaction solution was poured into water and extracted with EtOAc twice. The resulting organic phases were combined, washed with saturated aqueous NaCl, dried over anhydrous Na₂SO₄, filtered, and concentrated under reduced pressure. The residue was purified by column chromatography to afford the title compound (yellow solid, 80 mg, yield 92.81%).

### Step D: N-(1-cyclobutyl-3-sulfonamide-1H-pyrazol-4-yl)-3-(1-(tetrahydro-2H-pyran-2-yl)-1H-pyrazol-5-yl)pyrazolo[1,5-a]pyrimidine-5-carboxamide

Under N₂, a mixed solution of 3-bromo-N-(1-cyclobutyl-3-sulfonamide-1H-pyrazol-4-yl)pyrazolo[1,5-a]pyrimidine-5-carboxamide (80 mg, 0.18 mmol, 1.0 eq), 1-(tetrahydro-2H-pyran-2-yl)-5-(4,4,5,5-tetramethyl-1,3,2-dioxaborolan-2-yl)-1H-pyrazole (53.07 mg, 0.19 mmol, 1.05 eq), Na₂CO₃ (38.52 mg, 0.36 mmol, 2.0 eq) and Pd(dppf)Cl₂ (13.30 mg, 0.02 mmol, 0.1 eq) in dioxane (2.7 mL) and water (0.3 mL) was heated to 95°C. After 18 h, the reaction solution was cooled to rt, poured into water, and extracted with EtOAc twice. The resulting organic phases were combined, washed with saturated aqueous NaCl, dried over anhydrous Na₂SO₄, filtered, and concentrated under reduced pressure. The residue was purified by column chromatography to afford the title compound (40 mg, yield 43.03%).

### Step E: N-(1-cyclobutyl-3-sulfonamide-1H-pyrazol-4-yl)-3-(1H-pyrazol-5-yl)pyrazolo[1,5-a]pyrimidine-5-carboxamide

At rt, to a solution of *N*-(1-cyclobutyl-3-sulfonamide-1H-pyrazol-4-yl)-3-(1-(tetrahydro-2H-pyran-2-yl)-1H-pyrazol-5-yl)pyrazolo[1,5-a]pyrimidine-5-carboxamide (40 mg, 0.08 mmol, 1.0 eq) in MeOH (2.0 mL) was added p-toluenesulfonic acid (16.16 mg, 0.09 mmol, 1.20 eq). The reaction solution was further reacted under N₂ at rt. After 3 h, the reaction solution was concentrated under reduced pressure. Water was added to the residue, and the mixture was extracted with EtOAc twice. The resulting organic phases were combined, washed with saturated aqueous NaCl, dried over anhydrous Na₂SO₄, filtered, and concentrated under reduced pressure. The residue was purified by preparative HPLC to afford the title compound (white solid, 10.5 mg, yield 31.42%). **¹H NMR** (400 MHz, DMSO-*d*₆) δ 13.08 (d, *J* = 136.5 Hz, 1H), 10.88 (s, 1H), 9.37 (t, *J* = 7.7 Hz, 1H), 8.79 (d, *J* = 37.8 Hz, 1H), 8.59 (d, *J* = 6.5 Hz, 1H), 8.00-7.57 (m, 4H), 7.26 (d, *J* = 31.3 Hz, 1H), 5.02 (p, *J* = 8.3 Hz, 1H), 2.59-2.39 (m, 4H), 1.93-1.76 (m, 2H).

### Preparation Example 154. Synthesis of Compound A279

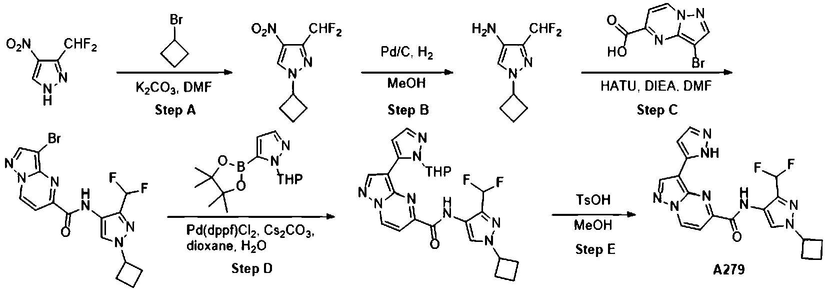

### Step A: 1-cyclobutyl-3-(difluoromethyl)-4-nitro-1H-pyrazole

At rt, to a solution of 3-(difluoromethyl)-4-nitro-1H-pyrazole (330 mg, 2.0 mmol, 1.0 eq) and bromocyclobutane (1.36 g, 10.1 mmol, 5.0 eq) in DMF (5.0 mL) was added potassium carbonate (1.98 g, 6.0 mmol, 3.0 eq). The reaction solution was reacted under N₂ at 90°C for 16 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow oil, 340 mg, yield 77.0%).

### Step B: 1-cyclobutyl-3-(difluoromethyl)-1H-pyrazol-4-amine

At rt, to a solution of 1-cyclobutyl-3-(difluoromethyl)-4-nitro-1H-pyrazole (150 mg, 0.6 mmol, 1.0 eq) in MeOH (10.0 mL) was added palladium on carbon (83.30 mg, 10%, 0.5 eq). The reaction solution was purged with H₂ (×3), and reacted at rt under H₂ for 2 h. Upon completion, the reaction solution was filtered, and the filtrate was concentrated under reduced pressure to afford the title compound (yellow oil, 250 mg, yield 85.0%).

### Step C: 3-bromo-N-(1-cyclobutyl-3-(difluoromethyl)-1H-pyrazol-4-yl)pyrazolo[1,5-a]pyrimidine-5-carboxamide

At rt, to a solution of 1-cyclobutyl-3-(difluoromethyl)-1H-pyrazol-4-amine (170 mg, 0.9 mmol, 1.0 eq) and 3-bromopyrazolo[1,5-a]pyrimidine-5-carboxylic acid (220 mg, 0.9 mmol, 1.0 eq) in DMF (15.0 mL) were added DIEA (0.5 mL, 2.7 mmol, 3.0 eq) and HATU (690 mg, 1.8 mmol, 2.0 eq). The reaction solution was reacted at rt for 16 h. Upon completion, the reaction solution was poured into aqueous NaHCO₃ and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 280 mg, yield 75.0%).

### Step D: N-(1-cyclobutyl-3-(difluoromethyl)-1H-pyrazol-4-yl)-3-(1-(tetrahydro-2H-pyrazol-2-yl)-1H-pyrazol-5-yl)pyrazolo[1,5-a]pyrimidine-5-carboxamide

At rt, to a mixed solution of 3-bromo-N-(1-cyclobutyl-3-(difluoromethyl)-1H-pyrazol-4-yl)pyrazolo[1,5-a]pyrimidine-5-carboxamide (280 mg, 0.7 mmol, 1.0 eq) and 1-(tetrahydro-2H-pyran-2-yl)-5-(4,4,5,5-tetramethyl-1,3,2-dioxaborolan-2-yl)-1H-pyrazole (378 mg, 1.4 mmol, 2.0 eq) in dioxane (20.0 mL) and water (4.0 mL) were added Pd(dppf)Cl₂ (50 mg, 0.07 mmol, 0.1 eq) and cesium carbonate (666 mg, 2.1 mmol, 3.0 eq). The reaction solution was reacted at 90°C for 16 h. Upon completion, the reaction solution was cooled to rt and concentrated under reduced pressure. The residue was purified by column chromatography to afford the title compound (yellow oil, 80 mg, yield 24%).

### Step E: N-(1-cyclobutyl-3-(difluoromethyl)-1H-pyrazol-4-yl)-3-(1H-pyrazol-5-yl)pyrazolo[1,5-a]pyrimidine-5-carboxamide

At rt, to a solution of N-(1-cyclobutyl-3-(difluoromethyl)-1H-pyrazol-4-yl)-3-(1-(tetrahydro-2H-pyrazol-2-yl)-1H-pyrazol-5-yl)pyrazolo[1,5-a]pyrimidine-5-carboxamide (80 mg, 0.2 mmol, 1.0 eq) in MeOH (10.0 mL) was added p-toluenesulfonic acid (86 mg, 0.5 mmol, 3.0 eq). The reaction solution was reacted at rt for 1 h under N₂. Upon completion, the reaction solution was poured into aqueous NaHCO₃, and extracted with DCM (×3). The combined organic phases were washed with saturated aqueous NaCl, dried over anhydrous Na₂SO₄, filtered, and concentrated under reduced pressure. The residue was purified by HPLC to afford the title compound (yellow solid, 43.9 mg, yield 66.0%). **¹H NMR** (400 MHz, DMSO-*d*₆) δ 13.10 (s, 1H), 10.44 (s, 1H), 9.35 (d, *J=* 7.2 Hz, 1H), 8.78 (s, 1H), 8.37 (s, 1H), 7.83-7.61 (m, 2H), 7.38-6.96 (m, 2H), 5.00-4.91 (m, 1H), 2.58-2.52 (m, 1H), 2.50-2.38 (m, 3H), 1.87-1.78 (m, 2H). LCMS (ESI): m/z 399 [M+H]⁺.

### Preparation Example 155. Synthesis of Compound A280

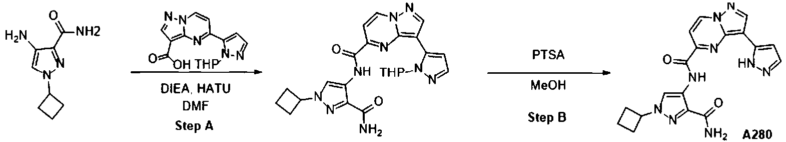

### Step A: N-(3-amino-1-cyclobutyl-1H-pyrazol-4-yl)-5-(1-(tetrahydro-2H-pyrazol-2-yl)-1H-pyrazin-5-yl)pyrazolo[1,5-a]pyrimidine-3-carboxamide

At rt, to a solution of 4-amino-1-cyclobutylpyrazole-3-carboxamide (150 mg, 0.8 mmol, 1.5 eq) and 3-[2-(3,4,5,6-tetrahydro-2H-pyran-2-yl)pyrazol-3-yl]pyrazolo[1,5-a]pyrimidine-5-carboxylic acid (100 mg, 0.3 mmol, 1.0 eq) in DMF (5.0 mL) were added DIEA (215 mg, 1.6 mmol, 3.0 eq) and HATU (316 mg, 0.8 mmol, 1.5 eq). The reaction solution was reacted at rt for 2 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 150 mg, yield 56.8%).

### Step B: N-(3-carbamoyl-1-cyclobutyl-1H-pyrazol-4-yl)-3-(1H-pyrazol-5-yl)pyrazolo[1,5-a]pyrimidine-5-carboxamide

At rt, to a solution of N-(3-amino-1-cyclobutyl-1H-pyrazol-4-yl)-5-(1-(tetrahydro-2H-pyrazol-2-yl)-1H-pyrazin-5-yl)pyrazolo[1,5-a]pyrimidine-3-carboxamide (100 mg, 0.2 mmol, 1.0 eq) in MeOH (5.0 mL) was added p-toluenesulfonic acid (43 mg, 0.3 mmol, 1.2 eq). The reaction solution was reacted at rt for 1 h under N₂. Upon completion, the reaction solution was poured into aqueous NaHCO₃, and extracted with DCM (×3). The combined organic phases were washed with saturated aqueous NaCl, dried over anhydrous Na₂SO₄, filtered, and concentrated under reduced pressure. The residue was purified by HPLC to afford the title compound (yellow solid, 50 mg, yield 60.7%). **¹H NMR** (400 MHz, DMSO) δ 13.11 (d, *J* = 154.0 Hz, 1H), 11.74 (t, *J* = 59.7 Hz, 1H), 9.52-9.21 (m, 1H), 8.79 (d, *J* = 38.8 Hz, 1H), 8.54-8.40 (m, 1H), 7.85-7.55 (m, 4H), 7.39 (d, *J* = 26.4 Hz, 1H), 4.98 (p, *J* = 8.5 Hz, 1H), 2.59 (dt, *J* = 11.9, 9.5 Hz, 2H), 2.42 (dt, *J* = 36.8, 17.7 Hz, 2H), 1.89-1.76 (m, 2H).

### Preparation Example 156. Synthesis of Compound A281

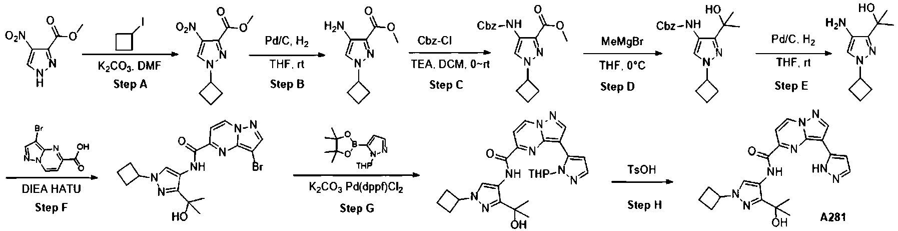

### Step A: Methyl 1-cyclobutyl-4-nitro-1H-pyrazole-3-carboxylate

At rt, to a solution of 4-nitro-1H-pyrazole-3-carboxylate (5 g, 29 mmol, 1.0 eq) and iodocyclobutane (7.9 g, 44 mmol, 1.5 eq) in DMF (100.0 mL) was added potassium carbonate (20 g, 146 mmol, 5.0 eq). The reaction solution was reacted at 80°C for 24 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (colorless oil, 4 g, yield 60.7%).

### Step B: Methyl 4-amino-1-cyclobutyl-1H-pyrazole-3-carboxylate

At rt, palladium on carbon (400 mg, 10% purity, 0.2 eq) was added to a solution of methyl 1-cyclobutyl-4-nitro-1H-pyrazole-3-carboxylate (800 mg, 3.6 mmol, 1.0 eq) in MeOH (10.0 mL). The reaction solution was purged with H₂ (×3), and then stirred at rt for 2 h. Upon completion, the reaction solution was filtered, and the filtrate was concentrated under reduced pressure to afford the title compound (white solid, 600 mg, yield 86.5%).

### Step C: Methyl 4-(benzyloxycarbonylamino)-1-cyclobutyl-1H-pyrazole-3-carboxylate

At rt, benzyl chloroformate (1.2 g, 4.6 mmol, 1.5 eq) was added to a solution of 4-amino-1-cyclobutyl-1H-pyrazole-3-carboxylate (600 mg, 3 mmol, 1.0 eq) and TEA (1.3 mL, 9 mmol, 3.0 eq) in DCM (5.0 mL). The reaction solution was reacted at rt for 2 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 800 mg, yield 79.0%).

### Step D: Benzyl (1-cyclobutyl-3-(2-hydroxypropan-2-yl)-1H-pyrazol-4-yl)carbamate

At-10°C, methylmagnesium bromide (5.0 mL, 2.1 mmol, 1 M, 3.0 eq) was added to a solution of methyl 4-(benzyloxycarbonylamino)-1-cyclobutyl-1H-pyrazole-3-carboxylate (500 mg, 1.5 mmol, 1.0 eq) in THF (5.0 mL). The reaction solution was reacted at -10°C for 2 h. Upon completion, the reaction solution was poured into saturated aqueous NH₄Cl and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow oil, 400 mg, yield 79.9%).

### Step E: 2-(4-amino-1-cyclobutyl-1H-pyrazol-3-yl)propan-2-ol

At rt, palladium on carbon (0.3 g, 10% purity, 0.2 eq) was added to a solution of benzyl (1-cyclobutyl-3-(2-hydroxypropan-2-yl)-1H-pyrazol-4-yl)carbamate (500 mg, 1.5 mmol, 1.0 eq) in MeOH (6.0 mL). The reaction solution was purged with H₂ (×3), and then stirred at rt for 16 h. Upon completion, the reaction solution was filtered, and the filtrate was concentrated under reduced pressure to afford the title compound (white solid, 250 mg, yield 84.3%).

### Step F: 3-bromo-N-(1-cyclobutyl-3-(2-hydroxypropan-2-yl)-1H-pyrazol-4-yl)pyrazolo[1,5-a]pyrimidine-5-carboxamide

At rt, DIEA (992 mg, 7.6 mmol, 3.0 eq) and HATU (876 mg, 2.3 mmol, 2.0 eq) were added to a solution of 2-(4-amino-l-cyclobutyl-1H-pyrazol-3-yl)propan-2-ol (300 mg, 1.5 mmol, 1.5 eq) and 3-bromopyrazolo[1,5-a]pyrimidine-5-carboxylic acid (557 mg, 2.3 mmol, 1.0 eq) in DMF (5.0 mL). The reaction solution was reacted at rt for 2 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 400 mg, yield 62.1%).

### Step G: N-(1-cyclobutyl-3-(2-hydroxypropan-2-yl)-1H-pyrazol-4-yl)-3-(1-(tetrahydro-2H-pyrazol-2-yl)-1-pyrazol-5-yl)pyrazolo[1,5-a]pyrimidine-5-carboxamide

At rt, Pd(dppf)Cl₂ (52 mg, 0.07 mmol, 0.1 eq) and potassium carbonate (296 mg, 2.1 mmol, 3.0 eq) were added to a mixed solution of 3-bromo-N-(1-cyclobutyl-3-(2-hydroxypropan-2-yl)-1H-pyrazol-4-yl)pyrazolo[1,5-a]pyrimidine-5-carboxamide (300 mg, 0.7 mmol, 1.0 eq) and 1-(tetrahydro-2H-pyran-2-yl)-5-(4,4,5,5-tetramethyl-1,3,2-dioxaborolan-2-yl)-1H-pyrazole (298 mg, 1.0 mmol, 1.5 eq) in THF (5.0 mL) and water (1.0 mL). The reaction solution was reacted at 80°C for 16 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 300.0 mg, yield 85.4%).

### Step H: N-(1-cyclobutyl-3-(2-hydroxypropan-2-yl)-1H-pyrazol-4-yl)-3-(1H-pyrazol-5-yl)pyrazolo[1,5-a]pyrimidine-5-carboxamide

At rt, p-toluenesulfonic acid (105 mg, 0.6 mmol, 1.2 eq) was added to a solution of N-(1-cyclobutyl-3-(2-hydroxypropan-2-yl)-1H-pyrazol-4-yl)-3-(1-(tetrahydro-2H-pyrazol-2-yl)-1-pyrazol-5-yl)pyrazolo[1,5-a]pyrimidine-5-carboxamide (200 mg, 0.4 mmol, 1.0 eq) in MeOH (5.0 mL). The reaction solution was reacted at rt for 2 h under N₂. Upon completion, the reaction solution was poured into aqueous NaHCO₃, and extracted with DCM (×3). The combined organic phases were washed with saturated aqueous NaCl, dried over anhydrous Na₂SO₄, filtered, and concentrated under reduced pressure. The residue was purified by HPLC to afford the title compound (yellow solid, 100.0 mg, yield 60.3%). **¹H NMR** (400 MHz, DMSO) δ 11.07 (d, *J* = 31.3 Hz, 1H), 9.33 (d, *J* = 6.8 Hz, 1H), 8.76 (d, *J* = 37.6 Hz, 1H), 8.35 (d, *J* = 6.8 Hz, 1H), 7.95-7.55 (m, 2H), 7.34-6.85 (m, 1H), 5.89 (d, *J* = 13.1 Hz, 1H), 4.82 (p, *J* = 8.3 Hz, 1H), 2.39 (ddd, *J* = 16.7, 15.0, 9.2 Hz, 4H), 1.76 (dt, *J* = 10.3, 8.1 Hz, 2H), 1.55 (d, *J* = 20.4 Hz, 6H).

### Preparation Example 157. Synthesis of Compound A282

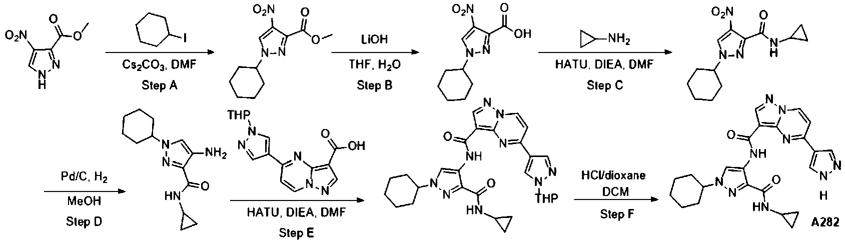

### Step A: Methyl 1-cyclohexyl-4-nitro-1H-pyrazole-3-carboxylate

At rt, to a solution of 4-nitro-1H-pyrazole-3-carboxylate (7 g, 40.9 mmol, 1.0 eq) and iodocyclohexane (17.2 g, 81.8 mmol, 2.0 eq) in DMF (25.0 mL) was added cesium carbonate (26.7 g, 81.8 mmol, 2.0 eq). The reaction solution was reacted at 90°C for 16 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (pale yellow oil, 2.04 g, yield 19%).

### Step B: 1-Cyclohexyl-4-nitro-1H-pyrazole-3-carboxylic acid

To a mixed solution of methyl 1-cyclohexyl-4-nitro-1H-pyrazole-3-carboxylate (670 mg, 2.6 mmol, 1.0 eq) in THF (15.0 mL) and water (3.0 mL) was added lithium hydroxide (222 mg, 5.2 mmol, 2.0 eq) at rt. The reaction solution was reacted at rt for 16 h. Upon completion, the reaction solution was adjusted to pH 4-5 with 1 N HCl and concentrated under reduced pressure to afford the title compound (white solid, 620 mg, crude).

### Step C: 1-cyclohexyl-N-cyclopropyl-4-nitro-1H-pyrazole-3-carboxamide

At rt, to a solution of 1-cyclohexyl-4-nitro-1H-pyrazole-3-carboxylic acid (620 mg, crude) and cyclopropylamine (1.4 mL, 7.8 mmol, 3.0 eq) in DMF (15.0 mL) were added DIEA (1.4 mL, 7.8 mmol, 3.0 eq) and HATU (1.9 g, 5.2 mmol, 1.0 eq). The reaction solution was reacted at rt for 4 h. Upon completion, the reaction solution was poured into NaHCO₃ solution and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (colorless oil, 700 mg, yield 97%).

### Step D: 4-amino-1-cyclohexyl-N-cyclopropyl-1H-pyrazole-3-carboxamide

To a solution of 1-cyclohexyl-N-cyclopropyl-4-nitro-1H-pyrazole-3-carboxamide (700 mg, 2.5 mmol, 1.0 eq) in MeOH (50.0 mL) was added palladium on carbon (70 mg, 10%) at rt. The reaction solution was purged with H₂ (×3), and then stirred at rt for 16 h. Upon completion, the reaction solution was filtered, and the filtrate was concentrated under reduced pressure to afford the title compound (brown oil, 560 mg, crude).

### Step E: N-(1-cyclohexyl-3-(cyclopropylcarbamoyl)-1H-pyrazol-4-yl)-5-(1-(tetrahydro-2H-pyrazol-2-yl)-1H-pyridin-4-yl)pyrazolo[1,5-a]pyrimidine-3-carboxamide

At rt, to a solution of 4-amino-1-cyclohexyl-N-cyclopropyl-1H-pyrazole-3-carboxamide (120 mg, crude) and 5-(1-(tetrahydro-2H-pyran-2-yl)-1H-pyrazol-4-yl)pyrazolo[1,5-a]pyrimidine-3-carboxylic acid (182 mg, 0.6 mmol, 1.2 eq) in DMF (12.0 mL) were added DIEA (0.26 mL, 1.5 mmol, 3.0 eq) and HATU (368 mg, 1.0 mmol, 2.0 eq). The reaction solution was reacted at rt for 16 h. Upon completion, the reaction solution was poured into NaHCO₃ solution and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow oil, 70 mg, yield 26%).

### Step F: N-(1-cyclohexyl-3-(cyclopropylcarbamoyl)-1H-pyrazol-4-yl)-5-(1H-pyrazol-4-yl)pyrazolo[1,5-a]pyrimidine-3-carboxamide

To a solution of *N*-(1-cyclohexyl-3-(cyclopropylcarbamoyl)-1H-pyrazol-4-yl)-5-(1-(tetrahydro-2H-pyrazol-2-yl)-1H-pyridin-4-yl)pyrazolo[1,5-a]pyrimidine-3-carboxamide (70 mg, 0.1 mmol, 1.0 eq) in DCM (4.0 mL) was added HCl/dioxane (2 mL, 4 M) at rt. The reaction solution was reacted at rt for 1 h. Upon completion, the reaction solution was concentrated under reduced pressure. The residue was purified by HPLC to afford the title compound (yellow solid, 21.0 mg, yield 37%). **¹H NMR** (400 MHz, DMSO-*d*₆) δ 13.42 (s, 1H), 11.19 (s, 1H), 9.52 (s, 1H), 9.22 (d, *J* = 7.3 Hz, 1H), 8.79 (s, 1H), 8.55 (s, 1H), 8.44 (s, 1H), 8.35 (d, *J* = 4.0 Hz, 1H), 7.65 (d, *J* = 7.4 Hz, 1H), 4.29-4.16 (m, 1H), 3.04-2.86 (m, 1H), 2.10-2.02 (m, 2H), 1.88-1.74 (m, 4H), 1.71-1.64 (m, 1H), 1.46-1.36 (m, 2H), 1.30-1.25 (m, 1H), 0.85-0.81 (m, 2H), 0.73-0.66 (m, 2H). LCMS (ESI): m/z 460 [M+H]⁺.

### Preparation Example 158. Synthesis of Compound A283

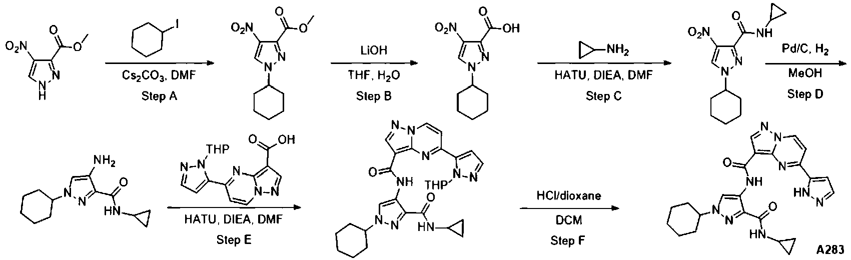

### Step A: Methyl 1-cyclohexyl-4-nitro-1H-pyrazole-3-carboxylate

To a solution of methyl 4-nitro-1H-pyrazole-3-carboxylate (7 g, 40.9 mmol, 1.0 eq) and iodocyclohexane ((17.2 g, 81.8 mmol, 2.0 eq) in DMF (100.0 mL) was added cesium carbonate (26.7 g, 81.8 mmol, 2.0 eq) at rt. The reaction solution was reacted at 90°C for 16 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (pale yellow oil, 2.04 g, yield 19%).

### Step B: 1-Cyclohexyl-4-nitro-1H-pyrazole-3-carboxylic acid

To a mixed solution of methyl 1-cyclohexyl-4-nitro-1H-pyrazole-3-carboxylate (670 mg, 2.6 mmol, 1.0 eq) in THF (15.0 mL) and water (3.0 mL) was added lithium hydroxide (222 mg, 5.2 mmol, 2.0 eq) at rt. The reaction solution was reacted at rt for 16 h. Upon completion, the reaction solution was adjusted to pH 4-5 with 1 N HCl and concentrated under reduced pressure to afford the title compound (white solid, 620 mg, crude).

### Step C: 1-cyclohexyl-N-cyclopropyl-4-nitro-1H-pyrazole-3-carboxamide

To a solution of 1-cyclohexyl-4-nitro-1H-pyrazole-3-carboxylic acid (620 mg, crude) and cyclopropylamine (222 mg, 3.9 mmol, 1.5 eq) in DMF (15.0 mL) were added DIEA (1.4 mL, 7.8 mmol, 3.0 eq) and HATU (1.9 g, 5.2 mmol, 1.0 eq) at rt. The reaction solution was reacted at rt for 4 h. Upon completion, the reaction solution was poured into NaHCO₃ solution and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (colorless oil, 700 mg, yield 97%).

### Step D: 4-amino-1-cyclohexyl-N-cyclopropyl-1H-pyrazole-3-carboxamide

To a solution of 1-cyclohexyl-N-cyclopropyl-4-nitro-1H-pyrazole-3-carboxamide (700 mg, 2.5 mmol, 1.0 eq) in MeOH (50.0 mL) was added palladium on carbon (70 mg, 10%) at rt. The reaction solution was purged with H₂ (×3), and then reacted at rt for 16 h. Upon completion, the reaction solution was filtered, and the filtrate was concentrated under reduced pressure to afford the title compound (brown oil, 560 mg, crude).

### Step E: N-(1-cyclohexyl-3-(cyclopropylcarbamoyl)-1H-pyrazol-4-yl)-5-(1-(tetrahydro-2H-pyrazol-2-yl)-1H-pyridin-4-yl)pyrazolo[1,5-a]pyrimidine-3-carboxamide

To a solution of 4-amino-1-cyclohexyl-N-cyclopropyl-1H-pyrazole-3-carboxamide (60 mg, crude) and 5-(1-(tetrahydro-2H-pyran-2-yl)-1H-pyrazol-5-yl)pyrazolo[1,5-a]pyrimidine-3-carboxylic acid (91 mg, 0.3 mmol, 1.2 eq) in DMF (10.0 mL) were added DIEA (156 mg, 1.2 mmol, 3.0 eq) and HATU (183 mg, 0.5 mmol, 2.0 eq) at rt. The reaction solution was reacted at rt for 16 h. Upon completion, the reaction solution was poured into NaHCO₃ solution and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow oil, 70 mg, yield 53%).

### Step F: N-(1-cyclohexyl-3-(cyclopropylcarbamoyl)-1H-pyrazol-4-yl)-5-(1H-pyrazol-5-yl)pyrazolo[1,5-a]pyrimidine-3-carboxamide

To a solution of *N*-(1-cyclohexyl-3-(cyclopropylcarbamoyl)-1H-pyrazol-4-yl)-5-(1-(tetrahydro-2H-pyrazol-2-yl)-1H-pyridin-4-yl)pyrazolo[1,5-a]pyrimidine-3-carboxamide (70 mg, 0.1 mmol, 1.0 eq) in DCM (4.0 mL) was added HCl/dioxane (2 mL, 4 M) at rt. The reaction solution was reacted at rt for 1 h. Upon completion, the reaction solution was concentrated under reduced pressure. The residue was purified by HPLC to afford the title compound (yellow solid, 11.0 mg, yield 19%). **¹H NMR** (400 MHz, DMSO-*d*₆) δ 14.01 (d, *J* = 464.1 Hz, 1H), 11.42 *(d, J=* 180.0 Hz, 1H), 9.31 (dd, *J* = 68.0, 7.3 Hz, 1H), 8.88 (d, *J* = 4.2 Hz, 0.5H), 8.64 (d, *J* = 20.1 Hz, 1H), 8.44 (d, *J* = 5.4 Hz, 1.5H), 8.31 (d, *J* = 3.9 Hz, 0.4H), 7.96 (s, 0.4H), 7.82 (dd, *J* = 13.4, 7.3 Hz, 1H), 7.75 (s, 0.6H), 7.45 (s, 0.6H), 4.30-4.18 (m, 1H), 3.18-2.86 (m, 1H), 2.08-2.01 (m, 2H), 1.87-1.74 (m, 4H), 1.71-1.65 (m, 1H), 1.47-1.37 (m, 2H), 1.31-1.26 (m, 1H), 0.89-0.71 (m, 4H). LCMS (ESI): m/z 460 [M+H]⁺.

### Preparation Example 159. Synthesis of Compound A284

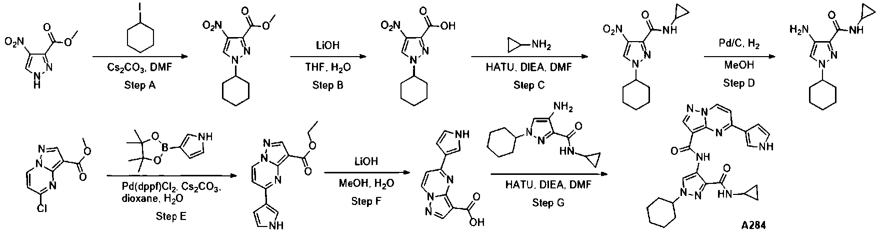

### Step A: Methyl 1-cyclohexyl-4-nitro-1H-pyrazole-3-carboxylate

To a solution of methyl 4-nitro-1H-pyrazole-3-carboxylate (7 g, 40.9 mmol, 1.0 eq) and iodocyclohexane ((17.2 g, 81.8 mmol, 2.0 eq) in DMF (100.0 mL) was added cesium carbonate (26.7 g, 81.8 mmol, 2.0 eq) at rt. The reaction solution was reacted at 90°C for 16 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (pale yellow oil, 2.04 g, yield 19%).

### Step B: 1-Cyclohexyl-4-nitro-1H-pyrazole-3-carboxylic acid

To a mixed solution of methyl 1-cyclohexyl-4-nitro-1H-pyrazole-3-carboxylate (670 mg, 2.6 mmol, 1.0 eq) in THF (15.0 mL) and water (3.0 mL) was added lithium hydroxide (222 mg, 5.2 mmol, 2.0 eq) at rt. The reaction solution was reacted at rt for 16 h. Upon completion, the reaction solution was adjusted to pH 4-5 with 1 N HCl and concentrated under reduced pressure to afford the title compound (white solid, 620 mg, crude).

### Step C: 1-cyclohexyl-N-cyclopropyl-4-nitro-1H-pyrazole-3-carboxamide

To a solution of 1-cyclohexyl-4-nitro-1H-pyrazole-3-carboxylic acid (620 mg, crude) and cyclopropylamine (222 mg, 3.9 mmol, 1.5 eq) in DMF (15.0 mL) were added DIEA (1.4 mL, 7.8 mmol, 3.0 eq) and HATU (1.9 g, 5.2 mmol, 1.0 eq) at rt. The reaction solution was reacted at rt for 4 h. Upon completion, the reaction solution was poured into NaHCO₃ solution and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (colorless oil, 700 mg, yield 97%).

### Step D: 4-amino-1-cyclohexyl-N-cyclopropyl-1H-pyrazole-3-carboxamide

To a solution of 1-cyclohexyl-N-cyclopropyl-4-nitro-1H-pyrazole-3-carboxamide (700 mg, 2.5 mmol, 1.0 eq) in MeOH (50.0 mL) was added palladium on carbon (70 mg, 10%) at rt. The reaction solution was purged with H₂ (×3), and then stirred at rt for 16 h. Upon completion, the reaction solution was filtered, and the filtrate was concentrated under reduced pressure to afford the title compound (brown oil, 560 mg, crude).

### Step E: Ethyl 5-(1H-pyrrol-3-yl)pyrazolo[1,5-a]pyrimidine-3-carboxylate

To a mixed solution of methyl 5-chloropyrazolo[1,5-a]pyrimidine-3-carboxylate (500 mg, 2.2 mmol, 1.0 eq) and 3-(4,4,5,5-tetramethyl-1,3,2-dioxaborolan-2-yl)-1H-pyrrole (644 mg, 3.3 mmol, 1.5 eq) in 1,4-dioxane (15.0 mL) and water (3.0 mL) were added Pd(dppf)Cl₂ (162 mg, 0.2 mmol, 0.1 eq) and cesium carbonate (2.2 g, 6.7 mmol, 3.0 eq) at rt. The reaction solution was reacted at 90°C for 16 h. Upon completion, the reaction solution was concentrated under reduced pressure. The residue was purified by column chromatography to afford the title compound (yellow solid, 470.0 mg, yield 79%).

### Step F: 5-(1H-pyrrol-3-yl)pyrazolo[1,5-a]pyrimidine-3-carboxylic acid

To a mixed solution of ethyl 5-(1H-pyrrol-3-yl)pyrazolo[1,5-a]pyrimidine-3-carboxylate (470 mg, 1.8 mmol, 1.0 eq) in MeOH (20.0 mL) and water (10.0 mL) was added lithium hydroxide (88 mg, 3.7 mmol, 2.0 eq) at rt. The reaction solution was reacted at 50°C for 16 h. Upon completion, the reaction solution was adjusted to pH 4-5 with 1 N HCl, resulting in the precipitation of a solid. The solid was filtered, collected, and dried to afford the title compound (brown solid, 370 mg, yield 88%).

### Step G: N-(1-cyclohexyl-3-(cyclopropylcarbamoyl)-1H-pyrazol-4-yl)-5-(1H-pyrrol-3-yl)pyrazolo[1,5-a]pyrimidine-3-carboxamide

To a solution of 4-amino-1-cyclohexyl-N-cyclopropyl-1H-pyrazole-3-carboxamide (80 mg, crude) and 5-(1H-pyrrol-3-yl)pyrazolo[1,5-a]pyrimidine-3-carboxylic acid (88 mg, 0.4 mmol, 1.2 eq) in DMF (10.0 mL) were added DIEA (0.27 mL, 1.5 mmol, 5.0 eq) and HATU (245 mg, 0.6 mmol, 2.0 eq) at rt. The reaction solution was reacted at rt for 16 h. Upon completion, the reaction solution was poured into NaHCO₃ solution and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (white solid, 41.1 mg, yield 28%). **¹H NMR** (400 MHz, DMSO-*d*₆) δ 11.48 (s, 1H), 11.09 (s, 1H), 9.05 (d, *J=* 7.4 Hz, 1H), 8.58 (s, 1H), 8.46 (d, *J* = 17.7 Hz, 2H), 8.25 (d, *J* = 4.2 Hz, 1H), 7.52 (d, *J* = 7.4 Hz, 1H), 7.37 (s, 1H), 6.91 (d, *J* = 2.2 Hz, 1H), 4.27-4.15 (m, 1H), 3.02-2.90 (m, 1H), 2.07-2.01 (m, 2H), 1.88-1.74 (m, 4H), 1.71-1.63 (m, 1H), 1.46-1.34 (m, 2H), 1.30-1.21 (m, 1H), 0.82-0.75 (m, 2H), 0.73-0.67 (m, 2H). LCMS (ESI): m/z 459 [M+H]⁺.

### Preparation Example 160. Synthesis of Compound A285

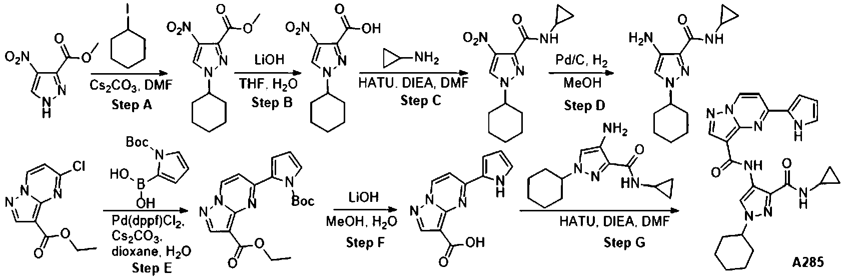

### Step A: Methyl 1-cyclohexyl-4-nitro-1H-pyrazole-3-carboxylate

To a solution of methyl 4-nitro-1H-pyrazole-3-carboxylate (7 g, 40.9 mmol, 1.0 eq) and iodocyclohexane ((17.2 g, 81.8 mmol, 2.0 eq) in DMF (100.0 mL) was added cesium carbonate (26.7 g, 81.8 mmol, 2.0 eq) at rt. The reaction solution was reacted at 90°C for 16 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (pale yellow oil, 2.04 g, yield 19%).

### Step B: 1-Cyclohexyl-4-nitro-1H-pyrazole-3-carboxylic acid

To a mixed solution of methyl 1-cyclohexyl-4-nitro-1H-pyrazole-3-carboxylate (670 mg, 2.6 mmol, 1.0 eq) in THF (15.0 mL) and water (3.0 mL) was added lithium hydroxide (222 mg, 5.2 mmol, 2.0 eq) at rt. The reaction solution was reacted at rt for 16 h. Upon completion, the reaction solution was adjusted to pH 4-5 with 1 N HCl and concentrated under reduced pressure to afford the title compound (white solid, 620 mg, crude).

### Step C: 1-cyclohexyl-N-cyclopropyl-4-nitro-1H-pyrazole-3-carboxamide

To a solution of 1-cyclohexyl-4-nitro-1H-pyrazole-3-carboxylic acid (620 mg, crude) and cyclopropylamine (222 mg, 3.9 mmol, 1.5 eq) in DMF (15.0 mL) were added DIEA (1.4 mL, 7.8 mmol, 3.0 eq) and HATU (1.9 g, 5.2 mmol, 1.0 eq) at rt. The reaction solution was reacted at rt for 4 h. Upon completion, the reaction solution was poured into NaHCO₃ solution and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (colorless oil, 700 mg, yield 97%).

### Step D: 4-amino-1-cyclohexyl-N-cyclopropyl-1H-pyrazole-3-carboxamide

To a solution of 1-cyclohexyl-N-cyclopropyl-4-nitro-1H-pyrazole-3-carboxamide (700 mg, 2.5 mmol, 1.0 eq) in MeOH (50.0 mL) was added palladium on carbon (70 mg, 10%) at rt. The reaction solution was purged with H₂ (×3), and then stirred at rt for 16 h. Upon completion, the reaction solution was filtered, and the filtrate was concentrated under reduced pressure to afford the title compound (brown oil, 560 mg, crude).

### Step E: Ethyl 5-(1-(tert-Butoxycarbonyl)-1H-pyrrol-2-yl)pyrazolo[1,5-a]pyrimidine-3-carboxylate

To a mixed solution of ethyl 5-chloropyrazolo[1,5-a]pyrimidine-3-carboxylate (1.0 g, 4.4 mmol, 1.0 eq) and (1-(tert-butoxycarbonyl)-1H-pyrrol-2-yl)boronic acid (1.4 g, 6.6 mmol, 1.5 eq) in dioxane (20.0 mL) and water (4.0 mL) were added Pd(dppf)Cl₂ (320 mg, 0.4 mmol, 0.1 eq) and cesium carbonate (4.3 g, 13.3 mmol, 3.0 eq) at rt. The reaction solution was reacted at 90°C for 16 h. Upon completion, the reaction solution was concentrated under reduced pressure. The residue was purified by column chromatography to afford the title compound (yellow solid, 1.3 g, yield 82%).

### Step F: 5-(1H-pyrrol-2-yl)pyrazolo[1,5-a]pyrimidine-3-carboxylic acid

To a mixed solution of ethyl 5-(1-(tert-butoxycarbonyl)-1H-pyrrol-2-yl)pyrazolo[1,5-a]pyrimidine-3-carboxylate (1.3 g, 3.6 mmol, 1.0 eq) in MeOH (20.0 mL) and water (10.0 mL) was added NaOH (290 mg, 7.3 mmol, 2.0 eq) at rt. The reaction solution was reacted at 50°C for 16 h. Upon completion, the reaction solution was adjusted to pH 4-5 with 1 N HCl, resulting in the precipitation of a solid. The solid was filtered, collected, and dried to afford the title compound (brown solid, 850 mg, yield 71%).

### Step G: N-(1-cyclohexyl-3-(cyclopropylcarbamoyl)-1H-pyrazol-4-yl)-5-(1H-pyrrol-2-yl)pyrazolo[1,5-a]pyrimidine-3-carboxamide

To a solution of 4-amino-1-cyclohexyl-N-cyclopropyl-1H-pyrazole-3-carboxamide (90 mg, 0.4 mmol, 1.0 eq) and 5-(1H-pyrrol-2-yl)pyrazolo[1,5-a]pyrimidine-3-carboxylic acid (119 mg, 0.4 mmol, 1.0 eq) in DMF (15.0 mL) were added DIEA (234 mg, 2.0 mmol, 5.0 eq) and HATU (275 mg, 0.8 mmol, 2.0 eq) at rt. The reaction solution was reacted at rt for 16 h. Upon completion, the reaction solution was poured into NaHCO₃ solution and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (white solid, 41.1 mg, yield 28%). **¹H NMR** (400 MHz, DMSO-*d*₆) δ 12.78 (s, 1H), 11.41 (s, 1H), 9.11 (d, *J* = 7.4 Hz, 1H), 8.72 (d, *J* = 4.0 Hz, 1H), 8.51 (s, 1H), 8.45 (s, 1H), 7.62 (d, *J* = 7.5 Hz, 1H), 7.32 (d, *J* = 15.3 Hz, 2H), 6.38-6.32 (m, 1H), 4.30-4.18 (m, 1H), 3.11-2.94 (m, 1H), 2.10-2.02 (m, 2H), 1.89-1.65 (m, 5H), 1.48-1.35 (m, 2H), 1.28-1.22 (m, 1H), 0.89-0.72 (m, 4H). LCMS (ESI): m/z 459 [M+H]⁺.

### Preparation Example 161. Synthesis of Compound A286

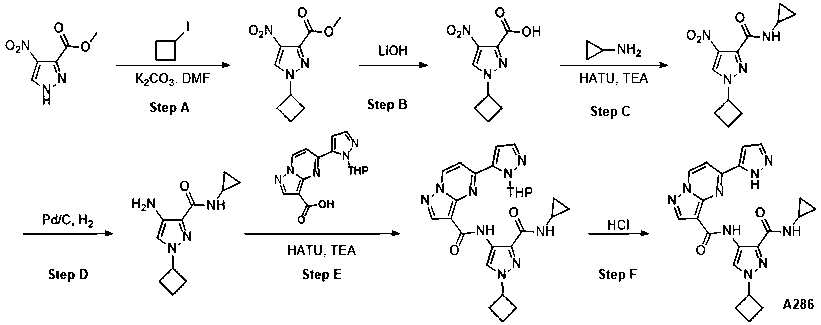

### Step A: Methyl 1-cyclobutyl-4-nitro-1H-pyrazole-3-carboxylate

To a solution of methyl 4-nitro-1H-pyrazole-3-carboxylate (5.0 g, 29.22 mmol, 1.0 eq) and iodocyclobutane (5.85 g, 32.14 mmol, 1.1 eq) in DMF (50.0 mL) was added potassium carbonate (14.28 g, 43.83 mmol, 1.5 eq) at rt. The reaction solution was reacted at 90°C for 3 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 2.5 g, yield 38.0%).

### Step B: 1-Cyclobutyl-4-nitro-1H-pyrazole-3-carboxylic acid

To a mixed solution of methyl 1-cyclobutyl-4-nitro-1H-pyrazole-3-carboxylate (1.0 g, 4.44 mmol, 1.0 eq) in MeOH (15.0 mL) and water (5.0 mL) was added lithium hydroxide (0.32 g, 13.29 mmol, 3.0 eq) at rt. The reaction solution was reacted at rt for 16 h. Upon completion, the reaction solution was poured into water, adjusted to pH 6 with 1 N HCl, and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 900 mg, yield 96.0%).

### Step C: 1-Cyclobutyl-N-cyclopropyl-4-nitro-1H-pyrazole-3-carboxamide

To a solution of 1-cyclobutyl-4-nitro-1H-pyrazole-3-carboxylic acid (1.0 g, 4.73 mmol, 1.0 eq) and cyclopropylamine (0.40 g, 7.09 mmol, 1.5 eq) in DMF (20.0 mL) at rt were added TEA (1.48 g, 14.22 mmol, 3.0 eq) and HATU (3.60 g, 9.46 mmol, 2.0 eq). The reaction solution was reacted at rt for 16 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 1.0 g, yield 84.4%).

### Step D: 4-Amino-1-cyclobutyl-N-cyclopropyl-1H-pyrazole-3-carboxamide

To a solution of 1-cyclobutyl-N-cyclopropyl-4-nitro-1H-pyrazole-3-carboxamide (1.0 g, 4.00 mmol, 1.0 eq) in ethanol (15.0 mL) was added palladium on carbon (0.25 g, 2.35 mmol, 10% purity, 0.2 eq) at rt. The reaction solution was purged with H₂ (×3), and then stirred at rt for 16 h. Upon completion, the reaction solution was filtered, and the filtrate was concentrated under reduced pressure to afford the title compound (colorless oil, 850 mg, yield 96.60%).

### Step E: N-(1-cyclobutyl-3-(cyclopropylcarbamoyl)-1H-pyrazol-4-yl)-5-(1-(tetrahydro-2H-pyrazol-2-yl)-1H-pyrazol-5-yl)pyrazolo[1,5-a]pyrimidine-3-carboxamide

To a solution of 4-amino-1-cyclobutyl-N-cyclopropyl-1H-pyrazole-3-carboxamide (100 mg, 0.45 mmol, 1.0 eq) and methyl 1-cyclobutyl-4-nitro-1H-pyrazole-3-carboxylate (170.68 mg, 0.55 mmol, 1.2 eq) in DMF (10.0 mL) were added TEA (176.03 mg, 1.36 mmol, 3.0 eq) and HATU (345.23 mg, 0.91 mmol, 2.0 eq) at rt. The reaction solution was reacted at rt for 16 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 110 mg, yield 47.0%).

### Step F: N-(1-cyclobutyl-3-(cyclopropylcarbamoyl)-1H-pyrazol-4-yl)-5-(1H-pyrazol-5-yl)pyrazolo[1,5-a]pyrimidine-3-carboxamide

To a solution of *N*-(1-cyclobutyl-3-(cyclopropylcarbamoyl)-1H-pyrazol-4-yl)-5-(1-(tetrahydro-2H-pyrazol-2-yl)-1H-pyrazol-5-yl)pyrazolo[1,5-a]pyrimidine-3-carboxamide (100 mg, 0.19 mmol, 1.0 eq) in DCM (10.0 mL) was added HCl/dioxane (2.0 mL, 4 M in dioxane) at rt. The reaction solution was reacted at rt for 20 min. Upon completion, the reaction solution was concentrated under reduced pressure. The residue was purified by HPLC to afford the title compound (white solid, 15.0 mg, yield 17.9%). ¹HNMR (400 MHz, DMSO-*d*₆) δ 14.01 (d, *J* = 459.8 Hz, 1H), 11.41 (d, *J* = 177.7 Hz, 1H), 9.52-9.09 (m, 1H), 9.05-8.41 (m, 3H), 8.18 (d, *J* = 171.6 Hz, 1H), 7.81 (s, 1H), 7.60 (d, *J* = 119.7 Hz, 1H), 4.95 (d, *J* = 7.6 Hz, 1H), 3.03 (d, *J* = 106.6 Hz, 1H), 2.63-2.52 (m, 2H), 2.42 (d, *J* = 7.3 Hz, 2H), 1.83 (dt, *J* = 9.9, 6.9 Hz, 2H), 0.93-0.71 (m, 4H). LC-MS (ESI): m/z 432 [M+H]⁺.

### Preparation Example 162. Synthesis of Compound A287

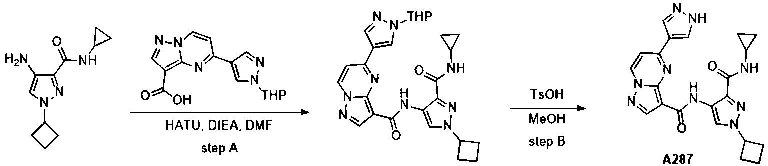

### Step A: N-(1-cyclobutyl-3-(cyclopropylcarbamoyl)-1H-pyrazol-4-yl)-5-(1-(tetrahydro-2H-pyrazol-2-yl)-1H-pyridin-4-yl)pyrazolo[1,5-a]pyrimidine-3-carboxamide

To a solution of 4-amino-1-cyclobutyl-N-cyclopropyl-1H-pyrazole-3-carboxamide (150 mg, 0.7 mmol, 1.0 eq) and 5-(1-(tetrahydro-2H-pyran-2-yl)-1H-pyrazol-4-yl)pyrazolo[1,5-a]pyrimidine-3-carboxylic acid (213 mg, 0.7 mmol, 1.0 eq) in DMF (20.0 mL) were added DIEA (0.6 mL, 3.4 mmol, 5.0 eq) and HATU (518 mg, 1.4 mmol, 2.0 eq) at rt. The reaction solution was reacted at rt for 16 h. Upon completion, the reaction solution was poured into NaHCO₃ solution and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow oil, 150 mg, yield 43.0%).

### Step B: N-(1-cyclobutyl-3-(cyclopropylcarbamoyl)-1H-pyrazol-4-yl)-5-(1H-pyrazol-4-ylpyrazolo[1,5-a]pyrimidine-3-carboxamide

To a solution of *N-*(1-cyclobutyl-3-(cyclopropylcarbamoyl)-1H-pyrazol-4-yl)-5-(1-(tetrahydro-2H-pyrazol-2-yl)-1H-pyridin-4-yl)pyrazolo[1,5-a]pyrimidine-3-carboxamide (80 mg, 0.2 mmol, 1.0 eq) in MeOH (10.0 mL) was added p-toluenesulfonic acid (80 mg, 0.5 mmol, 3.0 eq) at rt. The reaction solution was reacted at rt for 16 h. Upon completion, the reaction solution was poured into aqueous NaHCO₃, and extracted with DCM (×3). The combined organic phases were washed with saturated aqueous NaCl, dried over anhydrous Na₂SO₄, filtered, and concentrated under reduced pressure. The residue was purified by HPLC to afford the title compound (white solid, 25.0 mg, yield 30.0%). **¹H NMR** (400 MHz, DMSO-*d*₆) δ 13.43 (s, 1H), 11.20 (s, 1H), 9.53 (s, 1H), 9.22 (d, *J* = 7.4 Hz, 1H), 8.80 (s, 1H), 8.56 (s, 1H), 8.50 (s, 1H), 8.44 (d, *J* = 4.1 Hz, 1H), 7.66 (d, *J* = 7.4 Hz, 1H), 5.01-4.88 (m, 1H), 2.99-2.92 (m, 1H), 2.60-2.52 (m, 2H), 2.46-2.38 (m, 2H), 1.87-1.77 (m, 2H), 0.87-0.81 (m, 2H), 0.76-0.65 (m, 2H). LCMS (ESI): m/z 432 [M+H]⁺.

### Preparation Example 163. Synthesis of Compound A288

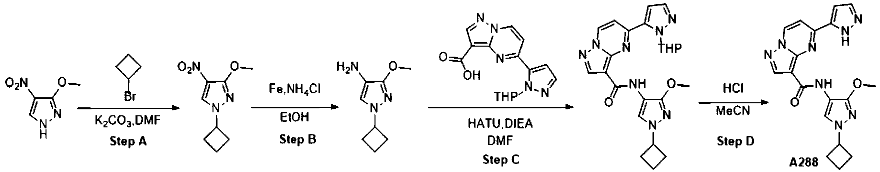

### Step A: 1-cyclobutyl-3-methoxy-4-nitro-1H-pyrazole

To a solution of 3-methoxy-4-nitro-1H-pyrazole (500 mg, 3.4 mmol, 1.0 eq) and bromocyclobutane (465 mg, 3.4 mmol, 1.0 eq) in DMF (100.0 mL) was added potassium carbonate (1.4 g, 10.2 mmol, 3.0 eq) at rt. The reaction solution was reacted at 80°C for 3 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 500 mg, yield 72.0%).

### Step B: 1-cyclobutyl-3-methoxy-1H-pyrazol-4-amine

To a mixed solution of 1-cyclobutyl-3-methoxy-4-nitro-1H-pyrazole (500 mg, 2.5 mol, 1.0 eq) in ethanol (5.0 mL) and water (1.0 mL) at rt were added iron powder (1.4 g, 25.0 mmol, 10.0 eq) and ammonium chloride (1.3 g, 25.0 mmol, 10.0 eq). The reaction solution was reacted at 80°C for 3 h. Upon completion, the reaction solution was cooled to rt and then filtered. The filtrate was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 350 mg, yield 82.0%).

### Step C: N-(1-cyclobutyl-3-methoxy-1H-pyrazol-4-yl)-5-(1-(tetrahydro-2H-pyrazol-2-yl)-1H-pyrazin-5-yl)pyrazolo[1,5-a]pyrimidine-3-carboxamide

To a solution of 1-cyclobutyl-3-methoxy-1H-pyrazol-4-amine (50 mg, 0.3 mmol, 1.0 eq) and 5-(1-(tetrahydro-2H-pyran-2-yl)-1H-pyrazol-5-yl)pyrazolo[1,5-a]pyrimidine-3-carboxylic acid (103 mg, 0.3 mmol, 1.1 eq) in DMF (5.0 mL) were added DIEA (116 mg, 0.9 mmol, 3.0 eq) and HATU (170 mg, 0.45 mmol, 1.5 eq) at rt. The reaction solution was reacted at rt for 3 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 70 mg, yield 50.0%).

### Step D: N-(1-cyclobutyl-3-methoxy-1H-pyrazol-4-yl)-5-(1H-pyrazol-5-yl)pyrazolo[1,5-a]pyrimidine-3-carboxamide

To a solution of *N*-(1-cyclobutyl-3-methoxy-1H-pyrazol-4-yl)-5-(1-(tetrahydro-2H-pyrazol-2-yl)-1H-pyrazin-5-yl)pyrazolo[1,5-a]pyrimidine-3-carboxamide (70 mg, 0.15 mmol, 1.0 eq) in acetonitrile (5.0 mL) was added HCl/dioxane (2 mL, 4 M) at rt. The reaction solution was reacted at rt for 1 h. Upon completion, the reaction solution was concentrated under reduced pressure. The residue was purified by HPLC to afford the title compound (white solid, 15.0 mg, yield 26%). **¹H NMR** (400 MHz, DMSO-*d*₆) δ 9.99 (s, 1H), 9.27 (d, *J* = 7.3 Hz, 1H), 8.59 (s, 1H), 8.06 (d, *J* = 2.1 Hz, 2H), 7.79 (d, *J* = 7.3 Hz, 1H), 7.25 (d, *J* = 2.3 Hz, 1H), 4.69 (d, *J* = 8.3 Hz, 1H), 4.04 (s, 3H), 2.45 (dd, *J* = 15.7, 6.0 Hz, 2H), 2.37-2.30 (m, 2H), 1.81-1.69 (m, 2H). LCMS (ESI): m/z 379 [M+H]⁺.

### Preparation Example 164. Synthesis of Compound A289

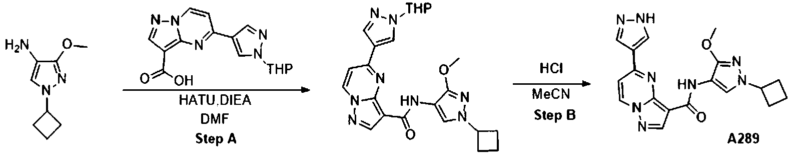

### Step A: N-(1-cyclobutyl-3-methoxy-1H-pyrazol-4-yl)-5-(1-(tetrahydro-2H-pyrazol-2-yl)-1H-pyrazin-4-yl)pyrazolo[1,5-a]pyrimidine-3-carboxamide

To a solution of 1-cyclobutyl-3-methoxy-1H-pyrazol-4-amine (50 mg, 0.3 mmol, 1.0 eq) and 5-(1-(tetrahydro-2H-pyran-2-yl)-1H-pyrazol-4-yl)pyrazolo[1,5-a]pyrimidine-3-carboxylic acid (103 mg, 0.3 mmol, 1.1 eq) in DMF (5.0 mL) were added DIEA (116 mg, 0.9 mmol, 3.0 eq) and HATU (170 mg, 0.45 mmol, 1.5 eq) at rt. The reaction solution was reacted at rt for 3 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 70 mg, yield 50.0%).

### Step B: N-(1-cyclobutyl-3-methoxy-1H-pyrazol-4-yl)-5-(1H-pyrazol-4-yl)pyrazolo[1,5-a]pyrimidine-3-carboxamide

To a solution of *N*-(1-cyclobutyl-3-methoxy-1H-pyrazol-4-yl)-5-(1-(tetrahydro-2H-pyrazol-2-yl)-1H-pyrazin-4-yl)pyrazolo[1,5-a]pyrimidine-3-carboxamide (70 mg, 0.15 mmol, 1.0 eq) in acetonitrile (5.0 mL) was added HCl/dioxane (2 mL, 4 M) at rt. The reaction solution was reacted at rt for 1 h. Upon completion, the reaction solution was concentrated under reduced pressure. The residue was purified by HPLC to afford the title compound (white solid, 15.0 mg, yield 26%). **¹H NMR** (400 MHz, DMSO-*d*₆) δ 13.57 (s, 1H), 10.07 (s, 1H), 9.27 (d, *J* = 7.3 Hz, 1H), 8.89-8.30 (m, 3H), 8.04 (s, 1H), 7.64 (d, *J* = 7.3 Hz, 1H), 4.70 (p, *J* = 8.3 Hz, 1H), 4.05 (s, 3H), 2.49-2.38 (m, 2H), 2.33 (dt, *J* = 11.3, 6.9 Hz, 2H), 1.83-1.68 (m, 2H). LCMS (ESI): m/z 379 [M+H]⁺.

### Preparation Example 165. Synthesis of Compound A290

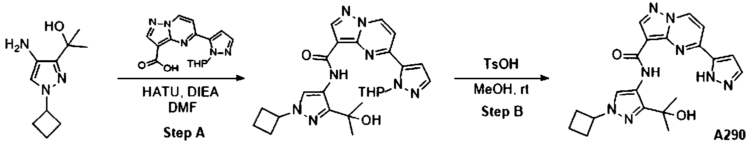

### Step A: N-(1-cyclobutyl-3-(2-hydroxypropan-2-yl)-1H-pyrazol-4-yl)-5-(1-(tetrahydro-2H-pyran-2-yl)-1H-pyrazol-5-yl)pyrazolo[1,5-a]pyrimidine-3-carboxamide

To a solution of 5-(1-(tetrahydro-2H-pyran-2-yl)-1H-pyrazol-5-yl)pyrazolo[1,5-a]pyrimidine-3-carboxylic acid (200 mg, 0.64 mmol, 1.0 eq) and 2-(4-amino-1-cyclobutyl-1H-pyrazol-3-yl)propan-2-ol (150 mg, 0.76 mmol, 1.2 eq) in DMF (5.0 mL) were added DIEA (248 mg, 1.91 mmol, 3.0 eq) and HATU (365 mg, 0.95 mmol, 1.5 eq) at rt. The reaction solution was reacted at rt for 2 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 200 mg, yield 64%).

### Step B: N-(1-cyclobutyl-3-(2-hydroxypropan-2-yl)-1H-pyrazol-4-yl)-5-(1H-pyrazol-5-yl)pyrazolo[1,5-a]pyrimidine-3-carboxamide

To a solution of N-(1-cyclobutyl-3-(2-hydroxypropan-2-yl)-1H-pyrazol-4-yl)-5-(1-(tetrahydro-2H-pyran-2-yl)-1H-pyrazol-5-yl)pyrazolo[1,5-a]pyrimidine-3-carboxamide (200 mg, 0.4 mmol, 1.0 eq) in MeOH (5.0 mL) was added p-toluenesulfonic acid (105 mg, 0.6 mmol, 1.2 eq) at rt. The reaction solution was reacted at rt for 1 h under N₂. Upon completion, the reaction solution was poured into aqueous NaHCO₃, and extracted with DCM (×3). The combined organic phases were washed with saturated aqueous NaCl, dried over anhydrous Na₂SO₄, filtered, and concentrated under reduced pressure. The residue was purified by HPLC to afford the title compound (yellow solid, 100 mg, yield 60%). **¹H NMR** (400 MHz, DMSO) δ 13.84 (d, *J* = 239.3 Hz, 1H), 10.53 (d, *J=* 108.4 Hz, 1H), 9.30 (dd, *J* = 56.6, 7.3 Hz, 1H), 8.62 (d, *J* = 19.7 Hz, 1H), 8.35 (d, *J* = 4.7 Hz, 1H), 7.98 (s, 1H), 7.88-7.70 (m, 2H), 7.40 (s, 1H), 7.13 (s, 1H), 5.91 (s, 1H), 4.87-4.68 (m, 1H), 2.40 (dt, *J* = 16.2, 8.3 Hz, 4H), 1.77 (dd, *J* = 17.1, 8.9 Hz, 2H), 1.62 (d, *J* = 21.6 Hz, 6H).

### Preparation Example 166. Synthesis of Compound A291

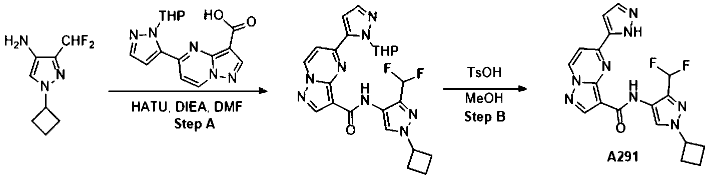

### Step A: N-(1-cyclobutyl-3-(difluoromethyl)-1H-pyrazol-4-yl)-5-(1-(tetrahydro-2H-pyrazol-2-yl)-1H-pyrazol-5-yl)pyrazolo[1,5-a]pyrimidine-3-carboxamide

To a solution of 1-cyclobutyl-3-(difluoromethyl)-1H-pyrazol-4-amine (80 mg, 0.42 mmol, 1.0 eq) and 5-(1-(tetrahydro-2H-pyran-2-yl)-1H-pyrazol-5-yl)pyrazolo[1,5-a]pyrimidine-3-carboxylic acid (160.6 mg, 0.51 mmol, 1.2 eq) in DMF (5.0 mL) were added DIEA (0.21 mL, 1.2 mmol, 3.0 eq) and HATU (325.0 mg, 0.85 mmol, 2.0 eq) at rt. The reaction solution was reacted at rt for 16 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow oil, 80 mg, yield 38.8%).

### Step B: N-(1-cyclobutyl-3-(difluoromethyl)-1H-pyrazol-4-yl)-5-(1H-pyrazol-5-yl)pyrazolo[1,5-a]pyrimidine-3-carboxamide

To a solution of *N*-(1-cyclobutyl-3-(difluoromethyl)-1H-pyrazol-4-yl)-5-(1-(tetrahydro-2H-pyrazol-2-yl)-1H-pyrazol-5-yl)pyrazolo[1,5-a]pyrimidine-3-carboxamide (80 mg, 0.16 mmol, 1.0 eq) in MeOH (5.0 mL) was added p-toluenesulfonic acid (34.2 mg, 0.20 mmol, 1.2 eq) at rt. The reaction solution was reacted at rt for 1 h under N₂. Upon completion, the reaction solution was poured into aqueous NaHCO₃, and extracted with DCM (×3). The combined organic phases were washed with saturated aqueous NaCl, dried over anhydrous Na₂SO₄, filtered, and concentrated under reduced pressure. The residue was purified by HPLC to afford the title compound (white solid, 27.3 mg, yield 41.3%). **¹H NMR** (400 MHz, DMSO-*d*₆) δ 13.61 (s, 1H), 9.78 (s, 1H), 9.29 (d, *J* = 7.3 Hz, 1H), 8.65 (s, 1H), 8.48 (s, 1H), 8.03 (d, *J* = 1.6 Hz, 1H), 7.84 (d, *J* = 7.3 Hz, 1H), 7.53-7.08 (m, 2H), 4.95 (p*, J* = 8.3 Hz, 1H), 2.57-2.37 (m, 4H), 1.90-1.69 (m, 2H). LCMS (ESI): m/z 399 [M+H]⁺.

### Preparation Example 167. Synthesis of Compound A292

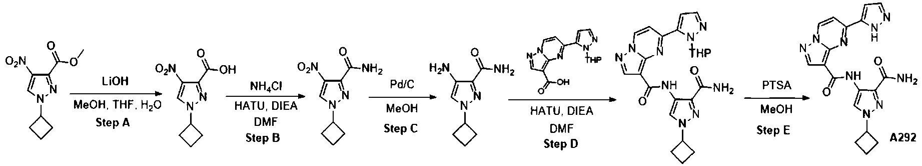

### Step A: 1-Cyclobutyl-4-nitro-1H-pyrazole-3-carboxylic acid

To a mixed solution of methyl 1-cyclobutyl-4-nitro-1H-pyrazole-3-carboxylate (500 mg, 2.2 mmol, 1.0 eq) in MeOH (2.0 mL), THF (2.0 mL), and water (2.0 mL) was added lithium hydroxide (159 mg, 6.6 mmol, 3.0 eq) at zero degrees Celsius. The reaction solution was reacted at rt for 2 h. Upon completion, the reaction solution was adjusted to pH 3 with 1 N HCl and concentrated under reduced pressure to afford the title compound (white solid, 400 mg, crude).

### Step B: 1-cyclobutyl-4-nitro-1H-pyrazole-3-carboxamide

To a solution of 1-cyclobutyl-4-nitro-1H-pyrazole-3-carboxylic acid (200 mg, crude) and ammonium chloride (60 mg, 1.1 mmol, 1.5 eq) in DMF (4.0 mL) at zero degrees Celsius were added DIEA (612 mg, 4.7 mmol, 3.0 eq) and HATU (720 mg, 1.9 mmol, 1.5 eq). The reaction solution was reacted at rt for 2 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 150 mg, yield 75%).

### Step C: 4-amino-1-cyclobutyl-1H-pyrazole-3-carboxamide

1-cyclobutyl-4-nitro-1H-pyrazole-3-carboxamide (100 mg, 0.5 mmol, 1.0 eq) in MeOH (5.0 mL) was added palladium on carbon (100 mg, 10%) at rt. The reaction solution was purged with H₂ (×3), and then stirred at rt for 1 h. Upon completion, the reaction solution was filtered, and the filtrate was concentrated under reduced pressure to afford the title compound (white solid, 60 mg, yield 70%).

### Step D: N-(3-Carbamoyl-1-cyclobutyl-1H-pyrazol-4-yl)-5-(1-(tetrahydro-2H-pyran-2-yl)-1H-pyrazol-5-yl)pyrazolo[1,5-a]pyrimidine-3-carboxamide

To a solution of 4-amino-1-cyclobutyl-1H-pyrazole-3-carboxamide (86 mg, 0.5 mmol, 1.5 eq) and 3-(1-(tetrahydro-2H-pyran-2-yl)-1H-pyrazol-5-yl)pyrazolo[1,5-a]pyrimidine-5-carboxylic acid (100 mg, 0.3 mmol, 1.0 eq) in DMF (5.0 mL) were added DIEA (123 mg, 0.9 mmol, 3.0 eq) and HATU (182 mg, 0.5 mmol, 1.5 eq) at zero degrees Celsius. The reaction solution was reacted at rt for 2 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 100 mg, yield 66%).

### Step E: N-(3-Carbamoyl-1-cyclobutyl-1H-pyrazol-4-yl)-5-(1H-pyrazol-5-yl)pyrazolo[1,5-a]pyrimidine-3-carboxamide

To a solution of N-(3-carbamoyl-1-cyclobutyl-1H-pyrazol-4-yl)-5-(1-(tetrahydro-2H-pyran-2-yl)-1H-pyrazol-5-yl)pyrazolo[1,5-a]pyrimidine-3-carboxamide (100 mg, 0.2 mmol, 1.0 eq) in MeOH (5.0 mL) was added p-toluenesulfonic acid (43 mg, 0.3 mmol, 1.2 eq) at rt. The reaction solution was reacted at rt for 1 h. Upon completion, the reaction solution was concentrated under reduced pressure. The residue was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (yellow solid, 25 mg, yield 30%). **¹H NMR** (400 MHz, DMSO) δ 13.76 (d, *J* = 240.0 Hz, 1H), 11.18 (d, *J* = 171.2 Hz, 1H), 9.32 (dd, *J* = 69.6, 7.3 Hz, 1H), 8.64 (d, *J* = 18.9 Hz, 1H), 8.51 (d, *J* = 6.1 Hz, 1H), 8.18-8.03 (m, 1H), 7.92 (d, *J* = 9.2 Hz, 1H), 7.82 (dd, *J* = 13.6, 7.3 Hz, 1H), 7.71 (d, *J* = 52.9 Hz, 1H), 7.50 (d, *J* = 20.3 Hz, 1H), 5.02-4.89 (m, 1H), 2.71-2.53 (m, 2H), 2.41 (dd, *J* = 22.6, 19.6 Hz, 2H), 1.81 (dd, *J* = 16.5, 8.7 Hz, 2H).

### Preparation Example 168. Synthesis of Compound A293

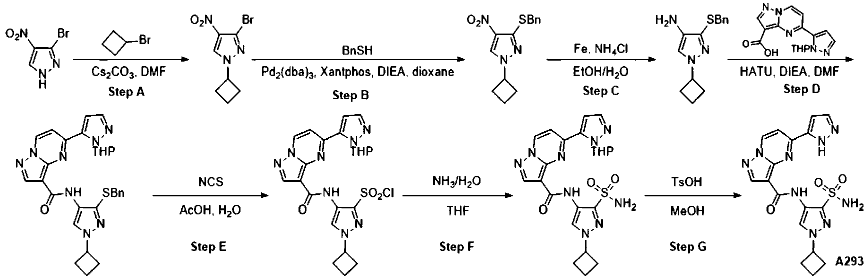

### Step A: 3-Bromo-1-cyclobutyl-4-nitro-1H-pyrazole

To a solution of 3-bromo-4-nitro-1H-pyrazole (1.50 g, 7.81 mmol, 1.0 eq) and bromocyclobutane (1.58 g, 11.72 mmol, 1.5 eq) in DMF (20.0 mL) was added cesium carbonate (6.36 g, 19.53 mmol, 2.5 eq) at rt. The reaction solution was reacted at 80°C for 18 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (brown oil, 1.0 g, yield 52.0%).

### Step B: 3-(Benzylthio)-1-cyclobutyl-4-nitro-1H-pyrazole

To a solution of 3-bromo-1-cyclobutyl-4-nitro-1H-pyrazole (500 mg, 2.03 mmol, 1.0 eq) and benzyl mercaptan (377.96 mg, 3.05 mmol, 2.0 eq) in dioxane (20.0 mL) were added Xantphos (117.58 mg, 0.20 mmol, 0.1 eq), Pd₂(dba)₃ (93.04 mg, 0.10 mmol, 0.05 eq), and DIEA (525.28 mg, 4.06 mmol, 2.0 eq) at rt. The reaction solution was reacted under N₂ at 100°C for 16 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (brown oil, 580 mg, yield 98.6%).

### Step C: 3-(Benzylthio)-1-cyclobutyl-1H-pyrazol-4-amine

To a mixed solution of 3-(benzylthio)-1-cyclobutyl-4-nitro-1H-pyrazole (500 mg, 1.73 mol, 1.0 eq) in ethanol (10.0 mL) and water (2.0 mL) at rt were added iron powder (482.46 mg, 8.64 mmol, 5.0 eq) and ammonium chloride (924.31 mg, 17.28 mmol, 10.0 eq). The reaction solution was reacted at 80°C for 3 h. Upon completion, the reaction solution was cooled to rt, and then filtered. The filtrate was concentrated under reduced pressure. The residue was purified by column chromatography to afford the title compound (black solid, 350 mg, yield 78.1%).

### Step D: N-(3-(Benzylthio)-1-cyclobutyl-1H-pyrazol-4-yl)-5-(1-(tetrahydro-2H-pyrazol-2-yl)-1H-pyrazin-5-yl)pyrazolo[1,5-a]pyrimidine-3-carboxamide

To a solution of 3-(benzylthio)-1-cyclobutyl-1H-pyrazol-4-amine (110 mg, 0.42 mmol, 1.0 eq) and 5-(1-(tetrahydro-2H-pyran-2-yl)-1H-pyrazol-5-yl)pyrazolo[1,5-a]pyrimidine-3-carboxylic acid (146.17 mg, 0.46 mmol, 1.1 eq) in DMF (4.0 mL) were added DIEA (219.26 mg, 1.70 mmol, 4.0 eq) and HATU (177.39 mg, 0.46 mmol, 1.1 eq) at rt. The reaction solution was reacted at rt for 1 h. Upon completion, the reaction solution was concentrated under reduced pressure. The residue was purified by column chromatography to afford the title compound (green solid, 100 mg, yield 42.5%).

### Step E: 1-cyclobutyl-4-(5-(1-(tetrahydro-2H-pyran-2-yl)-1H-pyrazol-5-yl)pyrazolo[1,5-a]pyrimidine-3-carboxamido)-1H-pyrazole-3-sulfonyl chloride

To *N*-(3-(benzylthio)-1-cyclobutyl-1H-pyrazol-4-yl)-5-(1-(tetrahydro-2H-pyrazol-2-yl)-1H-pyrazin-5-yl)pyrazolo[1,5-a]pyrimidine-3-carboxamide (100 mg, 0.18 mmol, 1.0 eq) was added AcOH (2.0 mL) at 0°C. Water (0.2 mL) and N-chlorosuccinimide (96.30 mg, 0.72 mmol, 4.0 eq) were then added under ice bath conditions. The reaction solution was reacted at rt for 3 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure to afford the title compound (gray solid, 130 mg, crude).

### Step F: N-(1-cyclobutyl-3-sulfamoyl-1H-pyrazol-4-yl)-5-(1-(tetrahydro-2H-pyrazol-2-yl)-1H-pyrazin-5-yl)pyrazolo[1,5-a]pyrimidine-3-carboxamide

To a solution of 1-cyclobutyl-4-(5-(1-(tetrahydro-2H-pyran-2-yl)-1H-pyrazol-5-yl)pyrazolo[1,5-a]pyrimidine-3-carboxamido)-1H-pyrazole-3-sulfonyl chloride (130 mg, crude) in THF (1.0 mL) was added an aqueous ammonia solution (1.0 mL) at rt. The reaction solution was reacted at rt for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure to afford the title compound (gray solid, 60.0 mg, crude).

### Step G: N-(1-cyclobutyl-3-sulfamoyl-1H-pyrazol-4-yl)-5-(1H-pyrazol-5-yl)pyrazolo[1,5-a]pyrimidine-3-carboxamide

To a solution of *N-*(1-cyclobutyl-3-sulfamoyl-1H-pyrazol-4-yl)-5-(1-(tetrahydro-2H-pyrazol-2-yl)-1H-pyrazin-5-yl)pyrazolo[1,5-a]pyrimidine-3-carboxamide (60 mg, crude) in MeOH (3.0 mL) was added p-toluenesulfonic acid (24.25 mg, 0.14 mmol, 1.2 eq) at rt. The reaction solution was reacted at rt for 3 h. Upon completion, the reaction solution was concentrated under reduced pressure. The residue was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (white solid, 12.0 g, yield 23.4%). **¹H NMR** (400 MHz, DMSO-*d*₆) δ 13.37 (d, *J* = 87.2 Hz, 1H), 10.17 (d, *J* = 62.5 Hz, 1H), 9.33 (dd, *J* = 74.2, 7.6 Hz, 1H), 8.67 (d, *J* = 22.2 Hz, 1H), 8.56 (s, 1H), 8.00 (s, 1H), 7.84 (d, *J* = 7.3 Hz, 1H), 7.65 (d, *J* = 111.0 Hz, 3H), 4.99 (p, *J* = 8.4 Hz, 1H), 2.59-2.35 (m, 4H), 1.98-1.71 (m, 2H). LCMS (ESI): m/z 428 (M+H)⁺.

### Preparation Example 169. Synthesis of Compound A294

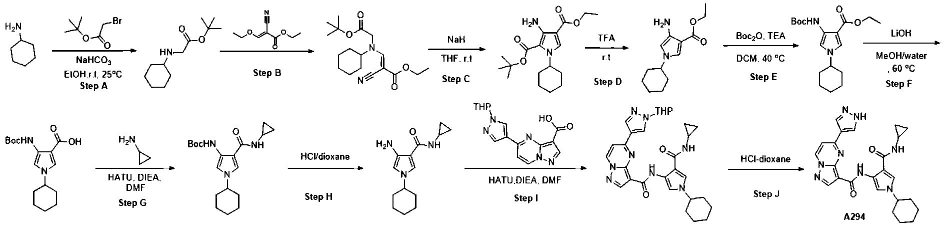

### Step A: Cyclohexylglycine tert-butyl ester

To a solution of cyclohexylamine (8.0 g, 80.0 mmol, 4.0 eq) and NaHCO₃ (1.6 g, 20.0 mmol, 1.0 eq) in ethanol (100.0 mL) at rt was added a solution of tert-butyl bromoacetate (4.0 g, 20.0 mmol, 1.0 eq) in ethanol (5 mL). The reaction solution was reacted at rt for 12 h. Upon completion, the reaction solution was concentrated under reduced pressure, poured into water, and extracted with DCM (×3). The combined organic phases were washed with saturated aqueous NaCl, dried over anhydrous Na₂SO₄, filtered, and concentrated under reduced pressure. The residue was purified by column chromatography to afford the title compound (yellow oil, 2.5 g, yield 58.1%).

### Step B: (E)-ethyl 3-((2-(tert-butoxy)-2-oxoethyl)(cyclohexyl)amino)-2-cyanoacrylate

To a solution of tert-butyl cyclohexylglycine (2.5 g, 11.7 mmol, 1.0 eq) in toluene (20.0 mL) was added ethyl (*E*)-2-cyano-3-ethoxyacrylate (1.9 g, 11.7 mmol, 1.0 eq) and TEA (4.9 mL, 35.1 mmol, 3.0 eq) at rt. The reaction solution was reacted at 110°C for 16 h. Upon completion, the reaction solution was concentrated under reduced pressure, poured into water, and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 2.5 g, yield 63.4%).

### Step C: 2-(Tert-butyl)-4-ethyl 3-amino-1-cyclohexyl-1H-pyrrole-2,4-dicarboxylate

At 0°C, to a solution of (*E*)-ethyl 3-((2-(tert-butoxy)-2-oxoethyl)(cyclohexyl)amino)-2-cyanoacrylate ((2.30 g, 6.84 mmol, 1.0 eq) in THF (30.0 mL) was added sodium hydride (820 mg, 20.51 mmol, 60% purity, 3.0 eq). The reaction solution was reacted at rt for 3 h. Upon completion, the reaction solution was poured into aqueous NH₄Cl and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow oil, 1.65 g, yield 72%).

### Step D: Ethyl 4-amino-1-cyclohexyl-1H-pyrrole-3-carboxylate

To a solution of 2-(tert-butyl)-4-ethyl 3-amino-1-cyclohexyl-1H-pyrrole-2,4-dicarboxylate (1.65 g, 4.90 mmol, 1.0 eq) in DCM (5.0 mL) was added TFA (15.0 mL) at rt. The reaction solution was reacted at rt for 2 h. Upon completion, the reaction solution was concentrated under reduced pressure to afford the title compound (yellow oil, 2.67 g, crude).

### Step E: Ethyl 4-((tert-butoxycarbonyl)amino)-1-cyclohexyl-1H-pyrrole-3-carboxylate

To a solution of ethyl 4-amino-1-cyclohexyl-1H-pyrrole-3-carboxylate (2.7 g, crude) in DCM (20.0 mL) were added TEA (3.48 g, 34.46 mmol, 3.5 eq) and di-tert-butyl dicarbonate (2.14 g, 9.82 mmol, 1.0 eq) at rt. The reaction solution was reacted at 40°C for 12 h. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by column chromatography to afford the title compound (yellow oil, 1.60 g, yield 96.9%).

### Step F: 4-((tert-butoxycarbonyl)amino)-1-cyclohexyl-1H-pyrrole-3-carboxylic acid

To a mixed solution of ethyl 4-((tert-butoxycarbonyl)amino)-1-cyclohexyl-1H-pyrrole-3-carboxylate (1.60 g, 4.76 mmol, 1.0 eq) in MeOH (8.0 mL) and water (8.0 mL) was added lithium hydroxide (342 mg, 14.27 mmol, 3.0 eq) at rt. The reaction solution was reacted at 60°C for 12 h. Upon completion, the reaction solution was concentrated under reduced pressure and poured into water. The pH was adjusted to 6-7 with 1 N HCl, and the mixture was extracted with EtOAc (×3). The combined organic phases were washed with saturated aqueous NaCl, dried over anhydrous Na₂SO₄, filtered, and concentrated under reduced pressure to afford the title compound (yellow solid, 1.20 g, yield 81.8%).

### Step G: Tert-butyl (1-cyclohexyl-4-(cyclopropylcarbamoyl)-1H-pyrrol-3-yl)carbamate

To a solution of 4-((tert-butoxycarbonyl)amino)-1-cyclohexyl-1H-pyrrole-3-carboxylic acid (1.0 g, 3.2 mmol, 1.0 eq) and cyclopropylamine (0.3 g, 4.8 mol, 1.5 eq) in DMF (15.0 mL) were added DIEA (1.2 g, 9.7 mmol, 3.0 eq) and HATU (1.8 g, 4.8 mmol, 1.5 eq) at rt. The reaction solution was reacted at rt for 3 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 1.0 g, yield 88.7%).

### Step H: 4-amino-1-cyclohexyl-N-cyclopropyl-1H-pyrrole-3-carboxamide

To a solution of tert-butyl (1-cyclohexyl-4-(cyclopropylcarbamoyl)-1H-pyrrol-3-yl)carbamate (1.0 g, 2.8 mmol, 1.0 eq) in DCM (10.0 mL) was added HCl/dioxane (5.0 mL, 4 N) at rt. The reaction solution was reacted at rt for 1 h. Upon completion, the reaction solution was concentrated under reduced pressure to afford the title compound as hydrochloride (white solid, 700 mg, hydrochloride, yield 98.3%).

### Step I: N-(1-cyclohexyl-4-(cyclopropylcarbamoyl)-1H-pyrrol-3-yl)-5-(1-(tetrahydro-2H-pyran-2-yl)-1H-pyrazol-4-yl)pyrazolo[1,5-a]pyrimidine-3-carboxamide

To a solution of 4-amino-1-cyclohexyl-N-cyclopropyl-1H-pyrrole-3-carboxamide (100 mg, 0.4 mmol, hydrochloride, 1.0 eq) and 5-(1-(tetrahydro-2H-pyran-2-yl)-1H-pyrazol-4-yl)pyrazolo[1,5-a]pyrimidine-3-carboxylic acid (190 mg, 0.6 mmol, 1.5 eq) in DMF (5.0 mL) were added DIEA (156 mg, 1.2 mmol, 3.0 eq) and HATU (230 mg, 0.6 mmol, 1.5 eq) at rt. The reaction solution was reacted at rt for 3 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 50.0 mg, yield 27.3%).

### Step J: N-(1-cyclohexyl-4-(cyclopropylcarbamoyl)-1H-pyrrol-3-yl)-5-(1H-pyrazol-4-yl)pyrazolo[1,5-a]pyrimidine-3-carboxamide

To a solution of N-(1-cyclohexyl-4-(cyclopropylcarbamoyl)-1H-pyrrol-3-yl)-5-(1-(tetrahydro-2H-pyran-2-yl)-1H-pyrazol-4-yl)pyrazolo[1,5-a]pyrimidine-3-carboxamide (50 mg, 0.1 mmol, 1.0 eq) in DCM (5.0 mL) was added HCl/dioxane (5.0 mL, 4 N) at rt. The reaction solution was reacted at rt for 1 h. Upon completion, the reaction solution was concentrated under reduced pressure. The residue was purified by HPLC to afford the title compound (yellow solid, 15.0 mg, yield 35.5%). **¹H NMR** (400 MHz, DMSO-*d*₆) δ 13.37 (s, 1H), 11.45 (s, 1H), 9.59 (s, 1H), 9.18 (d, *J* = 7.4 Hz, 1H), 8.81 (s, 1H), 8.51 (s, 1H), 8.04 (d, *J* = 3.4 Hz, 1H), 7.75-7.54 (m, 2H), 7.48 (d, *J* = 2.4 Hz, 1H), 3.99-3.79 (m, 1H), 2.98-2.75 (m, 1H), 2.06-1.98 (m, 2H), 1.90-1.77 (m, 2H), 1.72-1.51 (m, 3H), 1.42 (q, *J* = 12.8 Hz, 2H), 1.29-1.20 (m, 1H), 0.91-0.79 (m, 2H), 0.64-0.48 (m, 2H). LCMS (ESI): m/z 459 [M+H]⁺.

### Preparation Example 170. Synthesis of Compound A295

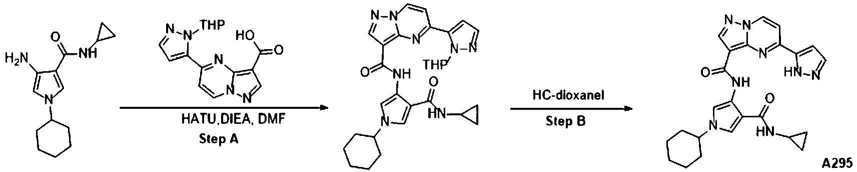

### Step A: N-(1-cyclohexyl-4-(cyclopropylcarbamoyl)-1H-pyrrol-3-yl)-5-(1-(tetrahydro-2H-pyran-2-yl)-1H-pyrazol-5-yl)pyrazolo[1,5-a]pyrimidine-3-carboxamide

To a solution of 4-amino-1-cyclohexyl-N-cyclopropyl-1H-pyrrole-3-carboxamide (100 mg, 0.4 mmol, 1.0 eq) and 5-(1-(tetrahydro-2H-pyran-2-yl)-1H-pyrazol-5-yl)pyrazolo[1,5-a]pyrimidine-3-carboxylic acid (190 mg, 0.6 mmol, 1.5 eq) in DMF (5.0 mL) were added DIEA (156 mg, 1.2 mmol, 3.0 eq) and HATU (230 mg, 0.6 mmol, 1.5 eq) at 0°C. The reaction solution was reacted at rt for 3 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 50.0 mg, yield 27.3%).

### Step B: N-(1-cyclohexyl-4-(cyclopropylcarbamoyl)-1H-pyrrol-3-yl)-5-(1H-pyrazol-5-yl)pyrazolo[1,5-a]pyrimidine-3-carboxamide

To a solution of *N*-(1-cyclohexyl-4-(cyclopropylcarbamoyl)-1H-pyrrol-3-yl)-5-(1-(tetrahydro-2H-pyran-2-yl)-1H-pyrazol-5-yl)pyrazolo[1,5-a]pyrimidine-3-carboxamide (50 mg, 0.1 mmol, 1.0 eq) in DCM (5.0 mL) was added HCl/dioxane (5.0 mL, 4 N) at rt. The reaction solution was reacted at rt for 1 h. Upon completion, the reaction solution was concentrated under reduced pressure. The residue was purified by HPLC to afford the title compound (yellow solid, 15 mg, yield 35.5%). **¹H NMR** (400 MHz, CDCl₃) δ 14.71 (s, 1H), 11.88 (s, 1H), 8.80-8.65 (m, 2H), 7.79-7.65 (m, 2H), 7.28-7.26 (m, 1H), 6.97-6.86 (m, 2H), 6.07 (s, 1H), 3.92-3.61 (m, 1H), 3.12 (s, 1H), 2.12 (d, *J* = 11.3 Hz, 2H), 1.90 (d, *J* = 13.5 Hz, 2H), 1.78-1.65 (m, 3H), 1.47-1.34 (m, 2H), 1.28-1.21 (m, 1H), 1.08 (d, *J=* 6.4 Hz, 2H), 0.65 (s, 2H). LCMS (ESI): m/z 459 [M+H]⁺.

### Preparation Example 171. Synthesis of Compound A296

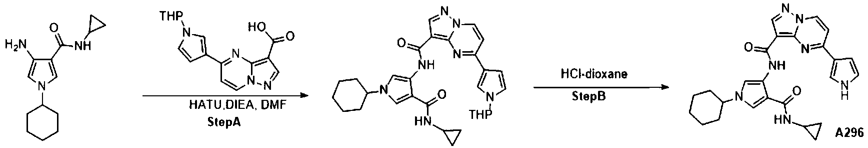

### Step A: N-(1-cyclohexyl-4-(cyclopropylcarbamoyl)-1H-pyrrol-3-yl)-5-(1-(tetrahydro-2H-pyrrol-2-yl)-1H-pyrazolo[1,5-a]pyrimidine-3-carboxamide

To a solution of 4-amino-1-cyclohexyl-N-cyclopropyl-1H-pyrrole-3-carboxamide (50 mg, 0.2 mmol, 1.0 eq) and 5-(1-(tetrahydro-2H-pyran-2-yl)-1H-pyrrol-3-yl)pyrazolo[1,5-a]pyrimidine-3-carboxylic acid (94.4 mg, 0.3 mmol, 1.5 eq) in DMF (5.0 mL) were added DIEA (78.4 mg, 0.6 mmol, 3.0 eq) and HATU (115 mg, 0.3 mmol, 1.5 eq) at 0°C. The reaction solution was reacted at rt for 3 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 50.0 mg, yield 45.6%).

### Step B: N-(1-cyclohexyl-4-(cyclopropylcarbamoyl)-1H-pyrrol-3-yl)-5-(1H-pyrrol-3-yl)pyrazolo[1,5-a]pyrimidine-3-carboxamide

To a solution of N-(1-cyclohexyl-4-(cyclopropylcarbamoyl)-1H-pyrrol-3-yl)-5-(1-(tetrahydro-2H-pyrrol-2-yl)-1H-pyrazolo[1,5-a]pyrimidine-3-carboxamide (50 mg, 0.1 mmol, 1.0 eq) in DCM (5.0 mL) was added HCl/dioxane (5.0 mL, 4 N) at 0°C. The reaction solution was reacted at rt for 1 h. Upon completion, the reaction solution was concentrated under reduced pressure. The residue was purified by HPLC to afford the title compound (yellow solid, 15 mg, yield 35.5%). **¹H NMR** (400 MHz, CDCl₃) δ 12.78 (s, 1H), 11.24 (s, 1H), 8.60 (s, 1H), 8.51 (d, *J* = 7.4 Hz, 1H), 7.23 (d, *J* = 2.9 Hz, 1H), 7.18 (d, *J* = 7.4 Hz, 1H), 7.13 (s, 1H), 6.97 (s, 1H), 6.82 (d, *J* = 3.0 Hz, 1H), 6.38-6.32 (m, 1H), 5.88 (s, 1H), 4.13-3.97 (m, 1H), 3.04-2.86 (m, 1H), 2.07-1.99 (m, 2H), 1.97-1.88 (m, 2H), 1.81-1.72 (m, 1H), 1.69-1.61 (m, 3H), 1.42-1.27 (m, 2H), 0.95 (q, *J* = 6.6 Hz, 2H), 0.73-0.66 (m, 2H). LCMS (ESI): m/z 458 [M+H]⁺.

### Preparation Example 172. Synthesis of Compound A297

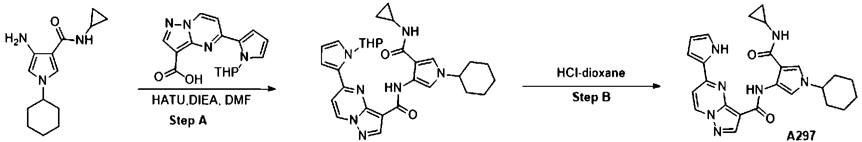

### Step A: N-(1-cyclohexyl-4-(cyclopropylcarbamoyl)-1H-pyrrol-3-yl)-5-(1-(tetrahydro-2H-pyrrol-2-yl)-1H-pyrazolo[1,5-a]pyrimidine-3-carboxamide

To a solution of 4-amino-1-cyclohexyl-N-cyclopropyl-1H-pyrrole-3-carboxamide (50 mg, 0.2 mmol, 1.0 eq) and 5-(1-(tetrahydro-2H-pyran-2-yl)-1H-pyrrol-2-yl)pyrazolo[1,5-a]pyrimidine-3-carboxylic acid (94.4 mg, 0.3 mmol, 1.5 eq) in DMF (5.0 mL) were added DIEA (78.4 mg, 0.6 mmol, 3.0 eq) and HATU (115 mg, 0.3 mmol, 1.5 eq) at 0°C. The reaction solution was reacted at rt for 3 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 50.0 mg, yield 45.6%).

### Step B: N-(1-cyclohexyl-4-(cyclopropylcarbamoyl)-1H-pyrrol-3-yl)-5-(1H-pyrrol-2-yl)pyrazolo[1,5-a]pyrimidine-3-carboxamide

To a solution of N-(1-cyclohexyl-4-(cyclopropylcarbamoyl)-1H-pyrrol-3-yl)-5-(1-(tetrahydro-2H-pyrrol-2-yl)-1H-pyrazolo[1,5-a]pyrimidine-3-carboxamide (50 mg, 0.1 mmol, 1.0 eq) in DCM (5.0 mL) was added HCl/dioxane (5.0 mL, 4 N) at rt. The reaction solution was reacted at rt for 1 h. Upon completion, the reaction solution was concentrated under reduced pressure. The residue was purified by HPLC to afford the title compound (yellow solid, 15 mg, yield 35.5%). **¹H NMR** (400 MHz, CDCl₃) δ 9.76 (s, 1H), 8.78 (d, *J* = 22.0 Hz, 1H), 8.66-8.57 (m, 2H), 7.70-7.59 (m, 2H), 7.17 (d, *J* = 7.4 Hz, 1H), 6.91-6.75 (m, 3H), 6.25 (d, *J* = 2.9 Hz, 1H), 4.91-4.63 (m, 1H), 2.87-2.71 (m, 1H), 2.26-2.09 (m, 2H), 1.93-1.82 (m, 2H), 1.78-1.69 (m, 1H), 1.52-1.39 (m, 3H), 1.30-1.16 (m, 2H), 0.68-0.60 (m, 2H), 0.43-0.34 (m, 2H). LCMS (ESI): m/z 458 [M+H]⁺.

### Preparation Example 173. Synthesis of Compound A455

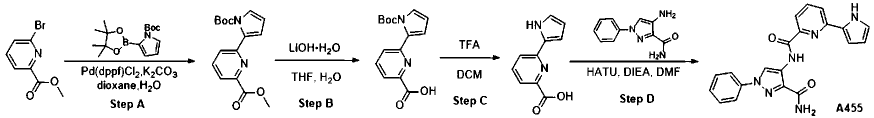

### Step A: Methyl 6-(1-(tert-Butoxycarbonyl)-1H-pyrrol-2-yl)picolinate

To a mixed solution of methyl 6-bromopyridine-2-carboxylate (1 g, 4.63 mmol, 1.0 eq) and tert-butyl 2-(4,4,5,5-tetramethyl-1,3,2-dioxaborolan-2-yl)-1H-pyrrole-1-carboxylate (1.63 g, 5.56 mmol, 1.2 eq) in 1,4-dioxane (20.0 mL) and water (4.0 mL) were added Pd(dppf)Cl₂ (0.17 g, 0.23 mmol, 0.05 eq) and potassium carbonate (1.60 g, 11.6 mmol, 2.5 eq) at rt. The reaction solution was reacted under N₂ at 100°C for 2 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by reverse-phase column chromatography to afford the title compound (white solid, 1.0 g, yield 71%).

### Step B: 6-(1-(Tert-butoxycarbonyl)-1H-pyrrol-2-yl)picolinic acid

To a mixed solution of methyl 6-(1-(tert-butoxycarbonyl)-1H-pyrrol-2-yl)picolinate (1.0 g, 3.31 mmol, 1.0 eq) in THF (10.0 mL) and water (3.0 mL) was added lithium hydroxide monohydrate (0.42 g, 9.92 mmol, 3.0 eq) at rt. The reaction solution was reacted at 25°C for 2 h. Upon completion, the reaction solution was poured into water, adjusted to neutral pH with 1 N HCl, and extracted with EtOAc (×3). The combined organic phases were washed with saturated aqueous NaCl, dried over anhydrous Na₂SO₄, filtered, and concentrated under reduced pressure to afford the title compound (white solid, 0.9 g, crude).

### Step C: 6-(1H-pyrrol-2-yl)picolinic acid

To a solution of 6-(1-(tert-butoxycarbonyl)-1H-pyrrol-2-yl)picolinic acid (900 mg, crude) in DCM (10.0 mL) was added TFA (3.0 mL) at rt. The reaction solution was reacted at rt for 0.5 h. Upon completion, the reaction solution was concentrated under reduced pressure. The residue was purified by reverse-phase column chromatography to afford the title compound (white solid, 0.4 g, yield 68%).

### Step D: N-(3-amino-1-phenyl-1H-pyrazol-4-yl)-6-(1H-pyrrol-2-yl)picolinamide

At rt, to a solution of 6-(1H-pyrrol-2-yl)picolinic acid (150 mg, 0.80 mmol, 1.0 eq) and 4-amino-1-phenyl-1H-pyrazole-3-carboxamide (81 mg, 0.40 mmol, 0.5 eq) in DMF (3.0 mL) were added DIEA (515 mg, 3.99 mmol, 5.0 eq) and HATU (394 mg, 1.04 mmol, 1.3 eq). The reaction solution was reacted at rt for 0.5 h. Upon completion, the reaction solution was concentrated under reduced pressure. The residue was purified by HPLC to afford the title compound (white solid, 27.0 mg, yield 9%). **¹H NMR** (400 MHz, DMSO-*d*₆) δ 12.16 (s, 1H), 11.40 (s, 1H), 8.99 (s, 1H), 8.12-7.84 (m, 7H), 7.57 (t, *J* = 7.8 Hz, 2H), 7.40 (t, *J* = 7.4 Hz, 1H), 7.22 (s, 1H), 7.05 (s, 1H), 6.27 (s, 1H). LCMS (ESI): m/z 372 [M+H]⁺.

### Preparation Example 174. Synthesis of Compound A456

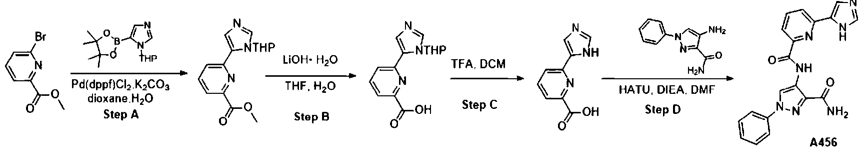

### Step A: Methyl 6-(1-(tetrahydro-2H-pyran-2-yl)-1H-imidazol-5-yl)picolinate

To a mixed solution of methyl 6-bromopicolinate (600 mg, 2.78 mmol, 1.0 eq) and 1-(tetrahydro-2H-pyran-2-yl)-5-(4,4,5,5-tetramethyl-1,3,2-dioxaborolan-2-yl)-1H-imidazole (927 mg, 3.33 mmol, 1.2 eq) in 1,4-dioxane (15.0 mL) and water (3.0 mL) were added Pd(dppf)Cl₂ (102 mg, 0.14 mmol, 0.05 eq) and potassium carbonate (960 mg, 6.94 mmol, 2.5 eq) at rt. The reaction solution was reacted under N₂ at 100°C for 2 h. Upon completion, the reaction solution was concentrated under reduced pressure. The residue was purified by reverse-phase column chromatography to afford the title compound (white solid, 0.6 g, yield 75%).

### Step B: 6-(1-(tetrahydro-2H-pyran-2-yl)-1H-imidazol-5-yl)picolinic acid

To a mixed solution of methyl 6-(1-(tetrahydro-2H-pyran-2-yl)-1H-imidazol-5-yl)picolinate (600 mg, 2.09 mmol, 1.0 eq) in THF (10.0 mL) and water (3.0 mL) was added lithium hydroxide monohydrate (263 mg, 6.27 mmol, 3.0 eq) at rt. The reaction solution was reacted at 25°C for 2 h. Upon completion, the reaction solution was poured into water, adjusted to neutral pH with 1 N HCl, and extracted with EtOAc (×3). The combined organic phases were washed with saturated aqueous NaCl, dried over anhydrous Na₂SO₄, filtered, and concentrated under reduced pressure. The residue was purified by reverse-phase column chromatography to afford the title compound (white solid, 0.5 g, yield 88%).

### Step C: 6-(1H-imidazol-5-yl)picolinic acid

To a solution of 6-(1-(tetrahydro-2H-pyran-2-yl)-1H-imidazol-5-yl)picolinic acid (500 mg, 1.83 mmol, 1.0 eq) in DCM (3.0 mL) was added TFA (1.0 mL) at rt. The reaction solution was reacted at rt for 0.5 h. Upon completion, the reaction solution was concentrated under reduced pressure. The residue was purified by reverse-phase column chromatography to afford the title compound (white solid, 250 mg, yield 72%).

### Step D: N-(3-amino-1-phenyl-1H-pyrazol-4-yl)-6-(1H-imidazol-5-yl)picolinamide

At rt, to a solution of 6-(1H-imidazol-5-yl)picolinic acid (150 mg, 0.80 mmol, 1.0 eq) and 4-amino-1-phenyl-1H-pyrazole-3-carboxamide (81 mg, 0.40 mmol, 0.5 eq) in DMF (3.0 mL) were added DIEA (512 mg, 3.97 mmol, 5.0 eq) and HATU (392 mg, 1.03 mmol, 1.3 eq). The reaction solution was reacted at rt for 0.5 h. Upon completion, the reaction solution was concentrated under reduced pressure. The residue was purified by HPLC to afford the title compound (white solid, 41.2 mg, yield 14%). **¹H NMR** (400 MHz, DMSO-*d*₆) δ 12.55 (s, 1H), 12.14 (s, 1H), 9.01 (s, 1H), 8.16-7.93 (m, 7H), 7.90-7.79 (m, 2H), 7.57 (t, *J=* 7.9 Hz, 2H), 7.40 (t, *J* = 7.4 Hz, 1H). LCMS (ESI): m/z 374 [M+H]⁺.

### Preparation Example 175. Synthesis of Compound A457

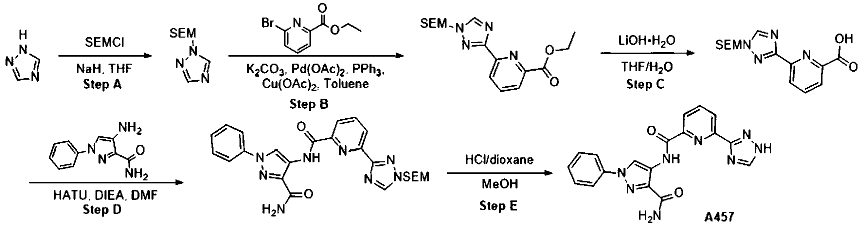

### Step A: 1-((2-(Trimethylsilyl)ethoxy)methyl)-1H-1,2,4-triazole

To a stirred solution of 1,2,4-triazole (2.0 g, 28.8 mmol, 1.0 eq) in THF (30 mL) at 25°C under N₂ was added portionwise NaH (1151 mg, 28.8 mmol, 1.0 eq, 60% purity). The mixture was stirred at 25°C for 0.5 h. SEMCl (5.1 mL, 28.8 mmol, 1.0 eq) was then added to the mixture, and the reaction was stirred at 25°C for 16 h. Upon completion, the reaction mixture was quenched with water (20 mL) and extracted with EtOAc (×3) (20 mL). The organic phases were combined, washed with aqueous NaCl (20 mL), dried over anhydrous Na₂SO₄, filtered, and concentrated under reduced pressure. Finally, the residue was purified by silica gel column chromatography to afford the title compound (colorless oil, 2.0 g, yield 35%).

### Step B: Ethyl 6-((1-(2-(trimethylsilyl)ethoxy)methyl)-1H-1,2,4-triazol-3-yl)picolinate

To a solution of ethyl 6-bromopyridine-2-carboxylate (2.0 g, 8.7 mmol, 1.0 eq), K2CO3 (2.4 g, 17.4 mmol, 2.0 eq), and 1-((2-(trimethylsilyl)ethoxy)methyl)-1H-1,2,4-triazole (4.32 g, 21.7 mmol, 2.5 eq) in toluene (30 mL) was added PPh3 (115 mg, 0.44 mmol, 0.05 eq), Cu(OAc)2 (32 mg, 0.17 mmol, 0.02 eq), and Pd(OAc)2 (10 mg, 0.043 mmol, 0.005 eq) at rt. The reaction mixture was degassed in vacuo, purged with N₂ several times, and then heated at 120°C under N₂ for 20 h. Upon completion, the reaction mixture was diluted in water (60 mL) and extracted with EtOAc (×3) (40 mL). The organic phases were combined, washed with aqueous NaCl (20 mL), dried over anhydrous Na₂SO₄, filtered, and concentrated under reduced pressure. Finally, the residue was purified by silica gel column chromatography to afford the title compound (black solid, 3.0 g, yield 99%).

### Step C: 6-(1-((2-(trimethylsilyl)ethoxy)methyl)-1H-1,2,4-triazol-3-yl)picolinic acid

To a mixed solution of ethyl 6-(1-(2-(trimethylsilyl)ethoxy)methyl-1H-1,2,4-triazol-3-yl)picolinate (3.0 g, 8.61 mmol, 1.0 eq) in THF (20 mL) and water (4 mL) was added lithium hydroxide monohydrate (1.08 g, 25.8 mmol, 3.0 eq) at rt, and the mixture was stirred at 25°C for 16 h. Upon completion, the mixture was acidified with 2N HCl until the pH was adjusted to 4-5. The resulting mixture was diluted in water (10 mL) and extracted with EtOAc (×3) (20 mL). The organic phases were combined, washed twice with aqueous NaCl (15 mL), dried over anhydrous Na₂SO₄, filtered, and then concentrated under reduced pressure to provide the title compound (1.9 g, white solid, 69%).

### Step D: N-(3-Carbamoyl-1-phenyl-1H-pyrazol-4-yl)-6-(1-((2-(trimethylsilyl)ethoxy)methyl)-1H-1,2,4-triazol-3-yl)picolinamide

At 25°C, to a solution of 6-(1-(2-(trimethylsilyl)ethoxy)methyl)-1H-1,2,4-triazol-3-yl)picolinic acid (120 mg, 0.38 mmol, 1.0 eq) and HATU (185 mg, 0.49 mmol, 1.3 eq) in DMF (2 mL) were added DIEA (194 mg, 1.50 mmol, 4.0 eq) and 4-amino-1-phenyl-1H-pyrazole-3-carboxamide (76 mg, 0.38 mmol, 1.0 eq). The mixture was stirred at 25°C for 1 h. Upon completion, the mixture was diluted in water (10 mL) and extracted with EtOAc (×3) (15 mL). The organic phases were combined, washed with aqueous NaCl (10 mL), dried over anhydrous Na₂SO₄, filtered, and concentrated under reduced pressure. Finally, the residue was purified by silica gel column chromatography to afford the title compound (white solid, 100 mg, yield 53%).

### Step E: N-(3-Carbamoyl-1-phenyl-1H-pyrazol-4-yl)-6-(1H-1,2,4-triazol-3-yl)picolinamide

At 25°C, to a solution of N-(3-carbamoyl-1-phenyl-1H-pyrazol-4-yl)-6-(1-((2-(trimethylsilyl)ethoxy)methyl)-1H-1,2,4-triazol-3-yl)picolinamide (100 mg, 0.20 mmol, 1.0 eq) in MeOH (2 mL) was added 4 M hydrogen chloride in dioxane (0.5 mL). The mixture was stirred at rt for 1 h. Upon completion, the mixture was concentrated under reduced pressure. The residue was purified by preparative high performance liquid chromatography to afford the title compound (white solid, 30 mg, yield 40%). **¹H NMR** (400 MHz, DMSO-*d*₆) δ 14.46 (s, 1H), 11.73 (s, 1H), 9.06 (s, 1H), 8.71 (d, *J* = 36.3 Hz, 1H), 8.36-8.27 (m, 1H), 8.25-8.12 (m, 2H), 8.04-7.94 (m, 3H), 7.72 (s, 1H), 7.62-7.50 (m, 2H), 7.46-7.38 (m, 1H). LCMS (ESI): m/z 375 [M+H]⁺.

### Preparation Example 176. Synthesis of Compound A458

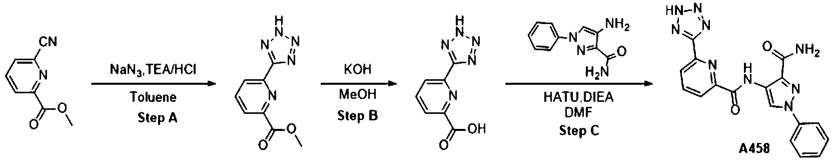

### Step A: Methyl 6-(2H-tetrazol-5-yl)picolinate

At rt, sodium azide (624 mg, 13.9 mmol, 4.5 eq) was added to a solution of methyl 6-cyanopyridine-2-carboxylate (500 mg, 3.08 mmol, 1.0 eq) and TEA hydrochloride (1901 mg, 13.9 mmol, 4.5 eq) in toluene (5.0 mL). The reaction solution was reacted under N₂ at 120°C for 12 h. Upon completion, NaHCO3 solution was added to adjust the pH to a weak base, and the mixture was extracted with EtOAc (×3). The combined organic phases were washed with saturated aqueous NaCl, dried over anhydrous Na₂SO₄, filtered, and concentrated under reduced pressure. The residue was purified by reverse-phase column chromatography to afford the title compound (white solid, 550 mg, yield 87%).

### Step B: 6-(2H-tetrazol-5-yl)picolinic acid

To a solution of methyl 6-(2H-tetrazol-5-yl)picolinate (450 mg, 2.19 mmol, 1.0 eq) in MeOH (5 mL) was added potassium hydroxide (369 mg, 6.58 mmol, 3.0 eq) at rt. The reaction solution was reacted at 25°C for 1 h. Upon completion, the reaction solution was poured into water, and the pH was adjusted to a weakly acidic state with 2 N HCl. The mixture was extracted with EtOAc (×3). The combined organic phases were washed with saturated aqueous NaCl, dried over anhydrous Na₂SO₄, filtered, and concentrated under reduced pressure. The residue was purified by reverse-phase column chromatography to afford the title compound (white solid, 300 mg, yield 72%).

### Step C: N-(3-Carbamoyl-1-phenyl-1H-pyrazol-4-yl)-6-(2H-tetrazol-5-yl)picolinamide

At rt, to a solution of 6-(2H-tetrazol-5-yl)picolinic acid (300 mg, 1.57 mmol, 1.0 eq) and 4-amino-1-phenyl-1H-pyrazole-3-carboxamide (318 mg, 1.57 mmol, 1.0 eq) in DMF (8.0 mL) were added DIEA (810 mg, 6.28 mmol, 4.0 eq) and HATU (717 mg, 1.88 mmol, 1.2 eq). The reaction solution was reacted at rt for 1 h. Upon completion, the reaction solution was concentrated under reduced pressure. The residue was purified by HPLC to afford the title compound (white solid, 19 mg, yield 10%). **¹H NMR** (400 MHz, DMSO-*d*₆) δ 11.64 (s, 1H), 9.06 (s, 1H), 8.37-8.31 (m, 1H), 8.29-8.25 (m, 2H), 8.04-7.96 (m, 3H), 7.76 (s, 1H), 7.61-7.53 (m, 2H), 7.45-7.37 (m, 1H). LCMS (ESI): m/z 376 [M+H]⁺.

### Preparation Example 177. Synthesis of Compound A459

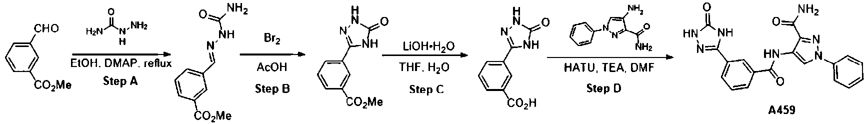

### Step A: Methyl (E)-3-((2-aminohydrazide)methyl)benzoate

To a solution of methyl 3-formylbenzoate (2.0 g, 12.2 mmol, 1.0 eq) and hydrazinecarboxamide (0.91 g, 12.2 mmol, 1.0 eq) in ethanol (20.0 mL) was added 4-dimethylaminopyridine (0.30 g, 2.44 mmol, 0.2 eq) at rt. The reaction solution was reacted at rt for 1 h. Upon completion, a solid was precipitated from the reaction solution, which was then filtered. The filter cake was recrystallized from ethanol to afford the title compound (white solid, 2.1 g, yield 78%).

### Step B: Methyl 3-(5-oxo-4,5-dihydro-1H-1,2,4-triazol-3-yl)benzoate

To a solution of methyl (E)-3-((2-aminohydrazide)methyl)benzoate (1.0 g, 4.52 mmol, 1.0 eq) in AcOH (15.0 mL) was added dropwise bromine (1.81 g, 11.3 mmol, 2.5 eq) at rt. The reaction solution was reacted at 80°C for 3 h. Upon completion, the reaction solution was cooled to rt, and methyl tert-butyl ether was then added and stirred for 10 min. The reaction solution was filtered, and the filter cake was dried to afford the title compound (white solid, 300 mg, yield 30%).

### Step C: 3-(5-oxo-4,5-dihydro-1H-1,2,4-triazol-3-yl)benzoic acid

To a mixed solution of methyl 3-(5-oxo-4,5-dihydro-1H-1,2,4-triazol-3-yl)benzoate (300 mg, 1.37 mmol, 1.0 eq) in THF (4.0 mL) and water (1.0 mL) was added lithium hydroxide monohydrate (82 mg, 3.42 mmol, 2.5 eq) at rt. The reaction solution was reacted at 25°C for 16 h. Upon completion, the reaction solution was poured into water, adjusted to neutral pH with 1 N HCl, and extracted with EtOAc (×3). The combined organic phases were washed with saturated aqueous NaCl, dried over anhydrous Na₂SO₄, filtered, and concentrated under reduced pressure. The residue was purified by reverse-phase column chromatography to afford the title compound (white solid, 235 mg, yield 84%).

### Step D: 4-(3-(5-oxo-4,5-dihydro-1H-1,2,4-triazol-3-yl)benzamido)-1-phenyl-1H-pyrazole-3-carboxamide

To a solution of 3-(5-oxo-4,5-dihydro-1H-1,2,4-triazol-3-yl)benzoic acid (80 mg, 0.39 mmol, 1.0 eq) and 4-amino-1-phenyl-1H-pyrazole-3-carboxamide (79 mg, 0.39 mmol, 1.0 eq) in DMF (1.5 mL) were added TEA (99 mg, 0.98 mmol, 2.5 eq) and HATU (156 mg, 0.41 mmol, 1.05 eq) at rt. The reaction solution was reacted at rt for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (white solid, 30.0 mg, yield 20%). **¹H NMR** (400 MHz, DMSO-*d*₆) δ 10.90 (s, 1H), 8.97 (s, 1H), 8.32 (s, 1H), 8.13 (s, 1H), 8.05 (d, *J* = 7.9 Hz, 1H), 8.03-7.96 (m, 3H), 7.84 (s, 1H), 7.78 (t, *J* = 7.8 Hz, 1H), 7.56 (t, *J* = 7.9 Hz, 2H), 7.46-7.33 (m, 3H). LCMS (ESI): m/z 390 [M+H]⁺.

### Preparation Example 178. Synthesis of Compound A460

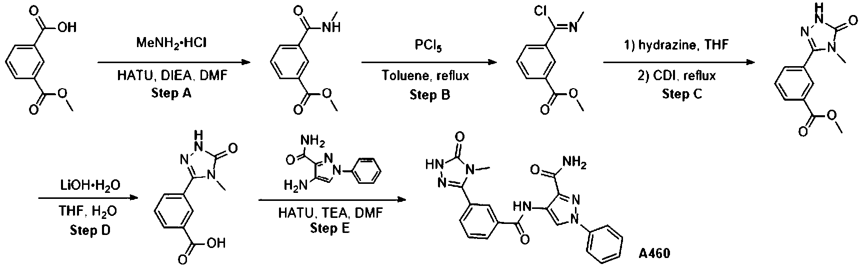

### Step A: Methyl 3-(methylcarbamoyl)benzoate

To a solution of 3-(methoxycarbonyl)benzoic acid (1.5 g, 8.33 mmol, 1.0 eq) in DMF (20.0 mL) was added HATU (3.48 g, 9.16 mmol, 1.1 eq) at rt, and the reaction solution was stirred at rt for 15 min. Methylamine hydrochloride (850 mg, 12.5 mmol, 1.5 eq) and DIEA (2.69 g, 20.8 mmol, 2.5 eq) were then added, and the reaction solution was reacted at rt for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by reverse-phase column chromatography to afford the title compound (white solid, 1.1 g, yield 74%).

### Step B: Methyl (Z)-3-(chloro(methylimino)methyl)benzoate

Phosphorus pentachloride (1.30 g, 6.26 mmol, 1.02 eq) was added to a solution of methyl 3-(methylcarbamoyl)benzoate (1.1 g, 6.14 mmol, 1.0 eq) in toluene (15.0 mL) at rt. The reaction solution was reacted at 110°C under N₂ for 3 h. Upon completion, the reaction solution was concentrated under reduced pressure to afford the title compound (off-white solid, 1.5 g, crude).

### Step C: Methyl 3-(4-methyl-5-oxo-4,5-dihydro-1H-1,2,4-triazol-3-yl)benzoate

To a solution of hydrazine hydrate (0.83 g, 25.8 mmol, 5.0 eq) in THF (15.0 mL) was added dropwise a solution of methyl (Z)-3-(chloro(methylimino)methyl)benzoate (3.48 g, 9.16 mmol, 1.1 eq) in THF (15.0 mL) at rt. The reaction solution was stirred at rt under N₂ for 4 h. N,N'-carbonyldiimidazole (4.18 g, 25.8 mmol, 5.0 eq) was then added at 0°C, and the reaction solution was reacted at 80°C for 4 h. Upon completion, the reaction solution was concentrated under reduced pressure. The residue was purified by reverse-phase column chromatography to afford the title compound (white solid, 295 mg, yield 25%).

### Step D: 3-(4-methyl-5-oxo-4,5-dihydro-1H-1,2,4-triazol-3-yl)benzoic acid

To a mixed solution of methyl 3-(4-methyl-5-oxo-4,5-dihydro-1H-1,2,4-triazol-3-yl)benzoate (295 mg, 1.27 mmol, 1.0 eq) in THF (4.0 mL) and water (1.0 mL) was added lithium hydroxide monohydrate (78 mg, 3.16 mmol, 2.5 eq) at rt. The reaction solution was reacted at 25°C for 16 h. Upon completion, the reaction solution was poured into water, adjusted to neutral pH with 1 N HCl, and extracted with EtOAc (×3). The combined organic phases were washed with saturated aqueous NaCl, dried over anhydrous Na₂SO₄, filtered, and concentrated under reduced pressure to afford the title compound (white solid, 235 mg, crude).

### Step E: 4-(3-(4-methyl-5-oxo-4,5-dihydro-1H-1,2,4-triazol-3-yl)benzamido)-1-phenyl-1H-pyrazole-3-carboxamide

To a solution of 3-(4-methyl-5-oxo-4,5-dihydro-1H-1,2,4-triazol-3-yl)benzoic acid (80 mg, 0.37 mmol, 1.0 eq) and HATU (146 mg, 0.38 mmol, 1.02 eq) in DMF (3.0 mL) were added 4-amino-1-phenyl-1H-pyrazole-3-carboxamide (74 mg, 0.37 mmol, 1.0 eq) and TEA (111 mg, 1.10 mmol, 3.0 eq) at rt. The reaction solution was reacted at rt for 1 h. Upon completion, the reaction solution was concentrated under reduced pressure. The residue was purified by HPLC to afford the title compound (white solid, 30.0 mg, yield 20%). **¹H NMR** (400 MHz, DMSO-*d*₆) δ 12.05 (s, 1H), 10.92 (s, 1H), 8.97 (s, 1H), 8.21 (s, 1H), 8.13 (s, 1H), 8.05 (d, *J* = 7.8 Hz, 1H), 7.99 (d, *J* = 8.0 Hz, 3H), 7.86 (s, 1H), 7.77 (t, *J* = 7.8 Hz, 1H), 7.56 (t, *J* = 7.9 Hz, 2H), 7.40 (t, *J* = 7.4 Hz, 1H), 3.34 (s, 3H). LCMS (ESI): m/z 404 [M+H]⁺.

### Preparation Example 179. Synthesis of Compound A461

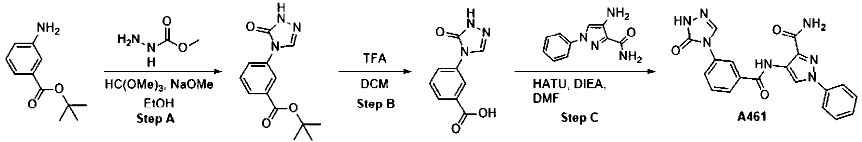

### Step A: Tert-butyl 3-(5-carbonyl-1,5-dihydro-4H-1,2,4-triazol-4-yl)benzoate

At rt, tert-butyl 3-aminobenzoate (2 g, 10.3 mmol, 1.0 eq), methyl carbazate (4.63 g, 51.5 mmol, 5.0 eq), and trimethyl orthoformate (5.673 ml, 51.5 mmol, 5.0 eq) were added to ethanol (50 ml). The reaction solution was stirred at 80°C under N₂ for 16 h. The reaction mixture was then cooled to 25°C, and sodium methoxide (2.80 g, 51.5 mmol, 5.0 eq) was added to the above solution. The reaction solution was stirred at 80°C under N₂ for 4 h. The reaction mixture was concentrated under reduced pressure, and the residue was purified by reverse-phase column chromatography to afford the title compound (white solid, 600 mg, yield 22%).

### Step B: 3-(5-Carbonyl-1,5-dihydro-4H-1,2,4-triazol-4-yl)benzoic acid

At rt, tert-butyl 3-(5-carbonyl-1,5-dihydro-4H-1,2,4-triazol-4-yl)benzoate (600 mg, 2.29 mmol, 1.0 eq) was added to a solution of DCM (10 ml), followed by the addition of TFA (3 ml). The mixture was stirred at 25°C under N₂ for 3 h. Upon completion, the reaction mixture was concentrated under reduced pressure to afford the title compound (white solid, 600 mg, TFA salt), which was used directly in the next reaction without further purification.

### Step C: 4-(3-(5-carbonyl-1,5-dihydro-4H-1,2,4-triazol-4-yl)benzamido)-1-phenyl-1H-pyrazole-3-carboxamide

At rt, 3-(5-carbonyl-1,5-dihydro-4H-1,2,4-triazol-4-yl)benzoic acid (51 mg, 0.25 mmol, 1.0 eq) and HATU (103 mg, 0.27 mmol, 1.1 eq) were added to DMF (3 mL), followed by the addition of 4-amino-1-phenyl-1H-pyrazole-3-carboxamide (50 mg, 0.25 mmol, 1.0 eq) and DIEA (160 mg, 1.24 mmol, 5 eq). The reaction solution was reacted at rt for 0.5 h. Upon completion, aqueous NaOH (2 N, 10 mL) and ethyl acetate (30 mL) were added to the reaction mixture. After shaking and allowing to stand, a white solid was precipitated. The suspension was filtered, and the filter cake was washed with water, slurried with methyl tert-butyl ether, and filtered to obtain the crude. The crude was purified by reverse-phase column chromatography to afford the title compound (white solid, 25 mg, yield 26%). **¹H NMR** (400 MHz, DMSO-d6) δ 12.07 (s, 1H), 10.83 (s, 1H), 8.97 (s, 1H), 8.51 (s, 1H), 8.31 (t, J = 1.7 Hz, 1H), 8.11 (s, 1H), 7.99 (d, J = 7.7 Hz, 2H), 7.95 (dd, J = 8.0, 1.3 Hz, 1H), 7.89-7.78 (m, 2H), 7.74 (t, J = 7.9 Hz, 1H), 7.56 (t, J = 8.0 Hz, 2H), 7.40 (t, J = 7.4 Hz, 1H).

### Preparation Example 180. Synthesis of Compound A462

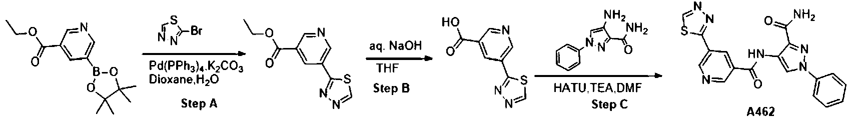

### Step A: Ethyl 5-(1,3,4-thiadiazol-2-yl)nicotinate

To a mixed solution of ethyl 5-((4,4,5,5-tetramethyl-1,3,2-dioxaborolan-2-yl)nicotinate (500 mg, 1.8 mmol, 1.0 eq) and 2-bromo-1,3,4-thiadiazole (506 mg, 3.1 mmol, 1.7 eq) in 1,4-dioxane (9.0 mL) and water (1.5 mL) were added Pd(PPh₃)₄ (313 mg, 0.3 mmol, 0.15 eq) and potassium carbonate (499 mg, 3.6 mmol, 2.0 eq) at rt. The reaction solution was reacted under N₂ at 100°C for 4 h. Upon completion, the reaction solution was concentrated under reduced pressure. The residue was purified by normal-phase column chromatography to afford the title compound (yellow solid, 256 mg, yield 42%).

### Step B: 5-(1,3,4-thiadiazol-2-yl)nicotinic acid

To a mixed solution of ethyl 5-(1,3,4-thiadiazol-2-yl)nicotinate (256 mg, 0.8 mmol, 1.0 eq) in THF (6.0 mL) and water (2.0 mL) was added NaOH (61 mg, 1.5 mmol, 2.0 eq) at rt. The reaction solution was reacted at 25°C for 3 h. Upon completion, the reaction solution was poured into water, adjusted to pH= 4-5 with 2 N HCl, and extracted with EtOAc (×3). The combined organic phases were washed with saturated aqueous NaCl, dried over anhydrous Na₂SO₄, filtered, and concentrated under reduced pressure. The title compound (white solid, 200 mg, crude) was used for the next purification step.

### Step C: N-(3-amino-1-phenyl-1H-pyrazol-4-yl)-5-(1,3,4-thiadiazol-2-yl)nicotinamide

To a solution of 5-(1,3,4-thiadiazol-2-yl)nicotinic acid (115 mg, 0.6 mmol, 1.0 eq) and 4-amino-1-phenyl-1H-pyrazole-3-carboxamide (112 mg, 0.60 mmol, 1.0 eq) in DMF (10 mL) were added TEA (112 mg, 1.1 mmol, 2.0 eq) and HATU (317 mg, 0.8 mmol, 1.5 eq) at rt. The reaction solution was reacted at rt for 2 h. Upon completion, the reaction solution was concentrated under reduced pressure. The residue was purified by HPLC to afford the title compound (white solid, 16.0 mg, yield 7%). **¹H NMR** (400 MHz, DMSO-*d*₆) δ 10.97 (s, 1H), 9.79 (s, 1H), 9.40 (s, 1H), 9.22 (s, 1H), 8.99 (s, 1H), 8.82 (s, 1H), 8.12 (s, 1H), 8.00 (d, *J* = 7.9 Hz, 2H), 7.85 (s, 1H), 7.56 (t, *J* = 7.8 Hz, 2H), 7.41 (t, *J* = 7.4 Hz, 1H). LCMS (ESI): m/z 392 [M+H]⁺.

### Preparation Example 181. Synthesis of Compound A463

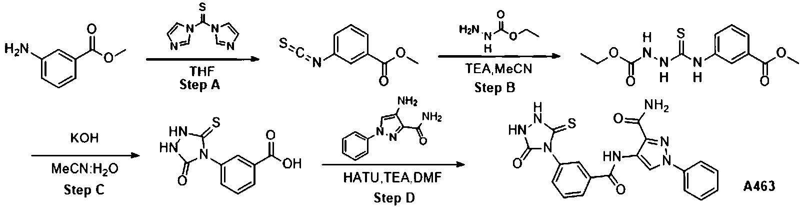

### Step A: Methyl 3-isothiocyanatobenzoate

To a solution of methyl 3-aminobenzoate (300 mg, 1.99 mmol, 1.0 eq) in THF (10.0 mL) was added di(1H-imidazol-1-yl)methanethione (354 mg, 1.99 mmol, 3.0 eq) at 0°C. The reaction solution was reacted at 0°C for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure to afford the title compound (white solid, 345 mg, crude).

### Step B: Ethyl 2-((3-(methoxycarbonyl)phenyl)aminomethylthio)hydrazine-1-carboxylate

To a solution of methyl 3-isothiocyanatobenzoate (345 mg, 1.8 mmol, 1.0 eq) and ethyl hydrazinecarboxylate (186 mg, 1.8 mmol, 1.0 eq) in acetonitrile (10.0 mL) was added TEA (542 mg, 5.4 mmol, 3.0 eq) at rt. The reaction solution was reacted at 80°C for 3 h. Upon completion, the reaction solution was concentrated under reduced pressure. The residue was purified by column chromatography to afford the title compound (white solid, 316 mg, yield 60%).

### Step C: 3-(3-oxo-5-thioxo-1,2,4-triazolidin-4-yl)benzoic acid

To a mixed solution of ethyl 2-((3-(methoxycarbonyl)phenyl)aminomethylthio)hydrazine-1-carboxylate (200 mg, 0.7 mmol, 1.0 eq) in acetonitrile (2.0 mL) and water (1.0 mL) was added potassium hydroxide (38 mg, 0.7 mmol, 3.0 eq) at rt. The reaction solution was reacted at 100°C for 2 h. Upon completion, the reaction solution was concentrated under reduced pressure. The residue was purified by column chromatography to afford the title compound (white solid, 140 mg, yield 88%).

### Step D: 4-(3-(3-oxo-5-thioxo-1,2,4-triazolidin-4-yl)benzamido)-1-phenyl-1H-pyrazole-3-carboxamide

To a solution of 3-(3-oxo-5-thioxo-1,2,4-triazolidin-4-yl)benzoic acid (140 mg, 0.6 mmol, 1.0 eq) and HATU (337 mg, 0.9 mmol, 1.5 eq) in DMF (3.0 mL) were added 4-amino-1-phenyl-1H-pyrazole-3-carboxamide (119 mg, 0.6 mmol, 1.0 eq) and TEA (119 mg, 1.2 mmol, 2.0 eq) at rt. The reaction solution was reacted at 40°C for 18 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (white solid, 28.0 mg, yield 11%). **¹H NMR** (400 MHz, DMSO-*d*₆) δ 10.79 (s, 1H), 8.97 (s, 1H), 8.07 (s, 1H), 8.02-7.95 (m, 3H), 7.85-7.76 (m, 2H), 7.69 (d, *J* = 8.0 Hz, 1H), 7.61 (t, *J* = 7.8 Hz, 1H), 7.55 (t, *J* = 8.0 Hz, 2H), 7.39 (t, *J* = 7.4 Hz, 1H), 7.08 (s, 2H). LCMS (ESI): m/z 422 [M+H]⁺.

### Preparation Example 182. Synthesis of Compound A464

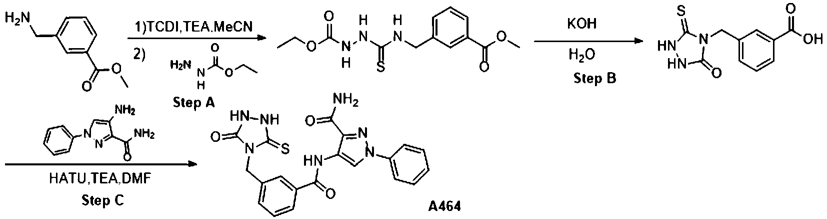

### Step A: Ethyl 2-((3-(methoxycarbonyl)benzyl)aminomethylthio)hydrazine-1-carboxylate

To a mixed solution of methyl 3-(aminomethyl)benzoate (500 mg, 2.5 mmol, 1.0 eq) and TEA (750 mg, 7.4 mmol, 3.0 eq) in acetonitrile (30.0 mL) was added N,N'-thiocarbonyldiimidazole (485 mg, 2.7 mmol, 1.1 eq) at rt. The reaction solution was reacted at rt for 1 h under N₂. Ethyl hydrazinecarboxylate (257 mg, 2.5 mmol, 1.0 eq) was then added, and the reaction solution was reacted at 70°C under N₂ for 16 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 230 mg, yield 28%).

### Step B: 3-((3-oxo-5-thioxo-1,2,4-triazolidin-4-yl)methyl)benzoic acid

To a mixed solution of ethyl 2-((3-(methoxycarbonyl)benzyl)aminomethylthio)hydrazine-1-carboxylate (230 mg, 0.9 mmol, 1.0 eq) in acetonitrile (2.0 mL) and water (1.0 mL) was slowly added potassium hydroxide (151 mg, 2.7 mmol, 3.0 eq) at rt. The reaction solution was reacted at 100°C for 2 h. Upon completion, the reaction solution was poured into water, adjusted to neutral pH with 1 N HCl, and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 90 mg, yield 40%).

### Step C: 4-(3-((3-oxo-5-oxy-1,2,4-triazolidin-4-yl)methyl)benzamido)-1-phenyl-1H-pyrazole-3-carboxamide

To a solution of 3-((3-oxo-5-thioxo-1,2,4-triazolidin-4-yl)methyl)benzoic acid (140 mg, 0.6 mmol, 1.0 eq) and HATU (190 mg, 0.5 mmol, 1.2 eq) in DMF (3.0 mL) at rt were added 4-amino-1-phenyl-1H-pyrazole-3-carboxamide (101 mg, 0.5 mmol, 1.2 eq) and TEA (121 mg, 1.2 mmol, 3.0 eq). The reaction solution was reacted at rt for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (white solid, 20.7 mg, yield 12%). **¹H NMR** (400 MHz, DMSO-*d*₆) δ 13.22 (s, 1H), 12.19 (s, 1H), 10.77 (s, 1H), 8.96 (s, 1H), 8.09 (s, 1H), 7.98 (d, *J* = 7.7 Hz, 2H), 7.92 (s, 1H), 7.82 (s, 1H), 7.78 (d, *J* = 7.3 Hz, 1H), 7.62 (d, *J* = 7.7 Hz, 1H), 7.60-7.55 (m, 2H), 7.54 (s, 1H), 7.40 (t, *J* = 7.4 Hz, 1H), 4.97 (s, 2H). LCMS (ESI): m/z 436 [M+H]⁺.

### Preparation Example 183. Synthesis of Compound A465

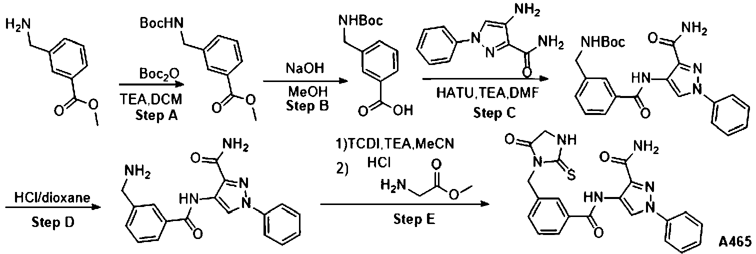

### Step A: Methyl 3-(((tert-butoxycarbonyl)amino)methyl)benzoate

To a solution of methyl 3-(aminomethyl)benzoate (200 mg, 1.2 mmol, 1.0 eq) and TEA (785 mg, 3.6 mmol, 3.0 eq) in DCM (30.0 mL) was added di-tert-butyl dicarbonate (327 mg, 1.5 mmol, 1.5 eq) at rt. The reaction solution was reacted at rt for 16 h under N₂. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 285 mg, yield 89%).

### Step B: 3-(((tert-butoxycarbonyl)amino)methyl)benzoic acid

To a mixed solution of methyl 3-(((tert-butoxycarbonyl)amino)methyl)benzoate (285 mg, 1.1 mmol, 1.0 eq) in MeOH (10.0 mL) and water (2.0 mL) was added NaOH (88 mg, 2.2 mmol, 2.0 eq) at 0°C. The reaction solution was reacted at 25°C for 2 h. Upon completion, the reaction solution was poured into ice water, adjusted to neutral pH with 1 N HCl, and extracted with EtOAc (×3). The combined organic phases were washed with saturated aqueous NaCl, dried over anhydrous Na₂SO₄, filtered, and concentrated under reduced pressure to afford the title compound (yellow solid, 240 mg, yield 86%, crude).

### Step C: Tert-butyl (3-((3-carbamoyl-1-phenyl-1H-pyrazol-4-yl)carbamoyl)benzyl)carbamate

To a solution of 3-(((tert-butoxycarbonyl)amino)methyl)benzoic acid (240 mg, 0.9 mmol, 1.0 eq) and 4-amino-1-phenyl-1H-pyrazole-3-carboxamide (223 mg, 1.1 mmol, 1.2 eq) in DMF (20.0 mL) were added HATU (418 mg, 1.1 mmol, 1.2 eq) and TEA (273 mg, 2.7 mmol, 3.0 eq) at rt. The reaction solution was reacted at rt for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 210 mg, yield 54%).

### Step D: 4-(3-(Aminomethyl)benzamido)-1-phenyl-1H-pyrazole-3-carboxamide

To a solution of tert-butyl (3-((3-carbamoyl-1-phenyl-1H-pyrazol-4-yl)carbamoyl)benzyl)carbamate (210 mg, 0.5 mmol, 1.0 eq) in 1,4-dioxane (2.0 mL) was added HCl/1,4-dioxane (10 mL, 4 M) at rt. The reaction solution was reacted at rt for 2 h. Upon completion, the reaction solution was concentrated under reduced pressure. The residue was poured into saturated NaHCO₃ solution and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 150 mg, yield 89%).

### Step E: 4-(3-((5-oxo-2-thioxoimidazolidin-1-yl)methyl)benzamido)-1-phenyl-1H-pyrazole-3-carboxamide

To a solution of 4-(3-(aminomethyl)benzamido)-1-phenyl-1H-pyrazole-3-carboxamide (150 mg, 0.5 mmol, 1.0 eq) and TEA (152 mg, 1.5 mmol, 3.0 eq) in acetonitrile (20.0 mL) was added N,N'-thiocarbonyldiimidazole (107 mg, 0.6 mmol, 1.1 eq) at rt. The reaction solution was reacted at rt for 1 h under N₂. Glycine methyl ester hydrochloride (63 mg, 0.5 mmol, 1.0 eq) was then added, and the reaction solution was reacted at 70°C under N₂ for 16 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (yellow solid, 25.6 mg, yield 12%). **¹H NMR** (400 MHz, DMSO-*d*₆) δ 10.79 (s, 1H), 10.36 (s, 1H), 8.95 (s, 1H), 8.10 (s, 1H), 7.98 (d, *J* = 7.7 Hz, 2H), 7.85 (d, *J* = 9.5 Hz, 2H), 7.78 (d, *J* = 7.3 Hz, 1H), 7.61 (d, *J* = 7.7 Hz, 1H), 7.58 (d, *J* = 3.3 Hz, 1H), 7.55 (dd, *J* = 7.9, 3.9 Hz, 2H), 7.40 (t, *J* = 7.4 Hz, 1H), 4.99 (s, 2H), 4.27 (s, 2H). LCMS (ESI): m/z 435 [M+H]⁺.

### Preparation Example 184. Synthesis of Compound A466

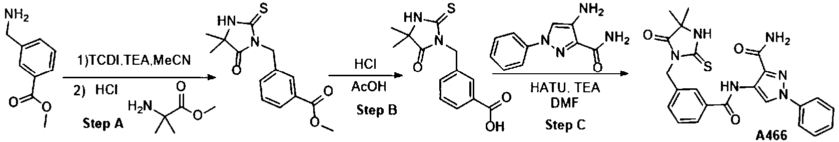

### Step A: Methyl 3-((4,4-dimethyl-5-oxo-2-thioxoimidazolidin-1-yl)methyl)benzoate

To a mixed solution of methyl 3-(aminomethyl)benzoate (1.0 g, 5.0 mmol, 1.0 eq) and TEA (2.5 g, 25.0 mmol, 3.0 eq) in acetonitrile (30.0 mL) was added N,N'-thiocarbonyldiimidazole (979 mg, 5.5 mmol, 1.1 eq) at rt. The reaction solution was reacted at rt for 1 h under N₂. Methyl 2-amino-2-methylpropanoate hydrochloride (835 mg, 5.0 mmol, 1.0 eq) was then added, and the reaction solution was reacted at 70°C under N₂ for 16 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 850 mg, yield 58%).

### Step B: 3-((4,4-dimethyl-5-oxo-2-thioxoimidazolidin-1-yl)methyl)benzoic acid

To a solution of methyl 3-((4,4-dimethyl-5-oxo-2-thioxoimidazolidin-1-yl)methyl)benzoate (850 mg, 2.9 mmol, 1.0 eq) in AcOH (10.0 mL) was slowly added concentrated HCl (5.0 mL) at rt. The reaction solution was reacted under N₂ at 100°C for 2 h. Upon completion, the reaction solution was poured into ice water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 420 mg, yield 52%).

### Step C: 4-(3-((4,4-dimethyl-5-oxo-2-thioxoimidazolidin-1-yl)methyl)benzamido)-1-phenyl-1H-pyrazole-3-carboxamide

To a solution of 3-((4,4-dimethyl-5-oxo-2-thioxoimidazolidin-1-yl)methyl)benzoic acid (100 mg, 0.4 mmol, 1.0 eq) and HATU (190 mg, 0.5 mmol, 1.2 eq) in DMF (10.0 mL) at rt were added 4-amino-1-phenyl-1H-pyrazole-3-carboxamide (101 mg, 0.5 mmol, 1.2 eq) and TEA (121 mg, 1.2 mmol, 3.0 eq). The reaction solution was reacted at rt for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (white solid, 17.0 mg, yield 9%). **¹H NMR** (400 MHz, DMSO-*d*₆) δ 10.78 (s, 1H), 10.62 (s, 1H), 8.96 (s, 1H), 8.09 (s, 1H), 8.04-7.94 (m, 2H), 7.86-7.73 (m, 3H), 7.62-7.49 (m, 4H), 7.43-7.37 (m, 1H), 4.99 (s, 2H), 1.36 (s, 6H). LCMS (ESI): m/z 463[M+H]⁺.

### Preparation Example 185. Synthesis of Compound A467

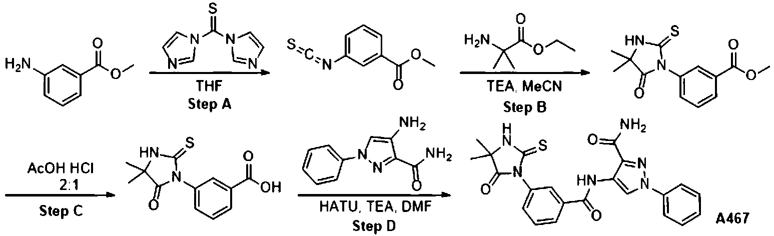

### Step A: Methyl 3-isothiocyanatobenzoate

At 0°C, to a solution of methyl 3-(aminomethyl)benzoate (500 mg, 2.5 mmol, 1.0 eq) in THF (15.0 mL) was added di(1H-imidazol-1-yl)methanethione (354 mg, 1.99 mmol, 1.0 eq). The reaction solution was reacted under N₂ at 0°C for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure to afford the title compound (white solid, 348 mg, crude).

### Step B: Methyl 3-(4,4-dimethyl-5-oxo-2-thioxoimidazolidin-1-yl)benzoate

To a solution of methyl 3-isothiocyanatobenzoate (348 mg, crude) and ethyl 2-amino-2-methylpropanoate (236 mg, 1.8 mmol, 1.0 eq) in acetonitrile (2.0 mL) was added TEA (547 mg, 5.4 mmol, 3.0 eq) at rt. The reaction solution was reacted at 80°C for 3 h. Upon completion, the reaction solution was concentrated under reduced pressure. The residue was purified by column chromatography to afford the title compound (white solid, 417 mg, yield 83%).

### Step C: 3-(4,4-dimethyl-5-oxo-2-thioxoimidazolidin-1-yl)benzoic acid

At rt, a mixed solution of methyl 3-(4,4-dimethyl-5-oxo-2-thioxoimidazolidin-1-yl)benzoate (300 mg, 1.1 mmol, 3.0 eq) in acetonitrile (2.0 mL) and HCl (1.0 mL) was reacted at 100°C for 2 h. Upon completion, the reaction solution was concentrated under reduced pressure. The residue was purified by column chromatography to afford the title compound (white solid, 262 mg, yield 92%).

### Step D: 4-(3-(4,4-dimethyl-5-oxo-2-thioxoimidazolidin-1-yl)benzamido)-1-phenyl-1H-pyrazole-3-carboxamide

To a solution of 3-(4,4-dimethyl-5-oxo-2-thioxoimidazolidin-1-yl)benzoic acid (100 mg, 0.4 mmol, 1.0 eq) and HATU (216 mg, 0.6 mmol, 1.5 eq) in DMF (5.0 mL) were added 4-amino-1-phenyl-1H-pyrazole-3-carboxamide (77 mg, 0.4 mmol, 1.0 eq) and TEA (77 mg, 0.8 mmol, 2.0 eq) at rt. The reaction solution was reacted at 40°C for 18 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (white solid, 43.0 mg, yield 25%). **¹H NMR** (400 MHz, DMSO-*d₆*) δ 10.81 (s, 1H), 10.75 (s, 1H), 8.95 (s, 1H), 8.10 (s, 1H), 7.99 (d, *J =* 7.8 Hz, 2H), 7.94 (d, *J =* 7.8 Hz, 1H), 7.90 (s, 1H), 7.83 (s, 1H), 7.73 (t, *J* = 7.8 Hz, 1H), 7.64 (d, *J =* 8.6 Hz, 1H), 7.56 (t, *J =* 7.9 Hz, 2H), 7.40 (t, *J* = 7.4 Hz, 1H), 1.47 (s, 6H). LCMS (ESI): m/z 449 [M+H]⁺.

### Preparation Example 186. Synthesis of Compound A468

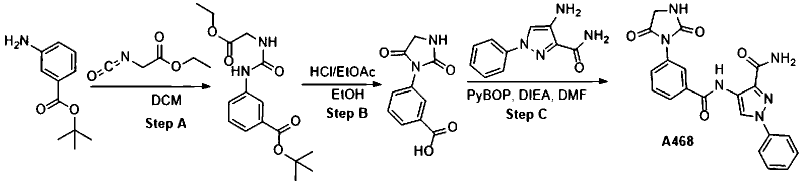

### Step A: Tert-butyl 3-(3-(2-ethoxy-2-oxoethyl)ureido)benzoate

To a solution of tert-butyl 3-aminobenzoate (500 mg, 2.59 mmol, 1.0 eq) dissolved in DCM (6 mL) was added ethyl 2-isocyanoacetate (501 mg, 3.88 mmol, 1.5 eq) at 0°C. The reaction solution was reacted at 25°C for 3 h. Upon completion, the reaction solution was concentrated under reduced pressure to afford the title compound (yellow oil, 0.8 g, yield 96%).

### Step B: 3-(2,5-dioxoimidazolidin-1-yl)benzoic acid

To a solution of tert-butyl 3-(3-(2-ethoxy-2-oxoethyl)ureido)benzoate (200 mg, 0.71 mmol, 1.0 eq) dissolved in ethanol (3 mL) was added HCl in EtOAc (8 mL, 4 M) at rt. The reaction solution was reacted at 90°C for 3 h. Upon completion, the reaction solution was concentrated under reduced pressure to afford the title compound (white solid, 122 mg, yield 88%).

### Step C: 4-(3-(2,5-dioxoimidazolidin-1-yl)benzamido)-1-phenyl-1H-pyrazole-3-carboxamide

To a solution of 3-(2,5-dioxoimidazolidin-1-yl)benzoic acid (100 mg, 0.45 mmol, 1.0 eq) in DMF (1 mL) were added 4-amino-1-phenyl-1H-pyrazole-3-carboxamide (92 mg, 0.45 mmol, 1.0 eq), PyBOP (355 mg, 0.68 mmol, 1.5 eq), and DIEA (176 mg, 1.36 mmol, 3.0 eq) at rt. The reaction solution was reacted at 40°C for 3 h. Upon completion, the reaction solution was concentrated under reduced pressure. The residue was purified by HPLC to afford the title compound (white solid, 20 mg, yield 11%). **¹H NMR** (400 MHz, DMSO-*d₆*) δ 10.84 (s, 1H), 8.97 (s, 1H), 8.41 (s, 1H), 8.11 (s, 1H), 8.00 (d, *J =* 7.8 Hz, 2H), 7.94 (t, *J =* 1.6 Hz, 1H), 7.89 (d, *J =* 7.7 Hz, 1H), 7.83 (s, 1H), 7.73 (t, *J* = 7.8 Hz, 1H), 7.69-7.64 (m, 1H), 7.57 (t, *J* = 7.9 Hz, 2H), 7.41 (t, *J =* 7.4 Hz, 1H), 4.12 (s, 2H). LCMS (ESI): m/z 405 [M+H]⁺.

### Preparation Example 187. Synthesis of Compound A469

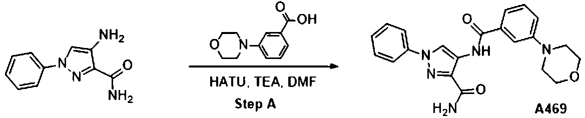

### Step A: 4-(3-morpholinylbenzoamino)-1-phenyl-1H-pyrazole-3-carboxamide

To a solution of 3-morpholinobenzoic acid (67 mg, 0.32 mmol, 1.3 eq) and 4-amino-1-phenylpyrazole-3-carboxamide (50 mg, 0.25 mmol, 1.0 eq) in DMF (2.0 mL) at 0°C were added TEA (0.1 mL, 0.74 mmol, 3.0 eq) and HATU (141 mg, 0.37 mmol, 1.5 eq). The reaction solution was reacted at rt for 5 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (white solid, 15 mg, yield 12%). **¹H NMR** (400 MHz, DMSO-*d₆*) δ 10.76 (s, 1H), 8.92 (s, 1H), 8.09 (s, 1H), 7.97 (d, *J =* 7.8 Hz, 2H), 7.83 (s, 1H), 7.56 (t, *J* = 7.9 Hz, 2H), 7.48-7.36 (m, 3H), 7.30 (d, *J =* 7.7 Hz, 1H), 7.24 (dd, *J* = 8.3, 2.3 Hz, 1H), 3.84-3.73 (m, 4H), 3.24-3.15 (m, 4H). LCMS (ESI): m/z 392 [M+H]⁺.

### Preparation Example 188. Synthesis of Compound A470

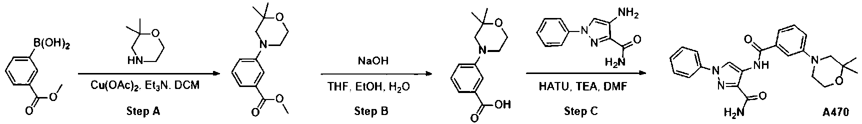

### Step A: Methyl 3-(2,2-dimethylmorpholinyl)benzoate

To a solution of methyl(3-(methoxycarbonyl)phenyl)boronic acid (100 mg, 0.24 mmol, 1.0 eq) in DCM (4.0 mL) at rt were added 2,2-dimethyl-1,4-oxazinane (154 mg, 1.33 mmol, 0.8 eq), TEA (0.9 mL, 6.67 mmol, 4.0 eq), and copper acetate (151 mg, 0.83 mmol, 0.5 eq). The reaction solution was reacted under O₂ at rt for 18 h. Upon completion, aqueous NH₄Cl was added, and the mixture was extracted with DCM (×3). The combined organic phases were washed with saturated aqueous NaCl, dried over anhydrous Na₂SO₄, filtered, and concentrated under reduced pressure. The residue was purified by column chromatography to afford the title compound (white solid, 170 mg, yield 41%).

### Step B: 3-(2,2-dimethylmorpholinyl)benzoic acid

To a mixed solution of methyl 3-(2,2-dimethylmorpholinyl)benzoate (150 mg, 0.60 mmol, 1.0 eq) in tetrahydrofuran/ethanol/water (6 mL, v/v/v = 1 : 1 : 1) was added NaOH (241 mg, 6.02 mmol, 10.0 eq) at rt. The reaction solution was reacted at rt for 5 h. Upon completion, the reaction solution was adjusted to pH 5-6 with dilute HCl in an ice bath, and extracted with EtOAc (×3). The combined organic phases were washed with saturated aqueous NaCl, dried over anhydrous Na₂SO₄, filtered, and concentrated under reduced pressure to afford the title compound (white solid, 110 mg, crude).

### Step C: 4-(3-(2,2-dimethylmorpholinyl)benzamido)-1-phenyl-1H-pyrazole-3-carboxamide

To a solution of 3-(2,2-dimethylmorpholinyl)benzoic acid (75 mg, 0.13 mmol, 1.0 eq) and 4-amino-1-phenylpyrazole-3-carboxamide (50 mg, 0.25 mmol, 1.0 eq) in DMF (2.0 mL) at 0°C were added TEA (0.1 mL, 0.74 mmol, 3.0 eq) and HATU (141 mg, 0.37 mmol, 1.5 eq). The reaction solution was reacted at rt for 5 h. Upon completion, the reaction solution was purified by HPLC to afford the title compound (white solid, 20 mg, yield 19%). **¹H NMR** (400 MHz, DMSO-*d₆*) δ 10.74 (s, 1H), 8.92 (s, 1H), 8.09 (s, 1H), 7.97 (d, *J* = 7.8 Hz, 2H), 7.84 (s, 1H), 7.56 (t, *J* = 7.9 Hz, 2H), 7.45-7.38 (m, 3H), 7.28-7.20 (m, 2H), 3.81-3.77 (m, 2H), 3.17-3.13 (m, 2H), 3.04 (s, 2H), 1.26 (s, 6H). LCMS (ESI): m/z 420 [M+H]⁺.

### Preparation Example 189. Synthesis of Compound A471

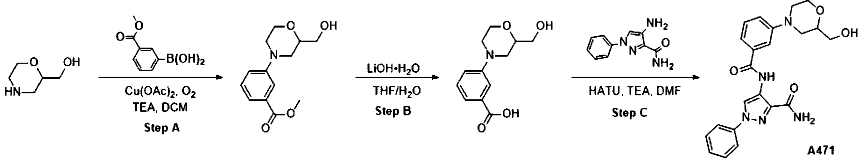

### Step A: Methyl 3-(2-(hydroxymethyl)morpholinyl)benzoate

To a solution of (3-(methoxycarbonyl)phenyl)boronic acid (350 mg, 1.95 mmol, 1.0 eq) and morpholin-2-ylmethanol (228 mg, 1.95 mmol, 1.0 eq) in DCM (5.0 mL) at 0°C were added TEA (1.1 mL, 7.78 mmol, 4.0 eq) and copper acetate (177 mg, 0.97 mmol, 0.5 eq). The reaction solution was reacted under O₂ at rt for 18 h. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by column chromatography to afford the title compound (white solid, 110 mg, yield 23%).

### Step B: 3-(2-(Hydroxymethyl)morpholinyl)benzoic acid

At 0°C, to a solution of methyl 3-(2-(hydroxymethyl)morpholinyl)benzoate (110 mg, 0.44 mmol, 1.0 eq) in THF (4.0 mL) and water (1 mL) was added lithium hydroxide monohydrate (92 mg, 2.19 mmol, 5.0 eq). The reaction solution was reacted at rt for 18 h. Upon completion, the reaction solution was adjusted to pH 5-6 with dilute HCl in an ice bath and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 30 mg, yield 29%).

### Step C: 4-(3-(2-(Hydroxymethyl)morpholinyl)benzamido)-1-phenyl-1H-pyrazole-3-carboxamide

To a solution of 3-(2-(hydroxymethyl)morpholinyl)benzoic acid (37 mg, 0.17 mmol, 1.5 eq) and 4-amino-1-phenyl-1H-pyrazole-3-carboxamide (25 mg, 0.12 mmol, 1.0 eq) in DMF (2.0 mL) at 0°C were added TEA (37 mg, 0.37 mmol, 3.0 eq) and HATU (71 mg, 0.19 mmol, 1.6 eq). The reaction solution was reacted at 60°C for 2 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (yellow solid, 18 mg, yield 35%). **¹H NMR** (400 MHz, DMSO-*d₆*) δ 10.74 (s, 1H), 8.92 (s, 1H), 8.09 (s, 1H), 7.97 (d, *J* = 7.7 Hz, 2H), 7.83 (s, 1H), 7.56 (t, *J =* 8.0 Hz, 2H), 7.45 (dd, *J =* 10.0, 5.7 Hz, 2H), 7.40 (t, *J* = 7.4 Hz, 1H), 7.28 (d, *J* = 7.8 Hz, 1H), 7.23 (dd, *J =* 8.3, 2.1 Hz, 1H), 4.82 (t, *J* = 5.8 Hz, 1H), 3.98 (dd, *J =* 11.3, 2.0 Hz, 1H), 3.73-3.62 (m, 2H), 3.59 (d, *J* = 10.4 Hz, 2H), 3.53 (dt, *J* = 10.8, 5.4 Hz, 1H), 3.46 (dt, *J* = 11.3, 5.8 Hz, 1H), 2.77 (td, *J* = 11.7, 3.3 Hz, 1H), 2.51 (s, 1H). LCMS (ESI): m/z 422 [M+H]⁺.

### Preparation Example 190. Synthesis of Compound A472

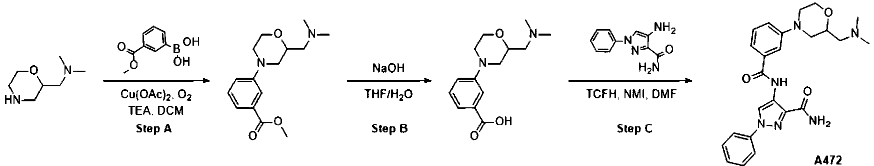

### Step A: Methyl 3-(2-((dimethylamino)methyl)morpholinyl)benzoate

To a solution of (3-(methoxycarbonyl)phenyl)boronic acid (470 mg, 2.61 mmol, 1.0 eq) and N,N-dimethyl-1-(morpholin-2-yl)methanamine (490 mg, 3.40 mmol, 1.3 eq) in DCM (5.0 mL) at 0°C were added TEA (1.5 mL, 10.4 mmol, 4.0 eq) and copper acetate (237 mg, 1.31 mmol, 0.5 eq). The reaction solution was reacted under O₂ at rt for 18 h. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by reverse-phase column chromatography to afford the title compound (black oil, 200 mg, yield 28%).

### Step B: 3-(2-((dimethylamino)methyl)morpholinyl)benzoic acid

To a solution of methyl 3-(2-((dimethylamino)methyl)morpholinyl)benzoate (120 mg, 0.43 mmol, 1.0 eq) in THF (3.0 mL) and water (1 mL) was added NaOH (86 mg, 2.16 mmol, 5.0 eq) at 0°C. The reaction solution was reacted at rt for 18 h. Upon completion, the reaction solution was adjusted to pH 5-6 with HCl in an ice bath, and extracted with EtOAc (×3). The combined organic phases were washed with saturated aqueous NaCl, dried over anhydrous Na₂SO₄, filtered, and concentrated under reduced pressure to afford the title compound (white solid, 100 mg, yield 88%).

### Step C: 4-(3-(2-((dimethylamino)methyl)morpholinyl)benzamido)-1-phenyl-1H-pyrazole-3-carboxamide

To a solution of 3-(2-((dimethylamino)methyl)morpholinyl)benzoic acid (37 mg, 0.17 mmol, 1.5 eq) and 4-amino-1-phenyl-1H-pyrazole-3-carboxamide (70 mg, 0.35 mmol, 2.0 eq) in DMF (2.0 mL) at 0°C were added N-methylimidazole (86 mg, 1.04 mmol, 6.0 eq) and TCFH (18 mg, 64 µmol, 0.4 eq). The reaction solution was reacted at 70°C for 1 h. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by HPLC to afford the title compound (white solid, 18.9 mg, yield 12%). **¹H NMR** (400 MHz, DMSO-*d₆*) δ 10.75 (s, 1H), 8.93 (s, 1H), 8.09 (s, 1H), 7.97 (d, *J* = 8.1 Hz, 2H), 7.82 (s, 1H), 7.56 (t, *J* = 7.9 Hz, 2H), 7.42 (dt, *J* = 14.8, 7.6 Hz, 3H), 7.25 (dd, *J* = 19.6, 8.0 Hz, 2H), 3.97 (d, *J* = 12.0 Hz, 1H), 3.74-3.54 (m, 4H), 2.77 (dd, *J =* 11.5, 8.1 Hz, 1H), 2.47-2.31 (m, 3H), 2.21 (s, 6H). LCMS (ESI): m/z 449 [M+H]⁺.

### Preparation Example 191. Synthesis of Compound A473

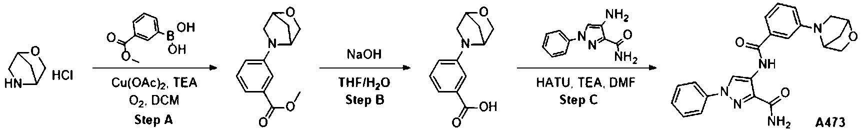

### Step A: Methyl 3-(2-oxa-5-azabicyclo[2.2.1]heptan-5-yl)benzoate

To a solution of (3-(methoxycarbonyl)phenyl)boronic acid (597 mg, 3.32 mmol, 1.5 eq) and 2-aza-5-oxabicyclo[2.2.1]heptane hydrochloride (300 mg, 2.21 mmol, 1.0 eq) in DCM (4.0 mL) at 0°C were added TEA (1.2 mL, 8.85 mmol, 4.0 eq) and copper acetate (201 mg, 1.11 mmol, 0.5 eq). The reaction solution was reacted under O₂ at rt for 18 h. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by column chromatography to afford the title compound (yellow oil, 80 mg, yield 16%).

### Step B: 3-(2-oxa-5-azabicyclo[2.2.1]hept-5-yl)benzoic acid

At 0°C, to a solution of methyl 3-(2-oxa-5-azabicyclo[2.2.1]heptan-5-yl)benzoate (70 mg, 0.30 mmol, 1.0 eq) in THF (0.5 mL) and water (0.2 mL) was added NaOH (48 mg, 1.20 mmol, 4.0 eq). The reaction solution was reacted at 50°C for 18 h. Upon completion, the reaction solution was adjusted to pH 5-6 with dilute HCl in an ice bath, and extracted with EtOAc (×3). The combined organic phases were washed with saturated aqueous NaCl, dried over anhydrous Na₂SO₄, filtered, and concentrated under reduced pressure to afford the title compound (yellow oil, 45 mg, crude).

### Step C: 4-(3-(2-oxa-5-azabicyclo[2.2.1]heptan-5-yl)benzamido)-1-phenyl-1H-pyrazole-3-carboxamide

To a solution of 3-(2-oxa-5-azabicyclo[2.2.1]heptan-5-yl)benzoic acid (37 mg, crude) and 4-amino-1-phenyl-1H-pyrazole-3-carboxamide (30 mg, 0.15 mmol, 1.0 eq) in DMF (2.0 mL) were added TEA (45 mg, 0.45 mmol, 3.0 eq) and HATU (85 mg, 0.22 mmol, 1.5 eq) at 0°C. The reaction solution was reacted at rt for 5 h. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by HPLC to afford the title compound (white solid, 17 mg, yield 28%). **¹H NMR** (400 MHz, DMSO-*d₆*) δ 10.74 (s, 1H), 8.92 (s, 1H), 8.09 (s, 1H), 7.97 (d, *J* = 8.1 Hz, 2H), 7.83 (s, 1H), 7.56 (t, *J =* 7.8 Hz, 2H), 7.38 (dd, *J* = 17.2, 7.6 Hz, 2H), 7.16-7.03 (m, 2H), 6.90 (d, *J* = 8.1 Hz, 1H), 4.65 (d, *J* = 10.5 Hz, 2H), 3.79 (d, *J* = 7.3 Hz, 1H), 3.69 (d, *J =* 7.4 Hz, 1H), 3.57 (d, *J* = 9.1 Hz, 1H), 3.03 (d, *J* = 9.4 Hz, 1H), 1.97 (d, *J* = 9.6 Hz, 1H), 1.89 (d, *J =* 9.5 Hz, 1H). LCMS (ESI): m/z 404 [M+H]⁺.

### Preparation Example 192. Synthesis of Intermediate Int3

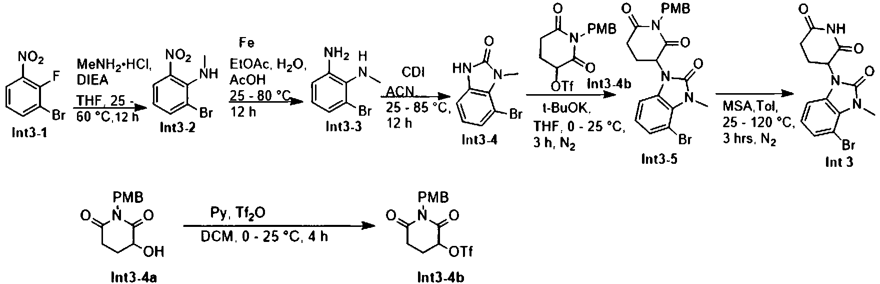

Compound Int3-1 (64.0 g, 291 mmol, 1.00 eq) was added to a 2.00 L three-necked round-bottom flask, THF (640 mL) was added at 25°C, and DIEA (169 g, 1.31 mol, 228 mL, 4.50 eq) and dimethylamine hydrochloride (88.4 g, 1.31 mol, 4.50 eq) were added at 25°C. The mixture was heated to 60°C and stirred for 12 h. LCMS indicated complete consumption of compound Int3-1, with the target mass spectrum detected (RT = 0.418 min). The reaction solution was poured into 1000 mL of water and extracted twice with EtOAc (1000 mL). The combined organic phases were washed twice with saturated aqueous NaCl (1000 mL), dried over Na₂SO₄, filtered, and concentrated to yield compound Int3-2 (67.0 g, 290 mmol, yield 99.6%, 100% purity) as a red liquid.

Compound Int3-2 (64.0 g, 291 mmol, 1.00 eq) was added to a 3 L three-necked round-bottom flask. Ethyl acetate (983 mL) and water (32.8 mL) were added to the three-necked flask at 25°C. Glacial acetic acid (328 mL) was also added at 25°C. The mixture was heated to 50°C, and iron powder (63.3 g, 1.13 mol, 4.00 eq) was added portionwise to the reaction solution. The mixture was heated to 80°C and reacted for 12 h. TLC (petroleum ether : ethyl acetate = 5 : 1) indicated complete consumption of compound Int3-2 (R_{f} = 0.60), with the formation of a new spot (R_{f} = 0.50). The mixture was cooled to 25°C and filtered. The filtrate was concentrated. Saturated aqueous Na₂CO₃ (1000 mL) was added, and the mixture was extracted twice with EtOAc (1000 mL). The organic phase was washed twice with saturated aqueous NaCl (1000 mL), dried over Na₂SO₄, and concentrated to yield compound Int3-3 (60.0 g, 274 mmol, yield 96.8%, 92% purity) as a yellow liquid.

Compound Int3-3 (59.1 g, 270 mmol, 1.00 eq) was added to a 2 L three-necked round-bottom flask. Acetonitrile (887 mL) was added at 25°C. CDI (87.7 g, 541 mmol, 2.00 eq) was added at 25°C under N₂. The mixture was heated to 85°C and reacted for 12 h. LCMS indicated complete consumption of compound Int3-3, with the target mass spectrum detected (RT = 0.410 min). The mixture was filtered and concentrated, and the residue was washed with water (500 mL), filtered, and dried to yield the crude. The crude was slurried with petroleum ether : ethyl acetate (1 : 1) (500 mL) to yield compound Int3-4 (52.2 g, 220 mmol, yield 81.4%, 95.8% purity) as an off-white solid.

Compound Int3-4a (50.0 g, 201 mmol, 1.00 eq) was added to a 2.00 L three-necked round-bottom flask, followed by the addition of DCM (500 mL). Pyridine (31.7 g, 401 mmol, 32.4 mL, 2.00 eq) was added at 20°C-25°C under N₂, and the mixture was stirred at 0°C-5°C for 1 h. Trifluoromethanesulfonic anhydride (113 g, 401 mmol, 66.5 mL, 2.00 eq) was then added at 0°C. The mixture was heated to 25°C and stirred for 3 h. TLC (petroleum ether : ethyl acetate = 2 : 1) indicated complete consumption of compound Int3-4a, forming a new major spot (R_{f} = 0.60). The reaction solution was filtered, and the filtrate was concentrated to obtain the crude. The crude was separated by silica gel column chromatography to obtain compound Int3-4b (55.1 g, 133 mmol, yield 66.4%, 92.2% purity) as a yellow oil.

Compound Int3-4 (12.1 g, 50.9 mmol, 1.00 eq) was added to a 1 L three-necked round-bottom flask. THF (302 mL) was added at 25°C. Potassium tert-butoxide (6.85 g, 61.1 mmol, 1.20 eq) was also added at 25°C. The temperature was lowered to 0°C and the reaction was allowed to proceed for 0.5 h. Compound Int3-4b (19.4 g, 50.9 mmol, 1.00 eq) dissolved in THF (97.0 mL) was added to a reaction flask at 0°C. The temperature was raised to 25°C and the reaction was allowed to proceed for 2.5 h. LCMS indicated complete consumption of compound Int3-4, with the target mass spectrum detected (RT = 0.508 min). The reaction mixture was poured into an aqueous NH₄Cl (500 mL). The aqueous phase was extracted with EtOAc (500 mL×2). The combined organic phases were washed with aqueous NaCl (500 mL×2), dried over anhydrous Na₂SO₄, filtered, and concentrated in vacuo to obtain the crude. The crude was purified by preparative HPLC. The eluent was concentrated in vacuo to remove ACN. The aqueous phase was extracted with EtOAc (100 mL×2). The organic phase was dried over anhydrous Na₂SO₄, filtered, and concentrated in vacuo to obtain compound Int3-5 (18.0 g, 37.6 mmol, yield 73.9%, 95.8% purity) as a purple solid.

Compound Int3-5 (16.1 g, 33.7 mmol, 1.00 eq) was added to a 500 mL three-necked round-bottom flask. Toluene (97 mL) and methanesulfonic acid (48.5 mL) were added at 20°C. The mixture was heated to 120°C and reacted for 3 h. LCMS indicated complete consumption of compound Int3-5, with the target mass spectrum detected (RT = 0.359 min). The mixture was filtered and concentrated, and the crude was poured into water (50 mL), and filtered again. The filter cake was collected and dried in vacuo. The crude was slurried with petroleum ether (50 mL) to obtain compound Int3 (7.40 g, 18.8 mmol, yield 55.7%, 85.9% purity) as an off-white solid. LCMS: m/z =339.9 (M+H)⁺. **¹H NMR:** (400 MHz, DMSO-*d₆*) *δ* 11.1 (s, 1H), 7.25-7.22 (m, 1H), 7.17 (d, J = 8.0 Hz, 1H), 7.00-6.96 (m, 1H), 5.41 (dd, J = 12.6 Hz, 1H), 3.63 (s, 3H), 2.93-2.84 (m, 1H), 2.76-2.61 (m, 2H), 2.05-1.98 (m, 1H).

### Preparation Example 193. Synthesis of Compound A109

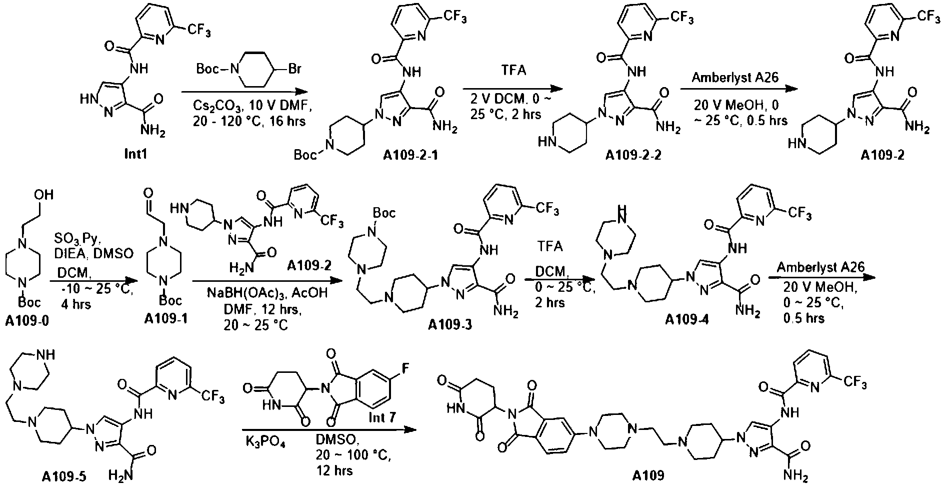

At 20°C-25°C, tert-butyl 4-bromopiperidine-1-carboxylate (2.98 g, 11.3 mmol, 2.00 eq) and DMF (20.0 mL) were added to a 100 mL three-necked round-bottom flask. Compound Int1 (2.00 g, 5.65 mmol, 1.00 eq) and Cs₂CO₃ (3.68 g, 11.3 mmol, 2.00 eq) were then added at 20°C-25°C. The mixture was reacted at 120°C for 16 h. LCMS indicated that 35.8% of compound Int1 remained unreacted, with the target mass spectrum (46.4%) detected (RT = 0.713 min). The mixture was filtered and concentrated, and the crude was purified by column chromatography (SiO₂, petroleum ether : ethyl acetate = 1 : 1). After concentration, compound A109-2-1 (1.50 g, 2.96 mmol, yield 52.3%, 95.1% purity) was obtained as a yellow solid.

DCM (3.00 mL) was added to a 40.0 mL single-necked flask. Compound A109-2-1 (1.50 g, 2.96 mmol, 1.00 eq) and TFA (10.1 g, 88.7 mmol, 6.59 mL, 30.0 eq) were added at 20°C-25°C, and the mixture was reacted at 20°C -25°C for 2 h. TLC (petroleum ether : ethyl acetate = 1 : 1) indicated the formation of a new major spot. After concentration, compound A109-2-2 (1.53 g, crude) was obtained as a white solid.

Compound A109-2-2 (1.53 g, 4.00 mmol, 1.00 eq) was added to a 100 mL three-necked flask, followed by the addition of MeOH (30.0 mL) and basic resin (10.0 g, 4.00 mmol, 1.00 eq), and the mixture was stirred for 0.5 h. TLC (DCM : MeOH=1 : 2) indicated the formation of a new major spot (R_{f} = 0). The mixture was filtered, and the filtrate was concentrated to afford compound A109-2 (0.80 g, 2.08 mmol, yield 52.0%, 99.5% purity) as a white solid.

DCM (50.0 mL) was added to a 250 mL three-necked flask. At-10°C under N₂, compound A109-0 (5.00 g, 21.7 mmol, 1.00 eq), DMSO (13.6 g, 174 mmol, 13.5 mL, 8.00 eq), and DIEA (11.2 g, 86.8 mmol, 15.0 mL, 4.00 eq) were added. Pyridine sulfur trioxide complex (12.1 g, 75.9 mmol, 3.50 eq) was then added portionwise. The mixture was reacted at -10°C -0°C for 4 h. TLC (DCM : MeOH = 20 : 1) indicated complete consumption of the starting material (R_{f} = 0.20), with the formation of a major spot (R_{f} = 0.30). The raw material was poured into water (80.0 mL) and DCM (250 mL), and the mixture was subjected to liquid separation, and extracted once with DCM (60.0 mL). The combined organic phase was washed with saturated aqueous NaCl (80.0 mL), dried over Na₂SO₄, filtered, and concentrated to afford compound A109-1 (4.96 g, crude) as a yellow oil, which was used directly in the next step.

Compound A109-1 (1.90 g, 8.33 mmol, 4.00 eq) and compound A109-2 (800 mg, 2.08 mmol, 1.00 eq) were added to a 40 mL single-necked flask, and DMF (8.00 mL), AcOH (500 mg, 8.33 mmol, 477 µL, 4.00 eq) and NaBH(OAc)₃ (882.49 mg, 4.16 mmol, 2.00 eq) were added. The reaction continued at 20°C-25°C for 12 h. LCMS indicated complete consumption of compound A109-1, with the target mass spectrum (88.1%) detected (RT = 0.594 min). Saturated aqueous NaHCO₃ (5.00 mL), water (80.0 mL) and ethyl acetate (150 mL) were added, and the mixture was subjected to liquid separation and extracted once with EtOAc (30.0 mL). The combined organic phases were washed with saturated aqueous NaCl (60.0 mL), dried over Na₂SO₄, filtered, and concentrated. The crude was purified by preparative HPLC, and the eluate was lyophilized to afford compound A109-3 (1.00 g, 1.65 mmol, yield 67.0%, 97.9% purity) as a yellow solid.

Compound A109-3 (1.00 g, 1.65 mmol, 1.00 eq) was added to a 40 mL single-necked flask, and TFA (2.82 g, 24.7 mmol, 1.83 mL, 15.0 eq) and DCM (10.0 mL) were added. The mixture was reacted at 20°C-25°C for 2 h. TLC (DCM : MeOH = 10 : 1) indicated the formation of a new spot (R_{f} = 0.30). The mixture was filtered, and the filtrate was concentrated to afford compound A109-4 (814 mg, crude) as a yellow solid.

Compound A109-4 (814 mg, 1.65 mmol, 1.00 eq) was added to a 100 mL three-necked flask, followed by the addition of MeOH (16.0 mL) and basic resin A26 (4.00 g, 1.65 mmol, 1.00 eq), and the mixture was stirred for 0.5 h. TLC (DCM : MeOH = 10 : 1) indicated the formation of a major spot (R_{f} = 0.1). The mixture was filtered, and the filtrate was concentrated to afford compound A109-5 (814 mg, crude) as a yellow solid.

Compound Int 7 (910 mg, 2.67 mmol, 2.00 eq) and DMSO (16.0 mL) were added to a 100 mL three-necked flask, followed by the addition of compound A109-5 (814 mg, 1.33 mmol, 1.00 eq) and K₃PO₄ (1.75 g, 6.67 mmol, 5.00 eq). The mixture was heated to 100°C and reacted for 12 h. LCMS indicated complete consumption of A109-5, with the target mass spectrum (32.1%) detected (RT = 0.553 min). Water (50.0 mL) and DCM (100 mL) were poured into the mixture, and the resulting mixture was subjected to liquid separation and extracted once with DCM (50.0 mL). The combined organic phase was washed with saturated aqueous NaCl (60.0 mL), dried over Na₂SO₄, filtered, and concentrated. The crude was purified by preparative HPLC to obtain compound A109 (70.0 mg, 88.8 µmol, yield 6.40%, 95.2% purity) as a yellow solid. LCMS: m/z =751.4 (M+H)⁺. ¹H **NMR:** (400 MHz, DMSO-*d₆*) *δ* 11.71 (s, 1H) 11.08 (br s, 1H) 8.34-8.47 (m, 3H) 8.18 (d, *J =* 7.2 Hz, 1H) 7.64-7.71 (m, 1H) 7.56 (br s, 1H) 7.34 (s, 1H) 7.26 (br d, *J* = 8.2 Hz, 1H) 5.07 (dd, *J =* 12.0, 5.4 Hz, 1H) 4.23-4.31 (m, 1H) 3.44 (br s, 5H) 3.02 (br d, *J =* 10.0 Hz, 2H) 2.80-2.94 (m, 1H) 2.53-2.62 (m, 9H) 2.09-2.17 (m, 2H) 2.03 (br d, *J =* 5.6 Hz, 5H).

### Preparation Example 194. Synthesis of Compound A110

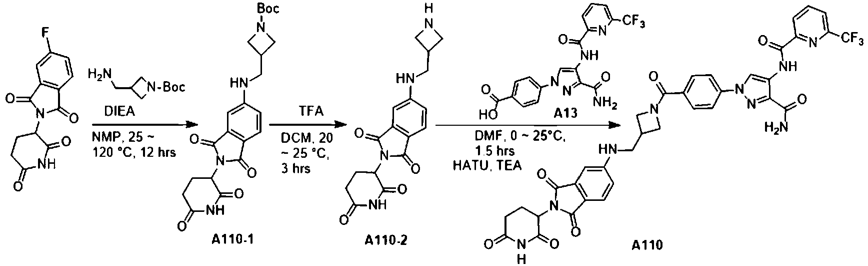

Compound Int7 (6.74 g, 24.4 mmol, 1.00 eq), 1-Boc-3-aminomethylazetidine (5.00 g, 26.8 mmol, 1.10 eq) and NMP (30.0 mL) were added to a 100 mL three-necked flask, and DIEA (6.31 g, 48.8 mmol, 8.50 mL, 2.00 eq) was added. The mixture was heated to 120°C and reacted for 12 h. LCMS indicated complete consumption of compound Int7, with the target mass spectrum detected (RT = 0.425 min). The reaction solution was poured into water (100 mL), and extracted with DCM (×3) (100 mL), and the combined organic phase was washed with saturated aqueous NaCl (200 mL), dried over Na₂SO₄, filtered, and concentrated. The crude was purified by column chromatography (SiO₂, petroleum ether/EtOAc = 10/1 to 8/1, R_{f} = 0.7) to afford compound A110-1 (3.40 g, 6.72 mmol, yield 27.5%, 87.5% purity) as a dark blue oil.

Compound A110-1 (500 mg, 989 µmol, 1.00 eq) and DCM (2.40 mL) were added to a 100 mL three-necked flask, followed by the addition of TFA. The mixture was reacted at 20°C-25°C for 3 h. LCMS indicated complete consumption of compound A110-1, with the target mass spectrum detected (RT = 0.203 min). The mixture was concentrated to afford compound A110-2 (600 mg, crude) as a green solid.

Compound A13 (100 mg, 238 µmol, 1.00 eq), TEA (144 mg, 1.43 mmol, 199 µL, 6.00 eq), DMF (5.00 mL), and HATU (90.6 mg, 238 µmol, 1.00 eq) were added to a 40 mL single-necked flask and reacted at 0°C-5°C for 0.5 h. Compound A110-2 (300 mg, 876 µmol, 3.67 eq) was then added and reacted at 20°C-25°C for 1 h. LCMS indicated complete consumption of compound A13, with the target mass spectrum detected (RT = 0.446 min). The mixture was filtered, and the filter cake was washed with DMF (0.5 mL). The crude was purified by preparative HPLC, and the mobile phase was lyophilized to afford compound A110 (44.6 mg, 57.9 µmol, yield 24.3%, 96.6% purity) as a yellow solid. LCMS: m/z = 744.1 **(M+H)⁺. ¹H NMR:** (400 MHz, DMSO-*d₆*) *δ* 11.87-11.78 (m, 1H) 11.16-11.00 (m, 1H) 9.25-9.06 (m, 1H) 8.53-8.38 (m, 2H) 8.23 (d, J = 7.6 Hz, 1H) 8.15-8.06 (m, 3H) 7.88-7.78 (m, 3H) 7.58 (d, J = 8.2 Hz, 1H) 7.34-7.27 (m, 1H) 7.06-7.00 (m, 1H) 6.95-6.88 (m, 1H) 5.10-4.99 (m, 1H) 4.52-4.42 (m, 1H) 4.07 (s, 2H) 3.87-3.79 (m, 1H) 3.50 (d, J = 5.6 Hz, 2H) 3.01-2.92 (m, 2H) 2.89-2.83 (m, 1H) 2.60 (d, J = 3.2 Hz, 1H) 2.04-1.97 (m, 1H).

### Preparation Example 195. Synthesis of Compound A111

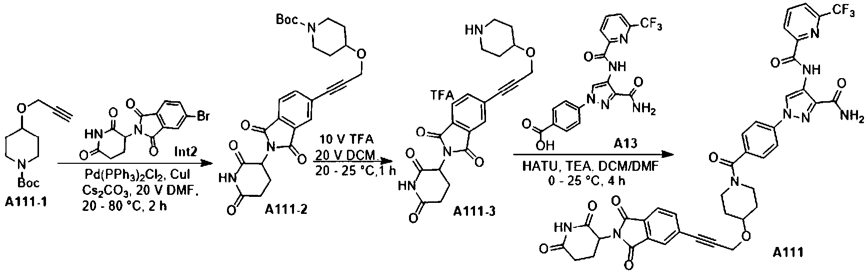

At 20°C-25°C, compound Int2 (375 mg, 1.11 mmol, 1.00 eq), compound A111-1 (400 mg, 1.67 mmol, 1.50 eq) and DMF (7.50 mL) were added to a 100 mL three-necked round-bottom flask. Under N₂, CuI (42.4 mg, 222 µmol, 0.20 eq), Pd (PPh₃)₂Cl₂ (156 mg, 222 µmol, 0.20 eq) and Cs₂CO₃ (1.45 g, 4.46 mmol, 4.00 eq) were added. The mixture was purged with N₂ (×3). The mixture was heated to 80°C and reacted for 2 h. LCMS indicated complete consumption of compound Int2, with the target mass spectrum detected (RT = 0.503 min). The mixture was cooled to 20°C-25°C, and poured into water (35.0 mL), and the aqueous phase was extracted with EtOAc (35.0 mL×3). The organic layer was washed with aqueous NaCl (40.0 mL), dried over Na₂SO₄, and concentrated. The crude was purified by preparative HPLC, and the eluate was lyophilized to afford compound A111-2 (130 mg, 251 µmol, yield 22.5%, 95.7% purity) as a yellow solid.

Compound A111-2 (130 mg, 251 mmol, 1.00 eq) and DCM (7.50 mL) were added to an 8 mL single-necked flask at 20°C-25°C. TFA (2.00 g, 17.5 mmol, 1.30 mL, 69.7 eq) was added at 20°C-25°C, and the reaction was allowed to proceed at 25°C for 1 h. LCMS indicated complete consumption of compound A111-2, with the target mass spectrum detected (RT = 0.272 min). The mixture was concentrated to afford compound A111-3 (130 mg, 243 µmol, yield 97.1%, 95.6% purity, TFA) as a yellow solid, which was used directly in the next step.

Compound A13 (131 mg, 314 mmol, 1.00 eq), TEA (95.4 mg, 942 µmol, 131 µL, 3.00 eq), and DCM (7.50 mL) were added to a 100 mL three-necked round-bottom flask (R1) at 20°C-25°C. HATU (119 mg, 314 µmol, 1.00 eq) was added at 20°C-25°C. The atmosphere was purged with N₂ (×3). The mixture was reacted at 0°C-5°C for 1 h. At 25°C, compound A111-3 (130 mg, 314 µmol, 1.00 eq, TFA), TEA (95.4 mg, 942 µmol, 131 µL, 3.00 eq) and DMF (4.00 mL) were added to another 10 mL single-necked flask (R2), and added to a three-necked flask at 0°C-5°C, and the mixture was reacted at 20°C-25°C for 3 h. LCMS indicated complete consumption of compound A111-3, with the target mass spectrum detected (RT = 1.620 min). The reaction solution was concentrated. The crude was purified by preparative HPLC, and the eluate was lyophilized to obtain compound A111 (63.3 mg, 78.9 µmol, yield 52.4%, purity 99.3%) as an off-white solid. LCMS: m/z = 797.1 (M+H)⁺. **¹H NMR:** (400 MHz, DMSO-*d₆*) *δ* 11.80 (s, 1H), 11.14 (s, 1H), 9.09 (s, 1H), 8.51-8.44 (m, 1H), 8.44-8.35 (m, 1H), 8.22 (d, *J* = 8.0 Hz, 1H), 8.08 (br d, *J=* 8.4 Hz, 3H), 8.01-7.87 (m, 3H), 7.82 (br s, 1H), 7.59 (d, *J* = 8.4 Hz, 2H), 5.17 (dd, *J* = 5.6, 12.8 Hz, 1H), 4.54 (s, 2H), 4.10-3.94 (m, 1H), 3.91-3.83 (m, 1H), 2.93-2.83 (m, 1H), 2.69-2.53 (m, 4H), 2.47-2.24 (m, 1H), 2.14-2.03 (m, 1H), 2.02-1.79 (m, 2H), 1.70-1.46 (m, 2H).

### Preparation Example 196. Synthesis of Compound A112

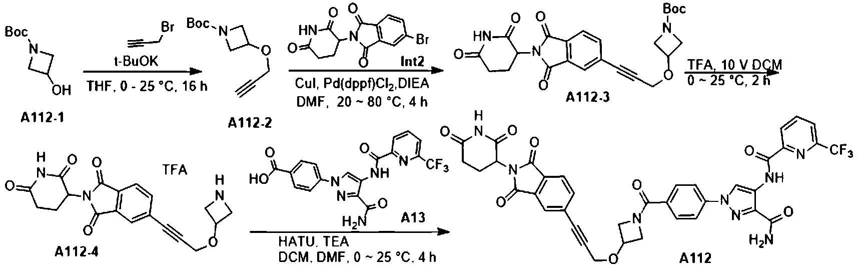

Compound A112-1 (150 mg, 361 µmol, 1.00 eq) and THF (10.0 mL) were added to a 40 mL single-necked reaction flask at 15°C-25°C. t-BuOK (777 mg, 6.93 mmol, 1.20 eq) and 3-bromopropyne (901 mg, 6.06 mmol, 653 µL, 1.05 eq) were then added at 0°C. The mixture was reacted at 15°C-25°C for 16 h. TLC (petroleum ether : ethyl acetate = 1 : 1) indicated complete consumption of compound A112-1 (Rf = 0.1), with a new spot (Rf = 0.4) was detected. The mixture was poured into water (20 mL) and extracted with DCM (×3) (20 mL). The combined organic phases were washed with saturated aqueous NaCl (50 mL), dried over anhydrous Na₂SO₄, filtered, and concentrated to yield compound A112-2 (1.07 g, 5.06 mmol, yield 87.6%, 100% purity) as a white solid.

Compound A112-2 (799 mg, 3.78 mmol, 1.50 eq), compound Int2 (850 mg, 2.52 mmol, 1.00 eq), and DMF (17.0 mL) were added to a 100 mL three-necked round-bottom flask at 20°C-25°C. DIEA (1.30 g, 10.1 mmol, 1.76 mL, 4.00 eq), Pd (dppf)Cl₂ (368 mg, 504 µmol, 0.20 eq), and CuI (96.0 mg, 504 µmol, 0.20 eq) were added. The mixture was purged with N₂ (×3), heated to 80°C and reacted for 4 h. LCMS indicated complete consumption of compound Int2, with the target mass spectrum (40.1%) detected. The product was poured into water (60.0 mL) and extracted with EtOAc (×3) (50.0 mL). The combined organic phases were washed with saturated aqueous NaCl (60.0 mL), dried over Na₂SO₄, filtered, and concentrated. The crude was purified by preparative HPLC and lyophilized to obtain compound A112-3 (0.45 g, 786 µmol, yield 29.7%, 81.7% purity) as a yellow solid.

Compound A112-3 (350 mg, 611 µmol, 1.00 eq) was added to a 10 mL single-necked flask. DCM (3.50 mL) and TFA (1.05 g, 9.18 mmol, 681 µL, 15.0 eq) were added at 0°C-5°C, and the mixture was heated to 25°C and reacted for 2 h. LCMS indicated complete consumption of compound A112-3, with the target mass spectrum detected (RT = 0.256 min). The reaction solution was concentrated to afford compound A112-4 (0.80 g, crude, TFA) as a black oil.

At 25°C under N₂, compound A13 (144 mg, 332 µmol, 1.00 eq), TEA (101 mg, 998 µmol, 139 µL, 3.00 eq), and DCM (7.00 mL) were added to a 100 mL three-necked round-bottom flask. The temperature was lowered to 0°C-5°C, and HATU (126 mg, 332 µmol, 1.00 eq) was added. The mixture was stirred for 1 h, and compound A112-4 (350 mg, 332 µmol, 1.00 eq, TFA), TEA (101 mg, 998 µmol, 139 µL, 3.00 eq), and DMF (10.5 mL) were added and reacted for 3 h. LCMS indicated 4.89% of compound A13 remained, with the target mass spectrum (58.0%) detected. The mixture was filtered, and the mother liquor was purified by preparative HPLC. The eluate was lyophilized to afford compound A112 (131 mg, 163 µmol, yield 49.1%, purity 95.7%) as a yellow solid. LCMS: m/z =769.1 (M+H)⁺. **¹H NMR:** (400 MHz, DMSO-*d₆*) *δ* 11.80 (s, 1H) 11.14 (s, 1H) 9.11 (s, 1H) 8.48-8.37 (m, 2H) 8.22 (dd, *J* = 7.6, 0.88 Hz, 1H) 8.10 (br d, *J* = 8.8 Hz, 3H) 8.00-7.92 (m, 3H) 7.87-7.80 (m, 3H) 5.17 (dd, *J =* 12.0, 5.4 Hz, 1H) 4.68-4.50 (m, 4H) 4.42-4.32 (m, 2H) 4.05-3.95 (m, 1H) 2.95-2.81 (m, 1H) 2.65-2.53 (m, 2H) 2.11-2.01 (m, 1H).

### Preparation Example 197. Synthesis of Compound A113

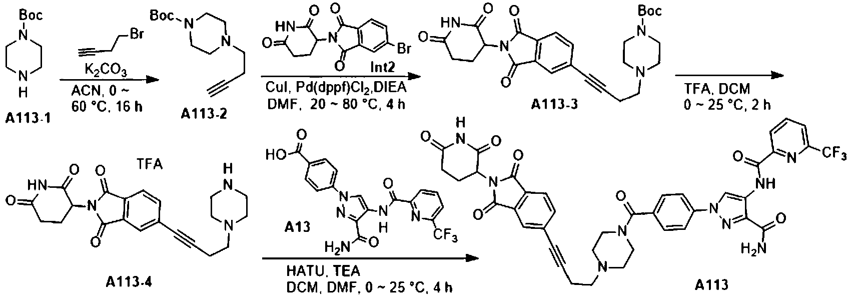

Compound A113-1 (1.00 g, 5.37 mmol, 1.00 eq) was added to a 40 mL single-necked flask at 15°C-25°C, followed by the addition of acetonitrile (5.00 mL) and potassium carbonate (1.11 g, 8.05 mmol, 1.50 eq). The temperature was lowered to 0°C, and 4-bromobutyne (785 mg, 5.91 mmol, 1.10 eq) was added. The mixture was heated to 60°C and reacted for 16 h. TLC (petroleum ether : ethyl acetate = 1 : 1) indicated complete consumption of compound A113-1 (Rf = 0.1), with the formation of a new major spot (Rf = 0.5). The mixture was poured into 20 mL of water and extracted with EtOAc (×3) (20 mL). The combined organic phases were washed with saturated aqueous NaCl (50 mL), dried over Na₂SO₄, filtered, and concentrated. The crude was separated by column chromatography (SiO₂, petroleum ether : ethyl acetate = 20 : 1 to 10 : 1) to obtain compound A113-2 (0.98 g, 4.13 mmol, yield 76.9%) as a white oil.

At 20°C-25°C, compound Int2 (620 mg, 1.84 mmol, 1.00 eq), compound A113-2 (657 mg, 2.76 mmol, 1.50 eq), and DMF (12.4 mL) were added to a 100 mL three-necked round-bottom flask, followed by the addition of Pd(dppf)Cl₂ (269 mg, 367 µmol, 0.20 eq), DIEA (950 mg, 7.36 mmol, 1.28 mL, 4.00 eq), and cuprous iodide (70.0 mg, 367 µmol, 0.20 eq). The mixture was purged with N₂ (×3), heated to 80°C and reacted for 4 h. LCMS indicated complete consumption of compound Int2, with the target mass spectrum (43.7%) detected. The reaction solution was poured into 40 mL of water and extracted with EtOAc (×3) (30 mL). The combined organic phases were washed with saturated aqueous NaCl (60 mL), dried over Na₂SO₄, filtered, and concentrated. The crude was purified by preparative HPLC, and the eluate was lyophilized to yield compound A113-3 (160 mg, 162 µmol, yield 8.81%, 50.1% purity) as a white solid.

Compound A113-3 (370 mg, 730 µmol, 1.00 eq) was added to a 10 mL single-necked flask, and the mixture was cooled to 0°C-5°C. DCM (3.70 mL) and TFA (1.25 g, 10.9 mmol, 813 µL, 15.0 eq) were added. The mixture was heated to 20°C-25°C and reacted for 2 h. LCMS indicated complete consumption of compound A1 13-3, with the target mass spectrum (95.8%) detected (RT = 0.236 min). The reaction solution was concentrated to yield compound A114-4 (1.00 g, crude, TFA) as a yellow oil.

At 25°C under N₂, compound A13 (200 mg, 463 µmol, 1.00 eq), TEA (140 mg, 1.39 mmol, 193 µL, 3.00 eq), and DCM (10.0 mL) were added to a 100 mL three-necked round-bottom flask. The mixture was cooled to 0°C-5°C, and HATU (176 mg, 463 µmol, 1.00 eq) was added. The mixture was stirred for 1 h, followed by the addition of compound A113-4 (500 mg, 463 µmol, 1.00 eq, TFA), TEA (140 mg, 1.39 mmol, 193 µL, 3.00 eq), and DMF (15.0 mL). The mixture was then heated to 20°C-25°C and reacted for 3 h. LCMS indicated 4.2% of compound A13 remained (RT = 0.440 min), with the target mass spectrum (68.9%) detected. The mixture was poured into 30 mL of water and extracted with EtOAc (×3) (25.0 mL). The combined organic phases were washed with saturated aqueous NaCl (50.0 mL), dried over Na₂SO₄, filtered, and concentrated. The crude is purified by preparative HPLC, and the eluate was lyophilized to obtain compound A113 (233 mg, 286 µmol, yield 61.7%, purity 97.7%) as a white solid. LCMS: m/z =796.2 (M+H)⁺. **¹H NMR:** (400 MHz, DMSO-*d₆*) *δ* 11.81 (s, 1H) 11.14 (s, 1H) 9.13 (s, 1H) 8.48-8.38 (m, 2H) 8.23 (dd, *J* = 7.6, 1.2 Hz, 1H) 8.18-8.04 (m, 3H) 7.98-7.89 (m, 3H) 7.85 (s, 1H) 7.66 (d, *J* = 8.6 Hz, 2H) 5.17 (dd, *J =* 12.0, 5.4 Hz, 1H) 3.58-3.13 (m, 9H) 3.05 (br d, *J* = 6.2 Hz, 2H) 2.95-2.83 (m, 1H) 2.68-2.53 (m, 3H) 2.11-2.02 (m, 1H).

### Preparation Example 198. Synthesis of Compound A114

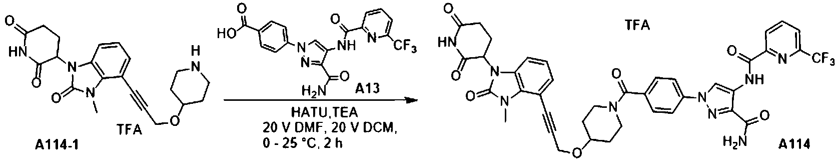

Compound A13 (261 mg, 602 µmol, 1.00 eq) and DCM (6.50 mL) were added to a 100 mL three-necked round-bottom flask. TEA (183 mg, 1.80 mmol, 251 µL, 3.00 eq) and HATU (229 mg, 602 µmol, 1.00 eq) were added at 20°C-25°C. The mixture was stirred at 0°C for 1 h. Compound A114-1 (250 mg, 602 µmol, 1.00 eq), TEA (183 mg, 1.80 mmol, 251 µL, 3.00 eq) and DMF (7.50 mL) were then added. The mixture was heated to 25°C and reacted for 2 h. LCMS indicated complete consumption of compound A114-1, with the target mass spectrum detected (RT = 0.419 min). The reaction solution was filtered, the mother liquor was concentrated, and the crude was purified by preparative HPLC to obtain compound A114 (129 mg, 154 µmol, yield 25.6%, purity 95.1%) as an off-white solid. LCMS: m/z = 798.2 (M+H)⁺. **¹H NMR:** (400 MHz, DMSO-*d₆*) *δ* 11.80 (s, 1H), 11.12 (s, 1H), 9.09 (s, 1H), 8.47-8.38 (m, 2H), 8.22 (d, J = 7.4 Hz, 1H), 8.07 (d, J = 8.6 Hz, 3H), 7.82 (s, 1H), 7.58 (d, J = 8.6 Hz, 2H), 7.18-7.01(m, 3H), 5.41-5.37 (m, 1H), 4.53 (s, 2H), 4.01-3.97 (m, 1H), 3.87-3.82 (m, 1H), 3.65 (s, 3H), 3.34-3.32 (m, 3H), 2.91-2.84 (m, 1H), 2.72-2.60 (m, 2H), 2.03-1.91 (m, 3H), 1.59-1.51 (m, 2H).

### Preparation Example 199. Synthesis of Compound A115

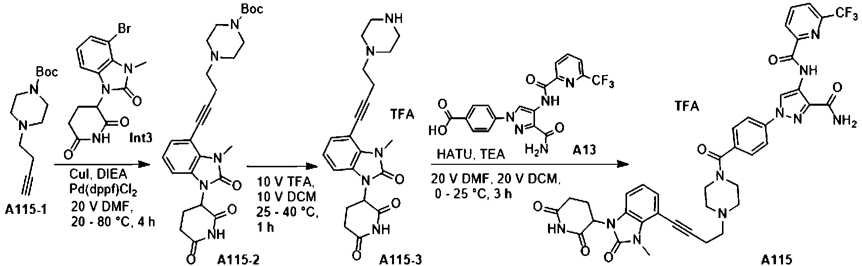

Compound A115-1 (500 mg, 1.27 mmol, 1.00 eq) and compound Int3 (757 mg, 3.18 mmol, 2.50 eq) were added to a 100 mL three-necked round-bottom flask, followed by the addition of DMF (10.0 mL). CuI (48.4 mg, 254 µmol, 0.20 eq), Pd(dppf)Cl₂ (186 mg, 254 µmol, 0.20 eq) and DIEA (657 mg, 5.08 mmol, 885 µL, 4.00 eq) were then added under N₂. The mixture was heated to 80°C and reacted for 4 h. LCMS indicated 22.1% of compound A115-1 remained (RT = 0.359 min), with the target mass spectrum detected (RT = 0.313 min). The reaction solution was filtered and concentrated, and the crude was purified by preparative HPLC. The eluate was concentrated to afford compound A115-2 (425 mg, 723 µmol, 56.9%, purity 84.3%) as a yellow solid.

Compound A115-2 (375 mg, 638 µmol, 1.00 eq) was added to a 100 mL three-necked round-bottom flask. DCM (4.00 mL) and TFA (6.14 g, 53.8 mmol, 4.00 mL, 84.4 eq) were added at 25°C, and the mixture was heated to 40°C and reacted for 1 h. LCMS indicated complete consumption of compound A1 15-2, with the target mass spectrum detected (RT = 0.224 min). The reaction solution was filtered, and the mother liquor was concentrated to afford compound A115-3 (400 mg, crude, TFA) as brown oil.

Compound A13 (340 mg, 785 µmol, 1.00 eq) was added to a 100 mL three-necked round-bottom flask. DMF (9.00 mL) and TEA (238 mg, 2.36 mmol, 328 µL, 3.00 eq) were added at 25°C under N₂. The temperature was lowered to 0°C-5°C, and HATU (298 mg, 785 µmol, 1.00 eq) was added. The reaction was allowed to proceed for 1 h. Compound A115-3 (400 mg, 785 µmol, 1.00 eq, TFA), TEA (238 mg, 2.36 mmol, 328 µL, 3.00 eq) and DCM (6.00 mL) were then added. The temperature was raised to 25°C and the reaction was allowed to proceed for 2 h. LCMS indicated complete consumption of compound A115-3, with the target mass spectrum detected (RT = 0.385 min). The reaction solution was filtered, the mother liquor was concentrated, the crude was purified by preparative HPLC, and the eluate was lyophilized to obtain compound A115 (75.1 mg, 92.0 µmol, yield 11.7%, purity 97.6%) as an off-white solid. LCMS: m/z = 797.3 (M+H)⁺. **¹H** NMR: (400 MHz, DMSO-*d₆*) δ 11.8 (s, 1H), 11.1 (s, 1H), 9.13 (s, 1H), 8.50-8.38 (m, 2H), 8.25-8.21 (m, 1H), 8.17-8.05 (m, 3H), 7.86 (s, 1H), 7.66 (d, *J =* 8.6 Hz, 2H), 7.23-6.97 (m, 3H), 5.42-5.37 (m, *J* = 12.8 Hz, 1H), 3.65 (s, 3H), 3.47-3.41 (m, 2H), 3.29-3.21(m, 3H), 3.02 (d, *J =* 8.8 Hz, 3H), 2.94-2.85 (m, 2H), 2.73-2.60 (m, 5H), 2.07-1.99 (m, 1H).

### Preparation Example 200. Synthesis of Compound A116

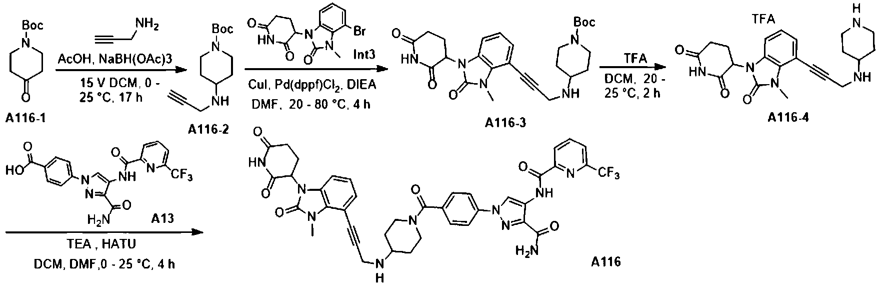

Compound A116-1 (2.00 g, 10.04 mmol, 1.00 eq), propargylamine (608 mg, 11.0 mmol, 707 µL, 1.10 eq), and DCM (30.0 mL) were added to a 100 mL three-necked round-bottom flask at 20°C-25°C. Glacial AcOH (1.21 g, 20.1 mmol, 1.15 mL, 2.00 eq) was then added. The mixture was stirred for 1 h and cooled to 0°C. NaBH(OAc)₃ (3.19 g, 15.0 mmol, 1.50 eq) was added. The temperature was then raised to 20°C-25°C and the reaction continued for 16 h. TLC (petroleum ether : ethyl acetate = 0 : 1) indicated complete consumption of compound A116-1 (R_{f} = 0.5), with a new major spot (R_{f} = 0.2) formed. Sodium carbonate was added to adjust the pH to 9, and water (30.0 mL) was added. The mixture was extracted with DCM (×3) (25.0 mL). The combined organic phases were washed with saturated aqueous NaCl (50.0 mL), dried over Na₂SO₄, filtered, and concentrated. The crude was separated by column chromatography (SiO₂, petroleum ether/ethyl acetate = 30/1 to 10/1) to obtain compound A116-2 (1.70 g, 7.13 mmol, yield 71.0%) as a yellow oil.

At 20°C-25°C, compound A116-2 (787 mg, 3.30 mmol, 3.00 eq), compound Int 3 (400 mg, 1.10 mmol, 1.00 eq) and DMF (8.00 mL) were added to a 100 mL three-necked round-bottom flask, followed by the addition of cuprous iodide (CuI) (41.9 mg, 220 µmol, 0.20 eq), DIEA (569 mg, 4.41 mmol, 767 µL, 4.00 eq) and Pd(dppf)Cl₂ (161 mg, 220 µmol, 0.20 eq). The mixture was purged with N₂ (×3), heated to 80°C and reacted for 4 h. LCMS indicated 25.1% of compound Int 3 remained (RT = 0.344 min), with the target mass spectrum (32.4%) detected (RT = 0.309 min). The reaction solution was filtered, and the filtrate was purified by preparative HPLC. The eluate was lyophilized to afford compound A116-3 (150 mg, 303 µmol, yield 24.9%, purity 100%) as a white solid.

Compound A116-3 (130 mg, 262 µmol, 1.00 eq) was added to a 10 mL single-necked flask, followed by the addition of DCM (3.00 mL) and TFA (897 mg, 7.87 mmol, 585 µL, 30.0 eq). The mixture was reacted at 20°C-25°C for 2 h. LCMS indicated complete consumption of compound A116-3, with the target mass spectrum (93.3%) detected (RT = 0.197 min). The reaction solution was concentrated to afford compound A116-4 (600 mg, 301 µmol, crude, TFA) as a yellow oil, which was used directly in the next step.

At 25°C and under N₂, compound A13 (86.9 mg, 200 µmol, 1.00 eq), TEA (60.9 mg, 602 µmol, 83.8 µL, 3.00 eq) and DCM (8.00 mL) were added to a 100 mL three-necked round-bottom flask. HATU (76.4 mg, 200 µmol, 1.00 eq) was added at 0°C-5°C. The mixture was stirred for 1 h, and then compound A116-4 (400 mg, 200 µmol, 1.00 eq, TFA), TEA (122 mg, 1.20 mmol, 167 µL, 6.00 eq) and DMF (12.0 mL) were added. The temperature was raised to 20°C-25°C and the mixture was reacted for 3 h. LCMS indicated complete consumption of compound A13, with the target mass spectrum (63.5%) detected (RT = 0.379 min). The reaction solution was concentrated and filtered. The crude was purified by preparative HPLC, and the eluate was lyophilized to obtain compound A116 (55.6 mg, 68.9 µmol, yield 34.3%, purity 98.7%) as an off-white solid. LCMS: m/z =797.4 (M+H)⁺. **¹H NMR:** (400 MHz, DMSO-*d₆*) δ 11.81 (s, 1H) 11.14 (s,1H) 9.42 (s, 1H) 9.11 (s, 1H) 8.49-8.34 (m, 2H) 8.23 (d, J = 7.6 Hz, 1 H) 8.13-8.03 (m, 3H) 7.85 (br s, 1H) 7.57 (d, J = 8.6 Hz, 2H) 7.28-7.15 (m, 2H) 7.12-7.02 (m, 1H) 5.42 (dd, J = 12.0 , 5.2 Hz, 1H) 4.63-4.44 (m, 1H) 4.35 (s, 2H) 3.66-3.47 (m, 6H) 3.31-3.09 (m, 1H) 3.05-2.82 (m, 2H) 2.80-2.58 (m, 2H) 2.26-2.06 (m, 2H) 2.06-1.98 (m, 1H) 1.67-1.42 (m, 2H).

### Preparation Example 201. Synthesis of Compound A117

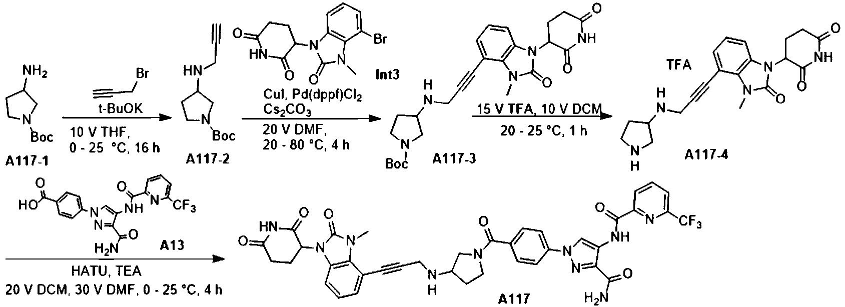

At 25°C under N₂, compound A117-1 (2.00 g, 10.7 mmol, 1.00 eq) was added to a 40.0 mL single-necked flask, followed by the addition of THF (20.0 mL). The temperature was lowered to 0°C, and potassium tert-butoxide (1.45 g, 12.9 mmol, 1.20 eq) and 3-bromopropyne (1.68 g, 11.3 mmol, 1.21 mL, 1.05 eq) were added. The temperature was raised to 15°C-25°C and the reaction was allowed to proceed for 16 h. TLC (petroleum ether : ethyl acetate = 0 : 1) indicated complete consumption of compound A117-1 (R_{f} = 0.1), yielding a major spot (R_{f} = 0.4). The mixture was poured into water (30.0 mL) and extracted with EtOAc (×3) (25.0 mL). The combined organic phases were washed with saturated aqueous NaCl (50.0 mL), dried over Na₂SO₄, filtered, and concentrated. The crude was separated by column chromatography (SiO₂, petroleum ether/ethyl acetate = 30/1 to 10/1) to obtain compound A117-2 (0.64 g, 2.85 mmol, yield 26.5%) as a yellow oil.

At 20°C-25°C, compound A117-2 (296 mg, 1.32 mmol, 1.00 eq), compound Int3 (520 mg, 1.32 mmol, 1.00 eq) and DMF (10.0 mL) were added to a 100 mL three-necked round-bottom flask, followed by the addition of cuprous iodide (50.3 mg, 264 µmol, 0.20 eq), cesium carbonate (1.72 g, 5.28 mmol, 4.00 eq) and Pd(dppf)Cl₂ (193 mg, 264 µmol, 0.20 eq). The mixture was purged with N₂ (×3), heated to 80°C and reacted for 4 h. LCMS indicated 9.54% of compound Int 3 remained (RT = 0.375 min), with the target mass spectrum (36.6%) detected (RT = 0.325 min). The reaction solution was filtered and concentrated to obtain a crude, which was purified using preparative thin layer chromatography (SiO₂, DCM : MeOH = 7 : 1, R_{f} = 0.5) to afford compound A117-3 (200 mg, 318 µmol, yield 24.1%, 76.6% purity) as a yellow solid.

Compound A117-3 (150 mg, 238 µmol, 1.00 eq) was added to a 10.0 mL single-necked flask at 20°C-25°C. DCM (1.50 mL) and TFA (408 mg, 3.58 mmol, 265 µL, 15.0 eq) were then added, and the mixture was stirred for 1 h. LCMS indicated complete consumption of compound A117-3, with the target mass spectrum (74.3%) detected. The reaction solution was concentrated to afford compound A117-4 (300 mg, crude, TFA) as a yellow oil.

At 25°C, compound A13 (101 mg, 234 µmol, 1.00 eq), TEA (71.2 mg, 704 µmol, 97.9 µL, 3.00 eq) and DCM (5.00 mL) were added to a 100 mL three-necked round-bottom flask, and the mixture was cooled to 0°C-5°C. HATU (89.2 mg, 234.6 µmol, 1.00 eq) was added, and the reaction was carried out for 1 h. Compound A117-4 (250 mg, 234 µmol, 1.00 eq, 6TFA), TEA (142 mg, 1.41 mmol, 196 µL, 6.00 eq) and DMF (7.50 mL) were added. The mixture was warmed to 20°C-25°C and reacted for 3 h. LCMS indicated complete consumption of compound A13, with the target mass spectrum (53.8%) detected (RT = 0.372 min). The reaction solution was concentrated and filtered, and the mother liquor was concentrated. The crude was purified by preparative HPLC, and the eluate was lyophilized to obtain compound A117 (21.1 mg, 26.5 µmol, yield 11.2%, purity 98.1%) as an off-white solid. LCMS: m/z =783.4 (M+H)⁺. **¹H NMR:** (400 MHz, DMSO-*d₆*) δ 11.80 (d, J = 2.8 Hz, 1H) 11.17-11.07 (m, 1H) 9.11-9.00 (m, 1H) 8.50-8.36 (m, 2H) 8.22 (d, J = 7.6 Hz, 1H) 8.13-7.95 (m, 3H) 7.82 (s, 1H) 7.74-7.61 (m, 2H) 7.18-7.00 (m, 2H) 6.96-6.85 (m, 1H) 5.44-5.31 (m, 1H) 3.75-3.66 (m, 3H) 3.64-3.55 (m, 5H) 3.51-3.47 (m, 2H) 3.00-2.81 (m, 1H) 2.76-2.57 (m, 3H) 2.12-1.93 (m, 2H) 1.85 (d, J = 4.8 Hz, 1H).

### Preparation Example 202. Synthesis of Compound A118

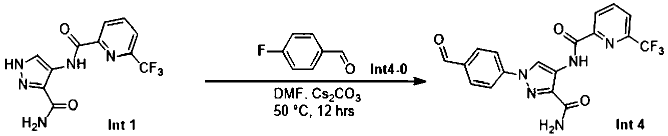

Compound Int 1 (10.0 g, 28.2 mmol, 1.00 eq), Int4-0 (3.51 g, 28.2 mmol, 2.98 mL, 1.00 eq), and dimethylformamide (DMF) (100 mL) were added to a 250 mL three-necked flask at 20°C-25°C. Cesium carbonate (18.4 g, 56.4 mmol, 2.00 eq) was then added, and the mixture was reacted at 50°C for 12 h. LCMS indicated complete consumption of compound Int 1, with the target product Int 4 detected by mass spectrometry. The reaction solution was poured into water and filtered, and the filter cake was directly concentrated to obtain compound Int 4 (10.0 g, 23.9 mmol, yield 84.9%, 96.7% purity) as a yellow solid. LCMS: m/z = 403.9 (M+H)⁺. **¹H NMR:** (400 MHz, DMSO) δ 11.80 (s, 1H), 10.05 (s, 1H), 9.19 (s, 1H), 8.50-8.44 (m, 1H), 8.43-8.34 (m, 1H), 8.27 (d, J = 8.8 Hz, 2H), 8.22 (d, J = 7.6 Hz, 1H), 8.16 (s, 1H), 8.08 (d, J *=* 8.8 Hz, 2H), 7.87 (s, 1H).

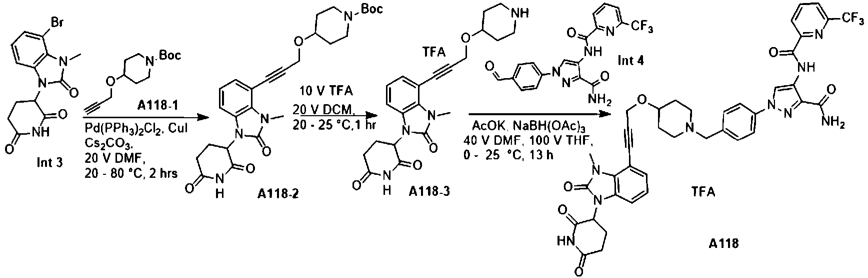

Compound A118-1 (1.06 g, 4.44 mmol, 1.50 eq), compound Int 3 (1.00 g, 2.96 mmol, 1.00 eq), and DMF (20.0 mL) were added to a 100 mL three-necked round-bottom flask. Cuprous iodide (CuI) (112 mg, 591 µmol, 0.20 eq), Pd(PPh₃)₂Cl₂ (415 mg, 591 µmol, 0.20 eq), and Cs₂CO₃ (3.85 g, 11.8 mmol, 4.00 eq) were then added at 20°C-25°C. The mixture was purged with N₂ (×3), heated to 80°C and reacted for 2 h. LCMS indicated that 25.1% of compound Int 3 was completely reacted, with the target mass spectrum detected (RT = 0.473 min). The reaction solution was poured into water (35.0 mL) and extracted with EtOAc (×3) (35.0 mL). The combined organic phases were washed with saturated aqueous NaCl (40.0 mL), dried over Na₂SO₄, filtered, and concentrated. The crude was purified by preparative HPLC, and the eluate was concentrated to yield compound A118-2 (790 mg, 1.53 mmol, yield 51.8%, 96.4% purity) as a yellow solid.

Compound A118-2 (780 mg, 1.51 mmol, 1.00 eq) and DCM (1.40 mL) were added to a 40.0 mL single-necked flask at 20°C-25°C. TFA (11.5 g, 101 mmol, 7.80 mL) was then added, and the mixture was stirred for 1 h. LCMS indicated complete consumption of compound A1 18-2, with the target mass spectrum detected (RT = 0.275 min). The reaction solution was concentrated to afford compound A118-3 (799 mg, 1.46 mmol, yield 96.4%, 93.3% purity, TFA) as a yellow oil.

Compound A118-3 (270 mg, 635 µmol, 1.00 eq, TFA), compound Int 4 (265 mg, 635 µmol, 1.00 eq), potassium acetate (124 mg, 1.27 mmol, 2.00 eq), DMF (11.0 mL), and THF (54.0 mL) were added to a 250 mL three-necked round-bottom flask at 20°C-25°C. The mixture was reacted for 1 h, and then cooled to 0°C-5°C. NaBH(OAc)₃ (269 mg, 1.27 mmol, 2.00 eq) was added. The mixture was purged with N₂ (×3), heated to 25°C and reacted for 12 h. LCMS indicated complete consumption of compound A118-3, with the target mass spectrum detected (RT = 0.399 min). The mixture was poured into water (100 mL) and extracted with EtOAc (×3) (100 mL). The combined organic phases were washed with saturated aqueous NaCl (100 mL), dried over Na₂SO₄, filtered, and concentrated. The crude was purified by preparative HPLC, and the eluate was lyophilized to obtain compound A118 (115 mg, 142 µmol, yield 22.3%, purity 96.3%, TFA) as an off-white solid. LCMS: m/z = 784.2 (M+H)⁺. **¹H NMR:** (400 MHz, DMSO-*d₆*) *δ* 11.80 (s, 1H), 11.13 (s, 1H), 9.11 (s, 1H), 8.50-8.37 (m, 2H), 8.23 (d, J = 7.6 Hz, 1H), 8.18-8.10 (m, 2H), 8.05 (s, 1H), 7.85 (s, 1H), 7.68 (t, J = 9.2 Hz, 2H), 7.19 (d, J = 7.2 Hz, 1H), 7.16-7.09 (m, 1H), 7.08-7.01 (m, 1H), 5.40 (dd, J = 5.6, 12.0 Hz, 1H), 4.53 (d, J = 6.4 Hz, 2H), 4.46-4.31 (m, 2H), 3.64 (d, J = 4.8 Hz, 3H), 3.50-3.48 (m, 1H), 3.30-3.23 (m, 2H), 3.17-3.04 (m, 2H), 2.96-2.83 (m, 1H), 2.77-2.62 (m, 2H), 2.29-2.20 (m, 1H), 2.13-2.00 (m, 2H), 1.96-1.83 (m, 1H), 1.69-1.56 (m, 1H).

### Preparation Example 203. Synthesis of Compound A119

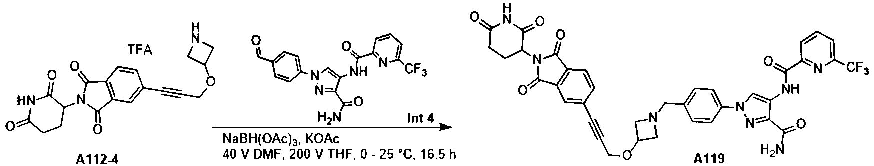

Compound A112-4 (380 mg, 361 µmol, 1.00 eq, 6.00 TFA), compound Int 4 (151 mg, 361 µmol, 1.00 eq), potassium acetate (70.9 mg, 722 µmol, 2.00 eq), DMF (15.2 mL) and THF (76.0 mL) were added to a 250 mL three-necked round-bottom flask and reacted for 0.5 h. The temperature was lowered to 0°C, and NaBH(OAc)₃ (153 mg, 723 µmol, 2.00 eq) was added. The temperature was raised to 20°C-25°C and the reaction continued for 16 h. LCMS indicated 18.3% of compound Int 4 remained (RT = 0.480 min), with the target mass spectrum (45.8%) detected (RT = 0.394 min). The reaction solution was filtered, and the crude was purified by preparative HPLC. The eluate was lyophilized to obtain compound A119 (81.2 mg, 103 µmol, yield 27.0%, purity 95.4%) as a white solid. LCMS: m/z =755.2 (M+H)⁺. **¹H NMR:** (400 MHz, DMSO-d6) δ 111.80 (s, 1H) 11.14 (s, 1H) 9.13 (s, 1H) 8.48-8.38 (m, 2H) 8.23 (dd, J = 7.6, 1.2 Hz, 1H) 8.18-8.04 (m, 2H) 7.98-7.89 (m, 4H) 7.85 (s, 1H) 7.66 (d, J = 8.6 Hz, 2H) 5.17 (dd, J = 12.0, 5.4 Hz, 1H) 4.58-4.37 (m, 7H) 4.22-4.12 (m, 2H) 2.95-2.83 (m, 1H) 2.68-2.53 (m, 2H) 2.11-2.02 (m, 1H).

### Preparation Example 204. Synthesis of Compound A120

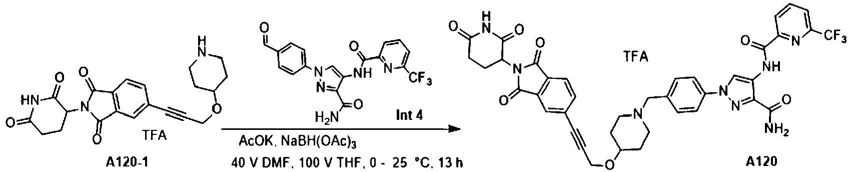

At 20°C-25°C, compound A120-1 (270 mg, 530 µmol, 1.00 eq, TFA), compound Int 4 (221 mg, 530 µmol, 1.00 eq), THF (54.0 mL) and DCM (2.00 mL) were added to a 100 mL three-necked round-bottom flask, and potassium acetate (104 mg, 1.06 mmol, 2.00 eq) was added. The reaction was carried out for 1 h, the temperature was lowered to 0°C-5°C, and NaBH(OAc)₃ (224 mg, 1.06 mmol, 2.00 eq) was added. The mixture was heated to 25°C and reacted for 12 h. LCMS indicated complete consumption of compound A120-1, with the target mass spectrum detected (RT = 0.408 min). The reaction solution was concentrated, and the crude was purified by preparative HPLC to afford compound A120 (83.4 mg, 103 µmol, yield 81.6%, purity 97.1%, TFA) as an off-white solid. LCMS: m/z = 783.2 (M+H)⁺. **¹H NMR:** (400 MHz, DMSO-*d₆*) *δ* 11.80 (s, 1H), 11.14 (s, 1H), 9.11 (s, 1H), 8.50-8.37 (m, 2H), 8.23 (d, J = 7.6 Hz, 1H), 8.18-8.09 (m, 2H), 8.03 (s, 1H), 8.00-7.89 (m, 3H), 7.85 (s, 1H), 7.74-7.63 (m, 2H), 5.23-5.11 (m, 1H), 4.54 (d, J = 7.6 Hz, 2H), 4.40 (dd, J = 3.2, 19.6 Hz, 2H), 4.05-3.95 (m, 1H), 3.83-3.75 (m, 1H), 3.45-3.42 (m, 1H), 3.30-3.23 (m, 1H), 3.19-3.02 (m, 2H), 2.95-2.83 (m, 1H), 2.73-2.63 (m, 1H), 2.32-2.22 (m, 1H), 2.15-2.01 (m, 2H), 1.97-1.82 (m, 1H), 1.72-1.56 (m, 1H).

### Preparation Example 205. Synthesis of Compound A121

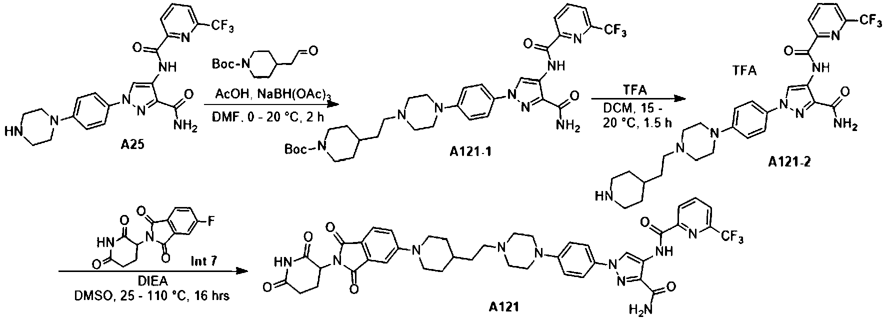

Under N₂ at 20°C, compound A25 (800 mg, 1.68 mmol, 1.00 eq), tert-butyl 4-(2-oxoethyl)piperidine-1-carboxylate (459 mg, 2.02 mmol, 1.20 eq), and glacial AcOH (202 mg, 3.37 mmol, 0.19 mL, 2.00 eq) were added to a 100 mL three-necked reaction flask containing DMF (16.0 mL). The mixture was stirred at 20°C for 1 h. The temperature was lowered to 0°C, and NaBH(OAc)₃ (714 mg, 3.37 mmol, 2.00 eq) was added. The temperature was raised to 20°C, and the reaction continued for 1 h. LCMS indicated complete consumption of compound A25, with the target mass spectrum (97.3%) detected. The reaction solution was poured into saturated Na₂CO₃ (320 mL) and filtered, and the filter cake was washed with water (8.00 mL). The filter cake was collected to yield compound A121-1 (1.27 g, crude) as a brown solid, which was used in the next reaction.

Compound A121-1 (1.27 g, 1.85 mmol, 1.00 eq) and DCM (13.0 mL) were added to a 100 mL three-necked reaction flask at 15°C-20°C under N₂. TFA (19.0 g, 167 mmol, 12.4 mL, 90.3 eq) was slowly added dropwise to the reaction flask under N₂ at 15°C-20°C. The reaction solution was stirred at 15°C-20°C for 1.5 h. LCMS indicated complete consumption of compound A121-1, with the target mass spectrum (95.2%) detected. The mixture was concentrated in vacuo to afford compound A121-2 (2.40 g, crude, 6TFA) as a dark brown solid, which was used in the next reaction.

At 25°C under N₂, compound A121-2 (400 mg, 0.70 mmol, 1.00 eq), compound Int 7 (232 mg, 0.84 mmol), and DMSO (8.00 mL) were added to a 100 mL three-necked reaction flask. DIEA (272 mg, 2.10 mmol, 0.37 mL, 3.00 eq) was then slowly added dropwise to the reaction flask under N₂ at 25°C. The reaction solution was stirred at 110°C for 16 h. LCMS indicated complete consumption of compound A121-2, with the target mass spectrum (22.9%) detected. The mixture was concentrated in vacuo to afford compound A121 (105 mg, 0.13 mmol, yield 18.1%, 99.2% purity) as a yellow solid. LCMS: m/z = 827.4 (M+H)⁺. **¹H NMR:** (400 MHz, DMSO-*d₆*) δ 11.79 (s, 1H), 11.08 (s, 1H), 8.87 (s, 1H), 8.47-8.36 (m, 2H), 8.21 (d, J = 7.6 Hz, 1H)), 7.95 (s, 1H), 7.78 (d, 2H), 7.71 (s, 1H), 7.64 (d, J = 8.8 Hz, 1H), 7.31 (s, 1H), 7.24 (dd, J = 8.4 Hz, J = 2.0 Hz, 1H), 7.07 (d, J = 9.2 Hz, 2H), 5.08 (dd, J = 12.0, 5.2 Hz, 1H), 4.04 (d, J = 12.0 Hz, 2H), 3.24-3.15 (s, 4H), 2.96 (t, J = 12.0 Hz, 2H), 2.90-2.82 (m, 1H), 2.74-2.51 (m, 6H), 2.38 (t, J = 7.2 Hz, 2H), 2.06-1.96 (m, 1H), 1.78 (d, J = 7.6 Hz, 2H), 1.69-1.55 (m, 1H), 1.49-1.36 (m, 2H), 1.28-1.14 (m, 2H).

### Preparation Example 206. Synthesis of Compound A122

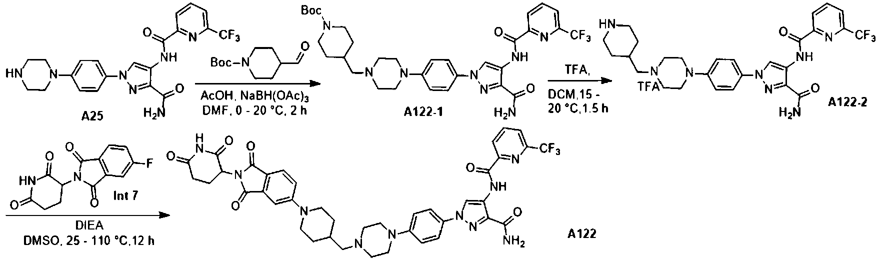

At 20°C under N₂, compound A25 (410 mg, 0.86 mmol, 1.00 eq), 1-tert-butoxycarbonylpiperidine-4-carboxaldehyde (221 mg, 1.04 mmol, 1.20 eq), and AcOH (104 mg, 1.73 mmol, 0.09 mL, 2.00 eq) were added to a 100 mL three-necked reaction flask containing DMF (9.00 mL). The reaction solution was stirred at 20°C for 1 h. Under N₂, the temperature was lowered to 0°C, and NaBH(OAc)₃ (366 mg, 1.73 mmol, 2.00 eq) was slowly added to the reaction flask. The temperature was raised to 20°C, and the reaction continued for 1 h. LCMS indicated complete consumption of compound A25, with the target mass spectrum (73.2%) detected. The reaction solution was poured into Na₂CO₃ (180 mL) and filtered. The filter cake was washed with water (45.0 mL) and collected. The crude was purified by preparative HPLC and lyophilized to yield compound A122-1 (199 mg, 0.28 mmol, yield 32.6%, 92.7% purity) as a dark brown solid.

Compound A122-1 (199 mg, 0.28 mmol, 1.00 eq) and DCM (2.00 mL) were added to a 100 mL three-necked reaction flask at 15°C-20°C under N₂. TFA (289 mg, 25.3 mmol, 1.99 mL, 90.3 eq) was slowly added dropwise to the reaction flask under N₂ at 15°C-20°C. The reaction was allowed to proceed at 20°C for 1.5 h. LCMS indicated complete consumption of compound A122-1, with the target mass spectrum (96.5%) detected. The mixture was concentrated in vacuo to afford compound A122-2 (146 mg, crude, TFA) as a brown solid, which was used in the next reaction.

At 25°C under N₂, compound A122-2 (146 mg, 0.26 mmol, 1.00 eq), compound Int 7 (87.0 mg, 0.32 mmol, 1.20 eq), and DMSO (3.00 mL) were added to a 100 mL three-necked reaction flask. DIEA (102 mg, 0.79 mmol, 0.14 mL, 3.00 eq) was then slowly added dropwise to the reaction flask under N₂ at 25°C. The reaction solution was stirred at 110°C for 12 h. LCMS indicated complete consumption of compound A122-2, with the target mass spectrum (50.8%) detected. The mixture was concentrated in vacuo to afford the crude, which was purified by preparative HPLC and lyophilized to afford compound A122 (64.7 mg, 0.08 mmol, yield 29.4%, purity 97.0%) as a yellow solid. LCMS: m/z = 813.2 (M+H)⁺. **¹H NMR:** (400 MHz, DMSO-*d₆*) *δ* 11.79 (s, 1H), 11.07 (s, 1H), 8.88 (s, 1H), 8.48-8.37 (m, 2H), 8.21 (d, J = 7.6 Hz, 1H), 7.96 (s, 1H), 7.79 (d, J = 8.8 Hz, 2H), 7.71 (s, 1H), 7.65 (d, J = 8.6 Hz, 1H), 7.32 (s, 1H), 7.24 (d, J = 8.6 Hz, 1H), 7.08 (d, J = 9.2 Hz, 2H), 5.07 (dd, J = 12.0, 5.4 Hz, 1H), 4.06 (d, J = 12.0 Hz, 2H), 3.22 (s, 4H), 2.99 (t, J = 12.0 Hz, 2H), 2.90-2.83 (m, 1H), 2.69-2.52 (m, 6H), 2.26-2.17 (m, 2H), 2.06-1.96 (m, 1H), 1.88-1.75 (m, 3H), 1.25-1.12 (m, 2H).

### Preparation Example 207. Synthesis of Compound A123

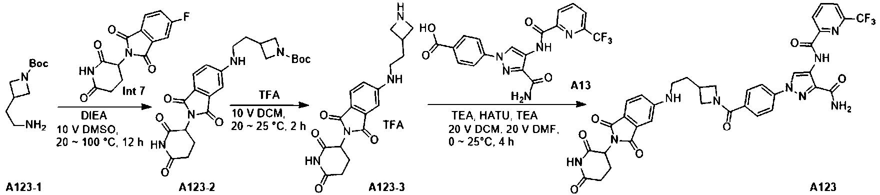

At 25°C under N₂, compound A123-1 (800 mg, 3.99 mmol, 1.00 eq), compound Int 7 (1.10 g, 3.99 mmol, 1.00 eq), and DMF (8.00 mL) were added to a 100 mL three-necked reaction flask. DIEA (1.55 g, 11.9 mmol, 2.09 mL, 3.00 eq) was then slowly added dropwise to the reaction flask under N₂ at 25°C. The reaction solution was stirred at 110°C for 12 h. TLC (petroleum ether : ethyl acetate = 1 : 1) indicated complete consumption of compound Int 7 (R_{f} = 0.500), with the formation of a new spot (R_{f} = 0.300). TLC indicated that the reaction was complete. The reaction solution was cooled to 25°C, poured into 50 mL of H₂O, and extracted with 100 mL of ethyl acetate (×3). The combined organic phases were dried over anhydrous Na₂SO₄, filtered, and concentrated to yield compound A123-2 (64.7 mg, 0.08 mmol, yield 29.4%, 97.0% purity) as a green solid.

Compound A123-2 (250 mg, 537 µmol, 1.00 eq) and DCM (5.00 mL) were added to a 40 mL single-necked reaction flask at 20°C-25°C. TFA (1.84 g, 16.1 mmol, 1.20 mL, 30.0 eq) was slowly added dropwise to the reaction flask at 20°C-25°C. The reaction was allowed to proceed at 20°C-25°C for 2 h. LCMS indicated complete consumption of compound A123-2, with the target mass spectrum detected. The mixture was concentrated in vacuo at 35°C-45°C to afford compound A123-3 (253 mg, crude, TFA) as a green oil, which was used in the next reaction.

At 25°C under N₂, compound A13 (229 mg, 531 µmol, 1.00 eq), TEA (161 mg, 1.59 mmol, 221 µL, 3.00 eq), and DCM (5.00 mL) were added to a 40 mL single-necked reaction flask. HATU (202 mg, 531 µmol, 1.00 eq) was added to the reaction flask under N₂ at 0°C-5°C and reacted at 0°C-5°C for 1 h. At 25°C, A123-3 (250 mg, 531 µmol, 1.00 eq, TFA), TEA (161 mg, 1.59 mmol, 221 µL, 3.00 eq) and DMF (5.00 mL) were added to a 10 mL single-necked flask. The mixture was cooled to 0°C-5°C under N₂ and added to a 40 mL reaction flask. The reaction solution was stirred at 20°C-25°C for 3 h. LCMS indicated complete consumption of compound A123-3, with the target mass spectrum detected. The reaction solution was filtered and concentrated in vacuo at 40°C-45°C to afford a crude, which was purified by preparative HPLC and lyophilized to afford compound A123 (81.8 mg, 105 µmol, yield 19.8%, purity 97.5%) as a yellow solid. LCMS: m/z = 758.3 (M+H)⁺ **¹H NMR:** (400 MHz, DMSO-*d*₆) *δ* 11.80 (s, 1H), 11.13 (s, 1H), 9.11 (s, 1H), 8.43 (m, 2H), 8.22 (d, J = 7.8 Hz, 1H), 8.08 (d, J = 8.6 Hz, 3H), 7.87-7.78 (m, 3H), 7.57 (d, J = 8.4 Hz, 1H), 7.13 (t, J = 5.4 Hz, 1H), 6.97 (s, 1H), 6.87 (d, J = 7.8 Hz, 1H), 5.03 (dd, J = 5.8, 12.6 Hz, 1H), 4.50-4.40 (m, 1H), 4.17 (t, J = 8.8 Hz, 1H), 4.12-4.04 (m, 1H), 4.13-4.03 (m, 1H), 3.77 (dd, J = 4.8, 8.6 Hz, 1H), 3.24-3.12 (m, 2H), 2.94-2.69 (m, 2H), 2.62-2.54 (m, 2H), 1.93 (m, 3H).

### Preparation Example 208. Synthesis of Compound A124

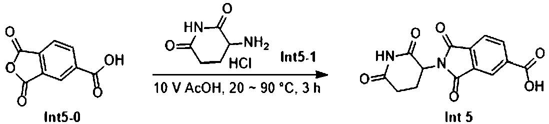

AcOH (5.00 mL), compound Int5-1 (471 mg, 2.86 mmol, 1.10 eq), and compound Int5-0 (500 mg, 2.60 mmol, 1.00 eq) were added to a 10 mL single-necked flask. Sodium acetate (320 mg, 3.90 mmol, 1.50 eq) was then added and the mixture was heated to 90°C and reacted for 3 h. LCMS indicated complete consumption of starting materials, with the main peak of the product detected. The mixture was slowly poured into water (200 mL), and stirred for 10 min. The reaction solution was filtered, and the mother liquor was concentrated to obtain crude compound Int 5 (560 mg, 1.85 mmol, yield 71.2%, 100% purity) as an off-white solid. LCMS: m/z = 302.8 (M+H)⁺. **¹H NMR:** (400 MHz, DMSO) δ 13.92-13.62 (m, 1H), 11.15 (s, 1H), 8.40 (dd, J = 1.4, 7.6 Hz, 1H), 8.28 (s, 1H), 8.05 (d, J = 7.8 Hz, 1H), 5.20 (dd, J = 5.4, 12.8 Hz, 1H), 2.96-2.84 (m, 1H), 2.69-2.53 (m, 2H), 2.13-2.02 (m, 1H).

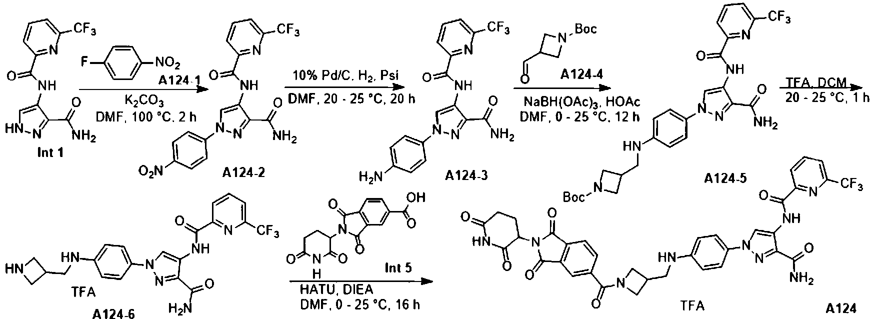

At 20°C-25°C, compound Intl (1.00 g, 3.21 mmol, 1.00 eq) and DMF (20.0 mL) were added to a 100 mL three-necked reaction flask. A124-1 and potassium carbonate (443 mg, 3.21 mmol, 1.00 eq) were also added to the flask at 20°C-25°C. The reaction was allowed to proceed at 100°C for 2 h. LCMS indicated complete consumption of compound Intl, with the target mass spectrum detected. The crude was concentrated in vacuo at 45°C-50°C, poured into 30 mL of water, and filtered to collect the filter cake, yielding compound A124-2 (850 mg, 1.96 mmol, yield 61.0%, 97.9% purity) as a yellow solid, which was used in the next step.

Under an argon atmosphere, Pd/C (208 mg, 196 µmol, 10.0%, 0.100 eq) was added to a 75 mL hydrogenation vessel. DMF (20.0 mL) and A124-2 (850 mg, 1.96 mmol, 1.00 eq) were added to the hydrogenation vessel. The mixture was purged with H₂ (×3), the pressure was adjusted to 15 psi, and the reaction was carried out at 25°C for 20 h. LCMS indicated complete consumption of compound A124-2, with the target mass spectrum detected. After the reaction, the mixture was purged with argon (×3) at 20°C-25°C. The mixture was filtered through Celite, and the filter cake was washed with DMF (50.0 mL). The filtrate was concentrated in vacuo at 40°C-45°C to obtain compound A124-3 (650 mg, 1.38 mmol, yield 70.4%, 83.2% purity) as a yellow solid, which was used directly in the next reaction.

At 20°C-25°C, compound A124-3 (0.65 g, 1.39 mmol, 1.00 eq), compound A124-4 (256 mg, 1.39 mmol, 1.00 eq) and DMF (6.50 mL) were added to a 100 mL three-necked reaction flask. At 20°C-25°C, HOAc (83.2 mg, 1.39 mmol, 79.3 µL, 1.00 eq) was added to the reaction flask and reacted at 20°C-25°C for 0.5 h. After cooling to 0°C-5°C, NaBH(OAc)₃ (880 mg, 4.16 mmol, 3.00 eq) was added. The reaction was allowed to proceed at 20°C-25°C for 15.5 h. LCMS indicated 35.4% of compound A124-3 remained, with the target mass spectrum detected. The reaction solution was poured into 500 mL of water and extracted with EtOAc (×3) (500 mL). The combined organic phases were washed with saturated aqueous NaCl (500 mL), dried over anhydrous Na₂SO₄, filtered, and concentrated to yield the crude. The crude was purified by preparative HPLC (TFA conditions) to afford compound A124-5 (400 mg, crude) as a yellow solid.

Compound A124-5 (400 mg, 714 µmol, 1.00 eq) and DCM (1.00 mL) were added to a 10 mL single-necked reaction flask at 20°C-25°C. TFA (6.14 g, 53.8 mmol, 4.00 mL, 75.3 eq) was slowly added dropwise to the reaction flask at 20°C-25°C and reacted at 20°C-25°C for 1 h. LCMS indicated 35.4% of compound A124-5 remained, with the target mass spectrum detected. The reaction mixture was concentrated in vacuo at 40°C-45°C to afford compound A124-6 (325 mg, crude, TFA) as a yellow solid, which was used directly in the next reaction.

Compound Int 5 (153 mg, 507 µmol, 1.00 eq) and DMF (5.00 mL) were added to a 10 mL single-necked reaction flask at 25°C. The reaction was cooled to 0°C-5°C, and HATU (231 mg, 608 µmol, 1.20 eq) and DIEA (393 mg, 3.04 mmol, 530 µL, 6.00 eq) were added to the reaction flask. The mixture was reacted at 0°C-5°C for 0.5 h. A124-6 (300 mg, 507 µmol, 1.00 eq, TFA) was added to the reaction flask. The mixture was reacted at 20°C-25°C for 15.5 h. LCMS indicated compound A124-6 remained, with the target mass spectrum detected. The mixture was filtered, and the filtrate was collected and concentrated in vacuo at 40°C-45°C to afford a crude, which was purified by preparative HPLC and lyophilized to afford compound A124 (118 mg, 136 µmol, yield 26.8%, purity 99.1%, TFA) as a yellow solid. LCMS: m/z = 744.3 (M+H)⁺. **¹H NMR:** (400 MHz, DMSO-*d₆*) δ 11.78 (s, 1H), 11.15 (s, 1H), 8.79 (s, 1H), 8.47-8.36 (m, 2H), 8.21 (dd, *J* = 7.2, 0.8 Hz, 1H), 8.11 (dd, *J* = 7.6, 1.2 Hz, 1H), 8.07 (s, 1H), 8.01 (d, *J* = 7.6 Hz, 1H,) 7.89 (br s, 1H), 7.70-7.62 (m, 3H), 6.72 (d, *J =* 8.8 Hz, 2H), 5.19 (dd, *J* = 12.8, 5.4 Hz, 1H), 4.41 (br t, *J* = 8.4 Hz, 1H), 4.20 (br t, *J* = 9.2 Hz, 1H), 4.10 (br dd, *J* = 8.0, 5.6 Hz, 1H), 3.84 (br s, 2H), 3.36 (br d, *J* = 8.0 Hz, 2H), 2.99-2.85 (m, 2H), 2.69-2.54 (m, 2H), 2.13-2.02 (m, 1H).

### Preparation Example 209. Synthesis of Compound A125

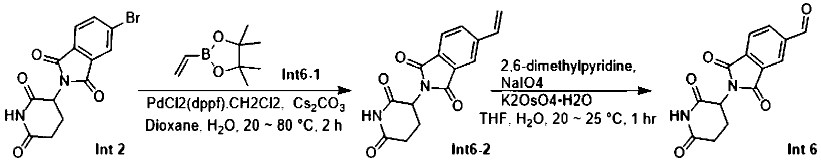

1,4-Dioxane (40.0 mL) and water (10.0 mL) were added to a 100 mL single-necked flask. Compound Int 2 (5.00 g, 14.8 mmol, 1.00 eq) and compound Int 6-1 (4.57 g, 29.6 mmol, 5.03 mL, 2.00 eq) were also added. PdCl₂(dppf)·CH₂Cl₂ (2.42 g, 2.97 mmol, 0.20 eq) and cesium carbonate (9.66 g, 29.6 mmol, 2.00 eq) were also added. The temperature was raised to 80°C and the mixture was reacted for 2 h. LCMS indicated complete consumption of compound Int 2, with a main peak detected. DCM (50.0 mL) was added, and the mixture was washed with water (×3) (100 mL) and concentrated under reduced pressure. The crude was purified by column chromatography (SiO2, DCM : MeOH = 10 : 1, Rf = 0.600) to afford the orange compound Int 6-2 (1.80 g, 5.78 mmol, yield 38.9%, 91.3% purity).

Compound Int 6-2 (1.08 g, 3.46 mmol, 1.00 eq), THF (20.0 mL), and water (4.00 mL) were added to a 100 mL three-necked flask. Sodium periodate (2.96 g, 13.8 mmol, 767 µL, 4.00 eq), potassium osmate monohydrate (127 mg, 346 µmol, 0.05 eq) and 2,6-dimethylpyridine (742 mg, 6.92 mmol, 806 µL, 2.00 eq) were then added and reacted at 20°C-25°C for 1 h. LCMS indicated complete consumption of starting materials, with the main peak of the product detected. The reaction solution was poured into water (100 mL) and extracted five times with MeOH/DCM (1/6, 200 mL). The solution was washed once with aqueous sodium sulfite solution (200 mL), washed with saturated aqueous NaCl (200 mL), dried over Na₂SO₄, and concentrated under reduced pressure. The crude was purified by thin layer chromatography to obtain compound Int 6 (630 mg, 2.20 mmol, yield 63.5%, purity 100%) as a white solid.

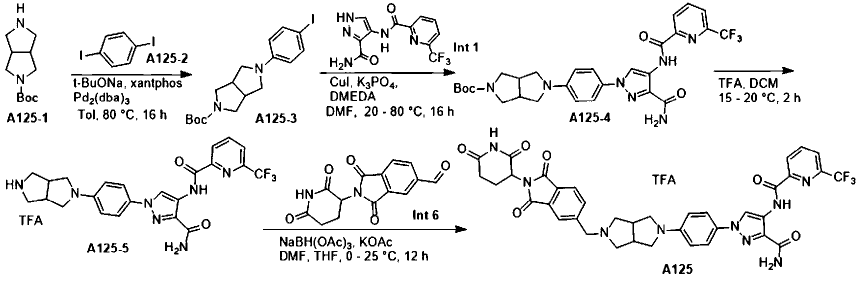

Compound A125-1 (1.00 g, 4.71 mmol, 1.00 eq) and toluene (30.0 mL) were added to a 100 mL reaction flask. t-BuONa (588 mg, 6.12 mmol, 1.30 eq), Xantphos (545 mg, 942 µmol, 0.200 eq) and compound A125-2 (15.5 g, 47.0 mmol, 10.0 eq) were added to the reaction flask. After the mixture was purged with N₂ (×3), Pd₂(dba)₃ (431 mg, 471 µmol, 0.100 eq) was added to the reaction flask. The reaction was carried out at 80°C for 16 h. LCMS indicated complete consumption of compound A125-1, with the target mass spectrum detected. The reaction solution was poured into 500 mL of water and extracted with EtOAc (×3) (500 mL). The combined organic phases were washed with saturated aqueous NaCl (500 mL), dried over anhydrous Na₂SO₄, filtered, and concentrated to yield the crude. The crude was purified by column chromatography (SiO₂, petroleum ether/ethyl acetate = 20/1 to 3/1, product: R*_{f}* = 0.45) to afford compound A125-3 (1.00 g, 2.37 mmol, yield 50.2%, 98.5% purity) as a red solid.

Under N₂, compound A125-3 (500 mg, 1.18 mmol, 1.00 eq) and DMF (5.00 mL) were added to a 100 mL single-necked reaction flask. Under N₂, compound Intl (441 mg, 1.42 mmol, 1.20 eq), CuI (225 mg, 1.18 mmol, 1.00 eq), DMEDA (208 mg, 2.37 mmol, 254 µL, 2.00 eq), and K₃PO₄ (753 mg, 3.55 mmol, 3.00 eq) were added to a reaction flask. The reaction was carried out at 80°C for 16 h. LCMS indicated complete consumption of compound A125-3, with the target mass spectrum detected. The mixture was filtered, and the filtrate was collected and purified by preparative HPLC (TFA conditions; column: Welch Xtimate C18 150×25 mm ×5 µm; mobile phase: [water (TFA)-ACN]; gradient: 33%-63% B over 3 min). The fraction was extracted with EtOAc (×3) (20 mL). The combined organic phases were washed with saturated aqueous NaCl (20 mL), dried over anhydrous Na₂SO₄, filtered, and concentrated to yield compound A125-4 (300 mg, 491 µmol, yield 41.5%, 96.0% purity) as a gray solid.

Compound A125-4 (300 mg, 493 µmol, 1.00 eq) and DCM (3.00 mL) were added to a 10 mL reaction flask. TFA (4.61 g, 40.3 mmol, 3.00 mL, 81.8 eq) was also added to the reaction flask. The mixture was reacted at 20°C-25°C for 2 h. LCMS indicated compound A125-4 remained, with the target mass spectrum detected. The mixture was concentrated in vacuo at 40°C-45°C to afford compound A125-5 (336 mg, crude, TFA) as a yellow solid.

Compound A125-5 (310 mg, 449 µmol, 1.00 eq, TFA), compound Int 6 (128 mg, 449 µmol, 1.00 eq), KOAc (88.2 mg, 898 µmol, 2.00 eq), DMF (13.0 mL), and THF (66.0 mL) were added to a 100 mL reaction flask at 20°C-25°C. The mixture was reacted at 20°C-25°C for 1 h. After cooling to 0°C-5°C, NaBH(OAc)₃ (285 mg, 1.35 mmol, 3.00 eq) was added. The reaction was allowed to proceed at 20°C-25°C for 11 h. LCMS indicated complete consumption of compound A125-5, with the target mass spectrum detected. The filtrate was filtered and collected, and the filtrate was concentrated in vacuo at 40°C-45°C. The crude was purified by preparative HPLC and lyophilized to obtain compound A125 (153 mg, 202 µmol, yield 27.3%, 100% purity) as a yellow solid. LCMS: m/z = 756.2 (M+H)⁺. **¹H NMR:** (400 MHz, DMSO-*d₆*) *δ* 11.79 (s, 1H), 11.16 (s, 1H), 10.29-10.02 (d, J = 13.2 Hz, 1H), 8.94-8.81 (d, J *=* 6.8 Hz, 1H), 8.48-8.35 (m, 2H), 8.21 (d, J = 7.6 Hz, 1H), 8.17-8.10 (m, 1H), 8.09-8.04 (m, 1H), 8.03-7.97 (m, 1H), 7.84-7.77 (m, 2H) 7.93 (m, 1H), 7.72 (s, 1H), 6.90-6.74 (m, 2H), 5.19 (dd, J *=* 12.8, 5.2 Hz, 1H), 4.72-4.52 (m, 2H), 3.66-3.32 (m, 6H), 3.19-3.01 (m, 4H), 2.97-2.82 (m, 1H), 2.68-2.55 (m, 2H), 2.18-2.00 (m, 1H).

### Preparation Example 210. Synthesis of Compound A126

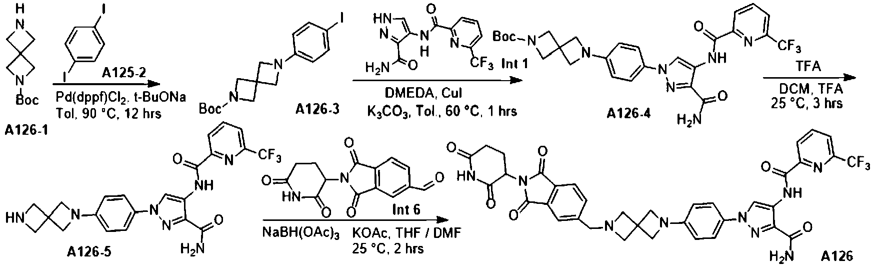

Compound Int 3-2 (23.0 g, 98.7 mmol, 1.00 eq) and THF (176 mL) were added to a 500 mL three-necked flask, and Boc₂O (129 g, 592 mmol, 136 mL, 4.00 eq), DMAP (18.1 g, 148 mmol, 1.50 eq) and DIPEA (38.3 g, 296 mmol, 51.6 mL, 3.00 eq) were added at 0°C. The mixture was heated to 50°C and reacted for 16 h. The main peak of the product was detected by LCMS. Water (600 mL) was added, and the mixture was extracted with EtOAc (×3) (1000 mL). The organic phases were combined, washed with saturated aqueous NaCl (400 mL), dried over Na₂SO₄, filtered, and concentrated to yield the crude, which was purified by column chromatography to afford A128-1-0 (15.0 g, 45.3 mmol, yield 45.8%, 100% purity) as a yellow oil.

Compound A128-1-0 (15.0 g, 45.3 mmol, 1.00 eq) and ethanol (37.5 mL) were added to a 500 mL three-necked flask, followed by the addition of THF (37.5 mL) and NH₄Cl (24.2 g, 453 mmol, 10.0 eq). At 20°C-25°C, Zn (29.2 g, 447 mmol, 9.87 eq) was added and reacted for 4 h. LCMS indicated complete consumption of compound A128-1-0, with the main peak of the product detected. The mixture was filtered, and the filter cake was washed with 1 L of ethanol. The filtrate was concentrated to obtain compound A128-1-1 (15.0 g, crude) as a white solid. Compound A128-1-1 (15.0 g, 49.6 mmol, 1.00 eq) and tert-butanol (240 mL) were added to a 1 L three-necked flask, and Cs₂CO₃ (48.5 g, 148 mmol, 3.00 eq), RuPhos (2.32 g, 4.97 mmol, 0.10 eq), and Pd₂(dba)₃ (2.27 g, 2.48 mmol, 0.05 eq) were sequentially added, followed by the addition of compound A128-1-2 (28.6 g, 59.6 mmol, 1.20 eq). The mixture was reacted at 100°C under N₂ for 16 h. LCMS indicated complete consumption of A128-1-1, with the main peak of the product detected. Ethyl acetate (500 mL) was added, and the mixture was filtered. The filter cake was washed with 100 mL of ethyl acetate. Water (200 mL) was added, and extraction was performed with EtOAc (×3) (300 mL). The organic phases were combined, washed with saturated aqueous NaCl, dried over Na₂SO₄, filtered, and concentrated to obtain the crude, which was purified by column chromatography to obtain Compound A128-1 as a yellow solid (9.40 g, 18.1 mmol, yield 36.5%, purity 99.6%).

Compound A125-2 (49.9 g, 151 mmol, 10.0 eq), compound A126-1 (3.00 g, 5.13 mmol, 1.00 eq), toluene (120 mL), t-BuONa (14.5 g, 151 mmol, 10.0 eq), and Pd(dppf)Cl₂ (2.21 g, 3.03 mmol, 0.20 eq) were added sequentially to a 250 mL reaction flask at 20°C-30°C. The mixture was purged with N₂ (×3), and reacted at 90°C for 12 h. LCMS indicated complete consumption of compound A126-1, with the target mass spectrum detected. The reaction solution was diluted in 900 mL of ethyl acetate and washed twice with 300 mL of aqueous NaCl. The organic phase was concentrated in vacuo to obtain a crude, which was purified by column chromatography (SiO₂, petroleum ether/ethyl acetate = 5/1, Rf = 0.5) to obtain compound A126-3 (1.90 g, 3.96 mmol, yield 26.2%, purity 83.4%) as a yellow solid.

At 20°C-25°C, compound A126-3 (700 mg, 1.75 mmol, 1.00 eq), DMF (7.00 mL), compound Int1 (627 mg, 2.10 mmol, 1.20 eq), DMEDA (308 mg, 3.50 mmol, 376 µL, 0.60 eq), K₂CO₃ (1.11 g, 5.25 mmol, 2.00 eq) and CuI (333 mg, 1.75 mmol, 0.30 eq) were sequentially added to a 50 mL reaction flask. The mixture was purged with N₂ (×3), and reacted at 60°C for 1 h. LCMS indicated complete consumption of compound A126-3, with the target mass spectrum detected. The reaction solution was filtered and extracted with 50 mL of ethyl acetate. The filter cake was washed (×3), then diluted in 20 mL of water and extracted with of ethyl acetate (×3) (100 mL). The organic phases were combined, washed once with 20 mL of aqueous NaCl, dried over anhydrous Na₂SO₄, and concentrated in vacuo at 35°C-40°C to yield compound A126-4 (1.90 g, crude) as a white solid, which was used directly in the next step.

Compound A126-4 (0.55 g, 962 µmol, 1.00 eq), DCM (2.00 V), and TFA (0.60 V) were added sequentially to a 50 mL reaction flask at 20°C-25°C and reacted at 25°C for 3 h. LCMS indicated complete consumption of compound A126-4, with the target mass spectrum detected. The reaction solution was concentrated in vacuo at 25°C and lyophilized to afford compound A126-5 (700 mg, 757 µmol, yield 78.6%, 88.0% purity, 3 TFA) as a white solid.

Compound A126-5 (120 mg, 246 µmol, 1.00 eq), DMF (6.00 mL), THF (24.0 mL), compound lnt 6 (77.7 mg, 271 µmol, 1.20 eq), potassium acetate (48.5 mg, 494 µmol, 2.00 eq), and NaBH(OAc)₃ (156 mg, 740 µmol, 3.00 eq) were added sequentially to a 50 mL reaction flask at 20°C-25°C and reacted at 25°C for 2 h. LCMS indicated complete consumption of compound A126-5, with the target mass spectrum detected. The mixture was diluted in 10 mL of water, and extracted with EtOAc (×3) (30 mL). The organic phases were combined, washed once with 20 mL of aqueous NaCl, dried over anhydrous Na₂SO₄, and concentrated in vacuo. The crude was purified by preparative HPLC to afford compound A126 (50.2 mg, 105 µmol, yield 42.4%, purity 97.0%) as an off-white solid. LCMS: m/z = 742.4 (M+H)⁺. **¹H** NMR: (400 MHz, DMSO-*d₆*) *δ* 11.8 (s, 1H), 11.1 (s, 1H), 8.82 (s, 1H), 8.48-8.36 (m, 2H), 8.22-8.19 (m, 1H), 7.97-7.85 (m, 2H), 7.84-7.76 (m, 2H), 7.76-7.65 (m, 3H), 6.54 (d, J = 8.8 Hz, 2H), 5.28-5.03 (q, 1H), 3.96 (s, 4H), 3.76 (s, 3H), 3.39 (s, 3H), 2.95-2.84 (m, 1H), 2.64-2.54 (m, 2H), 2.16-1.97 (m, 1H).

### Preparation Example 211. Synthesis of Compound A127

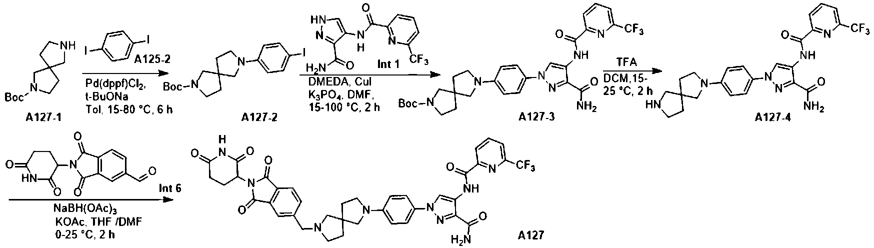

At 15°C-25°C, compound A127-1 (2.00 g, 8.84 mmol, 1.00 eq), compound A125-2 (29.2 g, 88.4 mmol, 10.0 eq), toluene (80.0 mL), t-BuONa (8.49 g, 88.4 mmol, 10.0 eq), and Pd(dppf)Cl₂ (1.29 g, 1.77 mmol, 0.20 eq) were added sequentially to a reaction flask. The mixture was purged with N₂ (×3), and reacted at 80°C for 6 h. LCMS indicated complete consumption of compound A127-1, with the target mass spectrum detected. The mixture was diluted in 200 mL of ethyl acetate, and the organic phase was filtered and washed once with 200 mL of aqueous NaCl. The organic layer was concentrated in vacuo to obtain a crude, which was purified by silica gel column chromatography (petroleum ether : ethyl acetate = 10 : 1 to 0 : 1) to obtain the product (petroleum ether : ethyl acetate = 5 : 1, R_{f} = 0.30). The organic phase was concentrated in vacuo to obtain compound A127-2 (2.00 g, 4.06 mmol, yield 45.9%, purity 86.9%) as a yellow solid.

At 15°C-25°C, compound A127-2 (2.30 g, 5.37 mmol, 1.00 eq), compound Int1 (1.93 g, 6.44 mmol, 1.20 eq), DMF (23.0 mL), K₃PO₄ (3.42 g, 16.1 mmol, 3.00 eq), DMEDA (947 mg, 10.7 mmol, 1.16 mL, 2.00 eq) and CuI (1.02 g, 5.37 mmol, 1.00 eq) were sequentially added to the reaction flask. The reaction was allowed to proceed at 100°C for 2 h. LCMS indicated complete consumption of compound A127-2, with the target mass spectrum detected. The mixture was diluted in 50 mL of ethyl acetate and filtered to obtain a filtrate. The filtrate was concentrated in vacuo, and the crude was purified by preparative HPLC to yield compound A127-3 (380 mg, 618 µmol, yield 11.5%, 97.6% purity) as a yellow solid.

Compound A127-3 (330 mg, 550 µmol, 1.00 eq), TFA (1.80 mL), and DCM (5.50 mL) were added sequentially to the reaction flask at 15°C-25°C and reacted at 25°C for 2 h. LCMS indicated complete consumption of compound A127-3, with the target mass spectrum detected. The reaction solution was concentrated in vacuo to yield compound A127-4 (370 mg, crude, TFA) as a brown solid.

At 15°C-25°C, compound A127-4 (200 mg, 400 µmol, 1.00 eq), compound Int 6 (115 mg, 400 µmol, 1.00 eq), DMF (10.0 mL), THF (40.0 mL) and KOAc (78.6 mg, 801 µmol, 2.00 eq) were sequentially added to a reaction flask. The reaction was allowed to proceed at 25°C for 1 h. NaBH(OAc)₃ (255 mg, 1.20 mmol, 3.00 eq) was added to the reaction flask and reacted at 25°C for 2 h. LCMS indicated complete consumption of compound A127-4, with the target mass spectrum detected. The reaction solution was diluted in 100 mL of ethyl acetate, and 100 mL of water was added. The mixture was extracted with EtOAc (×3) (100 mL). The organic phases were combined, washed once with 200 mL of saturated aqueous NaCl, dried over anhydrous Na₂SO₄, filtered and concentrated in vacuo. The crude was purified by preparative HPLC and lyophilized to obtain compound A127 (50.07 mg, 61.4 µmol, yield 10.2%, purity 94.4%) as a yellow solid. LCMS: m/z = 770.2 (M+H)⁺. **¹H NMR:** (400 MHz, DMSO-*d₆*) *δ* 11.78 (s, 1H), 11.12 (s, 1H), 8.80 (s, 1H), 8.32-8.52 (m, 2H), 8.12-8.26 (m, 1H), 7.82-7.96 (m, 4H), 7.64-7.76 (m, 3H), 6.62 (d, J = 8.8 Hz, 2H), 5.14 (dd, J = 5.2, 13.0 Hz, 1H), 3.80 (s, 2H), 3.24 (d, J = 9.2 Hz, 5H), 2.82-2.96 (m, 1H), 2.54-2.74 (m, 5H), 1.94-2.12 (m, 3H), 1.78-1.90 (m, 2H).

### Preparation Example 212. Synthesis of Compound A128

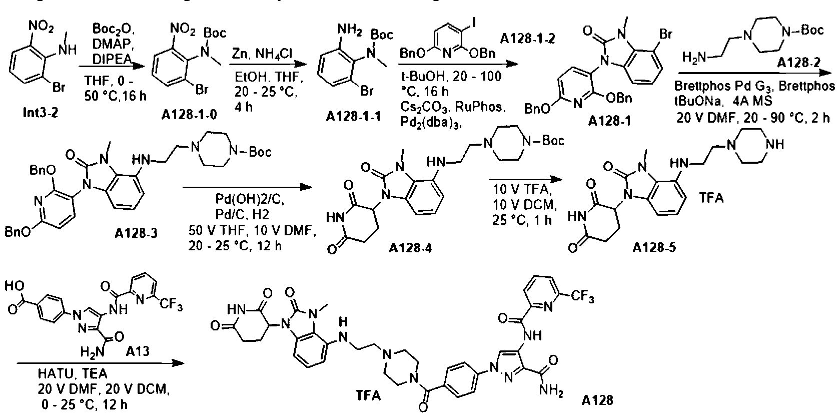

Compound Int 3-2 (23.0 g, 98.7 mmol, 1.00 eq) and THF (176 mL) were added to a 500 mL three-necked flask. Boc₂O (129 g, 592 mmol, 136 mL, 4.00 eq), DMAP (18.1 g, 148 mmol, 1.50 eq), and DIPEA (38.3 g, 296 mmol, 51.6 mL, 3.00 eq) were added at 0°C. The mixture was heated to 50°C and reacted for 16 h. The main peak of the product was detected by LCMS. Water (600 mL) was added, and the mixture was extracted with EtOAc (×3) (1000 mL). The organic phases were combined, washed with saturated aqueous NaCl (400 mL), dried over Na₂SO₄, filtered, and concentrated. The crude was purified by column chromatography to obtain compound A128-1-0 (15.0 g, 45.3 mmol, yield 45.8%, 100% purity) as a yellow oil.

Compound A128-1-0 (15.0 g, 45.3 mmol, 1.00 eq) and ethanol (37.5 mL) were added to a 500 mL three-necked flask, followed by the addition of THF (37.5 mL) and NH₄Cl (24.2 g, 453 mmol, 10.0 eq). Zn (29.2 g, 447 mmol, 9.87 eq) was added at 20°C-25°C, and the reaction was allowed to proceed for 4 h. LCMS indicated complete consumption of compound A128-1-0, with the main peak of the product detected. The mixture was filtered, the filter cake was washed with 1 L of ethanol, and the filtrate was concentrated to obtain compound A128-1-1 (15.0 g, crude) as a white solid. Compound A128-1-1 (15.0 g, 49.6 mmol, 1.00 eq) and tert-butanol (240 mL) were added to a 1 L three-necked flask, and then Cs₂CO₃ (48.5 g, 148 mmol, 3.00 eq), RuPhos (2.32 g, 4.97 mmol, 0.10 eq), and Pd₂(dba)₃ (2.27 g, 2.48 mmol, 0.05 eq) were sequentially added. Compound A128-1-2 (28.6 g, 59.6 mmol, 1.20 eq) was then added, and the mixture was reacted at 100°C under N₂ for 16 h. LCMS indicated complete consumption of A128-1-1, with the main peak of the product detected. Ethyl acetate (500 mL) was added, and the mixture was filtered. The filter cake was washed with 100 mL of ethyl acetate. Water (200 mL) was added, and the mixture was extracted with EtOAc (×3) (300 mL). The organic phases were combined, washed with saturated aqueous NaCl, dried over Na₂SO₄, filtered, and concentrated. The crude was purified by column chromatography to obtain compound A128-1 (9.40 g, 18.1 mmol, yield 36.5%, 99.6% purity) as a yellow solid.

Compound A128-1 (600 mg, 1.16 mmol, 1.00 eq), compound A128-2 (531 mg, 2.31 mmol, 2.00 eq), and DMF (30.0 mL) were added to a 50 mL round-bottom flask at 20°C-25°C. Sodium tert-butoxide (334 mg, 3.47 mmol, 3.00 eq), molecular sieves (300 mg), BrettPhos Pd G₃ (210 mg, 231 µmol, 0.20 eq), and BRETTPHOS (124 mg, 231 µmol, 0.20 eq) were then added. The atmosphere was purged with N₂ (×3), and the mixture was heated to 90°C and reacted for 2 h. LCMS indicated complete consumption of compound A128-1, with the target mass spectrum detected. The product was poured into water (100 mL) and extracted twice with EtOAc (50 mL). The combined organic phases were washed with saturated aqueous NaCl (100 mL), dried over Na₂SO₄, filtered, and concentrated. The crude was purified by preparative HPLC and lyophilized to obtain compound A128-3 (270 mg, 368 µmol, yield 31.8%, 90.7% purity) as a yellow solid.

At 20°C-25°C, Pd/C (135 mg, 127 µmol, 10.0%), Pd(OH)₂/C (135 mg, 192 µmol, 20%), DMF (2.70 mL), and THF (13.5 mL) were added to a 75 mL hydrogenation vessel. Compound A128-3 (270 mg, 368 µmol, 1.00 eq) was then added, the mixture was purged with H₂ (×3), and the reaction was allowed to proceed at rt for 2 h. LCMS indicated complete consumption of compound A128-3, with the target mass spectrum detected. The reaction solution was filtered, the filter cake was washed with THF, and the filtrate was concentrated to obtain compound A128-4 (230 mg, crude) as a yellow oil.

Compound A128-4 (230 mg, 473 µmol, 1.00 eq) and DCM (2.30 mL) were added to a 10 mL round-bottom flask at 25°C. TFA (2.30 mL) was added, and the reaction was allowed to proceed at rt for 1 h. LCMS indicated complete consumption of compound A128-4, with the target mass spectrum detected. The reaction solution was concentrated at 50°C to yield compound A128-5 (379 mg, crude, TFA) as a yellow oil.

Compound A13 (391 mg, 903 µmol, 1.00 eq) and DCM (8.00 mL) were added to a 50 mL round-bottom flask at 25°C. Compound A128-5 (349 mg, 903 µmol, 1.00 eq) was dissolved in DMF (7.00 mL) and added dropwise to the reaction solution. TEA (274 mg, 2.71 mmol, 377 µL, 3.00 eq) was added at 0°C. The mixture was purged with N₂ (×3), and HATU (378 mg, 993 µmol, 1.10 eq) was added. The mixture was heated to rt and reacted for 12 h. LCMS indicated complete consumption of compound A128-5, with the target mass spectrum detected. After concentration, the crude was purified by preparative HPLC and lyophilized to obtain compound A128 (42.9 mg, 53.7 µmol, yield 5.94%, purity 98.5%) as a purple solid. LCMS: m/z = 788.1 (M+H)⁺ **¹H** NMR: (400 MHz, DMSO-*d₆*) δ 11.8 (s, 1H), 11.1 (s, 1H), 9.13 (s, 1H), 8.47-8.39 (m, 2H), 8.24-8.22 (m, 1H), 8.14-8.07 (m, 3H), 7.85 (s, 1H), 7.66 (d, J = 8.4 Hz, 2H), 6.92 (t, J = 8.0 Hz, 1H), 6.60-6.51 (m, 2H), 5.33-5.29 (m, 1H), 3.64 (s, 3H), 3.52-3.51 (m, 3H), 3.45-3.37 (m, 9H), 2.92-2.84 (m, 1H), 2.70-2.59 (m, 3H), 1.99-1.94 (m, 1H).

### Preparation Example 213. Synthesis of Compound A129

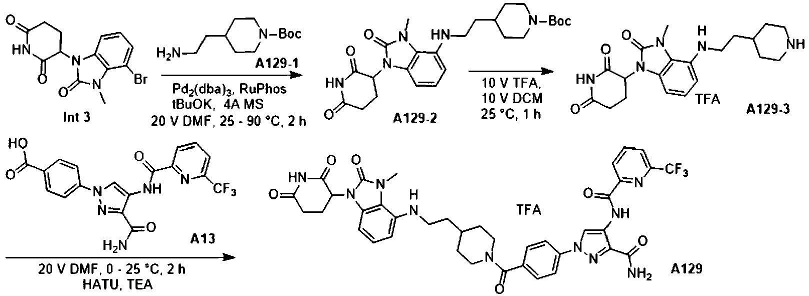

At 25°C, compound Int 3 (200 mg, 544 µmol, 1.00 eq), compound A129-1 (248 mg, 1.09 mmol, 2.00 eq), and DMF (4.0.0 mL) were added to a 50 mL round-bottom flask. Pd₂(dba)₃ (49.8 mg, 54.4 µmol, 0.10 eq), potassium tert-butoxide (104 mg, 1.09 mmol, 2.00 eq), and RuPhos (25.4 mg, 54.4 µmol, 0.10 eq) were then added. The atmosphere was purged with N₂ (×3), and the mixture was heated to 90°C and reacted for 2 h. LCMS indicated 30.4% of compound Int 3 remained, with the target mass spectrum detected. The mixture was poured into water (50.0 mL), adjusted to pH 3-4 with TFA, and concentrated. The crude was purified by preparative HPLC and concentrated in vacuo to afford compound A129-2 (51.0 mg, 101 µmol, yield 9.33%, 96.6% purity) as a brown oil.

Compound A129-2 (51.0 mg, 101 µmol, 1.00 eq) and DCM (1.00 mL) were added to a 10 mL round-bottom flask at 25°C. TFA (768 mg, 6.73 mmol, 0.50 mL, 66.3 eq) was then added and reacted at rt for 1 h. LCMS indicated complete consumption of compound A129-2, with the target mass spectrum detected. The reaction solution was concentrated at 50°C to afford compound A129-3 (50.0 mg, crude, TFA) as a yellow oil.

Compound A13 (56.2 mg, 129 µmol, 1.00 eq) and DCM (1.12 mL) were added to a 50 mL round-bottom flask at 25°C. The atmosphere was purged with N₂ (×3), and TEA (39.4 mg, 389 µmol, 54.2 µL, 3.00 eq) was added. HATU (49.3 mg, 129 µmol, 1.00 eq) was added at 0°C and reacted for 0.5 h. Compound A129-3 (50 mg, 129 µmol, 1.00 eq), TEA (39.4 mg, 389 µmol, 54.2 µL, 3.00 eq), and DMF (1.00 mL) were then added. The mixture was allowed to warm to rt and reacted for 1.5 h. LCMS indicated complete consumption of compound A129-3, with the target mass spectrum detected. The crude was obtained after concentration, purified by preparative HPLC, and lyophilized to afford compound A129 (44.1 mg, 54.8 µmol, yield 97.6%, purity 97.6%) as an off-white solid.

LCMS: m/z = 787.3 (M+H)⁺. **¹H NMR:** (400 MHz, DMSO-*d₆*) *δ* 11.80 (s, 1H), 11.1 (s, 1H), 9.09 (s, 1H), 8.45-8.40 (m, 2H), 8.22-8.08 (m, 4H), 7.81 (s, 1H), 7.56 (d, J = 5.2 Hz, 2H), 6.87 (s, 1H), 6.52-6.44 (m, 2H), 5.29 (s, 1H), 4.53-4.43 (m, 1H), 3.61 (s, 3H), 3.12 (s, 2H), 2.87-2.81 (m, 2H), 2.63 (s, 2H), 2.07-1.61 (m, 6H), 1.19 (d, J = 10.0 Hz, 2H), 1.05 (s, 3H).

### Preparation Example 214. Synthesis of Compound A130

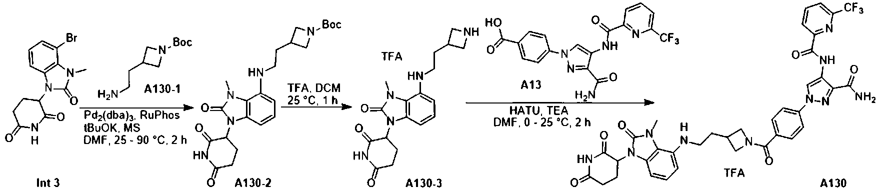

At 25°C, compound Int 3 (200 mg, 544 µmol, 1.00 eq), compound A130-1 (218 mg, 1.09 mmol, 2.00 eq), and DMF (4.00 mL) were added to a 10 mL flat-bottom flask. Pd₂(dba)₃ (99.5 mg, 109 µmol, 0.20 eq), RuPhos (50.7 mg, 109 µmol, 0.20 eq), molecular sieves (100 mg, 544 µmol, 1.00 eq), and potassium tert-butoxide (104 mg, 1.09 mmol, 2.00 eq) were then added. The atmosphere was purged with N₂ (×3), and the mixture was heated to 90°C and reacted for 2 h. LCMS indicated complete consumption of compound Int 3, with the target mass spectrum detected. The reaction solution was concentrated, and the crude was purified by preparative HPLC and concentrated in vacuo to afford compound A130-2 (83.0 mg, 179 µmol, yield 32.8%, 98.4% purity) as a brown oil.

Compound A130-2 (83.0 mg, 179 µmol, 1.00 eq) and DCM (0.50 mL) were added to a 10 mL round-bottom flask at 25°C. TFA (768 mg, 6.73 mmol, 0.50 mL, 37.7 eq) was then added and reacted at rt for 1 h. LCMS indicated complete consumption of compound A130-2, with the target mass spectrum detected. The reaction solution was concentrated at 50°C to afford compound A130-3 (85.0 mg, crude) as a yellow oil.

At 25°C, compound A13 (56.2 mg, 129 µmol, 1.00 eq), TEA (104 mg, 1.02 mmol, 143 µL, 3.00 eq), and DCM (2.94 mL) were added to a 10 mL round-bottom flask. The atmosphere was purged with N₂ (×3), and HATU (130 mg, 341.35 µmol, 1.00 eq) was added at 0°C. The reaction was allowed to proceed for 0.5 h. Compound A130-3 (122 mg, 341 µmol, 1.00 eq), TEA (104 mg, 1.02 mmol, 143 µL, 3.00 eq), and DMF (2.44 mL) were then added. The mixture was allowed to warm to rt and react for 1.5 h. LCMS indicated complete consumption of compound A130-3, with the target mass spectrum detected. After concentration, the residue was purified by silica gel column chromatography and concentrated in vacuo to afford compound A130 (33.8 mg, 42.3 µmol, yield 12.4%, purity 95.1%) as a bright yellow solid. LCMS: m/z = 759.4 (M+H)⁺. **¹H NMR:** (400 MHz, DMSO-*d₆*) δ 11.8 (s, 1H), 11.1 (s, 1H), 9.14 (s, 1H), 8.74 (t, J = 9.6 Hz, 1H), 8.47-8.39 (m, 2H), 8.23 (d, J = 8.0 Hz, 1H), 8.13 (d, J = 8.8 Hz, 3H), 8.04 (d, J = 8.8 Hz, 1H), 7.83 (s, 1H), 6.98 (d, J = 4.8 Hz, 2H), 6.88-6.85 (m, 1H), 5.37-5.33 (m, 1H), 3.62 (s, 3H), 3.18-3.12 (m, 2H), 2.98-2.92 (m, 2H), 2.90 -2.50 (m, 6H), 2.12-2.07 (m, 1H), 2.02-1.98 (m, 1H), 1.75-1.66 (m, 1H).

### Preparation Example 215. Synthesis of Compound A131

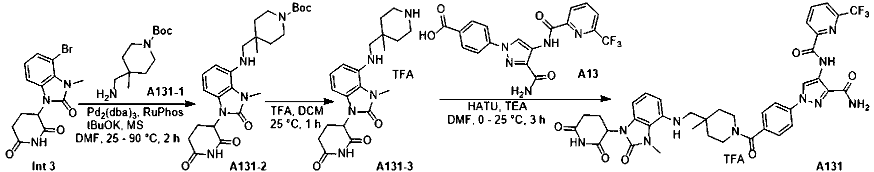

Compound Int 3 (200 mg, 544 µmol, 1.00 eq), compound A131-1 (248 mg, 1.09 mmol, 2.00 eq), and DMF (2.00 mL) were added to a 10 mL flat-bottom flask at 25°C. Pd₂(dba)₃ (49.8 mg, 54.4 µmol, 0.10 eq), RuPhos (25.4 mg, 54.4 µmol, 0.10 eq), and tBuONa (104 mg, 1.09 mmol, 2.00 eq) were then added. The atmosphere was purged with N₂ (×3), and the mixture was heated to 90°C and reacted for 2 h. LCMS indicated 18.7% of compound Int 3 remained, with the target mass spectrum detected. The reaction solution was concentrated, and the crude was purified by preparative HPLC and concentrated in vacuo to afford compound A131-2 (270 mg, 530 µmol, yield 24.4%, 95.3% purity) as a yellow solid.

Compound A131-2 (270 mg, 530 µmol, 1.00 eq) and DCM (2.70 mL) were added to a 10 mL round-bottom flask at 25°C. TFA (4.14 g, 36.3 mmol, 2.70 mL, 68.6 eq) was then added and reacted at rt for 1 h. LCMS indicated complete consumption of compound A131-2, with the target mass spectrum detected. The reaction solution was concentrated to afford compound A131-3 (420 mg, crude, TFA) as a yellow oil.

At 25°C, compound A13 (472 mg, 1.09 mmol, 1.00 eq), DCM (10.0 mL), and TEA (331 mg, 3.27 mmol, 455 µL, 3.00 eq) were added to a 50 mL round-bottom flask. The atmosphere was purged with N₂ (×3), and HATU (414 mg, 1.09 mmol, 1.00 eq) was added at 0°C. The reaction was allowed to proceed for 1 h. Compound A131-3 (420 mg, 1.09 mmol, 1.00 eq) and DMF (10.0 mL) were then added, and the mixture was allowed to warm to rt and react for 2 h. LCMS indicated complete consumption of compound A131-3, with the target mass spectrum detected. Water was added and filtered, and the filter cake was concentrated to obtain a crude, which was purified by preparative HPLC and concentrated in vacuo to afford compound A131 (127 mg, 157 µmol, yield 14.4%, purity 97.5%) as an off-white solid. LCMS: m/z = 787.4 (M+H)⁺. **¹H NMR:** (400 MHz, DMSO-*d₆*) *δ* 11.80 (s, 1H), 11.06 (s, 1H), 9.10 (s, 1H), 8.47-8.39 (m, 2H), 8.22 (d, J = 7.6 Hz, 1H), 8.08 (d, J = 8.4 Hz, 3H), 7.82 (s, 1H), 7.56 (d, J = 8.6 Hz, 2H), 6.87 (t, J = 8.0 Hz, 1H), 6.59-6.52 (m, 2H), 5.32-5.27 (m, 1H), 4.82 (s, 1H), 4.01-3.97 (m, 1H), 3.65 (s, 3H), 3.38-3.33 (m, 2H), 3.04-3.01 (m, 2H), 2.92-2.84 (m, 1H), 2.71-2.59 (m, 3H), 2.00-1.96 (m, 1H), 1.67-1.32 (m, 4H), 1.09 (s, 3H).

### Preparation Example 216. Synthesis of Compound A132

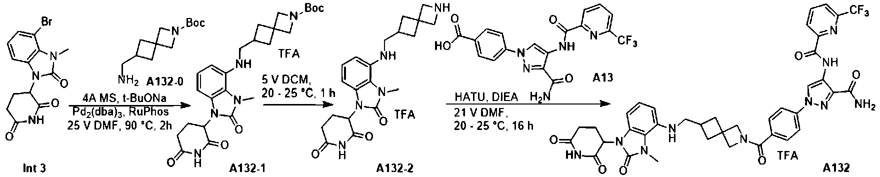

Compound Int 3 (400 mg, 1.04 mmol, 1.00 eq), compound A132-0 (706 mg, 3.12 mmol, 3.00 eq), DMF (10.0 mL), Pd₂(dba)₃ (190 mg, 208 µmol, 0.200 eq), t-BuONa (300 mg, 3.12 mmol, 3.00 eq), RuPhos (97.1 mg, 208 µmol, 0.200 eq), and 4A MS (100 mg) were added to a 100 mL single-necked flask at 20°C-25°C under N₂. The reaction solution was heated to 90°C and reacted for 2 h. LCMS indicated complete consumption of compound Int 3, with the target mass spectrum (48.2%) detected (RT = 0.448 min). The reaction solution was filtered and concentrated, and the crude was purified by preparative HPLC, subjected to rotary evaporation to dryness, and lyophilized to afford compound A132-1 (172 mg, 274 µmol, yield 26.3%, 95.4% purity, TFA) as a yellow solid.

Compound A132-1 (95.0 mg, 187 µmol, 1.00 eq) and DCM (0.5 mL) were added to a 10.0 mL round-bottom flask at 20°C-25°C. TFA (139 mg, 1.22 mmol, 90.6 µL, 6.51 eq) was then slowly added and reacted at 20°C-25°C for 1 h. LCMS indicated complete consumption of compound A132-1, with the target MS (91.2%) detected. The reaction solution was concentrated to afford compound A132-2 (95.0 mg, crude, TFA) as a red oil.

At 20°C-25°C, compound A13 (90.9 mg, 210 µmol, 1.10 eq) and DMF (2.00 mL) were added to a 10 mL single-necked round-bottom flask, and the mixture was cooled to 0°C. DIEA (148 mg, 1.15 mmol, 199 µL, 6.00 eq), HATU (87.1 mg, 229 µmol, 1.20 eq) and compound A132-2 (95.0 mg, 190 µmol, 1.00 eq, TFA) were added at 0°C and reacted for 16 h. LCMS indicated complete consumption of compound A132-2, with the target mass spectrum detected (RT = 0.712 min). The reaction solution was concentrated in vacuo to obtain compound A132 (49.3 mg, 53.7 µmol, yield 28.1%, purity 98.5%, TFA) as a white solid. LCMS: m/z = 785.4 (M+H)⁺. **¹H NMR:** (400 MHz, DMSO-*d₆*) δ 11.81 (s, 1 H), 11.06 (s, 1 H), 9.11 (s, 1 H), 8.45-8.40 (m, 2 H), 8.23 (d, J = 7.6 Hz, 1 H), 8.15-8.06 (m, 3 H), 7.86-7.77 (m, 3 H), 6.86 (t, J = 8.4 Hz, 1 H), 6.51 (d, J = 8.0 Hz, 1 H), 6.42 (d, J = 8.8 Hz, 1 H), 5.29 (dd, J = 12.0, 4.8 Hz, 1 H), 4.46-4.26 (m, 2 H), 4.16-3.98 (m, 4 H), 3.61 (s, 3 H), 3.13-3.02 (m, 2 H), 2.95-2.80 (m, 1 H), 2.70-2.61 (m, 2 H), 2.38-2.28 (m, 2 H). 2.06-1.91 (m, 3 H).

### Preparation Example 217. Synthesis of Compound A133

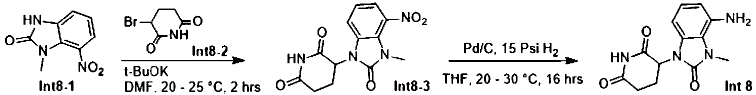

At 20°C-25°C, DMF (200 mL) was added to a 500 mL three-necked flask, and compound Int 8-1 (8.00 g, 41.4 mmol, 1.00 eq), compound Int 8-2 (15.9 g, 82.8 mmol, 2.00 eq), and potassium tert-butoxide (9.29 g, 82.8 mmol, 1.50 eq) were sequentially added. The mixture was purged with N₂ (×3) and reacted at 20°C-25°C for 2 h. LCMS analysis indicated 68.9% of compound Int 8-1 remained unreacted, with the target product (21.4%) detected. The reaction solution was poured into water (600 mL) and filtered. The filter cake was washed with water (50.0 mL) and added to THF (400 mL) and DMF (100 mL). The mixture was dried over Na₂SO₄ and concentrated to afford compound Int 8-3 (8.00 g, crude) as a yellow oil, which was used directly in the next reaction.

Under an argon atmosphere, 10% Pd/C (5.25 g, 4.93 mmol, 10% purity, 0.10 eq) was added to a 75 mL hydrogenation vessel. THF (200 mL) was added, followed by the addition of compound Int 8-3 (15.0 g, 49.3 mmol, 1.00 eq). The mixture was purged with H₂ (×3), and reacted at 30°C under hydrogen (15 psi) for 16 h. LCMS indicated complete consumption of starting materials, with the target product (45.6%) detected. The mixture was purged with argon (×3) and filtered, and the filtrate was purified by thin layer chromatography to obtain compound Int 8 (714 mg, 2.60 mmol, yield 5.28%, 100% purity) as a white solid.

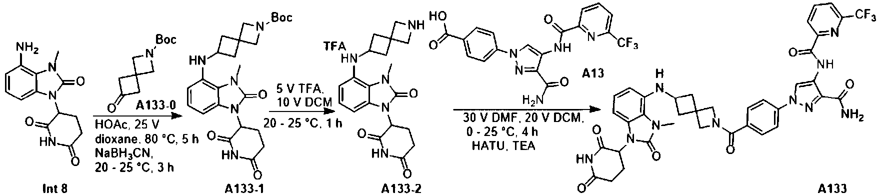

Compound Int 8 (300 mg, 1.06 mmol, 1.00 eq), compound A133-0 (448 mg, 2.12 mmol, 2.00 eq), glacial AcOH (191 mg, 3.18 mmol, 182 µL, 3.00 eq) and dioxane (7.50 mL) were added to a 40 mL single-necked flask, and the mixture was heated to 80°C and reacted for 5 h. After cooling to 20°C-25°C, NaBH₃CN (200 mg, 3.18 mmol, 3.00 eq) was added. The mixture was reacted at 20°C-25°C for another 3 h. LCMS indicated 42.6% of compound Int 8 remained (RT = 0.184 min), with the target mass spectrum (48.3%) detected (RT = 0.427 min). The reaction solution was concentrated and filtered, and the filter cake was washed with DMF. The crude was purified by preparative HPLC, and the mobile phase was lyophilized to afford compound A133-1 (90.0 mg, 186 µmol, yield 16.5%, 96.9% purity) as a pink solid.

Compound A133-1 (90.0 mg, 186 µmol, 1.00 eq), TFA (691 mg, 6.06 mmol, 450 µL, 32.6 eq), and DCM (1.00 mL) were added to a 10 mL single-necked round-bottom flask at 20°C-25°C and reacted for 1 h at 20°C-25°C. LCMS indicated complete consumption of compound A133-1, with the target mass spectrum detected (RT = 0.287 min). The reaction solution was concentrated in vacuo to afford compound A133-2 (0.14 g, crude, TFA) as a yellow oil.

At 20°C-25°C and under N₂, compound A13 (125 mg, 289 µmol, 1.00 eq), TEA (87.9 mg, 869 µmol, 121 µL, 3.00 eq) and DCM (2.80 mL) were added to a 40 mL single-necked round-bottom flask, and the mixture was cooled to 0°C. Compound HATU (110 mg, 289 µmol, 1.00 eq) was added, and the reaction was carried out at 0°C-5°C for 1 h. At 0°C, compound A133-2 (140 mg, 289 µmol, 1.00 eq, TFA), TEA (87.9 mg, 869 µmol, 121 µL, 3.00 eq) and DMF (4.20 mL) were added to a 10 mL round-bottom flask, and the solution in the 10 mL round-bottom flask was added to a 40 mL three-necked round-bottom flask at 0°C and reacted at 20°C-25°C for 3 h. LCMS indicated 21.3% of compound A133-2 remained, with the target mass spectrum detected (RT = 0.461 min). The reaction solution was concentrated in vacuo and washed with DMF. The crude was purified by preparative HPLC and lyophilized to afford compound A133 (52.1 mg, 66.8 µmol, yield 23.0%, purity 98.8%) as a white solid. LCMS: m/z = 771.1 (M+H)⁺. **¹H NMR:** (400 MHz, DMSO-*d₆*) *δ* 11.80 (s, 1H) 11.05 (s, 1H) 9.20 (s, 1H) 8.53-8.37 (m, 2H) 8.24-7.99 (m, 4H) 7.82 (d, J = 7.6 Hz, 3H) 6.89-6.85 (m, 1H) 6.59-6.50 (m, 1H) 6.38 (d, J = 1.2 Hz, 1H) 5.37-5.16 (m, 2H) 4.55-4.30 (m, 2H) 4.25-4.01 (m, 2H) 3.85-3.67 (m, 1H) 3.61 (d, J = 5.6 Hz, 3H) 2.97-2.80 (m, 1H) 2.74-2.57 (m, 4H) 2.28-2.10 (m, 2H) 1.88 (s, 1H).

### Preparation Example 218. Synthesis of Compound A134

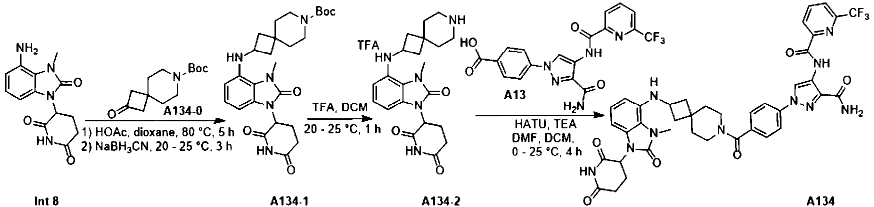

Compound Int 8 (300 mg, 1.06 mmol, 1.00 eq), compound A134-0 (508 mg, 2.12 mmol, 2.00 eq), HOAc (191 mg, 3.18 mmol, 182 µL, 3.00 eq), and dioxane (7.50 mL) were added to a 40 mL single-necked flask and reacted at 80°C for 5 h. The temperature then lowered to 20°C-25°C, and NaBH₃CN (200 mg, 3.18 mmol, 3.00 eq) was added. The reaction continued at 20°C-25°C for 3 h. LCMS indicated 15.2% of compound Int 8 remained (RT = 0.129 min), with the target mass spectrum (78.3%) detected (RT = 0.469 min). The reaction solution was concentrated and filtered, and the filter cake was washed with DMF. The crude was purified by preparative HPLC, and the mobile phase was lyophilized to afford compound A134-1 (120 mg, 226 µmol, yield 20.1%, 93.9% purity) as an off-white solid.

Compound A134-1 (120 mg, 226 µmol, 1.00 eq) was added to DCM (1.20 mL) at 20°C-25°C, followed by the addition of TFA (921 mg, 8.08 mmol, 600 µL, 35.6 eq). The mixture was reacted for 1 h. LCMS indicated complete consumption of compound A134-1, with the target mass spectrum detected (RT = 0.326 min). The reaction solution was concentrated to afford compound A134-2 (0.25 g, crude, TFA) as a yellow oil.

Compound A13 (211 mg, 489 µmol, 1.00 eq), TEA (148 mg, 1.47 mmol, 204 µL, 3.00 eq), and DCM (5.00 mL) were added to a 40 mL single-necked flask, and the mixture was cooled to 0°C-5°C. HATU (186 mg, 489 µmol, 1.00 eq) was added. The reaction was allowed to proceed for 1 h. Compound A134-2 (250 mg, 489 µmol, 1.00 eq, TFA), TEA (148 mg, 1.47 mmol, 204 µL, 3.00 eq), and DMF (7.50 mL) were then added. The temperature was raised to 20°C-25°C and the reaction was allowed to proceed for 3 h. LCMS indicated complete consumption of compound A134-2, with the target mass spectrum (73.7%) detected (RT = 0.476 min). The reaction solution was concentrated and filtered, the filter cake was washed with DMF, and the crude was purified by preparative HPLC. The mobile phase was lyophilized to obtain compound A134 (128 mg, 158 µmol, yield 32.4%, purity 98.7%) as an off-white solid. LCMS: m/z =799.1 (M+H)⁺. **¹H NMR:** (400 MHz, DMSO-*d₆*) *δ* 11.80 (s, 1H) 11.1 (s, 1H) 9.09 (s, 1H) 8.48-8.44 (m, 1H) 8.43-8.37 (m, 1H) 8.26-8.17 (m, 1H) 8.07 (d, J = 8.4 Hz, 3H) 7.87-7.75 (m, 1H) 7.66-7.47 (m, 2H) 6.85 (t, J = 8.0 Hz, 1H) 6.53 (d, J = 8.0 Hz, 1H) 6.36-6.21 (m, 1H) 5.35-5.20 (m, 1H) 3.91-3.76 (m, 2H) 3.61 (s, 3 H) 3.58-3.49 (m, 1H) 3.43-3.19 (m, 2H) 2.96-2.79 (m, 1H) 2.74-2.57 (m, 2H) 2.44-2.29 (m, 2H) 2.03-1.90 (m, 1H) 1.87-1.49 (m, 5H).

### Preparation Example 219. Synthesis of Compound A135

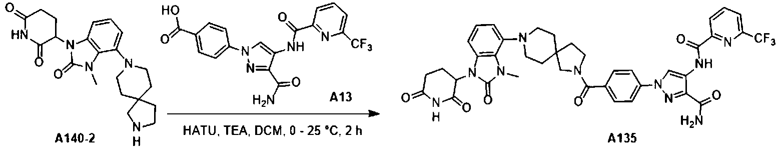

Compound A13 (73.7 mg, 175 µmol, 1.00 eq), TEA (53.4 mg, 527 µmol, 73.4 µL, 3.00 eq), and DCM (1.20 mL) were added to a 100 mL three-necked round-bottom flask at 20°C-25°C, and the mixture was cooled to 0°C. HATU (66.9 mg, 175 µmol, 1.00 eq) was added at 0°C and reacted for 0.5 h. Compound A140-2 (90.0 mg, 175 µmol, 1.00 eq, TFA), TEA (53.4 mg, 527 µmol, 73.4 µL, 3.00 eq), and DCM (1.20 mL) were added to a 50 mL three-necked round-bottom flask at 20°C-25°C. The solution in the 50 mL three-necked round-bottom flask was added to the 100 mL three-necked round-bottom flask at 0°C. The reaction continued at 20°C-25°C for 1.5 h. LCMS indicated complete consumption of compound A140-2, with the target mass spectrum detected (RT = 0.496 min). The products were mixed and concentrated in vacuo. The crude was purified by reversed-phase high performance liquid chromatography, concentrated in vacuo to remove ACN, and lyophilized to obtain compound A135 (59.0 mg, 72.3 µmol, yield 31.6%, 98.0% purity) as a white solid. LCMS: m/z = 799.2 (M+H)⁺. **¹H NMR:** (400 MHz, DMSO-*d₆*) *δ* 11.8 (s, 1H), 11.1 (d, J = 8.0 Hz, 1H), 9.11 (s, 1H), 8.47-8.39 (m, 2H), 8.22 (d, J = 8.0 Hz, 1H), 8.10-8.06 (m, 3H), 7.82 (s, 1H), 7.76-7.12 (m, 2H), 7.00-6.84 (m, 3H), 5.38-5.30 (m, 1H), 3.67 (s, 3H), 3.57 (s, 4H), 3.14-2.85 (m, 5H), 2.73-2.56 (m, 3H), 1.99-1.90 (m, 2H), 1.88-1.71 (m, 4H).

### Preparation Example 220. Synthesis of Compound A136

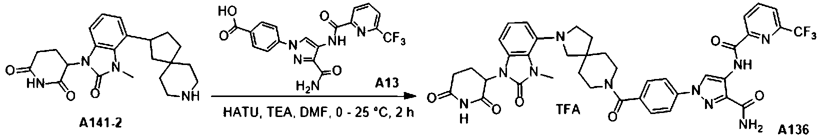

Compound A13 (122 mg, 293 µmol, 1.00 eq), TEA (89.0 mg, 879 µmol, 122 µL, 3.00 eq), and DMF (3.00 mL) were added to a 100 mL three-necked round-bottom flask at 20°C-25°C, and the mixture was cooled to 0°C. HATU (111 mg, 293 µmol, 1.00 eq) was added at 0°C and reacted for 0.5 h. Compound A141-2 (150 mg, 293 µmol, 1.00 eq, TFA), TEA (89.0 mg, 879 µmol, 122 µL, 3.00 eq), and DMF (3.00 mL) were added to a 50 mL three-necked round-bottom flask at 20°C-25°C. The solution in the 50 mL three-necked round-bottom flask was added to the 100 mL three-necked round-bottom flask at 0°C. The reaction continued at 20°C-25°C for 1.5 h. LCMS indicated complete consumption of compound A141-2, with the target mass spectrum detected (RT = 0.475 min). The products were mixed, and the mixture was filtered and concentrated in vacuo. The crude was purified by reverse-phase high-performance liquid chromatography, concentrated in vacuo to remove ACN, and lyophilized to obtain compound A136 (63.5 mg, 68.2 µmol, yield 18.0%, purity 98.9%, TFA) as a yellow solid. LCMS: m/z = 799.3 (M+H)⁺. **¹H NMR:** (400 MHz, DMSO-*d₆*) *δ* 11.8 (s, 1H), 11.1 (s, 1H), 9.10 (s, 1H), 8.47-8.38 (m, 2H), 8.22 (d, J = 7.2 Hz, 1H), 8.08 (d, J = 8.8 Hz, 3H), 7.81 (s, 1H), 7.58 (d, J = 8.4 Hz, 2H), 6.98 (d, J = 4.4 Hz, 2H), 6.89-6.85 (m, 1H), 5.37-5.32 (m, 1H), 3.76-3.67 (m, 4H), 3.59 (s, 3H), 3.10 (s, 2H), 2.98 (s, 2H), 2.92-2.85 (m, 1H), 2.74-2.60 (m, 2H), 2.02-1.97 (m, 1H), 1.84 (s, 2H), 1.75-1.68 (m, 4H).

### Preparation Example 221. Synthesis of Compound A137

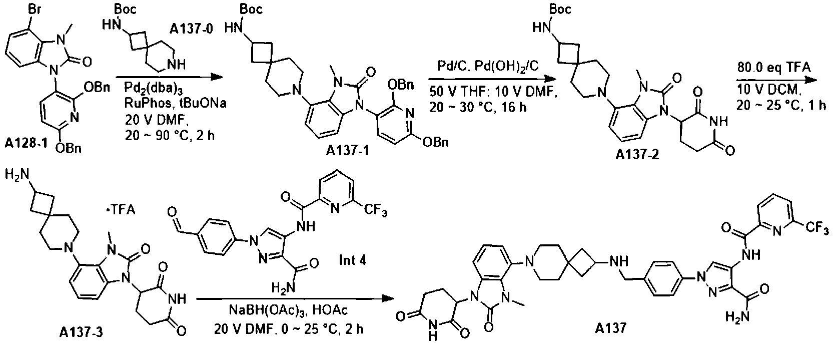

At 20°C-25°C, compound A128-1 (50.0 mg, 96.4 µmol, 1.00 eq), compound A137-0 (46.3 mg, 192 µmol, 2.00 eq), RuPhos (4.50 mg, 9.64 µmol, 0.100 eq), tBuONa (18.5 mg, 192 µmol, 2.00 eq), Pd₂(dba)₃ (8.83 mg, 9.64 µmol, 0.100 eq) and DMF (2.50 mL) were added to a 10 mL parallel reaction flask, and the mixture was purged with N₂ (×3) and then reacted at 90°C for 2 h. LCMS indicated 53% of compound A128-1 remained (RT = 0.579 min), with the target mass spectrum (14.7%) detected (RT = 0.618 min). The reaction solution was filtered in vacuo, concentrated, and purified by column chromatography (SiO₂, petroleum ether : ethyl acetate = 5 : 2, Rf = 0.450) to afford compound A137-1 (140 mg, 190 µmol, yield 24.7%, 92.0% purity) as a yellow solid.

Pd/C (70.0 mg, 65.7 µmol, 10%) and Pd(OH)₂/C (70.0 mg, 190 µmol, 20%) were slowly added to a 35 mL hydrogenation vessel under an argon atmosphere at 20°C-25°C. Compound A137-1 (140 mg, 190 µmol, 1.00 eq), THF (7.00 mL), and DMF (1.40 mL) were then added sequentially. The mixture was purged with H₂ (20 psi) (×3), and then reacted at 25°C-30°C for 16 h. LCMS indicated complete consumption of compound A137-1, with the target mass spectrum detected (RT = 0.452 min). The mixture was degassed and purged with Ar (×3). The mixture was filtered through Celite and washed with THF (20.0 mL×3) at 25°C. The mixture was concentrated at 40°C in vacuo to afford compound A137-2 (110 mg, 169 µmol, yield 88.7%, 76.4% purity) as a yellow oil.

Compound A137-2 (110 mg, 169 µmol, 1.00 eq), TFA (1.54 g, 13.5 mmol, 1.00 mL, 80.0 eq), and DCM (1.00 mL) were added to a 100 mL three-necked round-bottom flask at 20°C-25°C and reacted at 20°C-25°C for 1 h. LCMS indicated complete consumption of compound A137-2, with the target mass spectrum detected (RT = 0.264 min). The mixture was concentrated at 40°C-45°C to afford compound A137-3 (235 mg, crude, TFA) as a yellow oil.

Compound A137-3 (160 mg, 272 µmol, 1.00 eq, TFA), compound Int 4 (113 mg, 272 µmol, 1.00 eq), DMF (8.00 mL), and HOAc (326 mg, 5.44 mmol, 311 µL, 20.0 eq) were added to a 10 mL parallel reaction flask at 20°C-25°C. The mixture was reacted at 20°C-25°C for 1 h. NaBH(OAc)₃ (230 mg, 1.09 mmol, 4.00 eq) was then added at 0°C-5°C. The mixture was reacted at 20°C-25°C for 1 h. LCMS indicated 3.2% of compound A137-3 remained (RT = 0.262 min), with the target mass spectrum (49.96%) detected (RT = 0.39 min). The reaction solution was filtered and concentrated in vacuo and purified using preparative HPLC (TFA conditions; column: Welch Xtimate C18 150×25 mm×5 µm; mobile phase: [water (TFA)-ACN]; gradient: 30%-50% B, more than 10 min). After concentration, compound A137 (61.0 mg, 66.8 µmol, yield 24.5%, purity 98.5%, TFA) was obtained as an off-white solid. LCMS: m/z = 785.1 (M+H)⁺. **¹H NMR:** (400 MHz, DMSO-*d₆*) *δ* 11.80 (s, 1H), 11.08 (s, 1H), 9.20-9.13 (m, 1H), 9.18-9.13 (s, 1H), 8.48-8.36 (m, 2H), 8.23 (dd, J = 0.8, 7.6 Hz, 1H), 8.13 (d, J = 8.6 Hz, 2H), 8.07-8.01 (s, 1H), 7.84 (s, 1H), 7.68 (d, J = 8.6 Hz, 2H), 7.00-6.82 (m, 3H), 5.41-5.28 (m, 1H), 4.14 (s, 2H), 3.87 (s, 1H), 3.62 (s, 3H) , 3.06-2.82 (m, 3H), 2.77-2.55 (m, 4H), 2.43-2.28 (m, 1H), 2.16-1.91 (m, 4H), 1.87-1.63 (m, 4H).

### Preparation Example 222. Synthesis of Compound A138

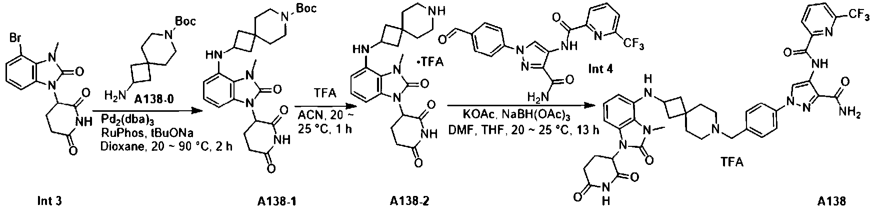

Compound Int 3 (200 mg, 547 µmol, 1.00 eq), compound A138-0 (197 mg, 821 µmol, 1.50 eq), and tBuONa (105 mg, 1.10 mmol, 2.00 eq) were added to a 10 mL parallel reaction flask at 20°C-25°C. RuPhos (25.5 mg, 54.7 µmol, 0.100 eq), Pd₂(dba)₃ (50.1 mg, 54.7 µmol, 0.100 eq), and DMF (4.00 mL) were then added to the reaction flask under N₂. The mixture was purged with N₂ (×3), and then reacted at 90°C under N₂ for 2 h. LCMS indicated 24% of compound Int 3 remained (RT = 0.352 min), with the target mass spectrum (33%) detected (RT = 0.452 min). The reaction solution was filtered, concentrated, and purified by high-performance liquid chromatography. After concentration, compound A138-1 (200 mg, 397 µmol, yield 18.1%, 99.0% purity) was obtained as a yellow oil.

Compound A138-1 (200 mg, 397 µmol, 1.00 eq), ACN (4.00 mL), and TFA (6.14 g, 53.8 mmol, 4.00 mL, 135 eq) were added to a 10 mL parallel reaction flask at 20°C-25°C and reacted for 1 h. LCMS indicated complete consumption of compound A138-1, with the target mass spectrum detected (RT = 0.239 min). The reaction solution was concentrated to afford compound A138-2 (200 mg, crude, TFA) as a yellow oil.

At 20°C-25°C, compound Int 4 (150 mg, 361 µmol, 1.00 eq), compound A138-2 (190 mg, 361 µmol, 1.00 eq, TFA), DMF (8.00 mL), THF (40.0 mL) and KOAc (71.0 mg, 723 µmol, 2.00 eq) were added to a 100 mL three-necked round-bottom flask, and the mixture was reacted at 20°C-25°C for 1 h. At 20°C-25°C, NaBH(OAc)₃ (230 mg, 1.09 mmol, 3.00 eq) was then added. The mixture was reacted at 20°C-25°C for 12 h. LCMS indicated complete consumption of compound A138-2, with the target mass spectrum detected (RT = 0.379 min). The reaction solution was filtered, concentrated, and purified by high-performance liquid chromatography. The mixture was concentrated and lyophilized to afford compound A138 (60.8 mg, 64.2 µmol, yield 17.7%, 95.0% purity, TFA) as an off-white solid. LCMS: m/z = 785.1 (M+H)⁺. **¹H NMR:** (400 MHz, DMSO-*d₆*) *δ* 11.80 (s, 1H), 11.07 (s, 1H), 9.50-9.37 (s, 1H), 9.12 (s, 1H), 8.49-8.37 (m, 2H), 8.23 (d, J = 7.6 Hz, 1H), 8.14 (d, J = 8.4 Hz, 2H), 8.05 (s, 1H), 7.85 (s, 1H), 7.71-7.63 (m, 2H), 6.85 (t, J = 8.2 Hz, 1H), 6.58-6.52 (m, 1H), 6.30 (d, J = 8.4 Hz, 1H), 5.33-5.21 (m, 1H), 4.37 (d, J = 3.8 Hz, 2H), 3.88-3.79 (m, 1H), 3.61 (s, 3H), 3.10-2.98 (m, 1H), 2.97-2.81 (m, 2H), 2.71-2.57 (m, 5H), 2.32 (d, J = 1.8 Hz, 1H), 2.29-2.20 (m, 1H), 2.08-1.92 (m, 2H), 1.91-1.65 (m, 4H).

### Preparation Example 223. Synthesis of Compound A139

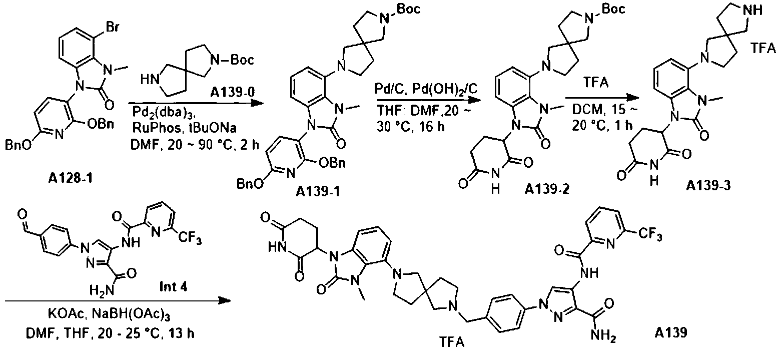

Compound A128-1 (300 mg, 578 µmol, 1.00 eq), compound A139-0 (261 mg, 1.16 mmol, 2.00 eq), and RuPhos (27.0 mg, 57.8 µmol, 0.100 eq) were added to a 50 mL three-necked round-bottom flask at 20°C-25°C. t-BuONa (111 mg, 1.16 mmol, 2.00 eq), Pd₂(dba)₃ (52.9 mg, 57.8 µmol, 0.100 eq), and DMF (15.0 mL) were then added. The atmosphere was purged with N₂ (×3), and the mixture was heated to 90°C and reacted for 2 h. LCMS indicated approximately 11% of compound A128-1 remained, with the target mass spectrum (53.7%) detected. The reaction solution was filtered, and the filtrate was concentrated. The crude was purified by column chromatography and concentrated to yield compound A139-1 (190 mg, 280 µmol, yield 24.2%, 97.7% purity) as a yellow solid.

Pd/C (170 mg, 159 µmol, 10.0%) and Pd(OH)₂/C (170 mg, 20.0%) were added to a 75 mL hydrogenation vessel at 20°C-25°C. Compound A139-1 (340 mg, 502 µmol, 1.00 eq) in THF (17.0 mL) and DMF (3.40 mL) was added under an argon atmosphere. The mixture was purged with H₂ (×3), and the temperature was raised to 20°C-30°C. The reaction was allowed to proceed for 16 h. LCMS indicated complete consumption of compound A139-1, with the target mass spectrum detected. The reaction solution was filtered through Celite, and the filtrate was concentrated. After concentration, compound A139-2 (380 mg, crude) was obtained as a yellow oil.

Compound A139-2 (340 mg, 653 µmol, 1.00 eq) and DCM (6.60 mL) were added to a 100 mL single-necked flask at 20°C-25°C. TFA (5.37 g, 47.1 mmol, 3.50 mL, 72.1 eq) was then added, and the mixture was reacted at 20°C-25°C for 1 h. LCMS indicated complete consumption of compound A139-2, with the target mass spectrum detected. The reaction solution was directly concentrated to obtain compound A139-3 (732 mg, crude, TFA) as a yellow oil.

Compound A139-3 (350 mg, 659 µmol, 1.00 eq, TFA), compound Int 4 (274 mg, 659 µmol, 1.00 eq), DMF (8.40 mL), and THF (42.0 mL) were added to a 250 mL single-necked flask at 20°C-25°C. KOAc (129 mg, 1.32 mmol, 2.00 eq) was then added and the mixture was reacted at 20°C-25°C for 1 h. NaBH(OAc)₃ (279 mg, 1.32 mmol, 2.00 eq) was then added and the reaction continued at 20°C-25°C for 12 h. LCMS indicated approximately 39.5% of compound Int 4 remained, with the target mass spectrum (approximately 47.4%) detected. The reaction solution was filtered, the filtrate was directly concentrated, and the crude was purified by preparative HPLC to afford compound A139 (141 mg, 158 µmol, yield 24.0%, purity 99.2%, TFA) as a yellow solid. LCMS: m/z = 771.5 (M+H)⁺. **¹H** NMR: (400 MHz, DMSO-*d₆*) *δ* 11.80 (s, 1H), 11.09 (d, J = 4.0 Hz, 1H), 9.12 (s, 1H), 8.48-8.36 (m, 2H), 8.23 (d, J = 7.6 Hz, 1H), 8.14 (d, J = 8.4 Hz, 2H), 8.08 (s, 1H), 7.84 (s, 1H), 7.73 (dd, J = 3.2, 8.6 Hz, 2H), 7.05-6.83 (m, 3H), 5.40-5.30 (m, 1H), 4.54-4.41 (m, 2H), 3.58 (d, J = 10.2 Hz, 3H), 3.24-3.04 (m, 5H), 2.94-2.81 (m, 2H), 2.66 (m, 4H), 2.35-2.29 (m, 1H), 2.17-1.92 (m, 4H).

### Preparation Example 224. Synthesis of Compound A140

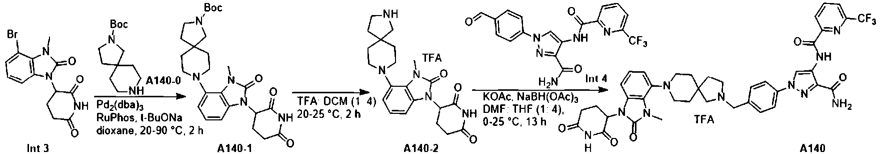

At 20°C-25°C, compound Int 3 (100 mg, 274 µmol, 1.00 eq), compound A140-0 (98.9 mg, 411 µmol, 1.50 eq) and dioxane (2.00 mL) were added to a 250 mL single-necked flask. RuPhos (12.8 mg, 27.4 µmol, 0.10 eq), Pd₂(dba)₃ (25.1 mg, 27.4 µmol, 0.10 eq) and t-BuONa (52.7 mg, 548 µmol, 2.00 eq) were then added. The reaction was carried out at 90°C for 2 h. LCMS indicated approximately 11.9% of compound Int 3 remained, with the target mass spectrum detected. The reaction solution was cooled to 20°C-25°C, poured into 5% AcOH solution, and filtered. The crude was purified by preparative HPLC and lyophilized to afford compound A140-1 (80.0 mg, 159 µmol, yield 14.5%, 99.4% purity) as a pink solid.

Compound A140-1 (80.0 mg, 159 µmol, 1.00 eq) and DCM (8.00 mL) were added to a 100 mL single-necked flask at 20°C-25°C. TFA (3.07 g, 26.9 mmol, 2.00 mL, 168 eq) was then added, and the mixture was reacted at 20°C-25°C for 2 h. LCMS indicated complete consumption of compound A140-1, with the target mass spectrum detected. The reaction solution was directly concentrated to afford compound A140-2 (95.0 mg, crude, TFA) as a yellow solid.

Compound A140-2 (95.0 mg, 239 µmol, 1.00 eq), compound Int 4 (96.4 mg, 239 µmol, 1.00 eq), DMF (5.00 mL), and THF (20.0 mL) were added to a 250 mL single-necked flask at 20°C-25°C. KOAc (46.9 mg, 478 µmol, 2.00 eq) was then added, and the mixture was reacted at 20°C-25°C for 1 h. NaBH(OAc)₃ (151 mg, 717 µmol, 3.00 eq) was then added, and the mixture was reacted at 20°C-25°C for 12 h. LCMS indicated complete consumption of compound A140-2, with the target mass spectrum detected. The reaction solution was directly concentrated, and the crude was purified by preparative HPLC to afford compound A140 (52.0 mg, 55.7 µmol, yield 23.3%, purity 96.4%, TFA) as a pink solid. LCMS: m/z = 785.4 (M+H)⁺. **¹H** NMR: (400 MHz, DMSO-*d₆*) *δ* 11.8 (s, 1H), 11.1 (s, 1H), 10.1-10.0 (m, 1H), 9.12 (s, 1H), 8.47-8.39 (m, 2H), 8.23 (d, J = 7.6 Hz, 1H), 8.15 (d, J = 8.0 Hz, 2H), 8.05 (s, 1H), 7.85 (s, 1H), 7.73 (d, J = 8.0 Hz, 2H), 6.98-6.87 (m, 3H), 5.37-5.33 (m, 1H), 4.48 (s, 2H), 3.63 (s, 3H), 3.15-3.00 (m, 4H), 2.90-2.84 (m, 2H), 2.75-2.59 (m, 4H), 2.21-2.12 (m, 1H), 2.07 (s, 1H), 2.02-1.96 (m, 1H), 1.92-1.82 (m, 3H), 1.76 (s, 2H).

### Preparation Example 225. Synthesis of Compound A141

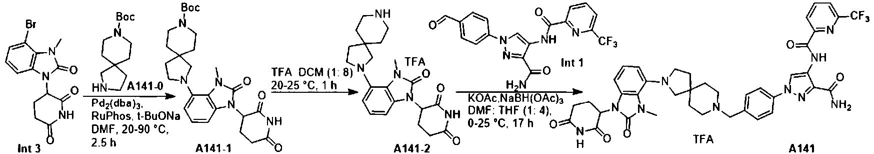

Compound Int 3 (200 mg, 520 µmol, 1.00 eq), compound A141-0 (187 mg, 780 µmol, 1.50 eq), and DMF (8.00 mL) were added to a 100 mL three-necked round-bottom flask at 20°C-25°C. RuPhos (48.5 mg, 104 µmol, 0.20 eq), Pd₂(dba)₃ (95.3 mg, 104 µmol, 0.20 eq), and tBuONa (150 mg, 1.56 mmol, 3.00 eq) were then added. The atmosphere was purged with N₂ (×3), and the mixture was heated to 90°C and reacted for 2.5 h. LCMS indicated complete consumption of compound Int 3, with the target mass spectrum detected. The reaction solution was cooled to 20°C-25°C, poured into 5% AcOH solution and filtered. The filtrate was concentrated. The crude was purified by preparative HPLC and concentrated to yield compound A141-1 (430 mg, 777 µmol, yield 29.8%, 90.0% purity) as a yellow solid.

Compound A141-1 (430 mg, 777 µmol, 1.00 eq) and DCM (8.60 mL) were added to a 100 mL single-necked flask at 20°C-25°C. TFA (1.54 g, 13.4 mmol, 1.00 mL, 17.3 eq) was then added, and the mixture was reacted at 20°C-25°C for 1 h. LCMS indicated complete consumption of compound A141-1, with the target mass spectrum detected. The reaction solution was directly concentrated to yield compound A141-2 (660 mg, crude, TFA) as a yellow oil.

Compound A141-2 (460 mg, 899 µmol, 1.00 eq, TFA), compound Int 4 (362 mg, 899 µmol, 1.00 eq), DMF (23.0 mL), and THF (92.0 mL) were added to a 250 mL single-necked flask at 20°C-25°C. KOAc (264 mg, 2.70 mmol, 3.00 eq) was then added, and the mixture was reacted at 20°C-25°C for 1 h. NaBH(OAc)₃ (762 mg, 3.60 mmol, 4.00 eq) was then added, and the mixture was reacted at 20°C-25°C for 17 h. LCMS indicated complete consumption of compound A141-2, with the target mass spectrum detected. The reaction solution was directly concentrated, and the crude was purified by preparative HPLC to afford compound A141 (199 mg, 216 µmol, yield 21.9%, purity 97.5%, TFA) as a white solid. LCMS: m/z = 785.3 (M+H)⁺. **¹H NMR:** (400 MHz, DMSO-*d₆*) *δ* 11.8 (s, 1H), 11.1 (d, J = 4.0 Hz, 1H), 9.69-9.61 (m, 1H), 9.11 (d, J = 4.4 Hz, 1H), 8.47-8.39 (m, 2H), 8.22 (d, J = 7.6 Hz, 1H), 8.14 (t, J = 8.0 Hz, 2H), 8.05 (s, 1H), 7.84 (s, 1H), 7.70-7.64 (m, 2H), 7.00-6.94 (m, 2H), 6.90-6.87 (m, 1H), 5.38-5.32 (m, 1H), 4.39 (d, J = 22.8 Hz, 2H), 3.35 (t, J = 11.6 Hz, 3H), 3.17-3.03 (m, 5H), 2.90-2.60 (m, 5H), 2.01-1.80 (m, 8H).

### Preparation Example 226. Synthesis of Compound A142

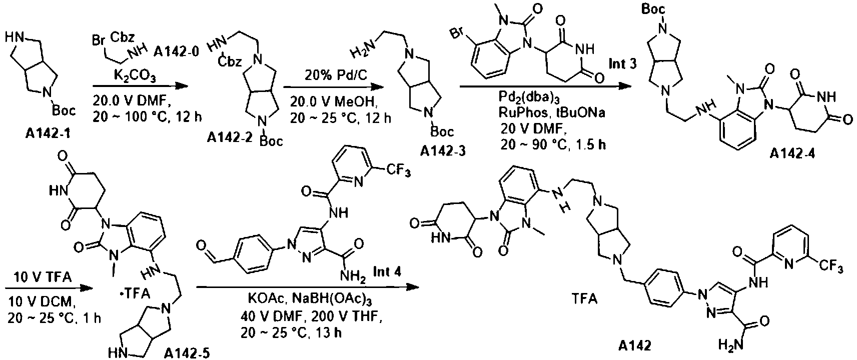

Compound A142-1 (6.50 g, 30.6 mmol, 1.00 eq), compound A142-0 (10.2 g, 39.8 mmol, 1.30 eq), K₂CO₃ (12.7 g, 91.8 mmol, 3.00 eq), and DMF (162 mL) were added to a 250 mL single-necked flask at 20°C-25°C. The reaction was allowed to proceed at 100°C for 12 h. LCMS indicated complete consumption of compound A142-1, with the target mass spectrum detected. The reaction solution was directly concentrated, and the crude was purified by column chromatography to obtain compound A142-2 (10.4 g, 24.2 mmol, yield 58.2%, 90.8% purity) as a white solid.

Pd/C (3.00 g, 2.82 mmol, 10%) was added to a 250 mL hydrogenation vessel at 20°C-25°C. Under an argon atmosphere, compound A142-2 (6.00 g, 13.9 mmol, 1.00 eq) in MeOH (60.0 mL) was added, and the mixture was purged with H₂ (×3) and reacted at 20°C-25°C and 15 psi for 12 h. LCMS indicated complete consumption of compound A142-2, with the target mass spectrum detected. The reaction solution was filtered through Celite, and the filtrate was concentrated. After concentration, compound A142-3 (2.85 g, 10.4 mmol, yield 74.8%, 93.8% purity) was obtained as a yellow oil.

At 20°C-25°C, compound Int 3 (60.0 mg, 156 µmol, 1.00 eq), compound A142-3 (85.0 mg, 312 µmol, 2.00 eq) and tBuONa (30.0 mg, 312 µmol, 2.00 eq) were added to a 100 mL three-necked round-bottom flask. RuPhos (7.29 mg, 15.6 µmol, 0.100 eq), Pd₂(dba)₃ (14.3 mg, 15.6 µmol, 0.100 eq) and DMF (4.00 mL) were then added. The atmosphere was purged with N₂ (×3) and the mixture was heated to 90°C and reacted for 1.5 h. LCMS indicated approximately 31.3% of compound Int 3 remained, with the target mass spectrum detected. The reaction solution was filtered, and the filtrate was concentrated. The crude was purified by preparative HPLC and concentrated to afford compound A142-4 (60.0 mg, 111 µmol, yield 23.8%, 95.3% purity) as a yellow solid.

Compound A142-4 (60.0 mg, 111 µmol, 1.00 eq) and DCM (3.00 mL) were added to a 100 mL single-necked flask at 20°C-25°C. TFA (5.17 g, 45.3 mmol, 3.37 mL, 406 eq) was then added, and the mixture was reacted at 20°C-25°C for 1 h. LCMS indicated complete consumption of compound A141-4, with the target mass spectrum detected. The reaction solution was directly concentrated to afford compound A142-5 (150 mg, crude, TFA) as a yellow oil.

At 20°C-25°C, compound A142-5 (150 mg, 238 µmol, 1.00 eq, TFA), compound Int 4 (99.3 mg, 238 µmol, 1.00 eq), DMF (3.60 mL), and THF (18.0 mL) were added to a 250 mL single-necked flask. KOAc (46.7 mg, 476 µmol, 2.00 eq) was then added, and the mixture was reacted at 20°C-25°C. for 1 h. NaBH(OAc)₃ (100 mg, 476 µmol, 2.00 eq) was then added, and the reaction continued at 20°C-25°C for 12 h. LCMS indicated approximately 36.6% of compound A142-5 remained, with the target mass spectrum detected. The reaction solution was directly filtered and concentrated, and the crude was purified by preparative HPLC to afford compound A142 (51.3 mg, 42.8 µmol, yield 17.9%, purity 95.2%, TFA) as a white solid. LCMS: m/z = 800.2 (M+H)⁺. **¹H NMR:** (400 MHz, DMSO-*d₆*) *δ* 11.80 (s, 1H), 11.06 (s, 1H), 9.10 (s, 1H), 8.49-8.37 (m, 2H), 8.23 (d, J = 7.6 Hz, 1H), 8.18-7.98 (m, 3H), 7.84 (s, 1H), 7.64 (s, 2H), 6.94-6.85 (s, 1H), 6.59 (d, J = 7.6 Hz, 2H), 5.53-5.35 (m, 1H), 5.35-5.25 (m, 1H), 4.54-4.24 (m, 2H), 4.03-3.85 (m, 1H), 3.63 (s, 3H), 3.27-2.81 (m, 7H), 2.54-2.33 (s, 7H), 2.35-2.28 (m, 2H), 2.01-1.89 (m, 2H).

### Preparation Example 227. Synthesis of Compound A143

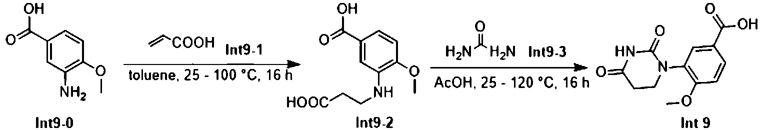

At 25°C, compound Int 9-0 (2.50 g, 15.0 mmol, 1.00 eq) was added to toluene (10.0 mL), followed by the addition of compound Int 9-1 (4.31 g, 59.8 mmol, 4.10 mL, 4.00 eq). The mixture was heated to 100°C and reacted for 16 h. LCMS indicated complete consumption of compound Int 9-0, with the target product (62.9%) detected. The reaction was cooled to 25°C, methyl tert-butyl ether (20.0 mL) was added, and the mixture was further cooled to 0°C with stirring for 0.5 h. The mixture was filtered, and the filter cake was washed with petroleum ether (10.0 mL). The filter cake was dried under reduced pressure to obtain compound Int 9-2 (3.56 g, 14.82 mmol, yield 99.1%, 99.6% purity) as a white solid, which was used directly in the next reaction.

At 25°C, compound Int 9-2 (3.56 g, 14.8 mmol, 1.00 eq) was added to AcOH (18.0 mL), followed by the addition of compound Int 9-3 (7.10 g, 118 mmol, 6.36 mL, 7.98 eq). The mixture was heated to 120°C and reacted for 16 h. LCMS indicated complete consumption of starting materials, with the main peak of the product detected. The reaction solution was poured into HCl (0.50 M, 62.0 mL), stirred at 25°C for 1 h and filtered, and the filter cake was washed with cold water (39.0 mL). The filter cake was dried under reduced pressure to obtain compound Int 9 (2.84 g, 9.75 mmol, yield 65.7%, 90.7% purity) as a yellow solid.

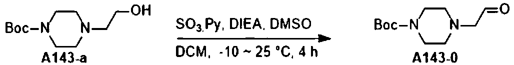

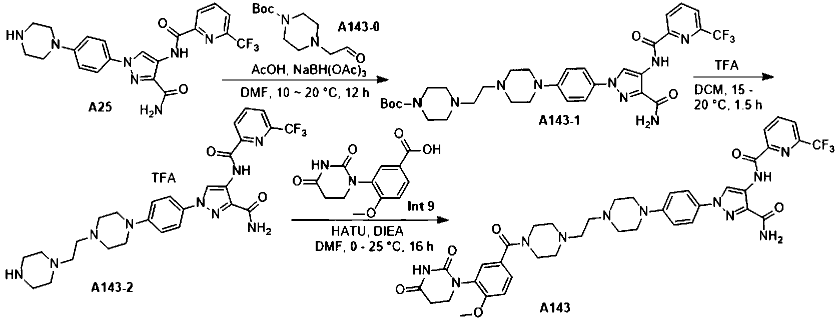

Compound A143-a (3.00 g, 13.0 mmol, 1.00 eq) and DCM (30.0 mL) were added to a 250 mL three-necked round-bottom flask at -10°C-25°C. Pyridine sulfur trioxide complex (7.26 g, 45.6 mmol, 3.50 eq), DIEA (6.73 g, 52.1 mmol, 9.08 mL, 4.00 eq), and DMSO (8.14 g, 104 mmol, 8.14 mL, 8.00 eq) were then added, and the mixture was reacted at -10°C-25°C for 4 h. Thin layer chromatography (TLC) (DCM : MeOH = 20 : 1) indicated complete consumption of compound A143-a (Rf= 0.2), yielding a major spot (Rf = 0.3). Water (80.0 mL) and DCM (250 mL) were added separately. After liquid separation, the mixture was extracted with DCM (×3) (60.0 mL). The organic phases were combined, washed with saturated aqueous NaCl (80.0 mL), dried over Na₂SO₄, filtered, and concentrated to afford compound A143-0 (1.35 g, 5.91 mmol, yield 45.4%) as a yellow oil.

CompoundA25 (193 mg, 0.84 mmol, 1.00 eq), A143-0 (96.0 mg, 0.84 mmol, 1.00 eq), AcOH (202 mg, 3.37 mmol, 0.19 mL, 4.00 eq), and DMF (4.00 mL) were added to a 100 mL three-necked round-bottom flask at 10°C-20°C. NaBH(OAc)₃ (358 mg, 1.69 mmol, 2.00 eq) was added, and the mixture was reacted at 10°C-20°C for 12 h. LCMS indicated complete consumption of compound A25, with the target mass spectrum (68.2%) detected. The product was poured into saturated Na₂CO₃ (28.0 mL) and filtered, and the filter cake was washed with water (4.00 mL), concentrated and dried. The crude was purified by preparative HPLC to obtain compound A143-1 (300 mg, 0.41 mmol, yield 48.4%, 91.5% purity) as a yellow solid.

Compound A143-1 (300 mg, 0.41 mmol, 1.00 eq) and DCM (3.00 mL) were added to a 100 mL three-necked round-bottom flask at 15°C-20°C. TFA (4.21 g, 36.9 mmol, 2.74 mL, 90.3 eq) was added, and the mixture was reacted at 20°C for 1.5 h. LCMS indicated complete consumption of compound A143-1, with the target mass spectrum (94.3%) detected. The reaction solution was concentrated to obtain compound A143-2 (551 mg, crude, 7 TFA) as a brown solid.

Compound A143-2 (551 mg, 0.40 mmol, 1.00 eq, 7 TFA), compound Int 9 (141 mg, 0.48 mmol, 1.20 eq), and DMF (11.0 mL) were added to a 100 mL three-necked round-bottom flask at 25°C. DIEA (520 mg, 4.02 mmol, 0.70 mL, 10.0 eq) and HATU (184 mg, 0.48 mmol, 1.20 eq) were added at 0°C, and the mixture was reacted at 25°C for 16 h. LCMS indicated complete consumption of compound A143-2, with the target mass spectrum (75.3%) detected. The reaction solution was concentrated, and the crude was purified by preparative HPLC to obtain compound A143 (195 mg, 0.24 mmol, yield 58.6%, 98.9% purity) as a milky white solid. LCMS: m/z = 818.2 (M+H)⁺. **¹H NMR:** (400 MHz, DMSO-*d₆*) *δ* 11.79 (s, 1H), 10.35 (s, 1H), 8.87 (s, 1H), 8.43-8.39 (m, 2H), 8.21 (d, J = 7.6 Hz, 1H), 7.95 (s, 1H), 7.78 (d, J = 9.2 Hz, 2H), 7.71 (s, 1H), 7.37-7.34 (m, 1H), 7.32 (d, J = 2.0 Hz, 1H), 7.16 (d, J = 8.8 Hz, 1H), 7.07 (d, J = 9.2 Hz, 2H), 3.84 (s, 3H), 3.60 (t, J = 6.8 Hz, 3H), 3.50-3.49 (m, 5H), 3.19 (s, 4H), 2.69-2.66 (m, 3H), 2.60-2.54 (m, 5H), 2.47-2.36 (m, 4H).

### Preparation Example 228. Synthesis of Compound A144

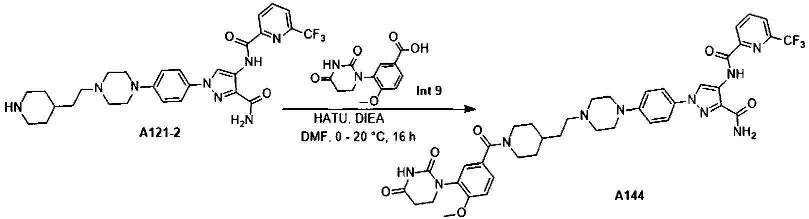

At 20°C, compound A121-2 (600 mg, 0.48 mmol, 1.00 eq, 6 TFA), compound Int 9 (141 mg, 0.48 mmol, 1.20 eq) and DMF (12.0 mL) were added to a 100 mL three-necked round-bottom flask. At 0°C, DIEA (556 mg, 4.30 mmol, 0.75 mL, 9.00 eq) and HATU (218 mg, 0.57 mmol, 1.20 eq) were added and reacted at 20°C for 16 h. LCMS indicated complete consumption of compound A121-2, with the target mass spectrum (69.1%) detected. The reaction solution was concentrated, and the crude was purified by preparative HPLC to obtain compound A144 (227 mg, 0.28 mmol, yield 58.2%, 100% purity) as a yellow solid. LCMS: m/z = 817.3 (M+H)⁺. **¹H NMR:** (400 MHz, DMSO-*d₆*) *δ* 11.79 (s, 1H), 10.34 (s, 1H), 8.87 (s, 1H), 8.44-8.39 (m, 2H), 8.21 (d, J = 7.2 Hz, 1H), 7.79 (s, 1H), 7.78 (d, J = 9.2 Hz, 2H), 7.71 (s, 1H), 7.36 (d, J = 8.4 Hz, 1H), 7.32 (d, J = 2.0 Hz, 1H), 7.15 (d, J = 8.8 Hz, 1H), 7.07 (d, J = 9.2 Hz, 2H), 4.56-4.21 (m, 1H), 3.84 (s, 4H), 3.61-3.58 (m, 2H), 3.21 (s, 4H), 3.12-2.75 (m, 2H), 2.70-2.67 (m, 2H), 2.60-2.51 (m, 4H), 2.38-2.34 (m, 2H), 1.71-1.53 (m, 3H), 1.46-1.43 (m, 2H), 1.14-1.10 (m, 2H).

### Preparation Example 229. Synthesis of Compound A145

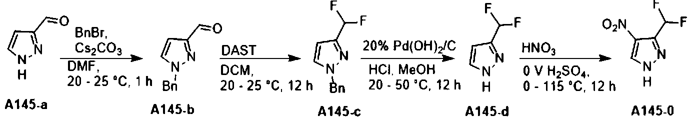

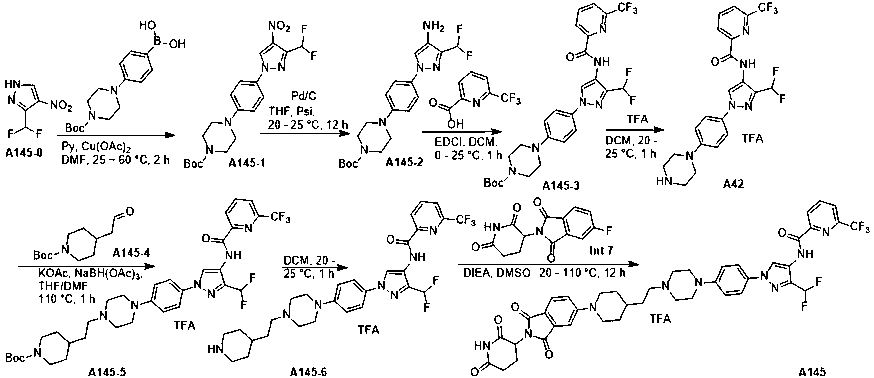

Compound A145-a (43.7 g, 454 mmol, 1.00 eq), Cs₂CO₃ (370 g, 1.14 mol, 2.50 eq), and DMF (440 mL) were added to a 1000 mL single-necked flask at 20°C-25°C. The mixture was purged with N₂ (×3), and then BnBr (81.6 g, 477 mmol, 56.7 mL, 1.05 eq) was added. The mixture was reacted at 20°C-25°C for 1 h. TLC indicated complete consumption of compound A145-a, with a new spot detected. The reaction solution was poured directly into water, extracted with EtOAc, washed with aqueous NaCl, dried over anhydrous Na₂SO₄, filtered, and concentrated to yield compound A145-b (96.2 g, crude) as a yellow oil.

Compound A145-b (96.2 g, 516 mmol, 1.00 eq) and DCM (520 mL) were added to a 1000 mL single-necked flask at 20°C-25°C. The mixture was purged with N₂ (×3), and DAST (333 g, 2.07 mol, 273 mL, 4.00 eq) was added. The mixture was reacted at 20°C-25°C for 12 h. TLC indicated complete consumption of compound A145-b, with a new spot detected. The reaction solution was poured directly into MeOH (600 mL), concentrated directly, and purified by column chromatography (petroleum ether : ethyl acetate = 50 : 1 to 20 : 1) to yield compound A145-c (96.2 g, crude) as a yellow oil.

Pd(OH)₂/C (24.0 g, 34.1 mmol, 20%) was added to a 2.00 L hydrogenation vessel at 20°C-25°C. Under an argon atmosphere, compound A145-c (24.0 g, 114 mmol, 1.00 eq), MeOH (480 mL), and HCl (2 M, 172 mL, 3.00 eq) were added. The mixture was purged with H₂ (×3), and the temperature was raised to 20°C-50°C. The reaction was allowed to proceed for 12 h. LCMS indicated complete consumption of compound A145-c, with a new spot detected. The reaction solution was filtered through Celite, and the filtrate was concentrated to obtain compound A145-d (17.2 g, 105 mmol, yield 92.1%, 95.0% purity, HCl) as a yellow oil.

Compound A145-d (12.7 g, 107 mmol, 1.00 eq) and H₂SO₄ (127 mL) were added to a 500 mL three-necked round-bottom flask at 20°C-25°C. HNO₃ (27.5 g, 284 mmol, 19.7 mL, 65%, 2.65 eq) was added, and the mixture was purged with N₂ (×3) and reacted at 0°C-5°C for 10 min. Then, the resulting mixture was heated to 115°C and reacted for 12 h. TLC indicated complete consumption of compound A145-d, with a new spot detected. The reaction solution was poured into water, and the mixture was adjusted to pH 8 with NaHCO₃, extracted with EtOAc, washed with aqueous NaCl and dried over anhydrous Na₂SO₄. The filtrate was concentrated to obtain compound A145-0 (25.8 g, 158 mmol, yield 73.5%) as a yellow solid.

Compound A145-0 (4.00 g, 24.5 mmol, 1.00 eq), 4-(4-BOC-1-piperazinyl)phenylboronic acid (8.26 g, 26.9 mmol, 1.10 eq), Cu(OAc)₂ (534 mg, 2.94 mmol, 0.12 eq), and DMF (40.0 mL) were added to a 100 mL three-necked round-bottom flask at 20°C-25°C. Pyridine (5.82 g, 73.5 mmol, 5.94 mL, 3.00 eq) was then added. The atmosphere was purged with N₂ (×3), the temperature was raised to 60°C, and the reaction was allowed to proceed for 12 h. LCMS indicated complete consumption of compound A145-0, with the target mass spectrum detected (RT = 0.556 min). The mixture was poured into water (200 mL) and extracted with EtOAc (×3) (200 mL). The combined organic phases were washed with saturated aqueous NaCl (200 mL), dried over Na₂SO₄, filtered, and concentrated to yield compound A145-1 (7.80 g, 15.4 mmol, yield 63.1%, 84.1% purity) as a yellow solid.

Pd/C (1.53 g, 1.43 mmol, 10%) was added to a 1.00 L hydrogenation vessel at 20°C-25°C. Under an Ar atmosphere, compound A145-1 (7.80 g, 15.4 mmol, 1.00 eq) and THF (400 mL) were added. The mixture was purged with H₂ (×3), and reacted at 25°C under H₂ (15 psi) for 12 h. LCMS indicated complete consumption of compound A145-1, with the target mass spectrum detected (RT = 0.428 min). The mixture was purged with Ar (×3), and the reaction solution was filtered. The filter cake was washed with THF (500 mL). The filtrate was collected, concentrated, and dried to afford compound A145-2 (5.62 g, 14.2 mmol, yield 92.2%) as a yellow solid.

Compound A145-2 (3.50 g, 8.90 mmol, 1.00 eq), 6-(trifluoromethyl)pyridine-2-carboxylic acid (1.73 g, 9.07 mmol, 1.02 eq), and DCM (35.0 mL) were added to a 100 mL three-necked round-bottom flask at 20°C-25°C. EDCI (2.05 g, 10.6 mmol, 1.20 eq) was then added at 0°C-5°C, and the mixture was reacted at 0°C for 1 h. LCMS indicated complete consumption of compound A145-2, with the target mass spectrum detected (RT = 0.608 min). The mixture was poured into water (100 mL) and extracted with EtOAc (×3) (100 mL). The combined organic phases were washed with saturated NaHCO₃ solution (100 mL), dried over Na₂SO₄, filtered, and concentrated to yield compound A145-3 (4.80 g, 7.90 mmol, yield 88.7%, 93.2% purity) as a yellow solid.

Compound A145-3 (2.27 g, 3.73 mmol, 1.00 eq) and DCM (60.0 mL) were added to a 250 mL three-necked round-bottom flask at 20°C-25°C. TFA (46.0 g, 403 mmol, 30.0 mL) was then added and the mixture was reacted at 25°C for 1 h. LCMS indicated complete consumption of compound A145-3, with the target mass spectrum detected (RT = 0.397 min). The reaction solution was concentrated to yield compound A42 (4.50 g, 3.26 mmol, yield 87.2%, 91.5% purity, TFA) as a yellow oil.

Compound A42 (1.00 g, 1.96 mmol, 1.00 eq, TFA), compound A145-4 (445 mg, 1.96 mmol, 1.00 eq), KOAc (385 mg, 3.92 mmol, 2.00 eq), THF (200 mL), and DMF (40.0 mL) were added to a 500 mL three-necked round-bottom flask at 20°C-25°C and reacted for 1 h at 25°C. NaBH(OAc)₃ (1.25 g, 5.89 mmol, 3.00 eq) was then added and the reaction continued at 25°C for 1 h. LCMS indicated complete consumption of compound A42, with the target mass spectrum detected (RT = 0.468 min). The reaction solution was concentrated to obtain compound A145-5 (1.13 g, 1.43 mmol, yield 72.6%, 85.5% purity) as a yellow oil.

Compound A145-5 (1.13 g, 1.43 mmol, 1.00 eq) and DCM (30.0 mL) were added to a 100 mL three-necked round-bottom flask at 20°C-25°C. TFA (14.8 g, 130 mmol, 9.66 mL, 91.2 eq) was then added and reacted at 20°C-25°C for 1 h. LCMS indicated complete consumption of compound A145-5 (RT = 0.372 min), with the target mass spectrum detected. The reaction solution was concentrated, poured into water (30.0 mL), and extracted with DCM (×3) (30.0 mL). The combined organic phases were washed with saturated aqueous NaCl (50.0 mL), dried over Na₂SO₄, filtered, and concentrated to yield compound A145-6 (792 mg, 1.14 mmol, yield 79.6%, 82.8% purity, TFA) as a yellow oil.

Compound A145-6 (250 mg, 358 µmol, 1.00 eq, TFA), compound Int 7 (118 mg, 430 µmol, 1.20 eq), and DMSO (5.00 mL) were added to a 40.0 mL parallel reaction flask at 20°C-25°C. DIEA (138 mg, 1.08 mmol, 187 µL, 3.00 eq) was then added. The temperature was raised to 110°C and the mixture was reacted for 12 h. LCMS indicated complete consumption of compound A145-6 (RT = 0.449 min), with the target mass spectrum detected. The reaction solution was concentrated, and the crude was purified by preparative HPLC to obtain compound A145 (53.3 mg, 62.8 µmol, yield 17.5%, 98.2% purity, TFA) as a yellow solid. LCMS: m/z = 834.5 (M+H)⁺. **¹H NMR:** (400 MHz, DMSO-*d₆*) *δ* 11.08 (s, 1H), 10.27 (s, 1H), 8.84 (s, 1H), 8.42-8.40 (m, 2H), 8.24-8.23 (m, 1H), 7.77 (d, J = 8.8 Hz, 2H), 7.67 (d, J = 8.4 Hz, 1H), 7.34 (d, J = 2.0 Hz, 1H), 7.29-7.25 (m, 1H), 7.17 (d, J = 8.8 Hz, 2H), 5.09-5.04 (m, 1H), 4.04 (d, J = 12.4 Hz, 2H), 3.94 (d, J = 12.4 Hz, 2H), 3.62 (d, J = 10.8 Hz, 3H), 3.29-3.21 (m, 4H), 3.16 (d, J = 11.6 Hz, 2H), 3.07-2.98 (m, 3H), 2.96-2.81 (m, 2H), 2.03-2.00 (m, 1H), 1.78 (d, J = 13.2 Hz, 2H), 1.66 (d, J = 3.2 Hz, 3H), 1.27- 1.23 (m, 2H).

### Preparation Example 230. Synthesis of Compound A146

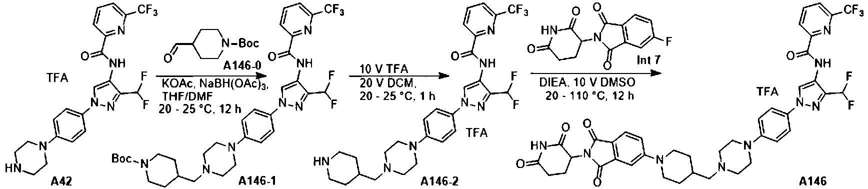

At 20°C-25°C, compound A42 (500 mg, 980 µmol, 1.00 eq), compound A146-0 (209 mg, 980 µmol, 1.00 eq), KOAc (192 mg, 1.96 mmol, 2.00 eq), DMF (20.0 mL), and THF (100 mL) were added to a 25 mL three-necked round-bottom flask. NaBH(OAc)₃ (623 mg, 2.94 mmol, 3.00 eq) was then added at 0°C-5°C. The reaction was allowed to proceed at 25°C for 12 h. LCMS indicated complete consumption of compound A42 (RT = 0.458 min), with the target mass spectrum detected. The reaction solution was concentrated to obtain compound A146-1 (568 mg, 713 µmol, yield 72.7%, 83.4% purity) as a yellow oil.

Compound A146-1 (568 mg, 713.77 µmol, 1.00 eq) and DCM (12.0 mL) were added to a 40.0 mL parallel reaction flask at 20°C-25°C. TFA (7.27 g, 63.7 mmol, 5.68 mL) was then added and the mixture was reacted at 25°C for 1 h. LCMS indicated complete consumption of compound A146-1 (RT = 0.366 min), with the target mass spectrum detected. The reaction solution was concentrated, and the crude was purified by preparative HPLC to obtain compound A146-2 (321 mg, 504 µmol, yield 70.7%, 88.6% purity, TFA) as a yellow solid.

Compound A146-2 (267 mg, 419 µmol, 1.00 eq, TFA), compound Int 7 (139 mg, 503 µmol, 1.20 eq), and DMSO (5.50 mL) were added to a 40.0 mL parallel reaction flask at 20°C-25°C. DIEA (162 mg, 1.26 mmol, 219 µL, 3.00 eq) was then added. The temperature was raised to 110°C and the mixture was reacted for 12 h. LCMS indicated complete consumption of compound A146-2 (RT = 0.440 min), with the target mass spectrum detected. The reaction solution was concentrated, and the crude was purified by preparative HPLC to obtain compound A146 (84.8 mg, 99.0 µmol, yield 23.6%, purity 95.7%, TFA) as a yellow solid. LCMS: m/z = 820.4 (M+H)⁺. **¹H NMR:** (400 MHz, DMSO-*d₆*) *δ* 11.09 (s, 1H), 10.28 (s, 1H), 8.84 (s, 1H), 8.43-8.40 (m, 2H), 8.25-8.22 (m, 1H), 7.77 (d, J = 8.8 Hz, 2H), 7.69 (d, J = 8.8 Hz, 1H), 7.44-7.38 (m, 1H), 7.30-7.28 (m, 1H), 7.18 (d, J = 8.8 Hz, 2H), 5.10-5.05 (m, 1H), 4.12 (d, J = 13.2 Hz, 2H), 3.92 (d, J = 10.4 Hz, 3H), 3.63 (s, 2H), 3.18-3.15 (m, 4H), 2.89-2.86 (m, 1H), 2.61-2.54 (m, 4H), 2.15-2.25 (m, 1H), 2.04-2.01 (m, 1H), 1.87 (d, J = 11.2 Hz, 2H), 1.31-1.23 (m, 3H).

### Preparation Example 231.Synthesis of Compound A147

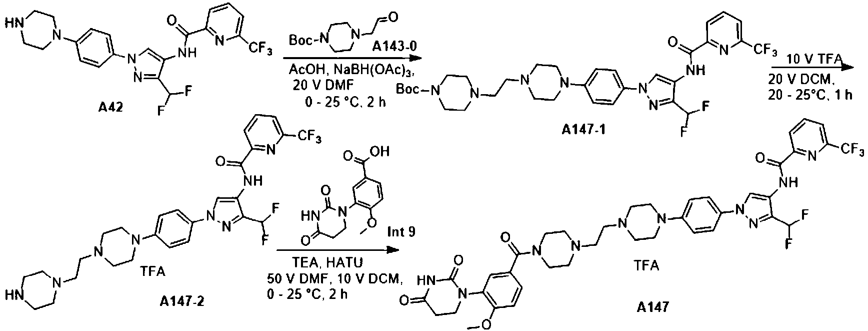

Compound A42 (770 mg, 1.55 mmol, 1.00 eq), compound A143-0 (3.53 g, 15.45 mmol, 10 eq), HOAc (1.86 g, 30.9 mmol, 1.77 mL, 20.0 eq), and DMF (16.0 mL) were added to a 40 mL parallel reaction flask at 20°C-25°C and reacted at 25°C for 1 h. NaBH(OAc)₃ (1.31 g, 6.18 mmol, 4.00 eq) was then added at 0°C and reacted at 25°C for 1 h. LCMS indicated complete consumption of compound A42 (RT = 0.422 min), with the target mass spectrum detected. The reaction solution was concentrated to afford compound A147-1 (665 mg, 836 µmol, yield 54.1%, purity 85.4%) as a yellow solid.

Compound A147-1 (665 mg, 836 µmol, 1.00 eq) and DCM (14.0 mL) were added to a 40 mL parallel reaction flask at 20°C-25°C. TFA (10.2 g, 90.2 mmol, 6.7 mL) was then added and the mixture was reacted at 25°C for 1 h. LCMS indicated complete consumption of compound A147-1 (RT = 0.367 min), with the target mass spectrum detected. The reaction solution was concentrated to afford compound A147-2 (520 mg, 750 µmol, yield 89.7%, pure TFA) as a yellow solid.

At 20°C-25°C, compound Int 9 (198 mg, 750 µmol, 1.00 eq), TEA (227 mg, 2.25 mmol, 313 µL, 3.00 eq), and DCM (10.0 mL) were added to a 100 mL three-necked round-bottom flask (R1). HATU (371 mg, 976 µmol, 1.30 eq) was then added at 0°C-5°C, and the mixture was reacted at 0°C-5°C for 1 h. At 25°C, compound A147-2 (0.52 g, 750 µmol, 1.00 eq, TFA) and DMF (2.00 mL) were added to a 10 mL single-necked flask (R2). The mixed solution in R2 was added to the mixed solution in R1 at 0°C-5°C, and the mixture was reacted at 20°C-25°C for 1 h. LCMS indicated complete consumption of compound A147-2 (RT = 0.397 min), with the target mass spectrum detected. The reaction solution was concentrated, and the crude was purified by preparative HPLC to obtain compound A147 (30.5 mg, 36.4 µmol, 4.86% yield, 98.4% purity, TFA) as a milky white solid. LCMS: m/z = 825.5 (M+H)⁺. **¹H** NMR: (400 MHz, DMSO-*d₆*) *δ* 10.36 (s, 1H), 10.29 (s, 1H), 8.83 (s, 1H), 8.44-8.40 (m, 2H), 8.21 (d, J = 7.0 Hz, 1H), 7.76 (d, J = 9.2 Hz, 2H), 7.43-7.39 (m, 2H), 7.30 (s, 1H), 7.20-7.14 (m, 2H), 3.86 (s, 3H), 3.62-3.59 (m, 17H), 3.33-3.23 (m, 6H), 2.69 (t, J = 6.4 Hz, 2H).

### Preparation Example 232.Synthesis of Compound A148

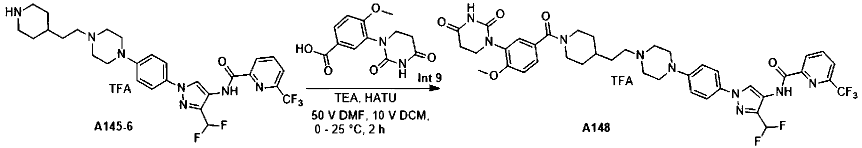

Compound Int 9 (114 mg, 432 µmol, 1.00 eq), TEA (131 mg, 1.30 mmol, 180 µL, 3.00 eq), and DCM (6.00 mL) were added to a 40 ml single-necked flask at 20°C-25°C. HATU (164 mg, 432 µmol, 1.00 eq) was added at 0°C-5°C, and the mixture was stirred at 0°C-5°C for 1 h. Compound A145-6 (301 mg, 432 µmol, 1.00 eq, TFA), TEA (43.8 mg, 432 µmol, 60.2 µL, 3.00 eq), and DMF (10.0 mL) were added to a parallel reaction flask at 0°C-5°C. The reaction was stirred at 25°C for 1 h. LCMS indicated complete consumption of compound A145-6, with the target mass spectrum detected. The reaction solution was concentrated, and the crude was purified by preparative HPLC and lyophilized to afford compound A148 (83.3 mg, 99.3 µmol, yield 22.9%, purity 98.1%, TFA) as a white solid. LCMS: m/z = 824.4 (M+H)⁺. **¹H NMR:** (400 MHz, DMSO-*d₆*) *δ* 10.35 (s, 1H), 10.28 (s, 1H), 8.84 (s, 1H), 8.44-8.40 (m, 2H), 8.24 (d, J = 6.8, 1H), 7.77 (d, J = 8.8 Hz, 2H), 7.39-7.36 (m, 1H), 7.33-7.30 (m, 1H), 7.18-7.15 (m, 3H), 3.95 (d, J = 12, 2H), 3.85 (s, 3H), 3.63-3.58 (m, 4H), 3.37-3.35 (m, 2H), 3.24-3.22 (m, 5H), 3.21-3.03 (m, 3H), 2.70-2.67 (m, 3H), 1.68-1.64 (m, 5H), 1.21-1.12 (m, 2H).

### Preparation Example 233.Synthesis of Compound A149

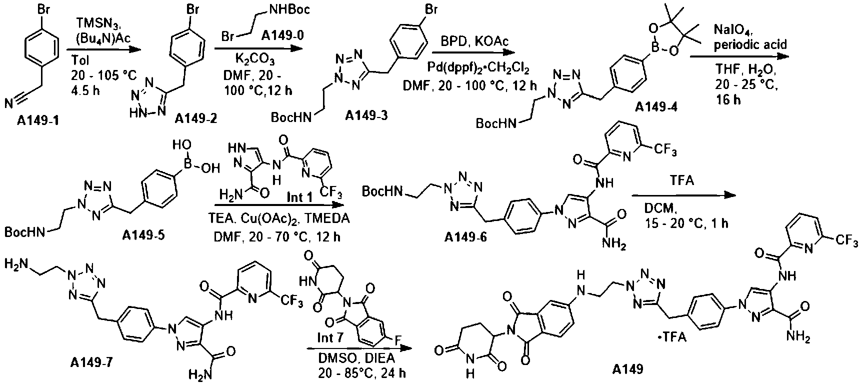

At 20°C-25°C, toluene (50.0 mL) was added to a 250 ml three-necked flask. Compound A149-1 was added to the reaction flask. (Bu₄N)Ac (11.5 g, 38.2 mmol, 11.6 mL, 1.50 eq) was added to the reaction flask. The mixture was purged with N₂ (×3). At 20°C-25°C, TMSN₃ (5.88 g, 51.0 mmol, 6.71 mL, 2.00 eq) was added to the reaction flask under N₂. The mixture was stirred at 20°C-25°C for 0.5 h. The mixture was heated to 105°C and stirred for 4 h. LCMS indicated complete consumption of compound A149-1, with the target product detected. The reaction solution was cooled to 20°C-25°C, and poured into 200 mL of ice water under N₂. The mixture was extracted with EtOAc (×3), and the organic phase was collected and washed with 300 ml of aqueous NaCl (×3). The organic phase was dried over anhydrous Na₂SO₄, filtered, and concentrated in vacuo at 35°C to yield compound A149-2 (6.11 g, 25.1 mmol, yield 98.4%, purity 98.2%).

Compound A149-2 (6.11 g, 25.1 mmol, 1.00 eq), compound A149-0 (8.44 g, 37.6 mmol, 1.50 eq), K₂CO₃ (10.4 g, 75.2 mmol, 3.00 eq), and DMF (60.0 mL) were added to a 100 ml three-necked flask at 20°C-25°C. The mixture was heated to 100°C and stirred for 12 h. LCMS indicated complete consumption of compound A149-2, with the target product detected. The reaction solution was cooled to 20°C-25°C and poured into 0°C-5°C water under N₂. The reaction solution was extracted (×3) with 200 ml of ethyl acetate, and the organic phase was collected, washed (×3) with 300 ml of aqueous NaCl, dried over anhydrous Na₂SO₄ and filtered. The organic phase was concentrated in vacuo at 45°C to obtain the crude. The crude was purified by preparative HPLC and lyophilized to obtain compound A149-3 (5.80 g, 15.1 mmol, yield 60.4%, purity 100%).

At 20°C under N₂, compound A149-3 (3.10 g, 8.11 mmol, 1.00 eq), BPD (3.09 g, 12.1 mmol, 1.50 eq), KOAc (2.39 g, 24.3 mmol, 3.00 eq), Pd(dppf)₂•CH₂Cl₂ (1.32 g, 1.62 mmol, 0.20 eq), and DMF (20.0 mL) were added to a 250 ml three-necked reaction flask. The mixture was heated to 100°C and stirred for 12 h. LCMS indicated complete consumption of compound A149-3, with the target product detected. 100 ml of water was added to the reaction, and the reaction solution was extracted (×3) with 200 ml of ethyl acetate. The organic phases were combined and washed (×3) with 100 ml of aqueous NaCl. The combined organic phases were dried over anhydrous Na₂SO₄, filtered, and concentrated in vacuo at 40°C to yield compound A149-4 (3.70 g, 7.64 mmol, yield 94.1%, purity 88.6%).

Compound A149-4 (3.70 g, 7.64 mmol, 1.00 eq) was added to a 100 ml three-necked flask at 20°C-25°C. NaIO₄ (3.27 g, 15.2 mmol, 846 µL, 2.00 eq), periodic acid (222 mg, 977 µmol, 222 µL, 0.12 eq), water (18.5 mL), and THF (37.0 mL) were added to the reaction flask at 20°C-25°C. The mixture was stirred at 20°C-25°C for 16 h. LCMS indicated complete consumption of compound A149-4, with the target product detected. A 10 M aqueous NaOH solution (20.0 ml) was slowly added to the reaction solution to adjust the pH to 7.0. A saturated aqueous Na₂SO₃ solution (100 ml) was added at 0°C-5°C. The mixture was extracted with EtOAc (×3) (300 mL), and the organic phase was washed with aqueous NaCl (×1) (100 mL), dried over anhydrous Na₂SO₄, and concentrated. The crude was purified by preparative HPLC (TFA system). The fraction was extracted with EtOAc (×3) (100 mL), and the organic phase was washed once with 300 ml of aqueous NaCl, dried over anhydrous Na₂SO₄, and concentrated to obtain compound A149-5 (1.40 g, 3.83 mmol, yield 50.1%, purity 94.9%).

At 20°C-25°C, compound A149-5 (1.40 g, 3.83 mmol, 1.00 eq), compound Int 1 (2.38 g, 7.65 mmol, 2.00 eq), Cu(OAc)₂ (347 mg, 1.91 mmol, 0.50 eq), TEA (1.16 g, 11.4 mmol, 1.60 mL, 3.00 eq), TMEDA (444 mg, 3.83 mmol, 577 µL, 1.00 eq), and DMF (10.0 mL) were added to a 100 ml single-necked reaction flask. The mixture was heated to 70°C and stirred for 12 h. LCMS indicated 16.4% of compound A149-5 remained, with the target product detected. The reaction solution was concentrated to obtain a crude, which was then purified by preparative HPLC. The fraction was extracted (×3) with 200 ml of ethyl acetate. The organic phase was washed (×3) with 200 ml of aqueous NaCl, dried over anhydrous Na₂SO₄, and concentrated to yield compound A149-6 (320 mg, 452 µmol, yield 11.8%, purity 84.9%).

Compound A149-6 (320 mg, 452 µmol, 1.00 eq), TFA (1.03 g, 9.05 mmol, 672 µL, 20.0 eq), and DCM (1.00 mL) were added to a 100 ml three-necked flask at 20°C-25°C. The mixture was stirred at 20°C-25°C for 1 h. LCMS indicated complete consumption of compound A149-6, with the target product detected. The reaction solution was concentrated and then added to 5.00 ml of saturated aqueous NaHCO₃ solution. The mixture was extracted (×3) with 20 ml of ethyl acetate. The organic phase was washed (×3) with 20 ml of aqueous NaCl, dried over anhydrous Na₂SO₄, and concentrated to yield compound A149-7 (210 mg, 328 µmol, yield 72.7%, purity 78.4%).

Compound A149-7 (150 mg, 235 µmol, 1.00 eq), compound Int 7 (77.8 mg, 281 µmol, 1.20 eq), DIEA (91.1 mg, 704 µmol, 122 µL, 3.00 eq), and DMSO (1.00 mL) were added to a 100 ml single-necked flask at 20°C-25°C. The mixture was heated to 85°C and stirred for 24 h. LCMS indicated 14.1% of compound A149-7 remained, with the target product detected. The reaction solution was concentrated and purified by preparative HPLC, and the fraction was lyophilized to obtain compound A149 (23.7 mg, 29.9 µmol, yield 12.7%, purity 95.7%). LCMS: m/z = 757.0 (M+H)⁺.**¹H NMR:** (400 MHz, DMSO-*d₆*) *δ* 11.79 (s, 1H), 11.05 (s, 1H), 8.99 (s, 1H), 8.46-8.40 (m, 2H), 8.21 (d, J = 7.2 Hz, 1H), 8.00 (s, 1H), 7.91 (d, J = 8.4 Hz, 2H), 7.79 (s, 1H), 7.53 (d, J = 8.0 Hz, 1H), 7.41 (d, J = 8.4 Hz, 2H), 7.22 (t, J = 5.6 Hz, 1H), 6.95 (s, 1H), 6.95-6.84 (m, 1H), 5.07-5.02 (m, 1H), 4.82 (t, J = 5.6 Hz, 2H), 4.29 (s, 2H), 3.85 (d, J = 4.4 Hz, 2H), 2.87-2.85 (m, 1H), 2.59-2.54 (m, 2H), 2.00-1.96 (m, 1H).

### Preparation Example 234. Synthesis of Compound A150

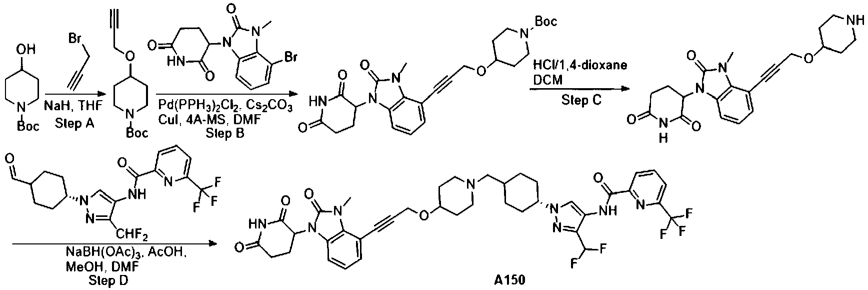

### Step A: Tert-butyl 4-(prop-2-yn-1-yloxy)piperidine-1-carboxylate

At 0°C, to tert-butyl 4-hydroxypiperidine-1-carboxylate (4 g, 19.9 mmol, 1.0 eq) in THF (50 mL) was added sodium hydride (1.0 g, 23.8 mmol, purity 60%). The reaction solution was stirred at 0°C under N₂ for 0.5 h. 3-Bromoprop-1-yne (2.4 g, 19.9 mmol, 1.0 eq) was then added, and the reaction was stirred at rt for 16 h. Upon completion, the reaction was quenched with saturated aqueous NH₄Cl and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow oil, 3 g, yield 63%).

### Step B: Tert-butyl 4-((3-(1-(2,6-dioxypiperidin-3-yl)-3-methyl-2-oxo-2,3-dihydro-1H-benzo[d]imidazol-4-yl)prop-2-yn-1-yl)oxy)piperidine-1-carboxylate

To a solution of 3-(4-bromo-3-methyl-2-oxo-2,3-dihydro-1H-benzo[d]imidazol-1-yl)piperidine-2,6-dione (1 g, 3.0 mmol, 1.0 eq) in DMF (10 mL) at rt were added tert-butyl 4-(prop-2-yn-1-yloxy)piperidine-1-carboxylate (1.1 g, 4.4 mmol, 1.5 eq), Pd(PPh₃)₂Cl₂ (210 mg, 0.3 mmol, 0.1 eq), cuprous iodide (110 mg, 0.6 mmol, 0.2 eq), cesium carbonate (2.9 g, 8.9 mmol, 3.0 eq), and 4A molecular sieves (400 mg). The reaction solution was stirred at 90°C under N₂ for 2 h. Upon completion, the reaction solution was quenched with water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow oil, 600 mg, yield 41%).

### Step C: 3-(3-Methyl-2-oxo-4-(3-(piperidin-4-yloxy)prop-1-yn-1-yl)-2,3-dihydro-1H-benzo[d]imidazol-1-yl)piperidine-2,6-dione

To a solution of tert-butyl 4-((3-(1-(2,6-dioxypiperidin-3-yl)-3-methyl-2-oxo-2,3-dihydro-1H-benzo[d]imidazol-4-yl)prop-2-yn-1-yl)oxy)piperidine-1-carboxylate (300 mg, 0.604 mmol, 1.0 eq) in DCM (5 mL) was added HCl/1,4-dioxane (5 mL, 4 M) at 0°C. The reaction solution was stirred at rt for 1 h. Upon completion, the reaction solution was concentrated under reduced pressure to afford the title compound (yellow solid, 220 mg, yield 92%, crude).

### Step D: N-(3-(difluoromethyl)-1-((1r,4r)-4-(4-((3-(1-(2,6-dioxopiperidin-3-yl)-3-methyl-2-oxo-2,3-dihydro-1H-benzo[d]imidazol-4-yl)prop-2-yn-1-yl)oxy)piperidin-1-yl)methyl)cyclohexyl)-1H-pyrazol-4-yl)-6-(trifluoromethyl)picolinamide

To a solution of 3-(3-methyl-2-oxo-4-(3-(piperidin-4-yloxy) prop-1-yn-1-yl)-2,3-dihydro-1H-benzo[d]imidazol-1-yl)piperidine-2,6-dione (300 mg, 0.8 mmol, 1.0 eq) in DMF (2.0 mL) and MeOH (2.0 mL) at rt were added N-(3-(difluoromethyl)-1-((1r,4r)-4-formylcyclohexyl)-1H-pyrazol-4-yl)-6-(trifluoromethyl)picolinamide (315 mg, 0.8 mmol, 1.0 eq) and AcOH (0.02 mL). The reaction solution was stirred at rt for 0.5 h. Then, sodium triacetoxyborohydride (481 mg, 2.3 mmol, 3.0 eq) was then added, and the reaction solution was stirred at rt for 1 h. Upon completion, the reaction solution was quenched with water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (white solid, 25 mg, yield 4%). ¹HNMR (400 MHz, DMSO-*d₆*) δ 11.12 (s, 1H), 10.10 (s, 1H), 8.42-8.35 (m, 2H), 8.32 (s, 1H), 8.23-8.15 (m, 1H), 7.30-7.01 (m, 4H), 5.42-5.34 (m, 1H), 4.47 (s, 2H), 4.24-4.17 (m, 1H), 3.64 (s, 3H), 3.59-3.54 (m, 1H), 2.94-2.84 (m, 1H), 2.76-2.60 (m, 4H), 2.16-1.99 (m, 7H), 1.91-1.84 (m, 4H), 1.82-1.70 (m, 2H), 1.63-1.54 (m, 1H), 1.54-1.42 (m, 2H), 1.10-0.96 (m, 2H). LCMS (ESI): m/z 797 [M+H]⁺.

### Preparation Example 235. Synthesis of Compound A151

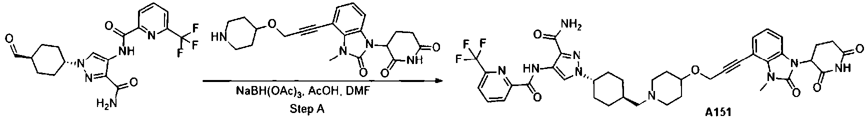

### Step A: N-(3-carbonyl-1-((1r,4r)-4-((4-((3-(1-(2,6-dioxopiperidin-3-yl)-3-methyl-2-oxo-2,3-dihydro-1H-benzo[d]imidazol-4-yl)prop-2-yn-1-yl)oxy)piperidin-1-yl)methyl)cyclohexyl)-1H-pyrazol-4-yl)-6-(trifluoromethyl)picolinamide

To a solution of 3-(3-methyl-2-oxo-4-(3-(piperidin-4-yloxy) prop-1-yn-1-yl)-2,3-dihydro-1H-benzo[d]imidazol-1-yl)piperidine-2,6-dione (300 mg, 0.8 mmol, 1.0 eq) in DMF (5.0 mL) at rt were added N-(3-amino-1-((1r,4r)-4-formylcyclohexyl)-1H-pyrazol-4-yl)-6-(trifluoromethyl)picolinamide (310 mg, 0.8 mmol, 1.0 eq) and AcOH (0.02 mL). The reaction solution was stirred at rt for 0.5 h. Then, sodium triacetoxyborohydride (481 mg, 2.3 mmol, 3.0 eq) was added, and the reaction solution was stirred at rt for 1 h. Upon completion, the reaction solution was quenched with water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (yellow solid, 30 mg, yield 5%). ¹HNMR (400 MHz, DMSO-*d₆*) δ 11.71 (s, 1H), 11.13 (s, 1H), 8.46-8.42 (m, 1H), 8.42-8.34 (m, 2H), 8.19 (d, *J* = 7.4 Hz, 1H), 7.63 (s, 1H), 7.55 (s, 1H), 7.18 (d, *J* = 7.7 Hz, 1H), 7.12 (d, *J* = 7.7 Hz, 1H), 7.06-7.01 (m, 1H), 5.45-5.36 (m, 1H), 4.49 (s, 2H), 4.28-4.18 (m, 1H), 3.65 (s, 3H), 2.94-2.85 (m, 1H), 2.79-2.72 (m, 2H), 2.71-2.60 (m, 2H), 2.28-2.16 (m, 4H), 2.14-1.98 (m, 4H), 1.96-1.88 (m, 4H), 1.85-1.76 (m, 2H), 1.66-1.51 (m, 3H), 1.13-1.01 (m, 2H). LCMS (ESI): m/z 790 [M+H]⁺.

### Preparation Example 236. Synthesis of Compound A152

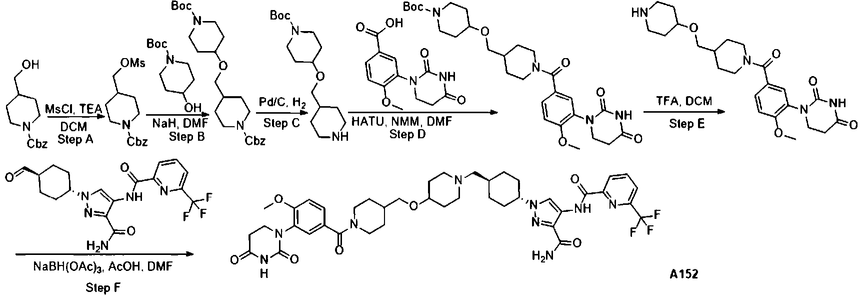

### Step A: Benzyl 4-(((methylsulfonyl)oxy)methyl)piperidine-1-carboxylate

To a solution of benzyl 4-(hydroxymethyl)piperidine-1-carboxylate (5 g, 20 mmol, 1.0 eq) in DCM (60 mL) at 0°C were added TEA (4.2 mL, 30 mmol, 1.5 eq) and methanesulfonyl chloride (2.8 g, 24 mmol, 1.2 eq). The reaction solution was stirred at 0°C under N₂ for 0.5 h. Upon completion, the reaction was quenched with water and extracted with DCM (×3). The combined organic phases were washed with water and saturated aqueous NaCl, dried over anhydrous Na₂SO₄, and concentrated under reduced pressure to afford the title compound (yellow oil, 6 g, crude), which was used directly without purification.

### Step B: Benzyl 4-(((1-(tert-Butoxycarbonyl)piperidin-4-yl)oxy)methyl)piperidine-1-carboxylate

At 0°C, to a solution of tert-butyl 4-hydroxypiperidine-1-carboxylate (2 g, 10.1 mmol, 1.0 eq) in DMF (50 mL) was added portionwise 60 wt% sodium hydride (1 g, 25.2 mmol, 2.5 eq). The mixture was stirred at rt for 0.5 h. Benzyl 4-(((methylsulfonyl)oxy)methyl)piperidine-1-carboxylate (3.3 g, 10.1 mmol, 1.0 eq) was then added to the above reaction solution. The mixture was stirred at 60°C under N₂ for 4.5 h. Upon completion, the reaction was quenched with saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow liquid, 500 mg, yield 11%).

### Step C: Tert-butyl 4-(piperidin-4-ylmethoxy)piperidine-1-carboxylate

To a solution of benzyl 4-(((1-(tert-butoxycarbonyl)piperidin-4-yl)oxy)methyl)piperidine-1-carboxylate (280 mg, 0.6 mmol, 1.0 eq) in MeOH (5 mL) was added 10% palladium on carbon (50 mg, 0.75 mmol, 1.0 eq) at rt. The reaction solution was purged with H₂ (using a hydrogen balloon) (×3) and stirred under H₂ at rt for 2 h. Upon completion, the mixture was filtered, and the filtrate was concentrated under reduced pressure to afford the title compound (yellow oil, 150 mg, crude), which was used directly without purification.

### Step D: Tert-butyl 4-((1-(3-(2,4-dioxotetrahydropyrimidin-1(2H)-yl)-4-methoxybenzoyl)piperidin-4-yl)methoxy)piperidine-1-carboxylate

To a solution of tert-butyl 4-(piperidin-4-ylmethoxy)piperidine-1-carboxylate (150 mg, 0.5 mmol, 1.0 eq) and 3-(2,4-dioxohexahydropyrimidin-1-yl)-4-methoxybenzoic acid (150 mg, 0.6 mmol, 1.2 eq) in DMF (3 mL) were added N-methylmorpholine (277 µL, 2.5 mmol, 5.0 eq) and HATU (287 mg, 0.8 mmol, 1.5 eq) at rt. The reaction solution was stirred at rt under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 150 mg, yield 55%).

### Step E: 1-(2-methoxy-5-(4-(piperidin-4-yloxy)methyl)piperidine-1-carbonyl)phenyl)dihydropyrimidine-2,4(1H,3H)-dione

To a solution of tert-butyl 4-((1-(3-(2,4-dioxotetrahydropyrimidin-1(2H)-yl)-4-methoxybenzoyl)piperidin-4-yl)methoxy)piperidine-1-carboxylate (250 mg, 0.5 mmol, 1.0 eq) in DCM (3 mL) was added TFA (3 mL) at 0°C. The reaction solution was stirred at rt for 1 h. Upon completion, the mixture was concentrated under reduced pressure to afford the title compound (yellow solid, 200 mg, crude), which was used directly without purification.

### Step F: N-(3-carbonyl-1-((1r,4r)-4-((4-((1-(3-(2,4-dioxotetrahydropyrimidin-1(2H)-yl)-4-methoxybenzoyl)piperidin-4-yl)methoxy)piperidin-1-yl)methyl)cyclohexyl)-1H-pyrazol-4-yl)-6-(trifluoromethyl)picolinamide

To a solution of 1-(2-methoxy-5-(4-(piperidin-4-yloxy)methyl)piperidine-1-carbonyl)phenyl)dihydropyrimidine-2,4(1H,3H)-dione (217 mg, 0.5 mmol, 1.0 eq) in DMF (2 mL) at rt was added TEA (0.2 mL). The reaction solution was stirred at rt for 5 min. N-(3-carbamoyl-1-((1R,4R)-4-formylcyclohexyl)-1H-pyrazol-4-yl)-6-(trifluoromethyl)picolinamide (200 mg, 0.5 mmol, 1.0 eq) and AcOH (0.3 mL) were added sequentially to the above reaction solution. The reaction solution was stirred at rt for 15 h. Sodium triacetoxyborohydride (155 mg, 0.7 mmol, 1.5 eq) was then added. The reaction solution was stirred at rt for 1 additional hour. Upon completion, the reaction was quenched with water and extracted with EtOAc (×3). The mixture was concentrated under reduced pressure, and the residue was purified by HPLC to afford the title compound (white solid, 27.3 mg, yield 7%). **¹H NMR** (400 MHz, DMSO-*d*₆) δ 11.71 (s, 1H), 10.33 (s, 1H), 8.43-8.35 (m, 3H), 8.18 (dd, *J =* 7.4, 1.2 Hz, 1H), 8.15 (s, 1H), 7.63 (s, 1H), 7.54 (s, 1H), 7.36 (dd, *J* = 8.4, 2.0 Hz, 1H), 7.32 (d, *J* = 2.0 Hz, 1H), 7.15 (d, *J* = 8.8 Hz, 1H), 4.27-4.16 (m, 1H), 3.84 (s, 3H), 3.59 (t, *J =* 6.4 Hz, 2H), 3.28-3.23 (m, 7H), 2.67 (td, *J* = 10.6, 5.2 Hz, 4H), 2.14-2.03 (m, 6H), 1.94-1.87 (m, 2H), 1.83-1.78 (m, 4H), 1.73-1.64 (m, 2H), 1.62-1.53 (m, 2H), 1.42 (dt, *J =* 14.4, 7.2 Hz, 2H), 1.17-1.00 (m, 4H). LCMS (ESI): m/z 838 [M+H]⁺.

### Preparation Example 237. Synthesis of Compound A153

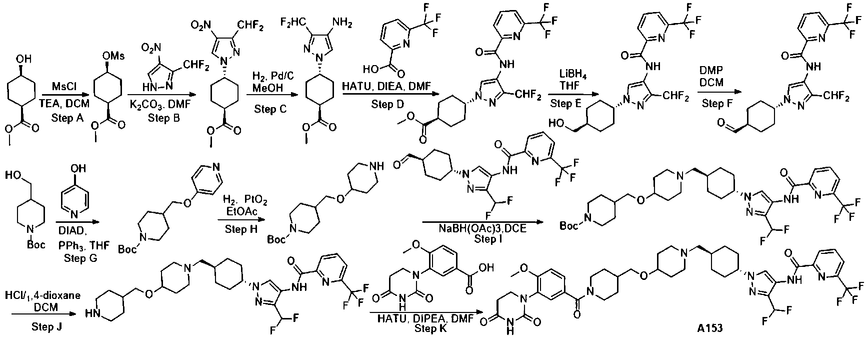

### Step A: Methyl (1s,4s)-4-((methylsulfonyl)oxy) cyclohexane-1-carboxylate

To a solution of methyl (1s,4s)-4-hydroxycyclohexane-1-carboxylate (20 g, 126.6 mmol, 1.0 eq) and TEA (25.62 g, 253.2 mmol, 2.0 eq) in DCM (200 mL) was added a solution of MsCl (17.4 g, 151.92 mmol, 1.2 eq) in DCM (20 mL) at 0°C. The reaction solution was reacted at rt for 2 h under N₂. Upon completion, the reaction solution was poured into NaHCO₃ solution and extracted with DCM (×3). The combined organic phases were washed with saturated aqueous NaCl, dried over anhydrous Na₂SO₄, filtered, and concentrated under reduced pressure to afford the title compound (yellow oil, 25 g, crude), which was used directly without purification.

### Step B: Methyl (1r,4r)-4-(3-(difluoromethyl)-4-nitro-1H-pyrazol-1-yl)cyclohexane-1-carboxylate

To a solution of 3-(difluoromethyl)-4-nitro-1H-pyrazole (5 g, 30.7 mmol, 1.0 eq) and methyl (1s,4s)-4-((methylsulfonyl)oxy) cyclohexane-1-carboxylate (8.7 g, 36.8 mmol, 1.2 eq) in DMF (50 mL) was added potassium carbonate (8.5 g, 61.4 mmol, 2.0 eq) at rt. The reaction solution was reacted under N₂ at 80°C for 16 h. Upon completion, the reaction solution was poured into ice water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 2.5 g, yield 27%).

### Step C: Methyl (1r,4r)-4-(4-amino-3-(difluoromethyl)-1H-pyrazol-1-yl)cyclohexane-1-carboxylate

To a solution of methyl (1r,4r)-4-(3-(difluoromethyl)-4-nitro-1H-pyrazol-1-yl)cyclohexane-1-carboxylate (2.5 g, 8.3 mmol, 1.0 eq) in MeOH (30 mL) was added 10% palladium on carbon (250 mg, 0.1 mmol, 10 wt%) at rt. The reaction solution was purged with H₂ (×3) and then reacted at 25°C under H₂ for 4 h. Upon completion, the reaction solution was filtered and washed twice with MeOH. The filtrate was concentrated under reduced pressure to afford the title compound (yellow solid, 2.1 g, yield 93%).

### Step D: Methyl (1r,4r)-4-(3-(difluoromethyl)-4-(6-(trifluoromethyl)picolinamido)-1H-pyrazol-1-yl)cyclohexane-1-carboxylate

To a solution of methyl (1r,4r)-4-(4-amino-3-(difluoromethyl)-1H-pyrazol-1-yl)cyclohexane-1-carboxylate (1.6 g, 5.9 mmol, 1.0 eq) and 6-(trifluoromethyl)picolinic acid (1.12 g, 5.9 mmol, 1.0 eq) in DMF (20 mL) were added DIEA (1.19 g, 11.8 mmol, 2 eq) and HATU (3.36 g, 8.8 mmol, 1.5 eq) at 0°C. The reaction solution was stirred at rt for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 2.1 g, yield 81%).

### Step E: N-(3-(difluoromethyl)-1-((1r,4r)-4-(hydroxymethyl)cyclohexyl)-1H-pyrazol-4-yl)-6-(trifluoromethyl)picolinamide

To a solution of methyl (1r,4r)-4-(3-(difluoromethyl)-4-(6-(trifluoromethyl)picolinamido)-1H-pyrazol-1-yl)cyclohexane-1-carboxylate (2.1 g, 4.7 mmol, 1.0 eq) in THF (20 mL) was added lithium borohydride (4.7 mL, 9.4 mmol, 2 M in THF) at 0°C under N₂. The mixture was stirred at rt for 2 h. Upon completion, the reaction solution was poured into aqueous citric acid solution and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (pale yellow solid, 1.2 g, yield 61%).

### Step F: N-(3-(difluoromethyl)-1-((1r,4r)-4-formylcyclohexyl)-1H-pyrazol-4-yl)-6-(trifluoromethyl)picolinamide

To a solution of N-(3-(difluoromethyl)-1-((1r,4r)-4-(hydroxymethyl)cyclohexyl)-1H-pyrazol-4-yl)-6-(trifluoromethyl)picolinamide (1.2 g, 2.9 mmol, 1.0 eq) in DCM (12 mL) was added DMP (2.46 g, 5.8 mmol, 2.0 eq) at rt. The reaction solution was stirred at rt for 12 h. Upon completion, the reaction solution was poured into aqueous NaHCO₃ and extracted with EtOAc (×3). The combined organic phases were washed with saturated aqueous NaCl, dried over anhydrous Na₂SO₄, filtered, and concentrated under reduced pressure to afford the title compound (colorless oil, 1.0 g, crude), which was used directly without purification.

### Step G: Tert-butyl 4-(pyridin-4-yloxy)methylpiperidine-1-carboxylate

To a solution of tert-butyl 4-(hydroxymethyl)piperidinecarboxylate (5 g, 23.2 mmol, 1.0 eq) in THF (50 mL) at rt were added pyridin-4-ol (2.21 g, 23.2 mmol, 1.0 eq), triphenylphosphine (12.5 g, 46.5 mmol, 2.0 eq), and DIAD (9.5 g, 46.5 mmol, 9.0 mL, 2.0 eq). The reaction solution was stirred at 25°C under N₂ for 16 h. Upon completion, the reaction solution was quenched with water and extracted with DCM (×3). The combined organic phases were washed with saturated aqueous NaCl, dried over anhydrous Na₂SO₄, filtered, and concentrated under reduced pressure. The residue was purified by reverse-phase column chromatography to afford the title compound (white solid, 2.0 g, yield 30%).

### Step H: Tert-butyl 4-(piperidin-4-yloxy)methylpiperidine-1-carboxylate

To a solution of tert-butyl 4-(pyridin-4-yloxy)methylpiperidine-1-carboxylate (2 g, 6.84 mmol, 1.0 eq) in ethanol (20 mL) was added platinum dioxide (1.55 g, 6.84 mmol, 1.0 eq) at rt. The reaction solution was purged with H₂ (×3) and then stirred at 40°C under H₂ for 16 h. Upon completion, the reaction solution was filtered, and the filtrate was concentrated under reduced pressure. NaHCO₃ solution was added to the residue, and the mixture was extracted with EtOAc (×3). The combined organic phases were washed with saturated aqueous NaCl, dried over anhydrous Na₂SO₄, filtered, and concentrated under reduced pressure to afford the title compound (colorless oil, 600 mg, yield 30%).

### Step I: Tert-butyl 4-(((1-(((1r,4r)-4-(3-(difluoromethyl)-4-(6-(trifluoromethyl)picolinamido)-1H-pyrazol-1-yl)cyclohexyl)methyl)piperidin-4-yl)oxy)methyl)piperidine-1-carboxylate

To a solution of N-(3-(difluoromethyl)-1-((1r,4r)-4-formylcyclohexyl)-1H-pyrazol-4-yl)-6-(trifluoromethyl)picolinamide (100 mg, 0.24 mmol, 1.0 eq) in dichloroethane (2.0 mL) were added tert-butyl 4-(piperidin-4-yloxy)methylpiperidine-1-carboxylate (86 mg, 0.29 mmol, 1.2 eq) and AcOH (0.02 mL) at rt. The reaction solution was reacted at rt for 0.5 h. Then, sodium triacetoxyborohydride (153 mg, 0.72 mmol, 3.0 eq) was added, and the reaction solution was stirred at rt for 1 h. Upon completion, the reaction solution was added to an aqueous solution and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 90 mg, yield 54%).

### Step J: N-(3-(difluoromethyl)-1-((1r,4r)-4-((4-(piperidin-4-ylmethoxy)piperidin-1-yl)methyl)cyclohexyl)-1H-pyrazol-4-yl)-6-(trifluoromethyl)picolinamide

To a solution of tert-butyl 4-(((1-(((1r,4r)-4-(3-(difluoromethyl)-4-(6-(trifluoromethyl)picolinamido)-1H-pyrazol-1-yl)cyclohexyl)methyl)piperidin-4-yl)oxy)methyl)piperidine-1-carboxylate (100 mg, 0.24 mmol, 1.0 eq) in 1,4-dioxane (2.0 mL) was added HCl/1,4-dioxane (1 mL, 4 M) at rt. The reaction solution was reacted at rt for 1 h. Upon completion, NaHCO₃ solution was added, and the mixture was extracted with EtOAc (×3). The combined organic phases were washed with saturated aqueous NaCl, dried over anhydrous Na₂SO₄, filtered, and concentrated under reduced pressure to afford the title compound (brown solid, 75 mg, yield 97%). **Step K: N-(3-(difluoromethyl)-1-((1r,4r)-4-(4-((1-(3-(2,4-dioxotetrahydropyrimidin-1(2H)-yl)-4-methoxybenzoyl)piperidin-4-yl)methoxy)piperidin-1-yl)methyl)cyclohexyl)-1H-pyrazol-4-yl)-6-(trifluoromethyl)picolinamide**

At 0°C, to a solution of N-(3-(difluoromethyl)-1-((1r,4r)-4-((4-(piperidin-4-ylmethoxy)piperidin-1-yl)methyl)cyclohexyl)-1H-pyrazol-4-yl)-6-(trifluoromethyl)picolinamide (75 mg, 0.13 mmol, 1.0 eq) and 3-(2,4-dioxotetrahydropyrimidin-1(2H)-yl)-4-methoxybenzoic acid (34 mg, 0.13 mmol, 1.0 eq) in DMF (2.0 mL) were added DIPEA (50.3 mg, 0.39 mmol, 3 eq) and HATU (74 mg, 0.20 mmol, 1.5 eq). The reaction solution was reacted at rt for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (white solid, 16 mg, yield 15%).

### Preparation Example 238. Synthesis of Compound A154

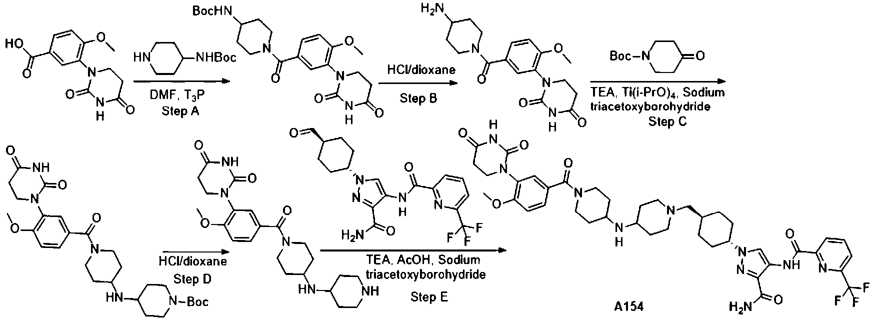

### Step A: Tert-butyl (1-(3-(2,4-dioxotetrahydropyrimidin-1(2H)-yl)-4-methoxybenzoyl)piperidin-4-yl)carbamate

To a solution of 3-(2,4-dioxotetrahydropyrimidin-1(2H)-yl)-4-methoxybenzoic acid (300 mg, 1.14 mmol, 1.03 eq) and tert-butyl piperidin-4-ylcarbamate (227 mg, 1.1 mmol, 1.0 eq) in DMF (5 mL) were added 50 wt% T₃P ethyl acetate solution (722 mg, 2.2 mmol, 2.0 eq) and DIEA (440 mg, 3.4 mmol, 3.0 eq) at rt. The mixture was stirred at rt for 2 h. Upon completion, the mixture was diluted in water and extracted with EtOAc (×3). The combined organic phases were washed with water and saturated aqueous NaCl, and dried over anhydrous Na₂SO₄. The residue was purified by column chromatography to afford the title compound (white solid, 360 mg, yield 71%).

### Step B: 1-5-(4-aminopiperidine-1-carbonyl)-2-methoxyphenyl)dihydropyrimidine-2,4(1H,3H)-dione

To tert-butyl (1-(3-(2,4-dioxotetrahydropyrimidin-1(2H)-yl)-4-methoxybenzoyl)piperidin-4-yl)carbamate (100 mg, 0.22 mmol, 1.0 eq) was added 4 M HCl in 1,4-dioxane (5 mL) at 0°C. The reaction solution was stirred at rt under N₂ for 2 h. The reaction solution was concentrated under reduced pressure to afford the title compound (white oil, 70 mg, crude), which was used directly without purification.

### Step C: Tert-butyl 4-((1-(3-(2,4-dioxotetrahydropyrimidin-1(2H)-yl)-4-methoxybenzoyl)piperidin-4-yl)amino)piperidine-1-carboxylate

To a solution of 1-5-(4-aminopiperidine-1-carbonyl)-2-methoxyphenyl)dihydropyrimidine-2,4(1H,3H)-dione (70 mg, 0.2 mmol, 1.0 eq) in DMF (2 mL) and THF (2 mL) at rt were added TEA (0.08 mL, 0.6 mmol, 3.0 eq), tert-butyl 4-oxopiperidine-1-carboxylate (40 mg, 0.2 mmol, 1.0 eq), and tetraisopropyl titanate (57 mg, 0.2 mmol, 1.01 eq). The mixture was stirred at rt for 3 days. Sodium triacetoxyborohydride (43 mg, 0.20 mmol, 1.0 eq) was then added. The mixture was stirred at rt for 3 h. Upon completion, the reaction solution was poured into water and extracted with EtOAc (×3). The combined organic phases were washed with water and saturated aqueous NaCl, dried over anhydrous Na₂SO₄, filtered, and concentrated under pressure. The residue was purified by column chromatography to afford the title compound (white solid, 60 mg, yield 56%).

### Step D: 1-(2-methoxy-5-(4-(piperidin-4-ylamino)piperidine-1-carbonyl)phenyl)dihydropyrimidine-2,4(1H,3H)-dione

At 0°C, to tert-butyl 4-((1-(3-(2,4-dioxotetrahydropyrimidin-1(2H)-yl)-4-methoxybenzoyl)piperidin-4-yl)amino)piperidine-1-carboxylate (60 mg, 0.113 mmol, 1.0 eq) was added 4 M HCl in 1,4-dioxane (5 mL). The reaction solution was stirred at rt under N₂ for 2 h. The reaction solution was concentrated under reduced pressure to afford the title compound (white solid, 45 mg, crude), which was used directly without purification.

### Step E: N-(3-carbamoyl-1-((1r,4r)-4-((4-((1-(3-(2,4-dioxotetrahydropyrimidin-1(2H)-yl)-4-methoxybenzoyl)piperidin-4-yl)amino)piperidin-1-yl)methyl)cyclohexyl)-1H-pyrazol-4-yl)-6-(trifluoromethyl)picolinamide

To a solution of 1-(2-methoxy-5-(4-(piperidin-4-ylamino)piperidine-1-carbonyl)phenyl)dihydropyrimidine-2,4(1H,3H)-dione (200 mg, 0.46 mmol, 1.0 eq) in DMF (5 mL) and DCM (5 mL) was added TEA (0.19 mL, 1.3 mmol, 3.0 eq) at rt, and the mixture was stirred for 15 min. AcOH (0.08 mL, 1.3 mmol, 3.0 eq) and N-(3-carbonyl-1-((1r,4r)-4-formylcyclohexyl)-1H-pyrazol-4-yl)-6-(trifluoromethyl)picolinamide (190 mg, 0.46 mmol, 1.0 eq) were then added. The mixture was stirred at rt for 18 h. Sodium triacetoxyborohydride (491 mg, 2.3 mmol, 5.0 eq) was then added to the above reaction solution. The mixture was stirred at rt for 3 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (white solid, 40 mg, yield 10%). **¹H NMR** (400 MHz, DMSO-*d*₆) δ 11.71 (s, 1H), 10.33 (s, 1H), 8.40 (dt, *J =* 15.5, 11.1 Hz, 3H), 8.19 (d, *J* = 7.5 Hz, 1H), 7.64 (s, 1H), 7.54 (s, 1H), 7.43-7.24 (m, 2H), 7.16 (d, *J* = 8.6 Hz, 1H), 4.22 (s, 1H), 3.84 (s, 3H), 3.60 (t, *J* = 6.7 Hz, 2H), 2.99 (s, 2H), 2.89-2.72 (m, 3H), 2.68 (t, *J =* 6.3 Hz, 2H), 2.57-2.53 (m, 2H), 2.14-2.00 (m, 4H), 1.95-1.69 (m, 10H), 1.57 (s, 2H), 1.21 (d, *J* = 11.9 Hz, 4H), 1.10-0.95 (m, 2H). LCMS (ESI): m/z 823 [M+H]⁺.

### Preparation Example 239. Synthesis of Compound A155

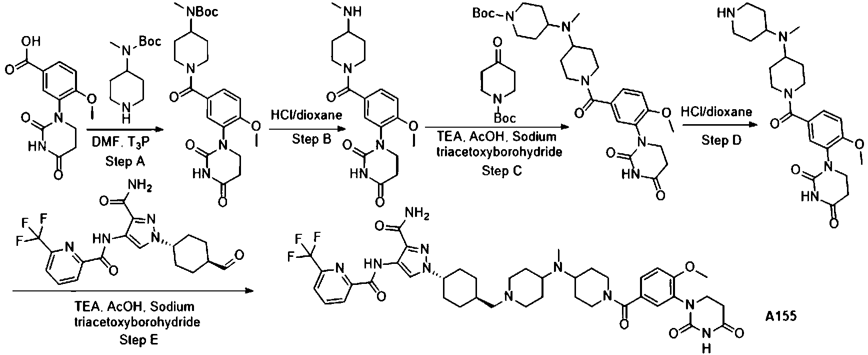

### Step A: (1-(3-(2,4-dioxotetrahydropyrimidin-1(2H)-yl)-4-methoxybenzoyl)piperidin-4-yl)(methyl)carbamate

To a solution of 3-(2,4-dioxotetrahydropyrimidin-1(2H)-yl)-4-methoxybenzoic acid (300 mg, 1.1 mmol, 1.0 eq) and tert-butyl methyl (piperidin-4-yl)carbamate (227 mg, 1.1 mmol, 1.0 eq) in DMF (5 mL) were added 50 wt% T₃P ethyl acetate solution (722 mg, 2.2 mmol, 2.0 eq) and DIEA (440 mg, 3.4 mmol, 3.0 eq) at rt. The mixture was stirred at rt for 2 h. Upon completion, the mixture was diluted in water and extracted with EtOAc (×3). The combined organic phases were washed with water and saturated aqueous NaCl, and dried over anhydrous Na₂SO₄. The residue was purified by column chromatography to afford the title compound (white solid, 360 mg, yield 71%).

### Step B: 1-(2-methoxy-5-(4-(methylamino)piperidine-1-carbonyl)phenyl)dihydropyrimidine-2,4-(1H,3H)-dione

To (1-(3-(2,4-dioxotetrahydropyrimidin-1(2H)-yl)-4-methoxybenzoyl)piperidin-4-yl)(methyl)carbamate (360 mg, 0.75 mmol, 1.0 eq) was added 4 M HCl in 1,4-dioxane (5 mL) at 0°C. The reaction solution was stirred at rt under N₂ for 2 h. The reaction solution was concentrated under reduced pressure to afford the title compound (white solid, 250 mg, crude), which was used directly without purification.

### Step C: Tert-butyl 4-((1-(3-(2,4-dioxotetrahydropyrimidin-1(2H)-yl)-4-methoxybenzoyl)piperidin-4-yl)(methyl)amino)piperidine-1-carboxylate

**At** rt, to a solution of 1-(2-methoxy-5-(4-(methylamino)piperidine-1-carbonyl)phenyl)dihydropyrimidine-2,4-(1H,3H)-dione (360 mg, 1 mmol, 1.0 eq) in DMF (2 mL) and DCM (2 mL) was added TEA (0.41 mL, 3 mmol, 3.0 eq), and the mixture was stirred for 15 min. AcOH (0.17 mL, 2.99 mmol, 3.0 eq) and tert-butyl 4-oxopiperidine-1-carboxylate (199 mg, 1.0 mmol, 1.0 eq) were then added. The mixture was stirred at rt for 18 h. Sodium triacetoxyborohydride (630 mg, 3 mmol, 3.0 eq) was then added to the above reaction solution. The mixture was stirred at rt for 3 h. Upon completion, the mixture was diluted in water, extracted with EtOAc (×3), washed with saturated aqueous NaCl, dried over anhydrous Na₂SO₄, filtered, and concentrated under reduced pressure. The residue was purified by column chromatography to afford the title compound (white solid, 200 mg, yield 37%).

### Step D: 1-(2-methoxy-5-(4-(methyl(piperidin-4-yl)amino)piperidine-1-carbonyl)phenyl)dihydropyrimidine-2,4-(1H,3H)-dione

To tert-butyl 4-((1-(3-(2,4-dioxotetrahydropyrimidin-1(2H)-yl)-4-methoxybenzoyl)piperidin-4-yl)(methyl)amino)piperidine-1-carboxylate (200 mg, 0.37 mmol, 1.0 eq) was added 4 M HCl in 1,4-dioxane (5 mL) at 0°C. The reaction solution was stirred at rt under N₂ for 2 h. The filtrate was concentrated under reduced pressure to afford the title compound (white solid, 150 mg, crude), which was used directly without purification.

### Step E: N-(3-carbonyl-1-((1r,4r)-4-((4-((1-(3-(2,4-dioxotetrahydropyrimidin-1(2H)-yl)-4-methoxybenzoyl)piperidin-4-yl)(methyl)amino)piperidin-1-yl)methyl)cyclohexyl)-1H-pyrazol-4-yl)-6-(trifluoromethyl)picolinamide

To a solution of 1-(2-methoxy-5-(4-(methyl(piperidin-4-yl)amino)piperidine-1-carbonyl)phenyl)dihydropyrimidine-2,4(1H,3H)-dione (200 mg, 0.451 mmol, 1.0 eq) in DMF (5 mL) and DCM (5 mL) was added TEA (0.2 mL, 1.3 mmol, 1.3 eq) at rt, and the mixture was stirred for 15 min. AcOH (0.07 mL, 1.3 mmol, 1.3 eq) and N-(3-carbonyl-1-((1r,4r)-4-formylcyclohexyl)-1H-pyrazol-4-yl)-6-(trifluoromethyl)picolinamide (184 mg, 0.45 mmol, 1.0 eq) were then added. The mixture was stirred at rt for 18 h. Sodium triacetoxyborohydride (475 mg, 2.2 mmol, 5.0 eq) was then added to the above reaction solution. The mixture was stirred at rt for 3 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (white solid, 30 mg, yield 8%). **¹H NMR** (400 MHz, DMSO-*d*₆) δ 11.66 (s, 1H), 8.49-8.31 (m, 2H), 8.10 (t, *J* = 7.7 Hz, 1H), 7.85 (d, *J* = 7.6 Hz, 1H), 7.51-7.34 (m, 2H), 7.01 (d, *J* = 8.3 Hz, 1H), 6.80-6.69 (m, 1H), 5.73 (s, 1H), 4.62 (s, 1H), 4.10 (s, 1H), 3.90 (s, 3H), 3.72 (s, 2H), 3.14-2.69 (m, 10H), 2.34 (s, 3H), 2.29-1.72 (m, 19H). LCMS (ESI): m/z 837 [M+H]⁺.

### Preparation Example 240. Synthesis of Compound A156

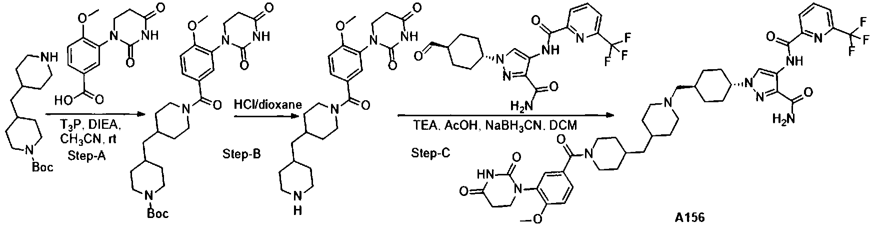

### Step A: Tert-butyl 4-((1-(3-(2,4-dioxotetrahydropyrimidin-1(2H)-yl)-4-methoxybenzoyl)piperidin-4-yl)methyl)piperidine-1-carboxylate

To a solution of 3-(2,4-dioxotetrahydropyrimidin-1 (2H)-yl)-4-methoxybenzoic acid (257 mg, 0.974 mmol) and tert-butyl 4-(piperidin-4-ylmethyl)piperidine-1-carboxylate (250 mg, 0.89 mmol) in acetonitrile (5 mL) were added 50 wt% T₃P ethyl acetate solution (563 mg, 1.77 mmol) and DIEA (345 mg, 2.66 mmol) at rt. The mixture was stirred at rt for 2 h. Upon completion, the mixture was diluted in water and extracted with EtOAc (×3). The combined organic phases were washed with water and saturated aqueous NaCl, dried over anhydrous Na₂SO₄, filtered, and concentrated under reduced pressure. The residue was purified by column chromatography to afford the title compound (white solid, 350 mg, yield 74%).

### Step B: 1-(2-methoxy-5-(4-(piperidin-4-ylmethyl)piperidine-1-carbonyl)phenyl)dihydropyrimidine-2,4(1H,3H)-dione

To tert-butyl 4-((1-(3-(2,4-dioxotetrahydropyrimidin-1(2H)-yl)-4-methoxybenzoyl)piperidin-4-yl)methyl)piperidine-1-carboxylate (300 mg, 0.57 mmol) was added 4 M HCl in 1,4-dioxane (5 mL) at 0°C. The reaction solution was stirred at rt under N₂ for 2 h. The reaction solution was concentrated under reduced pressure to afford the title compound (white solid, 200 mg, yield 82%).

### Step C: N-(3-carbamoyl-1-((1r,4r)-4-((4-((1-(3-(2,4-dioxotetrahydropyrimidin-1(2H)-yl)-4-methoxybenzoyl)piperidin-4-yl)methyl)piperidin-1-ylmethyl)cyclohexyl)-1H-pyrazol-4-yl)-6-(trifluoromethyl)picolinamide

To a solution of 1-(2-methoxy-5-(4-(piperidin-4-ylmethyl)piperidine-1-carbonyl)phenyl)dihydropyrimidine-2,4(1H,3H)-dione (200 mg, 0.47 mmol) in DCM (5 mL) were added TEA (0.2 mL, 1.40 mmol), AcOH (0.04 mL, 0.70 mmol), and N-(3-carbonyl-1-((1r,4r)-4-formylcyclohexyl)-1H-pyrazol-4-yl)-6-(trifluoromethyl)picolinamide (210 mg, 0.51 mmol) at rt. The mixture was stirred at rt for 18 h. Sodium triacetoxyborohydride (295 mg, 1.40 mmol) was then added to the above reaction solution. The mixture was stirred at rt for 3 h. Upon completion, the mixture was diluted in water, extracted with DCM (×3), washed with saturated aqueous NaCl, dried over anhydrous Na₂SO₄, filtered, and concentrated under reduced pressure. The residue was purified by HPLC to afford the title compound (white solid, 40 mg, yield 10%). **¹H NMR** (400 MHz, DMSO-*d*₆) δ 11.71 (s, 1H), 10.33 (s, 1H), 8.49-8.28 (m, 3H), 8.18 (d, *J* = 7.2 Hz, 1H), 7.63 (s, 1H), 7.54 (s, 1H), 7.35 (d, *J =* 8.4 Hz, 1H), 7.31 (d, *J =* 1.6 Hz, 1H), 7.15 (d, *J =* 8.4 Hz, 1H), 4.22 (t, *J =* 11.6 Hz, 2H), 3.84 (s, 3H), 3.59 (t, *J =* 6.4 Hz, 2H), 2.80 (d, *J* = 10.4 Hz, 4H), 2.68 (t, *J* = 6.4 Hz, 2H), 2.09 (d, *J* = 7.2 Hz, 4H), 1.98-1.74 (m, 6H), 1.61 (d, *J =* 10.8 Hz, 6H), 1.43-1.20 (m, 2H), 1.19-0.94 (m, 8H). LCMS (ESI): m/z 822 [M+H]⁺.

### Preparation Example 241. Synthesis of Compound A157

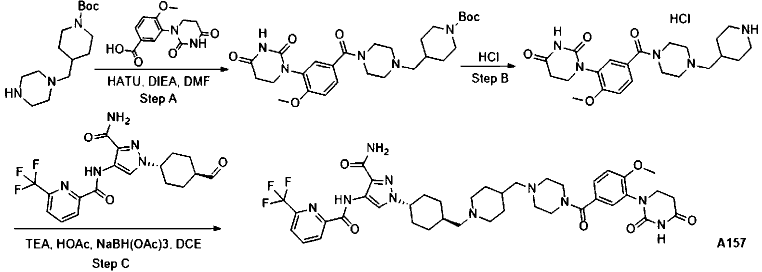

### Step A: Tert-butyl 4-((4-(3-(2,4-dioxotetrahydropyrimidin-1(2H)-yl)-4-methoxybenzoyl)piperazin-1-yl)methyl)piperidine-1-carboxylate

To a solution of 3-(2,4-dioxotetrahydropyrimidin-1 (2H)-yl)-4-methoxybenzoic acid (200 mg, 0.757 mmol, 1.0 eq) in DMF (4 mL) at rt were added HATU (345 mg, 0.908 mmol, 1.2 eq) and DIPEA (293 mg, 2.27 mmol, 3.0 eq). The reaction solution was stirred at rt for 15 min. Then, tert-butyl 4-(piperazin-1-ylmethyl)piperidine-1-carboxylate (219 mg, 0.772 mmol, 1.02 eq) was added. The reaction solution was stirred at 25°C under N₂ for 2 h. Upon completion, the reaction solution was quenched with water and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (brown solid, 330 mg, yield 82%).

### Step B: 1-(2-methoxy-5-(4-(piperidin-4-ylmethyl)piperazine-1-carbonyl)phenyl)dihydropyrimidine-2,4(1H,3H)-dione hydrochloride

To a solution of tert-butyl 4-((4-(3-(2,4-dioxotetrahydropyrimidin-1(2H)-yl)-4-methoxybenzoyl)piperazin-1-yl)methyl)piperidine-1-carboxylate (330 mg, 0.623 mmol, 1.0 eq) in dioxane (5 mL) was added HCl/dioxane (5 mL, 4 M) at rt. The reaction solution was stirred at rt for 3 h. Upon completion, the reaction solution was concentrated under reduced pressure to afford the title compound (white solid, 280 mg, crude).

### Step C: N-(3-Carbonyl-1-((1r,4r)-4-((4-(4-(3-(2,4-dioxotetrahydropyrimidin-1(2H)-yl)-4-methoxybenzoyl)piperazin-1-yl)methyl)piperidin-1-yl)methyl)cyclohexyl)-1H-pyrazol-4-yl)-6-(trifluoromethyl)picolinamide

To a solution of 1-(2-methoxy-5-(4-(piperidin-4-ylmethyl)piperazine-1-carbonyl)phenyl)dihydropyrimidine-2,4(1H,3H)-dione hydrochloride (100 mg, 0.215 mmol, 1.0 eq) in 1,2-dichloroethane (4 mL) at rt was added TEA (43 mg, 0.429 mmol, 2.0 eq). The reaction solution was stirred at rt for 20 min. N-(3-carbonyl-1-((1r,4r)-4-formylcyclohexyl)-1H-pyrazol-4-yl)-6-(trifluoromethyl)picolinamide (92 mg, 0.225 mmol, 1.05 eq) and AcOH (80 mg, 0.429 mmol, 2.0 eq) were then added. The reaction solution was stirred at 25°C under N₂ for 1 h. Sodium triacetoxyborohydride (91 mg, 0.430 mmol, 2.0 eq) was then added, and the reaction solution was stirred at rt for 16 h. Upon completion, the reaction solution was quenched with NaHCO₃ solution and extracted with DCM (×3). The combined organic phases were washed with saturated aqueous NaCl, dried over anhydrous Na₂SO₄, filtered, and concentrated under reduced pressure. The residue was purified by HPLC to afford the title compound (white solid, 40 mg, yield 23%). **¹H** NMR (400 MHz, DMSO-*d*₆) δ 11.71 (s, 1H), 10.33 (s, 1H), 8.43 (d, *J* = 3.0 Hz, 1H), 8.38 (dd, *J* = 14.4, 6.7 Hz, 2H), 8.19 (s, 1H), 7.58 (d, *J* = 37.8 Hz, 2H), 7.38 (d, *J* = 8.5 Hz, 1H), 7.34 (d, *J=* 1.9 Hz, 1H), 7.16 (d, *J* = 8.6 Hz, 1H), 4.22 (s, 1H), 3.84 (s, 3H), 3.61 (d, *J* = 6.6 Hz, 2H), 2.83 (d, *J* = 9.9 Hz, 2H), 2.68 (t, *J* = 6.5 Hz, 2H), 2.54 (s, 4H), 2.35 (s, 4H), 2.15 (d, *J* = 6.4 Hz, 4H), 2.09 (d, *J* = 10.8 Hz, 2H), 1.88 (t, *J* = 10.9 Hz, 4H), 1.81 (d, *J* = 12.4 Hz, 2H), 1.68 (d, *J* = 12.1 Hz, 2H), 1.54 (d, *J*= 44.5 Hz, 2H), 1.08 (dd, *J=* 26.1, 11.8 Hz, 4H). LCMS (ESI): m/z 823 [M+H]⁺.

### Preparation Example 242. Synthesis of Compound A158

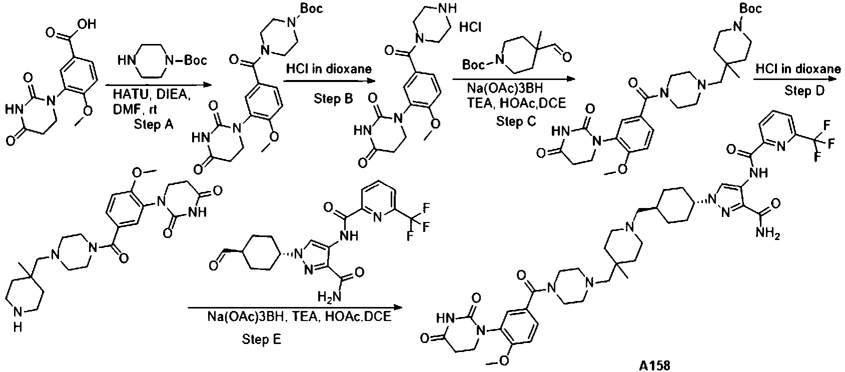

### Step A: Tert-butyl 4-(3-(2,4-dioxotetrahydropyrimidin-1(2H)-yl)-4-methoxybenzoyl)piperazine-1-carboxylate

To a solution of 3-(2,4-dioxotetrahydropyrimidin-1(2H)-yl)-4-methoxybenzoic acid (300 mg, 1.14 mmol, 1.0 eq) in DMF (6.0 mL) were added DIPEA (440 mg, 3.41 mmol, 3.0 eq) and HATU (518 mg, 1.36 mmol, 1.2 eq) at rt. The reaction solution was reacted at rt for 15 min. Then, tert-butyl piperazine-1-carboxylate (211 mg, 1.14 mmol, 1.0 eq) was added. The reaction was reacted at rt for 2 h under N₂. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (brown solid, 480 mg, yield 98%).

### Step B: 1-(2-methoxy-5-(piperazine-1-carbonyl)phenyl)dihydropyrimidine-2,4(1H,3H)-dione hydrochloride

**To a** solution of tert-butyl 4-(3-(2,4-dioxotetrahydropyrimidin-1(2H)-yl)-4-methoxybenzoyl)piperazine-1-carboxylate (500 mg, 1.16 mmol, 1.0 eq) in 1,4-dioxane (5.0 mL) was added HCl/1,4-dioxane (5 mL, 4 M) at rt. The reaction solution was reacted at rt for 3 h. Upon completion, the reaction solution was concentrated under reduced pressure to afford the title compound (white solid, 360 mg, crude).

### Step C: Tert-butyl 4-((4-(3-(2,4-dioxotetrahydropyrimidin-1(2H)-yl)-4-methoxybenzoyl)piperazin-1-yl)methyl)-4-methylpiperidine-1-carboxylate

To a solution of 1-(2-methoxy-5-(piperazine-1-carbonyl)phenyl)dihydropyrimidine-2,4(1H,3H)-dione hydrochloride (360 mg, 0.976 mmol, 1.0 eq) in 1,2-dichloroethane (10.0 mL) was added TEA (198 mg, 1.95 mmol, 2.0 eq) at rt. The mixture was reacted at rt for 15 min. Tert-butyl 4-formyl-4-methylpiperidine-1-carboxylate (222 mg, 0.976 mmol, 1.0 eq) and AcOH (117 mg, 1.95 mmol, 2.0 eq) were then added. The reaction was reacted at rt for 1 h under N₂. Sodium triacetoxyborohydride (414 mg, 1.95 mmol, 2.0 eq) was then added, and the reaction was allowed to proceed at rt for 16 h. Upon completion, the mixture was poured into aqueous NaHCO₃, and extracted with DCM (×3). The combined organic phases were washed with saturated aqueous NaCl, dried over anhydrous Na₂SO₄, filtered, and concentrated under reduced pressure. The residue was purified by column chromatography to afford the title compound (white solid, 220 mg, yield 41%).

### Step D: 1-(2-methoxy-5-(4-(4-methylpiperidin-4-yl)methyl)piperazine-1-carbonyl)phenyl)dihydropyrimidine-2,4(1H,3H)-dione hydrochloride

To a solution of tert-butyl 4-((4-(3-(2,4-dioxotetrahydropyrimidin-1(2H)-yl)-4-methoxybenzoyl)piperazin-1-yl)methyl)-4-methylpiperidine-1-carboxylate (220 mg, 0.405 mmol, 1.0 eq) in 1,4-dioxane (10.0 mL) was added HCl/1,4-dioxane (3 mL, 4 M) at rt. The reaction solution was reacted at rt for 3 h. Upon completion, the reaction solution was concentrated under reduced pressure to afford the title compound (brown solid, 180 mg, crude).

### Step E: N-(3-carbonyl-1-((1r,4r)-4-((4-(4-(3-(2,4-dioxotetrahydropyrimidin-1(2H)-yl)-4-methoxybenzoyl)piperazin-1-yl)methyl)-4-methylpiperidin-1-yl)methyl ester)cyclohexyl)-1H-pyrazol-4-yl)-6-(trifluoromethyl)picolinamide

To a solution of 1-(2-methoxy-5-(4-(4-methylpiperidin-4-yl)methyl)piperazine-1-carbonyl)phenyl)dihydropyrimidine-2,4(1H,3H)-dione hydrochloride (150 mg, 0.312 mmol) in 1,2-dichloroethane (5.0 mL) at rt was added TEA (63.2 mg, 0.625 mmol, 2.0 eq). The reaction solution was reacted at rt for 20 min. Then, N-(3-carbonyl-1-((1r,4r)-4-formylcyclohexyl)-1H-pyrazol-4-yl)-6-(trifluoromethyl)picolinamide (154 mg, 0.375 mmol, 1.2 eq) and AcOH (37.5 mg, 0.625 mmol, 2.0 eq) were added. The reaction was reacted at rt for 1 h under N₂. Sodium triacetoxyborohydride (132 mg, 0.625 mmol, 2.0 eq) was then added, and the reaction was allowed to proceed at rt for 16 h. Upon completion, the reaction solution was poured into aqueous NaHCO₃, and extracted with DCM (×3). The combined organic phases were washed with saturated aqueous NaCl, dried over anhydrous Na₂SO₄, filtered, and concentrated under reduced pressure. The residue was purified by HPLC to afford the title compound (white solid, 136 mg, yield 52%). **¹H NMR** (400 MHz, DMSO-*d*₆) δ 11.71 (s, 1H), 10.34 (s, 1H), 8.38 (dd, *J* = 14.4, 6.8 Hz, 2H), 8.22-8.15 (m, 2H), 7.59 (d, *J =* 33.8 Hz, 2H), 7.38 (dd, *J* = 8.5, 2.1 Hz, 1H), 7.34 (d, *J* = 2.1 Hz, 1H), 7.16 (d, *J* = 8.6 Hz, 1H), 4.23 (t, *J* = 11.7 Hz, 2H), 3.84 (s, 3H), 3.66 (s, 2H), 3.63-3.57 (m, 4H), 2.68 (t, *J =* 6.5 Hz, 2H), 2.54 (d, *J* = 4.5 Hz, 2H), 2.49-2.41 (m, 3H), 2.27 (dd, *J* = 26.7, 8.4 Hz, 4H), 2.16 (s, 2H), 2.09 (d, *J* = 10.8 Hz, 2H), 1.98-1.72 (m, 4H), 1.62 (s, 1H), 1.51 (t, *J* = *9.7* Hz, 2H), 1.27 (d, *J* = 12.3 Hz, 2H), 1.17-0.99 (m, 2H), 0.89 (s, 3H). LCMS (ESI): m/z 837 [M+H]⁺.

### Preparation Example 243. Synthesis of Compound A159

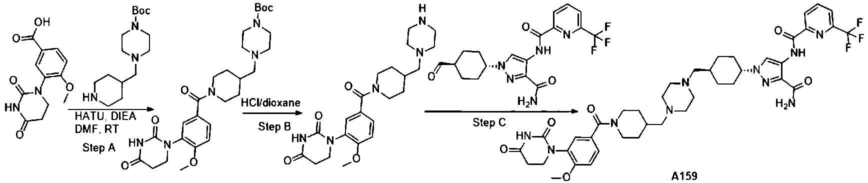

### Step A: Tert-butyl 4-((1-(3-(2,4-dioxotetrahydropyrimidin-1(2H)-yl)-4-methoxybenzoyl)piperidin-4-yl)methyl)piperazine-1-carboxylate

At 0°C, to a solution of 3-(2,4-dioxotetrahydropyrimidin-1(2H)-yl)-4-methoxybenzoic acid (200 mg, 0.757 mmol, 1.0 eq) in DMF (2.0 mL) were added DIPEA (195 mg, 1.51 mmol, 2.0 eq) and HATU (431 mg, 1.13 mmol, 1.5 eq). The reaction solution was reacted at rt for 15 min. Then, tert-butyl 4-(piperidin-4-ylmethyl)piperazine-1-carboxylate (218 mg, 0.772 mmol, 1.02 eq) was added, and the reaction solution was stirred at rt for 2 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (brown solid, 200 mg, yield 49%).

### Step B: 1-(2-methoxy-5-(4-(piperazin-1-ylmethyl)piperidine-1-carbonyl)phenyl)dihydropyrimidine-2,4(1H,3H)-dione

To a solution of tert-butyl 4-((1-(3-(2,4-dioxotetrahydropyrimidin-1(2H)-yl)-4-methoxybenzoyl)piperidin-4-yl)methyl)piperazine-1-carboxylate (200 mg, 0.378 mmol, 1.0 eq) in 1,4-dioxane (5.0 mL) was added HCl/1,4-dioxane (5 mL, 4 M) at rt. The reaction solution was reacted at rt for 3 h. Upon completion, the reaction solution was concentrated under reduced pressure to afford the title compound (white solid, 120 mg, crude).

### Step C: N-(3-carbonyl-1-((1r,4r)-4-((4-((1-(3-(2,4-dioxotetrahydropyrimidin-1(2H)-yl)-4-methoxybenzoyl)piperidin-4-yl)methyl)piperazin-1-yl)methyl]cyclohexyl)-1H-pyrazol-4-yl)-6-(trifluoromethyl)picolinamide

To a solution of 1-(2-methoxy-5-(4-(piperazin-1-ylmethyl)piperidine-1-carbonyl)phenyl)dihydropyrimidine-2,4(1H,3H)-dione (120 mg, 0.258 mmol, 1.0 eq) in 1,2-dichloroethane (4.0 mL) at rt was added TEA (52 mg, 0.515 mmol, 2.0 eq). The reaction solution was reacted at rt for 20 min. N-(3-carbonyl-1-((1r,4r)-4-formylcyclohexyl)-1H-pyrazol-4-yl)-6-(trifluoromethyl)picolinamide (110.69 mg, 0.270 mmol, 1.05 eq) and AcOH (96 mg, 0.515 mmol, 2.0 eq) were then added. The reaction was reacted at rt for 1 h under N₂. Sodium triacetoxyborohydride (109 mg, 0.515 mmol, 2.0 eq) was then added, and the reaction was allowed to proceed at rt for 16 h. Upon completion, the reaction solution was poured into aqueous NaHCO₃, and extracted with DCM (×3). The combined organic phases were washed with saturated aqueous NaCl, dried over anhydrous Na₂SO₄, filtered, and concentrated under reduced pressure. The residue was purified by HPLC to afford the title compound (white solid, 46.2 mg, yield 22%). **¹H NMR** (400 MHz, DMSO-*d*₆) δ 11.71 (s, 1H), 10.33 (s, 1H), 8.43 (s, 1H), 8.40 (d, *J* = 6.0 Hz, 1H), 8.36 (d, *J* = 7.8 Hz, 1H), 8.19 (d, *J* = 7.6 Hz, 1H), 7.63 (s, 1H), 7.54 (s, 1H), 7.39-7.34 (m, 1H), 7.31 (d, *J* = 2.0 Hz, 1H), 7.15 (d, *J* = 8.6 Hz, 1H), 4.23 (t, *J =* 11.8 Hz, 1H), 3.84 (s, 3H), 3.60 (t, *J =* 6.6 Hz, 3H), 2.68 (t, *J* = 6.6 Hz, 2H), 2.35 (s, 8H), 2.16-2.03 (m, 7H), 1.95-1.85 (m, 3H), 1.84-1.67 (m, 5H), 1.58 (s, 1H), 1.23 (s, 1H), 1.10-1.00 (m, 4H). LCMS (ESI): m/z 823 [M+H]⁺.

### Preparation Example 244. Synthesis of Compound A160

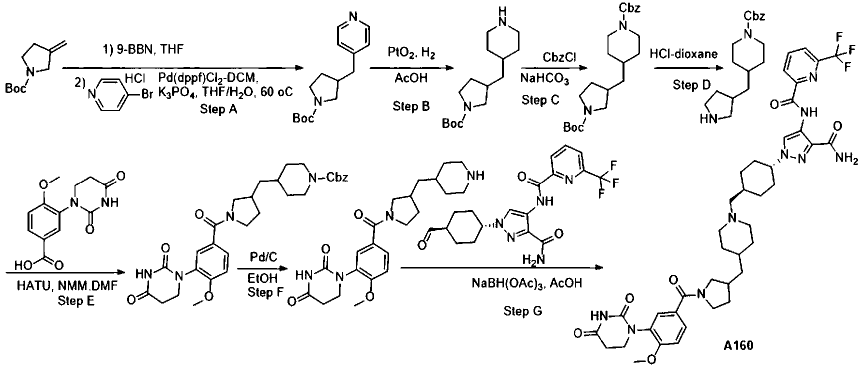

### Step A: Tert-butyl 3-(pyridin-4-ylmethyl)pyrrolidine-1-carboxylate

At 0°C, to a solution of tert-butyl 3-methylpyrrolidine-1-carboxylate (350 mg, 1.91 mmol, 1.0 eq) in THF (1 mL) was added 1 M 9-borabicyclo[3.3.1]nonane (9-BBN) (3.9 mL, 3.9 mmol, 2.0 eq). The reaction solution was stirred at 60°C under N₂ for 5 h. Upon completion, the mixture was cooled to rt, and the reaction solution was used directly in the next step. To the above mixture were added THF (5 mL) and water (1 mL), followed by the addition of 4-bromopyridine (158 mg, 1.0 mmol, 0.5 eq), potassium phosphate (424.5 mg, 2.0 mmol, 1.0 eq), and Pd(dppf)Cl₂ in DCM (163.7 mg, 0.2 mmol, 0.1 eq). The reaction solution was stirred at 60°C under N₂ for 13 h. Upon completion, the mixture was cooled to rt, diluted in water, and extracted with EtOAc (×3). The combined organic phases were washed with water and saturated aqueous NaCl, dried over anhydrous Na₂SO₄, filtered, and concentrated under reduced pressure. The residue was purified by column chromatography to afford the title compound (yellow solid, 200 mg, yield 76%).

### Step B: Tert-butyl 3-(piperidin-4-ylmethyl)pyrrolidine-1-carboxylate

To a solution of tert-butyl 3-(pyridin-4-ylmethyl)pyrrolidine-1-carboxylate (200 mg, 0.8 mmol, 1.0 eq) in MeOH (5 mL) were added platinum dioxide (50 mg) and AcOH (0.1 mL) at rt. The reaction solution was purged with H₂ (using a hydrogen balloon) (×3) and stirred under H₂ at rt for 2 h. Upon completion, the mixture was filtered, and the filtrate was concentrated under reduced pressure to afford the title compound (white solid, 180 mg, crude), which was used directly without purification.

### Step C: Benzyl 4-((1-(tert-Butoxycarbonyl)pyrrolidin-3-yl)methyl)piperidine-1-carboxylate

To a solution of tert-butyl 3-(piperidin-4-ylmethyl)pyrrolidine-1-carboxylate (200 mg, 0.7 mmol, 1.0 eq) in water (3 mL) and DCM (3 mL) were added NaHCO₃ (626 mg, 7 mmol, 10.0 eq) and benzyl chloroformate (190.7 mg, 1.1 mmol, 1.5 eq) at rt. The mixture was stirred at rt for 2 h. Upon completion, the mixture was diluted in water and extracted with DCM (×3). The combined organic phases were washed with water and saturated aqueous NaCl, dried over anhydrous Na₂SO₄, filtered, and concentrated under reduced pressure. The residue was purified by column chromatography to afford the title compound (white solid, 150 mg, yield 50%).

### Step D: Benzyl 4-(pyrrolidin-3-ylmethyl)piperidine-1-carboxylate

To benzyl 4-((1-(tert-butoxycarbonyl)pyrrolidin-3-yl)methyl)piperidine-1-carboxylate (200 mg, 0.5 mmol, 1.0 eq) was added 4 M HCl in 1,4-dioxane (3 mL, 12 mmol, 24 eq) at 0°C. The reaction solution was stirred at rt under N₂ for 2 h. The reaction solution was concentrated under reduced pressure to afford the title compound (white solid, 150 mg, crude), which was used in the next step without purification.

### Step E: Benzyl 4-((1-(3-(2,4-dioxotetrahydropyrimidin-1(2H)-yl)-4-methoxybenzoyl)pyrrolidin-3-yl)methyl)piperidine-1-carboxylate

To a solution of benzyl 4-(pyrrolidin-3-ylmethyl)piperidine-1-carboxylate (151.2 mg, 0.5 mmol, 1.0 eq) and 3-(2,4-dioxotetrahydropyrimidin-1(2H)-yl)-4-methoxybenzoic acid (158.5 mg, 0.6 mmol, 1.2 eq) in DMF (5.0 mL) were added N-methylmorpholine (0.3 mL, 2.5 mmol, 2.0 eq) and HATU (285.2 mg, 0.8 mmol, 3.0 eq) at rt. The reaction solution was stirred at rt under N₂ for 5 h. Upon completion, the reaction solution was diluted in EtOAc, washed sequentially with water and saturated aqueous NaCl, dried over anhydrous Na₂SO₄, filtered, and concentrated under reduced pressure. The residue was purified by column chromatography to afford the title compound (yellow solid, 250 mg, yield 91%).

### Step F: 1-(2-methoxy-5-(3-(piperidin-4-ylmethyl)pyrrolidine-1-carbonyl)phenyl)dihydropyrimidine-2,4(1H,3H)-dione

To a solution of benzyl 4-((1-(3-(2,4-dioxotetrahydropyrimidin-1(2H)-yl)-4-methoxybenzoyl)pyrrolidin-3-yl)methyl)piperidine-1-carboxylate (200 mg, 0.4 mmol, 1.0 eq) in ethanol (5 mL) was added 10% palladium on carbon (50 mg) at rt. The reaction solution was purged with H₂ (using a hydrogen balloon) (×3) and stirred under H₂ at rt for 2 h. Upon completion, the mixture was filtered, and the filtrate was concentrated under reduced pressure to afford the title compound (yellow solid, 120 mg, crude), which was used directly without purification.

### Step G: N-(3-carbonyl-1-((1r,4r)-4-((4-((1-(3-(2,4-dioxotetrahydropyrimidin-1(2H)-yl)-4-methoxybenzoyl)pyrrolidin-3-yl)methyl)piperidin-1-yl)methyl]cyclohexyl)-1H-pyrazol-4-yl)-6-(trifluoromethyl)picolinamide

To a solution of N-(3-carbonyl-1-((1r,4r)-4-formylcyclohexyl)-1H-pyrazol-4-yl)-6-(trifluoromethyl)picolinamide (118.5 mg, 0.3 mmol, 1.0 eq) and 1-(2-methoxy-5-(3-(piperidin-4-ylmethyl)pyrrolidine-1-carbonyl)phenyl)dihydropyrimidine-2,4(1H,3H)-dione (120 mg, 0.3 mmol, 1.0 eq) in DMF (5 mL) was added AcOH (0.1 mL) at rt. The mixture was stirred at rt for 5 h. Sodium triacetoxyborohydride (122.7 mg, 0.6 mmol, 1.0 eq) was then added to the above reaction solution. The mixture was stirred at rt for 1 h. Upon completion, the mixture was diluted in water, extracted with EtOAc (×3), washed with saturated aqueous NaCl, dried over anhydrous Na₂SO₄, filtered, and concentrated under reduced pressure. The residue was purified by HPLC to afford the title compound (yellow solid, 24.6 mg, yield 11%). **¹H NMR** (400 MHz, DMSO-*d*₆) δ 11.72 (s, 1H), 10.34 (s, 1H), 8.51-8.31 (m, 3H), 8.26-8.12 (m, 2H), 7.63 (s, 1H), 7.53 (d, *J* = 10.4 Hz, 2H), 7.48 (d, *J* = 2.0 Hz, 1H), 7.14 (d, *J* = 8.4 Hz, 1H), 4.31-4.22 (m, 1H), 3.85 (s, 3H), 3.76-3.32 (m, 6H), 3.17-2.98 (m, 1H), 2.97-2.80 (m, 2H), 2.69 (t, *J* = 6.5 Hz, 2H), 2.43-2.15 (m, 3H), 2.10-1.78 (m, 8H), 1.75-1.40 (m, 5H), 1.37-1.03 (m, 6H). LCMS (ESI): m/z 808 [M+H]⁺.

### Preparation Example 245. Synthesis of Compound A161

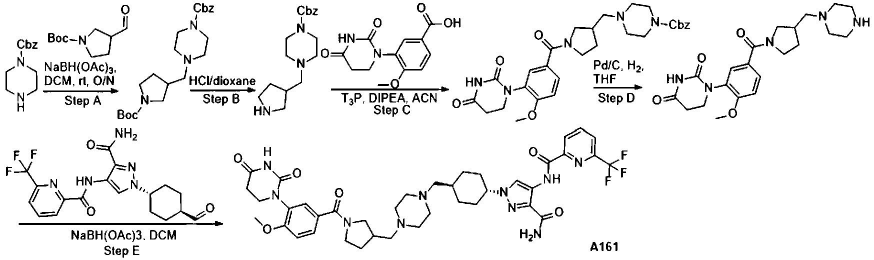

### Step A: Benzyl 4-((1-(tert-butoxycarbonyl)pyrrolidin-3-yl)methyl)piperazine-1-carboxylate

At rt, to a solution of benzyl piperazine-1-carboxylate (2 g, 9.08 mmol, 1.0 eq) in DCM (25 mL) was added tert-butyl 3-formylpyrrolidine-1-carboxylate (1.8 g, 9.08 mmol, 1.0 eq). The mixture was stirred at rt for 2 h. Sodium triacetoxyborohydride (3.1 g, 14.5 mmol, 1.5 eq) was then added to the above reaction solution. The reaction solution was stirred at rt under N₂ for 18 h. Upon completion, the mixture was diluted in saturated NaHCO₃ solution and water, extracted with DCM (×3), washed with saturated aqueous NaCl, dried over anhydrous Na₂SO₄, filtered, and concentrated under reduced pressure. The residue was purified by column chromatography to afford the title compound (white solid, 3.5 g, yield 95%).

### Step B: Benzyl 4-(pyrrolidin-3-ylmethyl)piperazine-1-carboxylate

To benzyl 4-((1-(tert-butoxycarbonyl)pyrrolidin-3-yl)methyl)piperazine-1-carboxylate (3.5 g, 8.67 mmol, 1.0 eq) was added 4 M HCl in 1,4-dioxane (5 mL) at 0°C. The reaction solution was stirred at rt under N₂ for 3 h. The reaction solution was concentrated under reduced pressure to afford the title compound (yellow oil, 2.8 g, crude), which was used directly without purification.

### Step C: Benzyl 4-((1-(3-(2,4-dioxotetrahydropyrimidin-1(2H)-yl)-4-methoxybenzoyl)pyrrolidin-3-yl)methyl)piperazine-1-carboxylate

To a solution of benzyl 4-(pyrrolidin-3-ylmethyl)piperazine-1-carboxylate (459 mg, 1.51 mmol, 2.0 eq) and 3-(2,4-dioxotetrahydropyrimidin-1(2H)-yl)-4-methoxybenzoic acid (200 mg, 0.76 mmol, 1.0 eq) in acetonitrile (6 mL) at rt were added 50 wt% T₃P in EtOAc (289 mg, 0.908 mmol, 1.2 eq) and DIEA (0.7 mL, 3.78 mmol, 5.0 eq). The reaction solution was stirred at rt under N₂ for 18 h. Upon completion, the mixture was diluted in 28% aqueous ammonia and water, extracted with EtOAc (×3), washed with saturated aqueous NaCl, dried over anhydrous Na₂SO₄, filtered, and concentrated under reduced pressure. The residue was purified by column chromatography to afford the title compound (white solid, 210 mg, yield 50%).

### Step D: 1-(2-methoxy-5-(3-(piperazin-1-ylmethyl)pyrrolidine-1-carbonyl)phenyl)dihydropyrimidine-2,4(1H,3H)-dione

To a solution of benzyl 4-((1-(3-(2,4-dioxotetrahydropyrimidin-1(2H)-yl)-4-methoxybenzoyl)pyrrolidin-3-yl)methyl)piperazine-1-carboxylate (200 mg, 0.36 mmol, 1.0 eq) in THF (10 mL) was added 10% palladium on carbon (38 mg, 0.36 mmol, 1.0 eq) at rt. The reaction solution was purged with H₂ (using a hydrogen balloon) (×3) and stirred under H₂ at rt for 2 h. Upon completion, the mixture was filtered, and the filtrate was concentrated under reduced pressure to afford the title compound (yellow oil, 100 mg, crude), which was used directly without purification.

### Step E: N-(3-carbonyl-1-((1r,4r)-4-((4-((1-(3-(2,4-dioxotetrahydropyrimidin-1(2H)-yl)-4-methoxybenzoyl)pyrrolidin-3-yl)methyl)piperazin-1-yl)methyl]cyclohexyl)-1H-pyrazol-4-yl)-6-(trifluoromethyl)picolinamide

To a solution of 1-(2-methoxy-5-(3-(piperazin-1-ylmethyl)pyrrolidine-1-carbonyl)phenyl)dihydropyrimidine-2,4(1H,3H)-dione (101 mg, 0.24 mmol, 1.0 eq) in DCM (5 mL) was added N-(3-carbonyl-1-((1r,4r)-4-formylcyclohexyl)-1H-pyrazol-4-yl)-6-(trifluoromethyl)picolinamide (100 mg, 0.24 mmol, 1.0 eq) at rt. The mixture was stirred at rt for 2 h. Sodium triacetoxyborohydride (55 mg, 0.49 mmol, 2.0 eq) was then added to the above reaction solution. The reaction solution was stirred at rt under N₂ for 18 h. Upon completion, the mixture was diluted in saturated NaHCO₃ solution and water, extracted with DCM (×3), washed with saturated aqueous NaCl, dried over anhydrous Na₂SO₄, filtered, and concentrated under reduced pressure. The residue was purified by HPLC to afford the title compound (white solid, 20 mg, yield 10%). **¹H NMR** (400 MHz, DMSO-*d*₆) δ 11.71 (s, 1H), 10.34 (s, 1H), 8.45-8.32 (m, 3H), 8.19 (d, *J=* 7.4 Hz, 1H), 7.64 (s, 1H), 7.50 (dd, *J =* 21.3, 7.2 Hz, 3H), 7.15 (d, *J* = 4.7 Hz, 1H), 4.23 (s, 1H), 3.85 (s, 3H), 3.61-3.45 (m, 5H), 3.21 (d, *J=* 19.6 Hz, 2H), 2.69 (t, *J=* 6.0 Hz, 2H), 2.43-2.23 (m, 9H), 2.08 (s, 4H), 1.85 (d, *J=* 37.2 Hz, 6H), 1.58 (s, 2H), 1.04 (s, 2H). LCMS (ESI): m/z 809 [M+H]⁺.

### Preparation Example 246. Synthesis of Compound A162

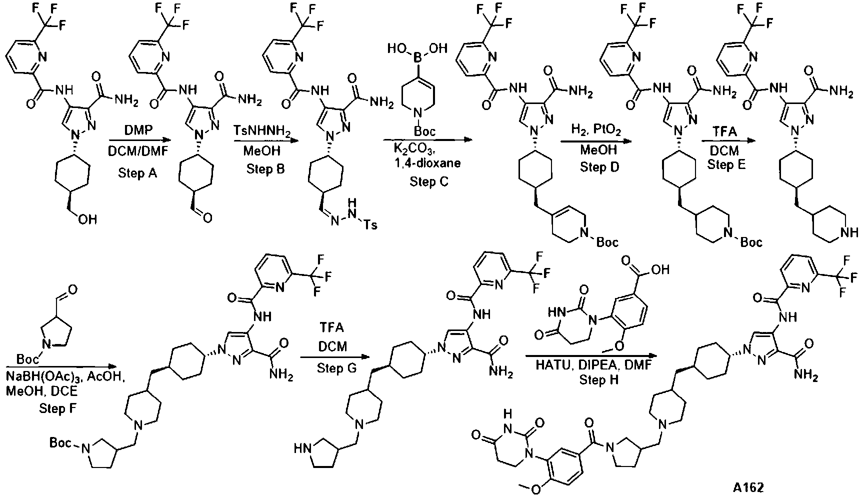

### Step A: N-(3-Carbonyl-1-((1r,4r)-4-formylcyclohexyl)-1H-pyrazol-4-yl)-6-(trifluoromethyl)picolinamide

To a solution of N-(3-carbonyl-1-((1r,4r)-4-(hydroxymethyl)cyclohexyl)-1H-pyrazol-4-yl)-6-(trifluoromethyl)picolinamide (1 g, 2.4 mmol, 1.0 eq) in DCM (10 mL) and DMF (10 mL) at rt was added DMP (0.87 g, 7.2 mmol, 3.0 eq). The reaction solution was stirred at rt under N₂ for 0.5 h. Upon completion, the reaction solution was poured into aqueous NH₄Cl and extracted with EtOAc (×3). The combined organic phases were washed with saturated aqueous NaCl, dried over anhydrous Na₂SO₄, filtered, and concentrated under reduced pressure to yield the title compound (yellow solid, 900 mg, crude).

### Step B: N-(3-Carbonyl-1-((1r,4r)-4-((Z)-(2-toluenesulfonylhydrazino)methyl)cyclohexyl)-1H-pyrazol-4-yl)-6-(trifluoromethyl)picolinamide

To a solution of N-(3-carbonyl-1-((1r,4r)-4-formylcyclohexyl)-1H-pyrazol-4-yl)-6-(trifluoromethyl)picolinamide (1 g, 2.4 mmol, 1.0 eq) in MeOH (10 mL) at rt was added 4-toluenesulfonylhydrazide (327 mg, 2.64 mmol, 1.1 eq). The reaction solution was stirred at rt under N₂ for 0.5 h. Upon completion, the reaction solution was filtered, and the filtrate was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 900 mg, yield 92%).

### Step C: Tert-butyl 4-(((1r,4r)-4-(3-carbonyl-4-(6-(trifluoromethyl)picolinamido)-1H-pyrazol-1-yl)cyclohexyl)methyl)-3,6-dihydropyridine-1(2H)-carboxylate

To a solution of N-(3-carbonyl-1-((1r,4r)-4-((Z)-(2-tosylhydrazino)methyl)cyclohexyl)-1H-pyrazol-4-yl)-6-(trifluoromethyl)picolinamide (900 mg, 1.5 mmol, 1.0 eq) in 1,4-dioxane (10 mL) at rt were added (1-(tert-butoxycarbonyl)-1,2,3,6-tetrahydropyridin-4-yl)boronic acid (389 mg, 1.7 mmol, 1.1 eq) and potassium carbonate (538 mg, 3.8 mmol, 2.5 eq). The reaction solution was stirred at 110°C under N₂ for 0.5 h. Upon completion, the reaction solution was poured into water and the pH was adjusted to 5-6 with 1 M HCl. The mixture was extracted with EtOAc (×3), and the combined organic phases were washed with saturated aqueous NaCl, dried over anhydrous Na₂SO₄, filtered, and concentrated under reduced pressure. The residue was purified by column chromatography to afford the title compound (yellow solid, 380 mg, yield 42%).

### Step D: Tert-butyl 4-(((1r,4r)-4-(3-carbonyl-4-(6-(trifluoromethyl)picolinamido)-1H-pyrazol-1-yl)cyclohexyl)methyl)piperidine-1-carboxylate

To a solution of tert-butyl 4-(((1r,4r)-4-(3-carbonyl-4-(6-(trifluoromethyl)picolinamido)-1H-pyrazol-1-yl)cyclohexyl)methyl)-3,6-dihydropyridine-1(2H)-carboxylate (360 mg, 1.5 mmol, 1.0 eq) in MeOH (5 mL) at rt was added platinum dioxide (20 mg, 10% wt.). The reaction solution was purged with H₂ (×3) and stirred at rt under H₂ for 0.5 h. Upon completion, the reaction solution was filtered, and washed with MeOH (×3), and the filtrate was concentrated under reduced pressure. The residue was purified by column chromatography to afford the title compound (yellow solid, 100 mg, yield 33%).

### Step E: N-(3-Carbonyl-1-((1r,4r)-4-(piperidin-4-ylmethyl)cyclohexyl)-1H-pyrazol-4-yl)-6-(trifluoromethyl)picolinamide

At 0°C, to a solution of tert-butyl 4-(((1r,4r)-4-(3-carbonyl-4-(6-(trifluoromethyl)picolinamido)-1H-pyrazol-1-yl)cyclohexyl)methyl)piperidine-1-carboxylate (100 mg, 0.17 mmol, 1.0 eq) in DCM (2 mL) was added TFA (2 mL). The reaction solution was stirred at rt for 0.5 h. Upon completion, the reaction solution was poured into NaHCO₃ solution and extracted with DCM (×3). The combined organic phases were washed with saturated aqueous NaCl, dried over anhydrous Na₂SO₄, filtered, and concentrated under reduced pressure to yield the title compound (white solid, 78 mg, crude).

### Step F: Tert-butyl 3-((4-(((1r,4r)-4-(3-carbamoyl-4-(6-(trifluoromethyl)picolinamido)-1H-pyrazol-1-yl)cyclohexyl)methyl)piperidin-1-yl)methyl)pyrrolidine-1-carboxylate

At 0°C, to a solution of N-(3-carbonyl-1-((1r,4r)-4-(piperidin-4-ylmethyl)cyclohexyl)- 1 H-pyrazol-4-yl)-6-(trifluoromethyl)picolinamide (100 mg, 0.17 mmol, 1.0 eq) in 1,2-dichloroethane (2.0 mL) and MeOH (2.0 mL) was added tert-butyl 3-formylpyrrolidine-1-carboxylate (62.46 mg, 0.34 mmol, 2.0 eq). Sodium triacetoxyborohydride (109 mg, 0.515 mmol, 2.0 eq) and AcOH (0.2 mL) were then added, and the reaction was allowed to proceed at rt for 0.5 h. Upon completion, the reaction solution was concentrated under reduced pressure. The residue was purified by column chromatography to afford the title compound (yellow oil, 80 mg, yield 58%).

### Step G: N-(3-Carbamoyl-1-((1r,4r)-4-((1-(pyrrolidin-3-ylmethyl)piperidin-4-yl)methyl)cyclohexyl)-1H-pyrazol-4-yl)-6-(trifluoromethyl)picolinamide

To a solution of tert-butyl 3-((4-(((1r,4r)-4-(3-carbamoyl-4-(6-(trifluoromethyl)picolinamido)-1H-pyrazol-1-yl)cyclohexyl)methyl)piperidin-1-yl)methyl)pyrrolidine-1-carboxylate (80 mg, 0.12 mmol, 1.0 eq) in DCM (2 mL) was added TFA (2 mL) at 0°C. The reaction solution was stirred at rt for 0.5 h. Upon completion, the reaction solution was poured into NaHCO₃ solution and extracted with DCM (×3). The combined organic phases were washed with saturated aqueous NaCl, dried over anhydrous Na₂SO₄, filtered, and concentrated under reduced pressure to yield the title compound (yellow oil, 62 mg, crude).

### Step H: N-(3-carbamoyl-1-((1r,4r)-4-((1-(1-(3-(2,4-dioxotetrahydropyrimidin-1(2H)-yl)-4-methoxybenzoyl)pyrrolidin-3-yl)methyl)piperidin-4-ylmethyl)cyclohexyl)-1H-pyrazol-4-yl)-6-(trifluoromethyl)picolinamide

At 0°C, to a solution of N-(3-carbamoyl-1-((1r,4r)-4-((1-(pyrrolidin-3-ylmethyl)piperidin-4-yl)methyl)cyclohexyl)-1H-pyrazol-4-yl)-6-(trifluoromethyl)picolinamide (62 mg, 0.11 mmol, 1.0 eq) and 3-(2,4-dioxotetrahydropyrimidin-1(2H)-yl)-4-methoxybenzoic acid (32 mg, 0.12 mmol, 1.1 eq) in DMF (3 mL) were added DIPEA (82 mg, 0.33 mmol, 3.0 eq) and HATU (63 mg, 0.16 mmol, 1.5 eq). The reaction solution was reacted at rt under N₂ for 0.5 h. Upon completion, the reaction solution was concentrated under reduced pressure. The residue was purified by HPLC to afford the title compound (white solid, 14.3 mg, yield 16%). **¹H NMR** (400 MHz, CDCl₃) δ 11.66 (s, 1H), 8.43-8.31 (m, 2H), 8.16-8.04 (m, 1H), 7.85 (d, *J* = 7.2 Hz, 1H), 7.64-7.41 (m, 3H), 7.03-6.96 (m, 1H), 6.80-6.58 (m, 1H), 5.56-5.36 (m, 1H), 4.18-4.01 (m, 1H), 3.98-3.83 (m, 4H), 3.77-3.54 (m, 4H), 3.40-3.11 (m, 3H), 2.88-2.71 (m, 3H), 2.68-2.53 (m, 2H), 2.38-2.06 (m, 6H), 1.95-1.86 (m, 3H), 1.39-1.17 (m, 8H), 1.14-1.05 (m, 2H), 0.89-0.81 (m, 1H). LCMS (ESI): m/z 808 [M+H]⁺.

### Preparation Example 247. Synthesis of Compound A163

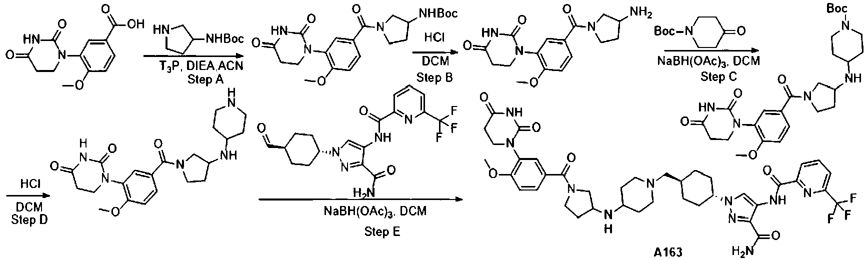

### Step A: Tert-butyl (1-(3-(2,4-dioxotetrahydropyrimidin-1(2H)-yl)-4-methoxybenzoyl)pyrrolidin-3-yl)carbamate

To a solution of 3-(2,4-dioxotetrahydropyrimidin-1(2H)-yl)-4-methoxybenzoic acid (500 mg, 1.89 mmol, 1.0 eq) and tert-butyl pyrrolidin-3-ylcarbamate (388 mg, 2.081 mmol, 1.1 eq) in acetonitrile (10 mL) at rt were added 50 wt% T₃P in EtOAc (900 mg, 2.83 mmol, 1.5 eq) and DIEA (980 mg, 7.57 mmol, 4.0 eq). The reaction solution was stirred at rt under N₂ for 18 h. Upon completion, the mixture was diluted in water, extracted with EtOAc (×3), washed with saturated aqueous NaCl, dried over anhydrous Na₂SO₄, filtered, and concentrated under reduced pressure. The residue was purified by column chromatography to afford the title compound (white solid, 600 mg, yield 73%).

### Step B: 1-(5-(3-aminopyrrolidine-1-carbonyl)-2-methoxyphenyl)dihydropyrimidine-2,4(1H,3H)-dione

At 0°C, to a solution of tert-butyl (1-(3-(2,4-dioxotetrahydropyrimidin-1(2H)-yl)-4-methoxybenzoyl)pyrrolidin-3-yl)carbamate (600 mg, 1.38 mmol, 1.0 eq) in DCM (10 mL) was added 4 M HCl in 1,4-dioxane (5 mL, 20 mmol, 15 eq). The reaction solution was stirred at rt under N₂ for 1 h. The reaction solution was concentrated under reduced pressure to afford the title compound (white solid, 500 mg, yield 95%).

### Step C: Tert-butyl 4-((1-(3-(2,4-dioxotetrahydropyrimidin-1(2H)-yl)-4-methoxybenzoyl)pyrrolidin-3-yl)amino)piperidine-1-carboxylate

To a solution of 1-(5-(3-aminopyrrolidine-1-carbonyl)-2-methoxyphenyl)dihydropyrimidine-2,4(1H,3H)-dione (500 mg, 1.5 mmol, 1.0 eq) in DCM (5 mL) was added TEA (0.21 mL, 1.5 mmol, 1.0 eq) at rt, and the mixture was stirred at rt for 15 min. AcOH (0.5 mL) and tert-butyl 4-oxopiperidine-1-carboxylate (900 mg, 4.51 mmol, 3.0 eq) were then added. The mixture was stirred at rt for 18 h. Sodium triacetoxyborohydride (950 mg, 4.51 mmol, 3.0 eq) was then added to the above reaction solution. The mixture was stirred at rt for 1 h. Upon completion, the mixture was diluted in water, extracted with DCM (×3), washed with saturated aqueous NaCl, dried over anhydrous Na₂SO₄, filtered, and concentrated under reduced pressure. The residue was purified by column chromatography to afford the title compound (white solid, 300 mg, yield 38%).

### Step D: 1-(2-methoxy-5-(3-(piperidin-4-ylamino)pyrrolidine-1-carbonyl)phenyl)dihydropyrimidine-2,4(1H,3H)-dione

At 0°C, to a solution of tert-butyl 4-((1-(3-(2,4-dioxotetrahydropyrimidin-1(2H)-yl)-4-methoxybenzoyl)pyrrolidin-3-yl)amino)piperidine-1-carboxylate (300 mg, 0.58 mmol, 1.0 eq) in DCM (5 mL) was added 4 M HCl in 1,4-dioxane (1.5 mL, 6.0 mmol, 10.0 eq). The reaction solution was stirred at rt under N₂ for 1 h. The reaction solution was concentrated under reduced pressure to afford the title compound (white solid, 240 mg, yield 91%). **Step E: N-(3-carbamoyl-1-((1r,4r)-4-((4-((1-(3-(2,4-dioxotetrahydropyrimidin-1(2H)-yl)-4-methoxybenzoyl)pyrrolidin-3-yl)amino)piperidin-1-yl)methyl)cyclohexyl)-1H-pyrazol-4-yl)-6-(trifluoromethyl)picolinamide**

To a solution of 1-(2-methoxy-5-(3-(piperidin-4-ylamino)pyrrolidine-1-carbonyl)phenyl)dihydropyrimidine-2,4(1H,3H)-dione (240 mg, 0.72 mmol, 1.0 eq) in DCM (5 mL) was added TEA (0.100 mL, 0.72 mmol, 1.0 eq) at rt, and the mixture was stirred at rt for 15 min. AcOH (0.5 mL) and N-(3-carbonyl-1-((1r,4r)-4-formylcyclohexyl)-1H-pyrazol-4-yl)-6-(trifluoromethyl)picolinamide (355 mg, 0.87 mmol, 1.2 eq) were then added. The mixture was stirred at rt for 18 h. Sodium triacetoxyborohydride (457 mg, 2.17 mmol, 3.0 eq) was then added to the above reaction solution. The mixture was stirred at rt for 1 h. Upon completion, the mixture was diluted in water, extracted with DCM (×3), washed with saturated aqueous NaCl, dried over anhydrous Na₂SO₄, filtered, and concentrated under reduced pressure. The residue was purified by HPLC to afford the title compound (white solid, 148 mg, yield 25%). **¹H** NMR (400 MHz, DMSO-*d*₆) *δ* 11.71 (s, 1H), 10.33 (s, 1H), 8.48-8.30 (m, 3H), 8.24-8.15 (m, 1H), 7.64 (s, 1H), 7.51 (dd, *J=* 14.4, 12.4 Hz, 3H), 7.15 (d, *J=* 8.4 Hz, 1H), 4.32-4.15 (m, 1H), 3.85 (s, 3H), 3.67-3.51 (m, 4H), 3.44 (s, 2H), 3.23-3.15 (m, 1H), 2.84-2.63 (m, 4H), 2.37-2.18 (m, 1H), 2.16-2.01 (m, 4H), 1.97-1.49 (m, 12H), 1.32-0.88 (m, 4H). LCMS (ESI): m/z 809 [M+H]⁺.

### Preparation Example 248. Synthesis of Compound A164

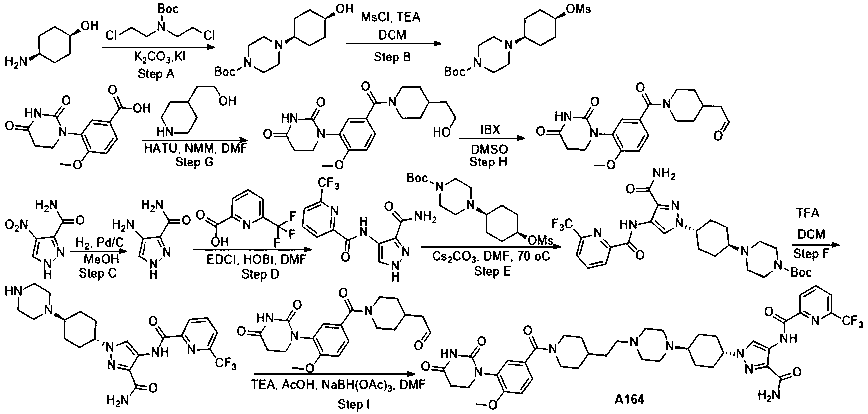

### Step A: Tert-butyl 4-((1s,4s)-4-hydroxycyclohexyl)piperazine-1-carboxylate

To a solution of tert-butyl bis(2-chloroethyl)carbamate (10.0 g, 41.3 mmol, 1.0 eq) and (1s,4s)-4-aminocyclohexan-1-ol (5.0 g, 43.8 mmol, 1.1 eq) in 1,4-dioxane (100 mL) were added potassium carbonate (17.1 g, 124 mmol, 3.0 eq) and potassium iodide (20.6 g, 124 mmol, 3.0 eq) at rt. The reaction solution was stirred at 110°C under N₂ for 16 h. Upon completion, the reaction solution was cooled to rt, filtered, and concentrated under reduced pressure. The residue was purified by column chromatography to afford the title compound (yellow solid, 800 mg, yield 7%).

### Step B: Tert-butyl 4-((1s,4s)-4-((methylsulfonyl)oxy) cyclohexyl)piperazine-1-carboxylate

At 0°C, to a solution of tert-butyl 4-((1s,4s)-4-hydroxycyclohexyl)piperazine-1-carboxylate (438 mg, 1.5 mmol, 1.0 eq) in DCM (10 mL) were added TEA (0.3 mL, 2.3 mmol, 1.5 eq) and methanesulfonyl chloride (211.7 mg, 1.8 mmol, 1.2 eq). The reaction solution was stirred at rt under N₂ for 3 h. Upon completion, the reaction solution was quenched with water and extracted with DCM (×3). The combined organic phases were washed sequentially with water and saturated aqueous NaCl, dried over anhydrous Na₂SO₄, filtered, and concentrated under reduced pressure at low temperature to afford the title compound (white solid, 410 mg, crude), which was used directly without purification.

### Step C: 4-amino-1H-pyrazole-3-carboxamide

To a solution of 4-nitro-1H-pyrazole-3-carboxamide (2 g, 12.8 mmol, 1.0 eq) in MeOH (25 mL) was added 10% palladium on carbon (200 mg) at rt. The reaction solution was purged with H₂ (using a hydrogen balloon) (×3) and stirred under H₂ at rt for 3 h. Upon completion, the mixture was filtered, and the filtrate was concentrated under reduced pressure to afford the title compound (colorless oil, 1.4 g, crude), which was used directly without purification.

### Step D: N-(3-amino-1H-pyrazol-4-yl)-6-(trifluoromethyl)picolinamide

To a solution of 4-amino-1H-pyrazole-3-carboxamide (1.4 g, 11.1 mmol, 1.0 eq) and 6-(trifluoromethyl)pyridine-2-carboxylic acid (2.3 g, 12.2 mmol, 1.0 eq) in DMF (15 mL) at rt were added EDCI (3.2 g, 16.7 mmol, 1.5 eq), HOBt (2.3 g, 16.7 mmol, 1.5 eq), and TEA (4.6 mL, 33.3 mmol, 3.0 eq). The reaction solution was stirred at rt under N₂ for 3 h. Upon completion, the reaction solution was diluted in EtOAc, washed sequentially with water and saturated aqueous NaCl, dried over anhydrous Na₂SO₄, filtered, and concentrated under reduced pressure. The residue was purified by column chromatography to afford the title compound (yellow solid, 1.9 g, yield 57%).

### Step E: Tert-butyl 4-((1r,4r)-4-(3-carbonyl-4-(6-(trifluoromethyl)picolinamido)-1H-pyrazol-1-yl)cyclohexyl)piperazine-1-carboxylate

To a solution of N-(3-amino-1H-pyrazol-4-yl)-6-(trifluoromethyl)picolinamide (310.4 mg, 1.0 mmol, 1.0 eq) in DMF (8 mL) at rt were added tert-butyl 4-((1s,4s)-4-((methylsulfonyl)oxy) cyclohexyl)piperazine-1-carboxylate (376 mg, 1.0 mmol, 1.0 eq) and cesium carbonate (506.9 mg, 1.6 mmol, 1.6 eq). The reaction solution was stirred at 70°C under N₂ for 3 h. Upon completion, the reaction was quenched with saturated aqueous NH₄Cl and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 120 mg, yield 20%).

### Step F: N-(3-amino-1-((1r,4r)-4-(piperazin-1-yl)cyclohexyl)-1H-pyrazol-4-yl)-6-(trifluoromethyl)picolinamide

At 0°C, to a solution of tert-butyl 4-((1r,4r)-4-(3-carbonyl-4-(6-(trifluoromethyl)picolinamido)-1H-pyrazol-1-yl)cyclohexyl)piperazine-1-carboxylate (110 mg, 0.19 mmol, 1.0 eq) in DCM (2 mL) was added TFA (3 mL). The reaction solution was stirred at rt under N₂ for 1 h. The reaction solution was concentrated under reduced pressure to afford the title compound (yellow solid, 90 mg, crude), which was used directly without purification.

### Step G: 1-(5-(4-(2-hydroxyethyl)piperidine-1-carbonyl)-2-methoxyphenyl)dihydropyrimidine-2,4(1H,3H)-dione

To a solution of 3-(2,4-dioxohexahydropyrimidin-1-yl)-4-methoxybenzoic acid (1 g, 3.8 mmol, 1.0 eq), 2-(piperidin-4-yl)ethan-1-ol (0.5 g, 4.2 mmol, 1.2 eq), and 4-methylmorpholine (2.1 mL) in DMF (12 mL) was added HATU (2.1 g, 5.7 mmol, 1.5 eq) at rt. The reaction solution was stirred at rt under N₂ for 2 h. Upon completion, the reaction solution was diluted in EtOAc, washed sequentially with water and saturated aqueous NaCl, dried over anhydrous Na₂SO₄, filtered, and concentrated under reduced pressure. The residue was purified by column chromatography to afford the title compound (white solid, 1 g, yield 70%).

### Step H: 2-(1-(3-(2,4-dioxotetrahydropyrimidin-1(2H)-yl)-4-methoxybenzoyl)piperidin-4-yl)acetaldehyde

To a solution of 1-(5-(4-(2-hydroxyethyl)piperidine-1-carbonyl)-2-methoxyphenyl)dihydropyrimidine-2,4(1H,3H)-dione (600 mg, 1.6 mmol, 1.0 eq) in dimethyl sulfoxide (6 mL) at rt was added IBX (895 mg, 3.2 mmol, 2.0 eq). The reaction solution was stirred at rt for 0.5 h. Upon completion, the reaction solution was diluted in EtOAc, washed sequentially with water and saturated aqueous NaCl, dried over anhydrous Na₂SO₄, filtered, and concentrated under reduced pressure. The residue was purified by reverse-phase column chromatography to afford the title compound (white solid, 500 mg, yield 83%).

### Step I: N-(3-amino-1-((1r,4r)-4-(4-(2-(1-(3-(2,4-dioxotetrahydropyrimidin-1(2H)-yl)-4-methoxybenzoyl)piperidin-4-yl)ethyl)piperazin-1-yl)cyclohexyl)-1H-pyrazol-4-yl)-6-(trifluoromethyl)picolinamide

At rt, to a solution of 2-(1-(3-(2,4-dioxotetrahydropyrimidin-1(2H)-yl)-4-methoxybenzoyl)piperidin-4-yl)acetaldehyde (100 mg, 0.27 mmol, 1.0 eq) and N-(3-amino-1-((1r,4r)-4-(piperazin-1-yl)cyclohexyl)-1H-pyrazol-4-yl)-6-(trifluoromethyl)picolinamide (124.6 mg, 0.27 mmol, 1.0 eq) in DMF (3 mL) was added TEA (0.2 mL), and the mixture was stirred for 15 min. AcOH (0.2 mL) was then added. The mixture was stirred at rt for 5 h. Sodium triacetoxyborohydride (85.2 mg, 0.40 mmol, 1.5 eq) was then added to the above reaction solution. The mixture was stirred at rt for 1 h. Upon completion, the mixture was diluted in water, extracted with EtOAc (×3), washed with saturated aqueous NaCl, dried over anhydrous Na₂SO₄, filtered, and concentrated under reduced pressure. The residue was purified by HPLC to afford the title compound (white solid, formate, 33.8 mg, yield 15%). **¹H** NMR (400 MHz, DMSO-*d*₆) δ 11.71 (s, 1H), 10.34 (s, 1H), 8.46-8.31 (m, 3H), 8.20-8.17 (m, 1H), 8.16 (s, 1H), 7.64 (s, 1H), 7.55 (s, 1H), 7.36 (dd, *J* = 8.4, 2.0 Hz, 1H), 7.32 (d, *J* = 2.0 Hz, 1H), 7.15 (d, *J* = 8.4 Hz, 1H), 4.71-4.05 (m, 4H), 3.84 (s, 3H), 3.61 (d, *J* = 6.8 Hz, 2H), 3.07-2.77 (m, 2H), 2.68 (t, *J* = 6.8 Hz, 2H), 2.57 (ddd, *J=* 15.2, 9.2, 5.2 Hz, 4H), 2.47-2.31 (m, 6H), 2.17-2.08 (m, 2H), 1.98-1.78 (m, 4H), 1.75-1.50 (m, 3H), 1.47-1.32 (m, 4H), 1.18-1.01 (m, 2H). LCMS (ESI): m/z 823 [M+H]⁺.

### Preparation Example 249. Synthesis of Compound A165

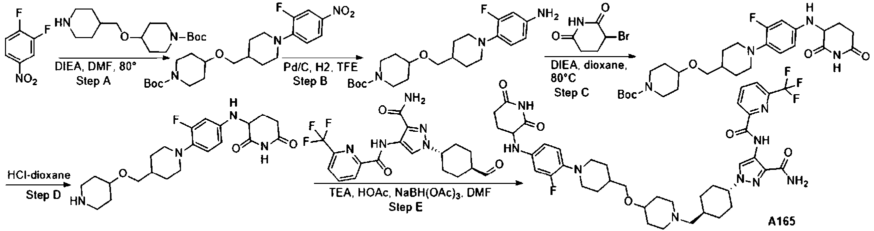

### Step A: Tert-butyl 4-((1-(2-fluoro-4-nitrophenyl)piperidin-4-yl)methoxy)piperidine-1-carboxylate

To a solution of 1,2-difluoro-4-nitrobenzene (128 mg, 0.8 mmol, 1.1 eq) in DMF (10 mL) at rt were added tert-butyl 4-(piperidin-4-ylmethoxy)piperidine-1-carboxylate (200 mg, 0.7 mmol, 1.0 eq) and DIEA (173 mg, 1.3 mmol, 2.0 eq). The reaction solution was stirred at 80°C under N₂ for 5 h. Upon completion, the reaction solution was cooled to rt, diluted in EtOAc, washed sequentially with water and saturated aqueous NaCl, dried over anhydrous Na₂SO₄, filtered, and concentrated under reduced pressure. The residue was purified by column chromatography to afford the title compound (yellow oil, 200 mg, yield 68%).

### Step B: Tert-butyl 4-((1-(4-amino-2-fluorophenyl)piperidin-4-yl)methoxy)piperidine-1-carboxylate

To a solution of tert-butyl 4-((1-(2-fluoro-4-nitrophenyl)piperidin-4-yl)methoxy)piperidine-1-carboxylate (200 mg, 0.5 mmol, 1 eq) in 2,2,2-trifluoroethanol (10 mL) was added 10% palladium on carbon (49 mg, 0.5 mmol, 1.0 eq) at rt. The reaction solution was purged with H₂ (using a hydrogen balloon) (×3) and stirred under H₂ at rt for 2 h. Upon completion, the mixture was filtered, and the filtrate was concentrated under reduced pressure to afford the title compound (yellow oil, 150 mg, crude), which was used directly without purification.

### Step C: Tert-butyl 4-((1-(4-((2,6-dioxopiperidin-3-yl)amino)-2-fluorophenyl)piperidin-4-yl)methoxy)piperidine-1-carboxylate

To a solution of tert-butyl 4-((1-(4-amino-2-fluorophenyl)piperidin-4-yl)methoxy)piperidine-1-carboxylate (150 mg, 0.4 mmol, 1.0 eq) in 1,4-dioxane (2 mL) at rt were added 3-bromopiperidine-2,6-dione (141 mg, 0.8 mmol, 2.0 eq) and DIEA (95 mg, 0.8 mmol, 2.0 eq). The reaction solution was stirred at 80°C under N₂ for 16 h. Upon completion, the reaction solution was cooled to rt, diluted in EtOAc, washed sequentially with water and saturated aqueous NaCl, dried over anhydrous Na₂SO₄, filtered, and concentrated under reduced pressure. The residue was purified by column chromatography to afford the title compound (blue oil, 70 mg, yield 37%).

### Step D: 3-((3-fluoro-4-(4-(piperidin-4-yloxy)methyl)piperidin-1-yl)phenyl)amino)piperidine-2,6-dione

To tert-butyl 4-((1-(4-((2,6-dioxopiperidin-3-yl)amino)-2-fluorophenyl)piperidin-4-yl)methoxy)piperidine-1-carboxylate (70 mg, 0.1 mmol, 1.0 eq) was added 4 M HCl in 1,4-dioxane (3 mL) at 0°C. The reaction solution was stirred at rt under N₂ for 1 h. The reaction solution was concentrated under reduced pressure to afford the title compound (white solid, 50 mg, crude), which was used directly without purification.

### Step E: N-(3-carbamoyl-1-((1r,4r)-4-((4-(1-(4-((2,6-dioxopiperidin-3-yl)amino)-2-fluorophenyl)piperidin-4-yl)methoxy)piperidin-1-yl)methyl)cyclohexyl)-1H-pyrazol-4-yl)-6-(trifluoromethyl)picolinamide

To a solution of 3-((3-fluoro-4-(4-(piperidin-4-yloxy)methyl)piperidin-1-yl)phenyl)amino)piperidine-2,6-dione (50 mg, 0.1 mmol, 1.0 eq) in DMF (5 mL) was added TEA (0.02 mL, 0.1 mmol, 1.0 eq) at rt, and the mixture was stirred for 15 min. AcOH (0.007 mL, 0.1 mmol, 1.0 eq) and N-(3-carbonyl-1-((1r,4r)-4-formylcyclohexyl)-1H-pyrazol-4-yl)-6-(trifluoromethyl)picolinamide (49 mg, 0.1 mmol, 1.0 eq) were then added. The mixture was stirred at rt for 0.5 h. Sodium triacetoxyborohydride (51 mg, 0.2 mmol, 2.0 eq) was then added to the above reaction solution. The mixture was stirred at rt for 1 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (yellow solid, 15 mg, yield 15%). **¹H** NMR (400 MHz, DMSO-*d*₆) δ 11.70 (s, 1H), 10.76 (s, 1H), 8.44-8.34 (m, 3H), 8.22-8.17 (m, 1H), 7.64 (s, 1H), 7.53 (s, 1H), 6.90-6.76 (m, 1H), 6.58-6.37 (m, 2H), 5.80-5.72 (m, 1H), 4.30-4.15 (m, 2H), 3.28 (d, *J=* 6.2 Hz, 2H), 3.10 (d, *J= 10.8* Hz, 2H), 2.76-2.58 (m, 5H), 2.14-2.01 (m, 8H), 1.93-1.70 (m, 9H), 1.62-1.27 (m, 7H), 1.11-1.02 (m, 2H). LCMS (ESI): m/z 812 [M+H]⁺.

### Preparation Example 250. Synthesis of Compound A166

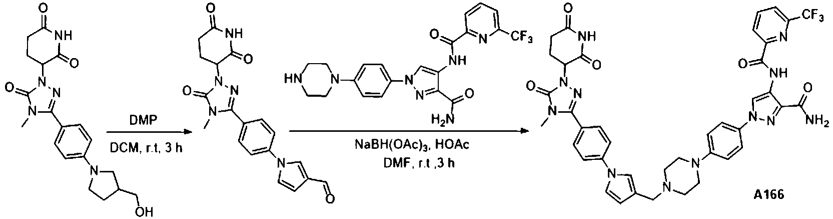

### Step A: 1-(4-(1-(2,6-dioxopiperidin-3-yl)-4-methyl-5-oxo-4,5-dihydro-1H-1,2,4-triazol-3-yl)phenyl)-1H-pyrrole-3-carbaldehyde

To a solution of 3-(3-(4-(3-(hydroxymethyl)pyrrolidin-1-yl)phenyl)-4-methyl-5-oxo-4,5-dihydro-1H-1,2,4-triazol-1-yl)piperidine-2,6-dione (100 mg, 0.26 mmol, 1.0 eq) in DCM (4 mL) was added Dess-Martin periodinane (220 mg, 0.52 mmol, 2.0 eq) at 0°C. The mixture was stirred at rt for 3 h. Upon completion, the reaction solution was filtered, and the filtrate was concentrated under pressure. The residue was purified by thin layer chromatography to afford the title compound (white solid, 60 mg, yield 60%).

### Step B: N-(3-carbamoyl-1-(4-((1-(4-(1-(2,6-dioxopiperidin-3-yl)-4-methyl-5-oxo-4,5-dihydro-1H-1,2,4-triazol-3-yl)phenyl)-1H-pyrrol-3-yl)methyl)piperazin-1-yl)phenyl]-1H-pyrazol-4-yl)-6-(trifluoromethyl)picolinamide

To a solution of 1-(4-(1-(2,6-dioxopiperidin-3-yl)-4-methyl-5-oxo-4,5-dihydro-1H-1,2,4-triazol-3-yl)phenyl)-1H-pyrrole-3-carbaldehyde (60 mg, 0.16 mmol, 1.0 eq) and N-(3-amino-1-((1r,4r)-4-(piperazin-1-yl)cyclohexyl)-1H-pyrazol-4-yl)-6-(trifluoromethyl)picolinamide (57.5 mg, 0.12 mmol, 0.8 eq) in DMF (2 mL) was added AcOH (2.81 mg, 0.05 mmol, 0.3 eq) at rt. The mixture was stirred at rt for 0.5 h. Sodium triacetoxyborohydride (66 mg, 0.31 mmol, 2.0 eq) was then added to the above reaction solution. The mixture was stirred at rt for 3 h. Upon completion, the mixture was diluted in water, extracted with EtOAc (×3), washed with saturated aqueous NaCl, dried over anhydrous Na₂SO₄, filtered, and concentrated under reduced pressure. The residue was purified by HPLC to afford the title compound (white solid, 9.5 mg, yield 7%). **¹H** NMR (400 MHz, DMSO-*d*₆) δ 11.79 (s, 1H), 11.05 (s, 1H), 8.87 (s, 1H), 8.46-8.38 (m, 2H), 8.21 (d, *J* = 7.6 Hz, 1H), 7.95 (s, 1H), 7.79-7.72 (m, 6H), 7.71 (s, 1H), 7.45 (d, *J* = 12.8 Hz, 2H), 7.07 (d, *J* = 8.4 Hz, 2H), 6.30 (s, 1H), 5.24 (dd, *J=* 12.8, 5.2 Hz, 1H), 3.45 (s, 3H), 3.41(s, 2H), 3.23(s, 4H), 2.93-2.87 (m, 1H), 2.87-2.65 (m, 5H), 2.65-2.57(m, 1H), 2.15 (s, 1H). LCMS (ESI): m/z 823 [M+H]⁺.

### Preparation Example 251. Synthesis of Compound A167

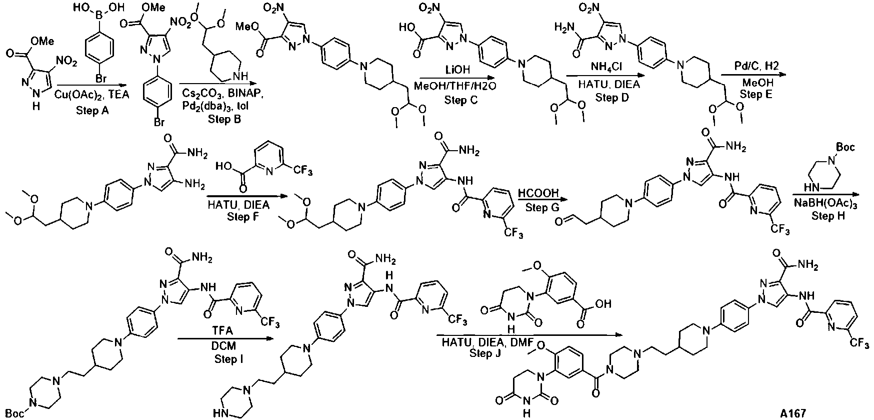

### Step A: Methyl 1-(4-bromophenyl)-4-nitro-1H-pyrazole-3-carboxylate

To a solution of 4-bromophenylboronic acid (2 g, 10 mmol, 1.0 eq) and AcOH (0.1 mL) in DCM (20 mL) at rt were added methyl 4-nitro-1H-pyrazole-3-carboxylate (3.41 g, 20 mmol, 2.0 eq), TEA (2.8 mL, 20 mmol), copper acetate (2.7 g, 15 mmol, 1.0 eq), and 4A molecular sieves (2 g). The reaction solution was stirred at rt under O₂ for 16 h. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by column chromatography to afford the title compound (yellow solid, 1.4 g, yield 43%).

### Step B: Methyl 1-(4-(4-(2,2-dimethoxyethyl)piperidin-1-yl)phenyl)-4-nitro-1H-pyrazole-3-carboxylate

To a solution of methyl 1-(4-bromophenyl)-4-nitro-1H-pyrazole-3-carboxylate (700 mg, 2.15 mmol, 1.0 eq) and 4-(2,2-dimethoxyethyl)piperidine (559 mg, 3.23 mmol, 1.5 eq) in toluene (20 mL) at rt were added BINAP (267.7 mg, 0.43 mmol, 0.2 eq), Pd₂(dba)₃ (196 mg, 0.22 mmol, 0.1 eq), and cesium carbonate (2.1 g, 6.45 mmol, 3.0 eq). The reaction solution was stirred at 110°C under N₂ for 16 h. Upon completion, the reaction solution was cooled to rt, poured into water, and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow oil, 400 mg, yield 44%).

### Step C: 1-(4-(4-(2,2-dimethoxyethyl)piperidin-1-yl)phenyl)-4-nitro-1H-pyrazole-3-carboxylic acid

To a solution of methyl 1-(4-(4-(2,2-dimethoxyethyl)piperidin-1-yl)phenyl)-4-nitro-1H-pyrazole-3-carboxylate in MeOH/tetrahydrofuran/water (16 mL, 10/1/5) was added lithium hydroxide (8.6 mg, 0.36 mmol, 1.5 eq) at rt. The reaction solution was stirred at rt for 1 h. Upon completion, the reaction solution was concentrated under reduced pressure to afford the title compound (yellow solid, 92 mg, yield 95%).

### Step D: 1-(4-(4-(2,2-dimethoxyethyl)piperidin-1-yl)phenyl)-4-nitro-1H-pyrazole-3-carboxamide

To a solution of 1-(4-(4-(2,2-dimethoxyethyl)piperidin-1-yl)phenyl)-4-nitro-1H-pyrazole-3-carboxylic acid (92 mg, 0.23 mmol, 1.0 eq) and ammonium chloride (13.8 mg, 0.27 mmol, 1.2 eq) in DMF (2.0 mL) at 0°C were added DIEA (119 mg, 0.92 mmol, 4 eq) and HATU (173 mg, 0.46 mmol, 2.0 eq). The reaction solution was reacted at rt for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 80 mg, yield 87%).

### Step E: 4-amino-1-(4-(4-(2,2-dimethoxyethyl)piperidin-1-yl)phenyl)-1H-pyrazole-3-carboxamide

To a solution of 1-(4-(4-(2,2-dimethoxyethyl)piperidin-1-yl)phenyl)-4-nitro-1H-pyrazole-3-carboxamide (80 mg, 0.2 mmol, 1.0 eq) in MeOH (15 mL) was added 10% palladium on carbon (24 mg) at rt. The reaction solution was purged with H₂ (×3). The mixture was reacted at rt for 1 h under H₂. Upon completion, the reaction solution was filtered, the filter cake was washed with MeOH (×3), and the filtrate was concentrated under reduced pressure and purified to afford the title compound (yellow solid, 68 mg, yield 91%).

### Step F: N-(3-carbonyl-1-(4-(4-(2,2-dimethoxyethyl)piperidin-1-yl)phenyl)-1H-pyrazol-4-yl)-6-(trifluoromethyl)picolinamide

To a solution of 4-amino-1-(4-(4-(2,2-dimethoxyethyl)piperidin-1-yl)phenyl)-1H-pyrazole-3-carboxamide (68 mg, 0.18 mmol, 1.0 eq) and 6-(trifluoromethyl)picolinic acid (34.4 mg, 0.18 mmol, 1 eq) in DMF (2.0 mL) at 0°C were added DIEA (93 mg, 0.72 mmol, 4 eq) and HATU (138 mg, 0.36 mmol, 2.0 eq). The reaction solution was reacted at rt for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 80 mg, yield 81%).

### Step G: N-(3-Carbonyl-1-(4-(4-(2-oxoethyl)piperidin-1-yl)phenyl)-1H-pyrazol-4-yl)-6-(trifluoromethyl)picolinamide

A mixed solution of N-(3-carbonyl-1-(4-(4-(2,2-dimethoxyethyl)piperidin-1-yl)phenyl)-1H-pyrazol-4-yl)-6-(trifluoromethyl)picolinamide (80 mg, 0.15 mmol, 1.0 eq) in formic acid (5 mL) was reacted at rt for 2 h. Upon completion, the reaction solution was concentrated under reduced pressure to afford the title compound (yellow solid, 70 mg, yield 93%).

### Step H: Tert-butyl 4-(2-(1-(4-(3-amino-4-(6-(trifluoromethyl)picolinamido)-1H-pyrazol-1-yl)phenyl)piperidin-4-yl)ethyl)piperazine-1-carboxylate

To a solution of N-(3-carbonyl-1-(4-(4-(2-oxoethyl)piperidin-1-yl)phenyl)-1H-pyrazol-4-yl)-6-(trifluoromethyl)picolinamide (70 mg, 0.14 mmol, 1.0 eq) and tert-butyl piperazine-1-carboxylate (31.2 mg, 0.17 mmol, 1.2 eq) in DMF (2.0 mL) was added AcOH (0.004 mL, 0.07 mmol, 0.5 eq) at rt. The reaction solution was reacted at rt for 1 h. Sodium triacetoxyborohydride (115 mg, 0.56 mmol, 4.0 eq) was then added, and the reaction was allowed to proceed at rt for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 65 mg, yield 69%).

### Step I: Tert-butyl piperazine-1-carboxylate N-(3-amino-1-(4-(2-(piperazin-1-yl)ethyl)piperidin-1-yl)phenyl)-1H-pyrazol-4-yl)-6-(trifluoromethyl)picolinamide

To a solution of tert-butyl 4-(2-(1-(4-(3-amino-4-(6-(trifluoromethyl)picolinamido)-1H-pyrazol-1-yl)phenyl)piperidin-4-yl)ethyl)piperazine-1-carboxylate (65 mg, 0.097 mmol, 1.0 eq) in DCM (10 mL) was added TFA (3 mL) at rt. The reaction solution was reacted at rt for 1 h. Upon completion, the reaction solution was concentrated under reduced pressure and purified to afford the title compound (yellow solid, 54 mg, crude).

### Step J: N-(3-carbamoyl-1-(4-(4-(3-(2,4-dioxotetrahydropyrimidin-1(2H)-yl)-4-methoxybenzoyl)piperazin-1-yl)ethyl)piperidin-1-yl)phenyl)-1H-pyrazol-4-yl)-6-(trifluoromethyl)picolinamide

To a solution of tert-butyl piperazine-1-carboxylate N-(3-amino-1-(4-(2-(piperazin-1-yl)ethyl)piperidin-1-yl)phenyl)-1H-pyrazol-4-yl)-6-(trifluoromethyl)picolinamide (54 mg, 0.095 mmol, 1.0 eq) and 3-(2,4-dioxotetrahydropyrimidin-1(2H)-yl)-4-methoxybenzoic acid (25 mg, 0.095 mmol, 1 eq) in DMF (2.0 mL) were added DIEA (49 mg, 0.38 mmol, 4 eq) and HATU (71.8 mg, 0.189 mmol, 2.0 eq) at 0°C. The reaction solution was reacted at rt for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (white solid, 7 mg, yield 9%). **¹H NMR** (400 MHz, DMSO-*d*₆) *δ* 11.79 (s, 1H), 10.37 (s, 1H), 8.87 (s, 1H), 8.49-8.35 (m, 2H), 8.21 (d, *J=* 7.5 Hz, 1H), 7.93 (s, 1H), 7.81-7.66 (m, 3H), 7.43 (d, *J* = 17.0 Hz, 2H), 7.20 (d, *J=* 8.3 Hz, 1H), 7.08 (d, *J=* 9.0 Hz, 2H), 3.86 (s, 3H), 3.78 (m, 2H), 3.59 (m, 3H), 3.22-3.03 (m, 3H), 2.79-2.66 (m, 5H), 2.00 (m, 1H), 1.84-1.74 (m, 2H), 1.70-1.41 (m, 4H), 1.37-1.19 (m, 5H). LCMS (ESI): m/z 817 [M+H]⁺.

### Preparation Example 252. Synthesis of Compound A168

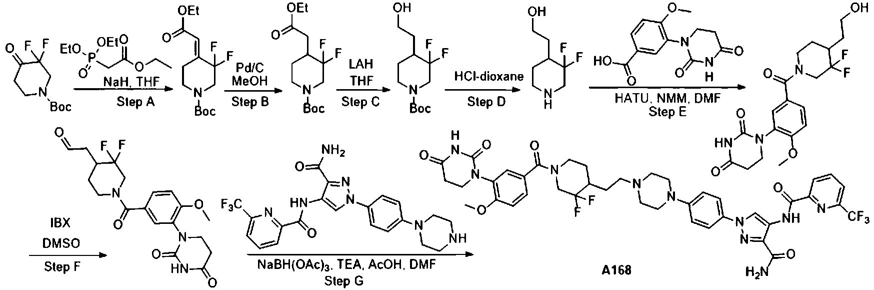

### Step A: Tert-butyl (E)-4-(2-ethoxy-2-oxoethylidene)-3,3-difluoropiperidine-1-carboxylate

To a solution of diethyl (2-ethoxy-2-oxoethyl)phosphonate (1.9 g, 8.5 mmol, 1.0 eq) in THF (80 mL), 60 wt% sodium hydride (0.2 g, 8.5 mmol, 1.0 eq) was added portionwise under N₂ at 0°C. The mixture was stirred at rt for 1 h. Tert-butyl 3,3-difluoro-4-oxopiperidine-1-carboxylate (2 g, 8.5 mmol, 1.0 eq) was then added. The mixture was stirred at 0°C under N₂ for 2 h. Upon completion, the reaction solution was quenched with water and extracted with EtOAc (×3). The combined organic phases were washed with water and saturated aqueous NaCl, dried over anhydrous Na₂SO₄, filtered, and concentrated under reduced pressure. The residue was purified by column chromatography to afford the title compound (yellow solid, 1.0 g, yield 39%).

### Step B: Tert-butyl 4-(2-ethoxy-2-oxoethyl)-3,3-difluoropiperidine-1-carboxylate

To a solution of tert-butyl (E)-4-(2-ethoxy-2-oxoethylidene)-3,3-difluoropiperidine-1-carboxylate (1.2 g, 3.9 mmol, 1.0 eq) in MeOH (25 mL) was added 10% palladium on carbon (400 mg) at rt. The reaction solution was purged with H₂ (using a hydrogen balloon) (×3) and stirred under H₂ at rt for 3 h. Upon completion, the mixture was filtered, and the filtrate was concentrated under reduced pressure to afford the title compound (yellow oil, 1.2 g, crude), which was used directly without purification.

### Step C: Tert-butyl 3,3-difluoro-4-(2-hydroxyethyl)piperidine-1-carboxylate

To a solution of tert-butyl 4-(2-ethoxy-2-oxoethyl)-3,3-difluoropiperidine-1-carboxylate (600 mg, 2.0 mmol, 1.0 eq) in THF (15 mL) was added lithium aluminum tetrahydride (148.2 mg, 3.9 mmol, 2.0 eq) in portions at 0°C under N₂. The mixture was stirred at rt for 0.5 h. The mixture was stirred at rt under N₂ for 0.5 h, Upon completion, the reaction solution was diluted in tetrahydrofuran, quenched with Na₂SO₄ decahydrate, and filtered. The filtrate was concentrated under reduced pressure to obtain the title compound (yellow oil, 400 mg, crude), which was used directly without purification.

### Step D: 2-(3,3-difluoropiperidin-4-yl)ethan-1-ol

To tert-butyl 3,3-difluoro-4-(2-hydroxyethyl)piperidine-1-carboxylate (400 mg, 1.5 mmol, 1.0 eq) was added 4 M HCl in 1,4-dioxane (5 mL) at 0°C. The reaction solution was stirred at rt under N₂ for 2 h. Upon completion, the reaction solution was concentrated under reduced pressure to afford the title compound (yellow oil, 200 mg, crude), which was used directly without purification.

### Step E: 1-(5-(3,3-difluoro-4-(2-hydroxyethyl)piperidine-1-carbonyl)-2-methoxyphenyl)dihydropyrimidine-2,4(1H,3H)-dione

To a solution of 3-(2,4-dioxohexahydropyrimidin-1-yl)-4-methoxybenzoic acid (319.9 mg, 1.2 mmol, 1.0 eq) and 2-(3,3-difluoropiperidin-4-yl)ethan-1-ol (200 mg, 1.2 mmol, 1.0 eq) in DMF (5 mL) were added HATU (690.6 mg, 1.8 mmol, 1.5 eq) and N-methylmorpholine (0.67 mL, 6.1 mmol, 5.0 eq) at rt. The mixture was stirred at rt for 1 h. Upon completion, the reaction solution was diluted in saturated aqueous NH₄Cl, and extracted with EtOAc (×3). The combined organic phases were washed sequentially with water and saturated aqueous NaCl, dried over anhydrous Na₂SO₄, and concentrated under reduced pressure. The residue was purified by column chromatography to afford the title compound (yellow solid, 300 mg, yield 60%).

### Step F: 2-(1-(3-(2,4-dioxotetrahydropyrimidin-1(2H)-yl)-4-methoxybenzoyl)-3,3-difluoropiperidin-4-yl)acetaldehyde

To a solution of 1-(5-(3,3-difluoro-4-(2-hydroxyethyl)piperidine-1-carbonyl)-2-methoxyphenyl)dihydropyrimidine-2,4(1H,3H)-dione (300 mg, 0.7 mmol, 1.0 eq) in dimethyl sulfoxide (5 mL) was added IBX (408.4 mg, 1.4 mmol, 2.0 eq) at rt. The reaction solution was stirred at rt for 1 h. Upon completion, the reaction solution was diluted in EtOAc, washed sequentially with water and saturated aqueous NaCl, dried over anhydrous Na₂SO₄, filtered, and concentrated under reduced pressure. The residue was purified by reverse-phase column chromatography to afford the title compound (yellow solid, 50 mg, yield 17%).

### Step G: N-(3-carbonyl-1-(4-(4-(2-(3-(2,4-dioxotetrahydropyrimidin-1(2H)-yl)-4-methoxybenzoyl)-3,3-difluoropiperidin-4-yl)ethyl)piperazin-1-yl)phenyl)-1H-pyrazol-4-yl)-6-(trifluoromethyl)picolinamide

At rt, to a solution of 1-[4-(piperazin-1-yl)phenyl]-4-({[6-(trifluoromethyl)pyridin-2-yl]carbonyl}amino)pyrazole-3-carboxamide (56.1 mg, 0.12 mmol, 1.0 eq) in DMF (5 mL) was added TEA (0.2 mL), and the mixture was stirred for 5 min. AcOH (0.3 mL) and 2-(1-(3-(2,4-dioxotetrahydropyrimidin-1(2H)-yl)-4-methoxybenzoyl)-3,3-difluoropiperidin-4-yl)acetaldehyde (50 mg, 0.12 mmol, 1.0 eq) were then added. The mixture was stirred at rt for 15 h. Sodium triacetoxyborohydride (51 mg, 0.24 mmol, 2.0 eq) was then added to the above reaction solution. The mixture was stirred at rt for 1 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (yellow solid, 24.6 mg, yield 24%). **¹H NMR** (400 MHz, DMSO-*d*₆) δ 11.79 (s, 1H), 10.35 (s, 1H), 8.88 (s, 1H), 8.47-8.36 (m, 2H), 8.26 (s, 1H), 8.21 (dd, *J* = 7.6, 1.2 Hz, 1H), 7.95 (s, 1H), 7.79 (d, *J* = 9.1 Hz, 2H), 7.71 (s, 1H), 7.40 (dd, *J* = 8.4, 2.0 Hz, 1H), 7.36 (d, *J* = 2.0 Hz, 1H), 7.19 (d, *J* = 8.6 Hz, 1H), 7.07 (d, *J* = 9.2 Hz, 2H), 3.98 (d, *J* = 44.6 Hz, 2H), 3.86 (s, 3H), 3.61 (t, *J* = 6.4 Hz, 2H), 3.21 (d, *J= 4.8* Hz, 6H), 2.69 (t, *J* = 6.6 Hz, 2H), 2.58-2.51 (m, 4H), 2.48-2.36 (m, 2H), 2.27-2.07 (m, 1H), 1.92 (s, 2H), 1.47-1.31 (m, 2H). LCMS (ESI): m/z 853 [M+H]⁺.

### Preparation Example 253. Synthesis of Compound A169

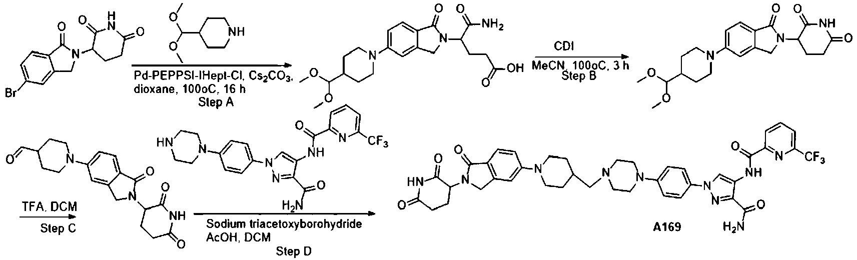

### Step A: 5-amino-4-(5-(4-(dimethoxymethyl)piperidin-1-yl)-1-oxoisoindol-2-yl)-5-oxopentanoic acid

To a solution of 3-(5-bromo-1-oxo-2,3-dihydro-1H-isoindol-2-yl)piperidine-2,6-dione (500 mg, 1.5 mmol, 1.0 eq) and 4-(dimethoxymethyl)piperidine (369 mg, 2.3 mmol, 1.5 eq) in 1,4-dioxane (10 mL) at rt were added cesium carbonate (1.5 g, 4.6 mmol, 3.0 eq) and Pd-PEPPSI-IHept-Cl (150 mg, 0.16 mmol, 0.1 eq). The mixture was stirred at 100°C under N₂ for 16 h. Upon completion, the mixture was cooled to rt and concentrated under reduced pressure. The residue was purified by column chromatography to afford the title compound (brown solid, 300 mg, yield 42%).

### Step B: 3-(5-(4-(dimethoxymethyl)piperidin-1-yl)-1-oxoisoindol-2-yl)piperidine-2,6-dione

To a solution of 5-amino-4-(5-(4-(dimethoxymethyl)piperidin-1-yl)-1-oxoisoindol-2-yl)-5-oxopentanoic acid (300 mg, 0.72 mmol, 1.0 eq) in acetonitrile (10 mL) was added N,N'-carbonyldiimidazole (231 mg, 1.4 mmol, 2.0 eq) at rt. The mixture was stirred at 100°C under N₂ for 3 h. Upon completion, the mixture was cooled to rt and concentrated under reduced pressure. The residue was purified by column chromatography to afford the title compound (brown solid, 300 mg, yield 42%).

### Step C: 1-(2-(2,6-dioxopiperidin-3-yl)-1-oxoisoindolin-5-yl)piperidine-4-carbaldehyde

To a solution of 3-(5-(4-(dimethoxymethyl)piperidin-1-yl)-1-oxoisoindol-2-yl)piperidine-2,6-dione (100 mg, 0.25 mmol, 1.0 eq) in DCM (5 mL) was added TFA (1 mL) at rt. The mixture was stirred at rt under N₂ for 0.5 h, Upon completion, the reaction solution was concentrated under reduced pressure to afford the title compound (yellow oil, 90 mg, crude), which was used directly without purification.

### Step D: N-(3-carbamoyl-1-(4-((1-(2-(2,6-dioxopiperidin-3-yl)-1-oxoisoindol-5-yl)piperidin-4-yl)methyl)piperazin-1-yl)phenyl)-1H-pyrazol-4-yl)-6-(trifluoromethyl)picolinamide

To a solution of 1-(2-(2,6-dioxopiperidin-3-yl)-1-oxoisoindolin-5-yl)piperidine-4-carbaldehyde (100 mg, 0.28 mmol, 1.0 eq) in DCM (10 mL) was added 0.1 mL AcOH and N-(3-amino-1-(4-(piperazin-1-yl)phenyl)-1H-pyrazol-4-yl)-6-(trifluoromethyl)picolinamide (155 mg, 0.32 mmol, 1.5 eq) at rt. The mixture was stirred at rt for 2 h. Sodium triacetoxyborohydride (118 mg, 0.56 mmol, 2.0 eq) was then added. The mixture was stirred at rt for 2 h. Upon completion, the mixture was diluted in water, extracted with DCM (×3), washed with saturated aqueous NaCl, dried over anhydrous Na₂SO₄, filtered, and concentrated under reduced pressure. The residue was purified by column chromatography and HPLC to afford the title compound (white solid, 18.7 mg, yield 8%). **¹H** NMR (400 MHz, DMSO-*d*₆) δ 11.80 (s, 1H), 10.95 (s, 1H), 8.88 (s, 1H), 8.48-8.37 (m, 2H), 8.22 (d, *J=* 7.6 Hz, 1H), 7.95 (s, 1H), 7.80 (d, *J=* 8.8 Hz, 2H), 7.71 (s, 1H), 7.51 (d, *J=* 8.8 Hz, 1H), 7.07 (dd, *J* = 12.4, 8.5 Hz, 4H), 5.05 (dd, *J* = 13.2, 5.2 Hz, 1H), 4.27 (dd, *J* = 49.8, 16.7 Hz, 2H), 3.90 (d, *J* = 12.8 Hz, 2H), 3.23 (s, 6H), 2.85 (t, *J* = 10.8 Hz, 3H), 2.59 (d, *J* = 19.0 Hz, 1H), 2.36 *(d, J=* 18.0 Hz, 1H), 2.24 (d, *J=* 6.4 Hz, 2H), 1.98 (s, 1H), 1.83 (d, *J= 11.2* Hz, 3H), 1.22 (d, *J= 13.2* Hz, 3H). LCMS (ESI) m/z: 799 [M+H]⁺.

### Preparation Example 254. Synthesis of Compound A170

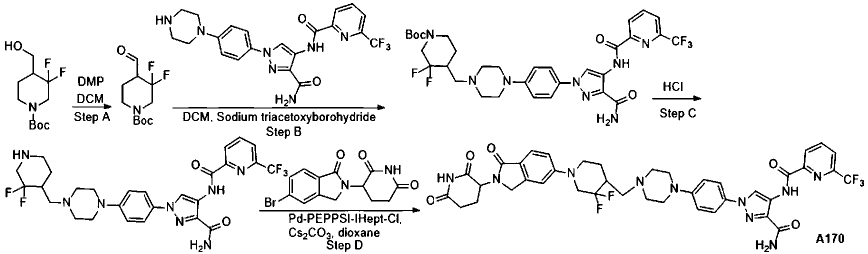

### Step A: Tert-butyl 3,3-difluoro-4-formylpiperidine-1-carboxylate

At 0°C, to a solution of tert-butyl 3,3-difluoro-4-(hydroxymethyl)piperidine-1-carboxylate (500 mg, 1.9 mmol, 1.0 eq) in DCM (5 mL) was added Dess-Martin periodinane (843 mg, 1.9 mmol, 1.0 eq). The mixture was stirred at rt for 2 h. Upon completion, the reaction was quenched with saturated sodium thiosulfate and NaHCO₃ solution and stirred for 0.5 h. The mixture was extracted with DCM (×3), and the combined organic phases were washed with water and saturated aqueous NaCl, dried over anhydrous Na₂SO₄, and concentrated under reduced pressure to afford the title compound (colorless oil, 500 mg, crude), which was used directly without purification.

### Step B: Tert-butyl 4-((4-(4-(3-amino-4-(6-(trifluoromethyl)picolinamido)-1H-pyrazol-1-yl)phenyl)piperazin-1-yl)methyl)-3,3-difluoropiperidine-1-carboxylate

To a solution of tert-butyl 3,3-difluoro-4-formylpiperidine-1-carboxylate (500 mg, 2.0 mmol, 1.0 eq) in DCM (10 mL) was added N-(3-amino-1-(4-(piperazin-1-yl)phenyl)-1H-pyrazol-4-yl)-6-(trifluoromethyl)picolinamide (921 mg, 2.0 mmol, 1.0 eq) at rt. The mixture was stirred at rt for 2 h. Sodium triacetoxyborohydride (846 mg, 4.0 mmol, 2.0 eq) was then added to the above reaction solution. The mixture was stirred at rt for 2 h. Upon completion, the mixture was diluted in water, extracted with DCM (×3), washed with saturated aqueous NaCl, dried over anhydrous Na₂SO₄, filtered, and concentrated under reduced pressure. The residue was purified by column chromatography to afford the title compound (brown solid, 250 mg, yield 18%).

### Step C: N-(3-carbamoyl-1-(4-(((3,3-difluoropiperidin-4-yl)methyl)piperazin-1-yl)phenyl)-1H-pyrazol-4-yl)-6-(trifluoromethyl)picolinamide

At 0°C, to a solution of tert-butyl 4-((4-(4-(3-amino-4-(6-(trifluoromethyl)picolinamido)-1H-pyrazol-1-yl)phenyl)piperazin-1-yl)methyl)-3,3-difluoropiperidine-1-carboxylate (200 mg, 0.3 mmol, 1.0 eq) in DCM (10 mL) was added 4 M HCl in 1,4-dioxane (2 mL). The reaction solution was stirred at rt under N₂ for 0.5 h. The reaction solution was concentrated under reduced pressure to afford the title compound (white solid, 120 mg, crude), which was used directly without purification.

### Step D: N-(3-carbamoyl-1-(4-((1-(2-(2,6-dioxopiperidin-3-yl)-1-oxoisoindol-5-yl)-3,3-difluoropiperidin-4-yl)methyl)piperazin-1-yl)phenyl)-1H-pyrazol-4-yl)-6-(trifluoromethyl)picolinamide

To a solution of N-(3-carbamoyl-1-(4-(((3,3-difluoropiperidin-4-yl)methyl)piperazin-1-yl)phenyl)-1H-pyrazol-4-yl)-6-(trifluoromethyl)picolinamide (120 mg, 0.20 mmol, 1.0 eq) and 3-(5-bromo-1-oxoisoindol-2-yl)piperidine-2,6-dione (72.1 mg, 0.2 mmol, 1.0 eq) in 1,4-dioxane (10 mL) were added cesium carbonate (198.4 mg, 0.6 mmol, 3.0 eq) and Pd-PEPPSI-IHept-Cl (30.4 mg, 0.03 mmol, 0.1 eq) at rt. The mixture was stirred at 100°C under N₂ for 16 h. Upon completion, the mixture was cooled to rt, diluted in water, extracted with EtOAc (×3), washed with saturated aqueous NaCl, dried over anhydrous Na₂SO₄, filtered, and concentrated under reduced pressure. The residue was purified by column chromatography and HPLC to afford the title compound (white solid, 15.3 mg, yield 9%). **¹H** NMR (400 MHz, CDCl₃) δ 11.70 (s, 1H), 8.73 (s, 1H), 8.37 (d, *J=* 7.4 Hz, 1H), 8.04 (s, 1H), 7.95 (s, 1H), 7.80 (d, *J=* 7.7 Hz, 1H), 7.69 (d, *J* = 8.0 Hz, 1H), 7.57 (d, *J=* 8.3 Hz, 2H), 6.97-6.75 (m, 5H), , 5.56 (s, 1H), 5.13 (d, *J* = 10.4 Hz, 1H), 4.36 (d, *J* = 15.6 Hz, 1H), 4.20 (d, *J=* 15.8 Hz, 1H), 3.92 (s, 1H), 3.77 (s, 1H), 3.20 (s, 4H), 3.05 (dd, *J* = 28.4, 13.1 Hz, 1H), 2.87-2.62 (m, , 6H), 2.52-2.35 (m, 3H), 2.35-1.97 (m, 5H). LCMS (ESI) m/z: 835 [M+H]⁺.

### Preparation Example 255. Synthesis of Compound A171

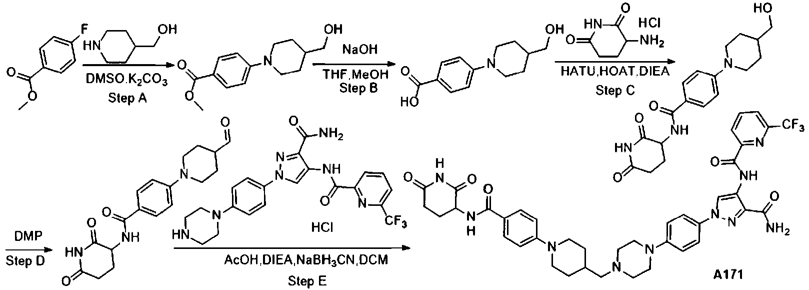

### Step A: Methyl 4-(4-(hydroxymethyl)piperidin-1-yl)benzoate

To a solution of piperidin-4-ylmethanol (11.21 g, 97.314 mmol, 3.0 eq) and methyl 4-fluorobenzoate (5 g, 32.438 mmol, 1.0 eq) in dimethyl sulfoxide (150 mL) was added potassium carbonate (13.45 g, 97.314 mmol, 3.0 eq) at rt. The reaction solution was stirred at 100°C for 18 h. Upon completion, the mixture was cooled to rt, diluted in water, and extracted with EtOAc (×3). The combined organic phases were washed with water and saturated aqueous NaCl, dried over anhydrous Na₂SO₄, filtered, and concentrated under reduced pressure. The residue was purified by column chromatography to afford the title compound (yellow solid, 7 g, yield 86%).

### Step B: 4-(4-(Hydroxymethyl)piperidin-1-yl)benzoic acid

At 0°C, to a mixed solution of methyl 4-(4-(hydroxymethyl)piperidin-1-yl)benzoate (3 g, 12.033 mmol, 1.0 eq) in MeOH (12 mL) and THF (12 mL) was added NaOH (0.96 g, 24.066 mmol, 2.0 eq). The reaction solution was stirred at 50°C for 18 h. Upon completion, the mixture was cooled to rt, and a solid was precipitated and filtered. The filter cake was washed with water and dried completely to obtain the title compound (white solid, 2.3 g, crude), which was used directly without purification.

### Step C: N-(2,6-dioxopiperidin-3-yl)-4-(4-(hydroxymethyl)piperidin-1-yl)benzamide

To a solution of 4-(4-(hydroxymethyl)piperidin-1-yl)benzoic acid (1.6 g, 6.800 mmol, 1.0 eq) and 3-aminopiperidine-2,6-dione hydrochloride (1.31 g, 10.201 mmol, 1.5 eq) in DCM (80 mL) were added N-hydroxy-7-azabenzotriazole (0.19 g, 1.360 mmol, 0.2 eq), HATU (5.17 g, 13.601 mmol, 2.0 eq) and DIEA (5.27 g, 40.802 mmol, 6.0 eq) at rt. The reaction solution was stirred at rt under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 1.1 g, yield 47%).

### Step D: N-(2,6-dioxopiperidin-3-yl)-4-(4-formylpiperidin-1-yl)benzamide

At 0°C, to a solution of N-(2,6-dioxopiperidin-3-yl)-4-(4-(hydroxymethyl)piperidin-1-yl)benzamide (100 mg, 0.290 mmol, 1.0 eq) in DCM (10 mL) was added Dess-Martin periodinane (184.20 mg, 0.434 mmol, 1.5 eq). The mixture was stirred at rt for 18 h. Upon completion, the reaction was quenched with water. The mixture was extracted with DCM (×3). The combined organic phases were washed with water and saturated aqueous NaCl, dried over anhydrous Na₂SO₄, filtered and concentrated under reduced pressure. The residue was purified by column chromatography to afford the title compound (white solid, 68 mg, yield 68%).

### Step E: N-(3-carbamoyl-1-(4-((1-(4-(2,6-dioxopiperidin-3-yl)carbamoyl)phenyl)piperidin-4-yl)methyl)piperazin-1-yl)phenyl)-1H-pyrazol-4-yl)-6-(trifluoromethyl)picolinamide

At rt, to a solution of N-(3-amino-1-(4-(piperazin-1-yl)phenyl)-1H-pyrazol-4-yl)-6-(trifluoromethyl)picolinamide hydrochloride (90.98 mg, 0.198 mmol, 1.0 eq) in DCM (5 mL) was added DIEA (51.18 mg, 0.396 mmol, 2.0 eq), and the mixture was stirred for 5 min. AcOH (0.034 mL, 0.594 mmol, 3.0 eq) and N-(2,6-dioxopiperidin-3-yl)-4-(4-formylpiperidin-1-yl)benzamide (68 mg, 0.198 mmol, 1.0 eq) were then added. The mixture was stirred at rt for 0.5 h. Sodium triacetoxyborohydride (124.42 mg, 1.980 mmol, 10 eq) was then added. The mixture was stirred at rt for 1 h. Upon completion, the reaction solution was concentrated under reduced pressure and the residue was purified by HPLC to afford the title compound (white solid, 25 mg, yield 16%). **¹H NMR** (400 MHz, DMSO-*d*₆) *δ* 11.80 (s, 1H), 10.84 (s, 1H), 10.54 (s, 1H), 8.93 (s, 1H), 8.58 (d, *J* = 8.3 Hz, 1H), 8.47-8.38 (m, 2H), 8.22 (d, *J* = 7.6 Hz, 1H), 7.97 (s, 1H), 7.85 (dd, *J* = 19.3, 8.8 Hz, 4H), 7.74 (s, 1H), 7.20 (t, *J* = 12.4 Hz, 4H), 4.82-4.70 (m, 1H), 3.94-3.83 (m, 8H), 3.37-3.31 (m, 2H), 3.16-3.12 (m, 2H), 3.00 (s, 1H), 2.84-2.76 (m, 1H), 2.21-2.09 (m, 2H), 2.03-1.95 (m, 3H), 1.51-1.42 (m, 2H), 1.28-1.19 (s, 2H). LCMS (ESI): m/z 787 [M+H]⁺.

### Preparation Example 256. Synthesis of Compound A172

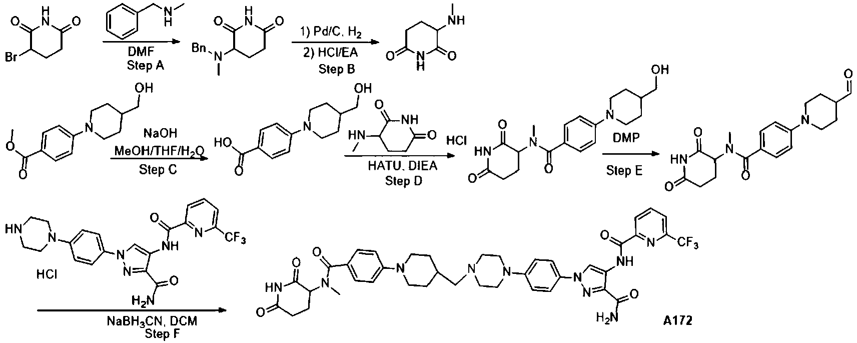

### Step A: 3-(Benzyl (methyl)amino)piperidine-2,6-dione

To a solution of 3-bromopiperidine-2,6-dione (3 g, 15.6 mmol, 1.0 eq) in DMF (20 mL) at rt was added N-methyl-1-phenylmethanamine (4.7 g, 39.1 mmol, 2.5 eq). The mixture was stirred at rt for 16 h. Upon completion, the reaction solution was concentrated under reduced pressure. The residue was purified by column chromatography to afford the title compound (purple solid, 2.6 g, yield 71%).

### Step B: 3-(Methylamino)piperidine-2,6-dione hydrochloride

To a solution of 3-(benzyl(methyl)amino)piperidine-2,6-dione (2 g, 8.6 mmol, 1.0 eq) in EtOAc (25 mL) was added 10% palladium on carbon (0.6 g, 5.6 mmol) at rt. The reaction solution was purged with H₂ (using a hydrogen balloon) (×3) and stirred under H₂ at rt for 16 h. Upon completion, the mixture was filtered, and HCl in EtOAc was added to the filtrate. The mixture was concentrated under reduced pressure to afford the title compound (purple solid, 1.3 g, yield 84%).

### Step C: 4-(4-(Hydroxymethyl)piperidin-1-yl)benzoic acid

At 0°C, to a mixed solution of methyl 4-(4-(hydroxymethyl)piperidin-1-yl)benzoate (1 g, 4.0 mmol, 1.0 eq) in MeOH (8 mL), THF (8 mL), and water (8 mL) was added NaOH (0.64 g, 16.0 mmol, 4.0 eq). The reaction solution was stirred at 50°C for 16 h. Upon completion, the reaction solution was adjusted to pH 2-3 using 2 N HCl, and the crude was precipitated. The product was filtered and dried completely to afford the title compound as a pink solid (0.6 g, yield 63%).

### Step D: N-(2,6-dioxopiperidin-3-yl)-4-(4-(hydroxymethyl)piperidin-1-yl)-N-methylbenzamide

To a solution of 4-(4-(hydroxymethyl)piperidin-1-yl)benzoic acid (400 mg, 1.7 mmol, 1.0 eq) and 3-(methylamino)piperidine-2,6-dione hydrochloride (483 mg, 3.4 mmol, 2.0 eq) in DCM (15 mL) were added DIEA (219 mg, 1.7 mmol, 1.0 eq) and HATU (646 mg, 1.7 mmol, 1.0 eq) at rt. The mixture was stirred at rt under N₂ for 16 h. Upon completion, the mixture was diluted in water to precipitate the crude. After filtration and complete drying, the title compound was obtained as a blue solid (410 mg, yield 69%).

### Step E: N-(2,6-dioxopiperidin-3-yl)-4-(4-formylpiperidin-1-yl)-N-methylbenzamide

To a solution of N-(2,6-dioxopiperidin-3-yl)-4-(4-(hydroxymethyl)piperidin-1-yl)-N-methylbenzamide (100 mg, 0.29 mmol, 1.0 eq) in DCM (15 mL) was added Dess-Martin periodinane (212 mg, 4.5 mmol, 1.5 eq) at 0°C. The mixture was stirred at rt under N₂ for 3 h, Upon completion, the reaction solution was concentrated under reduced pressure. The residue was purified by column chromatography to afford the title compound (white solid, 80 mg, yield 80%).

### Step F: N-(3-carbamoyl-1-(4-((1-(4-(2,6-dioxopiperidin-3-yl)(methyl)carbamoyl)phenyl)piperidin-4-yl)methyl)piperazin-1-yl)phenyl)-1H-pyrazol-4-yl)-6-(trifluoromethyl)picolinamide

At rt, to a solution of N-(2,6-dioxopiperidin-3-yl)-4-(4-formylpiperidin-1-yl)-N-methylbenzamide (100 mg, 0.28 mmol, 1.0 eq) in DCM (5 mL) were added 0.1 mL of AcOH, N-(3-amino-1-(4-(piperazin-1-yl)phenyl)-1H-pyrazol-4-yl)-6-(trifluoromethyl)picolinamide (192 mg, 0.42 mmol, 1.5 eq) and DIEA (72 mg, 0.56 mmol, 2.0 eq), and the mixture was stirred for 5 min. Then, AcOH (84 mg, 1.4 mmol, 5.0 eq) was added. The mixture was stirred at rt for 0.5 h. Sodium cyanoborohydride (38 mg, 0.56 mmol, 2.0 eq) was then added to the above reaction solution. The mixture was stirred at rt under N₂ for 16 h, Upon completion, the reaction solution was concentrated under reduced pressure. The residue was purified by HPLC to afford the title compound (white solid, 17 mg, yield 8%). **¹H NMR** (400 MHz, DMSO-d6) δ 11.80 (s, 1H), 10.86 (s, 1H), 9.33 (s, 1H), 8.93 (s, 1H), 8.52-8.34 (m, 2H), 8.22 (d, J = 7.3 Hz, 1H), 7.99-7.68 (m, 4H), 7.39-7.23 (m, 2H), 7.17 (s, 1H), 7.02-6.89 (m, 2H), 5.24-4.48 (m, 1H), 3.97-3.61 (m, 5H), 3.28-3.06 (m, 6H), 2.95-2.71 (m, 6H), 2.47-2.14 (m, 3H), 2.13-1.81 (m, 4H), 1.37-1.22 (m, 2H). LC-MS (ESI): m/z 801 [M+H]⁺.

### Preparation Example 257. Synthesis of Compound A173

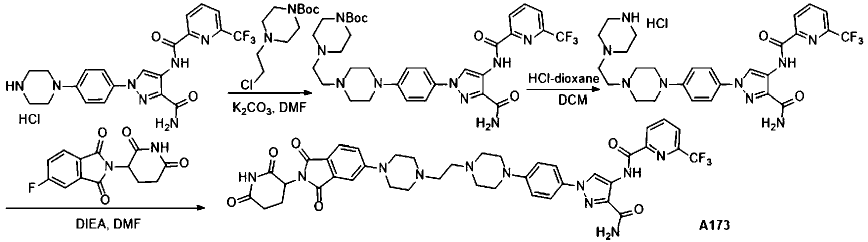

### Step A: Tert-butyl 4-(2-(4-(4-(3-amino-4-(6-(trifluoromethyl)picolinamido)-1H-pyrazol-1-yl)phenyl)piperazin-1-yl)ethyl)piperazine 1-carboxylate

To a solution of N-(3-amino-1-(4-(piperazin-1-yl)phenyl)-1H-pyrazol-4-yl)-6-(trifluoromethyl)picolinamide hydrochloride (650 mg, 1.5 mmol, 1.25 eq) and tert-butyl 4-(2-chloroethyl)piperazine-1-carboxylate (300 mg, 1.2 mmol, 1.0 eq) in DMF (10 mL) was added potassium carbonate (13.45 g, 97.314 mmol, 3.0 eq) at rt. The reaction solution was stirred at 90°C for 16 h. Upon completion, the mixture was cooled to rt, diluted in water, and extracted with EtOAc (×3). The combined organic phases were washed with water and saturated aqueous NaCl, dried over anhydrous Na₂SO₄, filtered, and concentrated under reduced pressure. The residue was purified by column chromatography to afford the title compound (white solid, 500 mg, yield 62%).

### Step B: N-(3-amino-1-(4-(4-(2-(piperazin-1-yl)ethyl)piperazine-1-carbonyl)phenyl)-1H-pyrazol-4-yl)-6-(trifluoromethyl)picolinamide hydrochloride

At 0°C, to a solution of tert-butyl 4-(2-(4-(4-(3-amino-4-(6-(trifluoromethyl)picolinamido)-1H-pyrazol-1-yl)phenyl)piperazin-1-yl)ethyl)piperazine 1-carboxylate (500 mg, 0.75 mmol, 1.0 eq) in DCM (5 mL) was added 4 M HCl in 1,4-dioxane (3 mL, 12 mmol, 16 eq). The reaction solution was stirred at rt under N₂ for 1 h. The reaction solution was concentrated under reduced pressure to afford the title compound (white solid, 380 mg, yield 90%).

### Step C: N-(3-carbamoyl-1-(4-(4-(2-(2-(2,6-dioxopiperidin-3-yl)-1,3-dioxoisoindolin-5-yl)piperazin-1-yl)ethyl)piperazin-1-yl)phenyl)-1H-pyrazol-4-yl)-6-(trifluoromethyl)picolinamide

To a solution of N-(3-amino-1-(4-(4-(2-(piperazin-1-yl)ethyl)piperazine-1-carbonyl)phenyl)-1H-pyrazol-4-yl)-6-(trifluoromethyl)picolinamide hydrochloride (150 mg, 0.27 mmol, 1.0 eq) and 2-(2,6-dioxopiperidin-3-yl)-5-fluoroisoindoline-1,3-dione (73 mg, 0.27 mmol, 1.0 eq) in DMF (5 mL) was added DIEA (34 mg, 0.27 mmol, 1.0 eq) at rt. The mixture was stirred at 90°C for 18 h. Upon completion, the mixture was cooled to rt, diluted in water, and extracted with EtOAc (×3). The combined organic phases were washed with water and saturated aqueous NaCl, dried over anhydrous Na₂SO₄, filtered, and concentrated under reduced pressure. The residue was purified by column chromatography to afford the title compound (white solid, 20 mg, yield 10%). **¹H NMR** (400 MHz, DMSO-*d*₆) δ 11.79 (s, 1H), 11.08 (s, 1H), 8.88 (s, 1H), 8.43 (dd, *J=* 16.6, 7.4 Hz, 2H), 8.21 (d, *J* = 7.6 Hz, 1H), 7.94 (s, 1H), 7.79 (d, *J* = 9.1 Hz, 2H), 7.72-7.66 (m, 2H), 7.34 (s, 1H), 7.26 (d, *J* = 8.6 Hz, 1H), 7.08 (d, *J* = 9.2 Hz, 2H), 5.07 (d, *J* = 12.9, 5.4 Hz, 1H), 3.44 (s, 6H), 3.21 (s, 6H), 2.58 (d, *J =* 3.7 Hz, 12H). LCMS (ESI) m/z: 828 [M+H]⁺.

### Preparation Example 258. Synthesis of Compound A174

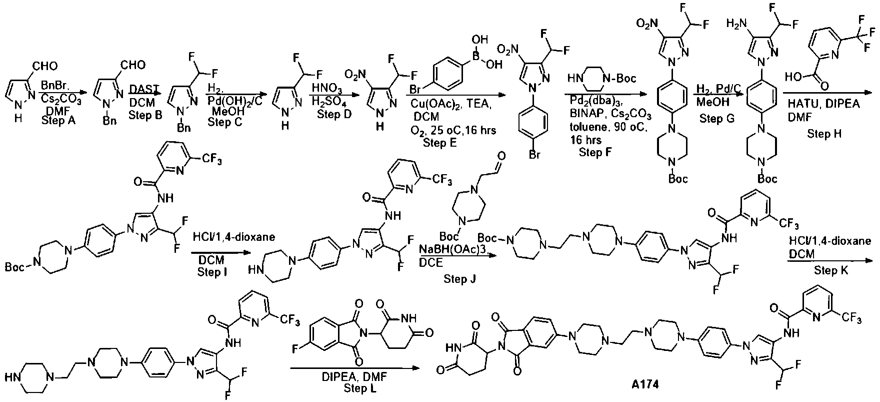

### Step A: 1-Benzyl-1H-pyrazole-3-carbaldehyde

To a solution of 1H-pyrazole-3-carboxaldehyde (23 g, 240 mmol, 1.0 eq) and cesium carbonate (156 g, 480 mmol, 1.0 eq) in DMF (250 mL) was added benzyl bromide (49 g, 288 mmol, 1.0 eq) at rt. The reaction solution was stirred at rt for 16 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (colorless oil, 41 g, yield 91%).

### Step B: 1-Benzyl-3-(difluoromethyl)-1H-pyrazole

To a solution of 1-benzyl-1H-pyrazole-3-carbaldehyde (40 g, 215.2 mmol, 1.0 eq) in DCM (30 mL) was added diethylaminosulfur trifluoride (138.4 g, 428 mmol, 4.0 eq) at 0°C. The reaction solution was stirred at rt for 5 h. Upon completion, the reaction solution was quenched with MeOH at 0°C and concentrated under reduced pressure. The residue was purified by column chromatography to afford the title compound (white solid, 25 g, yield 57%).

### Step C: 3-(Difluoromethyl)-1H-pyrazole

To a solution of 1-benzyl-3-(difluoromethyl)-1H-pyrazole (25 g, 120 mmol, 1.0 eq) in MeOH (250 mL) was added 10% palladium hydroxide on carbon (2.5 g, 1.2 mmol) at rt. The reaction solution was purged with H₂ (×3) and stirred at 40°C under H₂ for 12 h. Upon completion, the reaction solution was filtered and washed with MeOH (×3). The filtrate was concentrated under reduced pressure to yield the title compound (12 g, colorless oil, crude).

### Step D: 3-(Difluoromethyl)-4-nitro-1H-pyrazole

To a solution of 3-(difluoromethyl)-1H-pyrazole (17 g, 144 mmol, 1.0 eq) in sulfuric acid (200 mL) was slowly added concentrated nitric acid (34.7 g, 358 mmol) at 0°C. The reaction solution was stirred for 10 min, then heated to 110°C and stirred for 12 h. Upon completion, the reaction solution was cooled to rt, poured into water, and extracted with EtOAc (×3). The combined organic phases were washed with saturated aqueous NaCl, dried over anhydrous Na₂SO₄, filtered, and concentrated under reduced pressure to afford the title compound (colorless oil, 17 g, yield 74%).

### Step E: 1-(4-bromophenyl)-3-(difluoromethyl)-4-nitro-1H-pyrazole

To a solution of 3-(difluoromethyl)-4-nitro-1H-pyrazole (6.0 g, 36.8 mmol, 1.0 eq) and (4-bromophenyl)boronic acid (11 g, 55.2 mmol, 1.5 eq) in DCM (20 mL) was added copper acetate (8.7 g, 47.8 mmol, 1.3 eq) and 2 mL of TEA at rt. The reaction solution was stirred at rt under O₂ for 16 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 5.5 g, yield 47%).

### Step F: Tert-butyl 4-(4-(3-(difluoromethyl)-4-nitro-1H-pyrazol-1-yl)phenyl)piperazine-1-carboxylate

To a solution of 1-(4-bromophenyl)-3-(difluoromethyl)-4-nitro-1H-pyrazole (5.1 g, 16.1 mmol, 1.0 eq) and tert-butyl piperazine-1-carboxylate (4.5 g, 24.2 mmol, 1.2 eq) in toluene (20 mL) at rt were added BINAP (1.97 g, 3.2 mmol), cesium carbonate (15.7 g, 48.3 mmol, 3.0 eq), and Pd₂(dba)₃ (1.5 g, 1.6 mmol, 0.1 eq). The reaction solution was stirred at 90°C under N₂ for 16 h. Upon completion, the reaction solution was concentrated under reduced pressure. The residue was purified by column chromatography to afford the title compound (brown solid, 4.9 g, yield 72%).

### Step G: Tert-butyl 4-(4-(4-amino-3-(difluoromethyl)-1H-pyrazol-1-yl)phenyl)piperazine-1-carboxylate

To a solution of tert-butyl 4-(4-(3-(difluoromethyl)-4-nitro-1H-pyrazol-1-yl)phenyl)piperazine-1-carboxylate (4.9 g, 11.6 mmol, 1.0 eq) in MeOH (250 mL) was added 10% palladium on carbon (500 mg, 0.1 mmol) at rt. The reaction solution was purged with H₂ (×3) and stirred at rt under H₂ for 4 h. Upon completion, the reaction solution was filtered and washed with MeOH (×3), and the filtrate was concentrated under reduced pressure to yield the title compound (white solid, 4.1 g, crude).

### Step H: Tert-butyl 4-(4-(3-(difluoromethyl)-4-(6-(trifluoromethyl)picolinamido)-1H-pyrazol-1-yl)phenyl)piperazine-1-carboxylate

To a solution of tert-butyl 4-(4-(4-amino-3-(difluoromethyl)-1H-pyrazol-1-yl)phenyl)piperazine-1-carboxylate (3 g, 7.6 mmol, 1.0 eq) and 6-(trifluoromethyl)picolinic acid (1.46 g, 7.6 mmol, 1.0 eq) in DMF (30 mL) at 0°C were added DIPEA (1.96 g, 15.2 mmol, 2.0 eq) and HATU (4.33 g, 11.4 mmol, 1.5 eq). The reaction solution was reacted at rt for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (pale yellow solid, 2.5 g, yield 58%).

### Step I: N-(3-(difluoromethyl)-1-(4-(piperazin-1-yl)phenyl)-1H-pyrazol-4-yl)-6-(trifluoromethyl)picolinamide

To a solution of tert-butyl 4-(4-(3-(difluoromethyl)-4-(6-(trifluoromethyl)picolinamido)-1H-pyrazol-1-yl)phenyl)piperazine-1-carboxylate (2.5 g, 4.42 mmol, 1.0 eq) in DCM (12 mL) was added HCl/1,4-dioxane (12 mL, 4 M) at 0°C. The reaction solution was reacted at rt for 1 h. Upon completion, the reaction solution was poured into NaHCO₃ solution, and extracted with EtOAc (×3). The combined organic phases were washed with saturated aqueous NaCl, dried over anhydrous Na₂SO₄, filtered, and concentrated under reduced pressure to afford the title compound (brown solid, 1.8 g, yield 97%).

### Step J: Tert-butyl 4-(2-(4-(4-(3-(difluoromethyl)-4-(6-(trifluoromethyl)picolinamido)-1H-pyrazol-1-yl)phenyl)piperazin-1-yl)ethyl)piperazinyl-1-carboxylate

To a solution of N-(3-(difluoromethyl)-1-(4-(piperazin-1-yl)phenyl)-1H-pyrazol-4-yl)-6-(trifluoromethyl)picolinamide (700 mg, 1.50 mmol, 1.0 eq) in 1,2-dichloroethane (10 mL) at rt were added tert-butyl 4-(2-oxoethyl)piperazine-1-carboxylate (410 mg, 1.80 mmol, 1.2 eq) and AcOH (0.02 mL). The reaction solution was reacted at rt for 0.5 h. Sodium triacetoxyborohydride (954 mg, 4.50 mmol, 3.0 eq) was then added, and the reaction solution was stirred at rt for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 520 mg, yield 62%).

### Step K: N-(3-(difluoromethyl)-1-(4-(4-(2-(piperazin-1-yl)ethyl)piperazine-1-carbonyl)phenyl)-1H-pyrazol-4-yl)-6-(trifluoromethyl)picolinamide

To a solution of tert-butyl 4-(2-(4-(4-(3-(difluoromethyl)-4-(6-(trifluoromethyl)picolinamido)-1H-pyrazol-1-yl)phenyl)piperazin-1-yl)ethyl)piperazinyl-1-carboxylate (250 mg, 0.37 mmol, 1.0 eq) in DCM (1 mL) was added HCl/1,4-dioxane (2 mL, 4 M) at rt. The reaction solution was reacted at rt for 1 h. Upon completion, the reaction solution was poured into NaHCO₃ solution, and extracted with EtOAc (×3). The combined organic phases were washed with saturated aqueous NaCl, dried over anhydrous Na₂SO₄, filtered, and concentrated under reduced pressure to afford the title compound (brown solid, 180 mg, yield 85%).

### Step L: N-(3-(difluoromethyl)-1-(4-(4-(2-(2-(2,6-dioxopiperidin-3-yl)-1,3-dioxoisoindolin-5-yl)piperazin-1-yl)ethyl)piperazin-1-yl)phenyl)-1H-pyrazol-4-yl)-6-(trifluoromethyl)picolinamide

To a solution of N-(3-(difluoromethyl)-1-(4-(4-(2-(piperazin-1-yl)ethyl)piperazine-1-carbonyl)phenyl)-1H-pyrazol-4-yl)-6-(trifluoromethyl)picolinamide (180 mg, 0.31 mmol, 1.0 eq) and 2-(2,6-dioxopiperidin-3-yl)-5-fluoroisoindoline-1,3-dione (128 mg, 0.47 mmol, 1.5 eq) in DMF (3 mL) was added DIPEA (120 mg, 0.62 mmol, 2.0 eq) at 0°C. The reaction solution was reacted at rt for 16 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (white solid, 20 mg, yield 8%). ¹HNMR (400 MHz, DMSO-*d₆*) δ 11.09 (s, 1H), 10.27 (s, 1H), 8.84 (s, 1H), 8.46-8.37 (m, 2H), 8.26-8.21 (m, 1H), 7.76 (d, *J=* 8.9 Hz, 3H), 7.50-7.14 (m, 5H), 5.15-5.04 (m, 1H), 4.16-3.58 (m, 10H), 3.30-3.13 (m, 8H), 2.95-2.83 (m, 2H), 2.64-2.52 (m, 3H), 2.08-1.98 (m, 1H). LCMS (ESI): m/z 835 [M+H]⁺.

### Preparation Example 259. Synthesis of Compound A175

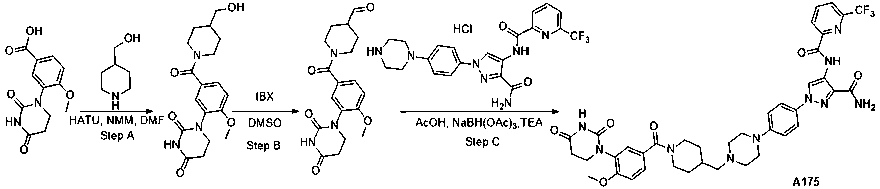

### Step A: 1-(5-(4-(hydroxymethyl)piperidine-1-carbonyl)-2-methoxyphenyl)dihydropyrimidine-2,4(1H,3H)-dione

To a solution of 3-(2,4-dioxotetrahydropyrimidin-1(2H)-yl)-4-methoxybenzoic acid (800 mg, 3.03 mmol, 1.0 eq) and piperidin-4-ylmethanol (348.7 mg, 3.03 mmol, 1.0 eq) in DMF (10 mL) were added N-methylmorpholine (1.67 mL, 15.1 mmol, 5.0 eq) and HATU (1.7 g, 4.5 mmol, 1.5 eq) at rt. The reaction solution was stirred at rt under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 900 mg, yield 82%).

### Step B: 1-(3-(2,4-dioxotetrahydropyrimidin-1(2H)-yl)-4-methoxybenzoyl)piperidine-4-carbaldehyde

To a solution of 1-(5-(4-(hydroxymethyl)piperidine-1-carbonyl)-2-methoxyphenyl)dihydropyrimidine-2,4(1H,3H)-dione (300 mg, 0.8 mmol, 1.0 eq) in dimethyl sulfoxide (5 mL) was added IBX (464.9 mg, 1.6 mmol, 2.0 eq) at rt. The reaction solution was stirred at rt for 1 h. Upon completion, the reaction was quenched with saturated NaHCO₃ and sodium thiosulfate solution and extracted with DCM (×3). The combined organic phases were washed with water and saturated aqueous NaCl, dried over anhydrous Na₂SO₄, and concentrated under reduced pressure to afford the title compound (yellow solid, 250 mg, crude), which was used directly without purification.

### Step C: 1 N-(3-carbamoyl-1-(4-((1-(3-(2,4-dioxotetrahydropyrimidin-1(2H)-yl)-4-methoxybenzoyl)piperidin-4-yl)methyl)piperazin-1-yl)phenyl)-1H-pyrazol-4-yl)-6-(trifluoromethyl)picolinamide

To a solution of N-(3-amino-1-(4-(piperazin-1-yl)phenyl)-1H-pyrazol-4-yl)-6-(trifluoromethyl)picolinamide hydrochloride (367.5 mg, 0.8 mmol, 1.0 eq) in MeOH (5 mL) was added TEA (0.2 mL) at rt. The reaction solution was stirred at rt for 5 min. 1-(3-(2,4-dioxotetrahydropyrimidin-1(2H)-yl)-4-methoxybenzoyl)piperidine-4-carbaldehyde (287.5 mg, 0.8 mmol, 1.0 eq) and AcOH (0.3 mL) were added to the above reaction solution. The reaction solution was stirred at rt for 15 h. Sodium triacetoxyborohydride (253.1 mg, 1.2 mmol, 1.5 eq) was then added. The reaction solution was stirred at rt for an additional 3 h. Upon completion, the reaction was quenched with water and extracted with DCM (×3). The mixture was concentrated under reduced pressure, and the residue was purified by HPLC to afford the title compound (white solid, 21.3 mg, yield 4%). ¹HNMR (400 MHz, DMSO-*d*₆) δ 11.79 (s, 1H), 10.34 (s, 1H), 8.89 (d, *J* = 10.2 Hz, 1H), 8.45 (d, *J* = 7.6 Hz, 1H), 8.40 (t, *J=* 7.6 Hz, 1H), 8.21 (d, *J* = 7.6 Hz, 1H), 7.95 (s, 1H), 7.80 (t, *J=* 9.8 Hz, 2H), 7.71 (s, 1H), 7.37 (dd, *J* = 8.4, 2.0 Hz, 1H), 7.33 (d, *J* = 2.0 Hz, 1H), 7.16 (d, *J=* 8.4 Hz, 1H), 7.07 (d, *J* = 9.2 Hz, 2H), 3.85 (s, 3H), 3.65-3.57 (m, 2H), 3.39-3.36 (m, 4H), 3.33-3.30 (m, 2H), 3.24-3.16 (m, 4H), 2.73-2.65 (m, 2H), 2.54-2.52 (m, 2H), 2.26-2.19 (m, 2H), 1.92-1.66 (m, 3H), 1.10 (qd, *J =* 13.2, 3.6 Hz, 2H). LCMS (ESI): m/z 802 [M+H]⁺.

### Preparation Example 260. Synthesis of Compound A176

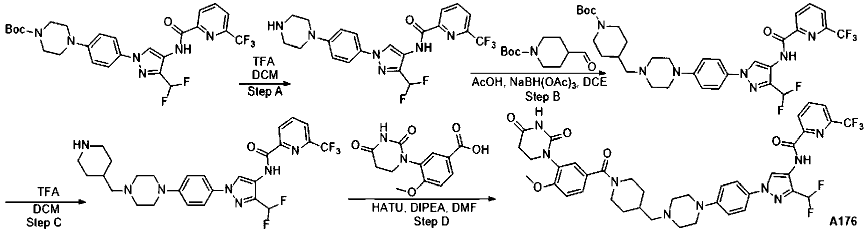

### Step A: N-(3-(difluoromethyl)-1-(4-(piperazin-1-yl)phenyl)-1H-pyrazol-4-yl)-6-(trifluoromethyl)picolinamide

To a solution of tert-butyl 4-(4-(3-(difluoromethyl)-4-(6-(trifluoromethyl)picolinamido)-1H-pyrazol-1-yl)phenyl)piperazine-1-carboxylate (1.0 g, 1.76 mmol, 1.0 eq) in DCM (6.0 mL) was added TFA (3.0 mL) at rt. The reaction solution was reacted at rt for 1 h. Upon completion, the reaction solution was poured into NaHCO₃ solution and extracted with DCM (×3). The combined organic phases were washed with saturated aqueous NaCl, dried over anhydrous Na₂SO₄, filtered, and concentrated under reduced pressure to yield the title compound (yellow oil, 800 mg, crude).

### Step B: Tert-butyl 4-((4-(4-(3-(difluoromethyl)-4-(6-(trifluoromethyl)picolinamido)-1H-pyrazol-1-yl)phenyl)piperazin-1-yl)methyl)piperidine-1-carboxylate

To a solution of N-(3-(difluoromethyl)-1-(4-(piperazin-1-yl)phenyl)-1H-pyrazol-4-yl)-6-(trifluoromethyl)picolinamide (164 mg, 0.352 mmol, 1.0 eq) and tert-butyl 4-formylpiperidine-1-carboxylate (150 mg, 0.703 mmol, 2.0 eq) in 1,2-dichloroethane (2.0 mL) was added AcOH (0.004 mL, 0.07 mmol, 0.5 eq) at rt. The reaction solution was reacted at rt for 0.5 h. Sodium triacetoxyborohydride (224 mg, 1.05 mmol, 3.0 eq) was then added, and the reaction was allowed to proceed at rt for 1 h. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by column chromatography to afford the title compound (yellow solid, 150 mg, yield 64%).

### Step C: N-(3-(difluoromethyl)-1-(4-(4-(piperidin-4-ylmethyl)piperazin-1-yl)phenyl)-1H-pyrazol-4-yl)-6-(trifluoromethyl)picolinamide

At 0°C, to a solution of tert-butyl 4-((4-(4-(3-(difluoromethyl)-4-(6-(trifluoromethyl)picolinamido)-1H-pyrazol-1-yl)phenyl)piperazin-1-yl)methyl)piperidine-1-carboxylate (150 mg, 0.226 mmol, 1.0 eq) in DCM (1.0 mL) was added TFA (0.5 mL). The reaction solution was reacted at rt for 1 h. Upon completion, the reaction solution was poured into NaHCO₃ solution and extracted with DCM (×3). The combined organic phases were washed with saturated aqueous NaCl, dried over anhydrous Na₂SO₄, filtered, and concentrated under reduced pressure to yield the title compound (yellow oil, 120 mg, yield 94%). **Step D: N-(3-(difluoromethyl)-1-(4-(4-((1-(3-(2,4-dioxotetrahydropyrimidin-1(2H)-yl)-4-methoxybenzoyl)piperidin-4-yl)methyl)piperazin-1-yl)phenyl)-1H-pyrazol-4-yl)-6-(trifluoromethyl)picolinamide**

At 0°C, to a solution of N-(3-(difluoromethyl)-1-(4-(4-(piperidin-4-ylmethyl)piperazin-1-yl)phenyl)-1H-pyrazol-4-yl)-6-(trifluoromethyl)picolinamide (100 mg, 0.177 mmol, 1.0 eq) and 3-(2,4-dioxotetrahydropyrimidin-1(2H)-yl)-4-methoxybenzoic acid (51.6 mg, 0.195 mmol, 1.1 eq) in DMF (2.0 mL) were added DIPEA (0.088 mL, 0.532 mmol, 3.0 eq) and HATU (101 mg, 0.266 mmol, 1.5 eq). The reaction solution was reacted for 1 h at rt under N₂. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (white solid, 40 mg, yield 28%). ¹HNMR (400 MHz, DMSO-*d₆*) δ 10.34 (s, 1H), 10.26 (s, 1H), 8.79 (s, 1H), 8.46-8.35 (m, 2H), 8.21-8.18 (m, 1H), 7.69 (d, *J=* 8.9 Hz, 2H), 7.46-7.26 (m, 3H), 7.17-7.14 (m, 1H), 7.07 (d, *J=* 9.0 Hz, 2H), 4.64-4.16 (m, 1H), 3.84 (s, 3H), 3.60 (t, *J=* 6.6 Hz, 2H), 3.27-2.87 (m, 8H), 2.68 (t, *J=* 6.5 Hz, 2H), 2.66-2.53 (m, 3H), 2.40-2.18 (m, 2H), 1.96-1.85 (m, 1H), 1.81-1.65 (m, 2H), 1.18-1.05 (m, 2H). LCMS (ESI): m/z 810 [M+H]⁺.

### Preparation Example 261. Synthesis of Compound A177

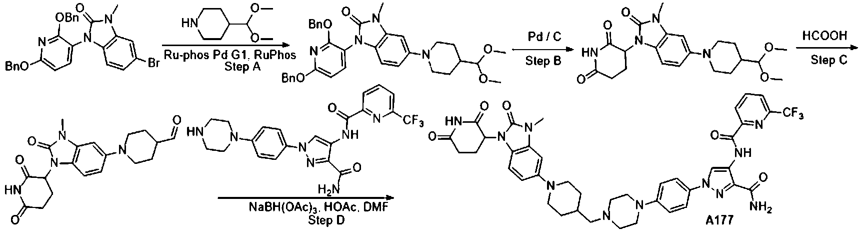

### Step A: 1-(2,6-bis(benzyloxy)pyridin-3-yl)-5-(4-(dimethoxymethyl)piperidin-1-yl)-3-methyl-1,3-dihydro-2H-benzo[d]imidazol-2-one

To a solution of 1-(2,6-bis(benzyloxy)pyridin-3-yl)-5-bromo-3-methyl-1,3-dihydro-2H-benzo[d]imidazol-2-one (400 mg, 0.78 mmol, 1.0 eq) and 4-(dimethoxymethyl)piperidine (263 mg, 1.2 mmol, 1.5 eq) in 1,4-dioxane (10 mL) were added cesium carbonate (760 mg, 2.34 mmol, 3.0 eq), Ruphos (72 mg, 0.14 mmol, 0.2 eq), and Ruphos Pd G₁ (127 mg, 0.14 mmol, 0.2 eq) at rt. The mixture was stirred at 100°C under N₂ for 16 h. Upon completion, the mixture was cooled to rt, diluted in water, extracted with EtOAc, washed with saturated aqueous NaCl, dried over anhydrous Na₂SO₄, filtered, and concentrated under reduced pressure. The residue was purified by column chromatography to afford the title compound (yellow solid, 350 mg, yield 76%).

### Step B: 3-(5-(4-(dimethoxymethyl)piperidin-1-yl)-3-methyl-2-oxo-2,3-dihydro-1H-benzo[d]imidazol-1-yl)piperidine-2,6-dione

To a solution of 1-(2,6-bis(benzyloxy)pyridin-3-yl)-5-(4-(dimethoxymethyl)piperidin-1-yl)-3-methyl-1,3-dihydro-2H-benzo[d]imidazol-2-one (350 mg, 0.59 mmol, 1.0 eq) in ethanol (5 mL) was added 10% palladium on carbon (200 mg) at rt. The reaction solution was purged with H₂ (using a hydrogen balloon) (×3) and stirred under H₂ at 50°C for 16 h. Upon completion, the mixture was filtered, and the filtrate was concentrated under reduced pressure to afford the title compound (white solid, 200 mg, crude), which was used directly without purification.

### Step C: 1-(1-(2,6-dioxopiperidin-3-yl)-3-methyl-2-oxo-2,3-dihydro-1H-benzo[d]imidazol-5-yl)piperidine-4-carbaldehyde

To 3-(5-(4-(dimethoxymethyl)piperidin-1-yl)-3-methyl-2-oxo-2,3-dihydro-1H-benzo[d]imidazol-1-yl)piperidine-2,6-dione (200 mg, 0.48 mmol, 1.0 eq) was added formic acid (2 mL) at 0°C. The reaction solution was stirred at rt under N₂ for 2 h. Upon completion, the reaction solution was concentrated under reduced pressure to afford the title compound (100 mg, colorless oil, crude), which was used directly without purification.

### Step D: N-(3-carbamoyl-1-(4-((1-(1-(2,6-dioxopiperidin-3-yl)-3-methyl-2-oxo-2,3-dihydro-1H-benzo[d]imidazol-5-yl)piperidin-4-yl)methyl)piperazin-1-yl)phenyl)-1H-pyrazol-4-yl)-6-(trifluoromethyl)picolinamide

To a solution of 1-(1-(2,6-dioxopiperidin-3-yl)-3-methyl-2-oxo-2,3-dihydro-1H-benzo[d]imidazol-5-yl)piperidine-4-carbaldehyde (50 mg, 0.14 mmol, 1.0 eq) and N-(3-amino-1-((1r,4r)-4-(piperazin-1-yl)cyclohexyl)-1H-pyrazol-4-yl)-6-(trifluoromethyl)picolinamide (62 mg, 0.14 mmol, 1.0 eq) in DMF (5 mL) was added AcOH (5 drops) at rt. The mixture was stirred at rt for 1 h. Sodium triacetoxyborohydride (115 mg, 0.54 mmol, 4.0 eq) was then added to the above reaction solution. The mixture was stirred at rt for 5 min. Upon completion, the mixture was diluted in water, extracted with EtOAc (×3), washed with saturated aqueous NaCl, dried over anhydrous Na₂SO₄, filtered, and concentrated under reduced pressure. The residue was purified by HPLC to afford the title compound (white solid, 15.6 mg, yield 14%). **¹H NMR** (400 MHz, DMSO-*d*₆) δ 11.79 (s, 1H), 11.06 (s, 1H), 8.88 (s, 1H), 8.47-8.36 (m, 2H), 8.21 (d, *J* = 7.6 Hz, 1H), 7.95 (s, 1H), 7.79 (d, *J* = 9.1 Hz, 2H), 7.71 (s, 1H), 7.08 (d, *J =* 9.3 Hz, 2H), 6.93 (d, *J =* 8.6 Hz, 1H), 6.83 (s, 1H), 6.64 (d, *J* = 10.7 Hz, 1H), 5.31-5.26 (m, 1H), 3.60 (d, *J* = 11.3 Hz, 2H), 3.31-3.29 (m, 5H), 3.26-3.21 (m, 4H), 2.92-2.84 (m, 1H), 2.67-2.61 (m, 3H), 2.54-2.53 (m, 1H), 2.27-2.22 (m, 2H), 2.00-1.96 (m, 1H), 1.83 (d, *J* = 11.8 Hz, 2H), 1.73-1.66 (m, 1H), 1.27-1.23 (m, 4H). LCMS (ESI): m/z 814 [M+H]⁺.

### Preparation Example 262. Synthesis of Compound A178

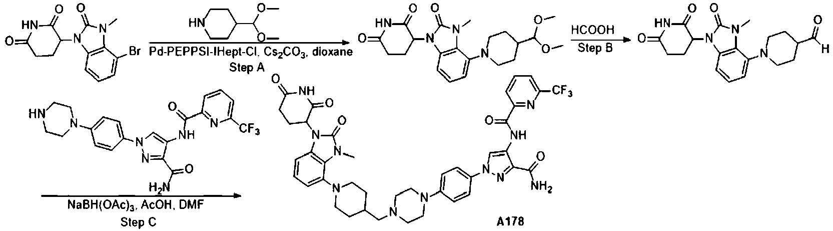

### Step A: 3-(4-(4-(dimethoxymethyl)piperidin-1-yl)-3-methyl-2-oxo-2,3-dihydro-1H-benzo[d]imidazol-1-yl)piperidine-2,6-dione

To a solution of 3-(4-bromo-3-methyl-2-oxo-2,3-dihydro-1H-benzo[d]imidazol-1-yl)piperidine-2,6-dione (500 mg, 1.4 mmol, 1.0 eq) and 4-(dimethoxymethyl)piperidine (353 mg, 2.2 mmol, 1.5 eq) in 1,4-dioxane (10 mL) were added cesium carbonate (1.4 g, 4.4 mmol, 3.0 eq) and Pd-PEPPSI-IHept-Cl (144 mg, 0.15 mmol, 0.1 eq) at rt. The mixture was stirred at 120°C under N₂ for 16 h. Upon completion, the mixture was cooled to rt and poured into a 5% AcOH solution in ice-cold water. The mixture was extracted with EtOAc (×3), washed with saturated aqueous NaCl, dried over anhydrous Na₂SO₄, and concentrated under reduced pressure. The residue was purified by column chromatography to afford the title compound (yellow solid, 120 mg, yield 19%).

### Step B: 1-(-(2,6-dioxopiperidin-3-yl)-3-methyl-2-oxo-2,3-dihydro-1H-benzo[d]imidazol-4-yl)piperidine-4-carbaldehyde

To 3-(4-(4-(dimethoxymethyl)piperidin-1-yl)-3-methyl-2-oxo-2,3-dihydro-1H-benzo[d]imidazol-1-yl)piperidine-2,6-dione (120 mg, 0.29 mmol, 1.0 eq) was added formic acid (3 mL). The mixture was stirred at 30°C for 1 h. Upon completion, the mixture was concentrated under reduced pressure to afford the title compound (brown oil, 100 mg, crude), which was used in the next step without purification.

### Step C: N-(3-carbamoyl-1-(4-((1-(1-(2,6-dioxopiperidin-3-yl)-3-methyl-2-oxo-2,3-dihydro-1H-benzo[d]imidazol-4-yl)piperidin-4-yl)methyl)piperazin-1-yl)phenyl)-1H-pyrazol-4-yl)-6-(trifluoromethyl)picolinamide

To a solution of 1-(2,6-dioxopiperidin-3-yl)-3-methyl-2-oxo-2,3-dihydro-1H-benzo[d]imidazol-4-yl)piperidine-4-carbaldehyde (100 mg, 0.27 mmol, 1.0 eq) and N-(3-amino-1-(4-(piperazin-1-yl)phenyl)-1H-pyrazol-4-yl)-6-(trifluoromethyl)picolinamide (124 mg, 0.27 mmol, 1.0 eq) in DMF (5 mL) was added AcOH (0.02 mL, 0.27 mmol, 0.1 eq) at rt, and the mixture was stirred for 0.5 h. Sodium triacetoxyborohydride (171 mg, 0.81 mmol, 3.0 eq) was then added to the above reaction solution. The mixture was stirred at rt for 2 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (yellow solid, 48 mg, yield 21%). **¹H NMR** (400 MHz, DMSO-d6) δ 11.79 (s, 1H), 11.08 (s, 1H), 8.88 (s, 1H), 8.45 (d, J = 7.6 Hz, 1H), 8.39 (t, J = 7.8 Hz, 1H), 8.21 (d, J = 7.6 Hz, 1H), 7.94 (s, 1H), 7.79 (d, J = 8.9 Hz, 2H), 7.70 (s, 1H), 7.08 (d, J = 8.6 Hz, 2H), 7.00-6.94 (m, 1H), 6.92-6.85 (m, 2H), 5.45-5.27 (m, 1H), 3.63 (s, 3H), 3.27-3.19 (m, 4H), 3.15-3.09 (m, 2H), 2.94-2.85 (m, 1H), 2.74-2.59 (m, 4H), 2.55-2.53 (m, 4H), 2.27 (d, J = 5.0 Hz, 2H), 2.04-1.96 (m, 1H), 1.85 (d, J = 11.9 Hz, 2H), 1.74-1.63 (m, 1H), 1.35 (d, J = 10.3 Hz, 2H). LC/MS (ESI): m/z 814 (M+H)⁺.

### Preparation Example 263. Synthesis of Compound A179

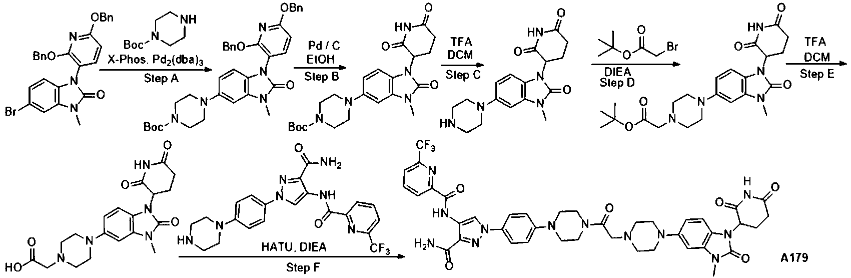

### Step A: Tert-butyl 4-(1-(2,6-bis(benzyloxy)pyridin-3-yl)-3-methyl-2-oxo-2,3-dihydro-1H-benzo[d]imidazol-5-yl)piperazine-1-carboxylate

To a solution of 1-(2,6-bis(benzyloxy)pyridin-3-yl)-5-bromo-3-methyl-1,3-dihydro-2H-benzo[d]imidazol-2-one (700 mg, 0.39 mmol, 1.0 eq) and tert-butyl piperazine-1-carboxylate (108 mg, 0.58 mmol, 1.5 eq) in 1,4-dioxane (10 mL) at rt were added X-Phos (37 mg, 0.078 mmol, 0.2 eq), Pd₂(dba)₃ (71 mg, 0.078 mmol, 0.2 eq), and cesium carbonate (380 mg, 1.17 mmol, 3.0 eq). The reaction solution was stirred at 90°C under N₂ for 16 h. Upon completion, the reaction solution was cooled to rt, poured into water, and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 180 mg, yield 74%).

### Step B: Tert-butyl 4-(1-(2,6-dioxopiperidin-3-yl)-3-methyl-2-oxo-2,3-dihydro-1H-benzo[d]imidazol-5-yl)piperazine-1-carboxylate

To a solution of tert-butyl 4-(1-(2,6-bis(benzyloxy)pyridin-3-yl)-3-methyl-2-oxo-2,3-dihydro-1H-benzo[d]imidazol-5-yl)piperazine-1-carboxylate (180 mg, 0.29 mmol, 1.0 eq) in ethanol (15 mL) at rt was added 10% palladium on carbon (180 mg). The reaction solution was purged with H₂ (×3). The mixture was reacted at 50°C under H₂ for 16 h. Upon completion, the reaction solution was filtered, the filter cake was washed with ethanol (×3), and the filtrate was concentrated under reduced pressure and purified to afford the title compound (white solid, 110 mg, yield 86%).

### Step C: 3-(3-methyl-2-oxo-5-(piperazin-1-yl)-2,3-dihydro-1H-benzo[d]imidazol-1-yl)piperidine-2,6-dione

To a solution of tert-butyl 4-(1-(2,6-dioxopiperidin-3-yl)-3-methyl-2-oxo-2,3-dihydro-1H-benzo[d]imidazol-5-yl)piperazine-1-carboxylate (110 mg, 0.25 mmol, 1.0 eq) in DCM (10 mL) was added TFA (2 mL) at rt. The reaction solution was reacted at rt for 1 h. Upon completion, the reaction solution was concentrated under reduced pressure and purified to afford the title compound (yellow solid, 80 mg, crude trifluoroacetate).

### Step D: Tert-butyl 2-(4-(1-(2,6-dioxopiperidin-3-yl)-3-methyl-2-oxo-2,3-dihydro-1H-benzo[d]imidazol-5-yl)piperazin-1-yl)acetate

To a solution of 3-(3-methyl-2-oxo-5-(piperazin-1-yl)-2,3-dihydro-1H-benzo[d]imidazol-1-yl)piperidine-2,6-dione (80 mg, 0.23 mmol, 1.0 eq) and tert-butyl 2-bromoacetate (67 mg, 0.345 mmol, 1.5 eq) in DMF (10 mL) was added DIEA (149 mg, 1.15 mmol, 5 eq) at rt. The reaction solution was reacted at rt for 5 h under N₂. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 80 mg, yield 76%).

### Step E: 2-(4-(1-(2,6-dioxopiperidin-3-yl)-3-methyl-2-oxo-2,3-dihydro-1H-benzo[d]imidazol-5-yl)piperazin-1-yl)acetic acid

To a solution of tert-butyl 2-(4-(1-(2,6-dioxopiperidin-3-yl)-3-methyl-2-oxo-2,3-dihydro-1H-benzo[d]imidazol-5-yl)piperazin-1-yl)acetate (80 mg, 0.175 mmol, 1.0 eq) in DCM (10 mL) was added TFA (2 mL) at rt. The reaction solution was reacted at rt for 1 h. Upon completion, the reaction solution was concentrated under reduced pressure and purified to afford the title compound (yellow solid, 65 mg, crude trifluoroacetate).

### Step F: N-(3-carbonyl-1-(4-(4-(2-(4-(1-(2,6-dioxopiperidin-3-yl)-3-methyl-2-oxo-2,3-dihydro-1H-benzo[d]imidazol-5-yl)piperazin-1-yl)acetyl)piperazin-1-yl)phenyl)-1H-pyrazol-4-yl)-6-(trifluoromethyl)picolinamide

To a solution of 2-(4-(1-(2,6-dioxopiperidin-3-yl)-3-methyl-2-oxo-2,3-dihydro-1H-benzo[d]imidazol-5-yl)piperazin-1-yl)acetic acid (65 mg, 0.16 mmol, 1.0 eq) and N-(3-amino-1-(4-(piperazin-1-yl)phenyl)-1H-pyrazol-4-yl)-6-(trifluoromethyl)picolinamide (88 mg, 0.19 mmol, 1.2 eq) in DMF (8.0 mL) were added DIEA (83.2 mg, 0.64 mmol, 4 eq) and HATU (121.6 mg, 0.32 mmol, 2.0 eq) at 0°C. The reaction solution was reacted at rt for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (white solid, 15.1 mg, yield 15%). ¹HNMR (400 MHz, DMSO-*d*₆) δ 11.85-11.74 (m, 1H), 11.07 (s, 1H), 8.90 (s, 1H), 8.51-8.34 (m, 2H), 8.22 (d, *J* = 7.5 Hz, 1H), 7.95 (s, 1H), 7.81 (t, *J* = 16.5 Hz, 2H), 7.73 (s, 1H), 7.14 (d, *J* = 7.3 Hz, 2H), 6.94 (d, *J* = 32.1 Hz, 2H), 6.69 (s, 1H), 5.30 (d, *J* = 9.0 Hz, 1H), 4.48 (s, 1H), 3.76-3.56 (m, 8H), 3.29-3.09 (m, 8H), 2.91 (m, 2H), 2.76-2.59 (m, 5H), 2.00 (d, *J=* 7.3 Hz, 1H). LCMS (ESI): m/z 843 [M+H]⁺.

### Preparation Example 264. Synthesis of Compound A180

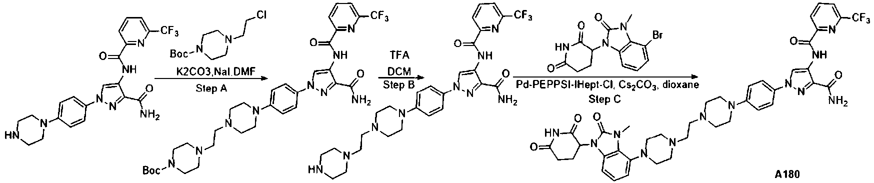

### Step A: Tert-butyl 4-(2-(4-(4-(3-amino-4-(6-(trifluoromethyl)picolinamido)-1H-pyrazol-1-yl)phenyl)piperazin-1-yl)ethyl)piperazine 1-carboxylate

To a solution of 1-[4-(piperazin-1-yl)phenyl]-4-({[6-(trifluoromethyl)pyridin-2-yl]carbonyl}amino)pyrazole-3-carboxamide (2.0 g, 4.353 mmol, 1.0 eq) and tert-butyl 4-(2-chloroethyl)piperazine-1-carboxylate (1.08 g, 4.353 mmol, 1.0 eq) in DMF (20 mL) at rt were added potassium carbonate (2.41 g, 17.413 mmol, 3.0 eq) and sodium iodide (0.13 g, 0.871 mmol, 0.2 eq). The reaction solution was stirred at 70°C under N₂ for 16 h. Upon completion, the reaction solution was cooled to rt and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (yellow solid, 2.6 g, yield 89%).

### Step B: N-(3-amino-1-(4-(4-(2-(piperazin-1-yl)ethyl)piperazine-1-carbonyl)phenyl)-1H-pyrazol-4-yl)-6-(trifluoromethyl)picolinamide

To a solution of tert-butyl 4-(2-(4-(4-(3-amino-4-(6-(trifluoromethyl)picolinamido)-1H-pyrazol-1-yl)phenyl)piperazin-1-yl)ethyl)piperazine 1-carboxylate (2.6 g, 3.871 mmol, 1.0 eq) in DCM (20 mL) was added TFA (5 mL) at 0°C. The reaction solution was stirred at rt under N₂ for 2 h. Upon completion, the reaction solution was adjusted to pH 8-9 with 1N aqueous NaOH. The mixture was extracted with DCM (×3), and the combined organic phases were washed sequentially with water and saturated aqueous NaCl, dried over anhydrous Na₂SO₄ and filtered. The filtrate was concentrated under reduced pressure to yield the title compound (yellow solid, 1.8 g, crude), which was used directly without purification.

### Step C: N-(3-carbonyl-1-(4-(4-(2-(4-(1-(2,6-dioxopiperidin-3-yl)-3-methyl-2-oxo-2,3-dihydro-1H-benzo[d]imidazol-4-yl)piperazin-1-yl)ethyl)piperazin-1-yl)phenyl)-1H-pyrazol-4-yl)-6-(trifluoromethyl)picolinamide

To a solution of N-(3-amino-1-(4-(4-(2-(piperazin-1-yl)ethyl)piperazine-1-carbonyl)phenyl)-1H-pyrazol-4-yl)-6-(trifluoromethyl)picolinamide (400 mg, 0.700 mmol, 1.0 eq) and 3-(4-bromo-3-methyl-2-oxo-2,3-dihydro-1H-benzo[d]imidazol-1-yl)piperidine-2,6-dione (260.30 mg, 0.770 mmol, 1.1 eq) in 1,4-dioxane (10 mL) at rt were added cesium carbonate (684.00 mg, 2.099 mmol, 3.0 eq) and Pd-PEPPSI-IHept-Cl (68.09 mg, 0.070 mmol, 0.1 eq). The mixture was stirred at 120°C under N₂ for 16 h. Upon completion, the mixture was cooled to rt, diluted in water, extracted with EtOAc, washed with saturated aqueous NaCl, dried over anhydrous Na₂SO₄, filtered, and concentrated under reduced pressure. The residue was purified by HPLC to afford the title compound (white solid, 14 mg, yield 3%). **¹H NMR** (400 MHz, DMSO-*d*₆) δ 11.79 (s, 1H), 11.09 (s, 1H), 8.88 (s, 1H), 8.45-8.38 (m, 2H), 8.21 (s, 1H), 7.95 (s, 1H), 7.79 (d, *J=* 8.8 Hz, 2H), 7.71 (s, 1H), 7.08 (d, *J =* 8.8 Hz, 2H), 6.99-6.90 (m, 3H), 5.37-5.32 (m, 1H), 3.63 (s, 3H), 3.22 (s, 4H), 2.98-2.91 (m, 8H), 2.60 (s, 4H), 2.55 (s, 4H), 2.33 (s, 1H), 2.00 (d, *J =* 7.4 Hz, 2H), 1.75 (s, 1H). LCMS (ESI): m/z 829 [M+H]⁺.

### Preparation Example 265. Synthesis of Compound A181

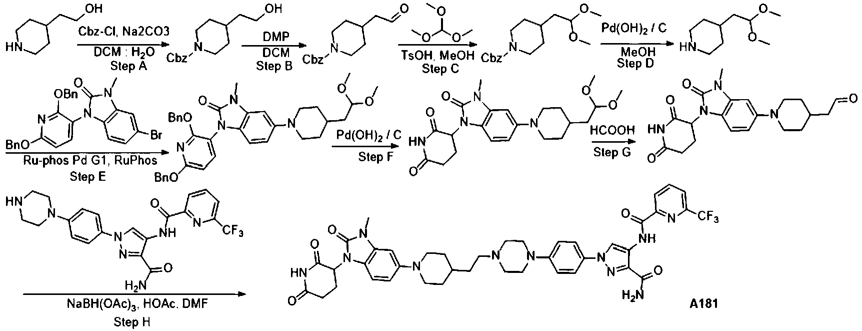

### Step A: Benzyl 4-(2-hydroxyethyl)piperidine-1-carboxylate

To a solution of 2-(piperidin-4-yl)ethan-1-ol (12.5 g, 96.7 mmol, 1.0 eq) in DCM (250 mL) and water (250 mL) at 0°C were added sodium carbonate (41 g, 387.0 mmol, 4.0 eq) and benzyl chloroformate (19.8 g, 116.1 mmol, 1.2 eq). The reaction solution was stirred at rt for 6 h. Upon completion, the reaction solution was quenched with water and extracted with DCM (×3). The combined organic phases were washed sequentially with water and saturated aqueous NaCl, dried over anhydrous Na₂SO₄, filtered, and concentrated under reduced pressure at low temperature. The residue was purified by column chromatography to afford the title compound (yellow oil, 17.5 g, 69%).

### Step B: Benzyl 4-(2-oxoethyl)piperidine-1-carboxylate

At 0°C, to a solution of benzyl 4-(2-hydroxyethyl)piperidine-1-carboxylate (12.5 g, 47.5 mmol, 1.0 eq) in DCM (150 mL) was added Dess-Martin periodinane (22.2 g, 52.2 mmol, 1.1 eq). The mixture was stirred at rt for 3 h. Upon completion, the reaction was quenched with saturated NaHCO₃ solution and stirred for 0.5 h. The mixture was extracted with DCM (×3). The combined organic phases were washed with water and saturated aqueous NaCl, dried over anhydrous Na₂SO₄, filtered and concentrated under reduced pressure. The residue was purified by column chromatography to afford the title compound (yellow oil, 10 g, yield 80%).

### Step C: Benzyl 4-(2,2-dimethoxyethyl)piperidine-1-carboxylate

At 0°C, to a solution of benzyl 4-(2-oxoethyl)piperidine-1-carboxylate (10 g, 38.2 mmol, 1.0 eq) in MeOH (100 mL) was added trimethoxymethane (4.2 mL, 38.2 mmol, 1.0 eq) and p-toluenesulfonic acid (0.66 g, 3.8 mmol, 0.1 eq). The reaction solution was stirred at 15°C for 1 h. Upon completion, the reaction solution was quenched with water and extracted with DCM (×3). The combined organic phases were washed sequentially with water and saturated aqueous NaCl, dried over anhydrous Na₂SO₄, filtered, and concentrated under reduced pressure at low temperature to afford the title compound (11 g, yellow oil, crude).

### Step D: 4-(2,2-dimethoxyethyl)piperidine

To a solution of benzyl 4-(2,2-dimethoxyethyl)piperidine-1-carboxylate (11 g, 35.8 mmol, 1.0 eq) in MeOH (110 mL) was added 10% palladium hydroxide on carbon (1 g) at rt. The reaction solution was purged with H₂ (×3) and stirred at rt under H₂ for 24 h. Upon completion, the mixture was filtered, and the filtrate was concentrated under reduced pressure. The residue was purified by column chromatography to afford the title compound (colorless oil, 6 g, yield 97%).

### Step E: 1-(2,6-bis(benzyloxy)pyridin-3-yl)-5-(4-(2,2-dimethoxyethyl)piperidin-1-yl)-3-methyl-1,3-dihydro-2H-benzo[d]imidazol-2-one

To a solution of 1-(2,6-bis(benzyloxy)pyridin-3-yl)-5-bromo-3-methyl-1,3-dihydro-2H-benzo[d]imidazol-2-one (500 mg, 1.0 mmol, 1.0 eq) and 4-(2,2-dimethoxyethyl)piperidine (252 mg, 1.5 mmol, 1.5 eq) in 1,4-dioxane (5 mL) were added cesium carbonate (946 mg, 3.0 mmol, 3.0 eq), Ru-phos (45 mg, 0.1 mmol, 0.1 eq), and Ru-Phos Pd G1 (79 mg, 0.1 mmol, 0.1 eq) at rt. The mixture was stirred at 100°C under N₂ for 16 h. Upon completion, the mixture was cooled to rt, diluted in water, extracted with EtOAc, washed with saturated aqueous NaCl, dried over anhydrous Na₂SO₄, filtered, and concentrated under reduced pressure. The residue was purified by column chromatography to afford the title compound (yellow solid, 400 mg, yield 68%).

### Step F: 3-(5-(4-(2,2-dimethoxyethyl)piperidin-1-yl)-3-methyl-2-oxo-2,3-dihydro-1H-benzo[d]imidazol-1-yl)piperidine-2,6-dione

To a solution of 1-(2,6-bis(benzyloxy)pyridin-3-yl)-5-(4-(2,2-dimethoxyethyl)piperidin-1-yl)-3-methyl-1,3-dihydro-2H-benzo[d]imidazol-2-one (200 mg, 0.3 mmol, 1.0 eq) in ethanol (5 mL) was added 10% palladium hydroxide on carbon (200 mg) at rt. The reaction solution was purged with H₂ (×3) and stirred at 50°C under H₂ for 16 h. Upon completion, the mixture was filtered, and the filtrate was concentrated under reduced pressure. The residue was purified by column chromatography to afford the title compound (yellow solid, 100 mg, crude), which was used directly without purification.

### Step G: 2-(1-(1-(2,6-dioxopiperidin-3-yl)-3-methyl-2-oxo-2,3-dihydro-1H-benzo[d]imidazol-5-yl)piperidin-4-yl)acetaldehyde

To 3-(5-(4-(2,2-dimethoxyethyl)piperidin-1-yl)-3-methyl-2-oxo-2,3-dihydro-1H-benzo[d]imidazol-1-yl)piperidine-2,6-dione (100 mg, 0.232 mmol, 1.0 eq) was added formic acid (4 mL) at 0°C. The reaction solution was stirred at rt under N₂ for 2 h. Upon completion, the reaction solution was concentrated under reduced pressure to afford the title compound (white oil, 80 mg, crude), which was used directly without purification.

### Step H: N-(3-carbamoyl-1-(4-(4-(2-(1-(1-(2,6-dioxopiperidin-3-yl)-3-methyl-2-oxo-2,3-dihydro-1H-benzo[d]imidazol-5-yl)piperidin-4-yl)ethyl)piperazin-1-yl)phenyl)-1H-pyrazol-4-yl)-6-(trifluoromethyl)picolinamide

To a solution of 2-(1-(1-(2,6-dioxopiperidin-3-yl)-3-methyl-2-oxo-2,3-dihydro-1H-benzo[d]imidazol-5-yl)piperidin-4-yl)acetaldehyde (80 mg, 0.2 mmol, 1.0 eq) and N-(3-amino-1-((1r,4r)-4-(piperazin-1-yl)cyclohexyl)-1H-pyrazol-4-yl)-6-(trifluoromethyl)picolinamide (96 mg, 0.2 mmol, 1.0 eq) in DMF (3 mL) was added AcOH (5 drops) at rt. The mixture was stirred at rt for 1 h. Sodium triacetoxyborohydride (176 mg, 0.8 mmol, 4.0 eq) was then added to the above reaction solution. The mixture was stirred at rt for 5 min. Upon completion, the mixture was diluted in water, extracted with EtOAc (×3), washed with saturated aqueous NaCl, dried over anhydrous Na₂SO₄, filtered, and concentrated under reduced pressure. The residue was purified by column chromatography to afford the title compound (white solid, 20.3 mg, yield 12%). **¹H NMR** (400 MHz, DMSO-*d*₆) δ 11.79 (s, 1H), 11.06 (s, 1H), 8.88 (s, 1H), 8.45 (d, *J* = 7.5 Hz, 1H), 8.40 (t, *J=* 7.8 Hz, 1H), 8.22-8.20 (m, 1H), 7.95 (s, 1H), 7.79 (d, *J=* 9.0 Hz, 2H), 7.71 (s, 1H), 7.08 (d, *J =* 9.2 Hz, 2H), 6.92 (d, *J =* 8.6 Hz, 1H), 6.82 (d, *J =* 2.0 Hz, 1H), 6.63 (dd, *J =* 8.7, 1.9 Hz, 1H), 5.29 (dd, *J =* 12.9, 5.2 Hz, 1H), 3.57 (d, *J =* 12.1 Hz, 2H), 3.30 (s, 3H), 3.24-3.20 (m, 4H), 2.91-2.85 (m, 1H), 2.73-2.66 (m, 1H), 2.65-2.56 (m, 4H), 2.55-2.52 (m, 4H), 2.42-2.38 (m, 2H), 2.01-1.96 (m, 1H), 1.79 (d, *J=* 12.0 Hz, 2H), 1.48-1.45 (m, 2H), 1.36-1.29 (m, 2H). LCMS (ESI): m/z 828 [M+H]⁺.

### Preparation Example 266. Synthesis of Compound A182

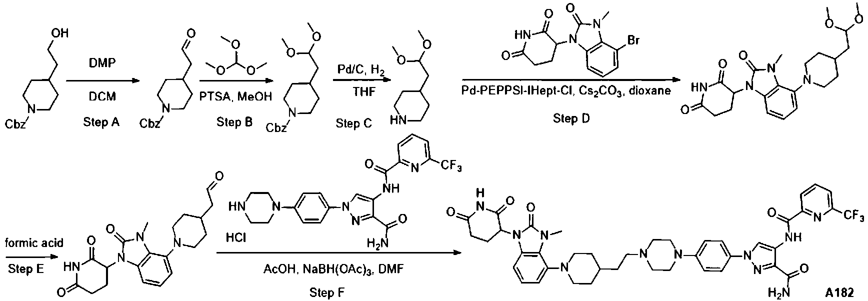

### Step A: Benzyl 4-(2-oxoethyl)piperidine-1-carboxylate

At 0°C, to a solution of benzyl 4-(2-hydroxyethyl)piperidine-1-carboxylate (1 g, 3.8 mmol, 1.0 eq) in DCM (15 mL) was added Dess-Martin periodinane (2.4 g, 5.7 mmol, 1.5 eq). The mixture was stirred at 30°C for 3 h. Upon completion, the reaction was quenched with saturated NaHCO₃ solution and stirred for 0.5 h. The mixture was extracted with DCM (×3). The combined organic phases were washed with water and saturated aqueous NaCl, dried over anhydrous Na₂SO₄, filtered and concentrated under reduced pressure. The residue was purified by column chromatography to afford the title compound (yellow oil, 700 mg, crude), which was used directly without purification.

### Step B: Benzyl 4-(2,2-dimethoxyethyl)piperidine-1-carboxylate

To a solution of benzyl 4-(2-oxoethyl)piperidine-1-carboxylate (700 mg, 2.7 mmol, 1.0 eq) and trimethoxymethane (0.44 mL, 4.0 mmol, 1.5 eq) in MeOH (100 mL) was added 4-methylbenzenesulfonic acid (46 mg, 0.7 mmol, 0.25 eq) at rt. The reaction solution was stirred at 30°C under N₂ for 5 h. The reaction solution was concentrated under reduced pressure. The residue was purified by column chromatography to afford the title compound (yellow oil, 500 mg, yield 61%).

### Step C: 4-(2,2-Dimethoxyethyl)piperidine

To a solution of benzyl 4-(2,2-dimethoxyethyl)piperidine-1-carboxylate (500 mg, 1.6 mmol, 1.0 eq) in THF (10 mL) at rt was added 10% palladium on carbon (173 mg, 0.1 eq). The reaction solution was purged with H₂ (using a hydrogen balloon) (×3) and stirred under H₂ at 30°C for 4 h. Upon completion, the mixture was filtered, and the filtrate was concentrated under reduced pressure to afford the title compound (yellow oil, 250 mg, yield 89%, crude), which was used directly without purification.

### Step D: 3-(4-(4-(2,2-dimethoxyethyl)piperidin-1-yl)-3-methyl-2-oxo-2,3-dihydro-1H-benzo[d]imidazol-1-yl)piperidine-2,6-dione

To a solution of 4-(2,2-dimethoxyethyl)piperidine (250 mg, 1.4 mmol, 1.0 eq) and 3-(4-bromo-3-methyl-2-oxo-2,3-dihydro-1H-benzo[d]imidazol-1-yl)piperidine-2,6-dione (488 mg, 1.4 mmol, 1.0 eq) in 1,4-dioxane (10 mL) were added cesium carbonate (940 mg, 2.9 mmol, 2.0 eq) and Pd-PEPPSI-IHept-Cl (70 mg, 0.07 mmol, 0.05 eq) at rt. The mixture was stirred at 120°C under N₂ for 5 h. Upon completion, the mixture was cooled to rt, diluted in water, extracted with EtOAc, washed with saturated aqueous NaCl, dried over anhydrous Na₂SO₄, and concentrated under reduced pressure. The residue was purified by column chromatography to afford the title compound (yellow solid, 150 mg, yield 24%).

### Step E: 2-(1-(1-(2,6-dioxopiperidin-3-yl)-3-methyl-2-oxo-2,3-dihydro-1H-benzo[d]imidazol-4-yl)piperidin-4-yl)acetaldehyde

To 3-(4-(4-(2,2-dimethoxyethyl)piperidin-1-yl)-3-methyl-2-oxo-2,3-dihydro-1H-benzo[d]imidazol-1-yl)piperidine-2,6-dione (150 mg, 0.35 mmol, 1.0 eq) was added formic acid (5 mL) at rt. The reaction solution was stirred at 30°C for 2 h. Upon completion, the reaction solution was concentrated under reduced pressure to afford the title compound (120 mg, crude).

### Step F: N-(3-carbamoyl-1-(4-(4-(2-(1-(1-(2,6-dioxopiperidin-3-yl)-3-methyl-2-oxo-2,3-dihydro-1H-benzo[d]imidazol-4-yl)piperidin-4-yl)ethyl)piperazin-1-yl)phenyl)-1H-pyrazol-4-yl)-6-(trifluoromethyl)picolinamide

To a solution of 2-(1-(1-(2,6-dioxopiperidin-3-yl)-3-methyl-2-oxo-2,3-dihydro-1H-benzo[d]imidazol-4-yl)piperidin-4-yl)acetaldehyde (80 mg, 0.21 mmol, 1.0 eq) and N-(3-amino-1-(4-(piperazin-1-yl)phenyl)-1H-pyrazol-4-yl)-6-(trifluoromethyl)picolinamide (95.61 mg, 0.21 mmol, 1.0 eq) in DMF (5 mL) was added AcOH (0.012 mL, 0.21 mmol, 1.0 eq) at rt. The mixture was stirred at rt for 1 h. Sodium triacetoxyborohydride (88 mg, 0.42 mmol, 2.0 eq) was then added to the above reaction solution. The mixture was stirred at 30°C for 3 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (white solid, 20 mg, yield 12%). **¹H NMR** (400 MHz, DMSO-d₆) δ 11.79 (s, 1H), 11.08 (s, 1H), 8.88 (s, 1H), 8.48-8.35 (m, 2H), 8.21 (d, J = 6.7 Hz, 1H), 7.95 (s, 1H), 7.79 (d, J = 9.1 Hz, 2H), 7.71 (s, 1H), 7.08 (d, J = 9.2 Hz, 2H), 7.00-6.82 (m, 3H), 5.35 (dd, J = 12.5, 5.3 Hz, 1H), 3.63 (s, 3H), 3.26-3.18 (m, 5H), 3.10 (d, J = 11.2 Hz, 2H), 2.94-2.84 (m, 1H), 2.78-2.62 (m, 4H), 2.61-2.53 (m, 4H), 2.44-2.39 (m, 2H), 2.04-1.95 (m, 1H), 1.81 (d, J = 10.8 Hz, 2H), 1.53-1.38 (m, 4H). LCMS (ESI): m/z 828 [M+H]⁺.

### Preparation Example 267. Synthesis of Compound A183

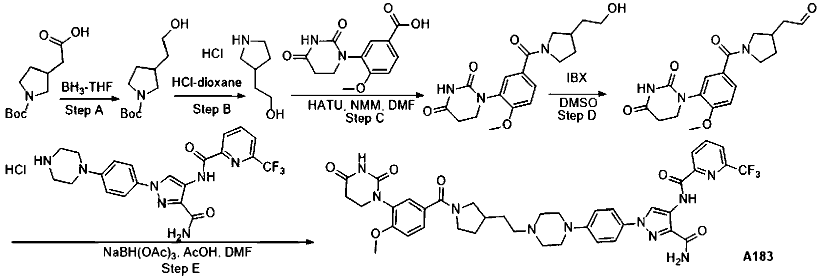

### Step A: Tert-butyl 3-(2-hydroxyethyl)pyrrolidine-1-carboxylate

At 0°C, to a solution of 1-{[(2-methylpropan-2-yl)oxy]carbonyl } tetrahydro-1H-pyrrol-3-yl)acetic acid (2 g, 8.7 mmol, 1.0 eq) in THF (3 mL) was added 1 M borane in THF (10 mL, 10 mmol, 1.1 eq). The reaction solution was stirred at rt under N₂ for 18 h. Upon completion, the reaction was quenched with MeOH and stirred for 1 h. The mixture was concentrated under reduced pressure. The residue was purified by column chromatography to afford the title compound (yellow oil, 1.8 g, yield 96%).

### Step B: 2-(Pyrrolidin-3-yl)ethan-1-ol hydrochloride

To tert-butyl 3-(2-hydroxyethyl)pyrrolidine-1-carboxylate (500 mg, 2.3 mmol, 1.0 eq) was added 4 M HCl in 1,4-dioxane (5 mL, 20 mmol, 9 eq) at 0°C. The reaction solution was stirred at rt under N₂ for 1 h. The filtrate was concentrated under reduced pressure to afford the title compound (yellow oil, 300 mg, crude), which was used directly without purification.

### Step C: 1-(5-(3-(2-hydroxyethyl)pyrrolidine-1-carbonyl)-2-methoxyphenyl)dihydropyrimidine-2,4(1H,3H)-dione

To a solution of 3-(2,4-dioxohexahydropyrimidin-1-yl)-4-methoxybenzoic acid (600 mg, 2.3 mmol, 1.0 eq) and 2-(pyrrolidin-3-yl)ethan-1-ol hydrochloride (261.5 mg, 2.31 mmol, 1.0 eq) in DMF (10 mL) were added N-methylmorpholine (1.3 mL, 11.4 mmol, 5.0 eq) and HATU (1.3 g, 3.4 mmol, 1.5 eq) at rt. The reaction solution was stirred at rt under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 700 mg, yield 85%).

### Step D: 2-(1-(3-(2,4-dioxotetrahydropyrimidin-1(2H)-yl)-4-methoxybenzoyl)pyrrolidin-3-yl)acetaldehyde

To a solution of 1-(5-(3-(2-hydroxyethyl)pyrrolidine-1-carbonyl)-2-methoxyphenyl)dihydropyrimidine-2,4-(1H,3H)-dione (500 mg, 1.4 mmol, 1.0 eq) in dimethyl sulfoxide (10 mL) at rt was added IBX (775 mg, 2.8 mmol, 2.0 eq). The reaction solution was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by reverse-phase column chromatography to afford the title compound (yellow solid, 300 mg, yield 60%).

### Step E: N-(3-carbonyl-1-(4-(4-(2-(1-(3-(2,4-dioxotetrahydropyrimidin-1(2H)-yl)-4-methoxybenzoyl)pyrrolidin-3-yl)ethyl)piperazin-1-yl)phenyl)-1H-pyrazol-4-yl)-6-(trifluoromethyl)picolinamide

At rt, to a solution of 1-[4-(piperazin-1-yl)phenyl]-4-({[6-(trifluoromethyl)pyridin-2-yl]carbonyl}amino)pyrazole-3-carboxamide hydrochloride (191.8 mg, 0.4 mmol, 1.0 eq) in DMF (5 mL) was added DIEA (0.2 mL), and the mixture was stirred for 5 min. AcOH (0.3 mL) and 2-(1-(3-(2,4-dioxotetrahydropyrimidin-1(2H)-yl)-4-methoxybenzoyl)pyrrolidin-3-yl)acetaldehyde (150 mg, 0.4 mmol, 1.0 eq) were then added. The mixture was stirred at rt for 15 h. Sodium triacetoxyborohydride (154.6 mg, 0.7 mmol, 1.8 eq) was then added to the above reaction solution. The mixture was stirred at rt for 1 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (yellow solid, 20.2 mg, yield 6%). **¹H NMR** (400 MHz, DMSO-*d*₆) δ 11.79 (s, 1H), 10.33 (s, 1H), 8.88 (s, 1H), 8.49-8.35 (m, 2H), 8.21 (dd, *J* = 7.6, 1.2 Hz, 1H), 7.95 (s, 1H), 7.78 (d, *J* = 8.6 Hz, 2H), 7.71 (s, 1H), 7.54 (dd, *J* = 8.6, 2.0 Hz, 1H), 7.49 (d, *J* = 3.6 Hz, 1H), 7.17-7.00 (m, 3H), 3.83 (d, *J =* 16.6 Hz, 3H), 3.72-3.42 (m, 5H), 3.24-3.05 (m, 5H), 2.66 (dt, *J* = 10.2, 7.2 Hz, 2H), 2.55 (ddd, *J* = 4.8, 3.6, 1.0 Hz, 2H), 2.46 (dd, *J=* 8.8, 5.2 Hz, 2H), 2.40 (dd, *J=* 10.6, 6.8 Hz, 1H), 2.35-2.28 (m, 1H), 2.25-2.11 (m, 1H), 2.09-1.97 (m, 1H), 1.68-1.48 (m, 3H). LCMS (ESI): m/z 803 [M+H]⁺.

### Preparation Example 268. Synthesis of Compound A184

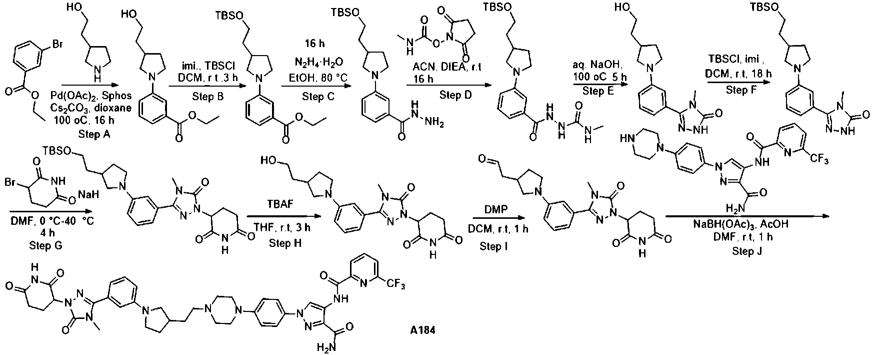

### Step A: Ethyl 3-(3-(2-hydroxyethyl)pyrrolidin-1-yl)benzoate

To a solution of ethyl 3-bromobenzoate (2.5 g, 10.91 mmol, 1.0 eq) and 2-(pyrrolidin-3-yl)ethane-1-ol (1.9 g, 16.37 mmol, 1.5 eq) in 1,4-dioxane (50 mL) at rt were added cesium carbonate (10.7 g, 32.74 mmol, 3.0 eq), S-Phos (0.9 g, 2.18 mmol, 0.2 eq), and Pd(OAc)₂ (0.25 g, 1.09 mmol, 0.1 eq). The mixture was stirred at 100°C under N₂ for 16 h. Upon completion, the mixture was cooled to rt, diluted in water, extracted with EtOAc, washed with saturated aqueous NaCl, dried over anhydrous Na₂SO₄, and concentrated under reduced pressure. The residue was purified by column chromatography to afford the title compound (white solid, 2.2 g, yield 77%).

### Step B: Ethyl 3-(3-(2-((tert-butyldimethylsilyl)oxy)ethyl))pyrrolidin-1-yl)benzoate

At 0°C, to a solution of ethyl 3-(3-(2-hydroxyethyl)pyrrolidin-1-yl)benzoate (2.2 g, 8.35 mmol, 1.0 eq) and imidazole (1.1 g, 16.71 mmol, 2.0 eq) in DCM (15 mL) was added tert-butyldimethylsilyl chloride (1.9 g, 12.53 mmol, 1.5 eq). The reaction solution was stirred at rt under N₂ for 3 h. Upon completion, the reaction solution was quenched with water and extracted with DCM (×3). The combined organic phases were washed sequentially with water and saturated aqueous NaCl, dried over anhydrous Na₂SO₄, filtered, and concentrated under reduced pressure. The residue was purified by column chromatography to afford the title compound (colorless liquid, 2.1 g, yield 67%).

### Step C: 3-(3-(2-((tert-Butyldimethylsilyl)oxy)ethyl)pyrrolidin-1-yl)benzohydrazide

At 0°C, to a solution of ethyl 3-(3-(2-((tert-butyldimethylsilyl)oxy)ethyl)pyrrolidin-1-yl)benzoate (2.1 g, 5.561 mmol) in ethanol (15 mL) was added 80% hydrazine hydrate (15 mL). The reaction solution was stirred at 80°C under N₂ for 16 h. Upon completion, the reaction solution was cooled to rt, quenched with water, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (colorless liquid, 1.9 g, yield 94%).

### Step D: 2-(3-(3-(2-((tert-Butyldimethylsilyl)oxy)ethyl)pyrrolidin-1-yl)benzoyl)-N-methylhydrazine-1-carboxamide

To a solution of 3-(3-(2-((tert-butyldimethylsilyl)oxy)ethyl)pyrrolidin-1-yl)benzohydrazide (1.9 g, 5.23 mmol, 1.0 eq) and 2,5-dioxopyrrolidin-1-ylmethylcarbamate (1.1 g, 7.84 mmol, 1.5 eq) in acetonitrile (20 mL) was added DIEA (2.0 g, 15.67 mmol, 3.0 eq) at rt. The mixture was stirred at rt under N₂ for 16 h, Upon completion, water was added, and the mixture was extracted with EtOAc, washed with saturated aqueous NaCl, dried over anhydrous Na₂SO₄, filtered, and concentrated under reduced pressure to afford the title compound (off-white solid, 2.1 g, crude), which was used directly without purification.

### Step E: 5-(3-(3-(2-hydroxyethyl)pyrrolidin-1-yl)phenyl)-4-methyl-2,4-dihydro-3H-1,2,4-triazol-3-one

At 0°C, to 2-(3-(3-(2-((tert-butyldimethylsilyl)oxy)ethyl)pyrrolidin-1-yl)benzoyl)-N-methylhydrazine-1-carboxamide (2.1 g, 4.99 mmol, 1.0 eq) was added aqueous NaOH (25.0 mL, 14.98 mmol, 3.0 eq). The reaction solution was stirred at 100°C under N₂ for 5 h. Upon completion, the reaction solution was cooled to rt, quenched with water, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 1.2 g, yield 83%).

### Step F: 5-(3-(3-(2-((tert-Butyldimethylsilyl)oxy)ethyl)pyrrolidin-1-yl)phenyl)-4-methyl-2,4-dihydro-3H-1,2,4-triazol-3-one

To a solution of 5-(3-(3-(2-hydroxyethyl)pyrrolidin-1-yl)phenyl)-4-methyl-2,4-dihydro-3H-1,2,4-triazol-3-one (1.2 g, 4.16 mmol, 1.0 eq) and imidazole (0.57 g, 8.32 mmol, 2.0 eq) in DCM (15 mL) was added tert-butyldimethylsilyl chloride (0.9 g, 6.24 mmol, 1.5 eq) at 0°C. The reaction solution was stirred at rt under N₂ for 18 h. Upon completion, the reaction solution was quenched with water and extracted with DCM (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 1.1 g, yield 66%).

### Step G: 3-(3-(-3-(2-((tert-butyldimethylsilyl)oxy)ethyl)pyrrolidin-1-yl)phenyl)-4-methyl-5-oxo-4,5-dihydro-1H-1,2,4-triazol-1-yl)piperidine-2,6-dione

To a solution of 5-(3-(3-(2-((tert-butyldimethylsilyl)oxy)ethyl)pyrrolidin-1-yl)phenyl)-4-methyl-2,4-dihydro-3H-1,2,4-triazol-3-one (1.1 g, 2.73 mmol, 1.0 eq) in DMF (10 mL) was added portionwise 60 wt% sodium hydride (0.3 g, 8.20 mmol, 3.0 eq) at 0°C under N₂. The mixture was stirred at rt for 1 h. 3-Bromopiperidine-2,6-dione (1.1 g, 5.46 mmol, 2.0 eq) was then added to the above reaction solution. The mixture was stirred at 40°C under N₂ for 4 h. Upon completion, the reaction was quenched with saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by column chromatography to afford the title compound (white solid, 0.92 g, yield 66%).

### Step H: 3-(3-(-3-(3-(2-hydroxyethyl)pyrrolidin-1-yl)phenyl)-4-methyl-5-oxo-4,5-dihydro-1H-1,2,4-triazol-1-yl)piperidine-2,6-dione

At 0°C, to a solution of 3-(3-(3-(3-(2-((tert-butyldimethylsilyl)oxy)ethyl)pyrrolidin-1-yl)phenyl)-4-methyl-5-oxo-4,5-dihydro-1H-1,2,4-triazol-1-yl)piperidine-2,6-dione (0.92 g, 1.79 mmol, 1.0 eq) in THF (10 mL) was added 1N TBAF solution in THF (3.91 mL, 3.91 mmol, 2.0 eq). The reaction solution was stirred at rt under N₂ for 3 h. Upon completion, the reaction solution was cooled to rt, quenched with water, and extracted with EtOAc (×3). The combined organic phases were washed sequentially with water and saturated aqueous NaCl, dried over anhydrous Na₂SO₄, filtered, and concentrated under reduced pressure. The residue was purified by thin layer chromatography to afford the title compound (white solid, 450 mg, yield 63%).

### Step I: 2-(1-(3-(1-(2,6-dioxopiperidin-3-yl)-4-methyl-5-oxo-4,5-dihydro-1H-1,2,4-triazol-3-yl)phenyl)pyrrolidin-3-yl)acetaldehyde

At 0°C, to a solution of 3-(3-(-3-(3-(2-hydroxyethyl)pyrrolidin-1-yl)phenyl)-4-methyl-5-oxo-4,5-dihydro-1H-1,2,4-triazol-1-yl)piperidine-2,6-dione (400 mg, 0.78 mmol, 1.0 eq) in DCM (5 mL) was added Dess-Martin periodinane (495.4 mg, 1.17 mmol, 1.5 eq). The mixture was stirred at rt for 3 h. Upon completion, the reaction solution was filtered, and the filtrate was concentrated under pressure. The residue was purified by thin layer chromatography to afford the title compound (white solid, 140 mg, yield 45%).

### Step J: N-(3-carbamoyl-1-(4-(4-(2-(3-(1-(2,6-dioxopiperidin-3-yl)-4-methyl-5-oxo-4,5-dihydro-1H-1,2,4-triazol-3-yl)phenyl)pyrrolidin-3-yl)ethyl)piperazin-1-yl)phenyl ]-1H-pyrazol-4-yl)-6-(trifluoromethyl)picolinamide

To a solution of 2-(1-(3-(1-(2,6-dioxopiperidin-3-yl)-4-methyl-5-oxo-4,5-dihydro-1H-1,2,4-triazol-3-yl)phenyl)pyrrolidin-3-yl)acetaldehyde (90 mg, 0.23 mmol, 1.0 eq) and N-(3-amino-1-((1r,4r)-4-(piperazin-1-yl)cyclohexyl)-1H-pyrazol-4-yl)-6-(trifluoromethyl)picolinamide (93.6 mg, 0.20 mmol, 0.87 eq) in DMF (3 mL) was added AcOH (4.1 mg, 0.07 mmol, 0.3 eq) at rt. The mixture was stirred at rt for 0.5 h. Sodium triacetoxyborohydride (143.3 mg, 0.68 mmol, 3.0 eq) was then added to the above reaction solution. The mixture was stirred at rt for 1 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (white solid, 15.6 mg, yield 14%). **¹H NMR** (400 MHz, DMSO-*d*₆) δ 11.79 (s, 1H), 11.04 (s, 1H), 8.88 (s, 1H), 8.47-8.36 (m, 2H), 8.21 (d, *J =* 6.8 Hz, 1H), 7.95 (s, 1H), 7.78 (d, *J= 9.2* Hz, 2H), 7.70 (s, 1H), 7.30 (t, *J=* 7.8 Hz, 1H), 7.08 (d, *J* = 9.2 Hz, 2H), 6.84 (d, *J=* 7.8 Hz, 1H), 6.73-6.66 (m, 2H), 5.21 (dd, *J=* 12.8, 5.2 Hz, 1H), 3.51-3.44 (m, 2H), 3.30 (s, 3H), 3.22 (s, 5H), 2.95-2.84 (m, 2H), 2.64-2.53 (m, 5H), 2.48-2.39 (m, 3H), 2.34-2.27 (m, 1H), 2.18-2.06 (m, 2H), 1.71-1.60 (m, 3H). LCMS (ESI) : m/z 841 [M+H]⁺.

### Preparation Example 269. Synthesis of Compound A185

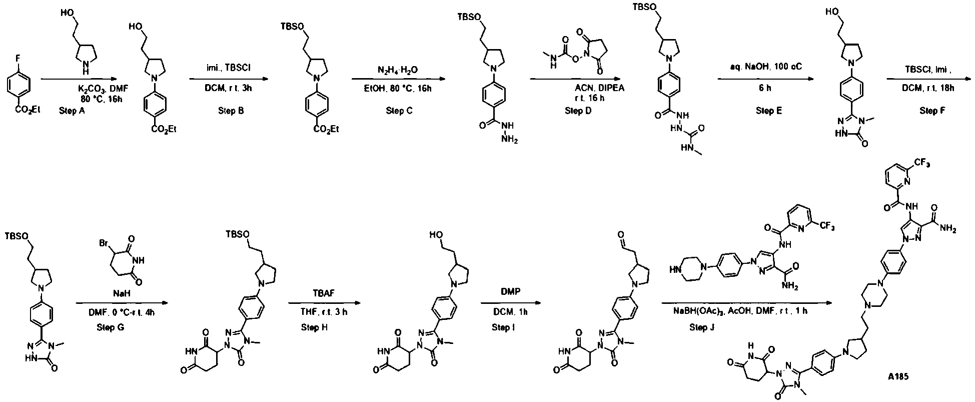

### Step A: Ethyl 4-(3-(2-hydroxyethyl)pyrrolidin-1-yl)benzoate

At 0°C, to a solution of ethyl 4-fluorobenzoate (6.0 g, 35.68 mmol, 1.0 eq) and 2-(pyrrolidin-3-yl)ethan-1-ol (6.2 g, 53.52 mmol, 1.5 eq) in DMF (2.0 mL) was added potassium carbonate (14.8 g, 107.04 mmol, 3.0 eq). The reaction solution was reacted at 80°C under N₂ for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (colorless oil, 2.3 g, yield 24%).

### Step B: Ethyl 4-(3-(2-((tert-butyldimethylsilyl)oxy)ethyl)pyrrolidin-1-yl)benzoate

To a solution of ethyl 4-(3-(2-hydroxyethyl)pyrrolidin-1-yl)benzoate (2.2 g, 8.35 mmol, 1.0 eq) and imidazole (1.1 g, 16.71 mmol, 2.0 eq) in DCM (15 mL) was added tert-butyldimethylsilyl chloride (1.9 g, 12.53 mmol, 1.5 eq) at 0°C. The reaction solution was reacted for 3 h under N₂ at rt. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by column chromatography to afford the title compound (colorless oil, 2.1 g, yield 67%).

### Step C: 4-(3-(2-((tert-Butyldimethylsilyl)oxy)ethyl)pyrrolidin-1-yl)benzohydrazide

To a solution of ethyl 4-(3-(2-((tert-butyldimethylsilyl)oxy)ethyl)pyrrolidin-1-yl)benzoate (2.1 g, 5.561 mmol, 1.0 eq) in ethanol (15 mL) was added 80% hydrazine hydrate (15 mL) at rt. The reaction solution was reacted at 80°C under N₂ for 16 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (colorless oil, 1.9 g, yield 94%).

### Step D: 2-(4-(3-(2-((tert-Butyldimethylsilyl)oxy)ethyl)pyrrolidin-1-yl)benzoyl)-N-methylhydrazine-1-carboxamide

To a solution of 4-(3-(2-((tert-butyldimethylsilyl)oxy)ethyl)pyrrolidin-1-yl)benzohydrazide (1.9 g, 5.23 mmol, 1.0 eq) and 2,5-dioxopyrrolidin-1-ylmethylcarbamate (1.1 g, 7.84 mmol, 1.5 eq) in acetonitrile (20 mL) was added DIPEA (2.0 g, 15.67 mmol, 3.0 eq) at rt. The reaction solution was reacted for 16 h under N₂ at rt. Upon completion, the reaction solution was poured into water and extracted with EtOAc (×3). The combined organic phases were washed with saturated aqueous NaCl, dried over anhydrous Na₂SO₄, filtered, and concentrated under reduced pressure to provide the title compound (off-white solid, 2.1 g, yield 96%).

### Step E: 5-(4-(3-(2-hydroxyethyl)pyrrolidin-1-yl)phenyl)-4-methyl-2,4-dihydro-3H-1,2,4-triazol-3-one

At rt, to a NaOH solution (25.0 mL, 14.98 mmol, 3.0 eq) was added 2-(4-(3-(2-((tert-butyldimethylsilyl)oxy)ethyl)pyrrolidin-1-yl)benzoyl)-N-methylhydrazine-1-carboxamide (2.1 g, 4.99 mmol, 1.0 eq). The reaction solution was reacted under N₂ at 100°C for 16 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 1.2 g, yield 83%).

### Step F: 5-(4-(3-(2-((tert-Butyldimethylsilyl)oxy)ethyl)pyrrolidin-1-yl)phenyl)-4-methyl-2,4-dihydro-3H-1,2,4-triazol-3-one

To a solution of 5-(4-(3-(2-hydroxyethyl)pyrrolidin-1-yl)phenyl)-4-methyl-2,4-dihydro-3H-1,2,4-triazol-3-one (1.2 g, 4.16 mmol, 1.0 eq) and imidazole (0.57 g, 8.32 mmol, 2.0 eq) in DCM (15 mL) was added tert-butyldimethylsilyl chloride (0.9 g, 6.24 mmol, 1.5 eq) at 0°C. The reaction solution was reacted for 18 h under N₂ at rt. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by column chromatography to afford the title compound (white solid, 1.1 g, yield 66%).

### Step G: 3-(3-(4-(3-(2-((tert-butyldimethylsilyl)oxy)ethyl)pyrrolidin-1-yl)phenyl)-4-methyl-5-oxo-4,5-dihydro-1H-1,2,4-triazol-1-yl)piperidine-2,6-dione

To a solution of 5-(4-(3-(2-((tert-butyldimethylsilyl)oxy)ethyl)pyrrolidin-1-yl)phenyl)-4-methyl-2,4-dihydro-3H-1,2,4-triazol-3-one (1.1 g, 2.73 mmol, 1.0 eq) in DMF (10 mL) at 0°C was added 60% sodium hydride (0.3 g, 8.20 mmol, 3.0 eq). The reaction solution was reacted at 0°C under N₂ for 1 h. 3-Bromopiperidine-2,6-dione (1.1 g, 5.46 mmol, 2.0 eq) was then added, and the reaction solution was reacted at 40°C under N₂ for 4 h. Upon completion, the reaction solution was poured into aqueous NH₄Cl and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 0.92 g, yield 66%).

### Step H: 3-(3-(4-(3-(2-hydroxyethyl)pyrrolidin-1-yl)phenyl)-4-methyl-5-oxo-4,5-dihydro-1H-1,2,4-triazol-1-yl)piperidine-2,6-dione

To a solution of 3-(3-(4-(3-(2-((tert-butyldimethylsilyl)oxy)ethyl)pyrrolidin-1-yl)phenyl)-4-methyl-5-oxo-4,5-dihydro-1H-1,2,4-triazol-1-yl)piperidine-2,6-dione (0.92 g, 1.79 mmol, 1.0 eq) in THF (10 mL) was added 1 M TBAF (3.91 mL, 3.91 mmol, 2.0 eq) at rt. The reaction solution was reacted for 3 h under N₂ at rt. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by thin layer chromatography to afford the title compound (white solid, 0.45 g, yield 63%).

### Step I: 2-(1-(4-(1-(2,6-dioxopiperidin-3-yl)-4-methyl-5-oxo-4,5-dihydro-1H-1,2,4-triazol-3-yl)phenyl)pyrrolidin-3-yl)acetaldehyde

To a solution of 3-(3-(4-(3-(2-hydroxyethyl)pyrrolidin-1-yl)phenyl)-4-methyl-5-oxo-4,5-dihydro-1H-1,2,4-triazol-1-yl)piperidine-2,6-dione (400 mg, 0.78 mmol, 1.0 eq) in DCM (5 mL) was added Dess-Martin periodinane (495 mg, 1.17 mmol, 1.5 eq) at rt. The mixture was reacted for 3 h under N₂ at rt. Upon completion, the mixture was filtered and concentrated under reduced pressure. The residue was purified by thin layer chromatography to afford the title compound (white solid, 140 mg, yield 45%). **Step J: N-(3-carbamoyl-1-(4-(4-(2-(4-(1-(2,6-dioxopiperidin-3-yl)-4-methyl-5-oxo-4,5-dihydro-1H-1,2,4-triazol-3-yl)phenyl)pyrrolidin-3-yl)ethyl)piperazin-1-yl)phenyl)-1H-pyrazol-4-yl)-6-(trifluoromethyl)picolinamide**

To a solution of 2-(1-(4-(1-(2,6-dioxopiperidin-3-yl)-4-methyl-5-oxo-4,5-dihydro-1H-1,2,4-triazol-3-yl)phenyl)pyrrolidin-3-yl)acetaldehyde (90 mg, 0.23 mmol, 1.0 eq) and N-(3-amino-1-(4-(piperazin-1-yl)phenyl)-1H-pyrazol-4-yl)-6-(trifluoromethyl)picolinamide (94 mg, 0.20 mmol, 0.87 eq) in DMF (30 mL) was added AcOH (4 mg, 0.07 mmol, 0.3 eq) at rt. The reaction solution was reacted at rt for 0.5 h. Sodium triacetoxyborohydride (143 mg, 0.68 mmol, 3.0 eq) was then added, and the reaction solution was stirred at rt for 1 h. Upon completion, the reaction solution was purified by HPLC to afford the title compound (white solid, 17.6 mg, yield 9%). **¹H NMR** (400 MHz, DMSO-*d*₆) δ 11.79 (s, 1H), 11.02 (s, 1H), 8.88 (s, 1H), 8.52-8.30 (m, 2H), 8.21 (d, *J=* 8.0 Hz, 1H), 7.95 (s, 1H), 7.80 (d, *J=* 8.8 Hz, 2H), 7.71 (s, 1H), 7.48 (d, *J* = 8.8 Hz, 2H), 7.09 (d, *J=* 8.8 Hz, 2H), 6.62 (d, *J =* 8.8 Hz, 2H), 5.17 (dd, *J=* 12.4, 5.2 Hz, 1H), 3.55-3.48 (m, 1H), 3.41-3.38 (m, 1H), 3.30 (s, 3H), 3.29-3.23 (m, 5H), 2.95 (t, *J=* 8.8 Hz, 1H), 2.89-2.82 (m, 1H), 2.68-2.58 (m, 5H), 2.45-2.40 (m, 2H), 2.33 (s, 2H), 2.14 (d, *J=* 28.0 Hz, 2H), 1.68 (s, 3H). LCMS (ESI) : m/z 841 [M+H]⁺.

### Preparation Example 270. Synthesis of Compound A186

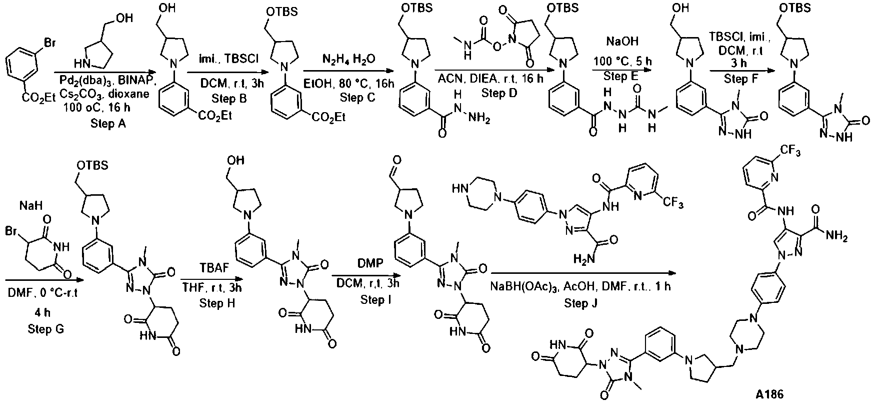

### Step A: Ethyl 3-(3-(hydroxymethyl)pyrrolidin-1-yl)benzoate

At rt, to a solution of ethyl 3-bromobenzoate (5.0 g, 21.83 mmol, 1.0 eq) and pyrrolidin-3-ylmethanol (2.65 g, 26.19 mmol, 1.2 eq) in 1,4-dioxane (10 mL) were added BINAP (1.36 g, 2.18 mmol, 0.1 eq), Pd₂(dba)₃ (1.0 g, 1.09 mmol, 0.05 eq) and cesium carbonate (21.34 g, 65.48 mmol, 3.0 eq). The reaction solution was stirred at 100°C under N₂ for 16 h. Upon completion, the reaction solution was cooled to rt, poured into water, and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 3.0 g, yield 55%).

### Step B: Ethyl 3-(3-((tert-butyldimethylsilyl)oxy)methyl)pyrrolidin-1-yl)benzoate

At 0°C, to a solution of ethyl 3-(3-(hydroxymethyl)pyrrolidin-1-yl)benzoate (3.0 g, 12.03 mmol, 1.0 eq) and imidazole (2.05 g, 30.08 mmol, 2.5 eq) in DCM (15 mL) was added tert-butyldimethylsilyl chloride (2.18 g, 14.44 mmol, 1.2 eq). The reaction solution was reacted for 3 h under N₂ at rt. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by column chromatography to afford the title compound (white solid, 3.0 g, yield 68%).

### Step C: 3-(3-(((tert-Butyldimethylsilyl)oxy)methyl)pyrrolidin-1-yl)benzohydrazide

To a solution of ethyl 3-(3-((tert-butyldimethylsilyl)oxy)methyl)pyrrolidin-1-yl)benzoate (3.0 g, 8.25 mmol, 1.0 eq) in ethanol (15 mL) was added 80% hydrazine hydrate (15 mL) at rt. The reaction solution was reacted at 80°C under N₂ for 16 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 2.5 g, yield 86%).

### Step D: 2-(3-(3-(((tert-Butyldimethylsilyl)oxy)methyl)pyrrolidin-1-yl)benzoyl)-N-methylhydrazine-1-carboxamide

To a solution of 3-(3-(((tert-butyldimethylsilyl)oxy)methyl)pyrrolidin-1-yl)benzohydrazide (2.5 g, 7.15 mmol, 1.0 eq) and 2,5-dioxopyrrolidin-1-ylmethylcarbamate (1.23 g, 7.15 mmol, 1.0 eq) in acetonitrile (25 mL) was added DIEA (1.84 g, 14.3 mmol, 2.0 eq) at rt. The reaction solution was reacted for 16 h under N₂ at rt. Upon completion, the reaction solution was poured into water and extracted with EtOAc (×3). The combined organic phases were washed with saturated aqueous NaCl, dried over anhydrous Na₂SO₄, filtered, and concentrated under reduced pressure to provide the title compound (off-white solid, 2.6 g, yield 89%).

### Step E: 5-(3-(3-(Hydroxymethyl)pyrrolidin-1-yl)phenyl)-4-methyl-2,4-dihydro-3H-1,2,4-triazol-3-one

To a NaOH solution (26.15 mL, 15.97 mmol, 2.5 eq) was added 2-(3-(3-(((tert-butyldimethylsilyl)oxy)methyl)pyrrolidin-1-yl)benzoyl)-N-methylhydrazine-1-carboxamide (2.6 g, 6.39 mmol, 1.0 eq) at rt. The reaction solution was reacted under N₂ at 100°C for 5 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 1.6 g, yield 91%).

### Step F: 5-(3-(((tert-Butyldimethylsilyl)oxy)methyl)pyrrolidin-1-yl)phenyl)-4-methyl-2,4-dihydro-3H-1,2,4-triazol-3-one

To a solution of 5-(3-(3-(hydroxymethyl)pyrrolidin-1-yl)phenyl)-4-methyl-2,4-dihydro-3H-1,2,4-triazol-3-one (1.6 g, 5.83 mmol, 1.0 eq) and imidazole (0.79 g, 11.67 mmol, 2.0 eq) in DCM (15 mL) was added tert-butyldimethylsilyl chloride (1.05 g, 6.99 mmol, 1.2 eq) at 0°C. The reaction solution was reacted for 3 h under N₂ at rt. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by column chromatography to afford the title compound (white solid, 1.5 g, yield 66%).

### Step G: 3-(3-(-3-(((tert-Butyldimethylsilyl)oxy)methyl)pyrrolidin-1-yl)phenyl)-4-methyl-5-oxo-4,5-dihydro-1H-1,2,4-triazol-1-yl)piperidine-2,6-dione

To a solution of 5-(3-(((tert-butyldimethylsilyl)oxy)methyl)pyrrolidin-1-yl)phenyl)-4-methyl-2,4-dihydro-3H-1,2,4-triazol-3-one (1.5 g, 3.86 mmol, 1.0 eq) in DMF (10 mL) at 0°C was added 60% sodium hydride (0.46 g, 11.58 mmol, 3.0 eq). The reaction solution was reacted at 0°C under N₂ for 0.5 h. 3-Bromopiperidine-2,6-dione (1.48 g, 7.72 mmol, 2.0 eq) was then added, and the reaction solution was reacted at rt under N₂ for 4 h. Upon completion, the reaction solution was poured into aqueous NH₄Cl and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 0.8 g, yield 41%).

### Step H: 3-(3-(3-(hydroxymethyl)pyrrolidin-1-yl)phenyl)-4-methyl-5-oxo-4,5-dihydro-1H-1,2,4-triazol-1-yl)piperidine-2,6-dione

To a solution of 3-(3-(-3-(((tert-butyldimethylsilyl)oxy)methyl)pyrrolidin-1-yl)phenyl)-4-methyl-5-oxo-4,5-dihydro-1H-1,2,4-triazol-1-yl)piperidine-2,6-dione (800 mg, 1.60 mmol, 1.0 eq) in THF (5 mL) was added 1 M tetrabutylammonium fluoride (3.2 mL, 3.2 mmol, 2.0 eq) at rt. The reaction solution was reacted for 3 h under N₂ at rt. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by thin layer chromatography to afford the title compound (white solid, 0.26 g, yield 42%).

### Step I: 1-(3-(1-(2,6-dioxopiperidin-3-yl)-4-methyl-5-oxo-4,5-dihydro-1H-1,2,4-triazol-3-yl)phenyl)pyrrolidine-3-carbaldehyde

To a solution of 3-(3-(3-(hydroxymethyl)pyrrolidin-1-yl)phenyl)-4-methyl-5-oxo-4,5-dihydro-1H-1,2,4-triazol-1-yl)piperidine-2,6-dione (100 mg, 0.26 mmol, 1.0 eq) in DCM (4 mL) at rt was added Dess-Martin periodinane (220 mg, 0.52 mmol, 2.0 eq). The mixture was reacted for 3 h under N₂ at rt. Upon completion, the reaction solution was filtered and concentrated under reduced pressure. The residue was purified by thin layer chromatography to afford the title compound (white solid, 60 mg, yield 60%). **Step J: N-(3-carbamoyl-1-(4-((1-(3-(1-(2,6-dioxopiperidin-3-yl)-4-methyl-5-oxo-4,5-dihydro-1H-1,2,4-triazol-3-yl)phenyl)pyrrolidin-3-yl)methyl)piperazin-1-yl)phenyl)-1H-pyrazol-4-yl)-6-(trifluoromethyl)picolinamide**

To a solution of 1-(3-(1-(2,6-dioxopiperidin-3-yl)-4-methyl-5-oxo-4,5-dihydro-1H-1,2,4-triazol-3-yl)phenyl)pyrrolidine-3-carbaldehyde (60 mg, 0.16 mmol, 1.0 eq) and N-(3-amino-1-(4-(piperazin-1-yl)phenyl)-1H-pyrazol-4-yl)-6-(trifluoromethyl)picolinamide (58 mg, 0.12 mmol, 0.8 eq) in DMF (2 mL) was added AcOH (3 mg, 0.05 mmol, 0.3 eq) at rt. The reaction solution was reacted at rt for 0.5 h. Sodium triacetoxyborohydride (66 mg, 0.31 mmol, 2.0 eq) was then added, and the reaction solution was stirred at rt for 3 h. Upon completion, the reaction solution was purified by HPLC to afford the title compound (white solid, 6.1 mg, yield 5%). ¹HNMR (400 MHz, DMSO-*d*₆) δ 11.79 (s, 1H), 11.04 (s, 1H), 8.89 (s, 1H), 8.48-8.37 (m, 2H), 8.21 (d, *J* = 7.6 Hz, 1H), 7.95 (s, 1H), 7.80 (d, *J=* 8.8 Hz, 2H), 7.71 (s, 1H), 7.31 (t, *J* = 8.0 Hz, 1H), 7.10 (d, *J= 9.2* Hz, 2H), 6.86 (d, *J=* 7.6 Hz, 1H), 6.71 (d, *J =* 8.0 Hz, 2H), 5.21 (dd, *J=* 12.8, 5.3 Hz, 1H), 3.41 (s, 3H), 3.31 (s, 3H), 3.26 (d, *J =* 10.8 Hz, 5H), 3.08-3.02 (m, 1H), 2.95-2.76 (m, 2H), 2.62 (d, *J* = 17.2 Hz, 5H), 2.49-2.41 (m, 2H), 2.18-2.07 (m, 2H), 1.84-1.71 (m, 1H). LCMS (ESI) : m/z 827 [M+H]⁺.

### Preparation Example 271. Synthesis of Compound A187

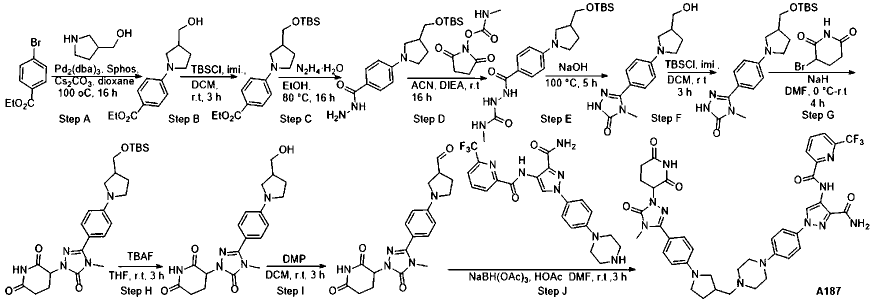

### Step A: Ethyl 4-(3-(hydroxymethyl)pyrrolidin-1-yl)benzoate

To a solution of ethyl 4-bromobenzoate (5.0 g, 21.83 mmol, 1.0 eq) and pyrrolidin-3-ylmethanol (2.65 g, 26.19 mmol, 1.2 eq) in 1,4-dioxane (50 mL) at rt were added cesium carbonate (21.34 g, 65.48 mmol, 3.0 eq), BINAP (1.36 g, 2.18 mmol, 0.1 eq), and Pd₂(dba)₃ (1.0 g, 1.09 mmol, 0.05 eq). The mixture was stirred at 100°C under N₂ for 16 h. Upon completion, the mixture was cooled to rt, diluted in water, extracted with EtOAc, washed with saturated aqueous NaCl, dried over anhydrous Na₂SO₄, and concentrated under reduced pressure. The residue was purified by column chromatography to afford the title compound (white solid, 3.0 g, yield 55%).

### Step B: Ethyl 4-(3-(((tert-Butyldimethylsilyl)oxy)methyl)pyrrolidin-1-yl)benzoate

At 0°C, to a solution of ethyl 4-(3-(hydroxymethyl)pyrrolidin-1-yl)benzoate (3.0 g, 12.03 mmol, 1.0 eq) and imidazole (2.05 g, 30.08 mmol, 2.5 eq) in DCM (15 mL) was added tert-butyldimethylsilyl chloride (2.18 g, 14.44 mmol, 1.2 eq). The reaction solution was stirred at rt under N₂ for 3 h. Upon completion, the reaction solution was quenched with water and extracted with DCM (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 3.0 g, yield 68%).

### Step C: 4-(3-(((tert-Butyldimethylsilyl)oxy)methyl)pyrrolidin-1-yl)benzohydrazide

To a solution of ethyl 4-(3-(((tert-butyldimethylsilyl)oxy)methyl)pyrrolidin-1-yl)benzoate (3.0 g, 8.25 mmol, 1.0 eq) in ethanol (15 mL) was added 80% hydrazine hydrate (15 mL) at 0°C. The reaction solution was stirred at 80°C under N₂ for 16 h. Upon completion, the reaction solution was cooled to rt, quenched with water, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 2.5 g, yield 86%).

### Step D: 2-(4-(3-(((tert-Butyldimethylsilyl)oxy)methyl)pyrrolidin-1-yl)benzoyl)-N-methylhydrazine-1-carboxamide

To a solution of 4-(3-(((tert-butyldimethylsilyl)oxy)methyl)pyrrolidin-1-yl)benzohydrazide (2.5 g, 7.15 mmol, 1.0 eq) and 2,5-dioxopyrrolidin-1-ylmethylcarbamate (1.23 g, 7.15 mmol, 1.0 eq) in acetonitrile (25 mL) was added DIEA (1.84 g, 14.3 mmol, 2.0 eq) at rt. The mixture was stirred at rt under N₂ for 16 h, Upon completion, water was added, and the mixture was extracted with EtOAc, washed with saturated aqueous NaCl, dried over anhydrous Na₂SO₄, filtered, and concentrated under reduced pressure to afford the title compound (off-white solid, 2.6 g, crude), which was used directly without purification.

### Step E: 5-(4-(3-(Hydroxymethyl)pyrrolidin-1-yl)phenyl)-4-methyl-2,4-dihydro-3H-1,2,4-triazol-3-one

At 0°C, to a solution of 2-(4-(3-(((tert-butyldimethylsilyl)oxy)methyl)pyrrolidin-1-yl)benzoyl)-N-methylhydrazine-1-carboxamide (2.6 g, 6.39 mmol, 1.0 eq) was added aqueous NaOH (26.15 mL, 15.97 mmol, 2.5 eq). The mixture was stirred at 100°C under N₂ for 5 h. Upon completion, the mixture was cooled to rt, quenched with water, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 1.6 g, yield 91%).

### Step F: 5-(4-(3-(((tert-Butyldimethylsilyl)oxy)methyl)pyrrolidin-1-yl)phenyl)-4-methyl-2,4-dihydro-3H-1,2,4-triazol-3-one

To a solution of 5-(4-(3-(hydroxymethyl)pyrrolidin-1-yl)phenyl)-4-methyl-2,4-dihydro-3H-1,2,4-triazol-3-one (1.6 g, 5.83 mmol, 1.0 eq) and imidazole (0.79 g, 11.67 mmol, 2.0 eq) in DCM (15 mL) was added tert-butyldimethylsilyl chloride (1.05 g, 6.99 mmol, 1.2 eq) at 0°C. The reaction solution was stirred at rt under N₂ for 3 h. Upon completion, the reaction solution was quenched with water and extracted with DCM (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 1.5 g, yield 66%).

### Step G: 3-(3-(4-(3-(((tert-Butyldimethylsilyl)oxy)methyl)pyrrolidin-1-yl)phenyl)-4-methyl-5-oxo-4,5-dihydro-1H-1,2,4-triazol-1-yl)piperidine-2,6-dione

To a solution of 5-(4-(3-(((tert-butyldimethylsilyl)oxy)methyl)pyrrolidin-1-yl)phenyl)-4-methyl-2,4-dihydro-3H-1,2,4-triazol-3-one (1.5 g, 3.86 mmol, 1.0 eq) in DMF (20 mL) was added portionwise 60 wt% sodium hydride (0.46 g, 11.58 mmol, 3.0 eq) at 0°C under N₂. The mixture was stirred at rt for 0.5 h. 3-Bromopiperidine-2,6-dione (1.48 g, 7.72 mmol, 2.0 eq) was then added to the above reaction solution. The mixture was stirred at rt under N₂ for 4 h, Upon completion, the reaction was quenched with saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by column chromatography to afford the title compound (white solid, 0.8 g, yield 41%).

### Step H: 3-(3-(4-(3-(hydroxymethyl)pyrrolidin-1-yl)phenyl)-4-methyl-5-oxo-4,5-dihydro-1H-1,2,4-triazol-1-yl)piperidine-2,6-dione

At 0°C, to a solution of 3-(3-(4-(3-((tert-butyldimethylsilyl)oxy)methyl)pyrrolidin-1-yl)phenyl)-4-methyl-5-oxo-4,5-dihydro-1H-1,2,4-triazol-1-yl)piperidine-2,6-dione (800 mg, 1.60 mmol, 1.0 eq) in THF (5 mL) was added 1N TBAF solution in THF (3.2 mL, 3.2 mmol, 2.0 eq). The reaction solution was stirred at rt under N₂ for 3 h. Upon completion, the mixture was cooled to rt, quenched with water, and extracted with EtOAc (×3). The combined organic phases were washed sequentially with water and saturated aqueous NaCl, dried over anhydrous Na₂SO₄, filtered, and concentrated under reduced pressure. The residue was purified by thin layer chromatography to afford the title compound (white solid, 260 mg, yield 42%).

### Step I: 1-(4-(1-(2,6-dioxopiperidin-3-yl)-4-methyl-5-oxo-4,5-dihydro-1H-1,2,4-triazol-3-yl)phenyl)pyrrolidine-3-carbaldehyde

To a solution of 3-(3-(4-(3-(hydroxymethyl)pyrrolidin-1-yl)phenyl)-4-methyl-5-oxo-4,5-dihydro-1H-1,2,4-triazol-1-yl)piperidine-2,6-dione (100 mg, 0.26 mmol, 1.0 eq) in DCM (4 mL) was added Dess-Martin periodinane (220 mg, 0.52 mmol, 2.0 eq) at 0°C. The mixture was stirred at rt for 3 h. Upon completion, the mixture was filtered, and the filtrate was concentrated under pressure. The residue was purified by thin layer chromatography to afford the title compound (white solid, 60 mg, yield 60%).

### Step J: N-(3-Carbamoyl-1-(4-((1-(4-(1-(2,6-dioxopiperidin-3-yl)-4-methyl-5-oxo-4,5-dihydro-1H-1,2,4-triazol-3-yl)phenyl)pyrrolidin-3-yl)methyl)piperazin-1-yl)phenyl)-1H-pyrazol-4-yl)-6-(trifluoromethyl)picolinamide

To a solution of 1-(4-(1-(2,6-dioxopiperidin-3-yl)-4-methyl-5-oxo-4,5-dihydro-1H-1,2,4-triazol-3-yl)phenyl)pyrrolidine-3-carbaldehyde (60 mg, 0.16 mmol, 1.0 eq) and N-(3-amino-1-((1r,4r)-4-(piperazin-1-yl)cyclohexyl)-1H-pyrazol-4-yl)-6-(trifluoromethyl)picolinamide (57.5 mg, 0.12 mmol, 0.8 eq) in DMF (2 mL) was added AcOH (2.81 mg, 0.05 mmol, 0.3 eq) at rt. The mixture was stirred at rt for 0.5 h. Sodium triacetoxyborohydride (66 mg, 0.31 mmol, 2.0 eq) was then added to the above reaction solution. The mixture was stirred at rt for 1 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (white solid, 6.1 mg, yield 5%). **¹H NMR** (400 MHz, DMSO-*d*₆) δ 11.79 (s, 1H), 11.02 (s, 1H), 8.88 (s, 1H), 8.46-8.38 (m, 2H), 8.21 (d, *J* = 7.2 Hz, 1H), 7.95 (s, 1H), 7.79 (d, *J* = 8.8 Hz, 2H), 7.70 (s, 1H), 7.49 (d, *J =* 8.4 Hz, 2H), 7.09 (d, *J* = 9.2 Hz, 2H), 6.63 (d, *J* = 8.8 Hz, 2H), 5.17 (dd, *J =* 12.8, 5.6 Hz, 1H), 3.26-3.24 (m, 10H), 3.08-3.05 (m, 1H), 2.91-2.84 (m, 2H), 2.67-2.56 (m, 4H), 2.44-2.42 (m, 2H), 2.33 (s, 1H), 2.12-1.99 (m, 3H), 1.79-1.77 (m, 1H), 1.46 (s, 1H), 0.87-0.84 (m, 1H). LCMS (ESI): m/z 827 [M+H]⁺.

### Preparation Example 272. Synthesis of Compound A188

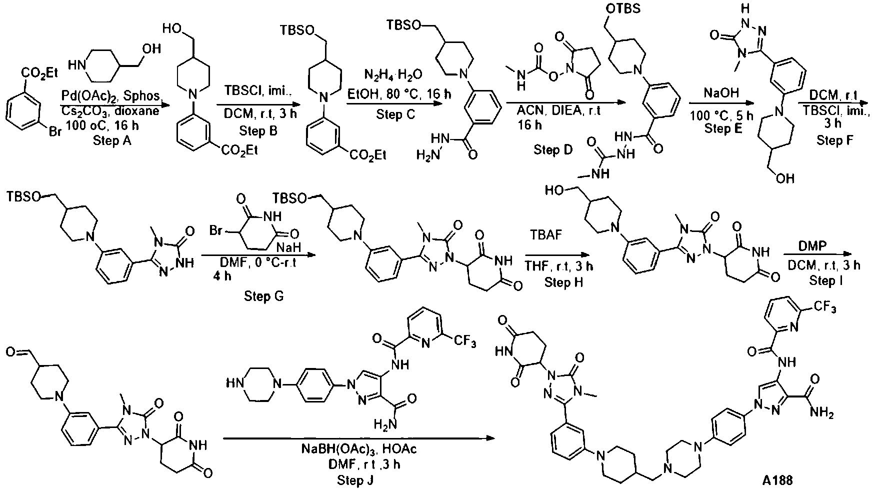

### Step A: Ethyl 3-(4-(hydroxymethyl)piperidin-1-yl)benzoate

To a solution of ethyl 4-bromobenzoate (3.0 g, 13.09 mmol, 1.0 eq) and piperidin-4-ylmethanol (2.3 g, 19.65 mmol, 1.5 eq) in 1,4-dioxane (50 mL) at rt were added cesium carbonate (12.8 g, 39.29 mmol, 3.0 eq), S-Phos (1.1 g, 2.62 mmol, 0.2 eq), and Pd(OAc)₂(0.3 g, 1.31 mmol, 0.1 eq). The mixture was stirred at 100°C under N₂ for 16 h. Upon completion, the mixture was cooled to rt, diluted in water, extracted with EtOAc, washed with saturated aqueous NaCl, dried over anhydrous Na₂SO₄, and concentrated under reduced pressure. The residue was purified by column chromatography to afford the title compound (white solid, 3.0 g, yield 43%).

### Step B: Ethyl 3-(4-(((tert-Butyldimethylsilyl)oxy)methyl)piperidin-1-yl)benzoate

At 0°C, to a solution of ethyl 3-(4-(hydroxymethyl)piperidin-1-yl)benzoate (1.5 g, 5.70 mmol, 1.0 eq) and imidazole (0.8 g, 11.39 mmol, 2.0 eq) in DCM (15 mL) was added tert-butyldimethylsilyl chloride (1.3 g, 8.55 mmol, 2.0 eq). The reaction solution was stirred at rt under N₂ for 3 h. Upon completion, the reaction solution was quenched with water and extracted with DCM (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 1.7 g, yield 77%).

### Step C: 3-(4-(((tert-Butyldimethylsilyl)oxy)methyl)piperidin-1-yl)benzohydrazide

At 0°C, to a solution of ethyl 3-(4-(((tert-butyldimethylsilyl)oxy)methyl)piperidin-1-yl)benzoate (1.7 g, 4.37 mmol, 1.0 eq) in ethanol (15 mL) was added 80% hydrazine hydrate (15 mL). The reaction solution was stirred at 80°C under N₂ for 16 h. Upon completion, the reaction solution was cooled to rt, quenched with water, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 1.1 g, yield 68%).

### Step D: 2-(3-(4-(((tert-Butyldimethylsilyl)oxy)methyl)piperidin-1-yl)benzoyl)-N-methylhydrazine-1-carboxamide

To a solution of 3-(4-(((tert-butyldimethylsilyl)oxy)methyl)piperidin-1-yl)benzohydrazide (1.1 g, 2.89 mmol, 1.0 eq) and 2,5-dioxopyrrolidin-1-ylmethylcarbamate (0.8 g, 4.33 mmol, 1.5 eq) in acetonitrile (25 mL) was added DIEA (1.1 g, 8.66 mmol, 3.0 eq) at rt. The mixture was stirred at rt under N₂ for 16 h, Upon completion, water was added, and the mixture was extracted with EtOAc, washed with saturated aqueous NaCl, dried over anhydrous Na₂SO₄, filtered, and concentrated under reduced pressure to afford the title compound (off-white solid, 1.1 g, crude), which was used directly without purification.

### Step E: 5-(3-(4-(Hydroxymethyl)piperidin-1-yl)phenyl)-4-methyl-2,4-dihydro-3H-1,2,4-triazol-3-one

At 0°C, to 2-(3-(4-(((tert-butyldimethylsilyl)oxy)methyl)piperidin-1-yl)benzoyl)-N-methylhydrazine-1-carboxamide (1.1 g, 2.62 mmol, 1.0 eq) was added aqueous NaOH (13.1 mL, 7.85 mmol, 3.0 eq). The reaction solution was stirred at 100°C under N₂ for 5 h. Upon completion, the reaction solution was cooled to rt, quenched with water, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 0.6 g, yield 80%).

### Step F: 5-(3-(4-(((tert-Butyldimethylsilyl)oxy)methyl)piperidin-1-yl)phenyl)-4-methyl-2,4-dihydro-3H-1,2,4-triazol-3-one

To a solution of 5-(3-(4-(hydroxymethyl)piperidin-1-yl)phenyl)-4-methyl-2,4-dihydro-3H-1,2,4-triazol-3-one (0.6 g, 2.08 mmol, 1.0 eq) and imidazole (0.29 g, 4.16 mmol, 2.0 eq) in DCM (15 mL) was added tert-butyldimethylsilyl chloride (0.5 g, 3.12 mmol, 1.5 eq) at 0°C. The reaction solution was stirred at rt under N₂ for 3 h. Upon completion, the reaction solution was quenched with water and extracted with DCM (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 0.6 g, yield 72%).

### Step G: 3-(3-(4-(((tert-Butyldimethylsilyl)oxy)methyl)piperidin-1-yl)phenyl)-4-methyl-5-oxo-4,5-dihydro-1H-1,2,4-triazol-1-yl)piperidine-2,6-dione

To a solution of 5-(3-(4-(((tert-butyldimethylsilyl)oxy)methyl)piperidin-1-yl)phenyl)-4-methyl-2,4-dihydro-3H-1,2,4-triazol-3-one (0.6 g, 1.37 mmol, 1.0 eq) in DMF (10 mL) was added portionwise 60 wt% sodium hydride (150 mg, 4.10 mmol, 3.0 eq) at 0°C under N₂. The mixture was stirred at rt for 1 h. 3-Bromopiperidine-2,6-dione (1.1 g, 5.46 mmol, 4.0 eq) was then added to the above reaction solution. The mixture was stirred at rt under N₂ for 4 h, Upon completion, the reaction was quenched with saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by column chromatography to afford the title compound (white solid, 530 mg, yield 76%).

### Step H: 3-(3-(4-(Hydroxymethyl)piperidin-1-yl)phenyl)-4-methyl-5-oxo-4,5-dihydro-1H-1,2,4-triazol-1-yl)piperidine-2,6-dione

At 0°C, to a solution of 3-(3-(4-(((tert-butyldimethylsilyl)oxy)methyl)piperidin-1-yl)phenyl)-4-methyl-5-oxo-4,5-dihydro-1H-1,2,4-triazol-1-yl)piperidine-2,6-dione (480 mg, 0.93 mmol, 1.0 eq) in THF (5 mL) was added 1N TBAF solution in THF (1.87 mL, 1.87 mmol, 2.0 eq). The mixture was stirred at rt under N₂ for 3 h. Upon completion, the mixture was cooled to rt, quenched with water, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 240 mg, yield 64%).

### Step I: 1-(3-(1-(2,6-dioxopiperidin-3-yl)-4-methyl-5-oxo-4,5-dihydro-1H-1,2,4-triazol-3-yl)phenyl)piperidine-4-carbaldehyde

To a solution of 3-(3-(4-(hydroxymethyl)piperidin-1-yl)phenyl)-4-methyl-5-oxo-4,5-dihydro-1H-1,2,4-triazol-1-yl)piperidine-2,6-dione (120 mg, 0.30 mmol, 1.0 eq) in DCM (4 mL) was added Dess-Martin periodinane (178.38 mg, 0.42 mmol, 1.5 eq) at 0°C. The mixture was stirred at rt for 3 h. Upon completion, the reaction solution was filtered, and the filtrate was concentrated under pressure. The residue was purified by thin layer chromatography to afford the title compound (white solid, 64 mg, yield 54%). **Step J: N-(3-carbamoyl-1-(4-((1-(3-(1-(2,6-dioxopiperidin-3-yl)-4-methyl-5-oxo-4,5-dihydro-1H-1,2,4-triazol-3-yl)phenyl)piperidin-4-yl)methyl)piperazin-1-yl)phenyl)-1H-pyrazol-4-yl)-6-(trifluoromethyl)picolinamide**

To a solution of 1-(3-(1-(2,6-dioxopiperidin-3-yl)-4-methyl-5-oxo-4,5-dihydro-1H-1,2,4-triazol-3-yl)phenyl)piperidine-4-carbaldehyde (60 mg, 0.15 mmol, 1.0 eq) and N-(3-amino-1-((1r,4r)-4-(piperazin-1-yl)cyclohexyl)-1H-pyrazol-4-yl)-6-(trifluoromethyl)picolinamide (55.5 mg, 0.12 mmol, 0.8 eq) in DMF (2 mL) was added AcOH (2.7 mg, 0.05 mmol, 0.3 eq) at rt. The mixture was stirred at rt for 0.5 h. Sodium triacetoxyborohydride (95.6 mg, 0.45 mmol, 3.0 eq) was then added to the above reaction solution. The mixture was stirred at rt for 3 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (white solid, 16.2 mg, yield 13%). **¹H NMR** (400 MHz, DMSO-*d*₆) δ 11.79 (s, 1H), 11.04 (s, 1H), 8.88 (s, 1H), 8.52-8.33 (m, 2H), 8.21 (d, *J* = 8.0 Hz, 1H), 8.19-8.18 (m, 1H), 7.95 (s, 1H), 7.79 (d, *J=* 9.2 Hz, 2H), 7.71 (s, 1H), 7.35 (t, *J=* 8.0 Hz, 1H), 7.10 (dd, *J=* 17.2, 8.4 Hz, 4H), 7.01 (d, *J= 7.6* Hz, 1H), 5.22 (dd, *J=* 12.8, 5.6 Hz, 1H), 3.77 (d, *J* = 12.4 Hz, 2H), 3.34 (s, 4H), 3.22 (s, 4H), 2.94-2.83 (m, 1H), 2.74 (t, *J =* 11.2 Hz, 2H), 2.65-2.58 (m, 1H), 2.52 (s, 3H), 2.49-2.46 (m, 1H), 2.23 (d, *J =* 6.8 Hz, 2H), 2.15-2.07 (m, 1H), 1.84-1.75 (m, 3H), 1.27-1.19 (m, 2H). LCMS (ESI): m/z 841 [M+H]⁺.

### Preparation Example 273. Synthesis of Compound A189

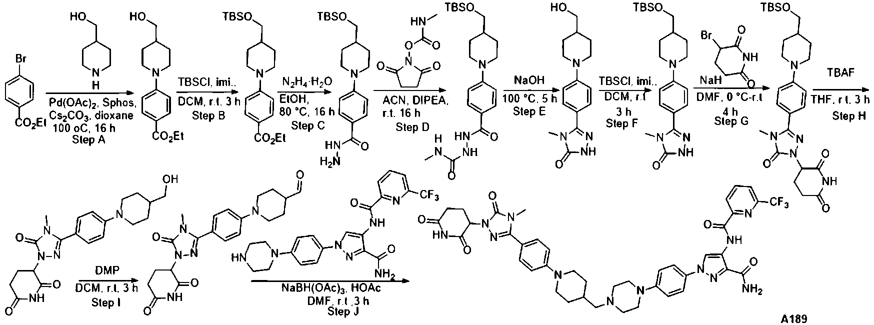

### Step A: Ethyl 4-(4-(hydroxymethyl)piperidin-1-yl)benzoate

To a solution of ethyl 4-bromobenzoate (6.0 g, 26.19 mmol, 1.0 eq) and piperidin-4-ylmethanol (4.5 g, 39.29 mmol, 1.5 eq) in 1,4-dioxane (50 mL) at rt were added cesium carbonate (25.6 g, 78.58 mmol, 3.0 eq), S-Phos (2.15 g, 5.24 mmol, 0.2 eq), and Pd(OAc)₂ (0.6 g, 2.62 mmol, 0.1 eq). The mixture was stirred at 100°C under N₂ for 16 h. Upon completion, the mixture was cooled to rt, diluted in water, extracted with EtOAc, washed with saturated aqueous NaCl, dried over anhydrous Na₂SO₄, and concentrated under reduced pressure. The residue was purified by column chromatography to afford the title compound (white solid, 3.0 g, yield 43%).

### Step B: Ethyl 4-(4-(((tert-Butyldimethylsilyl)oxy)methyl)piperidin-1-yl)benzoate

**At 0°C, to a solution of ethyl 4-(4-(hydroxymethyl)piperidin-1-yl)benzoate (3.0 g, 11.39 mmol, 1.0** eq) and imidazole (1.6 g, 22.78 mmol, 2.0 eq) in DCM (15 mL) was added tert-butyldimethylsilyl chloride (2.6 g, 17.09 mmol, 2.0 eq). The reaction solution was stirred at rt under N₂ for 3 h. Upon completion, the reaction solution was quenched with water and extracted with DCM (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 3.3 g, yield 77%).

### Step C: 4-(4-(((tert-Butyldimethylsilyl)oxy)methyl)piperidin-1-yl)benzohydrazide

At 0°C, to a solution of ethyl 4-(4-(((tert-butyldimethylsilyl)oxy)methyl)piperidin-1-yl)benzoate (3.3 g, 8.74 mmol, 1.0 eq) in ethanol (15 mL) was added 80% hydrazine hydrate (15 mL). The reaction solution was stirred at 80°C under N₂ for 16 h. Upon completion, the reaction solution was cooled to rt, quenched with water, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 2.1 g, yield 66%).

### Step D: 2-(4-(4-(((tert-Butyldimethylsilyl)oxy)methyl)piperidin-1-yl)benzoyl)-N-methylhydrazine-1-carboxamide

To a solution of 4-(4-(((tert-butyldimethylsilyl)oxy)methyl)piperidin-1-yl)benzohydrazide (2.1 g, 5.78 mmol, 1.0 eq) and 2,5-dioxopyrrolidin-1-ylmethylcarbamate (1.5 g, 8.66 mmol, 1.5 eq) in acetonitrile (25 mL) was added DIPEA (2.2 g, 17.33 mmol, 3.0 eq) at rt. The mixture was stirred at rt under a N₂ for 16 h, Upon completion, water was added, and the mixture was extracted with EtOAc, washed with saturated aqueous NaCl, dried over anhydrous Na₂SO₄, filtered, and concentrated under reduced pressure to afford the title compound (off-white solid, 2.2 g, crude), which was used directly without purification.

### Step E: 5-(4-(4-(Hydroxymethyl)piperidin-1-yl)phenyl)-4-methyl-2,4-dihydro-3H-1,2,4-triazol-3-one

At 0°C, to 2-(4-(4-(((tert-butyldimethylsilyl)oxy)methyl)piperidin-1-yl)benzoyl)-N-methylhydrazine-1-carboxamide (2.2 g, 5.23 mmol, 1.0 eq) was added aqueous NaOH (26.15 mL, 15.69 mmol, 3.0 eq). The mixture was stirred at 100°C under N₂ for 5 h. Upon completion, the reaction solution was cooled to rt, quenched with water, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 1.2 g, yield 80%).

### Step F: 5-(4-(4-(((tert-Butyldimethylsilyl)oxy)methyl)piperidin-1-yl)phenyl)-4-methyl-2,4-dihydro-3H-1,2,4-triazol-3-one

To a solution of 5-(4-(4-(hydroxymethyl)piperidin-1-yl)phenyl)-4-methyl-2,4-dihydro-3H-1,2,4-triazol-3-one (1.2 g, 4.16 mmol, 1.0 eq) and imidazole (0.57 g, 8.32 mmol, 2.0 eq) in DCM (15 mL) was added tert-butyldimethylsilyl chloride (0.9 g, 6.24 mmol, 1.5 eq) at 0°C. The reaction solution was stirred at rt under N₂ for 3 h. Upon completion, the reaction solution was quenched with water and extracted with DCM (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 1.2 g, yield 72%).

### Step G: 3-(3-(4-(((tert-Butyldimethylsilyl)oxy)methyl)piperidin-1-yl)phenyl)-4-methyl-5-oxo-4,5-dihydro-1H-1,2,4-triazol-1-yl)piperidine-2,6-dione

To a solution of 5-(4-(4-(((tert-butyldimethylsilyl)oxy)methyl)piperidin-1-yl)phenyl)-4-methyl-2,4-dihydro-3H-1,2,4-triazol-3-one (1.1 g, 2.73 mmol, 1.0 eq) in DMF (10 mL) at 0°C, 60 wt% sodium hydride (0.3 g, 8.20 mmol, 3.0 eq) was added portionwise under N₂. The mixture was stirred at rt for 0.5 h. 3-Bromopiperidine-2,6-dione (1.1 g, 5.46 mmol, 2.0 eq) was then added to the above reaction solution. The mixture was stirred at rt under N₂ for 4 h, Upon completion, the reaction was quenched with saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by column chromatography to afford the title compound (white solid, 530 mg, yield 38%).

### Step H: 3-(3-(4-(4-(Hydroxymethyl)piperidin-1-yl)phenyl)-4-methyl-5-oxo-4,5-dihydro-1H-1,2,4-triazol-1-yl)piperidine-2,6-dione

At 0°C, to a solution of 3-(3-(4-(((tert-butyldimethylsilyl)oxy)methyl)piperidin-1-yl)phenyl)-4-methyl-5-oxo-4,5-dihydro-1H-1,2,4-triazol-1-yl)piperidine-2,6-dione (480 mg, 0.93 mmol, 1.0 eq) in THF (5 mL) was added 1N tetrabutylammonium fluoride solution in THF (1.87 mL, 1.87 mmol, 2.0 eq). The reaction solution was stirred at rt under N₂ for 3 h. Upon completion, the reaction solution was cooled to rt, quenched with water, and extracted with EtOAc (×3). The combined organic phases were washed sequentially with water and saturated aqueous NaCl, dried over anhydrous Na₂SO₄, filtered, and concentrated under reduced pressure. The residue was purified by thin layer chromatography to afford the title compound (white solid, 240 mg, yield 64%).

### Step I: 1-(4-(1-(2,6-dioxopiperidin-3-yl)-4-methyl-5-oxo-4,5-dihydro-1H-1,2,4-triazol-3-yl)phenyl)piperidine-4-carbaldehyde

To a solution of 3-(3-(4-(4-(hydroxymethyl)piperidin-1-yl)phenyl)-4-methyl-5-oxo-4,5-dihydro-1H-1,2,4-triazol-1-yl)piperidine-2,6-dione (120 mg, 0.30 mmol, 1.0 eq) in DCM (4 mL) was added Dess-Martin periodinane (178.38 mg, 0.42 mmol, 1.5 eq) at 0°C. The mixture was stirred at rt for 3 h. Upon completion, the reaction solution was filtered, and the filtrate was concentrated under pressure. The residue was purified by thin layer chromatography to afford the title compound (white solid, 64 mg, yield 54%).

### Step J: Tert-butyl N-(3-carbamoyl-1-(4-((1-(4-(1-(2,6-dioxopiperidin-3-yl)-4-methyl-5-oxo-4,5-dihydro-1H-1,2,4-triazol-3-yl)phenyl)piperidin-4-yl)methyl)piperazin-1-yl)phenyl)-1H-pyrazol-4-yl)-6-(trifluoromethyl)picolinamide

To a solution of 1-(4-(1-(2,6-dioxopiperidin-3-yl)-4-methyl-5-oxo-4,5-dihydro-1H-1,2,4-triazol-3-yl)phenyl)piperidine-4-carbaldehyde (60 mg, 0.15 mmol, 1.0 eq) and N-(3-amino-1-((1r,4r)-4-(piperazin-1-yl)cyclohexyl)-1H-pyrazol-4-yl)-6-(trifluoromethyl)picolinamide (55.5 mg, 0.12 mmol, 0.8 eq) in DMF (2 mL) was added AcOH (2.7 mg, 0.05 mmol, 0.3 eq) at rt. The mixture was stirred at rt for 0.5 h. Sodium triacetoxyborohydride (95.6 mg, 0.45 mmol, 3.0 eq) was then added to the above reaction solution. The mixture was stirred at rt for 1 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (white solid, 24.8 mg, yield 20%). **¹H NMR** (400 MHz, DMSO-*d*₆) δ 11.79 (s, 1H), 11.02 (s, 1H), 8.88 (s, 1H), 8.47-8.37 (m, 2H), 8.24-8.18 (m, 1H), 8.14 (s, 1H), 7.95 (s, 1H), 7.79 (d, *J=* 9.2 Hz, 2H), 7.71 (s, 1H), 7.51 (d, *J* = 8.8 Hz, 2H), 7.06 (dd, *J=* 19.6, 9.2 Hz, 4H), 5.18 (dd, *J=* 12.6, 5.2 Hz, 1H), 3.85 (d, *J=* 12.4 Hz, 2H), 3.31 (s, 4H), 3.23 (s, 4H), 2.91-2.75 (m, 3H), 2.65-2.59 (m, 1H), 2.55-2.52 (m, 3H), 2.48-2.44 (m, 1H), 2.27-2.20 (m, 2H), 2.14-2.07 (m, 1H), 1.87-1.76 (m, 3H), 1.26-1.16 (m, 2H). LCMS (ESI): m/z 841 [M+H]⁺.

### Preparation Example 274. Synthesis of Compound A190

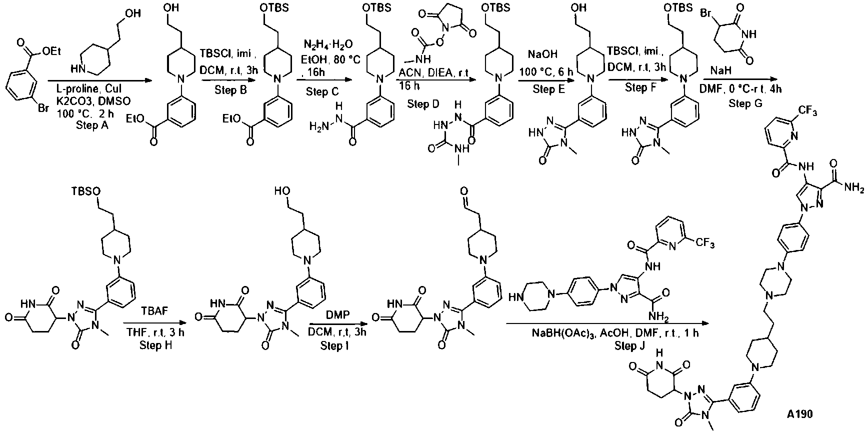

### Step A: Ethyl 3-(4-(2-hydroxyethyl)piperidin-1-yl)benzoate

To a solution of ethyl 3-bromobenzoate (10.0 g, 43.16 mmol, 1.0 eq) and 2-(pyrrolidin-3-yl)ethan-1-ol (6.76 g, 52.38 mmol, 1.2 eq) in dimethyl sulfoxide (100 mL) at rt were added potassium carbonate (18.10 g, 130.96 mmol, 3.0 eq), L-proline (0.50 g, 4.36 mmol, 0.1 eq), and cuprous iodide (1.66 g, 8.74 mmol, 0.2 eq). The reaction solution was reacted at 100°C under N₂ for 12 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 4.2 g, yield 35%).

### Step B: Ethyl 3-(4-(2-((tert-butyldimethylsilyl)oxy)ethyl)piperidin-1-yl)benzoate

To a solution of ethyl 3-(4-(2-hydroxyethyl)piperidin-1-yl)benzoate (3.8 g, 13.70 mmol, 1.0 eq) and imidazole (1.87 g, 27.40 mmol, 2.0 eq) in DCM (15 mL) was added tert-butyldimethylsilyl chloride (3.1 g, 20.55 mmol, 1.5 eq) at 0°C. The reaction solution was reacted for 3 h under N₂ at rt. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by column chromatography to afford the title compound (white solid, 3.3 g, yield 62%).

### Step C: 3-(4-(2-((tert-Butyldimethylsilyl)oxy)ethyl)piperidin-1-yl)benzohydrazide

To a solution of ethyl 3-(4-(2-((tert-butyldimethylsilyl)oxy)ethyl)piperidin-1-yl)benzoate (3.3 g, 8.43 mmol, 1.0 eq) in ethanol (15 mL) was added 80% hydrazine hydrate (15 mL) at rt. The reaction solution was reacted at 80°C under N₂ for 16 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 2.6 g, yield 82%).

### Step D: 2-(3-(4-(2-((tert-Butyldimethylsilyl)oxy)ethyl)piperidin-1-yl)benzoyl)-N-methylhydrazine-1-carboxamide

To a solution of 3-(4-(2-((tert-butyldimethylsilyl)oxy)ethyl)piperidin-1-yl)benzohydrazide (2.6 g, 6.89 mmol, 1.0 eq) and 2,5-dioxopyrrolidin-1-ylmethylcarbamate (1.42 g, 8.26 mmol, 1.2 eq) in acetonitrile (30 mL) was added DIEA (1.78 g, 13.77 mmol, 2.0 eq) at rt. The reaction solution was reacted for 16 h under N₂ at rt. Upon completion, the reaction solution was poured into water and extracted with EtOAc (×3). The combined organic phases were washed with saturated aqueous NaCl, dried over anhydrous Na₂SO₄, filtered, and concentrated under reduced pressure to afford the title compound (off-white solid, 2.5 g, crude).

### Step E: 5-(3-(4-(2-hydroxyethyl)piperidin-1-yl)phenyl)-4-methyl-2,4-dihydro-3H-1,2,4-triazol-3-one

At rt, to a NaOH solution (30 mL, 23.0 mmol, 4.0 eq) was added 2-(3-(4-(2-((tert-butyldimethylsilyl)oxy)ethyl)piperidin-1-yl)benzoyl)-N-methylhydrazine-1-carboxamide (2.5 g, 5.75 mmol, 1.0 eq). The reaction solution was reacted under N₂ at 100°C for 5 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 1.6 g, yield 92%).

### Step F: 5-(3-(4-(2-((tert-Butyldimethylsilyl)oxy)ethyl)piperidin-1-yl)phenyl)-4-methyl-2,4-dihydro-3H-1,2,4-triazol-3-one

To a solution of 5-(3-(4-(2-hydroxyethyl)piperidin-1-yl)phenyl)-4-methyl-2,4-dihydro-3H-1,2,4-triazol-3-one (1.6 g, 5.29 mmol, 1.0 eq) and imidazole (1.6 g, 5.29 mmol, 1.0 eq) in DCM (20 mL) was added tert-butyldimethylsilyl chloride (0.96 g, 6.35 mmol, 1.2 eq) at 0°C. The reaction solution was reacted for 3 h under N₂ at rt. Upon completion, the reaction solution was poured into water and extracted with DCM (×3). The combined organic phases were washed with saturated aqueous NaCl, dried over anhydrous Na₂SO₄, filtered, and concentrated under reduced pressure. The residue was purified by column chromatography to afford the title compound (white solid, 1.2 g, yield 54%).

### Step G: 3-(3-(4-(2-((tert-Butyldimethylsilyl)oxy)ethyl)piperidin-1-yl)phenyl)-4-methyl-5-oxo-4,5-dihydro-1H-1,2,4-triazol-1-yl)piperidine-2,6-dione

At 0°C, to a solution of 5-(3-(4-(2-((tert-butyldimethylsilyl)oxy)ethyl)piperidin-1-yl)phenyl)-4-methyl-2,4-dihydro-3H-1,2,4-triazol-3-one (600 mg, 1.44 mmol, 1.0 eq) in DMF (10 mL) was added 60% sodium hydride (173 mg, 4.32 mmol, 3.0 eq). The reaction solution was reacted at 0°C under N₂ for 0.5 h. 3-Bromopiperidine-2,6-dione (553 mg, 2.88 mmol, 2.0 eq) was then added, and the reaction solution was reacted at rt under N₂ for 4 h. Upon completion, the reaction solution was poured into aqueous NH₄Cl and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 0.5 g, yield 65%).

### Step H: 3-(3-(4-(2-hydroxyethyl)piperidin-1-yl)phenyl)-4-methyl-5-oxo-4,5-dihydro-1H-1,2,4-triazol-1-yl)piperidine-2,6-dione

To a solution of 3-(3-(4-(2-((tert-butyldimethylsilyl)oxy)ethyl)piperidin-1-yl)phenyl)-4-methyl-5-oxo-4,5-dihydro-1H-1,2,4-triazol-1-yl)piperidine-2,6-dione (500 mg, 0.95 mmol, 1.0 eq) in THF (10 mL) was added 1 M tetrabutylammonium fluoride (1.9 mL, 1.9 mmol, 2.0 eq) at rt. The reaction solution was reacted for 3 h under N₂ at rt. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by thin layer chromatography to afford the title compound (white solid, 0.26 g, yield 66%).

### Step I: 2-(1-(3-(1-(2,6-dioxopiperidin-3-yl)-4-methyl-5-oxo-4,5-dihydro-1H-1,2,4-triazol-3-yl)phenyl)piperidin-4-yl)acetaldehyde

To a solution of 3-(3-(4-(2-hydroxyethyl)piperidin-1-yl)phenyl)-4-methyl-5-oxo-4,5-dihydro-1H-1,2,4-triazol-1-yl)piperidine-2,6-dione (150 mg, 0.36 mmol, 1.0 eq) in DCM (4 mL) was added Dess-Martin periodinane (307 mg, 0.73 mmol, 2.0 eq) at rt. The reaction solution was reacted for 3 h under N₂ at rt. Upon completion, the reaction solution was filtered and concentrated under reduced pressure. The residue was purified by thin layer chromatography to afford the title compound (yellow solid, 100 mg, yield 67%). **Step J: N-(3-carbamoyl-1-(4-(4-(2-(3-(1-(2,6-dioxopiperidin-3-yl)-4-methyl-5-oxo-4,5-dihydro-1H-1,2,4-triazol-3-yl)phenyl)piperidin-4-yl)ethyl)piperazin-1-yl)phenyl)-1H-pyrazol-4-yl)-6-(trifluoromethyl)picolinamide**

To a solution of 2-(1-(3-(1-(2,6-dioxopiperidin-3-yl)-4-methyl-5-oxo-4,5-dihydro-1H-1,2,4-triazol-3-yl)phenyl)piperidin-4-yl)acetaldehyde (100 mg, 0.24 mmol, 1.0 eq) and N-(3-amino-1-(4-(piperazin-1-yl)phenyl)-1H-pyrazol-4-yl)-6-(trifluoromethyl)picolinamide (89 mg, 0.19 mmol, 0.8 eq) in DMF (30 mL) was added AcOH (4 mg, 0.07 mmol, 0.3 eq) at rt. The reaction solution was reacted at rt for 0.5 h. Sodium triacetoxyborohydride (103 mg, 0.49 mmol, 2.0 eq) was then added, and the reaction solution was stirred at rt for 1 h. Upon completion, the reaction solution was purified by HPLC to afford the title compound (off-white solid, 75.3 mg, yield 36%). ¹HNMR (400 MHz, DMSO-*d*₆) δ 11.79 (s, 1H), 11.05 (s, 1H), 8.88 (s, 1H), 8.48-8.36 (m, 2H), 8.21 (d, *J=* 7.6 Hz, 1H), 7.95 (s, 1H), 7.79 (d, *J* = 8.8 Hz, 2H), 7.71 (s, 1H), 7.34 (t, *J=* 8.0 Hz, 1H), 7.09 (dd, *J=* 15.6, 8.8 Hz, 4H), 7.01 (d, *J* = 7.6 Hz, 1H), 5.22 (dd, *J=* 12.8, 5.2 Hz, 1H), 3.74 (d, *J =* 12.4 Hz, 2H), 3.31 (s, 3H), 3.21(s, 4H), 2.94-2.83 (m, 1H), 2.67 (dt, *J* = 17.6, 12.8 Hz, 3H), 2.54 (s, 4H), 2.47 (d, *J* = 4.4 Hz, 1H), 2.41 (d, *J=* 7.2 Hz, 2H), 2.17-2.07 (m, 1H), 1.78 (d, *J =* 14.0 Hz, 2H), 1.46 (s, 3H), 1.27 (dd, *J =* 20.4, 9.6 Hz, 2H). LCMS (ESI): m/z 855 [M+H]⁺.

### Preparation Example 275. Synthesis of Compound A191

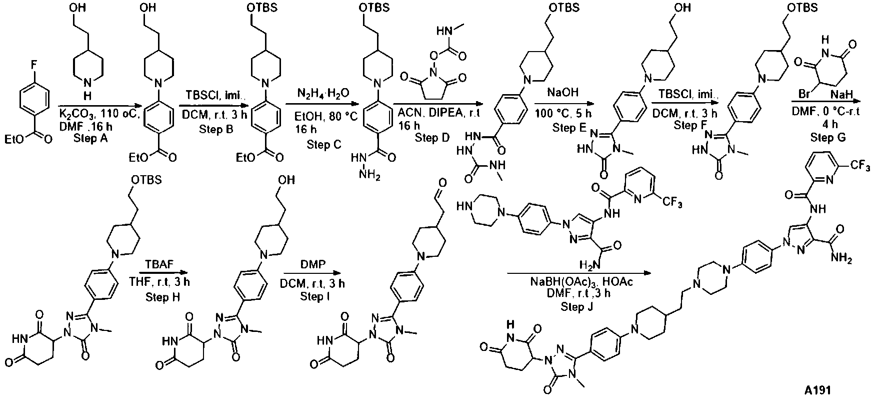

### Step A: Ethyl 4-(4-(2-hydroxyethyl)piperidin-1-yl)benzoate

To a solution of ethyl 4-fluorobenzoate (5.0 g, 29.73 mmol, 1.0 eq) and 2-(piperidin-4-yl)ethan-1-ol (4.61 g, 35.68 mmol, 1.2 eq) in DMF (50 mL) was added potassium carbonate (17.1 g, 124 mmol, 3.0 eq) at rt. The reaction solution was stirred at 110°C under N₂ for 16 h. Upon completion, the reaction solution was cooled to rt, diluted in saturated aqueous NH₄Cl, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 3.8 g, yield 46%).

### Step B: Ethyl 4-(4-(2-((tert-Butyldimethylsilyl)oxy)ethyl)piperidin-1-yl)benzoate

To a solution of ethyl 4-(4-(2-hydroxyethyl)piperidin-1-yl)benzoate (3.8 g, 13.70 mmol, 1.0 eq) and imidazole (1.87 g, 27.40 mmol, 2.0 eq) in DCM (15 mL) was added tert-butyldimethylsilyl chloride (3.1 g, 20.55 mmol, 1.5 eq) at 0°C. The reaction solution was stirred at rt under N₂ for 3 h. Upon completion, the reaction solution was quenched with water and extracted with DCM (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 3.3 g, yield 61%).

### Step C: 4-(4-(2-((tert-Butyldimethylsilyl)oxy)ethyl)piperidin-1-yl)benzohydrazide

At 0°C, to a solution of ethyl 4-(4-(2-((tert-butyldimethylsilyl)oxy)ethyl)piperidin-1-yl)benzoate (3.3 g, 8.43 mmol, 1.0 eq) in ethanol (15 mL) was added 80% hydrazine hydrate (15 mL). The reaction solution was stirred at 80°C under N₂ for 16 h. Upon completion, the reaction solution was cooled to rt, quenched with water, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 2.6 g, yield 82%).

### Step D: 2-(4-(4-(2-((tert-Butyldimethylsilyl)oxy)ethyl)piperidin-1-yl)benzoyl)-N-methylhydrazine-1-carboxamide

To a solution of 4-(4-(2-((tert-butyldimethylsilyl)oxy)ethyl)piperidin-1-yl)benzohydrazide (2.6 g, 6.89 mmol, 1.0 eq) and 2,5-dioxopyrrolidin-1-ylmethylcarbamate (1.42 g, 8.26 mmol, 1.2 eq) in acetonitrile (30 mL) was added DIPEA (1.78 g, 13.77 mmol, 2.0 eq) at rt. The mixture was stirred at rt under N₂ for 16 h, Upon completion, water was added, and the mixture was extracted with EtOAc, washed with saturated aqueous NaCl, dried over anhydrous Na₂SO₄, filtered, and concentrated under reduced pressure to afford the title compound (off-white solid, 2.5 g, crude), which was used directly without purification.

### Step E: 5-(4-(4-(2-hydroxyethyl)piperidin-1-yl)phenyl)-4-methyl-2,4-dihydro-3H-1,2,4-triazol-3-one

At 0°C, to 2-(4-(4-(2-((tert-butyldimethylsilyl)oxy)ethyl)piperidin-1-yl)benzoyl)-N-methylhydrazine-1-carboxamide (2.5 g, 5.75 mmol, 1.0 eq) was added aqueous NaOH (30 mL, 23.0 mmol, 4.0 eq). The reaction solution was stirred at 100°C under N₂ for 5 h. Upon completion, the reaction solution was cooled to rt, quenched with water, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 1.6 g, yield 92%).

### Step F: 5-(4-(4-(2-((tert-Butyldimethylsilyl)oxy)ethyl)piperidin-1-yl)phenyl)-4-methyl-2,4-dihydro-3H-1,2,4-triazol-3-one

To a solution of 5-(4-(4-(2-hydroxyethyl)piperidin-1-yl)phenyl)-4-methyl-2,4-dihydro-3H-1,2,4-triazol-3-one (1.6 g, 5.29 mmol, 1.0 eq) and imidazole (0.72 g, 10.58 mmol, 2.0 eq) in DCM (20 mL) was added tert-butyldimethylsilyl chloride (0.96 g, 6.35 mmol, 1.2 eq) at 0°C. The reaction solution was stirred at rt under N₂ for 3 h. Upon completion, the reaction solution was quenched with water and extracted with DCM (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 1.2 g, yield 54%).

### Step G: 3-(3-(4-(4-(2-((tert-butyldimethylsilyl)oxy)ethyl)piperidin-1-yl)phenyl)-4-methyl-5-oxo-4,5-dihydro-1H-1,2,4-triazol-1-yl)piperidine-2,6-dione

To a solution of 5-(4-(4-(2-((tert-butyldimethylsilyl)oxy)ethyl)piperidin-1-yl)phenyl)-4-methyl-2,4-dihydro-3H-1,2,4-triazol-3-one (600 mg, 1.44 mmol, 1.0 eq) in DMF (10 mL) was added portionwise 60 wt% sodium hydride (173 mg, 4.32 mmol, 3.0 eq) at 0°C under N₂. The mixture was stirred at rt for 0.5 h. 3-Bromopiperidine-2,6-dione (553 mg, 2.88 mmol, 2.0 eq) was then added to the above reaction solution. The mixture was stirred at rt under N₂ for 4 h. Upon completion, the reaction was quenched with saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by column chromatography to afford the title compound (white solid, 500 mg, yield 65%).

### Step H: 3-(3-(4-(4-(2-hydroxyethyl)piperidin-1-yl)phenyl)-4-methyl-5-oxo-4,5-dihydro-1H-1,2,4-triazol-1-yl)piperidine-2,6-dione

At 0°C, to a solution of 3-(3-(4-(4-(2-((tert-butyldimethylsilyl)oxy)ethyl)piperidin-1-yl)phenyl)-4-methyl-5-oxo-4,5-dihydro-1H-1,2,4-triazol-1-yl)piperidine-2,6-dione (500 mg, 0.95 mmol, 1.0 eq) in THF (5 mL) was added 1N tetrabutylammonium fluoride solution in THF (1.9 mL, 1.9 mmol, 2.0 eq). The reaction solution was stirred at rt under N₂ for 3 h. Upon completion, the reaction solution was cooled to rt, quenched with water, and extracted with EtOAc (×3). The combined organic phases were washed sequentially with water and saturated aqueous NaCl, dried over anhydrous Na₂SO₄, filtered, and concentrated under reduced pressure. The residue was purified by thin layer chromatography to afford the title compound (white solid, 260 mg, yield 66%).

### Step I: 2-(1-(4-(1-(2,6-dioxopiperidin-3-yl)-4-methyl-5-oxo-4,5-dihydro-1H-1,2,4-triazol-3-yl)phenyl)piperidin-4-yl)acetaldehyde

To a solution of 3-(3-(4-(4-(2-hydroxyethyl)piperidin-1-yl)phenyl)-4-methyl-5-oxo-4,5-dihydro-1H-1,2,4-triazol-1-yl)piperidine-2,6-dione (150 mg, 0.36 mmol, 1.0 eq) in DCM (4 mL) was added Dess-Martin periodinane (307 mg, 0.73 mmol, 2.0 eq) at 0°C. The mixture was stirred at rt for 3 h. Upon completion, the reaction solution was filtered, and the filtrate was concentrated under pressure. The residue was purified by thin layer chromatography to afford the title compound (yellow solid, 100 mg, yield 67%).

### Step J: N-(3-carbamoyl-1-(4-(4-(2-(4-(1-(2,6-dioxopiperidin-3-yl)-4-methyl-5-oxo-4,5-dihydro-1H-1,2,4-triazol-3-yl)phenyl)piperidin-4-yl)ethyl)piperazin-1-yl)phenyl)-1H-pyrazol-4-yl)-6-(trifluoromethyl)picolinamide

To a solution of 2-(1-(4-(1-(2,6-dioxopiperidin-3-yl)-4-methyl-5-oxo-4,5-dihydro-1H-1,2,4-triazol-3-yl)phenyl)piperidin-4-yl)acetaldehyde (100 mg, 0.24 mmol, 1.0 eq) and N-(3-amino-1-((1r,4r)-4-(piperazin-1-yl)cyclohexyl)-1H-pyrazol-4-yl)-6-(trifluoromethyl)picolinamide (89.3 mg, 0.19 mmol, 0.8 eq) in DMF (2 mL) was added AcOH (4.38 mg, 0.07 mmol, 0.3 eq) at rt. The mixture was stirred at rt for 0.5 h. Sodium triacetoxyborohydride (103 mg, 0.49 mmol, 2.0 eq) was then added to the above reaction solution. The mixture was stirred at rt for 3 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (white solid, 75.3 mg, yield 36%). **¹H NMR** (400 MHz, DMSO-*d*₆) δ 11.79 (s, 1H), 11.02 (s, 1H), 8.88 (s, 1H), 8.46-8.37 (m, 2H), 8.21 (dd, *J=* 7.6 ,1.2 Hz, 1H), 7.95 (s, 1H), 7.79 (d, *J=* 8.8 Hz, 2H), 7.71 (s, 1H), 7.50 (d, *J=* 8.8 Hz, 2H), 7.05 (dd, *J=* 20.0, 9.2 Hz, 4H), 5.18 (dd, *J =* 12.4, 5.2 Hz, 1H), 3.84 (d, *J =* 12.8 Hz, 2H), 3.31 (s, 6H), 3.21 (s, 4H), 2.89-2.83 (m, 1H), 2.79-2.73 (m, 2H), 2.64-2.51 (m, 4H), 2.41-2.37 (m, 2H), 2.12-2.07 (m, 1H), 1.80-1.77 (m, 2H), 1.48-1.44 (m, 2H), 1.29-1.23 (m, 2H). LCMS (ESI): m/z 855 [M+H]⁺.

### Preparation Example 276. Synthesis of Compound A192

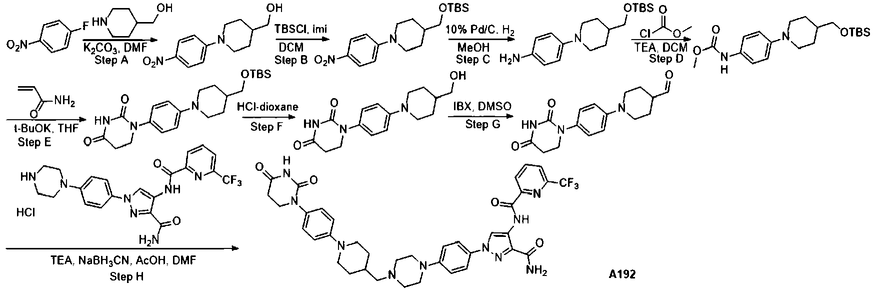

### Step A: (1-(4-nitrophenyl)piperidin-4-yl)methanol

To a solution of 4-fluoro-1-nitrobenzene (1.5 g, 10.6 mmol, 1.0 eq) in DMF (50 mL) at rt were added piperidin-4-ylmethanol (1.84 g, 15.9 mmol, 1.5 eq) and potassium carbonate (2.94 g, 21.3 mmol, 2.0 eq). The reaction solution was stirred at 60°C under N₂ for 15 h. Upon completion, the reaction solution was cooled to rt, diluted in EtOAc, washed sequentially with water and saturated aqueous NaCl, dried over anhydrous Na₂SO₄, filtered, and concentrated under reduced pressure. The residue was purified by column chromatography to afford the title compound (yellow solid, 2.5 g, yield 99%).

### Step B: 4-((tert-Butyldimethylsilyl)oxy)methyl)-1-(4-nitrophenyl)piperidine

To a solution of (1-(4-nitrophenyl)piperidin-4-yl)methanol (2.50 g, 9.98 mmol, 1.0 eq) in DCM (50 mL) at rt were added imidazole (1.0 g, 14.98 mmol, 1.5 eq) and tert-butyldimethylsilyl chloride (2.26 g, 14.98 mmol, 1.5 eq). The reaction solution was stirred at rt under N₂ for 15 h. Upon completion, the mixture was diluted in DCM, washed sequentially with water and saturated aqueous NaCl, dried over anhydrous Na₂SO₄, filtered, and concentrated under reduced pressure. The residue was purified by column chromatography to afford the title compound (yellow solid, 2.1 g, yield 58%).

### Step C: 4-(4-(((tert-Butyldimethylsilyl)oxy)methyl)piperidin-1-yl)aniline

To a solution of 4-((tert-butyldimethylsilyl)oxy)methyl)-1-(4-nitrophenyl)piperidine (2.1 g, 5.99 mmol, 1.0 eq) in MeOH (50 mL) was added 10% palladium on carbon (1.0 g, 9.39 mmol, 1.5 eq) at rt. The reaction solution was purged with H₂ (using a hydrogen balloon) (×3) and stirred under H₂ at rt for 18 h. Upon completion, the mixture was filtered, and the filtrate was concentrated under reduced pressure to afford the title compound (yellow oil, 1.6 g, crude), which was used directly without purification.

### Step D: Methyl (4-(((tert-Butyldimethylsilyl)oxy)methyl)piperidin-1-yl)phenyl)carbamate

To a solution of 4-(4-((tert-butyldimethylsilyl)oxy)methyl)piperidin-1-yl)aniline (1.6 g, 4.99 mmol, 1.0 eq) in DCM (30 mL) were added TEA (2.1 mL, 14.97 mmol, 3.0 eq) and methyl chloroformate (0.5 mL, 5.99 mmol, 1.2 eq) at 0°C. The reaction solution was stirred at rt under N₂ for 3 h. Upon completion, the mixture was diluted in DCM, washed sequentially with water and saturated aqueous NaCl, dried over anhydrous Na₂SO₄, filtered, and concentrated under reduced pressure. The residue was purified by column chromatography to afford the title compound (yellow solid, 1.1 g, yield 58%).

### Step E: 1-(4-(4-(((tert-Butyldimethylsilyl)oxy)methyl)piperidin-1-yl)phenyl)dihydropyrimidine-2,4(1H,3H)-dione

To a solution of methyl (4-(((tert-butyldimethylsilyl)oxy)methyl)piperidin-1-yl)phenyl)carbamate (1.1 g, 2.91 mmol, 1.0 eq) in THF (50 mL) at 0°C were added potassium tert-butoxide (0.36 g, 3.20 mmol, 1.1 eq) and acrylamide (0.41 g, 5.81 mmol, 2.0 eq). The reaction solution was stirred at 60°C under N₂ for 3 h. Upon completion, the reaction solution was cooled to rt, poured into glacial AcOH, and extracted with EtOAc (×3). The mixture was washed sequentially with water and saturated aqueous NaCl, dried over anhydrous Na₂SO₄, filtered, and concentrated under reduced pressure. The residue was purified by column chromatography to afford the title compound (off-white solid, 220 mg, yield 18%).

### Step F: 1-(4-(4-(hydroxymethyl)piperidin-1-yl)phenyl)dihydropyrimidine-2,4(1H,3H)-dione

To a solution of 1-(4-(4-(((tert-butyldimethylsilyl)oxy)methyl)piperidin-1-yl)phenyl)dihydropyrimidine-2,4(1H,3H)-dione (220 mg, 0.53 mmol, 1.0 eq) was added 4 M HCl in 1,4-dioxane (2 mL, 8.0 mmol, 15.0 eq) at 0°C. The reaction solution was stirred at rt under N₂ for 6 h. The reaction solution was concentrated under reduced pressure to afford the title compound (yellow solid, 100 mg, crude), which was used directly without purification.

### Step G: 1-(4-(2,4-dioxotetrahydropyrimidin-1(2H)-yl)phenyl)piperidine-4-carbaldehyde

To a solution of 1-(4-(4-(hydroxymethyl)piperidin-1-yl)phenyl)dihydropyrimidine-2,4(1H,3H)-dione (100 mg, 0.33 mmol, 1.0 eq) in dimethyl sulfoxide (5.0 mL) was added IBX (166 mg, 0.59 mmol, 1.5 eq) at rt. The reaction solution was stirred at rt for 6 h. Upon completion, the mixture was diluted in water, and the reaction solution was extracted with EtOAc, washed sequentially with water and saturated aqueous NaCl, dried over anhydrous Na₂SO₄, and concentrated under reduced pressure. The residue was purified by column chromatography to afford the title compound (yellow solid, 60 mg, yield 60%).

### Step H: N-(3-carbamoyl-1-(4-((1-(4-(2,4-dioxotetrahydropyrimidin-1(2H)-yl)phenyl)piperidin-4-yl)methyl)piperazin-1-ylphenyl)-1H-pyrazol-4-yl)-6-(trifluoromethyl)picolinamide

To a solution of N-(3-amino-1-(4-(piperazin-1-yl)phenyl)-1H-pyrazol-4-yl)-6-(trifluoromethyl)picolinamide hydrochloride (96.1 mg, 0.21 mmol, 1.05 eq) in DMF (5 mL) was added TEA (0.1 mL), and the mixture was stirred for 10 min at rt. AcOH (0.2 mL) and 1-(4-(2,4-dioxotetrahydropyrimidin-1(2H)-yl)phenyl)piperidine-4-carbaldehyde (60 mg, 0.20 mmol, 1.0 eq) were then added. The mixture was stirred at rt for 5 h. Sodium triacetoxyborohydride (18.8 mg, 0.30 mmol, 1.5 eq) was then added to the above reaction solution. The mixture was stirred at rt for 2 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (white solid, 27 mg, yield 18%). **¹H NMR** (400 MHz, DMSO-*d*₆) δ 11.79 (s, 1H), 10.25 (s, 1H), 8.88 (s, 1H), 8.53-8.35 (m, 2H), 8.21 (d, *J =* 7.7 Hz, 1H), 7.95 (s, 1H), 7.79 (d, *J =* 9.1 Hz, 2H), 7.71 (s, 1H), 7.11 (dd, *J =* 20.7, 9.1 Hz, 4H), 6.93 (d, *J* = 9.0 Hz, 2H), 3.69 (t, *J =* 6.7 Hz, 4H), 3.35 (s, 2H), 3.30-3.29 (m, 2H), 3.23 (s, 4H), 2.68 (t, *J =* 6.7 Hz, 4H), 2.24 (d, *J =* 7.0 Hz, 2H), 1.82 (d, *J =* 13.2 Hz, 2H), 1.74 (s, 1H), 1.24 (s, 2H). LC-MS (ESI): m/z 745 [M+H]⁺.

### Preparation Example 277. Synthesis of Compound A193

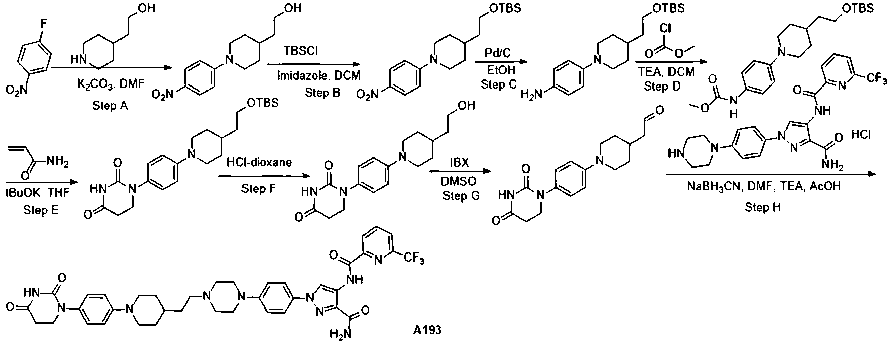

### Step A: 2-(1-(4-nitrophenyl)piperidin-4-yl)ethan-1-ol

To a solution of 4-fluoro-1-nitrobenzene (3 g, 21.3 mmol, 1.0 eq) in DMF (50.0 mL) at rt were added 2-(piperidin-4-yl)ethan-1-ol (3.3 g, 25.2 mmol, 1.2 eq) and potassium carbonate (5.9 g, 42.5 mmol, 2.0 eq). The reaction solution was stirred at 60°C under N₂ for 15 h. Upon completion, the reaction solution was cooled to rt, diluted in EtOAc, washed sequentially with water and saturated aqueous NaCl, dried over anhydrous Na₂SO₄, filtered, and concentrated under reduced pressure. The residue was purified by column chromatography to afford the title compound (yellow solid, 3.9 g, yield 73%).

### Step B: 4-(2-((tert-butyldimethylsilyl)oxy)ethyl)-1-(4-nitrophenyl)piperidine

To a solution of 2-(1-(4-nitrophenyl)piperidin-4-yl)ethane-1-ol (3 g, 12.0 mmol, 1.0 eq) in DCM (50 mL) at rt were added imidazole (1.2 g, 18.0 mmol, 1.5 eq) and tert-butyldimethylsilyl chloride (2.7 g, 18.0 mmol, 1.5 eq). The reaction solution was stirred at rt under N₂ for 15 h. Upon completion, the mixture was diluted in DCM, washed sequentially with water and saturated aqueous NaCl, dried over anhydrous Na₂SO₄, filtered, and concentrated under reduced pressure. The residue was purified by column chromatography to afford the title compound (yellow solid, 4.0 g, yield 92%).

### Step C: 4-(4-(2-((tert-butyldimethylsilyl)oxy)ethyl)piperidin-1-yl)aniline

To a solution of 4-(2-((tert-butyldimethylsilyl)oxy)ethyl)-1-(4-nitrophenyl)piperidine (3.8 g, 10.4 mmol, 1.0 eq) in ethanol (50 mL) was added 10% palladium on carbon (200 mg) at rt. The reaction solution was purged with H₂ (using a hydrogen balloon) (×3) and stirred under H₂ at rt for 2 h. Upon completion, the mixture was filtered, and the filtrate was concentrated under reduced pressure to afford the title compound (yellow solid, 2.5 g, crude), which was used directly without purification.

### Step D: Methyl (4-(4-(2-((tert-butyldimethylsilyl)oxy)ethyl)piperidin-1-yl)phenyl)carbamate

To a solution of 4-(4-(2-((tert-butyldimethylsilyl)oxy)ethyl)piperidin-1-yl)aniline (5 g, 14.9 mmol, 1.0 eq) in DCM (30 mL) at 0°C were added TEA (6.2 mL, 44.8 mmol, 3.0 eq) and methyl chloroformate (1.4 mL, 17.9 mmol, 1.2 eq). The reaction solution was stirred at rt under N₂ for 3 h. Upon completion, the mixture was diluted in DCM, washed sequentially with water and saturated aqueous NaCl, dried over anhydrous Na₂SO₄, filtered, and concentrated under reduced pressure. The residue was purified by column chromatography to afford the title compound (yellow solid, 2.5 g, yield 43%).

### Step E: 1-(4-(4-(2-((tert-butyldimethylsilyl)oxy)ethyl)piperidin-1-yl)phenyl)dihydropyrimidine-2,4(1H,3H)-dione

To a solution of methyl (4-(4-(2-((tert-butyldimethylsilyl)oxy)ethyl)piperidin-1-yl)phenyl)carbamate (1.3 g, 3.1 mmol, 1.0 eq) in anhydrous THF (50 mL) at 0°C were added potassium tert-butoxide (0.69 g, 6.1 mmol, 2.0 eq) and acrylamide (0.33 g, 4.6 mmol, 1.5 eq). The reaction solution was stirred at 60°C under N₂ for 3 h. Upon completion, the reaction solution was cooled to rt, poured into glacial AcOH, and extracted with EtOAc (×3). The mixture was washed sequentially with water and saturated aqueous NaCl, dried over anhydrous Na₂SO₄, filtered, and concentrated under reduced pressure. The residue was purified by column chromatography to afford the title compound (off-white solid, 400 mg, yield 30%).

### Step F: 1-(4-(4-(2-hydroxyethyl)piperidin-1-yl)phenyl)dihydropyrimidine-2,4(1H,3H)-dione

To a solution of 1-(4-(4-(2-((tert-butyldimethylsilyl)oxy)ethyl)piperidin-1-yl)phenyl)dihydropyrimidine-2,4(1H,3H)-dione (400 mg, 0.93 mmol, 1.0 eq) in 1,4-dioxane (1 mL) was added 4 M HCl in 1,4-dioxane (5 mL) at 0°C. The reaction solution was stirred at rt under N₂ for 6 h. The reaction solution was concentrated under reduced pressure to afford the title compound (yellow solid, 200 mg, crude), which was used directly without purification.

### Step G: 2-(1-(4-(2,4-dioxotetrahydropyrimidin-1(2H)-yl)phenyl)piperidin-4-yl)acetaldehyde

To a solution of 1-(4-(4-(2-hydroxyethyl)piperidin-1-yl)phenyl)dihydropyrimidine-2,4(1H,3H)-dione (300 mg, 0.9 mmol, 1.0 eq) in dimethyl sulfoxide (5.0 mL) was added IBX (529.4 mg, 1.8 mmol, 2.0 eq) at rt. The reaction solution was stirred at rt for 3 h. Upon completion, the mixture was diluted in water, and the reaction solution was extracted with EtOAc, washed sequentially with water and saturated aqueous NaCl, dried over anhydrous Na₂SO₄, and concentrated under reduced pressure. The residue was purified by reverse-phase column chromatography to afford the title compound (yellow solid, 150 mg, yield 50%).

### Step H: N-(3-amino-1-(4-(2-(1-(4-(2,4-dioxotetrahydropyrimidin-1(2H)-yl)phenyl)piperidin-4-yl)ethyl)piperazin-1-yl)phenyl]-1H-pyrazol-4-yl)-6-(trifluoromethyl)picolinamide

To a solution of N-(3-amino-1-(4-(piperazin-1-yl)phenyl)-1H-pyrazol-4-yl)-6-(trifluoromethyl)picolinamide hydrochloride (98.3 mg, 0.2 mmol, 1.0 eq) in DMF (5 mL) was added TEA (0.1 mL), and the mixture was stirred for 10 min at rt. AcOH (0.2 mL) and 2-(1-(4-(2,4-dioxotetrahydropyrimidin-1(2H)-yl)phenyl)piperidin-4-yl)acetaldehyde (75 mg, 0.2 mmol, 1.0 eq) were then added. The mixture was stirred at rt for 15 h. Sodium cyanoborohydride (100.3 mg, 0.5 mmol, 2.5 eq) was then added to the above reaction solution. The mixture was stirred at rt for 1 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (yellow solid, 16.2 mg, yield 9%). **¹H NMR** (400 MHz, DMSO-*d*₆) δ 11.79 (s, 1H), 10.25 (s, 1H), 8.88 (s, 1H), 8.42 (d, *J=* 17.2 Hz, 2H), 8.21 (d, *J=* 7.6 Hz, 1H), 7.95 (s, 1H), 7.79 (d, *J* = 9.2 Hz, 2H), 7.71 (s, 1H), 7.13 (d, *J =* 8.8 Hz, 2H), 7.08 (d, *J* = 9.2 Hz, 2H), 6.92 (d, *J* = 9.2 Hz, 2H), 3.73-3.61 (m, 4H), 3.23-3.18 (m, 4H), 2.71-2.60 (m, 4H), 2.55-2.51 (m, 4H), 2.43-2.35 (m, 2H), 1.82-1.71 (m, 2H), 1.52-1.41 (m, 3H), 1.31-1.21 (m, 2H). LC-MS (ESI): m/z 759 [M+H]⁺.

### Preparation Example 278. Synthesis of Compound A194

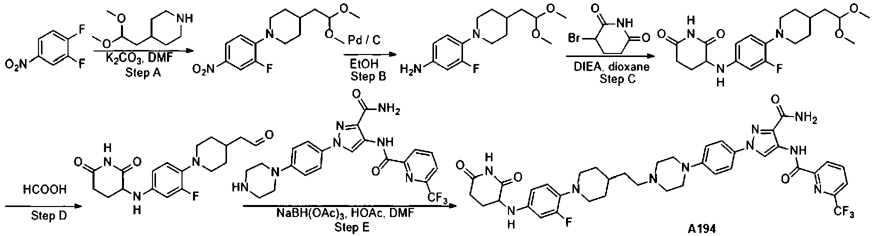

### Step A: 4-(2,2-dimethoxyethyl)-1-(2-fluoro-4-nitrophenyl)piperidine

To a solution of 4-(2,2-dimethoxyethyl)piperidine (2.5 g, 14.4 mmol, 1.0 eq) and 1,2-difluoro-4-nitrobenzene (2.8 g, 17.3 mmol, 1.2 eq) in DMF (20 mL) was added potassium carbonate (6 g, 43.2 mmol, 3.0 eq) at rt. The reaction solution was stirred at 80°C under N₂ for 16 h. Upon completion, the reaction solution was cooled to rt, diluted in saturated aqueous NH₄Cl, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (yellow solid, 3.4 g, yield 75%).

### Step B: 4-(4-(2,2-dimethoxyethyl)piperidin-1-yl)-3-fluoroaniline

To a solution of 4-(2,2-dimethoxyethyl)-1-(2-fluoro-4-nitrophenyl)piperidine (1.3 g, 4.1 mmol, 1 eq) in ethanol (10 mL) was added 10% palladium on carbon (400 mg) at rt. The reaction solution was purged with H₂ (using a hydrogen balloon) (×3) and stirred under H₂ at rt for 2 h. Upon completion, the mixture was filtered, and the filtrate was concentrated under reduced pressure to afford the title compound (yellow oil, 1 g, crude), which was used directly without purification.

### Step C: 3-((4-(4-(2,2-dimethoxyethyl)piperidin-1-yl)-3-fluorophenyl)amino)piperidine-2,6-dione

To a solution of 4-(4-(2,2-dimethoxyethyl)piperidin-1-yl)-3-fluoroaniline (1.3 g, 4.6 mmol, 1.0 eq) and 3-bromopiperidine-2,6-dione (1.8 g, 9.2 mmol, 2.0 eq) in 1,4-dioxane (10 mL) was added DIEA (1.6 mL, 9.2 mmol, 2.0 eq) at rt. The mixture was stirred at 80°C under N₂ for 16 h. Upon completion, the mixture was cooled to rt, diluted in water, extracted with EtOAc, washed with saturated aqueous NaCl, dried over anhydrous Na₂SO₄, filtered, and concentrated under reduced pressure. The residue was purified by column chromatography to afford the title compound (yellow solid, 1.3 g, yield 71%).

### Step D: 2-(1-(4-((2,6-dioxopiperidin-3-yl)amino)-2-fluorophenyl)piperidin-4-yl)acetaldehyde

To 3-((4-(4-(2,2-dimethoxyethyl)piperidin-1-yl)-3-fluorophenyl)amino)piperidine-2,6-dione (300 mg, 0.7 mmol, 1.0 eq) was added formic acid (3 mL) at 0°C. The reaction solution was stirred at rt under N₂ for 2 h. Upon completion, the reaction solution was concentrated under reduced pressure to afford the title compound (yellow oil, 185 mg, crude), which was used directly without purification.

### Step E: N-(3-Carbonyl-1-(4-(4-(2-(4-(((2,6-dioxopiperidin-3-yl)amino)-2-fluorophenyl)piperidin-4-yl)ethyl)piperazin-1-yl)phenyl)-1H-pyrazol-4-yl)-6-(trifluoromethyl)picolinamide

To a solution of 2-(1-(4-((2,6-dioxopiperidin-3-yl)amino)-2-fluorophenyl)piperidin-4-yl)acetaldehyde (88 mg, 0.2 mmol, 1 eq) and N-(3-amino-1-((1r,4r)-4-(piperazin-1-yl)cyclohexyl)-1H-pyrazol-4-yl)-6-(trifluoromethyl)picolinamide (100 mg, 0.2 mmol, 1.0 eq) in DMF (3 mL) was added AcOH (0.5 mL, 8.7 mmol, 44 eq) at rt. The mixture was stirred at rt for 0.5 h. Sodium triacetoxyborohydride (103 mg, 0.49 mmol, 2.0 eq) was then added to the above reaction solution. The mixture was stirred at rt for 1 h. Upon completion, the mixture was diluted in water, extracted with EtOAc (×3), washed with saturated aqueous NaCl, dried over anhydrous Na₂SO₄, filtered, and concentrated under reduced pressure. The residue was purified by HPLC to afford the title compound (white solid, 30 mg, yield 15%). **¹H NMR** (400 MHz, DMSO-*d*₆) δ 11.79 (s, 1H), 10.77 (s, 1H), 8.88 (s, 1H), 8.47-8.37 (m, 2H), 8.23-8.19 (m, 1H), 7.98-7.68 (m, 4H), 7.12-7.05 (m, 2H), 6.86-6.79 (m, 1H), 6.53-6.46 (m, 1H), 6.43-6.38 (m, 1H), 5.82-5.73 (m, 1H), 4.30-4.20 (m, 1H), 3.27-3.17 (m, 4H), 3.13-3.07 (m, 2H), 2.79-2.64 (m, 2H), 2.62-2.53 (m, 5H), 2.12-2.05 (m, 1H), 2.02-1.97 (m, 1H), 1.90-1.81 (m, 1H), 1.78-1.70 (m, 2H), 1.51-1.27 (m, 7H). LCMS (ESI): m/z 791 [M+H]⁺.

### Preparation Example 279. Synthesis of Compound A195

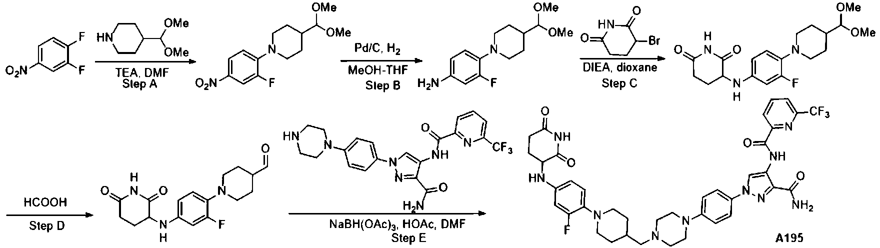

### Step A: 4-(Dimethoxymethyl)-1-(2-fluoro-4-nitrophenyl)piperidine

To a solution of 4-(dimethoxymethyl)piperidine (3.0 g, 18.8 mmol, 1.5 eq) and 4-(dimethoxymethyl)piperidine (2.0 g, 13.6 mmol, 1.0 eq) in dimethyl sulfoxide (20 mL) at rt was added TEA (3.6 mL, 25.2 mmol, 2.0 eq). The reaction solution was stirred at 90°C under N₂ for 16 h. Upon completion, the reaction solution was cooled to rt, diluted in saturated aqueous NH₄Cl, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (yellow solid, 3.6 g, yield 98%).

### Step B: 4-(4-(Dimethoxymethyl)piperidin-1-yl)-3-fluoroaniline

To a solution of 4-(dimethoxymethyl)-1-(2-fluoro-4-nitrophenyl)piperidine (3.0 g, 10.0 mmol, 1.0 eq) in MeOH (10 mL) and THF (10 mL) was added 10% palladium on carbon (600 mg) at rt. The reaction solution was purged with H₂ (using a hydrogen balloon) (×3) and stirred under H₂ at rt for 2 h. Upon completion, the mixture was filtered, and the filtrate was concentrated under reduced pressure to afford the title compound (yellow solid, 2.4 g, crude), which was used directly without purification.

### Step C: 3-((4-(4-(Dimethoxymethyl)piperidin-1-yl)-3-fluorophenyl)amino)piperidine-2,6-dione

To a solution of 4-(4-(dimethoxymethyl)piperidin-1-yl)-3-fluoroaniline (2.4 g, 8.9 mmol, 1.0 eq) and 3-3-bromopiperidine-2,6-dione (3.4 g, 17.8 mmol, 2.0 eq) in 1,4-dioxane (10 mL) were added DIEA (3 mL, 17.8 mmol, 2.0 eq) at rt. The mixture was stirred at 80°C under N₂ for 16 h. Upon completion, the mixture was cooled to rt, diluted in water, extracted with EtOAc, washed with saturated aqueous NaCl, dried over anhydrous Na₂SO₄, filtered, and concentrated under reduced pressure. The residue was purified by column chromatography to afford the title compound (brown solid, 400 mg, yield 15%).

### Step D: 1-(4-((2,6-dioxopiperidin-3-yl)amino)-2-fluorophenyl)piperidine-4-carbaldehyde

To 3-((4-(4-(dimethoxymethyl)piperidin-1-yl)-3-fluorophenyl)amino)piperidine-2,6-dione (400 mg, 1.0 mmol, 1.0 eq) was added formic acid (5 mL) at 0°C. The reaction solution was stirred at rt under N₂ for 2 h. Upon completion, the reaction solution was concentrated under reduced pressure to afford the title compound (yellow oil, 200 mg, crude), which was used directly without purification.

### Step E: N-(3-Carbamoyl-1-(4-((1-(4-(2,6-dioxopiperidin-3-yl)amino)-2-fluorophenyl)piperidin-4-yl)methyl)piperazin-1-yl)phenyl)-1H-pyrazol-4-yl)-6-(trifluoromethyl)picolinamide

To a solution of 1-(4-((2,6-dioxopiperidin-3-yl)amino)-2-fluorophenyl)piperidine-4-carbaldehyde (200 mg, 0.6 mmol, 1.0 eq) and N-(3-amino-1-((1r,4r)-4-(piperazin-1-yl)cyclohexyl)-1H-pyrazol-4-yl)-6-(trifluoromethyl)picolinamide (220 mg, 0.48 mmol, 0.8 eq) in DMF (6 mL) was added AcOH (0.3 mL) at rt. The mixture was stirred at rt for 1 h. Sodium triacetoxyborohydride (506 mg, 2.4 mmol, 4.0 eq) was then added to the above reaction solution. The mixture was stirred at rt for 1 h. Upon completion, the reaction was quenched with saturated NaHCO₃ solution, extracted with EtOAc (×3), washed with saturated aqueous NaCl, dried over anhydrous Na₂SO₄, filtered, and concentrated under reduced pressure. The residue was purified by HPLC to afford the title compound (white solid, 50 mg, yield 11%). **¹H NMR** (400 MHz, DMSO-*d*₆) δ 11.80 (s, 1H), 10.78 (s, 1H), 8.89 (s, 1H), 8.49-8.33 (m, 2H), 8.27-8.17 (m, 1H), 7.95 (s, 1H), 7.82-7.75 (m, 2H), 7.72 (s, 1H), 7.08 (d, *J=* 9.2 Hz, 2H), 6.92-6.81 (m, 1H), 6.55-6.39 (m, 2H), 5.79 (d, *J* = 7.6 Hz, 1H), 4.34-4.20 (m, 1H), 3.52-3.35 (m, 1H), 3.28-3.18 (m, 4H), 3.16-3.10 (m, 2H), 2.78-2.69 (m, 1H), 2.61-2.52 (m, 6H), 2.34-2.17 (m, 2H), 2.14-2.06 (m, 1H), 1.93-1.83 (m, 1H), 1.82-1.72 (m, 2H), 1.69-1.58 (m, 1H), 1.33-1.24 (m, 2H). LCMS (ESI): m/z 777 [M+H]⁺.

### Preparation Example 280. Synthesis of Compound A196

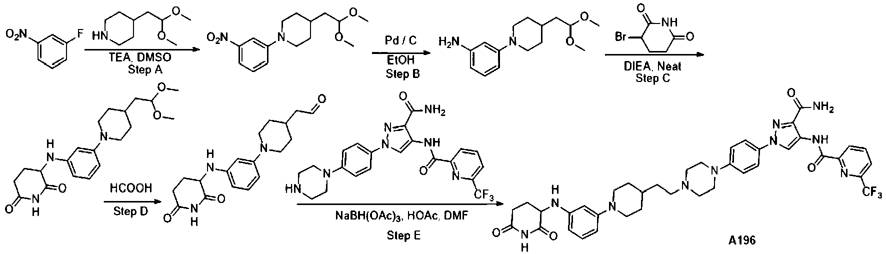

### Step A: 4-(2,2-dimethoxyethyl)-1-(3-nitrophenyl)piperidine

To a solution of 1,2-difluoro-4-nitrobenzene (1.5 g, 9.5 mmol, 1.5 eq) and 2-(piperidin-4-yl)-1,1-dimethoxyethane (1 g, 5.7 mmol, 1.0 eq) in dimethyl sulfoxide (10 mL) was added TEA (1.6 mL, 2.0 eq) at rt. The reaction solution was stirred at 90°C under N₂ for 16 h. Upon completion, the reaction solution was cooled to rt, diluted in saturated aqueous NH₄Cl, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (yellow solid, 855 mg, yield 51%).

### Step B: 3-(4-(2,2-dimethoxyethyl)piperidin-1-yl)aniline

To a solution of 4-(2,2-dimethoxyethyl)-1-(3-nitrophenyl)piperidine (850 mg, 2.9 mmol, 1.0 eq) in ethanol (10 mL) was added 10% palladium on carbon (200 mg) at rt. The reaction solution was purged with H₂ (using a hydrogen balloon) (×3) and stirred under H₂ at rt for 2 h. Upon completion, the mixture was filtered, and the filtrate was concentrated under reduced pressure to afford the title compound (yellow solid, 750 mg, crude), which was used directly without purification.

### Step C: 3-((3-(4-(2,2-dimethoxyethyl)piperidin-1-yl)phenyl)amino)piperidine-2,6-dione

To a solution of 3-(4-(2,2-dimethoxyethyl)piperidin-1-yl)aniline (220 mg, 0.8 mmol, 1.0 eq) and 3-3-bromopiperidine-2,6-dione (320 mg, 1.7 mmol, 2.0 eq) in 1,4-dioxane (2 mL) was added DIEA (0.27 mL, 1.6 mmol, 2.0 eq) at rt. The mixture was stirred at 80°C under N₂ for 16 h. Upon completion, the mixture was cooled to rt, diluted in water, extracted with EtOAc, washed with saturated aqueous NaCl, dried over anhydrous Na₂SO₄, filtered, and concentrated under reduced pressure. The residue was purified by column chromatography to afford the title compound (blue solid, 181 mg, yield 58%).

### Step D: 2-(1-(3-((2,6-dioxopiperidin-3-yl)amino)phenyl)piperidin-4-yl)acetaldehyde

To 3-((3-(4-(2,2-dimethoxyethyl)piperidin-1-yl)phenyl)amino)piperidine-2,6-dione (180 mg, 0.5 mmol, 1.0 eq) was added formic acid (5 mL) at 0°C. The reaction solution was stirred at rt under N₂ for 2 h. Upon completion, the reaction solution was concentrated under reduced pressure to afford the title compound (yellow oil, 150 mg, crude), which was used directly without purification.

### Step E: N-(3-Carbamoyl-1-(4-(2-(1-(3-((2,6-dioxopiperidin-3-yl)amino)phenyl)piperidin-4-yl)ethyl)piperazin-1-yl)phenyl)-1H-pyrazol-4-yl)-6-(trifluoromethyl)picolinamide

To a solution of 2-(1-(3-((2,6-dioxopiperidin-3-yl)amino)phenyl)piperidin-4-yl)acetaldehyde (180 mg, 0.55 mmol, 1.0 eq) and N-(3-amino-1-((1r,4r)-4-(piperazin-1-yl)cyclohexyl)-1H-pyrazol-4-yl)-6-(trifluoromethyl)picolinamide (251 mg, 0.55 mmol, 1.0 eq) in DMF (6 mL) was added AcOH (0.3 mL) at rt. The mixture was stirred at rt for 1 h. Sodium triacetoxyborohydride (461 mg, 2.0 mmol, 4 eq) was then added to the above reaction solution. The mixture was stirred at rt for 1 h. Upon completion, the reaction was quenched with saturated NaHCO₃ solution, extracted with EtOAc (×3), washed with saturated aqueous NaCl, dried over anhydrous Na₂SO₄, filtered, and concentrated under reduced pressure. The residue was purified by HPLC to afford the title compound (white solid, 18 mg, yield 5%). **¹H NMR** (400 MHz, DMSO-*d*₆) δ 11.79 (s, 1H), 10.76 (s, 1H), 8.88 (s, 1H), 8.47-8.37 (m, 2H), 8.21 (d, *J =* 7.6 Hz, 1H), 8.17 (s, 1H), 7.95 (s, 1H), 7.78 (d, *J* = 9.0 Hz, 2H), 7.70 (s, 1H), 7.08 (d, *J =* 9.1 Hz, 2H), 6.90 (t, *J =* 8.0 Hz, 1H), 6.26 (s, 1H), 6.19 (d, *J=* 8.1 Hz, 1H), 6.11 (d, *J =* 7.9 Hz, 1H), 5.59 (d, *J* = 7.4 Hz, 1H), 4.36-4.27 (m, 1H), 3.59 (d, *J =* 11.2 Hz, 4H), 3.22 (s, 4H), 2.73 (dd, *J =* 11.9, 5.4 Hz, 1H), 2.57 (dd, *J =* 15.5, 11.0 Hz, 6H), 2.39 (d, *J =* 6.6 Hz, 2H), 2.13-2.06 (m, 1H), 1.86 (dd, *J* = 12.0, 3.9 Hz, 1H), 1.74 (d, *J* = 11.8 Hz, 2H), 1.44 (s, 2H), 1.25 (d, *J =* 10.8 Hz, 2H). LCMS (ESI): m/z 773[M+H]⁺.

### Preparation Example 281. Synthesis of Compound A197

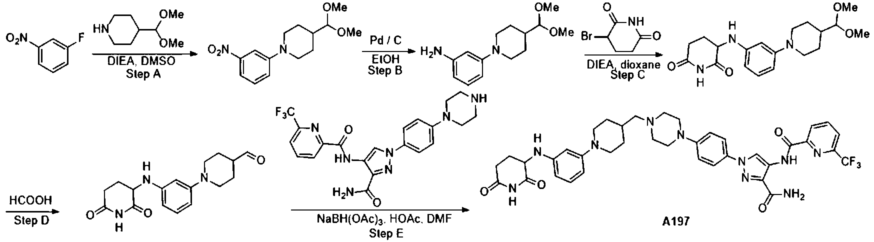

### Step A: 4-(dimethoxymethyl)-1-(3-nitrophenyl)piperidine

To a solution of 4-(dimethoxymethyl)piperidine (2.7 g, 17.0 mmol, 1.2 eq) and 1-fluoro-3-nitrobenzene (2.0 g, 14.2 mmol, 1.0 eq) in dimethyl sulfoxide (20 mL) at rt was added DIE A (7.5 mL, 42.5 mmol, 3.0 eq). The reaction solution was stirred at 120°C under N₂ for 16 h. Upon completion, the reaction solution was cooled to rt, diluted in saturated aqueous NH₄Cl, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (yellow oil, 2 g, yield 50%).

### Step B: 3-(4-(dimethoxymethyl)piperidin-1-yl)aniline

To a solution of 4-(dimethoxymethyl)-1-(3-nitrophenyl)piperidine (2 g, 7.1 mmol, 1.0 eq) in ethanol (20 mL) was added 10% palladium on carbon (400 mg) at rt. The reaction solution was purged with H₂ (using a hydrogen balloon) (×3) and stirred under H₂ at rt for 2 h. Upon completion, the mixture was filtered, and the filtrate was concentrated under reduced pressure to afford the title compound (yellow solid, 1.6 g, crude), which was used directly without purification.

### Step C: 3-((3-(4-(dimethoxymethyl)piperidin-1-yl)phenyl)amino)piperidine-2,6-dione

To a solution of 3-bromopiperidine-2,6-dione (1.5 g, 8.0 mmol, 2.0 eq) and 3-(4-(dimethoxymethyl)piperidin-1-yl)aniline (1 g, 4.0 mmol, 1.0 eq) in 1,4-dioxane (20 mL) was added DIEA (1.4 mL, 8.0 mmol, 2.0 eq) at rt. The mixture was stirred at 80°C under N₂ for 16 h. Upon completion, the mixture was cooled to rt, diluted in water, extracted with EtOAc, washed with saturated aqueous NaCl, dried over anhydrous Na₂SO₄, filtered, and concentrated under reduced pressure. The residue was purified by column chromatography to afford the title compound (yellow oil, 150 mg, yield 82%).

### Step D: 1-(3-((2,6-dioxopiperidin-3-yl)amino)phenyl)piperidine-4-carbaldehyde

To 3-((3-(4-(dimethoxymethyl)piperidin-1-yl)phenyl)amino)piperidine-2,6-dione (200 mg, 0.6 mmol, 1.0 eq) was added formic acid (5 mL) at 0°C. The reaction solution was stirred at rt under N₂ for 2 h. Upon completion, the reaction solution was concentrated under reduced pressure to afford the title compound (yellow oil, 150 mg, crude), which was used directly without purification.

### Step E: N-(3-Carbamoyl-1-(4-((1-(3-((2,6-dioxopiperidin-3-yl)amino)phenyl)piperidin-4-yl)methyl)piperazin-1-yl)phenyl)-1H-pyrazol-4-yl)-6-(trifluoromethyl)picolinamide

To a solution of 1-(3-((2,6-dioxopiperidin-3-yl)amino)phenyl)piperidine-4-carbaldehyde (100 mg, 0.3 mmol, 1.0 eq) and N-(3-amino-1-((1r,4r)-4-(piperazin-1-yl)cyclohexyl)-1H-pyrazol-4-yl)-6-(trifluoromethyl)picolinamide (116 mg, 0.3 mmol, 1.0 eq) in DMF (5 mL) was added AcOH (5 drops) at rt. The mixture was stirred at rt for 0.5 h. Sodium triacetoxyborohydride (267 mg, 1.3 mmol, 4.0 eq) was then added to the above reaction solution. The mixture was stirred at rt for 1.5 h. Upon completion, the reaction was quenched with saturated NaHCO₃ solution, extracted with EtOAc (×3), washed with saturated aqueous NaCl, dried over anhydrous Na₂SO₄, filtered, and concentrated under reduced pressure. The residue was purified by HPLC to afford the title compound (white solid, 15 mg, yield 6%). **¹H NMR** (400 MHz, DMSO-*d*₆) δ 11.79 (s, 1H), 10.75 (s, 1H), 8.88 (s, 1H), 8.43 (dd, *J* = 16.8, 7.7 Hz, 2H), 8.21 (dd, *J* = 7.6, 1.2 Hz, 1H), 7.95 (s, 1H), 7.79 (d, *J* = 9.0 Hz, 2H), 7.71 (s, 1H), 7.08 (d, *J* = 9.1 Hz, 2H), 6.90 (t, *J =* 8.1 Hz, 1H), 6.27 (s, 1H), 6.22-6.17 (m, 1H), 6.11 (d, *J* = 7.8 Hz, 1H), 5.60 (d, *J =* 7.5 Hz, 1H), 4.41-4.19 (m, 1H), 3.86-3.53 (m, 2H), 3.22 (s, 6H), 2.80-2.58 (m, 6H), 2.25-2.19 (m, 2H), 2.13-2.07 (m, 1H), 1.90-1.64 (m, 4H), 1.26-1.16 (m, 2H). LCMS (ESI): m/z 759 [M+H]⁺.

### Preparation Example 282. Synthesis of Compound A198

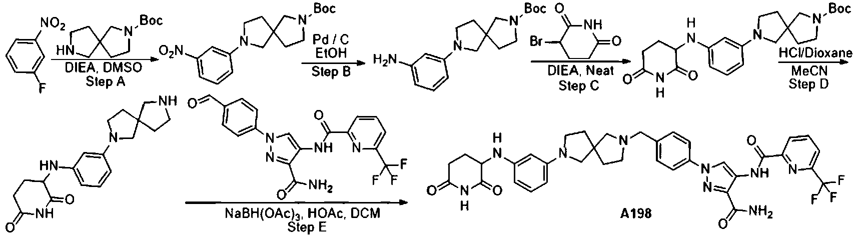

### Step A: Tert-butyl 7-(3-nitrophenyl)-2,7-diazaspiro[4.4]nonane-2-carboxylate

To a solution of 1-fluoro-3-nitrobenzene (374 mg, 2.7 mmol, 1.0 eq) and tert-butyl 2,7-diazaspiro[4.4]nonane-2-carboxylate (500 mg, 2.2 mmol, 1.2 eq) in dimethyl sulfoxide (5 mL) at rt was added DIEA (857 mg, 6.6 mmol, 3.0 eq). The reaction solution was stirred at 120°C under N₂ for 16 h. Upon completion, the reaction solution was cooled to rt, diluted in saturated aqueous NH₄Cl, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (yellow solid, 0.7 g, yield 91%).

### Step B: Tert-butyl 7-(3-aminophenyl)-2,7-diazaspiro[4.4]nonane-2-carboxylate

To a solution of tert-butyl 7-(3-nitrophenyl)-2,7-diazaspiro[4.4]nonane-2-carboxylate (700 mg, 2.0 mmol, 1.0 eq) in ethanol (10 mL) was added 10% palladium on carbon (300 mg) at rt. The reaction solution was purged with H₂ (using a hydrogen balloon) (×3) and stirred under H₂ at rt for 2 h. Upon completion, the mixture was filtered, and the filtrate was concentrated under reduced pressure to afford the title compound (yellow solid, 600 mg, crude), which was used directly without purification.

### Step C: Tert-butyl 7-(3-((2,6-dioxopiperidin-3-yl)amino)phenyl)-2,7-diazaspiro[4.4]nonane-2-carboxylate

To a solution of tert-butyl 7-(3-aminophenyl)-2,7-diazaspiro[4.4]nonane-2-carboxylate (200 mg, 0.6 mmol, 1.0 eq) and 3-bromopiperidine-2,6-dione (181, 0.95 mmol, 1.5 eq) in 1,4-dioxane (2 mL) was added DIEA (0.3 mL, 1.9 mmol, 3.0 eq) at rt. The mixture was stirred at 80°C under N₂ for 12 h. Upon completion, the mixture was cooled to rt, diluted in water, extracted with EtOAc, washed with saturated aqueous NaCl, dried over anhydrous Na₂SO₄, filtered, and concentrated under reduced pressure. The residue was purified by column chromatography to afford the title compound (yellow solid, 100 mg, yield 37%).

### Step D: 3-((3-(2,7-diazaspiro[4.4]nonan-2-yl)phenyl)amino)piperidine-2,6-dione

At 0°C, to a solution of tert-butyl 7-(3-((2,6-dioxopiperidin-3-yl)amino)phenyl)-2,7-diazaspiro[4.4]nonane-2-carboxylate (100 mg, 0.2 mmol, 1.0 eq) in acetonitrile (2 mL) was added 4 M HCl in 1,4-dioxane (1 mL). The reaction solution was stirred at rt under N₂ for 16 h. The reaction solution was concentrated under reduced pressure to afford the title compound (brown solid, 70 mg, crude), which was used directly without purification.

### Step E: N-(3-Carbamoyl-1-(4-((7-(3-((2,6-dioxopiperidin-3-yl)amino)phenyl)-2,7-diazaspiro[4.4]nonan-2-yl)methyl)phenyl)-1H-pyrazol-4-yl)-6-(trifluoromethyl)picolinamide

To a solution of 3-((3-(2,7-diazaspiro[4.4]nonan-2-yl)phenyl)amino)piperidine-2,6-dione (50 mg, 0.2 mmol, 1.0 eq) and N-(3-amino-1-((1r,4r)-4-(piperazin-1-yl)cyclohexyl)-1H-pyrazol-4-yl)-6-(trifluoromethyl)picolinamide (62 mg, 0.2 mmol, 1.0 eq) in DMF (5 mL) was added TEA (0.4 mL, 2.0 mmol, 10.0 eq) at rt. The mixture was stirred at rt for 0.5 h. AcOH (0.2 mL, 0.4 mmol, 2.0 eq) and sodium triacetoxyborohydride (194 mg, 0.4 mmol, 2.0 eq) were then added to the above reaction solution. The mixture was stirred at rt for 1 h. Upon completion, the reaction was quenched with saturated NaHCO₃ solution, extracted with EtOAc (×3), washed with saturated aqueous NaCl, dried over anhydrous Na₂SO₄, filtered, and concentrated under reduced pressure. The residue was purified by HPLC to afford the title compound (white solid, 25 mg, yield 21%). **¹H NMR** (400 MHz, DMSO-*d*₆) δ 11.79 (s, 1H), 10.74 (s, 1H), 9.00 (s, 1H), 8.47-8.36 (m, 2H), 8.25-8.18 (m, 1H), 8.01 (s, 1H), 7.95-7.90 (m, 2H), 7.76 (s, 1H), 7.53-7.46 (m, 2H), 6.87-6.81 (m, 1H), 5.96 (d, *J =* 8.1 Hz, 1H), 5.84 (s, 1H), 5.80 (d, *J =* 8.0 Hz, 1H), 5.55-5.47 (m, 1H), 4.33-4.25 (m, 1H), 3.67 (s, 2H), 3.29-3.18 (m, 4H), 3.18-3.07 (m, 2H), 2.79-2.66 (m, 2H), 2.61-2.59 (m, 1H), 2.56-2.53 (m, 1H), 2.47-2.42 (m, 1H), 2.13- 2.06 (m, 1H), 1.97-1.88 (m, 2H), 1.83-1.78 (m, 2H). LCMS (ESI): m/z 716 [M+H]⁺.

### Preparation Example 283. Synthesis of Compound A199

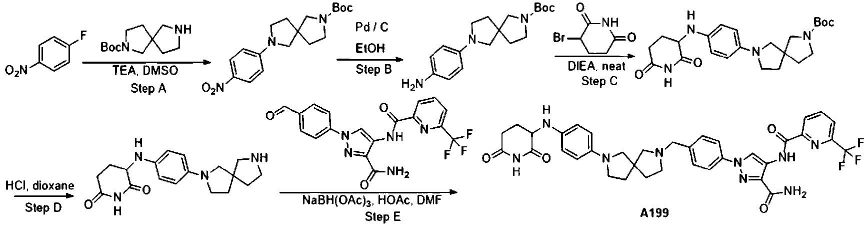

### Step A: Tert-butyl 7-(4-nitrophenyl)-2,7-diazaspiro[4.4]nonane-2-carboxylate

To a solution of 1-fluoro-4-nitrobenzene (0.94 g, 6.6 mmol, 1.5 eq) and tert-butyl 2,7-diazaspiro[4.4]nonane-2-carboxylate (1 g, 4.4 mmol, 1.0 eq) in dimethyl sulfoxide (20 mL) was added TEA (1.8 mL, 13.2 mmol, 3.0 eq) at rt. The reaction solution was stirred at 90°C under N₂ for 3 h. Upon completion, the reaction solution was cooled to rt, diluted in saturated aqueous NH₄Cl, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (yellow solid, 1.4 g, yield 91%).

### Step B: Tert-butyl 7-(4-aminophenyl)-2,7-diazaspiro[4.4]nonane-2-carboxylate

To a solution of tert-butyl 7-(4-nitrophenyl)-2,7-diazaspiro[4.4]nonane-2-carboxylate (1.4 g, 4.0 mmol, 1.0 eq) in ethanol (20 mL) was added 10% palladium on carbon (500 mg) at rt. The reaction solution was purged with H₂ (using a hydrogen balloon) (×3) and stirred under H₂ at rt for 2 h. Upon completion, the mixture was filtered, and the filtrate was concentrated under reduced pressure to afford the title compound (yellow solid, 1.2 g, crude), which was used directly without purification.

### Step C: Tert-butyl 7-(4-((2,6-dioxopiperidin-3-yl)amino)phenyl)-2,7-diazaspiro[4.4]nonane-2-carboxylate

To a solution of tert-butyl 7-(4-aminophenyl)-2,7-diazaspiro[4.4]nonane-2-carboxylate (1.0 g, 3.2 mmol, 1.0 eq) and 3-bromopiperidine-2,6-dione (1.21 g, 6.3 mmol, 2.0 eq) in 1,4-dioxane (10 mL) was added DIEA (0.56 mL, 6.3 mmol, 2.0 eq) at rt. The mixture was stirred at 80°C under N₂ for 16 h. Upon completion, the mixture was cooled to rt, diluted in water, extracted with EtOAc, washed with saturated aqueous NaCl, dried over anhydrous Na₂SO₄, filtered, and concentrated under reduced pressure. The residue was purified by column chromatography to afford the title compound (yellow solid, 500 mg, yield 37%).

### Step D: 3-((4-(2,7-diazaspiro[4.4]nonan-2-yl)phenyl)amino)piperidine-2,6-dione

At 0°C, to a solution of tert-butyl 7-(4-((2,6-dioxopiperidin-3-yl)amino)phenyl)-2,7-diazaspiro[4.4]nonane-2-carboxylate (500 mg, 1.2 mmol, 1.0 eq) in DCM (4 mL) was added 4 M HCl in 1,4-dioxane (1 mL). The reaction solution was stirred at rt under N₂ for 1 h. The reaction solution was concentrated under reduced pressure to afford the title compound (blue solid, 300 mg, crude), which was used directly without purification.

### Step E: N-(3-Carbamoyl-1-(4-(7-(4-((2,6-dioxopiperidin-3-yl)amino)phenyl)-2,7-diazaspiro[4.4]nonan-2-yl)methyl)phenyl)-1H-pyrazol-4-yl)-6-(trifluoromethyl)picolinamide

To a solution of 3-((4-(2,7-diazaspiro[4.4]nonan-2-yl)phenyl)amino)piperidine-2,6-dione (300 mg, 0.9 mmol, 1.0 eq) and N-(3-amino-1-((1r,4r)-4-(piperazin-1-yl)cyclohexyl)-1H-pyrazol-4-yl)-6-(trifluoromethyl)picolinamide (368 mg, 0.9 mmol, 1.0 eq) in DMF (5 mL) was added TEA (0.057 mL, 0.4 mmol, 2.0 eq) at rt, and the mixture was stirred for 10 min. AcOH (0.2 mL, 1 mmol, 5.0 eq) was added, and the mixture was stirred at rt for 20 min. Sodium triacetoxyborohydride (578 mg, 2.7 mmol, 3.0 eq) was then added to the above reaction solution. The mixture was stirred at rt for 16 h. Upon completion, the reaction was quenched with saturated NaHCO₃ solution, extracted with EtOAc (×3), washed with saturated aqueous NaCl, dried over anhydrous Na₂SO₄, filtered, and concentrated under reduced pressure. The residue was purified by HPLC to afford the title compound (blue solid, 16.5 mg, yield 2%). **¹H NMR** (400 MHz, DMSO-*d*₆) δ 11.79 (s, 1H), 10.73 (s, 1H), 9.00 (s, 1H), 8.42 (dd, *J* = 16.7, 9.0 Hz, 2H), 8.22 (d, *J* = 7.7 Hz, 1H), 8.15 (s, 1H), 8.02 (s, 1H), 7.93 (d, *J =* 8.4 Hz, 2H), 7.77 (s, 1H), 7.50 (d, *J =* 8.3 Hz, 2H), 6.62 (d, *J =* 8.4 Hz, 2H), 6.37 (d, *J* = 8.4 Hz, 2H), 5.04 (s, 1H), 4.11 (d, *J* = 5.8 Hz, 1H), 3.68 (s, 2H), 3.15 (d, *J =* 8.3 Hz, 2H), 3.07 (d, *J* = 8.8 Hz, 2H), 2.76-2.55 (m, 6H), 2.15-2.08 (m, 1H), 1.97-1.88 (m, 2H), 1.86-1.77 (m, 3H). LCMS (ESI): m/z 716 [M+H]⁺.

### Preparation Example 284. Synthesis of Compound A200

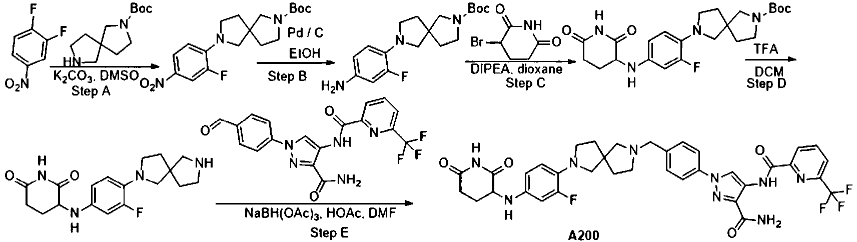

### Step A: Tert-butyl 7-(2-fluoro-4-nitrophenyl)-2,7-diazaspiro[4.4]nonane-2-carboxylate

Potassium carbonate (1.71 g, 12.4 mmol, 2.0 eq) was added to a solution of 1,2-difluoro-4-nitrobenzene (1.48 g, 9.3 mmol, 1.5 eq) and tert-butyl 2,7-diazaspiro[4.4]nonane-2-carboxylate (1.4 g, 6.2 mmol, 1.0 eq) in dimethyl sulfoxide (20 mL) at rt. The reaction solution was stirred at 100°C under N₂ for 16 h. Upon completion, the reaction solution was cooled to rt, diluted in saturated aqueous NH₄Cl, and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (yellow solid, 1.8 g, yield 79%).

### Step B: Tert-butyl 7-(4-amino-2-fluorophenyl)-2,7-diazaspiro[4.4]nonane-2-carboxylate

To a solution of tert-butyl 7-(2-fluoro-4-nitrophenyl)-2,7-diazaspiro[4.4]nonane-2-carboxylate (500 mg, 1.4 mmol, 1.0 eq) in ethanol (10 mL) was added 10% palladium on carbon (100 mg) at rt. The reaction solution was purged with H₂ (using a hydrogen balloon) (×3) and stirred under H₂ at rt for 4 h. Upon completion, the mixture was filtered, and the filtrate was concentrated under reduced pressure to afford the title compound (yellow solid, 0.5 g, crude), which was used directly without purification.

### Step C: Tert-butyl 7-(4-((2,6-dioxopiperidin-3-yl)amino)-2-fluorophenyl)-2,7-diazaspiro[4.4]nonane-2-carboxylate

To a solution of tert-butyl 7-(4-amino-2-fluorophenyl)-2,7-diazaspiro[4.4]nonane-2-carboxylate (500 mg, 1.5 mmol, 1.0 eq) and 3-bromopiperidine-2,6-dione (571 mg, 3 mmol, 2.0 eq) in 1,4-dioxane (5 mL) was added DIPEA (0.5 mL, 3 mmol, 2.0 eq) at rt. The mixture was stirred at 80°C under N₂ for 16 h. Upon completion, the mixture was cooled to rt, diluted in water, extracted with EtOAc, washed with saturated aqueous NaCl, dried over anhydrous Na₂SO₄, filtered, and concentrated under reduced pressure. The residue was purified by column chromatography to afford the title compound (gray solid, 250 mg, yield 38%).

### Step D: 3-((3-Fluoro-4-(2,7-diazaspiro[4.4]nonan-2-yl)phenyl)amino)piperidine-2,6-dione

To a solution of tert-butyl 7-(4-((2,6-dioxopiperidin-3-yl)amino)-2-fluorophenyl)-2,7-diazaspiro[4.4]nonane-2-carboxylate (250 mg, 0.56 mmol, 1.0 eq) in DCM (6 mL) was added TFA (2 mL) at 0°C. The reaction solution was stirred at rt under N₂ for 1 h. The reaction solution was concentrated under reduced pressure to afford the title compound (brown solid, 188 mg, crude), which was used directly without purification.

### Step E: N-(3-amino-1-(4-(7-(4-((2,6-dioxopiperidin-3-yl)amino)-2-fluorophenyl)-2,7-diazaspiro[4.4]nonan-2-yl)methyl)phenyl)-1H-pyrazol-4-yl)-6-(trifluoromethyl)picolinamide

To a solution of 3-((3-fluoro-4-(2,7-diazaspiro[4.4]nonan-2-yl)phenyl)amino)piperidine-2,6-dione (100 mg, 0.29 mmol, 1.0 eq) and N-(3-amino-1-((1r,4r)-4-(piperazin-1-yl)cyclohexyl)-1H-pyrazol-4-yl)-6-(trifluoromethyl)picolinamide (116 mg, 0.29 mmol, 1.0 eq) in DMF (5 mL) was added TEA (a few drops), and the mixture was stirred for 10 min at rt. AcOH (0.017 mL, 0.29 mmol, 1.0 eq) was added, and the mixture was stirred at rt for 1 h. Sodium triacetoxyborohydride (122 mg, 0.58 mmol, 2.0 eq) was then added to the above reaction solution. The mixture was stirred at rt for 2 h. Upon completion, the reaction was quenched with saturated NaHCO₃ solution, extracted with EtOAc (×3), washed with saturated aqueous NaCl, dried over anhydrous Na₂SO₄, filtered, and concentrated under reduced pressure. The residue was purified by HPLC to afford the title compound (purple solid, 15.5 mg, yield 7%). **¹H NMR** (400 MHz, DMSO-*d*₆) δ 11.79 (s, 1H), 10.75 (s, 1H), 9.00 (s, 1H), 8.47-8.38 (m, 2H), 8.22 (d, *J =* 8.4 Hz, 1H), 8.01 (s, 1H), 7.93 (d, *J* = 8.5 Hz, 2H), 7.76 (s, 1H), 7.48 (d, *J =* 8.5 Hz, 2H), 6.60-6.47 (m, 2H), 6.41-6.35 (m, 1H), 5.51 (d, *J =* 7.7 Hz, 1H), 4.23-4.16 (m, 1H), 3.65 (s, 2H), 3.19-3.14 (m, 3H), 3.08-3.05 (m, 1H), 2.74-2.56 (m, 5H), 2.45-2.41 (m, 1H), 2.13-2.05 (m, 1H), 1.92-1.77 (m, 5H). LCMS (ESI): m/z 734 [M+H]⁺.

### Preparation Example 285. Synthesis of Compound A201

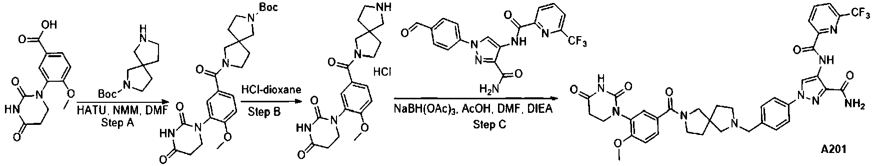

### Step A: Tert-butyl 7-(3-(2,4-dioxotetrahydropyrimidin-1(2H)-yl)-4-methoxybenzoyl)-2,7-diazaspiro[4.4]nonane-2-carboxylate

To a solution of 3-(2,4-dioxotetrahydropyrimidin-1(2H)-yl)-4-methoxybenzoic acid (200 mg, 0.8 mmol, 1.0 eq) and 2-methylpropan-2-yl 2,7-diazaspiro[4.4]nonane-2-carboxylate (205.6 mg, 0.9 mmol, 1.2 eq) in DMF (10 mL) at rt were added N-methylmorpholine (0.42 mL, 3.8 mmol, 5.0 eq) and HATU (431.7 mg, 1.1 mmol, 1.5 eq). The reaction solution was stirred at rt under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (pink solid, 238 mg, yield 66%).

### Step B: 1-(2-methoxy-5-(2,7-diazaspiro[4.4]nonane-2-carbonyl)phenyl)dihydropyrimidine-2,4(1H,3H)-dione

At 0°C, to a solution of tert-butyl 7-(3-(2,4-dioxotetrahydropyrimidin-1(2H)-yl)-4-methoxybenzoyl)-2,7-diazaspiro[4.4]nonane-2-carboxylate (300 mg, 0.6 mmol, 1.0 eq) in acetonitrile (5 mL) was added 4 M HCl in 1,4-dioxane (5 mL, 20 mmol, 33 eq). The reaction solution was stirred at rt under N₂ for 1 h. The filtrate was concentrated under reduced pressure to afford the title compound (yellow oil, 200 mg, yield 84%, crude), which was used directly without purification.

### Step C: N-(3-amino-1-(4-((7-(3-(2,4-dioxotetrahydropyrimidin-1(2H)-yl)-4-methoxybenzoyl)-2,7-diazaspiro[4.4]nonan-2-yl)methyl)phenyl)-1H-pyrazol-4-yl)-6-(trifluoromethyl)picolinamide

At rt, to a solution of 1-(2-methoxy-5-(2,7-diazaspiro[4.4]nonane-2-carbonyl)phenyl)dihydropyrimidine-2,4(1H,3H)-dione (200 mg, 0.5 mmol, 1.0 eq) in DMF (6 mL) was added DIEA (0.1 mL), and the mixture was stirred for 5 min, followed by the addition of AcOH (0.2 mL) and N-(3-carbamoyl-1-(4-formylphenyl)-1H-pyrazol-4-yl)-6-(trifluoromethyl)picolinamide (216.6 mg, 0.5 mmol, 1.0 eq). The mixture was stirred at rt for 15 h. Sodium triacetoxyborohydride (113.3 mg, 0.5 mmol, 1.0 eq) was then added to the above reaction solution. The mixture was stirred at rt for 1 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (yellow solid, 26.8 mg, yield 7%). **¹H NMR** (400 MHz, DMSO-*d*₆) δ 11.80 (s, 1H), 10.32 (d, *J =* 7.2 Hz, 1H), 9.01 (s, 1H), 8.47 (t, *J =* 7.2 Hz, 1H), 8.40 (t, *J =* 7.6 Hz, 1H), 8.22 (d, *J* = 7.6 Hz, 1H), 8.15 (s, 1H), 8.01 (s, 1H), 7.95 (d, *J* = 8.0 Hz, 1H), 7.90 (d, *J =* 8.4 Hz, 1H), 7.77 (s, 1H), 7.51 (dd, *J =* 18.8, 9.6 Hz, 3H), 7.40 (d, *J=* 8.4 Hz, 1H), 7.14 (dd, *J =* 12.8, 8.8 Hz, 1H), 3.85 (d, *J =* 11.2 Hz, 3H), 3.63 (dd, *J =* 20.2, 13.6 Hz, 4H), 3.49 (s, 2H), 3.40 (dd, *J =* 10.8, 6.8 Hz, 3H), 2.72-2.56 (m, 4H), 2.45-2.27 (m, 1H), 1.93-1.61 (m, 4H). LCMS (ESI): m/z 760 [M+H]⁺.

### Preparation Example 286. Synthesis of Compound A202

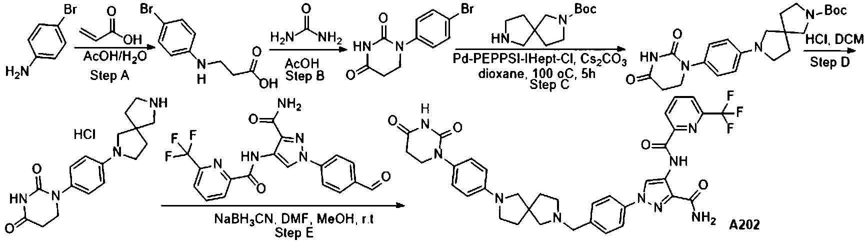

### Step A: 3-((4-bromophenyl)amino)propanoic acid

Acrylic acid (1.4 mL, 20 mmol, 1.0 eq) was added to a solution of 4-bromoaniline (15.0 g, 87.2 mmol, 1.0 eq) in water (250 mL) and AcOH (50 mL) at rt. The mixture was stirred at 80°C under N₂ for 18 h. Upon completion, the reaction solution was cooled to rt, poured into water, and extracted with EtOAc (×3). The combined organic phases were washed with water and saturated aqueous NaCl, dried over anhydrous Na₂SO₄, and concentrated under reduced pressure to afford the title compound (yellow oil, 21 g, crude), which was used directly without purification.

### Step B: 1-(4-bromophenyl)dihydropyrimidine-2,4(1H,3H)-dione

To a solution of 3-((4-bromophenyl)amino)propanoic acid (21.0 g, 86.1 mmol, 1.0 eq) in AcOH (250 mL) was added urea (10.3 g, 172.1 mmol, 2.0 eq) at rt. The mixture was stirred at 120°C under N₂ for 18 h. Upon completion, the reaction solution was cooled to rt, poured into water (200 mL), and stirred for 2 h. The mixture was filtered, and the filter cake was washed twice with water, and dried completely to yield the title compound (gray solid, 17 g, yield 74%, crude).

### Step C: Tert-butyl 7-(4-(2,4-dioxotetrahydropyrimidin-1(2H)-yl)phenyl)-2,7-diazaspiro[4.4]nonane-2-carboxylate

To a solution of 1-(4-bromophenyl)dihydropyrimidine-2,4(1H,3H)-dione (300 mg, 1.1 mmol, 1.0 eq) and 2-methylpropan-2-yl 2,7-diazaspiro[4.4]nonane-2-carboxylate (277.5 mg, 1.2 mmol, 1.1 eq) in 1,4-dioxane (5 mL) were added cesium carbonate (1.1 g, 3.3 mmol, 3.0 eq) and Pd-PEPPSI-Ihept-Cl (54.22 mg, 0.05 mmol, 0.05 eq) at rt. The mixture was stirred at 100°C under N₂ for 5 h. Upon completion, the mixture was cooled to rt, diluted in water, extracted with EtOAc, washed with saturated aqueous NaCl, dried over anhydrous Na₂SO₄, and concentrated under reduced pressure. The residue was purified by column chromatography to afford the title compound (yellow solid, 220 mg, yield 48%).

### Step D: 1-(4-(2,7-diazaspiro[4.4]non-2-yl)phenyl)dihydropyrimidine-2,4(1H,3H)-dione hydrochloride

At 0°C, to a solution of tert-butyl 7-(4-(2,4-dioxotetrahydropyrimidin-1(2H)-yl)phenyl)-2,7-diazaspiro[4.4]nonane-2-carboxylate (220 mg, 0.5 mmol, 1.0 eq) in DCM (5 mL) was added 4 M HCl in MeOH (1 mL, 4.0 mmol, 8.0 eq). The reaction solution was stirred at rt under N₂ for 1 h. The filtrate was concentrated under reduced pressure to afford the title compound (yellow solid, 160 mg, crude), which was used directly without purification.

### Step E: N-(3-amino-1-(4-(7-(4-(2,4-dioxotetrahydropyrimidin-1(2H)-yl)phenyl)-2,7-diazaspiro[4.4]nonan-2-yl)methyl)phenyl)-1H-pyrazol-4-yl)-6-(trifluoromethyl)picolinamide

To a solution of 1-(4-(2,7-diazaspiro[4.4]nonan-2-yl)phenyl)dihydropyrimidine-2,4(1H,3H)-dione hydrochloride (160 mg, 0.5 mmol, 1.0 eq) in DMF (10 mL) and MeOH (1 mL) at rt were added N-(3-carbamoyl-1-(4-formylphenyl)-1H-pyrazol-4-yl)-6-(trifluoromethyl)picolinamide (246.3 mg, 0.6 mmol, 1.1 eq) and sodium cyanoborohydride (64.0 mg, 1.0 mmol, 2.0 eq). The mixture was stirred at rt for 16 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (white solid, 33 mg, yield 9%). **¹H NMR** (400 MHz, DMSO-*d*₆) δ 11.79 (s, 1H), 10.19 (s, 1H), 9.00 (s, 1H), 8.50-8.37 (m, 2H), 8.22 (d, *J =* 6.9 Hz, 1H), 8.01 (s, 1H), 7.93 (d, *J* = 8.6 Hz, 2H), 7.76 (s, 1H), 7.49 (d, *J =* 8.6 Hz, 2H), 7.07 (d, *J =* 8.7 Hz, 2H), 6.48 (d, *J =* 8.9 Hz, 2H), 3.65 (t, *J* = 5.5 Hz, 4H), 3.21 (dd, *J* = 34.4, 9.1 Hz, 4H), 2.67 (d, *J* = 6.6 Hz, 2H), 2.57 (dd, *J* = 23.6, 8.2 Hz, 3H), 2.45 (d, *J =* 8.9 Hz, 1H), 2.04-1.90 (m, 2H), 1.82 (s, 2H). LCMS (ESI): m/z 702 [M+H]⁺.

### Preparation Example 287. Synthesis of Compound A203

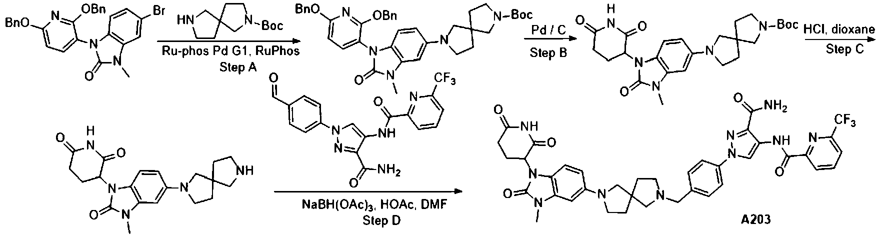

### Step A: Tert-butyl 7-(1-(2,6-bis(benzyloxy)pyridin-3-yl)-3-methyl-2-oxo-2,3-dihydro-1H-benzo[d]imidazol-5-yl)-2,7-diazaspiro [4.4]nonane-2-carboxylate

To a solution of 1-(2,6-bis(benzyloxy)pyridin-3-yl)-5-bromo-3-methyl-1,3-dihydro-2H-benzo[d]imidazol-2-one (400 mg, 0.78 mmol, 1.0 eq) and tert-butyl 2,7-diazaspiro[4.4]nonane-2-carboxylate (272 mg, 1.2 mmol, 1.5 eq) in 1,4-dioxane (10 mL) were added cesium carbonate (760 mg, 2.34 mmol, 3.0 eq), Ru-Phos (72 mg, 0.14 mmol, 0.2 eq), and Ru-Phos Pd G1 (127 mg, 0.14 mmol, 0.2 eq) at rt. The mixture was stirred at 100°C under N₂ for 16 h. Upon completion, the mixture was cooled to rt, diluted in water, extracted with EtOAc, washed with saturated aqueous NaCl, dried over anhydrous Na₂SO₄, filtered, and concentrated under reduced pressure. The residue was purified by column chromatography to afford the title compound (yellow solid, 330 mg, yield 66%).

### Step B: Tert-butyl 7-(1-(2,6-dioxopiperidin-3-yl)-3-methyl-2-oxo-2,3-dihydro-1H-benzo[d]imidazol-5-yl)-2,7-diazaspiro[4.4]nonane-2-carboxylate

To a solution of tert-butyl 7-(1-(2,6-bis(benzyloxy)pyridin-3-yl)-3-methyl-2-oxo-2,3-dihydro-1H-benzo[d]imidazol-5-yl)-2,7-diazaspiro[4.4]nonane-2-carboxylate (330 mg, 0.50 mmol, 1.0 eq) in ethanol (10 mL) was added 10% palladium on carbon (300 mg) at rt. The reaction solution was purged with H₂ (using a hydrogen balloon) (×3) and stirred under H₂ at 50°C for 2 h. Upon completion, the mixture was filtered, and the filtrate was concentrated under reduced pressure to afford the title compound (white solid, 200 mg, crude), which was used directly without purification.

### Step C: 3-(3-methyl-2-oxo-5-(2,7-diazaspiro[4.4]nonan-2-yl)-2,3-dihydro-1H-benzo[d]imidazol-1-yl)piperidine-2,6-dione

To a solution of tert-butyl 7-(1-(2,6-dioxopiperidin-3-yl)-3-methyl-2-oxo-2,3-dihydro-1H-benzo[d]imidazol-5-yl)-2,7-diazaspiro[4.4]nonane-2-carboxylate (100 mg, 0.2 mmol, 1.0 eq) in acetonitrile (5 mL) was added 4 M HCl in 1,4-dioxane (1 mL) at 0°C. The reaction solution was stirred at rt under N₂ for 16 h. The reaction solution was concentrated under reduced pressure to afford the title compound (yellow solid, 79 mg, crude), which was used directly without purification.

### Step D: N-(3-carbamoyl-1-(4-((7-(1-(2,6-dioxopiperidin-3-yl)-3-methyl-2-oxo-2,3-dihydro-1H-benzo[d]imidazol-5-yl)-2,7-diazaspiro[4.4]nonan-2-yl)methyl)phenyl)-1H-pyrazol-4-yl)-6-(trifluoromethyl)picolinamide

To a solution of 3-(3-methyl-2-oxo-5-(2,7-diazaspiro[4.4]nonan-2-yl)-2,3-dihydro-1H-benzo[d]imidazol-1-yl)piperidine-2,6-dione (79 mg, 0.2 mmol, 1.0 eq) and N-(3-amino-1-((1r,4r)-4-(piperazin-1-yl)cyclohexyl)-1H-pyrazol-4-yl)-6-(trifluoromethyl)picolinamide (82 mg, 0.2 mmol, 1.0 eq) in DMF (5 mL) was added TEA (0.057 mL, 0.4 mmol, 2.0 eq) at rt, and the mixture was stirred for 10 min. AcOH (0.2 mL, 1 mmol, 5.0 eq) was added, and the mixture was stirred at rt for 20 min. Sodium triacetoxyborohydride (258 mg, 1.2 mmol, 6.0 eq) was then added to the above reaction solution. The mixture was stirred at rt for 1 h. Upon completion, the reaction was quenched with saturated NaHCO₃ solution, extracted with EtOAc (×3), washed with saturated aqueous NaCl, dried over anhydrous Na₂SO₄, filtered, and concentrated under reduced pressure. The residue was purified by HPLC to afford the title compound (white solid, 37.2 mg, yield 24%). **¹H NMR** (400 MHz, DMSO-*d*₆) δ 11.79 (s, 1H), 11.03 (s, 1H), 9.00 (s, 1H), 8.50-8.32 (m, 2H), 8.21 (d, *J =* 6.7 Hz, 1H), 8.01 (s, 1H), 7.93 (d, *J* = 8.6 Hz, 2H), 7.76 (s, 1H), 7.49 (d, *J =* 8.5 Hz, 2H), 6.88 (d, *J =* 8.5 Hz, 1H), 6.36 (d, *J =* 2.0 Hz, 1H), 6.19 (dd, *J =* 8.6, 2.0 Hz, 1H), 5.25 (dd, *J =* 12.8, 5.3 Hz, 1H), 3.67 (s, 2H), 3.28 (d, *J =* 5.7 Hz, 3H), 3.27-3.15 (m, 4H), 2.93-2.82 (m, 1H), 2.73-2.54 (m, 5H), 2.48-2.45 (m, 1H), 2.03-1.91 (m, 3H), 1.86-1.79 (m, 2H). LCMS (ESI): m/z 771 [M+H]⁺.

### Preparation Example 288. Synthesis of Compound A204

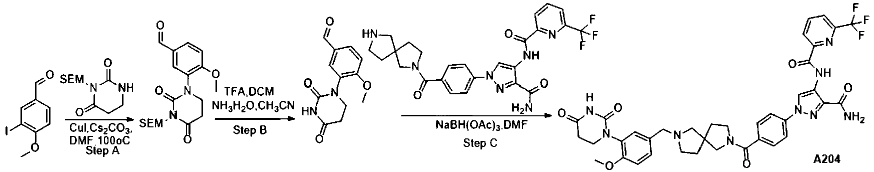

### Step A: 3-(2,4-dioxo-3-((2-(trimethylsilyl)ethoxy)methyl)tetrahydropyrimidin-1(2H)-yl)-4-methoxybenzaldehyde

To a solution of 3-iodo-4-methoxybenzaldehyde (1000 mg, 4.650 mmol, 1.0 eq) in DMF (10 mL) at rt were added 3-((2-(trimethylsilyl)ethoxy)methyl)dihydropyrimidine-2,4(1H,3H)-dione (1249.97 mg, 5.115 mmol, 1.2 eq), cesium carbonate (3030.18 mg, 9.300 mmol, 2.0 eq), cuprous iodide (88.35 mg, 0.465 mmol, 0.1 eq), and (1R,2R)-(-)-1,2-diaminocyclohexane (106.20 mg, 0.930 mmol, 0.2 eq). The reaction solution was stirred at 100°C under N₂ for 16 h. Upon completion, the reaction solution was cooled to rt and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (yellow oil, 300 mg, yield 17%).

### Step B: 3-(2,4-dioxotetrahydropyrimidin-1(2H)-yl)-4-methoxybenzaldehyde

At 0°C, to a solution of 3-(2,4-dioxo-3-((2-(trimethylsilyl)ethoxy)methyl)tetrahydropyrimidin-1(2H)-yl)-4-methoxybenzaldehyde (300 mg, 0.793 mmol, 1.0 eq) in DCM (10 mL) was added TFA (0.061 mL, 0.793 mmol, 1.0 eq). The reaction solution was stirred at rt under N₂ for 0.5 h. Upon completion, the mixture was concentrated under reduced pressure. The residue was dissolved in acetonitrile (100 mL), aqueous ammonia (3 mL) was added, and the mixture was stirred at rt for 0.5 h. The mixture was diluted in saturated ammonium chloride and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (yellow oil, 121 mg, yield 62%).

### Step C: N-(3-carbonyl-1-(4-(7-(3-(2,4-dioxotetrahydropyrimidin-1(2H)-yl)-4-methoxybenzyl)-2,7-diazaspiro[4.4]nonane-2-carbonyl)phenyl)-1H-pyrazol-4-yl)-6-(trifluoromethyl)picolinamide

A solution of 3-(2,4-dioxotetrahydropyrimidin-1(2H)-yl)-4-methoxybenzaldehyde (120 mg, 0.483 mmol, 1.0 eq) and N-(1-(4-(2,7-diazaspiro[4.4]nonane-2-carbonyl)phenyl)-3-formyl-1H-pyrazol-4-yl)-6-(trifluoromethyl)picolinamide (267.75 mg, 0.508 mmol, 1.2 eq) in DMF (5 mL) was stirred at rt for 30 min. Sodium triacetoxyborohydride (307.36 mg, 1.450 mmol, 3.0 eq) was then added to the above reaction solution. The mixture was stirred at rt for 2 h. Upon completion, the reaction was quenched with saturated NaHCO₃ solution, extracted with EtOAc (×3), washed with saturated aqueous NaCl, dried over anhydrous Na₂SO₄, filtered, and concentrated under reduced pressure. The residue was purified by HPLC to afford the title compound (white solid, 40 mg, yield 11%). **¹H NMR** (400 MHz, DMSO-*d*₆) δ 11.81 (s, 1H), 10.28 (s, 1H), 9.10 (d, *J* = 4.8 Hz, 1H), 8.48-8.45 (m, 1H), 8.41 (t, *J* = 7.6 Hz, 1H), 8.22 (d, *J* = 7.6 Hz, 1H), 8.08-8.04 (m, 3H), 7.82 (d, *J* = 7.6 Hz, 1H), 7.72-7.65 (m, 2H), 7.28-7.00 (m, 3H), 3.78 (d, *J* = 9.6 Hz, 3H), 3.58-3.46 (m, 8H), 2.66-2.54 (m, 4H), 2.44-2.30 (m, 2H), 1.94-1.67 (m, 4H). LCMS (ESI) m/z: 760 [M+H]⁺.

### Preparation Example 289. Synthesis of Compound A205

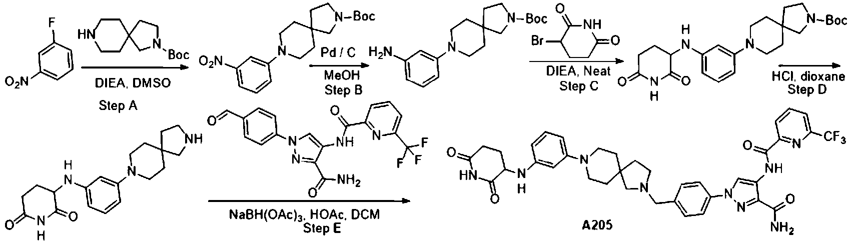

### Step A: Tert-butyl 8-(3-nitrophenyl)-2,8-diazaspiro[4.5]decane-2-carboxylate

To a solution of 1-fluoro-3-nitrobenzene (352 mg, 2.5 mmol, 1.2 eq) and 2-methylpropan-2-yl 2,8-diazaspiro[4.5]decane-2-carboxylate (500 mg, 2.1 mmol, 1.0 eq) in dimethyl sulfoxide (10 mL) was added DIEA (1.1 mL, 6.2 mmol, 3.0 eq) at rt. The reaction solution was stirred at 100°C under N₂ for 16 h. Upon completion, the reaction solution was cooled to rt and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (yellow solid, 190 mg, yield 25%).

### Step B: Tert-butyl 8-(3-aminophenyl)-2,8-diazaspiro[4.5]decane-2-carboxylate

To a solution of tert-butyl 8-(3-nitrophenyl)-2,8-diazaspiro[4.5]decane-2-carboxylate (190 mg, 0.5 mmol, 1.0 eq) in MeOH (10 mL) was added 10% palladium on carbon (40 mg) at rt. The reaction solution was purged with H₂ (using a hydrogen balloon) (×3) and stirred under H₂ at rt for 2 h. Upon completion, the mixture was filtered, and the filtrate was concentrated under reduced pressure to give the title compound (white solid, 160 mg, crude), which was used directly without purification.

### Step C: Tert-butyl 8-(3-((2,6-dioxopiperidin-3-yl)amino)phenyl)-2,8-diazaspiro[4.5]decane-2-carboxylate

To a solution of tert-butyl 8-(3-aminophenyl)-2,8-diazaspiro[4.5]decane-2-carboxylate (160 mg, 0.5 mmol, 1.0 eq) in 1,4-dioxane (0.3 mL) at rt were added 3-bromopiperidine-2,6-dione (185 mg, 1 mmol, 2.0 eq) and DIEA (0.2 mL, 2 mmol, 2.0 eq). The reaction solution was stirred at 100°C under N₂ for 16 h. Upon completion, the reaction solution was cooled to rt and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (white solid, 90 mg, yield 42%).

### Step D: 3-((3-(2,8-diazaspiro[4.5]dec-8-yl)phenyl)amino)piperidine-2,6-dione

At 0°C, to a solution of tert-butyl 8-(3-((2,6-dioxopiperidin-3-yl)amino)phenyl)-2,8-diazaspiro[4.5]decane-2-carboxylate (90 mg, 0.2 mmol, 1.0 eq) in acetonitrile (5 mL) was added 4 M HCl in 1,4-dioxane (1 mL). The reaction solution was stirred at rt under N₂ for 1 h. The reaction solution was concentrated under reduced pressure to afford the title compound (yellow solid, 70 mg, crude), which was used directly without purification.

### Step E: N-(3-Carbonyl-1-(4-((8-(3-((2,6-dioxopiperidin-3-yl)amino)phenyl)-2,8-diazaspiro[4.5]dec-2-yl)methyl)phenyl)-1H-pyrazol-4-yl)-6-(trifluoromethyl)picolinamide

To a solution of 3-((3-(2,8-diazaspiro[4.5]dec-8-yl)phenyl)amino)piperidine-2,6-dione (70 mg, 0.2 mmol, 1.0 eq) and 1-(4-formylphenyl)-4-({[6-(trifluoromethyl)pyridin-2-yl]carbonyl}amino)pyrazole-3-carboxamide (82 mg, 0.2 mmol, 1.0 eq) in DMF (5 mL) was added TEA (0.057 mL, 0.4 mmol, 2.0 eq) at rt, and the mixture was stirred for 10 min. AcOH (0.2 mL, 1 mmol, 5.0 eq) was then added. The mixture was stirred at rt for 20 min. Sodium triacetoxyborohydride (258 mg, 1.2 mmol, 6.0 eq) was then added to the above reaction solution. The mixture was stirred at rt for 1 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (white solid, 44.6 mg, yield 30%). **¹H NMR** (400 MHz, DMSO-*d*₆) δ 11.79 (s, 1H), 10.74 (s, 1H), 9.00 (s, 1H), 8.48-8.37 (m, 2H), 8.18 (dd, *J =* 25.4, 5.0 Hz, 2H), 8.01 (s, 1H), 7.94 (d, *J =* 8.4 Hz, 2H), 7.77 (s, 1H), 7.49 (d, *J* = 8.3 Hz, 2H), 6.88 (t, *J =* 8.0 Hz, 1H), 6.26 (s, 1H), 6.18 (d, *J* = 8.2 Hz, 1H), 6.10 (d, *J =* 7.8 Hz, 1H), 5.58 (d, *J =* 7.6 Hz, 1H), 4.33-4.26 (m, 1H), 3.67-3.63 (m, 2H), 3.07-2.98 (m, 4H), 2.77-2.68 (m, 1H), 2.64-2.54 (m, 3H), 2.45-2.40 (m, 2H), 2.12-2.04 (m, 1H), 1.90-1.79 (m, 1H), 1.68-1.57 (m, 6H). LCMS (ESI): m/z 730 [M+H]⁺.

### Preparation Example 290. Synthesis of Compound A206

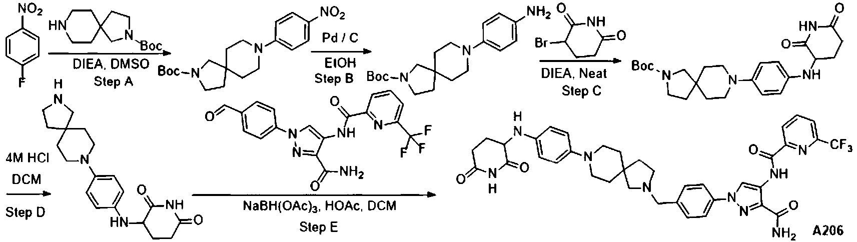

### Step A: Tert-butyl 8-(4-nitrophenyl)-2,8-diazaspiro[4.5]decane-2-carboxylate

To a solution of tert-butyl 2,8-diazaspiro[4.5]decane-2-carboxylate (500 mg, 2.1 mmol, 1 eq) and 1-fluoro-4-nitrobenzene (440 mg, 3.1 mmol, 1.5 eq) in dimethyl sulfoxide (5 mL) was added DIEA (1.1 mL, 6.2 mmol, 3 eq) at rt. The reaction solution was stirred at 120°C under N₂ for 16 h. Upon completion, the reaction solution was cooled to rt and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (yellow solid, 670 mg, yield 89%).

### Step B: Tert-butyl 8-(4-aminophenyl)-2,8-diazaspiro[4.5]decane-2-carboxylate

To a solution of tert-butyl 8-(4-nitrophenyl)-2,8-diazaspiro[4.5]decane-2-carboxylate (670 mg, 1.8 mmol, 1.0 eq) in ethanol (15 mL) was added 10% palladium on carbon (118 mg) at rt. The reaction solution was purged with H₂ (using a hydrogen balloon) (×3) and stirred under H₂ at rt for 2 h. Upon completion, the mixture was filtered, and the filtrate was concentrated under reduced pressure to give the title compound (yellow solid, 580 mg, crude), which was used directly without purification.

### Step C: Tert-butyl 8-(4-((2,6-dioxopiperidin-3-yl)amino)phenyl)-2,8-diazaspiro[4.5]decane-2-carboxylate

To a solution of tert-butyl 8-(4-aminophenyl)-2,8-diazaspiro[4.5]decane-2-carboxylate (580 mg, 1.7 mmol, 1.0 eq) in 1,4-dioxane (4.0 mL) at rt were added 3-bromopiperidine-2,6-dione (672 mg, 3.5 mmol, 2.0 eq) and DIEA (610 µL, 3.50 mmol, 2.0 eq). The reaction solution was stirred at 85°C under N₂ for 16 h. Upon completion, the reaction solution was cooled to rt and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (brown solid, 300 mg, yield 38%).

### Step D: 3-((4-(2,8-diazaspiro[4.5]dec-8-yl)phenyl)amino)piperidine-2,6-dione

At 0°C, to a solution of tert-butyl 8-(4-((2,6-dioxopiperidin-3-yl)amino)phenyl)-2,8-diazaspiro[4.5]decane-2-carboxylate (300 mg, 678 µmol, 1.0 eq) in DCM (5 mL) was added 4 M HCl in 1,4-dioxane (4 mL). The reaction solution was stirred at rt under N₂ for 1 h. The reaction solution was concentrated under reduced pressure to afford the title compound (brown solid, 200 mg, crude), which was used directly without purification.

### Step E: N-(3-Carbamoyl-1-(4-(8-(4-((2,6-dioxopiperidin-3-yl)amino)phenyl)-2,8-diazaspiro[4.5]dec-2-yl)methyl)phenyl)-1H-pyrazol-4-yl)-6-(trifluoromethyl)picolinamide

To a solution of 3-((4-(2,8-diazaspiro[4.5]dec-8-yl)phenyl)amino)piperidine-2,6-dione (200 mg, 584 µmol, 1.0 eq) and N-(3-carbamoyl-1-(4-formylphenyl)-1H-pyrazol-4-yl)-6-(trifluoromethyl)picolinamide (236 mg, 584 µmol, 1.0 eq) in DMF (10 mL) was added TEA (407 µL, 2.9 mmol, 5.0 eq) at rt, and the mixture was stirred for 10 min. AcOH (1.0 mL) was then added. The mixture was stirred at rt for 10 min. Sodium triacetoxyborohydride (743 mg, 3.5 mmol, 6.0 eq) was then added to the above reaction solution. The mixture was stirred at rt for 1 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (brown solid, 33.8 mg, yield 8%). **¹H NMR** (400 MHz, DMSO-*d*₆) δ 11.79 (s, 1H), 10.74 (s, 1H), 9.01 (s, 1H), 8.48-8.38 (m, 2H), 8.25-8.20 (m, 1H), 8.15 (s, 1H), 8.01 (s, 1H), 7.96-7.92 (m, 2H), 7.77 (s, 1H), 7.49 (d, *J =* 8.3 Hz, 2H), 6.80-6.70 (m, 2H), 6.62-6.53 (m, 2H), 5.44-5.21 (m, 1H), 4.25-4.08 (m, 1H), 3.66 (s, 2H), 2.93-2.82 (m, 4H), 2.75-2.67 (m, 1H), 2.63-2.53 (m, 3H), 2.42 (s, 2H), 2.14-2.06 (m, 1H), 1.87-1.77 (m, 1H), 1.68-1.58 (m, 6H). LCMS (ESI): m/z 730 [M+H]⁺.

### Preparation Example 291. Synthesis of Compound A207

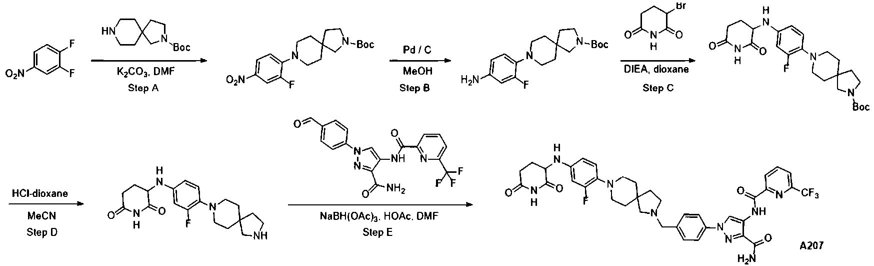

### Step A: Tert-butyl 8-(2-fluoro-4-nitrophenyl)-2,8-diazaspiro[4.5]decane-2-carboxylate

Potassium carbonate (575 mg, 4.2 mmol, 2.0 eq) was added to a solution of tert-butyl 2,8-diazaspiro[4.5]decane-2-carboxylate (500 mg, 2.1 mmol, 1.0 eq) and 1,2-difluoro-4-nitrobenzene (397 mg, 2.5 mmol, 1.2 eq) in DMF (20 mL) at rt. The reaction solution was stirred at 80°C under N₂ for 6 h. Upon completion, the reaction solution was cooled to rt and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (yellow solid, 700 mg, yield 89%).

### Step B: Tert-butyl 8-(4-amino-2-fluorophenyl)-2,8-diazaspiro[4.5]decane-2-carboxylate

To a solution of tert-butyl 8-(2-fluoro-4-nitrophenyl)-2,8-diazaspiro[4.5]decane-2-carboxylate (700 mg, 1.8 mmol, 1.0 eq) in MeOH (15 mL) was added 10% palladium on carbon (100 mg) at rt. The reaction solution was purged with H₂ (using a hydrogen balloon) (×3) and stirred under H₂ at rt for 16 h. Upon completion, the mixture was filtered, and the filtrate was concentrated under reduced pressure to give the title compound (white solid, 550 mg, crude), which was used directly without purification.

### Step C: Tert-butyl 8-(4-((2,6-dioxopiperidin-3-yl)amino)-2-fluorophenyl)-2,8-diazaspiro[4.5]decane-2-carboxylate

To a solution of tert-butyl 8-(4-amino-2-fluorophenyl)-2,8-diazaspiro[4.5]decane-2-carboxylate (500 mg, 1.4 mmol, 1.0 eq) in 1,4-dioxane (2 mL) at rt were added 3-bromopiperidine-2,6-dione (550 mg, 2.9 mmol, 2.0 eq) and DIEA (0.5 mL, 2.9 mmol, 2.0 eq). The reaction solution was stirred at 80°C under N₂ for 16 h. Upon completion, the reaction solution was cooled to rt and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (green solid, 500 mg, yield 76%).

### Step D: 3-((3-Fluoro-4-(2,8-diazaspiro[4.5]dec-8-yl)phenyl)amino)piperidine-2,6-dione

To a solution of tert-butyl 8-(4-((2,6-dioxopiperidin-3-yl)amino)-2-fluorophenyl)-2,8-diazaspiro[4.5]decane-2-carboxylate (200 mg, 0.4 mmol, 1.0 eq) in acetonitrile (8 mL) was added 4 M HCl in 1,4-dioxane (1.6 mL) at 0°C. The reaction solution was stirred at rt under N₂ for 1 h. The reaction solution was concentrated under reduced pressure to afford the title compound (yellow solid, 130 mg, crude), which was used directly without purification.

### Step E: N-(3-amino-1-(4-(8-(4-((2,6-dioxopiperidin-3-yl)amino)-2-fluorophenyl)-2,8-diazaspiro[4.5]dec-2-yl)methyl)phenyl)-1H-pyrazol-4-yl)-6-(trifluoromethyl)picolinamide

To a solution of N-(3-carbamoyl-1-(4-formylphenyl)-1H-pyrazol-4-yl)-6-(trifluoromethyl)picolinamide (120 mg, 0.3 mmol, 1.0 eq) and 3-((3-fluoro-4-(2,8-diazaspiro[4.5]dec-8-yl)phenyl)amino)piperidine-2,6-dione (107 mg, 0.3 mmol, 1.0 eq) in DMF (6 mL) was added TEA (0.057 mL, 0.4 mmol, 2.0 eq) at rt, and the mixture was stirred for 10 min. AcOH (0.2 mL, 1 mmol, 5.0 eq) was then added. The mixture was stirred at rt for 30 min. Sodium triacetoxyborohydride (126 mg, 0.6 mmol, 2.0 eq) was then added to the above reaction solution. The mixture was stirred at rt for 1 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (white solid, 40 mg, yield 18%). **¹H NMR** (400 MHz, DMSO-*d*₆) δ 11.79 (s, 1H), 10.77 (s, 1H), 9.03 (s, 1H), 8.47-8.39 (m, 2H), 8.23-8.21 (m, 1H), 8.14 (s, 1H), 8.03-7.97 (m, 3H), 7.79 (s, 1H), 7.58-7.52 (m, 2H), 6.83 (t, *J =* 9.4 Hz, 1H), 6.52-6.45 (m, 1H), 6.42-6.37 (m, 1H), 5.78 (d, *J =* 7.6 Hz, 1H), 4.28-4.21 (m, 1H), 3.94-3.72 (m, 2H), 2.83-2.66 (m, 8H), 2.60-2.53 (m, 2H), 2.10-2.04 (m, 1H), 1.87-1.79 (m, 1H), 1.73-1.63 (m, 6H). LCMS (ESI): m/z 748 [M+H]⁺.

### Preparation Example 292. Synthesis of Compound A208

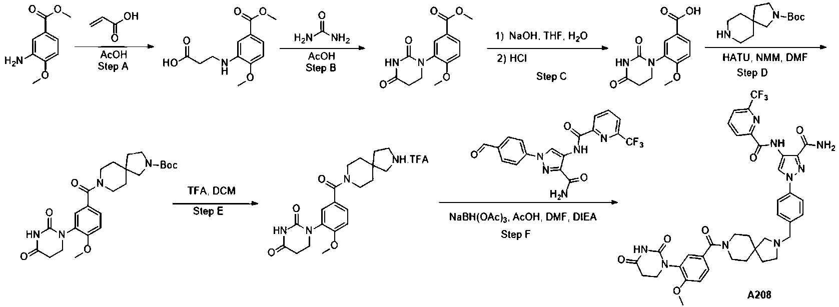

### Step A: 3-((2-methoxy-5-(methoxycarbonyl)phenyl)amino)propanoic acid

To a solution of methyl 3-amino-4-methoxybenzoate (15 g, 82.8 mmol, 1.0 eq) in water (250 mL) and AcOH (50 mL) at rt was added prop-2-enoic acid (6 g, 82.8 mmol, 1.0 eq). The mixture was stirred at 120°C under N₂ for 18 h. Upon completion, the reaction solution was cooled to rt, poured into water, and extracted with EtOAc (×3). The combined organic phases were washed with water and saturated aqueous NaCl, dried over anhydrous Na₂SO₄, and concentrated under reduced pressure to give the title compound (gray solid, 20 g, crude), which was used directly without purification.

### Step B: Methyl 3-(2,4-dioxotetrahydropyrimidin-1(2H)-yl)-4-methoxybenzoate

To a solution of 3-((2-methoxy-5-(methoxycarbonyl)phenyl)amino)propanoic acid (25 g, 98.7 mmol, 1.0 eq) in AcOH (250 mL) was added urea (11.9 g, 197.4 mmol, 2.0 eq) at rt. The mixture was stirred at 120°C under N₂ for 18 h. Upon completion, the reaction solution was cooled to rt, poured into ice water (200 mL), and stirred for 2 h. The mixture was filtered, and the filter cake was washed twice with water, and dried completely to yield the title compound (gray solid, 14 g, crude), which was used directly without purification.

### Step C: 3-(2,4-dioxotetrahydropyrimidin-1(2H)-yl)-4-methoxybenzoic acid

At 0°C, to a mixed solution of methyl 3-(2,4-dioxotetrahydropyrimidin-1(2H)-yl)-4-methoxybenzoate (8 g, 28.75 mmol, 1.0 eq) in THF (90 mL) was added a 1 M NaOH solution (143.75 mL, 143.75 mmol, 5.0 eq). The reaction solution was stirred at rt for 18 h. Upon completion, the MeOH and THF solvents were removed under reduced pressure, and the pH was adjusted to 3-4 with 1N HCl. The mixture was dissolved in concentrated HCl (100 mL), and the reaction was stirred at 100°C for 1.5 h. The mixture was concentrated under reduced pressure, the residue was diluted in water, and the precipitated solid was filtered. The filter cake was washed twice with water and dried completely to obtain the title compound (gray solid, 6 g, crude), which was used directly without purification.

### Step D: Tert-butyl 8-(3-(2,4-dioxotetrahydropyrimidin-1(2H)-yl)-4-methoxybenzoyl)-2,8-diazaspiro[4.5]decane-2-carboxylate

To a solution of 3-(2,4-dioxotetrahydropyrimidin-1(2H)-yl)-4-methoxybenzoic acid (150 mg, 0.6 mmol, 1.0 eq) and 2-methylpropan-2-yl 2,8-diazaspiro[4.5]decane-2-carboxylate (163.7 mg, 0.7 mmol, 1.2 eq) in DMF (4 mL) at rt were added N-methylmorpholine (312 µL, 2.8 mmol, 5.0 eq) and HATU (324 mg, 0.8 mmol, 1.4 eq). The reaction solution was stirred at rt under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (pink solid, 214.2 mg, yield 78%).

### Step E: 1-(2-methoxy-5-(2,8-diazaspiro[4.5]decane-8-carbonyl)phenyl)dihydropyrimidine-2,4(1H,3H)-dione

To a solution of tert-butyl 8-(3-(2,4-dioxotetrahydropyrimidin-1(2H)-yl)-4-methoxybenzoyl)-2,8-diazaspiro[4.5]decane-2-carboxylate in DCM (3 mL) was added trifluoroformic acid (2 mL) at 0°C. The reaction solution was stirred at rt for 1 h. Upon completion, the mixture was concentrated under reduced pressure to afford the title compound (orange oil, 230 mg, crude), which was used directly without purification.

### Step F: N-(3-amino-1-(4-((8-(3-(2,4-dioxotetrahydropyrimidin-1(2H)-yl)-4-methoxybenzoyl)-2,8-diazaspiro[4.5]dec-2-yl)methyl)phenyl)-1H-pyrazol-4-yl)-6-(trifluoromethyl)picolinamide

At rt, to a solution of 1-(2-methoxy-5-(2,8-diazaspiro[4.5]decane-8-carbonyl)phenyl)dihydropyrimidine-2,4(1H,3H)-dione (115 mg, 0.3 mmol, 1.0 eq) in DMF (2 mL) was added DIEA (0.2 mL), and the mixture was stirred for 25 min. AcOH (0.3 mL) and 1-(4-formylphenyl)-4-({[6-(trifluoromethyl)pyridin-2-yl]carbonyl}amino)pyrazole-3-carboxamide (216.6 mg, 0.5 mmol, 1.0 eq) were then added. The mixture was stirred at rt for 15 h. Sodium triacetoxyborohydride (171 mg, 0.81 mmol, 3.0 eq) was then added to the above reaction solution. The mixture was stirred at rt for 1 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (white solid, 29.3 mg, yield 9%). **¹H NMR** (400 MHz, DMSO-*d*₆) δ 10.33 (s, 1H), 9.01 (s, 1H), 8.46 (d, *J* = 7.6 Hz, 1H), 8.40 (t, *J* = 7.6 Hz, 1H), 8.22 (d, *J* = 7.6 Hz, 1H), 8.14 (s, 1H), 8.01 (s, 1H), 7.95 (d, *J =* 8.4 Hz, 2H), 7.78 (s, 1H), 7.49 (d, *J =* 8.4 Hz, 2H), 7.36 (dd, *J =* 8.4, 2.0 Hz, 1H), 7.32 (d, *J =* 2.0 Hz, 1H), 7.15 (d, *J =* 8.6 Hz, 1H), 3.84 (s, 3H), 3.73-3.68 (m, 2H), 3.60 (d, *J =* 6.8 Hz, 2H), 3.47-3.37 (m, 6H), 2.73-2.62 (m, 4H), 1.68 (t, *J =* 6.8 Hz, 2H), 1.61-1.46 (m, 4H). LCMS (ESI): m/z 774 [M+H]⁺.

### Preparation Example 293. Synthesis of Compound A209

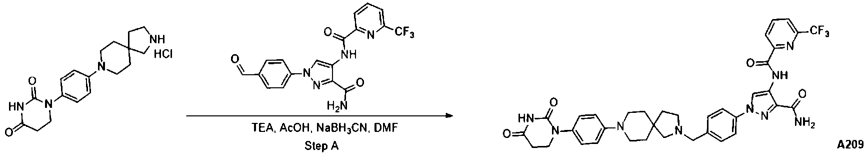

### Step A: N-(3-amino-1-(4-(8-(4-(2,4-dioxotetrahydropyrimidin-1(2H)-yl)phenyl)-2,8-diazaspiro[4.5]dec-2-yl)methyl)phenyl)-1H-pyrazol-4-yl)-6-(trifluoromethyl)picolinamide

To a solution of 1-(4-(2,8-diazaspiro[4.5]dec-8-yl)phenyl)dihydropyrimidine-2,4(1H,3H)-dione hydrochloride (90 mg, 0.3 mmol, 1.0 eq) in DMF (5 mL) was added TEA (0.1 mL) at rt, and the mixture was stirred at 0°C for 10 min. To the mixture were added N-(3-carbamoyl-1-(4-formylphenyl)-1H-pyrazol-4-yl)-6-(trifluoromethyl)picolinamide (110.5 mg, 0.3 mmol, 1.0 eq) and AcOH (0.2 mL). The mixture was stirred at rt for 18 h. Sodium triacetoxyborohydride (34.4 mg, 0.5 mmol, 2.0 eq) was then added to the above reaction solution. The mixture was stirred at rt for 2 h. Upon completion, the mixture was diluted in water, extracted with EtOAc (×3), washed with saturated aqueous NaCl, dried over anhydrous Na₂SO₄, filtered, and concentrated under reduced pressure. The residue was purified by HPLC to afford the title compound (white solid, 15.6 mg, yield 8%). **¹H NMR** (400 MHz, DMSO-*d*₆) δ 11.79 (s, 1H), 10.24 (s, 1H), 9.00 (s, 1H), 8.42 (d, *J* = 18.0 Hz, 2H), 8.22 (d, *J* = 7.7 Hz, 1H), 8.06-7.88 (m, 3H), 7.77 (s, 1H), 7.48 (d, *J* = 8.5 Hz, 2H), 7.12 (d, *J* = 8.9 Hz, 2H), 6.92 (d, *J* = 9.0 Hz, 2H), 3.68 (t, *J* = 6.7 Hz, 2H), 3.63 (s, 2H), 3.18-3.06 (m, 4H), 2.67 (t, *J* = 6.7 Hz, 2H), 2.58 (t, *J* = 6.9 Hz, 2H), 2.40 (s, 2H), 1.63 (s, 6H). LCMS (ESI): m/z 716 (M+H)⁺.

### Preparation Example 294. Synthesis of Compound A210

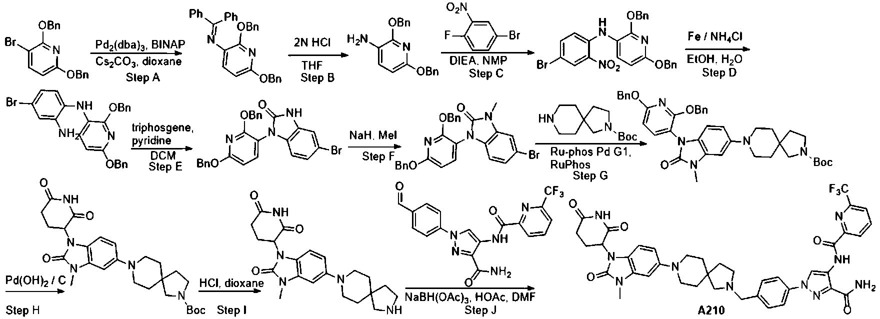

### Step A: N-(2,6-bis(benzyloxy)pyridin-3-yl)-1,1-diphenylmethanimine

To a solution of 2,6-bis(benzyloxy)-3-bromopyridine (25 g, 67.6 mmol, 1 eq) and benzophenone imine (14.7 g, 81 mmol, 1.2 eq) in 1,4-dioxane (200 mL) were added cesium carbonate (44 g, 135 mmol, 2 eq), Pd₂(dba)₃ (6.19 g, 6.76 mmol, 0.1 eq), and BINAP (8.4 g, 6.76 mmol, 0.1 eq) at rt. The mixture was stirred at 100°C under N₂ for 16 h. Upon completion, the mixture was cooled to rt, diluted in water, extracted with EtOAc, washed with saturated aqueous NaCl, dried over anhydrous Na₂SO₄, and concentrated under reduced pressure. The residue was purified by column chromatography to afford the title compound (yellow oil, 31 g, yield 98%).

### Step B: 2,6-Bis(benzyloxy)pyridin-3-amine

At 0°C, to a solution of N-(2,6-bis(benzyloxy)pyridin-3-yl)-1,1-diphenylcarbamylamine (31 g, 65.8 mmol, 1.0 eq) in THF (150 mL) was added a 2 M HCl solution (30 mL). The reaction solution was stirred at rt under N₂ for 1 h. The reaction solution was concentrated under reduced pressure, and the crude was slurried (EtOAc/PE (20 mL, 1/10)) and filtered. The filter cake was dried completely to afford the title compound (yellow solid, 15 g, yield 74%).

### Step C: 2,6-Bis(benzyloxy)-N-(4-bromo-2-nitrophenyl)pyridin-3-amine

To a solution of 4-bromo-1-fluoro-2-nitrobenzene (8.58 g, 39 mmol, 1.0 eq) and 2,6-bis(benzyloxy)pyridin-3-amine (12 g, 39.2 mmol, 1.0 eq) in N-methylpyrrolidone (120 mL) was added DIEA (34.7 mL, 195.8 mmol, 5.0 eq) at rt. The reaction solution was stirred at 100°C under N₂ for 56 h. Upon completion, the reaction solution was cooled to rt and extracted with EtOAc (×3). The combined organic phases were washed sequentially with water and saturated aqueous NaCl, dried over anhydrous Na₂SO₄, filtered, and concentrated under reduced pressure. The crude was slurried (EtOAc/PE (20 mL, 1/10)) and filtered, and the filter cake was dried completely to afford the title compound (yellow solid, 10 g, yield 50%).

### Step D: N¹-(2,6-bis(benzyloxy)pyridin-3-yl)-4-bromobenzene-1,2-diamine

To a solution of 2,6-bis(benzyloxy)-N-(4-bromo-2-nitrophenyl)pyridin-3-amine (10 g, 19.7 mmol, 1.0 eq) in ethanol (50 mL) and water (50 mL) at rt were added iron powder (5.5 g, 98.7 mmol, 5.0 eq) and ammonium chloride (5.2 g, 98.7 mmol, 5.0 eq). The mixture was stirred at 70°C for 16 h. Upon completion, the mixture was filtered, and the filtrate was concentrated under reduced pressure to afford the title compound (brown solid, 7 g, crude), which was used directly without purification.

### Step E: 1-(2,6-bis(benzyloxy)pyridin-3-yl)-5-bromo-1,3-dihydro-2H-benzo[d]imidazol-2-one

To a solution of N¹-(2,6-bis(benzyloxy)pyridin-3-yl)-4-bromobenzene-1,2-diamine (7 g, 14.6 mmol, 1.0 eq) in DCM (50 mL) was added pyridine (1.1 mL, 14.6 mmol, 1.0 eq) at 0°C, and the mixture was stirred for 5 min. A solution of triphosgene (4.3 g, 14.6 mmol, 1.0 eq) in DCM was added dropwise to the above mixture. The mixture was stirred at rt for 16 h. Upon completion, the reaction was quenched with ice water and extracted with DCM (×3). The combined organic phases were washed sequentially with water and saturated aqueous NaCl, dried over anhydrous Na₂SO₄, filtered, and concentrated under reduced pressure. The residue was purified by column chromatography to afford the title compound (off-white solid, 4.6 g, yield 62%).

### Step F: 1-(2,6-bis(benzyloxy)pyridin-3-yl)-5-bromo-3-methyl-1,3-dihydro-2H-benzo[d]imidazol-2-one

To a solution of 1-(2,6-bis(benzyloxy)pyridin-3-yl)-5-bromo-1,3-dihydro-2H-benzo[d]imidazol-2-one (4.6 g, 9.1 mmol, 1.0 eq) in DMF (40 mL) at 0°C under N₂ was added portionwise 60 wt% sodium hydride (0.9 g, 22.8 mmol, 2.5 eq). The mixture was stirred at rt for 0.5 h. Potassium iodide (3.2 g, 22.8 mmol, 2.5 eq) was then added to the above reaction solution. The mixture was stirred at 0°C under N₂ at rt for 1 h. Upon completion, the reaction was quenched with saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow solid, 4 g, yield 85%).

### Step G: Tert-butyl 8-(1-(2,6-bis(benzyloxy)pyridin-3-yl)-3-methyl-2-oxo-2,3-dihydro-1H-benzo[d]imidazol-5-yl)-2,8-diazaspiro[4.5]decane-2-carboxylate

To a solution of 1-(2,6-bis(benzyloxy)pyridin-3-yl)-5-bromo-3-methyl-1,3-dihydro-2H-benzo[d]imidazol-2-one (400 mg, 0.78 mmol, 1.0 eq) and tert-butyl 2,8-diazaspiro[4.5]decane-2-carboxylate (288 mg, 1.2 mmol, 1.5 eq) in 1,4-dioxane (10 mL) were added Ru-Phos Pd G1 (127 mg, 0.14 mmol, 0.2 eq), Ru-Phos (72 mg, 0.14 mmol, 0.2 eq), and cesium carbonate (760 mg, 2.34 mmol, 3.0 eq) at rt. The reaction solution was stirred at 100°C under N₂ for 16 h. Upon completion, the reaction solution was cooled to rt and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (yellow solid, 350 mg, yield 66%).

### Step H: Tert-butyl 8-(1-(2,6-dioxopiperidin-3-yl)-3-methyl-2-oxo-2,3-dihydro-1H-benzo[d]imidazol-5-yl)-2,8-diazaspiro[4.5]decane-2-carboxylate

To a solution of tert-butyl 8-(1-(2,6-bis(benzyloxy)pyridin-3-yl)-3-methyl-2-oxo-2,3-dihydro-1H-benzo[d]imidazol-5-yl)-2,8-diazaspiro[4.5]decane-2-carboxylate (350 mg, 0.52 mmol, 1.0 eq) in ethanol (10 mL) was added 10% palladium hydroxide on carbon (300 mg) at rt. The reaction solution was purged with H₂ (using a hydrogen balloon) (×3) and stirred under H₂ at 50°C for 16 h. Upon completion, the mixture was filtered, and the filtrate was concentrated under reduced pressure to afford the title compound (white solid, 200 mg, crude), which was used directly without purification.

### Step I: 3-(3-methyl-2-oxo-5-(2,8-diazaspiro[4.5]dec-8-yl)-2,3-dihydro-1H-benzo[d]imidazol-1-yl)piperidine-2,6-dione

To a solution of tert-butyl 8-(1-(2,6-dioxopiperidin-3-yl)-3-methyl-2-oxo-2,3-dihydro-1H-benzo[d]imidazol-5-yl)-2,8-diazaspiro[4.5]decane-2-carboxylate (100 mg, 0.2 mmol, 1.0 eq) in acetonitrile (5 mL) was added 4 M HCl in 1,4-dioxane (1 mL) at 0°C. The reaction solution was stirred at rt under N₂ for 1 h. The reaction solution was concentrated under reduced pressure to afford the title compound (yellow solid, 79 mg, crude), which was used directly without purification.

### Step J: N-(3-carbamoyl-1-(4-((8-(1-(2,6-dioxopiperidin-3-yl)-3-methyl-2-oxo-2,3-dihydro-1H-benzo[d]imidazol-5-yl)-2,8-diazaspiro[4.5]dec-2-yl)methyl)phenyl)-1H-pyrazol-4-yl)-6-(trifluoromethyl)picolinamide

To a solution of 3-(3-methyl-2-oxo-5-(2,8-diazaspiro[4.5]dec-8-yl)-2,3-dihydro-1H-benzo[d]imidazol-1-yl)piperidine-2,6-dione (79 mg, 0.2 mmol, 1.0 eq) and N-(3-carbamoyl-1-(4-formylphenyl)-1H-pyrazol-4-yl)-6-(trifluoromethyl)picolinamide (82 mg, 0.2 mmol, 1.0 eq) in DMF (5 mL) was added TEA (0.057 mL, 0.4 mmol, 2.0 eq) at rt, and the mixture was stirred for 10 min. AcOH (0.2 mL, 1 mmol, 5.0 eq) was then added. The mixture was stirred at rt for 20 min. Sodium triacetoxyborohydride (258 mg, 1.2 mmol, 6.0 eq) was then added to the above reaction solution. The mixture was stirred at rt for 1 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (white solid, 47.1 mg, yield 30%). **¹H NMR** (400 MHz, DMSO-*d*₆) δ 11.80 (s, 1H), 11.06 (s, 1H), 9.01 (s, 1H), 8.49-8.38 (m, 2H), 8.22 (d, *J* = 7.6 Hz, 1H), 8.16 (s, 1H), 8.02 (s, 1H), 7.94 (d, *J* = 8.5 Hz, 2H), 7.78 (s, 1H), 7.50 (d, *J* = 8.4 Hz, 2H), 6.92 (d, *J =* 8.6 Hz, 1H), 6.83 (d, *J =* 1.7 Hz, 1H), 6.64 (d, *J* = 8.6 Hz, 1H), 5.28 (dd, *J =* 12.7, 5.1 Hz, 1H), 3.65 (s, 2H), 3.30 (s, 3H), 3.05 (s, 4H), 2.87 (d, *J* = 11.3 Hz, 1H), 2.68 (s, 1H), 2.61 (d, *J* = 6.9 Hz, 3H), 2.43 (s, 2H), 2.02-1.93 (m, 1H), 1.66 (dd, *J* = 13.7, 6.2 Hz, 6H). LCMS (ESI): m/z 785 [M+H]⁺.

### Preparation Example 295. Synthesis of Compound A211

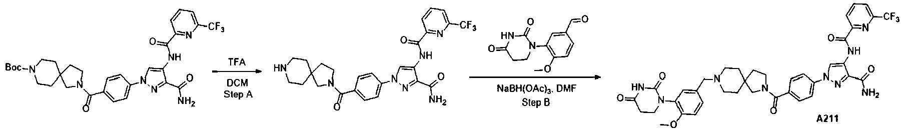

### Step A: N-(1-(4-(2,8-diazaspiro[4.5]dec-2-carbonyl)phenyl)-3-formyl-1H-pyrazol-4-yl)-6-(trifluoromethyl)picolinamide

To a solution of tert-butyl 2-(4-(3-amino-4-(6-(trifluoromethyl)picolinamido)-1H-pyrazol-1-yl)benzoyl)-2,8-diazaspiro[4.5]decane-8-carboxylate (120 mg, 0.187 mmol, 1.0 eq) in DCM (3 mL) was added TFA (3 mL) at 0°C. The reaction solution was stirred at rt under N₂ for 1 h. Upon completion, the mixture was concentrated under reduced pressure. The residue was dissolved in EtOAc (30 mL), and the organic phase was washed with saturated NaHCO₃ and aqueous NaCl, dried over anhydrous Na₂SO₄, filtered, and concentrated under reduced pressure to afford the title compound (yellow solid, 90 mg, crude).

### Step B: N-(3-carbonyl-1-(4-(8-(3-(2,4-dioxotetrahydropyrimidin-1(2H)-yl)-4-methoxybenzyl)-2,8-diazaspiro[4.5]dec-2-carbonyl)phenyl)-1H-pyrazol-4-yl)-6-(trifluoromethyl)picolinamide

To a solution of 3-(2,4-dioxohexahydropyrimidin-1-yl)-4-methoxybenzene-1-carbaldehyde (90 mg, 0.363 mmol, 2.0 eq) and N-(1-(4-(2,8-diazaspiro[4.5]dec-2-carbonyl)phenyl)-3-formyl-1H-pyrazol-4-yl)-6-(trifluoromethyl)picolinamide (90 mg, 0.166 mmol, 1.0 eq) in DMF (3 mL) was added AcOH (cat., 1 drop) at rt. The mixture was stirred at rt for 30 min. Sodium triacetoxyborohydride (111 mg, 0.524 mmol, 3.0 eq) was then added to the above reaction solution. The mixture was stirred at rt for 16 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (white solid, 30 mg, yield 23%). **¹H NMR** (400 MHz, DMSO-*d*₆) δ 11.80 (s, 1H), 10.27 (d, *J* = 11.2 Hz, 1H), 9.10 (d, *J* = 4.3 Hz, 1H), 8.46 (d, *J =* 7.8 Hz, 1H), 8.41 (t, *J =* 7.8 Hz, 1H), 8.22 (d, *J* = 7.7 Hz, 1H), 8.12-8.02 (m, 3H), 7.82 (s, 1H), 7.70 (dd, *J =* 13.0, 8.6 Hz, 2H), 7.27-7.09 (m, 2H), 7.04 (dd, *J =* 18.3, 8.5 Hz, 1H), 3.78 (d, *J =* 13.5 Hz, 3H), 3.60-3.50 (m, 4H), 3.43 (s, 4H), 2.71-2.61 (m, 2H), 2.34 (d, *J* = 11.9 Hz, 3H), 2.17 (s, 1H), 1.77 (dd, *J =* 15.7, 8.1 Hz, 2H), 1.60 (s, 2H), 1.47 (s, 2H). LCMS (ESI) m/z: 774 [M+H]⁺.

### Preparation Example 296. Synthesis of Compound A212

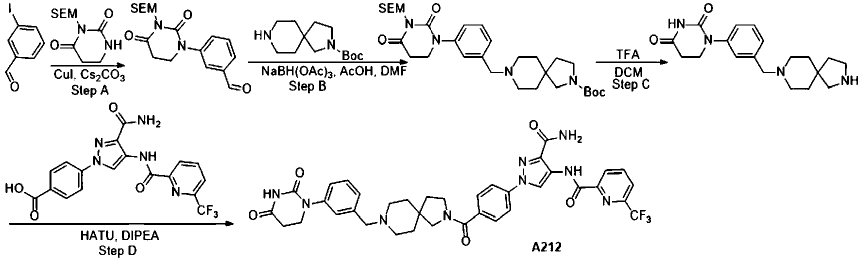

### Step A: 3-(2,4-dioxo-3-((2-(trimethylsilyl)ethoxy)methyl)tetrahydropyrimidin-1(2H)-yl)benzaldehyde

To a solution of 3-iodobenzene-1-carboxaldehyde (2 g, 8.6 mmol, 1.0 eq) in 1,4-dioxane (20 mL) at rt were added 3-((2-(trimethylsilyl)ethoxy)methyl)dihydropyrimidine-2,4(1H,3H)-dione (2.5 g, 10 mmol, 1.2 eq), cesium carbonate (8.4 g, 26 mmol, 3.0 eq), cuprous iodide (0.7 g, 3.5 mmol, 0.4 eq), and (1R,2S)-N1,N2-dimethylcyclohexane-1,2-diamine (0.5 g, 3.5 mmol, 0.4 eq). The reaction solution was stirred at 100°C under N₂ for 16 h. Upon completion, the reaction solution was cooled to rt and extracted with EtOAc (×3). The combined organic phases were washed sequentially with water and saturated aqueous NaCl, dried over anhydrous Na₂SO₄, filtered, and concentrated under reduced pressure. The residue was purified by column chromatography to afford the title compound (yellow solid, 522 mg, yield 16%).

### Step B: Tert-butyl 8-(3-(2,4-dioxo-3-((2-(trimethylsilyl)ethoxy)methyl)tetrahydropyrimidin-1(2H)-yl)benzyl)-2,8-diazaspiro[4.5]decane-2-carboxylate

To a solution of 3-(2,4-dioxo-3-((2-(trimethylsilyl)ethoxy)methyl)tetrahydropyrimidin-1(2H)-yl)benzaldehyde (522 mg, 1.5 mmol, 1.2 eq) and tert-butyl 2,8-diazaspiro[4.5]decane-2-carboxylate (300 mg, 1.3 mmol, 1.0 eq) in DMF (8 mL) was added AcOH (0.7 mL, 12.5 mmol, 10.0 eq) at rt. The mixture was stirred at rt for 1 h. Sodium triacetoxyborohydride (794 mg, 3.7 mmol, 3.0 eq) was then added to the above reaction solution. The mixture was stirred at rt for 2 h. Upon completion, the mixture was diluted in water, extracted with EtOAc (×3), washed with saturated aqueous NaCl, dried over anhydrous Na₂SO₄, filtered, and concentrated under reduced pressure. The residue was purified by HPLC to afford the title compound (yellow solid, 80 mg, yield 11%).

### Step C: 1-(3-((2,8-diazaspiro[4.5]dec-8-yl)methyl)phenyl)dihydropyrimidine-2,4(1H,3H)-dione

At 0°C, to a solution of tert-butyl 8-(3-(2,4-dioxo-3-((2-(trimethylsilyl)ethoxy)methyl)tetrahydropyrimidin-1(2H)-yl)benzyl)-2,8-diazaspiro[4.5]decane-2-carboxylate (80 mg, 0.2 mmol, 1.0 eq) in DCM (3 mL) was added TFA (1 mL). The reaction solution was stirred at rt under N₂ for 1 h. The reaction solution was concentrated under reduced pressure to afford the title compound (yellow solid, 50 mg, crude), which was used directly without purification.

### Step D: N-(3-carbonyl-1-(4-(8-(3-(2,4-dioxotetrahydropyrimidin-1(2H)-yl)benzyl)-2,8-diazaspiro[4.5]dec-2-carbonyl)phenyl)-1H-pyrazol-4-yl)-6-(trifluoromethyl)picolinamide

To a solution of 4-(3-amino-4-(6-(trifluoromethyl)picolinamido)-1H-pyrazol-1-yl)benzoic acid (84 mg, 0.2 mmol, 2.0 eq) in DMF (4 mL) at rt were added 1-(3-((2,8-diazaspiro[4.5]dec-8-yl)methyl)phenyl)dihydropyrimidine-2,4(1H,3H)-dione (50 mg, 0.1 mmol, 1.0 eq), DIEA (0.05 mL, 0.3 mmol, 3 eq), and HATU (102 mg, 0.3 mmol, 3 eq). The reaction solution was stirred at rt under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 15 mg, yield 15%). **¹H NMR** (400 MHz, DMSO-*d*₆) δ 11.81 (s, 1H), 10.36 (d, *J* = 11.6 Hz, 1H), 9.11 (s, 1H), 8.46 (s, 1H), 8.41 (t, *J =* 7.7 Hz, 1H), 8.23 (d, *J* = 7.5 Hz, 1H), 8.09-8.05 (m, 2H), 7.82 (s, 2H), 7.74-7.67 (m, 2H), 7.38-7.15 (m, 4H), 3.84-3.74 (m, 4H), 3.64-3.61 (m, 2H), 2.78-2.66 (m, 3H), 2.62-2.53 (m, 2H), 2.33-2.25 (m, 1H), 2.04-1.74 (m, 3H), 1.69-1.59 (m, 2H), 1.55-1.48 (m, 2H), 1.24 (s, 1H). LCMS (ESI): m/z 744 [M+H]⁺.

### Preparation Example 297. Synthesis of Compound A213

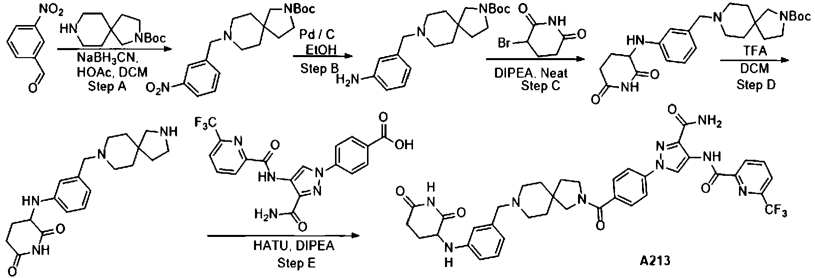

### Step A: Tert-butyl 8-(3-nitrobenzyl)-2,8-diazaspiro[4.5]decane-2-carboxylate

To a solution of 3-nitrobenzene-1-carboxaldehyde (629 mg, 4.2 mmol, 2.0 eq) and 2-methylpropan-2-yl 2,8-diazaspiro[4.5]decane-2-carboxylate (90 mg, 0.166 mmol, 1.0 eq) in DCM (10 mL) was added AcOH (5 drops) at rt. The mixture was stirred at rt for 10 min. Sodium cyanoborohydride (261 mg, 4.2 mmol, 2.0 eq) was then added to the above reaction solution. The mixture was stirred at rt for 2 h. Upon completion, the mixture was diluted in water, extracted with EtOAc (×3), washed with saturated aqueous NaCl, dried over anhydrous Na₂SO₄, and concentrated under reduced pressure. The residue was purified by column chromatography to afford the title compound (white solid, 520 mg, yield 67%).

### Step B: Tert-butyl 8-(3-aminobenzyl)-2,8-diazaspiro[4.5]decane-2-carboxylate

To a solution of tert-butyl 8-(3-nitrobenzyl)-2,8-diazaspiro[4.5]decane-2-carboxylate (500 mg, 1.3 mmol, 1.0 eq) in ethanol (15 mL) was added 10% palladium on carbon (142 mg) at rt. The reaction solution was purged with H₂ (using a hydrogen balloon) (×3) and stirred under H₂ at rt for 18 h. Upon completion, the mixture was filtered, and the filtrate was concentrated under reduced pressure to afford the title compound (white solid, 400 mg, crude), which was used directly without purification.

### Step C: Tert-butyl 8-(3-((2,6-dioxopiperidin-3-yl)amino)benzyl)-2,8-diazaspiro[4.5]decane-2-carboxylate

To tert-butyl 8-(3-aminobenzyl)-2,8-diazaspiro[4.5]decane-2-carboxylate (400 mg, 2.2 mmol, 1.0 eq) were added DIPEA (0.4 mL, 4.4 mmol, 2.0 eq) and 3-bromopiperidine-2,6-dione (444 mg, 4.4 mmol, 2.0 eq) at rt. The mixture was stirred at 80°C for 16 h. Upon completion, the mixture was cooled to rt, dissolved in a mixed solution of DCM/MeOH (10 : 1), and filtered. The crude filter cake was purified by column chromatography to afford the title compound (yellow solid, 200 mg, yield 38%).

### Step D: 3-((3-((2,8-diazaspiro[4.5]dec-8-yl)methyl)phenyl)amino)piperidine-2,6-dione

At 0°C, to a solution of tert-butyl 8-(3-((2,6-dioxopiperidin-3-yl)amino)benzyl)-2,8-diazaspiro[4.5]decane-2-carboxylate (200 mg, 0.5 mmol, 1.0 eq) in DCM (5 mL) was added TFA (1 mL). The reaction solution was stirred at 30°C under N₂ for 0.5 h. The reaction solution was concentrated under reduced pressure to afford the title compound (yellow solid, 120 mg, crude), which was used in the next step without purification.

### Step E: N-(3-carbonyl-1-(4-(8-(3-((2,6-dioxopiperidin-3-yl)amino)benzyl)-2,8-diazaspiro[4.5]dec-2-carbonyl)phenyl)-1H-pyrazol-4-yl)-6-(trifluoromethyl)picolinamide

To a solution of 3-((3-((2,8-diazaspiro[4.5]dec-8-yl)methyl)phenyl)amino)piperidine-2,6-dione (50 mg, 0.15 mmol, 1.0 eq) in DMF (5 mL) were added 4-(3-amino-4-(6-(trifluoromethyl)picolinamido)-1H-pyrazol-1-yl)benzoic acid (71 mg, 0.17 mmol, 1.2 eq), DIPEA (0.2 mL, 0.3 mmol), and HATU (107 mg, 0.3 mmol, 2.0 eq) at rt. The reaction solution was stirred at 30°C under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 30 mg, yield 28%). **¹H NMR** (400 MHz, DMSO-*d*₆) δ 11.81 (s, 1H), 10.83 (d, *J* = 9.7 Hz, 1H), 9.17 (d, *J =* 43.2 Hz, 2H), 8.48-8.39 (m, 2H), 8.23 (d, *J =* 7.5 Hz, 1H), 8.08 (d, *J* = 8.5 Hz, 2H), 7.83 (s, 1H), 7.73 (d, *J =* 8.2 Hz, 2H), 7.19 (t, *J =* 7.7 Hz, 1H), 6.79-6.62 (m, 3H), 6.10 (s, 1H), 4.38-4.04 (m, 3H), 3.52 (d, *J =* 34.3 Hz, 4H), 3.09 (s, 2H), 2.91-2.56 (m, 3H), 2.17-1.59 (m, 9H). LCMS (ESI): m/z 758 [M+H]⁺.

### Preparation Example 298. Synthesis of Compound A214

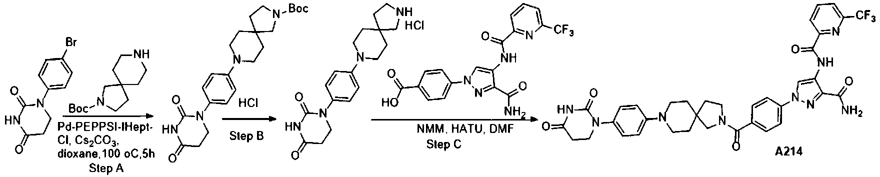

### Step A: 8-(4-(2,4-dioxotetrahydropyrimidin-1(2H)-yl)phenyl)-2,8-diazaspiro[4.5]decane-2-carboxylate

To a solution of 1-(4-bromophenyl)dihydropyrimidine-2,4(1H,3H)-dione (200 mg, 0.7 mmol, 1.0 eq) and tert-butyl 2,8-diazaspiro[4.5]decane-2-carboxylate (214 mg, 0.9 mmol, 1.2 eq) in 1,4-dioxane (5 mL) at rt were added cesium carbonate (726 mg, 2.3 mmol, 1.3 eq) and Pd-PEPPSI-Ihept-Cl (36 mg, 0.04 mmol, 0.05 eq). The mixture was stirred at 100°C under N₂ for 5 h. Upon completion, the mixture was cooled to rt, diluted in water, extracted with EtOAc, washed with saturated aqueous NaCl, dried over anhydrous Na₂SO₄, and concentrated under reduced pressure. The residue was purified by column chromatography to afford the title compound (yellow solid, 127 mg, yield 40%).

### Step B: 1-(4-(2,8-diazaspiro[4.5]dec-8-yl)phenyl)dihydropyrimidine-2,4(1H,3H)-dione hydrochloride

To a solution of 8-(4-(2,4-dioxotetrahydropyrimidin-1(2H)-yl)phenyl)-2,8-diazaspiro[4.5]decane-2-carboxylate (127 mg, 0.3 mmol, 1.0 eq) in EtOAc (5 mL) was added 4 M HCl in MeOH (1 mL, 4.0 mmol, 8.0 eq) at 0°C. The reaction solution was stirred at rt under N₂ for 1 h. The filtrate was concentrated under reduced pressure to give the title compound (yellow solid, 90 mg, crude), which was used directly without purification.

### Step C: N-(3-carbonyl-1-(4-(8-(4-(2,4-dioxotetrahydropyrimidin-1(2H)-yl)phenyl)-2,8-diazaspiro[4.5]dec-2-carbonyl)phenyl)-1H-pyrazol-4-yl)-6-(trifluoromethyl)picolinamide

To a solution of 4-(3-amino-4-(6-(trifluoromethyl)picolinamido)-1H-pyrazol-1-yl)benzoic acid (132 mg, 0.3 mmol, 1.0 eq) in DMF (5 mL) at rt were added 1-(4-(2,8-diazaspiro[4.5]dec-8-yl)phenyl)dihydropyrimidine-2,4(1H,3H)-dione hydrochloride (90 mg, 0.3 mmol, 1.0 eq), DIPEA (177 mg, 1.4 mmol, 5.0 eq), and HATU (208 mg, 0.5 mmol, 2.0 eq). The reaction solution was stirred at rt under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 17.4 mg, yield 10%). **¹H NMR** (400 MHz, DMSO-*d*₆) δ 11.80 (d, *J* = 4.2 Hz, 1H), 10.25 (d, *J =* 11.2 Hz, 1H), 9.10 (d, *J =* 7.2 Hz, 1H), 8.50-8.36 (m, 2H), 8.22 (d, *J =* 7.6 Hz, 1H), 8.07 (d, *J =* 7.8 Hz, 3H), 7.82 (s, 1H), 7.73 (dd, *J =* 18.8, 8.6 Hz, 2H), 7.13 (dd, *J* = 19.0, 8.8 Hz, 2H), 6.94 (dd, *J* = 28.3, 8.9 Hz, 2H), 3.69 (dt, *J* = 16.2, 6.6 Hz, 2H), 3.63-3.53 (m, 2H), 3.43 (s, 1H), 3.37 (s, 1H), 3.24 (s, 1H), 3.16 (d, *J* = 9.3 Hz, 2H), 3.07 (d, *J =* 6.2 Hz, 1H), 2.67 (dt, *J* = 13.2, 6.7 Hz, 2H), 1.84 (dt, *J* = 18.8, 7.0 Hz, 2H), 1.74 (d, *J =* 13.9 Hz, 2H), 1.60 (s, 2H). LCMS (ESI): m/z 730 [M+H]⁺.

### Preparation Example 299. Synthesis of Compound A215

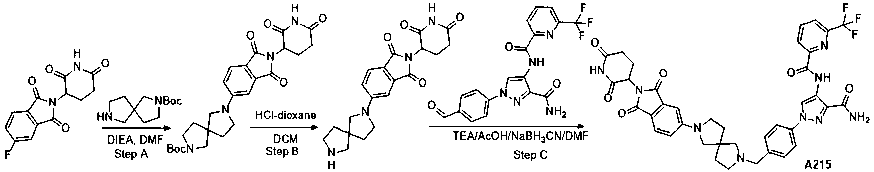

### Step A: Tert-butyl 7-(2-(2,6-dioxopiperidin-3-yl)-1,3-dioxoisoindolin-5-yl)-2,7-diazaspiro[4.4]nonane-2-carboxylate

To a solution of 2-(2,6-dioxopiperidin-3-yl)-5-fluoroisoindoline-1,3-dione (1.0 g, 3.62 mmol, 1.0 eq) and tert-butyl 2,7-diazaspiro[4.4]nonane-2-carboxylate (0.9 g, 3.98 mmol, 1.1 eq) in DMF (10 mL) was added DIEA (1.4 g, 10.86 mmol, 3.0 eq) at rt. The reaction solution was stirred at 90°C for 5 h. Upon completion, the mixture was cooled to rt, diluted in water, and extracted with EtOAc (×3). The combined organic phases were washed with water and saturated aqueous NaCl, dried over anhydrous Na₂SO₄, filtered, and concentrated under reduced pressure. The residue was purified by column chromatography to afford the title compound (yellow solid, 0.8 g, yield 46%).

### Step B: 2-(2,6-dioxopiperidin-3-yl)-5-(2,7-diazaspiro[4.4]nonan-2-yl)isoindoline-1,3-dione

At 0°C, to a solution of tert-butyl 7-(2-(2,6-dioxopiperidin-3-yl)-1,3-dioxoisoindolin-5-yl)-2,7-diazaspiro[4.4]nonane-2-carboxylate (300 mg, 0.62 mmol, 1.0 eq) in DCM (5 mL) was added 4 M HCl in 1,4-dioxane (1.5 mL, 6.00 mmol, 10.0 eq). The reaction solution was stirred at rt under N₂ for 1 h. The reaction solution was concentrated under reduced pressure to give the title compound (yellow solid, 200 mg, crude), which was used directly without purification.

### Step C: N-(3-carbamoyl-1-(4-((7-(2-(2,6-dioxopiperidin-3-yl)-1,3-dioxoisoindolin-5-yl)-2,7-diazaspiro[4.4]nonan-2-yl)methyl)phenyl)-1H-pyrazol-4-yl)-6-(trifluoromethyl)picolinamide

To a solution of 2-(2,6-dioxopiperidin-3-yl)-5-(2,7-diazaspiro[4.4]nonan-2-yl)isoindoline-1,3-dione (200 mg, 0.52 mmol, 1.0 eq) in DMF (5 mL) was added TEA (132 mg, 1.31 mmol, 2.5 eq) at rt, and the mixture was stirred for 5 min. AcOH (188 mg, 3.14 mmol, 6.0 eq) and N-(3-carbamoyl-1-(4-formylphenyl)-1H-pyrazol-4-yl)-6-(trifluoromethyl)picolinamide (211 mg, 0.52 mmol, 1.0 eq) were then added. The mixture was stirred at rt for 18 h. Sodium triacetoxyborohydride (131 mg, 2.04 mmol, 4.0 eq) was then added to the above reaction solution. The mixture was stirred at 40°C for 2 h. Upon completion, the mixture was diluted in water, extracted with EtOAc (×3), washed with saturated aqueous NaCl, dried over anhydrous Na₂SO₄, filtered, and concentrated under reduced pressure. The residue was purified by HPLC to afford the title compound (yellow solid, 42.4 mg, yield 11%). **¹H NMR** (400 MHz, DMSO-*d*₆) δ 11.79 (s, 1H), 11.06 (s, 1H), 9.00 (s, 1H), 8.47-8.37 (m, 2H), 8.22 (d, *J* = 7.6 Hz, 1H), 8.14 (s, 1H), 8.01 (s, 1H), 7.94 (d, *J* = 8.5 Hz, 2H), 7.77 (s, 1H), 7.63 (d, *J =* 8.4 Hz, 1H), 7.51 (d, *J* = 8.5 Hz, 2H), 6.90 (d, *J* = 1.8 Hz, 1H), 6.82-6.77 (m, 1H), 5.05 (m, 1H), 3.71 (s, 2H), 3.46-3.41 (m, 3H), 3.39-3.35 (m, 3H), 2.93-2.83 (m, 1H), 2.76-2.64 (m, 2H), 2.62-2.59 (m, 1H), 2.56-2.53 (m, 1H), 2.07-1.96 (m, 3H), 1.88-1.78 (m, 2H). LCMS (ESI) m/z: 770 [M+H]⁺.

### Preparation Example 300. Synthesis of Compound A216

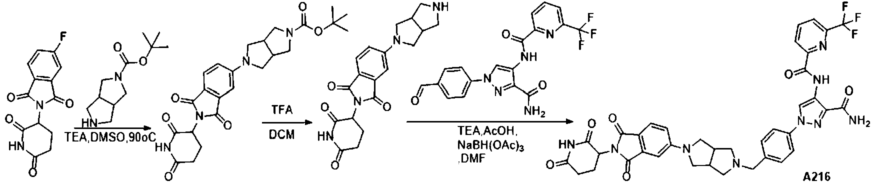

### Step A: Tert-butyl 5-(2-(2,6-dioxopiperidin-3-yl)-1,3-dioxoisoindolin-5-yl)hexahydropyrrolo[3,4-c]pyrrole-2(1H)-carboxylate

To a solution of 2-(2,6-dioxopiperidin-3-yl)-5-fluoroisoindoline-1,3-dione (200 mg, 0.72 mmol, 1.0 eq) in dimethyl sulfoxide (10 mL) at rt were added TEA (0.20 mL, 1.45 mmol, 2.0 eq) and tert-butyl hexahydropyrrolo[3,4-c]pyrrole-2(1H)-carboxylate (169 mg, 0.8 mmol, 1.1 eq). The mixture was stirred at 90°C under N₂ for 18 h. Upon completion, the mixture was cooled to rt, diluted in water, and extracted with EtOAc (×3). The combined organic phases were washed with saturated aqueous NaCl, dried over anhydrous Na₂SO₄, and concentrated under reduced pressure. The residue was purified by column chromatography to afford the title compound (yellow solid, 150 mg, yield 44%).

### Step B: 2-(2,6-dioxopiperidin-3-yl)-5-(hexahydropyrrolo[3,4-c]pyrrol-2(1H)-yl)isoindoline-1,3-dione

To a solution of tert-butyl 5-(2-(2,6-dioxopiperidin-3-yl)-1,3-dioxoisoindolin-5-yl)hexahydropyrrolo[3,4-c]pyrrole-2(1H)-carboxylate (150 mg, 0.32 mmol, 1.0 eq) in DCM (3 mL) was added TFA (3 mL) at 0°C. The mixture was stirred at rt for 1 h. Upon completion, the mixture was concentrated under reduced pressure to afford the title compound (yellow oil, 120 mg, crude), which was used directly without purification.

### Step C: N-(3-carbamoyl-1-(4-((5-(2-(2,6-dioxopiperidin-3-yl)-1,3-dioxoisoindol-5-ylhhexahydropyrrolo[3,4-c]|pyrrol-2(1H)-yl)methyl)phenyl)-1H-pyrazol-4-yl)-6-(trifluoromethyl)picolinamide

To a solution of 2-(2,6-dioxopiperidin-3-yl)-5-(hexahydropyrrolo[3,4-c]pyrrol-2(1H)-yl)isoindoline-1,3-dione (120 mg, 0.33 mmol, 1.0 eq) in DMF (5 mL) was added TEA (0.07 mL) at rt. The reaction solution was stirred at rt for 5 min. N-(3-Carbamoyl-1-(4-formylphenyl)-1H-pyrazol-4-yl)-6-(trifluoromethyl)picolinamide (158 mg, 0.39 mmol, 1.2 eq) and AcOH (0.04 mL, 0.65 mmol, 2.0 eq) were then added to the above reaction solution. The reaction solution was stirred at rt for 0.5 h. Sodium triacetoxyborohydride (137 mg, 0.65 mmol, 2.0 eq) was then added. The reaction solution was stirred at rt for 1 additional hour. Upon completion, the reaction was quenched with water and extracted with EtOAc (×3). The combined organic phases were washed with saturated aqueous NaCl, dried over anhydrous Na₂SO₄, and concentrated under reduced pressure. The residue was purified by HPLC to afford the title compound (yellow solid, 50 mg, yield 20%). **¹H NMR** (400 MHz, DMSO-*d*₆) δ 11.78 (s, 1H), 11.06 (s, 1H), 8.99 (s, 1H), 8.48-8.36 (m, 2H), 8.21 (d, *J* = 7.5 Hz, 1H), 7.99 (s, 1H), 7.92 (d, *J* = 8.4 Hz, 2H), 7.75 (s, 1H), 7.66 (d, *J* = 8.4 Hz, 1H), 7.46 (d, *J =* 8.4 Hz, 2H), 6.98 (s, 1H), 6.88 (d, *J =* 8.5 Hz, 1H), 5.16-4.92 (m, 1H), 3.71-3.66 (m, 2H), 3.65 (s, 2H), 3.40-3.37 (m, 2H), 3.04-2.97 (m, 2H), 2.94-2.82 (m, 1H), 2.66-2.52 (m, 6H), 2.08-1.93 (m, 1H). LC-MS (ESI): m/z 756 [M+H]⁺.

### Preparation Example 301. Synthesis of Compound A217

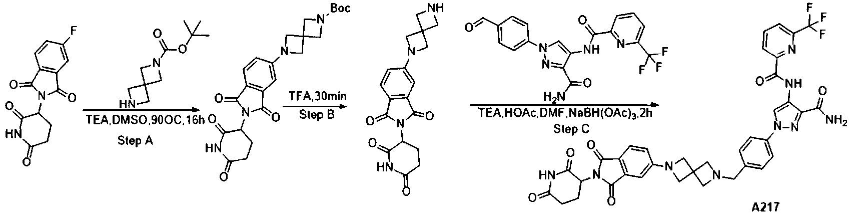

### Step A: Tert-butyl 6-(2-(2,6-dioxopiperidin-3-yl)-1,3-dioxoisoindolin-5-yl)-2,6-diazaspiro[3.3]heptane-2-carboxylate

To a solution of 2-(2,6-dioxopiperidin-3-yl)-5-fluoroisoindoline-1,3-dione (200 mg, 0.72 mmol, 1.0 eq) in dimethyl sulfoxide (10 mL) at rt were added TEA (TEA) (1 mL) and tert-butyl 2,6-diazaspiro[3.3]heptane-2-carboxylate (172 mg, 0.87 mmol, 1.2 eq). The mixture was stirred at 90°C under N₂ for 18 h. Upon completion, the mixture was cooled to rt, diluted in water, and extracted with EtOAc (×3). The combined organic phases were washed with saturated aqueous NaCl, dried over anhydrous Na₂SO₄, and concentrated under reduced pressure. The residue was purified by column chromatography to afford the title compound (yellow solid, 204 mg, yield 62%).

### Step B: 2-(2,6-dioxopiperidin-3-yl)-5-(2,6-diazaspiro[3.3]heptan-2-yl)isoindoline-1,3-dione

To a solution of tert-butyl 6-(2-(2,6-dioxopiperidin-3-yl)-1,3-dioxoisoindolin-5-yl)-2,6-diazaspiro[3.3]heptane-2-carboxylate (80 mg, 0.18 mmol, 1.0 eq) in DCM (5 mL) was added TFA (1 mL) at 0°C. The mixture was stirred at rt for 0.5 h. Upon completion, the mixture was concentrated under reduced pressure to afford the title compound (yellow solid, 60 mg, crude), which was used directly without purification.

### Step C: N-(3-carbamoyl-1-(4-((6-(2-(2,6-dioxopiperidin-3-yl)-1,3-dioxoisoindolin-5-yl)-2,6-diazaspiro[3.3]heptan-2-yl)methyl)phenyl)-1H-pyrazol-4-yl)-6-(trifluoromethyl)picolinamide

To a solution of 2-(2,6-dioxopiperidin-3-yl)-5-(2,6-diazaspiro[3.3]heptan-2-yl)isoindoline-1,3-dione (60 mg, 0.17 mmol, 1.0 eq) in DMF (5 mL) was added TEA (0.1 mL) at rt. The reaction solution was stirred at rt for 10 min. N-(3-Carbamoyl-1-(4-formylphenyl)-1H-pyrazol-4-yl)-6-(trifluoromethyl)picolinamide (82 mg, 0.2 mmol, 1.2 eq) and AcOH (0.2 mL) were added to the above reaction solution. The reaction solution was stirred at rt for 0.5 h. Sodium triacetoxyborohydride (71.76 mg, 0.339 mmol, 2.0 eq) was then added. The reaction solution was stirred at rt for an additional 2 h. Upon completion, the reaction was quenched with water and extracted with EtOAc (×3). The combined organic phases were washed with saturated aqueous NaCl, dried over anhydrous Na₂SO₄, and concentrated under reduced pressure. The residue was purified by HPLC to afford the title compound (yellow solid, 28 mg, yield 22%). **¹H NMR** (400 MHz, DMSO-*d*₆) δ 11.79 (s, 1H), 11.06 (s, 1H), 9.00 (s, 1H), 8.47-8.38 (m, 2H), 8.21 (d, *J =* 7.6 Hz, 1H), 8.01 (s, 1H), 7.93 (d, *J* = 8.2 Hz, 2H), 7.76 (s, 1H), 7.64 (d, *J =* 8.1 Hz, 1H), 7.45 (d, *J =* 8.3 Hz, 2H), 6.79 (s, 1H), 6.65 (d, *J =* 8.0 Hz, 1H), 5.08-5.02 (m, 1H), 4.13 (s, 4H), 3.62 (s, 2H), 3.38 (s, 4H), 2.91-2.83 (m, 1H), 2.62-2.51 (m, 2H), 2.04-1.98 (m, 1H). LCMS (ESI): m/z 742 [M+H]⁺.

### Preparation Example 302. Synthesis of Compound A218

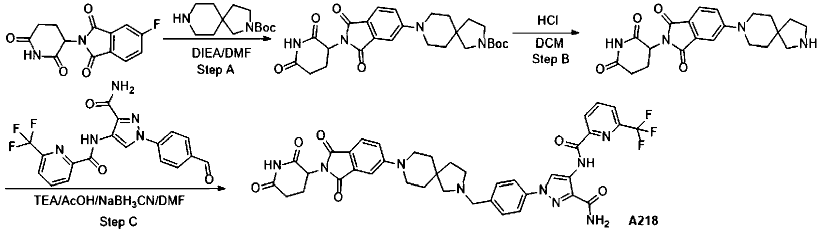

### Step A: Tert-butyl 8-(2-(2,6-dioxopiperidin-3-yl)-1,3-dioxoisoindolin-5-yl)-2,8-diazaspiro[4.5]decane-2-carboxylate

To a solution of 2-(2,6-dioxopiperidin-3-yl)-5-fluoroisoindoline-1,3-dione (700 mg, 2.534 mmol, 1.0 eq) in DMF (15 mL) at rt were added tert-butyl 2,8-diazaspiro[4.5]decane-2-carboxylate (730 mg, 3.037 mmol, 1.2 eq) and DIEA (980 mg, 7.582 mmol, 3.0 eq). The reaction solution was stirred at 100°C under N₂ for 2 h. Upon completion, the reaction solution was cooled to rt, diluted in EtOAc, washed sequentially with water and saturated aqueous NaCl, dried over anhydrous Na₂SO₄, filtered, and concentrated under reduced pressure. The residue was purified by column chromatography to afford the title compound (yellow oil, 900 mg, yield 64%).

### Step B: 2-(2,6-dioxopiperidin-3-yl)-5-(2,8-diazaspiro[4.5]decan-8-yl)isoindoline-1,3-dione

At 0°C, to a solution of tert-butyl 8-(2-(2,6-dioxopiperidin-3-yl)-1,3-dioxoisoindolin-5-yl)-2,8-diazaspiro[4.5]decane-2-carboxylate (400 mg, 0.806 mmol, 1.0 eq) in DCM (5 mL) was added 4 M HCl in 1,4-dioxane (1.5 mL, 6.0 mmol, 7.4 eq). The reaction solution was stirred at rt under N₂ for 1 h. The reaction solution was concentrated under reduced pressure to afford the title compound (yellow solid, 300 mg, crude), which was used directly without purification.

### Step C: N-(3-carbamoyl-1-(4-((8-(2-(2,6-dioxopiperidin-3-yl)-1,3-dioxoisoindolin-5-yl)-2,8-diazaspiro[4.5]dec-2-yl)methyl)phenyl)-1H-pyrazol-4-yl)-6-(trifluoromethyl)picolinamide

At rt, to a solution of 2-(2,6-dioxopiperidin-3-yl)-5-(2,8-diazaspiro[4.5]decan-8-yl)isoindoline-1,3-dione (100 mg, 0.248 mmol, 1.0 eq) in DMF (3 mL) was added TEA (0.05 mL, 0.360 mmol, 1.5 eq), and the mixture was stirred for 30 min. AcOH (0.06 mL, 1.048 mmol, 4.2 eq) and N-(3-carbamoyl-1-(4-formylphenyl)-1H-pyrazol-4-yl)-6-(trifluoromethyl)picolinamide (120 mg, 0.303 mmol, 1.2 eq) were then added. The mixture was stirred at rt for 18 h. Sodium cyanoborohydride (50 mg, 0.796 mmol, 3.2 eq) was then added to the above reaction solution. The mixture was stirred at 40°C for 2 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (yellow solid, 18 mg, yield 9%). **¹H NMR** (400 MHz, DMSO-*d*₆) δ 11.79 (s, 1H), 11.07 (s, 1H), 9.01 (s, 1H), 8.47-8.37 (m, 2H), 8.22 (d, *J =* 7.6 Hz, 1H), 8.14 (s, 1H), 8.01 (s, 1H), 7.94 (d, *J =* 8.2 Hz, 2H), 7.77 (s, 1H), 7.64 (d, *J* = 8.5 Hz, 1H), 7.49 (d, *J =* 8.3 Hz, 2H), 7.31 (s, 1H), 7.23 (d, *J* = 8.9 Hz, 1H), 5.06 (dd, *J =* 12.8, 5.3 Hz, 1H), 3.68 (s, 2H), 3.48 (s, 2H), 3.44-3.42 (m, 2H), 2.91-2.84 (m, 1H), 2.67-2.57 (m, 3H), 2.56-2.54 (m, 1H), 2.47 (s, 2H), 2.04-1.96 (m, 1H), 1.70-1.65 (m, 2H), 1.65-1.54 (m, 4H). LCMS (ESI): m/z 784 [M+H]⁺.

### Preparation Example 303. Synthesis of Compound A219

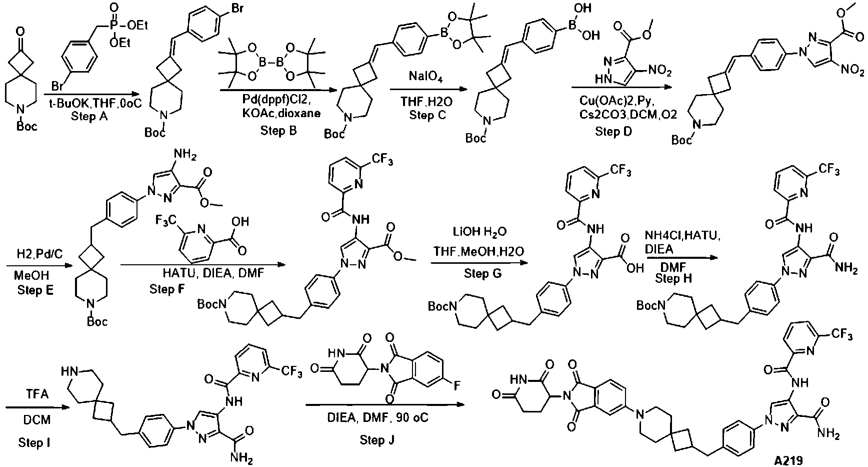

### Step A: Tert-butyl 2-(4-bromobenzylidene)-7-azaspiro[3.5]nonane-7-carboxylate

To a solution of tert-butyl 2-oxo-7-azaspiro[3.5]nonane-7-carboxylate (2 g, 8.357 mmol, 1.0 eq) and diethyl[(4-bromophenyl)methyl]phosphonate (2.82 g, 9.193 mmol, 1.1 eq) in THF (9 mL) was added potassium tert-butoxide (1.87 g, 16.715 mmol, 2.0 eq) at 0°C. The reaction solution was stirred at rt under N₂ for 3 h. Upon completion, the reaction solution was poured into saturated aqueous NH₄Cl and extracted with EtOAc (×3). The combined organic phases were washed with water and saturated aqueous NaCl, dried over anhydrous Na₂SO₄, and concentrated under reduced pressure. The residue was purified by column chromatography to afford the title compound (white solid, 2 g, yield 61%).

### Step B: Tert-butyl 2-(4-(4,4,5,5-tetramethyl-1,3,2-dioxaborolan-2-yl)benzylidene)-7-azaspiro[3.5]nonane-7-carboxylate

To a solution of tert-butyl 2-(4-bromobenzylidene)-7-azaspiro[3.5]nonane-7-carboxylate (2 g, 5.098 mmol, 1.0 eq) and 4,4,5,5-tetramethyl-2-(4,4,5,-5-tetramethyl-1,3,2-dioxaborolan-2-yl)-1,3,2-dioxolane (1.94 g, 7.646 mmol, 1.5 eq) in 1,4-dioxane (20 mL) were added potassium acetate (1.00 g, 10.195 mmol, 2.0 eq) and Pd(dppf)Cl₂ (0.37 g, 0.510 mmol, 0.1 eq) at rt. The reaction solution was stirred at 90°C under O₂ for 3 h. Upon completion, the mixture was cooled to rt, diluted in saturated aqueous NH₄Cl, and extracted with EtOAc (×3). The combined organic phases were washed with water and saturated aqueous NaCl, dried over anhydrous Na₂SO₄, and concentrated under reduced pressure. The residue was purified by column chromatography to afford the title compound (white solid, 1.7 g, yield 76%).

### Step C: (4-((7-(tert-Butoxycarbonyl)-7-azaspiro[3.5]nonan-2-ylidene)methyl)phenyl)boronic acid

At 0°C, to a solution of tert-butyl 2-(4-(4,4,5,5-tetramethyl-1,3,2-dioxaborolan-2-yl)benzylidene)-7-azaspiro[3.5]nonane-7-carboxylate (1.7 g, 3.869 mmol, 1.0 eq) in THF (12 mL) and water (4 mL) was added sodium periodate (2.48 g, 11.607 mmol, 3.0 eq). The reaction solution was stirred at rt under N₂ for 16 h. Upon completion, the reaction solution was filtered, and the filtrate was poured into saturated aqueous NaHCO₃ and extracted with EtOAc (×3). The combined organic phases were washed with water and saturated aqueous NaCl, dried over anhydrous Na₂SO₄, and concentrated under reduced pressure. The residue was purified by column chromatography to afford the title compound (white solid, 1.3 g, yield 94%).

### Step D: 2-(4-(3-(Methoxycarbonyl)-4-nitro-1H-pyrazol-1-yl)benzylidene)-7-azaspiro[3.5]nonane-7-carboxylate

To a solution of (4-((7-(tert-butoxycarbonyl)-7-azaspiro[3.5]nonan-2-ylidene)methyl)phenyl)boronic acid (1.3 g, 3.639 mmol, 1.0 eq), methyl 4-nitro-1H-pyrazole-3-carboxylate (0.93 g, 5.458 mmol, 1.5 eq), cesium carbonate (3.56 g, 10.916 mmol, 3.0 eq), and pyridine (1.766 mL, 21.833 mmol, 5.0 eq) in acetonitrile (13 mL) was added copper acetate (1.17 g, 5.84 mmol, 1.0 eq) at rt. The reaction solution was stirred at 80°C under O₂ for 16 h. Upon completion, the reaction solution was filtered, and the filtrate was diluted in water and extracted with DCM (×3). The combined organic phases were washed with water and saturated aqueous NaCl, dried over anhydrous Na₂SO₄, and concentrated under reduced pressure. The residue was purified by column chromatography to afford the title compound (yellow solid, 530 mg, yield 30%).

### Step E: Tert-butyl 2-(4-(4-amino-3-(methoxycarbonyl)-1H-pyrazol-1-yl)benzyl)-7-azaspiro[3.5]nonane-7-carboxylate

To a solution of 2-(4-(3-(methoxycarbonyl)-4-nitro-1H-pyrazol-1-yl)benzylidene)-7-azaspiro[3.5]nonane-7-carboxylate (530 mg, 1.098 mmol, 1.0 eq) in MeOH (5 mL) was added 10% palladium on carbon (1168.87 mg, 10.984 mmol, 10 eq) at rt. The reaction solution was purged with H₂ (using a hydrogen balloon) (×3) and stirred under H₂ at rt for 3 h. Upon completion, the mixture was filtered, and the filtrate was concentrated under reduced pressure. The residue was purified by column chromatography to afford the title compound (yellow oil, 390 mg, yield 78%).

### Step F: Tert-butyl 2-(4-(3-(methoxycarbonyl)-4-(6-(trifluoromethyl)picolinamido)-1H-pyrazol-1-yl)benzyl)-7-azaspiro[3.5]nonane-7-carboxylate

To a solution of tert-butyl 2-(4-(4-amino-3-(methoxycarbonyl)-1H-pyrazol-1-yl)benzyl)-7-azaspiro[3.5]nonane-7-carboxylate (390 mg, 0.858 mmol, 1.0 eq) in DMF (5 mL) at rt were added 6-(trifluoromethyl)pyridine-2-carboxylic acid (163.96 mg, 0.858 mmol, 1.0 eq), DIEA (443.56 mg, 3.432 mmol, 4.0 eq), and HATU (489.34 mg, 1.287 mmol). The reaction solution was stirred at rt under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 250 mg, yield 46%).

### Step G: 1-(4-((7-(tert-Butoxycarbonyl)-7-azaspiro[3.5]non-2-yl)methyl)phenyl)-4-(6-(trifluoromethyl)picolinamido)-1H-pyrazole-3-carboxylic acid

To a mixed solution of tert-butyl 2-(4-(3-(methoxycarbonyl)-4-(6-(trifluoromethyl)picolinamido)-1H-pyrazol-1-yl)benzyl)-7-azaspiro[3.5]nonane-7-carboxylate (250 mg, 0.398 mmol, 1.0 eq) in THF (2 mL), MeOH (1 mL), and water (1 mL) was added lithium hydroxide (41.78 mg, 0.996 mmol, 3.0 eq) at 0°C. The reaction solution was stirred at rt under N₂ for 2 h. Upon completion, the reaction solution was adjusted to pH 5-6 using 1 N HCl. The mixture was extracted with EtOAc (×3). The combined organic phases were washed with water and saturated aqueous NaCl, dried over anhydrous Na₂SO₄, filtered and concentrated under reduced pressure. The residue was purified by column chromatography to afford the title compound (yellow solid, 200 mg, yield 82%).

### Step H: Tert-butyl 2-(4-(3-amino-4-(6-(trifluoromethyl)picolinamido)-1H-pyrazol-1-yl)benzyl)-7-azaspiro[3.5]nonane-7-carboxylate

To a solution of 1-(4-((7-(tert-butoxycarbonyl)-7-azaspiro[3.5]nonan-2-yl)methyl)phenyl)-4-(6-(trifluoromethyl)picolinamido)-1H-pyrazole-3-carboxylic acid (220 mg, 0.359 mmol, 1.0 eq) in DMF (3 mL) at rt were added ammonium chloride (38.35 mg, 0.717 mmol, 2.0 eq), DIEA (92.68 mg, 0.717 mmol, 2.0 eq), and HATU (204.48 mg, 0.538 mmol, 1.5 eq). The reaction solution was stirred at rt under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 200 mg, yield 91%).

### Step I: N-(1-(4-((7-azaspiro[3.5]nonan-2-yl)methyl)phenyl)-3-formyl-1H-pyrazol-4-yl)-6-(trifluoromethyl)picolinamide

At 0°C, to a solution of tert-butyl 2-(4-(3-amino-4-(6-(trifluoromethyl)picolinamido)-1H-pyrazol-1-yl)benzyl)-7-azaspiro[3.5]nonane-7-carboxylate (200 mg, 0.326 mmol, 1.0 eq) in DCM (2 mL) was added TFA (0.5 mL). The reaction solution was stirred at rt under N₂ for 2 h. Upon completion, the reaction was quenched with 1N NaOH and extracted with DCM (×3). The combined organic phases were washed with water and saturated aqueous NaCl, dried over anhydrous Na₂SO₄, filtered, and concentrated under reduced pressure to afford the title compound (yellow oil, 150 mg, crude), which was used directly without purification.

### Step J: N-(3-Carbamoyl-1-(4-((7-(2-(2,6-dioxopiperidin-3-yl)-1,3-dioxoisoindolin-5-yl)-7-azaspiro[3.5]nonan-2-yl)methyl)phenyl)-1H-pyrazol-4-yl)-6-(trifluoromethyl)picolinamide

To a solution of N-(1-(4-((7-azaspiro[3.5]nonan-2-yl)methyl)phenyl)-3-formyl-1H-pyrazol-4-yl)-6-(trifluoromethyl)picolinamide (150 mg, 0.293 mmol, 1.0 eq) in DMF (2 mL) at rt were added 2-(2,6-dioxopiperidin-3-yl)-5-fluoroisoindoline-1,3-dione (161.68 mg, 0.585 mmol, 2.0 eq) and DIEA (75.65 mg, 0.585 mmol, 2.0 eq). The reaction solution was stirred at 90°C under N₂ for 1 h. Upon completion, the mixture was cooled to rt. The reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 20 mg, yield 9%). **¹H NMR** (400 MHz, DMSO-*d*₆) δ 11.79 (s, 1H), 11.07 (s, 1H), 8.98 (s, 1H), 8.45 (d, *J* = 7.5 Hz, 1H), 8.40 (s, 1H), 8.21 (s, 1H), 8.00 (s, 1H), 7.87 (d*, J =* 8.8 Hz, 2H), 7.77 (s, 1H), 7.63 (d, *J =* 8.6 Hz, 1H), 7.34 (s, 2H), 7.31 (d, *J =* 2.0 Hz, 1H), 7.23 (d, *J =* 6.6 Hz, 1H), 5.08-5.04 (m, 1H), 3.45-3.38 (m, 4H), 2.88 (d, *J* = 5.8 Hz, 1H), 2.77-2.74 (m, 2H), 2.65-2.57 (m, 2H), 1.97-1.94 (m, 2H), 1.64 (s, 4H), 1.58-1.53 (m, 4H). LCMS (ESI): m/z 769 [M+H]⁺.

### Preparation Example 304. Synthesis of Compound A220

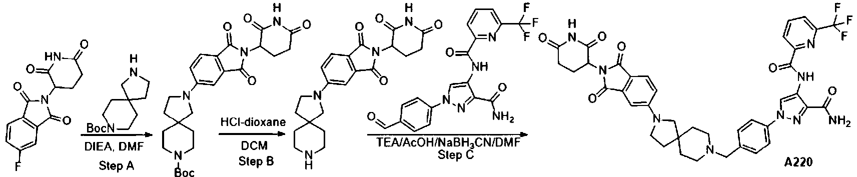

### Step A: Tert-butyl 2-(2-(2,6-dioxopiperidin-3-yl)-1,3-dioxoisoindolin-5-yl)-2,8-diazaspiro[4.5]decane-8-carboxylate

At 0°C, to a solution of 2-(2,6-dioxopiperidin-3-yl)-5-fluoroisoindoline-1,3-dione (500 mg, 1.81 mmol, 1.0 eq) in DMF (5 mL) were added tert-butyl 2,8-diazaspiro[4.5]decane-8-carboxylate (479 mg, 1.99 mmol, 1.1 eq) and DIEA (702 mg, 5.43 mmol, 3.0 eq). The mixture was stirred at 90°C for 5 h. Upon completion, the reaction was quenched with water, and extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow solid, 280 mg, yield 31%).

### Step B: 2-(2,6-dioxopiperidin-3-yl)-5-(2,8-diazaspiro[4.5]dec-2-yl)isoindoline-1,3-dione

To a solution of tert-butyl 2-(2-(2,6-dioxopiperidin-3-yl)-1,3-dioxoisoindolin-5-yl)-2,8-diazaspiro[4.5]decane-8-carboxylate (280 mg, 0.564 mmol, 1.0 eq) in DCM (5 mL) was added 4 M HCl in 1,4-dioxane (1.5 mL, 6.00 mmol, 10.0 eq) at 0°C. The reaction solution was stirred at rt under N₂ for 1 h. The reaction solution was concentrated under reduced pressure to give the title compound (yellow solid, 200 mg, crude), which was used directly without purification.

### Step C: N-(3-carbamoyl-1-(4-((2-(2-(2,6-dioxopiperidin-3-yl)-1,3-dioxoisoindolin-5-yl)-2,8-diazaspiro[4.5]decan-8-yl)methyl)phenyl)-1H-pyrazol-4-yl)-6-(trifluoromethyl)picolinamide

At rt, to a solution of 2-(2,6-dioxopiperidin-3-yl)-5-(2,8-diazaspiro[4.5]dec-2-yl)isoindoline-1,3-dione (200 mg, 0.504 mmol, 1.0 eq) in DMF (5 mL) was added TEA (102 mg, 1.01 mmol, 2.0 eq), and the mixture was stirred for 5 min. N-(3-carbamoyl-1-(4-formylphenyl)-1H-pyrazol-4-yl)-6-(trifluoromethyl)picolinamide and AcOH (151 mg, 2.52 mmol, 5.0 eq) were then added. The mixture was stirred at rt for 18 h. Sodium triacetoxyborohydride (95.1 mg, 1.51 mmol, 3.0 eq) was then added to the above reaction solution. The mixture was stirred at 40°C for 2 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (yellow solid, 100 mg, yield 25%). **¹H NMR** (400 MHz, DMSO-*d*₆) δ 11.79 (s, 1H), 11.06 (s, 1H), 9.01 (s, 1H), 8.52-8.32 (m, 2H), 8.21 (d, *J* = 7.6 Hz, 1H), 8.01 (s, 1H), 7.94 (d, *J =* 8.5 Hz, 2H), 7.77 (s, 1H), 7.64 (d, *J* = 8.4 Hz, 1H), 7.48 (d, *J =* 8.5 Hz, 2H), 6.92 (s, 1H), 6.84-6.78 (m, 1H), 5.09-5.01 (m, 1H), 3.58 (s, 2H), 3.50-3.44 (m, 2H), 3.41-3.36 (m, 2H), 3.30 (s, 2H), 2.94-2.82 (m, 1H), 2.62-2.54 (m, 2H), 2.45-2.32 (m, 2H), 2.05-1.95 (m, 1H), 1.94-1.82 (m, 2H), 1.66-1.53 (m, 4H). LCMS (ESI) m/z 784 [M+H]⁺.

### Preparation Example 305. Synthesis of Compound A221

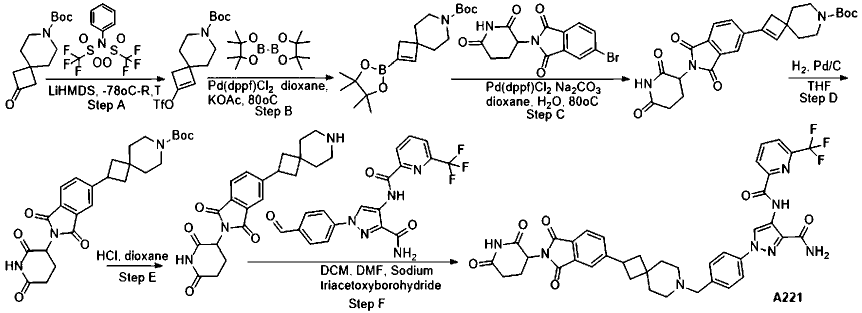

### Step A: Tert-butyl 2-(((trifluoromethyl)sulfonyl)oxy)-7-azaspiro[3.5]non-1-ene-7-carboxylate

To a solution of tert-butyl 2-oxo-7-azaspiro[3.5]nonane-7-carboxylate (2 g, 8.3 mmol, 1.0 eq) in THF (10 mL) was slowly added 1 M lithium bis(trimethylsilyl)amide in THF (16.6 mL, 16.6 mmol, 2.0 eq) at -78°C under N₂. The reaction solution was stirred at -78°C under N₂ for 1 h. 1,1,1-Trifluoro-N-phenyl-N-((trifluoromethyl)sulfonyl)methanesulfonamide (3.3 g, 9.2 mmol, 1.1 eq) was then added to the above reaction solution. The reaction solution was slowly warmed to rt and stirred at rt under N₂ for 18 h. Upon completion, the reaction was quenched with saturated aqueous NH₄Cl and extracted with EtOAc (×3). The combined organic phases were washed with water and saturated aqueous NaCl, dried over anhydrous Na₂SO₄, filtered, and concentrated under reduced pressure. The residue was purified by column chromatography to afford the title compound (colorless oil, 2 g, yield 64%).

### Step B: Tert-butyl 2-(4,4,5,5-tetramethyl-1,3,2-dioxaborolan-2-yl)-7-azaspiro[3.5]non-1-ene-7-carboxylate

To a solution of tert-butyl 2-(((trifluoromethyl)sulfonyl)oxy)-7-azaspiro[3.5]non-1-ene-7-carboxylate (2 g, 5.4 mmol, 1.0 eq) in 1,4-dioxane (20 mL) at rt were added 4,4,4',4',5,5',5',5'-octamethyl-2,2'-bis(1,3,2-dioxaborolane) (1.4 g, 5.4 mmol, 1.0 eq), Pd(dppf)Cl₂ (0.4 g, 0.5 mmol, 0.1 eq), and potassium acetate (1.1 g, 10.8 mmol, 2.0 eq). The reaction solution was stirred at 80°C under N₂ for 3 h. Upon completion, the reaction solution was used directly in the next step without further treatment.

### Step C: Tert-butyl 2-(2-(2,6-dioxopiperidin-3-yl)-1,3-dioxoisoindolin-5-yl)-7-azaspiro[3.5]non-1-ene-7-carboxylate

To a solution of tert-butyl 2-(4,4,5,5-tetramethyl-1,3,2-dioxaborolan-2-yl)-7-azaspiro[3.5]non-1-ene-7-carboxylate (2 g, 5.8 mmol, 1.0 eq) and 5-bromo-2-(2,6-dioxopiperidin-3-yl)isoindoline-1,3-dione (1.9 g, 5.8 mmol, 1.0 eq) in 1,4-dioxane (30 mL) and water (3 mL) were added sodium carbonate (1.8 g, 17.2 mmol, 3.0 eq) and Pd(dppf)Cl₂ (0.42 g, 0.58 mmol, 0.1 eq) at rt. The mixture was stirred at 80°C under N₂ for 3 h. Upon completion, the mixture was cooled to rt, diluted in water, extracted with EtOAc, washed with saturated aqueous NaCl, dried over anhydrous Na₂SO₄, and concentrated under reduced pressure. The residue was purified by column chromatography to afford the title compound (yellow solid, 1.5 g, yield 55%).

### Step D: Tert-butyl 2-(2-(2,6-dioxopiperidin-3-yl)-1,3-dioxoisoindolin-5-yl)-7-azaspiro[3.5]nonane-7-carboxylate

To a solution of tert-butyl 2-(2-(2,6-dioxopiperidin-3-yl)-1,3-dioxoisoindolin-5-yl)-7-azaspiro[3.5]non-1-ene-7-carboxylate (500 mg, 1.0 mmol, 1.0 eq) in THF (10 mL) was added 10% palladium on carbon (100 mg) at rt. The reaction solution was purged with H₂ (using a hydrogen balloon) (×3) and stirred under H₂ at 40°C for 16 h. Upon completion, the mixture was filtered, and the filtrate was concentrated under reduced pressure. The residue was purified by column chromatography to afford the title compound (white solid, 400 mg, yield 80%).

### Step E: 2-(2,6-dioxopiperidin-3-yl)-5-(7-azaspiro[3.5]nonan-2-yl)isoindoline-1,3-dione

At 0°C, to a solution of tert-butyl 2-(2-(2,6-dioxopiperidin-3-yl)-1,3-dioxoisoindolin-5-yl)-7-azaspiro[3.5]nonane-7-carboxylate (400 mg, 0.8 mmol, 1.0 eq) in DCM (10 mL) was added 4 M HCl in 1,4-dioxane (2 mL, 8 mmol, 10 eq). The reaction solution was stirred at rt under N₂ for 0.5 h. The reaction solution was concentrated under reduced pressure to afford the title compound (white solid, 300 mg, crude), which was used directly without purification.

### Step F: N-(3-Carbamoyl-1-(4-((2-(2-(2,6-dioxopiperidin-3-yl)-1,3-dioxoisoindolin-5-yl)-7-azaspiro[3.5]nonan-7-yl)methyl)phenyl)-1H-pyrazol-4-yl)-6-(trifluoromethyl)picolinamide

To a solution of 2-(2,6-dioxopiperidin-3-yl)-5-(7-azaspiro[3.5]nonan-2-yl)isoindoline-1,3-dione (300 mg, 0.8 mmol, 1.0 eq) in DCM (10 mL) at rt was added N-(3-amino-1-(4-(piperazin-1-yl)phenyl)-1H-pyrazol-4-yl)-6-(trifluoromethyl)pyridinecarboxamide (348 mg, 0.9 mmol, 1.1 eq). The mixture was stirred at rt for 2 h. Sodium triacetoxyborohydride (829 mg, 3.9 mmol, 5.0 eq) was then added to the above reaction solution. The mixture was stirred at rt for 2 h. Upon completion, the mixture was diluted in water, extracted with DCM (×3), washed with saturated aqueous NaCl, dried over anhydrous Na₂SO₄, filtered, and concentrated under reduced pressure. The residue was purified by column chromatography to afford the title compound (white solid, 52 mg, yield 9%). **¹H NMR** (400 MHz, CDCl₃) δ 11.79 (s, 1H), 11.12 (s, 1H), 9.01 (s, 1H), 8.46 (d, *J =* 7.5 Hz, 1H), 8.40 (t, *J =* 7.8 Hz, 1H), 8.22 (d, *J =* 7.6 Hz, 1H), 8.01 (s, 1H), 7.94 (d, *J* = 8.3 Hz, 2H), 7.84 (d, *J* = 7.7 Hz, 1H), 7.78 (s, 1H), 7.76-7.71 (m, 2H), 7.48 (d, *J =* 8.3 Hz, 2H), 5.14 (dd, *J =* 12.9, 5.3 Hz, 1H), 3.72 (p, *J* = 9.2 Hz, 1H), 3.52 (s, 2H), 2.96-2.80 (m, 1H), 2.65-2.52 (m, 2H), 2.42 (s, 2H), 2.29 (t, *J* = 9.9 Hz, 3H), 2.11-2.01 (m, 1H), 1.94-1.82 (m, 2H), 1.76 (s, 2H), 1.57 (s, 2H). LCMS (ESI): m/z 769 [M+H]⁺.

### Preparation Example 306. Synthesis of Compound A222

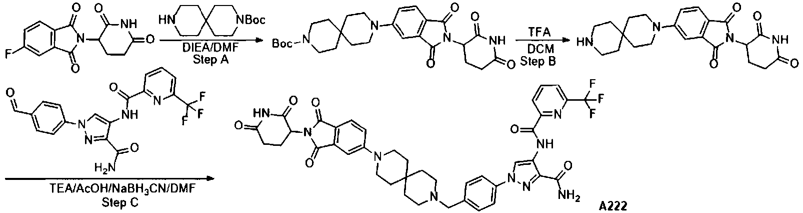

### Step A: Tert-butyl 9-(2-(2,6-dioxopiperidin-3-yl)-1,3-dioxoisoindolin-5-yl)-3,9-diazaspiro[5.5]undecane-3-carboxylate

To a solution of 2-(2,6-dioxopiperidin-3-yl)-5-fluoroisoindoline-1,3-dione (700 mg, 2.534 mmol, 1.0 eq) and tert-butyl 3,9-diazaspiro[5.5]undecane-3-carboxylate (709 mg, 2.788 mmol, 1.1 eq) in DMF (10 mL) was added DIEA (981 mg, 7.62 mmol, 3.0 eq) at rt. The reaction solution was stirred at 90°C for 5 h. Upon completion, the mixture was cooled to rt, diluted in water, and extracted with EtOAc (×3). The combined organic phases were washed with water and saturated aqueous NaCl, dried over anhydrous Na₂SO₄, filtered, and concentrated under reduced pressure. The residue was purified by column chromatography to afford the title compound (yellow solid, 700 mg, yield 54%).

### Step B: 2-(2,6-dioxopiperidin-3-yl)-5-(3,9-diazaspiro[5.5]undecan-3-yl)isoindoline-1,3-dione

At 0°C, to a solution of tert-butyl 9-(2-(2,6-dioxopiperidin-3-yl)-1,3-dioxoisoindolin-5-yl)-3,9-diazaspiro[5.5]undecane-3-carboxylate (300 mg, 1.036 mmol, 1.0 eq) in DCM (5 mL) was added TFA (1 mL). The reaction solution was stirred at rt under N₂ for 1 h. The reaction solution was concentrated under reduced pressure to give the title compound (yellow solid, 200 mg, crude), which was used directly without purification.

### Step C: N-(3-carbamoyl-1-(4-((9-(2-(2,6-dioxopiperidin-3-yl)-1,3-dioxoisoindolin-5-yl)-3,9-diazaspiro[5.5]undecan-3-yl)methyl)phenyl)-1H-pyrazol-4-yl)-6-(trifluoromethyl)picolinamide

To a solution of 2-(2,6-dioxopiperidin-3-yl)-5-(3,9-diazaspiro[5.5]undecan-3-yl)isoindoline-1,3-dione (200 mg, 0.487 mmol, 1.0 eq) in DMF (5 mL) was added TEA (123 mg, 1.21 mmol, 2.5 eq) at rt, and the mixture was stirred for 5 min. AcOH (292 mg, 4.87 mmol, 6.0 eq) and N-(3-carbamoyl-1-(4-formylphenyl)-1H-pyrazol-4-yl)-6-(trifluoromethyl)picolinamide (196 mg, 0.487 mmol, 1.0 eq) were then added. The mixture was stirred at rt for 18 h. Sodium triacetoxyborohydride (131 mg, 2.04 mmol, 4.0 eq) was then added to the above reaction solution. The mixture was stirred at 40°C for 2 h. Upon completion, the mixture was diluted in water, extracted with EtOAc (×3), washed with saturated aqueous NaCl, dried over anhydrous Na₂SO₄, filtered, and concentrated under reduced pressure. The residue was purified by column chromatography to afford the title compound (yellow solid, 41.2 mg, yield 11%). **¹H NMR** (400 MHz, DMSO-*d*₆) δ 11.79 (s, 1H), 11.07 (s, 1H), 9.01 (s, 1H), 8.47-8.38 (m, 2H), 8.22 (d, *J =* 7.5 Hz, 1H), 8.15 (s, 1H), 8.01 (s, 1H), 7.94 (d, *J* = 8.6 Hz, 2H), 7.77 (s, 1H), 7.65 (d, *J* = 8.6 Hz, 1H), 7.47 (d, *J* = 8.5 Hz, 2H), 7.30 (s, 1H), 7.22 (d, *J =* 8.7 Hz, 1H), 5.14-4.94 (m, 1H), 3.57 (s, 2H), 3.49-3.42 (m, 5H), 2.92-2.82 (m, 1H), 2.68-2.54 (m, 2H), 2.43-2.39 (m, 3H), 2.06-1.97 (m, 1H), 1.59-1.46 (m, 8H). LCMS (ESI): m/z 798 [M+H]⁺.

### Preparation Example 307. Synthesis of Compound A223

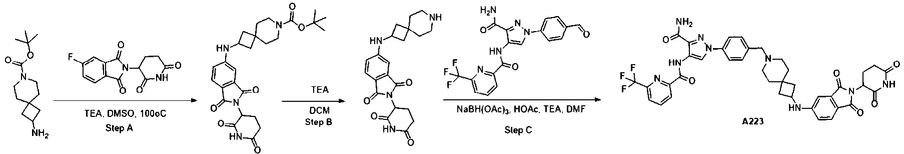

### Step A: Tert-butyl 2-((2-(2,6-dioxopiperidin-3-yl)-1,3-dioxoisoindolin-5-yl)amino)-7-azaspiro[3.5]nonane-7-carboxylate

To a solution of tert-butyl 2-amino-7-azaspiro[3.5]nonane-7-carboxylate (200 mg, 0.83 mmol, 1.0 eq) in dimethyl sulfoxide (5 mL) at rt were added TEA (TEA) (0.2 mL) and 2-(2,6-dioxopiperidin-3-yl)-5-fluoroisoindoline-1,3-dione (200 mg, 0.83 mmol, 1.0 eq). The mixture was stirred at 100°C under N₂ for 18 h. Upon completion, the mixture was cooled to rt, diluted in water, and extracted with EtOAc (×3). The combined organic phases were washed with saturated aqueous NaCl, dried over anhydrous Na₂SO₄, and concentrated under reduced pressure. The residue was purified by column chromatography to afford the title compound (yellow solid, 320 mg, yield 77%).

### Step B: 5-(7-azaspiro[3.5]nonan-2-yl)amino)-2-(2,6-dioxopiperidin-3-yl)isoindoline-1,3-dione

At 0°C, to a solution of tert-butyl 2-((2-(2,6-dioxopiperidin-3-yl)-1,3-dioxoisoindolin-5-yl)amino)-7-azaspiro[3.5]nonane-7-carboxylate (100 mg, 0.2 mmol, 1.0 eq) in DCM (3 mL) was added TFA (1 mL). The mixture was stirred at rt for 0.5 h. Upon completion, the mixture was concentrated under reduced pressure to afford the title compound (yellow solid, 75 mg, crude), which was used directly without purification.

### Step C: N-(3-carbamoyl-1-(4-(2-(((2-(2,6-dioxopiperidin-3-yl)-1,3-dioxoisoindolin-5-yl)amino)-7-azaspiro[3.5]nonan-7-yl)methyl)phenyl)-1H-pyrazol-4-yl)-6-(trifluoromethyl)picolinamide

To a solution of 5-(7-azaspiro[3.5]nonan-2-yl)amino)-2-(2,6-dioxopiperidin-3-yl)isoindoline-1,3-dione (75 mg, 0.19 mmol, 1.0 eq) in DMF (3 mL) at rt was added TEA (0.1 mL). The reaction solution was stirred at rt for 10 min. To the above mixture were sequentially added 1-(4-formylphenyl)-4-({[6-(trifluoromethyl)pyridin-2-yl]carbonyl}amino)pyrazole-3-carboxamide (91.56 mg, 0.227 mmol, 1.2 eq) and AcOH (0.2 mL). The reaction solution was stirred at rt for 0.5 h. Sodium triacetoxyborohydride (80.17 mg, 0.38 mmol, 2.0 eq) was then added. The reaction solution was stirred at rt for an additional 2 h. Upon completion, the reaction was quenched with water and extracted with EtOAc (×3). The combined organic phases were washed with saturated aqueous NaCl, dried over anhydrous Na₂SO₄, and concentrated under reduced pressure. The residue was purified by HPLC to afford the title compound (yellow solid, 25 mg, yield 17%). ¹HNMR (400 MHz, DMSO-*d*₆) δ 11.79 (s, 1H), 11.05 (s, 1H), 9.02 (s, 1H), 8.46 (d, *J =* 7.7 Hz, 1H), 8.42-8.38 (m, 1H), 8.22 (d, *J =* 7.6 Hz, 1H), 8.14 (s, 0.5H), 8.00 (s, 1H), 7.95 (d, *J =* 8.4 Hz, 2H), 7.77 (s, 1H), 7.56 (d, *J =* 8.3 Hz, 1H), 7.49 (d, *J* = 8.4 Hz, 2H), 7.35 (d, *J =* 5.9 Hz, 1H), 6.86 (s, 1H), 6.78 (d, *J* = 8.4 Hz, 1H), 5.03 (dd, *J =* 12.9, 5.4 Hz, 1H), 4.03-3.97 (m, 1H), 3.60 (s, 2H), 2.86 (d, *J =* 11.9 Hz, 1H), 2.62-2.52 (m, 2H), 2.47 (s, 2H), 2.40-2.30 (m, 4H), 2.02-1.97 (m, 1H), 1.76 (s, 1H), 1.70-1.57 (m, 6H). LC-MS (ESI): m/z 784 [M+H]⁺.

### Preparation Example 308. Synthesis of Compound A224

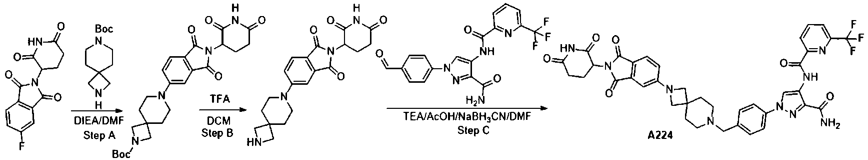

### Step A: Tert-butyl 7-(2-(2,6-dioxopiperidin-3-yl)-1,3-dioxoisoindolin-5-yl)-2,7-diazaspiro[3.5]nonane-2-carboxylate

To a solution of 2-(2,6-dioxopiperidin-3-yl)-5-fluoroisoindoline-1,3-dione (500 mg, 1.810 mmol, 1.0 eq) and tert-butyl 2,7-diazaspiro[3.5]nonane-7-carboxylate (450 mg, 1.99 mmol, 1.1 eq) in DMF (5 mL) was added DIEA (701 mg, 5.43 mmol, 3.0 eq) at rt. The reaction solution was stirred at 90°C for 5 h. Upon completion, the mixture was cooled to rt, diluted in water, and extracted with EtOAc (×3). The combined organic phases were washed with water and saturated aqueous NaCl, dried over anhydrous Na₂SO₄, filtered, and concentrated under reduced pressure. The residue was purified by column chromatography to afford the title compound (yellow solid, 500 mg, yield 57%).

### Step B: 2-(2,6-dioxopiperidin-3-yl)-5-(2,7-diazaspiro[3.5]nonan-7-yl)isoindoline-1,3-dione

At 0°C, to a solution of tert-butyl 7-(2-(2,6-dioxopiperidin-3-yl)-1,3-dioxoisoindolin-5-yl)-2,7-diazaspiro[3.5]nonane-2-carboxylate (500 mg, 1.04 mmol, 1.0 eq) in DCM (5 mL) was added TFA (1 mL). The reaction solution was stirred at rt under N₂ for 1 h. The reaction solution was concentrated under reduced pressure to afford the title compound (yellow solid, 300 mg, crude), which was used directly without purification.

### Step C: N-(3-carbamoyl-1-(4-((2-(2-(2,6-dioxopiperidin-3-yl)-1,3-dioxoisoindolin-5-yl)-2,7-diazaspiro[3.5]nonan-7-yl)methyl)phenyl)-1H-pyrazol-4-yl)-6-(trifluoromethyl)picolinamide

At rt, to a solution of 2-(2,6-dioxopiperidin-3-yl)-5-(2,7-diazaspiro[3.5]nonan-7-yl)isoindoline-1,3-dione (200 mg, 0.52 mmol, 1.0 eq) in DMF (5 mL) was added TEA (132 mg, 1.31 mmol, 2.5 eq), and the mixture was stirred for 5 min. AcOH (188 mg, 3.14 mmol, 6.0 eq) and N-(3-carbamoyl-1-(4-formylphenyl)-1H-pyrazol-4-yl)-6-(trifluoromethyl)picolinamide (211 mg, 0.52 mmol, 1.0 eq) were then added. The mixture was stirred at rt for 18 h. Sodium triacetoxyborohydride (131 mg, 2.04 mmol, 4.0 eq) was then added to the above reaction solution. The mixture was stirred at 40°C for 2 h. Upon completion, the mixture was cooled to rt, quenched with water, extracted with EtOAc (×3), washed with saturated aqueous NaCl, dried over anhydrous Na₂SO₄, filtered, and concentrated under reduced pressure. The residue was purified by column chromatography to afford the title compound (yellow solid, 33.8 mg, yield 8%). **¹H NMR** (400 MHz, DMSO-*d*₆) δ 11.80 (s, 1H), 11.06 (s, 1H), 9.01 (s, 1H), 8.47-8.37 (m, 2H), 8.22 (d, *J =* 7.6 Hz, 1H), 8.14 (s, 1H), 8.01 (s, 1H), 7.95 (d, *J =* 8.6 Hz, 2H), 7.78 (s, 1H), 7.63 (d, *J =* 8.3 Hz, 1H), 7.48 (d, *J =* 8.5 Hz, 2H), 6.78 (d, *J =* 1.8 Hz, 1H), 6.68-6.62 (m, 1H), 5.09-5.01 (m, 1H), 3.76 (s, 4H), 3.53 (s, 2H), 2.94-2.81 (m, 1H), 2.69-2.52 (m, 2H), 2.48-2.30 (m, 4H), 2.03-1.96 (m, 1H), 1.85-1.72 (m, 4H). LCMS (ESI) m/z: 770 [M+H]⁺.

### Preparation Example 309. Synthesis of Compound A225

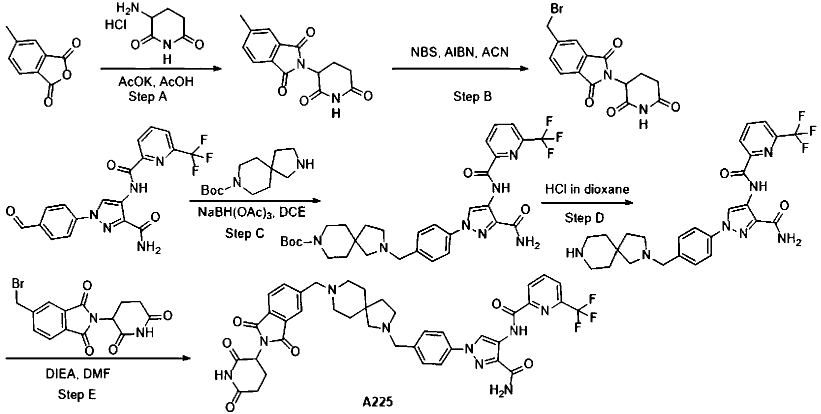

### Step A: 2-(2,6-Dioxopiperidin-3-yl)-5-methylisoindoline-1,3-dione

To a solution of 5-methylisobenzofuran-1,3-dione (2 g, 12.3 mmol, 1.0 eq) in AcOH (20 mL) were added 3-aminopiperidine-2,6-dione (2.03 g, 12.3 mmol, 1.0 eq) and potassium acetate (2.42 g, 24.7 mmol, 2.0 eq) at rt. The reaction solution was stirred at 110°C under N₂ for 16 h. Upon completion, the reaction solution was cooled to rt, diluted in EtOAc, washed sequentially with water and saturated aqueous NaCl, dried over anhydrous Na₂SO₄, filtered, and concentrated under reduced pressure. The residue was purified by column chromatography to afford the title compound (brown solid, 3.3 g, crude).

### Step B: 5-(Bromomethyl)-2-(2,6-dioxopiperidin-3-yl)isoindoline-1,3-dione

To a solution of 2-(2,6-dioxopiperidin-3-yl)-5-methylisoindoline-1,3-dione (3.3 g, 12.1 mmol, 1.0 eq) in acetonitrile (35 mL) were added N-bromosuccinimide (2.59 g, 14.5 mmol, 1.2 eq) and azobisisobutyronitrile (0.30 g, 1.82 mmol, 0.15 eq) at rt. The reaction solution was stirred at 90°C under N₂ for 16 h. Upon completion, the reaction solution was cooled to rt, diluted in water and stirred. The precipitated solid was collected by filtration. The crude was washed with EtOAc and dried completely to afford the title compound (brown solid, 3.5 g, crude).

### Step C: Tert-butyl 2-(4-(3-amino-4-(6-(trifluoromethyl)picolinamido)-1H-pyrazol-1-yl)benzyl)-2,8-diazaspiro[4.5]decane-8-carboxylate

To a solution of N-(3-carbamoyl-1-(4-formylphenyl)-1H-pyrazol-4-yl)-6-(trifluoromethyl)picolinamide (700 mg, 1.74 mmol, 1.0 eq) in 1,2-dichloroethane (15 mL) was added tert-butyl 2,8-diazaspiro[4.5]decane-8-carboxylate (459 mg, 1.91 mmol, 1.1 eq) at rt. The mixture was stirred at rt for 2 h. Sodium triacetoxyborohydride (736 mg, 3.47 mmol, 2.0 eq) was then added to the above reaction solution. The mixture was stirred at rt for 16 h. Upon completion, the mixture was diluted in water, extracted with DCM (×3), washed with saturated aqueous NaCl, dried over anhydrous Na₂SO₄, filtered, and concentrated under reduced pressure. The residue was purified by column chromatography to afford the title compound (white solid, 700 mg, yield 64%).

### Step D: N-(1-(4-((2,8-Diazaspiro[4.5]decan-2-yl)methyl)phenyl)-3-formyl-1H-pyrazol-4-yl)-6-(trifluoromethyl)picolinamide

To a solution of tert-butyl 2-(4-(3-amino-4-(6-(trifluoromethyl)picolinamido)-1H-pyrazol-1-yl)benzyl)-2,8-diazaspiro[4.5]decane-8-carboxylate (700 mg, 1.12 mmol, 1.0 eq) in DCM (20 mL) was added 4 M HCl in 1,4-dioxane (5 mL). The reaction solution was stirred at rt under N₂ for 3 h. The reaction solution was concentrated under reduced pressure to afford the title compound (brown oil, 550 mg, crude), which was used directly without purification.

### Step E: N-(3-Carbamoyl-1-(4-((8-((2-(2,6-dioxopiperidin-3-yl)-1,3-dioxoisoindolin-5-yl)methyl)-2,8-diazaspiro[4.5]decan-2-yl)methylphenyl)-1H-pyrazol-4-yl)-6-(trifluoromethyl)picolinamide

To a solution of N-(1-(4-((2,8-diazaspiro[4.5]decan-2-yl)methyl)phenyl)-3-formyl-1H-pyrazol-4-yl)-6-(trifluoromethyl)picolinamide (200 mg, 0.379 mmol, 1.0 eq) in DMF (2 mL) were added 5-(bromomethyl)-2-(2,6-dioxopiperidin-3-yl)isoindoline-1,3-dione (133 mg, 0.379 mmol, 1.0 eq) and DIEA (98 mg, 0.758 mmol, 2.0 eq) at rt. The reaction solution was stirred at 50°C under N₂ for 2 h. Upon completion, the reaction solution was cooled to rt, diluted in EtOAc, washed sequentially with water and saturated aqueous NaCl, dried over anhydrous Na₂SO₄, filtered, and concentrated under reduced pressure. The residue was purified by HPLC to afford the title compound (white solid, 150 mg, yield 50%). **¹H NMR** (400 MHz, DMSO-*d*₆) δ 11.80 (s, 1H), 11.16 (s, 1H), 10.62 (s, 1H), 10.03 (s, 1H), 9.12 (s, 1H), 8.43 (dt, *J* = 15.5, 7.6 Hz, 2H), 8.23 (dd, *J* = 7.6, 1.0 Hz, 1H), 8.14 (d, *J* = 8.0 Hz, 2H), 8.08 (s, 1H), 8.07-8.01 (m, 2H), 7.95 (d, *J =* 6.9 Hz, 1H), 7.85 (s, 1H), 7.71 (d, *J* = 7.6 Hz, 2H), 5.19 (dd, *J =* 12.7, 5.4 Hz, 1H), 4.48 (d, *J =* 29.4 Hz, 4H), 3.53 (d, *J =* 19.0 Hz, 2H), 3.34 (s, 4H), 3.12 (s, 3H), 2.96-2.81 (m, 1H), 2.71-2.52 (m, 2H), 2.09-2.06 (m, 2H), 1.87-1.76 (m, 4H). LCMS (ESI): m/z 798 [M+H]⁺.

### Preparation Example 310. Synthesis of Compound A226

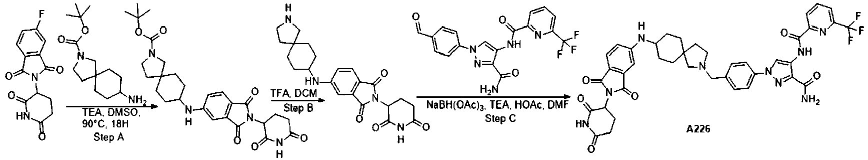

### Step A: Tert-butyl 8-((2-(2,6-dioxopiperidin-3-yl)-1,3-dioxoisoindolin-5-yl)amino)-2-azaspiro[4.5]decane-2-carboxylate

To a solution of tert-butyl 8-amino-2-azaspiro[4.5]decane-2-carboxylate (217 mg, 0.8 mmol, 1.0 eq) in dimethyl sulfoxide (10 mL) at rt were added TEA (0.2 mL, 1.6 mmol, 2.0 eq) and 2-(2,6-dioxopiperidin-3-yl)-5-fluoroisoindoline-1,3-dione (200 mg, 0.8 mmol, 1.0 eq). The mixture was stirred at 90°C under N₂ for 18 h. Upon completion, the mixture was cooled to rt, diluted in water, and extracted with EtOAc (×3). The combined organic phases were washed with saturated aqueous NaCl, dried over anhydrous Na₂SO₄, and concentrated under reduced pressure. The residue was purified by column chromatography to afford the title compound (yellow solid, 180 mg, yield 45%).

### Step B: 5-(2-azaspiro[4.5]dec-8-yl)amino)-2-(2,6-dioxopiperidin-3-yl)isoindoline-1,3-dione

To a solution of tert-butyl 8-((2-(2,6-dioxopiperidin-3-yl)-1,3-dioxoisoindolin-5-yl)amino)-2-azaspiro[4.5]decane-2-carboxylate (180 mg, 0.4 mmol, 1.0 eq) in DCM (3 mL) was added TFA (3 mL) at 0°C. The mixture was stirred at rt for 0.5 h. Upon completion, the mixture was concentrated under reduced pressure to afford the title compound (yellow solid, 140 mg, crude), which was used directly without purification.

### Step C: N-(3-carbamoyl-1-(4-((8-((2-(2,6-dioxopiperidin-3-yl)-1,3-dioxoisoindolin-5-yl)amino)-2-azaspiro[4.5]dec-2-yl)methyl)phenyl)-1H-pyrazol-4-yl)-6-(trifluoromethyl)picolinamide

To a solution of 5-(2-azaspiro[4.5]dec-8-yl)amino)-2-(2,6-dioxopiperidin-3-yl)isoindoline-1,3-dione (140 mg, 0.35 mmol, 1.0 eq) in DMF (5 mL) at rt was added TEA (35 mg, 0.35 mmol, 1.0 eq). The reaction solution was stirred at rt for 10 min. To the above mixture were added N-(3-carbamoyl-1-(4-formylphenyl)-1H-pyrazol-4-yl)-6-(trifluoromethyl)picolinamide (165 mg, 0.4 mmol, 1.2 eq) and AcOH (20 mg, 0.3 mmol, 1.0 eq). The reaction solution was stirred at rt for 0.5 h. Then, sodium triacetoxyborohydride (145 mg, 0.7 mmol, 2.0 eq) was added. The reaction solution was stirred at rt for 1 additional hour. Upon completion, the reaction was quenched with water and extracted with EtOAc (×3). The combined organic phases were washed with saturated aqueous NaCl, dried over anhydrous Na₂SO₄, and concentrated under reduced pressure. The residue was purified by HPLC to afford the title compound (yellow solid, 100 mg, yield 37%). **¹H NMR** (400 MHz, DMSO-*d*₆) δ 11.79 (s, 1H), 11.04 (s, 1H), 9.02 (s, 1H), 8.48-8.34 (m, 2H), 8.22 (d, *J* = 7.4 Hz, 1H), 8.13 (s, 0.77H), 8.03-7.92 (m, 3H), 7.78 (s, 1H), 7.56-7.47 (m, 3H), 6.98-6.92 (m, 1H), 6.90-6.81 (m, 1H), 5.02 (dd, *J =* 12.3, 4.7 Hz, 1H), 3.74 (d, *J =* 14.9 Hz, 2H), 2.85 (d, *J =* 12.1 Hz, 1H), 2.69 (s, 2H), 2.57-2.53 (m, 2H), 2.46-2.30 (m, 2H), 2.02-1.96 (m, 1H), 1.84 (d, *J* = 10.9 Hz, 2H), 1.74-1.58 (m, 4H), 1.51-1.43 (m, 2H), 1.36-1.29 (m, 2H), 1.18 (s, 1H). LCMS (ESI): m/z 798 [M+H]⁺.

### Preparation Example 311. Synthesis of Compound A227

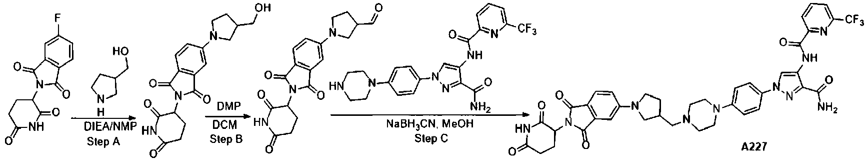

### Step A: 2-(2,6-dioxopiperidin-3-yl)-5-(3-(hydroxymethyl)pyrrolidin-1-yl)isoindoline-1,3-dione

To a solution of 2-(2,6-dioxopiperidin-3-yl)-5-fluoroisoindoline-1,3-dione (1.1 g, 3.9 mmol, 1.0 eq) in N-methylpyrrolidone (11 mL) at rt were added DIEA (1.54 g, 11.9 mmol, 3.0 eq) and tetrahydro-1H-pyrrol-3-ylmethanol (0.4 g, 3.9 mmol, 1.0 eq). The mixture was stirred at 120°C for 0.5 h. Upon completion, the reaction was quenched with water, and extracted with EtOAc (×3). The combined organic phases were washed with water and saturated aqueous NaCl, dried over anhydrous Na₂SO₄, and concentrated under reduced pressure. The residue was purified by reverse-phase column chromatography to afford the title compound (yellow solid, 1.2 g, yield 81%). LCMS (ESI) m/z 358 [M+H]⁺.

### Step B: 1-(2-(2,6-dioxopiperidin-3-yl)-1,3-dioxoisoindolin-5-yl)pyrrolidine-3-carbaldehyde

To a solution of 2-(2,6-dioxopiperidin-3-yl)-5-(3-(hydroxymethyl)pyrrolidin-1-yl)isoindoline-1,3-dione (800 mg, 2.2 mmol, 1.0 eq) in DCM (15 mL) was added DMP (1.4 g, 3.3 mmol, 1.5 eq) at 0°C. The mixture was stirred at rt under N₂ for 2 h. Upon completion, the mixture was filtered and concentrated under reduced pressure. The residue was purified by column chromatography to afford the title compound (yellow solid, 700 mg, yield 84%). LCMS (ESI) m/z: 356[M+H]⁺.

### Step C: N-(3-carbamoyl-1-(4-((1-(2-(2,6-dioxopiperidin-3-yl)-1,3-dioxoisoindol-5-yl)pyrrolidin-3-yl)methyl)piperazin-1-yl)phenyl)-1H-pyrazol-4-yl)-6-(trifluoromethyl)picolinamide

At rt, to a solution of N-(3-carbamoyl-1-(4-((1-(2-(2,6-dioxopiperidin-3-yl)-1,3-dioxoisoindol-5-yl)pyrrolidin-3-yl)methyl)piperazin-1-yl)phenyl)-1H-pyrazol-4-yl)-6-(trifluoromethyl)picolinamide (388 mg, 0.84 mmol, 1.0 eq) in MeOH (20 mL) was added TEA (0.117 mL, 0.84 mmol, 1.0 eq), and the mixture was stirred for 5 min. AcOH (0.097 mL, 1.69 mmol, 2.0 eq) and 1-(2-(2,6-dioxopiperidin-3-yl)-1,3-dioxoisoindolin-5-yl)pyrrolidine-3-carbaldehyde (300 mg, 0.84 mmol, 1.0 eq) were added. The mixture was stirred at rt for 18 h. Sodium cyanoborohydride (212.21 mg, 3.4 mmol, 4.0 eq) was then added to the above reaction solution. The mixture was stirred at 40°C for 2 h. Upon completion, the mixture was diluted in water, extracted with EtOAc (×3), washed with saturated aqueous NaCl, dried over anhydrous Na₂SO₄, and concentrated under reduced pressure. The residue was purified by HPLC to afford the title compound (yellow solid, 40 mg, yield 6%). **¹H NMR** (400 MHz, DMSO-*d*₆) δ 11.79 (s, 1H), 11.06 (s, 1H), 8.88 (s, 1H), 8.46-8.37 (m, 2H), 8.21 (d, *J* = 7.7 Hz, 1H), 8.15 (s, 1H), 7.95 (s, 1H), 7.79 (d, *J* = 9.0 Hz, 2H), 7.71 (s, 1H), 7.64 (d, *J =* 8.4 Hz, 1H), 7.09 (d, *J* = 9.1 Hz, 2H), 6.91 (s, 1H), 6.82 (d, *J* = 8.6 Hz, 1H), 5.09-5.03 (m, 1H), 3.63-3.57 (m, 1H), 3.51 (s, 1H), 3.43 (t, *J =* 8.6 Hz, 1H), 3.24 (s, 4H), 3.20-3.14 (m, 1H), 2.87 (dd, *J =* 21.5, 9.9 Hz, 1H), 2.69-2.55 (m, 7H), 2.43 (d, *J =* 7.3 Hz, 2H), 2.16 (d, *J =* 6.6 Hz, 1H), 2.04-1.97 (m, 1H), 1.81-1.78 (m, 1H). LCMS (ESI) m/z: 799 [M+H]⁺.

### Preparation Example 312. Synthesis of Compound A228

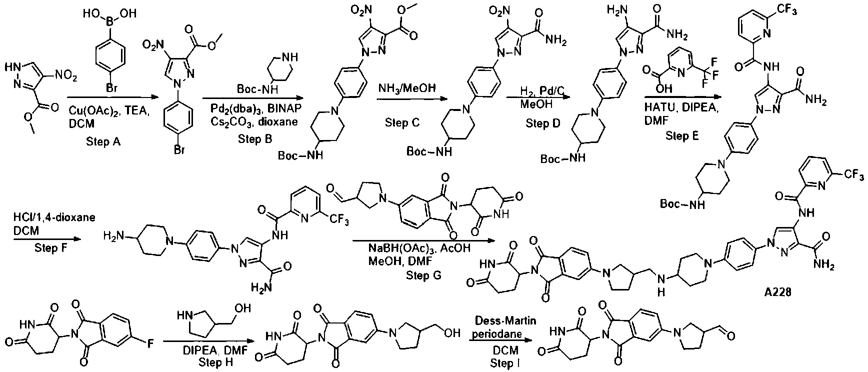

### Step A: Methyl 1-(4-bromophenyl)-4-nitro-1H-pyrazole-3-carboxylate

To a solution of methyl 4-nitro-1H-pyrazole-3-carboxylate (4 g, 23.4 mmol, 1.0 eq) and (4-bromophenyl)boronic acid (4.7 g, 23.4 mmol, 1.0 eq) in DCM (40 mL) at rt were added copper acetate (4.3 g, 23.4 mmol, 1.0 eq) and TEA (6.5 mL, 46.8 mmol, 2.0 eq). The reaction solution was reacted at rt under O₂ for 16 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (white solid, 3 g, yield 39%).

### Step B: Methyl 1-(4-(4-(((tert-Butoxycarbonyl)amino)piperidin-1-yl)phenyl)-4-nitro-1H-pyrazole-3-carboxylate

To a solution of methyl 1-(4-bromophenyl)-4-nitro-1H-pyrazole-3-carboxylate (2.4 g, 7.4 mmol, 1.0 eq) and tert-butyl piperidin-4-ylcarbamate (2.4 g, 7.4 mmol, 1.0 eq) in 1,4-dioxane (100 mL) were added cesium carbonate (4.8 g, 14.7 mmol, 2.0 eq), BINAP (461 mg, 0.74 mmol, 0.1 eq), and Pd₂(dba)₃ (678 mg, 0.74 mmol, 0.1 eq) at rt. The reaction solution was reacted at 100°C under N₂ for 16 h. Upon completion, the reaction solution was cooled to rt, poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 1.7 g, yield 52%).

### Step C: Tert-butyl (1-(4-(3-carbamoyl-4-nitro-1H-pyrazol-1-yl)phenyl)piperidin-4-yl)carbamate

A solution of methyl 1-(4-(4-((tert-butoxycarbonyl)amino)piperidin-1-yl)phenyl)-4-nitro-1H-pyrazole-3-carboxylate (1.7 g, 3.816 mmol, 1.0 eq) in ammonia/MeOH (20 mL, 2 mol/L) was reacted at 80°C for 16 h. Upon completion, the reaction solution was concentrated under reduced pressure to afford the title compound (yellow solid, 1.5 g, crude).

### Step D: Tert-butyl (1-(4-(4-amino-3-carbamoyl-1H-pyrazol-1-yl)phenyl)piperidin-4-yl)carbamate

To a solution of tert-butyl (1-(4-(3-carbamoyl-4-nitro-1H-pyrazol-1-yl)phenyl)piperidin-4-yl)carbamate (1.5 g, 3.5 mmol, 1.0 eq) in tetrahydrofuran/MeOH (8/8 mL) was added 10% palladium on carbon (150 mg, 10 wt%) at rt. The reaction solution was purged with H₂ (×3), and reacted at rt under H₂ for 16 h. Upon completion, the reaction solution was filtered and washed with MeOH (×3), and the filtrate was concentrated under reduced pressure to afford the title compound (brown solid, 1.1 g, crude).

### Step E: Tert-butyl (1-(4-(3-carbamoyl-4-(6-(trifluoromethyl)picolinamido)-1H-pyrazol-1-yl)phenyl)piperidin-4-yl)carbamate

To a solution of tert-butyl (1-(4-(4-amino-3-carbamoyl-1H-pyrazol-1-yl)phenyl)piperidin-4-yl)carbamate (1.1 g, 2.6 mmol, 1.0 eq) and 6-(trifluoromethyl)picolinic acid (0.5 g, 2.6 mmol, 1.0 eq) in DMF (30 mL) at 0°C were added DIEA (1.0 g, 0.7 mmol, 7.7 eq) and HATU (1.2 g, 3.1 mmol, 1.2 eq). The reaction solution was reacted at rt for 4 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 1.2 g, yield 82%).

### Step F: N-(1-(4-(4-aminopiperidin-1-yl)phenyl)-3-amino-1H-pyrazol-4-yl)-6-(trifluoromethyl)picolinamide

At 0°C, to a solution of tert-butyl (1-(4-(3-carbamoyl-4-(6-(trifluoromethyl)picolinamido)-1H-pyrazol-1-yl)phenyl)piperidin-4-yl)carbamate (500 mg, 0.9 mmol, 1.0 eq) in DCM (5 mL) was added HCl/1,4-dioxane (5 mL, 4 M). The reaction solution was reacted at rt for 1 h. Upon completion, the reaction solution was concentrated under reduced pressure to afford the title compound (yellow solid, 0.4 g, crude).

### Step G: 2-(2,6-dioxopiperidin-3-yl)-5-(3-(hydroxymethyl)pyrrolidin-1-yl)isoindoline-1,3-dione

To a solution of 2-(2,6-dioxopiperidin-3-yl)-5-fluoroisoindoline-1,3-dione (500 mg, 1.8 mmol, 1.0 eq) in DMF (30 mL) at 0°C were added pyrrolidin-3-ylmethanol (183 mg, 1.8 mmol, 1.0 eq) and DIEA (1.0 g, 0.7 mmol, 7.7 eq). The reaction solution was reacted at 80°C for 16 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 390 mg, yield 60%).

### Step H: 1-(2-(2,6-dioxopiperidin-3-yl)-1,3-dioxoisoindolin-5-yl)pyrrolidine-3-carbaldehyde

To a solution of 2-(2,6-dioxopiperidin-3-yl)-5-(3-(hydroxymethyl)pyrrolidin-1-yl)isoindoline-1,3-dione (300 mg, 0.8 mmol, 1.0 eq) in DCM (10 mL) at rt was added Dess-Martin periodinane (427 mg, 1.0 mmol, 1.2 eq). The reaction solution was reacted for 1.5 h under N₂ at rt. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by column chromatography to afford the title compound (yellow solid, 220 mg, yield 74%).

### Step I: N-(3-carbonyl-1-(4-(4-((1-(2-(2,6-dioxopiperidin-3-yl)-1,3-dioxoisoindol-5-yl)pyrrolidin-3-yl)methyl)amino)piperidin-1-yl)phenyl)-1H-pyrazol-4-yl)-6-(trifluoromethyl)picolinamide

To a solution of N-(1-(4-(4-aminopiperidin-1-yl)phenyl)-3-amino-1H-pyrazol-4-yl)-6-(trifluoromethyl)picolinamide (187 mg, 0.5 mmol, 1.0 eq) in DMF (5 mL) at rt were added 1-(2-(2,6-dioxopiperidin-3-yl)-1,3-dioxoisoindolin-5-yl)pyrrolidine-3-carbaldehyde (250 mg, 0.7 mmol, 1.4 eq) and AcOH (0.02 mL). The reaction solution was reacted at rt for 0.5 h. Sodium triacetoxyborohydride (336 mg, 1.6 mmol, 3.0 eq) was then added, and the reaction solution was stirred at rt for 1 h. Upon completion, the reaction solution was poured into water and extracted with EtOAc (×3). The combined organic phases were washed with saturated aqueous NaCl, dried over anhydrous Na₂SO₄, filtered, and concentrated under reduced pressure. The residue was purified by HPLC to afford the title compound (yellow solid, 20 mg, yield 7%). ¹HNMR (400 MHz, DMSO-*d*₆) δ 11.79 (s, 1H), 11.06 (s, 1H), 8.86 (s, 1H), 8.48-8.37 (m, 2H), 8.25-8.19 (m, 1H), 7.97-7.91 (m, 1H), 7.80 -7.74 (m, 2H), 7.70 (s, 1H), 7.64 (d, *J =* 8.4 Hz, 1H), 7.12-7.02 (m, 2H), 6.94-6.88 (m, 1H), 6.86-6.78 (m, 1H), 5.09-5.01 (m, 1H), 3.80-3.70 (m, 2H), 3.62-3.53 (m, 1H), 3.53-3.46 (m, 2H), 3.23-3.16 (m, 2H), 2.89 (s, 1H), 2.86-2.79 (m, 2H), 2.73 (s, 1H), 2.69-2.59 (m, 3H), 2.58-2.53 (m, 1H), 2.48-2.43 (m, 1H), 2.21-2.10 (m, 1H), 2.07-1.97 (m, 1H), 1.98-1.89 (m, 2H), 1.86-1.75 (m, 1H), 1.48-1.33 (m, 2H). LCMS (ESI): m/z 813[M+H]⁺.

### Preparation Example 313. Synthesis of Compound A298

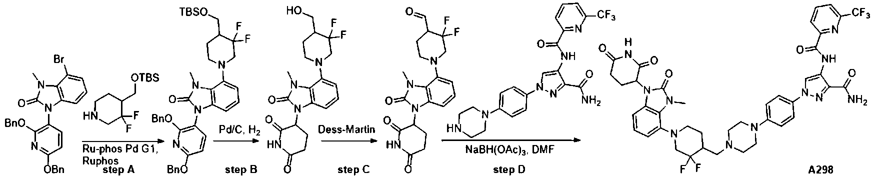

### Step A: 1-(2,6-bis(benzyloxy)pyridin-3-yl)-4-(4-(((tert-butyldimethylsilyloxy)methyl)-3,3-difluoropiperidin-1-yl)-3-methyl-1,3-dihydro-2H-benzo[d]imidazol-2-one

1-(2,6-Bis(benzyloxy)pyridin-3-yl)-4-bromo-3-methyl-1,3-dihydro-2H-benzo[d]imidazol-2-one (1.0 g, 1.94 mmol, 1.0 eq), 4-(((tert-butyldimethylsilyloxy)methyl)-3,3-difluoropiperidine (515 mg, 1.94 mmol, 1.0 eq), Ruphos (181 mg, 0.39 mmol, 0.2 eq), Ruphos Pd G₁ (317 mg, 0.39 mmol, 0.2 eq) and cesium carbonate (1.89 g, 5.82 mmol, 3.0 eq) were added to dioxane (30 ml), and the mixture was stirred at 100°C under N₂ for 16 h. The solution was then diluted in water (50 mL) and extracted with EtOAc (50 mL). The combined organic layers were washed with water (50 mL), dried over anhydrous Na₂SO₄, and concentrated under reduced pressure. The residue was purified by silica gel column chromatography (100-200 mesh silica gel, 0%-100% ethyl acetate in PE) to afford the title compound (yellow solid, 700 mg, yield 51%).

### Step B: 3-(4-(3,3-difluoro-4-(hydroxymethyl)piperidin-1-yl)-3-methyl-2-oxo-2,3-dihydro-1H-benzo[d]imidazol-1-yl)piperidine-2,6-dione

1-(2,6-Bis(benzyloxy)pyridin-3-yl)-4-(4-(((tert-butyldimethylsilyloxy)methyl)-3,3-difluoropiperidin-1-yl)-3-methyl-1,3-dihydro-2H-benzo[d]imidazol-2-one (700 mg, 1.0 mmol, 1.0 eq) was added to ethanol (15 mL), and palladium on carbon (500 mg, 10 wt%) and palladium hydroxide on carbon (200 mg, 10 wt%) were added at 25°C. The mixture was degassed with H₂ (×3) and stirred at 60°C under H₂ for 16 h. The reaction solution was filtered through a pad of silica gel and concentrated under reduced pressure to give the title compound (white solid, 300.0 mg, yield 73%).

### Step C: 1-(1-(2,6-dioxopiperidin-3-yl)-3-methyl-2-oxo-2,3-dihydro-1H-benzo[d]imidazol-4-yl)-3,3-difluoropiperidine-4-carbaldehyde

3-(4-(3,3-difluoro-4-(hydroxymethyl)piperidin-1-yl)-3-methyl-2-oxo-2,3-dihydro-1H-benzo[d]imidazol-1-yl)piperidine-2,6-dione (300 mg, 0.73 mmol, 1.0 eq) was added to DCM (20 mL), and Dess-Martin periodinane (622 mg, 1.46 mmol, 2.0 eq) was added at 0°C. The mixture was stirred at 0°C for 2 h. The reaction solution was then diluted in 50 mL of saturated sodium thiosulfate solution and extracted with DCM (100 mL×3). The organic layer was combined, washed with water (100 mL), dried over anhydrous Na₂SO₄, and concentrated under reduced pressure to afford the title compound (white solid, 250 mg, yield 84%).

### Step D: N-(3-Carbamoyl-1-(4-(4-((1-(1-(2,6-dioxopiperidin-3-yl)-3-methyl-2-oxo-2,3-dihydro-1H-benzo[d]imidazol-4-yl)-3,3-difluoropiperidin-4-yl)methyl)piperazin-1-yl)phenyl)-1H-pyrazol-4-yl)-6-(trifluoromethyl)picolinamide

At rt, to a solution of 1-(1-(2,6-dioxopiperidin-3-yl)-3-methyl-2-oxo-2,3-dihydro-1H-benzo[d]imidazol-4-yl)-3,3-difluoropiperidine-4-carbaldehyde (251 mg, 0.62 mmol, 1.0 eq) in DMF (10 mL) were added N-(3-carbamoyl-1-(4-(piperazin-1-yl)phenyl)-1H-pyrazol-4-yl)-6-(trifluoromethyl)picolinamide (283 mg, 0.62 mmol, 1.0 eq) and AcOH (0.5 mL). The mixture was stirred at 25°C for 1 h. Then sodium triacetoxyborohydride (526 mg, 2.48 mmol, 4.0 eq) was added to the mixture. The mixture was stirred at 25°C for 30 min. The reaction solution was diluted in water (50 mL) and extracted with EtOAc (50 mL×3). The organic layer was washed with aqueous NaCl (50 mL), dried over Na₂SO₄, and concentrated under reduced pressure. The residue was purified by preparative HPLC to afford the title compound (white solid, 40.6 mg, yield 8%). LCMS (ESI): m/z 850 [M+H]⁺. **¹H NMR** (400 MHz, DMSO-*d₆*) δ 11.79 (s, 1H), 11.10 (s, 1H), 8.88 (s, 1H), 8.48-8.35 (m, 2H), 8.21 (dd, *J* = 7.6, 1.0 Hz, 1H), 7.95 (s, 1H), 7.79 (d, *J* = 9.1 Hz, 2H), 7.71 (s, 1H), 7.09 (d, *J =* 9.2 Hz, 2H), 7.04-6.93 (m, 3H), 5.43-5.30 (m, 1H), 3.61 (s, 3H), 3.41-3.30 (m, 4H), 3.27-3.13 (m, 6H), 2.94-2.78 (m, 2H), 2.76-2.60 (m, 5H), 2.39-2.24 (m, 1H), 2.12-1.96 (m, 2H), 1.81-1.43 (m, 1H).

### Preparation Example 314. Synthesis of Compound A299

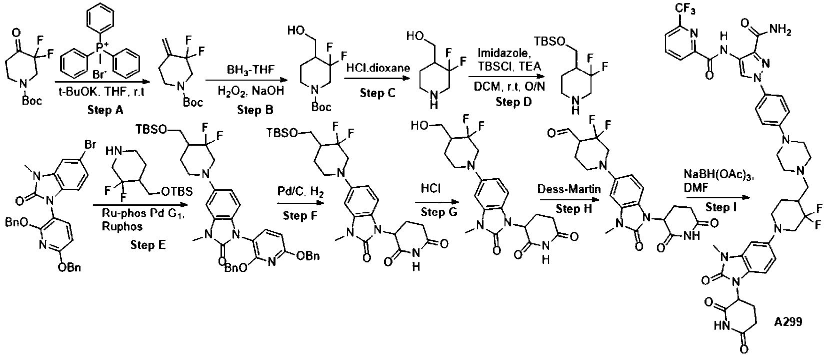

### Step A: Tert-butyl 3,3-difluoro-4-methylenepiperidine-1-carboxylate

At 0°C, to a stirred solution of methyltriphenylphosphonium bromide (113 g, 318.8 mmol, 1.5 eq) in THF (300 mL) was added potassium tert-butoxide (35.7 g, 318.8 mmol, 1.5 eq). The mixture was stirred at rt under N₂ for 1 h. The mixture was then cooled to 0°C, and a solution of tert-butyl 3,3-difluoro-4-oxopiperidine-1-carboxylate (50 g, 212.5 mmol, 1.0 eq) dissolved in THF (200 mL) was added. The mixture was stirred at rt under N₂ for 16 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (bright oil, 30 g, yield 60%).

### Step B: Tert-butyl 3,3-difluoro-4-(hydroxymethyl)piperidine-1-carboxylate

At rt, to a stirred solution of tert-butyl 3,3-difluoro-4-methylenepiperidine-1-carboxylate (30 g, 128.75 mmol, 1.0 eq) in THF (500 mL) was added borane in THF (257.5 mL, 257.52 mmol, 2.0 eq, 1 M) dropwise. The reaction solution was stirred at 70°C under N₂ for 2 h. The reaction solution was cooled to 0°C, and NaOH (366 mL, 733.08 mmol, 5.7 eq, 2 mol/L) and hydrogen peroxide (360 mL, 24.43 mmol, 0.2 eq, wt = 30%) were added to the mixture at the same temperature. The mixture was stirred at 40°C under N₂ overnight. Upon completion, the reaction solution was quenched with ice water, diluted in EtOAc, washed sequentially with water and saturated aqueous NaCl, dried over anhydrous Na₂SO₄, and concentrated under reduced pressure. The residue was purified by column chromatography to afford the title compound (bright oil, 10 g, yield 31%).

### Step C: (3,3-Difluoropiperidin-4-yl)methanol

At rt, to a stirred solution of tert-butyl 3,3-difluoro-4-(hydroxymethyl)piperidine-1-carboxylate (1.0 g, 3.98 mmol, 1.0 eq) in acetonitrile (20 mL) was added HCl (10 mL, 4 M in 1,4-dioxane). The mixture was stirred at rt under N₂ for 4 h, Upon completion, the reaction solution was concentrated under reduced pressure to afford the title compound (white solid, 500 mg, crude).

### Step D: 4-(((tert-Butyldimethylsilyl)oxy)methyl)-3,3-difluoropiperidine

At rt, to a solution of (3,3-difluoropiperidin-4-yl)methanol (500 mg, crude) in DCM (20 mL) were added TEA (2.29 mL, 16.53 mmol, 5.0 eq), imidazole (450 mg, 6.61 mmol, 2.0 eq), and tertbutyldimethylsilyl chloride (744 mg, 4.96 mmol, 1.5 eq). The mixture was stirred at rt under N₂ overnight. Upon completion, the reaction solution was quenched with ice water, diluted in EtOAc, washed sequentially with water and saturated aqueous NaCl, dried over anhydrous Na₂SO₄, and concentrated under reduced pressure. The residue was purified by column chromatography to afford the title compound (bright oil, 500 mg, yield 56% over two steps).

### Step E: 1-(2,6-Bis(benzyloxy)pyridin-3-yl)-5-(4-(((tert-butyldimethylsilyl)oxy)methyl)-3,3-difluoropiperidin-1-yl)-3-methyl-1,3-dihydro-2H-benzo[d]imidazol-2-one

At rt, to a solution of 1-(2,6-bis(benzyloxy)pyridin-3-yl)-5-bromo-3-methyl-1,3-dihydro-2H-benzo[d]imidazol-2-one (1.0 g, 1.94 mmol, 1.0 eq) in 1,4-dioxane were added 4-(((tertbutyldimethylsilyl)oxy)methyl)-3,3-difluoropiperidine (515 mg, 1.94 mmol, 1.0 eq), RuPhos (181 mg, 0.39 mmol, 0.2 eq), Ru-Phos Pd G₁ (317 mg, 0.39 mmol, 0.2 eq), and cesium carbonate (1.9 g, 5.82 mmol, 3.0 eq). The reaction solution was stirred at 100°C under N₂ for 16 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 700 mg, yield 51%).

### Step F: 3-(5-(4-(((tert-Butyldimethylsilyl)oxy)methyl)-3,3-difluoropiperidin-1-yl)-3-methyl-2-oxo-2,3-dihydro-1H-benzo[d]imidazol-1-yl)piperidine-2,6-dione

At rt, to a solution of 1-(2,6-bis(benzyloxy)pyridin-3-yl)-5-(4-(((tertbutyldimethylsilyl)oxy)methyl)-3,3-difluoropiperidin-1-yl)-3-methyl-1,3-dihydro-2H-benzo[d]imidazol-2-one (350 mg, 0.50 mmol, 1.0 eq) in ethanol (12 mL) was added 10% wet palladium on carbon (350 mg, w.t = 10%). The reaction solution was stirred under H₂ at rt for 16 h. Upon completion, the reaction solution was filtered through Celite and concentrated under reduced pressure to afford the title compound (colorless oil, 250 mg, yield 96%).

### Step G: 3-(5-(3,3-Difluoro-4-(hydroxymethyl)piperidin-1-yl)-3-methyl-2-oxo-2,3-dihydro-1H-benzo[d]imidazol-1-yl)piperidine-2,6-dione

At rt, to a stirred solution of 3-(5-(4-(((tert-butyldimethylsilyl)oxy)methyl)-3,3-difluoropiperidin-1-yl)-3-methyl-2-oxo-2,3-dihydro-1H-benzo[d]imidazol-1-yl)piperidine-2,6-dione (260 mg, 0.497 mmol, 1.0 eq) in acetonitrile (9 mL) was added HCl (3 mL, 4 M in 1,4-dioxane). The mixture was stirred at rt under N₂ for 0.5 h, Upon completion, the reaction solution was concentrated under reduced pressure to afford the title compound (white solid, 200 mg, crude).

### Step H: 1-(1-(2,6-Dioxopiperidin-3-yl)-3-methyl-2-oxo-2,3-dihydro-1H-benzo[d]imidazol-5-yl)-3,3-difluoropiperidine-4-carbaldehyde

At rt, to a solution of 3-(5-(3,3-difluoro-4-(hydroxymethyl)piperidin-1-yl)-3-methyl-2-oxo-2,3-dihydro-1H-benzo[d]imidazol-1-yl)piperidine-2,6-dione (120 mg, crude) in dimethyl sulfoxide (10 mL) was added 2-iodoxybenzoic acid (329 mg, 1.18 mmol, 4.0 eq). The mixture was stirred at 30°C under N₂ for 50 min. Upon completion, the reaction solution was diluted in EtOAc, washed sequentially with water and saturated aqueous NaCl, dried over anhydrous Na₂SO₄, and concentrated under reduced pressure to afford the title compound (yellow solid, 60 mg, yield 50% over two steps).

### Step I: N-(3-Carbamoyl-1-(4-(4-((1-(1-(2,6-dioxopiperidin-3-yl)-3-methyl-2-oxo-2,3-dihydro-1H-benzo[d]imidazol-5-yl)-3,3-difluoropiperidin-4-yl)methyl)piperazin-1-yl)phenyl)-1H-pyrazol-4-yl)-6-(trifluoromethyl)picolinamide

At rt, to a solution of 1-(1-(2,6-dioxopiperidin-3-yl)-3-methyl-2-oxo-2,3-dihydro-1H-benzo[d]imidazol-5-yl)-3,3-difluoropiperidine-4-carbaldehyde (60 mg, 0.15 mmol, 1.0 eq) in DMF (3 mL) were added N-(3-carbamoyl-1-(4-(piperazin-1-yl)phenyl)-1H-pyrazol-4-yl)-6-(trifluoromethyl)picolinamide (68 mg, 0.15 mmol, 1.0 eq) and AcOH (3 drops). The reaction was stirred at rt for 1 h, and then sodium triacetoxyborohydride (94 mg, 0.44 mmol, 3.0 eq) was added. The reaction solution was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (off-white solid, 2.2 mg, yield 2%). LCMS (ESI): m/z 850 [M+H]⁺. **¹H NMR** (400 MHz, DMSO) *δ* 11.79 (s, 1H), 11.07 (s, 1H), 8.88 (s, 1H), 8.46-8.38 (m, 2H), 8.23-8.19 (m, 1H), 7.95 (s, 1H), 7.80 (d, J = 9.0 Hz, 2H), 7.71 (s, 1H), 7.10 (d, *J* = 9.1 Hz, 2H), 6.96 (d, *J* = 8.6 Hz, 1H), 6.91 (d, 1H), 6.69 (d, *J* = 8.5 Hz, 1H), 5.30 (dd, *J* = 12.8, 5.4 Hz, 1H), 3.84-3.75 (m, 1H), 3.61 (d, *J =* 12.1 Hz, 1H), 3.35-3.34 (m, 1H), 3.30-3.28 (m, 1H), 3.27-3.21 (m, 5H), 3.09-3.00 (m, 1H), 2.89-2.78 (m, 2H), 2.72-2.57 (m, 6H), 2.47-2.42 (m, 2H), 2.36-2.30 (m, 1H), 2.11-2.06 (m, 1H), 2.01-1.97 (m, 1H), 1.63-1.54 (m, 1H).

### Preparation Example 315. Synthesis of Compound A300

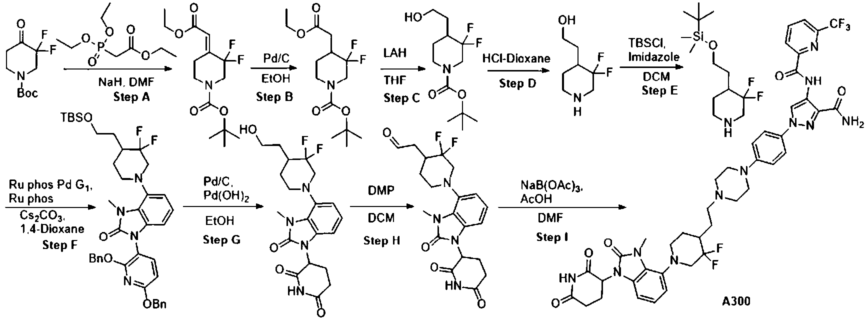

### Step A: Tert-butyl (E)-4-(2-ethoxy-2-oxoethylidene)-3,3-difluoropiperidine-1-carboxylate

At 0°C under N₂, to a solution of ethyl 2-(diethoxyphosphoryl)acetate (9.1 g, 40.43 mmol, 1.0 eq) in DMF (100 mL) was added 60% sodium hydride in mineral oil (1.5 g, 60.64 mmol, 1.5 eq). The mixture was stirred at 0°C for 30 min, and then a solution of tert-butyl 3,3-difluoro-4-oxopiperidine-1-carboxylate (9.5 g, 40.43 mmol, 1.0 eq) in DMF (50 mL) was added dropwise. The resulting mixture was stirred at rt for 2 h. Upon completion, the reaction was quenched with saturated aqueous NH₄Cl at 0°C and extracted with EtOAc (×3). The organic phases were sequentially washed with water and saturated aqueous NaCl, dried over anhydrous Na₂SO₄, and concentrated under reduced pressure. The residue was purified by column chromatography to afford the title compound (white solid, 3 g, yield 23%).

### Step B: Tert-butyl 4-(2-ethoxy-2-oxoethyl)-3,3-difluoropiperidine-1-carboxylate

At rt, to a solution of tert-butyl (E)-4-(2-ethoxy-2-oxoethylidene)-3,3-difluoropiperidine-1-carboxylate (3 g, 9.84 mmol, 1.0 eq) in EtOH (50 mL) was added 10% Pd/C (600 mg, wt=10%). The mixture was purged with H₂ (×3) and then stirred under H₂ at 50°C for 14 h. Upon completion, the reaction solution was filtered through a pad of Celite and concentrated to afford the title compound (white solid, 2.8 g, yield 93%).

### Step C: Tert-butyl 3,3-difluoro-4-(2-hydroxyethyl)piperidine-1-carboxylate

At 0°C under N₂, to a solution of tert-butyl 4-(2-ethoxy-2-oxoethyl)-3,3-difluoropiperidine-1-carboxylate (2.8 g, 9.12 mmol, 1.0 eq) in THF (30 mL) was added lithium aluminum hydride (10.9 mL, 27.36 mmol, 3.0 eq, 2.5 M in tetrahydrofuran). The mixture was stirred at 0°C under N₂ for 2 h. The reaction solution was quenched with sodium thiosulfate decahydrate at 0°C. The mixture was filtered and concentrated under reduced pressure to afford the title compound (colorless oil, 1.6 g, crude).

### Step D: 2-(3,3-Difluoropiperidin-4-yl)ethan-1-ol

Tert-butyl 3,3-difluoro-4-(2-hydroxyethyl)piperidine-1-carboxylate (1.6 g, crude) was dissolved in dioxane (30 mL, 4 M hydrogen chloride in dioxane), and the mixture was stirred at 0°C for 1 h. The reaction solution was concentrated to dryness, and the residue was washed with methyl tert-butyl ether and dried in vacuo to afford the title compound (white solid, 900 mg, crude).

### Step E: 4-(2-((tert-Butyldimethylsilyl)oxy)ethyl)-3,3-difluoropiperidine

To a solution of 2-(3,3-difluoropiperidin-4-yl)ethan-1-ol (900 mg, 5.45 mmol, 1.0 eq) in DCM (15 mL) at 0°C were added imidazole (556 mg, 8.18 mmol, 1.5 eq) and tert-butyldimethylsilyl chloride (985 mg, 6.5 mmol, 1.2 eq). The mixture was stirred at rt for 1 h. The mixture was quenched with ice water and extracted with DCM (20 mL×3). The combined organic layers were washed with saturated aqueous NaCl (20 mL), dried over anhydrous Na₂SO₄, filtered, and concentrated under reduced pressure. The residue was purified by column chromatography to afford the title compound (oil, 1.2 g, yield 79% over three steps).

### Step F: 1-(2,6-Bis(benzyloxy)pyridin-3-yl)-4-(4-(2-((tert-butyldimethylsilyl)oxy)ethyl)-3,3-difluoropiperidin-1-yl)-3-methyl-1,3-dihydro-2H-benzo[d]imidazol-2-one

1-(2,6-bis(benzyloxy)pyridin-3-yl)-4-bromo-3-methyl-1,3-dihydro-2H-benzo[d]imidazol-2-one (1.9 g, 3.58 mmol, 1.0 eq), 4-(2-((tert-butyldimethylsilyl)oxy)ethyl)-3,3-difluoropiperidine (1.0 g, 3.58 mmol, 1.0 eq), Ruphos (334 mg, 0.72 mmol, 0.2 eq), Ruphos Pd G₁ (292 mg, 0.36 mmol, 0.1 eq), and cesium carbonate (3.5 g, 10.75 mmol, 3.0 eq) were added to 1,4-dioxane (30 mL), and the mixture was stirred at 100°C under N₂ for 16 h. The solution was diluted in water (50 mL) and extracted with EtOAc (50 mL×3). The combined organic layers were washed with saturated aqueous NaCl (30 mL), dried over anhydrous Na₂SO₄, and concentrated under reduced pressure. The residue was purified by column chromatography to afford the title compound (white solid, 400 mg, yield 16%).

### Step G: 3-(4-(3,3-Difluoro-4-(2-hydroxyethyl)piperidin-1-yl)-3-methyl-2-oxo-2,3-dihydro-1H-benzo[d]imidazol-1-yl)piperidine-2,6-dione

To a solution of 1-(2,6-bis(benzyloxy)pyridin-3-yl)-4-(4-(2-((tert-butyldimethylsilyl)oxy)ethyl)-3,3-difluoropiperidin-1-yl)-3-methyl-1,3-dihydro-2H-benzo[d]imidazol-2-one (400 mg, 0.56 mmol, 1.0 eq) in ethanol (15 mL) were added Pd/C (300 mg, 10 wt%) and Pd(OH)₂/C (100 mg, 10 wt%). The mixture was degassed with H₂ (×3) and then stirred under a hydrogen balloon at 60°C for 14 h. The reaction solution was filtered through Celite and concentrated to afford the title compound (white solid, 200 mg, yield 85%, crude).

### Step H: 2-(1-(1-(2,6-Dioxopiperidin-3-yl)-3-methyl-2-oxo-2,3-dihydro-1H-benzo[d]imidazol-4-yl)-3,3-difluoropiperidin-4-yl)acetaldehyde

To a solution of 3-(4-(3,3-difluoro-4-(2-hydroxyethyl)piperidin-1-yl)-3-methyl-2-oxo-2,3-dihydro-1H-benzo[d]imidazol-1-yl)piperidine-2,6-dione (200 mg, 0.47 mmol, 1.0 eq) in DCM (8 mL) was added Dess-Martin periodinane (151 mg, 0.71 mmol, 1.5 eq). The mixture was stirred at rt for 1 h. The reaction solution was diluted in water (20 mL) and extracted with DCM (20 mL×3). The combined organic layers were washed with saturated aqueous NaCl (30 mL), dried over anhydrous Na₂SO₄, and concentrated under reduced pressure to afford the title compound (white solid, 130 mg, crude).

### Step I: N-(3-Carbamoyl-1-(4-(4-(2-(1-(2,6-dioxopiperidin-3-yl)-3-methyl-2-oxo-2,3-dihydro-1H-benzo[d]imidazol-4-yl)-3,3-difluoropiperidin-4-yl)ethyl)piperazin-1-yl)phenyl)-1H-pyrazol-4-yl)-6-(trifluoromethyl)picolinamide

To a stirred solution of 2-(1-(1-(2,6-dioxopiperidin-3-yl)-3-methyl-2-oxo-2,3-dihydro-1H-benzo[d]imidazol-4-yl)-3,3-difluoropiperidin-4-yl)acetaldehyde (130 mg, crude) and N-(3-carbamoyl-1-(4-(piperazin-1-yl)phenyl)-1H-pyrazol-4-yl)-6-(trifluoromethyl)picolinamide (142 mg, 0.31 mmol, 1.0 eq) in DMF (10 mL) was added AcOH (0.5 mL). The mixture was stirred at rt for 16 h. Then sodium triacetoxyborohydride (196 mg, 0.93 mmol, 3.0 eq) was added to the mixture at the same temperature. The mixture was stirred at rt for 30 min. The reaction solution was concentrated under reduced pressure. The residue was purified by column chromatography to obtain a crude, and the crude was purified by preparative HPLC to afford the title compound (white solid, 28.6 mg, yield 11%). LCMS (ESI): m/z 864 [M+H]⁺. **¹H NMR** (400 MHz, DMSO) *δ* 11.79 (s, 1H), 11.09 (s, 1H), 8.91-8.86 (m, 1H), 8.48-8.37 (m, 2H), 8.21 (d, *J* = 7.4 Hz, 1H), 7.95 (s, 1H), 7.79 (d, *J =* 8.7 Hz, 2H), 7.71 (s, 1H), 7.09 (d, *J =* 8.9 Hz, 2H), 7.04-6.92 (m, 3H), 5.37 (dd, *J =* 12.5, 5.3 Hz, 1H), 3.61 (s, 3H), 3.26-3.21 (m, 4H), 3.20-3.04 (m, 2H), 2.95-2.63 (m, 4H), 2.62-2.52 (m, 5H), 2.47-2.40 (m, 2H), 2.09-1.93 (m, 4H), 1.72-1.55 (m, 1H), 1.51-1.39 (m, 1H).

### Preparation Example 316. Synthesis of Compound A301

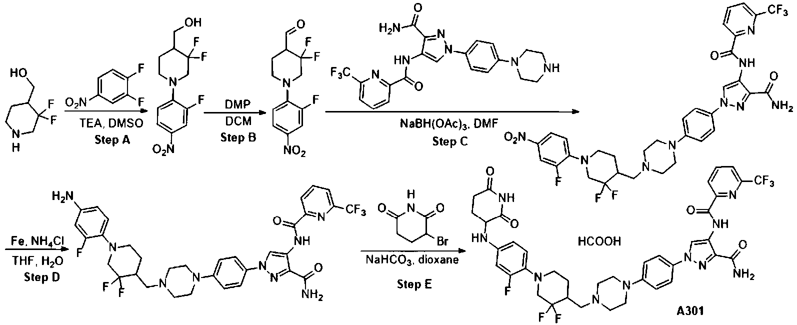

### Step A: (3,3-Difluoro-1-(2-fluoro-4-nitrophenyl)piperidin-4-yl)methanol

At rt, (3,3-difluoropiperidin-4-yl)methanol (500 mg, 3.31 mmol, 1.0 eq), 1,2-difluoro-4-nitrobenzene (631 mg, 4.0 mmol, 1.2 eq), and TEA (1.4 mL, 9.93 mmol, 3.0 eq) were mixed in dimethyl sulfoxide (10 mL). The reaction solution was stirred at 90°C under N₂ for 16 h. Upon completion, the reaction solution was diluted in DCM, washed sequentially with water and saturated aqueous NaCl, dried over anhydrous Na₂SO₄, and concentrated under reduced pressure. The residue was purified by column chromatography to afford the title compound (yellow solid, 700 mg, yield 73%).

### Step B: 3,3-Difluoro-1-(2-fluoro-4-nitrophenyl)piperidine-4-carbaldehyde

To a solution of (3,3-difluoro-1-(2-fluoro-4-nitrophenyl)piperidin-4-yl)methanol (500 mg, 1.72 mmol, 1.0 eq) in DCM (10 mL) was added Dess-Martin periodinane (1.5 g, 3.45 mmol, 2.0 eq) at rt. The reaction solution was stirred at 25°C under N₂ for 4 h. Upon completion, the reaction solution was diluted in DCM, washed sequentially with water and saturated aqueous NaCl, dried over anhydrous Na₂SO₄, and concentrated under reduced pressure. The residue was purified by column chromatography to afford the title compound (yellow oil, 630 mg, yield 99%).

### Step C: N-(3-Carbamoyl-1-(4-(4-((3,3-difluoro-1-(2-fluoro-4-nitrophenyl)piperidin-4-yl)methyl)piperazin-1-yl)phenyl)-1H-pyrazol-4-yl)-6-(trifluoromethyl)picolinamide

To a solution of 3,3-difluoro-1-(2-fluoro-4-nitrophenyl)piperidine-4-carboxaldehyde (198 mg, 0.69 mmol, 1.0 eq) and N-(3-carbamoyl-1-(4-(piperazin-1-yl)phenyl)-1H-pyrazol-4-yl)-6-(trifluoromethyl)picolinamide (300 mg, 0.65 mmol, 1.0 eq) in DMF (10 mL) was added AcOH (12 mg, 0.06 mmol, 0.1 eq) at rt. The reaction solution was stirred at 25°C under N₂ for 1 h, and acetic acid borohydride (554 mg, 2.61 mmol, 4.0 eq) was added. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 200 mg, yield 42%).

### Step D: N-(1-(4-(4-((1-(4-amino-2-fluorophenyl)-3,3-difluoropiperidin-4-yl)methyl)piperazin-1-yl)phenyl)-3-carbamoyl-1H-pyrazol-4-yl)-6-(trifluoromethyl)picolinamide

N-(3-carbamoyl-1-(4-(4-((3,3-difluoro-1-(2-fluoro-4-nitrophenyl)piperidin-4-yl)methyl)piperazin-1-yl)phenyl)-1H-pyrazol-4-yl)-6-(trifluoromethyl)picolinamide (120 mg, 0.16 mmol, 1.0 eq), iron powder (92 mg, 1.64 mmol, 10.0 eq), and ammonium chloride (88 mg, 1.64 mmol, 10.0 eq) were dissolved in THF (10 mL) and water (3 mL) at rt. The reaction solution was stirred at 70°C under N₂ for 16 h. Upon completion, the solution was passed through a pad of Celite, diluted in DCM, washed sequentially with water and saturated aqueous NaCl, dried over anhydrous Na₂SO₄, and concentrated under reduced pressure to afford the title compound (yellow solid, 100 mg, yield 87%).

### Step E: N-(3-Carbamoyl-1-(4-(4-((1-(4-((2,6-dioxopiperidin-3-yl)amino)-2-fluorophenyl)-3,3-difluoropiperidin-4-yl)methyl)piperazin-1-yl)phenyl)-1H-pyrazol-4-yl)-6-(trifluoromethyl)picolinamide

At rt, N-(1-(4-(4-((1-(4-amino-2-fluorophenyl)-3,3-difluoropiperidin-4-yl)methyl)piperazin-1-yl)phenyl)-3-carbamoyl-1H-pyrazol-4-yl)-6-(trifluoromethyl)picolinamide (100 mg, 0.14 mmol, 1.0 eq), 3-bromo-2,6-piperidinedione (108 mg, 0.57 mmol, 4.0 eq), and NaHCO₃ (47 mg, 0.57 mmol, 4.0 eq) were dissolved in dioxane (2 mL). The reaction solution was stirred at 80°C under N₂ for 5 h. Upon completion, the reaction solution was diluted in EtOAc, washed sequentially with water and saturated aqueous NaCl, dried over anhydrous Na₂SO₄, and concentrated under reduced pressure. The residue was purified by high performance liquid chromatography to afford the title compound (yellow solid, 10 mg, yield 10%) (0.5% formate). LCMS (ESI): m/z 813 [M+H]⁺. **¹H NMR** (400 MHz, DMSO) *δ* 11.79 (s, 1H), 10.78 (s, 1H), 8.88 (s, 1H), 8.47-8.38 (m, 2H), 8.21 (d, *J* = 7.6 Hz, 1H), 8.17 (s, 1H), 7.95 (s, 1H), 7.79 (d, *J* = 8.7 Hz, 2H), 7.71 (s, 1H), 7.09 (d, *J* = 9.0 Hz, 2H), 6.87 (t, *J* = 9.3 Hz, 1H), 6.48 (dd, *J* = 35.2, 11.7 Hz, 2H), 5.89 (d, *J=* 7.8 Hz, 1H), 4.31-4.24 (m, 1H), 3.24-3.18 (m, 6H), 3.17-3.13 (m, 2H), 3.02-2.91 (m, 2H), 2.70-2.64 (m, 3H), 2.61-2.56 (m, 2H), 2.35-2.20 (m, 2H), 2.10-2.03 (m, 2H), 1.89-1.81 (m, 1H), 1.58-1.50 (m, 1H).

### Preparation Example 317. Synthesis of Compound A302

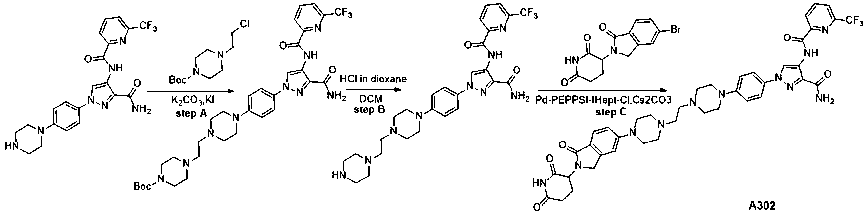

### Step A: Tert-butyl 4-(2-(4-(3-amino-4-(6-(trifluoromethyl)picolinamido)-1H-pyrazol-1-yl)phenyl)piperazin-1-yl)ethyl)piperazine 1-carboxylate

To a solution of tert-butyl 4-(2-(4-(4-(3-carbamoyl-4-(6-(trifluoromethyl)pyridinylamino)-1H-pyrazol-1-yl)phenyl)piperazin-1-yl)ethyl)piperazine-1-carboxylate (500 mg, 1.1 mmol, 1.0 eq) and tert-butyl 4-(2-chloroethyl)piperazine-1-carboxylate (546 mg, 2.2 mmol, 2.0 eq) in acetonitrile (25 mL) at rt were added potassium iodide (183 mg, 1.1 mmol, 1.0 eq) and potassium carbonate (455 mg, 3.3 mmol, 3.0 eq). The reaction solution was stirred at 80°C under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 400 mg, yield 54%).

### Step B: N-(3-Carbamoyl-1-(4-(4-(2-(piperazin-1-yl)ethyl)piperazin-1-yl)phenyl)-1H-pyrazol-4-yl)-6-(trifluoromethyl)picolinamide

At rt, to a solution of tert-butyl 4-(2-(4-(3-amino-4-(6-(trifluoromethyl)picolinamido)-1H-pyrazol-1-yl)phenyl)piperazin-1-yl)ethyl)piperazine 1-carboxylate (400 mg, 0.6 mmol, 1.0 eq) in DCM (10 mL) was added 4 M hydrogen chloride-dioxane (2 mL). The reaction solution was stirred at rt under N₂ for 2 h. Upon completion, the mixture was concentrated under reduced pressure to provide the title compound (white solid, 310 mg, crude).

### Step C: N-(3-carbamoyl-1-(4-(4-(2-(2,6-dioxopiperidin-3-yl)-1-oxoisoindolin-5-yl)piperazin-1-yl)ethyl)piperazin-1-yl)phenyl)-1H-pyrazol-4-yl)-6-(trifluoromethyl)picolinamide

To a solution of N-(3-carbamoyl-1-(4-(4-(2-(piperazin-1-yl)ethyl)piperazin-1-yl)phenyl)-1H-pyrazol-4-yl)-6-(trifluoromethyl)picolinamide (114 mg, crude) and 3-(5-bromo-1-oxoisoindolin-2-yl)piperidine-2,6-dione (200 mg, 0.35 mmol, 1.0 eq) in 1,4-dioxane (10 mL) at rt were added PEPPSI-IHept-Cl (17 mg, 17.5 umol, 0.05 eq) and cesium carbonate (341 g, 1.05 mmol, 3.0 eq). The reaction solution was stirred at 110°C under N₂ for 16 h. Upon completion, the reaction solution was quenched with aqueous AcOH, extracted with DCM, washed sequentially with water and saturated aqueous NaCl, dried over anhydrous Na₂SO₄, and concentrated under reduced pressure. The residue was purified by HPLC to afford the title compound (white solid, 2 mg, yield 7%). LCMS (ESI): m/z 814 [M+H]⁺. **¹H NMR** (400 MHz, DMSO) *δ* 11.80 (s, 1H), 10.95 (s, 1H), 8.92 (s, 1H), 8.42 (d, *J =* 13.6 Hz, 2H), 8.22 (d, *J* = 7.4 Hz, 1H), 7.94 (s, 1H), 7.86 (d, *J =* 6.1 Hz, 2H), 7.74 (s, 1H), 7.58 (d, *J =* 5.6 Hz, 1H), 7.16 (s, 5H), 5.06 (d, *J =* 12.0 Hz, 1H), 4.29 (dd, *J =* 48.9, 16.7 Hz, 4H), 3.26-3.14 (m, 9H), 2.91 (s, 2H), 2.74-2.60 (m, 3H), 2.42-2.32 (m, 2H), 2.00-1.94 (m, 1H).

### Preparation Example 318. Synthesis of Compound A303

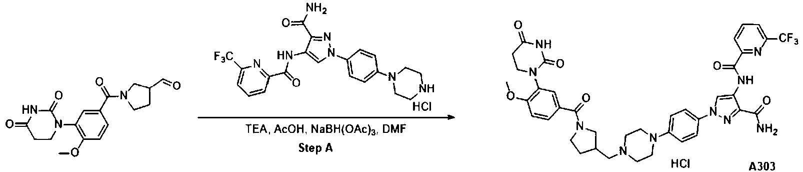

### Step A: N-(3-carbamoyl-1-(4-((1-(3-(2,4-dioxotetrahydropyrimidin-1(2H)yl)-4-methoxybenzoyl)pyrrolidin-3-yl)methyl)piperazin-1-yl)phenyl)-1H-pyrazol-4-yl)-6-(trifluoromethyl)picolinamide hydrochloride

To a solution of N-(3-carbamoyl-1-(4-(piperazin-1-yl)phenyl)-1H-pyrazol-4-yl)-6-(trifluoromethyl)picolinamide hydrochloride (133 mg, 0.3 mmol, 1.0 eq) and 1-(3-(2,4-dioxotetrahydropyrimidin-1(2H)yl)-4-methoxybenzoyl)pyrrolidine-3-carbaldehyde (100 mg, 0.3 mmol, 1.0 eq) in DMF (5 mL) was added TEA (0.1 mL) at rt. The reaction solution was stirred for 5 min, and then AcOH (0.2 mL) was added. The reaction solution was stirred for another 15 h. Sodium triacetoxyborohydride (95 mg, 0.5 mmol, 1.5 eq) was then added, and the mixture was stirred for 1 h. Upon completion, the reaction solution was diluted in water and extracted with DCM. The organic phase was washed sequentially with saturated aqueous NaCl, dried over anhydrous Na₂SO₄, filtered, and concentrated. The residue was purified by HPLC to afford the title compound (white solid, 27.2 mg, yield 11%). **¹H NMR** (400 MHz, DMSO-*d*₆) *δ* 11.79 (s, 1H), 11.11 (d, *J =* 58.8 Hz, 1H), 10.35 (s, 1H), 8.92 (s, 1H), 8.42 (dt, *J =* 15.4, 7.6 Hz, 2H), 8.22 (d, *J =* 7.6 Hz, 1H), 7.97 (s, 1H), 7.86 (s, 2H), 7.74 (s, 1H), 7.55 (d, *J =* 19.4 Hz, 2H), 7.17 (d, *J =* 8.0 Hz, 3H), 3.98-3.80 (m, 6H), 3.72-3.50 (m, 6H), 3.43-3.08 (m, 7H), 2.87-2.64 (m, 3H), 2.23-2.09 (m, 1H), 1.83-1.70 (m, 1H). LCMS (ESI): m/z 789 [M+H]⁺.

### Preparation Example 319. Synthesis of Compound A304

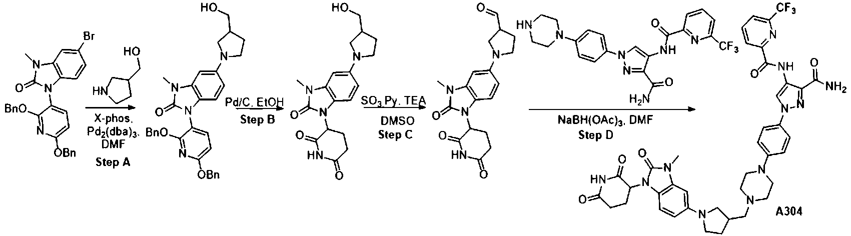

### Step A: 1-(2,6-bis(benzyloxy)pyridin-3-yl)-5-(3-(hydroxymethyl)pyrrolidin-1-yl)-3-methyl-1,3-dihydro-2H-benzo[d]imidazol-2-one mixture

At rt, to a solution of 1-(2,6-bis(benzyloxy)pyridin-3-yl)-5-bromo-3-methyl-1,3-dihydro-2H-benzo[d]imidazol-2-one (1.0 g, 1.94 mmol, 1.0 eq) in DMF (15 mL) were added pyrrolidin-3-ylmethanol (0.2 g, 1.94 mmol, 1.0 eq), Pd₂(dba)₃(180 mg, 0.2 mmol, 0.2 eq), X-Phos (180 mg, 0.2 mmol, 0.4 eq), and Cs₂CO₃(1.9 g, 5.8 mmol, 3.0 eq). The reaction solution was stirred at 100°C under N₂ for 5 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 200 mg, yield 19%).

### Step B: 3-(5-(3-(Hydroxymethyl)pyrrolidin-1-yl)-3-methyl-2-oxo-2,3-dihydro-1H-benzo[d]imidazol-1-yl)piperidine-2,6-dione

To a solution of 1-(2,6-bis(benzyloxy)pyridin-3-yl)-5-(3-(hydroxymethyl)pyrrolidin-1-yl)-3-methyl-1,3-dihydro-2H-benzo[d]imidazol-2-one mixture (200 mg, 0.37 mmol, 1.0 eq) in ethanol (10 mL) was added 10% wet palladium on carbon (226 mg) at rt. The reaction solution was stirred at 60°C under H₂ for 3 h. Upon completion, the reaction solution was filtered through silica gel and concentrated under reduced pressure to afford the title compound (white solid, 50 mg, yield 37%).

### Step C: 1-(1-(2,6-dioxopiperidin-3-yl)-3-methyl-2-oxo-2,3-dihydro-1H-benzo[d]imidazol-5-yl)pyrrolidine-3-carbaldehyde

To a solution of 3-(5-(3-(hydroxymethyl)pyrrolidin-1-yl)-3-methyl-2-oxo-2,3-dihydro-1H-benzo[d]imidazol-1-yl)piperidine-2,6-dione (50 mg, 0.14 mmol, 1.0 eq) in DMSO (30 mL) at rt were added SO₃.Py (222 mg, 1.4 mmol, 10.0 eq) and TEA (0.39 mL, 2.8 mmol, 20.0 eq). The reaction solution was stirred at rt for 1 h. Upon completion, the reaction solution was diluted in DCM, washed sequentially with water and saturated aqueous NaCl, dried over anhydrous Na₂SO₄, and concentrated under reduced pressure. The product was used directly in the next step without purification.

### Step D: N-(3-carbamoyl-1-(4-(4-((1-(1-(2,6-dioxopiperidin-3-yl)-3-methyl-2-oxo-2,3-dihydro-1H-benzo[d]imidazol-5-yl)pyrrolidin-3-yl)methyl)piperazin-1-yl)phenyl)-1H-pyrazol-4-yl)-6-(trifluoromethyl)picolinamide

To a solution of 1-(1-(2,6-dioxopiperidin-3-yl)-3-methyl-2-oxo-2,3-dihydro-1H-benzo[d]imidazol-5-yl)pyrrolidine-3-carbaldehyde (45 mg, crude) in DMF (5 mL) at rt were added N-(3-carbamoyl-1-(4-(piperazin-1-yl)phenyl)-1H-pyrazol-4-yl)-6-(trifluoromethyl)picolinamide (58 mg, 0.13 mmol, 1.0 eq) and 5 drops of AcOH. The reaction was stirred at rt for 2 h, and then sodium triacetoxyborohydride (134 mg, 0.65 mmol, 5.0 eq) was added. The reaction solution was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 4.5 mg, yield 4%). LCMS (ESI): m/z 800.8 [M+H]⁺. **¹H NMR** (400 MHz, DMSO) *δ* 11.79 (s, 1H), 11.04 (s, 1H), 8.88 (s, 1H), 8.48-8.36 (m, 2H), 8.21 (d, *J =* 8.7 Hz, 1H), 7.95 (s, 1H), 7.79 (d, *J* = 9.1 Hz, 2H), 7.71 (s, 1H), 7.09 (d, *J* = 9.2 Hz, 2H), 6.89 (d, *J =* 8.5 Hz, 1H), 6.39 (d, *J =* 1.9 Hz, 1H), 6.23 (d, *J* = 6.7 Hz, 1H), 5.26 (dd, *J =* 12.8, 5.3 Hz, 1H), 3.44-3.35 (m, 2H), 3.30 (s, 3H), 3.27-3.23 (m, 4H), 3.04-2.97 (m, 1H), 2.96-2.85 (m, 1H), 2.71-2.52 (m, 8H), 2.46-2.38 (m, 2H), 2.17-2.08 (m, 1H), 2.02-1.94 (m, 1H), 1.79-1.68 (m, 1H).

### Preparation Example 320. Synthesis of Compound A305

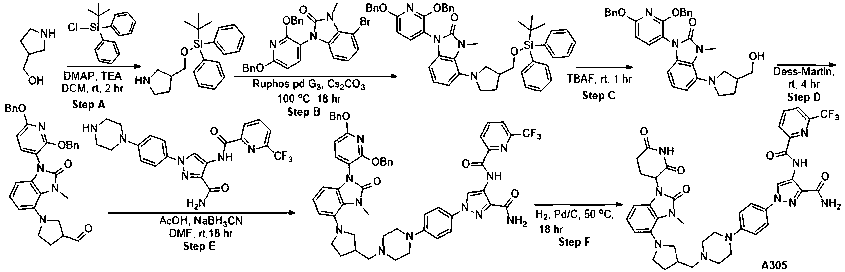

### Step A: 3-(((tert-Butyldiphenylsilyloxy)methyl)pyrrolidine

To a stirred mixture of pyrrolidin-3-ylmethanol (500 mg, 4.9 mmol, 1.0 eq) and tert-butyldiphenylsilyl chloride (1.5 g, 5.4 mmol, 1.1 eq) in DCM (5 mL) were added DMAP (60 mg, 0.49 mmol, 0.1 eq) and TEA (2.0 mL, 14.8 mmol, 3.0 eq). The reaction solution was stirred at rt for 2 h. The reaction mixture was poured into water and extracted with DCM. The organic layer was washed with saturated aqueous NaCl, dried over anhydrous Na₂SO₄ and concentrated. The residue was purified by silica gel column chromatography to afford the title compound (clear oil, 600 mg, yield 35%).

### Step B: 1-(2,6-bis(benzyloxy)pyridin-3-yl)-4-(3-(((tert-butyldiphenylsilyl)oxy)methyl)pyrrolidin-1-yl)-3-methyl-1,3-dihydro-2H-benzo[d]imidazol-2-one

To a stirred mixture of 1-(2,6-bis(benzyloxy)pyridin-3-yl)-4-bromo-3-methyl-1,3-dihydro-2H-benzo[d]imidazol-2-one (1.0 g, 1.9 mmol, 1.0 eq) and 3-(((tert-Butyldiphenylsilyloxy)methyl)pyrrolidine (657 mg, 1.9 mmol, 1.0 eq) in dioxane (10 mL) were added cesium carbonate (1.8 g, 5.8 mmol, 3.0 eq) and RuPhos Pd G₃ (162 mg, 0.19 mmol, 0.1 eq). The reaction solution was purged with N₂ (×3) and then stirred at 100°C under N₂ for 18 h. The reaction mixture was poured into water and extracted with EtOAc. The organic layer was washed with saturated aqueous NaCl, dried over anhydrous Na₂SO₄, and concentrated. The residue was purified by silica gel column chromatography to afford the title compound (clear oil, 500 mg, yield 33%).

### Step C: 1-(2,6-bis(benzyloxy)pyridin-3-yl)-4-(3-(hydroxymethyl)pyrrolidin-1-yl)-3-methyl-1,3-dihydro-2H-benzo[d]imidazol-2-one

1-(2,6-bis(benzyloxy)pyridin-3-yl)-4-(3-(((tert-butyldiphenylsilyl)oxy)methyl)pyrrolidin-1-yl)-3-methyl-1,3-dihydro-2H-benzo[d]imidazol-2-one (400 mg, 0.5 mmol, 1.0 eq) was added to a solution of TBAF (4 mL, 1 M in tetrahydrofuran) at rt, and the reaction solution was stirred at rt for 1 h. Upon completion, the mixture was diluted in EtOAc and extracted with aqueous solution. The organic layer was washed with saturated aqueous NaCl, dried over anhydrous Na₂SO₄, and concentrated. The residue was purified by silica gel column chromatography to afford the title compound (white solid, 200 mg, yield 72%).

### Step D: 1-(1-(2,6-bis(benzyloxy)pyridin-3-yl)-3-methyl-2-oxo-2,3-dihydro-1H-benzo[d]imidazol-4-yl)pyrrolidine-3-carbaldehyde

To a solution of 1-(2,6-bis(benzyloxy)pyridin-3-yl)-4-(3-(hydroxymethyl)pyrrolidin-1-yl)-3-methyl-1,3-dihydro-2H-benzo[d]imidazol-2-one (100 mg, 0.2 mmol, 1.0 eq) in DCM (1 mL) was added Dess-Martin periodinane (170 mg, 0.4 mmol, 2.0 eq) at rt. The reaction solution was stirred at rt for 4 h. Upon completion, the reaction mixture was poured into water and extracted with DCM. The organic layer was washed with saturated aqueous NaCl, dried over anhydrous Na₂SO₄, and concentrated. The residue was purified by silica gel column chromatography to afford the title compound (clear oil, 60 mg, yield 54%).

### Step E: N-(1-(4-(4-((1-(1-(2,6-bis(benzyloxy)pyridin-3-yl)-3-methyl-2-oxo-2,3-dihydro-1H-benzo[d]imidazol-4-yl)pyrrolidin-3-yl)methyl)piperazin-1-yl)phenyl)-3-carbamoyl-1H-pyrazol-4-yl)-6-(trifluoromethyl)picolinamide

To a solution of 1-(1-(2,6-bis(benzyloxy)pyridin-3-yl)-3-methyl-2-oxo-2,3-dihydro-1H-benzo[d]imidazol-4-yl)pyrrolidine-3-carbaldehyde (60 mg, 0.1 m mol, 1.0 eq) and N-(3-carbamoyl-1-(4-(piperazin-1-yl)phenyl)-1H-pyrazol-4-yl)-6-(trifluoromethyl)picolinamide (103 mg, 0.2 mmol, 2.0 eq) in DMF (3 mL) at rt were added AcOH (0.12 mL, 2.0 mmol, 20 eq) and sodium cyanoborohydride (35 mg, 0.6 mmol, 3.0 eq). The reaction solution was then stirred at rt for 18 h. Upon completion, the mixture was poured into water and extracted with EtOAc. The combined organic layers were washed sequentially with saturated aqueous NaCl, dried over anhydrous Na₂SO₄, and concentrated. The residue was purified by HPLC to afford the title compound (yellow solid, 5 mg, yield 4%).

### Step F: N-(3-carbamoyl-1-(4-(4-((1-(1-(2,6-dioxopiperidin-3-yl)-3-methyl-2-oxo-2,3-dihydro-1H-benzo[d]imidazol-4-yl)pyrrolidin-3-yl)methyl)piperazin-1-yl)phenyl)-1H-pyrazol-4-yl)-6-(trifluoromethyl)picolinamide

To a solution of N-(1-(4-(4-((1-(1-(2,6-bis(benzyloxy)pyridin-3-yl)-3-methyl-2-oxo-2,3-dihydro-1H-benzo[d]imidazol-4-yl)pyrrolidin-3-yl)methyl)piperazin-1-yl)phenyl)-3-carbamoyl-1H-pyrazol-4-yl)-6-(trifluoromethyl)picolinamide (5 mg, 0.005 mmol, 1.0 eq) in ethanol (3 mL) were added 10% wet Pd(OH)₂ (11 mg, 0.07 mmol, 1.0 eq) and 10% wet Pd/C (10 mg, 0.09 mmol, 18 eq) at rt. The mixture was stirred at 50°C for 18 h. Upon completion, the mixture was concentrated to obtain a residue. The residue was purified by HPLC to afford the title compound (white solid, 1.7 mg, yield 41%). **¹H NMR** (400 MHz, DMSO-*d*₆) *δ* 11.80 (s, 1H), 11.10 (s, 1H), 9.46 (s, 1H), 8.94 (s, 1H), 8.47-8.38 (m, 2H), 8.23 (d, *J* = 7.5 Hz, 1H), 7.95 (s, 1H), 7.88 (d, *J* = 8.7 Hz, 2H), 7.75 (s, 1H), 7.19 (d, *J =* 8.8 Hz, 2H), 7.02-6.96 (m, 2H), 6.92-6.88 (m, 1H), 5.40-5.33 (m, 1H), 3.99-3.91 (m, 2H), 3.62 (s, 3H), 3.18-3.01 (m, 7H), 2.95-2.79 (m, 4H), 2.77-2.59 (m, 3H), 2.28-2.23 (m, 1H), 2.05-1.95 (m, 2H), 1.77-1.68 (m, 1H). LCMS (ESI): m/z 800.4 [M+H]⁺.

### Preparation Example 321. Synthesis of Compound A307

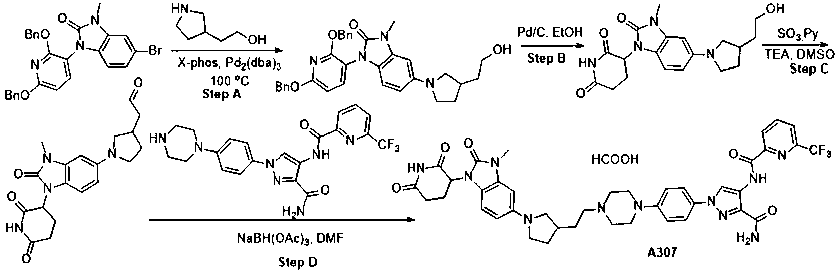

### Step A: 1-(2,6-Bis(benzyloxy)pyridin-3-yl)-5-(3-(2-hydroxyethyl)pyrrolidin-1-yl)-3-methyl-1,3-dihydro-2H-benzo[d]imidazol-2-one

At rt, to a solution of 2-(pyrrolidin-3-yl)ethan-1-ol (670 mg, 5.81 mmol, 1.5 eq) in DMF (10 mL) were added 1-(2,6-bis(benzyloxy)pyridin-3-yl)-5-bromo-3-methyl-1,3-dihydro-2H-benzo[d]imidazol-2-one (2 g, 3.87 mmol, 1.0 eq), Pd₂(dba)₃ (350 mg, 0.39 mmol, 0.1 eq), X-Phos (370 mg, 0.78 mmol, 0.2 eq), and cesium carbonate (3.8 g, 11.62 mmol, 3.0 eq). The mixture was degassed with N₂ (×3) and stirred at 100°C under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 300 mg, yield 13%).

### Step B: 3-(5-(3-(2-Hydroxyethyl)pyrrolidin-1-yl)-3-methyl-2-oxo-2,3-dihydro-1H-benzo[d]imidazol-1-yl)piperidine-2,6-dione

At rt, to a solution of 1-(2,6-bis(benzyloxy)pyridin-3-yl)-5-(3-(2-hydroxyethyl)pyrrolidin-1-yl)-3-methyl-1,3-dihydro-2H-benzo[d]imidazol-2-one (180 mg, 0.33 mmol, 1.0 eq) in ethanol (10 mL) was added 10% wet palladium on carbon (360 mg). The mixture was degassed with H₂ (×3) and stirred at 60°C under H₂ for 3 h. The reaction solution was filtered through a pad of Celite and concentrated to afford the title compound (white solid, 100 mg, yield 78%).

### Step C: 2-(1-(1-(2,6-Dioxopiperidin-3-yl)-3-methyl-2-oxo-2,3-dihydro-1H-benzo[d]imidazol-5-yl)pyrrolidin-3-yl)acetaldehyde

At rt, to a mixture of 3-(5-(3-(2-hydroxyethyl)pyrrolidin-1-yl)-3-methyl-2-oxo-2,3-dihydro-1H-benzo[d]imidazol-1-yl)piperidine-2,6-dione (100 mg, 0.27 mmol, 1.0 eq) in dimethyl sulfoxide (5 mL) were added pyridine sulfur trioxide complex (428 mg, 2.7 mmol, 10.0 eq) and TEA (0.75 mL, 5.4 mmol, 20.0 eq). The mixture was stirred at 25°C for 1 h. Upon completion, the reaction solution was diluted in EtOAc, washed sequentially with water and saturated aqueous NaCl, dried over anhydrous Na₂SO₄, and concentrated under reduced pressure to afford the title compound (yellow solid, 99 mg, crude). **Step D: N-(3-Carbamoyl-1-(4-(4-(2-(1-(2,6-dioxopiperidin-3-yl)-3-methyl-2-oxo-2,3-dihydro-1H-benzo[d]imidazol-5-yl)pyrrolidin-3-yl)ethyl)piperazin-1-yl)phenyl)-1H-pyrazol-4-yl)-6-(trifluoromethyl)picolinamide formate**

At rt, to a solution of 2-(1-(1-(2,6-dioxopiperidin-3-yl)-3-methyl-2-oxo-2,3-dihydro-1H-benzo[d]imidazol-5-yl)pyrrolidin-3-yl)acetaldehyde (99 mg, crude) in DMF (5 mL) were added N-(3-carbamoyl-1-(4-(piperazin-1-yl)phenyl)-1H-pyrazol-4-yl)-6-(trifluoromethyl)picolinamide (123 mg, 0.27 mmol, 1.0 eq) and a few drops of AcOH. The mixture was stirred at rt for 1 h. Then sodium triacetoxyborohydride (211 mg, 1.0 mmol, 4.0 eq) was added, and the mixture was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 6.2 mg, yield 3%). LCMS (ESI): m/z 814 [M+H]⁺. **¹H NMR** (400 MHz, DMSO) *δ* 11.79 (s, 1H), 11.04 (s, 1H), 8.88 (s, 1H), 8.43 (dd, *J =* 16.9, 7.6 Hz, 2H), 8.22 (s, 1H), 8.17 (s, 1H), 7.95 (s, 1H), 7.79 (d, *J* = 9.0 Hz, 2H), 7.71 (s, 1H), 7.08 (d, *J =* 9.2 Hz, 2H), 6.89 (d, *J =* 8.6 Hz, 1H), 6.37 (s, 1H), 6.21 (d, *J =* 8.7 Hz, 1H), 5.26 (dd, *J* = 12.9, 5.3 Hz, 1H), 3.48-3.42 (m, 2H), 3.29 (s, 3H), 3.25-3.20 (m, 6H), 2.92-2.84 (m, 2H), 2.75-2.60 (m, 2H), 2.59-2.53 (m, 4H), 2.45-2.41 (m, 2H), 2.33-2.28 (m, 1H), 2.17-2.12 (m, 1H), 2.02-1.95 (m, 1H), 1.67-1.63 (m, 2H).

### Preparation Example 322. Synthesis of Compound A308

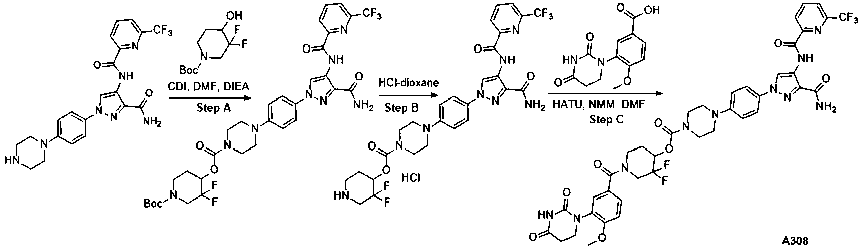

### Step A: 1-(tert-butoxycarbonyl)-3,3-difluoropiperidin-4-yl 4-(4-(3-carbamoyl-4-(6-(trifluoromethyl)pyridinylamino)-1H-pyrazol-1-yl)phenyl)piperazine-1-carboxylate

To a solution of tert-butyl 3,3-difluoro-4-hydroxypiperidine-1-carboxylate (200 mg, 0.8 mmol, 1.0 eq) in DMF (3 mL) at rt was added CDI (136 mg, 0.8 mmol, 1.0 eq). The reaction solution was stirred at rt for 1 h. N-(3-Carbamoyl-1-(4-(piperazin-1-yl)phenyl)-1H-pyrazol-4-yl)-6-(trifluoromethyl)picolinamide (194 mg, 0.4 mmol, 0.5 eq) and DIEA (0.2 mL, 1.1 mmol, 1.5 eq) were then added, and the reaction solution was reacted at 60°C for 15 h. Upon completion, the reaction solution was diluted in water and extracted with DCM. The organic phase was washed sequentially with saturated aqueous NaCl, dried over anhydrous Na₂SO₄, filtered, and concentrated. The residue was purified by column chromatography to afford the title compound (yellow solid, 150 mg, yield 27%).

### Step B: 3,3-Difluoropiperidin-4-yl 4-(4-(3-carbamoyl-4-(6-(trifluoromethyl)pyridinylamino)-1H-pyrazol-1-yl)phenyl)piperazine-1-carboxylate

1-(tert-butoxycarbonyl)-3,3-difluoropiperidin-4-yl4-(4-(3-carbamoyl-4-(6-(trifluoromethyl)pyridinylamino)-1H-pyrazol-1-yl)phenyl)piperazine-1-carboxylate (250 mg, 0.3 mmol, 1.0 eq) was added to hydrogen chloride-dioxane solution (4 mol/L) (5 mL) was stirred at rt for 2 h. Upon completion, the reaction solution was concentrated to afford the title compound (yellow solid, 200 mg, crude).

### Step C: 1-(3-(2,4-dioxotetrahydropyrimidin-1(2H)-yl)-4-methoxybenzoyl)-3,3-difluoropiperidin-4-yl 4-(4-(3-carbamoyl-4-(6-(trifluoromethyl)pyridinylamino)-1H-pyrazol-1-yl)phenyl)piperazine-1-carboxylate

To a solution of 3-(2,4-dioxotetrahydropyrimidin-1(2H)-yl)-4-methoxybenzoic acid (85 mg, 0.3 mmol, 1.0 eq) and 3,3-difluoropiperidin-4-yl 4-(4-(3-carbamoyl-4-(6-(trifluoromethyl)pyridinylamino)-1H-pyrazol-1-yl)phenyl)piperazine-1-carboxylate (200 mg, crude) in DMF (5 mL) were added N-methylmorpholine (0.2 mL, 1.6 mmol, 1.0 eq) and HATU (183 mg, 0.5 mmol, 1.5 eq) at rt. The reaction solution was stirred at rt for 2 h. Upon completion, the reaction solution was diluted in water and extracted with DCM. The organic phase was dried over anhydrous Na₂SO₄, filtered, and concentrated. The residue was purified by HPLC to afford the title compound (white solid, 27.8 mg, yield 10%). **¹H NMR** (400 MHz, DMSO-*d*₆) *δ* 11.79 (s, 1H), 10.36 (s, 1H), 8.90 (s, 1H), 8.53-8.33 (m, 2H), 8.21 (d, *J=* 7.6 Hz, 1H), 7.96 (s, 1H), 7.83 (d, *J* = 8.8 Hz, 2H), 7.72 (s, 1H), 7.49-7.36 (m, 2H), 7.20 (d, *J =* 8.6 Hz, 1H), 7.13 (d, *J* = 9.2 Hz, 2H), 5.32-5.13 (m, 1H), 3.91-3.79 (m, 5H), 3.78-3.39 (m, 8H), 3.34-3.12 (m, 4H), 2.69 (t, *J* = 4.0 Hz, 2H), 2.10-1.95 (m, 1H), 1.91-1.74 (m, 1H). LCMS (ESI): m/z 869 [M+H]⁺.

### Preparation Example 323. Synthesis of Compound A309

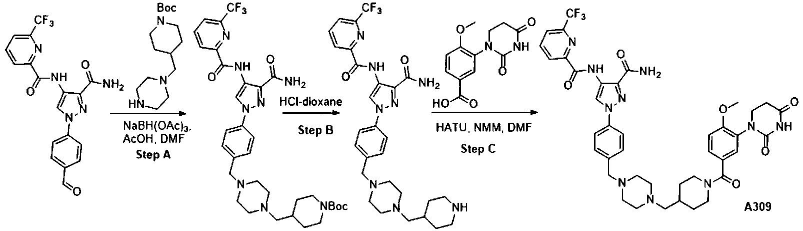

### Step A: Tert-butyl 4-((4-(4-(3-carbamoyl-4-(6-(trifluoromethyl)pyridinylamino)-1H-pyrazol-1-yl)benzyl)piperazin-1-yl)methyl)piperidine-1-carboxylate

To a solution of tert-butyl 4-(piperazine-1-methyl)piperidine-1-carboxylate (200 mg, 0.7 mmol, 1.0 eq) and N-(3-carbamoyl-1-(4-formylphenyl)-1H-pyrazol-4-yl)-6-(trifluoromethyl)picolinamide (285 mg, 0.7 mmol, 1.0 eq) in DMF (5 mL) was added AcOH (0.1 mL) at rt. The reaction solution was stirred at rt overnight, followed by the addition of sodium triacetoxyborohydride (223 mg, 1.1 mmol, 1.5 eq). The reaction solution was further stirred for 1 h. Upon completion, the reaction solution was diluted in water and extracted with DCM. The organic phase was dried, filtered, and concentrated. The residue was purified by column chromatography to afford the title compound (yellow solid, 150 mg, yield 32%).

### Step B: N-(3-Carbamoyl-1-(4-((4-(piperidin-4-ylmethyl)piperazin-1-yl)methyl)phenyl)-1H-pyrazol-4-yl)-6-(trifluoromethyl)picolinamide

Tert-butyl 4-((4-(4-(3-carbamoyl-4-(6-(trifluoromethyl)pyridinylamino)-1H-pyrazol-1-yl)benzyl)piperazin-1-yl)methyl)piperidine-1-carboxylate (150 mg, 0.2 mmol, 1.0 eq) was added to hydrogen chloride-dioxane solution (4 mol/L) (5 mL), and the mixture was stirred at rt for 2 h. Upon completion, the reaction solution was concentrated to afford the title compound (yellow oil, 100 mg, crude).

### Step C: N-(3-carbamoyl-1-(4-(4-((1-(3-(2,4-dioxotetrahydropyrimidin-1(2H)yl)-4-methoxybenzoyl)piperidin-4-yl)methyl)piperazin-1-yl)methyl)phenyl)-1H-pyrazol-4-yl)-6-(trifluoromethyl)picolinamide

To a solution of N-(3-carbamoyl-1-(4-((4-(piperidin-4-ylmethyl)piperazin-1-yl)methyl)phenyl)-1H-pyrazol-4-yl)-6-(trifluoromethyl)picolinamide (100 mg, crude) in DMF (5 mL) at rt were added N-methylmorpholine (0.1 mL, 0.9 mmol, 5.0 eq) and HATU (100 mg, 0.3 mmol, 1.5 eq). The reaction solution was stirred at rt for 18 h. Upon completion, the reaction solution was diluted in water and extracted with DCM. The organic phase was washed sequentially with saturated aqueous NaCl, dried over anhydrous Na₂SO₄, filtered, and concentrated. The residue was purified by HPLC to afford the title compound (white solid, 30 mg, yield 21% over two steps). **¹H NMR** (400 MHz, DMSO-*d*₆) *δ* 11.80 (s, 1H), 10.33 (s, 1H), 9.01 (s, 1H), 8.44 (dd, *J* = 17.6, 7.8 Hz, 2H), 8.22 (d, *J* = 7.8 Hz, 1H), 8.01 (s, 1H), 7.94 (d, *J* = 8.4 Hz, 2H), 7.77 (s, 1H), 7.46 (d, *J=* 8.4 Hz, 2H), 7.36 (dd, *J* = 8.4*,* 2.0 Hz, 1H), 7.31 (d, *J* = 2.0 Hz, 1H), 7.15 (d, *J* = 8.4 Hz, 1H), 4.64-4.03 (m, 1H), 3.97-3.71 (m, 4H), 3.63-3.56 (m, 2H), 3.54-3.48 (m, 2H), 3.08-2.75 (m, 2H), 2.68 (t, *J* = 6.6 Hz, 2H), 2.45-2.22 (m, 7H), 2.15 (d, *J =* 6.8 Hz, 2H), 1.82-1.59 (m, 3H), 1.12-0.99 (m, 2H). LCMS (ESI): m/z 817 [M+H]⁺.

### Preparation Example 324. Synthesis of Compound A310

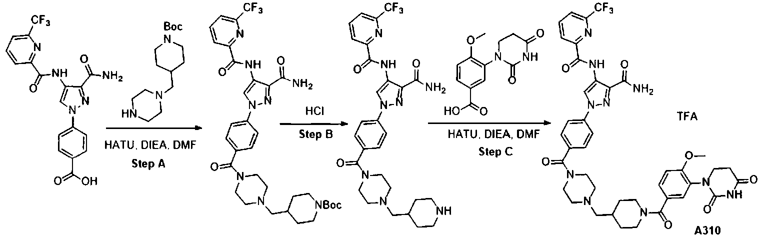

### Step A: Tert-butyl 4-(4-(4-(3-carbamoyl-4-(6-(trifluoromethyl)picolinamido)-1H-pyrazol-1-yl)benzoyl)piperazin-1-yl)methyl)piperidine-1-carboxylate

To a solution of 4-(3-carbamoyl-4-(6-(trifluoromethyl)picolinamido)-1H-pyrazol-1-yl)benzoic acid (500 mg, 1.2 mmol, 1.0 eq) and HATU (680 mg, 1.8 mmol, 1.5 eq) in DMF (10 mL) were added tert-butyl 4-(piperazine-1-methyl)piperidine-1-carboxylate (372 mg, 1.312 mmol, 1.1 eq) and DIEA (0.6 mL, 3.577 mmol, 3.0 eq) at rt. The reaction solution was stirred at rt for 1 h. Upon completion, the mixture was diluted in water and extracted with EtOAc. The organic phase was washed sequentially with saturated aqueous NaCl, dried over anhydrous Na₂SO₄, filtered, and concentrated. The residue was purified by column chromatography to afford the title compound (yellow oil, 410 mg, yield 50%).

### Step B: N-(3-Carbamoyl-1-(4-(4-(piperidin-4-ylmethyl)piperazine-1-carboxyl)phenyl)-1H-pyrazol-4-yl)-6-(trifluoromethyl)picolinamide

To a solution of tert-butyl 4-(4-(4-(3-carbamoyl-4-(6-(trifluoromethyl)picolinamido)-1H-pyrazol-1-yl)benzoyl)piperazin-1-yl)methyl)piperidine-1-carboxylate (410 mg, 0.6 mmol, 1.0 eq) in EtOAc (5 mL) was added a solution of hydrogen chloride in MeOH (4 mol/L, 1 mL). The reaction solution was stirred at rt for 1 h. Upon completion, the reaction solution was concentrated to afford the title compound (yellow solid, 330 mg, crude).

### Step C: N-(3-carbamoyl-1-(4-((1-(3-(2,4-dioxotetrahydropyrimidin-1(2H)yl)-4-methoxybenzoyl)piperidin-4-yl)methyl)piperazine-1-carbonyl)phenyl)-1H-pyrazol-4-yl)-6-(trifluoromethyl)picolinamide trifluoroacetate

To a solution of 3-(2,4-dioxotetrahydropyrimidin-1(2H)yl)-4-methoxybenzoic acid (100 mg, 0.4 mmol, 1.0 eq) and HATU (187 mg, 0.5 mmol, 1.5 eq) in DMF (5 mL) were added N-(3-carbamoyl-1-(4-(4-(piperidin-4-ylmethyl)piperazine-1-carbonyl)phenyl)- 1H-pyrazol-4-yl)-6-(trifluoromethyl)picolinamide (330 mg, crude) and DIEA (0.3 mL, 1.9 mmol, 5.0 eq) at rt. The reaction solution was stirred at rt for 1 h. Upon completion, the reaction solution was diluted in water and extracted with EtOAc. The organic phase was washed sequentially with saturated aqueous NaCl, dried over anhydrous Na₂SO₄, filtered, and concentrated. The residue was purified by HPLC to afford the title compound (white solid, trifluoroacetate, 26.7 mg, yield 8%). **¹H NMR** (400 MHz, DMSO-*d*₆) *δ* 11.81 (s, 1H), 10.34 (s, 1H), 9.54 (s, 1H), 9.13 (s, 1H), 8.50-8.36 (m, 2H), 8.23 (d, *J=* 6.7 Hz, 1H), 8.13 (d, *J =* 8.5 Hz, 2H), 8.07 (s, 1H), 7.85 (s, 1H),7.66 (d, *J* = 8.3 Hz, 2H), 7.38 (dd, *J =* 8.4, 2.0 Hz, 1H), 7.33 (d, *J =* 2.1 Hz, 1H), 7.17 (d, *J =* 8.6 Hz, 1H), 4.41 (s, 2H), 3.85 (s, 3H), 3.60 (t, *J=* 6.6 Hz, 4H), 3.09 (s, 7H), 2.69 (t, *J =* 6.6 Hz, 2H), 2.13 (s, 1H), 2.04-1.50 (m, 3H), 1.44-1.08 (m, 4H). LCMS (ESI): m/z 831 [M+H]⁺.

### Preparation Example 325. Synthesis of Compound A311

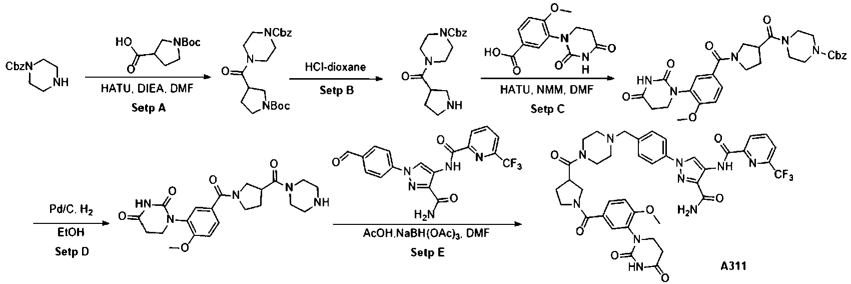

### Step A: Benzyl 4-(1-tert-butoxycarbonyl)pyrrolidine-3-carbonyl)piperazine-1-carboxylate

To a solution of 1-(tert-butoxycarbonyl)pyrrolidine-3-carboxylic acid (1 g, 4.6 mmol, 1.0 eq) and 1-benzylpiperazinecarboxylate (1 g, 4.6 mmol, 1.0 eq) in DMF (13 mL) at rt were added DIEA (2.4 mL, 13.9 mmol, 3.0 eq) and HATU (2.7 g, 7.0 mmol, 1.5 eq). The reaction solution was stirred at rt for 5 h. Upon completion, the reaction solution was diluted in water and extracted with EtOAc. The organic phase was washed with aqueous NaCl, dried over anhydrous Na₂SO₄, filtered, and concentrated. The residue was purified by column chromatography to afford the title compound (colorless oil, 1.2 g, yield 62%).

### Step B: Benzyl 4-(pyrrolidine-3-carbonyl)piperazine-1-carboxylate

Benzyl 4-(1-tert-butoxycarbonyl)pyrrolidine-3-carbonyl)piperazine-1-carboxylate (300 mg, 0.7 mmol, 1.0 eq) was added to hydrogen chloride-dioxane solution (4 mol/L) (5 mL), and the mixture was stirred at rt for 2 h. Upon completion, the reaction solution was concentrated to afford the title compound as a yellow oil (200 mg, crude).

### Step C: Benzyl 4-(1-(3-(2,4-dioxotetrahydropyrimidin-1(2H)-yl)-4-methoxybenzoyl)pyrrolidine-3-carbonyl)piperazine-1-carboxylate

To a solution of 3-(2,4-dioxotetrahydropyrimidin-1(2H)yl)-4-methoxybenzoic acid (209 mg, 0.8 mmol, 1.1 eq) and benzyl 4-(pyrrolidine-3-carbonyl)piperazine-1-carboxylate (200 mg, crude) in DMF (5 mL) were added N-methylmorpholine (0.4 mL, 3.6 mmol, 5.0 eq) and HATU (410 mg, 1.1 mmol, 1.5 eq) at rt. The reaction solution was stirred at rt for 18 h. Upon completion, the reaction solution was diluted in water and extracted with DCM. The organic phase was washed with aqueous NaCl, dried over anhydrous Na₂SO₄, filtered, and concentrated. The residue was purified by column chromatography to afford the title compound (yellow solid, 300 mg, yield 74% over two steps).

### Step D: 1-(2-methoxy-5-(3-(piperazine-1-carbonyl)pyrrolidine-1-carbonyl)phenyl)dihydropyrimidine-2,4(1H,3H)-dione

To a solution of benzyl 4-(1-(3-(2,4-dioxotetrahydropyrimidin-1(2H)-yl)-4-methoxybenzoyl)pyrrolidine-3-carbonyl)piperazine-1-carboxylate (200 mg, 0.4 mmol, 1.0 eq) in ethanol (5 mL) was added 10% Pd/C (40 mg). The reaction mixture was purged with H₂ (×3) and further reacted under H₂ for 2 h. Upon completion, the reaction solution was filtered and the filtrate was concentrated to provide the title compound (yellow solid, 130 mg, yield 85%).

### Step E: N-(3-carbamoyl-1-(4-(4-(1-(3-(2,4-dioxotetrahydropyrimidin-1(2H)yl)-4-methoxybenzoyl)pyrrolidine-3-carbonyl)piperazin-1-yl)methyl)phenyl)-1H-pyrazol-4-yl)-6-(trifluoromethyl)picolinamide

To a solution of N-(3-carbamoyl-1-(4-formylphenyl)-1H-pyrazol-4-yl)-6-(trifluoromethyl)picolinamide (188.0 mg, 0.5 mmol, 1.0 eq) and 1-(2-methoxy-5-(3-(piperazine-1-carbonyl)pyrrolidine-1-carbonyl)phenyl)dihydropyrimidine-2,4(1H,3H)-dione (200 mg, 0.5 mmol, 1.0 eq) in DMF (5 mL) was added AcOH (0.1 mL) at rt. The reaction solution was stirred at rt for 15 h. Sodium triacetoxyborohydride (147 mg, 0.7 mmol, 1.5 eq) was added, and the reaction solution was stirred for another 1 h. Upon completion, the reaction solution was diluted in water and extracted with DCM. The organic phase was concentrated, and then the residue was purified by HPLC to afford the title compound (white solid, 27.8 mg, yield 7%). **¹H NMR** (400 MHz, DMSO-*d*₆) *δ* 11.79 (s, 1H), 10.33 (s, 1H), 9.01 (s, 1H), 8.50-8.35 (m, 2H), 8.22 (d, *J* = 7.6 Hz, 1H), 8.01 (s, 1H), 7.95 (d, *J* = 7.8 Hz, 2H), 7.78 (s, 1H), 7.58-7.43 (m, 4H), 7.15 (d, *J =* 8.6 Hz, 1H), 3.85 (s, 3H), 3.71-3.39 (m, 12H), 2.68 (t, *J =* 6.4 Hz, 2H), 2.44-2.31 (m, 4H), 2.23-1.74 (m, 3H). LCMS (ESI): m/z 817 [M+H]⁺.

### Preparation Example 326. Synthesis of Compound A312

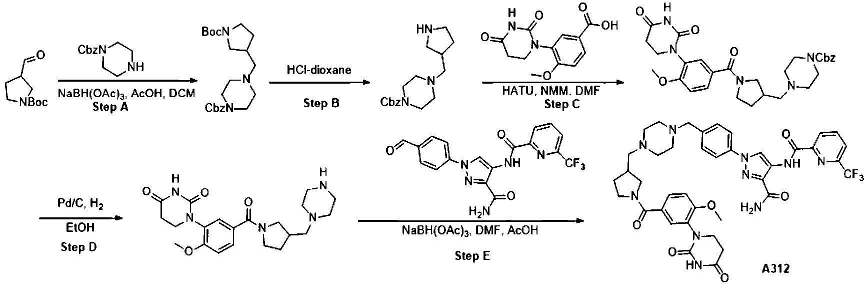

### Step A: Benzyl 4-(1-(tert-butoxycarbonyl)pyrrolidin-3-yl)methyl)piperazine-1-carboxylate

To a solution of tert-butyl 3-formylpyrrolidine-1-carboxylate (1.0 g, 5.0 mmol, 1.0 eq) and benzyl piperazine-1-carboxylate (1.2 g, 5.5 mmol, 1.1 eq) in DCM (20 mL) was added AcOH (0.1 mL) at rt. The reaction solution was stirred at rt for 2 h. Sodium triacetoxyborohydride (1.6 g, 7.5 mmol, 1.5 eq) was then added, and the mixture was stirred for 2 h. Upon completion, the reaction solution was diluted in water and extracted with DCM. The organic phase was washed sequentially with saturated aqueous NaCl, dried over anhydrous Na₂SO₄, filtered, and concentrated. The residue was purified by column chromatography to afford the title compound (yellow oil, 1.4 g, yield 69%).

### Step B: Benzyl 4-(pyrrolidin-3-ylmethyl)piperazine-1-carboxylate

Benzyl 4-(1-(tert-butoxycarbonyl)pyrrolidin-3-yl)methyl)piperazine-1-carboxylate (700 mg, 1.7 mmol, 1.0 eq) was added to hydrogen chloride-dioxane solution (4 mol/L, 5 mL). The reaction solution was stirred at rt overnight. Upon completion, the reaction solution was concentrated to afford the title compound (white solid, 500 mg, crude).

### Step C: Benzyl 4-(1-(3-(2,4-dioxotetrahydropyrimidin-1(2H)yl)-4-methoxybenzoyl)pyrrolidin-3-yl)methyl)piperazine-1-carboxylate

To a solution of 3-(2,4-dioxotetrahydropyrimidin-1(2H)yl)-4-methoxybenzoic acid (457 mg, 1.7 mmol, 1.0 eq), benzyl 4-(pyrrolidin-3-ylmethyl)piperazine-1-carboxylate (500 mg, crude), and N-methylmorpholine (1.0 mL, 8.6 mmol, 5.0 eq) in DMF (20 mL) was added HATU (986 mg, 2.6 mmol, 1.5 eq) at rt. The reaction solution was stirred at rt overnight. Upon completion, the mixture was diluted in water and extracted with DCM. The organic phase was washed sequentially with saturated aqueous NaCl, dried over anhydrous Na₂SO₄, filtered, and concentrated. The residue was purified by column chromatography to afford the title compound (yellow solid, 500 mg, yield 53%).

### Step D: 1-(2-methoxy-5-(3-(piperazine-1-methyl)pyrrolidine-1-carbonyl)phenyl)dihydropyrimidine-2,4(1H,3H)-dione

To a solution of benzyl 4-(1-(3-(2,4-dioxotetrahydropyrimidin-1(2H)yl)-4-methoxybenzoyl)pyrrolidin-3-yl)methyl)piperazine-1-carboxylate (200 mg, 0.4 mmol, 1.0 eq) in ethanol (5 mL) was added 10% Pd/C (40 mg). The reaction mixture was purged with H₂ (×3) and further reacted under H₂ for 2 h. Upon completion, the reaction solution was filtered and the filtrate was concentrated to afford the title compound (yellow solid, 150 mg, yield 99%). **Step E: N-(3-carbamoyl-1-(4-(4-((1-(3-(2,4-dioxotetrahydropyrimidin-1(2H)yl)-4-methoxybenzoyl)pyrrolidin-3-yl)methyl)piperazin-1-yl)methyl)phenyl)-1H-pyrazol-4-yl)-6-(trifluoromethyl)picolinamide**

To a solution of N-(3-carbamoyl-1-(4-formylphenyl)-1H-pyrazol-4-yl)-6-(trifluoromethyl)picolinamide (291 mg, 0.7 mmol, 1.0 eq) and 1-(2-methoxy-5-(3-(piperazine-1-methyl)pyrrolidine-1-carbonyl)phenyl)dihydropyrimidine-2,4(1H,3H)-dione (300 mg, 0.7 mmol, 1.0 eq) in DMF (5 mL) was added AcOH (0.1 mL) at rt. The reaction solution was stirred at rt for 15 h. Sodium triacetoxyborohydride (228.4 mg, 1.1 mmol, 1.5 eq) was added, and the reaction solution was stirred for another 1 h. Upon completion, the reaction solution was diluted in water and extracted with DCM. The organic phase was washed sequentially with saturated aqueous NaCl, dried over anhydrous Na₂SO₄, filtered, and concentrated. The residue was purified by HPLC to afford the title compound (white solid, 35 mg, yield 6%). **¹H NMR** (400 MHz, DMSO-*d*₆) *δ* 11.79 (s, 1H), 10.33 (d, *J =* 5.2 Hz, 1H), 9.00 (s, 1H), 8.48-8.38 (m, 2H), 8.22 (d, *J =* 6.8 Hz, 1H), 8.01 (s, 1H), 7.96-7.88 (m, 2H), 7.77 (s, 1H), 7.56-7.41 (m, 4H), 7.18-7.08 (m, 1H), 3.85 (s, 3H), 3.70-3.35 (m, 8H), 3.29-3.12 (m, 2H), 2.68 (t, *J =* 6.4 Hz, 2H), 2.45-2.18 (m, 9H), 2.02-1.84 (m, 1H), 1.65-1.48 (m, 1H). LCMS (ESI): m/z 803 [M+H]⁺.

### Preparation Example 327. Synthesis of Compound A313

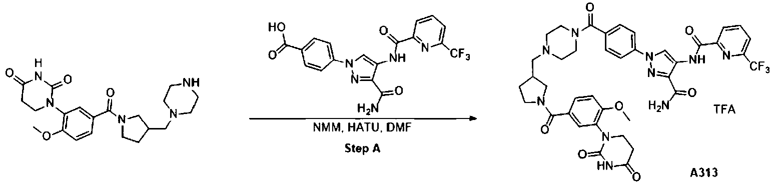

### Step A: N-(3-carbamoyl-1-(4-((1-(3-(2,4-dioxotetrahydropyrimidin-1(2H)yl)-4-methoxybenzoyl)pyrrolidin-3-yl)methyl)piperazine-1-carbonyl)phenyl)-1H-pyrazol-4-yl)-6-(trifluoromethyl)picolinamide trifluoroacetate

To a solution of 4-(3-carbamoyl-4-(6-(trifluoromethyl)picolinamido)-1H-pyrazol-1-yl)benzoic acid (100 mg, 0.2 mmol, 1.0 eq) and 1-(2-methoxy-5-(3-(piperazine-1-methyl)pyrrolidine-1-carbonyl)phenyl)dihydropyrimidine-2,4(1H,3H)-dione (117 mg, 0.2 mmol, 1.0 eq) in DMF (5 mL) at rt were added N-methylmorpholine (0.1 mL, 0.7 mmol, 3.0 eq) and HATU (136 mg, 0.3 mmol, 3.0 eq). The reaction solution was stirred at rt for 18 h. Upon completion, the reaction solution was diluted in water and extracted with DCM. The organic phase was concentrated, and then the residue was purified by HPLC to afford the title compound (white solid, 35 mg, yield 19%). **¹H NMR** (400 MHz, DMSO-*d*₆) *δ* 11.81 (s, 1H), 10.35 (s, 1H), 9.54 (s, 1H), 9.14 (s, 1H), 8.50-8.38 (m, 2H), 8.23 (d, *J =* 6.8 Hz, 1H), 8.14 (s, 2H), 8.07 (s, 1H), 7.86 (s, 1H), 7.68 (d, *J* = 8.4 Hz, 2H), 7.55 (d*, J* = 8.4 Hz, 1H), 7.50 (s, 1H), 7.17 (d, *J* = 8.8 Hz, 1H), 4.81-4.14 (m, 1H), 3.86 (s, 3H), 3.83-3.48 (m, 8H), 3.34-3.09 (m, 7H), 2.78-2.59 (m, 3H), 2.23-2.04 (m, 1H), 1.79-1.62 (m, 1H). LCMS (ESI): m/z 817 [M+H]⁺.

### Preparation Example 328. Synthesis of Compound A314

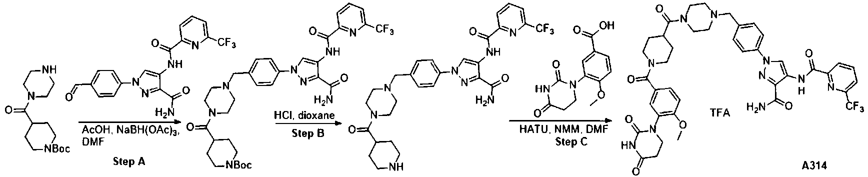

### Step A: Tert-butyl 4-(4-(4-(3-carbamoyl-4-(6-(trifluoromethyl)pyridinylamino)-1H-pyrazol-1-yl)benzyl)piperazine-1-carbonyl)piperidine-1-carboxylate

To a solution of tert-butyl 4-(piperazine-1-formyl)piperidine-1-carboxylate (250 mg, 0.8 mmol, 1.0 eq) and N-(3-carbamoyl-1-(4-formylphenyl)-1H-pyrazol-4-yl)-6-(trifluoromethyl)picolinamide (373 mg, 0.9 mmol, 1.1 eq) in DMF (7 mL) was added AcOH (0.3 mL) at rt. The reaction solution was stirred at rt for 15 h, followed by the addition of sodium triacetoxyborohydride (355 mg, 1.7 mmol, 2.0 eq). The mixture was stirred for another 2 h. Upon completion, the reaction solution was diluted in water and extracted with DCM. The organic phase was concentrated, and then the residue was purified by column chromatography to afford the title compound (yellow oil, 90 mg, yield 15%).

### Step B: N-(3-Carbamoyl-1-(4-((4-(piperidine-4-carbonyl)piperazin-1-yl)methyl)phenyl)-1H-pyrazol-4-yl)-6-(trifluoromethyl)picolinamide

Tert-butyl 4-(4-(4-(3-carbamoyl-4-(6-(trifluoromethyl)pyridinylamino)-1H-pyrazol-1-yl)benzyl)piperazine-1-carbonyl)piperidine-1-carboxylate (90 mg, 0.1 mmol, 1.0 eq) was added to hydrogen chloride-dioxane solution (4 mol/L) (4 mL), and the mixture was stirred at rt for 2 h. Upon completion, the reaction solution was concentrated to afford the title compound (yellow solid, 70 mg, crude).

### Step C: N-(3-Carbamoyl-1-(4-((4-(1-(3-(2,4-dioxotetrahydropyrimidin-1(2H)-yl)-4-methoxybenzoyl)piperidine-4-carbonyl)piperazin-1-yl)methyl)phenyl)-1H-pyrazol-4-yl)-6-(trifluoromethyl)picolinamide trifluoroacetate

To a solution of N-(3-carbamoyl-1-(4-((4-(piperidine-4-carbonyl)piperazin-1-yl)methyl)phenyl)-1H-pyrazol-4-yl)-6-(trifluoromethyl)picolinamide (70 mg, crude) and 3-(2,4-dioxotetrahydropyrimidin-1(2H)-yl)-4-methoxybenzoic acid (35 mg, 0.1 mmol, 1.0 eq) in DMF (5 mL) at rt were added N-methylmorpholine (0.1 mL, 0.6 mmol, 5.0 eq) and HATU (68 mg, 0.2 mmol, 1.5 eq). The reaction solution was stirred at rt for 16 h. Upon completion, the reaction solution was diluted in water and extracted with DCM. The organic phase was washed sequentially with saturated aqueous NaCl, dried over anhydrous Na₂SO₄, filtered, and concentrated. The residue was purified by HPLC to afford the title compound (white solid, 27.6 mg, yield 26%). **¹H NMR** (400 MHz, DMSO-*d*₆) *δ* 11.80 (s, 1H), 10.34 (s, 1H), 10.09 (s, 1H), 9.12 (s, 1H), 8.52-8.34 (m, 2H), 8.24 (d, *J =* 7.4 Hz, 1H), 8.15 (d, *J* = 8.2 Hz, 2H), 8.05 (s, 1H), 7.85 (s, 1H), 7.67 (d, *J* = 8.2 Hz, 2H), 7.39 (d, *J =* 8.4 Hz, 1H), 7.35 (s, 1H), 7.17 (d, *J =* 8.6 Hz, 1H), 4.32 (d, *J =* 69.0 Hz, 4H), 3.85 (s, 3H), 3.60 (t, *J =* 6.5 Hz, 2H), 3.36 (s, 4H), 3.01 (dd, *J =* 36.7, 26.2 Hz, 7H), 2.69 (t, *J =* 6.3 Hz, 2H), 1.69 (s, 2H), 1.58-1.43 (m, 2H). LCMS (ESI): m/z 831 [M+H]⁺.

### Preparation Example 329. Synthesis of Compound A315

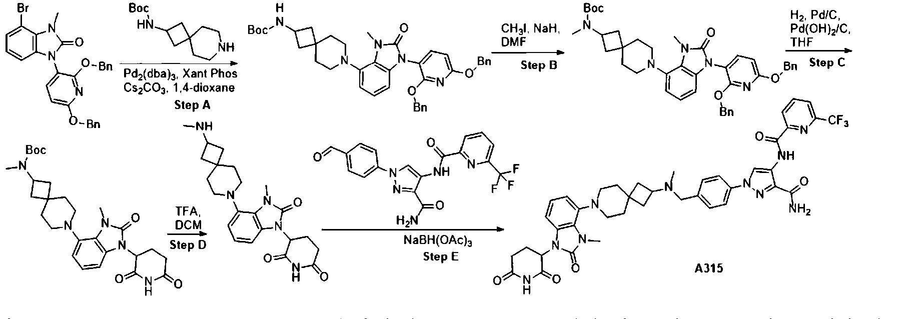

### Step A: Tert-butyl (7-(1-(2,6-bis(benzyloxy)pyridin-3-yl)-3-methyl-2-oxo-2,3-dihydro-1H-benzo[d]imidazol-4-yl)-7-azaspiro[3.5]nonan-2-yl)carbamate

To a solution of tert-butyl (7-azaspiro[3.5]-2-nonyl)carbamate (2.8 g, 12 mmol, 1.5 eq) in 1,4-dioxane (48 mL) at rt under N₂ were added 1-(2,6-bis(benzyloxy)pyridin-3-yl)-4-bromo-3-2-methyl-1,3-dihydro-2H-benzo[d]imidazol-2-one (4 g, 8 mmol, 1.0 eq), Pd₂(dba)₃ (1 g, 1.2 mmol, 0.15 eq), and Xant Phos (680 mg, 1.2 mmol, 0.15 eq). The mixture was stirred at rt under N₂ for 18 h. Upon completion, the mixture was diluted in EtOAc, washed sequentially with water and saturated aqueous NaCl, dried over anhydrous Na₂SO₄, and concentrated under reduced pressure. The residue was purified by column chromatography to afford the title compound (yellow solid, 2.8 g, yield 52%).

### Step B: Tert-butyl (7-(1-(2,6-bis(benzyloxy)pyridin-3-yl)-3-methyl-2-oxo-2,3-dihydro-1H-benzo[d]imidazol-4-yl)-7-azaspiro[3.5]nonan-2-yl)(methyl)carbamate

To a solution of tert-butyl (7-(1-(2,6-bis(benzyloxy)pyridin-3-yl)-3-methyl-2-oxo-2,3-dihydro-1H-benzo[d]imidazol-4-yl)-7-azaspiro[3.5]nonan-2-yl)carbamate (500 mg, 0.74 mmol, 1.0 eq) in DMF (5 mL) was added NaH (44 mg, 1.11 mmol, 1.5 eq, 60% dispersion in mineral oil) at 0°C. The reaction solution was stirred at 0°C for 30 min under N₂. Then iodomethane (158 mg, 1.11 mmol, 1.5 eq) was added, and the mixture was stirred at rt overnight. Upon completion, the reaction solution was quenched with saturated aqueous NH₄Cl, diluted in EtOAc, washed sequentially with water and saturated aqueous NaCl, dried over anhydrous Na₂SO₄, and concentrated under reduced pressure. The residue was purified by column chromatography to afford the title compound (yellow solid, 490 mg, yield 96%).

### Step C: Tert-butyl (7-(1-(2,6-dioxopiperidin-3-yl)-3-methyl-2-oxo-2,3-dihydro-1H-benzo[d]imidazol-4-yl)-7-azaspiro[3.5]nonan-2-yl)(methyl)carbamate

To a solution of tert-butyl (7-(1-(2,6-bis(benzyloxy)pyridin-3-yl)-3-methyl-2-oxo-2,3-dihydro-1H-benzo[d]imidazol-4-yl)-7-azaspiro[3.5]nonan-2-yl)(methyl)carbamate (490 mg, 0.71 mmol, 1.0 eq) in THF (5 mL) at rt were added 10% wet Pd/C (0.2 g) and 10% wet Pd(OH)₂/C (0.2 g). The reaction solution was degassed (×3) under H₂ and stirred at rt overnight under H₂. Upon completion, the reaction solution was filtered through Celite, and the filtrate was concentrated under reduced pressure to afford the title compound (yellow solid, 280 mg, yield 77%).

### Step D: 3-(3-methyl-4-(2-(methylamino)-7-azaspiro[3.5]nonan-7-yl)-2-oxo-2,3-dihydro-1H-benzo[d]imidazol-1-yl)piperidine-2,6-dione

To a solution of tert-butyl (7-(1-(2,6-dioxopiperidin-3-yl)-3-methyl-2-oxo-2,3-dihydro-1H-benzo[d]imidazol-4-yl)-7-azaspiro[3.5]nonan-2-yl)(methyl)carbamate (280 mg, 0.55 mmol, 1.0 eq) in DCM (3 mL) was added TFA (1.5 mL) under an ice-water bath. The mixture was then stirred at rt for 1 h. Upon completion, the reaction solution was concentrated under reduced pressure and the residue was purified by HPLC to afford the title compound (white solid, 180 mg, yield 80%).

### Step E: N-(3-carbamoyl-1-(4-(methyl(7-(3-methyl-2-oxo-1-(6-oxopiperidin-3-yl)-2,3-dihydro-1H-benzo[d]imidazol-4-yl)-7-azaspiro[3.5]nonan-2-yl)amino)methyl)phenyl)-1H-pyrazol-4-yl)-6-(trifluoromethyl)picolinamide

Under N₂, magnesium sulfate (150 mg) and sodium triacetoxyborohydride (126 mg, 0.60 mmol, 3.0 eq) were sequentially added to a solution of N-(3-carbamoyl-1-(4-formylphenyl)-1H-pyrazol-4-yl)-6-(trifluoromethyl)picolinamide (80 mg, 0.20 mmol, 1.0 eq) and 3-(3-methyl-4-(2-(methylamino)-7-azaspiro[3.5]nonan-7-yl)-2-oxo-2,3-dihydro-1H-benzo[d]imidazol-1-yl)piperidine-2,6-dione (106 mg, 0.26 mmol, 1.3 eq) in DMF (3 mL). The mixture was stirred at rt under N₂ overnight. Upon completion, the reaction mixture was filtered. The filtrate was concentrated under reduced pressure, and the residue was purified by HPLC to afford the title compound (white solid, 30 mg, yield 19%). LCMS (ESI): m/z 785 [M+H]⁺. **¹H NMR** (400 MHz, DMSO-*d*₆) *δ* 11.80 (s, 1H), 11.08 (s, 1H), 9.01 (s, 1H), 8.47-8.37 (m, 2H), 8.21 (d, *J =* 7.6 Hz, 1H), 8.16 (s, 1H), 8.02 (s, 1H), 7.94 (d, *J =* 8.5 Hz, 2H), 7.77 (s, 1H), 7.46 (d, *J =* 8.4 Hz, 2H), 6.97 (t, *J =* 7.9 Hz, 1H), 6.92-6.84 (m, 2H), 5.39-5.30 (m, 1H), 3.63 (s, 3H), 3.40 (s, 2H), 2.99-2.84 (m, 4H), 2.76-2.58 (m, 4H), 2.27-2.15 (m, 1H), 2.04-1.94 (m, 5H), 1.81-1.61 (m, 6H).

### Preparation Example 330. Synthesis of Compound A316

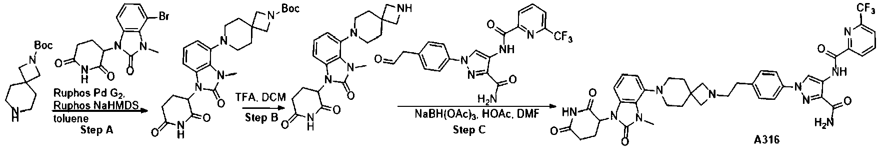

### Step A: Tert-butyl 7-(1-(2,6-dioxopiperidin-3-yl)-3-methyl-2-oxo-2,3-dihydro-1H-benzo[d]imidazol-4-yl)-2,7-diazaspiro[3.5]nonane-2-carboxylate

To a solution of tert-butyl 2,7-diazaspiro[3.5]nonane-2-carboxylate (339 mg, 1.48 mmol, 1.0 eq) in toluene (48 mL) at rt under N₂ were added 3-(4-bromo-3-methyl-2-oxo-2,3-dihydro-1H-benzimidazol-1-yl)piperidine-2,6-dione (500 mg, 1.48 mmol, 1.0 eq), NaHMDS (541 mg, 2.96 mmol, 2.0 eq) and Ruphos (138 mg, 0.23 mmol, 0.15 eq), followed by the addition of Ruphos Pd G₂ (116 mg, 0.15 mmol, 0.1 eq). The reaction solution was stirred at 100°C under N₂ for 3 h. Upon completion, the reaction solution was diluted in EtOAc, washed sequentially with water and saturated aqueous NaCl, dried over anhydrous Na₂SO₄, and concentrated under reduced pressure. The residue was purified by column chromatography (eluent: DCM/MeOH, gradient: 0%-10% MeOH) to afford the title compound (yellow oil, 270 mg, yield 37%).

### Step B: 3-(3-methyl-2-oxo-4-(2,7-diazaspiro[3.5]nonan-7-yl)-2,3-dihydro-1H-benzimidazol-1-yl)piperidine-2,6-dione

To a solution of tert-butyl 7-(1-(2,6-dioxopiperidin-3-yl)-3-methyl-2-oxo-2,3-dihydro-1H-benzo[d]imidazol-4-yl)-2,7-diazaspiro[3.5]nonane-2-carboxylate (250 mg, 0.50 mmol, 1.0 eq) in DCM (5 mL) was added TFA (1 mL) at rt. The reaction solution was stirred at rt under N₂ for 2 h. Upon completion, the reaction solution was concentrated under reduced pressure, and the residue was neutralized with saturated NaHCO₃ solution and extracted with DCM (×3). The organic phases were combined and concentrated under reduced pressure to afford the title compound (white solid, 150 mg, crude), which was used directly without purification.

### Step C: N-(3-carbamoyl-1-(4-(2-(7-(1-(2,6-dioxopiperidin-3-yl)-3-methyl-2-oxo-2,3-dihydro-1H-benzo[d]imidazol-4-yl)-2,7-diazaspiro[3.5]nonan-2-yl)ethyl)phenyl)-1H-pyrazol-4-yl)-6-(trifluoromethyl)picolinamide

To a solution of N-(3-carbamoyl-1-(4-(2-oxoethyl)phenyl)-1H-pyrazol-4-yl)-6-(trifluoromethyl)picolinamide (110 mg, 0.26 mmol, 1.0 eq) and 3-(3-methyl-2-oxo-4-(2,7-diazaspiro[3.5]nonan-7-yl)-2,3-dihydro-1H-benzimidazol-1-yl)piperidine-2,6-dione (101 mg, crude) in DMF (2 mL) at rt under N₂ were added AcOH (0.01 mL, 0.02 mmol, 0.1 eq) and NaBH(OAc)₃ (117 mg, 0.53 mmol, 2.0 eq). The reaction solution was stirred at rt under N₂ for 0.5 h. Upon completion, the reaction solution was quenched with saturated NaHCO₃ solution and extracted with EtOAc twice. The organic phases were combined, washed sequentially with water and saturated aqueous NaCl, dried over anhydrous Na₂SO₄, and concentrated under reduced pressure. The residue was purified by preparative HPLC (C₁₈, 30%-90% acetonitrile in water containing 0.1% formic acid) to afford the title compound (white solid, 8.8 mg, yield 4%). LCMS (ESI): m/z 785.4 [M+H]⁺. **¹H NMR** (400 MHz, DMSO-*d*₆) *δ* 11.79 (s, 1H), 11.08 (s, 1H), 8.99 (s, 1H), 8.47-8.38 (m, 2H), 8.21 (s, 1H), 8.00 (s, 1H), 7.89 (d, *J =* 8.5 Hz, 2H), 7.76 (s, 1H), 7.40 (d, *J* = 8.5 Hz, 2H), 7.03-6.92 (m, 1H), 6.87 (t, *J =* 7.7 Hz, 2H), 5.36-5.32 (m, 1H), 3.62 (s, 3H), 3.22-2.82 (m, 8H), 2.76-2.61 (m, 7H), 2.00-1.99 (m, 1H), 1.88-1.83 (m, 4H).

### Preparation Example 331. Synthesis of Compound A317

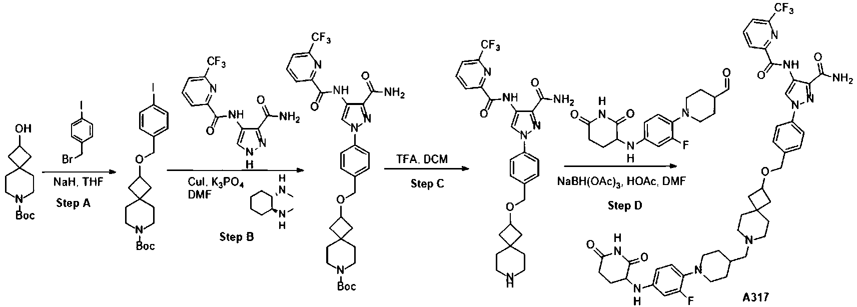

### Step A: Tert-butyl 2-((4-iodobenzyl)oxy)-7-azaspiro[3.5]nonane-7-carboxylate

Under an ice-water bath, sodium hydride (238 mg, 6.0 mmol, 60% dispersion in mineral oil, 1.5 eq) was added portionwise to a solution of tert-butyl 2-hydroxy-7-azaspiro[3.5]nonane-7-carboxylate (960 mg, 3.98 mmol, 1.0 eq) in THF (10 mL). The reaction solution was stirred at rt for 0.5 h under N₂. 1-(Bromomethyl)-4-iodobenzene (1.182 g, 3.98 mmol, 1.0 eq) was then added. The reaction was stirred at rt for 6 h. Upon completion, the reaction solution was quenched with saturated ammonium chloride at 0°C and extracted twice with DCM. The combined organic phases were washed with saturated aqueous NaCl, dried over anhydrous Na₂SO₄, and concentrated under reduced pressure. The residue was purified by column chromatography to afford the title compound (yellow solid, 1.18 g, yield 65%).

### Step B: Tert-butyl 2-((4-(3-carbamoyl-4-(6-(trifluoromethyl)pyridinylamino)-1H-pyrazol-1-yl)benzyl)oxy)-7-azaspiro[3.5]nonane-7-carboxylate

Under N₂, potassium phosphate (638 mg, 3.0 mmol, 3.0 eq), cuprous iodide (95 mg, 0.5 mmol, 0.5 eq), and trans-(1R,2R)-N,N'-dimethyl-1,2-cyclohexanediamine (170 mg, 1.0 mmol, 1.0 eq) were added sequentially to a solution of tert-butyl 2-((4-iodobenzyl)oxy)-7-azaspiro[3.5]nonane-7-carboxylate (458 mg, 1.0 mmol, 1.0 eq) and N-(3-carbamoyl-1H-pyrazol-4-yl)-6-(trifluoromethyl)picolinamide (300 mg, 1.0 mmol, 1.0 eq) in DMF (5 mL). The reaction solution was purged with N₂ (×3), and the reaction was stirred at 80°C under N₂ for 16 h. Upon completion, the reaction solution was diluted in water and extracted with EtOAc. The organic phase was washed with saturated aqueous NaCl, dried over anhydrous Na₂SO₄, and concentrated under reduced pressure. The residue was purified by column chromatography to afford the title compound (white solid, 0.45 g, yield 29%).

### Step C: N-(1-(4-(((7-Azaspiro[3.5]nonan-2-yl)oxy)methyl)phenyl)-3-carbamoyl-1H-pyrazol-4-yl)-6-(trifluoromethyl)picolinamide

To a solution of tert-butyl 2-((4-(3-carbamoyl-4-(6-(trifluoromethyl)pyridinylamino)-1H-pyrazol-1-yl)benzyl)oxy)-7-azaspiro[3.5]nonane-7-carboxylate (25 mg, 0.04 mmol, 1.0 eq) in DCM (15 mL) was added TFA (1 mL) at rt. The reaction solution was stirred at rt for 30 min. Upon completion, the mixture was concentrated under reduced pressure. The residue was used directly in the next step without purification.

### Step D: N-(3-Carbamoyl-1-(4-(((7-((1-(4-((2,6-dioxopiperidin-3-yl)amino)-2-fluorophenyl)piperidin-4-yl)methyl)-7-azaspiro[3.5]nonan-2-yl)oxy)methyl)phenyl)-1H-pyrazol-4-yl)-6-(trifluoromethyl)picolinamide

To a solution of N-(1-(4-(((7-azaspiro[3.5]nonan-2-yl)oxy)methyl)phenyl)-3-carbamoyl-1H-pyrazol-4-yl)-6-(trifluoromethyl)picolinamide (20 mg, crude) in DMF (5 mL) at rt were added 1-(4-((2,6-dioxopiperidin-3-yl)amino)-2-fluorophenyl)piperidine-4-carbaldehyde (15 mg, 0.04 mol, 1.0 eq) and 5 drops of AcOH. The reaction was stirred at rt for 2 h, and then sodium triacetoxyborohydride (48 mg, 0.23 mmol, 6.0 eq) was added. The reaction solution was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 25.4 mg, yield 79%). LCMS (ESI): m/z 846 [M+H]⁺. **¹H NMR** (400 MHz, DMSO-*d*₆) *δ* 11.80 (s, 1H), 10.77 (s, 1H), 9.02 (s, 1H), 8.48-8.36 (m, 2H), 8.22 (d, *J* = 7.7 Hz, 1H), 8.03 (s, 1H), 7.97 (d, *J =* 8.5 Hz, 2H), 7.78 (s, 1H), 7.52-7.46 (m, 2H), 6.86-6.74 (m, 1H), 6.48 (d, *J =* 15.0 Hz, 1H), 6.40 (d, *J =* 8.6 Hz, 1H), 5.77 (d, *J* = 7.6 Hz, 1H), 4.41 (s, 2H), 4.28-4.20 (m, 1H), 4.11-4.02 (m, 1H), 3.13-3.06 (m, 2H), 2.78-2.67 (m, 1H), 2.60-2.52 (m, 3H), 2.34-2.17 (m, 4H), 2.16-2.05 (m, 5H), 1.89-1.80 (m, 1H), 1.77-1.62 (m, 4H), 1.60-1.48 (m, 5H), 1.26-1.14 (m, 2H).

### Preparation Example 332. Synthesis of Compound A318

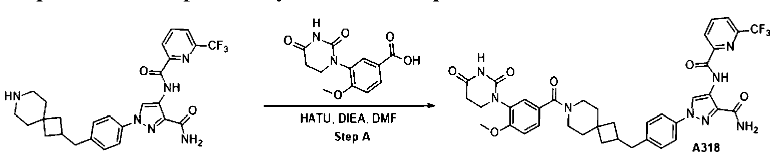

### Step A: N-(3-Carbamoyl-1-(4-((7-(3-(2,4-dioxotetrahydropyrimidin-1(2H)-yl)-4-methoxybenzoyl)-7-azaspiro[3.5]nonan-2-yl)methyl)phenyl)-1H-pyrazol-4-yl)-6-(trifluoromethyl)picolinamide

To a solution of N-(1-(4-((7-azaspiro[3.5]nonan-2-yl)methyl)phenyl)-3-carbamoyl-1H-pyrazol-4-yl)-6-(trifluoromethyl)picolinamide (70 mg, 0.137 mmol, 1.0 eq) and 3-(2,4-dioxotetrahydropyrimidin-1(2H)-yl)-4-methoxybenzoic acid (46 mg, 0.178 mmol, 1.3 eq) in DMF (5 mL) at rt were added HATU (67 mg, 0.178 mmol, 1.3 eq) and DIEA (53 mg, 0.410 mmol, 3.0 eq). The reaction solution was stirred at rt under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 30.8 mg, yield 29%). LCMS (ESI): m/z 759 [M+H]⁺. **¹H NMR** (400 MHz, DMSO-*d*₆) *δ* 11.79 (s, 1H), 10.33 (s, 1H), 8.97 (s, 1H), 8.51-8.32 (m, 2H), 8.23 (s, 1H), 7.99 (s, 1H), 7.84 (t, *J* = 24.2 Hz, 3H), 7.33 (dt, *J* = 8.4, 2.6 Hz, 4H), 7.15 (d, *J* = 8.6 Hz, 1H), 3.84 (s, 3H), 3.59 (t, *J =* 6.6 Hz, 4H), 2.76 (d, *J =* 7.4 Hz, 2H), 2.68 (t, *J =* 6.5 Hz, 2H), 2.55 (d, *J* = 8.1 Hz, 1H), 1.93 (t, *J =* 9.7 Hz, 2H), 1.62 -1.43 (m, 6H), 1.25 (d, *J =* 6.5 Hz, 2H).

### Preparation Example 333. Synthesis of Compound A319

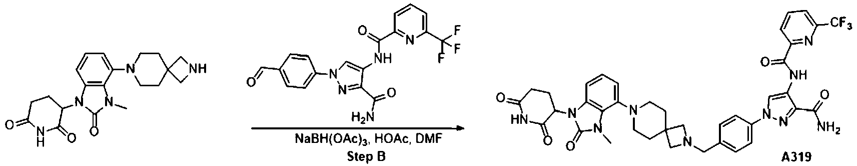

### Step A: N-(3-carbamoyl-1-(4-((7-(1-(2,6-dioxopiperidin-3-yl)-3-methyl-2-oxo-2,3-dihydro-1H-benzo [d]imidazol-4-yl)-2,7-diazaspiro[3.5]nonan-2-yl)methyl)phenyl)-1H-pyrazol-4-yl)-6-(trifluoromethyl)picolinamide

To a solution of 3-(3-methyl-2-oxo-4-(2,7-diazaspiro[3.5]nonan-7-yl)-2,3-dihydro-1H-benzimidazol-1-yl)piperidine-2,6-dione (80 mg, crude) and N-(3-carbamoyl-1-(4-formylphenyl)-1H-pyrazol-4-yl)-6-(trifluoromethyl)picolinamide (84 mg, 0.21 mmol, 1.0 eq) in DMF (2 mL) at rt under N₂ were added AcOH (0.01 mL, 0.02 mmol, 0.1 eq) and NaBH(OAc)₃ (92 mg, 0.42 mmol, 2.0 eq). The reaction solution was stirred at rt under N₂ for 0.5 h. Upon completion, the reaction solution was quenched with saturated NaHCO₃ solution at 0°C and extracted twice with EtOAc. The organic phase was washed sequentially with water and saturated aqueous NaCl, dried over anhydrous Na₂SO₄, and concentrated under reduced pressure. The residue was purified by preparative HPLC (C₁₈, 30%-90% acetonitrile in water containing 0.1% formic acid) to afford the title compound (white solid, 25.1 mg, yield 15%). LCMS (ESI): m/z 771.4 [M+H]⁺. **¹H NMR(**400 MHz, DMSO-*d*₆) *δ* 11.80 (s, 1H), 11.08 (s, 1H), 9.00 (s, 1H), 8.49-8.37 (m, 2H), 8.22 (d, *J =* 7.5 Hz, 1H), 8.01 (s, 1H), 7.93 (d, *J =* 8.4 Hz, 2H), 7.77 (s, 1H), 7.46 (d, *J =* 8.4 Hz, 2H), 6.96 (t, *J* = 7.9 Hz, 1H), 6.87 (t, *J =* 7.3 Hz, 2H), 5.36-5.32 (m, 1H), 3.71 (s, 2H), 3.62 (s, 3H), 3.17-2.95 (m, 6H), 2.91-2.84 (m, 1H), 2.72-2.58 (m, 4H), 2.01-1.95 (m, 3H), 1.83-1.75 (m, 2H).

### Preparation Example 334. Synthesis of Compound A320

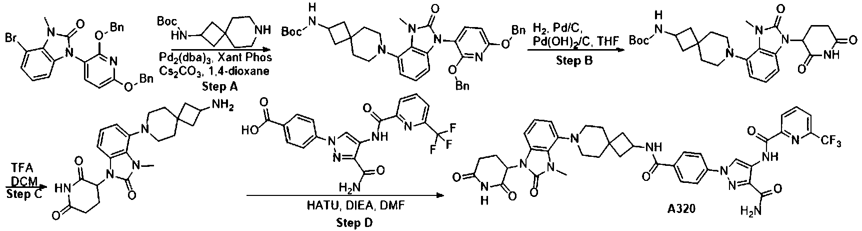

### Step A: (7-(1-(2,6-dibenzyloxy)pyridin-3-yl)-3-methyl-2-oxo-2,3-dihydro-1H-benzo[d]imidazol-4-yl)-7-azaspiro[3.5]nonan-2-yl)carbamate

To a solution of tert-butyl (7-azaspiro[3.5]-2-nonyl)carbamate (2.8 g, 12 mmol, 1.5 eq) in 1,4-dioxane (48 mL) at rt under N₂ were added 1-(2,6-bis(benzyloxy)pyridin-3-yl)-4-bromo-3-2-methyl-1,3-dihydro-2H-benzo[d]imidazol-2-one (4 g, 8 mmol, 1.0 eq), Pd₂(dba)₃ (1 g, 1.2 mmol, 0.15 eq), and Xant Phos (680 mg, 1.2 mmol, 0.15 eq). The reaction solution was stirred at rt under N₂ for 18 h. Upon completion, the reaction solution was diluted in EtOAc, washed sequentially with water and saturated aqueous NaCl, dried over anhydrous Na₂SO₄, and concentrated under reduced pressure. The residue was purified by column chromatography (eluent: petroleum ether/ethyl acetate, gradient: 0%-20% ethyl acetate) to afford the title compound (yellow solid, 2.8 g, yield 52%).

### Step B: Tert-butyl (7-(1-(2,6-dioxopiperidin-3-yl))-3-methyl-2-oxo-2,3-dihydro-1H-benzo [d]imidazol-4-yl)-7-azaspiro[3.5]nonan-2-yl)carbamate

To a solution of tert-butyl (7-(1-(2,6-dibenzyloxy)pyridin-3-yl)-3-methyl-2-oxo-2,3-dihydro-1H-benzo[d]imidazol-4-yl)-7-azaspiro[3.5]nonan-2-yl)carbamate (1 g, 1.48 mmol, 1.0 eq) in THF (20 mL) was added portionwise 10% (mass fraction) palladium hydroxide on carbon (0.5 g) and 10% (mass fraction) palladium on carbon (0.5 g) at rt. The reaction solution was purged with N₂ (×3), and the reaction was stirred at rt under hydrogen at 1 atmosphere for 18 h. Upon completion, the reaction solution was filtered through Celite, and the filtrate was dried over anhydrous Na₂SO₄ and concentrated under reduced pressure. The residue was purified by column chromatography (eluent: petroleum ether/ethyl acetate, gradient: 0%-20% ethyl acetate) to afford the title compound (yellow solid, 650 mg, yield 88%).

### Step C: 3-(4-(2-amino-7-azaspiro[3.5]nonan-7-yl)-3-methyl-2-oxo-2,3-dihydro-1H-benzo[d]imidazol-1-yl)piperidine-2,6-dione

To a solution of tert-butyl (7-(1-(2,6-dioxopiperidin-3-yl))-3-methyl-2-oxo-2,3-dihydro-1H-benzo[d]imidazol-4-yl)-7-azaspiro[3.5]nonan-2-yl)carbamate (120 mg, 0.2 mmol, 1.0 eq) in DCM (3 mL) was added TFA (3 mL) at rt. The reaction solution was stirred at rt under N₂ for l h. Upon completion, the reaction solution was concentrated under reduced pressure to afford the title compound (white solid, 70 mg, crude), which was used directly in the next reaction without purification.

### Step D: N-(3-carbamoyl-1-(4-((7-(1-(2,6-dioxopiperidin-3-yl))-3-methyl-2-oxo-2,3-dihydro-1H-benzo[d]imidazol-4-yl)-7-azaspiro[3.5]nonan-2-yl)carbamoyl)phenyl)-1H-pyrazol-4-yl)-6-(trifluoromethyl)picolinamide

At rt, to 4-(3-carbamoyl-4-(6(trifluoromethyl)pyridinylcarbamoyl)-1H-pyrazol-1-yl)benzoic acid (95 mg, 0.22 mmol, 1.2 eq) in DMF (3 mL) were added HATU (90 mg, 0.06 mmol, 0.22 mmol, 1.2 eq), 3-(4-(2-amino-7-azaspiro[3.5]nonan-7-yl)-3-methyl-2-oxo-2,3-dihydro-1H-benzo[d]imidazol-1-yl)piperidine-2,6-dione (75 mg, crude), and DIEA (85 mg, 0.6 mmol, 3.0 eq). The reaction solution was stirred at rt under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by preparative HPLC (column: YMC-Actus Triart C₁₈, 10%-50% acetonitrile in water containing 0.1% aqueous ammonia) to afford the title compound (white solid, 25.5 mg, yield 16.8%). LCMS (ESI): m/z 799 [M+H]⁺. **¹H NMR** (400 MHz, DMSO-*d*₆) *δ* 11.81 (s, 1H), 11.09 (s, 1H), 9.14 (s, 1H), 8.74 (d, *J =* 7.4 Hz, 1H), 8.49-8.38 (m, 2H), 8.22 (d, *J =* 7.6 Hz, 1H), 8.14-8.12 (m, 3H), 8.06-8.04 (m, 2H), 7.83 (s, 1H), 7.00-6.85 (m, 3H), 5.37-5.33 (m, 1H), 4.52-4.46 (m, 1H), 3.64 (s, 3H), 3.08-2.98 (m, 1H), 2.90-2.85 (m, 2H), 2.82-2.74 (m, 1H), 2.72-2.64 (m, 2H), 2.64-2.56 (m, 1H), 2.46-2.36 (m, 1H), 2.26-2.11 (m, 1H), 2.05-1.96 (m, 2H), 1.94-1.83 (m, 2H), 1.83-1.75 (m, 2H), 1.75-1.69 (m, 1H).

### Preparation Example 335. Synthesis of Compound A321

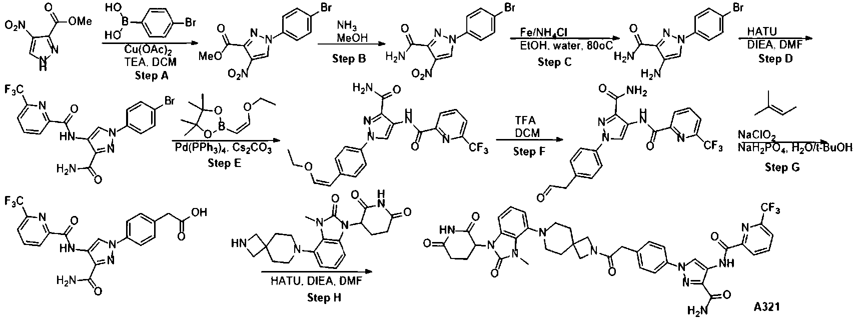

### Step A: Methyl 1-(4-bromophenyl)-4-nitro-1H-pyrazole-3-carboxylate

Under O₂, to a solution of methyl 4-nitro-1H-pyrazole-3-carboxylate (25 g, 150 mmol, 1.0 eq) and (4-bromophenyl)boronic acid (29.3 g, 0.15 mol, 1.0 eq) in DCM (300 mL) were sequentially added copper acetate (32.1 g, 0.16 mol, 1.1 eq) and TEA (40.4 g, 0.38 mol, 2.5 eq). The reaction solution was purged with O₂ (×3) and stirred at 40°C under O₂ for 16 h. Upon completion, the reaction solution was diluted in water and extracted with DCM. The organic phase was washed with saturated aqueous NaCl, dried over anhydrous Na₂SO₄, and concentrated under reduced pressure. The residue was purified by column chromatography (eluent: MeOH/DCM, gradient: 0%-10% MeOH) to afford the title compound (yellow solid, 13.0 g, yield 27%).

### Step B: 1-(4-Bromophenyl)-4-nitro-1H-pyrazole-3-carboxamide

At rt, to methyl 1-(4-bromophenyl)-4-nitro-1H-pyrazole-3-carboxylate (12.2 g, 37.2 mmol, 1.0 eq) was added ammonia in MeOH (260 mL, 5 M). The reaction solution was stirred in a sealed tube at rt overnight. Upon completion, the reaction solution was concentrated under reduced pressure to afford the title compound (white solid, 11.0 g, yield 95%).

### Step C: 4-Amino-1-(4-bromophenyl)-1H-pyrazole-3-carboxamide

At rt, to a solution of 1-(4-bromophenyl)-4-nitro-1H-pyrazole-3-carboxamide (8.4 g, 25.8 mmol, 1.0 eq) in ethanol (160 mL) and water (80 mL) were added iron powder (8.4 g, 150 mmol, 5.8 eq) and NH₄Cl (14.1 g, 260 mmol, 10.1 eq). The reaction solution was stirred at 80°C under N₂ overnight. Upon completion, the reaction solution was filtered, and the filter cake was washed with DCM twice. The filtrate was washed sequentially with water and saturated aqueous NaCl, dried over anhydrous Na₂SO₄, and concentrated under reduced pressure. The residue was purified by column chromatography (eluent: DCM/ MeOH, gradient: 0%-10% MeOH) to afford the title compound (white solid, 4.0 g, yield 52%).

### Step D: N-(1-(4-Bromophenyl)-3-carbamoyl-1H-pyrazol-4-yl)-6-(trifluoromethyl)picolinamide

At rt, to a solution of 6-(trifluoromethyl)pyridine-2-carboxylic acid (2.67 g, 13.5 mmol, 1.0 eq) in DMF (30 mL) were added DIEA (43.5 g, 33.8 mmol, 2.5 eq), 4-amino-1-(4-bromophenyl)-1H-pyrazole-3-carboxamide (4.0 g, 13.5 mmol, 1.0 eq), and HATU (6.1 g, 6.2 mmol, 1.2 eq). The reaction solution was stirred at rt for 3 h. Upon completion, the reaction solution was diluted in DCM, washed sequentially with water and saturated aqueous NaCl, dried over anhydrous Na₂SO₄, and concentrated under reduced pressure. The residue was purified by column chromatography (eluent: DCM/MeOH, gradient: 0%-10% MeOH) to afford the title compound (white solid, 4.5 g, yield 74%).

### Step E: (Z)-N-(3-Carbamoyl-1-(4-(2-ethoxyvinyl)phenyl)-1H-pyrazol-4-yl)-6-(trifluoromethyl)picolinamide

Under N₂, to a solution of N-(1-(4-bromophenyl)-3-carbamoyl-1H-pyrazol-4-yl)-6-(trifluoromethyl)picolinamide (1.0 g, 2.02 mmol, 1.0 eq) and (Z)-2-(2-ethoxyvinyl)-4,4,5,5-tetramethyl-1,3,2-dioxaborolane (440 mg, 2.02 mmol, 1.0 eq) in 1,4-dioxane (15 mL) and water (3 mL) were sequentially added cesium carbonate (1.44 g, 4.40 mmol, 2.2 eq) and Pd(PPh₃)₄ (250 mg, 0.22 mmol, 0.1 eq). The reaction solution was purged with N₂ (×3), and the reaction was stirred at 80°C under N₂ for 0.5 h. Upon completion, the reaction solution was diluted in water and extracted with EtOAc. The organic phase was washed with saturated aqueous NaCl, dried over anhydrous Na₂SO₄, and concentrated under reduced pressure. The residue was purified by column chromatography (eluent: DCM/MeOH, gradient: 0%-10% MeOH) to afford the title compound (yellow solid, 810 mg, yield 90%).

### Step F: N-(3-Carbamoyl-1-(4-(2-oxoethyl)phenyl)-1H-pyrazol-4-yl)-6-(trifluoromethyl)picolinamide

In an ice-water bath, to a solution of (Z)-N-(3-carbamoyl-1-(4-(2-ethoxyvinyl)phenyl)-1H-pyrazol-4-yl)-6-(trifluoromethyl)picolinamide (700 mg, 1.56 mmol, 1.0 eq) in DCM (10 mL) was added TFA (2 mL). The reaction solution was stirred at 0°C for 1 h. Upon completion, the reaction solution was added dropwise to an ice-water cooled 5% aqueous NaHCO₃. The layers were separated, and the aqueous phase was extracted with DCM. The organic phase was washed with saturated aqueous NaCl, dried over anhydrous Na₂SO₄, and concentrated under reduced pressure. The residue was preliminarily purified to afford the title compound (yellow solid, crude).

### Step G: 2-(4-(3-Carbamoyl-4-(6-(trifluoromethyl)pyridinylamino)-1H-pyrazol-1-yl)phenyl)acetic acid

At rt, to a solution of N-(3-carbamoyl-1-(4-(2-oxoethyl)phenyl)-1H-pyrazol-4-yl)-6-(trifluoromethyl)picolinamide (243 mg, crude) in tert-butanol (3 mL) and water (3 mL) were added 2-methyl-2-butene (818 mg, 11.7 mmol, 20 eq), sodium dihydrogen phosphate (210 mg, 1.479 mmol, 2.5 eq), and NaClO₂ (136 mg, 1.75 mmol, 3.0 eq). The reaction solution was stirred at rt for 16 h. Upon completion, the reaction solution was diluted in DCM, washed sequentially with water and saturated aqueous NaCl, dried over anhydrous Na₂SO₄, and concentrated under reduced pressure. The residue was purified by column chromatography (eluent: DCM/MeOH, gradient: 0%-10% MeOH) to afford the title compound (yellow solid, 233 mg, yield 93%).

### Step H: N-(3-Carbamoyl-1-(4-(2-(7-(1-(2,6-dioxopiperidin-3-yl)-3-methyl-2-oxo-2,3-dihydro-1H-benzimidazol-4-yl)-2,7-diazaspiro[3.5]nonan-2-yl)-2-oxoethyl)phenyl)-1H-pyrazol-4-yl)-6-(trifluoromethyl)picolinamide

At rt, to a solution of 2-(4-(3-carbamoyl-4-(6-(trifluoromethyl)pyridinylamino)-1H-pyrazol-1-yl)phenyl)acetic acid (110 mg, 0.25 mmol, 1.0 eq) in DMF (2 mL) were added DIEA (65 mg, 0.51 mmol, 2.0 eq), 3-(3-methyl-2-oxo-4-(2,7-diazaspiro[3.5]nonan-7-yl)-2,3-dihydro-1H-benzimidazol-1-yl)piperidine-2,6-dione (97 mg, 0.25 mmol, 1.0 eq), and HATU (135 mg, 0.36 mmol, 1.4 eq). The reaction solution was stirred at rt for 3 h. Upon completion, the reaction solution was diluted in EtOAc, washed sequentially with water and saturated aqueous NaCl, dried over anhydrous Na₂SO₄, and concentrated under reduced pressure. The residue was purified by preparative HPLC (C₁₈, 10%-50% acetonitrile in water containing 0.1% FA) to afford the title compound (yellow solid, 5.2 mg, yield 3%). LCMS (ESI): m/z 799.4 [M+H]⁺. **¹H NMR** (400 MHz, DMSO-*d*₆) *δ* 11.79 (s, 1H), 11.09 (s, 1H), 9.01 (s, 1H), 8.55-8.31 (m, 2H), 8.22-8.19 (m, 1H), 8.01-7.90 (m, 3H), 7.77-7.75 (m, 1H), 7.46-7.39 (m, 2H), 7.00-6.85 (m, 3H), 5.39-5.29 (m, 1H), 4.15-3.83 (m, 2H), 3.69-3.50 (m, 6H), 3.15-2.82 (m, 5H), 2.72-2.62 (m, 2H), 2.05-1.81 (m, 6H).

### Preparation Example 336. Synthesis of Compound A322

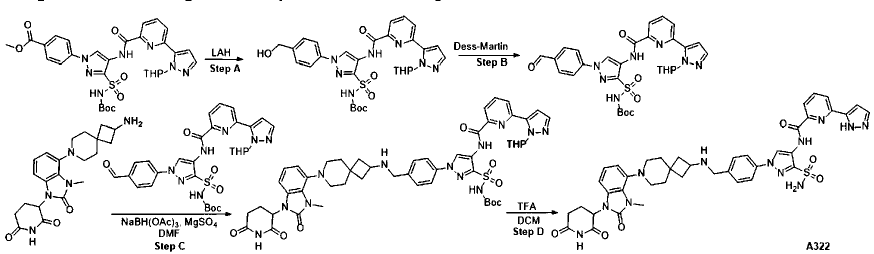

### Step A: Tert-butyl ((1-(4-(hydroxymethyl)phenyl)-4-(6-(1-(tetrahydro-2H-pyran-2-yl)-1H-pyrazol-5-yl)picolinamido)-1H-pyrazol-3-yl)sulfonyl)carbamate

To a solution of methyl 4-(3-(N-(tert-butoxycarbonyl)sulfamoyl)-4-(6-(1-(tetrahydro-2H-pyran-2-yl)-1H-pyrazol-5-yl)picolinamido)-1H-pyrazol-1-yl)benzoate (2.5 g, 3.8 mmol, 1.0 eq) in THF (100 ml) was slowly added lithium aluminum tetrahydride (150 mg, 3.8 mmol, 1.0 eq) at 0°C. The reaction solution was stirred at 0°C for 40 min. The reaction solution was quenched with Na₂SO₄ decahydrate. THF and MeOH were added to the treatment system and filtered. The filtrate was concentrated to give tert-butyl (1-(4-(hydroxymethyl)phenyl)-4-(6-(1-(tetrahydro-2H-pyran-2-yl)-1H-pyrazol-5-yl)picolinamide)-1H-pyrazol-3-yl)sulfonyl)carbamate (white solid, 2 g, yield 80%).

### Step B: Tert-butyl ((1-(4-formylphenyl)-4-(6-(1-(tetrahydro-2H-pyran-2-yl)-1H-pyrazol-5-yl)picolinamide)-1H-pyrazol-3-yl)sulfonyl)carbamate

To a solution of tert-butyl (1-(4-(hydroxymethyl)phenyl)-4-(6-(1-(tetrahydro-2H-pyran-2-yl)-1H-pyrazol-5-yl)picolinamido)-1H-pyrazol-3-yl)sulfonyl)carbamate (2.0 g, 3.2 mmol, 1.0 eq) in THF (400 ml) at rt was added Dess-Martin periodinane (1.5 g, 3.5 mmol, 1.1 eq). The reaction solution was stirred at rt for 2 h. The reaction solution was quenched with aqueous NaHCO₃, and extracted with tetrahydrofuran. The organic phase was dried over anhydrous Na₂SO₄. The organic layer was concentrated to dryness under reduced pressure and slurried with MeOH, and the filter cake was collected by filtration to give tert-butyl ((1-(4-formylphenyl)-4-(6-(1-(tetrahydro-2H-pyran-2-yl)-1H-pyrazol-5-yl)picolinamide)-1H-pyrazol-3-yl)sulfonyl)carbamate (white solid, 1.6 g, yield 80%).

### Step C: Tert-butyl ((1-(4-(((7-(1-(2,6-dioxopiperidin-3-yl)-3-methyl-2-oxo-2,3-dihydro-1H-benzo[d]imidazol-4-yl)-7-azaspiro[3.5]nonan-2-yl)amino)methyl)phenyl)-4-(6-(1-(tetrahydro-2H-pyran-2-yl)-1H-pyrazol-5-yl)picolinamide)-1H-pyrazol-3-yl)sulfonyl)carbamate

To a solution of 3-(4-(2-amino-7-azaspiro[3.5]nonan-7-yl)-3-methyl-2-oxo-2,3-dihydro-1H-benzo[d]imidazol-1-yl)piperidine-2,6-dione (150 mg, 0.38 mmol, 1.0 eq) and tert-butyl ((1-(4-formylphenyl)-4-(6-(1-(tetrahydro-2H-pyran-2-yl)-1H-pyrazol-5-yl)picolinamide)-1H-pyrazol-3-yl)sulfonyl)carbamate (236 mg, 0.38 mmol, 1.0 eq) in DMF (3 mL) at rt were added NaBH(OAc)₃ (424mg, 2.0 mmol, 5.0 eq) and magnesium sulfate (160 mg, 1.33 mmol, 6.6 eq). The reaction solution was stirred at rt under N₂ for 18 h. Upon completion, the reaction solution was diluted in EtOAc and water was added until a solid precipitated. The solid was filtered and the filter cake was dried under reduced pressure to afford the title compound (white solid, 100 mg, yield 94%). The crude was used directly in the next reaction without purification.

### Step D: N-(1-(4-(((7-(1-(2,6-dioxopiperidin-3-yl)-3-methyl-2-oxo-2,3-dihydro-1H-benzo[d]imidazol-4-yl)-7-azaspiro[3.5]nonan-2-yl)amino)methyl)phenyl)-3-sulfamoyl-1H-pyrazol-4-yl)-6-(1H-pyrazol-5-yl)picolinamide

To a solution of tert-butyl ((1-(4-(((7-(1-(2,6-dioxopiperidin-3-yl)-3-methyl-2-oxo-2,3-dihydro-1H-benzo[d]imidazol-4-yl)-7-azaspiro[3.5]nonan-2-yl)amino)methyl)phenyl)-4-(6-(1-(tetrahydro-2H-pyran-2-yl)-1H-pyrazol-5-yl)picolinamide)-1H-pyrazol-3-yl)sulfonyl)carbamate (100 mg, 0.1 mmol, 1.0 eq) in DCM (3 mL) wad added TFA (3 mL) at rt. The reaction solution was stirred at rt under N₂ for 1 h. Upon completion, the reaction solution was concentrated under reduced pressure, and the residue was purified by preparative HPLC (YMC-Actus Triart C₁₈, 10%-50% acetonitrile in water containing 0.1% TFA ) to afford the title compound (white solid, 25.8 mg, yield 32%). LCMS (ESI): m/z 819 [M+H]⁺. **¹H NMR** (400 MHz, DMSO-*d*₆) *δ* 13.24 (s, 1H), 11.32 (s, 1H), 11.08 (s, 1H), 9.09 (s, 1H), 8.28-8.19 (m, 2H), 8.17-8.04 (m, 3H), 7.93 (s, 1H), 7.90-7.87 (m, 2H), 7.56 (d, *J =* 8.4 Hz, 2H), 7.22 (d, *J =* 2.2 Hz, 1H), 6.98-6.94 (m, 1H), 6.90-6.84 (m, 2H), 5.37-5.31 (m, 1H), 3.76 (s, 3H), 3.36-3.33 (m, 3H), 2.92-2.88 (m, 3H), 2.70-2.59 (m, 5H), 2.27-2.24 (m, 1H), 2.01-1.97 (m, 2H), 1.71-1.35 (m, 6H).

### Preparation Example 337. Synthesis of Compound A324

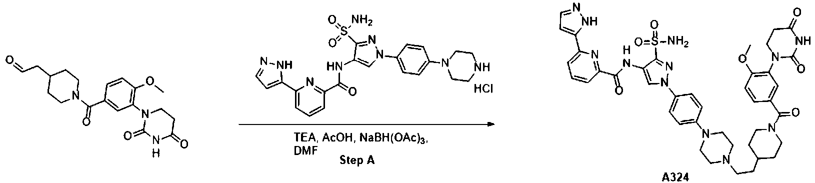

### Step A: N-(1-(4-(2-(1-(3-(2,4-dioxotetrahydropyrimidin-1(2H)-yl)-4-methoxybenzoyl)piperidin-4-yl)ethyl)piperazin-1-yl)phenyl)-3-sulfonylamino-1H-pyrazol-4-yl)-6-(1H-pyrazol-5-yl)picolinamide

To a solution of N-(1-(4-(piperazin-1-yl)phenyl)-3-sulfamoyl-1H-pyrazol-4-yl)-6-(1H-pyrazol-5-yl)picolinamide hydrochloride (100 mg, 0.2 mmol, 1.0 eq) and 2-(1-(3-(2,4-dioxotetrahydropyrimidin-1(2H)-yl)-4-methoxybenzoyl)piperidin-4-yl)acetaldehyde (76 mg, 0.2 mmol, 1.0 eq) in DMF (5 mL) was added TEA (0.2 mL) at rt. The reaction solution was stirred at rt for 15 min, followed by the addition of AcOH (0.3 mL). The reaction solution was stirred for an additional 15 h. Sodium triacetoxyborohydride (68 mg, 0.3 mmol, 1.6 eq) was added, and the reaction solution was stirred at rt for 1 additional hour. Upon completion, the reaction solution was diluted in water and extracted with DCM. The organic phase was concentrated, and then the residue was purified by HPLC to afford the title compound (white solid, 28.2 mg, yield 16%). **¹H** NMR (400 MHz, DMSO-*d*₆) *δ* 11.32 (s, 1H), 10.35 (s, 1H), 9.72 (s, 1H), 9.00 (s, 1H), 8.25 (d, *J* = 7.8 Hz, 1H), 8.14 (t, *J =* 7.7 Hz, 1H), 8.07 (d, *J* = 7.4 Hz, 1H), 7.99 (s, 2H), 7.93 (d, *J =* 1.9 Hz, 1H), 7.81 (d, *J* = 8.9 Hz, 2H), 7.39 (d, *J =* 8.4 Hz, 1H), 7.34 (d, *J =* 1.7 Hz, 1H), 7.25-7.15 (m, 4H), 3.97 (d, *J =* 12.2 Hz, 2H), 3.86 (s, 4H), 3.62 (dd, *J =* 13.7, 7.5 Hz, 5H), 3.29-2.87 (m, 8H), 2.70 (t, *J =* 6.5 Hz, 2H), 1.68 (d, *J* = 8.4 Hz, 5H), 1.18 (d, *J =* 10.5 Hz, 2H). LCMS (ESI): m/z 851 [M+H]⁺.

### Preparation Example 338. Synthesis of Compound A325

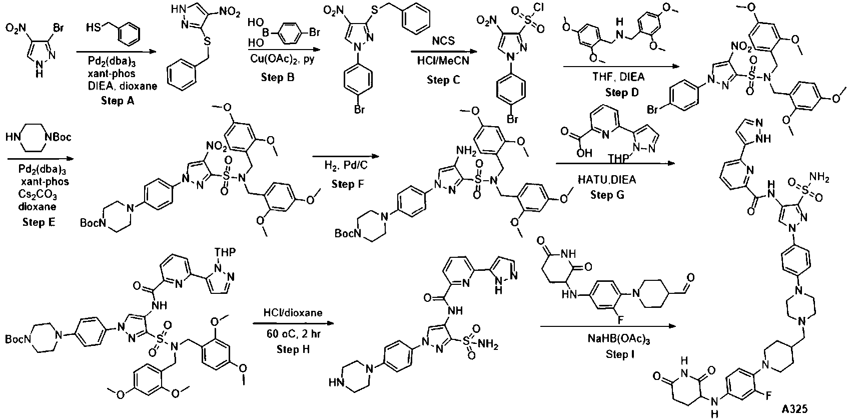

### Step A: 3-(Benzylthio)-4-nitro-1H-pyrazole

At rt, to a solution of 3-bromo-4-nitro-1H-pyrazole (20 g, 104 mmol, 1.0 eq) in dioxane (1 L) were added (phenylmethyl)mercaptan (14 g, 114 mmol, 1.1 eq), Pd2(dba)3 (9.5 g, 10.4 mmol, 0.1 eq), Xant Phos (12 g, 20 mmol, 0.2 eq) and DIEA (40.4 g, 312 mmol, 3.0 eq). The mixture was stirred at 100°C under N₂ for 16 h. The reaction mixture was poured into ice water and extracted with EtOAc, and then the organic layer was washed sequentially with saturated aqueous NaCl, dried over anhydrous Na₂SO₄ and concentrated. The residue was purified by silica gel column chromatography, eluting with EtOAc in DCM (gradient: 0%-20%) to afford 3-(benzylthio)-4-nitro-1H-pyrazole (light yellow solid, 13 g, yield 53%).

### Step B: 3-(Benzylthio)-1-(4-bromophenyl)-4-nitro-1H-pyrazole

At rt, to a solution of 3-(benzylthio)-4-nitro-1H-pyrazole (13 g, 55.2 mmol, 1.0 eq) in DMF (300 mL) were added (4-bromophenyl)boranediol (12.2 g, 60.7 mmol, 1.1 eq), copper acetate monohydrate (1.1 g, 5.5 mmol, 0.1 eq) and pyridine (13.4 ml, 165.7 mmol, 3.0 eq). The mixture was stirred at 80°C under O₂ for 16 h. The reaction mixture was filtered, and the filtrate was poured into ice water and extracted with EtOAc. The organic layer was washed with aqueous NaCl, dried over anhydrous Na₂SO₄ and concentrated. The residue was purified by silica gel column chromatography eluting with DCM in petroleum ether (gradient: 0%-70%) to afford 3-(benzylthio)-1-(4-bromophenyl)-4-nitro-1H-pyrazole (pale yellow solid, 13 g, yield 60%).

### Step C: 1-(4-Bromophenyl)-4-nitro-1H-pyrazole-3-sulfonyl chloride

To a solution of 3-(benzylthio)-1-(4-bromophenyl)-4-nitro-1H-pyrazole (13 g, 33.3 mmol, 1.0 eq) in MeCN (200 mL) were added aqueous HCl solution (100 mL, 3 M in H2O) and NCS (17.7 g, 133.2 mmol, 14.0 eq) at rt. The mixture was stirred at rt for 3 h. The reaction mixture was poured into ice water and extracted with EtOAc. The organic layer was washed with saturated aqueous NaCl, dried over anhydrous Na₂SO₄ and concentrated to afford 1-(4-bromophenyl)-4-nitro-1H-pyrazole-3-sulfonyl chloride (light yellow oil, 12 g, yield 98%).

### Step D: 1-(4-bromophenyl)-N,N-bis(2,4-dimethoxybenzyl)-4-nitro-1H-pyrazole-3-sulfonamide

To a solution of 1-(4-bromophenyl)-4-nitro-1H-pyrazole-3-sulfonyl chloride (12 g, 32.7 mmol, 1.0 eq) in THF (200 mL) were added bis(2,4-dimethoxybenzyl)amine (10.3 g, 32.7 mmol, 1.0 eq) and DIEA (4.2 g, 32.7 mmol, 1.0 eq) at rt. The mixture was stirred at 60°C for 2 h. The reaction mixture was poured into ice water and extracted with EtOAc. The organic layer was washed with aqueous NaCl, dried over anhydrous Na₂SO₄ and concentrated. The residue was purified by silica gel column chromatography eluting with DCM in petroleum ether (gradient: 0%-50%) to afford 1-(4-bromophenyl)-N,N-bis(2,4-dimethoxybenzyl)-4-nitro-1H-pyrazole-3-sulfonamide (light yellow solid, 13 g, yield 61%).

### Step E: Tert-butyl 4-(4-(3-(N,N-bis(2,4-dimethoxybenzyl)sulfamoyl)-4-nitro-1H-pyrazol-1-yl)phenyl)piperazine-1-carboxylate

At rt, to a solution of 1-(4-bromophenyl)-N,N-bis[(2,4-dimethoxyphenyl)methyl]-4-nitropyrazole-3-sulfonamide (11 g, 16.9 mmol, 1.0 eq) in dioxane (500 mL) was added tert-butyl piperazine-1-carboxylate (3.8 g, 20.3 mmol, 1.5 eq), Pd2(dba)3 (1.5 g, 1.6 mmol, 0.1 eq), Xant Phos (1.9 g, 3.3 mmol, 0.2 eq) and Cesium carbonate (16.6 g, 50.9 mmol, 3.0 eq). The mixture was stirred at 105°C under N₂ for 16 h. The reaction mixture was filtered, and the filtrate was poured into ice water and extracted with EtOAc. The organic layer was washed with saturated aqueous NaCl, dried over anhydrous Na₂SO₄ and concentrated. The residue was purified by silica gel column chromatography, eluting with EtOAc in DCM (gradient: 0%-30%) to afford tert-butyl 4-(4-(3-(N,N-bis(2,4-dimethoxybenzyl)sulfamoyl)-4-nitro-1H-pyrazol-1-yl)phenyl)piperazine-1-carboxylate (pale yellow solid, 7.5 g, yield 59%).

### Step F: Tert-butyl 4-(4-(4-amino-3-(N,N-bis(2,4-dimethoxybenzyl)sulfamoyl)-1H-pyrazol-1-yl)phenyl)piperazine-1-carboxylate

At rt, to a solution of tert-butyl 4-(4-(3-(N,N-bis(2,4-dimethoxybenzyl)sulfamoyl)-4-nitro-1H-pyrazol-1-yl)phenyl)piperazine-1-carboxylate (7.5 g, 9.9 mmol, 1.0 eq) in THF (100 mL) and MeOH (100 mL) were added Pd/C (800 mg). The mixture was stirred at 40°C under H₂ for 16 h. The reaction mixture was filtered and the filtrate was concentrated to afford tert-butyl 4-(4-(4-amino-3-(N,N-bis(2,4-dimethoxybenzyl)sulfamoyl)-1H-pyrazol-1-yl)phenyl)piperazine-1-carboxylate (pale yellow solid, 6.5 g, yield 90%).

### Step G: Tert-butyl 4-(4-(3-(N,N-bis(2,4-dimethoxybenzyl)sulfamoyl)-4-(6-(1-(tetrahydro-2H-pyran-2-yl)-1H-pyrazol-5-yl)picolinamido)-1H-pyrazol-1-yl)phenyl)piperazine-1-carboxylate

To a solution of 6-[2-(3,4,5,6-tetrahydro-2H-pyran-2-yl)pyrazol-3-yl]pyridine-2-carboxylic acid (2.9 g, 10.7 mmol, 1.0 eq) in acetonitrile (200 mL) were added HATU (5.1 g, 13.4 mmol, 1.3 eq), DIEA (3.4 g, 26.9 mmol, 3.0 eq) and tert-butyl 4-(4-(4-amino-3-(N,N-bis(2,4-dimethoxybenzyl)sulfamoyl)-1H-pyrazol-1-yl)phenyl)piperazine-1-carboxylate (6.5 g, 8.9 mmol, 0.9 eq). The mixture was stirred at rt for 16 h. The reaction mixture was poured into ice water and extracted with EtOAc, and the organic layer was washed with saturated aqueous NaCl, dried over anhydrous Na₂SO₄ and concentrated. The residue was purified by silica gel column chromatography, eluting with EtOAc in petroleum ether (gradient: 0%-60%) to afford tert-butyl 4-(4-(3-(N,N-bis(2,4-dimethoxybenzyl)sulfamoyl)-4-(6-(1-(tetrahydro-2H-pyran-2-yl)-1H-pyrazol-5-yl)picolinamido)-1H-pyrazol-1-yl)phenyl)piperazine-1-carboxylate (pale yellow solid, 6.5 g, yield 74%).

### Step H: N-(1-(4-(piperazin-1-yl)phenyl)-3-sulfamoyl-1H-pyrazol-4-yl)-6-(1H-pyrazol-5-yl)picolinamide

Tert-Butyl 4-(4-(3-(N,N-bis(2,4-dimethoxybenzyl)sulfamoyl)-4-(6-(1-(tetrahydro-2H-pyran-2-yl)-1H-pyrazol-5-yl)picolinamido)-1H-pyrazol-1-yl)phenyl)piperazine-1-carboxylate (6.5 g, 6.6 mmol, 1.0 eq) was added to 4 M hydrogen chloride-dioxane (50 mL). The mixture was stirred at 60°C for 4 h. The reaction mixture was concentrated in vacuo to afford N-(1-(4-(piperazin-1-yl)phenyl)-3-sulfamoyl-1H-pyrazol-4-yl)-6-(1H-pyrazol-5-yl)picolinamide (purple solid, 3.2 g, crude).

### Step I: N-(1-(4-(4-((1-(-4-((2,6-dioxopiperidin-3-yl)amino)-2-fluorophenyl)piperidin-4-yl)methyl)piperazin-1-yl)phenyl)-3-sulfamoyl-1H-pyrazol-4-yl)-6-(1H-pyrazol-5-yl)picolinamide

To a solution of N-(1-(4-(piperazin-1-yl)phenyl)-3-sulfamoyl-1H-pyrazol-4-yl)-6-(1H-pyrazol-5-yl)picolinamide (148 mg, 0.3 mmol, 1.0 eq) in DMF (5 mL) was added AcOH (0.5 mL) and 1-(4-((2,6-dioxopiperidin-3-yl)amino)-2-fluorophenyl)piperidine-4-carbaldehyde (100 mg, 0.3 mol, 1.0 eq). The mixture was stirred at rt for 16 h, and then sodium triacetoxyborohydride (316 mg, 1.5 mmol, 5.0 eq) was added. The mixture was stirred at rt for 1 h. The reaction mixture was concentrated. The residue was purified by silica gel column chromatography and eluted with MeOH in DCM (gradient: 0%-10%) to give a residue. The residue was purified by HPLC to afford N-(1-(4-(((2,6-dioxopiperidin-3-yl)amino)-2-fluorophenyl)piperidin-4-yl)methyl)piperazin-1-yl)phenyl)-3-sulfamoyl-1H-pyrazol-4-yl)-6-(1H-pyrazol-5-yl)picolinamide (white solid, 28 mg, yield 11%). **¹H NMR** (400 MHz, DMSO-*d*₆) *δ* 13.21 (s, 1H), 11.33 (s, 1H), 10.97-10.44 (m, 1H), 8.95 (s, 1H), 8.24 (d, J = 7.5 Hz, 1H), 8.12 (d, J = 7.4 Hz, 1H), 8.07 (d, J = 7.3 Hz, 1H), 7.98-7.62 (m, 5H), 7.23 (s, 1H), 7.10 (d, J = 9.0 Hz, 2H), 6.84 (t, J = 9.2 Hz, 1H), 6.50 (d, J = 14.8 Hz, 1H), 6.42 (d, J = 8.5 Hz, 1H), 5.79 (d, J = 7.9 Hz, 1H), 4.26 (s, 1H), 3.24 (s, 4H), 3.17-3.08 (m, 2H), 2.74 (t, J = 12.4 Hz, 1H), 2.62-2.54 (m, 5H), 2.25 (d, J = 6.7 Hz, 2H), 2.08 (s, 2H), 1.91-1.76 (m, 3H), 1.65 (s, 1H), 1.35-1.19 (m, 3H). LCMS (ESI): m/z 811 [M+H]⁺.

### Preparation Example 339. Synthesis of Compound A326

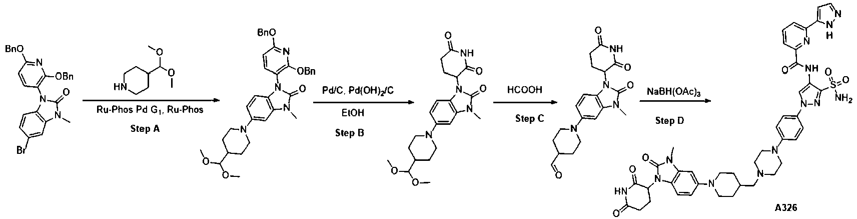

### Step A: 1-[2,6-bis(benzyloxy)pyridin-3-yl]-3-methyl-5-[4-(2,4-dioxolan-3-yl)piperidin-1-yl]-2,3-dihydro-1H-benzo[d]imidazol-2-one

To a solution of 1-[2,6-bis(benzyloxy)pyridin-3-yl]-5-bromo-3-methyl-2,3-dihydro-1H-benzo[d]imidazol-2-one (1.0 g, 1.94 mmol, 1.0 eq) in 1,4-dioxane (20 ml) at rt were added 4-(dimethoxymethyl)-piperidine (463 mg, 2.9 mmol, 1.5 eq), Ruphos (180 mg, 0.39 mmol, 0.2 eq), Ruphos Pd G₁ (154 mg, 0.19 mmol, 0.1 eq), and cesium carbonate (1.9 g, 5.82 mmol, 3.0 eq). The reaction solution was stirred at 100°C under N₂ for 16 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 600 mg, yield 52%).

### Step B: 3-{3-methyl-2-oxo-5-[4-(2,4-dioxolan-3-yl)piperidin-1-yl]benzo[d]imidazol-1-yl}piperidine-2,6-dione

At rt, to a solution of 1-[2,6-bis(benzyloxy)pyridin-3-yl]-3-methyl-5-[4-(2,4-dioxolan-3-yl)piperidin-1-yl]-2,3-dihydro-1H-benzo[d]imidazol-2-one (600 mg, 1.01 mmol, 1.0 eq) in ethanol (20 mL) was added 10% wet palladium on carbon (300 mg) and 10% wet palladium hydroxide on carbon (100 mg). The reaction solution was stirred at rt under H₂ for 16 h. Upon completion, the reaction solution was filtered through Celite and concentrated under reduced pressure. The residue was purified by column chromatography to afford the title compound (white solid, 400 mg, yield 95%).

### Step C: 1-[1-(2,6-dioxopiperidin-3-yl)-3-methyl-2-oxobenzo[d]imidazol-5-yl]piperidine-4-carbaldehyde

3-{3-methyl-2-oxo-5-[4-(2,4-dioxolan-3-yl)piperidin-1-yl]benzo[d]imidazol-1-yl}piperidine-2,6-dione (200 mg, 0.48 mmol, 1.0 eq) was dissolved in formic acid (3 mL), and the reaction was stirred at 60°C for 1 h. Upon completion, the reaction solution was concentrated under reduced pressure to afford the title compound (green oil, 170 mg, crude), which was used directly without purification.

### Step D: N-[3-(aminodioxyylidene-λ6-thio)-1-{4-[4-({1-[1-(2,6-dioxopiperidin-3-yl)-3-methyl-2-oxobenzo[d]imidazol-5-yl]piperidin-4-yl}methyl)piperazin-1-yl]phenyl}pyrazol-4-yl]-6-(2H-pyrazol-3-yl)pyridine-2-carboxamide

To a solution of 1-[1-(2,6-dioxopiperidin-3-yl)-3-methyl-2-oxobenzo[d]imidazol-5-yl]piperidine-4-carboxaldehyde (170 mg, crude) in DMF (10 mL) at rt were added N-[3-(aminodioxyylidene-λ6-thio)-1-[4-(piperazin-1-yl)phenyl]pyrazol-4-yl]-6-(2H-pyrazol-3-yl)pyridine-2-carboxamide (201 mg, 0.44 mmol, 1.0 eq) and 5 drops of AcOH. The reaction was stirred at rt for 2 h, and then sodium triacetoxyborohydride (484 mg, 2.31 mmol, 5.0 eq) was added. The reaction solution was stirred at rt for 30 min. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (off-white solid, 25.0 mg, yield 6%). LCMS (ESI): m/z 848 [M+H]⁺. **¹H NMR** (400 MHz, DMSO-*d*₆) *δ* 13.31-13.07 (m, 1H), 11.32 (s, 1H), 11.06 (s, 1H), 8.95 (s, 1H), 8.24 (d, *J* = 7.8 Hz, 1H), 8.12 (t, *J* = 7.6 Hz, 1H), 8.07 (d, *J* = 7.5 Hz, 1H), 7.95 (s, 3H), 7.73 (d, *J =* 9.0 Hz, 2H), 7.22 (s, 1H), 7.10 (d, *J* = 9.1 Hz, 2H), 6.93 (d, *J* = 8.6 Hz, 1H), 6.83 (d, *J =* 1.8 Hz, 1H), 6.64 (d, *J =* 8.6 Hz, 1H), 5.32-5.27 (m, 1H), 3.60 (d, *J* = 11.8 Hz, 2H), 3.31 (s, 3H), 3.26-3.21 (m, 4H), 2.93-2.85 (m, 1H), 2.75-2.58 (m, 5H), 2.56-2.52 (m, 3H), 2.27-2.22 (m, 2H), 2.03-1.93 (m, 2H), 1.87-1.81 (m, 2H), 1.74-1.65 (m, 1H), 1.35-1.30 (m, 1H).

### Preparation Example 340. Synthesis of Compound A327

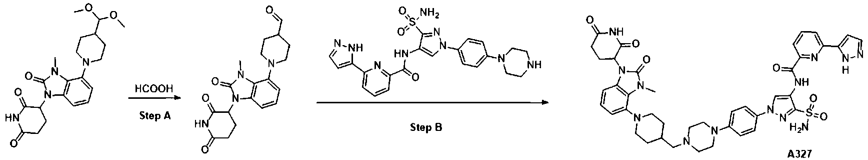

### Step A: 1-(1-(2,6-dioxopiperidin-3-yl)-3-methyl-2-oxo-2,3-dihydro-1H-benzo[d]imidazol-4-yl)piperidine-4-carbaldehyde

To 3-(4-(4-(dimethoxymethyl)piperidin-1-yl)-3-methyl-2-oxo-2,3-dihydro-1H-benzo[d]imidazol-1-yl)piperidine-2,6-dione (550 mg, 1.2 mmol, 1.0 eq) was added 8 mL of formic acid at rt. The reaction solution was stirred at 60°C under N₂ for 2 h. Upon completion, the reaction solution was concentrated under reduced pressure to directly afford the title compound (yellow oily liquid, 400 mg, crude). **Step B: N-(1-(4-(4-((1-(1-(2,6-dioxopiperidin-3-yl)-3-methyl-2-oxo-2,3-dihydro-1H-benzo[d]imidazol-4-yl)piperidin-4-yl)methyl)piperazin-1-yl)phenyl)-3-sulfamoyl-1H-pyrazol-4-yl)-6-(1H-pyrazol-5-yl)picolinamide**

To a solution of 1-(1-(2,6-dioxopiperidin-3-yl)-3-methyl-2-oxo-2,3-dihydro-1H-benzo[d]imidazol-4-yl)piperidine-4-carbaldehyde (120 mg, 0.32 mmol, 1.0 eq) in DMF (5 mL) was added N-(1-(4-(piperazin-1-yl)phenyl)-3-sulfamoyl-1H-pyrazol-4-yl)-6-(1H-pyrazol-5-yl)picolinamide (176 mg, 0.36 mmol, 1.2 eq). The reaction solution was stirred at rt under N₂ for 30 min. Sodium triacetoxyborohydride (342 mg, 1.62 mmol, 5.0 eq) was then added, and the reaction solution was stirred at rt for another 3 h. Finally, the reaction solution was poured into NaHCO₃ solution (20 mL) and extracted with DCM (×3). The organic phase was dried over anhydrous Na₂SO₄ and concentrated under reduced pressure. The reaction solution was stirred at -60°C under N₂ for 30 min, then slowly warmed to rt and stirred for 1 h. Upon completion, the reaction solution was diluted in DCM, washed sequentially with water and saturated aqueous NaCl, dried over anhydrous Na₂SO₄, and concentrated under reduced pressure. The residue was purified by preparative HPLC to afford the title compound (white solid, 39 mg, yield 14%). LCMS (ESI): m/z 848.6 [M+H]⁺. **¹H NMR** (400 MHz, DMSO-*d*₆) *δ* 13.20 (s, 1H), 11.32 (s, 1H), 11.09 (s, 1H), 8.95 (s, 1H), 8.24 (d, *J* = 7.*8* Hz, 1H), 8.12 (t, *J* = 7.7 Hz, 1H), 8.07 (d, *J =* 7.4 Hz, 1H), 7.96 (d, *J =* 9.1 Hz, 3H), 7.73 (d, *J =* 9.0 Hz, 2H), 7.22 (s, 1H), 7.10 (d, *J =* 9.2 Hz, 2H), 6.98 (t, *J=* 7.9 Hz, 1H), 6.89 (dd, *J =* 16.1, 7.8 Hz, 2H), 5.35 (dd, *J =* 12.7, 5.3 Hz, 1H), 3.64 (s, 3H), 3.27-3.21 (m, 4H), 3.16-3.09 (m, 2H), 2.92-2.84 (m, 1H), 2.75-2.66 (m, 3H), 2.66-2.56 (m, 2H), 2.56-2.52 (m, 4H), 2.32-2.23 (m, 2H), 2.02-1.97 (m, 1H), 1.89-1.83 (m, 2H), 1.76-1.66 (m, 1H), 1.38-1.33 (m, 1H).

### Preparation Example 341. Synthesis of Compound A328

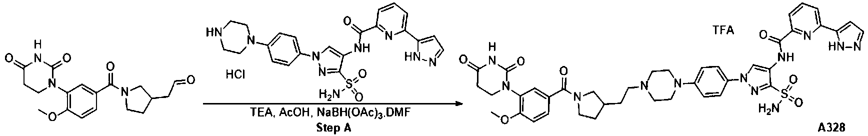

### Step A: N-(1-(4-(4-(1-(3-(2,4-dioxotetrahydropyrimidin-1(2H)-yl)-4-methoxybenzoyl)pyrrolidin-3-ethyl)piperazin-1-yl)phenyl)-3-sulfonylamino-1H-pyrazol-4-yl)-6-(1H-pyrazol-5-yl)picolinamide trifluoroacetate

To a solution of N-(1-(4-(piperazin-1-yl)phenyl)-3-sulfamoyl-1H-pyrazol-4-yl)-6-(1H-pyrazol-5-yl)picolinamide hydrochloride (165 mg, 0.3 mmol, 1.0 eq) and 2-(1-(3-(2,4-dioxotetrahydropyrimidin-1(2H)-yl)-4-methoxybenzoyl)pyrrolidin-3-yl)acetaldehyde (120 mg, 0.3 mmol, 1.0 eq) in DMF (5 mL) was added TEA (0.2 mL) at rt. The reaction solution was stirred at rt for 15 min, followed by the addition of AcOH (0.3 mL). The reaction solution was stirred for an additional 15 h. Sodium triacetoxyborohydride (106 mg, 0.5 mmol, 1.6 eq) was added, and the reaction solution was stirred at rt for l additional hour. Upon completion, the reaction solution was diluted in water and extracted with DCM. The organic phase was concentrated, and then the residue was purified by HPLC to afford the title compound (white solid, 39.3 mg, yield 14%). **¹H NMR** (400 MHz, DMSO-*d*₆) *δ* 13.21 (s, 1H), 11.31 (s, 1H), 10.35 (s, 1H), 9.58 (s, 1H), 8.99 (s, 1H), 8.24 (d, *J =* 7.6 Hz, 1H), 8.13 (t, *J=* 7.6 Hz, 1H), 8.07 (d, *J* = 7.2 Hz, 1H), 7.98 (s, 2H), 7.93 (s, 1H), 7.81 (d, *J* = 8.4 Hz, 2H), 7.55 (d, *J =* 8.4 Hz, 1H), 7.49 (d, *J =* 2.0 Hz, 1H), 7.24-7.13 (m, 4H), 3.99 (dt, *J =* 20.0, 9.8 Hz, 2H), 3.86 (s, 3H), 3.72-3.57 (m, 8H), 3.27-3.07 (m, 6H), 2.73-2.64 (m, 2H), 2.25-2.00 (m, 2H), 1.92-1.68 (m, 2H), 1.67-1.53 (m, 1H). LCMS (ESI): m/z 837 [M+H]⁺.

### Preparation Example 342. Synthesis of Compound A329

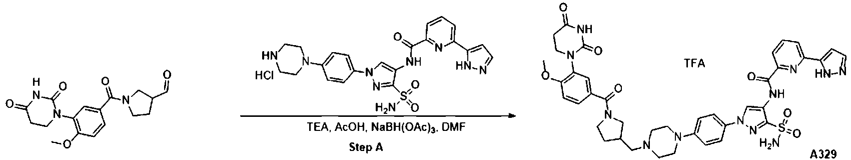

### Step A: N-(1-(4-(4-((1-(3-(2,4-dioxotetrahydropyrimidin-1(2H)-yl)-4-methoxybenzoyl)pyrrolidin-3-yl)methyl)piperazin-1-yl)phenyl)-3-sulfamoyl-1H-pyrazol-4-yl)-6-(1H-pyrazol-5-yl)picolinamide trifluoroacetate

To a solution of N-(1-(4-(piperazin-1-yl)phenyl)-3-sulfamoyl-1H-pyrazol-4-yl)-6-(1H-pyrazol-5-yl)picolinamide hydrochloride (171 mg, 0.3 mmol, 1.0 eq) and 1-(3-(2,4-dioxotetrahydropyrimidin-1(2H)-yl)-4-methoxybenzoyl)pyrrolidine-3-carbaldehyde (120 mg, 0.3 mmol, 1.0 eq) in DMF (5 mL) was added TEA (0.1 mL) at rt. The reaction solution was stirred at rt for 15 min, followed by the addition of AcOH (0.2 mL). The reaction solution was further stirred for 15 h. Sodium triacetoxyborohydride (146 mg, 0.6 mmol, 2.0 eq) was added, and the reaction solution was stirred at rt for 1 h. Upon completion, the reaction solution was diluted in water and extracted with DCM. The organic phase was concentrated, and then the residue was purified by HPLC to afford the title compound (white solid, 50.9 mg, yield 18%). **¹H NMR** (400 MHz, DMSO-*d*₆) *δ* 13.19 (s, 1H), 11.31 (s, 1H), 10.36 (s, 1H), 9.51 (s, 1H), 8.99 (s, 1H), 8.24 (d, *J =* 7.9 Hz, 1H), 8.13 (t, *J =* 7.7 Hz, 1H), 8.07 (d, *J =* 7.5 Hz, 1H), 7.98 (s, 2H), 7.93 (s, 1H), 7.80 (s, 2H), 7.56 (d, *J =* 8.7 Hz, 1H), 7.51 (d, *J=* 2.0 Hz, 1H), 7.19 (dd, *J =* 19.2, 5.4 Hz, 4H), 3.95 (s, 2H), 3.86 (s, 3H), 3.81 (s, 1H), 3.61 (d, *J* = 6.7 Hz, 7H), 3.37-3.27 (m, 3H), 3.15 (d, *J =* 12.8 Hz, 3H), 2.70 (t, *J =* 6.6 Hz, 3H), 2.10 (d, *J* = 22.0 Hz, 1H), 1.71 (s, 1H). LCMS (ESI): m/z 823 [M+H]⁺.

### Preparation Example 343. Synthesis of Compound A330-Int

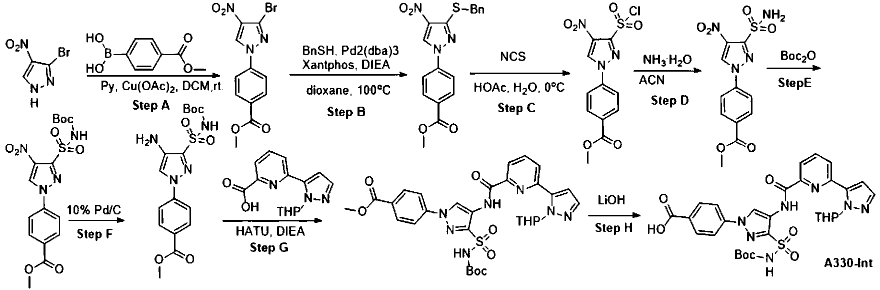

### Step A: Methyl 4-(3-bromo-4-nitro-1H-pyrazol-1-yl)benzoate

At rt, to a solution of 3-bromo-4-nitro-1H-pyrazole (150 g, 0.79 mol, 1.0 eq), 4-methoxycarbonylphenylboronic acid (185 g, 1.03 mol, 1.3 eq), and pyridine (315 g, 3.9 mol, 5.0 eq) in DCM (900 mL) was added copper acetate (213 g, 1.2 mol, 1.5 eq). The reaction solution was stirred under O₂ at rt for 18 h. Upon completion, the reaction mixture was filtered through Celite, and the filter cake was washed with DCM. The combined filtrates were concentrated under reduced pressure and dried. The crude residue was purified by column chromatography on silica gel to afford the title compound (white solid, 65 g, yield 25%).

### Step B: Methyl 4-(3-(benzylthio)-4-nitro-1H-pyrazol-1-yl)benzoate

To a solution of methyl 4-(3-bromo-4-nitro-1H-pyrazol-1-yl)benzoate (60 g, 0.181 mol, 1.0 eq), (phenylmethyl)mercaptan (21.8 g, 0.175 mol, 1.0 eq), and DIEA (27 g, 0.21 mol, 1.1 eq) in 1,4-dioxane (800 mL) were added Pd₂(dba)₃ (16.8 g, 18.5 mmol, 0.1 eq) and Xant Phos (21.3 g, 36.9 mmol, 0.2 eq) at rt. The reaction solution was purged with N₂ (×3), and the reaction was stirred at 100°C under N₂ for 18 h. Upon completion, the mixture was cooled to rt, diluted in EtOAc, washed sequentially with water and saturated aqueous NaCl, dried over anhydrous Na₂SO₄, filtered, and concentrated under reduced pressure. The residue was purified by column chromatography to afford the title compound (yellow solid, 42 g, yield 62%).

### Step C: Methyl 4-(3-(chlorosulfonyl)-4-nitro-1H-pyrazol-1-yl)benzoate

To a solution of methyl 4-(3-(benzylthio)-4-nitro-1H-pyrazol-1-yl)benzoate (15 g, 40.6 mmol, 1.0 eq) in AcOH (150 mL) and water (25 mL) was added N-chlorosuccinimide (16.2 g, 121.9 mmol, 3.0 eq) at 0°C. The reaction solution was stirred at rt under N₂ for 1 h. Upon completion, the reaction solution was extracted with EtOAc. The combined organic phases were washed with water and saturated aqueous NaCl, dried over anhydrous Na₂SO₄, filtered, and concentrated under reduced pressure. The residue was purified by column chromatography to afford the title compound (white solid, 9 g, yield 65%).

### Step D: Methyl 4-(4-nitro-3-sulfamoyl-1H-pyrazol-1-yl)benzoate

To a solution of methyl 4-(3-(chlorosulfonyl)-4-nitro-1H-pyrazol-1-yl)benzoate (9 g, 26.1 mmol, 1.0 eq) in acetonitrile (100 mL) was added 25 wt% aqueous ammonia (30 mL) at 0°C. The reaction solution was stirred at rt for 1 h. The reaction solution was stirred at rt for 1 h. Upon completion, the mixture was concentrated under reduced pressure. The residue was slurried with water and purified to afford the title compound (white solid, 6.6 g, yield 78%).

### Step E: Methyl 4-(3-((N-(tert-butoxycarbonyl)sulfamoyl)-4-nitro-1H-pyrazol-1-yl)benzoate

To a solution of methyl 4-(4-nitro-3-sulfamoyl-1H-pyrazol-1-yl)benzoate (6.6 g, 20.2 mmol, 1.0 eq) and TEA (6.1 g, 60.6 mmol, 3.0 eq) in THF (300 ml) was added di-tert-butyl dicarbonate (8.8 g, 40.4 mmol, 2.0 eq) at rt. The reaction solution was stirred at rt overnight. The reaction solution was poured into water and extracted with EtOAc. The combined organic layers were washed with aqueous NaCl, dried over anhydrous Na₂SO₄, and concentrated under reduced pressure. The residue was purified by column chromatography to afford the title compound (white solid, 5.0 g, yield 58%).

### Step F: Methyl 4-(4-amino-3-((N-(tert-butoxycarbonyl)sulfamoyl)-1H-pyrazol-1-yl)benzoate

To a solution of methyl 4-(3-((N-(tert-butoxycarbonyl)sulfamoyl)-4-nitro-1H-pyrazol-1-yl)benzoate (5.0 g, 11.7 mmol, 1.0 eq) in THF (100 mL) and MeOH (20 mL) was added Pd/C (10%, 500 mg) at rt. The reaction solution was purged with H₂ (×3), and then stirred under H₂ at rt for 1 h. The reaction solution was filtered through Celite to remove palladium on carbon, and the filtrate was concentrated to afford the title compound (white solid, 4 g, yield 86%).

### Step G: Methyl 4-(3-((N-(tert-butoxycarbonyl)sulfamoyl)-4-(6-(1-(tetrahydro-2H-pyran-2-yl)-1H-pyrazol-5-yl)picolinamido)-1H-pyrazol-1-yl)benzoate

To a solution of methyl 4-(4-amino-3-((N-(tert-butoxycarbonyl)sulfamoyl)-1H-pyrazol-1-yl)benzoate (3.5 g, 8.8 mmol, 1.0 eq), 6-(1-(tetrahydro-2H-pyran-2-yl)-1H-pyrazol-5-yl)picolinic acid (2.9 g, 10.6 mmol, 1.2 eq), and DIEA (3.5 g, 26.5 mmol, 3.0 eq) in DMF (50 mL) was added HATU (3.8 g, 11.2 mmol, 1.3 eq) at rt. The reaction solution was stirred at rt for 3 h. The reaction solution was poured into water and extracted with EtOAc. The organic phase was washed with saturated aqueous NaCl, dried over anhydrous Na₂SO₄, filtered, and concentrated under reduced pressure. The residue was purified by column chromatography to afford the title compound (white solid, 4 g, yield 70%).

### Step H: 4-(3-(N-(tert-butoxycarbonyl)sulfamoyl)-4-(6-(1-(tetrahydro-2H-pyran-2-yl)-1H-pyrazol-5-yl)picolinamido)-1H-pyrazol-1-yl)benzoic acid

To a solution of methyl 4-(3-((N-(tert-butoxycarbonyl)sulfamoyl)-4-(6-(1-(tetrahydro-2H-pyran-2-yl)-1H-pyrazol-5-yl)picolinamido)-1H-pyrazol-1-yl)benzoate (1.5 g, 2.3 mmol, 1.0 eq) in MeOH (5 mL), THF (10 mL), and water (10 ml) was added lithium hydroxide (500 mg, 23 mmol, 10 eq) at rt. The reaction solution was stirred at rt for 3 h. The reaction solution was diluted in water and back-extracted with EtOAc. The aqueous phase was adjusted to pH 3 with 3 N HCl solution. The precipitated solid was filtered, and the filter cake was dried to afford the title compound (white solid, 1.2 g, yield 77%). LCMS (ESI): m/z 638.3[M+H]⁺.

### Preparation Example 344. Synthesis of Compound A330

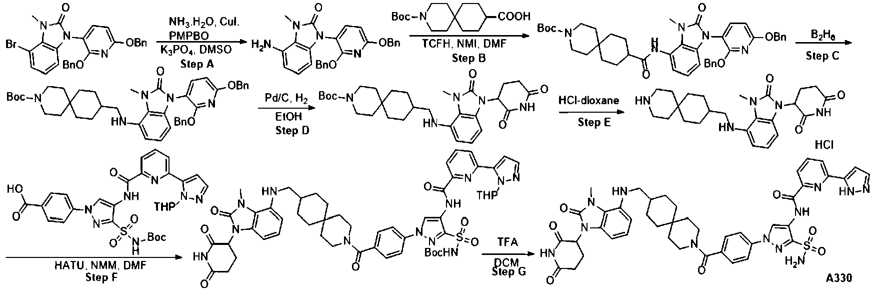

### Step A: 4-Amino-1-(2,6-bis(benzyloxy)pyridin-3-yl)-3-methyl-1,3-dihydro-2H-benzo[d]imidazol-2-one

At rt, to a solution of 1-(2,6-bis(benzyloxy)pyridin-3-yl)-4-bromo-3-methyl-1,3-dihydro-2H-benzo[d]imidazol-2-one (5.0 g, 9.7 mmol, 1.0 eq), potassium phosphate (2.3 g, 10.7 mmol, 1.1 eq), cuprous iodide (0.2 g, 0.97 mmol, 0.1 eq), and N¹-benzyl-N²-(5-methyl-2-biphenyl)oxalamide (0.3 g, 0.97 mmol, 0.1 eq) in dimethyl sulfoxide (80 mL) was added 27% aqueous ammonia (80 mL). The reaction solution was stirred at 90°C in a sealed tube under N₂ for 24 h. Upon completion, the reaction solution was diluted in water and extracted with DCM. The organic phase was washed repeatedly with saturated aqueous NaCl, dried over anhydrous Na₂SO₄, filtered, and concentrated. The residue was slurried with EtOAc-petroleum ether to afford the title compound (yellow solid, 4.0 g, yield 91%).

### Step B: Tert-butyl 9-(1-(2,6-bis(benzyloxy)pyridin-3-yl)-3-methyl-2-oxo-2,3-dihydro-1H-benzoldlimidazol-4-yl)carbamoyl)-3-azaspiro[5.5]undecane-3-carboxylate

At rt, to a solution of 4-amino-1-(2,6-bis(benzyloxy)pyridin-3-yl)-3-methyl-1,3-dihydro-2H-benzo[d]imidazol-2-one (609 mg, 1.3 mmol, 1.0 eq) and 3-(tert-butoxycarbonyl)-3-azaspiro[5.5]undecane-9-carboxylic acid (400 mg, 1.3 mmol, 1.0 eq) in DMF (10 mL) were added N-methylimidazole (331 mg, 4.0 mmol, 3.0 eq) and TCFH (566 mg, 2.0 mmol, 1.5 eq). The reaction solution was stirred at rt for 15 h. Upon completion, the reaction solution was diluted in water and extracted with DCM. The organic phase was washed with aqueous NaCl, dried over anhydrous Na₂SO₄, filtered, and concentrated. The residue was purified by column chromatography to afford the title compound (yellow solid, 600 mg, yield 61%).

### Step C: Tert-butyl 9-((1-(2,6-bis(benzyloxy)pyridin-3-yl)-3-methyl-2-oxo-2,3-dihydro-1H-benzo[d]imidazol-4-yl)amino)methyl)-3-azaspiro[5.5]undecane-3-carboxylate

Tert-butyl 9-(1-(2,6-bis(benzyloxy)pyridin-3-yl)-3-methyl-2-oxo-2,3-dihydro-1H-benzo[d]imidazol-4-yl)carbamoyl)-3-azaspiro[5.5]undecane-3-carboxylate (600 mg, 0.8 mmol, 1.0 eq) was added to borane-dimethyl sulfide in THF (2 mol/L, 10 mL) at rt. The reaction solution was stirred at rt under N₂ for 15 h. Upon completion, the reaction solution was cooled to 0°C, and MeOH (10 mL) was added dropwise slowly, followed by stirring for 1 additional hour. The reaction solution was concentrated, and then the residue was purified by column chromatography to afford the title compound (yellow solid, 400 mg, yield 68%).

### Step D: Tert-butyl 9-((1-(2,6-dioxopiperidin-3-yl)-3-methyl-2-oxo-2,3-dihydro-1H-benzo[d]imidazol-4-yl)amino)methyl)-3-azaspiro[5.5]undecane-3-carboxylic acid

At rt, to a solution of tert-butyl 9-((1-(2,6-bis(benzyloxy)pyridin-3-yl)-3-methyl-2-oxo-2,3-dihydro-1H-benzo[d]imidazol-4-yl)amino)methyl)-3-azaspiro[5.5]undecane-3-carboxylate (400 mg, 0.6 mmol, 1.0 eq) in ethanol (10 mL) was added Pd/C (10%, 80 mg). The reaction solution was purged with H₂ (×3), and then stirred under H₂ at 50°C for 15 h. Upon completion, the mixture was filtered to remove Pd/C, and the filtrate was concentrated to afford the title compound (yellow solid, 150 mg, yield 50%).

### Step E: 3-(4-(((3-Azaspiro[5.5]undecan-9-yl)methyl)amino)-3-methyl-2-oxo-2,3-dihydro-1H-benzo[d]imidazol-1-yl)piperidine-2,6-dione hydrochloride

Tert-butyl 9-((1-(2,6-dioxopiperidin-3-yl)-3-methyl-2-oxo-2,3-dihydro-1H-benzo[d]imidazol-4-yl)amino)methyl)-3-azaspiro[5.5]undecane-3-carboxylic acid (150 mg, 0.3 mmol, 1.0 eq) was added to hydrogen chloride-dioxane solution (4 mol/L) (4 mL). The mixture was stirred at rt for l h. Upon completion, the reaction solution was concentrated to afford the title compound (yellow solid, 100 mg, crude).

### Step F: Tert-butyl ((1-(4-(9-((1-(2,6-dioxopiperidin-3-yl)-3-methyl-2-oxo-2,3-dihydro-1H-benzo[d]imidazol-4-yl)amino)methyl)-3-azaspiro[5.5]undecane-3-carbonyl)phenyl)-4-(6-(1-(1-(tetrahydro-2H-pyran-2-yl)-1H-pyrazol-5-yl)picolinamido)-1H-pyrazol-3-yl)sulfonyl)carbamate

At rt, to a solution of 3-(4-((3-azaspiro[5.5]undecan-9-yl)methyl)amino)-3-methyl-2-oxo-2,3-dihydro-1H-benzo[d]imidazol-1-yl)piperidine-2,6-dione hydrochloride (100 mg, crude) and 4-(3-(N-(tert-butoxycarbonyl)sulfamoyl)-4-(6-(1-(tetrahydro-2H-pyran-2-yl)-1H-pyrazol-5-yl)picolinamido)-1H-pyrazol-1-yl)benzoic acid (145 mg, 0.2 mmol, 1.0 eq) in DMF (5 mL) were added N-methylmorpholine (0.08 mL, 0.7 mmol, 3.0 eq) and HATU (130 mg, 0.3 mmol, 1.5 eq). The reaction solution was stirred at rt for 2 h. Upon completion, the reaction solution was diluted in water and extracted with DCM. The organic phase was washed sequentially with saturated aqueous NaCl, dried over anhydrous Na₂SO₄, filtered, and concentrated. The residue was purified by column chromatography to afford the title compound (yellow solid, 100 mg, yield 42%).

### Step G: N-(1-(4-(9-((1-(2,6-Dioxopiperidin-3-yl)-3-methyl-2-oxo-2,3-dihydro-1H-benzo[d]imidazol-4-yl)amino)methyl)-3-azaspiro[5.5]undecane-3-carbonyl)phenyl)-3-sulfamamido-1H-pyrazol-4-yl)-6-(1H-pyrazol-5-yl)picolinamide

At rt, to a solution of tert-butyl ((1-(4-(9-((1-(2,6-dioxopiperidin-3-yl)-3-methyl-2-oxo-2,3-dihydro-1H-benzo[d]imidazol-4-yl)amino)methyl)-3-azaspiro[5.5]undecane-3-carbonyl)phenyl)-4-(6-(1-(1-(tetrahydro-2H-pyran-2-yl)-1H-pyrazol-5-yl)picolinamido)-1H-pyrazol-3-yl)sulfonyl)carbamate (80 mg, 0.1 mmol, 1.0 eq) in DCM (3 mL) was added TFA (3 mL). The reaction solution was stirred at rt for 2 h. After concentration, the residue was purified by HPLC to afford the title compound (yellow solid, 20 mg, yield 8%). **¹H NMR** (400 MHz, DMSO-*d*₆) *δ* 13.22 (s, 1H), 11.33 (s, 1H), 11.06 (s, 1H), 9.17 (s, 1H), 8.33-7.90 (m, 8H), 7.60 (d, *J =* 6.8 Hz, 2H), 7.23 (s, 1H), 6.86 (s, 1H), 6.46 (d, *J* = 29.0 Hz, 2H), 5.29 (s, 1H), 5.01 (s, 1H), 3.82-3.57 (m, 5H), 3.06-2.81 (m, 4H), 2.76-2.55 (m, 3H), 2.05-1.92 (m, 1H), 1.80-1.47 (m, 7H), 1.39-1.10 (m, 6H). LCMS (ESI): m/z 875 [M+H]⁺.

### Preparation Example 345. Synthesis of Compound A331

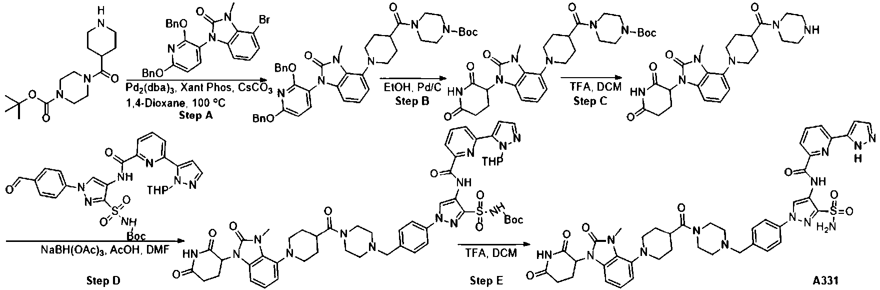

### Step A: Tert-butyl 4-(1-(1-(2,6-bis(benzyloxy)pyridin-3-yl)-3-methyl-2-oxo-2,3-dihydro-1H-benzo[d]imidazol-4-yl)piperidine-4-carbonyl)piperazine-1-carboxylate

To a solution of 1-(2,6-bis(benzyloxy)pyridin-3-yl)-4-bromo-3-methyl-1,3-dihydro-2H-benzo[d]imidazol-2-one (500 mg, 0.968 mmol, 1.0 eq), tert-butyl 4-(piperidine-4-carbonyl)piperazine-1-carboxylate (288 mg, 0.968 mmol, 1.0 eq), and cesium carbonate (946 mg, 2.905 mmol, 3.0 eq) in dioxane (10 mL) were added Pd₂(dba)₃ (89 mg, 0.097 mmol, 0.1 eq) and Xant Phos (112 mg, 0.194 mmol, 0.2 eq) at rt. The reaction solution was stirred at 100°C overnight under N₂. Upon completion, the reaction solution was cooled to rt, poured into saturated aqueous NH₄Cl, and extracted with EtOAc. The organic phase was dried, filtered, and concentrated. The residue was purified by column chromatography to afford the title compound (white solid, 424 mg, yield 60%).

### Step B: Tert-butyl 4-(1-(1-(2,6-dioxopiperidin-3-yl)-3-methyl-2-oxo-2,3-dihydro-1H-benzo[d]imidazol-4-yl)piperidine-4-carbonyl)piperazine-1-carboxylate

To a solution of tert-butyl 4-(1-(1-(2,6-bis(benzyloxy)pyridin-3-yl)-3-methyl-2-oxo-2,3-dihydro-1H-benzo[d]imidazol-4-yl)piperidine-4-carbonyl)piperazine-1-carboxylate (200 mg, 0.273 mmol, 1.0 eq) in ethanol (20 mL) was added 10% Pd/C (80 mg) at rt. The reaction solution was stirred at 50°C under H₂ overnight. Upon completion, the reaction solution was filtered, and the filtrate was concentrated to afford the title compound (yellow solid, 150 mg, yield 100%).

### Step C: 3-(3-methyl-2-oxo-4-(4-(piperazine-1-carbonyl)piperidin-1-yl)-2,3-dihydro-1H-benzo[d]imidazol-1-yl)piperidine-2,6-dione

To a solution of tert-butyl 4-(1-(1-(2,6-dioxopiperidin-3-yl)-3-methyl-2-oxo-2,3-dihydro-1H-benzo[d]imidazol-4-yl)piperidine-4-carbonyl)piperazine-1-carboxylate (150 mg, 0.270 mmol, 1.0 eq) in DCM (2 mL) was added TFA (2 mL) at rt. The reaction solution was stirred at rt under N₂ for 2 h, then basified with aqueous NaOH (1 mol/L) and extracted with DCM. The organic phase was dried, filtered, and concentrated to afford the title compound (yellow oil, 120 mg, crude).

### Step D: Tert-butyl ((1-(4-(1-(1-(2-(2,6-dioxopiperidin-3-yl)-3-methyl-2-oxo-2,3-dihydro-1H-benzo[d]imidazol-4-yl)piperidine-4-carbonyl)piperazin-1-yl)methyl)phenyl)-4-(6-(1-(tetrahydro-2H-pyran-2-yl)-1H-pyrazol-5-yl)picolinamido)-1H-pyrazol-3-yl)sulfonyl)carbamate

To a solution of 3-(3-methyl-2-oxo-4-(4-(piperazine-1-carbonyl)piperidin-1-yl)-2,3-dihydro-1H-benzo[d]imidazol-1-yl)piperidine-2,6-dione (120 mg, crude) and tert-butyl ((1-(4-formylphenyl)-4-(6-(1-(tetrahydro-2H-pyran-2-yl)-1H-pyrazol-5-yl)pyridinamino)-1H-pyrazol-3-yl)sulfonyl)carbamate (164 mg, 0.264 mmol, 1.0 eq) in DMF (5 mL) at rt was added AcOH (0.3 mL). The reaction solution was stirred at rt overnight. Sodium triacetoxyborohydride (168 mg, 0.792 mmol, 3.0 eq) was then added, and the reaction was stirred for l additional hour. Upon completion, the reaction solution was diluted in water and extracted with DCM. The organic phase was dried, filtered, and concentrated. The residue was purified by column chromatography to afford the title compound (white solid, 30 mg, yield 13%).

### Step E: N-(1-(4-((4-(1-(1-(2,6-dioxopiperidin-3-yl)-3-methyl-2-oxo-2,3-dihydro-1H-benzo[d]imidazol-4-yl)piperidine-4-carbonyl)piperazin-1-yl)methyl)phenyl)-3-sulfamoyl-1H-pyrazol-4-yl)-6-(1H-pyrazol-5-yl)picolinamide

To a solution of tert-butyl ((1-(4-(1-(1-(2-(2,6-dioxopiperidin-3-yl)-3-methyl-2-oxo-2,3-dihydro-1H-benzo[d]imidazol-4-yl)piperidine-4-carbonyl)piperazin-1-yl)methyl)phenyl)-4-(6-(1-(tetrahydro-2H-pyran-2-yl)-1H-pyrazol-5-yl)picolinamido)-1H-pyrazol-3-yl)sulfonyl)carbamate (30 mg, 0.028 mmol, 1.0 eq) in DCM (3 mL) at rt was added TFA (1 mL). The reaction solution was stirred at rt for 2 h. After concentration, the residue was purified by HPLC to afford the title compound (white solid, 4 mg, yield 16%). **¹H NMR** (400 MHz, DMSO-*d*₆) *δ* 13.21 (s, 1H), 11.34 (s, 1H), 11.09 (s, 1H), 9.11 (s, 1H), 8.25 (d, *J =* 5.1 Hz, 1H), 8.08 (dd, *J =* 17.8, 10.4 Hz, 3H), 7.94 (d, *J =* 6.8 Hz, 2H), 7.57-7.23 (m, 4H), 7.01-6.87 (m, 3H), 5.36 (dd, *J =* 11.9, 5.1 Hz, 1H), 3.77-3.55 (m, 10H), 3.13 (d, *J* = 10.2 Hz, 2H), 2.96-2.59 (m, 9H), 2.03-1.98 (m, 1H), 1.84-1.71 (m, 4H). LCMS (ESI): m/z 876 [M+H]⁺.

### Preparation Example 346. Synthesis of Compound A332

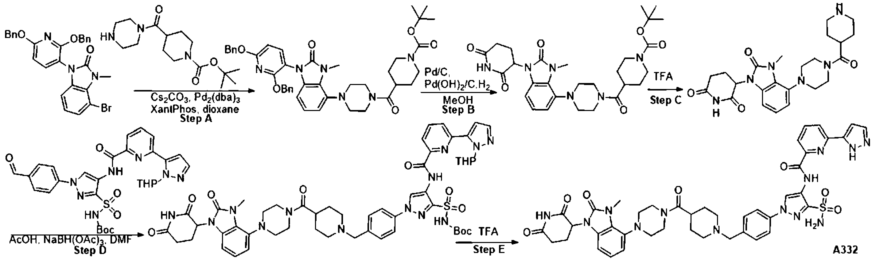

### Step A: Tert-butyl 4-(4-(1-(2,6-bis(benzyloxy)pyridin-3-yl)-3-methyl-2-oxo-2,3-dihydro-1H-benzo[d]imidazol-4-yl)piperazine-1-carbonyl)piperidine-1-carboxylate

At rt, to a solution of 1-(2,6-bis(benzyloxy)pyridin-3-yl)-4-bromo-3-methyl-1,3-dihydro-2H-benzo[d]imidazol-2-one (550 mg, 1.1 mmol, 1.0 eq), tert-butyl 4-(piperazine-1-carbonyl)piperidine-1-carboxylate (348 mg, 1.2 mmol, 1.1 eq), and cesium carbonate (694 mg, 2.1 mmol, 2.0 eq) in dioxane (15 mL) were added Pd₂(dba)₃ (97 mg, 0.1 mmol, 0.1 eq) and Xant Phos (61 mg, 0.1 mmol, 0.1 eq). The reaction solution was stirred at 110°C under N₂ for 18 h. Upon completion, the reaction solution was cooled to rt, poured into saturated aqueous NH₄Cl, and extracted with EtOAc. The organic phase was dried, filtered, and concentrated. The residue was purified by column chromatography to afford the title compound (yellow solid, 660 mg, yield 84%).

### Step B: Tert-butyl 4-(4-(1-(2,6-dioxopiperidin-3-yl)-3-methyl-2-oxo-2,3-dihydro-1H-benzo[d]imidazol-4-yl)piperazine-1-carbonyl)piperidine-1-carboxylate

At rt, to a solution of tert-butyl 4-(4-(1-(2,6-bis(benzyloxy)pyridin-3-yl)-3-methyl-2-oxo-2,3-dihydro-1H-benzo[d]imidazol-4-yl)piperazine-1-carbonyl)piperidine-1-carboxylate (550 mg, 0.7 mmol, 1.0 eq) in MeOH (10 mL) were added 10% Pd/C (159 mg) and 10% Pd(OH)₂/C (210 mg). The reaction solution was stirred under H₂ at 60°C overnight. Upon completion, the reaction solution was filtered, and the filtrate was concentrated to afford the title compound (yellow oil, 440 mg, yield 96%).

### Step C: 3-(3-Methyl-2-oxo-4-(4-(piperidine-4-carbonyl)piperazin-1-yl)-2,3-dihydro-1H-benzo[d]imidazol-1-yl)piperidine-2,6-dione

At rt, to a solution of tert-butyl 4-(4-(1-(2,6-dioxopiperidin-3-yl)-3-methyl-2-oxo-2,3-dihydro-1H-benzo[d]imidazol-4-yl)piperazine-1-carbonyl)piperidine-1-carboxylate (200 mg, 0.4 mmol, 1.0 eq) in DCM (3 mL) was added TFA (1 mL). The reaction solution was stirred at rt under N₂ for 2 h, and then concentrated under reduced pressure to afford the title compound (yellow oil, 150 mg, crude).

### Step D: Tert-butyl ((1-(4-(4-(1-(2,6-dioxopiperidin-3-yl)-3-methyl-2-oxo-2,3-dihydro-1H-benzo[d]imidazol-4-yl)piperazine-1-carbonyl)piperidin-1-yl)methyl)phenyl)-4-(6-(1-(tetrahydro-2H-pyran-2-yl)-1H-pyrazol-5-yl)picolinamido)-1H-pyrazol-3-yl)sulfonyl)carbamate

At rt, to a solution of 3-(3-methyl-2-oxo-4-(4-(piperidine-4-carbonyl)piperazin-1-yl)-2,3-dihydro-1H-benzo[d]imidazol-1-yl)piperidine-2,6-dione (150 mg, crude) and tert-butyl ((1-(4-formylphenyl)-4-(6-(1-(tetrahydro-2H-pyran-2-yl)-1H-pyrazol-5-yl)pyridinylamino)-1H-pyrazol-3-yl)sulfonyl)carbamate (226 mg, 0.4 mmol, 1.3 eq) in DMF (5 mL) was added AcOH (0.05 mL). The reaction solution was stirred at rt overnight. Then sodium triacetoxyborohydride (209 mg, 1.0 mmol, 3.0 eq) was added, and the reaction was stirred for 3 h. Upon completion, the reaction solution was purified by reverse-phase column chromatography (C₁₈) to afford the title compound (white solid, 70 mg, 20% yield over two steps).

### Step E: N-(1-(4-((4-(4-(1-(2,6-Dioxopiperidin-3-yl)-3-methyl-2-oxo-2,3-dihydro-1H-benzo[d]imidazol-4-yl)piperazine-1-carbonyl)piperidin-1-yl)methyl)phenyl)-3-sulfamoyl-1H-pyrazol-4-yl)-6-(1H-pyrazol-5-yl)picolinamide

At rt, to a solution of tert-butyl ((1-(4-(4-(1-(2,6-dioxopiperidin-3-yl)-3-methyl-2-oxo-2,3-dihydro-1H-benzo[d]imidazol-4-yl)piperazine-1-carbonyl)piperidin-1-yl)methyl)phenyl)-4-(6-(1-(tetrahydro-2H-pyran-2-yl)-1H-pyrazol-5-yl)picolinamido)-1H-pyrazol-3-yl)sulfonyl)carbamate (70 mg, 0.07 mmol, 1.0 eq) in DCM (2 mL) was added TFA (1 mL). The reaction solution was stirred at rt for 1 h. After concentration, the residue was purified by HPLC to afford the title compound (white solid, 10 mg, yield 17%). **¹H NMR** (400 MHz, DMSO-*d*₆) *δ* 13.20 (s, 1H), 11.32 (s, 1H), 11.09 (s, 1H), 9.08 (s, 1H), 8.24 (d, *J* = 7.7 Hz, 1H), 8.18-8.05 (m, 3H), 8.03 (s, 1H), 7.94 (s, 1H), 7.88 (d, *J= 8.5* Hz, 2H), 7.51 (d, *J* = 8.5 Hz, 2H), 7.22 (d, *J =* 2.0 Hz, 1H), 7.02-6.96 (m, 1H), 6.92 (dd, *J* = 7.3, 4.7 Hz, 2H), 5.36 (dd, *J =* 12.6, 5.2 Hz, 1H), 4.45 (s, 1H), 4.01 (s, 1H), 3.65 (s, 3H), 3.55 (s, 2H), 3.07 (s, 2H), 2.91 (d, *J =* 10.1 Hz, 1H), 2.87 (d, *J =* 11.3 Hz, 4H), 2.76-2.63 (m, 4H), 2.60 (s, 1H), 2.13-1.99 (m, 3H), 1.71-1.60 (m, 4H). LCMS (ESI): m/z 876 [M+H]⁺.

### Preparation Example 347. Synthesis of Compound A333

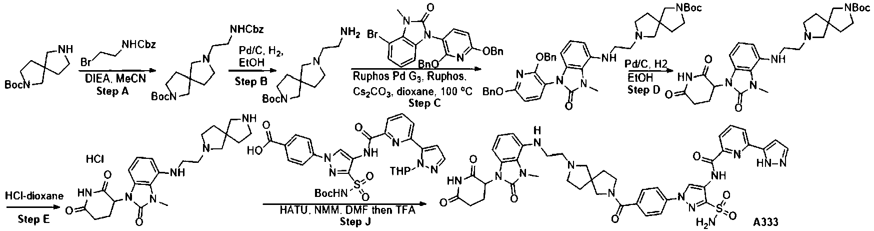

### Step A: Tert-butyl 7-(2-(((benzyloxy)carbonyl)amino)ethyl)-2,7-diazaspiro[4.4]nonane-2-carboxylate

At rt, to a solution of tert-butyl 2,7-diazaspiro[4.4]nonane-2-carboxylate (1 g, 4.4 mmol, 1.0 eq) in acetonitrile (15 mL) were added DIEA (2.3 mL, 13.3 mmol, 3.0 eq) and 2-bromoethyl benzyl carbamate (1.1 g, 4.4 mmol, 1.0 eq). The reaction solution was stirred at rt for 18 h. Upon completion, the reaction solution was diluted in water and extracted with DCM. The organic phase was washed with aqueous NaCl, dried over anhydrous Na₂SO₄, filtered, and concentrated. The residue was purified by column chromatography to afford the title compound (yellow oil, 1.0 g, yield 56%).

### Step B: Tert-butyl 7-(2-aminoethyl)-2,7-diazaspiro[4.4]nonane-2-carboxylate

At rt, to a solution of tert-butyl 7-(2-(((benzyloxy)carbonyl)amino)ethyl)-2,7-diazaspiro[4.4]nonane-2-carboxylate (800 mg, 2.0 mmol, 1.0 eq) in ethanol (10 mL) was added 10% Pd/C (100 mg). The reaction solution was purged with H₂ (×3) and then stirred under H₂ at 25°C for 2 h. Upon completion, the mixture was filtered to remove Pd/C, and the filtrate was concentrated to afford the title compound (yellow oil, 500 mg, yield 94%).

### Step C: Tert-butyl 7-(2-(1-(2,6-bis(benzyloxy)pyridin-3-yl)-3-methyl-2-oxo-2,3-dihydro-1H-benzo[d]imidazol-4-yl)amino)ethyl)-2,7-diazaspiro[4.4]nonane-2-carboxylate

At rt, to a solution of 1-(2,6-bis(benzyloxy)pyridin-3-yl)-4-bromo-3-methyl-1,3-dihydro-2H-benzo[d]imidazol-2-one (1.4 g, 2.7 mmol, 1.0 eq) and tert-butyl 7-(2-aminoethyl)-2,7-diazaspiro[4.4]nonane-2-carboxylate (740 mg, 2.7 mmol, 1.0 eq) in dioxane (15 mL) were added cesium carbonate (2.7 g, 8.2 mmol, 3.0 eq), RuPhos Pd G₃ (230 mg, 0.27 mmol, 0.1 eq), and RuPhos (128 mg, 0.27 mmol, 0.1 eq). The reaction solution was stirred at 100°C under N₂ for 15 h. Upon completion, the reaction solution was diluted in water and extracted with DCM. The organic phase was washed sequentially with saturated aqueous NaCl, dried over anhydrous Na₂SO₄, filtered, and concentrated. The residue was purified by column chromatography to afford the title compound (yellow solid, 900 mg, yield 46%).

### Step D: Tert-butyl 7-(2-(1-(2,6-dioxopiperidin-3-yl)-3-methyl-2-oxo-2,3-dihydro-1H-benzo[d]imidazol-4-yl)amino)ethyl)-2,7-diazaspiro[4.4]nonane-2-carboxylate

At rt, to a solution of tert-butyl 7-(2-(1-(2,6-bis(benzyloxy)pyridin-3-yl)-3-methyl-2-oxo-2,3-dihydro-1H-benzo[d]imidazol-4-yl)amino)ethyl)-2,7-diazaspiro[4.4]nonane-2-carboxylate (520 mg, 0.7 mmol, 1.0 eq) in ethanol (10 mL) were added 10% Pd/C (100 mg) and 10% Pd(OH)₂/C (100 mg). The reaction solution was purged with H₂ (×3), and then stirred under H₂ at 50°C for 18 h. Upon completion, the mixture was filtered, and the filtrate was concentrated to afford the title compound (yellow solid, 200 mg, yield 51%).

### Step E: 3-(4-(2-(2,7-Diazaspiro[4.4]nonan-2-yl)ethyl)amino)-3-methyl-2-oxo-2,3-dihydro-1H-benzo[d]imidazol-1-yl)piperidine-2,6-dione hydrochloride

Tert-butyl 7-(2-(1-(2,6-dioxopiperidin-3-yl)-3-methyl-2-oxo-2,3-dihydro-1H-benzo[d]imidazol-4-yl)amino)ethyl)-2,7-diazaspiro[4.4]nonane-2-carboxylate (200 mg, 0.4 mmol, 1.0 eq) was added to hydrogen chloride-dioxane solution (4 mL, 4 mol/L), and the mixture was stirred at rt for 1 h. Upon completion, the mixture was concentrated to afford the title compound (yellow solid, 160 mg, crude).

### Step F: N-(1-(4-(7-((1-(2,6-Dioxopiperidin-3-yl)-3-methyl-2-oxo-2,3-dihydro-1H-benzo[d]imidazol-4-yl)amino)ethyl)-2,7-diazaspiro[4.4]nonane-2-carbonyl)phenyl)-3-sulfamoyl-1H-pyrazol-4-yl)-6-(1H-pyrazol-5-yl)picolinamide

At rt, to a solution of 3-(4-(2-(2,7-diazaspiro[4.4]nonan-2-yl)ethyl)amino)-3-methyl-2-oxo-2,3-dihydro-1H-benzo[d]imidazol-1-yl)piperidine-2,6-dione hydrochloride (120 mg, crude) and 4-(3-(N-(tert-butoxycarbonyl)sulfamoyl)-4-(6-(1-(tetrahydro-2H-pyran-2-yl)-1H-pyrazol-5-yl)picolinamido)-1H-pyrazol-1-yl)benzoic acid (165 mg, 0.26 mmol, 1.0 eq) in DMF (5 mL) were added N-methylmorpholine (78 mg, 0.78 mmol, 3.0 eq) and HATU (147 mg, 0.4 mmol, 1.5 eq). The reaction solution was stirred at rt for 18 h. Upon completion, the reaction solution was diluted in water and extracted with DCM. The organic phase was washed sequentially with saturated aqueous NaCl, dried over anhydrous Na₂SO₄, filtered, and concentrated. The residue was dissolved in TFA (3 mL), and the mixture was stirred for 2 h. The reaction solution was concentrated, and then the residue was purified by HPLC to afford the title compound (white solid, 34 mg, yield 41%). ¹H NMR (400 MHz, DMSO-*d*₆) *δ* 13.21 (s, 1H), 11.33 (s, 1H), 11.05 (s, 1H), 9.16 (s, 1H), 8.25 (d, *J =* 7.6 Hz, 1H), 8.13 (dd, *J =* 16.8, 7.6 Hz, 2H), 8.08 (s, 2H), 7.97 (t, *J =* 8.4 Hz, 3H), 7.67 (d, *J* = 8.8 Hz, 2H), 7.22 (d, *J* = 2.4 Hz, 1H), 6.88 (t, *J* = 8.0 Hz, 1H), 6.52 (d, *J* = 7.6 Hz, 1H), 6.43 (d, *J =* 8.0 Hz, 1H), 5.32 (dd, *J =* 12.4, 5.2 Hz, 1H), 5.09 (s, 1H), 3.89-3.79 (m, 1H), 3.77-3.67 (m, 1H), 3.61 (s, 3H), 3.56-3.39 (m, 3H), 3.19-3.12 (m, 2H), 2.93-2.81 (m, 3H), 2.78-2.55 (m, 7H), 2.05-1.93 (m, 1H). LCMS (ESI): m/z 862 [M+H]⁺.

### Preparation Example 348. Synthesis of Compound A334

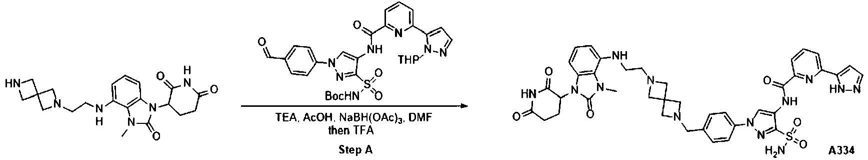

### Step A: N-(1-(4-(6-(2-(1-(2,6-Dioxopiperidin-3-yl)-3-methyl-2-oxo-2,3-dihydro-1H-benzo[d]imidazol-4-yl)amino)ethyl)-2,6-diazaspiro[3.3]heptan-2-yl)methyl)phenyl)-3-sulfamoyl-1H-pyrazol-4-yl)-6-(1H-pyrazol-5-yl)picolinamide

At rt, to a solution of tert-butyl (1-(4-formylphenyl)-4-(6-(1-(tetrahydro-2H-pyran-2-yl)-1H-pyrazol-5-yl)picolinamido)-1H-pyrazol-3-yl)sulfonyl)carbamate (156 mg, 0.25 mmol, 1.0 eq) and 3-(4-(2-(2,6-diazaspiro[3.3]heptan-2-yl)ethyl)amino)-3-methyl-2-oxo-2,3-dihydro-1H-benzo[d]imidazol-1-yl)piperidine-2,6-dione trifluoroacetate (100 mg, 0.25 mmol, 1.0 eq) in DMF (5 mL) was added TEA (0.2 mL). The reaction solution was reacted at rt for 0.5 h. Then AcOH (0.3 mL) was added to the reaction solution, and the mixture was stirred for 18 h. Sodium triacetoxyborohydride (79 mg, 0.38 mmol, 1.5 eq) was added, and the mixture was stirred for 1 h. Upon completion, the reaction solution was diluted in water and extracted with DCM. The organic phase was washed sequentially with saturated aqueous NaCl, dried over anhydrous Na₂SO₄, filtered, and concentrated. The residue was dissolved in TFA (3 mL), and the mixture was stirred at rt for 2 h. After concentration, the residue was purified by HPLC to afford the title compound (white solid, 7.6 mg, yield 21%). **¹H NMR** (400 MHz, DMSO-*d*₆) *δ* 13.21 (s, 1H), 11.32 (s, 1H), 11.06 (s, 1H), 9.11 (s, 1H), 8.25 (d, *J =* 8.0 Hz, 1H), 8.16-8.12 (m, 1H), 8.09-8.03 (m, 3H), 7.98-7.89 (m, 3H), 7.48 (d, *J* = 33.6 Hz, 2H), 7.22 (d, *J* = 2.4 Hz, 1H), 6.90 (t, *J =* 8.0 Hz, 1H), 6.57 (d, *J* = 7.6 Hz, 1H), 6.46 (d, *J =* 8.4 Hz, 1H), 5.30 (dd, *J =* 12.8, 5.2 Hz, 2H), 4.32-3.98 (m, 5H), 3.80-3.53 (m, 6H), 3.31-3.19 (m, 6H), 2.93-2.82 (m, 1H), 2.72-2.59 (m, 2H), 2.02-1.92 (m, 1H). LCMS (ESI): m/z 820 [M+H]⁺.

### Preparation Example 349. Synthesis of Compound A335

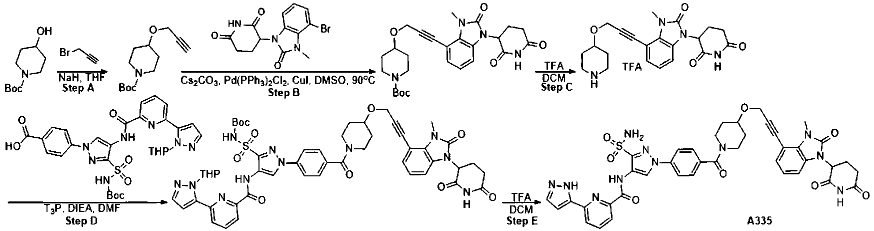

### Step A: Tert-butyl 4-(prop-2-en-1-yloxy)piperidine-1-carboxylate

At 0°C, to a solution of tert-butyl 4-hydroxypiperidine-1-carboxylate (2.0 g, 9.95 mmol, 1.0 eq) in THF (20 mL) was added sodium hydride (477 mg, 11.94 mmol, 1.2 eq, 60% dispersion in mineral oil). The mixture was stirred at 0°C for 30 min. Then, a solution of 3-bromoprop-1-yne (1.76 g, 14.93 mmol, 1.5 eq) was added to the reaction mixture, and the reaction solution was stirred at rt overnight. Upon completion, the reaction solution was slowly poured into ice water and extracted with EtOAc twice. The organic phases were combined, washed with aqueous NaCl, dried over anhydrous Na₂SO₄, and concentrated under reduced pressure. The residue was purified by column chromatography (eluent: petroleum ether/ethyl acetate, gradient: 0%-50% ethyl acetate) to afford the title compound (yellow solid, 2.0 g, yield 84%).

### Step B: Tert-butyl 4-((3-(1-(2,6-dioxopiperidin-3-yl)-3-methyl-2-oxo-2,3-dihydro-1H-benzo[d]imidazol-4-yl)prop-2-yn-1-yl)oxy)piperidine-1-carboxylate

Under N₂, to a solution of tert-butyl 4-(prop-2-en-1-yloxy)piperidine-1-carboxylate (425 mg, 1.78 mmol, 2.0 eq) and 3-(4-bromo-3-methyl-2-oxo-2,3-dihydro-1H-benzo[d]imidazol-1-yl)piperidine-2,6-dione (300 mg, 0.89 mmol, 1.0 eq) in dimethyl sulfoxide (4 mL) were added cesium carbonate (871 mg, 2.67 mmol, 3.0 eq), cuprous iodide (169 mg, 0.18 mmol, 0.2 eq), and Pd(PPh)₃Cl₂ (125 mg, 0.18 mmol, 0.2 eq). The reaction solution was purged with N₂ (×3) and stirred at 90°C under N₂ overnight. Upon completion, the reaction solution was slowly poured into ice water. The layers were separated, and the aqueous phase was extracted with EtOAc. The organic phases were combined, washed with saturated aqueous NaCl, dried over anhydrous Na₂SO₄, and concentrated under reduced pressure. The residue was purified by column chromatography (eluent: petroleum ether/ethyl acetate, gradient: 0%-67% ethyl acetate) to afford the title compound (brown solid, 300 mg, yield 68%).

### Step C: 3-(3-Methyl-2-oxo-4-(3-(piperidin-4-yloxy)prop-1-yn-1-yl)-2,3-dihydro-1H-benzo[d]imidazol-1-yl)piperidine-2,6-dione

In an ice-water bath, to a solution of tert-butyl 4-((3-(1-(2,6-dioxopiperidin-3-yl)-3-methyl-2-oxo-2,3-dihydro-1H-benzo[d]imidazol-4-yl)prop-2-yn-1-yl)oxy)piperidine-1-carboxylate (300 mg, 0.60 mmol, 1.0 eq) in DCM (3 mL) was added TFA (1.0 mL). The reaction solution was then stirred at rt for 1 h. Upon completion, the reaction solution was concentrated under reduced pressure to afford the title compound (yellow oil, 230 mg, crude),

### Step D: Tert-butyl ((1-(4-((3-(1-(2,6-dioxopiperidin-3-yl)-3-methyl-2-oxo-2,3-dihydro-1H-benzo[d]imidazol-4-yl)prop-2-yn-1-yl)oxy)piperidine-1-carbonyl)phenyl)-4-(6-(1-(tetrahydro-2H-pyran-2-yl)-1H-pyrazol-5-yl)picolinamido)-1H-pyrazol-3-yl)sulfonyl)carbamate

At rt, to a solution of 4-(3-(N-(tert-butoxycarbonyl)sulfamoyl)-4-(6-(1-(tetrahydro-2H-pyran-2-yl)-1H-pyrazol-5-yl)pyridinylamino)-1H-pyrazol-1-yl)benzoic acid (200 mg, 0.31 mmol, 1.0 eq) and 3-(3-methyl-2-oxo-4-(3-(piperidin-4-yloxy)prop-1-yn-1-yl)-2,3-dihydro-1H-benzo[d]imidazol-1-yl)piperidine-2,6-dione (249 mg, crude) in DMF (3 mL) were added DIEA (243 mg, 1.88 mmol, 6.0 eq) and T₃P (293 mg, 0.46 mmol, 1.5 eq, 50% in EtOAc). The mixture was stirred at rt overnight. Upon completion, the reaction solution was slowly poured into ice water and extracted with EtOAc twice. The organic phases were combined, washed with aqueous NaCl, dried over anhydrous Na₂SO₄, and concentrated under reduced pressure to afford the title compound (brown solid, 150 mg, yield 47%).

### Step E: N-(1-(4-(4-((3-(1-(2,6-Dioxopiperidin-3-yl)-3-methyl-2-oxo-2,3-dihydro-1H-benzo[d]imidazol-4-yl)prop-2-yn-1-yl)oxy)piperidine-1-carbonyl)phenyl)-3-sulfamoyl-1H-pyrazol-4-yl)-6-(1H-pyrazol-5-yl)picolinamide

In an ice-water bath, to a solution of tert-butyl ((1-(4-((3-(1-(2,6-dioxopiperidin-3-yl)-3-methyl-2-oxo-2,3-dihydro-1H-benzo[d]imidazol-4-yl)prop-2-yn-1-yl)oxy)piperidine-1-carbonyl)phenyl)-4-(6-(1-(tetrahydro-2H-pyran-2-yl)-1H-pyrazol-5-yl)picolinamido)-1H-pyrazol-3-yl)sulfonyl)carbamate (150 mg, 0.15 mmol, 1.0 eq) in DCM (3 mL) was added TFA (1 mL). The reaction solution was then stirred at rt for 1 h. Upon completion, the reaction solution was concentrated under reduced pressure, and the residue was purified by preparative HPLC (C₁₈, 10%-95% acetonitrile in water containing 0.1% FA) to afford the title compound (white solid, 30 mg, yield 41%). LCMS (ESI): m/z 832 [M+H]⁺. **¹H NMR** (400 MHz, DMSO-*d*₆) *δ* 13.20 (s, 1H), 11.33 (s, 1H), 11.12 (s, 1H), 9.16 (s, 1H), 8.25 (d, *J =* 7.6 Hz, 1H), 8.15-8.11 (m, 1H), 8.09-8.07 (m, 3H), 8.01-7.99 (m, 2H), 7.95-7.74 (m, 1H), 7.61 (d, *J =* 8.6 Hz, 2H), 7.24-7.21 (m, 1H), 7.19-7.17 (m, 1H), 7.14-7.12 (m, 1H), 7.03 (t, *J =* 7.8 Hz, 1H), 5.44-5.36 (m, 1H), 4.53 (s, 2H), 4.03-3.95 (m, 1H), 3.88-3.84 (m, 1H), 3.59 (s, 3H), 3.55-3.46 (m, 2H), 2.92-2.84 (m, 1H), 2.73-2.64 (m, 2H), 2.05-1.88 (m, 4H), 1.56-1.54 (m, 2H).

### Preparation Example 350. Synthesis of Compound A336

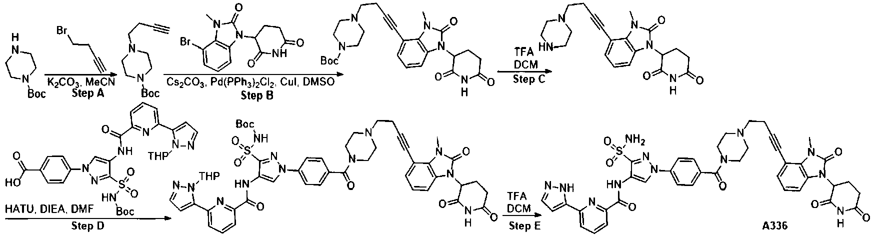

### Step A: Tert-butyl 4-(but-3-yn-1-yl)piperazine-1-carboxylate

At rt, to a solution of tert-butyl piperazine-1-carboxylate (4 g, 21.5 mmol, 1.0 eq) in acetonitrile (40 mL) were added K₂CO₃ (7.4 g, 53.75 mmol, 2.5 eq) and 4-bromobut-1-yne (3 g, 22.6 mmol, 1.0 eq). The reaction solution was stirred at rt for 16 h. Upon completion, the reaction solution was diluted in EtOAc, washed sequentially with water and saturated aqueous NaCl, dried over anhydrous Na₂SO₄, and concentrated under reduced pressure. The residue was purified by column chromatography (eluent: petroleum ether/ethyl acetate, gradient: 0%-15% ethyl acetate) to afford the title compound (yellow oil, 3 g, yield 59%).

### Step B: Tert-butyl 4-(4-(1-(2,6-dioxopiperidin-3-yl)-3-methyl-2-oxo-2,3-dihydro-1H-benzo[d]imidazol-4-yl)but-3-yn-1-yl)piperazine-1-carboxylate

At rt, to a solution of tert-butyl 4-(but-3-yn-1-yl)piperazine-1-carboxylate (422 mg, 1.8 mmol, 1.5 eq) and 3-(4-bromo-3-methyl-2-oxo-2,3-dihydro-1H-benzo[d]imidazol-1-yl]piperidine-2,6-dione (400 mg, 1.18 mmol, 1.0 eq) in dimethyl sulfoxide (5 mL) were sequentially added Cs₂CO₃ (1.15 g, 3.54 mmol, 3 eq), Pd(PPh₃)₂Cl₂ (83 mg, 0.12 mmol, 0.1 eq), and cuprous iodide (23 mg, 0.12 mmol, 0.1 eq). The reaction solution was purged with N₂ (×3), and the reaction was stirred at 90°C under N₂ for 2 h. Upon completion, the reaction solution was diluted in water and extracted with EtOAc. The organic phase was washed with saturated aqueous NaCl, dried over anhydrous Na₂SO₄, and concentrated under reduced pressure. The residue was purified by column chromatography (eluent: petroleum ether/ethyl acetate, gradient: 0%-50% ethyl acetate) to afford the title compound (yellow solid, 350 mg, yield 60%).

### Step C: 3-(3-Methyl-2-oxo-4-(4-(piperazin-1-yl)but-1-yn-1-yl)-2,3-dihydro-1H-benzo[d]imidazol-1-yl)piperidine-2,6-dione

At 0°C, to a solution of tert-butyl 4-(4-(1-(2,6-dioxopiperidin-3-yl)-3-methyl-2-oxo-2,3-dihydro-1H-benzo[d]imidazol-4-yl)but-3-yn-1-yl)piperazine-1-carboxylate (150 mg, 0.3 mmol, 1.0 eq) in DCM (2 mL) was added TFA (1 mL, 15 mmol, 50 eq). The solution was stirred at rt for 30 min. The reaction solution was concentrated under reduced pressure, and the residue was purified by preparative HPLC (YMC-Actus Triart C18, 30%-90% acetonitrile in water containing 0.1% aqueous ammonia) to afford the title compound (yellow solid, 90 mg, yield 76%).

### Step D: Tert-butyl ((1-(4-(4-(1-(2,6-dioxopiperidin-3-yl)-3-methyl-2-oxo-2,3-dihydro-1H-benzo[d]imidazol-4-yl)but-3-yn-1-yl)piperazine-1-carbonyl)phenyl)-4-(6-(1-(tetrahydro-2H-pyran-2-yl)-1H-pyrazol-5-yl)picolinamide)-1H-pyrazol-3-yl)sulfonyl)carbamate

At rt, to a solution of 3-(3-methyl-2-methyl-2-oxo-4-(4-(piperazin-1-yl)but-1-yn-1-yl)-2,3-dihydro-1H-benzo[d]imidazol-1-yl)piperidine-2,6-dione (75 mg, 0.19 mmol, 1.2 eq) in DMF (3 mL) were sequentially added 4-(3-(N-(tert-butoxycarbonyl)sulfamoyl)-4-(6-(1-(tetrahydro-2H-pyran-2-yl)-1H-pyrazol-5-yl)pyridinylamino)-1H-pyrazol-1-yl)benzoic acid (100 mg, 0.16 mmol, l eq), DIEA (62 mg, 0.48 mmol, 3 eq), and HATU (73 mg, 0.19 mmol, 1.2 eq). The reaction solution was stirred at rt under N₂ for l h. Upon completion, the reaction solution was diluted in EtOAc, washed sequentially with water and saturated aqueous NaCl, dried over anhydrous Na₂SO₄, and concentrated under reduced pressure to afford the title compound (yellow solid, 100 mg, crude), which was used directly in the next reaction without purification.

### Step E: N-(1-(4-(4-(1-(2,6-Dioxopiperidin-3-yl)-3-methyl-2-oxo-2,3-dihydro-1H-benzo[d]imidazol-4-yl)but-3-yn-1-yl)piperazine-1-carbonyl)phenyl)-3-sulfamoyl-1H-pyrazol-4-yl)-6-(1H-pyrazol-5-yl)picolinamide

At 0°C, to a solution of tert-butyl ((1-(4-(4-(1-(2,6-dioxopiperidin-3-yl)-3-methyl-2-oxo-2,3-dihydro-1H-benzo[d]imidazol-4-yl)but-3-yn-1-yl)piperazine-1-carbonyl)phenyl)-4-(6-(1-(tetrahydro-2H-pyran-2-yl)-1H-pyrazol-5-yl)picolinamido)-1H-pyrazol-3-yl)sulfonyl)carbamate (100 mg, crude) in DCM (2 mL) was added TFA (1 mL). The solution was stirred at rt for 30 min. Upon completion, the mixture was concentrated under reduced pressure, and the residue was purified by preparative HPLC (YMC-Actus Triart C₁₈, 25%-90% acetonitrile in water containing 0.1% aqueous ammonia) to afford the title compound (white solid, 29 mg, 35% yield over two steps). LCMS (ESI): m/z 831[M+H]⁺. **¹H NMR** (400 MHz, DMSO-*d*₆) *δ* 13.21 (s, 1H), 11.33 (s, 1H), 11.11 (s, 1H), 9.17 (s, 1H), 8.26-8.24 (m, 1H), 8.15-8.12 (m, 1H), 9.09-8.08 (m, 3H), 8.02-8.00 (m, 2H), 7.95 (s, 1H), 7.62 (d, *J =* 8.6 Hz, 2H), 7.23 (s, 1H), 7.12 (d, *J =* 8.0 Hz, 1H), 7.06 (d, *J* = 7.0 Hz, 1H), 7.00 (t*, J* = 7.8 Hz, 1H), 5.41- 5.37 (m, 1H), 3.69 (s, 3H), 2.94-2.84 (m, 3H), 2.79-2.57 (m, 11H), 2.11-1.91 (m, 2H).

### Preparation Example 351. Synthesis of Compound A337

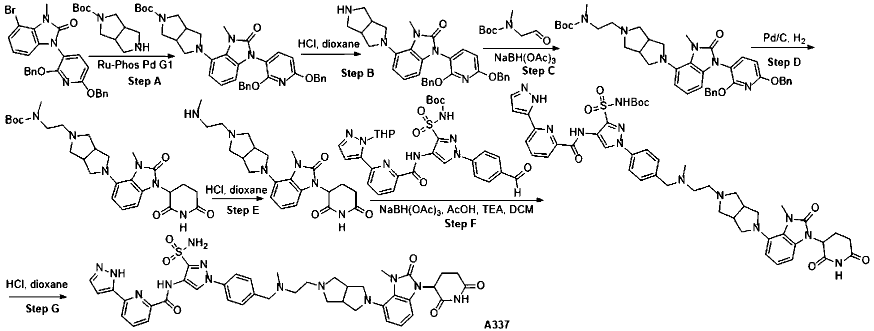

### Step A: Tert-butyl 5-(1-(2,6-bis(benzyloxy)pyridin-3-yl)-3-methyl-2-oxo-2,3-dihydro-1H-benzo[d]imidazol-4-yl)hexahydropyrrolo[3,4-c]pyrrole-2(1H)-carboxylate

A mixture of 1-(2,6-bis(benzyloxy)pyridin-3-yl)-4-bromo-3-methyl-1,3-dihydro-2H-benzo[d]imidazol-2-one (2.0 g, 3.87 mmol, 1.0 eq), tert-butyl hexahydropyrrolo[3,4-c]pyrrole-2(1H)-carboxylate (0.82 g, 3.87 mmol, 1.0 eq), 2-dicyclohexylphosphino-2',6'-diisopropoxy-1,1'-biphenyl (0.36 g, 0.76 mmol, 0.2 eq), Ru-Phos Pd G1 (0.32 g, 0.39 mmol, 0.1 eq), and cesium carbonate (3.79 g, 11.62 mmol, 3.0 eq) was dissolved in dioxane (100 mL), and the reaction was stirred at 100°C under N₂ for 16 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (colorless clear liquid, 1.1 g, yield 44%).

### Step B: 1-(2,6-Bis(benzyloxy)pyridin-3-yl)-4-(hexahydropyrrolo[3,4-c]pyrrol-2(1H)-yl)-3-methyl-1,3-dihydro-2H-benzo[d]imidazol-2-one

At rt, to a solution of tert-butyl 5-(1-(2,6-bis(benzyloxy)pyridin-3-yl)-3-methyl-2-oxo-2,3-dihydro-1H-benzo[d]imidazol-4-yl)hexahydropyrrolo[3,4-c]pyrrole-2(1H)-carboxylate (1.1 g, 1.69 mmol, 1.0 eq) in acetonitrile (20 mL) was added hydrogen chloride-dioxane solution (10 mL). The mixture was stirred at rt under N₂ for l h. Upon completion, the reaction solution was concentrated under reduced pressure. The residue was purified by column chromatography to afford the title compound (white solid, 900 mg, crude).

### Step C: Tert-butyl (2-(5-(1-(2,6-bis(benzyloxy)pyridin-3-yl)-3-methyl-2-oxo-2,3-dihydro-1H-benzo[d]imidazol-4-yl)hexahydropyrrolo[3,4-c]pyrrol-2(1H)-yl)ethyl)(methyl)carbamate

At rt, to a solution of 1-(2,6-bis(benzyloxy)pyridin-3-yl)-4-(hexahydropyrrolo[3,4-c]pyrrol-2(1H)-yl)-3-methyl-1,3-dihydro-2H-benzo[d]imidazol-2-one (900 mg, crude) in DCM (20 mL) were added TEA (0.46 mL, 3.28 mmol, 2.0 eq), AcOH (0.38 mL, 6.57 mmol, 4.0 eq), and tert-butyl methyl(2-oxoethyl)carbamate (284 mg, 1.64 mmol, 1.0 eq). The mixture was stirred at rt under N₂ for 30 min, and then sodium triacetoxyborohydride (697 mg, 3.28 mmol, 2.0 eq) was added at the same temperature. The reaction solution was stirred at rt under N₂ for 3 h. Upon completion, the mixture was diluted in DCM, washed sequentially with NaHCO₃ and saturated aqueous NaCl, dried over anhydrous Na₂SO₄, and concentrated under reduced pressure. The residue was purified by column chromatography to afford the title compound (colorless clear liquid, 800 mg, yield 69%).

### Step D: Tert-butyl (2-(5-(1-(2,6-dioxopiperidin-3-yl)-3-methyl-2-oxo-2,3-dihydro-1H-benzo[d]imidazol-4-yl)hexahydropyrrolo[3,4-c]pyrrol-2(1H)-yl)ethyl)(methyl)carbamate

At rt, to a solution of tert-butyl (2-(5-(1-(2,6-bis(benzyloxy)pyridin-3-yl)-3-methyl-2-oxo-2,3-dihydro-1H-benzo[d]imidazol-4-yl)hexahydropyrrolo[3,4-c]pyrrol-2(1H)-yl)ethyl)(methyl)carbamate (800 mg, 1.13 mmol, 1.0 eq) in ethanol (30 mL) was added palladium on carbon (120 mg, 10 wt%). The reaction solution was stirred at rt under H₂ for 12 h. Upon completion, the reaction solution was filtered through Celite, and the filtrate was concentrated under reduced pressure to afford the title compound (yellow liquid, 500 mg, yield 80%).

### Step E: 3-(3-Methyl-4-(5-(2-(methylamino)ethyl)hexahydropyrrolo[3,4-c]pyrrol-2(1H)-yl)-2-oxo-2,3-dihydro-1H-benzo[d]imidazol-1-yl)piperidine-2,6-dione

At rt, to a solution of tert-butyl (2-(5-(1-(2,6-dioxopiperidin-3-yl)-3-methyl-2-oxo-2,3-dihydro-1H-benzo[d]imidazol-4-yl)hexahydropyrrolo[3,4-c]pyrrol-2(1H)-yl)ethyl)(methyl)carbamate (500 mg, 0.95 mmol, 1.0 eq) in acetonitrile (20 mL) was added hydrogen chloride-dioxane solution (10 mL, 4 M). The reaction solution was stirred at rt under N₂ for l h. Upon completion, the reaction solution was concentrated under reduced pressure to afford the title compound (white solid, 350 mg, crude).

### Step F: Tert-butyl ((4-(6-(1H-pyrazol-5-yl)picolinamido)-1-(4-((2-(5-(1-(2,6-dioxopiperidin-3-yl)-3-methyl-2-oxo-2,3-dihydro-1H-benzo[d]imidazol-4-yl)hexahydropyrrolo[3,4-c]pyrrol-2(1H)-yl)ethyl)(methyl)amino)methyl)phenyl)-1H-pyrazol-3-yl)sulfonyl)carbamate

At rt, to a solution of 3-(3-methyl-4-(5-(2-(methylamino)ethyl)hexahydropyrrolo[3,4-c]pyrrol-2(1H)-yl)-2-oxo-2,3-dihydro-1H-benzo[d]imidazol-1-yl)piperidine-2,6-dione (130 mg, crude) in DMF (20 mL) were added TEA (0.09 mL, 0.61 mmol, 2.0 eq), AcOH (0.09 mL, 1.52 mmol, 5.0 eq), and tert-butyl ((1-(4-formylphenyl)-4-(6-(1-(tetrahydro-2H-pyran-2-yl)-1H-pyrazol-5-yl)pyridinylamino)-1H-pyrazol-3-yl)sulfonyl)carbamate (227 mg, 0.37 mmol, 1.2 eq). The reaction solution was stirred at rt under N₂ for 30 min, and then sodium triacetoxyborohydride (129 mg, 0.61 mmol, 2.0 eq) was added at the same temperature to the reaction solution. The reaction solution was stirred at rt under N₂ for 4 h. Upon completion, the reaction solution was poured into water (50 mL), and a yellow solid was precipitated. The yellow solid was dissolved in EtOAc (20 mL), filtered, and concentrated under reduced pressure to afford the title compound (yellow solid, 80 mg, 28% yield over two steps).

### Step G: N-(1-(4-(((2-(5-(1-(2,6-Dioxopiperidin-3-yl)-3-methyl-2-oxo-2,3-dihydro-1H-benzo[d]imidazol-4-yl)hexahydropyrrolo[3,4-c]pyrrol-2(1H)-yl)ethyl)(methyl)amino)methyl)phenyl)-3-sulfamoyl-1H-pyrazol-4-yl)-6-(1H-pyrazol-5-yl)picolinamide

At rt, to a solution of tert-butyl ((4-(6-(1H-pyrazol-5-yl)picolinamido)-1-(4-((2-(5-(1-(2,6-dioxopiperidin-3-yl)-3-methyl-2-oxo-2,3-dihydro-1H-benzo[d]imidazol-4-yl)}hexahydropyrrolo[3,4-c]pyrrol-2(1H)-yl)ethyl)(methyl)amino)methyl)phenyl)-lH-pyrazol-3-yl)sulfonyl)carbamate (80 mg, 0.08 mmol, 1.0 eq) in acetonitrile (10 mL) was added hydrogen chloride-dioxane solution (5 mL). The reaction solution was stirred at 50°C under N₂ for 2 h. Upon completion, the reaction solution was concentrated under reduced pressure. The residue was purified by HPLC to afford the title compound (white solid, 26.0 mg, yield 36%). **¹H NMR** (400 MHz, DMSO) *δ* 13.21 (s, 1H), 11.32 (s, 1H), 11.08 (s, 1H), 9.07 (s, 1H), 8.27-8.22 (m, 1H), 8.18 (s, 1H), 8.13 (t, *J =* 7.7 Hz, 1H), 8.09-8.06 (m, 1H), 8.04-8.01 (m, 1H), 7.93 (s, 1H), 7.88-7.83 (m, 2H), 7.51 (d, *J* = 8.5 Hz, 2H), 7.25-7.20 (m, 1H), 6.99-6.91 (m, 2H), 6.89-6.83 (m, 1H), 5.34 (dd, *J=* 12.5, 5.3 Hz, 1H), 3.63 (s, 3H), 3.59 (s, 2H), 3.16-3.11 (m, 2H), 2.92-2.86 (m, 1H), 2.85-2.78 (m, 4H), 2.72-2.67 (m, 1H), 2.65-2.60 (m, 4H), 2.59-2.52 (m, 5H), 2.20 (s, 3H), 2.02-1.97 (m, 1H).

### Preparation Example 352. Synthesis of Compound A338

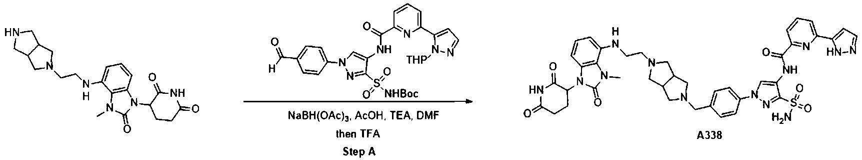

### Step A: N-(1-(4-(5-(2-(1-(2,6-Dioxopiperidin-3-yl)-3-methyl-2-oxo-2,3-dihydro-1H-benzo[d]imidazol-4-yl)amino)ethyl)hexahydropyrrolo[3,4-c]pyrrol-2(1H)-yl)methyl)phenyl)-3-sulfamoyl-1H-pyrazol-4-yl)-6-(1H-pyrazol-5-yl)picolinamide

At rt, to a solution of tert-butyl (1-(4-formylphenyl)-4-(6-(1-(tetrahydro-2H-pyran-2-yl)-1H-pyrazol-5-yl)picolinamido)-1H-pyrazol-3-yl)sulfonyl)carbamate (150 mg, 0.24 mmol, 1.0 eq) and 3-(4-(2-(hexahydropyrrolo[3,4-c]pyrrol-2(1H)-yl)ethyl)amino)-3-methyl-2-oxo-2,3-dihydro-1H-benzo[d]imidazol-1-yl)piperidine-2,6-dione (100 mg, 0.24 mmol, 1.0 eq) in DMF (5 mL) was added TEA (0.2 mL). The reaction solution was reacted at rt for 0.5 h. Then AcOH (0.3 mL) was added to the reaction solution, and the mixture was stirred for 18 h. Sodium triacetoxyborohydride (77 mg, 0.36 mmol, 1.5 eq) was added, and the mixture was stirred for 1 h. Upon completion, the reaction solution was diluted in water and extracted with DCM. The organic phase was washed sequentially with saturated aqueous NaCl, dried over anhydrous Na₂SO₄, filtered, and concentrated. The residue was dissolved in TFA (3 mL), and the mixture was stirred at rt for 2 h. After concentration, the residue was purified by HPLC to afford the title compound (white solid, 80 mg, yield 32%). **¹H NMR** (400 MHz, DMSO-*d*₆) *δ* 13.23 (s, 1H), 11.32 (s, 1H), 11.07 (s, 1H), 9.17 (s, 1H), 8.25 *(d, J =* 7.2 Hz, 1H), 8.14 (t, *J* = 7.6 Hz, 1H), 8.08 (d, *J* = 8.0 Hz, 5H), 7.93 (s, 1H), 7.74-7.61 (m, 2H), 7.22 (d, *J* = 2.4 Hz, 1H), 6.92 (t, *J* = 8.0 Hz, 1H), 6.60 (d, *J* = 8.0 Hz, 1H), 6.51 (d, *J =* 6.8 Hz, 1H), 5.68-5.07 (m, 2H), 4.53-4.33 (m, 2H), 4.00-3.90 (m, 2H), 3.76-3.71 (m, 3H), 3.64 (s, 3H), 3.49-3.35 (m, 6H), 3.20-3.09 (m, 2H), 2.97-2.81 (m, 2H), 2.73-2.60 (m, 2H), 1.99-1.89 (m, 1H). LCMS (ESI): m/z 834 [M+H]⁺.

### Preparation Example 353. Synthesis of Compound A339

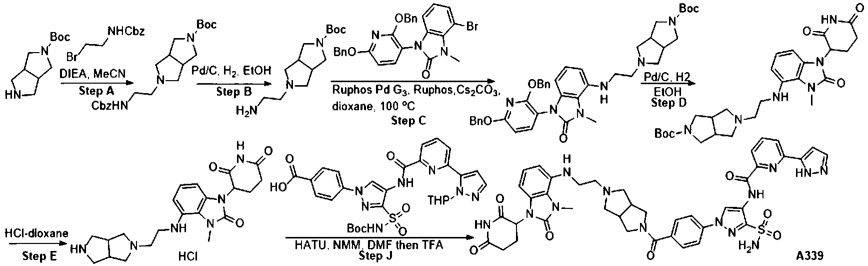

### Step A: Tert-butyl 5-(2-(((benzyloxy)carbonyl)amino)ethyl)hexahydropyrrolo[3,4-c]pyrrole-2(1H)-carboxylate

At rt, to a solution of tert-butyl hexahydropyrrolo[3,4-c]pyrrole-2(1H)-carboxylate (1 g, 4.7 mmol, 1.0 eq) in acetonitrile (15 mL) were added DIEA (2.5 mL, 14.1 mmol, 3.0 eq) and 2-bromoethyl benzyl carbamate (1.2 g, 4.7 mmol, 1.0 eq). The reaction solution was stirred at rt for 18 h. Upon completion, the reaction solution was diluted in water and extracted with DCM. The organic phase was washed sequentially with saturated aqueous NaCl, dried over anhydrous Na₂SO₄, filtered, and concentrated. The residue was purified by column chromatography to afford the title compound (yellow oil, 0.8 g, yield 44%).

### Step B: Tert-butyl 5-(2-aminoethyl)hexahydropyrrolo[3,4-c]pyrrole-2(1H)-carboxylate

At rt, to a solution of tert-butyl 5-(2-(((benzyloxy)carbonyl)amino)ethyl)hexahydropyrrolo[3,4-c]pyrrole-2(1H)-carboxylate (800 mg, 2.0 mmol, 1.0 eq) in ethanol (10 mL) was added 10% Pd/C (100 mg). The reaction solution was purged with H₂ (×3), and then stirred under H₂ at 25°C for 2 h. Upon completion, the mixture was filtered to remove Pd/C, and the filtrate was concentrated to afford the title compound (yellow oil, 300 mg, yield 57%).

### Step C: Tert-butyl 5-(2-(1-(2,6-bis(benzyloxy)pyridin-3-yl)-3-methyl-2-oxo-2,3-dihydro-1H-benzo[d]imidazol-4-yl)amino)ethyl)hexahydropyrrolo[3,4-c]pyrrole-2(1H)-carboxylate

At rt, to a solution of 1-(2,6-bis(benzyloxy)pyridin-3-yl)-4-bromo-3-methyl-1,3-dihydro-2H-benzo[d]imidazol-2-one (1.3 g, 2.6 mmol, 1.0 eq) and tert-butyl 5-(2-aminoethyl)hexahydropyrrolo[3,4-c]pyrrole-2(1H)-carboxylate (660 mg, 2.6 mmol, 1.0 eq) in dioxane (15 mL) were added cesium carbonate (2.5 g, 7.8 mmol, 3.0 eq), RuPhos Pd G₃ (216 mg, 0.26 mmol, 0.1 eq), and RuPhos (120 mg, 0.26 mmol, 0.1 eq). The mixture was stirred at 100°C under N₂ for 15 h. Upon completion, the mixture was diluted in water and extracted with DCM. The organic phase was washed sequentially with saturated aqueous NaCl, dried over anhydrous Na₂SO₄, filtered, and concentrated. The residue was purified by column chromatography to afford the title compound (yellow solid, 1.0 g, yield 56%).

### Step D: Tert-butyl 5-(2-(1-(2,6-dioxopiperidin-3-yl)-3-methyl-2-oxo-2,3-dihydro-1H-benzo[d]imidazol-4-yl)amino)ethyl)hexahydropyrrolo[3,4-c]pyrrole-2(1H)-carboxylate

At rt, to a solution of tert-butyl 5-(2-(1-(2,6-bis(benzyloxy)pyridin-3-yl)-3-methyl-2-oxo-2,3-dihydro-1H-benzo[d]imidazol-4-yl)amino)ethyl)hexahydropyrrolo[3,4-c]pyrrole-2(1H)-carboxylate (900 mg, 1.3 mmol, 1.0 eq) in ethanol (15 mL) were added 10% Pd/C (100 mg) and 10% Pd(OH)₂/C (100 mg). The reaction solution was purged with H₂ (×3), and then stirred under H₂ at 50°C for 18 h. Upon completion, the mixture was filtered, and the filtrate was concentrated to afford the title compound (yellow solid, 400 mg, yield 60%).

### Step E: 3-(4-(2-(Hexahydropyrrolo[3,4-c]pyrrol-2(1H)-yl)ethyl)amino)-3-methyl-2-oxo-2,3-dihydro-1H-benzo[d]imidazol-1-yl)piperidine-2,6-dione hydrochloride

A solution of tert-butyl 5-(2-(1-(2,6-dioxopiperidin-3-yl)-3-methyl-2-oxo-2,3-dihydro-1H-benzo[d]imidazol-4-yl)amino)ethyl)hexahydropyrrolo[3,4-c]pyrrole-2(1H)-carboxylate (400 mg, 0.8 mmol, 1.0 eq) in hydrogen chloride-dioxane solution (5 mL, 4 mol/L) was stirred at rt for 1 h. Upon completion, the reaction solution was concentrated to afford the title compound (yellow solid, 280 mg, crude).

### Step F: N-(1-(4-(5-((1-(2,6-Dioxopiperidin-3-yl)-3-methyl-2-oxo-2,3-dihydro-1H-benzo[d]imidazol-4-yl)amino)ethyl)octahydropyrrolo[3,4-c]pyrrole-2-carbonyl)phenyl)-3-sulfamoyl-1H-pyrazol-4-yl)-6-(1H-pyrazol-5-yl)picolinamide

At rt, to a solution of 4-(3-(N-(tert-butoxycarbonyl)sulfamoyl)-4-(6-(1-(tetrahydro-2H-pyran-2-yl)-1H-pyrazol-5-yl)picolinamido)-1H-pyrazol-1-yl)benzoic acid (155 mg, 0.24 mmol, 1.0 eq) and 3-(4-(2-(hexahydropyrrolo[3,4-c]pyrrol-2(1H)-yl)ethyl)amino)-3-methyl-2-oxo-2,3-dihydro-1H-benzo[d]imidazol-1-yl)piperidine-2,6-dione hydrochloride (108 mg, crude) in DMF (5 mL) were added N-methylmorpholine (74 mg, 0.73 mmol, 3.0 eq) and HATU (138 mg, 0.36 mmol, 1.5 eq). The reaction solution was stirred at rt for 18 h. Upon completion, the reaction solution was diluted in water and extracted with DCM. The organic phase was washed sequentially with saturated aqueous NaCl, dried over anhydrous Na₂SO₄, filtered, and concentrated. The residue was dissolved in TFA (3 mL), and the mixture was stirred for 2 h. The reaction solution was concentrated, and then the residue was purified by HPLC to afford the title compound (white solid, 28 mg, yield 43%). **¹H NMR** (400 MHz, DMSO-*d*₆) 13.21 (s, 1H), 11.33 (s, 1H), 11.05 (s, 1H), 9.16 (s, 1H), 8.25 (d, *J* = 7.6 Hz, 1H), 8.13 (dd, *J =* 16.6, 7.6 Hz, 2H), 8.08 (s, 2H), 7.97 (t, *J =* 8.8 Hz, 3H), 7.67 (d, *J =* 8.8 Hz, 2H), 7.22 (d, *J* = 2.4 Hz, 1H), 6.88 (t, *J* = 8.0 Hz, 1H), 6.52 (d, *J* = 7.6 Hz, 1H), 6.43 (d, *J* = 8.0 Hz, 1H), 5.32 (dd, *J* = 12.4, 5.2 Hz, 1H), 5.09 (s, 1H), 3.89-3.79 (m, 1H), 3.77-3.67 (m, 1H), 3.61 (s, 3H), 3.56-3.39 (m, 3H), 3.19-3.12 (m, 2H), 2.93-2.81 (m, 3H), 2.78-2.55 (m, 7H), 2.05-1.93 (m, 1H). LCMS (ESI): m/z 848 [M+H]⁺.

### Preparation Example 354. Synthesis of Compound A340

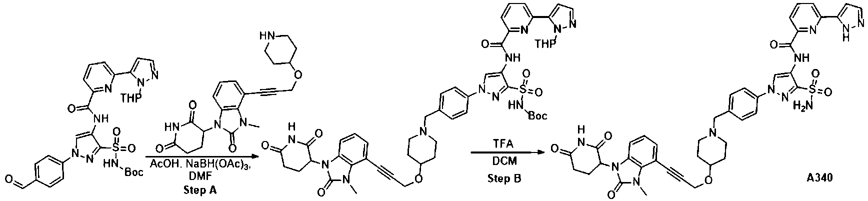

### Step A: ((1-(4-((4-((3-(1-(1-(2,6-dioxopiperidin-3-yl)-3-methyl-2-oxo-2,3-dihydro-1H-benzo[d]imidazol-4-yl)prop-2-yn-1-yl)oxy)piperidin-1-yl)methyl)phenyl)-4-(6-(1-(tetrahydro-2H-pyran-2-yl)-1H-pyrazol-5-yl)picolinamido)-1H-pyrazol-3-yl)sulfonyl)carbamate

Under N₂, to a solution of tert-butyl ((1-(4-formylphenyl)-4-(6-(1-(tetrahydro-2H-pyran-2-yl)-1H-pyrazol-5-yl)pyridinylamino)-1H-pyrazol-3-yl)sulfonyl)carbamate (50 mg, 0.08 mmol, 1.0 eq) and 3-(3-methyl-2-oxo-4-(3-(piperidin-4-yloxy)prop-1-yn-1-yl)-2,3-dihydro-1H-benzimidazol-1-yl)piperidine-2,6-dione (32 mg, 0.08 mmol, 1.0 eq) in DMF (2 mL) were added AcOH (0.01 mL, 0.02 mmol, 0.1 eq) and NaBH(OAc)₃ (59 mg, 0.26 mmol, 3.0 eq). The reaction solution was stirred at rt under N₂ for 0.5 h. Upon completion, the reaction solution was quenched with saturated NaHCO₃ solution and extracted with EtOAc twice. The organic phase was washed sequentially with water and saturated aqueous NaCl, dried over anhydrous Na₂SO₄, and concentrated under reduced pressure. The residue was purified by preparative HPLC (C₁₈, 10%-50% acetonitrile in water containing 0.1% FA) to afford the title compound (white solid, 35.1 mg, yield 44%).

### Step B: N-(1-(4-((4-((3-(1-(2,6-Dioxopiperidin-3-yl)-3-methyl-2-oxo-2,3-dihydro-1H-benzo[d]imidazol-4-yl)prop-2-yn-1-yl)oxy)piperidin-1-yl)methyl)phenyl)-3-sulfamoyl-1H-pyrazol-4-yl)-6-(1H-pyrazol-5-yl)picolinamide

At rt, to a solution of tert-butyl ((1-(4-((4-((3-(1-(1-(2,6-dioxopiperidin-3-yl)-3-methyl-2-oxo-2,3-dihydro-1H-benzo[d]imidazol-4-yl)prop-2-yn-1-yl)oxy)piperidin-1-yl)methyl)phenyl)-4-(6-(1-(tetrahydro-2H-pyran-2-yl)-1H-pyrazol-5-yl)picolinamido)-1H-pyrazol-3-yl)sulfonyl)carbamate (35 mg, 0.04 mmol, 1.0 eq) in DCM (3 mL) was added TFA (1 mL). The reaction solution was stirred at rt under N₂ for 3 h. The residue was purified by HPLC (C₁₈, 10%-50% acetonitrile in water containing 0.1% FA) to afford the title compound (white solid, 5.0 mg, yield 18%). LCMS (ESI): m/z 818.4 [M+H]⁺. **¹H NMR** (400 MHz, DMSO-*d*₆) *δ* 13.20 (s, 1H), 11.32 (s, 1H), 11.12 (s, 1H), 9.07 (s, 1H), 8.24 (d, *J =* 7.3 Hz, 1H), 8.19-8.10 (m, 2H), 8.06-8.03 (m, 2H), 7.94 (s, 1H), 7.87 *(d, J =* 8.5 Hz, 2H), 7.50 (d, J *=* 8.4 Hz, 2H), 7.22 (s, 1H), 7.17 (d,*J =* 7.7 Hz, 1H), 7.12 (d, *J =* 7.2 Hz, 1H), 7.07-6.99 (m, 1H), 5.42-5.38 (m, 1H), 4.48 (s, 2H), 3.64 (s, 3H), 3.62-3.57 (m, 1H), 3.54 (s, 2H), 2.92-2.85 (m, 1H), 2.72-2.61 (m, 4H), 2.23-2.12 (m, 2H), 2.07-2.00 (m, 1H), 1.95-1.86 (m, 2H), 1.59-1.48 (m, 2H).

### Preparation Example 355. Synthesis of Compound A341

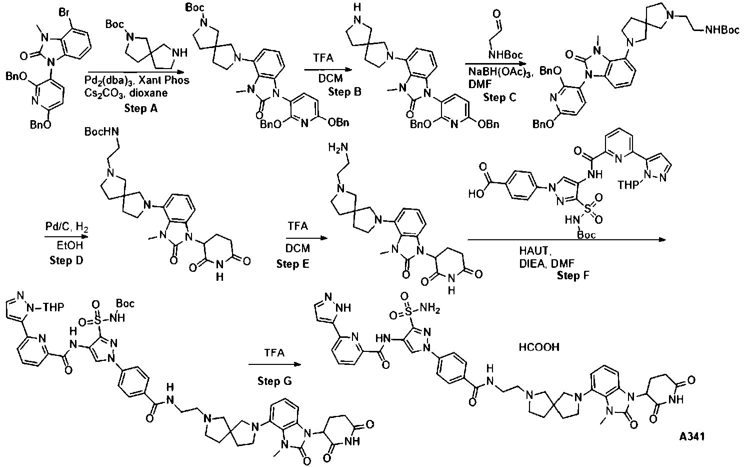

### Step A: Tert-butyl 7-(1-(2,6-bis(benzyloxy)pyridin-3-yl)-3-methyl-2-oxo-2,3-dihydro-1H-benzo[d]imidazol-4-yl)-2,7-diazaspiro[4.4]nonane-2-carboxylate

At rt, to a solution of 1-(2,6-bis(benzyloxy)pyridin-3-yl)-4-bromo-3-methyl-1,3-dihydro-2H-benzo[d]imidazol-2-one (500 mg, 0.97 mmol, 1.0 eq), tert-butyl 2,7-diazaspiro[4.4]nonane-2-carboxylate (329 mg, 1.5 mmol, 1.5 eq), and cesium carbonate (946 mg, 2.9 mmol, 3.0 eq) in dioxane (10 mL) were added Pd₂(dba)₃ (89 mg, 0.1 mmol, 0.1 eq) and Xant Phos (112 mg, 0.2 mmol, 0.2 eq). The mixture was stirred at 100°C under N₂ for 12 h. Upon completion, the mixture was cooled to rt and filtered, and the filter cake was washed with DCM. The filtrate was concentrated. The residue was purified by column chromatography to afford the title compound (yellow solid, 600 mg, yield 94%).

### Step B: 1-(2,6-Bis(benzyloxy)pyridin-3-yl)-3-methyl-4-(2,7-diazaspiro[4.4]nonan-2-yl)-1,3-dihydro-2H-benzo[d]imidazol-2-one

At rt, to a solution of tert-butyl 7-(1-(2,6-bis(benzyloxy)pyridin-3-yl)-3-methyl-2-oxo-2,3-dihydro-1H-benzo[d]imidazol-4-yl)-2,7-diazaspiro[4.4]nonane-2-carboxylate (300 mg, 0.5 mmol, 1.0 eq) in DCM (10 mL) was added TFA (3 mL). The mixture was stirred at rt under N₂ for 1 h, and then concentrated under reduced pressure to afford the title compound (yellow oil, 200 mg, crude).

### Step C: Tert-butyl (2-(7-(1-(2,6-bis(benzyloxy)pyridin-3-yl)-3-methyl-2-oxo-2,3-dihydro-1H-benzo[d]imidazol-4-yl)-2,7-diazaspiro[4.4]nonan-2-yl)ethyl)carbamate

At rt, to a solution of 1-(2,6-bis(benzyloxy)pyridin-3-yl)-3-methyl-4-(2,7-diazaspiro[4.4]nonan-2-yl)-1,3-dihydro-2H-benzo[d]imidazol-2-one (200 mg, 0.4 mmol, 1.0 eq) in DMF (5 mL) was added tert-butyl (2-oxoethyl)carbamate (135 mg, 0.8 mmol, 2.0 eq). The mixture was stirred at rt for 1 h. Then sodium triacetoxyborohydride (270 mg, 1.3 mmol, 3.0 eq) was added, and the reaction was stirred overnight. Upon completion, the reaction solution was basified with saturated aqueous NaHCO₃ and extracted with EtOAc. The organic phase was washed with saturated aqueous NaCl, dried, filtered, and concentrated. The residue was purified by column chromatography to afford the title compound (yellow solid, 150 mg, yield 58%).

### Step D: Tert-butyl (2-(7-(1-(2,6-dioxopiperidin-3-yl)-3-methyl-2-oxo-2,3-dihydro-1H-benzo[d]imidazol-4-yl)-2,7-diazaspiro[4.4]nonan-2-yl)ethyl)carbamate

At rt, to a solution of tert-butyl (2-(7-(1-(2,6-bis(benzyloxy)pyridin-3-yl)-3-methyl-2-oxo-2,3-dihydro-1H-benzo[d]imidazol-4-yl)-2,7-diazaspiro[4.4]nonan-2-yl)ethyl)carbamate (150 mg, 0.2 mmol, 1.0 eq) in ethanol (10 mL) was added 10% Pd/C (26 mg). The reaction solution was stirred under H₂ at 60°C overnight. Upon completion, the reaction solution was filtered, and the filtrate was concentrated to afford the title compound (yellow solid, 100 mg, yield 78%).

### Step E: 3-(4-(7-(2-Aminoethyl)-2,7-diazaspiro[4.4]nonan-2-yl)-3-methyl-2-oxo-2,3-dihydro-1H-benzo[d]imidazol-1-yl)piperidine-2,6-dione

At rt, to a solution of tert-butyl (2-(7-(1-(2,6-dioxopiperidin-3-yl)-3-methyl-2-oxo-2,3-dihydro-1H-benzo[d]imidazol-4-yl)-2,7-diazaspiro[4.4]nonan-2-yl)ethyl)carbamate (100 mg, 0.2 mmol, 1.0 eq) in DCM (3 mL) was added TFA (1 mL). The mixture was stirred at rt under N₂ for 2 h, and then concentrated under reduced pressure to afford the title compound (yellow oil, 70 mg, crude).

### Step F: Tert-butyl (((1-(4-((2-(7-(1-(2,6-dioxopiperidin-3-yl)-3-methyl-2-oxo-2,3-dihydro-1H-benzo[d]imidazol-4-yl)-2,7-diazaspiro[4.4]nonan-2-yl)ethyl)carbamoyl)phenyl)-4-(6-(1-(tetrahydro-2H-pyran-2-yl)-1H-pyrazol-5-yl)picolinamido)-1H-pyrazol-3-yl)sulfonyl)carbamate

At rt, to a solution of 3-(4-(7-(2-aminoethyl)-2,7-diazaspiro[4.4]nonan-2-yl)-3-methyl-2-oxo-2,3-dihydro-1H-benzo[d]imidazol-1-yl)piperidine-2,6-dione (70 mg, 0.2 mmol, 1.0 eq), 4-(3-(N-(tert-butoxycarbonyl)sulfamoyl)-4-(6-(1-(tetrahydro-2H-pyran-2-yl)-1H-pyrazol-5-yl)pyridinylamino)-1H-pyrazol-1-yl)benzoic acid (91 mg, 0.2 mmol, 1.0 eq), and DIEA (127 mg, 1.0 mmol, 5.0 eq) in DMF (5 mL) was added HATU (94 mg, 0.2 mmol, 1.0 eq). The mixture was stirred at rt for 5 h. Upon completion, the reaction solution was diluted in saturated aqueous NH₄Cl and extracted with EtOAc. The organic phase was washed with saturated aqueous NaCl, dried, filtered, and concentrated. The residue was purified by column chromatography to afford the title compound (yellow solid, 40 mg, yield 25%).

### Step G: N-(1-(4-((2-(7-(1-(2,6-Dioxopiperidin-3-yl)-3-methyl-2-oxo-2,3-dihydro-1H-benzo[d]imidazol-4-yl)-2,7-diazaspiro[4.4]nonan-2-yl)ethyl)carbamoyl)phenyl)-3-sulfamoyl-1H-pyrazol-4-yl)-6-(1H-pyrazol-5-yl)picolinamide formate

At rt, to a solution of tert-butyl ((1-(4-((2-(7-(1-(2,6-dioxopiperidin-3-yl)-3-methyl-2-oxo-2,3-dihydro-1H-benzo[d]imidazol-4-yl)-2,7-diazaspiro[4.4]nonan-2-yl)ethyl)carbamoyl)phenyl)-4-(6-(1-(tetrahydro-2H-pyran-2-yl)-1H-pyrazol-5-yl)picolinamido)-1H-pyrazol-3-yl)sulfonyl)carbamate (40 mg, 0.04 mmol, 1.0 eq) in DCM (3 mL) was added TFA (1 mL). The reaction solution was stirred at rt under N₂ for 1 h. Upon completion, the reaction solution was concentrated under reduced pressure, and the residue was purified by HPLC to afford the title compound (yellow solid, 11 mg, yield 31%). **¹H NMR** (400 MHz, DMSO-*d*₆) *δ* 13.20 (s, 1H), 11.33 (s, 1H), 11.08 (s, 1H), 9.19 (s, 1H), 8.58 (t, *J* = 5.4 Hz, 1H), 8.25 (d, *J* = 7.6 Hz, 1H), 8.17 (s, 0.6H), 8.14 (t, *J =* 7.7 Hz, 1H), 8.08 (d, *J =* 6.9 Hz, 3H), 8.03 (s, 4H), 7.94 (s, 1H), 7.22 (d, *J =* 2.1 Hz, 1H), 6.99-6.92 (m, 2H), 6.85 (d, *J =* 6.5 Hz, 1H), 5.35 (dd, *J =* 12.3, 4.5 Hz, 1H), 3.60 (s, 3H), 3.44-3.40 (m, 2H), 3.13-3.05 (m, 3H), 3.00-2.83 (m, 3H), 2.76-2.62 (m, 6H), 2.56 (d, *J =* 8.8 Hz, 1H), 2.03-1.81 (m, 5H). LCMS (ESI): m/z 862 [M+H]⁺.

### Preparation Example 356. Synthesis of Compound A343

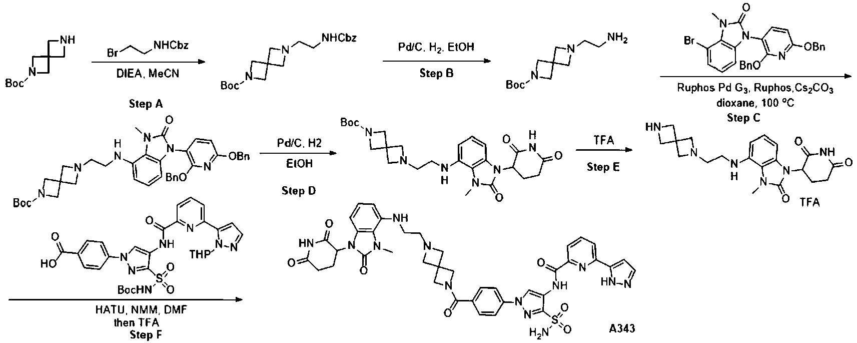

### Step A: Tert-butyl 6-(2-(((benzyloxy)carbonyl)amino)ethyl)-2,6-diazaspiro[3.3]heptane-2-carboxylate

At rt, to a solution of tert-butyl 2,6-diazaspiro[3.3]heptane-2-carboxylate (1 g, 5.0 mmol, 1.0 eq) in acetonitrile (10 mL) were added DIEA (2.7 mL, 15.0 mmol, 3.0 eq) and 2-bromoethyl benzyl carbamate (1.3 g, 5.0 mmol, 1.0 eq). The mixture was stirred at rt for 18 h. Upon completion, the mixture was diluted in water and extracted with DCM. The organic phase was washed sequentially with saturated aqueous NaCl, dried over anhydrous Na₂SO₄, filtered, and concentrated. The residue was purified by column chromatography to afford the title compound (yellow oil, 1.1 g, yield 58%).

### Step B: Tert-butyl 6-(2-aminoethyl)-2,6-diazaspiro[3.3]heptane-2-carboxylate

At rt, to a solution of tert-butyl 6-(2-(((benzyloxy)carbonyl)amino)ethyl)-2,6-diazaspiro[3.3]heptane-2-carboxylate (1.0 g, 2.7 mmol, 1.0 eq) in ethanol (10 mL) was added 10% Pd/C (100 mg). The reaction solution was purged with H₂ (×3), and then stirred under H₂ at 25°C for 2 h. Upon completion, the mixture was filtered to remove Pd/C, and the filtrate was concentrated to afford the title compound (yellow oil, 600 mg, yield 93%).

### Step C: Tert-butyl 6-(2-(1-(2,6-bis(benzyloxy)pyridin-3-yl)-3-methyl-2-oxo-2,3-dihydro-1H-benzo[d]imidazol-4-yl)amino)ethyl)-2,6-diazaspiro[3.3]heptane-2-carboxylate

At rt, to a solution of 1-(2,6-bis(benzyloxy)pyridin-3-yl)-4-bromo-3-methyl-1,3-dihydro-2H-benzo[d]imidazol-2-one (1.7 g, 3.3 mmol, 1.0 eq) and tert-butyl 6-(2-aminoethyl)-2,6-diazaspiro[3.3]heptane-2-carboxylate (800 mg, 3.3 mmol, 1.0 eq) in dioxane (15 mL) were added cesium carbonate (3.2 g, 9.9 mmol, 3.0 eq), RuPhos Pd G₃ (278 mg, 0.33 mmol, 0.1 eq), and RuPhos (155 mg, 0.33 mmol, 0.1 eq). The reaction solution was stirred at 100°C under N₂ for 15 h. Upon completion, the reaction solution was diluted in water and extracted with DCM. The organic phase was washed sequentially with saturated aqueous NaCl, dried over anhydrous Na₂SO₄, filtered, and concentrated. The residue was purified by column chromatography to afford the title compound (yellow solid, 900 mg, yield 40%).

### Step D: Tert-butyl 6-(2-(1-(2,6-dioxopiperidin-3-yl)-3-methyl-2-oxo-2,3-dihydro-1H-benzo[d]imidazol-4-yl)amino)ethyl)-2,6-diazaspiro[3.3]heptane-2-carboxylate

At rt, to a solution of tert-butyl 6-(2-(1-(2,6-bis(benzyloxy)pyridin-3-yl)-3-methyl-2-oxo-2,3-dihydro-1H-benzo[d]imidazol-4-yl)amino)ethyl)-2,6-diazaspiro[3.3]heptane-2-carboxylate (800 mg, 1.2 mmol, 1.0 eq) in ethanol (15 mL) were added 10% Pd/C (100 mg) and 10% Pd(OH)₂/C (100 mg). The reaction solution was purged with H₂ (×3), and then stirred under H₂ at 50°C for 18 h. Upon completion, the mixture was filtered, and the filtrate was concentrated to afford the title compound (yellow solid, 400 mg, yield 68%).

### Step E: 3-(4-(2-(2,6-Diazaspiro[3.3]heptan-2-yl)ethyl)amino)-3-methyl-2-oxo-2,3-dihydro-1H-benzo[d]imidazol-1-yl)piperidine-2,6-dione trifluoroacetate

Tert-Butyl 6-(2-(1-(2,6-dioxopiperidin-3-yl)-3-methyl-2-oxo-2,3-dihydro-1H-benzo[d]imidazol-4-yl)amino)ethyl)-2,6-diazaspiro[3.3]heptane-2-carboxylate (400 mg, 0.8 mmol, 1.0 eq) was dissolved in TFA (5 mL), and the reaction solution was stirred at rt for 1 h. Upon completion, the reaction solution was concentrated to afford the title compound (yellow solid, 300 mg, crude).

### Step F: N-(1-(4-(6-((1-(2,6-Dioxopiperidin-3-yl)-3-methyl-2-oxo-2,3-dihydro-1H-benzo[d]imidazol-4-yl)amino)ethyl)-2,6-diazaspiro[3.3]heptane-2-carbonyl)phenyl)-3-sulfamoyl-1H-pyrazol-4-yl)-6-(1H-pyrazol-5-yl)picolinamide

At rt, to a solution of 4-(3-(N-(tert-butoxycarbonyl)sulfamoyl)-4-(6-(1-(tetrahydro-2H-pyran-2-yl)-1H-pyrazol-5-yl)picolinamido)-1H-pyrazol-1-yl)benzoic acid (128 mg, 0.2 mmol, 1.0 eq) in DMF (5 mL) were added N-methylmorpholine (0.5 mL) and HATU (114 mg, 0.3 mmol, 1.5 eq). The reaction solution was stirred at rt for 1 h. Then a solution of 3-(4-(2-(2,6-diazaspiro[3.3]heptan-2-yl)ethyl)amino)-3-methyl-2-oxo-2,3-dihydro-1H-benzo[d]imidazol-1-yl)piperidine-2,6-dione trifluoroacetate (102 mg, crude) in DMF (1 mL) was added to the reaction solution. The reaction solution was stirred at rt for 1 h. Upon completion, the reaction solution was diluted in water and extracted with DCM. The organic phase was washed with saturated aqueous NaCl, dried over anhydrous Na₂SO₄, filtered, and concentrated. The residue was dissolved in TFA (3 mL), and the mixture was stirred for 2 h. The reaction solution was concentrated, and then the residue was purified by HPLC to afford the title compound (pink solid, 13.9 mg, yield 21%). **¹H NMR** (400 MHz, DMSO-*d*₆) *δ* 13.21 (s, 1H), 11.32 (s, 1H), 11.06 (s, 1H), 9.19 (s, 1H), 8.27-8.19 (m, 2H), 8.10 (dd, *J =* 18.2, 7.2 Hz, 3H), 8.02 (d, *J =* 8.8 Hz, 2H), 7.94 (s, 1H), 7.84 (d, *J =* 8.8 Hz, 2H), 7.22 (d, *J* = 2.0 Hz, 1H), 6.87 (t, *J* = 8.0 Hz, 1H), 6.50 (d, *J =* 7.6 Hz, 1H), 6.41 (d, *J =* 8.0 Hz, 1H), 5.36-5.22 (m, 1H), 5.06 (s, 1H), 4.47 (s, 2H), 4.16 (s, 2H), 3.61 (s, 3H), 3.37 (s, 4H), 3.05-2.99 (m, 2H), 2.92-2.84 (m, 1H), 2.71-2.61 (m, 4H), 2.02-1.91 (m, 1H). LCMS (ESI): m/z 834 [M+H]⁺.

### Preparation Example 357. Synthesis of Compound A348

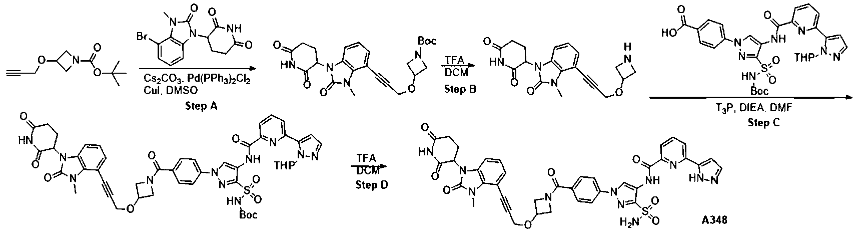

### Step A: Tert-butyl 3-((3-(1-(2,6-dioxopiperidin-3-yl)-3-methyl-2-oxo-2,3-dihydro-1H-benzo[d]imidazol-4-yl)prop-2-yn-1-yl)oxy)azetidine-1-carboxylate

Under N₂, to a solution of tert-butyl 3-(prop-2-yn-1-yloxy)azetidine-1-carboxylate (424 mg, 2.01 mmol, 2.0 eq) and 3-(4-bromo-3-methyl-2-oxo-2,3-dihydro-1H-benzo[d]imidazol-1-yl)piperidine-2,6-dione (340 mg, 1.01 mmol, 1.0 eq) in dimethyl sulfoxide (6 mL) were added cesium carbonate (980 mg, 3.0 mmol, 3.0 eq), cuprous iodide (38 mg, 0.20 mmol, 0.2 eq), and bis(triphenylphosphine)palladium(II) dichloride (141 mg, 0.20 mmol, 0.2 eq). The reaction solution was purged with N₂ (×3), and the reaction was stirred at 90°C under N₂ for 16 h. Upon completion, the reaction solution was diluted in water and extracted with EtOAc. The organic phase was washed with saturated aqueous NaCl, dried over anhydrous Na₂SO₄, and concentrated under reduced pressure. The residue was purified by column chromatography (eluent: petroleum ether/ethyl acetate, gradient: 0%-70% ethyl acetate) to afford the title compound (yellow solid, 80 mg, yield 17%).

### Step B: 3-(4-(3-(Azetidin-3-yloxy)prop-1-yn-1-yl)-3-methyl-2-oxo-2,3-dihydro-1H-benzo[d]imidazol-1-yl)piperidine-2,6-dione

In an ice-water bath, to a solution of tert-butyl 3-((3-(1-(2,6-dioxopiperidin-3-yl)-3-methyl-2-oxo-2,3-dihydro-1H-benzo[d]imidazol-4-yl)prop-2-yn-1-yl)oxy)azetidine-1-carboxylate (80 mg, 0.17 mmol, 1.0 eq) in DCM (1 mL) was added TFA (1.0 mL). The reaction solution was stirred at rt for 1 h. Upon completion, the mixture was concentrated under reduced pressure to afford the title compound (yellow oil, 60 mg, crude), which was used directly in the next reaction without purification.

### Step C: Tert-butyl ((1-(4-(3-(1-(2,6-dioxopiperidin-3-yl)-3-methyl-2-oxo-2,3-dihydro-1H-benzo[d]imidazol-4-yl)prop-2-yn-1-yl)oxy)azetidine-1-carbonyl)phenyl)-4-(6-(1-(tetrahydro-2H-pyran-2-yl)-1H-pyrazol-5-yl)picolinamido)-1H-pyrazol-3-yl)sulfonyl)carbamate

At rt, to a solution of 4-(3-(N-(tert-butoxycarbonyl)sulfamoyl)-4-(6-(1-(tetrahydro-2H-pyran-2-yl)-1H-pyrazol-5-yl)picolinamido)-1H-pyrazol-1-yl)benzoic acid (104 mg, 0.16 mmol, 1.0 eq) and 3-(4-(3-(azetidin-3-yloxy)prop-1-yn-1-yl)-3-methyl-2-oxo-2,3-dihydro-1H-benzo[d]imidazol-1-yl)piperidine-2,6-dione (60 mg, 0.16 mmol, 1.0 eq) in DMF (2 mL) were added DIEA (105 mg, 0.82 mmol, 5.0 eq) and T₃P (156 mg, 0.24 mmol, 1.5 eq, 50% w/w in DMF). The reaction solution was stirred at rt for 4 h. Upon completion, the reaction solution was slowly poured into ice water and extracted with EtOAc twice. The organic phases were combined, washed with aqueous NaCl, dried over anhydrous Na₂SO₄, and concentrated under reduced pressure to afford the title compound (yellow oil, 150 mg, crude).

### Step D: N-(1-(4-(3-(3-(1-(2,6-Dioxopiperidin-3-yl)-3-methyl-2-oxo-2,3-dihydro-1H-benzo[d]imidazol-4-yl)prop-2-yn-1-yl)oxy)azetidine-1-carbonyl)phenyl)-3-sulfamoyl-1H-pyrazol-4-yl)-6-(1H-pyrazol-5-yl)picolinamide

In an ice-water bath, to a solution of tert-butyl ((1-(4-(3-(1-(2,6-dioxopiperidin-3-yl)-3-methyl-2-oxo-2,3-dihydro-1H-benzo[d]imidazol-4-yl)prop-2-yn-1-yl)oxy)azetidine-1-carbonyl)phenyl)-4-(6-(1-(tetrahydro-2H-pyran-2-yl)-1H-pyrazol-5-yl)picolinamido)-1H-pyrazol-3-yl)sulfonyl)carbamate (150 mg, 0.15 mmol, 1.0 eq) in DCM (3 mL) was added TFA (1 mL). The reaction solution was stirred at rt for 1 h. Upon completion, the mixture was concentrated under reduced pressure. The residue was purified by HPLC (C18, 15%-95% acetonitrile in water containing 0.1% FA) to afford the title compound (white solid, 26.0 mg, 21% yield over three steps). LCMS (ESI): m/z 804 [M+H]⁺. **¹H NMR** (400 MHz, DMSO-*d*₆) *δ* 13.20 (s, 1H), 11.32 (s, 1H), 11.12 (s, 1H), 9.18 (s, 1H), 8.25 (d, *J* = 7.8 Hz, 1H), 8.15-8.11 (m, 1H), 8.08-8.07 (m, 3H), 8.02 (d, *J =* 8.7 Hz, 2H), 7.94 (s, 1H), 7.85 (d, *J =* 8.7 Hz, 2H), 7.22 (d, *J =* 2.0 Hz, 1H), 7.19 (d, *J =* 7.8 Hz, 1H), 7.14 (d, *J =* 7.3 Hz, 1H), 7.04 (t, *J =* 7.9 Hz, 1H), 5.43-5.37 (m, 1H), 4.66-4.57 (m, 2H), 4.53 (s, 2H), 4.39-4.29 (m, 2H), 4.04-3.94 (m, 1H), 3.64 (s, 3H), 2.92-2.84 (m, 1H), 2.75-2.61 (m, 2H), 2.04-2.00 (m, 1H).

### Preparation Example 358. Synthesis of Compound A349

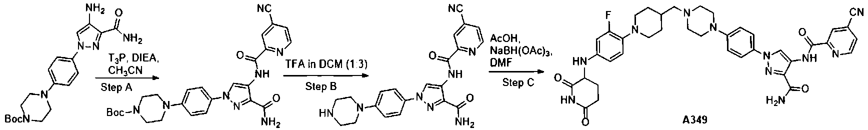

### Step A: Tert-butyl 4-(4-(3-carbamoyl-4-((4-cyanopicolinamido)-1H-pyrazol-1-yl)phenyl)piperazine-1-carboxylate

To a solution of tert-butyl 4-(4-(4-amino-3-carbamoyl-1H-pyrazol-1-yl)phenyl)piperazine-1-carboxylate (500 mg, 1.3 mmol, 1.0 eq) in acetonitrile (20 mL) were added 4-cyanopicolinic acid (210 mg, 1.4 mmol, 1.1 eq), DIEA (0.7 mL, 3.9 mmol, 3.0 eq), and 50% T₃P in EtOAc (1.6 mL, 2.6 mmol, 2.0 eq). The mixture was stirred at rt for 16 h. The reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 500 mg, yield 75%).

### Step B: N-(3-Carbamoyl-1-(4-(piperazin-1-yl)phenyl)-1H-pyrazol-4-yl)-4-cyanopicolinamide

At rt, tert-butyl 4-(4-(3-carbamoyl-4-((4-cyanopicolinamido)-1H-pyrazol-1-yl)phenyl)piperazine-1-carboxylate (500 mg, 0.9 mmol, 1.0 eq) was added to a mixed solution of TFA (2 mL) and DCM (6 mL). The mixture was stirred for 2 h, and then concentrated to afford the title compound (white solid, 400 mg, crude).

### Step C: N-(3-Carbamoyl-1-(4-(4-((1-(4-((2,6-dioxopiperidin-3-yl)amino)-2-fluorophenyl)piperidin-4-yl)methyl)piperazin-1-yl)phenyl)-1H-pyrazol-4-yl)-4-cyanopicolinamide

At rt, to a solution of N-(3-carbamoyl-1-(4-(piperazin-1-yl)phenyl)-1H-pyrazol-4-yl)-4-cyanopicolinamide (100 mg, 0.24 mmol, 1.0 eq) in DMF (3 mL) were added AcOH (1 mL) and 1-(4-(2,6-dioxopiperidin-3-yl)amino)-2-fluorophenyl)piperidine-4-carbaldehyde (88 mg, 0.26 mmol, 1.1 eq). The mixture was stirred at rt for 16 h, and then sodium triacetoxyborohydride (151 mg, 0.71 mmol, 3.0 eq) was added. The mixture was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (yellow solid, 66.4 mg, yield 38%). LCMS (ESI): m/z 734 [M+H]⁺. **¹H NMR** (400 MHz, DMSO-*d₆*)*δ* 11.67 (s, 1H), 10.77 (s, 1H), 9.01 (d, *J =* 5.2 Hz, 1H), 8.89 (s, 1H), 8.51 (s, 1H), 8.19 (dd, *J =* 5.2, 1.6 Hz, 1H), 7.95 (s, 1H), 7.79 (d, *J =* 9.2 Hz, 2H), 7.68 (s, 1H), 7.08 (d, *J =* 9.2 Hz, 2H), 6.84 (t, *J* = 9.2 Hz, 1H), 6.50 (dd, *J =* 15.2, 2.4 Hz, 1H), 6.45-6.35 (m, 1H), 5.78 (d, *J =* 7.6 Hz, 1H), 4.36-4.15 (m, 1H), 3.27-3.05 (m, 6H), 2.73-2.70 (m, 1H), 2.56-2.52 (m, 7H), 2.30-2.25 (m, 2H), 2.15-2.02 (m, 1H), 1.93-1.73 (m, 3H), 1.65-1.62 (m, 1H), 1.28-1.25 (m, 2H).

### Preparation Example 359. Synthesis of Compound A350

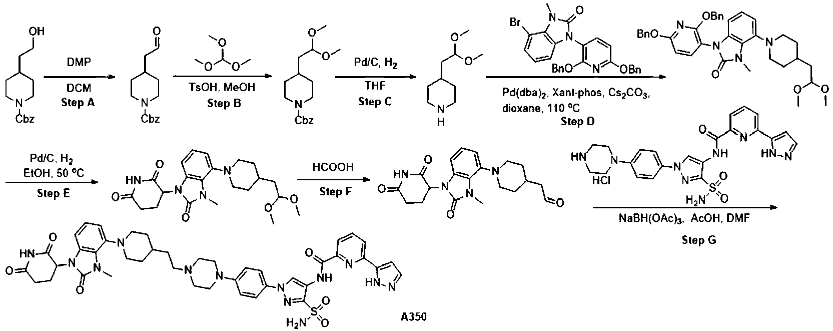

### Step A: Benzyl 4-(2-oxoethyl)piperidine-1-carboxylate

In an ice bath, Dess-Martin periodinane (6.3 g, 14.8 mmol, 1.5 eq) was added portionwise to a solution of benzyl 4-(2-hydroxyethyl)piperidine-1-carboxylate (2.6 g, 9.9 mmol, 1.0 eq) in DCM (30 mL). The reaction solution was stirred at rt for 2 h. Upon completion, the reaction solution was diluted in water and extracted with DCM. The organic phase was washed with aqueous NaCl, dried, filtered, and concentrated. The residue was purified by column chromatography to afford the title compound (colorless oil, 2.4 g, yield 93%).

### Step B: Benzyl 4-(2,2-dimethoxyethyl)piperidine-1-carboxylate

In an ice bath, to a solution of benzyl 4-(2-oxoethyl)piperidine-1-carboxylate (2.4 g, 9.2 mmol, 1.0 eq) and trimethyl orthoformate (5.0 mL, 46 mmol, 5.0 eq) in MeOH (20 mL) was added p-toluenesulfonic acid (0.2 g, 0.92 mmol, 0.1 eq). The reaction solution was stirred at rt under N₂ for 3 h. Upon completion, the reaction solution was diluted in water and extracted with DCM. The organic phase was washed with aqueous NaCl, dried, filtered, and concentrated. The residue was purified by column chromatography to afford the title compound (colorless oil, 2.6 g, yield 92%).

### Step C: 4-(2,2-Dimethoxyethyl)piperidine

At rt, to a solution of benzyl 4-(2,2-dimethoxyethyl)piperidine-1-carboxylate (2.6 g, 8.5 mmol, 1.0 eq) in THF (30 mL) was added 10% Pd/C (0.18 g). The reaction was purged with H₂ (×3), and then the reaction solution was stirred under H₂ at rt for 2 h. Upon completion, the reaction solution was filtered, and the filtrate was concentrated to afford the title compound (colorless oil, 1.2 g, yield 82%).

### Step D: 1-(2,6-Bis(benzyloxy)pyridin-3-yl)-4-(4-(2,2-dimethoxyethyl)piperidin-1-yl)-3-methyl-1,3-dihydro-2H-benzo[d]imidazol-2-one

At rt, to a solution of 4-(2,2-dimethoxyethyl)piperidine (300 mg, 1.7 mmol, 1.0 eq), 1-(2,6-bis(benzyloxy)pyridin-3-yl)-4-bromo-3-methyl-1,3-dihydro-2H-benzo[d]imidazol-2-one (894 mg, 1.7 mmol, 1.0 eq), and cesium carbonate (1.1 g, 3.5 mmol, 2.0 eq) in dioxane (15 mL) were added Xant Phos (200 mg, 0.34 mmol, 0.2 eq) and Pd₂(dba)₃ (158 mg, 0.17 mmol, 0.1 eq). The reaction solution was stirred at 110°C under N₂ for 18 h. Upon completion, the reaction solution was cooled to rt, diluted in saturated aqueous NH₄Cl, and extracted with EtOAc. The organic phase was washed with saturated aqueous NaCl, dried, filtered, and concentrated. The residue was purified by column chromatography to afford the title compound (yellow solid, 500 mg, yield 47%).

### Step E: 3-(4-(4-(2,2-Dimethoxyethyl)piperidin-1-yl)-3-methyl-2-oxo-2,3-dihydro-1H-benzo[d]imidazol-1-yl)piperidine-2,6-dione

At rt, to a solution of 1-(2,6-bis(benzyloxy)pyridin-3-yl)-4-(4-(2,2-dimethoxyethyl)piperidin-1-yl)-3-methyl-1,3-dihydro-2H-benzo[d]imidazol-2-one (300 mg, 0.5 mmol, 1.0 eq) in ethanol (30 mL) were added 10% Pd/C (18 mg) and 10% Pd(OH)₂/C (21 mg). The mixture was purged with H₂ (×3) and then stirred under H₂ at 60°C overnight. Upon completion, the mixture was filtered, and the filtrate was concentrated to afford the title compound (yellow solid, 200 mg, yield 94%).

### Step F: 2-(1-(1-(2,6-Dioxopiperidin-3-yl)-3-methyl-2-oxo-2,3-dihydro-1H-benzo[d]imidazol-4-yl)piperidin-4-yl)acetaldehyde

3-(4-(4-(2,2-dimethoxyethyl)piperidin-1-yl)-3-methyl-2-oxo-2,3-dihydro-1H-benzo[d]imidazol-1-yl)piperidine-2,6-dione (200 mg, 0.47 mmol, 1.0 eq) was added to formic acid (5 mL), and the mixture was stirred at rt for 1 h. Upon completion, the reaction solution was concentrated to afford the title compound as a yellow oil (170 mg, crude).

### Step G: N-(1-(4-(4-(2-(1-(1-(2,6-Dioxopiperidin-3-yl)-3-methyl-2-oxo-2,3-dihydro-1H-benzo[d]imidazol-4-yl)piperidin-4-yl)ethyl)piperazin-1-yl)phenyl)-3-sulfamoyl-1H-pyrazol-4-yl)-6-(1H-pyrazol-5-yl)picolinamide

At rt, to a solution of 2-(1-(1-(2,6-dioxopiperidin-3-yl)-3-methyl-2-oxo-2,3-dihydro-1H-benzo[d]imidazol-4-yl)piperidin-4-yl)acetaldehyde (80 mg, crude) and N-(1-(4-(piperazin-1-yl)phenyl)-3-sulfamoyl-1H-pyrazol-4-yl)-6-(1H-pyrazol-5-yl)picolinamide hydrochloride (111 mg, 0.21 mmol, 1.0 eq) in DMF (5 mL) was added AcOH (0.1 mL). The reaction solution was stirred at rt overnight. Then sodium triacetoxyborohydride (132 mg, 0.62 mmol, 1.0 eq) was added to the reaction solution, and the reaction was stirred for 2 h. Upon completion, the reaction solution was diluted in saturated aqueous NH₄Cl and extracted with a mixed solvent of ethyl acetate/tetrahydrofuran (1/1). The organic phase was dried, filtered, and concentrated. The residue was purified by HPLC to afford the title compound (white solid, 18.1 mg, yield 10%). **¹H NMR** (400 MHz, DMSO-*d*₆) *δ* 13.20 (s, 1H), 11.31 (s, 1H), 11.09 (s, 1H), 8.95 (s, 1H), 8.24 (d, *J* = 7.6 Hz, 1H), 8.16-8.09 (m, 2H), 8.07 (d, *J =* 7.5 Hz, 1H), 7.96 (d, *J =* 11.1 Hz, 2H), 7.74 (d, *J =* 9.0 Hz, 2H), 7.22 (d, *J* = 2.0 Hz, 1H), 7.11 (d, *J* = 9.2 Hz, 2H), 6.99-6.83 (m, 3H), 5.39-5.28 (m, 1H), 3.63 (s, 3H), 3.27-3.04 (m, 8H), 2.92-2.82 (m, 1H), 2.74-2.56 (m, 8H), 2.00 (d, *J =* 5.3 Hz, 1H), 1.81 (d, *J =* 10.1 Hz, 2H), 1.57-1.37 (m, 5H). LCMS (ESI): m/z 862 [M+H]⁺.

### Preparation Example 360. Synthesis of Compound A351

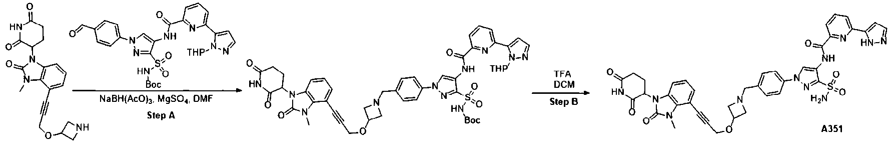

### Step A: Tert-butyl ((1-(4-((3-((3-(1-(2,6-dioxopiperidin-3-yl)-3-methyl-2-oxo-2,3-dihydro-1H-benzo[d]imidazol-4-yl)prop-2-yn-1-yl)oxy)azetidin-1-yl)methyl)phenyl)-4-(6-(1-(tetrahydro-2H-pyran-2-yl)-1H-pyrazol-5-yl)picolinamido)-1H-pyrazol-3-yl)sulfonyl)carbamate

Under N₂, to a solution of tert-butyl (1-(4-formylphenyl)-4-(6-(1-(tetrahydro-2H-pyran-2-yl)-1H-pyrazol-5-yl)picolinamido)-1H-pyrazol-3-yl)sulfonyl)carbamate (270 mg, 0.43 mmol, 1.0 eq) and 3-(4-(3-(azetidin-3-yloxy)prop-1-yn-1-yl)-3-methyl-2-oxo-2,3-dihydro-1H-benzo[d]imidazol-1-yl)piperidine-2,6-dione (160 mg, 0.43 mmol, 1.0 eq) in DMF (5 mL) were added sodium triacetoxyborohydride (277 mg, 1.3 mmol, 3.0 eq) and magnesium sulfate (160 mg). The reaction solution was stirred at rt for 2 h. Upon completion, the reaction solution was slowly poured into water and filtered. The filter cake was collected and slurried with petroleum ether/ethyl acetate (10% ethyl acetate) to afford the title compound (yellow solid, 300 mg, yield 71%).

### Step B: N-(1-(4-((3-((3-(1-(2,6-dioxopiperidin-3-yl)-3-methyl-2-oxo-2,3-dihydro-1H-benzo[d]imidazol-4-yl)prop-2-yn-1-yl)oxy)azetidin-1-yl)methyl)phenyl)-3-sulfamoyl-1H-pyrazol-4-yl)-6-(1H-pyrazol-5-yl)picolinamide

At rt, to a solution of tert-butyl ((1-(4-((3-((3-(1-(2,6-dioxopiperidin-3-yl)-3-methyl-2-oxo-2,3-dihydro-1H-benzo[d]imidazol-4-yl)prop-2-yn-1-yl)oxy)azetidin-1-yl)methyl)phenyl)-4-(6-(1-(tetrahydro-2H-pyran-2-yl)-1H-pyrazol-5-yl)picolinamido)-1H-pyrazol-3-yl)sulfonyl)carbamate (300 mg, 0.31 mmol, 1.0 eq) in DCM (2 mL) was added TFA (2 mL). The reaction solution was stirred at rt for 30 min. Upon completion, the mixture was concentrated under reduced pressure, and the residue was purified by preparative HPLC (C₁₈, 25%-95% acetonitrile in water containing 0.1% aqueous ammonia) to afford the title compound (white solid, 25 mg, yield 10%). LCMS (ESI): m/z 790 [M+H]⁺. **¹H NMR** (400 MHz, DMSO-*d*₆) *δ* 13.20 (s, 1H), 11.32 (s, 1H), 11.11 (s, 1H), 9.07 (s, 1H), 8.25-8.23 (m, 1H), 8.14-8.10 (m, 1H), 8.08-8.06 (m, 1H), 8.03-8.00 (m, 2H), 7.96-7.93 (m, 1H), 7.85 (d, *J =* 7.8 Hz, 2H), 7.46 (d, *J =* 8.0 Hz, 2H), 7.22-7.16 (m, 2H), 7.12-7.10 (m, 1H), 7.05-7.01 (m, 1H), 5.42-5.37 (m, 1H), 4.42 (s, 2H), 4.33-4.30 (m, 1H), 3.66 (s, 2H), 3.63 (s, 3H), 3.59-3.56 (m, 2H), 3.02-2.99 (m, 2H), 2.90-2.85 (m, 1H), 2.68-2.65 (m, 1H), 2.04-1.98 (m, 2H).

### Preparation Example 361. Synthesis of Compound A352

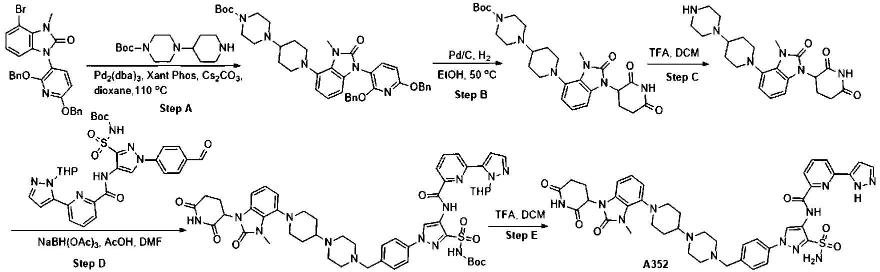

### Step A: Tert-butyl 4-(1-(1-(2,6-bis(benzyloxy)pyridin-3-yl)-3-methyl-2-oxo-2,3-dihydro-1H-benzo[d]imidazol-4-yl)piperidin-4-yl)piperazine-1-carboxylate

At rt, to a solution of 1-(2,6-bis(benzyloxy)pyridin-3-yl)-4-bromo-3-methyl-1,3-dihydro-2H-benzo[d]imidazol-2-one (500 mg, 0.97 mmol, 1.0 eq), tert-butyl 4-(piperidin-4-yl)piperazine-1-carboxylate (391 mg, 1.45 mmol, 1.5 eq), and cesium carbonate (631 mg, 1.94 mmol, 2.0 eq) in dioxane (10 mL) were added Pd₂(dba)₃ (88 mg, 0.097 mmol, 0.1 eq) and Xant Phos (112 mg, 0.19 mmol, 0.2 eq). The reaction solution was stirred at 110°C under N₂ for 18 h. Upon completion, the reaction solution was cooled to rt, diluted in saturated aqueous NH₄Cl, and extracted with EtOAc. The organic phase was washed with saturated aqueous NaCl, dried, filtered, and concentrated. The residue was purified by column chromatography to afford the title compound (yellow solid, 200 mg, yield 29%).

### Step B: Tert-butyl 4-(1-(1-(2,6-dioxopiperidin-3-yl)-3-methyl-2-oxo-2,3-dihydro-1H-benzo[d]imidazol-4-yl)piperidin-4-yl)piperazine-1-carboxylate

At rt, to a solution of tert-butyl 4-(1-(1-(2,6-bis(benzyloxy)pyridin-3-yl)-3-methyl-2-oxo-2,3-dihydro-1H-benzo[d]imidazol-4-yl)piperidin-4-yl)piperazine-1-carboxylate (200 mg, 0.28 mmol, 1.0 eq) in ethanol (20 mL) were added 10% Pd/C (6 mg) and 10% Pd(OH)₂/C (8 mg). The reaction solution was purged with H₂ (×3) and then stirred under H₂ at 60°C overnight. Upon completion, the reaction solution was filtered, and the filtrate was concentrated to afford the title compound (yellow solid, 100 mg, yield 67%).

### Step C: 3-(3-Methyl-2-oxo-4-(4-(piperazin-1-yl)piperidin-1-yl)-2,3-dihydro-1H-benzo[d]imidazol-1-yl)piperidine-2,6-dione

At rt, to a solution of tert-butyl 4-(1-(1-(2,6-dioxopiperidin-3-yl)-3-methyl-2-oxo-2,3-dihydro-1H-benzo[d]imidazol-4-yl)piperidin-4-yl)piperazine-1-carboxylate (100 mg, 0.19 mmol, 1.0 eq) in DCM (3 mL) was added TFA (1.5 mL). The reaction solution was stirred at rt for 1 h. The reaction solution was concentrated to afford the title compound (yellow solid, 80 mg, crude).

### Step D: Tert-butyl (1-(4-(4-(1-(1-(1-(2,6-dioxopiperidin-3-yl)-3-methyl-2-oxo-2,3-dihydro-1H-benzo[d]imidazol-4-yl)piperidin-4-yl)piperazin-1-yl)methyl)phenyl)-4-(6-(1-tetrahydro-2H-pyran-2-yl)-1H-pyrazol-5-yl)picolinamido)sulfamoyl)carbamate

At rt, to a solution of 3-(3-methyl-2-oxo-4-(4-(piperazin-1-yl)piperidin-1-yl)-2,3-dihydro-1H-benzo[d]imidazol-1-yl)piperidine-2,6-dione (80 mg, crude) and tert-butyl (1-(4-formylphenyl)-4-(6-(1-(tetrahydro-2H-pyran-2-yl)-1H-pyrazol-5-yl)picolinamido)-1H-pyrazol-3-yl)sulfonyl)carbamate (116 mg, 0.19 mmol, 1.0 eq) in DMF (5 mL) was added AcOH (0.1 mL). The reaction solution was stirred at rt overnight. Then, sodium triacetoxyborohydride (119 mg, 0.563 mmol, 3.0 eq) was added, and the reaction was stirred for an additional 2 h. Upon completion, the reaction solution was diluted in saturated aqueous NH₄Cl and extracted with a mixed solvent of ethyl acetate/tetrahydrofuran (1/1). The organic phase was dried, filtered, and concentrated. The residue was purified by preparative thin layer chromatography to afford the target compound (yellow solid, 50 mg, yield 26%).

### Step E: N-(1-(4-(4-(1-(1-(2,6-Dioxopiperidin-3-yl)-3-methyl-2-oxo-2,3-dihydro-1H-benzo[d]imidazol-4-yl)piperidin-4-yl)piperazin-1-yl)methyl)phenyl)-3-sulfamoyl-1H-pyrazol-4-yl)-6-(1H-pyrazol-5-yl)picolinamide

At rt, to a solution of tert-butyl (1-(4-(4-(1-(1-(1-(2,6-dioxopiperidin-3-yl)-3-methyl-2-oxo-2,3-dihydro-1H-benzo[d]imidazol-4-yl)piperidin-4-yl)piperazin-1-yl)methyl)phenyl)-4-(6-(1-tetrahydro-2H-pyran-2-yl)-1H-pyrazol-5-yl)picolinamido)sulfamoyl)carbamate (50 mg, 0.048 mmol, 1.0 eq) in DCM (2 mL) was added TFA (1 mL). The reaction solution was stirred at rt for 1 h. The reaction solution was concentrated, and then the residue was purified by HPLC to afford the title compound (white solid, 12.2 mg, yield 29%). **¹H NMR** (400 MHz, DMSO-*d*₆) *δ* 13.20 (s, 1H), 11.33 (s, 1H), 11.09 (s, 1H), 9.08 (s, 1H), 8.24 (d, *J* = 7.5 Hz, 1H), 8.16-8.07 (m, 2H), 8.01-7.83 (m, 4H), 7.50 (d, *J* = 8.5 Hz, 2H), 7.23 (d, *J* = 1.6 Hz, 1H), 6.97 (t, *J* = 7.9 Hz, 1H), 6.91-6.84 (m, 2H), 5.38-5.29 (m, 1H), 3.62 (s, 3H), 3.53 (s, 2H), 3.30 (s, 2H), 3.14 (d, *J* = 11.0 Hz, 2H), 2.92-2.83 (m, 1H), 2.74-2.63 (m, 4H), 2.60-2.54 (m, 3H), 2.46-2.39 (m, 3H), 2.33-2.28 (m, 1H), 2.02-1.96 (m, 1H), 1.89 (d, *J* = 10.3 Hz, 2H), 1.68-1.53 (m, 2H). LCMS (ESI): m/z 848 [M+H]⁺.

### Preparation Example 362. Synthesis of Compound A353

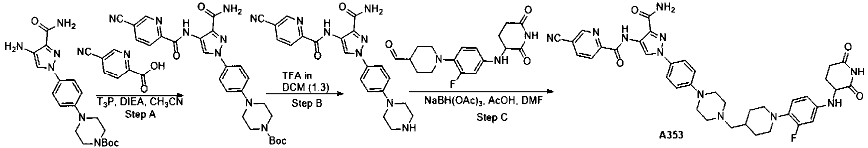

### Step A: Tert-butyl 4-(4-(3-carbamoyl-4-((5-cyanopicolinamido)-1H-pyrazol-1-yl)phenyl)piperazine-1-carboxylate

At rt, to a solution of tert-butyl 4-(4-(4-amino-3-carbamoyl-1H-pyrazol-1-yl)phenyl)piperazine-1-carboxylate (500 mg, 1.3 mmol, 1.0 eq) in acetonitrile (20 mL) were added 5-cyanopicolinic acid (210 mg, 1.4 mmol, 1.1 eq), DIEA (0.7 mL, 3.9 mmol, 3.0 eq), and 50% T₃P in EA (1.6 g, 2.6 mmol, 2.0 eq). The mixture was stirred at rt for 16 h. The reaction mixture was filtered, and the filter cake was dried to afford the title compound (white solid, 500 mg, yield 75%).

### Step B: N-(3-Carbamoyl-1-(4-(piperazin-1-yl)phenyl)-1H-pyrazol-4-yl)-5-cyanopicolinamide

At rt, tert-butyl 4-(4-(3-carbamoyl-4-((5-cyanopicolinamido)-1H-pyrazol-1-yl)phenyl)piperazine-1-carboxylate (500 mg, 0.9 mmol, 1.0 eq) was added to a mixture of TFA (2 mL) and DCM (6 mL). The mixture was stirred at rt for 2 h. The reaction mixture was concentrated to afford the title compound (white solid, 400 mg, crude), which was used directly without purification.

### Step C: N-(3-Carbamoyl-1-(4-(4-((1-(4-((2,6-dioxopiperidin-3-yl)amino)-2-fluorophenyl)piperidin-4-yl)methyl)piperazin-1-yl)phenyl)-1H-pyrazol-4-yl)-5-cyanopicolinamide

At rt, to a solution of N-(3-carbamoyl-1-(4-(piperazin-1-yl)phenyl)-1H-pyrazol-4-yl)-5-cyanopicolinamide (100 mg, 0.24 mmol, 1.0 eq) in DMF (3 mL) were added AcOH (1 mL) and 1-(4-((2,6-dioxopiperidin-3-yl)amino)-2-fluorophenyl)piperidine-4-carbaldehyde (88 mg, 0.26 mmol, 1.1 eq). The mixture was stirred at rt for 16 h, and then sodium triacetoxyborohydride (151 mg, 0.71 mmol, 3.0 eq) was added. The mixture was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (yellow solid, 25.0 mg, yield 14%). LCMS (ESI): m/z 734 [M+H]⁺. **¹H NMR** (400 MHz, DMSO-*d₆*) δ 10.84 (s, 1H), 10.79 (s, 1H), 9.27 (dd, *J* = 6.0, 2.0 Hz, 2H), 8.88 (s, 1H), 8.74 (s, 1H), 8.02 (s, 1H), 7.88 (d, *J* = 8.8 Hz, 2H), 7.81 (s, 1H), 7.18 (d, *J* = 9.2 Hz, 2H), 6.90 (s, 1H), 6.53 (d, *J =* 14.8 Hz, 1H), 6.45 (d, *J* = 8.4 Hz, 1H), 4.28 (dd, *J =* 11.2, 4.8 Hz, 1H), 3.94 (d, *J =* 10.8 Hz, 2H), 3.64 (s, 2H), 3.17-3.12 (m, 8H), 2.76-2.64 (m, 3H), 2.61-2.55 (m, 2H), 2.13-2.05 (m, 1H), 1.98 (s, 1H), 1.86 (d, *J =* 12.0 Hz, 3H), 1.43 (d, *J* = 11.6 Hz, 2H).

### Preparation Example 363. Synthesis of Compound A354

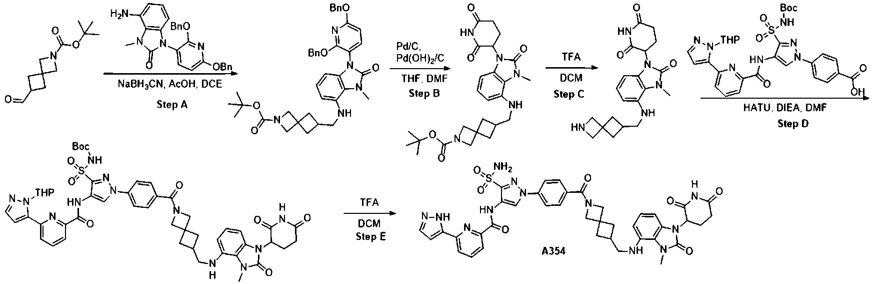

### Step A: Tert-butyl 6-(((1-(2,6-bis(benzyloxy)pyridin-3-yl)-3-methyl-2-oxo-2,3-dihydro-1H-benzo[d]imidazol-4-yl)amino)methyl)-2-azaspiro[3.3]heptane-2-carboxylate

At rt, to a solution of tert-butyl 6-formyl-2-azaspiro[3.3]heptane-2-carboxylate (100 mg, 0.444 mmol, 1.0 eq) in 1,2-dichloroethane (3 mL) were added 4-amino-1-(2,6-bis(benzyloxy)pyridin-3-yl)-3-methyl-1,3-dihydro-2H-benzimidazol-2-one (201 mg, 0.444 mmol, 1.0 eq), AcOH (0.003 mL, 0.044 mmol, 0.1 eq), and sodium cyanoborohydride (83.68 mg, 1.332 mmol, 3.0 eq). The reaction solution was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 155 mg, yield 53%).

### Step B: Tert-butyl 6-(((1-(2,6-dioxopiperidin-3-yl)-3-methyl-2-oxo-2,3-dihydro-1H-benzo[d]imidazol-4-yl)amino)methyl)-2-azaspiro[3.3]heptane-2-carboxylate

Under N₂, to a solution of tert-butyl 6-(((1-(2,6-bis(benzyloxy)pyridin-3-yl)-3-methyl-2-oxo-2,3-dihydro-1H-benzo[d]imidazol-4-yl)amino)methyl)-2-azaspiro[3.3]heptane-2-carboxylate (150 mg, 0.227 mmol, 1.0 eq) in anhydrous THF (3 mL) were added DMF (0.6 mL), 10% Pd/C (48 mg), and 10% Pd(OH)₂/C (32 mg). The mixture was purged with H₂ (using a hydrogen balloon) (×3), and the reaction solution was stirred under a hydrogen balloon atmosphere at rt for 16 h. Upon completion, the reaction solution was filtered through Celite, and the filtrate was concentrated under reduced pressure to afford the title compound (brown oil, 100 mg, yield 91%).

### Step C: 3-(4-(((2-Azaspiro[3.3]heptan-6-yl)methyl)amino)-3-methyl-2-oxo-2,3-dihydro-1H-benzimidazol-1-yl)piperidine-2,6-dione

At 0°C, to a solution of tert-butyl 6-(((1-(2,6-dioxopiperidin-3-yl)-3-methyl-2-oxo-2,3-dihydro-1H-benzo[d]imidazol-4-yl)amino)methyl)-2-azaspiro[3.3]heptane-2-carboxylate (100 mg, 0.207 mmol, 1.0 eq) in anhydrous DCM (3 mL) was added TFA (1 mL). The reaction solution was stirred at rt for 1 h. Upon completion, the reaction solution was concentrated under reduced pressure to afford the title compound (brown oil, 100 mg, crude).

### Step D: Tert-butyl ((1-(4-(6-(((1-(2,6-dioxopiperidin-3-yl)-3-methyl-2-oxo-2,3-dihydro-1H-benzo[d]imidazol-4-yl)amino)methyl)-2-azaspiro[3.3]heptane-2-carbonyl)phenyl)-4-(6-(1-(tetrahydro-2H-pyran-2-yl)-1H-pyrazol-5-yl)picolinamido)-1H-pyrazol-3-yl)sulfonyl)carbamate

At rt, to a solution of 3-(4-(((2-azaspiro[3.3]heptan-6-yl)methyl)amino)-3-methyl-2-oxo-2,3-dihydro-1H-benzimidazol-1-yl)piperidine-2,6-dione (100 mg, crude) and 4-(3-(N-(tert-butoxycarbonyl)sulfamoyl)-4-(6-(1-(tetrahydro-2H-pyran-2-yl)-1H-pyrazol-5-yl)pyridinylamino)-1H-pyrazol-1-yl)benzoic acid (144 mg, 0.261 mmol, 1.0 eq) in DMF (3 mL) were added DIEA (101 mg, 0.782 mmol, 3.0 eq) and HATU (149 mg, 0.391 mmol, 1.5 eq). The reaction solution was stirred at rt under N₂ for 1 h. Upon completion, the reaction solution was diluted in EtOAc, washed sequentially with water and saturated aqueous NaCl, dried over anhydrous Na₂SO₄, filtered, and concentrated under reduced pressure to afford the title compound (brown oil, 70 mg, yield 29%).

### Step E: N-(1-(4-(6-(((1-(2,6-Dioxopiperidin-3-yl)-3-methyl-2-oxo-2,3-dihydro-1H-benzo[d]imidazol-4-yl)amino)methyl)-2-azaspiro[3.3]heptane-2-carbonyl)phenyl)-3-sulfamoyl-1H-pyrazol-4-yl)-6-(1H-pyrazol-5-yl)picolinamide

At 0°C, to a solution of tert-butyl ((1-(4-(6-(((1-(2,6-dioxopiperidin-3-yl)-3-methyl-2-oxo-2,3-dihydro-1H-benzo[d]imidazol-4-yl)amino)methyl)-2-azaspiro[3.3]heptane-2-carbonyl)phenyl)-4-(6-(1-(tetrahydro-2H-pyran-2-yl)-1H-pyrazol-5-yl)picolinamido)-1H-pyrazol-3-yl)sulfonyl)carbamate (70 mg, 0.076 mmol, 1.0 eq) in anhydrous DCM (3 mL) was added TFA (1 mL). The reaction solution was stirred at rt for 1 h. Upon completion, the reaction solution was concentrated under reduced pressure. The residue was purified by preparative HPLC to afford the title compound (pink solid, 20.8 mg, yield 33%). LCMS (ESI): m/z 819 [M+H]⁺. **¹H NMR** (400 MHz, DMSO-*d*₆) *δ* 11.32 (s, 1H), 11.05 (s, 1H), 9.18 (s, 1H), 8.24 (d, *J* = 6.9 Hz, 1H), 8.14 (t, *J* = 7.7 Hz, 1H), 8.08 (d, *J* = 5.3 Hz, 3H), 8.01 (d, *J* = 8.5 Hz, 2H), 7.93 (d, *J* = 1.8 Hz, 1H), 7.83 (d, *J =* 8.3 Hz, 2H), 7.22 (d, *J* = 2.2 Hz, 1H), 6.86 (t, *J* = 8.0 Hz, 1H), 6.51 (d, *J* = 7.8 Hz, 1H), 6.42 (d, *J =* 8.2 Hz, 1H), 5.32-5.26 (m, 1H), 4.41 (s, 1H), 4.33 (s, 1H), 4.11 (s, 1H), 4.03 (s, 1H), 3.60 (s, 3H), 3.10-3.05 (m, 2H), 2.92 (s, 1H), 2.71-2.64 (m, 1H), 2.61 *(d, J =* 17.0 Hz, 1H), 2.35 *(d, J =* 11.5 Hz, 2H), 2.04-1.92 (m, 3H), 1.41 (d, *J =* 12.3 Hz, 1H), 1.25-1.19 (m, 1H).

### Preparation Example 364. Synthesis of Compound A355

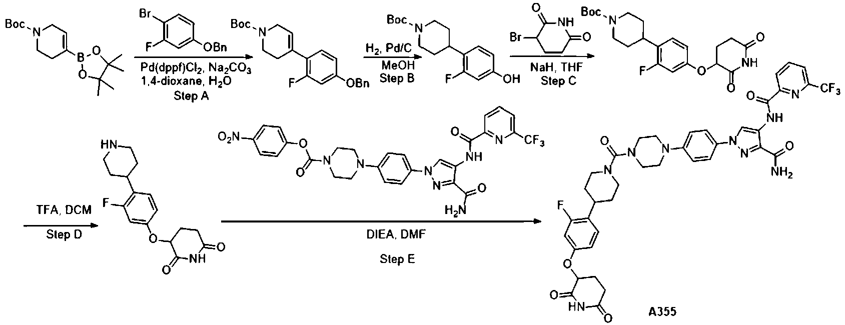

### Step A: Tert-butyl 4-(4-(benzyloxy)-2-fluorophenyl)-3,6-dihydropyridine-1(2H)-carboxylate

Under N₂ at rt, to a solution of tert-butyl 4-(4,4,5,5-tetramethyl-1,3,2-dioxaborolan-2-yl)-3,6-dihydropyridine-1(2H)-carboxylate (3 g, 9.7 mmol, 1.0 eq) in 1,4-dioxane (30 mL) and water (5 mL) were added 4-(benzyloxy)-1-bromo-2-fluorobenzene (5.4 g, 19.4 mmol, 2.0 eq), sodium carbonate (2 g, 19.4 mmol, 2.0 eq), and Pd(dppf)Cl₂ (350 mg, 0.48 mmol, 0.05 eq). The reaction solution was purged with N₂ (×3) and then stirred at 80°C under N₂ for 4 h. Upon completion, the reaction solution was diluted in EtOAc, washed sequentially with water and saturated aqueous NaCl, dried over anhydrous Na₂SO₄, and concentrated under reduced pressure. The residue was purified by column chromatography (eluent: petroleum ether/ethyl acetate, gradient: 0%-30% ethyl acetate) to afford the title compound (white solid, 3 g, yield 80%).

### Step B: Tert-butyl 4-(2-fluoro-4-hydroxyphenyl)piperidine-1-carboxylate

Under N₂ at rt, to a solution of tert-butyl 4-(4-(benzyloxy)-2-fluorophenyl)-3,6-dihydropyridine-1(2H)-carboxylate (3 g, 7.8 mmol, 1.0 eq) in MeOH (30 mL) was added 10% palladium on carbon (300 mg, 21.56 mmol, 0.1 eq). The reaction solution was purged with H₂ (×3) and then stirred under H₂ at rt for 6 h. Upon completion, the reaction solution was filtered and concentrated under reduced pressure to afford the title compound (white solid, 2 g, yield 87%).

### Step C: Tert-butyl 4-(4-((2,6-dioxopiperidin-3-yl)oxy)-2-fluorophenyl)piperidine-1-carboxylate

At rt, tert-butyl 4-(2-fluoro-4-hydroxyphenyl)piperidine-1-carboxylate (1.3 g, 4.40 mmol, 1.0 eq) was dissolved in THF (20 mL). Sodium hydride (60%, 0.26 g, 6.60 mmol, 1.5 eq) was added slowly, and the mixture was stirred at rt for 30 min. After 3-bromopiperidine-2,6-dione (1.69 g, 8.80 mmol, 2.0 eq) was added to the reaction solution, the reaction solution was stirred at 60°C overnight. The reaction solution was poured into saturated aqueous NH₄Cl and extracted with EtOAc (×3). The organic phase was washed with saturated aqueous NaCl (×2), dried over anhydrous Na₂SO₄, and concentrated under reduced pressure. The residue was purified by column chromatography (eluent: petroleum ether/ethyl acetate, gradient: 0%-60% ethyl acetate) to afford the title compound (white solid, 0.6 g, yield 40%).

### Step D: 3-(3-Fluoro-4-(piperidin-4-yl)phenoxy)piperidine-2,6-dione

Tert-butyl 4-(4-((2,6-dioxopiperidin-3-yl)oxy)-2-fluorophenyl)piperidine-1-carboxylate (300 mg, 0.74 mmol, 1.0 eq) was dissolved in a mixed solution of DCM and TFA (3:1), and the mixture was stirred at rt for 2 h. The mixture was concentrated under reduced pressure to afford the title compound (yellow oil, 200 mg, crude).

### Step E: N-(3-Carbamoyl-1-(4-(4-(4-(4-((2,6-dioxopiperidin-3-yl)oxy)-2-fluorophenyl)piperidine-1-carbonyl)piperazin-1-yl)phenyl)-1H-pyrazol-4-yl)-6-(trifluoromethyl)picolinamide

3-(3-fluoro-4-(piperidin-4-yl)phenoxy)piperidine-2,6-dione (200 mg, 0.65 mmol, 1.0 eq) and 4-nitrophenyl 4-(4-(3-carbamoyl-4-(6-(trifluoromethyl)picolinamido)-1H-pyrazol-1-yl)phenyl)piperazine-1-carboxylate (407 mg, 0.65 mmol, 1.0 eq) were dissolved in DMF (5 mL), and then DIEA (0.3 mL, 1.96 mmol, 2.0 eq) and DMAP (79 mg, 0.65 mmol, 1.0 eq) were added. The reaction was stirred at 90°C overnight. The mixture was purified directly by preparative HPLC and lyophilized to afford the target compound (yellow solid, 48 mg, yield 9%). LCMS (ESI): m/z 792 (M+H)⁺. **¹H NMR** (400 MHz, DMSO-*d₆*) *δ* 11.79 (s, 1H), 10.94 (s, 1H), 8.90 (s, 1H), 8.47-8.37 (m, 2H), 8.21 (dd, *J =* 7.6, 1.0 Hz, 1H), 7.96 (s, 1H), 7.82 (d, *J* = 9.2 Hz, 2H), 7.71 (s, 1H), 7.24 (t, *J* = 8.8 Hz, 1H), 7.11 (d, *J* = 9.2 Hz, 2H), 6.85 (ddd, *J =* 11.0, 10.6, 2.5 Hz, 2H), 5.22 (dd, *J =* 11.0, 5.2 Hz, 1H), 3.78 (d, *J* = 12.8 Hz, 2H), 3.50 (d, *J* = 3.6 Hz, 1H), 3.33 (d, *J =* 4.8 Hz, 4H), 3.24 (s, 4H), 2.90 (t, *J =* 11.6 Hz, 2H), 2.71-2.57 (m, 2H), 2.22-2.10 (m, 2H), 1.72 (d, *J =* 10.8 Hz, 2H), 1.25 (dd, *J =* 10.8, 4.8 Hz, 2H).

### Preparation Example 365. Synthesis of Compound A356

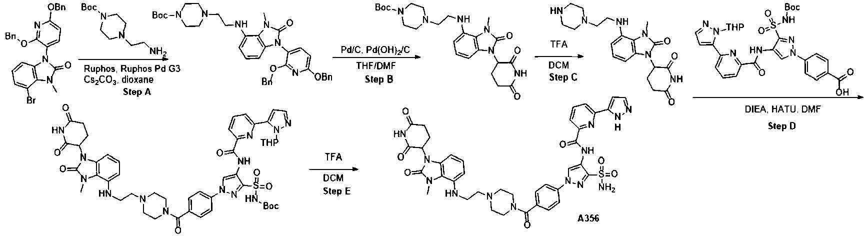

### Step A: Tert-butyl 4-(2-((1-(2,6-bis(benzyloxy)pyridin-3-yl)-3-methyl-2-oxo-2,3-dihydro-1H-benzo[d]imidazol-4-yl)amino)ethyl)piperazine-1-carboxylate

At rt, to a mixture of tert-butyl 4-(2-aminoethyl)piperazine-1-carboxylate (457 mg, 1.74 mmol, 1.5 eq), 1-(2,6-bis(benzyloxy)pyridin-3-yl)-4-bromo-3-methyl-1,3-dihydro-2H-benzo[d]imidazol-2-one (600 mg, 1.16 mmol, 1.0 eq), and cesium carbonate (1136 mg, 3.49 mmol, 3.0 eq) in anhydrous 1,4-dioxane (10 mL) were added RuPhos (108 mg, 0.23 mmol, 0.2 eq) and RuPhos Pd G₃ (97 mg, 0.12 mmol, 0.1 eq). The reaction solution was stirred at 100°C under N₂ for 18 h. Upon completion, the reaction solution was concentrated under reduced pressure, dried, diluted in EtOAc, washed sequentially with water and saturated aqueous NaCl, and concentrated under reduced pressure. The residue was purified by column chromatography to afford the title compound (yellow solid, 350 mg, yield 47%).

### Step B: Tert-butyl 4-(2-((1-(2,6-dioxopiperidin-3-yl)-3-methyl-2-oxo-2,3-dihydro-1H-benzo[d]imidazol-4-yl)amino)ethyl)piperazine-1-carboxylate

At rt, to a mixed solution of tert-butyl 4-(2-((1-(2,6-bis(benzyloxy)pyridin-3-yl)-3-methyl-2-oxo-2,3-dihydro-1H-benzo[d]imidazol-4-yl)amino)ethyl)piperazine-1-carboxylate (300 mg, 0.45 mmol, 1.0 eq) in THF (30 mL) and DMF (7 mL) were added 10% palladium hydroxide on carbon (70 mg) and 10% palladium on carbon (70 mg). The reaction was stirred under a hydrogen balloon atmosphere at rt for 18 h. LCMS indicated that the reaction was incomplete. The reaction solution was filtered through Celite, and additional 10% palladium hydroxide on carbon (70 mg) and 10% palladium on carbon (70 mg) were added. The reaction was stirred under a hydrogen balloon at rt for 5 h. The reaction solution was filtered through Celite, and the filter cake was concentrated under reduced pressure and dried. The solid was slurried with diethyl ether, filtered, and dried under reduced pressure. The residue was purified by column chromatography to afford the title compound (white solid, 55 mg, yield 25%).

### Step C: 3-(3-Methyl-2-oxo-4-((2-(piperazin-1-yl)ethyl)amino)-2,3-dihydro-1H-benzo[d]imidazol-1-yl)piperidine-2,6-dione

At rt, tert-butyl 4-(2-((1-(2,6-dioxopiperidin-3-yl)-3-methyl-2-oxo-2,3-dihydro-1H-benzo[d]imidazol-4-yl)amino)ethyl)piperazine-1-carboxylate (55 mg, 0.113 mmol, 1.0 eq), TFA (1 mL), and DCM (3 mL) were added to a reaction flask. The reaction solution was stirred at rt for 3 h. Upon completion, the reaction solution was concentrated under reduced pressure and dried. The solid was slurried with diethyl ether, filtered, and dried under reduced pressure to afford the title compound (white solid, 40 mg, crude).

### Step D: Tert-butyl (1-(4-(4-(2-((1-(2,6-dioxopiperidin-3-yl)-3-methyl-2-oxo-2,3-dihydro-1H-benzo[d]imidazol-4-yl)amino)ethyl)piperazine-1-carbonyl)phenyl)-4-(6-(1-(tetrahydro-2H-pyran-2-yl)-1H-pyrazol-5-yl)picolinamide)-1H-pyrazol-3-yl)sulfonyl)carbamate

At rt, to a solution of 3-(3-methyl-2-oxo-4-(2-(piperazin-1-yl)ethyl)amino)-2,3-dihydro-1H-benzo[d]imidazol-1-yl)piperidine-2,6-dione (40 mg, 0.104 mmol, crude), 4-(3-(N-(tert-butoxycarbonyl)sulfamoyl)-4-(6-(1-(tetrahydro-2H-pyran-2-yl)-1H-pyrazol-5-yl)pyridinylamino)-1H-pyrazol-1-yl)benzoic acid (57 mg, 0.104 mmol, 1.0 eq), and DIEA (40 mg, 0.311 mmol, 3.0 eq) in DMF (3 mL) was added HATU (59 mg, 0.155 mmol, 1.5 eq). The reaction was stirred at rt for 1 h. Upon completion, the reaction solution was concentrated under reduced pressure and dried. The solid was slurried with 10% NaOH solution for 1 min, then diluted in DCM, filtered, and dried under reduced pressure to afford the title compound (white solid, 60 mg, yield 63%).

### Step E: N-(1-(4-(4-(2-((1-(2,6-Dioxopiperidin-3-yl)-3-methyl-2-oxo-2,3-dihydro-1H-benzo[d]imidazol-4-yl)amino)ethyl)piperazine-1-carbonyl)phenyl)-3-sulfamoyl-1H-pyrazol-4-yl)-6-(1H-pyrazol-5-yl)picolinamide

At rt, tert-butyl (1-(4-(4-(2-((1-(2,6-dioxopiperidin-3-yl)-3-methyl-2-oxo-2,3-dihydro-1H-benzo[d]imidazol-4-yl)amino)ethyl)piperazine-1-carbonyl)phenyl)-4-(6-(1-(tetrahydro-2H-pyran-2-yl)-1H-pyrazol-5-yl)picolinamido)-1H-pyrazol-3-yl)sulfonyl)carbamate (60 mg, 0.065 mmol, 1.0 eq), TFA (1 mL), and DCM (3 mL) were added to a reaction flask. The mixture was stirred at rt for 1 h. Upon completion, the reaction solution was concentrated under reduced pressure and dried. The residue was purified by HPLC to afford the title compound (white solid, 1.9 mg, yield 4%). LCMS (ESI): m/z 822.6 [M+H]⁺. **¹H NMR** (400 MHz, DMSO-*d*₆) *δ* 13.22 (s, 1H), 11.34 (s, 1H), 11.07 (s, 1H), 9.17 (s, 1H), 8.26 (d, *J* = 8.1 Hz, 1H), 8.14 (s, 1H), 8.09 (d, *J* = 7.4 Hz, 2H), 8.02 (d, *J* = 8.6 Hz, 2H), 7.96 (s, 1H), 7.62 (d, *J* = 8.4 Hz, 2H), 7.23 (s, 1H), 6.88 (t, *J =* 8.1 Hz, 1H), 6.51 (d, *J =* 7.2 Hz, 1H), 6.44 (d, *J =* 7.9 Hz, 1H), 5.35-5.25 (m, 1H), 5.16 (s, 1H), 3.65 (s, 3H), 3.20-3.18 (m, 4H), 2.94-2.92 (m, 1H), 2.91-2.85 (m, 2H), 2.72-2.65 (m, 4H), 2.64-2.59 (m, 2H), 2.36-2.32 (m, 2H), 2.04-1.91 (m, 2H).

### Preparation Example 366. Synthesis of Compound A357

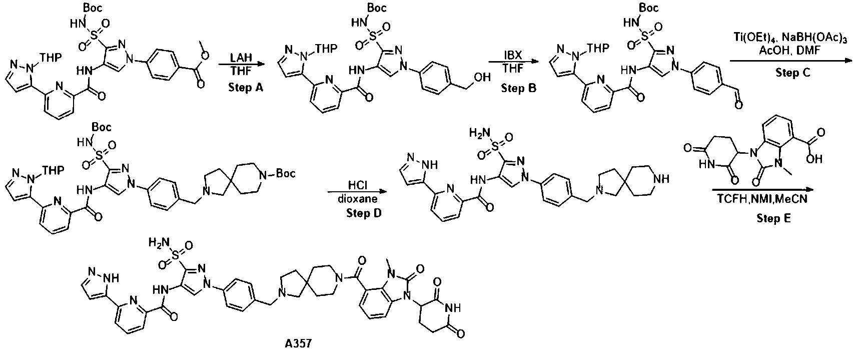

### Step A: Tert-butyl ((1-(4-(hydroxymethyl)phenyl)-4-(6-(1-(tetrahydro-2H-pyran-2-yl)-1H-pyrazol-5-yl)pyridinylamino)-1H-pyrazol-3-yl)sulfonyl)carbamate

In an ice-water bath, LiAlH₄ (150 mg, 3.84 mmol, 5.0 eq) was added portionwise to a solution of methyl 4-(3-(N-(tert-butoxycarbonyl)sulfamoyl)-4-(6-(1-(tetrahydro-2H-pyran-2-yl)-1H-pyrazol-5-yl)pyridinylamino)-1H-pyrazol-1-yl)benzoate (500 mg, 0.76 mmol, 1.0 eq) in THF (30 mL). After the addition, the reaction solution was stirred at 25°C under N₂ for 16 h. Upon completion, the reaction solution was quenched in an ice-water bath by successive addition of water (0.15 mL), 15% NaOH solution (0.15 mL, 15% wt), and water (0.45 mL). The mixture was diluted in EtOAc, dried over anhydrous Na₂SO₄, filtered, and concentrated under reduced pressure. The residue was purified by column chromatography (eluent: DCM/MeOH, gradient: 0%-10% MeOH) to afford the title compound (yellow solid, 450 mg, yield 94%).

### Step B: Tert-butyl ((1-(4-formylphenyl)-4-(6-(1-(tetrahydro-2H-pyran-2-yl)-1H-pyrazol-5-yl)pyridinylamino)-1H-pyrazol-3-yl)sulfonyl)carbamate

In an ice-water bath, IBX (560 mg, 2.0 mmol, 3.1 eq) was added to a solution of tert-butyl ((1-(4-(hydroxymethyl)phenyl)-4-(6-(1-(tetrahydro-2H-pyran-2-yl)-1H-pyrazol-5-yl)pyridinylamino)-1H-pyrazol-3-yl)sulfonyl)carbamate (400 mg, 0.64 mmol, 1.0 eq) in DMSO (10 mL). The reaction solution was stirred at 90°C overnight. Upon completion, the reaction solution was slowly poured into an ice-water cooled saturated aqueous NaHCO₃. The layers were separated, and the aqueous phase was extracted with DCM. The organic phases were combined, washed with saturated aqueous NaCl, dried over anhydrous Na₂SO₄, and concentrated under reduced pressure. The residue was purified by trituration at rt to afford the title compound (yellow solid, 350 mg, yield 88%).

### Step C: Tert-butyl 2-(4-(3-(N-(tert-butoxycarbonyl)sulfamoyl)-4-(6-(1-(tetrahydro-2H-pyran-2-yl)-1H-pyrazol-5-yl)pyridinylamino)-1H-pyrazol-1-yl)benzyl)-2,8-diazaspiro[4.5]decane-8-carboxylate

At rt, to a solution of tert-butyl ((1-(4-formylphenyl)-4-(6-(1-(tetrahydro-2H-pyran-2-yl)-1H-pyrazol-5-yl)pyridinylamino)-1H-pyrazol-3-yl)sulfonyl)carbamate (310 mg, 0.5 mmol, 1.0 eq) in DMF (2 mL) were added tert-butyl 2,8-diazaspiro[4.5]decane-8-carboxylate (144 mg, 0.6 mmol, 1.0 eq) and Ti(OEt)₄ (0.05 mL). The reaction solution was stirred at rt for 15 min. NaBH(OAc)₃ (232 mg, 1.5 mmol, 3.0 eq) was then added, and the reaction solution was stirred at rt for an additional 0.5 h. Upon completion, the reaction solution was poured into ice water and extracted with EtOAc twice. The organic phases were combined, washed with saturated aqueous NaCl, dried over anhydrous Na₂SO₄, and concentrated under reduced pressure. The residue was purified by column chromatography (eluent: DCM/MeOH, gradient: 0%-10% MeOH) to afford the title compound (yellow oil, 400 mg, yield 95%).

### Step D: N-(1-(4-((2,8-Diazaspiro[4.5]decan-2-yl)methyl)phenyl)-3-sulfamoyl-1H-pyrazol-4-yl)-6-(1H-pyrazol-5-yl)picolinamide

In an ice-water bath, tert-butyl 2-(4-(3-(N-(tert-butoxycarbonyl)sulfamoyl)-4-(6-(1-(tetrahydro-2H-pyran-2-yl)-1H-pyrazol-5-yl)pyridinylamino)-1H-pyrazol-1-yl)benzyl)-2,8-diazaspiro[4.5]decane-8-carboxylate (350 mg, 0.41 mmol, 1.0 eq) was added to a 4 M hydrogen chloride-dioxane solution (3 mL). The reaction solution was stirred at 0°C for 1 h. Upon completion, the reaction solution was added dropwise to an ice-water cooled 5% aqueous NaHCO₃. The layers were separated, and the aqueous phase was extracted with DCM. The organic phase was washed with saturated aqueous NaCl, dried over anhydrous Na₂SO₄, and concentrated under reduced pressure to afford the title compound (yellow oil, 160 mg, crude).

### Step E: N-(1-(4-((8-(1-(2,6-Dioxopiperidin-3-yl)-3-methyl-2-oxo-2,3-dihydro-1H-benzo[d]imidazole-4-carbonyl)-2,8-diazaspiro[4.5]decan-2-yl)methyl)phenyl)-3-sulfamoyl-1H-pyrazol-4-yl)-6-(1H-pyrazol-5-yl)picolinamide

At rt, to a solution of 1-(2,6-dioxopiperidin-3-yl)-3-methyl-2-oxo-2,3-dihydro-1H-benzo[d]imidazole-4-carboxylic acid (27 mg, 0.09 mmol, 1.0 eq) in acetonitrile (1 mL) were sequentially added N-(1-(4-((2,8-diazaspiro[4.5]decan-2-yl)methyl)phenyl)-3-sulfamoyl-1H-pyrazol-4-yl)-6-(1H-pyrazol-5-yl)picolinamide (50 mg, crude), TCFH (37 mg, 0.13 mmol, 1.4 eq), and NMI (1.0 mL). The reaction solution was stirred at rt for 3 h. Upon completion, the reaction solution was diluted in DCM, washed sequentially with water and saturated aqueous NaCl, dried over anhydrous Na₂SO₄, and concentrated under reduced pressure. The residue was purified by preparative HPLC (C₁₈, 10%-50% acetonitrile in water containing 0.1% FA to afford the title compound (white solid, 6.6 mg, yield 9%). LCMS (ESI): m/z 847.4 [M+H]⁺. **¹H NMR** (400 MHz, DMSO-*d*₆) *δ* 13.21 (s, 1H), 11.33 (s, 1H), 11.13 (s, 1H), 9.08 (s, 1H), 8.32-8.19 (m, 1H), 8.18-8.10 (m, 1H), 8.09-8.06 (m, 1H), 8.04-7.98 (m, 1H), 7.97-7.92 (m, 1H), 7.89-7.81 (m, 2H), 7.54-7.43 (m, 2H), 7.26-7.15 (m, 2H), 7.12-7.04 (m, 1H), 6.97-6.90 (m, 1H), 5.49-5.33 (m, 1H), 3.73-3.60 (m, 3H), 2.97-2.83 (m, 3H), 2.79-2.54 (m, 6H), 2.41-2.29 (m, 3H), 2.10-1.95 (m, 2H), 1.73-1.37 (m, 7H).

### Preparation Example 367. Synthesis of Compound A358

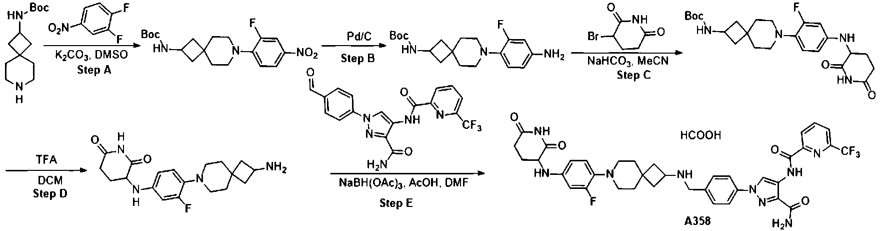

### Step A: Tert-butyl (7-(2-fluoro-4-nitrophenyl)-7-azaspiro[3.5]nonan-2-yl)carbamate

At rt, to a solution of tert-butyl (7-azaspiro[3.5]nonan-2-yl)carbamate (2.1 mL, 24.98 mmol, 1.0 eq) in dimethyl sulfoxide (50 mL) were added potassium carbonate (861 mg, 6.24 mmol, 3.0 eq) and 1,2-difluoro-4-nitrobenzene (397 mg, 2.5 mmol, 1.2 eq). The reaction solution was stirred at 60°C under N₂ for 4 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 2.7 g, yield 91%).

### Step B: Tert-butyl (7-(4-amino-2-fluorophenyl)-7-azaspiro[3.5]nonan-2-yl)carbamate

At rt, to a solution of tert-butyl (7-(2-fluoro-4-nitrophenyl)-7-azaspiro[3.5]nonan-2-yl)carbamate (500 mg, 1.52 mmol, 1.0 eq) in ethanol was added 10% Pd/C (300 mg). The reaction solution was purged with H₂ (×3), and then reacted under H₂ for 14 h. The reaction solution was filtered through Celite and concentrated to afford the title compound (gray solid, 410 mg, yield 91%).

### Step C: Tert-butyl (7-(4-((2,6-dioxopiperidin-3-yl)amino)-2-fluorophenyl)-7-azaspiro[3.5]nonan-2-yl)carbamate

At rt, to a solution of tert-butyl (7-(4-amino-2-fluorophenyl)-7-azaspiro[3.5]nonan-2-yl)carbamate (450 mg, 24.98 mmol, 1.0 eq) in acetonitrile (5 ml) were added NaHCO₃ (541 mg, 6.45 mmol, 5.0 eq) and 3-bromo-2,6-piperidinedione (495 mg, 2.58 mmol, 2.0 eq). The reaction solution was stirred at 80°C under N₂ for 14 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (gray solid, 350 mg, yield 59%).

### Step D: 3-((4-(2-Amino-7-azaspiro[3.5]nonan-7-yl)-3-fluorophenyl)amino)piperidine-2,6-dione

At 0°C, to a solution of tert-butyl (7-(4-((2,6-dioxopiperidin-3-yl)amino)-2-fluorophenyl)-7-azaspiro[3.5]nonan-2-yl)carbamate (120 mg, 0.26 mmol, 1.0 eq) in DCM (3 mL) was added TFA (1 mL). The reaction solution was stirred at 0°C under N₂ for 30 min. The reaction solution was concentrated to afford the title compound (brown oil, 93 mg, crude).

### Step E: N-(3-Carbamoyl-1-(4-(((7-(4-((2,6-dioxopiperidin-3-yl)amino)-2-fluorophenyl)-7-azaspiro[3.5]nonan-2-yl)amino)methyl)phenyl)-1H-pyrazol-4-yl)-6-(trifluoromethyl)picolinamide formate

At rt, to a solution of 3-((4-(2-amino-7-azaspiro[3.5]nonan-7-yl)-3-fluorophenyl)amino)piperidine-2,6-dione (93 mg, crude) in DMF (5 mL) were added N-(3-carbamoyl-1-(4-formylphenyl)-1H-pyrazol-4-yl)-6-(trifluoromethyl)picolinamide (124 mg, 0.31 mmol, 1.2 eq) and 5 drops of AcOH. The reaction was stirred at rt for 2 h, and then sodium triacetoxyborohydride (326 mg, 1.56 mmol, 6.0 eq) was added. The reaction solution was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (off-white solid, 25.2 mg, yield 13% over two steps). LCMS (ESI): m/z 748[m +H]⁺. **¹H NMR** (400 MHz, DMSO) *δ* 11.79 (s, 1H), 10.77 (s, 1H), 9.03 (d, *J* = 9.0 Hz, 1H), 8.46 (d, *J* = 7.4 Hz, 1H), 8.40 (t, *J* = 7.8 Hz, 1H), 8.24-8.21 (m, 1H), 8.20 (s, 1HCOOH), 8.02 (s, 1H), 7.95 (d, *J =* 8.6 Hz, 2H), 7.77 (s, 1H), 7.52 (d, *J* = 8.5 Hz, 2H), 6.86-6.77 (m, 1H), 6.49 (dd, *J =* 14.9, 2.5 Hz, 1H), 6.40 (dd, *J =* 8.7, 2.3 Hz, 1H), 5.77 (d, *J* = 7.7 Hz, 1H), 4.29-4.20 (m, 1H), 3.74 (s, 2H), 3.31-3.27 (m, 1H), 2.79-2.74 (m, 2H), 2.74-2.67 (m, 3H), 2.62-2.54 (m, 1H), 2.11-2.04 (m, 3H), 1.90-1.79 (m, 1H), 1.65-1.56 (m, 6H).

### Preparation Example 368. Synthesis of Compound A359

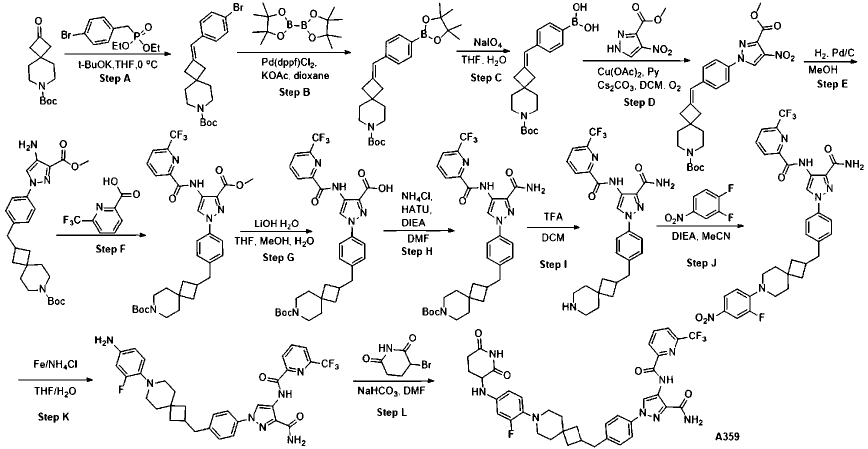

### Step A: Tert-butyl 2-((4-bromobenzylidene))-7-azaspiro[3.5]nonane-7-carboxylate

In an ice bath under N₂, potassium tert-butoxide (4.7 g, 41.787 mmol, 2.0 eq) was added to a solution of tert-butyl 2-oxo-7-azaspiro[3.5]nonane-7-carboxylate (5 g, 20.893 mmol, 1.0 eq) and diethyl (4-bromobenzyl)phosphonate (9.6 g, 31.340 mmol, 1.5 eq) in THF (50 mL). The reaction solution was stirred for 3 h, and then poured into saturated aqueous NH₄Cl, and the mixture was extracted with EtOAc. The organic phase was dried, filtered, and concentrated. The residue was purified by column chromatography to afford the title compound (white solid, 6.4 g, yield 78%).

### Step B: Tert-butyl 2-(4-(4,4,5,5-tetramethyl-1,3,2-dioxaborolan-2-yl)benzylidene))-7-azaspiro[3.5]nonane-7-carboxylate

At rt, Pd(dppf)Cl₂ (1.2 g, 1.631 mmol, 0.1 eq) was added to a solution of tert-butyl 2-(4-bromobenzylidene)-7-azaspiro[3.5]nonane-7-carboxylate (6.4 g, 16.312 mmol, 1.0 eq), bis(pinacolato)diboron (5.0 g, 19.575 mmol, 1.2 eq), and potassium acetate (3.2 g, 32.624 mmol, 2.0 eq) in dioxane (70 mL). The reaction solution was stirred at 90°C under N₂ for 3 h. Upon completion, the reaction solution was cooled to rt, poured into saturated aqueous NH₄Cl, and extracted with EtOAc. The organic phase was dried, filtered, and concentrated. The residue was purified by column chromatography to afford the title compound (white solid, 5.9 g, yield 82%).

### Step C: (4-((7-(tert-Butoxycarbonyl)-7-azaspiro[3.5]nonan-2-ylidene)methyl)phenyl)boronic acid

Sodium periodate (11.5 g, 53.710 mmol, 4.0 eq) was added to a solution of tert-butyl 2-(4-(4,4,5,5-tetramethyl-1,3,2-dioxaborolan-2-yl)benzylidene)-7-azaspiro[3.5]nonane-7-carboxylate (5.9 g, 13.427 mmol, 1.0 eq) in THF (60 mL) and water (20 mL) at rt. The reaction solution was stirred at rt under N₂ for 16 h. The reaction solution was filtered, and the filtrate was poured into saturated aqueous NaHCO₃ and extracted with EtOAc (×3). The organic phase was dried, filtered, and concentrated. The residue was purified by column chromatography to afford the title compound (white solid, 3.2 g, yield 67%).

### Step D: Tert-butyl 2-(4-(3-(methoxycarbonyl)-4-nitro-1H-pyrazol-1-yl)benzylidene)-7-azaspiro[3.5]nonane-7-carboxylate

At rt, copper acetate (0.4 g, 1.791 mmol, 0.2 eq) was added to a solution of (4-((7-(tert-butoxycarbonyl)-7-azaspiro[3.5]nonan-2-ylidene)methyl)phenyl)boronic acid (3.2 g, 8.957 mmol, 1.0 eq), methyl 4-nitro-1H-pyrazole-3-carboxylate (2.3 g, 13.436 mmol, 1.5 eq), cesium carbonate (8.8 g, 26.871 mmol, 3.0 eq), and pyridine (4.3 mL, 53.742 mmol, 6.0 eq) in acetonitrile (30 mL). The reaction solution was stirred at 80°C under O₂ for 16 h. Upon completion, the reaction solution was cooled to rt and filtered. The filtrate was concentrated. The residue was purified by column chromatography to afford the title compound (yellow solid, 1.5 g, yield 35%).

### Step E: Tert-butyl 2-(4-(4-amino-3-(methoxycarbonyl)-1H-pyrazol-1-yl)benzyl)-7-azaspiro[3.5]nonane-7-carboxylate

At rt, 10% Pd/C (6.6 g) was added to a solution of tert-butyl 2-(4-(3-(methoxycarbonyl)-4-nitro-1H-pyrazol-1-yl)benzylidene)-7-azaspiro[3.5]nonane-7-carboxylate (1.5 g, 3.109 mmol, 1.0 eq) in MeOH (15 mL). The mixture was stirred under H₂ at rt for 3 h. The reaction solution was filtered, and the filtrate was concentrated to afford the title compound (yellow solid, 1.3 g, yield 92%).

### Step F: Tert-butyl 2-(4-(3-(methoxycarbonyl)-4-(6-(trifluoromethyl)picolinamido)-1H-pyrazol-1-yl)benzyl)-7-azaspiro[3.5]nonane-7-carboxylate

To a solution of tert-butyl 2-(4-(4-amino-3-(methoxycarbonyl)-1H-pyrazol-1-yl)benzyl)-7-azaspiro[3.5]nonane-7-carboxylate (1.3 g, 2.860 mmol, 1.0 eq) 6-(trifluoromethyl)picolinic acid (0.6 g, 2.860 mmol, 1.0 eq) and DIEA (0.7 g, 5.720 mmol, 2.0 eq) in DMF (13 mL) were added HATU (1.6 g, 4.290 mmol, 1.5 eq) at rt. The reaction solution was stirred at rt for 2 h, diluted in saturated aqueous NH₄Cl, and extracted with EtOAc. The organic phase was dried, filtered, and concentrated. The residue was purified by column chromatography to afford the title compound (yellow solid, 1.5 g, yield 84%).

### Step G: 1-(4-((7-(tert-Butoxycarbonyl)-7-azaspiro[3.5]non-2-yl)methyl)phenyl)-4-(6-(trifluoromethyl)picolinamido)-1H-pyrazole-3-carboxylic acid

To a solution of tert-butyl 2-(4-(3-(methoxycarbonyl)-4-(6-(trifluoromethyl)picolinamido)-1H-pyrazol-1-yl)benzyl)-7-azaspiro[3.5]nonane-7-carboxylate (1.5 g, 2.390 mmol, 1.0 eq) in THF (10 mL), MeOH (5 mL), and water (2.5 mL) was added lithium hydroxide (0.5 g, 11.949 mmol, 5.0 eq) at rt. The reaction solution was stirred at rt for 2 h, acidified with dilute HCl (1 mol/L), and extracted with EtOAc. The organic phase was dried, filtered, and concentrated. The residue was purified by column chromatography to afford the title compound (yellow solid, 1.1 g, crude).

### Step H: Tert-butyl 2-(4-(3-carbamoyl-4-(6-(trifluoromethyl)pyridinylamino)-1H-pyrazol-1-yl)benzyl)-7-azaspiro[3.5]nonane-7-carboxylate

To a solution of 1-(4-((7-(tert-butoxycarbonyl)-7-azaspiro[3.5]non-2-yl)methyl)phenyl)-4-(6-(trifluoromethyl)picolinamido)-1H-pyrazole-3-carboxylic acid (1.1 g, 1.793 mmol, 1.0 eq), ammonium chloride (0.48 g, 8.963 mmol, 5.0 eq) and DIEA (0.5 g, 3.585 mmol, 2.0 eq) in DMF (11 mL) was added HATU (1.0 g, 2.689 mmol, 1.5 eq) at rt. The reaction solution was stirred at rt for 2 h, diluted in saturated aqueous NH₄Cl, and extracted with EtOAc. The organic phase was dried, filtered, and concentrated. The residue was purified by column chromatography to afford the title compound (yellow solid, 900 mg, yield 82%).

### Step I: N-(1-(4-((7-Azaspiro[3.5]nonan-2-yl)methyl)phenyl)-3-carbamoyl-1H-pyrazol-4-yl)-6-(trifluoromethyl)picolinamide

At rt, to a solution of tert-butyl 2-(4-(3-carbamoyl-4-(6-(trifluoromethyl)pyridinylamino)-1H-pyrazol-1-yl)benzyl)-7-azaspiro[3.5]nonane-7-carboxylate (900 mg, 1.469 mmol, 1.0 eq) in DCM (10 mL) was added TFA (2.5 mL). The reaction solution was stirred at rt for 2 h, then basified with aqueous NaOH (1 mol/L) and extracted with DCM. The organic phase was dried, filtered, and concentrated to afford the title compound (yellow oil, 600 mg, yield 80%).

### Step J: N-(3-Carbamoyl-1-(4-((7-(2-fluoro-4-nitrophenyl)-7-azaspiro[3.5]nonan-2-yl)methyl)phenyl)-1H-pyrazol-4-yl)-6-(trifluoromethyl)picolinamide

At rt, DIEA (177 mg, 1.366 mmol, 2.0 eq) was added to a solution of N-(1-(4-((7-azaspiro[3.5]nonan-2-yl)methyl)phenyl)-3-carbamoyl-1H-pyrazol-4-yl)-6-(trifluoromethyl)picolinamide (350 mg, 0.683 mmol, 1.0 eq) and 1,2-difluoro-4-nitrobenzene (130 mg, 0.819 mmol, 1.2 eq) in acetonitrile (4 mL). The reaction solution was stirred at 80°C under N₂ for 2 h. Upon completion, the reaction solution was cooled to rt, diluted in saturated aqueous NH₄Cl, and extracted with EtOAc. The organic phase was dried, filtered, and concentrated. The residue was purified by column chromatography to afford the title compound (yellow solid, 460 mg, yield 93%).

### Step K: N-(1-(4-((7-(4-Amino-2-fluorophenyl)-7-azaspiro[3.5]nonan-2-yl)methyl)phenyl)-3-carbamoyl-1H-pyrazol-4-yl)-6-(trifluoromethyl)picolinamide

At rt, ammonium chloride (170 mg, 3.177 mmol, 5.0 eq) and iron powder (355 mg, 6.353 mmol, 10 eq) were added to a solution of N-(3-carbamoyl-1-(4-((7-(2-fluoro-4-nitrophenyl)-7-azaspiro[3.5]nonan-2-yl)methyl)phenyl)-1H-pyrazol-4-yl)-6-(trifluoromethyl)picolinamide (460 mg, 0.635 mmol, 1.0 eq) in THF (6 mL) and water (2 mL). The mixture was stirred at 70°C under N₂ for 2 h. Upon completion, the reaction solution was cooled to rt, diluted in saturated aqueous NH₄Cl, and extracted with EtOAc. The organic phase was dried, filtered, and concentrated. The residue was purified by column chromatography to afford the title compound (yellow solid, 240 mg, yield 61%).

### Step L: N-(3-Carbamoyl-1-(4-((7-(4-((2,6-dioxopiperidin-3-yl)amino)-2-fluorophenyl)-7-azaspiro[3.5]nonan-2-yl)methyl)phenyl)-1H-pyrazol-4-yl)-6-(trifluoromethyl)picolinamide

At rt, NaHCO₃ (54 mg, 0.643 mmol, 2.0 eq) was added to a solution of N-(1-(4-((7-(4-amino-2-fluorophenyl)-7-azaspiro[3.5]nonan-2-yl)methyl)phenyl)-3-carbamoyl-1H-pyrazol-4-yl)-6-(trifluoromethyl)picolinamide (200 mg, 0.322 mmol, 1.0 eq) and 3-bromopiperidine-2,6-dione (93 mg, 0.483 mmol, 1.5 eq) in DMF (2 mL). The reaction solution was stirred at 80°C under N₂ for 16 h. Upon completion, the reaction solution was purified by HPLC to afford the title compound (yellow solid, 60 mg, yield 25%). **¹H NMR** (400 MHz, DMSO-*d*₆) *δ* 11.79 (s, 1H), 10.81 (s, 1H), 8.98 (s, 1H), 8.46-8.39 (m, 2H), 8.22 (d, *J* = 7.5 Hz, 1H), 7.99 (s, 1H), 7.89 (*d, J =* 8.4 Hz, 2H), 7.77 (s, 1H), 7.36 *(d, J =* 8.5 Hz, 2H), 6.93-6.20 (m, 3H), 4.34 (d, *J =* 9.5 Hz, 1H), 3.16-2.96 (m, 2H), 2.81-2.66 (m, 4H), 2.60-2.52 (m, 2H), 2.09-1.75 (m, 9H), 1.62-1.56 (m, 2H). LCMS (ESI): m/z 733 [M+H]⁺.

### Preparation Example 369. Synthesis of Compound A360

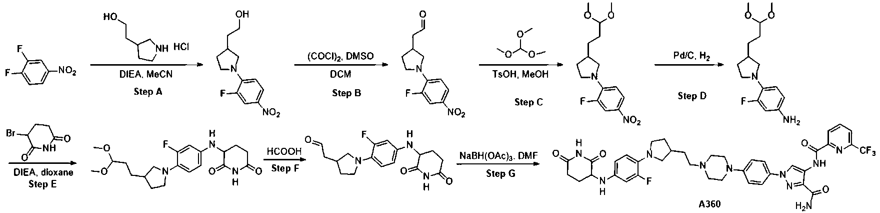

### Step A: 2-(1-(2-Fluoro-4-nitrophenyl)pyrrolidin-3-yl)ethanol

To a solution of 2-(pyrrolidin-3-yl)ethanol hydrochloride (1 g, 8.68 mmol, 1.2 eq) in acetonitrile (10 mL) were added 1,2-difluoro-4-nitrobenzene (1.15 g, 7.23 mmol, 1.0 eq) and DIEA (5.9 mL, 21.7 mmol, 3.0 eq). The mixture was stirred at 80°C for 3 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 1.2 g, yield 62%).

### Step B: 2-(1-(2-Fluoro-4-nitrophenyl)pyrrolidin-3-yl)acetaldehyde

At -78°C, dimethyl sulfoxide (1.4 mL, 18.9 mmol, 4.0 eq) was added to a solution of oxalyl chloride (0.8 mL, 9.45 mmol, 2.0 eq) in DCM (15 mL), and the reaction was stirred at -78°C for 15 min. Then 2-(1-(2-fluoro-4-nitrophenyl)pyrrolidin-3-yl)ethanol (1.2 g, 4.72 mmol, 1.0 eq) was added. The mixture was stirred at -78°C for 15 min, and then TEA (2.6 mL, 18.9 mmol, 4.0 eq) was added. The mixture was stirred at -78°C for 30 min. Upon completion, the reaction solution was diluted in DCM, washed sequentially with water and saturated aqueous NaCl, dried over anhydrous Na₂SO₄, and concentrated under reduced pressure. The residue was purified by column chromatography to afford the title compound (yellow oil, 1 g, yield 80%).

### Step C: 3-(3,3-Dimethoxypropyl)-1-(2-fluoro-4-nitrophenyl)pyrrolidine

To a solution of trimethyl orthoformate (0.65 mL, 5.94 mmol, 1.5 eq) in MeOH (10 mL) were added 2-(1-(2-fluoro-4-nitrophenyl)pyrrolidin-3-yl)acetaldehyde (1 g, 3.96 mmol, 1.0 eq) and p-toluenesulfonic acid monohydrate (0.3 g, 1.59 mmol, 0.4 eq). The mixture was stirred at rt for 30 min, The mixture was stirred at 80°C for 3 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 1 g, yield 77%)

### Step D: 4-(3-(3,3-Dimethoxypropyl)pyrrolidin-1-yl)-3-fluoroaniline

To a solution of 3-(3,3-dimethoxypropyl)-1-(2-fluoro-4-nitrophenyl)pyrrolidine (1 g, 3.21 mmol, 1.0 eq) in ethanol (20 mL) was added 10% wet palladium on carbon (300 mg). The mixture was degassed with H₂ (×3) and stirred under a hydrogen balloon at 25°C for 2 h. The reaction solution was filtered through Celite and concentrated to afford the title compound (white solid, 500 mg, yield 53%).

### Step E: 3-((4-(3-(3,3-Dimethoxypropyl)pyrrolidin-1-yl)-3-fluorophenyl)amino)piperidine-2,6-dione

To a solution of 3-bromo-2,6-piperidinedione (680 mg, 3.55 mmol, 2.0 eq) in 1,4-dioxane (0.5 mL) were added 4-(3-(3,3-dimethoxypropyl)pyrrolidin-1-yl)-3-fluoroaniline (500 mg, 1.77 mmol, 1.0 eq) and DIEA (0.62 mL, 3.55 mol, 2.0 eq). The mixture was stirred at 80°C for 3 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow oil, 500 mg, yield 68%)

### Step F: 2-(1-(4-((2,6-Dioxopiperidin-3-yl)amino)-2-fluorophenyl)pyrrolidin-3-yl)acetaldehyde

A solution of 3-((4-(3-(3,3-dimethoxypropyl)pyrrolidin-1-yl)-3-fluorophenyl)amino)piperidine-2,6-dione (500 mg, 1.27 mmol, 1.0 eq) in formic acid (10 mL) was stirred at 60°C for 2 h. The reaction solution was concentrated under reduced pressure to afford the title compound (green oil, 300 mg, crude).

### Step G: N-(3-Carbamoyl-1-(4-(4-(2-((2,6-dioxopiperidin-3-yl)amino)-2-fluorophenyl)pyrrolidin-3-yl)ethyl)piperazin-1-yl)phenyl)-1H-pyrazol-4-yl)-6-(trifluoromethyl)picolinamide

To a solution of N-(3-carbamoyl-1-(4-(piperazin-1-yl)phenyl)-1H-pyrazol-4-yl)-6-(trifluoromethyl)picolinamide (413 mg, 0.9 mmol, 0.9 eq) in DMF (10 mL) were added AcOH and 2-(1-(4-((2,6-dioxopiperidin-3-yl)amino)-2-fluorophenyl)pyrrolidin-3-yl)acetaldehyde (300 mg, crude). The mixture was stirred at rt for 1 h, and then sodium triacetoxyborohydride (949 mg, 4.5 mmol, 5.0 eq) was added. The mixture was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (blue solid, 25 mg, yield 3% over two steps). LCMS (ESI): m/z 777[M+H]⁺. **¹H NMR** (400 MHz, DMSO) *δ* 11.80 (s, 1H), 10.77 (s, 1H), 8.92-8.90 (m, 1H), 8.47-8.40 (m, 2H), 8.22 *(d, J* = 7.6 Hz, 1H), 7.96 (s, 1H), 7.84 (d, *J* = 8.5 Hz, 2H), 7.73 (s, 1H), 7.14 *(d, J =* 8.7 Hz, 2H), 6.65-6.60 (m, 1H), 6.53 *(d, J* = 15.5 Hz, 1H), 6.43-6.39 (m, 1H), 5.56 (d, *J =* 7.6 Hz, 1H), 4.24-4.18 (m, 1H), 3.24-3.12 (m, 8H), 2.90-2.84 (m, 2H), 2.75-2.67 (m, 2H), 2.62-2.58 (m, 1H), 2.34-2.17 (m, 2H), 2.12-1.96 (m, 4H), 1.89-1.83 (m, 1H), 1.79-1.70 (m, 2H), 1.60-1.54 (m, 1H).

### Preparation Example 370. Synthesis of Compound A361

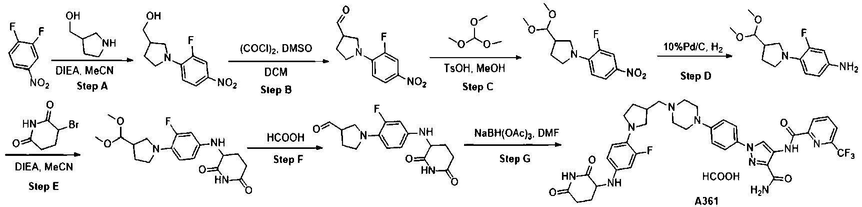

### Step A: (1-(2-Fluoro-4-nitrophenyl)pyrrolidin-3-yl)methanol

At rt, to a solution of 1,2-difluoro-4-nitrobenzene (4 g, 25.14 mmol, 1.0 eq) in acetonitrile (50 mL) were added pyrrolidin-3-ylmethanol (3.1 g, 30.17 mmol, 2.0 eq) and DIEA (13.4 mL, 75.43 mmol, 3.0 eq). The reaction solution was stirred at 80°C under N₂ for 3 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 4.5 g, yield 75%).

### Step B: 1-(2-Fluoro-4-nitrophenyl)pyrrolidine-3-carbaldehyde

At -60°C, DMSO (3.6 mL, 49.95 mmol, 1.0 eq) was added dropwise to a solution of oxalyl chloride (2.1 mL, 24.98 mmol,1.0 eq) in DCM (50 mL). The reaction solution was stirred at -60°C under N₂ for 15 min, and then a solution of (1-(2-fluoro-4-nitrophenyl)pyrrolidin-3-yl)methanol (3 g, 12.49 mmol, 1.0 eq) in DCM was added dropwise. The reaction solution was stirred at -60°C for an additional 15 min, and then TEA (7.0 mL, 49.95 mmol, 3.0 eq) was added dropwise. The reaction solution was stirred at -60°C under N₂ for 30 min, then slowly warmed to rt and stirred for 1 h. Upon completion, the reaction solution was diluted in DCM, washed sequentially with water and saturated aqueous NaCl, dried over anhydrous Na₂SO₄, and concentrated under reduced pressure. The residue was purified by column chromatography to afford the title compound (yellow solid, 2.7 g, yield 91%).

### Step C: 3-(Dimethoxymethyl)-1-(2-fluoro-4-nitrophenyl)pyrrolidine

At rt, to a solution of 1-(2-fluoro-4-nitrophenyl)pyrrolidine-3-carbaldehyde (2.7 g, 11.33 mmol, 1.0 eq) in MeOH (30 mL) were added trimethyl orthoformate (1.9 mL, 17.0 mmol, 1.5 eq) and p-toluenesulfonic acid (0.8 g, 4.53 mmol, 0.4 eq). The reaction solution was stirred at rt for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 2.4 g, yield 74%).

### Step D: 4-(3-(Dimethoxymethyl)pyrrolidin-1-yl)-3-fluoroaniline

At rt, to a solution of 3-(dimethoxymethyl)-1-(2-fluoro-4-nitrophenyl)pyrrolidine (500 mg, 1.76 mmol, 1.0 eq) in MeOH (10 mL) was added 10% wet palladium on carbon (100 mg). The reaction solution was stirred under H₂ at rt for 3 h. Upon completion, the reaction solution was filtered through silica gel and concentrated under reduced pressure to afford the title compound (white solid, 440 mg, yield 98%).

### Step E: 3-((4-(3-(Dimethoxymethyl)pyrrolidin-1-yl)-3-fluorophenyl)amino)piperidine-2,6-dione

At rt, to a solution of 4-(3-(dimethoxymethyl)pyrrolidin-1-yl)-3-fluoroaniline (440 mg, 1.81 mmol, 1.0 eq) in 1,4-dioxane (2 mL) were added 3-bromo-2,6-piperidinedione (695 mg, 3.62 mmol, 2.0 eq) and DIEA (0.6 mL, 3.62 mmol, 2.0 eq). The mixture was stirred at 100°C for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (purple solid, 470 mg, yield 71%).

### Step F: 1-(4-((2,6-Dioxopiperidin-3-yl)amino)-2-fluorophenyl)pyrrolidine-3-carbaldehyde

A solution of 3-((4-(3-(dimethoxymethyl)pyrrolidin-1-yl)-3-fluorophenyl)amino)piperidine-2,6-dione (170 mg, 0.47 mmol, 1.0 eq) in formic acid (5 mL) was stirred at 60°C for 2 h. TLC showed the reaction was completed. The mixture was concentrated under reduced pressure and used directly in the next step.

### Step G: N-(3-Carbamoyl-1-(4-(4-((1-(4-((2,6-dioxopiperidin-3-yl)amino)-2-fluorophenyl)pyrrolidin-3-yl)methyl)piperazin-1-yl)phenyl)-1H-pyrazol-4-yl)-6-(trifluoromethyl)picolinamide formate

At rt, to a solution of 1-(4-((2,6-dioxopiperidin-3-yl)amino)-2-fluorophenyl)pyrrolidine-3-carbaldehyde (140 mg, crude) in DMF (10 mL) were added N-(3-carbamoyl-1-(4-(piperazin-1-yl)phenyl)-1H-pyrazol-4-yl)-6-(trifluoromethyl)picolinamide (201 mg, 0.44 mmol, 1.0 eq) and 5 drops of AcOH. The reaction was stirred at rt for 2 h, and then sodium triacetoxyborohydride (465 mg, 2.19 mmol, 5.0 eq) was added. The reaction solution was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (off-white solid, 25.0 mg, yield 7%). LCMS (ESI): m/z 764 [M+H]⁺. **¹H NMR** (400 MHz, DMSO) *δ* 11.79 (s, 1H), 10.76 (s, 1H), 8.88 (s, 1H), 8.45 (d, *J* = 7.5 Hz, 1H), 8.40 (t, *J* = 7.8 Hz, 1H), 8.21 (d, *J* = 7.6 Hz, 1H), 8.14 (s, 0.7HCOOH), 7.95 (s, 1H), 7.79 (d, *J =* 9.1 Hz, 2H), 7.71 (s, 1H), 7.08 (d, *J =* 9.1 Hz, 2H), 6.66-6.59 (m, 1H), 6.52 (dd, *J =* 15.8, 2.4 Hz, 1H), 6.40 (dd, *J* = 8.7, 2.2 Hz, 1H), 5.55 (d, *J =* 7.5 Hz, 1H), 4.20 (dd, *J* = 10.7, 5.8 Hz, 1H), 3.23 (d, *J =* 4.5 Hz, 6H), 3.15 (t, *J =* 6.6 Hz, 2H), 2.97-2.92 (m, 1H), 2.79-2.69 (m, 1H), 2.64-2.54 (m, 5H), 2.39 (t, *J =* 7.0 Hz, 2H), 2.14-2.07 (m, 1H), 2.05-1.98 (m, 1H), 1.85 (td, J = 12.2, 4.6 Hz, 1H), 1.65-1.55 (m, 1H).

### Preparation Example 371. Synthesis of Compound A362

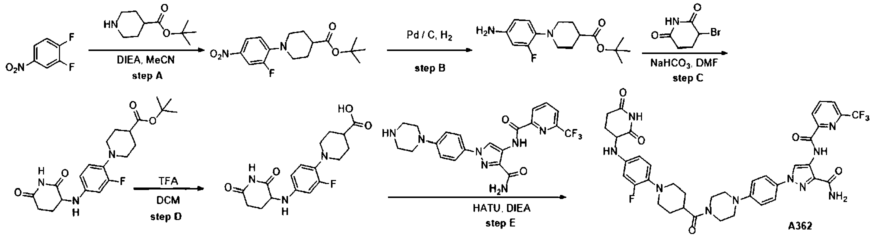

### Step A: Tert-butyl 1-(2-fluoro-4-nitrophenyl)piperidine-4-carboxylate

At rt, to a solution of 1,2-difluoro-4-nitrobenzene (2 g, 12.58 mmol, 1.0 eq) in acetonitrile (35 mL) were added tert-butyl piperidine-4-carboxylate (2.6 g, 13.84 mmol, 1.1 eq) and DIEA (5.5 mL, 31.45 mmol, 2.5 eq). The reaction solution was stirred at rt under N₂ for 8 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 1.5 g, yield 37%).

### Step B: Tert-butyl 1-(4-amino-2-fluorophenyl)piperidine-4-carboxylate

At rt, to a solution of tert-butyl 1-(2-fluoro-4-nitrophenyl)piperidine-4-carboxylate (1.5 g, 4.63 mmol, 1.0 eq) in EtOAc (40 mL) was added 10% wet palladium on carbon (300 mg). The reaction solution was stirred under H₂ at rt for 6 h. Upon completion, the reaction solution was filtered through Celite and concentrated under reduced pressure to afford the title compound (colorless oil, 1.3 g, yield 96%).

### Step C: Tert-butyl 1-(4-((2,6-dioxopiperidin-3-yl)amino)-2-fluorophenyl)piperidine-4-carboxylate

At rt, to a mixture of tert-butyl 1-(4-amino-2-fluorophenyl)piperidine-4-carboxylate (1.3 g, 4.42 mmol, 1.0 eq) in DMF (30 mL) were added 3-bromo-2,6-piperidinedione (1.0 g, 5.75 mmol, 1.3 eq) and NaHCO₃ (930 mg, 11.05 mmol, 2.5 eq). The reaction solution was stirred at 60°C under N₂ for 6 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 800 mg, yield 45%).

### Step D: 1-(4-((2,6-Dioxopiperidin-3-yl)amino)-2-fluorophenyl)piperidine-4-carboxylic acid

At rt, TFA (2 mL) was added to a solution of tert-butyl 1-(4-((2,6-dioxopiperidin-3-yl)amino)-2-fluorophenyl)piperidine-4-carboxylate (200 mg, 0.49 mmol, 1.0 eq) in DCM (10 mL). The reaction solution was stirred at rt for 3 h. Upon completion, the reaction solution was concentrated under reduced pressure to afford the title compound (brown solid, 170 mg, yield 99%).

### Step E: N-(3-Carbamoyl-1-(4-(4-(1-(4-((2,6-dioxopiperidin-3-yl)amino)-2-fluorophenyl)piperidine-4-carbonyl)piperazin-1-yl)phenyl)-1H-pyrazol-4-yl)-6-(trifluoromethyl)picolinamide

At 0°C, to a solution of 1-(4-((2,6-dioxopiperidin-3-yl)amino)-2-fluorophenyl)piperidine-4-carboxylic acid (170 mg, 0.49 mmol, 1.0 eq) in DMF (5 mL) were added N-(3-carbamoyl-1-(4-(piperazin-1-yl)phenyl)-1H-pyrazol-4-yl)-6-(trifluoromethyl)picolinamide (335 mg, 0.73 mmol, 1.5 eq), HATU (370 mg, 0.97 mmol, 2.0 eq), and DIEA (0.25 mL, 1.46 mmol, 3.0 eq). The reaction solution was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (yellow solid, 25.3 mg, yield 7%). LCMS (ESI): m/z 791 [M+H]⁺. **¹H NMR** (400 MHz, DMSO) *δ* 11.79 (s, 1H), 10.78 (s, 1H), 8.90 (s, 1H), 8.48-8.36 (m, 2H), 8.21 (d, *J =* 7.6 Hz, 1H), 7.96 (s, 1H), 7.83 (d, *J =* 8.4 Hz, 2H), 7.72 (s, 1H), 7.12 (d, *J =* 8.4 Hz, 2H), 6.84 (t, *J =* 9.1 Hz, 1H), 6.51 (d, *J =* 14.8 Hz, 1H), 6.43 (d, *J =* 8.6 Hz, 1H), 5.81 (d, *J =* 7.7 Hz, 1H), 4.32-4.20 (m, 1H), 3.74-3.61 (m, 4H), 3.29-3.11 (m, 8H), 2.77-2.73 (m, 1H), 2.68-2.66 (m, 1H), 2.62-2.57 (m, 1H), 2.08-2.07 (m, 1H), 1.90-1.82 (m, 1H), 1.78-1.68 (m, 4H).

### Preparation Example 372. Synthesis of Compound A363

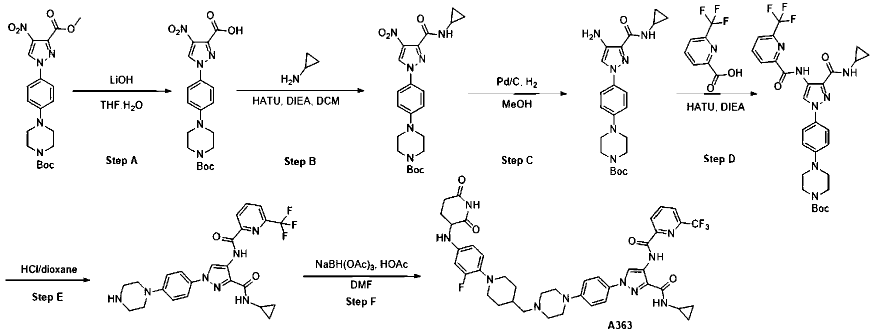

### Step A: 1-(4-(4-(tert-Butoxycarbonyl)piperazin-1-yl)phenyl)-4-nitro-1H-pyrazole-3-carboxylic acid

At rt, lithium hydroxide monohydrate (330 mg, 13.92 mmol, 3.0 eq) was added to a mixed solution of tert-butyl 4-(4-(3-(methoxycarbonyl)-4-nitro-1H-pyrazol-1-yl)phenyl)piperazine-1-carboxylate (2 g, 4.46 mmol, 1.0 eq) in THF (20 mL) and water (5 mL). The reaction solution was stirred at rt for 1 h. Upon completion, the reaction solution was diluted in water, adjusted to pH 4-5 with 2N HCl, and extracted with EtOAc. The organic phase was washed sequentially with saturated aqueous NaCl, dried over anhydrous Na₂SO₄, and concentrated under reduced pressure to afford the title compound (brown solid, 1.8 g, yield 93%).

### Step B: Tert-butyl 4-(4-(3-(cyclopropylcarbamoyl)-4-nitro-1H-pyrazol-1-yl)phenyl)piperazine-1-carboxylate

At rt, DIEA (2.3 mL, 13.92 mmol, 3.0 eq) was added to a solution of 1-(4-(4-(tert-butoxycarbonyl)piperazin-1-yl)phenyl)-4-nitro-1H-pyrazole-3-carboxylic acid (1.8 g, 4.32 mmol, 1.0 eq), cyclopropylamine (0.4 g, 6.47 mmol, 1.5 eq), and HATU (2.5 g, 12.96 mmol, 1.5 eq) in DCM (30 mL). The reaction solution was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 1.6 g, yield 81%).

### Step C: Tert-butyl 4-(4-(4-amino-3-(cyclopropylcarbamoyl)-1H-pyrazol-1-yl)phenyl)piperazine-1-carboxylate

At rt, 10% wet palladium on carbon (0.15 g, w.t.= 10%) was added to a solution of tert-butyl 4-(4-(3-(cyclopropylcarbamoyl)-4-nitro-1H-pyrazol-1-yl)phenyl)piperazine-1-carboxylate (1.5 g, 3.3 mmol, 1.0 eq) in anhydrous MeOH (30 mL). The reaction solution was stirred under H₂ at rt for 4 h. Upon completion, the reaction solution was filtered and concentrated under reduced pressure to afford the title compound (white solid, 1.4 g, yield 99%).

### Step D: Tert-butyl 4-(4-(3-(cyclopropylcarbamoyl)-4-(6-(trifluoromethyl)pyridinylamino)-1H-pyrazol-1-yl)phenyl)piperazine-1-carboxylate

At rt, DIEA (1.65 mL, 9.15 mmol, 3.0 eq) was added to a solution of tert-butyl 4-(4-(4-amino-3-(cyclopropylcarbamoyl)-1H-pyrazol-1-yl)phenyl)piperazine-1-carboxylate (1.3 g, 3.05 mmol, 1.0 eq), 6-(trifluoromethyl)pyridine-2-carboxylic acid (0.6 g, 3.35 mmol, 1.1 eq), and HATU (2.3 g, 6.1 mmol, 2.0 eq) in DMF (15 mL). The mixture was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 1.7 g, yield 93%).

### Step E: N-(3-(Cyclopropylcarbamoyl)-1-(4-(piperazin-1-yl)phenyl)-1H-pyrazol-4-yl)-6-(trifluoromethyl)picolinamide

At rt, a 4 M hydrogen chloride in dioxane solution (2 mL) was added to a solution of tert-butyl 4-(4-(3-(cyclopropylcarbamoyl)-4-(6-(trifluoromethyl)pyridinylamino)-1H-pyrazol-1-yl)phenyl)piperazine-1-carboxylate (1.7 g, 2.84 mmol, 1.0 eq) in acetonitrile (20 ml). The reaction solution was stirred at rt for 1 h. Upon completion, the reaction solution was concentrated under reduced pressure to afford the title compound (white solid, 1.2 g, crude).

### Step F: N-(3-(Cyclopropylcarbamoyl)-1-(4-(4-((1-(4-((2,6-dioxopiperidin-3-yl)amino)-2-fluorophenyl)piperidin-4-yl)methyl)piperazin-1-yl)phenyl)-1H-pyrazol-4-yl)-6-(trifluoromethyl)picolinamide

At rt, to a solution of N-(3-(cyclopropylcarbamoyl)-1-(4-(piperazin-1-yl)phenyl)-1H-pyrazol-4-yl)-6-(trifluoromethyl)picolinamide (150 mg, 0.3 mmol, 1.0 eq) in DMF (5 mL) were added 1-(4-((2,6-dioxopiperidin-3-yl)amino)-2-fluorophenyl)piperidine-4-carbaldehyde (100 mg, 0.26 mmol, 1.0 eq) and 3 drops of AcOH. The mixture was stirred at rt for 2 h, and then sodium triacetoxyborohydride (126 mg, 0.60 mmol, 2.0 eq) was added. The reaction solution was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (gray solid, 25 mg, yield 10%). LCMS (ESI): m/z 817 [M+H]⁺. **¹H NMR** (400 MHz, DMSO) *δ* 11.61 (s, 1H), 10.78 (s, 1H), 8.90 (s, 1H), 8.52 (d, *J* = 4.6 Hz, 1H), 8.46 (d, *J* = 7.6 Hz, 1H), 8.40 *(t, J=* 7.8 Hz, 1H), 8.22 (d, *J =* 7.7 Hz, 1H), 8.15 (s, 1H), 7.79 (d, *J =* 9.0 Hz, 2H), 7.06 (d, *J =* 9.2 Hz, 2H), 6.83 (t, *J =* 9.3 Hz, 1H), 6.50 (dd, *J =* 15.0, 2.4 Hz, 1H), 6.42 (dd, *J =* 8.7, 2.1 Hz, 1H), 5.78 (d, *J =* 7.6 Hz, 1H), 4.32-4.18 (m, 1H), 3.25-3.19 (m, 4H), 3.14-3.07 (m, 2H), 2.99-2.93 (m, 1H), 2.78-2.69 (m, 1H), 2.62-2.56 (m, 2H), 2.56-2.52 (m, 3H), 2.27-2.18 (m, 2H), 2.13-2.04 (m, 1H), 1.91-1.76 (m, 3H), 1.68-1.56 (m, 1H), 1.31-1.22 (m, 2H), 0.75-0.68 (m, 4H).

### Preparation Example 373. Synthesis of Compound A364

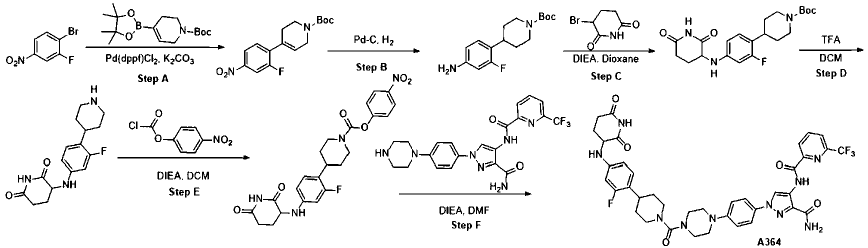

### Step A: Tert-butyl 4-(2-fluoro-4-nitrophenyl)-3,6-dihydropyridine-1(2H)-carboxylate

At rt, to a mixed solution of 1-bromo-2-fluoro-4-nitrobenzene (5 g, 22.73 mmol, 1.0 eq) in 1,4-dioxane (50 mL) and water (5 mL) were added tert-butyl 4-(4,4,5,5-tetramethyl-1,3,2-dioxaborolan-2-yl)-3,6-dihydropyridine-1(2H)-carboxylate (8.4 g, 27.27 mmol, 1.2 eq), potassium carbonate (9.4 g, 68.19 mmol, 3.0 eq), and Pd(dppf)Cl₂ (1.7 g, 2.27 mmol, 0.1 eq). The reaction solution was stirred at 90°C under N₂ for 3 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 3 g, yield 41%).

### Step B: Tert-butyl 4-(4-amino-2-fluorophenyl)piperidine-1-carboxylate

At rt, to a solution of tert-butyl 4-(2-fluoro-4-nitrophenyl)-3,6-dihydropyridine-1(2H)-carboxylate (3 g, 9.32 mmol, 1.0 eq) in ethanol (30 mL) were added palladium on carbon (1 g, w.t.= 10%), potassium carbonate (9.4 g, 68.19 mmol, 3.0 eq) and Pd(dppf)Cl₂ (1.7 g, 2.27 mmol, 0.1 eq). The reaction solution was stirred under H₂ at rt overnight. Upon completion, the reaction solution was diluted in EtOAc, filtered, dried over anhydrous Na₂SO₄, and concentrated under reduced pressure to afford the title compound (yellow solid, 2 g, yield 73%).

### Step C: Tert-butyl 4-(4-((2,6-dioxopiperidin-3-yl)amino)-2-fluorophenyl)piperidine-1-carboxylate

At rt, to a solution of tert-butyl 4-(4-amino-2-fluorophenyl)piperidine-1-carboxylate (2 g, 6.8 mmol, 1.0 eq) in 1,4-dioxane (5 mL) were added 3-bromo-2,6-piperidinedione (3.9 g, 20.41 mmol, 3.0 eq) and DIEA (2.4 mL, 13.6 mmol, 2.0 eq). The mixture was stirred at 90°C under N₂ overnight. Upon completion, the mixture was diluted in EtOAc, washed sequentially with water and saturated aqueous NaCl, dried over anhydrous Na₂SO₄, and concentrated under reduced pressure. The residue was purified by column chromatography to afford the title compound (yellow solid, 3 g, yield 41%).

### Step D: 3-((3-Fluoro-4-(piperidin-4-yl)phenyl)amino)piperidine-2,6-dione

At rt, TFA (3 mL) was added to a solution of tert-butyl 4-(4-((2,6-dioxopiperidin-3-yl)amino)-2-fluorophenyl)piperidine-1-carboxylate (800 mg, 1.96 mmol, 1.0 eq) in DCM (10 mL). The reaction solution was stirred at rt under N₂ for 1 h. Upon completion, the mixture was concentrated under reduced pressure to afford the title compound (white solid, 600 mg, crude).

### Step E: 4-Nitrophenyl 4-(4-((2,6-dioxopiperidin-3-yl)amino)-2-fluorophenyl)piperidine-1-carboxylate

At rt, to a solution of 3-((3-fluoro-4-(piperidin-4-yl)phenyl)amino)piperidine-2,6-dione (600 mg, crude) in DCM (10 mL) were added 4-nitrophenyl chloroformate (594 mg, 2.96 mmol, 1.5 eq) and DIEA (0.7 mL, 3.93 mmol, 2.0 eq). The reaction solution was stirred at rt under N₂ for 1 h. Upon completion, the reaction solution was diluted in DCM, washed sequentially with water and saturated aqueous NaCl, dried over anhydrous Na₂SO₄, and concentrated under reduced pressure. The residue was purified by column chromatography to afford the title compound (yellow solid, 400 mg, yield 43% over two steps).

### Step F: N-(3-Carbamoyl-1-(4-(4-(4-((2,6-dioxopiperidin-3-yl)amino)-2-fluorophenyl)piperidine-1-carbonyl)piperazin-1-yl)phenyl)-1H-pyrazol-4-yl)-6-(trifluoromethyl)picolinamide

At rt, to a solution of 4-nitrophenyl 4-(4-((2,6-dioxopiperidin-3-yl)amino)-2-fluorophenyl)piperidine-1-carboxylate (400 mg, 0.85 mmol, 1.0 eq) in DMF (5 mL) were added N-(3-carbamoyl-1-(4-(piperazin-1-yl)phenyl)-1H-pyrazol-4-yl)-6-(trifluoromethyl)picolinamide (469 mg, 1.02 mmol, 1.2 eq) and DIEA (0.3 mL, 1.7 mmol, 2.0 eq). The reaction solution was stirred at 100°C under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (51 mg, yield 7%). LCMS (ESI): m/z 792 [M+H]⁺. **¹H NMR** (400 MHz, DMSO) *δ* 11.79 (s, 1H), 10.79 (s, 1H), 8.90 (s, 1H), 8.47-8.34 (m, 2H), 8.21 (d, *J = 7.6* Hz, 1H), 7.96 (s, 1H), 7.82 (d, *J* = 9.0 Hz, 2H), 7.71 (s, 1H), 7.11 (d, *J* = 9.1 Hz, 2H), 7.00 (t, *J* = 8.9 Hz, 1H), 6.45 (dd, *J =* 11.8, 4.2 Hz, 2H), 6.03 (d, *J =* 7.7 Hz, 1H), 4.37-4.25 (m, 1H), 3.80-3.69 (m, 2H), 3.35-3.32 (m, 2H), 3.31-3.29 (m, 2H), 3.28-3.20 (m, 4H), 2.91-2.69 (m, 4H), 2.61-2.54 (m, 1H), 2.12-2.04 (m, 1H), 1.92-1.81 (m, 1H), 1.72-1.65 (m, 2H), 1.64-1.51 (m, 2H).

### Preparation Example 374. Synthesis of Compound A365

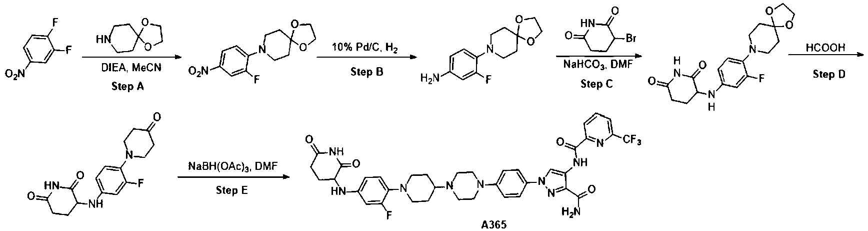

### Step A: 8-(2-Fluoro-4-nitrophenyl)-8-aza-1,4-dioxaspiro[4.5]decane

At rt, to a solution of 1,2-difluoro-4-nitrobenzene (10.0 g, 62.89 mmol, 1.0 eq) in acetonitrile (150 mL) were added 8-aza-1,4-dioxaspiro[4.5]decane (9.9 g, 69.18 mmol, 1.1 eq) and DIEA (27 mL, 157.23 mmol, 2.5 eq). The reaction solution was stirred at 80°C under N₂ for 16 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 17.1 g, yield 99%).

### Step B: 3-Fluoro-4-(8-aza-1,4-dioxaspiro[4.5]decan-8-yl)aniline

At rt, to a solution of 8-(2-fluoro-4-nitrophenyl)-8-aza-1,4-dioxaspiro[4.5]decane (5.0 g, 17.73 mmol, 1.0 eq) in EtOAc (60 mL) was added 10% wet palladium on carbon (500 mg). The reaction solution was stirred under H₂ at rt for 3 h. Upon completion, the reaction solution was filtered through silica gel and concentrated under reduced pressure to afford the title compound (yellow solid, 4.4 g, yield 98%).

### Step C: 3-{[3-Fluoro-4-(8-aza-1,4-dioxaspiro[4.5]decan-8-yl)phenyl]amino}piperidine-2,6-dione

At rt, to a solution of 3-fluoro-4-(8-aza-1,4-dioxaspiro[4.5]decan-8-yl)aniline (2.0 g, 7.94 mmol, 1.0 eq) in DMF (25 mL) were added NaHCO₃ (1.3 g, 15.87 mmol, 2.0 eq) and 3-bromopiperidine-2,6-dione (1.8 g, 9.53 mmol, 1.2 eq). The mixture was stirred at 60°C for 12 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 800 mg, yield 28%).

### Step D: 3-{[3-Fluoro-4-(4-oxopiperidin-1-yl)phenyl]amino}piperidine-2,6-dione

A solution of 3-{[3-fluoro-4-(8-aza-1,4-dioxaspiro[4.5]decan-8-yl)phenyl]amino} piperidine-2,6-dione (400 mg, 1.1 mmol, 1.0 eq) was dissolved in formic acid (5 mL), and the reaction was stirred at 80°C for 10 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (brown solid, 140 mg, yield 40%).

### Step E: 1-{4-[4-(1-{4-[(2,6-dioxopiperidin-3-yl)amino]-2-fluorophenyl}piperidin-4-yl)piperazin-1-yl]phenyl}-4-({[6-(trifluoromethyl)pyridin-2-yl]carbonyl}amino)pyrazole-3-carboxamide

At rt, to a solution of 3-{[3-Fluoro-4-(4-oxopiperidin-1-yl)phenyl]amino}piperidine-2,6-dione (140 mg, 0.44 mmol, 1.0 eq) in DMF (5 mL) were added 1-[4-(piperazin-1-yl)phenyl]-4-({[6-(trifluoromethyl)pyridin-2-yl]carbonyl}amino)pyrazole-3-carboxamide (201 mg, 0.44 mmol, 1.0 eq) and 5 drops of AcOH. The reaction was stirred at rt for 2 h, and then sodium triacetoxyborohydride (465 mg, 2.19 mmol, 5.0 eq) was added. The reaction solution was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (off-white solid, 25.0 mg, yield 7%). LCMS (ESI): m/z 763 [M+H]⁺. **¹H NMR** (400 MHz, DMSO) *δ* 11.80 (s, 1H), 10.79 (s, 1H), 8.92 (s, 1H), 8.48-8.37 (m, 2H), 8.22 (d, *J* = 7.6 Hz, 1H), 8.14 (s, 0.3HCOOH), 7.96 (s, 1H), 7.85 (d, *J =* 8.8 Hz, 2H), 7.74 (s, 1H), 7.16 (d, *J =* 8.9 Hz, 2H), 6.87 (t, *J =* 9.3 Hz, 1H), 6.52 (d, *J =* 14.9 Hz, 1H), 6.44 (d, *J =* 8.8 Hz, 1H), 5.85 (d, *J =* 7.8 Hz, 1H), 4.32-4.22 (m, 1H), 3.31-3.17 (m, 10H), 2.78-2.70 (m, 1H), 2.70-2.52 (m, 4H), 2.14-2.00 (m, 3H), 1.92-1.83 (m, 1H), 1.81-1.67 (m, 2H).

### Preparation Example 375. Synthesis of Compound A366

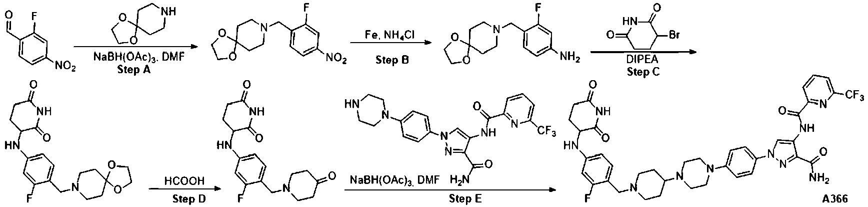

### Step A: 8-[(2-Fluoro-4-nitrophenyl)methyl]-8-aza-1,4-dioxaspiro[4.5]decane

At rt, 2-fluoro-4-nitrobenzaldehyde (2.3 g, 53.25 mmol, 1.0 eq) was dissolved in DCM (90 mL), and 2-fluoro-4-nitrobenzene-1-carboxaldehyde (8.18 g, 58.58 mmol, 1.1 eq) was added. The mixture was stirred at the same temperature for 1 h. Then NaBH₄ (6.0 g, 159.76 mmol, 3.0 eq) was added to the mixed solution. The reaction was stirred at rt for 2 h. Upon completion, the reaction solution was diluted in DCM, washed sequentially with water and saturated aqueous NaCl, dried over anhydrous Na₂SO₄, and concentrated under reduced pressure. The residue was purified by column chromatography to afford the title compound (yellow solid, 14.0 g, yield 89%).

### Step B: 3-Fluoro-4-(8-aza-1,4-dioxaspiro[4.5]decan-8-ylmethyl)aniline

At rt, iron powder (4.8 g, 86.49 mmol, 4.0 eq) and NH₄Cl (9.2 g, 172.97 mmol, 8.0 eq) were added to a solution of 8-[(2-fluoro-4-nitrophenyl)methyl]-8-aza-1,4-dioxaspiro[4.5]decane (6.4 g, 21.62 mmol, 1.0 eq) in water (10 mL) and ethanol (50 mL). The reaction solution was stirred at 60°C for 1 h. Upon completion, the reaction solution was filtered through Celite, washed sequentially with water and saturated aqueous NaCl, dried over anhydrous Na₂SO₄, and concentrated under reduced pressure to afford the title compound (yellow solid, 4.7 g, yield 82%).

### Step C: 3-{[3-Fluoro-4-(8-aza-1,4-dioxaspiro[4.5]decan-8-ylmethyl)phenyl]amino}piperidine-2,6-dione

At rt, to a solution of 3-fluoro-4-(8-aza-1,4-dioxaspiro[4.5]decan-8-ylmethyl)aniline (4.7 g, 17.67 mmol, 1.0 eq) in 1,4-dioxane were added 3-bromopiperidine-2,6-dione (6.8 g, 35.34 mmol, 2.0 eq) and DIEA (9.2 g, 52.9 mmol, 3.0 eq). The reaction solution was stirred at 80°C for 12 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 1.3 g, yield 20%).

### Step D: 3-({3-Fluoro-4-[(4-oxopiperidin-1-yl)methyl]phenyl}amino)piperidine-2,6-dione

At rt, a solution of 3-{[3-fluoro-4-(8-aza-1,4-dioxaspiro[4.5]decan-8-ylmethyl)phenyl]amino}piperidine-2,6-dione (400 mg, 1.06 mmol, 1.0 eq) in formic acid (6 mL) was stirred at 80°C for 4 h. Upon completion, the reaction solution was filtered through Celite, washed sequentially with water and saturated aqueous NaCl, dried over anhydrous Na₂SO₄, and concentrated under reduced pressure to afford the title compound (yellow oil, 500 mg, yield 68%).

### Step E: 1-(4-{4-[1-({4-[(2,6-dioxopiperidin-3-yl)amino]-2-fluorophenyl}methyl)piperidin-4-yl]piperazin-1-yl}phenyl)-4-({[6-(trifluoromethyl)pyridin-2-yl]carbonyl}amino)pyrazole-3-carboxamide

At rt, to 3-({3-fluoro-4-[(4-oxopiperidin-1-yl)methyl]phenyl}amino)piperidine-2,6-dione (200 mg, 0.6 mmol, 1.0 eq) and 1-[4-(piperazin-1-yl)phenyl]-4-({[6-(trifluoromethyl)pyridin-2-yl]carbonyl}amino)pyrazole-3-carboxamide (276 mg, 0.6 mmol, 1.0 eq). The reaction solution was stirred at 60°C for 1 h, then NaBH(OAc)₃ (506 mg, 2.4 mmol, 4.0 eq) was added, and the reaction solution was stirred at rt for 2 h. The mixture was diluted in EtOAc, washed sequentially with water and saturated aqueous NaCl, dried over anhydrous Na₂SO₄, and concentrated under reduced pressure. The residue was purified by column chromatography to afford the crude, which was further purified by HPLC to afford the title compound (white solid, 500 mg, yield 68%). LCMS (ESI): m/z 777.5[M+H]⁺. **¹H NMR** (400 MHz, DMSO-*d₆*) *δ* 11.79 (s, 1H), 10.80 (s, 1H), 8.87 (s, 1H), 8.45 (d, *J*= 7.6 Hz, 1H), 8.39 (t, *J =* 7.8 Hz, 1H), 8.21 (d, *J* = 7.7 Hz, 1H), 7.94 (s, 1H), 7.77 (d, *J=* 9.0 Hz, 2H), 7.70 (s, 1H), 7.08-7.00 (m, 3H), 6.50-6.41 (m, 2H), 6.13 (d, *J = 7.7* Hz, 1H), 4.37-4.29 (m, 1H), 3.35 (s, 2H), 3.20-3.17 (m, 4H), 2.88-2.83 (m, 2H), 2.78-2.71 (m, 1H), 2.65-2.55 (m, 5H), 2.24-2.18 (m, 1H), 2.13-2.06 (m, 1H), 1.99-1.88 (m, 3H), 1.79-1.72 (m, 2H), 1.46-1.37 (m, 2H).

### Preparation Example 376. Synthesis of Compound A367

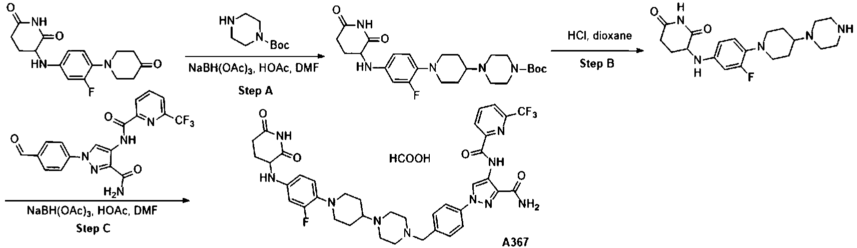

### Step A: Tert-butyl 4-(1-(4-((2,6-dioxopiperidin-3-yl)amino)-2-fluorophenyl)piperidin-4-yl)piperazine-1-carboxylate

At rt, to a solution of 3-((3-fluoro-4-(4-oxopiperidin-1-yl)phenyl)amino)piperidine-2,6-dione (500 mg, 1.57 mmol, 1.0 eq) in DMF (10 mL) were added tert-butyl piperazine-1-carboxylate (292 mg, 1.57 mmol, 1.0 eq) and 3 drops of AcOH. The reaction was stirred at rt for 2 h, and then sodium triacetoxyborohydride (660 mg, 3.13 mmol, 2.0 eq) was added. The reaction solution was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 300 mg, yield 39%).

### Step B: 3-((3-Fluoro-4-(4-(piperazin-1-yl)piperidin-1-yl)phenyl)amino)piperidine-2,6-dione

At rt, a 4 M hydrogen chloride in dioxane solution (2 mL) was added to a solution of tert-butyl 4-(1-(4-((2,6-dioxopiperidin-3-yl)amino)-2-fluorophenyl)piperidin-4-yl)piperazine-1-carboxylate (300 mg, 0.61 mmol, 1.0 eq) in acetonitrile (10 mL). The reaction solution was stirred at rt for 1 h. Upon completion, the reaction solution was concentrated under reduced pressure to afford the title compound (white solid, 240 mg, crude), which was used directly without purification.

### Step C: 1-(4-{[4-(1-{4-[(2,6-Dioxopiperidin-3-yl)amino]-2-fluorophenyl}piperidin-4-yl)piperazin-1-yl]methyl}phenyl)-4-({[6-(trifluoromethyl)pyridin-2-yl]carbonyl}amino)pyrazole-3-carboxamide formate

At rt, to a solution of 3-((3-fluoro-4-(4-(piperazin-1-yl)piperidin-1-yl)phenyl)amino)piperidine-2,6-dione (100 mg, crude) in DMF (5 mL) were added N-(3-carbamoyl-1-(4-formylphenyl)-1H-pyrazol-4-yl)-6-(trifluoromethyl)picolinamide (103 mg, 0.26 mmol, 1.0 eq) and 3 drops of AcOH. The mixture was stirred at rt for 2 h, and then sodium triacetoxyborohydride (109 mg, 0.52 mmol, 2.0 eq) was added. The reaction solution was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (gray solid, 25 mg, yield 12%). LCMS (ESI): m/z 777 [M+H]⁺. **¹H NMR** (400 MHz, DMSO) *δ* 11.80 (s, 1H), 10.77 (s, 1H), 9.01 (s, 1H), 8.50-8.36 (m, 2H), 8.25-8.19 (m, 1H), 8.17 (s, 1.3HCOOH), 8.02 (s, 1H), 7.97-7.91 (m, 2H), 7.78 (s, 1H), 7.56-7.43 (m, 2H), 6.82 (t, *J =* 9.3 Hz, 1H), 6.57-6.45 (m, 1H), 6.44-6.35 (m, 1H), 5.79 (d, *J* = 7.6 Hz, 1H), 4.27-4.23 (m, 1H), 3.53 (s, 2H), 3.19-3.12 (m, 2H), 2.78-2.62 (m, 2H), 2.62-2.53 (m, 6H), 2.49-2.27 (m, 5H), 2.12-2.04 (m, 1H), 1.90-1.78 (m, 3H), 1.61-1.48 (m, 2H).

### Preparation Example 377. Synthesis of Compound A368

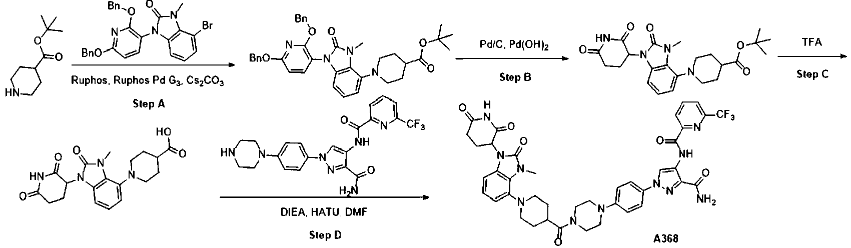

### Step A: Tert-butyl 1-(1-(2,6-bis(benzyloxy)pyridin-3-yl)-3-methyl-2-oxo-2,3-dihydro-1H-benzo[d]imidazol-4-yl)piperidine-4-carboxylate

At rt, to a mixture of tert-butyl piperidine-4-carboxylate (323 mg, 1.74 mmol, 1.5 eq), 1-(2,6-bis(benzyloxy)pyridin-3-yl)-4-bromo-3-methyl-1,3-dihydro-2H-benzo[d]imidazol-2-one (600 mg, 1.16 mmol, 1.0 eq), and cesium carbonate (1136 mg, 3.49 mmol, 3.0 eq) in anhydrous 1,4-dioxane (5 mL) were added RuPhos (108 mg, 0.23 mmol, 0.2 eq) and RuPhos Pd G3 (97.3 mg, 0.12 mmol, 0.1 eq). The reaction solution was stirred at 100°C under N₂ for 18 h. Upon completion, the reaction solution was concentrated under reduced pressure, dried, diluted in EtOAc, washed sequentially with water and saturated aqueous NaCl, and concentrated under reduced pressure. The residue was purified by column chromatography to afford the title compound (yellow solid, 220 mg, yield 30.5%).

### Step B: Tert-butyl 1-(1-(2,6-dioxopiperidin-3-yl)-3-methyl-2-oxo-2,3-dihydro-1H-benzo[d]imidazol-4-yl)piperidine-4-carboxylate

At rt, to a mixed solution of tert-butyl 1-(1-(2,6-bis(benzyloxy)pyridin-3-yl)-3-methyl-2-oxo-2,3-dihydro-1H-benzo[d]imidazol-4-yl)piperidine-4-carboxylate (220 mg, 0.35 mmol, 1.0 eq) THF (20 mL) and DMF (5 mL) were added palladium hydroxide on carbon (70 mg) and palladium on carbon (70 mg). The reaction was stirred under a hydrogen balloon atmosphere at rt for 18 h. LCMS indicated the reaction was incomplete. The reaction solution was filtered, and additional palladium hydroxide on carbon (70 mg) and palladium on carbon (70 mg) were added. The reaction was stirred under a hydrogen balloon at rt for 5 h. The reaction solution was filtered, and the filtrate was concentrated under reduced pressure and dried. The solid was slurried with diethyl ether, filtered, and dried under reduced pressure to afford the title compound (white solid, 120 mg, yield 76.5%).

### Step C: 1-(1-(2,6-dihydroxypyridin-3-yl)-3-methyl-2-oxo-2,3-dihydro-1H-benzo[d]imidazol-4-yl)piperidine-4-carboxylic acid

At rt, tert-butyl 1-(1-(2,6-dioxopiperidin-3-yl)-3-methyl-2-oxo-2,3-dihydro-1H-benzo[d]imidazol-4-yl)piperidine-4-carboxylate (100 mg, 0.226 mmol, 1.0 eq), TFA (3 mL), and DCM (6 mL) were added to a reaction flask. The mixture was stirred at rt for 3 h. Upon completion, the reaction solution was concentrated under reduced pressure and dried. The solid was slurried with diethyl ether, filtered, and dried under reduced pressure to afford the title compound (white solid, 110 mg, yield 126%).

### Step D: N-(3-Carbamoyl-1-(4-(4-(1-(1-(2,6-dioxopiperidin-3-yl)-3-methyl-2-oxo-2,3-dihydro-1H-benzo[d]imidazol-4-yl)piperidine-4-carbonyl)piperazin-1-yl)phenyl)-1H-pyrazol-4-yl)-6-(trifluoromethyl)picolinamide

At rt, to a solution of 1-(1-(2,6-dihydroxypyridin-3-yl)-3-methyl-2-oxo-2,3-dihydro-1H-benzo[d]imidazol-4-yl)piperidine-4-carboxylic acid (110 mg, 0.29 mmol, 1.0 eq), N-(3-carbamoyl-1-(4-(piperazin-1-yl)phenyl)-1H-pyrazol-4-yl)-6-(trifluoromethyl)picolinamide (131 mg, 0.29 mmol, 1.0 eq), and DIEA (184 mg, 1.42 mmol, 5.0 eq) in DMF (5 mL) was added HATU (162 mg, 0.43 mmol, 1.5 eq). The reaction was stirred at rt for 1 h. Upon completion, the reaction solution was concentrated under reduced pressure, dried, diluted in DCM, washed sequentially with water and saturated aqueous NaCl, and concentrated under reduced pressure. The residue was purified by HPLC to afford the title compound (white solid, 52 mg, yield 22%). **¹H NMR** (400 MHz, DMSO-*d*₆) *δ* 11.80 (s, 1H), 11.09 (s, 1H), 8.91 (s, 1H), 8.48-8.37 (m, 2H), 8.22 (d, *J =* 7.6 Hz, 1H), 7.96 (s, 1H), 7.83 (d, *J* = 9.0 Hz, 2H), 7.72 (s, 1H), 7.13 (d, *J* = 9.2 Hz, 2H), 7.00 (t, *J =* 7.9 Hz, 1H), 6.92-6.88 (m, 2H), 5.36 (dd, *J =* 12.5, 5.3 Hz, 1H), 3.74-3.70 (m, 4H), 3.65 (s, 3H), 3.20-3.14 (m, 7H), 2.93-2.79 (m, 4H), 2.74-2.60 (m, 2H), 1.90-1.76 (m, 4H). LCMS (ESI): m/z 828.4 [M+H]⁺.

### Preparation Example 378. Synthesis of Compound A369

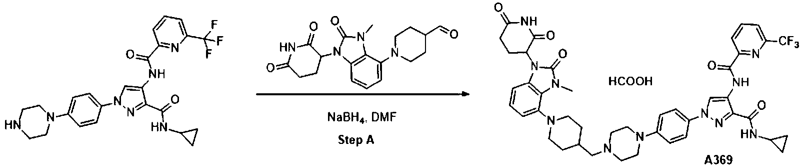

### Step A: N-{3-[(cyclopropylamino)carbonyl]-1-{4-[4-({1-[1-(2,6-dioxopiperidin-3-yl)-3-methyl-2-oxobenzo[d]imidazol-4-yl]piperidin-4-yl}methyl)piperazin-1-yl]phenyl}pyrazol-4-yl}-6-(trifluoromethyl)pyridine-2-carboxamide formate

To a solution of N-cyclopropyl-1-[4-(piperazin-1-yl)phenyl]-4-({[6-(trifluoromethyl)pyridin-2-yl]carbonyl}amino)pyrazole-3-carboxamide (140 mg, 0.28 mmol, 1.0 eq) and 1-[1-(2,6-dioxopiperidin-3-yl)-3-methyl-2-oxobenzo[d]imidazol-4-yl]piperidine-4-carbaldehyde (104 mg, 0.28 mmol, 1.0 eq) in DMF (5 mL) was added AcOH (0.2 mL, 3.36 mmol, 12.0 eq). The mixture was stirred at rt for 1 h. Then sodium borohydride (42 mg, 1.12 mmol, 4.0 eq) was added, and the mixture was stirred at rt for 2 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (off-white solid, 25.0 mg, yield 10%). LCMS (ESI): m/z 854 [M+H]⁺. **¹H NMR** (400 MHz, DMSO) *δ* 11.61 (s, 1H), 11.09 (s, 1H), 8.90 (s, 1H), 8.52 (d, *J =* 4.5 Hz, 1H), 8.48-8.45 (m, 1H), 8.40 (t, *J* = 7.8 Hz, 1H), 8.24-8.21 (m, 1H), 8.16 (s, 1H), 7.80 (d, *J =* 9.0 Hz, 2H), 7.07 (d, *J* = 9.1 Hz, 2H), 7.00-6.96 (m, 1H), 6.92-6.85 (m, 2H), 5.35 (dd, *J* = 12.6, 5.2 Hz, 1H), 3.63 (s, 3H), 3.24-3.22 (m, 4H), 3.13 (d, *J* = 10.2 Hz, 2H), 2.97-2.94 (m, 1H), 2.90-2.85 (m, 1H), 2.75-2.64 (m, 4H), 2.60-2.53 (m, 4H), 2.31-2.25 (m, 2H), 2.02-1.97 (m, 1H), 1.88-1.83 (m, 2H), 1.74-1.65 (m, 1H), 1.40-1.29 (m, 2H), 0.75-0.69 (m, 4H).

### Preparation Example 379. Synthesis of Compound A370

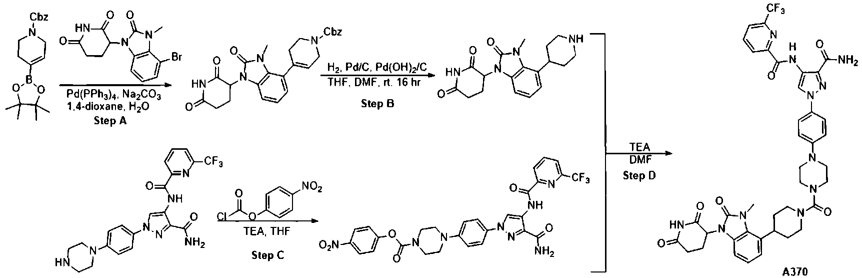

### Step A: Benzyl 4-(1-(2,6-dioxopiperidin-3-yl)-3-methyl-2-oxo-2,3-dihydro-1H-benzo[d]imidazol-4-yl)-3,6-dihydropyridine-1(2H)-carboxylate

At rt, to a solution of benzyl 4-(4,4,5,5-tetramethyl-1,3,2-dioxaborolan-2-yl)-1,2,3,6-tetrahydropyridin-1-carboxylate (300 mg, 0.87 mmol, 1.5 eq) in 1,4-dioxane (3 mL) and water (1 mL) were added 1-[2,6-bis(benzyloxy)pyridin-3-yl]-4-bromo-3-methyl-2,3-dihydro-1H-benzo[d]imidazol-2-one (300 mg, 0.58 mmol, 1.0 eq), Pd(PPh₃)₄ (67.3 mg, 0.06 mmol, 0.1 eq), and Na₂CO₃ (185 mg, 1.75 mmol, 3.0 eq). The reaction solution was stirred at 80°C under N₂ overnight. Upon completion, water was added to the mixture, and the resulting mixture was extracted with EtOAc. The combined organic layers were washed with saturated aqueous NaCl, dried over anhydrous Na₂SO₄, and concentrated under reduced pressure. The residue was purified by column chromatography (eluent: petroleum ether/ethyl acetate, gradient: 0%-30% ethyl acetate) to afford the title compound (white solid, 270 mg, yield 71%).

### Step B: 3-(3-Methyl-2-oxo-4-(piperidin-4-yl)-2,3-dihydro-1H-benzo[d]imidazol-1-yl)piperidine-2,6-dione

A mixture of benzyl 4-(1-(2,6-bis(benzyloxy)pyridin-3-yl)-3-methyl-2-oxo-2,3-dihydro-1H-benzo[d]imidazol-4-yl)-3,6-dihydropyridine-1(2H)-carboxylate (270 mg, 0.414 mmol, 1.0 eq) were dissolved in DMF (3 mL) and THF (3 mL), followed by the addition of Pd/C (10 wt%, 4 mg) and Pd(OH)₂/C (10 wt%, 6 mg) under H₂ at rt. The reaction system was purged with H₂ (×3) and then stirred under H₂ (1 atm) overnight. The reaction solution was filtered, and the filtrate was concentrated under reduced pressure to afford the title compound (yellow solid, 140 mg, yield 99%).

### Step C: 4-Nitrophenyl 4-(4-(3-carbamoyl-4-(6-(trifluoromethyl)pyridinylamino)-1H-pyrazol-1-yl)phenyl)piperazine-1-carboxylate

To a solution of N-(3-carbamoyl-1-(4-(piperazin-1-yl)phenyl)-1H-pyrazol-4-yl)-6-(trifluoromethyl)picolinamide (300 mg, 0.65 mmol, 1.0 eq) in THF (3 mL) were sequentially added TEA (198 mg, 1.96 mmol, 3.0 eq) and 4-nitrophenyl carbonochloridate (395 mg, 1.96 mmol, 3.0 eq) at 0°C. The mixture was stirred at rt for 3 h. Upon completion, water was added, and the mixture was extracted with EtOAc. The combined organic layers were washed with aqueous NaCl, dried over anhydrous Na₂SO₄, and concentrated under reduced pressure. The residue was purified by column chromatography (eluent: petroleum ether/ethyl acetate, gradient: 0%-20% ethyl acetate) to afford the title compound (yellow solid, 130 mg, yield 32%).

### Step D: N-(3-Carbamoyl-1-(4-(4-(1-(2,6-dioxopiperidin-3-yl)-3-methyl-2-oxo-2,3-dihydro-1H-benzo[d]imidazol-4-yl)piperidine-1-carbonyl)piperazin-1-yl)phenyl)-1H-pyrazol-4-yl)-6-(trifluoromethyl)picolinamide

To a solution of 3-(3-methyl-2-oxo-4-(piperidin-4-yl)-2,3-dihydro-1H-benzo[d]imidazol-1-yl)piperidine-2,6-dione (70 mg, 0.20 mmol, 1.0 eq) in DMF (3 mL) were added TEA (0.1 mL, 0.61 mmol, 3.0 eq) and 4-nitrophenyl 4-(4-(3-carbamoyl-4-(6-(trifluoromethyl)pyridinylamino)-1H-pyrazol-1-yl)phenyl)piperazine-1-carboxylate (125 mg, 0.20 mmol, 1.0 eq). The mixture was stirred at 90°C overnight. The reaction solution was concentrated under reduced pressure, and the crude was purified by preparative HPLC (C₁₈, 20%-95% acetonitrile in water with 0.1% FA) to afford the title compound (yellow solid, 35 mg, yield 21%). LCMS (ESI): m/z 827.8 [M+H]⁺. **¹H NMR** (400 MHz, DMSO-*d*₆) *δ* 11.79 (s, 1H), 11.25-11.02 (m, 1H), 8.90 (s, 1H), 8.56-8.31 (m, 2H), 8.21 (d, *J=* 7.4 Hz, 1H), 7.96 (s, 1H), 7.82 (d, *J=* 9.1 Hz, 2H), 7.71 (s, 1H), 7.12 (d, *J* = 9.3 Hz, 2H), 7.03-6.97 (m, 3H), 5.52-5.12 (m, 1H), 3.80 *(d, J=* 11.9 Hz, 2H), 3.62 (s, 3H), 3.27-3.25 (m, 4H), 3.01-2.95 (m, 4H), 2.79-2.64 (m, 3H), 2.07 (s, 2H), 2.03-2.01 (m, 2H), 1.88-1.81 (m, 2H), 1.77 m 1.66 (m, 2H).

### Preparation Example 380. Synthesis of Compound A371

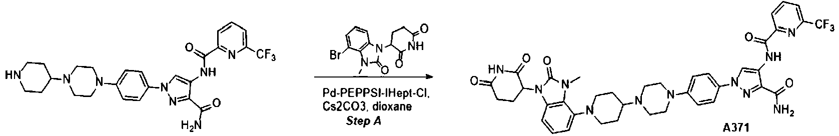

### Step A: N-(3-Carbamoyl-1-(4-(4-(1-(1-(2,6-Dioxopiperidin-3-yl)-3-methyl-2-oxo-2,3-dihydro-1H-benzo[d]imidazol-4-yl)piperidin-4-yl)piperazin-1-yl)phenyl)-1H-pyrazol-4-yl)-6-(trifluoromethyl)picolinamide bis(2,2,2-trifluoroacetate)

To a mixture of 1-{4-[4-(piperidin-4-yl)piperazin-1-yl]phenyl}-4-({[6-(trifluoromethyl)pyridin-2-yl]carbonyl}amino)pyrazole-3-carboxamide (100 mg, 0.18 mmol, 1.0 eq) in dioxane (10 mL) were added 3-(4-bromo-3-methyl-2-oxobenzo[d]imidazol-1-yl)piperidine-2,6-dione (68.5 mg, 0.2 mmol, 1.2 eq), Pd PEPPSI IHept Cl (14.7 mg, 0.018 mmol, 0.1 eq), and cesium carbonate (180 mg, 0.553 mmol, 3.0 eq). The mixture was stirred at 110°C for 16 h. The reaction mixture was poured into AcOH solution and concentrated in vacuo to give a residue. The residue was purified by HPLC to afford the title compound, N-(3-carbamoyl-1-(4-(4-(1-(1-(2,6-dioxopiperidin-3-yl)-3-methyl-2-oxo-2,3-dihydro-1H-benzo[d]imidazol-4-yl)piperidin-4-yl)piperazin-1-yl)phenyl)-1H-pyrazol-4-yl)-6-(trifluoromethyl)picolinamide bis(2,2,2-trifluoroacetate) (5.7 mg, trifluoroacetate, 3%). **¹H NMR** (400 MHz, DMSO) *δ* 11.42 (s, 1H), 11.14 (s, 1H), 11.07-10.97 (m, 1H), 10.45 (s, 1H), 9.06 (s, 1H), 8.95 (d, *J* = 9.1 Hz, 1H), 8.79 (s, 1H), 8.47 (d, *J* = 7.6 Hz, 1H), 8.41 (t,*J* = 7.8 Hz, 1H), 8.22 (d, *J* = 7.6 Hz, 1H), 7.97 *(d, J =* 9.0 Hz, 2H), 7.22 (d, *J* = 9.1 Hz, 2H), 7.18-7.13 (m, 1H), 7.10 (dd, *J =* 7.1, 4.9 Hz, 2H), 5.45 (dd, *J =* 12.7, 5.3 Hz, 1H), 3.98 (s, 3H), 3.49 (s, 6H), 3.22 (s, 4H), 3.03-2.86 (m, 4H), 2.84-2.71 (m, 1H), 2.66 (d, *J =* 16.4 Hz, 1H), 2.34 (d, *J =* 11.7 Hz, 2H), 2.09 (d, *J =* 6.8 Hz, 1H), 1.95 *(d, J =* 13.2 Hz, 2H). LCMS (ESI): m/z 800 [M+H]⁺.

### Preparation Example 381. Synthesis of Compound A372

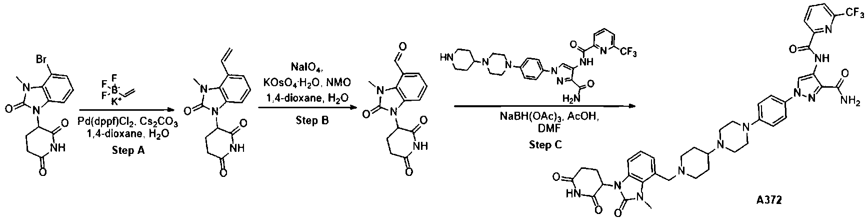

### Step A: 3-(3-Methyl-2-oxo-4-vinyl-2,3-dihydro-1H-benzo[d]imidazol-1-yl)piperidine-2,6-dione

To a solution of 3-(4-bromo-3-methyl-2-oxo-2,3-dihydro-1H-benzo[d]imidazol-1-yl)piperidine-2,6-dione (300 mg, 0.89 mmol, 1.0 eq) and potassium vinyltrifluoroborate (357 mg, 2.67 mmol, 3.0 eq) in 1,4-dioxane (5 mL) and water (1 mL) were added Pd(dppf)Cl₂ (65 mg, 0.09 mmol, 0.1 eq) and cesium carbonate (578 mg, 1.78 mmol, 3.0 eq) sequentially at rt. The reaction mixture was purged with N₂ (×3), and then stirred at 90°C under N₂ for 16 h. The mixture was diluted in water and extracted with EtOAc. The organic phase was washed with saturated aqueous NaCl, dried over anhydrous Na₂SO₄, filtered, and concentrated under reduced pressure. The residue was purified by column chromatography to afford the title compound (white solid, 150 mg, yield 59%).

### Step B: 1-(2,6-Dioxopiperidin-3-yl)-3-methyl-2-oxo-2,3-dihydro-1H-benzimidazole-4-carbaldehyde

To a solution of 3-(3-methyl-2-oxo-4-vinyl-2,3-dihydro-1H-benzo[d]imidazol-1-yl)piperidine-2,6-dione (130 mg, 0.46 mmol, 1.0 eq) in 1,4-dioxane (4 mL) and water (0.5 mL) were added osmium oxide dihydrate (168 mg, 0.46 mmol, 1.0 eq) and N-methylmorpholine N-oxide (27 mg, 0.23 mmol, 0.5 eq) at rt. The mixture was stirred at rt for 5 min, and then sodium periodate (197 mg, 0.91 mmol, 2.0 eq) was added. The reaction solution was stirred at rt for 16 h. The reaction solution was diluted in EtOAc, washed sequentially with saturated aqueous sodium sulfite solution and saturated aqueous NaCl, dried over anhydrous Na₂SO₄, filtered, and concentrated under reduced pressure. The residue was purified by column chromatography (eluent: ethyl acetate/petroleum ether, gradient: 0%-50% ethyl acetate) to afford the title compound (brown solid, 67.1 mg, yield 51%).

### Step C: N-(3-Carbamoyl-1-(4-(4-((1-((1-(2,6-dioxopiperidin-3-yl)-3-methyl-2-oxo-2,3-dihydro-1H-benzo[d]imidazol-4-yl)methyl)piperidin-4-yl)piperazin-1-yl)phenyl)-1H-pyrazol-4-yl)-6-(trifluoromethyl)picolinamide

At rt, to a solution of N-(3-carbamoyl-1-(4-(4-(piperidin-4-yl)piperazin-1-yl)phenyl)-1H-pyrazol-4-yl)-6-(trifluoromethyl)picolinamide (27 mg, 0.09 mmol, 1.0 eq) and 1-(2,6-dioxopiperidin-3-yl)-3-methyl-2-oxo-2,3-dihydro-1H-benzimidazole-4-carbaldehyde (26 mg, 0.09 mmol, 1.0 eq) in DMF (2 mL) was added AcOH (0.01 mL, 0.02 mmol, 0.2 eq), followed by the addition of sodium triacetoxyborohydride (57 mg, 0.27 mmol, 3.0 eq). The mixture was stirred at rt under N₂ for 0.5 h. The mixture was quenched with saturated aqueous NaHCO₃ in an ice-water bath and extracted with DCM twice. The organic phases were combined, washed with saturated aqueous NaCl, dried over anhydrous Na₂SO₄, filtered, and concentrated under reduced pressure. The residue was purified by preparative HPLC (C₁₈, 20%-95% acetonitrile in water containing 0.1% aqueous ammonia) to afford the title compound (white solid, 26.1 mg, yield 36%). LCMS (ESI): m/z 814.4 [M+H]⁺. **¹H NMR** (400 MHz, DMSO-*d*₆) *δ* 11.79 (s, 1H), 11.07 (s, 1H), 8.87 (s, 1H), 8.48-8.33 (m, 2H), 8.21 (d, *J =* 7.6 Hz, 1H), 7.94 (s, 1H), 7.77 (d, *J =* 9.1 Hz, 2H), 7.70 (s, 1H), 7.08-7.04 (m, 3H), 6.96 (t, *J =* 7.7 Hz, 1H), 6.88 (d, *J =* 7.4 Hz, 1H), 5.49-5.27 (m, 1H), 3.69 (s, 3H), 3.63 (s, 2H), 3.21-3.14 (m, 4H), 2.98-2.82 (m, 3H), 2.73-2.55 (m, 6H), 2.33-2.26 (m, 1H), 2.02-1.97 (m, 3H), 1.78-1.75 (m, 2H), 1.44-1.39 (m, 2H).

### Preparation Example 382. Synthesis of Compound A373

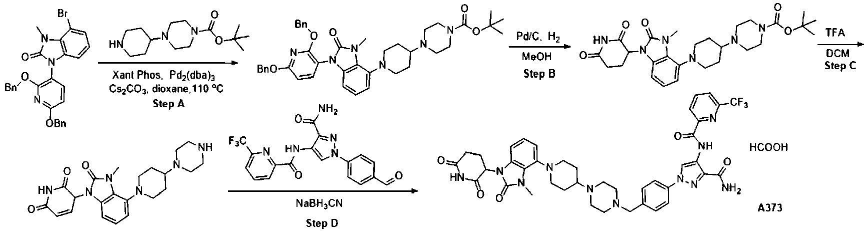

### Step A: Tert-butyl 4-(1-(1-(2,6-bis(benzyloxy)pyridin-3-yl)-3-methyl-2-oxo-2,3-dihydro-1H-benzo[d]imidazol-4-yl)piperidin-4-yl)piperazine-1-carboxylate

To a solution of 1-[2,6-bis(benzyloxy)pyridin-3-yl]-4-bromo-3-methyl-2,3-dihydro-1H-benzo[d]imidazol-2-one (1.0 g, 1.937 mmol, 1.0 eq) in dioxane (20 mL) were added 2-methylpropan-2-yl-4-(piperidin-4-yl)piperazine-1-carboxylate (0.63 g, 2.324 mmol, 1.2 eq), Xant Phos (0.22 g, 0.387 mmol, 0.2 eq), Cs₂CO₃ (1.26 g, 3.873 mmol, 2.0 eq), and Pd₂(dba)₃ (0.18 g, 0.194 mmol, 0.1 eq) at rt. The reaction solution was stirred at 110°C under N₂ for 10 h. Upon completion, the reaction solution was concentrated under reduced pressure. The residue was purified by column chromatography to afford the title compound (yellow solid, 500 mg, yield 36%).

### Step B: Tert-butyl 4-(1-(1-(2,6-dioxopiperidin-3-yl)-3-methyl-2-oxo-2,3-dihydro-1H-benzo[d]imidazol-4-yl)piperidin-4-yl)piperazine-1-carboxylate

To a solution of tert-butyl 4-(1-(1-(2,6-bis(benzyloxy)pyridin-3-yl)-3-methyl-2-oxo-2,3-dihydro-1H-benzo[d]imidazol-4-yl)piperidin-4-yl)piperazine-1-carboxylate (250 mg, 0.355 mmol, 1.2 eq) in MeOH (20 mL) was added 10% wet Pd/C (75 mg, 0.071 mmol, 0.2 eq) at rt. The reaction solution was stirred under a hydrogen balloon at 60°C for 10 h. Upon completion, the reaction solution was filtered and concentrated under reduced pressure to afford the title compound (yellow oil, 130 mg, yield 69%, crude), which was used directly without purification.

### Step C: 3-(3-Methyl-2-oxo-4-(4-(piperazin-1-yl)piperidin-1-yl)-2,3-dihydro-1H-benzo[d]imidazol-1-yl)pyridine-2,6(1H,3H)-dione

A solution of tert-butyl 4-(1-(1-(2,6-dioxopiperidin-3-yl)-3-methyl-2-oxo-2,3-dihydro-1H-benzo[d]imidazol-4-yl)piperidin-4-yl)piperazine-1-carboxylate (130 mg, 0.247 mmol, 1.0 eq) in TFA/DCM (1/4, 5 mL) was stirred at rt under N₂ for 1 h. Upon completion, the reaction solution was concentrated under reduced pressure to afford the title compound (yellow oil, 100 mg, crude), which was used directly without purification.

### Step D: N-(3-Carbamoyl-1-(4-(4-(1-(1-(2,6-dioxopiperidin-3-yl)-3-methyl-2-oxo-2,3-dihydro-1H-benzo[d]imidazol-4-yl)piperidin-4-yl)piperazin-1-yl)methyl)phenyl)-1H-pyrazol-4-yl)-6-(trifluoromethyl)picolinamide formate

To a solution of 3-(3-methyl-2-oxo-4-(4-(piperazin-1-yl)pyridin-1-yl)-2,3-dihydro-1H-benzo[d]imidazol-1-yl)piperidine-2,6(1H,3H)-dione (100 mg, crude) in MeOH (10 mL) were added 1-(4-formylphenyl)-4-({[6-(trifluoromethyl)pyridin-2-yl]carbonyl}amino)pyrazole-3-carboxamide (114 mg, 0.283 mmol, 1.2 eq) and sodium cyanoborohydride (44 mg, 0.707 mmol, 3.0 eq) at rt. The reaction solution was stirred at rt under N₂ for 10 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 50 mg, yield 26% over two steps). **¹H NMR** (400 MHz, DMSO) *δ* 11.80 (s, 1H), 11.09 (s, 1H), 9.01 (s, 1H), 8.49-8.37 (m, 2H), 8.22 *(d, J =* 7.6 Hz, 1H), 8.15 (s, 1H), 8.02 (s, 1H), 7.95 (d, *J =* 8.2 Hz, 2H), 7.78 (s, 1H), 7.48 (d, *J =* 8.2 Hz, 2H), 7.02-6.84 (m, 3H), 5.37-5.29 (m, 1H), 3.62 (s, 3H), 3.56 (s, 2H), 3.17-3.13 (m, 2H), 2.92-2.85 (m, 1H), 2.74-2.60 (m, 8H), 2.48-2.41 (m, 4H), 2.03-1.89 (m, 4H), 1.71-1.58 (m, 2H). LCMS (ESI): m/z 813.8 [M+H]⁺.

### Preparation Example 383. Synthesis of Compound A374

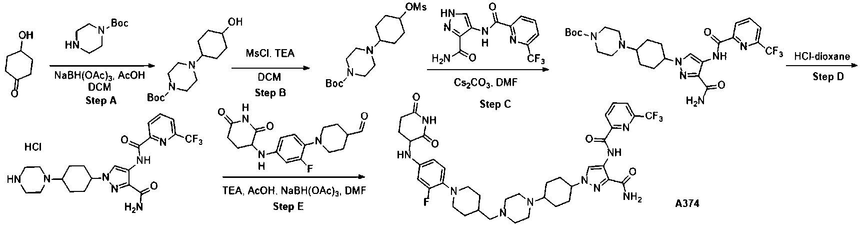

### Step A: Tert-butyl 4-(4-hydroxycyclohexyl)piperazine-1-carboxylate

To a solution of tert-butyl piperazine-1-carboxylate (3.3 g, 17.5 mmol, 1.0 eq) and 4-hydroxycyclohexan-1-one (2.0 g, 17.5 mmol, 1.0 eq) in DCM (30 mL) was added AcOH (3 mL, 52.6 mmol, 3.0 eq) at rt. The mixture was stirred at rt for 15 h. Then sodium triacetoxyborohydride (5.5 g, 26.3 mmol, 1.5 eq) was added to the reaction solution, and the mixture was stirred for 1 h. Upon completion, the reaction solution was filtered, and the filtrate was concentrated. The residue was purified by column chromatography to afford the title compound (yellow solid, 2.0 g, yield 40%).

### Step B: Tert-butyl 4-(4-((methylsulfonyl)oxy)cyclohexyl)piperazine-1-carboxylate

To a solution of tert-butyl 4-(4-hydroxycyclohexyl)piperazine-1-carboxylate (739 mg, 2.6 mmol, 1.0 eq) in DCM (10 mL) were added methanesulfonyl chloride (357 mg, 3.1 mmol, 1.2 eq) and TEA (0.5 mL, 3.9 mmol, 1.5 eq) in an ice bath. The reaction solution was stirred in an ice bath for 3 h. The reaction solution was diluted in water and extracted with DCM. The organic phase was washed with saturated aqueous NaCl, dried over anhydrous Na₂SO₄, and filtered. The filtrate was concentrated to afford the title compound (white solid, 0.41 g, yield 44%).

### Step C: Tert-butyl 4-(4-(3-carbamoyl-4-(6-(trifluoromethyl)picolinamido)-1H-pyrazol-1-yl)cyclohexyl)piperazine-1-carboxylate

To a solution of 4-({[6-(trifluoromethyl)pyridin-2-yl]carbonyl}amino)-1H-pyrazole-3-carboxamide (780 mg, 2.6 mmol, 1.0 eq) in DMF (15 mL) were added tert-butyl 4-(4-((methylsulfonyl)oxy)cyclohexyl)piperazine-1-carboxylate (945 mg, 2.6 mmol, 1.0 eq) and cesium carbonate (1.3 g, 3.9 mmol, 1.5 eq) at rt. The reaction solution was stirred at 70°C for 18 h. Upon completion, the reaction solution was cooled, diluted in water, and extracted with DCM. The organic phase was washed sequentially with saturated aqueous NaCl, dried over anhydrous Na₂SO₄, filtered, and concentrated. The residue was purified by column chromatography to afford the title compound (white solid, 780 mg, yield 53%).

### Step D: N-(3-Carbamoyl-1-(4-(piperazin-1-yl)cyclohexyl)-1H-pyrazol-4-yl)-6-(trifluoromethyl)picolinamide hydrochloride

Tert-butyl 4-(4-(3-carbamoyl-4-(6-(trifluoromethyl)picolinamido)-1H-pyrazol-1-yl)cyclohexyl)piperazine-1-carboxylate (250 mg, 0.4 mmol, 1.0 eq) was added to hydrogen chloride-dioxane solution (4 mol/L, 5 mL), and the mixture was stirred at rt for 1 h. Upon completion, the reaction solution was concentrated to afford the title compound (white solid, 200 mg, crude).

### Step E: N-(3-Carbamoyl-1-(4-(4-(4-(2,6-dioxopiperidin-3-yl)amino)-2-fluorophenylpiperidin-4-yl)methyl)piperazin-1-yl)cyclohexyl)-1H-pyrazol-4-yl)-6-(trifluoromethyl)picolinamide

To a solution of 1-(4-((2,6-dioxopiperidin-3-yl)amino)-2-fluorophenyl)piperidine-4-carbaldehyde (147 mg, 0.4 mmol, 1.0 eq) and N-(3-carbamoyl-1-(4-(piperazin-1-yl)cyclohexyl)-1H-pyrazol-4-yl)-6-(trifluoromethyl)picolinamide hydrochloride (200 mg, crude) in DMF (5 mL) was added TEA (0.2 mL) at rt. The reaction solution was reacted at rt for 0.5 h. Then AcOH (0.3 mL) was added to the reaction solution, and the mixture was stirred for 15 h. Sodium triacetoxyborohydride (140 mg, 0.7 mmol, 1.5 eq) was added, and the mixture was stirred for 1 h. Upon completion, the reaction solution was diluted in water and extracted with DCM. The organic phase was concentrated, and then the residue was purified by HPLC to afford the title compound (white solid, 36 mg, yield 10%). **¹H NMR** (400 MHz, DMSO-*d*₆) *δ* 11.72 (s, 1H), 10.81 (s, 1H), 8.48 (s, 1H), 8.44-8.35 (m, 2H), 8.20 (d, *J =* 7.2 Hz, 1H), 7.60 (d, *J =* 10.6 Hz, 2H), 7.03 (s, 1H), 6.57 (d, *J* = 15.2 Hz, 1H), 6.49 (d, *J* = 8.4 Hz, 1H), 5.99-4.94 (m, 2H), 4.39-4.24 (m, 2H), 3.84-2.95 (m, 10H), 2.95-2.65 (m, 5H), 2.63-2.54 (m, 1H), 2.29-2.01 (m, 5H), 1.99-1.76 (m, 6H), 1.74-1.58 (m, 2H), 1.55-1.26 (m, 2H). LCMS (ESI): m/z 783 [M+H]⁺.

### Preparation Example 384. Synthesis of Compound A377

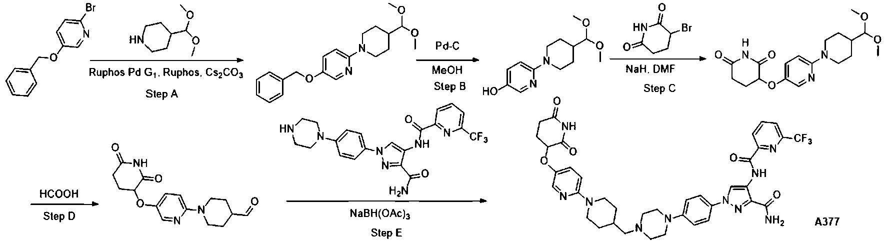

### Step A: 5-Benzyloxy-2-(4-(dimethoxymethyl)piperidin-1-yl)pyridine

At rt, to a solution of 5-(benzyloxy)-2-bromopyridine (5.0 g, 18.94 mmol, 1.0 eq) and 4-(dimethoxymethyl)piperidine (4.5 g, 28.41 mmol, 1.5 eq) in 1,4-dioxane (60 mL) were added Ruphos (1.8 g, 3.79 mmol, 0.2 eq), Ruphos Pd G1 (0.6 g, 2.65 mmol, 0.1 eq), and cesium carbonate (5.5 g, 56.82 mmol, 3.0 eq). The reaction solution was stirred at 90°C under N₂ for 16 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 2.7 g, yield 42%).

### Step B: 6-(4-(Dimethoxymethyl)piperidin-1-yl)pyridin-3-ol

To a solution of 5-benzyloxy-2-(4-(dimethoxymethyl)piperidin-1-yl)pyridine (2.7 g, 7.89 mmol, 1.0 eq) in MeOH (30 mL) was added 10% wet palladium on carbon (300 mg) at rt. The mixture was stirred under H₂ at rt for 16 h. Upon completion, the mixture was filtered through silica gel and concentrated under reduced pressure to afford the title compound (white solid, 1.8 g, yield 91%).

### Step C: 3-((6-(4-(Dimethoxymethyl)piperidin-1-yl)pyridin-3-yl)oxy)piperidine-2,6-dione

To a solution of 6-(4-(dimethoxymethyl)piperidin-1-yl)pyridin-3-ol (800 mg, 3.17 mmol, 1.0 eq) in DMF (15 mL) was added sodium hydride (60% in mineral oil, 380 mg, 6.35 mmol, 2.0 eq) at 0°C. The reaction solution was stirred at 0°C under N₂ for 30 min. Then, 3-bromo-2,6-dihydropyridone (913 mg, 4.76 mmol, 1.5 eq) was added to the reaction solution at 0°C under N₂. The reaction solution was stirred at rt under N₂ for 2 h. Upon completion, the reaction solution was quenched with ammonium chloride, diluted in EtOAc, washed sequentially with water and saturated aqueous NaCl, dried over anhydrous Na₂SO₄, and concentrated under reduced pressure. The residue was purified by column chromatography to afford the title compound (yellow solid, 200 mg, yield 17%).

### Step D: 1-(5-((2,6-Dioxopiperidin-3-yl)oxy)pyridin-2-yl)piperidine-4-carbaldehyde

At rt, 3-((6-(4-(dimethoxymethyl)piperidin-1-yl)pyridin-3-yl)oxy)piperidine-2,6-dione (200 mg, 0.55 mmol, 1.0 eq) was added to formic acid (5 mL), and the mixture was stirred at 50°C for 30 min. Upon completion, the reaction solution was concentrated under reduced pressure to afford the title compound (green oil, 160 mg, yield 92%), which was used directly without purification.

### Step E: N-(3-Carbamoyl-1-(4-(4-((1-(5-((2,6-dioxopiperidin-3-yl)oxy)pyridin-2-yl)piperidin-4-yl)methyl)piperazin-1-yl)phenyl)-1H-pyrazol-4-yl)-6-(trifluoromethyl)picolinamide

At rt, to a solution of 1-(5-((2,6-dioxopiperidin-3-yl)oxy)pyridin-2-yl)piperidine-4-carbaldehyde (160 mg, 0.50 mmol, 1.0 eq) in DMF (5 mL) were added N-(3-carbamoyl-1-(4-(piperazin-1-yl)phenyl)-1H-pyrazol-4-yl)-6-(trifluoromethyl)picolinamide (255 mg, 0.55 mmol, 1.0 eq) and AcOH (0.1 mL). The reaction was stirred at rt for 2 h, and then sodium triacetoxyborohydride (425 mg, 2.0 mmol, 4.0 eq) was added. The reaction solution was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (off-white solid, 26.0 mg, yield 7%). LCMS (ESI): m/z 761 [M+H]⁺. **¹H NMR**(400 MHz, DMSO-*d₆*) *δ* 11.80 (s, 1H), 10.91 (s, 1H), 8.88 (s, 1H), 8.50-8.33 (m, 2H), 8.21 (d, *J =* 7.2 Hz, 1H), 7.94 (d, *J* = 10.3 Hz, 2H), 7.81-7.68 (m, 3H), 7.32 (d, *J =* 9.2 Hz, 1H), 7.08 (d, *J =* 8.1 Hz, 2H), 6.79 (d, *J =* 8.9 Hz, 1H), 4.99 (dd, *J* = 10.5, 4.4 Hz, 1H), 4.15 *(d, J =* 12.2 Hz, 2H), 3.26-3.17 (m, 4H), 2.86-2.55 (m, 5H), 2.55-2.51 (m, 4H), 2.23-2.20 (m, 2H), 2.15-2.08 (m, 1H), 1.82-1.71 (m, 3H), 1.19-1.07 (m, 2H).

### Preparation Example 385. Synthesis of Compound A378

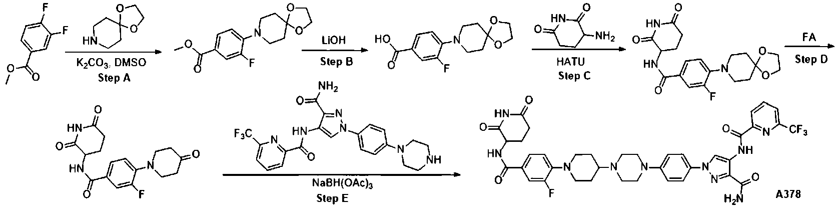

### Step A: Methyl 3-fluoro-4-(8-aza-1,4-dioxaspiro[4.5]decan-8-yl)benzoate

To a solution of methyl 3,4-difluorobenzoate (1.0 g, 5.8 mmol, 1.0 eq) in dimethyl sulfoxide (20 mL) were added 8-aza-1,4-dioxaspiro[4.5]decane (1.0 g, 6.971 mmol, 1.2 eq) and potassium carbonate (2.4 g, 17.4 mmol, 3.0 eq) at rt. The mixture was stirred at 80°C under N₂ for 16 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 1.2 g, yield 70%).

### Step B: 3-Fluoro-4-(8-aza-1,4-dioxaspiro[4.5]decan-8-yl)benzoic acid

To a solution of methyl 3-fluoro-4-(8-aza-1,4-dioxaspiro[4.5]decan-8-yl)benzoate (1.2 g, 4.06 mmol, 1.0 eq) in THF (15 mL) and water (7.5 mL) was added lithium hydroxide (0.26 g, 6.1 mmol, 1.5 eq) at rt. The mixture was stirred at rt for 1 h. The reaction solution was concentrated to afford the title compound (yellow solid, 1.0 g, crude).

### Step C: N-(2,6-Dioxopiperidin-3-yl)-3-fluoro-4-(8-aza-1,4-dioxaspiro[4.5]decan-8-yl)benzamide

To a solution of 3-fluoro-4-(8-aza-1,4-dioxaspiro[4.5]decan-8-yl)benzoic acid (1 g, crude) and 3-amino-2,6-piperidinedione (0.46 g, 3.56 mmol, 1.0 eq) in DMF (18 mL) were added HATU (2.7 g, 7.1 mmol, 2.0 eq) and DIEA (2.3 g, 17.78 mmol, 5.0 eq). The mixture was stirred at 25°C for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 600 mg, yield 43%).

### Step D: N-(2,6-Dioxopiperidin-3-yl)-3-fluoro-4-(4-oxopiperidin-1-yl)benzamide

A solution of N-(2,6-dioxopiperidin-3-yl)-3-fluoro-4-(8-aza-1,4-dioxaspiro[4.5]decan-8-yl)benzamide (600 mg, 1.5 mmol, 1.0 eq) in formic acid (5 mL) was stirred at 60°C for 16 h. Upon completion, the reaction solution was concentrated under reduced pressure to afford the title compound (yellow oil, 300 mg, crude), which was used directly without purification.

### Step E: 1-(4-{4-[1-(4-{[(2,6-Dioxopiperidin-3-yl)amino]carbonyl}-2-fluorophenyl)piperidin-4-yl]piperazin-1-yl}phenyl)-4-({[6-(trifluoromethyl)pyridin-2-yl]carbonyl}amino)pyrazole-3-carboxamide

At rt, to a solution of N-(2,6-dioxopiperidin-3-yl)-3-fluoro-4-(4-oxopiperidin-1-yl)benzamide (200 mg, crude) in DMF (10 mL) were added 1-[4-(piperazin-1-yl)phenyl]-4-({[6-(trifluoromethyl)pyridin-2-yl]carbonyl}amino)pyrazole-3-carboxamide (151 mg, 0.43 mmol, 1.0 eq) and 5 drops of AcOH. The reaction was stirred at rt for 1 h, and then sodium triacetoxyborohydride (465 mg, 2.19 mmol, 5.0 eq) was added. The reaction solution was stirred at rt for 2 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 20 mg, yield 6%). LCMS (ESI): m/z 791.4 [M+H]⁺. **¹H NMR** (400 MHz, DMSO-*d₆*) *δ* 11.79 (s, 1H), 10.86 (s, 1H), 8.88 (s, 1H), 8.66 (d, *J =* 8.3 Hz, 1H), 8.46-8.38 (m, 2H), 8.22 (s, 1H), 7.95 (s, 1H), 7.81-7.77 (m, 2H), 7.72-7.61 (m, 3H), 7.14-7.06 (m, 3H), 4.80-4.72 (m, 1H), 3.59-3.55 (m, 2H), 3.23 (s, 4H), 2.82-2.74 (m, 3H), 2.72-2.67 (m, 4H), 2.59-2.53 (m, 1H), 2.44-2.41 (m, 1H), 2.14-2.07 (m, 1H), 1.98-1.90 (m, 3H), 1.66-1.57 (m, 2H).

### Preparation Example 386. Synthesis of Compound A379

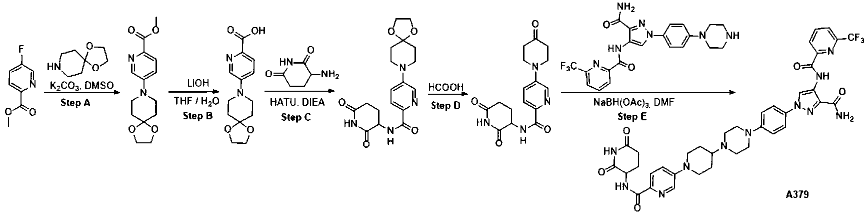

### Step A: Methyl 5-(1,4-dioxa-8-azaspiro[4.5]decan-8-yl)picolinate

To a solution of 1,4-dioxa-8-azaspiro[4.5]decane (2.2 g, 15.48 mmol, 1.2 eq) in dimethyl sulfoxide (20 mL) were added methyl 5-fluoropyridine-2-carboxylate (2.0 g, 12.9 mmol, 1.0 eq) and potassium carbonate (2.1 g, 15.49 mmol, 1.2 eq). The mixture was stirred at 110°C for 1 h. Upon completion, the mixture was diluted in EtOAc, washed sequentially with water and saturated aqueous NaCl, dried over anhydrous Na₂SO₄, and concentrated under reduced pressure. The residue was purified by column chromatography to afford the title compound (yellow solid, 1.7 g, yield 45%).

### Step B: 5-(1,4-dioxa-8-azaspiro[4.5]decan-8-yl)picolinic acid

To a solution of methyl 5-(1,4-dioxa-8-azaspiro[4.5]decan-8-yl)picolinate (1.7 g, 6.12 mmol, 1.0 eq) in THF (10 mL) and water (2 mL) was added lithium hydroxide (0.51 g, 12.23 mmol, 2.0 eq). The mixture was stirred at rt for 1 h. The reaction solution was concentrated to afford the title compound as the lithium hydroxide salt (yellow solid, 1.4 g, crude).

### Step C: N-(2,6-Dioxopiperidin-3-yl)-5-(1,4-dioxa-8-azaspiro[4.5]decan-8-yl)picolinamide

To a solution of 3-amino-2,6-piperidinedione (0.48 g, 3.79 mmol, 1.0 eq) in DMF (20 mL) were added 5-(1,4-dioxa-8-azaspiro[4.5]decan-8-yl)picolinic acid (1 g, crude), HATU (2.88 g, 7.58 mmol, 2.0 eq), and DIEA (1.3 mL, 7.58 mmol, 2.0 eq) at 0°C. The mixture was stirred at 0°C for 30 min. Upon completion, the mixture was diluted in EtOAc, washed sequentially with water and saturated aqueous NaCl, dried over anhydrous Na₂SO₄, and concentrated under reduced pressure. The residue was purified by column chromatography to afford the title compound (yellow solid, 1 g, yield 67%).

### Step D: N-(2,6-Dioxopiperidin-3-yl)-5-(4-oxopiperidin-1-yl)picolinamide

A solution of N-(2,6-dioxopiperidin-3-yl)-5-(1,4-dioxa-8-azaspiro[4.5]decan-8-yl)picolinamide (1 g, 2.67 mmol, 1.0 eq) in formic acid (10 mL) was stirred at 60°C for 2 h. The reaction solution was concentrated to afford the title compound (green oil, 500 mg, crude).

### Step E: N-(3-Carbamoyl-1-(4-(4-(1-(6-((2,6-dioxopiperidin-3-yl)carbamoyl)pyridin-3-yl)piperidin-4-yl)piperazin-1-yl)phenyl)-1H-pyrazol-4-yl)-6-(trifluoromethyl)picolinamide

To a solution of N-(3-carbamoyl-1-(4-(piperazin-1-yl)phenyl)-1H-pyrazol-4-yl)-6-(trifluoromethyl)picolinamide (695 mg, 1.52 mmol, 1.0 eq) in DMF (10 mL) were added AcOH (0.1 mL) and N-(2,6-dioxopiperidin-3-yl)-5-(4-oxopiperidin-1-yl)picolinamide (500 mg, crude). The mixture was stirred at 25°C for 1 h, and then sodium triacetoxyborohydride (1277 mg, 6.06 mmol, 4.0 eq) was added. The reaction was stirred at 25°C for 1 h. The reaction solution was quenched with saturated NaHCO₃ solution, diluted in EtOAc, washed sequentially with water and saturated aqueous NaCl, dried over anhydrous Na₂SO₄, and concentrated under reduced pressure. The residue was purified by HPLC to afford the title compound (yellow solid, 25.1 mg, yield 2%). LCMS (ESI): m/z 774[M+H]⁺. **¹H NMR** (400 MHz, DMSO) *δ* 11.80 (s, 1H), 10.86 (s, 1H), 8.88 (s, 1H), 8.72 (s, 1H), 8.53-8.30 (m, 3H), 8.22 (s, 1H), 7.97 (s, 1H), 7.79 (t, *J =* 24.9 Hz, 3H), 7.43 (s, 1H), 7.07 (s, 2H), 4.84-4.55 (m, 1H), 4.24-3.75 (m, 2H), 3.26-3.07 (m, 5H), 3.04-2.84 (m, 2H), 2.73-2.61 (m, 4H), 2.30-2.10 (m, 1H), 2.09-1.83 (m, 4H), 1.67-1.45 (m, 2H).

### Preparation Example 387. Synthesis of Compound A380

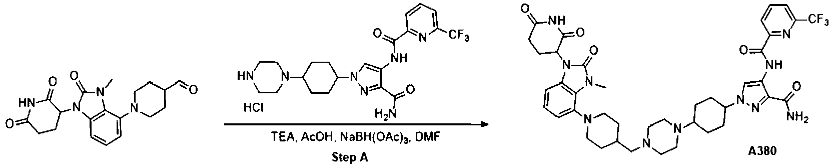

### Step A: N-(3-Carbamoyl-1-(4-((1-(1-(2,6-Dioxopiperidin-3-yl)-3-methyl-2-oxo-2,3-dihydro-1H-benzo[d]imidazol-4-yl)piperidin-4-yl)methyl)piperazin-1-yl)cyclohexyl)-1H-pyrazol-4-yl)-6-(trifluoromethyl)picolinamide

To a solution of 1-(1-(2,6-dioxopiperidin-3-yl)-3-methyl-2-oxo-2,3-dihydro-1H-benzo[d]imidazol-4-yl)piperidine-4-carbaldehyde (120 mg, 0.3 mmol, 1.0 eq) and N-(3-carbamoyl-1-(4-(piperazin-1-yl)cyclohexyl)-1H-pyrazol-4-yl)-6-(trifluoromethyl)picolinamide hydrochloride (150 mg, 0.3 mmol, 1.0 eq) in DMF (5 mL) was added TEA (0.2 mL) at rt. The reaction solution was reacted at rt for 0.5 h. Then AcOH (0.3 mL) was added to the reaction solution, and the mixture was stirred for 15 h. Sodium triacetoxyborohydride (103 mg, 0.5 mmol, 1.6 eq) was added, and the mixture was stirred for 1 h. Upon completion, the reaction solution was diluted in water and extracted with DCM. The organic phase was concentrated, and then the residue was purified by HPLC to afford the title compound (white solid, 34 mg, yield 13%). **¹H NMR** (400 MHz, DMSO-*d*₆) *δ* 11.72 (s, 1H), 11.10 (s, 1H), 8.48 (s, 1H), 8.45-8.35 (m, 2H), 8.20 (dd, *J* = 7.2, 1.2 Hz, 1H), 7.60 (d, *J =* 6.8 Hz, 2H), 7.00 (t, *J =* 8.0 Hz, 1H), 6.90 (t, *J =* 8.0 Hz, 2H), 5.43-5.29 (m, 1H), 4.33 (t, *J* = 11.6 Hz, 1H), 3.64 (s, 3H), 3.46 (s, 4H), 3.15 (d, *J=* 10.0 Hz, 6H), 2.94-2.58 (m, 8H), 2.28-2.12 (m, 4H), 2.05-1.78 (m, 6H), 1.71-1.55 (m, 2H), 1.49-1.29 (m, 2H). LCMS (ESI): m/z 820 [M+H]⁺.

### Preparation Example 388. Synthesis of Compound A383

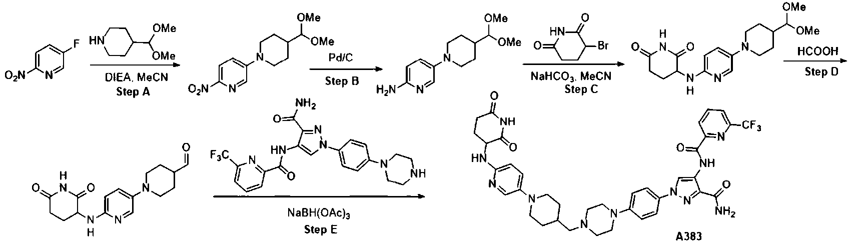

### Step A: 5-(4-(Dimethoxymethyl)piperidin-1-yl)-2-nitropyridine

To a solution of 5-fluoro-2-nitropyridine (2 g, 14.08 mmol, 1.0 eq) in acetonitrile (20 mL) were added DIEA (7.3 mL, 42.25 mmol, 3.0 eq) and (piperidin-4-yl)dimethoxymethane (3.36 g, 21.13 mmol, 1.5 eq) at rt. The reaction solution was stirred at 80°C under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 3.8 g, yield 98%).

### Step B: [1-(6-Aminopyridin-3-yl)piperidin-4-yl]dimethoxymethane

To a solution of 5-(4-(dimethoxymethyl)piperidin-1-yl)-2-nitropyridine (1.5 g, 5.33 mmol, 1.0 eq) in ethanol was added Pd/C (10%, 300 mg) at rt. The reaction solution was purged with H₂ (×3), and then reacted under H₂ for 3 h. The reaction solution was filtered through Celite and concentrated to afford the title compound (gray solid, 1.4 g, yield 93%).

### Step C: 3-((5-(4-(Dimethoxymethyl)piperidin-1-yl)pyridin-2-yl)amino)piperidine-2,6-dione

To a solution of [1-(6-aminopyridin-3-yl)piperidin-4-yl]dimethoxymethane (500 mg, 1.99 mmol, 1.0 eq) in acetonitrile (5 mL) were added NaHCO₃ (836 mg, 9.96 mmol, 5.0 eq) and 3-bromopiperidine-2,6-dione (764 mg, 3.98 mmol, 2.0 eq) at rt. The reaction solution was stirred at 80°C under N₂ for 16 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (blue solid, 300 mg, yield 41%).

### Step D: 1-(6-((2,6-Dioxopiperidin-3-yl)amino)pyridin-3-yl)piperidine-4-carbaldehyde

At 0°C, 3-((5-(4-(dimethoxymethyl)piperidin-1-yl)pyridin-2-yl)amino)piperidine-2,6-dione (0.2 g, 0.55 mmol, 1.0 eq) was added to formic acid (5 ml). The reaction solution was stirred at 60°C under N₂ for 2 h. The reaction solution was concentrated to afford the title compound (yellow solid, 174 mg, yield 99%).

### Step E: N-(3-Carbamoyl-1-(4-(4-((1-(6-((2,6-dioxopiperidin-3-yl)amino)pyridin-3-yl)piperidin-4-yl)methyl)piperazin-1-yl)phenyl)-1H-pyrazol-4-yl)-6-(trifluoromethyl)picolinamide

To a solution of 1-(6-((2,6-dioxopiperidin-3-yl)amino)pyridin-3-yl)piperidine-4-carbaldehyde (150 mg, 0.47 mmol, 1.0 eq) in DMF (5 mL) were added N-(3-carbamoyl-1-(4-(piperazin-1-yl)phenyl)-1H-pyrazol-4-yl)-6-(trifluoromethyl)picolinamide (220 mg, 0.47 mmol, 1.0 eq) and 5 drops of AcOH at rt. The reaction was stirred at rt for 2 h, and then sodium triacetoxyborohydride (606 mg, 2.85 mmol, 6.0 eq) was added. The reaction solution was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (off-white solid, 25 mg, yield 7%). LCMS (ESI): m/z 760 [M+H]⁺. **¹H NMR** (400 MHz, DMSO-*d*₆) *δ* 11.83-11.77 (m, 1H), 10.73 (s, 1H), 8.88 (s, 1H), 8.45 (d, *J* = 7.5 Hz, 1H), 8.40 *(t, J=* 7.7 Hz, 1H), 8.21 (d, *J=* 7.6 Hz, 1H), 8.14 (s, 1H), 7.95 (s, 1H), 7.79 (d, *J* = 9.0 Hz, 2H), 7.71 (s, 1H), 7.65 (d, *J =* 2.8 Hz, 1H), 7.26-7.18 (m, 1H), 7.08 (d, *J* = 9.1 Hz, 2H), 6.54 (d, *J= 9.0* Hz, 1H), 6.38 (d, *J=* 7.5 Hz, 1H), 4.71-4.60 (m, 1H), 3.43-3.33 (m, 4H), 3.25-3.19 (m, 4H), 2.79-2.69 (m, 1H), 2.58-2.55 (m, 1H), 2.55-2.52 (m, 4H), 2.26-2.20 (m, 2H), 2.13-2.06 (m, 1H), 2.03-1.93 (m, 1H), 1.84-1.77 (m, 2H), 1.68-1.59 (m, 1H), 1.29-1.20 (m, 2H).

### Preparation Example 389. Synthesis of Compound A384

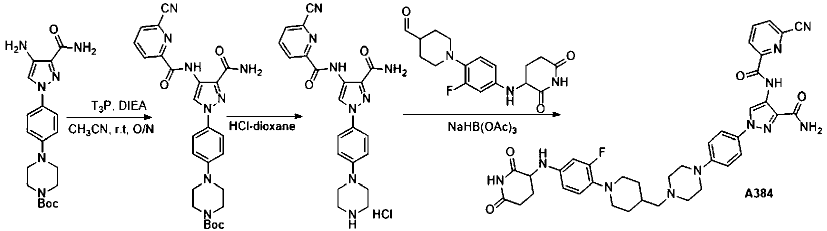

### Step A: Tert-butyl 4-(4-(3-carbamoyl-4-(6-cyanopicolinamido)-1H-pyrazol-1-yl)phenyl)piperazine-1-carboxylate

To a solution of tert-butyl 4-(4-(4-amino-3-carbamoyl-1H-pyrazol-1-yl)phenyl)piperazine-1-carboxylate (500 mg, 1.29 mmol, 1.0 eq) in acetonitrile (20 mL) were added 6-cyano-2-picolinic acid (210 mg, 1.42 mmol, 1.1 eq), DIEA (0.7 mL, 3.87 mmol, 3.0 eq), and T3P (50% in EtOAc, 1641 mg, 2.58 mmol, 2.0 eq). The mixture was stirred at rt under N₂ for 16 h. The solid precipitated was collected by filtration to afford the title compound (white solid, 500 mg, yield 75%).

### Step B: N-(3-Carbamoyl-1-(4-(piperazin-1-yl)phenyl)-1H-pyrazol-4-yl)-6-cyanopicolinamide hydrochloride

To a solution of tert-butyl 4-(4-(3-carbamoyl-4-(6-cyanopicolinamido)-1H-pyrazol-1-yl)phenyl)piperazine-1-carboxylate (500 mg, 0.9 mmol, 1.0 eq) was added to 4 M hydrogen chloride-dioxane (10 mL). The mixture was stirred at rt for 2 h. Upon completion, the reaction solution was concentrated under reduced pressure to afford the title compound (white solid, 400 mg, yield 99%).

### Step C: N-(3-Carbamoyl-1-(4-(4-((1-(4-((2,6-dioxopiperidin-3-yl)amino)-2-fluorophenyl)piperidin-4-yl)methyl)piperazin-1-yl)phenyl)-1H-pyrazol-4-yl)-6-cyanopicolinamide

To a solution of N-(3-carbamoyl-1-(4-(piperazin-1-yl)phenyl)-1H-pyrazol-4-yl)-6-cyanopicolinamide hydrochloride (100 mg, crude) in DMF (5 mL) were added AcOH (0.5 mL) and 1-(4-((2,6-dioxopiperidin-3-yl)amino)-2-fluorophenyl)piperidine-4-carbaldehyde (88 mg, 0.26 mmol, 1.1 eq). The mixture was stirred at rt for 16 h, and then sodium triacetoxyborohydride (151 mg, 0.71 mmol, 3.0 eq) was added. The mixture was stirred at rt for another 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 33 mg, yield 19%). **¹H NMR** (400 MHz, DMSO-d6) δ 11.67 (s, 1H), 10.80 (s, 1H), 9.47 (s, 1H), 8.93 (s, 1H), 8.45 (dd, *J =* 7.2, 1.8 Hz, 1H), 8.40-8.31 (m, 2H), 7.98 (s, 1H), 7.88 (d, *J =* 9.0 Hz, 2H), 7.76 (s, 1H), 7.19 (d, *J =* 9.1 Hz, 2H), 6.97 (d, *J=* 8.7 Hz, 1H), 6.55 (d, *J =* 14.9 Hz, 1H), 6.47 (d, *J=* 8.0 Hz, 1H), 4.30 (dd, *J=* 11.3, 4.6 Hz, 1H), 4.02-3.86 (m, 4H), 3.21 (d, *J* = 19.5 Hz, 8H), 2.73 (ddd, *J=* 20.1, 12.7, 6.7 Hz, 4H), 2.16-1.97 (m, 2H), 1.89 (d, *J=* 5.8 Hz, 3H), 1.47 (d, *J=* 12.9 Hz, 2H). LCMS (ESI): m/z 417 [M+H]⁺.

### Preparation Example 390. Synthesis of Compound A385

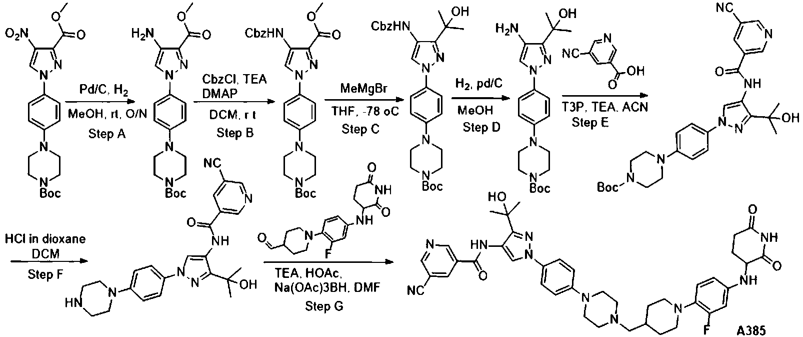

### Step A: Tert-butyl 4-(4-(4-amino-3-(methoxycarbonyl)-1H-pyrazol-1-yl)phenyl)piperazine-1-carboxylate

To a solution of tert-butyl 4-(4-(3-(methoxycarbonyl)-4-nitro-1H-pyrazol-1-yl)phenyl)piperazine-1-carboxylate in MeOH (60 mL) was added Pd/C (10%, 1 g). The reaction mixture was degassed under H₂ (×3), and then stirred under H₂ at rt for 16 h. Upon completion, the reaction solution was filtered through Celite, and the filtrate was concentrated. The residue was purified by column chromatography to afford the title compound (brown solid, 4.5 g, yield 81%).

### Step B: Tert-butyl 4-(4-(4-(((benzyloxy)carbonyl)amino)-3-(methoxycarbonyl)-1H-pyrazol-1-yl)phenyl)piperazine-1-carboxylate

To a solution of tert-butyl 4-(4-(4-amino-3-(methoxycarbonyl)-1H-pyrazol-1-yl)phenyl)piperazine-1-carboxylate (4.5 g, 11.21 mmol, 1.0 eq) in DCM (50 mL) were added DMAP (140 mg, 1.12 mmol, 0.1 eq), TEA (3.1 mL, 22.42 mmol, 2.0 eq), and benzyl chloroformate (1.9 mL, 13.45 mmol, 1.2 eq). The reaction solution was stirred at rt under N₂ for 2 h. Upon completion, the reaction solution was diluted in DCM, washed sequentially with water and saturated aqueous NaCl, dried over anhydrous Na₂SO₄, and concentrated under reduced pressure. The residue was purified by column chromatography to afford the title compound (brown solid, 6 g, yield 100%).

### Step C: Tert-butyl 4-(4-(4-(((benzyloxy)carbonyl)amino)-3-(2-hydroxypropan-2-yl)-1H-pyrazol-1-yl)phenyl)piperazine-1-carboxylate

To a solution of tert-butyl 4-(4-(4-(((benzyloxy)carbonyl)amino)-3-(methoxycarbonyl)-1H-pyrazol-1-yl)phenyl)piperazine-1-carboxylate (6 g, 11.20 mmol, 1.0 eq) in THF (60 mL) was added methylmagnesium bromide (33.6 mL, 33.60 mmol, 3.0 eq, 1.0 M in tetrahydrofuran) at -78°C under N₂. The mixture was stirred at -78°C under N₂ for 1 h. Upon completion, the reaction solution was quenched with saturated aqueous NH₄Cl and extracted with EtOAc. The organic layer was washed sequentially with water and saturated aqueous NaCl, dried over anhydrous Na₂SO₄, and concentrated under reduced pressure. The residue was purified by column chromatography to afford the title compound (brown solid, 5 g, yield 83%).

### Step D: Tert-butyl 4-(4-(4-amino-3-(2-hydroxypropan-2-yl)-1H-pyrazol-1-yl)phenyl)piperazine-1-carboxylate

To a solution of tert-butyl 4-(4-(4-(((benzyloxy)carbonyl)amino)-3-(2-hydroxypropan-2-yl)-1H-pyrazol-1-yl)phenyl)piperazine-1-carboxylate (5 g, 9.33 mmol, 1.0 eq) in MeOH (50 mL) was added Pd/C (10%, 1 g). The reaction solution was purged with H₂ (×3), and then stirred under H₂ at rt for 16 h. Upon completion, the reaction solution was filtered through Celite, and the filtrate was concentrated under reduced pressure. The residue mixture was stirred at a constant temperature under H2 for 16 h. The reaction mixture was filtered, and the filtrate was concentrated. The residue was purified by column chromatography to afford the title compound (brown solid, 2.2 g, yield 59%).

### Step E: Tert-butyl 4-(4-(4-(5-cyanonicotinamido)-3-(2-hydroxypropan-2-yl)-1H-pyrazol-1-yl)phenyl)piperazine-1-carboxylate

To a solution of 5-cyanonicotinic acid (1 g, 6.75 mmol, 1.0 eq) in acetonitrile (10 mL) were added tert-butyl 4-(4-(4-amino-3-(2-hydroxypropan-2-yl)-1H-pyrazol-1-yl)phenyl)piperazine-1-carboxylate (2.7 g, 6.75 mmol, 1.0 eq), TEA (1.9 mL, 13.50 mmol, 2.0 eq), and T3P (6.44 mL, 10.13 mmol, 1.5 eq, 50% in EtOAc). The mixture was stirred at rt for 16 h. Upon completion, the solid was precipitated from the reaction solution, and the reaction mixture was filtered. The filter cake was washed with acetonitrile, and concentrated to afford the title compound (yellow solid, 950 mg, yield 26%).

### Step F: 5-Cyano-N-(3-(2-hydroxypropan-2-yl)-1-(4-(piperazin-1-yl)phenyl)-1H-pyrazol-4-yl)nicotinamide

To a solution of tert-butyl 4-(4-(4-(5-cyanonicotinamido)-3-(2-hydroxypropan-2-yl)-1H-pyrazol-1-yl)phenyl)piperazine-1-carboxylate (200 mg, 0.38 mmol, 1.0 eq) in DCM (6 mL) was added 4 M hydrogen chloride in dioxane (2 mL). The mixture was stirred at rt for 2 h. Upon completion, the reaction mixture was concentrated to afford the title compound (brown solid, 200 mg, crude).

### Step G: 5-Cyano-N-(1-(4-(4-((1-(4-((2,6-dioxopiperidin-3-yl)amino)-2-fluorophenyl)piperidin-4-yl)methyl)piperazin-1-yl)phenyl)-3-(2-hydroxypropan-2-yl)-1H-pyrazol-4-yl)nicotinamide

To a solution of 5-cyano-N-(3-(2-hydroxypropan-2-yl)-1-(4-(piperazin-1-yl)phenyl)-1H-pyrazol-4-yl)nicotinamide (200 mg, crude) in DMF (5 mL) was added TEA (47 mg, 0.46 mmol, 0.8 eq). The mixture was stirred at rt under N₂ for 15 min. Then AcOH (173 mg, 0.93 mmol, 1.7 eq) and 1-(4-((2,6-dioxopiperidin-3-yl)amino)-2-fluorophenyl)piperidine-4-carbaldehyde (185 mg, 0.56 mmol, 1.0 eq) were added. The mixture was stirred for 16 h. Then sodium triacetoxyborohydride (293 mg, 1.39 mmol, 2.5 eq) was added, The mixture was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 21.2 mg, yield 6.11%). **¹H NMR** (400 MHz, DMSO) δ 10.77 (s, 1H), 10.20 (s, 1H), 9.24 (d, *J =* 2.0 Hz, 1H), 9.22 (d, *J=* 2.0 Hz, 1H), 8.66-8.65 (t, *J* = 2.1 Hz, 1H), 8.64 (s, 1H), 7.62-7.60 (d, *J =* 8.8 Hz, 2H), 7.04-702 *(d, J=* 8.8 Hz, 2H), 6.84-6.81 *(t, J=* 9.2 Hz, 1H), 6.52-6.48 (dd, 1H), 6.43- 6.41 (m, 1H), 5.94 (s, 1H), 5.79-5.77 (d, *J =* 7.6 Hz, 1H), 4.28-4.22 (m, 1H), 3.30 (s, 1H), 3.17 (s, 4H), 3.14-3.11 (m, 2H), 2.77-2.69 (m, 1H), 2.59-2.56 (m, 2H), 2.54 (m, 4H), 2.24-2.23 (d, *J =* 6.8 Hz, 2H), 2.11-2.07 (m, 1H), 1.86-1.83(m, 1H), 1.80-1.77 (m, 2H), 1.59 (m, 1H), 1.59 (s, 6H), 1.32-1.23 (m, 2H). LCMS (ESI): m/z 749 [M+H]⁺.

### Preparation Example 391. Synthesis of Compound A386

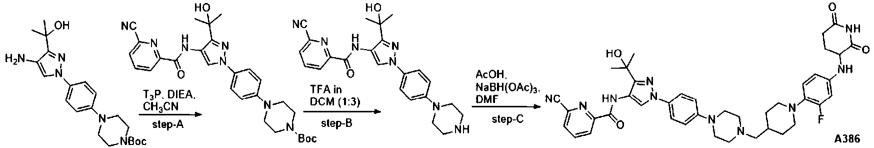

### Step A: Tert-butyl 4-(4-(4-(6-cyano-2-picolinamido)-3-(2-hydroxypropan-2-yl)-1H-pyrazol-1-yl)phenyl)piperazine-1-carboxylate

To a solution of tert-butyl 4-(4-(4-amino-3-(2-hydroxypropan-2-yl)-1H-pyrazol-1-yl)phenyl)piperazine-1-carboxylate (500 mg, 1.29 mmol, 1.0 eq) in acetonitrile (20 mL) were added 6-cyano-2-picolinic acid (210 mg, 1.42 mmol, 1.1 eq), DIEA (0.7 mL, 3.87 mmol, 3.0 eq), and T3P (1641 mg, 2.58 mmol, 2.0 eq, 50% in EtOAc). The mixture was stirred at rt for 16 h. Upon completion, the white solid was precipitated from the reaction solution, and the reaction mixture was filtered. The filter cake was concentrated to afford the title compound (white solid, 500 mg, yield 75%).

### Step B: 6-Cyano-N-(3-(2-hydroxypropan-2-yl)-1-(4-(piperazin-1-yl)phenyl)-1H-pyrazol-4-yl)picolinamide

Tert-butyl 4-(4-(4-(6-cyano-2-picolinamido)-3-(2-hydroxypropan-2-yl)-1H-pyrazol-1-yl)phenyl)piperazine-1-carboxylate (500 mg, 0.9 mmol, 1.0 eq) was added to a mixed solution of DCM (6 mL) and TFA (2 mL). The mixture was stirred at rt for 2 h. The reaction mixture was concentrated to afford the title compound (white solid, 400 mg, crude).

### Step C: 6-Cyano-N-(1-(4-(4-((1-(4-((2,6-dioxopiperidin-3-yl)amino)-2-fluorophenyl)piperidin-4-yl)methyl)piperazin-1-yl)phenyl)-3-(2-hydroxypropan-2-yl)-1H-pyrazol-4-yl)picolinamide

To a solution of 6-cyano-N-(3-(2-hydroxypropan-2-yl)-1-(4-(piperazin-1-yl)phenyl)-1H-pyrazol-4-yl)picolinamide (100 mg, crude) in DMF (5 mL) were added AcOH (0.5 mL) and 1-(4-((2,6-dioxopiperidin-3-yl)amino)-2-fluorophenyl)piperidine-4-carbaldehyde (88 mg, 0.26 mmol, 1.1 eq). The mixture was stirred at rt for 16 h, and then sodium triacetoxyborohydride (151 mg, 0.71 mmol, 3.0 eq) was added. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (yellow solid, 50 mg, yield 29% over two steps). **¹H NMR** (400 MHz, DMSO-d6) δ 11.14 (s, 1H), 10.77 (s, 1H), 8.74 (s, 1H), 8.43 (dd, *J =* 7.6, 1.6 Hz, 1H), 8.40-8.23 (m, 2H), 7.61 (d, *J* = 9.2 Hz, 2H), 7.03 (d, *J* = 9.2 Hz, 2H), 6.83 (t, *J* = 9.2 Hz, 1H), 6.50 (dd, *J=* 15.2, 2.4 Hz, 1H), 6.41 (dd, *J=* 8.8, 2.0 Hz, 1H), 6.07 (s, 1H), 5.78 (d, *J= 7.6* Hz, 1H), 4.32-4.12 (m, 1H), 3.36 (d, *J* = 5.2 Hz, 2H), 3.19-3.06 (m, 6H), 2.72 (m, 1H), 2.60-2.51 (m, 5H), 2.24 (s, 2H), 2.11-2.00 (m, 1H), 1.88-1.73 (m, 3H), 1.62 (s, 1H), 1.58 (s, 6H), 1.37-1.20 (m, 2H). LCMS (ESI): m/z 749 [M+H]⁺.

### Preparation Example 392. Synthesis of Compound A387

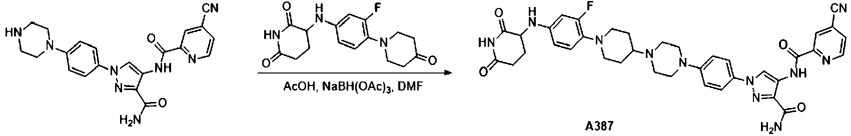

### N-(3-Carbamoyl-1-(4-(4-(1-(4-((2,6-Dioxopiperidin-3-yl)amino)-2-fluorophenyl)piperidin-4-yl)piperazin-1-yl)phenyl)-1H-pyrazol-4-yl)-4-cyanopicolinamide

To a solution of N-(3-carbamoyl-1-(4-(piperazin-1-yl)phenyl)-1H-pyrazol-4-yl)-4-cyanopicolinamide (200 mg, 0.48 mmol, 1.8 eq) and 3-((3-fluoro-4-(4-oxopiperidin-1-yl)phenyl)amino)piperidine-2,6-dione (88 mg, 0.26 mmol, 1.0 eq) in DMF (3 mL) was added AcOH (1 mL). The mixture was stirred at rt for 16 h, and then sodium triacetoxyborohydride (506 mg, 2.4 mmol, 9.2 eq) was added. The mixture was stirred at rt for 1 h. The reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 5.8 mg, yield 5%). LCMS (ESI): m/z 720 [M+H]⁺. **¹H NMR** (400 MHz, DMSO-d6) δ 11.67 (s, 1H), 10.77 (s, 1H), 9.01 (d, *J* = 5.2 Hz, 1H), 8.89 (s, 1H), 8.51 (s, 1H), 8.18 (dd, *J* = 4.8, 1.6 Hz, 1H), 7.96 (s, 1H), 7.79 (d, *J* = 9.2 Hz, 2H), 7.67 (s, 1H), 7.08 (d, *J* = 9.2 Hz, 2H), 6.84 (t, *J* = 9.2 Hz, 1H), 6.50 (d, *J =* 15.2 Hz, 1H), 6.42 (d, *J* = 8.8 Hz, 1H), 5.79 (d, *J* = 7.6 Hz, 1H), 4.25 (s, 1H), 3.21-3.15 (m, 6H), 2.69-2.65 (m, 4H), 2.60 (d, *J =* 9.6 Hz, 2H), 2.34-2.31 (m, 1H), 2.07-2.03 (m, 2H), 1.87-1.53 (m, 3H), 1.59-1.54 (m, 3H).

### Preparation Example 393. Synthesis of Compound A388

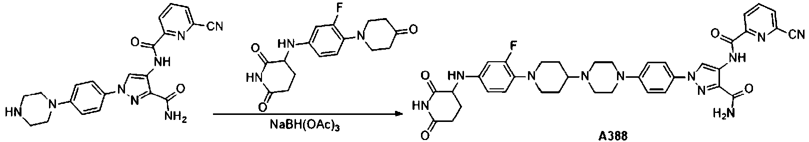

### N-(3-Carbamoyl-1-(4-(4-(1-(4-((2,6-Dioxopiperidin-3-yl)amino)-2-fluorophenyl)piperidin-4-yl)piperazin-1-yl)phenyl)-1H-pyrazol-4-yl)-6-cyanopicolinamide

To a solution of N-(3-carbamoyl-1-(4-(piperazin-1-yl)phenyl)-1H-pyrazol-4-yl)-6-cyanopicolinamide (200 mg, 0.48 mmol, 1.0 eq) and 3-((3-fluoro-4-(4-oxopiperidin-1-yl)phenyl)amino)piperidine-2,6-dione (153 mg, 0.48 mmol, 1.0 eq) in DMF (3 mL) was added AcOH (1 mL). The mixture was stirred at rt for 16 h, and then sodium triacetoxyborohydride (506 mg, 2.4 mmol, 5.0 eq) was added. The mixture was stirred at rt for 1 h. The crude was purified by preparative chromatography to afford the title compound (white solid, 10 mg, yield 3%). **¹H NMR** (400 MHz, DMSO-d6) δ 11.67 (s, 1H), 10.78 (s, 1H), 8.93 (s, 1H), 8.44 (dd, *J* = 7.2, 2.0 Hz, 1H), 8.38-8.31 (m, 2H), 7.98 (s, 1H), 7.88 (d, *J=* 9.2 Hz, 2H), 7.75 (s, 1H), 7.20 (d, *J= 9.2* Hz, 2H), 6.88 (t, *J =* 9.2 Hz, 1H), 6.58-6.39 (m, 2H), 4.27 (dd, *J* = 11.4, 4.8 Hz, 1H), 4.00 (d, *J =* 12.6 Hz, 2H), 3.76-3.68 (m, 4H), 3.33-3.21 (m, 4H), 3.12-2.97 (m, 2H), 2.88-2.60 (m, 3H), 2.25-2.03 (m, 3H), 1.94-1.72 (m, 3H). LCMS (ESI): m/z 720 [M+H]⁺.

### Preparation Example 394. Synthesis of Compound A389

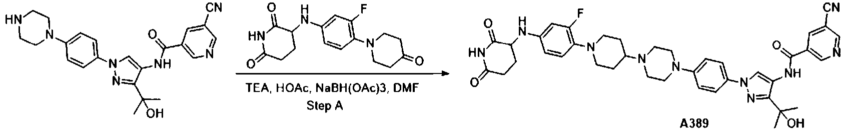

### Step A: 5-Cyano-N-(1-(4-(4-(1-(4-((2,6-dioxopiperidin-3-yl)amino)-2-fluorophenyl)piperidin-4-yl)piperazin-1-yl)phenyl)-3-(2-hydroxypropan-2-yl)-1H-pyrazol-4-yl)nicotinamide

To a solution of 5-cyano-N-(3-(2-hydroxypropan-2-yl)-1-(4-(piperazin-1-yl)phenyl)-1H-pyrazol-4-yl)nicotinamide (100 mg, 0.21 mmol, 1.0 eq) in DMF (3 mL) was added TEA (21.6 mg, 0.21 mmol, 1.0 eq) at rt. After the reaction solution was stirred for 15 min, AcOH (25.6 mg, 0.43 mmol, 2.0 eq) and 3-((3-fluoro-4-(4-oxopiperidin-1-yl)phenyl)amino)piperidine-2,6-dione (68.2 mg, 0.21 mmol, 1.0 eq) were added. The reaction solution was stirred for 16 h. Then sodium triacetoxyborohydride (135 mg, 0.64 mmol, 3.0 eq) was added. After stirring for 1 h, the reaction solution was filtered, and the filtrate was purified by preparative HPLC to afford the title compound (white solid, 5.2 mg, yield 3%). **¹H NMR** (400 MHz, DMSO-*d6)* δ 10.77 (s, 1H), 10.20 (s, 1H), 9.23 (dd, *J =* 6.2, 2.0 Hz, 2H), 8.65 (dd, *J=* 5.2, 3.1 Hz, 2H), 7.61 (d, *J* = 9.2 Hz, 2H), 7.03 (d, *J =* 9.2 Hz, 2H), 6.84 (t, *J =* 9.2 Hz, 1H), 6.51 (dd, *J =* 15.2, 2.3 Hz, 1H), 6.41 (dd, *J=* 8.8, 2.0 Hz, 1H), 5.95 (s, 1H), 5.79 (d, *J=* 7.6 Hz, 1H), 4.25 (t, *J=* 11.6 Hz, 1H), 3.20-3.17 (m, 6H), 2.78-2.72 (m, 1H), 2.68 (s, 4H), 2.61-2.58 (m, 2H), 2.56-2.54 (m, 1H), 2.36- 2.31 (m, 1H), 2.11-2.08 (m, 1H), 1.88-1.83 (m, 3H), 1.63-1.55 (m, 8H).

### Preparation Example 395. Synthesis of Compound A390

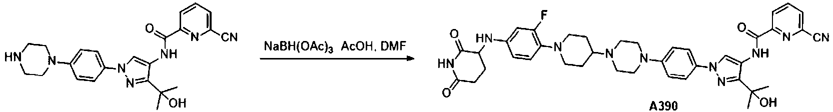

### 6-Cyano-N-(1-(4-(4-(1-(4-((2,6-dioxopiperidin-3-yl)amino)-2-fluorophenyl)piperidin-4-yl)piperazin-1-yl)phenyl)-3-(2-hydroxypropan-2-yl)-1H-pyrazol-4-yl)picolinamide

To a solution of 6-cyano-N-(3-(2-hydroxypropan-2-yl)-1-(4-(piperazin-1-yl)phenyl)-1H-pyrazol-4-yl)picolinamide (100 mg, 0.24 mmol, 1.0 eq) in DMF (3 mL) were added AcOH (0.5 mL) and 3-((3-fluoro-4-(4-oxopiperidin-1-yl)phenyl)amino)piperidine-2,6-dione (88 mg, 0.26 mmol, 1.1 eq). The mixture was stirred at rt for 16 h, and then sodium triacetoxyborohydride (151 mg, 0.71 mmol, 3.0 eq) was added. The reaction solution was diluted in EtOAc, washed sequentially with water and saturated aqueous NaCl, dried over anhydrous Na₂SO₄, and concentrated under reduced pressure. The residue was purified by preparative HPLC to afford the title compound (yellow solid, 2.5 mg, yield 2%). **¹H NMR** (400 MHz, DMSO-d6) δ 11.14 (s, 1H), 10.78 (s, 1H), 8.74 (s, 1H), 8.44 (d, *J=* 7.6 Hz, 1H), 8.40-8.25 (m, 2H), 7.61 (d, *J= 7.6* Hz, 2H), 7.04 (d, *J =* 8.0 Hz, 2H), 6.84 (t, *J= 9.2* Hz, 1H), 6.51 (d, *J=* 15.2 Hz, 1H), 6.42 (d, *J=* 8.4 Hz, 1H), 6.07 (s, 1H), 5.79 (d, *J =* 7.6 Hz, 1H), 4.26 (s, 1H), 3.18 (s, 6H), 2.69 (s, 4H), 2.59 (s, 2H), 2.34 (s, 1H), 2.07 (s, 1H), 2.00 (d, *J =* 7.8 Hz, 2H), 1.86 (s, 3H), 1.59 (s, 7H), 1.46 (s, 1H). LCMS (ESI): m/z 735 [M+H]⁺.

### Preparation Example 396. Synthesis of Compound A391

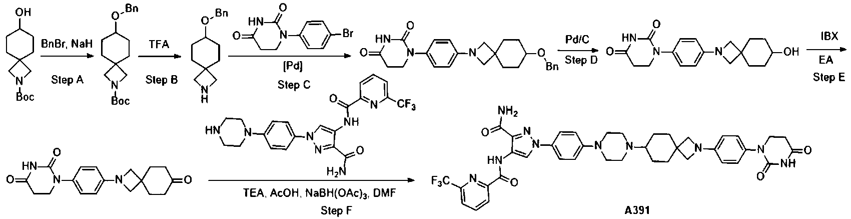

### Step A: Tert-butyl 7-(benzyloxy)-2-azaspiro[3.5]nonane-2-carboxylate

To a solution of tert-butyl 7-hydroxy-2-azaspiro[3.5]nonane-2-carboxylate (2.41 g, 9.99 mmol, 1.0 eq) in DMF (50 mL) was added NaH (60%) (0.8 g, 19.97 mmol, 2.0 eq). The mixture was stirred at rt for 0.5 h, and then (bromomethyl)benzene (1.42 mL, 11.98 mmol, 1.2 eq) was added dropwise. The mixture was stirred at rt for 1 h. Upon completion, the reaction solution was diluted in water and extracted with EtOAc. The organic phase was washed with aqueous NaCl, dried over anhydrous Na₂SO₄, and concentrated. The residue was purified by column chromatography to afford the title compound (colorless oil, 1.95 g, yield 59%).

### Step B: 7-(Benzyloxy)-2-azaspiro[3.5]nonane

To a solution of tert-butyl 7-(benzyloxy)-2-azaspiro[3.5]nonane-2-carboxylate (1 g, 3.02 mmol, 1.0 eq) in DCM (20 mL) was added TFA (10 mL). The mixture was stirred at rt for 0.5 h. The reaction solution was concentrated in vacuo to afford the title compound (yellow oil, 697 mg, crude), which was used directly in the next step.

### Step C: 1-(4-(7-(Benzyloxy)-2-azaspiro[3.5]nonan-2-yl)phenyl)dihydropyrimidine-2,4(1H,3H)-dione

To a solution of 7-(benzyloxy)-2-azaspiro[3.5]nonane (697 mg, 3.01 mmol, 1.0 eq) and 1-(4-bromophenyl)dihydropyrimidine-2,4(1H,3H)-dione (811 mg, 3.01 mmol, 1.0 eq) in 1,4-dioxane (30 mL) were added cesium carbonate (2.95 g, 9.04 mmol, 3.0 eq) and (SP-4-1)-[1,3-bis[2,6-bis(1-propylbutyl)phenyl]-4,5-dichloro-1,3-dihydro-2H-imidazol-2-ylidene]dichloro(3-chloropyridine-κN)palladium (293 mg, 0.30 mmol, 0.1 eq). The reaction solution was stirred at 100°C under N₂ for 3 h. The reaction mixture was cooled to rt, diluted in water and extracted with EtOAc. The organic phase was washed with aqueous NaCl, dried over anhydrous Na₂SO₄, and concentrated. The residue was purified by column chromatography to afford the title compound (pale yellow solid, 600 mg, yield 47%).

### Step D: 1-(4-(7-Hydroxy-2-azaspiro[3.5]nonan-2-yl)phenyl)dihydropyrimidine-2,4(1H,3H)-dione

1-(4-(7-(benzyloxy)-2-azaspiro[3.5]nonan-2-yl)phenyl)dihydropyrimidine-2,4(1H,3H)-dione (600 mg, 1.43 mmol, 1.0 eq) was dispersed in a mixed solution of in ethanol (20 mL) and THF (20 mL), and Pd/C (10%, 300 mg, 50 wt%) was added. The reaction solution was stirred under H₂ at 50°C for 4 days. The reaction solution was filtered through Celite, and the filtrate was concentrated to afford the title compound (white solid, 350 mg, yield 74%).

### Step E: 1-(4-(7-Oxo-2-azaspiro[3.5]nonan-2-yl)phenyl)dihydropyrimidine-2,4(1H,3H)-dione

To a solution of 1-(4-(7-hydroxy-2-azaspiro[3.5]nonan-2-yl)phenyl)dihydropyrimidine-2,4(1H,3H)-dione (205 mg, 0.62 mmol, 1.0 eq) in DMSO (5 mL) was added IBX (349 mg, 1.24 mmol, 2.0 eq). The mixture was stirred at 80°C for 2 h. After cooling, the reaction solution was diluted in water and extracted with EtOAc. The organic phase was washed with aqueous NaCl, dried over anhydrous Na₂SO₄, and concentrated. The residue was purified by column chromatography to afford the title compound (yellow solid, 100 mg, yield 49%).

### Step F: N-(3-Carbamoyl-1-(4-(4-(2-(4-(2,4-dioxotetrahydropyrimidin-1(2H)-yl)phenyl)-2-azaspiro[3.5]nonan-7-yl)piperazin-1-yl)phenyl)-1H-pyrazol-4-yl)-6-(trifluoromethyl)picolinamide

To a solution of 1-(4-(7-oxo-2-azaspiro[3.5]nonan-2-yl)phenyl)dihydropyrimidine-2,4(1H,3H)-dione (100 mg, 0.31 mmol, 1.0 eq) and N-(3-carbamoyl-1-(4-(piperazin-1-yl)phenyl)-1H-pyrazol-4-yl)-6-(trifluoromethyl)picolinamide (140 mg, 0.31 mmol, 1.0 eq) in DMF (10 mL) was added TEA (309 mg, 3.05 mmol, 10.0 eq). The mixture was stirred at rt for 3 min, and then glacial AcOH (275 mg, 4.58 mmol, 15.0 eq) was added. After stirring for 0.5 h, sodium triacetoxyborohydride (194 mg, 0.92 mmol, 3.0 eq) was added. The reaction solution was stirred at rt overnight. After filtration, the filtrate was purified by preparative HPLC to afford the title compound (white solid, 54.7 mg, yield 23%). **¹H NMR** (400 MHz, DMSO-d6) δ 11.80 (s, 1H), 10.23 (s, 1H), 8.93 (s, 1H), 8.55-8.30 (m, 2H), 8.22 (dd, *J=* 7.5, 1.2 Hz, 1H), 7.95 (s, 1H), 7.88 (d, *J= 9.0* Hz, 2H), 7.75 (s, 1H), 7.19 (t, *J=* 7.3 Hz, 2H), 7.10 (t, *J=* 7.3 Hz, 2H), 6.42 (d, *J=* 8.8 Hz, 2H), 3.99 (d, *J* = 13.0 Hz, 2H), 3.66 (t, *J =* 6.7 Hz, 2H), 3.64-3.58 (m, 4H), 3.52 (s, 2H), 3.29-3.19 (m, 3H), 3.05 (t, *J =* 12.2 Hz, 2H), 2.68 (t, *J=* 6.7 Hz, 2H), 2.08 (d, *J =* 10.2 Hz, 4H), 1.69-1.43 (m, 4H).

### Preparation Example 397. Synthesis of Compound A392

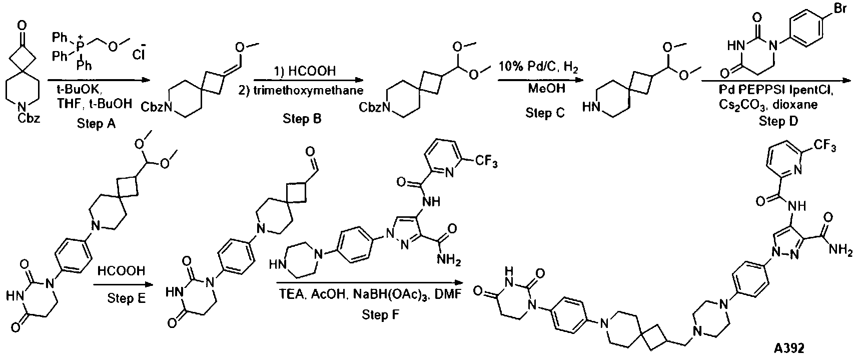

### Step A: Benzyl 2-(methoxymethylene)-7-azaspiro[3.5]nonane-7-carboxylate

To a suspension of (methoxymethyl)triphenylphosphonium chloride (7.53 g, 21.95 mmol, 3.0 eq) in THF (70 mL) and tert-butanol (3 mL) was added potassium tert-butoxide (2.46 g, 21.95 mmol, 3.0 eq) at -78°C under N₂. After stirring at 0°C for 1 h, a solution of benzyl 2-oxo-7-azaspiro[3.5]nonane-7-carboxylate (2 g, 7.32 mmol, 1.0 eq) in THF (10 mL) was added to the reaction solution. The reaction was stirred at 20°C for 16 h. The reaction mixture was poured into water and extracted twice with EtOAc. The organic phase was washed with saturated aqueous NaCl, dried over anhydrous Na₂SO₄, filtered, and concentrated. The residue was purified by column chromatography to afford the title compound (1.4 g, yield 63%).

### Step B: Benzyl 2-(dimethoxymethyl)-7-azaspiro[3.5]nonane-7-carboxylate

A solution of benzyl 2-(methoxymethylene)-7-azaspiro[3.5]nonane-7-carboxylate (1.2 g, 3.98 mmol) in formic acid (10 mL) was stirred at 20°C overnight. The reaction solution was concentrated and then dissolved in MeOH (20 mL). To this solution were added p-toluenesulfonic acid (69 mg, 0.40 mmol, 0.2 eq) and trimethyl orthoformate (1.31 mL, 11.94 mmol, 1.5 eq). The mixture was stirred at 70°C for 16 h. The mixture was concentrated under reduced pressure, and then the residue was purified by column chromatography to afford the title compound (brown solid, 400 mg, yield 30%).

### Step C: 2-(Dimethoxymethyl)-7-azaspiro[3.5]nonane

A mixture of benzyl 2-(dimethoxymethyl)-7-azaspiro[3.5]nonane-7-carboxylate (400 mg, 1.20 mmol) and Pd/C (10 wt%, 120 mg) in MeOH (10 mL) was stirred under H₂ (1 atm) for 2 h. The reaction solution was filtered through Celite, and concentrated under reduced pressure to afford the title compound (brown oil, 200 mg, crude).

### Step D: 1-(4-(2-(Dimethoxymethyl)-7-azaspiro[3.5]nonan-7-yl)phenyl)dihydropyrimidine-2,4(1H,3H)-dione

A mixture of 2-(dimethoxymethyl)-7-azaspiro[3.5]nonane (200 mg, 1.00 mmol, 1.0 eq), 1-(4-bromophenyl)dihydropyrimidine-2,4(1H,3H)-dione (130 mg, 0.48 mmol, 0.48 eq), Pd PEPPSI IpentCl (98 mg, 0.10 mmol, 0.1 eq), and cesium carbonate (327 mg, 1.00 mmol, 1.0 eq) in 1,4-dioxane (15 mL) was stirred at 120°C under N₂ overnight. The reaction solution was diluted in EtOAc, washed sequentially with water and saturated aqueous NaCl, dried over anhydrous Na₂SO₄, and concentrated under reduced pressure. The residue was purified by column chromatography to afford the title compound (brown solid, 160 mg, yield 41%).

### Step E: 7-(4-(2,4-Dioxotetrahydropyrimidin-1(2H)-yl)phenyl)-7-azaspiro[3.5]nonane-2-carbaldehyde

A solution of 1-(4-(2-(dimethoxymethyl)-7-azaspiro[3.5]nonan-7-yl)phenyl)dihydropyrimidine-2,4(1H,3H)-dione (160 mg, 0.41 mmol) in formic acid (5 mL) was stirred at 50°C for 2 h. The reaction mixture was concentrated under reduced pressure. The residue was purified by preparative HPLC to afford the title compound (yellow solid, 120 mg, yield 86%).

### Step F: N-(3-Carbamoyl-1-(4-(4-((7-(4-(2,4-dioxotetrahydropyrimidin-1(2H)-yl)phenyl)-7-azaspiro[3.5]nonan-2-yl)methyl)piperazin-1-yl)phenyl)-1H-pyrazol-4-yl)-6-(trifluoromethyl)picolinamide

A solution of 7-(4-(2,4-dioxotetrahydropyrimidin-1(2H)-yl)phenyl)-7-azaspiro[3.5]nonane-2-carbaldehyde (120 mg, 0.35 mmol, 1 eq), N-(3-carbamoyl-1-(4-(piperazin-1-yl)phenyl)-1H-pyrazol-4-yl)-6-(trifluoromethyl)picolinamide (210 mg, 0.42 mmol, 1.2 eq), TEA (0.07 mL, 0.53 mmol, 1.5 eq), and sodium triacetoxyborohydride (111 mg, 0.53 mmol, 1.5 eq) in DMF (5 mL) was stirred at 30°C overnight. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 5 mg, yield 2%). LCMS (ESI): m/z 785 [M+H]⁺. **¹H NMR** (400 MHz, DMSO-d6) δ 11.79 (s, 1H), 10.25 (s, 1H), 8.87 (s, 1H), 8.48-8.36 (m, 2H), 8.21 (d, *J* = 7.6 Hz, 1H), 7.95 (s, 1H), 7.78 (d, *J* = 9.0 Hz, 2H), 7.70 (s, 1H), 7.13 (d, *J* = 8.8 Hz, 2H), 7.07 (d, *J* = 9.1 Hz, 2H), 6.93 *(d, J=* 8.9 Hz, 2H), 3.72-3.62 (m, 2H), 3.28-3.18 (m, 4H), 3.15-3.01 (m, 6H), 2.72-2.62 (m, 3H), 2.46-2.36 (m, 3H), 2.07-1.87 (m, 3H), 1.70 (s, 2H), 1.58 (s, 2H), 1.52-1.43 (m, 2H).

### Preparation Example 398. Synthesis of Compound A393

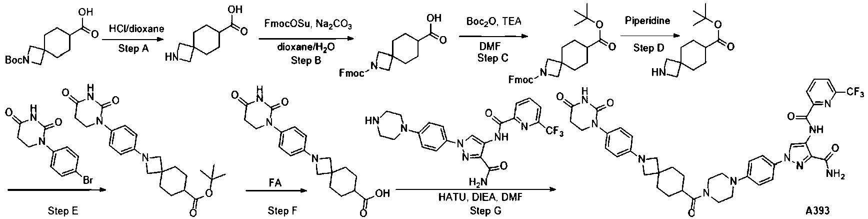

### Step A: 2-Azaspiro[3.5]nonane-7-carboxylic acid

A mixture of 2-(tert-butoxycarbonyl)-2-azaspiro[3.5]nonane-7-carboxylic acid (200 mg, 0.74 mmol) in DCM (3 mL) and TFA (1 mL) was stirred at rt for 1 h. The mixture was then concentrated to afford the title compound (yellow oil, 210 mg, crude).

### Step B: 2-(((9H-Fluoren-9-yl)methoxy)carbonyl)-2-azaspiro[3.5]nonane-7-carboxylic acid

To a mixed solution of 2-azaspiro[3.5]nonane-7-carboxylic acid (200 mg, 1.18 mmol, 1.0 eq) in dioxane (2 mL) and water (2 mL) were added sodium carbonate (375.80 mg, 3.55 mmol, 3.0 eq) and (9H-fluoren-9-yl)methyl (2,5-dioxopyrrolidin-1-yl)carbonate (478.42 mg, 1.42 mmol, 1.2 eq). The mixture was stirred at rt overnight. Upon completion, the reaction solution was poured into saturated aqueous NH₄Cl and extracted with EtOAc. The organic phase was washed with saturated aqueous NaCl, dried over anhydrous Na₂SO₄, filtered, and concentrated. The residue was purified by column chromatography to afford the title compound (white solid, 400 mg, yield 86%).

### Step C: 2-((9H-fluoren-9-yl)methyl)7-(tert-butyl)2-azaspiro[3.5]nonane-2,7-dicarboxylate

To a solution of 2-(((9H-fluoren-9-yl)methoxy)carbonyl)-2-azaspiro[3.5]nonane-7-carboxylic acid (400 mg, 1.02 mmol, 1.0 eq) in DCM (5 mL) were added di-tert-butyl dicarbonate (0.47 mL, 2.04 mmol, 2.0 eq), TEA (0.71 mL, 5.11 mmol, 5.0 eq), and 4-dimethylaminopyridine (12.48 mg, 0.10 mmol, 0.1 eq). The reaction solution was stirred at rt for 2 h. The reaction solution was poured into water and extracted with EtOAc. The organic phase was washed with aqueous NaCl, dried over anhydrous Na₂SO₄, and concentrated under reduced pressure. The residue was purified by column chromatography to afford the title compound (white solid, 350 mg, yield 77%).

### Step D: Tert-butyl 2-azaspiro[3.5]nonane-7-carboxylate

To a solution of 2-((9H-fluoren-9-yl)methyl)7-(tert-butyl)2-azaspiro[3.5]nonane-2,7-dicarboxylate (350 mg, 0.78 mmol) in DCM (4 mL) was added piperidine (1 mL). The reaction solution was stirred at rt for 30 min. The reaction solution was poured into water and extracted with DCM. The organic phase was washed with aqueous NaCl, dried over anhydrous Na₂SO₄, and concentrated under reduced pressure. The residue was purified by column chromatography to afford the title compound (yellow oil, 100 mg, yield 57%).

### Step E: Tert-butyl 2-(4-(2,4-dioxotetrahydropyrimidin-1(2H)-yl)phenyl)-2-azaspiro[3.5]nonane-7-carboxylate

To a solution of 1-(4-bromophenyl)dihydropyrimidine-2,4(1H,3H)-dione (100 mg, 0.37 mmol, 1.0 eq), tert-butyl 2-azaspiro[3.5]nonane-7-carboxylate (100 mg, 0.44 mmol, 1.19 eq), and Pd-PEPPSI-IPentCl (36.15 mg, 0.04 mmol, 0.1 eq) in dioxane (5 mL) was added cesium carbonate (242.16 mg, 0.74 mmol, 2.0 eq). The reaction solution was purged with N₂ (×3), and then stirred at 120°C under N₂ overnight. Upon completion, the mixture was cooled to rt, diluted in EtOAc, washed sequentially with water and saturated aqueous NaCl, dried over anhydrous Na₂SO₄, filtered, and concentrated under reduced pressure. The residue was purified by column chromatography to afford the title compound (white solid, 50 mg, yield 33%).

### Step F: 2-(4-(2,4-Dioxotetrahydropyrimidin-1(2H)-yl)phenyl)-2-azaspiro[3.5]nonane-7-carboxylic acid

A solution of tert-butyl 2-(4-(2,4-dioxotetrahydropyrimidin-1 (2H)-yl)phenyl)-2-azaspiro[3.5]nonane-7-carboxylate (50 mg, 0.12 mmol) in formic acid (3 mL) was stirred at 40°C for 1 h. The mixture was concentrated directly to afford the title compound (white solid, 40 mg, yield 93%).

### Step G: N-(3-Carbamoyl-1-(4-(4-(2-(4-(2,4-dioxotetrahydropyrimidin-1(2H)-yl)phenyl)-2-azaspiro[3.5]nonane-7-carbonyl)piperazin-1-yl)phenyl)-1H-pyrazol-4-yl)-6-(trifluoromethyl)picolinamide

To a solution of 2-(4-(2,4-dioxotetrahydropyrimidin-1(2H)-yl)phenyl)-2-azaspiro[3.5]nonane-7-carboxylic acid (40 mg, 0.11 mmol, 1.0 eq) in DMF (3 mL) were added HATU (64 mg, 0.17 mmol, 1.5 eq), DIEA (72 mg, 0.56 mmol, 5.0 eq), and N-(3-carbamoyl-1-(4-(piperazin-1-yl)phenyl)-1H-pyrazol-4-yl)-6-(trifluoromethyl)picolinamide (57 mg, 0.12 mmol, 1.0 eq) at rt. The reaction solution was stirred at rt for 1 h. Upon completion, the reaction solution was poured into saturated aqueous NH₄Cl and extracted with EtOAc. The organic phase was dried over anhydrous Na₂SO₄ and concentrated. The crude was purified by HPLC to afford the title compound (white solid, 9.5 mg, yield 11%). LCMS (ESI): m/z 799 [M+H]⁺. **¹H NMR** (400 MHz, DMSO-*d₆*) δ 11.79 (s, 1H), 10.23 (d, *J =* 3.0 Hz, 1H), 8.90 (d, *J =* 3.3 Hz, 1H), 8.50-8.35 (m, 2H), 8.24-8.18 (m, 1H), 7.96 (s, 1H), 7.83 (d, *J* = 4.4 Hz, 2H), 7.72 (s, 1H), 7.16-7.05 (m, 4H), 6.43 (d, *J =* 4.5 Hz, 2H), 3.75-3.59 (m, 9H), 3.32-3.16 (m, 5H), 2.71-2.64 (m, 3H), 1.94 (d, *J* = 11.0 Hz, 2H), 1.69-1.55 (m, 4H), 1.51-1.40 (m, 2H).

### Preparation Example 399. Synthesis of Compound A394

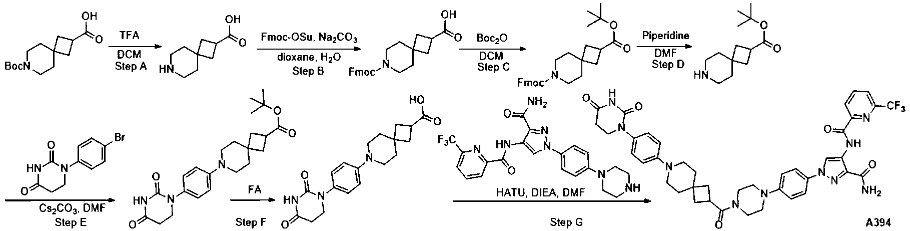

### Step A: 7-Azaspiro[3.5]nonane-2-carboxylic acid

To a solution of 7-(tert-butoxycarbonyl)-7-azaspiro[3.5]nonane-2-carboxylic acid (500 mg, 1.86 mmol, 1.0 eq) in DCM (3 mL) was added TFA (1 mL) at rt. The reaction solution was stirred at rt for 1 h. Upon completion, the reaction solution was directly concentrated under reduced pressure to afford the title compound (yellow oil, 300 mg, crude), which was used directly in the next step.

### Step B: 7-(((9H-Fluoren-9-yl)methoxy)carbonyl)-7-azaspiro[3.5]nonane-2-carboxylic acid

To a solution of 7-azaspiro[3.5]nonane-2-carboxylic acid (300 mg, 1.77 mmol, 1.0 eq) in 1,4-dioxane (4 mL) and water (1 mL) were added (9H-fluoren-9-yl)methyl (2,5-dioxopyrrolidin-1-yl)carbonate (658 mg, 1.95 mmol, 1.1 eq) and sodium carbonate (564 mg, 5.32 mmol, 3.0 eq) at rt. The reaction solution was stirred at rt for 18 h. The reaction solution was poured into water and extracted with EtOAc. The organic phase was washed with aqueous NaCl, dried over anhydrous Na₂SO₄, and concentrated under reduced pressure. The residue was purified by column chromatography to afford the title compound (white solid, 600 mg, yield 86%).

### Step C: 7-((9H-fluoren-9-yl)methyl) 2-(tert-butyl) 7-azaspiro[3.5]nonane-2,7-dicarboxylate

To a solution of 7-(((9H-fluoren-9-yl)methoxy)carbonyl)-7-azaspiro[3.5]nonane-2-carboxylic acid (600 mg, 1.53 mmol, 1.0 eq) in DCM (10 mL) were added pyridine (0.87 mL, 10.73 mmol, 7.0 eq), 4-dimethylaminopyridine (187 mg, 1.53 mmol, 1.0 eq), and di-tert-butyl dicarbonate (1.67 g, 7.66 mmol, 5.0 eq) at rt. The reaction solution was stirred at rt for 2 h, poured into water and extracted with EtOAc. The organic phase was washed with aqueous NaCl, dried over anhydrous Na₂SO₄, and concentrated under reduced pressure. The residue was purified by column chromatography to afford the title compound (white solid, 470 mg, yield 69%).

### Step D: Tert-butyl 7-azaspiro[3.5]nonane-2-carboxylate

To a solution of 7-((9H-fluoren-9-yl)methyl) 2-(tert-butyl) 7-azaspiro[3.5]nonane-2,7-dicarboxylate (470 mg, 1.05 mmol, 1.0 eq) in DMF (4 mL) was added piperidine (1 mL) at rt. After stirring at rt for 30 min, the reaction solution was poured into water and extracted with DCM. The organic phase was washed with aqueous NaCl, dried over anhydrous Na₂SO₄, and concentrated under reduced pressure. The residue was purified by column chromatography to afford the title compound (yellow oil, 80 mg, yield 34%).

### Step E: Tert-butyl 7-(4-(2,4-dioxotetrahydropyrimidin-1(2H)-yl)phenyl)-7-azaspiro[3.5]nonane-2-carboxylate

To a solution of tert-butyl 7-azaspiro[3.5]nonane-2-carboxylate (89 mg, 0.36 mmol, 1.0 eq) and 1-(4-bromophenyl)dihydropyrimidine-2,4(1H,3H)-dione (96 mg, 0.36 mmol, 1.0 eq) in 1,4-dioxane (2 mL) were added Pd-PEPPSI-IPentCl (35 mg, 0.04 mmol, 0.1 eq) and cesium carbonate (347 mg, 1.07 mmol, 3.0 eq) at rt. The reaction solution was purged with N₂ (×3), and then stirred at 110°C under N₂ for 18 h. Upon completion, the mixture was cooled to rt, diluted in EtOAc, washed sequentially with water and saturated aqueous NaCl, dried over anhydrous Na₂SO₄, filtered, and concentrated under reduced pressure. The residue was purified by column chromatography to afford the title compound (yellow oil, 40 mg, yield 27%).

### Step F: 7-(4-(2,4-Dioxotetrahydropyrimidin-1(2H)-yl)phenyl)-7-azaspiro[3.5]nonane-2-carboxylic acid

A solution of tert-butyl 7-(4-(2,4-dioxotetrahydropyrimidin-1(2H)-yl)phenyl)-7-azaspiro[3.5]nonane-2-carboxylate (40 mg, 0.10 mmol, 1.0 eq) in formic acid (2 mL) was stirred at rt for 2 h. Upon completion, the reaction solution was directly concentrated under reduced pressure to afford the title compound (black oil, 30 mg, crude), which was used directly without purification.

### Step G: N-(3-Carbamoyl-1-(4-(4-(7-(4-(2,4-dioxotetrahydropyrimidin-1(2H)-yl)phenyl)-7-azaspiro[3.5]nonane-2-carbonyl)piperazin-1-yl)phenyl)-1H-pyrazol-4-yl)-6-(trifluoromethyl)picolinamide

To a solution of 7-(4-(2,4-dioxotetrahydropyrimidin-1(2H)-yl)phenyl)-7-azaspiro[3.5]nonane-2-carboxylic acid (30 mg, 0.08 mmol, 1.0 eq) and N-(3-carbamoyl-1-(4-(piperazin-1-yl)phenyl)-1H-pyrazol-4-yl)-6-(trifluoromethyl)picolinamide (39 mg, 0.08 mmol, 1.0 eq) in DMF (2 mL) were added HATU (48 mg, 0.13 mmol, 1.5 eq) and DIEA (54 mg, 0.42 mmol, 5.0 eq) at rt. The reaction solution was stirred at rt for 1 h. The reaction solution was poured into water and extracted with EtOAc. The organic phase was washed with aqueous NaCl, dried over anhydrous Na₂SO₄, and concentrated under reduced pressure. The residue was purified by HPLC to afford the title compound (white solid, 8.2 mg, yield 12%). LCMS (ESI): m/z 799.6 [M+H]⁺. **¹H NMR** (400 MHz, DMSO-*d₆*) δ 11.80 (s, 1H), 10.25 (s, 1H), 8.90 (s, 1H), 8.47-8.38 (m, 2H), 8.24-8.19 (m, 1H), 7.96 (s, 1H), 7.85-7.80 (m, 2H), 7.72 (s, 1H), 7.16-7.08 (m, 4H), 6.97-6.89 (m, 2H), 3.72-3.67 (m, 2H), 3.66-3.59 (m, 2H), 3.55-3.47 (m, 2H), 3.43-3.39 (m, 1H), 3.23-3.18 (m, 4H), 3.16-3.11 (m, 2H), 3.07-3.01 (m, 2H), 2.71-2.65 (m, 2H), 2.05-1.97 (m, 4H), 1.78-1.71 (m, 2H), 1.60-1.53 (m, 2H).

### Preparation Example 400. Synthesis of Compound A395

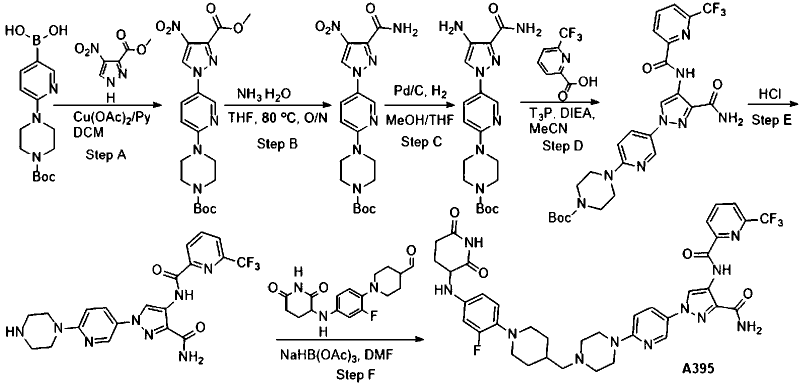

### Step A: Tert-butyl 4-(5-(3-(methoxycarbonyl)-4-nitro-1H-pyrazol-1-yl)pyridin-2-yl)piperazine-1-carboxylate

At rt, to a solution of (6-(4-(tert-butoxycarbonyl)piperazin-1-yl)pyridin-3-yl)boronic acid (1 g, 3.2 mmol, 1.0 eq) in DMF (10 mL) were added methyl 4-nitro-1H-pyrazole-3-carboxylate (0.6 g, 3.5 mmol, 1.1 eq), Cu(OAc)₂ (60 mg, 0.3 mmol, 0.1 eq), and pyridine (0.7 mL, 9.7 mmol, 3.0 eq). The mixture was stirred at 80°C under O₂ for 16 h. The reaction mixture was filtered, and the filtrate was poured into ice water and extracted with EtOAc. The organic layer was washed with aqueous NaCl, dried over Na₂SO₄, and concentrated. The residue was purified by silica gel column chromatography to afford the title compound (pale yellow solid, 700 mg, yield 50%).

### Step B: Tert-butyl 4-(5-(3-carbamoyl-4-nitro-1H-pyrazol-1-yl)pyridin-2-yl)piperazine-1-carboxylate

At rt, to a solution of tert-butyl 4-(5-(3-(methoxycarbonyl)-4-nitro-1H-pyrazol-1-yl)pyridin-2-yl)piperazine-1-carboxylate (700 mg, 1.6 mmol, 1.0 eq) in THF (5 mL) was added aqueous ammonia (5 mL). The mixture was stirred at 80°C for 16 h. The reaction mixture was concentrated. The residue was purified by silica gel column chromatography to afford the title compound (yellow solid, 500 mg, yield 74%).

### Step C: Tert-butyl 4-(5-(4-amino-3-carbamoyl-1H-pyrazol-1-yl)pyridin-2-yl)piperazine-1-carboxylate

At rt, to a solution of tert-butyl 4-(5-(3-carbamoyl-4-nitro-1H-pyrazol-1-yl)pyridin-2-yl)piperazine-1-carboxylate (500 mg, 1.1 mmol, 1.0 eq) in MeOH (5 mL) was added Pd/C (10%, 127 mg). The reaction mixture was purged with H₂ (×3), and then stirred under H₂ at rt for 16 h. Upon completion, the reaction solution was filtered through Celite, and the filtrate was concentrated in vacuo to afford the title compound (yellow solid, 400 mg, crude).

### Step D: Tert-butyl 4-(5-(3-carbamoyl-4-(6-(trifluoromethyl)picolinamido)-1H-pyrazol-1-yl)pyridin-2-yl)piperazine-1-carboxylate

At rt, to a solution of tert-butyl 4-(5-(4-amino-3-carbamoyl-1H-pyrazol-1-yl)pyridin-2-yl)piperazine-1-carboxylate (150 mg, crude) in acetonitrile (5 mL) were added 6-(trifluoromethyl)picolinic acid (88 mg, 0.46 mmol, 1.2 eq), DIEA (0.3 mL, 1.95 mmol, 5.0 eq), and T3P (50%, 496 mg, 0.78 mmol, 2.0 eq). The mixture was stirred at rt for 16 h. The reaction mixture was filtered, and the filter cake was concentrated. The residue was purified by silica gel column chromatography to afford the title compound (white solid, 180 mg, yield 83% over two steps).

### Step E: N-(3-Carbamoyl-1-(6-(piperazin-1-yl)pyridin-3-yl)-1H-pyrazol-4-yl)-6-(trifluoromethyl)picolinamide

Tert-butyl 4-(5-(3-carbamoyl-4-(6-(trifluoromethyl)picolinamido)-1H-pyrazol-1-yl)pyridin-2-yl)piperazine-1-carboxylate (180 mg, 0.3 mmol) was dissolved in 4 M hydrogen chloride-dioxane (5 mL). The mixture was stirred at rt for 2 h. Upon completion, the reaction mixture was concentrated under reduced pressure to afford the title compound (white solid, 140 mg, crude).

### Step F: N-(3-Carbamoyl-1-(6-(4-((1-(4-((2,6-dioxopiperidin-3-yl)amino)-2-fluorophenyl)piperidin-4-yl)methyl)piperazin-1-yl)pyridin-3-yl)-1H-pyrazol-4-yl)-6-(trifluoromethyl)picolinamide

To a solution of N-(3-carbamoyl-1-(6-(piperazin-1-yl)pyridin-3-yl)-1H-pyrazol-4-yl)-6-(trifluoromethyl)picolinamide (100 mg, crude) in DMF (5 mL) were added AcOH (0.5 mL) and 1-(4-((2,6-dioxopiperidin-3-yl)amino)-2-fluorophenyl)piperidine-4-carbaldehyde (79 mg, 0.24 mmol, 1.1 eq). The mixture was stirred at rt for 16 h, and then sodium triacetoxyborohydride (228 mg, 1.05 mmol, 5.0 eq) was added. The mixture was stirred at rt for 1 h. The reaction mixture was poured into ice water and filtered. The filter cake was collected. The residue was purified by preparative high performance liquid chromatography to afford the title compound (white solid, 35 mg, 0.04 mmol, yield 21% over two steps). **¹H NMR** (400 MHz, DMSO-*d6*) δ 11.80 (s, 1H), 10.78 (s, 1H), 8.92 (s, 1H), 8.69 (d, *J =* 2.8 Hz, 1H), 8.48-8.35 (m, 2H), 8.22 (d, *J=* 7.6 Hz, 1H), 8.11 (dd, *J= 9.2,* 2.7 Hz, 1H), 7.97 (s, 1H), 7.74 (s, 1H), 7.00 (d, *J* = 9.3 Hz, 1H), 6.84 *(t, J=* 9.3 Hz, 1H), 6.51 (dd, *J* = 14.9, 2.3 Hz, 1H), 6.43 *(d, J=* 8.7 Hz, 1H), 5.79 (d, *J* = 7.6 Hz, 1H), 4.35-4.15 (m, 1H), 3.57 (s, 4H), 3.13 (d, *J* = 11.3 Hz, 3H), 2.74 (ddd, *J=* 17.3, 12.1, 5.3 Hz, 1H), 2.63-2.53 (m, 4H), 2.24 (d, *J* = 7.0 Hz, 2H), 2.17-2.02 (m, 1H), 1.93-1.74 (m, 3H), 1.65 (s, 1H), 1.28 (dd, *J=* 20.8, 11.3 Hz, 2H). LCMS (ESI): m/z 778 [M+H]⁺.

### Preparation Example 401. Synthesis of Compound A396

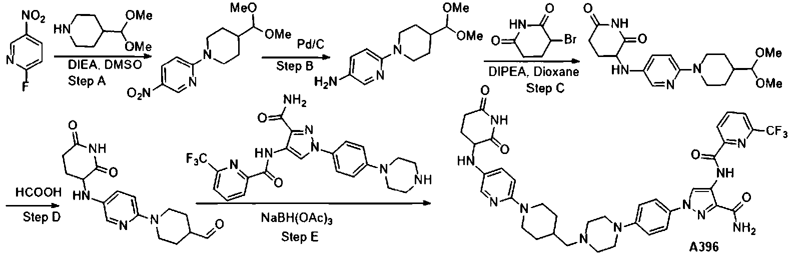

### Step A: 2-(4-(Dimethoxymethyl)piperidin-1-yl)-5-nitropyridine

At rt, DIEA (12.3 mL, 70.4 mmol, 2.0 eq) was added to a solution of 2-fluoro-5-nitropyridine (5 g, 35.2 mmol, 1.0 eq) and 4-(dimethoxymethyl)piperidine (6.7 g, 42.2 mmol, 1.2 eq) in dimethyl sulfoxide (60 mL). The mixture was stirred at 80°C for 12 h. Upon completion, the mixture was diluted in water (100 mL) and extracted with EtOAc (100 mL×3). The organic layers were washed with saturated aqueous NaCl (100 mL), dried over anhydrous Na₂SO₄, and concentrated under reduced pressure. The residue was purified by column chromatography to afford the title compound (yellow solid, 8.1 g, yield 82%).

### Step B: 6-(4-(Dimethoxymethyl)piperidin-1-yl)pyridin-3-amine

At rt, 10% Pd/C (0.3 g) was added to a solution of 2-(4-(dimethoxymethyl)piperidin-1-yl)-5-nitropyridine (2.5 g, 8.9 mmol, 1.0 eq) in ethanol (40 mL). The mixture was stirred at rt for 16 h. Upon completion, the mixture was filtered through Celite, and the filtrate was concentrated under reduced pressure. The residue was purified by column chromatography to afford the title compound (yellow solid, 1.8 g, yield 81%).

### Step C: 3-((6-(4-(Dimethoxymethyl)piperidin-1-yl)pyridin-3-yl)amino)piperidine-2,6-dione

At rt, DIEA (1.9 g, 14.3 mmol, 2.0 eq) was added to a solution of 6-(4-(dimethoxymethyl)piperidin-1-yl)pyridin-3-amine (1.8 g, 7.2 mmol, 1.0 eq) and 3-bromopiperidine-2,6-dione (1.7 g, 8.6 mmol, 1.2 eq) in dioxane (25 mL). The mixture was stirred at 80°C for 12 h. Upon completion, the reaction solution was diluted in water (100 mL) and extracted with EtOAc (100 mL×3). The organic layers were washed with saturated aqueous NaCl (100 mL), dried over anhydrous Na₂SO₄, and concentrated under reduced pressure. The residue was purified by column chromatography to afford the title compound (yellow solid, 1.2 g, yield 46%)

### Step D: 1-(5-((2,6-Dioxopiperidin-3-yl)amino)pyridin-2-yl)piperidine-4-carbaldehyde

At rt, 3-((6-(4-(dimethoxymethyl)piperidin-1-yl)pyridin-3-yl)amino)piperidine-2,6-dione (200 mg, 0.6 mmol, 1.0 eq) was added to a solution of formic acid (5 mL). The mixture was stirred at rt for 1 h. Upon completion, the reaction solution was concentrated under reduced pressure to afford the title compound (green oil, 170 mg, crude).

### Step E: N-(3-Carbamoyl-1-(4-(4-((1-(5-((2,6-dioxopiperidin-3-yl)amino)pyridin-2-yl)piperidin-4-yl)methyl)piperazin-1-yl)phenyl)-1H-pyrazol-4-yl)-6-(trifluoromethyl)picolinamide

At rt, 1-(5-((2,6-dioxopiperidin-3-yl)amino)pyridin-2-yl)piperidine-4-carbaldehyde (170 mg, crude) and N-(3-carbamoyl-1-(4-(piperazin-1-yl)phenyl)-1H-pyrazol-4-yl)-6-(trifluoromethyl)picolinamide (247 mg, 0.5 mmol, 1.0 eq) were added to DMF (5 mL), and the mixture was stirred at rt for 30 min. NaBH(OAc)3 (456 mg, 2.1 mmol, 4.0 eq) was then added. The mixture was stirred at rt for 1 h. Upon completion, the reaction solution was diluted in water (100 mL) and extracted with EtOAc (30 mL×3). The organic layer was washed with saturated aqueous NaCl, dried over anhydrous Na₂SO₄, and concentrated under reduced pressure. The residue was purified by column chromatography to afford the title compound (white solid, 59.6 mg, yield 15%). **¹H NMR** (400 MHz, DMSO-d6) δ 11.79 (s, 1H), 10.76 (s, 1H), 8.90-8.85 (m, 1H), 8.46-8.37 (m, 2H), 8.21 *(d, J=* 6.8 Hz, 1H), 7.95 (s, 1H), 7.79 (d, *J =* 9.0 Hz, 2H), 7.74-7.65 (m, 2H), 7.11-7.01 (m, 3H), 6.68 (d, *J=* 9.1 Hz, 1H), 5.36 (d, *J* = 7.4 Hz, 1H), 4.33-3.95 (m, 3H), 3.25-3.19 (m, 4H), 2.81-2.55 (m, 5H), 2.54-2.52 (m, 2H), 2.24-2.19 (m, 2H), 2.14-2.08 (m, 1H), 1.88-1.70 (m, 4H), 1.25-1.06 (m, 3H). LCMS (ESI): m/z 760 [M+H]⁺.

### Preparation Example 402. Synthesis of Compound A397

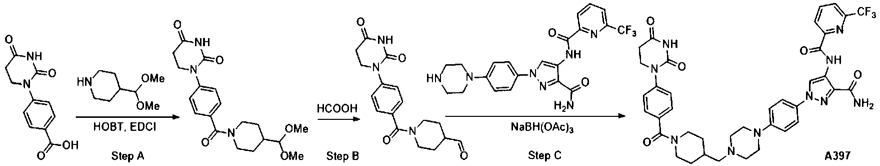

### Step A: 1-(4-(4-(Dimethoxymethyl)piperidine-1-carbonyl)phenyl)dihydropyrimidine-2,4(1H,3H)-dione

To a solution of 4-(2,4-dioxotetrahydropyrimidin-1(2H)-yl)benzoic acid (600 mg, 2.5 mmol, 1.0 eq) in DMF (10 mL) were added 4-(dimethoxymethyl)piperidine (489 mg, 3.1 mmol, 1.2 eq), HOBT (692 mg, 5.0 mmol, 2.0 eq), EDCI (982 mg, 5.0 mmol, 2.0 eq), and DIEA (1.3 mL, 7.5 mmol, 3.0 eq). The mixture was stirred at rt under N₂ for 16 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 300 mg, yield 31%).

### Step B: 1-(4-(2,4-Dioxotetrahydropyrimidin-1(2H)-yl)benzoyl)piperidine-4-carbaldehyde

A solution of 1-(4-(4-(dimethoxymethyl)piperidine-1-carbonyl)phenyl)dihydropyrimidine-2,4(1H,3H)-dione (100 mg, 0.27 mmol, 1.0 eq) in formic acid (5 mL) was stirred at rt for 1 h. Upon completion, the reaction solution was concentrated under reduced pressure to afford the title compound (white solid, 85 mg, crude).

### Step C: N-(3-Carbamoyl-1-(4-(4-((1-(4-(2,4-dioxotetrahydropyrimidin-1(2H)-yl)benzoyl)piperidin-4-yl)methyl)piperazin-1-yl)phenyl)-1H-pyrazol-4-yl)-6-(trifluoromethyl)picolinamide

At rt, to a solution of 1-(4-(2,4-dioxotetrahydropyrimidin-1(2H)-yl)benzoyl)piperidine-4-carbaldehyde (114 mg, crude) in DMF (10 mL) were added AcOH (32 mg, 0.17 mmol, 0.5 eq) and N-(3-carbamoyl-1-(4-(piperazin-1-yl)phenyl)-1H-pyrazol-4-yl)-6-(trifluoromethyl)picolinamide (167 mg, 0.36 mmol, 1.0 eq). The mixture was stirred for 1 h. Then, NaBH(OAc)3 (216 mg, 0.69 mmol, 2.0 eq) was added. The mixture was stirred at rt for 20 min. Upon completion, the resulting reaction solution was purified by HPLC to afford the title compound (white solid, 30 mg, yield 11%). **¹H NMR** (400 MHz, DMSO-d6) δ 11.79 (s, 1H), 10.43 (s, 1H), 8.87 (s, 1H), 8.48-8.34 (m, 2H), 8.21 (d, *J=* 7.6 Hz, 1H), 7.94 (s, 1H), 7.78 (d, *J* = 9.0 Hz, 2H), 7.71 (s, 1H), 7.40 (s, 4H), 7.07 (d, *J=* 9.1 Hz, 2H), 3.87-3.80 (m, 2H), 3.31-3.28 (m, 2H), 3.24-3.15 (m, 4H), 3.14-2.74 (m, 2H), 2.74-2.69 (m, 2H), 2.27-2.19 (m, 2H), 2.08-2.06 (m, 4H), 1.91-1.68 (m, 3H), 1.17-1.03 (m, 2H). LCMS (ESI): m/z 773 [M+H]⁺.

### Preparation Example 403. Synthesis of Compound A398

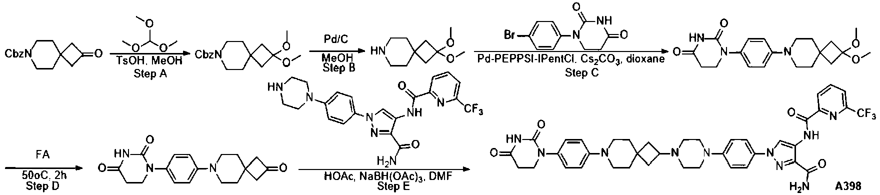

### Step A: Benzyl 2,2-dimethoxy-7-azaspiro[3.5]nonane-7-carboxylate

At rt, to a solution of benzyl 2-oxo-7-azaspiro[3.5]nonane-7-carboxylate (1 g, 3.66 mmol, 1.0 eq) in MeOH (5 mL) were added trimethyl orthoformate (10 mL) and p-toluenesulfonic acid (35 mg, 0.183 mmol, 0.05 eq). The reaction solution was stirred at 65°C overnight. Upon completion, the reaction solution was concentrated, diluted in EtOAc, washed sequentially with water and saturated aqueous NaCl, dried over anhydrous Na₂SO₄, and concentrated under reduced pressure to afford the title compound (colorless oil, 1.16 g, crude), which was used directly without purification.

### Step B: 2,2-Dimethoxy-7-azaspiro[3.5]nonane

At rt, to a solution of benzyl 2,2-dimethoxy-7-azaspiro[3.5]nonane-7-carboxylate (1.16 g, 3.66 mmol, 1.0 eq) in MeOH (4 mL) was added 10% wet palladium on carbon (110 mg). The reaction solution was stirred under H₂ at rt for 1 h. Upon completion, the reaction solution was filtered through a Buchner funnel, and the filtrate was concentrated under reduced pressure to afford the title compound (white solid, 600 mg, crude), which was used directly without purification.

### Step C: 1-(4-(2,2-Dimethoxy-7-azaspiro[3.5]nonan-7-yl)phenyl)dihydropyrimidine-2,4(1H,3H)-dione

At rt, to a solution of 2,2-dimethoxy-7-azaspiro[3.5]nonane (600 mg, 3.24 mmol, 1.0 eq) and 1-(4-bromophenyl)dihydropyrimidine-2,4(1H,3H)-dione (870 mg, 3.24 mmol, 1.0 eq) in 1,4-dioxane (15 mL) were added Pd-PEPPSI-IPentCl (158 mg, 0.162 mmol, 0.05 eq) and cesium carbonate (2.1 g, 6.48 mmol, 2.0 eq). The reaction solution was stirred at 120°C overnight under N₂. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 70 mg, yield 6%).

### Step D: 1-(4-(2-Oxo-7-azaspiro[3.5]nonan-7-yl)phenyl)dihydropyrimidine-2,4(1H,3H)-dione

At rt, formic acid (7 mL) was added to 1-(4-(2,2-dimethoxy-7-azaspiro[3.5]nonan-7-yl)phenyl)dihydropyrimidine-2,4(1H,3H)-dione (70 mg, 0.188 mmol, 1.0 eq). The reaction solution was stirred at 50°C for 2 h. Upon completion, the reaction solution was concentrated under reduced pressure to afford the title compound (yellow oil, 60 mg, yield 98%).

### Step E: N-(3-Carbamoyl-1-(4-(4-(7-(4-(2,4-dioxotetrahydropyrimidin-1(2H)-yl)phenyl)-7-azaspiro[3.5]nonan-2-yl)piperazin-1-yl)phenyl)-1H-pyrazol-4-yl)-6-(trifluoromethyl)picolinamide

At rt, to a solution of 1-(4-(2-oxo-7-azaspiro[3.5]nonan-7-yl)phenyl)dihydropyrimidine-2,4(1H,3H)-dione (60 mg, 0.183 mmol, 1.0 eq) and N-(3-carbamoyl-1-(4-(piperazin-1-yl)phenyl)-1H-pyrazol-4-yl)-6-(trifluoromethyl)picolinamide (84 mg, 0.183 mmol, 1.0 eq) in DMF (6 mL) was added AcOH (0.12 mL, 2.09 mmol, 11.0 eq), and the mixture was stirred for 1 h. Then sodium triacetoxyborohydride (78 mg, 0.366 mmol, 2.0 eq) was added, and the mixture was stirred at 50°C for 2 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (yellow solid, 5.5 mg, yield 4%). LCMS (ESI): m/z 771 [M+H]⁺. **¹H NMR** (400 MHz, DMSO-*d₆*) δ 11.80 (s, 1H), 10.26 (s, 1H), 8.88 (s, 1H), 8.47-8.37 (m, 2H), 8.21 (d, *J=* 7.6 Hz, 1H), 7.95 (s, 1H), 7.79 (d, *J =* 9.0 Hz, 2H), 7.71 (s, 1H), 7.13 (d, *J =* 8.9 Hz, 2H), 7.08 (d, *J=* 9.2 Hz, 2H), 6.93 (d, *J=* 9.0 Hz, 2H), 3.69 *(t, J=* 6.7 Hz, 2H), 3.22 (s, 4H), 3.14 (s, 2H), 3.06 (s, 2H), 2.78-2.72 (m, 1H), 2.68 (t, *J=* 6.7 Hz, 2H), 2.41 (s, 4H), 2.04-1.97 (m, 2H), 1.68-1.58 (m, 6H).

### Preparation Example 404. Synthesis of Compound A399

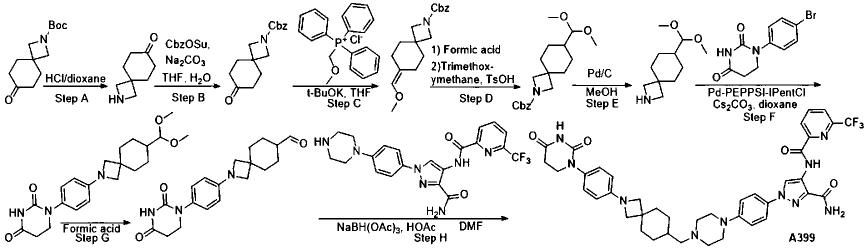

### Step A: 2-Azaspiro[3.5]nonan-7-one

At rt, a 4.0 M hydrogen chloride in dioxane solution (10.5 mL, 42 mmol, 10 eq) was added to a solution of tert-butyl 7-oxo-2-azaspiro[3.5]nonane-2-carboxylate (1.0 g, 4.2 mmol, 1.0 eq) in DCM (10 mL). The reaction solution was stirred at rt under N₂ for 2 h. Upon completion, the reaction solution was concentrated under reduced pressure to afford the title compound (white solid, 580 mg, crude), which was used directly without purification.

### Step B: Benzyl 7-oxo-2-azaspiro[3.5]nonane-2-carboxylate

At 0°C, to a mixed solution of 2-azaspiro[3.5]nonan-7-one (580 mg, 4.17 mmol, 1.0 eq) in THF (12 mL) and water (4 mL) were sequentially added sodium carbonate (883 mg, 8.34 mmol, 2.0 eq) and N-(benzyloxycarbonyloxy)succinimide (1.1 g, 4.59 mmol, 1.1 eq). The reaction solution was stirred at rt for 16 h. Upon completion, the reaction solution was diluted in water and extracted with EtOAc (×3). The organic phases were combined, washed with water and saturated aqueous NaCl, dried over anhydrous Na₂SO₄, and concentrated under reduced pressure. The residue was purified by column chromatography to afford the title compound (white solid, 1.0 g, yield 88%).

### Step C: Benzyl 7-(methoxymethylene)-2-azaspiro[3.5]nonane-2-carboxylate

At 0°C, potassium tert-butoxide (1.2 g, 10.9 mmol, 3.0 eq) was added to a solution of (methoxymethyl)triphenylphosphonium chloride (3.7 g, 10.9 mmol, 3.0 eq) in THF (30 mL). The reaction solution was stirred under N₂ for 1 h. Then a solution of benzyl 7-oxo-2-azaspiro[3.5]nonane-2-carboxylate (1.0 g, 3.7 mmol, 1.0 eq) in THF (10 mL) was added at 0°C, and the mixture was stirred at rt overnight. Upon completion, the reaction solution was quenched with saturated aqueous NH₄Cl at 0°C and extracted with EtOAc (×3). The organic phases were combined, washed with water and saturated aqueous NaCl, dried over anhydrous Na₂SO₄, and concentrated under reduced pressure. The residue was purified by column chromatography to afford the title compound (white solid, 1.0 g, yield 91%).

### Step D: Benzyl 7-(dimethoxymethyl)-2-azaspiro[3.5]nonane-2-carboxylate

A mixture of benzyl 7-(methoxymethylene)-2-azaspiro[3.5]nonane-2-carboxylate (1.0 g, 3.3 mmol, 1.0 eq) and formic acid (12.7 g, 66.3 mmol, 20.0 eq) was stirred at rt for 2 h, and then the reaction mixture was concentrated under reduced pressure to remove the formic acid. The crude was dissolved in MeOH (20 mL), and trimethoxymethane (0.5 mL, 4.9 mmol, 1.5 eq) and 4-methylbenzenesulfonic acid (0.06 g, 0.3 mmol, 0.1 eq) were added. The reaction mixture was purged with N₂ (×3) and then stirred at rt under N₂ overnight. Upon completion, the mixture was quenched with water and extracted with EtOAc (×3). The organic phases were combined, washed with water and saturated aqueous NaCl, dried over anhydrous Na₂SO₄, and concentrated under reduced pressure. The residue was purified by column chromatography to afford the title compound (white solid, 1.0 g, yield 90%).

### Step E: 7-(Dimethoxymethyl)-2-azaspiro[3.5]nonane

At rt, 10% wet palladium on carbon (0.2 g) was added to a solution of benzyl 7-(dimethoxymethyl)-2-azaspiro[3.5]nonane-2-carboxylate (1.0 g, 3.0 mmol, 1.0 eq) in MeOH (20 mL). The reaction system was purged with H₂ (×3). The mixture was stirred under H₂ at rt for 2 h. Upon completion, the reaction mixture was filtered and concentrated under reduced pressure to afford the title compound (white solid, 500 mg, crude), which was used directly without further purification.

### Step F: 1-(4-(7-(Dimethoxymethyl)-2-azaspiro[3.5]nonan-2-yl)phenyl)dihydropyrimidine-2,4(1H,3H)-dione

At rt, to a solution of 7-(dimethoxymethyl)-2-azaspiro[3.5]nonane (500 mg, 2.5 mmol, 1.0 eq) and 1-(4-bromophenyl)dihydropyrimidine-2,4(1H,3H)-dione (506 mg, 1.9 mmol, 0.75 eq) in 1,4-dioxane (10 mL) were added (SP-4-1)-[1,3-bis[2,6-bis(1-propylbutyl)phenyl]-4,5-dichloro-1,3-dihydro-2H-imidazol-2-ylidene]dichloro(3-chloropyridine-κN)palladium (122 mg, 0.1 mmol, 0.05 eq) and cesium carbonate (2.5 g, 7.5 mmol, 3.0 eq). The reaction system was purged with N₂ (×3) and then stirred at 110°C under N₂ for 16 h. Upon completion, the mixture was cooled to rt. The reaction solution was quenched with water, and extracted with EtOAc (×3). The organic phases were combined, washed with water and saturated aqueous NaCl, dried over anhydrous Na₂SO₄, and concentrated under reduced pressure. The residue was purified by column chromatography to afford the title compound (yellow solid, 500 mg, yield 51%).

### Step G: 2-(4-(2,4-Dioxotetrahydropyrimidin-1(2H)-yl)phenyl)-2-azaspiro[3.5]nonane-7-carbaldehyde

A solution of 1-(4-(7-(dimethoxymethyl)-2-azaspiro[3.5]nonan-2-yl)phenyl)dihydropyrimidine-2,4(1H,3H)-dione (500 mg, 1.3 mmol, 1.0 eq) in formic acid (10 mL) was stirred at 50°C for 2 h. Upon completion, the mixture was concentrated under reduced pressure to afford the title compound (colorless oil, 500 mg, crude), which was used directly without further purification.

### Step H: N-(3-Carbamoyl-1-(4-(4-((2-(4-(2,4-dioxotetrahydropyrimidin-1(2H)-yl)phenyl)-2-azaspiro[3.5]nonan-7-yl)methyl)piperazin-1-yl)phenyl)-1H-pyrazol-4-yl)-6-(trifluoromethyl)picolinamide

At rt, to a solution of 2-(4-(2,4-dioxotetrahydropyrimidin-1 (2H)-yl)phenyl)-2-azaspiro[3.5]nonane-7-carbaldehyde (220 mg, 0.6 mmol, 1.0 eq) and N-(3-carbamoyl-1-(4-(piperazin-1-yl)phenyl)-1H-pyrazol-4-yl)-6-(trifluoromethyl)picolinamide (296 mg, 0.6 mmol, 1.0 eq) in DMF (5 mL) was added AcOH (24 mg, 0.1 mmol, 0.2 eq). The reaction system was stirred at rt for 1 h, then sodium triacetoxyborohydride (272 mg, 1.3 mmol, 2.0 eq) was added, and the mixture was stirred for 1 additional hour. Upon completion, saturated aqueous NH₄Cl was then added, and the mixture was extracted with DCM (×3). The organic phases were combined, washed with water and saturated aqueous NaCl, dried over anhydrous Na₂SO₄, and concentrated under reduced pressure. The residue was purified by HPLC to afford the title compound (white solid, 25.8 mg, yield 5%). LCMS (ESI): m/z 785.5 [M+H]⁺. **¹H NMR** (400 MHz, DMSO-d6) δ 11.70 (s, 1H), 10.13 (s, 1H), 8.79 (s, 1H), 8.37-8.28 (m, 2H), 8.12 (d, J = 7.6 Hz, 1H), 7.85 (s, 1H), 7.73-7.59 (m, 3H), 6.99 (d, J = 8.6 Hz, 4H), 6.30 (d, J = 8.7 Hz, 2H), 3.57 (t, J = 6.7 Hz, 2H), 3.41 (d, J = 21.1 Hz, 4H), 3.23-3.18 (m, 4H), 3.11 (s, 4H), 2.58 (t, J = 6.7 Hz, 2H), 2.06 (s, 2H), 1.80 (d, J = 12.6 Hz, 2H), 1.63 (d, J = 11.6 Hz, 2H), 1.48-1.34 (m, 3H), 0.94-0.79 (m, 2H).

### Preparation Example 405. Synthesis of Compound A400

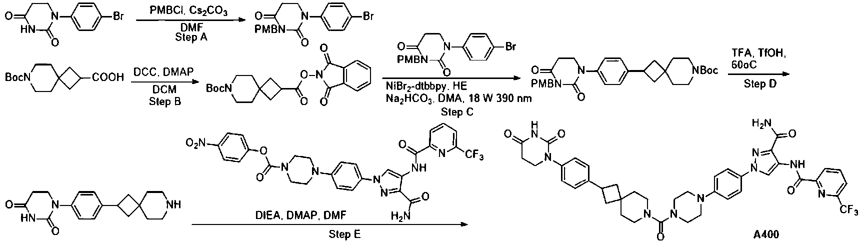

### Step A: 1-(4-Bromophenyl)-3-(4-methoxybenzyl)dihydropyrimidine-2,4(1H,3H)-dione

At rt, 1-chloromethyl-4-methoxybenzene (378 mg, 2.42 mmol, 1.3 eq) was added to a mixture of 1-(4-bromophenyl)dihydropyrimidine-2,4(1H,3H)-dione (500 mg, 1.86 mmol, 1.0 eq) and Cs₂CO₃ (726 mg, 2.23 mmol, 1.2 eq) in DMF (10 mL). The reaction solution was stirred at rt overnight. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 600 mg, yield 82%).

### Step B: 7-(tert-Butyl)2-(1,3-dioxoisoindolin-2-yl)7-azaspiro[3.5]nonane-2,7-dicarboxylate

At rt, N-hydroxyphthalimide (670 mg, 4.08 mmol, 1.1 eq), DCC (840 mg, 4.08 mmol, 1.1 eq), and DMAP (50 mg, 0.37 mmol, 0.1 eq) were added to DCM (20 mL). The mixture was cooled to 0°C, and 7-(tert-butoxycarbonyl)-7-azaspiro[3.5]nonane-2-carboxylic acid (1.0 g, 3.71 mmol, 1.0 eq) was added. The mixture was stirred at rt under N₂ overnight. Upon completion, the reaction solution was diluted in DCM, washed sequentially with water and saturated aqueous NaCl, dried over anhydrous Na₂SO₄, and concentrated under reduced pressure. The residue was purified by column chromatography to afford the title compound (white solid, 700 mg, yield 45%).

### Step C: Tert-butyl 2-(4-(3-(4-methoxybenzyl)-2,4-dioxotetrahydropyrimidin-1(2H)-yl)phenyl)-7-azaspiro[3.5]nonane-7-carboxylate

At rt, to a solution of 1-(4-bromophenyl)-3-(4-methoxybenzyl)dihydropyrimidine-2,4(1H,3H)-dione (500 mg, 1.28 mmol, 1.0 eq) and 7-(tert-butyl)2-(1,3-dioxoisoindolin-2-yl)7-azaspiro[3.5]nonane-2,7-dicarboxylate (700 mg, 1.93 mmol, 1.5 eq) in N,N-dimethylacetamide (15 mL) were added 2,6-dimethyl-1,4-diethyldihydropyridine-3,5-dicarboxylate (HE, 650 mg, 2.57 mmol, 2.0 eq), NiBr₂-dtbbpy (125 mg, 0.26 mmol, 0.2 eq), and NaHCO₃ (215 mg, 2.57 mmol, 2.0 eq). The reaction solution was stirred under N₂ and 18W 390 nm illumination at rt for 4 h. Upon completion, the reaction solution was diluted in EtOAc, washed sequentially with water and saturated aqueous NaCl, dried over anhydrous Na₂SO₄, and concentrated under reduced pressure. The residue was purified by reverse-phase column chromatography to afford the title compound (white solid, 200 mg, yield 29%).

### Step D: 1-(4-(7-Azaspiro[3.5]nonan-2-yl)phenyl)dihydropyrimidine-2,4(1H,3H)-dione

At rt, trifluoromethanesulfonic acid (0.5 mL) was added to a solution of tert-butyl 2-(4-(3-(4-methoxybenzyl)-2,4-dioxotetrahydropyrimidin-1(2H)-yl)phenyl)-7-azaspiro[3.5]nonane-7-carboxylate (200 mg, 0.37 mmol) in TFA (5 mL). The reaction solution was heated to 60°C and stirred for 3 h. The reaction solution was concentrated under reduced pressure, and the residue was adjusted to pH 7-8 with saturated NaHCO₃ solution and lyophilized to afford the title compound (yellow solid, 200 mg, crude), which was used directly without purification.

### Step E: N-(3-Carbamoyl-1-(4-(4-(2-(4-(2,4-dioxotetrahydropyrimidin-1(2H)-yl)phenyl)-7-azaspiro[3.5]nonane-7-carbonyl)piperazin-1-yl)phenyl)-1H-pyrazol-4-yl)-6-(trifluoromethyl)picolinamide

At rt, to a mixture of 1-(4-(7-azaspiro[3.5]nonan-2-yl)phenyl)dihydropyrimidine-2,4(1H,3H)-dione (200 mg, 0.20 mmol, 2.0 eq) and 4-nitrophenyl 4-(4-(3-carbamoyl-4-(6-(trifluoromethyl)picolinamido)-1H-pyrazol-1-yl)phenyl)piperazine-1-carboxylate (60 mg, 0.10 mmol, 1.0 eq) in DMF (5 mL) were added DIEA (24 mg, 0.19 mmol, 2.0 eq) and DMAP (1 mg, 0.01 mmol, 0.1 eq). The reaction solution was stirred at 60°C under N₂ for 16 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 21.9 mg, yield 28%). LCMS (ESI): m/z 799 [M+H]⁺. **¹H NMR** (400 MHz, DMSO-*d₆*) δ 11.79 (s, 1H), 10.33 (s, 1H), 8.90 (s, 1H), 8.46-8.38 (m, 2H), 8.21 (dd, *J =* 7.6, 1.1 Hz, 1H), 7.96 (s, 1H), 7.82 (d, *J =* 9.1 Hz, 2H), 7.71 (s, 1H), 7.25 (s, 4H), 7.11 (d, *J* = 9.2 Hz, 2H), 3.76 (t, *J =* 6.7 Hz, 2H), 3.58-3.49 (m, 1H), 3.29 (d, *J =* 3.6 Hz, 4H), 3.23 (s, 6H), 3.10 (s, 2H), 2.70 (t, *J =* 6.7 Hz, 2H), 2.27 (t, *J =* 10.2 Hz, 2H), 1.85 (t, *J =* 10.5 Hz, 2H), 1.71 (s, 2H), 1.53 (s, 2H).

### Preparation Example 406. Synthesis of Compound A401

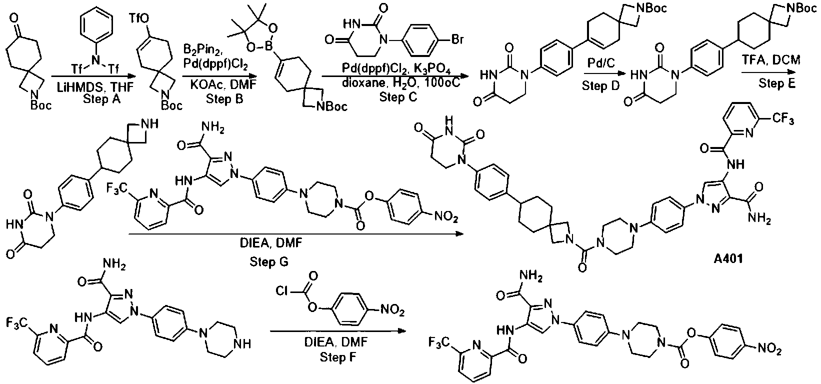

### Step A: Tert-butyl 7-(((trifluoromethyl)sulfonyl)oxy)-2-azaspiro[3.5]non-6-ene-2-carboxylate

At -60°C under N₂, lithium bis(trimethylsilyl)amide (10 mL, 10.03 mmol, 1 M in THF, 1.2 eq) was added to a solution of tert-butyl 7-oxo-2-azaspiro[3.5]nonane-2-carboxylate (2 g, 8.35 mmol, 1.0 eq) in THF (20 mL). The mixture was stirred under these conditions for 30 min, and then 1,1,1-trifluoro-N-phenyl-N-((trifluoromethyl)sulfonyl)methanesulfonamide (3.6 g, 10.03 mmol, 1.2 eq) was added to the reaction mixture. The reaction solution was then stirred at rt under N₂ for 2 h. Upon completion, the reaction was quenched with saturated aqueous NH₄Cl, diluted in EtOAc, washed sequentially with water and saturated aqueous NaCl, dried over anhydrous Na₂SO₄, and concentrated under reduced pressure. The residue was purified by column chromatography to afford the title compound (colorless oil, 2.5 g, yield 80%).

### Step B: Tert-butyl 7-(4,4,5,5-tetramethyl-1,3,2-dioxaborolan-2-yl)-2-azaspiro[3.5]non-6-ene-2-carboxylate

At rt, to a solution of tert-butyl 7-(((trifluoromethyl)sulfonyl)oxy)-2-azaspiro[3.5]non-6-ene-2-carboxylate (2.5 g, 6.73 mmol, 1.0 eq) in DMF (20 mL) were added potassium acetate (1.3 g, 13.46 mmol, 2.0 eq), 4,4,5,5-tetramethyl-2-(4,4,5,5-tetramethyl-1,3,2-dioxaborolan-2-yl)-1,3,2-dioxaborolane (2.1 g, 8.07 mmol, 1.2 eq), and Pd(dppf)Cl₂ (0.5 g, 0.67 mmol, 0.1 eq). The reaction solution was stirred at 70°C under N₂ for 4 h. Upon completion, the reaction was quenched with saturated aqueous NH₄Cl, diluted in EtOAc, washed sequentially with water and saturated aqueous NaCl, dried over anhydrous Na₂SO₄, and concentrated under reduced pressure. The residue was purified by column chromatography to afford the title compound (white solid, 2 g, yield 85%).

### Step C: Tert-butyl 7-(4-(2,4-dioxotetrahydropyrimidin-1(2H)-yl)phenyl)-2-azaspiro[3.5]non-6-ene-2-carboxylate

At rt, to a mixed solution of tert-butyl 7-(4,4,5,5-tetramethyl-1,3,2-dioxaborolan-2-yl)-2-azaspiro[3.5]non-6-ene-2-carboxylate (2 g, 5.72 mmol, 1.0 eq) in dioxane (20 mL) and water (2 mL) were added 1-(4-bromophenyl)dihydropyrimidine-2,4(1H,3H)-dione (1.5 g, 5.72 mmol, 1.0 eq), Pd(dppf)Cl₂ (0.4 g, 0.57 mmol, 0.1 eq), and K3PO4 (2.4 g, 11.45 mmol, 2.0 eq). The reaction mixture was degassed under N₂ (×3), and then stirred under N₂ at 100°C for 4 h. Upon completion, the reaction solution was cooled to rt, diluted in EtOAc, washed sequentially with water and saturated aqueous NaCl, dried over anhydrous Na₂SO₄, and concentrated under reduced pressure. The residue was purified by column chromatography to afford the title compound (white solid, 1.2 g, yield 51%).

### Step D: Tert-butyl 7-(4-(2,4-dioxotetrahydropyrimidin-1(2H)-yl)phenyl)-2-azaspiro[3.5]nonane-2-carboxylate

At rt, 10% Pd/C (0.2 g) was added to a solution of tert-butyl 7-(4-(2,4-dioxotetrahydropyrimidin-1(2H)-yl)phenyl)-2-azaspiro[3.5]non-6-ene-2-carboxylate (1.2 g, 2.91 mmol, 1.0 eq) in THF (20 mL). The mixture was stirred under H₂ at rt for 12 h. Upon completion, the reaction mixture was filtered through Celite, and the filtrate was collected and concentrated under reduced pressure to afford the title compound (white solid, 1 g, crude).

### Step E: 1-(4-(2-Azaspiro[3.5]nonan-7-yl)phenyl)dihydropyrimidine-2,4(1H,3H)-dione

At rt, TFA (2 mL) was added to a solution of tert-butyl 7-(4-(2,4-dioxotetrahydropyrimidin-1(2H)-yl)phenyl)-2-azaspiro[3.5]nonane-2-carboxylate (300 mg, crude) in DCM (6 mL). The reaction solution was stirred at rt for 2 h. Upon completion, the reaction solution was concentrated under reduced pressure to afford the title compound (white solid, 200 mg, crude).

### Step F: 4-Nitrophenyl 4-(4-(3-carbamoyl-4-(6-(trifluoromethyl)picolinamido)-1H-pyrazol-1-yl)phenyl)piperazine-1-carboxylate

At rt, to a solution of N-(3-carbamoyl-1-(4-(piperazin-1-yl)phenyl)-1H-pyrazol-4-yl)-6-(trifluoromethyl)picolinamide (300 mg, 0.65 mmol, 1.0 eq) in DMF (5 mL) were added DIEA (253 mg, 1.95 mmol, 3.0 eq) and 4-nitrophenyl chloroformate (393 mg, 1.95 mmol, 3.0 eq). The mixture was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 350 mg, yield 86%).

### Step G: N-(3-Carbamoyl-1-(4-(4-(7-(4-(2,4-dioxotetrahydropyrimidin-1(2H)-yl)phenyl)-2-azaspiro[3.5]nonane-2-carbonyl)piperazin-1-yl)phenyl)-1H-pyrazol-4-yl)-6-(trifluoromethyl)picolinamide

At rt, to a solution of 1-(4-(2-azaspiro[3.5]nonan-7-yl)phenyl)dihydropyrimidine-2,4(1H,3H)-dione (180 mg, crude) and 4-nitrophenyl 4-(4-(3-carbamoyl-4-(6-(trifluoromethyl)picolinamido)-1H-pyrazol-1-yl)phenyl)piperazine-1-carboxylate (359 mg, 0.57 mmol, 1.0 eq) in DMF (5 mL) was added DIEA (222 mg, 1.72 mmol, 3.0 eq). Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 72.6 mg, yield 16%). **¹H NMR** (400 MHz, DMSO-d6) δ 11.79 (s, 1H), 10.33 (s, 1H), 8.90 (s, 1H), 8.43 (d, *J=* 12.5 Hz, 2H), 8.22 (d, *J =* 6.6 Hz, 1H), 7.96 (s, 1H), 7.86-7.68 (m, 3H), 7.24 (s, 4H), 7.10 (d, *J =* 7.7 Hz, 2H), 3.76 (s, 4H), 3.62 (s, 2H), 3.42 (s, 2H), 3.21 (s, 4H), 2.89 (s, 1H), 2.71 (d, *J =* 16.0 Hz, 3H), 1.94 (d, *J =* 10.2 Hz, 2H), 1.71 (s, 2H), 1.50 (dd, *J=* 36.8, 12.5 Hz, 4H), 1.23 (s, 1H). LCMS (ESI): m/z 799 [M+H]⁺.

### Preparation Example 407. Synthesis of Compound A402

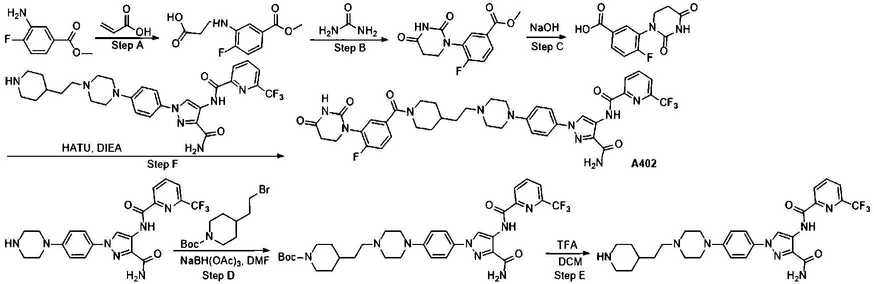

### Step A: 3-((2-Fluoro-5-(methoxycarbonyl)phenyl)amino)propanoic acid

At rt, methyl 3-amino-4-fluorobenzoate (5 g, 29.5 mmol, 1.0 eq) and acrylic acid (2.13 g, 29.5 mmol, 1.0 eq) were added to water (20 mL) and AcOH (4 mL). The mixture was stirred at 120°C under N₂ for 16 h. Upon completion, the mixture was diluted in EtOAc, washed sequentially with water and saturated aqueous NaCl, dried over anhydrous Na₂SO₄, and concentrated under reduced pressure. The residue was purified by column chromatography to afford the title compound (yellow solid, 2.5 g, yield 35%).

### Step B: Methyl 3-(2,4-dioxotetrahydropyrimidin-1(2H)-yl)-4-fluorobenzoate

At rt, 3-((2-fluoro-5-(methoxycarbonyl)phenyl)amino)propanoic acid (2 g, 8.3 mmol, 1.0 eq) and urea (4.02 g, 16.6 mmol, 2.0 eq) were added to AcOH (20 mL). The reaction solution was stirred at 120°C under N₂ for 16 h. Upon completion, the reaction solution was concentrated under reduced pressure and the residue was purified by HPLC to afford the title compound (yellow solid, 600 mg, yield 27%).

### Step C: 3-(2,4-Dioxotetrahydropyrimidin-1(2H)-yl)-4-fluorobenzoic acid

At rt, to a solution of methyl 3-(2,4-dioxotetrahydropyrimidin-1(2H)-yl)-4-fluorobenzoate (600 mg, 2.25 mmol, 1.0 eq) in MeOH (20 mL) and THF (20 mL) was added a mixture of NaOH (450 mg, 11.3 mmol, 5.0 eq) in water (4 mL). The reaction solution was stirred at rt under N₂ for 1 h. The resulting mixture was concentrated in vacuo. The residue was treated with 1N HCl and extracted with EtOAc. The organic phase was dried over anhydrous Na₂SO₄ and filtered. The filtrate was concentrated under reduced pressure. The residue was treated with an aqueous HCl (20 mL, 4N), and the resulting mixture was stirred at 100°C for 1 h. Upon completion, the mixture was cooled to rt, diluted in ice water (20 mL), and the precipitated solid was collected by filtration. The mixture was washed with diethyl ether and concentrated to afford the title compound (yellow solid, 150 mg, yield 26%).

### Step D: Tert-butyl 4-(2-(4-(4-(3-carbamoyl-4-(6-(trifluoromethyl)pyridinylamino)-1H-pyrazol-1-yl)phenyl)piperazin-1-yl)ethyl)piperidine-1-carboxylate

At rt, to a solution of N-(3-carbamoyl-1-(4-(piperazin-1-yl)phenyl)-1H-pyrazol-4-yl)-6-(trifluoromethyl)picolinamide (500 mg, 1.1 mmol, 1.0 eq) in DMF (10 mL) were added tert-butyl 4-(2-bromoethyl)piperidine-1-carboxylate (318 mg, 1.1 mmol, 1 eq) and AcOH (6 mg, 0.11 mmol, 0.1 eq). The reaction solution was stirred at rt for 30 min, and then sodium triacetoxyborohydride (344 mg, 1.7 mmol, 1.5 eq) was added. The mixture was stirred at rt for 12 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 400 mg, yield 55%).

### Step E: N-(3-Carbamoyl-1-(4-(4-(2-(piperidin-4-yl)ethyl)piperazin-1-yl)phenyl)-1H-pyrazol-4-yl)-6-(trifluoromethyl)picolinamide

At rt, TFA (2 mL) was added to a solution of tert-butyl 4-(2-(4-(4-(3-carbamoyl-4-(6-(trifluoromethyl)pyridinylamino)-1H-pyrazol-1-yl)phenyl)piperazin-1-yl)ethyl)piperidine-1-carboxylate (400 mg, 0.6 mmol, 1.0 eq) in DCM (10 mL). The mixture was stirred at rt for 1 h. Upon completion, the mixture was concentrated to afford the title compound (yellow oil, 320 mg, crude).

### Step F: N-(3-Carbamoyl-1-(4-(4-(2-(1-(3-(2,4-dioxotetrahydropyrimidin-1(2H)-yl)-4-fluorobenzoyl)piperidin-4-yl)ethyl)piperazin-1-yl)phenyl)-1H-pyrazol-4-yl)-6-(trifluoromethyl)picolinamide trifluoroacetate

At rt, to a solution of 3-(2,4-dioxotetrahydropyrimidin-1(2H)-yl)-4-fluorobenzoic acid (150 mg, 0.6 mmol, 1.0 eq) in DMF (4 mL) were added N-(3-carbamoyl-1-(4-(4-(2-(piperidin-4-yl)ethyl)piperazin-1-yl)phenyl)-1H-pyrazol-4-yl)-6-(trifluoromethyl)picolinamide (320 mg, crude), HATU (452 mg, 1.2 mmol, 2.0 eq), and DIEA (154 mg, 1.2 mmol, 2.0 eq). Upon completion, the mixture was diluted in EtOAc, washed sequentially with water and saturated aqueous NaCl, dried over anhydrous Na₂SO₄, and concentrated under reduced pressure. The residue was purified by HPLC to afford the title compound (white solid, 35 mg, yield 7%). **¹H NMR** (400 MHz, DMSO-d6) δ 11.80 (s, 1H), 10.54 (s, 1H), 9.56 (s, 1H), 8.93 (s, 1H), 8.42 (dt, *J* = 15.4, 7.6 Hz, 2H), 8.22 *(d, J=* 7.5 Hz, 1H), 7.95 (s, 1H), 7.87 *(d, J=* 9.0 Hz, 2H), 7.75 (s, 1H), 7.51 (d, *J* = 7.5 Hz, 1H), 7.40 (d, *J =* 8.1 Hz, 2H), 7.18 (d, *J =* 9.2 Hz, 2H), 4.45 (s, 1H), 3.96 (d, *J =* 12.6 Hz, 3H), 3.84 (s, 1H), 3.76 (t, *J=* 6.6 Hz, 3H), 3.62 (d, *J =* 10.9 Hz, 3H), 3.11 (dd, *J=* 41.4, 11.4 Hz, 5H), 2.74 (t, *J* = 6.6 Hz, 2H), 1.76 (s, 1H), 1.67 (s, 4H), 1.18 (d, *J =* 13.3 Hz, 2H). LCMS (ESI): m/z 805 [M+H]⁺.

### Preparation Example 408. Synthesis of Compound A403

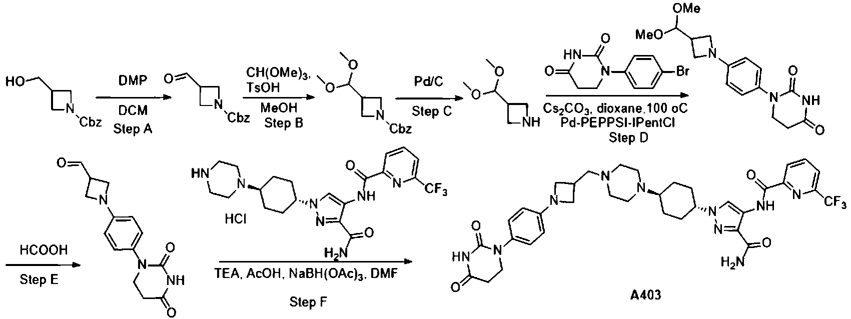

### Step A: Benzyl 3-formylazetidine-1-carboxylate

At 0°C, Dess-Martin periodinane (5.8 g, 13.56 mmol, 1.5 eq) was added portionwise to a solution of benzyl 3-(hydroxymethyl)azetidine-1-carboxylate (2 g, 9.04 mmol, 1.0 eq) in DCM (30 mL). The mixture was stirred at rt under N₂ for 2 h. Upon completion, the reaction mixture was quenched with water and extracted with DCM. The combined organic layers were washed with saturated aqueous NaCl, dried over anhydrous Na₂SO₄, filtered, and concentrated under reduced pressure. The residue was purified by column chromatography to afford the title compound (colorless oil, 1.65 g, yield 83%).

### Step B: Benzyl 3-(dimethoxymethyl)azetidine-1-carboxylate

At 0°C under N₂, to a solution of benzyl 3-formylazetidine-1-carboxylate (1.65 g, 7.53 mmol, 1.0 eq) in MeOH (20 mL) were added trimethoxymethane (4.1 mL, 37.63 mmol, 5.0 eq) and p-toluenesulfonic acid (130 mg, 0.75 mmol, 0.1 eq). The mixture was stirred at rt under N₂ for 4 h. Upon completion, the reaction mixture was quenched with water and extracted with DCM. The combined organic layers were washed with saturated aqueous NaCl, dried over anhydrous Na₂SO₄, filtered, and concentrated under reduced pressure. The residue was purified by column chromatography to afford the title compound (colorless oil, 1.6 g, yield 80%).

### Step C: 3-(Dimethoxymethyl)azetidine

At rt, 10% Pd/C (0.6 g) was added to a solution of benzyl 3-(dimethoxymethyl)azetidine-1-carboxylate (1.6 g, 6.03 mmol, 1.0 eq) in THF (20 mL). The mixture was stirred under H₂ at rt for 2 h. Upon completion, the reaction mixture was filtered through Celite, and the filtrate was concentrated under reduced pressure to afford the title compound (yellow oil, 700 mg, crude), which was used directly in the next step without further purification.

### Step D: 1-(4-(3-(Dimethoxymethyl)azetidin-1-yl)phenyl)dihydropyrimidine-2,4(1H,3H)-dione

At rt, to a solution of 1-(4-bromophenyl)dihydropyrimidine-2,4(1H,3H)-dione (130 mg, 0.48 mmol, 1.0 eq) in dioxane (4 mL) were added 3-(dimethoxymethyl)azetidine (95 mg, crude), Cs2CO3 (315 mg, 0.97 mmol, 2.0 eq), and Pd-PEPPSI-IpentCl (47 mg, 0.05 mmol, 0.1 eq). The reaction mixture was degassed under N₂ (×3), and then stirred under N₂ at 100°C for 18 h. Upon completion, the reaction solution was cooled to rt, and the reaction mixture was poured into saturated aqueous NH₄Cl and extracted with EtOAc. The organic layer was washed sequentially with water and saturated aqueous NaCl, dried over anhydrous Na₂SO₄, and concentrated under reduced pressure. The residue was purified by column chromatography to afford the title compound (yellow solid, 90 mg, yield 58%).

### Step E: 1-(4-(2,4-Dioxotetrahydropyrimidin-1(2H)-yl)phenyl)azetidine-3-carbaldehyde

1-(4-(3-(dimethoxymethyl)azetidin-1-yl)phenyl)dihydropyrimidine-2,4(1H,3H)-dione (90 mg, 0.28 mmol, 1.0 eq) was added to formic acid (4 mL), and the reaction solution was stirred at 60°C for 1 h. The mixture was concentrated under reduced pressure to afford the title compound (yellow oil, 70 mg, crude), which was used directly in the next step without further purification.

### Step F: N-(3-Carbamoyl-1-((1r,4r)-4-(4-((1-(4-(2,4-dioxotetrahydropyrimidin-1(2H)-yl)phenyl)azetidin-3-yl)methyl)piperazin-1-yl)cyclohexyl)-1H-pyrazol-4-yl)-6-(trifluoromethyl)picolinamide

At rt, to a solution of 1-(4-(2,4-dioxotetrahydropyrimidin-1(2H)-yl)phenyl)azetidine-3-carbaldehyde (70 mg, crude) in DMF (5 mL) were added N-(3-carbamoyl-1-((1r,4r)-4-(piperazin-1-yl)cyclohexyl)-1H-pyrazol-4-yl)-6-(trifluoromethyl)picolinamide hydrochloride (119 mg, 0.26 mmol, 1.0 eq) and TEA (0.1 mL). The mixture was stirred at rt for 15 min. Then AcOH (0.4 mL) was added, and the mixture was stirred at rt overnight. NaBH(OAc)3 (29 mg, 0.77 mmol, 3.0 eq) was then added, and the resulting mixture was stirred at rt for 2 h. Upon completion, the reaction was quenched with saturated aqueous NH₄Cl and extracted with a mixed solvent of ethyl acetate and THF (1:1). The organic layer was dried over anhydrous Na₂SO₄ and concentrated under reduced pressure. The residue was purified by HPLC to afford the title compound (yellow solid, 27.0 mg, yield 14%). **¹H NMR** (400 MHz, MeOD) δ 8.46-8.42 (m, 2H), 8.40 (s, 1H), 8.28 (t, *J* = 7.8 Hz, 1H), 8.03 (d, *J=* 7.8 Hz, 1H), 7.14 (d, *J =* 8.8 Hz, 2H), 6.50 (d, *J* = 8.8 Hz, 2H), 4.84-4.82 (m, 1H), 4.35-4.26 (m, 1H), 4.02 (t, *J =* 7.4 Hz, 2H), 3.77 (t, *J=* 6.8 Hz, 2H), 3.59-3.53 (m, 2H), 3.33-3.32 (m, 1H), 3.11-2.90 (m, 6H), 2.85 (d, *J* = 7.2 Hz, 2H), 2.79 (t, *J=* 6.8 Hz, 4H), 2.34-2.21 (m, 4H), 2.05-1.94 (m, 2H), 1.71-1.61 (m, 2H). LCMS (ESI): m/z 723 [M+H]⁺.

### Preparation Example 409. Synthesis of Compound A404

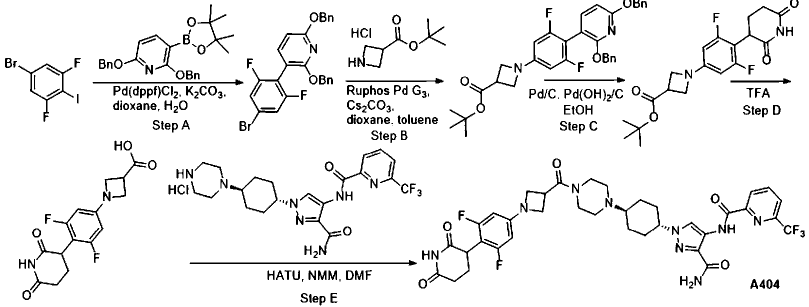

### Step A: 2,6-bis(benzyloxy)-3-(4-bromo-2,6-difluorophenyl)pyridine

To a solution of 2,6-bis(benzyloxy)-3-(4,4,5,5-tetramethyl-1,3,2-dioxaborolan-2-yl)pyridine (5 g, 11.98 mmol, 1.0 eq) and 5-bromo-1,3-difluoro-2-iodobenzene (4.6 g, 14.38 mmol, 1.2 eq) in 1,4-dioxane (50 mL) and water (10 mL) at rt were added potassium carbonate (3.3 g, 23.96 mmol, 2.0 eq) and Pd(dppf)Cl₂ (880 mg, 1.20 mmol, 0.1 eq). The reaction solution was then degassed (×3) under N₂ and heated to reflux with stirring for 5 h. Upon completion, the reaction solution was cooled to rt, diluted in EtOAc, washed sequentially with water and saturated aqueous NaCl, dried over anhydrous Na₂SO₄, and concentrated under reduced pressure. The residue was purified by column chromatography to afford the title compound (white solid, 2.749 g, yield 48%).

### Step B: Tert-butyl 1-(4-(2,6-bis(benzyloxy)pyridin-3-yl)-3,5-difluorophenyl)azetidine-3-carboxylate

To a solution of 2,6-bis(benzyloxy)-3-(4-bromo-2,6-difluorophenyl)pyridine (220 mg, 0.46 mmol, 1.0 eq) and tert-butyl azetidine-3-carboxylate hydrochloride (72 mg, 0.46 mmol, 1.0 eq) in 1,4-dioxane (5 mL) and toluene (3 mL) were added Cs2CO3 (300 mg, 0.91 mmol, 2.0 eq) and Ruphos Pd G3 (38 mg, 0.05 mmol, 0.1 eq) at rt. The reaction solution was degassed (×3) under N₂ and then heated to 100°C and stirred for 5 h. Upon completion, the reaction solution was cooled to rt, diluted in EtOAc, washed sequentially with water and saturated aqueous NaCl, dried over anhydrous Na₂SO₄, and concentrated under reduced pressure. The residue was purified by column chromatography to afford the title compound (white solid, 170 mg, yield 67%).

### Step C: Tert-butyl 1-(4-(2,6-dioxopiperidin-3-yl)-3,5-difluorophenyl)azetidine-3-carboxylate

To a solution of tert-butyl 1-(4-(2,6-bis(benzyloxy)pyridin-3-yl)-3,5-difluorophenyl)azetidine-3-carboxylate (170 mg, 0.304 mmol, 1.0 eq) in ethanol (5 mL) was added 10% Pd/C (50 mg) and 10% Pd(OH)2/C (50 mg) at rt. The reaction mixture was degassed (×3) under H₂ and stirred at rt for 12 h. Upon completion, the reaction solution was filtered through Celite, and the filtrate was concentrated under reduced pressure to afford the title compound (yellow solid, 83 mg, crude), which was used directly without further purification.

### Step D: 1-(4-(2,6-dioxopiperidin-3-yl)-3,5-difluorophenyl)azetidine-3-carboxylic acid

To a solution of tert-butyl 1-(4-(2,6-dioxopiperidin-3-yl)-3,5-difluorophenyl)azetidine-3-carboxylate (83 mg, crude) in DCM (1 mL) was added TFA (0.5 mL) at rt. The mixture was stirred at rt for 2 h. Upon completion, the reaction solution was concentrated under reduced pressure. The residue was diluted in EtOAc and washed sequentially with saturated NaHCO₃, water, and saturated aqueous NaCl, dried over anhydrous Na₂SO₄, and concentrated under reduced pressure to afford the title compound (colorless oil, 65 mg, crude), which was used directly without further purification.

### Step E: N-(3-Carbamoyl-1-((1r,4r)-4-(4-(1-(4-(2,6-dioxopiperidin-3-yl)-3,5-difluorophenyl)azetidine-3-carbonyl)piperazin-1-yl)cyclohexyl)-1H-pyrazol-4-yl)-6-(trifluoromethyl)picolinamide

To a solution of 1-(4-(2,6-dioxopiperidin-3-yl)-3,5-difluorophenyl)azetidine-3-carboxylic acid (65 mg, crude) in DMF (1 mL) were added N-(3-carbamoyl-1-((1r,4r)-4-(piperazin-1-yl)cyclohexyl)-1H-pyrazol-4-yl)-6-(trifluoromethyl)picolinamide hydrochloride (121 mg, 0.26 mmol, 1.3 eq), NMM (81 mg, 0.80 mmol, 4.0 eq), and HATU (114 mg, 0.30 mmol, 1.5 eq) at rt. The mixture was stirred at rt for 2 h. Upon completion, the mixture was diluted in EtOAc, washed sequentially with water and saturated aqueous NaCl, dried over anhydrous Na₂SO₄, and concentrated under reduced pressure. The residue was purified by HPLC to afford the title compound (white solid, 25 mg, 16% yield over three steps). **¹H NMR** (400 MHz, DMSO-*d6)* δ 11.71 (s, 1H), 10.86 (s, 1H), 8.46-8.33 (m, 3H), 8.18 (d, *J =* 7.4 Hz, 1H), 7.65 (s, 1H), 7.54 (s, 1H), 6.16 (d, *J=* 11.2 Hz, 2H), 4.23 (t, *J* = 7.2 Hz, 1H), 4.10-3.98 (m, 4H), 3.91 (d, *J* = 6.5 Hz, 2H), 3.83 (d, *J =* 7.7 Hz, 1H), 3.46 (s, 4H), 3.30 (s, 2H), 2.81-2.74 (m, 1H), 2.44-2.41 (m, 2H), 2.18-2.01 (m, 4H), 1.98-1.79 (m, 5H), 1.48-1.40 (m, 2H). LCMS (ESI): m/z 772 [M+H]⁺.

### Preparation Example 410. Synthesis of Compound A405

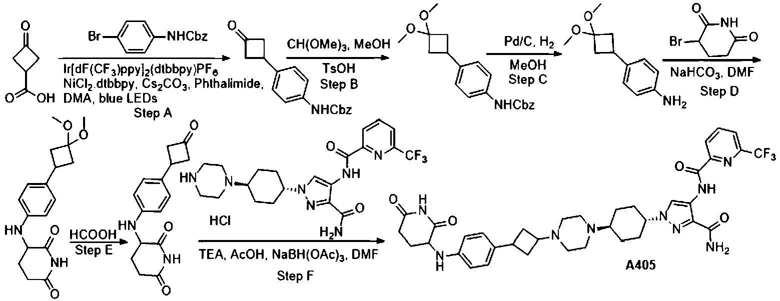

### Step A: Benzyl (4-(3-oxocyclobutyl)phenyl)carbamate

To a solution of benzyl (4-bromophenyl)carbamate (700 mg, 2.3 mmol, 1.0 eq) and 3-oxocyclobutane-1-carboxylic acid (521.8 mg, 4.6 mmol, 2.0 eq) in N,N-dimethylacetamide (18.0 mL) at rt were added NiCl2·dtbbpy (91.00 mg, 0.23 mmol, 0.1 eq), cesium carbonate (1.1 g, 3.4 mmol, 1.5 eq), phthalimide (403.7 mg, 2.7 mmol, 1.15 eq), and [Ir(dtbbpy)[dF(CF3)ppy]2]PF6 (256.5 mg, 0.23 mmol, 0.1 eq). The mixture was stirred at rt for 18 h under 450 nm LED illumination. Upon completion, the reaction solution was diluted in EtOAc and washed with aqueous NaCl. The aqueous layer was extracted with EtOAc. The combined organic phases were dried and concentrated under reduced pressure. The residue was purified by column chromatography to afford the title compound (yellow solid, 500 mg, yield 74%).

### Step B: Benzyl (4-(3,3-dimethoxycyclobutyl)phenyl)carbamate

To a solution of benzyl (4-(3-oxocyclobutyl)phenyl)carbamate (600 mg, 2.0 mmol, 1.0 eq) in MeOH (10.0 mL) at rt were added trimethyl orthoformate (0.4 mL, 4.0 mmol, 2.0 eq) and p-toluenesulfonic acid (38.6 mg, 0.2 mmol, 0.1 eq). The mixture was stirred at rt for 2 h. Upon completion, the reaction solution was diluted in water and extracted with EtOAc. The organic phase was dried over anhydrous Na₂SO₄, filtered, and concentrated under reduced pressure. The residue was purified by column chromatography to afford the title compound (yellow solid, 300 mg, yield 43%).

### Step C: 4-(3,3-dimethoxycyclobutyl)aniline

To a solution of benzyl (4-(3,3-dimethoxycyclobutyl)phenyl)carbamate (350 mg, 1.0 mmol, 1.0 eq) in MeOH (5.0 mL) was added 10% palladium on carbon (50 mg) at rt. The mixture was stirred at rt under H₂ for 2 h. Upon completion, the reaction solution was filtered, and the filtrate was concentrated under reduced pressure to afford the title compound (yellow oil, 150 mg, crude) without further purification.

### Step D: 3-(4-(3,3-dimethoxycyclobutylphenyl)amino)piperidine-2,6-dione

To a solution of 4-(3,3-dimethoxycyclobutyl)aniline (212 mg, 1.0 mmol, 1.0 eq) in DMF (5.0 mL) at rt were added NaHCO₃ (171.9 mg, 2.0 mmol, 1.0 eq) and 3-bromopiperidine-2,6-dione (392.8 mg, 2.0 mmol, 1.0 eq). The mixture was stirred at 80°C under N₂ for 16 h. Upon completion, the mixture was cooled to rt, diluted in water, and extracted with EtOAc. The organic phase was washed with aqueous NaCl, dried over anhydrous Na₂SO₄, filtered, and concentrated under reduced pressure. The residue was purified by column chromatography to afford the title compound (yellow solid, 160 mg, yield 49%).

### Step E: 3-(4-(3-Oxycyclobutylphenyl)amino)piperidine-2,6-dione

A solution of 3-(4-(3,3-dimethoxycyclobutylphenyl)amino)piperidine-2,6-dione (80 mg, 0.3 mmol, 1.0 eq) in formic acid (4.0 mL) was stirred at 60°C under N₂ for 1 h. Upon completion, the mixture was cooled to rt. The reaction solution was concentrated under reduced pressure to afford the title compound (yellow oil, 50 mg, yield 73%).

### Step F: N-(3-carbamoyl-1-(1r,4r)-4-(4-(3-(4-((2,6-dioxopiperidin-3-yl)amino)phenyl)cyclobutyl)piperazin-1-yl)cyclohexyl)-1H-pyrazol-4-yl)-6-(trifluoromethyl)picolinamide

To a solution of 3-(4-(3-oxocyclobutylphenyl)amino)piperidine-2,6-dione (29.3 mg, 0.11 mmol, 1.0 eq) and N-(3-carbamoyl-1-((1r,4r)-4-(piperazin-1-yl)cyclohexyl)-1H-pyrazol-4-yl)-6-(trifluoromethyl)picolinamide hydrochloride (50 mg, 0.11 mmol, 1.0 eq) in DMF (5.0 mL) was added TEA (0.1 mL) at rt. The mixture was stirred for 15 min. AcOH (0.44 mL) was then added, and the mixture was stirred at rt overnight. Sodium triacetoxyborohydride (68 mg, 0.32 mmol, 3.0 eq) was added, and the mixture was stirred for an additional 2 h. Upon completion, the reaction mixture was diluted in saturated aqueous NH₄Cl and extracted with EtOAc. The organic phase was dried, filtered, and concentrated. The residue was extracted with a mixed solvent of ethyl acetate and tetrahydrofuran. The organic phase was dried, filtered, and concentrated under reduced pressure. The residue was purified by preparative chromatography to afford the title compound (gray solid, 32.5 mg, yield 42%). **¹H NMR** (400 MHz, DMSO-d6) δ 11.72 (s, 1H), 10.77 (s, 1H), 8.46 (s, 1H), 8.43-8.35 (m, 2H), 8.23-8.15 (m, 1H), 7.59 (d, *J =* 8.0 Hz, 2H), 7.02 (d, *J =* 8.4 Hz, 2H), 6.64 (d, *J =* 8.4 Hz, 2H), 4.36-4.25 (m, 2H), 3.41-3.28 (m, 6H), 3.09-2.86 (m, 4H), 2.82-2.70 (m, 2H), 2.61-2.54 (m, 2H), 2.30-1.98 (m, 8H), 1.94-1.80 (m, 3H), 1.66-1.49 (m, 2H). LCMS (ESI): m/z 722 [M+H]⁺.

### Preparation Example 411. Synthesis of Compound A406

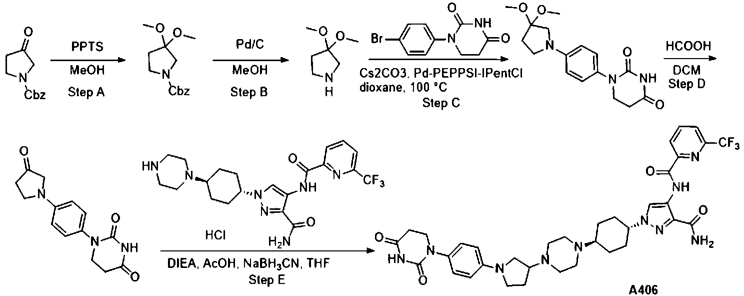

### Step A: Benzyl 3,3-dimethoxypyrrolidine-1-carboxylate

To a solution of benzyl 3-oxopyrrolidine-1-carboxylate (1.5 g, 6.84 mmol, 1.0 eq) in MeOH (20 mL) was added portionwise PPTS (170 mg, 0.68 mmol, 0.1 eq) at 0°C under N₂. The mixture was stirred at 50°C overnight under N₂. Upon completion, the reaction solution was cooled to rt. The reaction mixture was diluted in DCM, washed sequentially with saturated aqueous NaHCO₃ and aqueous NaCl, dried over anhydrous Na₂SO₄, and concentrated under reduced pressure. The residue was purified by column chromatography to afford the title compound (colorless oil, 1.8 g, yield 99%).

### Step B: 3,3-Dimethoxypyrrolidine

To a solution of benzyl 3,3-dimethoxypyrrolidine-1-carboxylate (1.8 g, 6.79 mmol, 1.0 eq) in THF (30 mL) was added 10% Pd/C (300 mg) at rt. The mixture was degassed (×3) under H₂ and then stirred at rt for 2 h. Upon completion, the reaction solution was filtered through Celite, and the filtrate was concentrated under reduced pressure to afford the title compound (colorless oil, 800 mg, crude), which was used directly in the next step without further purification.

### Step C: 1-(4-(3,3-Dimethoxypyrrolidin-1-yl)phenyl)dihydropyrimidine-2,4(1H,3H)-dione

At rt, to a solution of 3,3-dimethoxypyrrolidine (200 mg, crude) in dioxane (4 mL) were added 1-(4-bromophenyl)dihydropyrimidine-2,4(1H,3H)-dione (200 mg, 0.75 mmol, 1.0 eq), Cs2CO3 (484 mg, 1.49 mmol, 2.0 eq) and Pd-PEPPSI-IpentCl (72 mg, 0.07 mmol, 0.1 eq). The reaction solution was degassed (×3) under N₂, then heated to 100°C and stirred overnight. Upon completion, the reaction solution was cooled to rt. The reaction mixture was poured into saturated aqueous NH₄Cl and extracted with EtOAc. The organic layer was washed sequentially with saturated aqueous NaCl and water, and dried over anhydrous Na₂SO₄. The residue was purified by column chromatography to afford the title compound (brown solid, 140 mg, yield 59%).

### Step D: 1-(4-(3-Oxopyrrolidin-1-yl)phenyl)dihydropyrimidine-2,4(1H,3H)-dione

To a solution of 1-(4-(3,3-dimethoxypyrrolidin-1-yl)phenyl)dihydropyrimidine-2,4(1H,3H) -dione (80 mg, 0.25 mmol, 1.0 eq) in DCM (2 mL) was added formic acid (2 mL) at rt. The resulting mixture was stirred at rt for 30 min. Upon completion, the reaction solution was concentrated under reduced pressure to afford the title compound (brown solid, 140 mg, crude), which was used directly in the next step without further purification.

### Step E: N-(3-Carbamoyl-1-((1r,4r)-4-(4-(1-(4-(2,4-dioxotetrahydropyrimidin-1(2H)-yl)phenyl)pyrrolidin-3-yl)piperazin-1-yl)cyclohexyl)-1H-pyrazol-4-yl)-6-(trifluoromethyl)picolinamide

To a solution of 1-(4-(3-oxopyrrolidin-1-yl)phenyl)dihydropyrimidine-2,4(1H,3H)-dione (80 mg, crude) in THF (10 mL) were added N-(3-carbamoyl-1-((1r,4r)-4-(piperazin-1-yl)cyclohexyl)-1H-pyrazol-4-yl)-6-(trifluoromethyl)picolinamide hydrochloride (136 mg, 0.29 mmol, 1.0 eq) and DIEA (0.2 mL) at rt. The mixture was stirred at rt under N₂ for 5 min. AcOH (0.5 mL) and NaBH3CN (74 mg, 1.17 mmol, 4.0 eq) were then added, and the mixture was stirred overnight. Upon completion, the reaction solution was quenched with water, extracted with EtOAc, dried over anhydrous Na₂SO₄, and concentrated under reduced pressure. The residue was purified by HPLC to afford the title compound (white solid, 24.5 mg, yield 12%). **¹H NMR** (400 MHz, DMSO-*d₆*) δ 11.72 (s, 1H), 10.22 (s, 1H), 9.35 (s, 0.5H), 8.47 (s, 1H), 8.44-8.35 (m, 2H), 8.20 (dd, *J* = 7.3, 1.3 Hz, 1H), 7.61 (s, 1H), 7.58 (s, 1H), 7.12 (d, *J =* 8.6 Hz, 2H), 6.57 (d, *J =* 8.6 Hz, 2H), 4.39-4.29 (m, 1H), 3.68 (t, *J=* 6.7 Hz, 2H), 3.45-3.36 (m, 6H), 3.29-3.21 (m, 3H), 3.20-3.02 (m, 5H), 2.68 (t, *J =* 6.7 Hz, 2H), 2.31-2.16 (m, 5H), 1.98-1.85 (m, 3H), 1.73-1.58 (m, 2H). LCMS (ESI): m/z 723 [M+H]⁺.

### Preparation Example 412. Synthesis of Compound A407

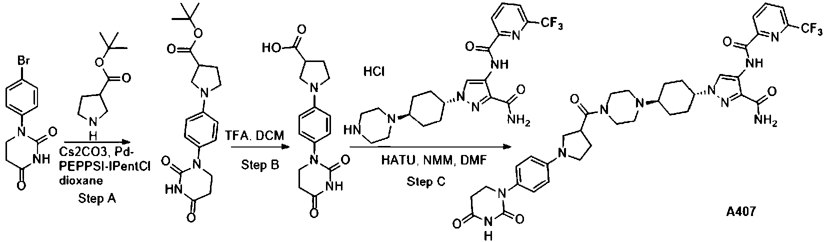

### Step A: Tert-butyl 1-(4-(2,4-dioxotetrahydropyrimidin-1(2H)-yl)phenyl)pyrrolidine-3-carboxylate

1-(4-bromophenyl)dihydropyrimidine-2,4(1H,3H)-dione (250 mg, 0.93 mmol, 1.0 eq), tert-butyl pyrrolidine-3-carboxylate (239 mg, 1.40 mmol, 1.5 eq), Cs2CO3 (605 mg, 1.86 mmol, 2.0 eq), and Pd-PEPPSI-IpentCl (90 mg, 0.09 mmol, 0.1 eq) were added to dioxane (6 mL) solution, and the mixture was degassed (×3) under N₂ and then stirred at 100°C under N₂ overnight. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 223 mg, yield 67%).

### Step B: 1-(4-(2,4-Dioxotetrahydropyrimidin-1(2H)-yl)phenyl)pyrrolidine-3-carboxylic acid

At rt, TFA (2 mL) was added to a solution of tert-butyl 1-(4-(2,4-dioxotetrahydropyrimidin-1(2H)-yl)phenyl)pyrrolidine-3-carboxylate (60 mg, 0.17 mmol, 1.0 eq) in DCM (1 mL). The mixture was stirred at rt for 1 h. The reaction mixture was concentrated under reduced pressure to afford 1-(4-(2,4-dioxotetrahydropyrimidin-1(2H)-yl)phenyl)pyrrolidine-3-carboxylic acid (yellow oil, 52 mg, crude). The crude was used directly in the next step without further purification.

### Step C: N-(3-Carbamoyl-1-((1r,4r)-4-(4-(1-(4-(2,4-dioxotetrahydropyrimidin-1(2H)-yl)phenyl)pyrrolidine-3-carbonyl)piperazin-1-yl)cyclohexyl)-1H-pyrazol-4-yl)-6-(trifluoromethyl)picolinamide

At rt, to a solution of 1-(4-(2,4-dioxotetrahydropyrimidin-1(2H)-yl)phenyl)pyrrolidine-3-carboxylic acid (50 mg, crude) and N-(3-carbamoyl-1-((1r,4r)-4-(piperazin-1-yl)cyclohexyl)-1H-pyrazol-4-yl)-6-(trifluoromethyl)picolinamide hydrochloride (77 mg, 0.17 mmol, 1.0 eq) in DMF (5 mL) were added NMM (0.04 mL, 0.33 mmol, 2.0 eq) and HATU (94 mg, 0.25 mmol, 1.5 eq). The mixture was stirred at 25°C for 1 h. Upon completion, the reaction solution was diluted in DCM, washed sequentially with water and saturated aqueous NaCl, dried over anhydrous Na₂SO₄, and concentrated under reduced pressure. The residue was purified by HPLC to afford the title compound (white solid, 27.6 mg, yield 21%). **¹H NMR** (400 MHz, DMSO-d6) δ 11.71 (s, 1H), 10.21 (s, 1H), 8.44 (s, 1H), 8.38 (dd, *J=* 14.6, 7.0 Hz, 2H), 8.19 (dd, *J=* 7.5, 1.2 Hz, 1H), 7.64 (s, 1H), 7.55 (s, 1H), 7.10 (d, *J= 9.0* Hz, 2H), 6.53 (d, *J= 9.0* Hz, 2H), 4.25 (t, *J* = 11.6 Hz, 1H), 3.67 (t, *J =* 6.7 Hz, 2H), 3.60-3.41 (m, 6H), 3.35 (d, *J =* 3.9 Hz, 1H), 3.31-3.24 (m, 4H), 2.68 (t, *J=* 6.7 Hz, 2H), 2.56-2. 50 (m, 2H), 2.46-2.42 (m, 1H), 2.21-2.06 (m, 4H), 1.98-1.80 (m, 4H), 1.53-1.39 (m, 2H). LCMS (ESI): m/z 751 [M+H]⁺.

### Preparation Example 413. Synthesis of Compound A408

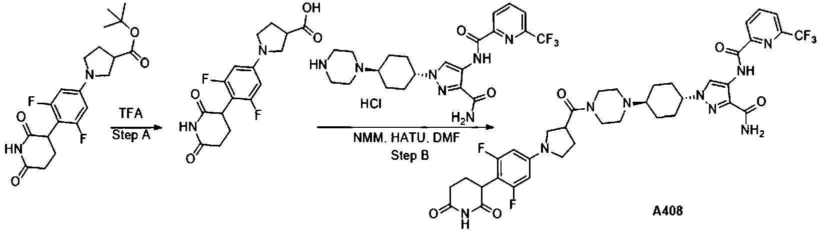

### Step A: 1-(4-(2,6-Dioxopiperidin-3-yl)-3,5-difluorophenyl)pyrrolidine-3-carboxylic acid

At rt, TFA (1.0 mL) was added to a solution of tert-butyl 1-(4-(2,6-dioxopiperidin-3-yl)-3,5-difluorophenyl)pyrrolidine-3-carboxylate (90 mg, 0.23 mmol, 1.0 eq) in DCM (5.0 mL). The mixture was stirred at rt for 2 h. Upon completion, the reaction solution was concentrated under reduced pressure to afford the title compound (yellow solid, 80 mg, crude).

### Step B: N-(3-Carbamoyl-1-((1r,4r)-4-(4-(1-(4-(2,6-dioxopiperidin-3-yl)-3,5-difluorophenyl)pyrrolidine-3-carbonyl)piperazin-1-yl)cyclohexyl)-1H-pyrazol-4-yl)-6-(trifluoromethyl)picolinamide

At rt, to a solution of 1-(4-(2,6-dioxopiperidin-3-yl)-3,5-difluorophenyl)pyrrolidine-3-carboxylic acid (80 mg, 0.24 mmol, 1.0 eq) and N-(3-carbamoyl-1-((1r,4r)-4-(piperazin-1-yl)cyclohexyl)-1H-pyrazol-4-yl)-6-(trifluoromethyl)picolinamide hydrochloride (142 mg, 0.28 mmol, 1.2 eq) in DMF (2.0 mL) were added N-methylmorpholine (100 mg, 0.99 mmol, 5.0 eq) and HATU (100 mg, 0.99 mmol, 5.0 eq). The mixture was stirred at rt for 1 h. Upon completion, the reaction solution was purified by HPLC to afford the title compound (white solid, 37.1 mg, yield 24%). **¹H NMR** (400 MHz, DMSO-*d*6) δ 11.71 (s, 1H), 10.84 (s, 1H), 8.44 (s, 1H), 8.42-8.35 (m, 2H), 8.19 (dd, *J =* 7.5, 1.2 Hz, 1H), 7.64 (s, 1H), 7.55 (s, 1H), 6.22 (d, *J* = 12.1 Hz, 2H), 4.24 (t, *J=* 11.8 Hz, 1H), 4.04-4.00 (m, 1H), 3.57-3.42 (m, 6H), 3.37-3.35 (m, 2H), 3.28-3.22 (m, 2H), 2.83-2.74 (m, 1H), 2.58-2.52 (m, 2H), 2.44-2.41 (m, 2H), 2.18-2.06 (m, 5H), 2.03-1.80 (m, 6H), 1.50-1.42 (m, 2H); LCMS (ESI): m/z 786 [M+H]⁺.

### Preparation Example 414. Synthesis of Compound A409

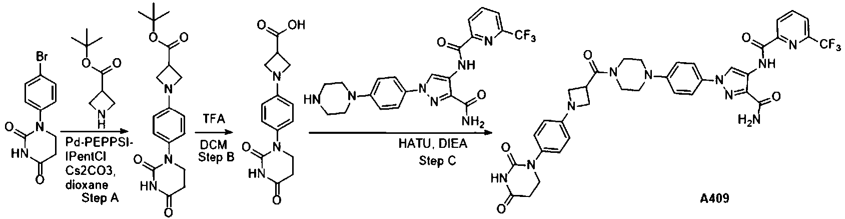

### Step A: Tert-butyl 1-(4-(2,4-dioxotetrahydropyrimidin-1(2H)-yl)phenyl)azetidine-3-carboxylate

At rt, to a solution of tert-butyl azetidine-3-carboxylate (400 mg, 2.54 mmol, 1.0 eq) in dioxane (10 mL) were added 1-(4-bromophenyl)dihydropyrimidine-2,4(1H,3H)-dione (685 mg, 2.54 mmol, 1.0 eq), Pd-PEPPSI-IpentCl (248 mg, 0.25 mmol, 0.1 eq), and cesium carbonate (2487 mg, 7.63 mmol, 3.0 eq). The reaction solution was stirred at 100°C under N₂ for 16 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 330 mg, yield 38%).

### Step B: 1-(4-(2,4-Dioxotetrahydropyrimidin-1(2H)-yl)phenyl)azetidine-3-carboxylic acid

At rt, tert-butyl 1-(4-(2,4-dioxotetrahydropyrimidin-1(2H)-yl)phenyl)azetidine-3-carboxylate (150 mg, 0.43 mmol, 1.0 eq) was added to DCM (10 mL) and TFA (4 mL), and the reaction solution was stirred at rt under N₂ for 2 h. Upon completion, the reaction solution was concentrated under reduced pressure to afford the title compound (yellow oil, 120 mg, crude).

### Step C: N-(3-Carbamoyl-1-(4-(4-(1-(4-(2,4-dioxotetrahydropyrimidin-1(2H)-yl)phenyl)azetidine-3-carbonyl)piperazin-1-yl)phenyl)-1H-pyrazol-4-yl)-6-(trifluoromethyl)picolinamide

At rt, to a mixed solution of 1-(4-(2,4-dioxotetrahydropyrimidin-1(2H)-yl)phenyl)azetidine-3-carboxylic acid (100 mg, crude) in DMF (10 mL) were added N-(3-carbamoyl-1-(4-(piperazin-1-yl)phenyl)-1H-pyrazol-4-yl)-6-(trifluoromethyl)picolinamide (318 mg, 0.69 mmol, 2.0 eq), HATU (263 mg, 0.69 mmol, 2.0 eq), and DIEA (89 mg, 0.69 mmol, 2.0 eq). The mixture was stirred at 0°C under N₂ for 30 min. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (gray solid, 61 mg, yield 23% over two steps). **¹H NMR** (400 MHz, DMSO) δ 11.80 (s, 1H), 10.24 (s, 1H), 8.90 (s, 1H), 8.58-8.32 (m, 2H), 8.21 (dd, J = 7.6, 1.2 Hz, 1H), 7.96 (s, 1H), 7.92-7.79 (m, 2H), 7.72 (s, 1H), 7.25-6.99 (m, 4H), 6.60-6.38 (m, 2H), 4.18-3.99 (m, 2H), 3.96-3.86 (m, 2H), 3.75-3.61 (m, 4H), 3.56-3.45 (m, 2H), 3.29-3.19 (m, 4H), 2.71-2.66 (m, 2H). LCMS (ESI): m/z 731 [M+H]⁺.

### Preparation Example 415. Synthesis of Compound A410

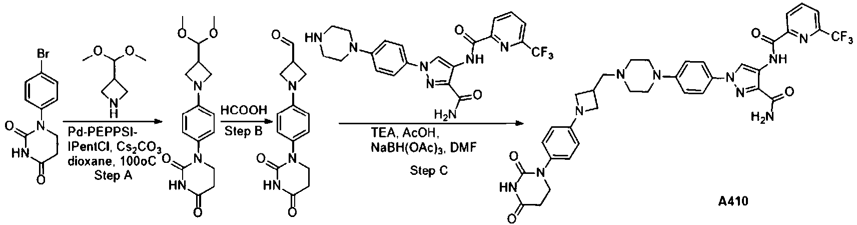

### Step A: 1-(4-(3-(dimethoxymethyl)azetidin-1-yl)phenyl)dihydropyrimidine-2,4(1H,3H)-dione

A mixture of 1-(4-bromophenyl)dihydropyrimidine-2,4(1H,3H)-dione (200 mg, 0.74 mmol, 1.0 eq), (azetidin-3-yl)dimethoxymethane (117 mg, 0.89 mmol, 1.2 eq), Pd-PEPPSI-IPentCl (72 mg, 0.07 mol, 0.1 eq), and Cs2CO3 (726 mg, 2.23 mmol, 3.0 eq) in dioxane (15 mL) was stirred at 110°C overnight under N₂. After filtration, the filtrate was diluted in water (100 mL) and extracted with EtOAc (100 mL×3). The combined organic layers were washed with aqueous NaCl, dried over anhydrous Na₂SO₄, filtered, and concentrated under reduced pressure. The residue was purified by column chromatography to afford the title compound (brown solid, 100 mg, yield 42%).

### Step B: 1-(4-(2,4-Dioxotetrahydropyrimidin-1(2H)-yl)phenyl)azetidine-3-carbaldehyde

A solution of methyl 1-(4-(3-(dimethoxymethyl)azetidin-1-yl)phenyl)dihydropyrimidine-2,4(1H,3H)-dione (150 mg, 0.31 mmol, 1.0 eq) in HCOOH (5 mL) was heated to 50°C and stirred for 3 h. After filtration, the reaction solution was concentrated under reduced pressure to afford 1-(4-(2,4-dioxotetrahydropyrimidin-1(2H)-yl)phenyl)azetidine-3-carbaldehyde (brown oil, 60 mg, yield 70%).

### Step C: N-(3-Carbamoyl-1-(4-(4-((1-(4-(2,4-dioxotetrahydropyrimidin-1(2H)-yl)phenyl)azetidin-3-yl)methyl)piperazin-1-yl)phenyl)-1H-pyrazol-4-yl)-6-(trifluoromethyl)picolinamide

To a solution of N-(3-carbamoyl-1-(4-(piperazin-1-yl)phenyl)-1H-pyrazol-4-yl)-6-(trifluoromethyl)picolinamide (100 mg, 0.22 mmol, 1.0 eq) in DMF (10 mL) were added TEA (0.06 mL, 0.44 mmol, 2.0 eq), AcOH (0.05 mL, 0.88 mmol, 4.0 eq), and 1-(4-(2,4-dioxotetrahydropyrimidin-1(2H)-yl)phenyl)azetidine-3-carbaldehyde (60 mg, 0.22 mmol, 1.0 eq). The mixture was stirred at rt for 30 min. NaBH(OAc)3 (92 mg, 0.44 mmol, 2.0 eq) was then added to the solution. The reaction mixture was then stirred at 60°C for 3 h. The reaction solution was poured into 100 mL of water and extracted with EtOAc (100 mL×3). The combined organic layers were washed with aqueous NaCl (100 mL), dried over anhydrous Na₂SO₄, and concentrated under reduced pressure. The residue was purified by reverse-phase preparative chromatography and lyophilized to afford the title compound (white solid, 16.1 mg, yield 10%). LCMS (ESI): m/z 717 [M+H]⁺. **¹H NMR** (400 MHz, DMSO-*d*6) δ 11.79 (s, 1H), 10.23 (s, 1H), 8.88 (s, 1H), 8.48-8.36 (m, 2H), 8.24-8.18 (m, 1H), 7.95 (s, 1H), 7.79 (d, *J=* 9.1 Hz, 2H), 7.70 (s, 1H), 7.15-7.03 (m, 4H), 6.43 (d, *J=* 8.8 Hz, 2H), 3.95 (t, *J=* 7.4 Hz, 2H), 3.67 (t, *J =* 6.7 Hz, 2H), 3.50-3.46 (m, 4H), 3.25-3.20 (m, 4H), 2.70-2.68 (m, 1H), 2.66-2.63 (m, 2H), 2.59-2.53 (m, 4H).

### Preparation Example 416. Synthesis of Compound A411

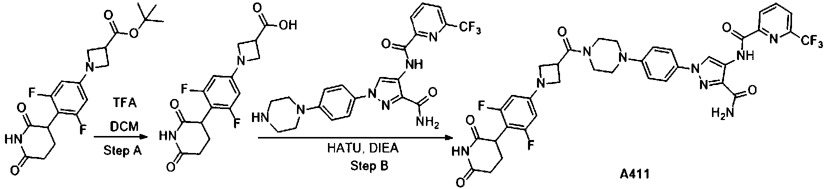

### Step A: 1-(4-(2,6-dioxopiperidin-3-yl)-3,5-difluorophenyl)azetidine-3-carboxylic acid

To a solution of tert-butyl 1-(4-(2,6-dioxopiperidin-3-yl)-3,5-difluorophenyl)azetidine-3-carboxylate (100 mg, 0.26 mmol, 1.0 eq) in DCM (3 mL) was added TFA (1 mL) at rt. The mixture was stirred at rt for 1 h. Upon completion, the reaction mixture was concentrated in vacuo and used directly in the next step without further purification.

### Step B: N-(3-carbamoyl-1-(4-(4-(1-(4-(2,6-dioxopiperidin-3-yl)-3,5-difluorophenyl)azetidine-3-carbonyl)piperazin-1-yl)phenyl)-1H-pyrazol-4-yl)-6-(trifluoromethyl)picolinamide

At 0°C, to a solution of 1-(4-(2,6-dioxopiperidin-3-yl)-3,5-difluorophenyl)azetidine-3-carboxylic acid (85 mg, crude) and N-(3-carbamoyl-1-(4-(piperazin-1-yl)phenyl)-1H-pyrazol-4-yl)-6-(trifluoromethyl)picolinamide (120 mg, 0.26 mmol, 1.0 eq) in DMF (4 mL) were added HATU (199 mg, 0.52 mmol, 2.0 eq) and DIEA (0.2 mL, 1.05 mmol, 4.0 eq). The reaction mixture was then stirred at rt for 30 min. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (yellow solid, 30 mg, yield 15%). **¹H NMR** (400 MHz, DMSO) δ 11.79 (s, 1H), 10.86 (s, 1H), 8.90 (s, 1H), 8.51-8.33 (m, 2H), 8.21 (d, *J= 7.6* Hz, 1H), 7.96 (s, 1H), 7.83 (d, *J=* 8.9 Hz, 2H), 7.72 (s, 1H), 7.12 (d, *J= 9.0* Hz, 2H), 6.18 (d, *J=* 11.1 Hz, 2H), 4.11-4.02 (m, 3H), 4.00-3.89 (m, 3H), 3.71-3.62 (m, 2H), 3.56-3.46 (m, 2H), 3.29-3.17 (m, 4H), 2.84-2.73 (m, 1H), 2.67 (s, 1H), 2.13-2.01 (m, 1H), 1.99-1.93 (m, 1H). LCMS (ESI): m/z 766.4 [M+H]⁺.

### Preparation Example 417. Synthesis of Compound A412

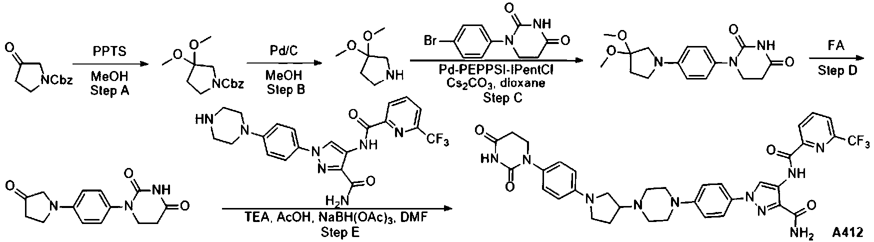

### Step A: Benzyl 3,3-dimethoxypyrrolidine-1-carboxylate

At rt, pyridinium 4-methylbenzenesulfonate (0.18 g, 0.73 mmol, 0.05 eq) was added to a solution of benzyl 3-oxopyrrolidine-1-carboxylate (3.2 g, 14.6 mmol, 1.0 eq) in anhydrous MeOH (3 mL). The reaction solution was stirred at rt under N₂ for 18 h. Upon completion, the reaction solution was poured into water and extracted with EtOAc. The organic phase was washed with aqueous NaCl, dried over anhydrous Na₂SO₄, and concentrated under reduced pressure. The residue was purified by column chromatography to afford the title compound (colorless oil, 2.2 g, yield 57%).

### Step B: 3,3-Dimethoxypyrrolidine

At rt, 10% wet palladium on carbon (0.22 g) was added to a solution of benzyl 3,3-dimethoxypyrrolidine-1-carboxylate (2.2 g, 8.29 mmol, 1.0 eq) in MeOH (22 mL). The mixture was stirred under H₂ at rt for 3 h. Upon completion, the reaction solution was filtered and concentrated under reduced pressure to afford the title compound (yellow oil, 1 g, crude), which was used directly without purification.

### Step C: 1-(4-(3,3-Dimethoxypyrrolidin-1-yl)phenyl)dihydropyrimidine-2,4(1H,3H)-dione

At rt, to a solution of 3,3-dimethoxypyrrolidine (146 mg, 1.11 mmol, 1.5 eq), 1-(4-bromophenyl)dihydropyrimidine-2,4(1H,3H)-dione (200 mg, 0.74 mmol, 1.0 eq), and Pd-PEPPSI-IPentCl (726 mg, 2.23 mmol, 0.1 eq) in 1,4-dioxane (4 mL) was added cesium carbonate (726 mg, 2.23 mmol, 3.0 eq). The reaction solution was purged with N₂ (×3), and then stirred at 110°C under N₂ for 18 h. Upon completion, the mixture was cooled to rt, diluted in EtOAc, washed sequentially with water and saturated aqueous NaCl, dried over anhydrous Na₂SO₄, filtered, and concentrated under reduced pressure. The residue was purified by column chromatography to afford the title compound (yellow oil, 200 mg, yield 84%).

### Step D: 1-(4-(3-Oxopyrrolidin-1-yl)phenyl)dihydropyrimidine-2,4(1H,3H)-dione

At rt, a solution of 1-(4-(3,3-dimethoxypyrrolidin-1-yl)phenyl)dihydropyrimidine-2,4(1H,3H)-dione (200 mg, 0.63 mmol, 1.0 eq) in formic acid (2 mL) was stirred for 2 h. Upon completion, the reaction solution was directly concentrated under reduced pressure to afford the title compound (black oil, 150 mg, yield 88%), which was used directly without purification.

### Step E: N-(3-Carbamoyl-1-(4-(4-(1-(4-(2,4-dioxotetrahydropyrimidin-1(2H)-yl)phenyl)pyrrolidin-3-yl)piperazin-1-yl)phenyl)-1H-pyrazol-4-yl)-6-(trifluoromethyl)picolinamide

At rt, to a solution of N-(3-carbamoyl-1-(4-(piperazin-1-yl)phenyl)-1H-pyrazol-4-yl)-6-(trifluoromethyl)picolinamide (252 mg, 0.55 mmol, 1.0 eq) in DMF (5 mL) was added TEA (0.5 mL) to adjust the pH to 10. After stirring for 10 min, AcOH (2 mL) was added to adjust the pH to 3. The mixture was stirred for 5 min. 1-(4-(3-oxopyrrolidin-1-yl)phenyl)dihydropyrimidine-2,4(1H,3H)-dione (150 mg, 0.55 mmol, 1.0 eq) was then added, and the mixture was stirred at rt for 1 h. Then sodium triacetoxyborohydride (232 mg, 1.1 mmol, 2.0 eq) was added. The reaction solution was stirred at rt for 18 h. The reaction solution was poured into water and extracted with EtOAc. The organic phase was washed with aqueous NaCl, dried over anhydrous Na₂SO₄, and concentrated under reduced pressure. The residue was purified by HPLC to afford the title compound (brown solid, 12.9 mg, yield 3%). LCMS (ESI): m/z 717.6 [M+H]⁺. **¹H NMR** (400 MHz, DMSO-*d₆*) δ 11.80 (s, 1H), 10.25 (s, 1H), 8.94 (s, 1H), 8.49-8.35 (m, 2H), 8.24-8.19 (m, 1H), 7.96 (s, 1H), 7.90-7.84 (m, 2H), 7.75 (s, 1H), 7.22-7.14 (m, 4H), 6.66 (d, J = 8.9 Hz, 2H), 4.17-3.92 (m, 2H), 3.72-3.65 (m, 3H), 3.62-3.49 (m, 3H), 3.34-3.22 (m, 4H), 3.18-2.98 (m, 2H), 2.75-2.63 (m, 2H), 2.34-2.23 (m, 1H), 1.36-1.14 (m, 2H).

### Preparation Example 418. Synthesis of Compound A413

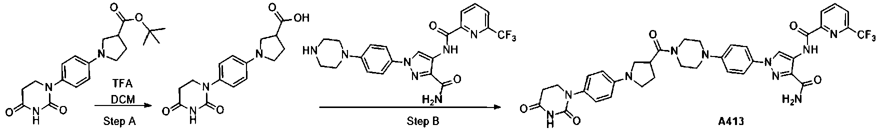

### Step A: 1-(4-(2,4-Dioxotetrahydropyrimidin-1(2H)-yl)phenyl)pyrrolidine-3-carboxylic acid

A mixture of tert-butyl 1-(4-(2,4-dioxotetrahydropyrimidin-1(2H)-yl)phenyl)pyrrolidine-3-carboxylate (60 mg, 0.17 mmol, 1.0 eq), DCM (3 mL), and TFA (1 mL) was stirred at 25°C for 30 min. The reaction solution was concentrated under reduced pressure to afford 1-(4-(2,4-dioxotetrahydropyrimidin-1(2H)-yl)phenyl)pyrrolidine-3-carboxylic acid (yellow oil, 50 mg, crude).

### Step B: N-(3-Carbamoyl-1-(4-(1-(4-(2,4-dioxotetrahydropyrimidin-1(2H)-yl)phenyl)pyrrolidine-3-carbonyl)piperazin-1-yl)phenyl)-1H-pyrazol-4-yl)-6-(trifluoromethyl)picolinamide

At 0°C, to a solution of 1-(4-(2,4-dioxotetrahydropyrimidin-1(2H)-yl)phenyl)pyrrolidine-3-carboxylic acid (45 mg, 0.15 mmol, 1.0 eq) in DMF (3 mL) were added N-(3-carbamoyl-1-(4-(piperazin-1-yl)phenyl)-1H-pyrazol-4-yl)-6-(trifluoromethyl)picolinamide (68 mg, 0.15 mmol, 1.0 eq), HATU (113 mg, 0.30 mmol, 2.0 eq), and DIEA (77 mg, 0.6 mmol, 4.0 eq). The mixture was stirred at 0°C for 30 min. The reaction solution was purified directly by preparative HPLC to afford N-(3-carbamoyl-1-(4-(4-(2,4-dioxotetrahydropyrimidin-1(2H)-yl)phenyl)pyrrolidine-3-carbonyl)piperazin-1-yl)phenyl)-1H-pyrazol-4-yl)-6-(trifluoromethyl)picolinamide (yellow solid, 28 mg, yield 25%). LCMS (ESI): m/z 745[M+H]⁺. **¹H NMR** (400 MHz, DMSO-d6) δ 11.79 (s, 1H), 10.21 (s, 1H), 8.90 (s, 1H), 8.51-8.38 (m, 2H), 8.25-8.19 (m, 1H), 7.96 (s, 1H), 7.86-7.80 (m, 2H), 7.72 (s, 1H), 7.16-7.08 (m, 4H), 6.55 (d, J = 8.9 Hz, 2H), 3.80-3.72 (m, 2H), 3.71-3.64 (m, 4H), 3.63-3.55 (m, 1H), 3.53-3.47 (m, 1H), 3.41-3.33 (m, 2H), 3.30-3.27 (m, 2H), 3.25-3.19 (m, 2H), 2.70-2.66 (m, 2H), 2.25-2.12 (m, 2H), 2.07 (s, 1H).

### Preparation Example 419. Synthesis of Compound A414

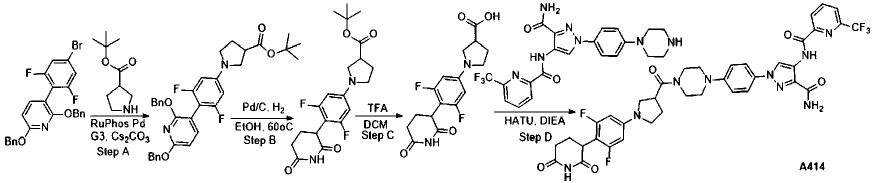

### Step A: Tert-butyl 1-(4-(2,6-bis(benzyloxy)pyridin-3-yl)-3,5-difluorophenyl)pyrrolidine-3-carboxylate

To a solution of 2,6-bis(benzyloxy)-3-(4-bromo-2,6-difluorophenyl)pyridine (1.7 g, 3.53 mmol, 1.0 eq) in toluene (15 mL) were added 2-methylpropan-2-yl pyrrolidine-3-carboxylatee (0.73 g, 4.24 mmol, 1.2 eq), RuPhos Pd G3 (0.29 g, 0.35 mmol, 0.1 eq), RuPhos (0.32 g, 0.7 mmol, 0.2 eq), and cesium carbonate (2.88 g, 8.83 mmol, 2.5 eq). The mixture was stirred at 100°C under N₂ for 18 h. Upon completion, the mixture was diluted in EtOAc, washed sequentially with water and saturated aqueous NaCl, dried over anhydrous Na₂SO₄, and concentrated under reduced pressure. The residue was purified by column chromatography to afford the title compound (yellow oil, 500 mg, yield 25%).

### Step B: Tert-butyl 1-(4-(2,6-dioxopiperidin-3-yl)-3,5-difluorophenyl)pyrrolidine-3-carboxylate

To a solution of tert-butyl 1-(4-(2,6-bis(benzyloxy)pyridin-3-yl)-3,5-difluorophenyl)pyrrolidine-3-carboxylate (500 mg, 0.87 mmol, 1.0 eq) in ethanol (5 mL) was added 10% Pd/C (50 mg). The mixture was stirred at 60°C under H₂ for 18 h. After filtration, the filtrate was concentrated under reduced pressure to afford the title compound (yellow solid, 333 mg, yield 97%).

### Step C: 1-(4-(2,6-Dioxopiperidin-3-yl)-3,5-difluorophenyl)pyrrolidine-3-carboxylic acid

A mixture of tert-butyl 1-(4-(2,6-dioxopiperidin-3-yl)-3,5-difluorophenyl)pyrrolidine-3-carboxylate (150 mg, 0.38 mmol, 1.0 eq) in DCM (10 mL) and TFA (4 mL) was stirred at 25°C for 2 h. The reaction solution was concentrated under reduced pressure to afford the title compound (yellow oil, 120 mg, yield 89%).

### Step D: N-(3-Carbamoyl-1-(4-(1-(4-(2,6-dioxopiperidin-3-yl)-3,5-difluorophenyl)pyrrolidine-3-carbonyl)piperazin-1-yl)phenyl)-1H-pyrazol-4-yl)-6-(trifluoromethyl)picolinamide

To a mixture of 1-(4-(2,6-dioxopiperidin-3-yl)-3,5-difluorophenyl)pyrrolidine-3-carboxylic acid (100 mg, 0.35 mmol, 1.0 eq) in DMF (10 mL) were added N-(3-carbamoyl-1-(4-(piperazin-1-yl)phenyl)-1H-pyrazol-4-yl)-6-(trifluoromethyl)picolinamide (318 mg, 0.69 mmol, 2.0 eq), HATU (263 mg, 0.691 mmol, 2.0 eq), and DIEA (89 mg, 0.69 mmol, 2.0 eq). The mixture was stirred at 0°C under N₂ for 30 min. The reaction solution was poured into water (30 mL), filtered, and concentrated under reduced pressure. The residue was purified by preparative chromatography to afford the title compound (gray solid, 84 mg, yield 37%). LCMS (ESI): m/z 780 [M+H]⁺. **¹H NMR** (400 MHz, DMSO-d6) δ 11.79 (s, 1H), 10.84 (s, 1H), 8.90 (s, 1H), 8.52-8.32 (m, 2H), 8.21 (dd, *J= 7.6,* 1.2 Hz, 1H), 7.96 (s, 1H), 7.86-7.81 (m, 2H), 7.72 (s, 1H), 7.16-7.11 (m, 2H), 6.27-6.22 (m, 2H), 4.02 (dd, J = 12.6, 5.1 Hz, 1H), 3.78-3.71 (m, 2H), 3.70-3.63 (m, 2H), 3.63-3.56 (m, 1H), 3.52-3.46 (m, 1H), 3.41-3.33 (m, 2H), 3.30-3.26 (m, 3H), 3.25-3.19 (m, 2H), 2.84-2.73 (m, 1H), 2.55-2.51 (m, 1H), 2.26-2.12 (m, 2H), 2.08-2.03 (m, 1H), 1.97-1.90 (m, 1H).

### Preparation Example 420. Synthesis of Compound A415

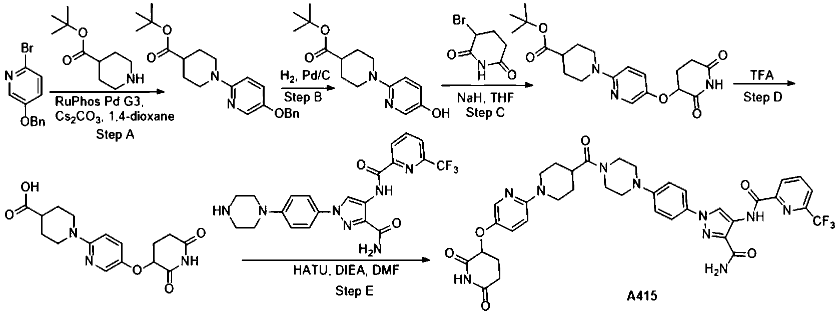

### Step A: Tert-butyl 1-(5-(benzyloxy)pyridin-2-yl)piperidine-4-carboxylate

A mixture of tert-butyl piperidine-4-carboxylate (1 g, 5.37 mmol, 1.0 eq), 5-(benzyloxy)-2-bromopyridine (1.69 g, 6.44 mmol, 1.2 eq), RuPhos Pd G3 (449 mg, 0.53 mmol, 0.1 eq), and Cs2CO3 (5.24 g, 16.1 mmol, 3.0 eq) in 1,4-dioxane (10 mL) was stirred at 110°C under N₂ for 3 h. Upon completion, the mixture was diluted in EtOAc, washed sequentially with water and saturated aqueous NaCl, dried over anhydrous Na₂SO₄, and concentrated under reduced pressure. The residue was purified by column chromatography to afford the title compound (white solid, 500 mg, yield 25%).

### Step B: Tert-butyl 1-(5-hydroxypyridin-2-yl)piperidine-4-carboxylate

To a solution of tert-butyl 1-(5-(benzyloxy)pyridin-2-yl)piperidine-4-carboxylate (450 mg, 1.22 mmol, 1.0 eq) in ethanol (10 mL) was added 10% wet palladium on carbon (50 mg) and then the reaction was stirred under H₂ at rt for 1 h. Upon completion, the reaction solution was filtered, and the filtrate was concentrated under reduced pressure to afford the title compound (yellow solid, 330 mg, crude), which was used directly without purification.

### Step C: Tert-butyl 1-(5-((2,6-dioxopiperidin-3-yl)oxy)pyridin-2-yl)piperidine-4-carboxylate

At -5°C under N₂, sodium hydride (120 mg, 3.21 mmol, 3.0 eq) was added to a solution of tert-butyl 1-(5-hydroxypyridin-2-yl)piperidine-4-carboxylate (300 mg, 1.07 mmol, 1.0 eq) in THF (5 mL). The mixture was stirred under N₂ for 15 min. Then 3-bromopiperidine-2,6-dione (250 mg, 1.28 mmol, 1.2 eq) was added, and the reaction solution was stirred at -5°C for 1 h. Upon completion, the reaction solution was quenched with saturated aqueous NH₄Cl, extracted with EtOAc, dried over anhydrous Na₂SO₄, and concentrated under reduced pressure. The residue was purified by column chromatography to afford the title compound (yellow solid, 220 mg, yield 54%).

### Step D: 1-(5-((2,6-Dioxopiperidin-3-yl)oxy)pyridin-2-yl)piperidine-4-carboxylic acid

TFA (2 mL) was added to a solution of tert-butyl 1-(5-((2,6-dioxopiperidin-3-yl)oxy)pyridin-2-yl)piperidine-4-carboxylate (180 mg, 0.46 mmol, 1.0 eq) in DCM (2 mL). The reaction solution was stirred at rt for 30 min. Upon completion, the reaction solution was concentrated under reduced pressure to afford the title compound (yellow solid, 150 mg, crude), which was used directly without purification.

### Step E: N-(3-Carbamoyl-1-(4-(4-(1-(5-((2,6-dioxopiperidin-3-yl)oxy)pyridin-2-yl)piperidine-4-carbonyl)piperazin-1-yl)phenyl)-1H-pyrazol-4-yl)-6-(trifluoromethyl)picolinamide

At rt, to a solution of 1-(5-((2,6-dioxopiperidin-3-yl)oxy)pyridin-2-yl)piperidine-4-carboxylic acid (100 mg, 0.29 mmol, 1.0 eq) and N-(3-carbamoyl-1-(4-(piperazin-1-yl)phenyl)-1H-pyrazol-4-yl)-6-(trifluoromethyl)picolinamide (151 mg, 0.32 mmol, 1.1 eq) in DMF (5 mL) were added HATU (136 mg, 0.35 mmol, 1.2 eq) and DIEA (115 mg, 0.87 mmol, 3.0 eq). The reaction solution was stirred at rt for 30 min. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 25 mg, yield 11%). LCMS (ESI): m/z 775 [M+H]⁺. **¹H NMR** (400 MHz, DMSO-d6) δ 11.79 (s, 1H), 10.91 (s, 1H), 8.90 (s, 1H), 8.54-8.32 (m, 2H), 8.21 (d, *J=* 7.3 Hz, 1H), 8.04-7.88 (m, 2H), 7.83 (d, *J=* 8.9 Hz, 2H), 7.72 (s, 1H), 7.38-7.32 (m, 1H), 7.12 (d, *J= 9.2* Hz, 2H), 6.82 (d, *J* = 9.1 Hz, 1H), 5.02-4.98 (m, 1H), 4.20-4.17 (m, 2H), 3.73-3.63 (m, 4H), 3.27-3.20 (m, 4H), 2.89-2.80 (m, 2H), 2.74-2.52 (m, 2H), 2.25-2.15 (m, 1H), 2.13-1.96 (m, 2H), 1.71-1.57 (m, 4H).

### Preparation Example 421. Synthesis of Compound A416

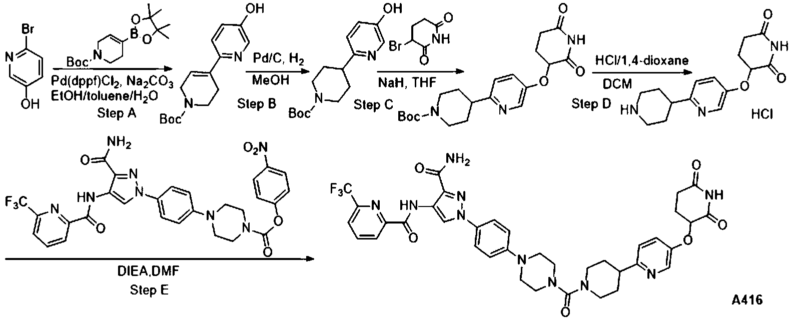

### Step A: Tert-butyl 5'-hydroxy-3',6'-dihydro-[2,4'-bipyridine]-1'(2'H)-carboxylate

To a mixed solution of 6-bromopyridin-3-ol (5 g, 28.7 mmol, 1.0 eq) and tert-butyl 4-(4,4,5,5-tetramethyl-1,3,2-dioxaborolan-2-yl)-3,6-dihydropyridine-1(2H)-carboxylate (13.3 g, 16.3 mmol, 1.5 eq) in ethanol (20 mL), toluene (40 mL), and water (10 mL) were added Pd(dppf)Cl₂ (800 mg, 1.08 mmol, 0.05 eq) and Na2CO3 (6.1 g, 57.4 mmol, 2.0 eq). The reaction mixture was degassed (×3) under N₂ and stirred at 100°C for 3 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 5.1 g, yield 63%).

### Step B: Tert-butyl 4-(5-hydroxypyridin-2-yl)piperidine-1-carboxylate

To a solution of tert-butyl 5'-hydroxy-3',6'-dihydro-[2,4'-bipyridine]-1'(2'H)-carboxylate (2.5 g, 9.06 mmol, 1.0 eq) in MeOH (40 mL) was added 5% wet Pd/C (200 mg). The reaction mixture was degassed (×3) under H₂ and stirred under H₂ at 25°C overnight. The mixture was filtered through Celite, and the filtrate was collected and concentrated under reduced pressure to afford the title compound (yellow solid, 1.8 g, crude).

### Step C: Tert-butyl 4-(5-((2,6-dioxopiperidin-3-yl)oxy)pyridin-2-yl)piperidine-1-carboxylate

To a solution of tert-butyl 4-(5-hydroxypyridin-2-yl)piperidine-1-carboxylate (1.7 g, crude) and 3-bromopiperidine-2,6-dione (2.3 g, 12.2 mmol, 2.0 eq) in THF (60 mL) was added NaH (488 mg, 12.2 mmol, 2.0 eq, 60% dispersion in mineral oil). The mixture was stirred at 45°C under N₂ for 18 h. Upon completion, the mixture was quenched with water and extracted with EtOAc twice. The combined organic layers were washed with saturated aqueous NaCl, dried over anhydrous Na₂SO₄, and concentrated under reduced pressure. The residue was purified by column chromatography to afford the title compound (yellow solid, 2.0 g, yield 84%).

### Step D: 3-((6-(Piperidin-4-yl)pyridin-3-yl)oxy)piperidine-2,6-dione hydrochloride

A 4 M hydrogen chloride-dioxane (2.5 mL) was added to a solution of tert-butyl 4-(5-((2,6-dioxopiperidin-3-yl)oxy)pyridin-2-yl)piperidine-1-carboxylate (1.2 g, 3.08 mmol, 1.0 eq) in DCM (20 mL). Upon completion, the reaction mixture was concentrated under reduced pressure to dryness to afford the title compound (yellow solid, 975 mg, crude).

### Step E: N-(3-Carbamoyl-1-(4-(4-(4-(5-((2,6-dioxopiperidin-3-yl)oxy)pyridin-2-yl)piperidine-1-carbonyl)piperazin-1-yl)phenyl)-1H-pyrazol-4-yl)-6-(trifluoromethyl)picolinamide

To a solution of 4-nitrophenyl 4-(4-(3-carbamoyl-4-(6-(trifluoromethyl)picolinamido)-1H-pyrazol-1-yl)phenyl)piperazine-1-carboxylate (312 mg, 0.5 mmol, 1.0 eq) and 3-((6-(piperidin-4-yl)pyridin-3-yl)oxy)piperidine-2,6-dione hydrochloride (145 mg, crude) in DMF (3 mL) was added DIEA (129 mg, 1.0 mmol, 2.0 eq). The mixture was stirred at 80°C for 3 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (yellow solid, 25.0 mg, yield 6%). **¹H NMR** (400 MHz, DMSO-d6) δ 11.79 (s, 1H), 10.97 (s, 1H), 8.89 (s, 1H), 8.46-8.38 (m, 2H), 8.26 (d, *J =* 3.0 Hz, 1H), 8.21 (d, *J= 7.6* Hz, 1H), 7.96 (s, 1H), 7.82 (d, *J= 9.0* Hz, 2H), 7.71 (s, 1H), 7.42-7.38 (m, 1H), 7.24 (d, *J* = 8.7 Hz, 1H), 7.11 (d, *J =* 9.1 Hz, 2H), 5.29-5.23 (m, 1H), 3.76 (d, *J =* 12.5 Hz, 2H), 3.26-3.23 (m, 4H), 2.94-2.81 (m, 4H), 2.72-2.58 (m, 4H), 2.26-2.16 (m, 2H), 2.04-1.94 (m, 1H), 1.84-1.78 (m, 2H), 1.73-1.65 (m, 2H). LCMS (ESI): m/z 775 [M+H]⁺.

### Preparation Example 422. Synthesis of Compound A417

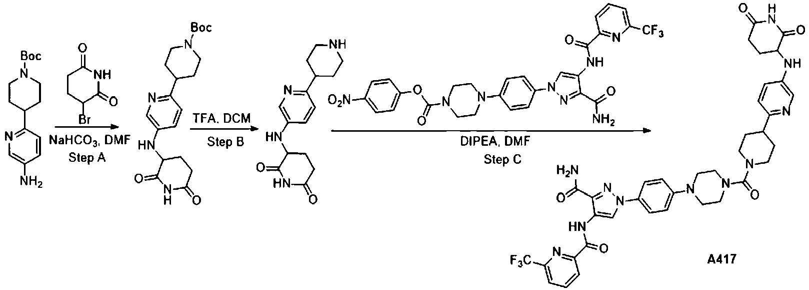

### Step A: Tert-butyl 4-(5-((2,6-dioxopiperidin-3-yl)amino)pyridin-2-yl)piperidine-1-carboxylate

At rt, to a solution of tert-butyl 4-(5-aminopyridin-2-yl)piperidine-1-carboxylate (500 mg, 1.80 mmol, 1.0 eq) in DMF (5 mL) were added 3-bromopiperidine-2,6-dione (518 mg, 2.70 mmol, 1.5 eq) and NaHCO₃ (758 mg, 9.02 mmol, 5.0 eq). The mixture was stirred at 100°C for 48 h. Upon completion, the reaction solution was diluted in saturated aqueous NH₄Cl and extracted with EtOAc. The organic layer was washed with saturated aqueous NaCl, dried over anhydrous Na₂SO₄, and concentrated under reduced pressure. The residue was purified by column chromatography to afford the title compound (yellow solid, 200 mg, yield 28%).

### Step B: 3-((6-(Piperidin-4-yl)pyridin-3-yl)amino)piperidine-2,6-dione

At 0°C, TFA (2 mL) was added to a solution of tert-butyl 4-(5-((2,6-dioxopiperidin-3-yl)amino)pyridin-2-yl)piperidine-1-carboxylate (200 mg, 0.51 mmol, 1.0 eq) in DCM (2 mL). The mixture was stirred at rt for 18 h. Upon completion, the reaction solution was concentrated to dryness. The residue was extracted with DCM and saturated aqueous NaHCO₃. The organic layer was washed with saturated aqueous NaCl, dried over anhydrous Na₂SO₄, and concentrated under reduced pressure. The residue was purified by column chromatography to afford the title compound (yellow solid, 120 mg, yield 81%).

### Step C: N-(3-Carbamoyl-1-(4-(4-(4-(5-((2,6-dioxopiperidin-3-yl)amino)pyridin-2-yl)piperidine-1-carbonyl)piperazin-1-yl)phenyl)-1H-pyrazol-4-yl)-6-(trifluoromethyl)picolinamide

At rt, to a solution of 3-((6-(piperidin-4-yl)pyridin-3-yl)amino)piperidine-2,6-dione (100 mg, 0.34 mmol, 1.0 eq) in DMF (3 mL) were added DIEA (150 mg, 1.16 mmol, 3.4 eq) and 4-nitrophenyl 4-(4-(3-carbamoyl-4-(6-(trifluoromethyl)pyridinylamino)-1H-pyrazol-1-yl)phenyl)piperazine-1-carboxylate (212 mg, 0.34 mmol, 1.0 eq). The mixture was stirred at rt for 30 min. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 40.0 mg, yield 15%). **¹H NMR** (400 MHz, DMSO-d6) δ 11.79 (s, 1H), 10.80 (s, 1H), 8.89 (s, 1H), 8.49-8.36 (m, 2H), 8.25-8.19 (m, 1H), 8.02-7.90 (m, 2H), 7.82 (d, *J= 9.0* Hz, 2H), 7.71 (s, 1H), 7.11 *(d, J=* 9.2 Hz, 2H), 7.03-6.93 (m, 2H), 5.96 *(d, J=* 7.8 Hz, 1H), 4.40-4.27 (m, 1H), 3.81-3.70 (m, 2H), 3.38-3.33 (m, 2H), 3.30-3.28 (m, 1H), 3.27-3.20 (m, 4H), 2.93-2.82 (m, 2H), 2.74-2.66 (m, 2H), 2.65-2.52 (m, 2H), 2.14-2.07 (m, 1H), 1.95-1.85 (m, 1H), 1.82-1.72 (m, 2H), 1.70-1.57 (m, 2H). LCMS (ESI): m/z 774 [M+H]⁺.

### Preparation Example 423. Synthesis of Compound A418

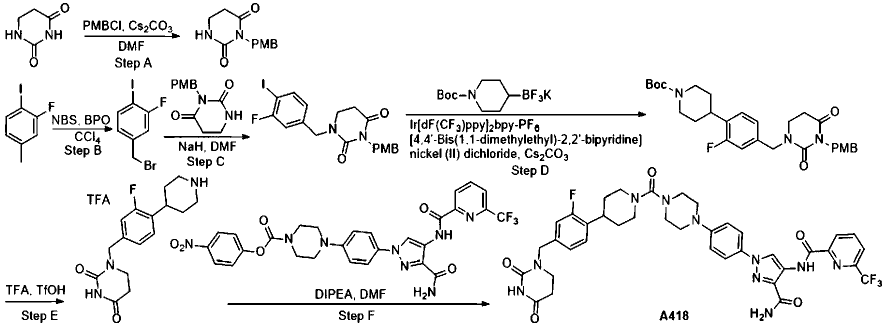

### Step A: 3-(4-Methoxybenzyl)dihydropyrimidine-2,4(1H,3H)-dione

At rt, to a solution of dihydropyrimidine-2,4(1H,3H)-dione (7 g, 61.35 mmol, 1.0 eq) in DMF (140 mL) were added Cs2CO3 (40 g, 122.69 mmol, 2.0 eq) and PMBCl (12.3 g, 78.35 mmol, 1.28 eq). The mixture was stirred at rt under N₂ overnight. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 6 g, yield 40%).

### Step B: 4-(Bromomethyl)-2-fluoro-1-iodobenzene

At rt, to a solution of 2-fluoro-1-iodo-4-methylbenzene (10 g, 42.36 mmol, 1.0 eq) in CCl4 (100 mL) were added BPO (0.5 g, 2.12 mmol, 0.05 eq) and NBS (8.7 g, 48.72 mmol, 1.15 eq). The mixture was stirred at 75°C under N₂ overnight. Upon completion, the reaction solution was cooled to rt, poured into water, and extracted with EtOAc. The organic layer was washed sequentially with saturated aqueous NaCl, dried over anhydrous Na₂SO₄, and concentrated under reduced pressure. The residue was purified by column chromatography to afford the title compound (yellow solid, 11 g, yield 82%).

### Step C: 1-(3-Fluoro-4-iodobenzyl)-3-(4-methoxybenzyl)dihydropyrimidine-2,4(1H,3H)-dione

At 0°C, NaH (200 mg, 60% dispersion in mineral oil, 8.18 mmol, 2.0 eq) was added to a solution of 3-(4-methoxybenzyl)dihydropyrimidine-2,4(1H,3H)-dione (1.0 g, 4.09 mmol, 1.0 eq) in DMF (10 mL) under N₂. The mixture was stirred at rt under N₂ for 20 min. At 0°C, a solution of 4-(bromomethyl)-2-fluoro-1-iodobenzene (1.0 g, 3.27 mmol, 0.8 eq) in DMF (10 mL) was added dropwise to the above mixture. The resulting mixture was stirred at rt for 2 h. Upon completion, the reaction mixture was quenched with saturated aqueous NH₄Cl and extracted with EtOAc twice. The organic layer was washed sequentially with saturated aqueous NaCl, dried over anhydrous Na₂SO₄, and concentrated under reduced pressure. The residue was purified by column chromatography to afford the title compound (colorless oil, 600 mg, yield 31%).

### Step D: Tert-butyl 4-(2-fluoro-4-((3-(4-methoxybenzyl)-2,4-dioxotetrahydropyrimidin-1(2H)-yl)methyl)phenyl)piperidine-1-carboxylate

At rt, to a mixture of 1-(3-fluoro-4-iodobenzyl)-3-(4-methoxybenzyl)dihydropyrimidine-2,4(1H,3H)-dione (300 mg, 0.64 mmol, 1.0 eq) and potassium tert-butyl 4-(trifluoro-λ⁴-boranyl)piperidine-1-carboxylate (280 mg, 0.96 mmol, 1.5 eq) in 1,4-dioxane (3 mL) were added Cs2CO3 (313 mg, 0.96 mmol, 1.0 eq), [4,4'-bis(1,1-dimethylethyl)-2,2'-bipyridine]nickel(II) chloride (13 mg, 0.03 mmol, 0.05 eq), and Ir[dF(CF₃)ppy]₂bpy-PF₆ (16 mg, 0.02 mmol, 0.03 eq). The reaction mixture was degassed (×3) under N₂ and stirred at rt under white light irradiation for 16 h. Upon completion, the mixture was diluted in water and extracted with EtOAc twice. The combined organic layers were washed with saturated aqueous NaCl, dried over anhydrous Na₂SO₄, and concentrated under reduced pressure. The residue was purified by column chromatography to afford the title compound (colorless oil, 230 mg, yield 68%).

### Step E: 1-(3-Fluoro-4-(piperidin-4-yl)benzyl)dihydropyrimidine-2,4(1H,3H)-dione trifluoroacetate

At rt, tert-butyl 4-(2-fluoro-4-((3-(4-methoxybenzyl)-2,4-dioxotetrahydropyrimidin-1(2H)-yl)methyl)phenyl)piperidine-1-carboxylate (230 mg, 0.43 mmol, 1.0 eq) was added to trifluoromethanesulfonic acid (1 mL) and TFA (4 mL), and the reaction solution was stirred at 80°C overnight. Upon completion, the mixture was cooled to rt. The reaction solution was purified by HPLC to afford the title compound (colorless oil, 100 mg, yield 75%).

### Step F: N-(3-Carbamoyl-1-(4-(4-(4-((2,4-dioxotetrahydropyrimidin-1(2H)-yl)methyl)-2-fluorophenyl)piperidine-1-carbonyl)piperazin-1-yl)phenyl)-1H-pyrazol-4-yl)-6-(trifluoromethyl)picolinamide

At rt, to a solution of 1-(3-fluoro-4-(piperidin-4-yl)benzyl)dihydropyrimidine-2,4(1H,3H)-dione trifluoroacetate (100 mg, 0.25 mmol, 1.0 eq) in DMF (2 mL) were added 4-nitrophenyl 4-(4-(3-carbamoyl-4-(6-(trifluoromethyl)pyridinylamino)-1H-pyrazol-1-yl)phenyl)piperazine-1-carboxylate (100 mg, 0.16 mmol, 0.67 eq) and DIEA (96 mg, 0.74 mmol, 3.0 eq). The mixture was stirred at 100°C under N₂ overnight. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (yellow solid, 23.0 mg, yield 12%). **¹H NMR** (400 MHz, DMSO-d6) δ 11.79 (s, 1H), 10.21 (s, 1H), 8.90 (d, *J* = 2.4 Hz, 1H), 8.48-8.36 (m, 2H), 8.21 (d, *J=* 8.1 Hz, 1H), 7.96 (s, 1H), 7.82 (d, *J =* 6.6 Hz, 2H), 7.71 (s, 1H), 7.33 (d, *J =* 9.8 Hz, 1H), 7.13-7.05 (m, 4H), 4.49 (s, 2H), 3.83-3.73 (m, 2H), 3.38-3.34 (m, 2H), 3.26-3.21 (m, 4H), 3.08-2.81 (m, 4H), 2.60-2.51 (m, 5H), 1.76-1.60 (m, 4H). LCMS (ESI): m/z 791 [M+H]⁺.

### Preparation Example 424. Synthesis of Compound A419

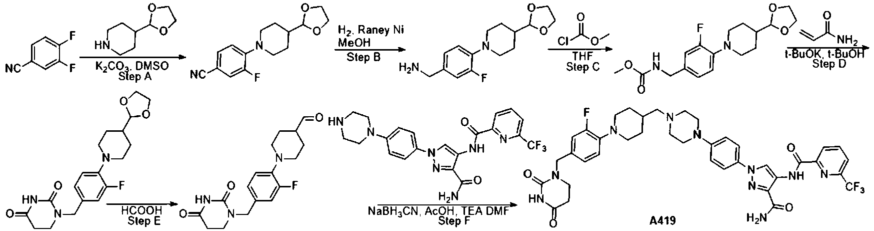

### Step A: 4-(4-(1,3-Dioxolan-2-yl)piperidin-1-yl)-3-fluorobenzonitrile

At rt, to a solution of 3,4-difluorobenzonitrile (1.77 g, 12.7 mmol, 1.0 eq) in dimethyl sulfoxide (15 mL) were added potassium carbonate (5.27 g, 38.2 mmol, 3.0 eq) and 4-(1,3-dioxolan-2-yl)piperidine (2.0 g, 12.7 mmol, 1.0 eq). The reaction was stirred at 70°C for 3 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 2.25 g, yield 64%).

### Step B: (4-(4-(1,3-Dioxolan-2-yl)piperidin-1-yl)-3-fluorophenyl)methanamine

At rt, Raney Nickel (316 mg, 1.45 mmol, 0.2 eq) was added to a solution of 4-(4-(1,3-dioxolan-2-yl)piperidin-1-yl)-3-fluorobenzonitrile (2.0 g, 7.24 mmol, 1.0 eq) in MeOH (30 mL). The mixture was stirred under H₂ at rt overnight. The mixture was filtered and concentrated to afford the title compound (white solid, 1.96 g, crude), which was used directly in the next step without further purification.

### Step C: Methyl (4-(4-(1,3-dioxolan-2-yl)piperidin-1-yl)-3-fluorobenzyl)carbamate

At rt, methyl chloroformate (0.55 mL, 7.13 mmol, 1.2 eq) was added to a solution of (4-(4-(1,3-dioxolan-2-yl)piperidin-1-yl)-3-fluorophenyl)methanamine (1.96 g, 6.99 mmol, 1.0 eq) in THF (10 mL). The mixture was stirred at rt for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 980 mg, yield 41%).

### Step D: 1-(4-(4-(1,3-Dioxolan-2-yl)piperidin-1-yl)-3-fluorobenzyl)dihydropyrimidine-2,4(1H,3H)-dione

At rt, to a solution of methyl (4-(4-(1,3-dioxolan-2-yl)piperidin-1-yl)-3-fluorobenzyl)carbamate (950 mg, 2.81 mmol, 1.0 eq) in tert-butanol (10 mL) were added acrylamide (998 mg, 14.0 mmol, 4.98 eq) and potassium tert-butoxide (945 mg, 8.42 mmol, 3.0 eq). The mixture was stirred at 55°C overnight. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 270 mg, yield 25%).

### Step E: 1-(4-((2,4-Dioxotetrahydropyrimidin-1(2H)-yl)methyl)-2-fluorophenyl)piperidine-4-carbaldehyde

At rt, formic acid (3 mL) was added to a flask containing 1-(4-(4-(1,3-dioxolan-2-yl)piperidin-1-yl)-3-fluorobenzyl)dihydropyrimidine-2,4(1H,3H)-dione (270 mg, 0.72 mmol, 1.0 eq). The mixture was stirred at 60°C for 3 h. The reaction solution was diluted in EtOAc and neutralized with saturated aqueous NaHCO3 solution. The organic phase was washed sequentially with water and saturated aqueous NaCl, dried over anhydrous Na₂SO₄, and concentrated under reduced pressure. The residue was purified by column chromatography to afford the title compound (white solid, 225 mg, crude), which was used directly without purification.

### Step F: N-(3-Carbamoyl-1-(4-(4-((1-(4-((2,4-dioxotetrahydropyrimidin-1(2H)-yl)methyl)-2-fluorophenyl)piperidin-4-yl)methyl)piperazin-1-yl)phenyl)-1H-pyrazol-4-yl)-6-(trifluoromethyl)picolinamide

At rt, TEA (0.07 mL, 0.48 mmol, 0.1 eq) was added to a solution of N-(3-carbamoyl-1-(4-(piperazin-1-yl)phenyl)-1H-pyrazol-4-yl)-6-(trifluoromethyl)picolinamide (265 mg, 0.58 mmol, 1.2 eq) in DMF (3 mL). After stirring at rt for 20 min, AcOH (0.03 mL, 0.48 mmol, 0.1 eq) and 1-(4-((2,4-dioxotetrahydropyrimidin-1(2H)-yl)methyl)-2-fluorophenyl)piperidine-4-carbaldehyde (160 mg, 0.48 mmol, 1.0 eq) were added to the mixture, and the reaction was stirred at rt for 30 min. Then sodium cyanoborohydride (60 mg, 0.96 mmol, 2.0 eq) was added to the mixture, and the reaction was stirred at rt for 2 h. The mixture was diluted in EtOAc, washed with water and aqueous NaCl, dried over anhydrous Na₂SO₄, filtered, and concentrated under reduced pressure to dryness. The residue was purified by HPLC to afford the title compound (white solid, 20 mg, yield 5%). LCMS (ESI): m/z 777 [M+H]⁺. **¹H NMR** (400 MHz, DMSO-d6) *δ* 11.79 (s, 1H), 10.19 (s, 1H), 8.88 (s, 1H), 8.47-8.37 (m, 2H), 8.21 (dd, *J =* 7.6, 1.0 Hz, 1H), 7.95 (s, 1H), 7.79 (d, J = 9.1 Hz, 2H), 7.71 (s, 1H), 7.10-6.98 (m, 5H), 4.43 (s, 2H), 3.37-3.34 (m, 2H), 3.28 (t, J = 6.8 Hz, 3H), 3.25-3.19 (m, 4H), 2.68-2.62 (m, 2H), 2.55-2.52 (m, 5H), 2.25 (d, J = 7.0 Hz, 2H), 1.83 (d, J = 12.5 Hz, 2H), 1.74-1.64 (m, 1H), 1.34-1.23 (m, 2H).

### Preparation Example 425. Synthesis of Compound A420

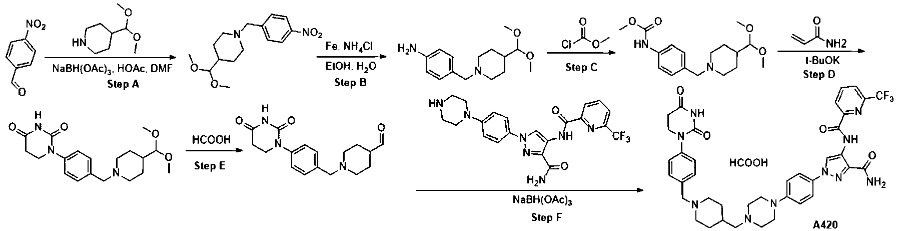

### Step A: 4-(Dimethoxymethyl)-1-(4-nitrobenzyl)piperidine

At 0°C, 4-nitrobenzaldehyde (5.0 g, 33.11 mmol, 1.0 eq) and 4-(dimethoxymethyl)piperidine (6.3 g, 39.73 mmol, 1.2 eq) were dissolved in DCM (50 mL), and the mixture was stirred for 1 h. Then sodium triacetoxyborohydride (14 g, 66.22 mmol, 2.0 eq) was added. The reaction solution was stirred at rt under N₂ for 2 h. Upon completion, the reaction solution was diluted in DCM, washed sequentially with water and saturated aqueous NaCl, dried over anhydrous Na₂SO₄, and concentrated under reduced pressure. The residue was purified by column chromatography to afford the title compound (yellow solid, 5 g, yield 51%).

### Step B: 4-((4-(Dimethoxymethyl)piperidin-1-yl)methyl)aniline

At rt, 4-(dimethoxymethyl)-1-(4-nitrobenzyl)piperidine (3.0 g, 10.2 mmol, 1.0 eq), iron powder (2.3 g, 40.8 mmol, 4.0 eq), and ammonium chloride (4.4 g, 81.63 mmol, 8.0 eq) were dissolved in water (10 mL) and ethanol (25 mL). The reaction solution was stirred at 60°C under N₂ for 2 h. Upon completion, the solution was filtered through a pad of Celite and extracted with EtOAc. The organic layer was washed sequentially with water and saturated aqueous NaCl, dried over anhydrous Na₂SO₄, and concentrated under reduced pressure to afford the title compound (yellow oil, 2.5 g, yield 93%).

### Step C: Methyl (4-((4-(dimethoxymethyl)piperidin-1-yl)methyl)phenyl)carbamate

At 0°C, to a solution of 4-((4-(dimethoxymethyl)piperidin-1-yl)methyl)aniline (1.0 g, 3.79 mmol, 1.0 eq) and pyridine (10 mL, 11.36 mmol, 3.0 eq) in DCM (10 mL) was added methyl chloroformate (0.6 mL, 7.58 mmol, 2.0 eq). The reaction solution was stirred at 0°C for 30 min under N₂. Upon completion, the reaction solution was diluted in DCM, washed sequentially with water and saturated aqueous NaCl, dried over anhydrous Na₂SO₄, and concentrated under reduced pressure to afford the title compound (yellow solid, 1.0 g, yield 82%).

### Step D: 1-(4-((4-(Dimethoxymethyl)piperidin-1-yl)methyl)phenyl)dihydropyrimidine-2,4(1H,3H)-dione

At rt, methyl (4-((4-(dimethoxymethyl)piperidin-1-yl)methyl)phenyl)carbamate (1.0 g, 3.11 mmol, 1.0 eq), acrylamide (0.26 g, 3.73 mmol, 1.2 eq), and potassium tert-butoxide (0.52 g, 4.67 mmol, 1.5 eq) were added to tert-butanol (10 mL). The reaction solution was stirred at 60°C under N₂ for 16 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 200 mg, yield 18%).

### Step E: 1-(4-(2,4-Dioxotetrahydropyrimidin-1(2H)-yl)benzyl)piperidine-4-carbaldehyde

At rt, 1-(4-((4-(dimethoxymethyl)piperidin-1-yl)methyl)phenyl)dihydropyrimidine-2,4(1H,3H)-dione (200 mg, 0.55 mmol, 1.0 eq) was dissolved in formic acid (4 mL). The reaction solution was stirred at 25°C under N₂ for 1 h. Upon completion, the mixture was concentrated under reduced pressure to afford the title compound (colorless oil, 170 mg, crude).

### Step F: N-(3-Carbamoyl-1-(4-(4-((1-(4-(2,4-dioxotetrahydropyrimidin-1(2H)-yl)benzyl)piperidin-4-yl)methyl)piperazin-1-yl)phenyl)-1H-pyrazol-4-yl)-6-(trifluoromethyl)picolinamide formate

At rt, to a solution of 1-(4-(2,4-dioxotetrahydropyrimidin-1(2H)-yl)benzyl)piperidine-4-carbaldehyde (144 mg, 0.46 mmol, 1.0 eq) and 1-[4-(piperazin-1-yl)phenyl]-4-({[6-(trifluoromethyl)pyridin-2-yl]carbonyl}amino)pyrazole-3-carboxamide (200 mg, 0.43 mmol, 1.0 eq) in DMF (10 mL) were added 5 drops of AcOH. The reaction solution was stirred at 25°C under N₂ for 1 h, and then sodium triacetoxyborohydride (543 mg, 1.72 mmol, 4.0 eq) was added. The reaction solution was stirred at 25°C under N₂ for 10 min. Upon completion, the reaction solution was diluted in EtOAc, washed sequentially with water and saturated aqueous NaCl, dried over anhydrous Na₂SO₄, and concentrated under reduced pressure. The residue was purified by high performance liquid chromatography to afford the title compound (white solid, 38 mg, yield 12%). LCMS (ESI): m/z 759 [M+H]⁺. **¹H NMR** (400 MHz, DMSO) δ 11.79 (s, 1H), 10.35 (s, 1H), 8.87 (s, 1H), 8.46-8.37 (m, 2H), 8.21 (d, *J* = 7.5 Hz, 1H), 8.15 (s, 1H), 7.94 (s, 1H), 7.78 (d, *J=* 9.0 Hz, 2H), 7.71 (s, 1H), 7.29 (q, *J* = 8.6 Hz, 4H), 7.07 (d, *J* = 9.1 Hz, 2H), 3.78 (t, *J* = 6.7 Hz, 2H), 3.49-3.46 (m, 2H), 3.28-3.12 (m, 8H), 2.83 (d, *J* = 11.1 Hz, 2H), 2.70 (t, *J* = 6.6 Hz, 2H), 2.21-2.17 (m, 2H), 2.02-1.93 (m, 2H), 1.73-1.67 (m, 2H), 1.59-1.50 (m, 1H), 1.19-1.10 (m, 2H).

### Preparation Example 426. Synthesis of Compound A421

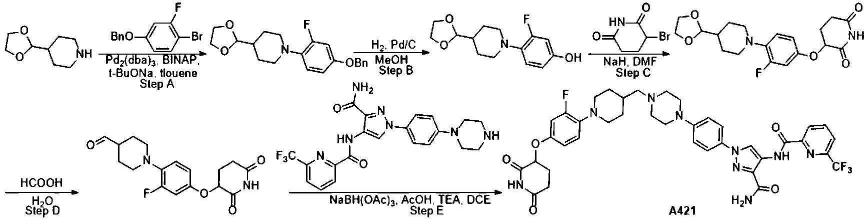

### Step A: 1-(4-(Benzyloxy)-2-fluorophenyl)-4-(1,3-dioxolan-2-yl)piperidine

At rt, to a solution of 4-(1,3-dioxolan-2-yl)piperidine (1 g, 6.36 mmol, 1.0 eq), 4-(benzyloxy)-1-bromo-2-fluorobenzene (2.7 g, 9.54 mmol, 1.5 eq), and sodium tert-butoxide (1.8 g, 19.08 mmol, 3.0 eq) in toluene (12 mL) were added Pd₂(dba)₃ (582 mg, 0.64 mmol, 0.1 eq) and BIANP (792 mg, 1.27 mmol, 0.2 eq). The mixture was degassed (×3) under N₂ and stirred at 110°C for 5 h. Upon completion, the mixture was diluted in EtOAc, washed sequentially with water and saturated aqueous NaCl, dried over anhydrous Na₂SO₄, and concentrated under reduced pressure. The residue was purified by column chromatography to afford the title compound (yellow solid, 2.0 g, yield 88%).

### Step B: 4-(4-(1,3-Dioxolan-2-yl)piperidin-1-yl)-3-fluorophenol

At rt, Pd/C (0.3 g, 5% wt) was added to a solution of 1-(4-(benzyloxy)-2-fluorophenyl)-4-(1,3-dioxolan-2-yl)piperidine (1.5 g, 4.20 mmol, 1.0 eq) in MeOH (20 mL). The reaction mixture was degassed (×3) under H₂ and stirred under H₂ at rt overnight. Upon completion, the reaction solution was filtered through Celite, and the filtrate was concentrated to afford the title compound (yellow solid, 1.1 g, crude).

### Step C: 3-(4-(4-(1,3-Dioxolan-2-yl)piperidin-1-yl)-3-fluorophenoxy)piperidine-2,6-dione

At 0°C, NaH (250 mg, 6.17 mmol, 1.5 eq, 60% dispersion in mineral oil) was added to a solution of 4-(4-(1,3-dioxolan-2-yl)piperidin-1-yl)-3-fluorophenol (1.1 g, crude) in DMF (11 mL). The reaction solution was stirred at 0°C for 30 min under N₂. Then, 3-bromopiperidine-2,6-dione (948 mg, 4.94 mmol, 1.2 eq) was added. The reaction solution was stirred at rt for 3 h. Upon completion, the reaction solution was quenched with saturated aqueous NH₄Cl and extracted with EtOAc. The organic phase was washed sequentially with water and saturated aqueous NaCl, dried over anhydrous Na₂SO₄, and concentrated under reduced pressure. The residue was purified by column chromatography to afford the title compound (white solid, 800 mg, yield 51% over two steps).

### Step D: 1-(4-((2,6-Dioxopiperidin-3-yl)oxy)-2-fluorophenyl)piperidine-4-carbaldehyde

At rt, 3-(4-(4-(1,3-dioxolan-2-yl)piperidin-1-yl)-3-fluorophenoxy)piperidine-2,6-dione (500 mg, 1.32 mmol, 1.0 eq) was added to water (3 mL) and formic acid (3 mL), and the reaction solution was stirred at 50°C for 6 h. Upon completion, the reaction solution was basified with saturated NaHCO₃ solution and extracted with EtOAc. The organic phase was washed sequentially with water and saturated aqueous NaCl, dried over anhydrous Na₂SO₄, and concentrated under reduced pressure to afford the title compound (yellow oil, 310 mg, crude).

### Step E: N-(3-Carbamoyl-1-(4-(4-((1-(4-((2,6-dioxopiperidin-3-yl)oxy)-2-fluorophenyl)piperidin-4-yl)methyl)piperazin-1-yl)phenyl)-1H-pyrazol-4-yl)-6-(trifluoromethyl)picolinamide

At rt, TEA (24 mg, 0.24 mmol, 1.0 eq) was added to a solution of N-(3-carbamoyl-1-(4-(piperazin-1-yl)phenyl)-1H-pyrazol-4-yl)-6-(trifluoromethyl)picolinamide (110 mg, 0.24 mmol, 1.0 eq) in 1,2-dichloroethane (4 mL). The mixture was stirred at rt for 5 min. Then AcOH (14 mg, 0.24 mmol, 1.0 eq) and 1-(4-((2,6-dioxopiperidin-3-yl)oxy)-2-fluorophenyl)piperidine-4-carbaldehyde (96 mg, crude) were added, and the mixture was further reacted for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 25.3 mg, yield 14%). LCMS (ESI): m/z 778 [M+H]⁺. **¹H NMR** (400 MHz, DMSO-*d₆*) *δ* 11.79 (s, 1H), 10.92 (s, 1H), 8.88 (s, 1H), 8.46-8.37 (m, 2H), 8.23-8.19 (m, 1H), 7.95 (s, 1H), 7.79 (d, J = 9.0 Hz, 2H), 7.71 (s, 1H), 7.08 (d, J = 9.1 Hz, 2H), 6.98 (t, J = 9.5 Hz, 1H), 6.92-6.87 (m, 1H), 6.79-6.75 (m, 1H), 5.15-5.10 (m, 1H), 3.30 (s, 1H), 3.25-3.19 (m, 6H), 2.74-2.59 (m, 4H), 2.58-2.53 (m, 3H), 2.27-2.22 (m, 2H), 2.20-2.07 (m, 2H), 1.85-1.79 (m, 2H), 1.72-1.63 (m, 1H), 1.35 -1.26 (m, 2H).

### Preparation Example 427. Synthesis of Compound A422

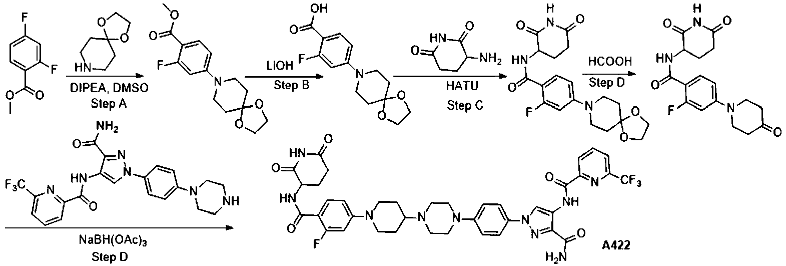

### Step A: Methyl 2-fluoro-4-(1,4-dioxa-8-azaspiro[4.5]decan-8-yl)benzoate

At 0°C, 1,4-dioxa-8-azaspiro[4.5]decane (4.2 g, 29.05 mmol, 1.0 eq) was added to a solution of methyl 2,4-difluorobenzoate (5 g, 29.05 mmol, 1.0 eq) in DMF (50 mL). The mixture was stirred at 0°C for 1 h. Then, DIEA (5 mL) was added, and the mixture was stirred at rt for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (brown solid, 5.0 g, yield 58%).

### Step B: 2-Fluoro-4-(1,4-dioxa-8-azaspiro[4.5]decan-8-yl)benzoic acid

At rt, methyl 2-fluoro-4-(1,4-dioxa-8-azaspiro[4.5]decan-8-yl)benzoate (5 g, 16.93 mmol, 1.0 eq) and lithium hydroxide monohydrate (1.4 g, 33.86 mmol, 2.0 eq) were added to a solution of THF (45 mL) and water (15 mL). The mixture was stirred at rt for 2 h. Upon completion, the reaction solution was concentrated under reduced pressure to afford the title compound (brown solid, 3 g, crude).

### Step C: N-(2,6-Dioxopiperidin-3-yl)-2-fluoro-4-(1,4-dioxa-8-azaspiro[4.5]decan-8-yl)benzamide

To a solution of 2-fluoro-4-(1,4-dioxa-8-azaspiro[4.5]decan-8-yl)benzoic acid (2 g, crude) in DMF (20 mL) were added 3-aminopiperidine-2,6-dione (0.9 g, 7.11 mmol, 1.0 eq), HATU (5.4 g, 14.22 mmol, 2.0 eq), and DIEA (2.8 g, 21.33 mmol, 3.0 eq). The reaction solution was stirred at rt under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (yellow solid, 1.0 g, yield 36%).

### Step D: N-(2,6-Dioxopiperidin-3-yl)-2-fluoro-4-(4-oxopiperidin-1-yl)benzamide

At rt, a solution of N-(2,6-dioxopiperidin-3-yl)-2-fluoro-4-(1,4-dioxa-8-azaspiro[4.5]decan-8-yl)benzamide (1 g, 2.56 mmol, 1.0 eq) was dissolved in formic acid (15 mL), and the mixture was stirred at 80°C for 12 h. Upon completion, the reaction solution was concentrated under reduced pressure to afford the title compound (brown solid, 350 mg, crude),

### Step E: N-(3-Carbamoyl-1-(4-(4-(1-(4-((2,6-dioxopiperidin-3-yl)carbamoyl)-3-fluorophenyl)piperidin-4-yl)piperazin-1-yl)phenyl)-1H-pyrazol-4-yl)-6-(trifluoromethyl)picolinamide

At rt, to a solution of N-(2,6-dioxopiperidin-3-yl)-2-fluoro-4-(4-oxopiperidin-1-yl)benzamide (350 mg, crude) in DMF (10 mL) were added N-(3-carbamoyl-1-(4-(piperazin-1-yl)phenyl)-1H-pyrazol-4-yl)-6-(trifluoromethyl)picolinamide (463 mg, 1.01 mmol, 1.0 eq) and AcOH (0.012 mL, 0.20 mmol, 0.5 eq). The reaction solution was stirred at rt under N₂ for 2 h. Then, NaBH(OAc)3 (854 mg, 4.03 mmol, 4.0 eq) was added. Stirring was continued for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (yellow solid, 13.2 mg, yield 2%). **¹H NMR** (400 MHz, DMSO-d6) δ 11.81-11.77 (m, 1H), 10.85 (s, 1H), 8.93 (s, 1H), 8.46-8.38 (m, 2H), 8.22 (dd, *J* = 7.5, 1.1 Hz, 1H), 8.09-8.03 (m, 1H), 7.97-7.85 (m, 3H), 7.75 (s, 1H), 7.66 (t, *J =* 9.2 Hz, 1H), 7.18 (d, *J* = 9.2 Hz, 2H), 6.93-6.84 (m, 2H), 4.78-4.68 (m, 1H), 4.05 (dd, *J* = 51.4, 12.5 Hz, 4H), 3.71-3.63 (m, 2H), 3.30-3.16 (m, 3H), 3.09-3.00 (m, 2H), 2.96-2.86 (m, 2H), 2.82-2.71 (m, 1H), 2.55-2.52 (m, 1H), 2.19-2.14 (m, 2H), 2.11-1.95 (m, 2H), 1.75-1.61 (m, 2H). LCMS (ESI): m/z 792 [M+H]⁺.

### Preparation Example 428. Synthesis of Compound A423

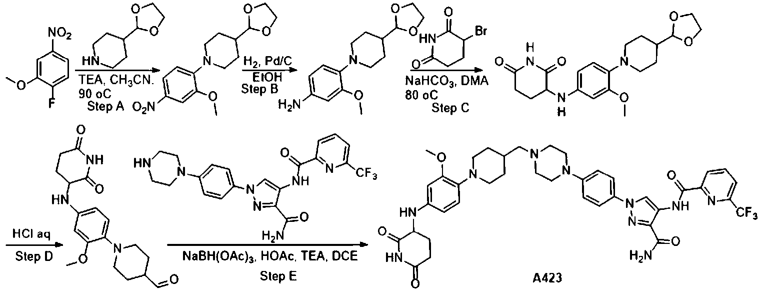

### Step A: 4-(1,3-Dioxolan-2-yl)-1-(2-methoxy-4-nitrophenyl)piperidine

At rt, to a solution of 4-(1,3-dioxolan-2-yl)piperidine (1.1 g, 7.01 mmol, 1.0 eq) and 1-fluoro-2-methoxy-4-nitrobenzene (1.56 g, 9.11 mmol, 1.3 eq) in acetonitrile (15 mL) was added TEA (2.12 g, 21.03 mmol, 3.0 eq). The reaction solution was stirred at 90°C overnight. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 1.4 g, yield 65%).

### Step B: 4-(4-(1,3-Dioxolan-2-yl)piperidin-1-yl)-3-methoxyaniline

Under N₂, 10% wet palladium on carbon (200 mg) was added to a solution of 4-(1,3-dioxolan-2-yl)-1-(2-methoxy-4-nitrophenyl)piperidine (1.4 g, 4.55 mmol, 1.0 eq) in ethanol (20 mL). The mixture was degassed (×3) under H₂ and stirred under H₂ overnight. Upon completion, the mixture was filtered, and the filtrate was concentrated to afford the title compound (brown solid, 1.0 g, yield 79%).

### Step C: 3-((4-(4-(1,3-Dioxolan-2-yl)piperidin-1-yl)-3-methoxyphenyl)amino)piperidine-2,6-dione

At rt, to a solution of 4-(4-(1,3-dioxolan-2-yl)piperidin-1-yl)-3-methoxyaniline (500 mg, 1.80 mmol, 1.0 eq) and 3-bromopiperidine-2,6-dione (687 mg, 3.60 mmol, 2.0 eq) in N,N-dimethylacetamide (10 mL) was added NaHCO₃ (453 mg, 5.40 mmol, 3.0 eq). The reaction solution was stirred at 80°C overnight. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (brown solid, 350 mg, yield 50%).

### Step D: 1-(4-((2,6-Dioxopiperidin-3-yl)amino)-2-methoxyphenyl)piperidine-4-carbaldehyde

At rt, dilute HCl (4 mL, 1N) was added to a solution of 3-((4-(4-(1,3-dioxolan-2-yl)piperidin-1-yl)-3-methoxyphenyl)amino)piperidine-2,6-dione (350 mg, 0.90 mmol, 1.0 eq) in THF (4 mL). The reaction solution was stirred at 40°C overnight. Upon completion, the reaction solution was neutralized with saturated aqueous NaHCO₃, diluted in EtOAc, washed sequentially with water and saturated aqueous NaCl, dried over anhydrous Na₂SO₄, and concentrated under reduced pressure to afford the title compound (brown solid, 150 mg, yield 48%).

### Step E: N-(3-Carbamoyl-1-(4-(4-((1-(4-((2,6-dioxopiperidin-3-yl)amino)-2-methoxyphenyl)piperidin-4-yl)methyl)piperazin-1-yl)phenyl)-1H-pyrazol-4-yl)-6-(trifluoromethyl)picolinamide

To a solution of N-(3-carbamoyl-1-(4-(piperazin-1-yl)phenyl)-1H-pyrazol-4-yl)-6-(trifluoromethyl)picolinamide (100 mg, 0.22 mmol, 1.0 eq) in 1,2-dichloroethane (2 mL) was added TEA (22 mg, 0.33 mmol, 1.0 eq), and the mixture was stirred at rt for 10 min. AcOH (13 mg, 0.33 mmol, 1.0 eq) and 1-(4-((2,6-dioxopiperidin-3-yl)amino)-2-methoxyphenyl)piperidine-4-carbaldehyde (150 mg, 0.44 mmol, 2.0 eq) were then added, and the mixture was stirred at rt for 1 h. NaBH(OAc)3 (277 mg, 1.32 mmol, 6.0 eq) was then added, and the mixture was stirred overnight. Upon completion, the reaction solution was diluted in EtOAc, washed sequentially with water and saturated aqueous NaCl, and dried over anhydrous Na₂SO₄. The residue was purified by HPLC to afford the title compound (gray solid, 12 mg, yield 7%). LCMS (ESI): m/z 789 [M+H]⁺. **¹H NMR** (400 MHz, CD3OD-*d4*) δ 8.87 (s, 1H), 8.47 (d, *J* = 7.8 Hz, 1H), 8.30 (t, *J =* 7.8 Hz, 1H), 8.05 (d, *J* = 7.9 Hz, 1H), 7.74 (d, *J* = 9.1 Hz, 2H), 7.12 (d, *J* = 9.1 Hz, 2H), 6.89 (d, *J =* 8.5 Hz, 1H), 6.46 (d, *J =* 2.4 Hz, 1H), 6.34-6.26 (m, 1H), 4.29-4.23 (m, 1H), 3.85 (s, 3H), 3.51-3.46 (m, 1H), 3.22-2.99 (m, 1H), 2.87-2.73 (m, 2H), 2.69-2.63 (m, 4H), 2.63-2.54 (m, 2H), 2.40-2.31 (m, 3H), 1.97-1.85 (m, 3H), 1.78-1.67 (m, 1H), 1.65-1.36 (m, 3H), 1.34-1.26 (m, 3H).

### Preparation Example 429. Synthesis of Compound A424

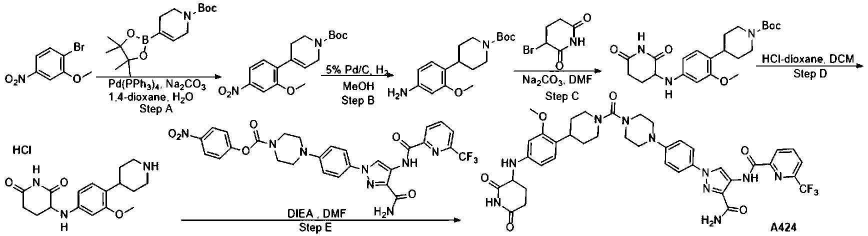

### Step A: Tert-butyl 4-(2-methoxy-4-nitrophenyl)-3,6-dihydropyridine-1(2H)-carboxylate

To a solution of 1-bromo-2-methoxy-4-nitrobenzene (2.5 g, 10.9 mmol, 1.0 eq) in dioxane (30 mL) and water (10 mL) were added tert-butyl 4-(4,4,5,5-tetramethyl-1,3,2-dioxaborolan-2-yl)-3,6-dihydropyridine-1(2H)-carboxylate (6.7 g, 16.3 mmol, 1.5 eq), Pd(PPh₃)₄ (1.3 g, 1.08 mmol, 0.05 eq), and Na2CO3 (4.5 g, 32.6 mmol, 3.0 eq). The reaction mixture was degassed under N₂ (×3), and then stirred under N₂ at 80°C for 18 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 3.5 g, yield 96%).

### Step B: Tert-butyl 4-(4-amino-2-methoxyphenyl)piperidine-1-carboxylate

To a solution of tert-butyl 4-(2-methoxy-4-nitrophenyl)-3,6-dihydropyridine-1(2H)-carboxylate (600 mg, 1.79 mmol, 1.0 eq) in MeOH (6 mL) was added 5% wet palladium on carbon (200 mg). The reaction mixture was degassed under H₂ (×3), and then stirred under H₂ at 50°C for 18 h. Upon completion, the reaction solution was filtered through Celite, and the filtrate was concentrated under reduced pressure to afford the title compound (yellow solid, 500 mg, crude).

### Step C: Tert-butyl 4-(4-((2,6-dioxopiperidin-3-yl)amino)-2-methoxyphenyl)piperidine-1-carboxylate

To a solution of tert-butyl 4-(4-amino-2-methoxyphenyl)piperidine-1-carboxylate (500 mg, crude) and 3-bromopiperidine-2,6-dione (470 mg, 2.46 mmol, 1.5 eq) in DMF (5 mL) was added sodium carbonate (521 mg, 4.92 mmol, 3.0 eq). The mixture was stirred at 100°C for 18 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 200 mg, yield 29% over two steps).

### Step D: 3-((3-Methoxy-4-(piperidin-4-yl)phenyl)amino)piperidine-2,6-dione hydrochloride

A 4 M hydrogen chloride-dioxane (2 mL) was added to a solution of tert-butyl 4-(4-((2,6-dioxopiperidin-3-yl)amino)-2-methoxyphenyl)piperidine-1-carboxylate (200 mg, 0.48 mmol, 1.0 eq) in DCM (2 mL). The reaction solution was stirred at rt for 2 h. Upon completion, the reaction solution was concentrated under reduced pressure to afford the title compound (yellow solid, 150 mg, crude),

### Step E: N-(3-Carbamoyl-1-(4-(4-(4-((2,6-dioxopiperidin-3-yl)amino)-2-methoxyphenyl)piperidine-1-carbonyl)piperazin-1-yl)phenyl)-1H-pyrazol-4-yl)-6-(trifluoromethyl)picolinamide

To a solution of 4-nitrophenyl 4-(4-(3-carbamoyl-4-(6-(trifluoromethyl)picolinamido)-1H-pyrazol-1-yl)phenyl)piperazine-1-carboxylate (80 mg, 0.13 mmol, 1.0 eq) in DMF (3 mL) were added 3-((3-methoxy-4-(piperidin-4-yl)phenyl)amino)piperidine-2,6-dione hydrochloride (90 mg, crude) and DIEA (83 mg, 0.65 mmol, 5.0 eq). The reaction solution was stirred at 90°C under N₂ for 18 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (yellow solid, 32 mg, yield 31%). LCMS (ESI): m/z 803 [M+H]⁺. **¹H NMR** (400 MHz, DMSO-*d₆*) δ 11.79 (s, 1H), 10.77 (s, 1H), 8.89 (s, 1H), 8.43 (dd, J = 17.1, 7.7 Hz, 2H), 8.21 (d, J = 7.7 Hz, 1H), 7.96 (s, 1H), 7.82 (d, J = 9.0 Hz, 2H), 7.71 (s, 1H), 7.11 (d, J = 9.2 Hz, 2H), 6.86 (d, J = 8.3 Hz, 1H), 6.35 (d, J = 2.0 Hz, 1H), 6.22 (d, J = 8.5 Hz, 1H), 5.71 (d, J = 7.4 Hz, 1H), 4.31 (dd, J = 14.0, 10.0 Hz, 1H), 3.75 (d, J = 14.2 Hz, 2H), 3.72 (s, 3H), 3.24 (d, J = 3.5 Hz, 4H), 2.95-2.51 (m, 9H), 2.15-2.08 (m, 1H), 1.91-1.84 (m, 1H), 1.66 (dd, J = 13.4, 1.9 Hz, 2H), 1.56-1.46 (m, 2H).

### Preparation Example 430. Synthesis of Compound A425

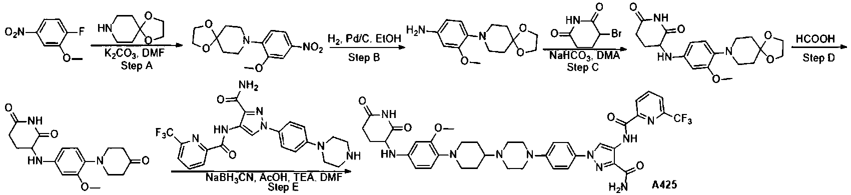

### Step A: 8-(2-Methoxy-4-nitrophenyl)-1,4-dioxa-8-azaspiro[4.5]decane

At rt, to a solution of 1-fluoro-2-methoxy-4-nitrobenzene (1.5 g, 8.77 mmol, 1.0 eq) and 1,4-dioxa-8-azaspiro[4.5]decane (1.51 g, 10.5 mmol, 1.2 eq) in DMF (15 mL) was added potassium carbonate (1.82 g, 13.2 mmol, 1.5 eq). The mixture was stirred at 60°C overnight. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 1.7 g, yield 68%).

### Step B: 3-Methoxy-4-(1,4-dioxa-8-azaspiro[4.5]decan-8-yl)aniline

At rt, to a solution of 8-(2-methoxy-4-nitrophenyl)-1,4-dioxa-8-azaspiro[4.5]decane (1.7 g, 5.78 mmol, 1.0 eq) in ethanol (20 mL) was added 10% wet palladium on carbon (0.3 g). The mixture was stirred under H₂ at rt overnight. Upon completion, the mixture was filtered, and the filtrate was concentrated to dryness to afford the title compound (yellow solid, 1.2 g, yield 79%).

### Step C: 3-((3-Methoxy-4-(1,4-dioxa-8-azaspiro[4.5]decan-8-yl)phenyl)amino)piperidine-2,6-dione

At rt, to a solution of 3-methoxy-4-(1,4-dioxa-8-azaspiro[4.5]decan-8-yl)aniline (1.2 g, 4.54 mmol, 1.0 eq) and 3-bromopiperidine-2,6-dione (1.05 g, 5.45 mmol, 1.2 eq) in N,N-dimethylacetamide (12 mL) was added NaHCO₃ (763 mg, 9.08 mmol, 2 eq). The mixture was stirred at 80°C overnight. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (brown solid, 320 mg, yield 19%).

### Step D: 3-((3-Methoxy-4-(4-oxopiperidin-1-yl)phenyl)amino)piperidine-2,6-dione

A solution of 3-((3-methoxy-4-(1,4-dioxa-8-azaspiro[4.5]decan-8-yl)phenyl)amino)piperidine-2,6-dione (320 mg, 0.85 mmol, 1.0 eq) in water (0.5 mL) and formic acid (5 mL) was stirred at rt for 48 h. The mixture was neutralized with saturated aqueous NaHCO₃ and extracted with EtOAc. The organic layer was washed with aqueous NaCl, dried over anhydrous Na₂SO₄, and concentrated under reduced pressure. The residue was purified by column chromatography to afford the title compound (yellow oil, 60 mg, yield 21%).

### Step E: N-(3-Carbamoyl-1-(4-(4-(1-(4-((2,6-dioxopiperidin-3-yl)amino)-2-methoxyphenyl)piperidin-4-yl)piperazin-1-yl)phenyl)-1H-pyrazol-4-yl)-6-(trifluoromethyl)picolinamide

At rt, to a solution of N-(3-carbamoyl-1-(4-(piperazin-1-yl)phenyl)-1H-pyrazol-4-yl)-6-(trifluoromethyl)picolinamide (83 mg, 0.18 mmol, 1.0 eq) and 3-((3-methoxy-4-(4-oxopiperidin-1-yl)phenyl)amino)piperidine-2,6-dione (60 mg, 0.18 mmol, 1.0 eq) in DMF (3 mL) was added TEA (18 mg, 0.18 mmol, 1.0 eq). After stirring for 10 min, AcOH (11 mg, 0.18 mmol, 1.0 eq) was added. After stirring for an additional 30 min, sodium cyanoborohydride (17 mg, 0.27 mmol, 1.5 eq) was added. The reaction solution was stirred at rt under N₂ for 4 h. Upon completion, the reaction was quenched with water and extracted with EtOAc. The organic phases were combined, washed with saturated aqueous NaCl, dried over anhydrous Na₂SO₄, filtered, and concentrated. The residue was purified by HPLC to afford the title compound (gray solid, 20.5 mg, yield 15%). LCMS (ESI): m/z 775 [M+H]⁺. **¹H NMR** (400 MHz, DMSO-*d*₆) δ 11.79 (s, 1H), 10.76 (s, 1H), 8.88 (s, 1H), 8.51-8.32 (m, 2H), 8.21 (d, *J* = 7.8 Hz, 1H), 7.95 (s, 1H), 7.79 (d, *J =* 9.0 Hz, 2H), 7.71 (s, 1H), 7.09 (d, *J*= 9.1 Hz, 2H), 6.69 (d, *J* = 8.5 Hz, 1H), 6.37 (s, 1H), 6.17 (d, *J =* 6.8 Hz, 1H), 5.51 (d, *J =* 7.4 Hz, 1H), 4.31-4.18 (m, 1H), 3.72 (s, 3H), 3.25-3.20 (m, 8H), 2.80-2.65 (m, 5H), 2.62-2.56 (m, 1H), 2.40-2.32 (m, 1H), 2.11 (d, *J* = 8.4 Hz, 1H), 1.91-1.79 (m, 3H), 1.64-1.52 (m, 2H).

### Preparation Example 431. Synthesis of Compound A426-Int

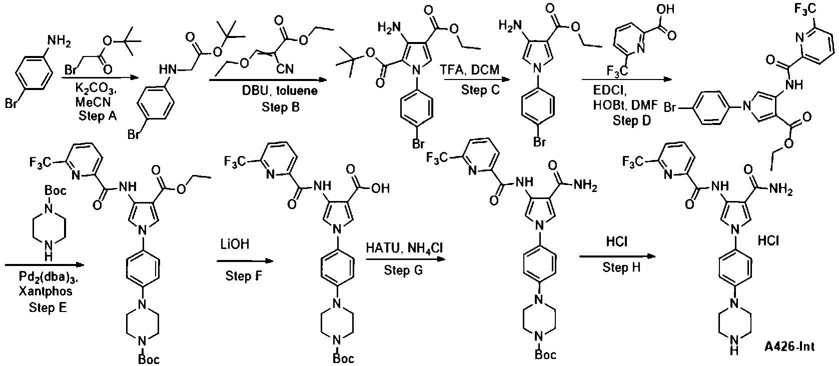

### Steps A-D: Ethyl 1-(4-bromophenyl)-4-(6-(trifluoromethyl)pyridinylamino)-1H-pyrrole-3-carboxylate

The synthesis of ethyl 1-(4-bromophenyl)-4-(6-(trifluoromethyl)pyridinylamino)-1H-pyrrole-3-carboxylate was carried out according to the method described for A47 (Steps A-D). The title compound was obtained after purification by column chromatography (yellow solid, 4 g, yield 5% over four steps).

### Step E: Tert-butyl 4-(4-(3-(ethoxycarbonyl)-4-(6-(trifluoromethyl)pyridinylamino)-1H-pyrrol-1-yl)phenyl)piperazine-1-carboxylate

Under N₂, to a solution of ethyl 1-(4-bromophenyl)-4-(6-(trifluoromethyl)pyridinylamino)-1H-pyrrole-3-carboxylate (1.0 g, 2.1 mmol, 1.0 eq) and tert-butyl piperazine-1-carboxylate (0.42 g, 2.3 mmol, 1.1 eq) in dioxane (15 mL) were added Pd₂(dba)₃ (0.19 g, 0.2 mmol, 0.1 eq), Xantphos (0.24 g, 0.4 mmol, 0.2 eq), and Cs2CO3 (2 g, 6.2 mmol, 3.0 eq). The reaction solution was stirred at 90°C for 5 h. The mixture was filtered through Celite, and the filtrate was concentrated in vacuo to obtain a residue. The residue was purified by column chromatography to afford the title compound (yellow solid, 0.74 g, yield 61%).

### Steps F-G: Tert-butyl 4-(4-(3-carbamoyl-4-(6-(trifluoromethyl)pyridinylamino)-1H-pyrrol-1-yl)phenyl)piperazine-1-carboxylate

The synthesis of tert-butyl 4-(4-(3-carbamoyl-4-(6-(trifluoromethyl)pyridinylamino)-1H-pyrrol-1-yl)phenyl)piperazine-1-carboxylate was carried out according to the method described for A47 (Steps E & F). The title compound was obtained after purification by column chromatography (yellow solid, 520 mg, yield 70% over two steps).

### Step H: N-(4-Carbamoyl-1-(4-(piperazin-1-yl)phenyl)-1H-pyrrol-3-yl)-6-(trifluoromethyl)picolinamide hydrochloride

Tert-butyl 4-(4-(3-carbamoyl-4-(6-(trifluoromethyl)pyridinylamino)-1H-pyrrol-1-yl)phenyl)piperazine-1-carboxylate (520 mg, 0.9 mmol, 1.0 eq) was dissolved in 4 N HCl-dioxane (10 mL), and the reaction was stirred for 2 h. The reaction solution was concentrated in vacuo to afford the title compound (yellow solid, 450 mg, yield 96%).

### Preparation Example 432. Synthesis of Compound A426

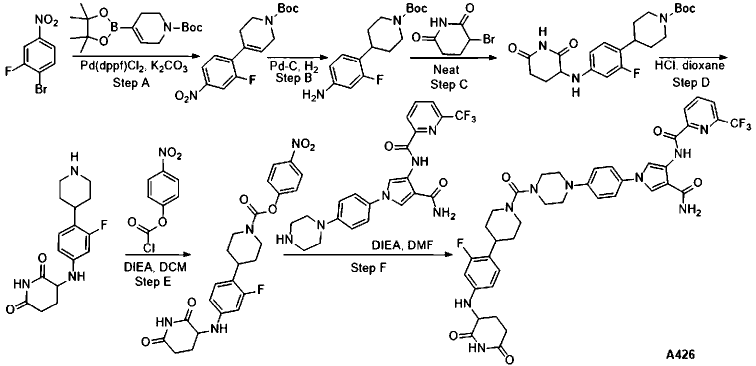

### Step A: Tert-butyl 4-(2-fluoro-4-nitrophenyl)-3,6-dihydropyridine-1(2H)-carboxylate

Under N₂, compound 1-bromo-2-fluoro-4-nitrobenzene (5.0 g, 22.7 mmol, 1.0 eq) and tert-butyl 4-(4,4,5,5-tetramethyl-1,3,2-dioxaborolan-2-yl)-3,6-dihydropyridine-1(2H)-carboxylate (8.4 g, 27.27 mmol, 1.2 eq) were dissolved in dioxane (50 mL) and water (50 mL), and to this system were added Pd(dppf)Cl₂ (1.7 g, 2.27 mmol, 0.1 eq) and K₂CO₃ (9.4 g, 68.19 mmol, 3.0 eq). The mixture was stirred at 90°C for 3 h. The reaction mixture was filtered through Celite. water (80 mL) was added to the filtrate, and the mixture was extracted with EtOAc (50 mL×3). The combined organic layers were washed with saturated aqueous NaCl, dried over anhydrous Na₂SO₄, and concentrated in vacuo to give the crude. The residue was purified by column chromatography to afford the title compound (yellow solid, 3 g, yield 41%).

### Step B: Tert-butyl 4-(4-amino-2-fluorophenyl)piperidine-1-carboxylate

Compound tert-butyl 4-(2-fluoro-4-nitrophenyl)-3,6-dihydropyridine-1(2H)-carboxylate (3.0 g, 9.32 mmol, 1.0 eq) was dissolved in EtOH (30.0 mL), and Pd/C (1 g, *w.t*.=10%) was added. Under H₂, the reaction solution was stirred at rt for 16 h. The reaction mixture was filtered through Celite, and the filtrate was concentrated in vacuo to afford the title compound (yellow solid, 2.0 g, yield 73%).

### Step C: Tert-butyl 4-(4-((2,6-dioxopiperidin-3-yl)amino)-2-fluorophenyl)piperidine-1-carboxylate

Compound tert-butyl 4-(4-amino-2-fluorophenyl)piperidine-1-carboxylate (2.0 g, 6.8 mmol, 1.0 eq) was dissolved in dioxane (5.0 mL), and to this system were added 3-bromopiperidine-2,6-dione (3.91 g, 20.41 mmol, 3.0 eq) and DIEA (2.4 mL, 13.6 mmol, 2.0 eq). The mixture was stirred at 90°C for 16 h. water (30 mL) was added to the reaction system, and the mixture was extracted with EtOAc (30 mL×3). The combined organic layers were washed with saturated aqueous NaCl, dried over anhydrous Na₂SO₄, and concentrated in vacuo to give the crude. The crude was purified by column chromatography to afford the title compound (yellow solid, 3.0 g, yield 41%).

### Step D: 3-((3-Fluoro-4-(piperidin-4-yl)phenyl)amino)piperidine-2,6-dione

Compound tert-butyl 4-(4-((2,6-dioxopiperidin-3-yl)amino)-2-fluorophenyl)piperidine-1-carboxylate (873 mg, 2.15 mmol, 1.0 eq) was dissolved in 4 N HCl in dioxane (8.0 mL), and the mixture was stirred at rt for 1 h. The reaction solution was concentrated in vacuo to afford the title compound (green solid, 657 mg, crude).

### Step E: 4-Nitrophenyl 4-(4-((2,6-dioxopiperidin-3-yl)amino)-2-fluorophenyl)piperidine-1-carboxylate

The compound 3-((3-fluoro-4-(piperidin-4-yl)phenyl)amino)piperidine-2,6-dione (360 mg, 1.18 mmol, 1.0 eq) was dissolved in DCM (5 mL). To this system were added DIEA (457.14 mg, 3.54 mmol, 3.0 eq) and 4-nitrophenyl carbonochloridate (356.45 mg, 1.77 mmol, 1.5 eq) in an ice bath. The reaction system was stirred at 30°C for 0.5 h. The reaction mixture was poured into water and extracted with DCM (50 mL×2). The combined organic layers were washed with aqueous NaCl, dried over anhydrous Na₂SO₄, and concentrated. The residue was purified by column chromatography to afford the title compound (green solid, 550 mg, yield 99%).

### Step F: N-(4-Carbamoyl-1-(4-(4-(4-(4-(4-(2,6-dioxopiperidin-3-yl)amino)-2-fluorophenyl)piperidine-1-carbonyl)piperazin-1-yl)phenyl)-1H-pyrrol-3-yl)-6-(trifluoromethyl)picolinamide

Compound 4-nitrophenyl 4-(4-((2,6-dioxopiperidin-3-yl)amino)-2-fluorophenyl)piperidine-1-carboxylate (100 mg, 0.21 mmol, 1.0 eq) was dissolved in DMF (2 mL), and to this system were added N-(4-carbamoyl-1-(4-(piperazin-1-yl)phenyl)-1H-pyrrol-3-yl)-6-(trifluoromethyl)picolinamide (194.89 mg, 0.43 mmol, 2.0 eq), and DIEA (137.37 mg, 1.06 mmol, 5.0 eq). The reaction system was stirred at 100°C for 48 h. The title compound was isolated by preparative HPLC (yellow solid, 31.2 mg, yield 18%). LCMS (ESI): m/z 790.3 [M+H]⁺. **¹H NMR** (400 MHz, DMSO-*d6*) δ 12.23 (s, 1H), 10.79 (s, 1H), 8.42-8.33 (m, 2H), 8.16 (d, *J* = 7.4 Hz, 1H), 7.96 (d, *J* = 2.2 Hz, 1H), 7.89 (d, *J* = 2.2 Hz, 1H), 7.64 (s, 1H), 7.43 (d, *J* = 8.8 Hz, 2H), 7.25 (s, 1H), 7.12 (d, *J* = 8.9 Hz, 2H), 7.00 (t, *J* = 8.8 Hz, 1H), 6.51-6.38 (m, 2H), 4.31 (dd, *J* = 11.5, 4.7 Hz, 1H), 3.76 (d, *J =* 12.4 Hz, 2H), 3.32 (s, 4H), 3.21 (s, 4H), 2.90-2.69 (m, 4H), 2.59 (s, 1H), 2.12-2.02 (m, 1H), 1.92-1.82 (m, 1H), 1.71-1.53 (m, 4H).

### Preparation Example 433. Synthesis of Compound A427

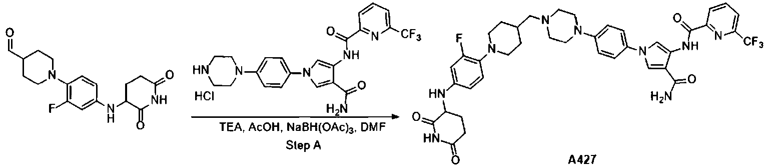

### Step A: N-(4-Carbamoyl-1-(4-(4-((1-(4-((2,6-Dioxopiperidin-3-yl)amino)-2-fluorophenyl)piperidin-4-yl)methyl)piperazin-1-yl)phenyl)-1H-pyrrol-3-yl)-6-(trifluoromethyl)picolinamide

Compound 1-(4-((2,6-dioxopiperidin-3-yl)amino)-2-fluorophenyl)piperidine-4-carbaldehyde (60 mg, 0.18 mmol, 1.0 eq) and N-(4-carbamoyl-1-(4-(piperazin-1-yl)phenyl)-1H-pyrrol-3-yl)-6-(trifluoromethyl)picolinamide hydrochloride (90 mg, 0.18 mmol, 1.0 eq) were dissolved in DMF (5 mL), and to this system was added two drops of TEA to maintain a basic system. After stirring for 5 min, HOAc (0.1 mL, 0.36 mmol, 2.0 eq) was added. After 30 min, NaBH(OAc)₃ (152 mg, 0.72 mmol, 4.0 eq) was added to the reaction system in portions. After 16 h, water (20 mL) was added to the reaction system, and the mixture was extracted with EtOAc (20 mL×3). The combined organic layers were washed with saturated aqueous NaCl, dried over anhydrous Na₂SO₄, and concentrated in vacuo to give the crude. The crude was purified by preparative HPLC to afford the title compound (white solid, 42 mg, yield 30%). LCMS (ESI): m/z 776.2 [M+H]⁺. **¹H NMR** (400 MHz, DMSO-*d*6) δ 12.23 (s, 1H), 10.77 (s, 1H), 8.48-8.29 (m, 2H), 8.16 (dd, *J* = 7.5, 1.1 Hz, 1H), 7.94 (d, *J* = 2.5 Hz, 1H), 7.88 (d, *J* = 2.4 Hz, 1H), 7.64 (s, 1H), 7.46-7.36 (m, 2H), 7.25 (s, 1H), 7.13-7.03 (m, 2H), 6.91-6.79 (m, 1H), 6.50 (dd, *J* = 14.9, 2.5 Hz, 1H), 6.42 (dd, *J* = 8.8, 2.3 Hz, 1H), 5.78 (d, *J* = 7.6 Hz, 1H), 4.33-4.18 (m, 1H), 3.25-3.15 (m, 4H), 3.12 (d, *J* = 11.0 Hz, 2H), 2.77-2.67 (m, 1H), 2.60-2.51 (m, 7H), 2.29-2.19 (m, 2H), 2.12-2.05 (m, 1H), 1.90-1.75 (m, 3H), 1.68-1.57 (m, 1H), 1.33-1.22 (m, 2H).

### Preparation Example 434. Synthesis of Compound A428

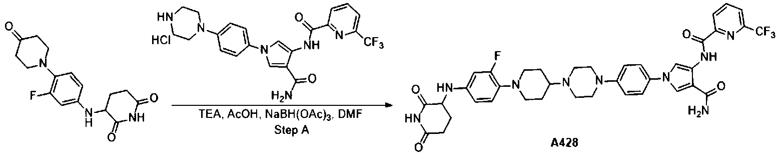

### Step A: N-(4-Carbamoyl-1-(4-(4-(1-(4-((2,6-Dioxopiperidin-3-yl)amino)-2-fluorophenyl)piperidin-4-yl)piperazin-1-yl)phenyl)-1H-pyrrol-3-yl)-6-(trifluoromethyl)picolinamide

The synthesis of the compound, N-(4-carbamoyl-1-(4-(4-(1-(4-(4-(2,6-dioxopiperidin-3-yl)amino)-2-fluorophenyl)piperidin-4-yl)piperazin-1-yl)phenyl)-1H-pyrrol-3-yl)-6-(trifluoromethyl)picolinamide, was performed according to the method described for A427. The title compound was obtained by preparative HPLC (yellow solid, 22 mg, yield 13%). LCMS (ESI): m/z 762.2 [M+H]⁺. **¹H NMR** (400 MHz, DMSO-*d*6) δ 12.23 (s, 1H), 10.77 (s, 1H), 8.47-8.33 (m, 2H), 8.16 (dd, *J =* 7.5, 1.2 Hz, 1H), 7.94 (d, *J* = 2.5 Hz, 1H), 7.88 (d, *J* = 2.4 Hz, 1H), 7.64 (s, 1H), 7.43-7.38 (m, 2H), 7.25 (s, 1H), 7.12-7.06 (m, 2H), 6.84 (t, *J* = 9.4 Hz, 1H), 6.56-6.47 (m, 1H), 6.42 (d, *J =* 8.3 Hz, 1H), 5.79 (d, *J =* 7.7 Hz, 1H), 4.33-4.17 (m, 1H), 3.22-3.15 (m, 6H), 2.76-2.67 (m, 5H), 2.62-2.54 (m, 3H), 2.35-2.29 (m, 1H), 2.13-2.04 (m, 1H), 1.92-1.80 (m, 3H), 1.67-1.54 (m, 2H).

### Preparation Example 435. Synthesis of Compound A429

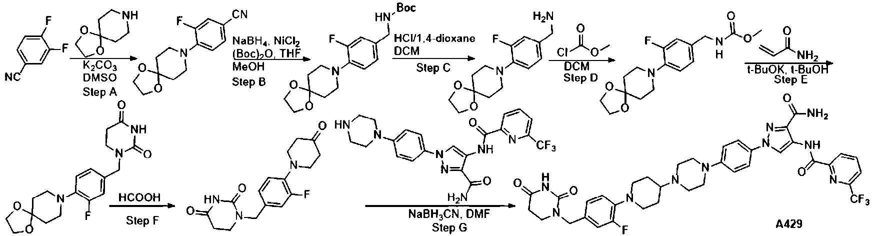

### Step A: 3-Fluoro-4-(1,4-dioxa-8-azaspiro[4.5]decan-8-yl)benzonitrile

Under N₂, to a mixture of 3,4-difluorobenzonitrile (10 g, 71.9 mmol, 1.0 eq) and 1,4-dioxa-8-azaspiro[4.5]decane (10 g, 69.9 mmol, 1.02 eq) in DMSO (100 mL) was added potassium carbonate (30.2 g, 211 mmol, 3.02 eq). The mixture was degassed (×3) under N₂ and stirred at 60°C for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 13 g, yield 71%).

### Step B: Tert-butyl (3-fluoro-4-(1,4-dioxa-8-azaspiro[4.5]decan-8-yl)benzyl)carbamate

Under N₂, to a mixed solution of 3-fluoro-4-(1,4-dioxa-8-azaspiro[4.5]decan-8-yl)benzonitrile (14 g, 53.4 mmol, 1.0 eq) in THF (30 mL) and MeOH (40 mL) were added di-tert-butyl dicarbonate (23.2 g, 107 mmol, 2.00 eq), nickel chloride (12.7 g, 53.4 mmol, 1.0 eq), and sodium borohydride (20.2 g, 534 mmol, 10 eq). The mixture was stirred at 25°C for 7 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 7.0 g, yield 36%).

### Step C: (3-Fluoro-4-(1,4-dioxa-8-azaspiro[4.5]decan-8-yl)phenyl)methanamine

To a solution of tert-butyl (3-fluoro-4-(1,4-dioxa-8-azaspiro[4.5]decan-8-yl)benzyl)carbamate (7.0 g, 19.1 mmol, 1.0 eq) in DCM (50 mL) was added hydrogen chloride in 1,4-dioxane (50 mL). The reaction solution was stirred at 25°C for 1 h. The mixture was basified with saturated aqueous NaHCO₃ and extracted with EtOAc. The organic phase was washed sequentially with water and saturated aqueous NaCl, dried over anhydrous Na₂SO₄, and concentrated under reduced pressure. The residue was purified by column chromatography to afford the title compound (yellow oil, 4.1 g, yield 81%).

### Step D: Methyl (3-fluoro-4-(1,4-dioxa-8-azaspiro[4.5]decan-8-yl)benzyl)carbamate

Under N₂, to a solution of (3-fluoro-4-(1,4-dioxa-8-azaspiro[4.5]decan-8-yl)phenyl)methanamine (2.0 g, 7.5 mmol, 1.0 eq) in DCM (20 mL) was added methyl carbonochloridate (0.84 g, 8.93 mmol, 1.20 eq). The mixture was stirred at 25°C for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 1.3 g, yield 54%).

### Step E: 1-(3-Fluoro-4-(1,4-dioxa-8-azaspiro[4.5]decan-8-yl)benzyl)dihydropyrimidine-2,4(1H,3H)-dione

Under N₂, to a solution of methyl (3-fluoro-4-(1,4-dioxa-8-azaspiro[4.5]decan-8-yl)benzyl)carbamate (1.3 g, 4.0 mmol, 1.0 eq) in tert-butanol (10 mL) were added potassium tert-butoxide (1.3 g, 11.6 mmol, 2.9 eq) and acrylamide (1.42 g, 20.0 mmol, 5.0 eq). The mixture was stirred at 60°C for 12 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 800 mg, yield 55%).

### Step F: 1-(3-Fluoro-4-(4-oxopiperidin-1-yl)benzyl)dihydropyrimidine-2,4(1H,3H)-dione

A solution of 1-(3-fluoro-4-(1,4-dioxa-8-azaspiro[4.5]decan-8-yl)benzyl)dihydropyrimidine-2,4(1H,3H)-dione (800 mg, 2.18 mmol, 1.0 eq) in formic acid (8 mL) was stirred at 50°C for 3 h. The reaction solution was diluted in EtOAc, basified with saturated aqueous NaHCO₃, washed sequentially with water and saturated aqueous NaCl, dried over anhydrous Na₂SO₄, and concentrated under reduced pressure. The residue was purified by column chromatography to afford the title compound (yellow oil, 401 mg, yield 58%).

### Step G: N-(3-Carbamoyl-1-(4-(4-(1-(4-((2,4-dioxotetrahydropyrimidin-1(2H)-yl)methyl)-2-fluorophenyl)piperidin-4-yl)piperazin-1-yl)phenyl)-1H-pyrazol-4-yl)-6-(trifluoromethyl)picolinamide

To a solution of 1-(3-fluoro-4-(4-oxopiperidin-1-yl)benzyl)dihydropyrimidine-2,4(1H,3H)-dione (200 mg, 0.62 mmol, 1.0 eq) and N-(3-carbamoyl-1-(4-(piperazin-1-yl)phenyl)-1H-pyrazol-4-yl)-6-(trifluoromethyl)picolinamide (284 mg, 0.62 mmol, 1.0 eq) in DMF (5 mL) was added 3 drops of AcOH, and the mixture was stirred for 2 h. Then, sodium cyanoborohydride (231 mg, 3.7 mmol, 6.2 eq) was added. The mixture was stirred at rt overnight. Upon completion, the reaction solution was quenched with water, diluted in EtOAc, washed sequentially with water and saturated aqueous NaCl, dried over anhydrous Na₂SO₄, and concentrated under reduced pressure. The crude was purified by HPLC to afford the title compound (white solid, 20.1 mg, yield 4%). LCMS (ESI): m/z 763 [M+H]⁺. **¹H NMR** (400 MHz, DMSO-d6) *δ* 11.79 (s, 1H), 10.19 (s, 1H), 8.88 (s, 1H), 8.47-8.35 (m, 2H), 8.23-8.19 (m, 1H), 7.95 (s, 1H), 7.84-7.76 (m, 2H), 7.70 (s, 1H), 7.10-7.00 (m, 5H), 4.43 (s, 2H), 3.43-3.38 (m, 2H), 3.35 (s, 1H), 3.28-3.26 (m, 2H), 3.24-3.20 (m, 4H), 2.71-2.66 (m, 6H), 2.55-2.53 (m, 2H), 1.96-1.85 (m, 2H), 1.66-1.56 (m, 2H).

### Preparation Example 436. Synthesis of Compound A430

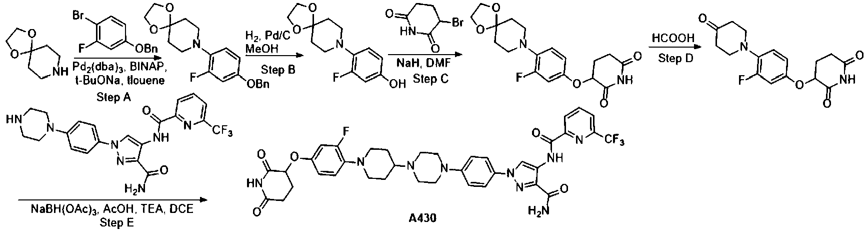

### Step A: 8-(4-(Benzyloxy)-2-fluorophenyl)-1,4-dioxa-8-azaspiro[4.5]decane

At rt, a mixture of 1,4-dioxa-8-azaspiro[4.5]decane (2 g, 14.0 mmol, 1.0 eq), 4-benzyloxy-1-bromo-2-fluorobenzene (4.32 g, 15.4 mmol, 1.1 eq), and sodium tert-butoxide (4.03 g, 41.9 mmol, 3 eq) in toluene was added tris(dibenzylideneacetone)dipalladium (640 mg, 0.7 mmol, 0.05 eq) and 2,2'-bis(diphenylphosphino)-1,1'-binaphthy (870 mg, 1.4 mmol, 0.10 eq). The mixture was stirred at 110°C under N₂ for 3 h. The mixture was diluted in EtOAc, washed with water and aqueous NaCl, dried over anhydrous Na₂SO₄, filtered, and concentrated under reduced pressure. The residue was purified by column chromatography to afford the title compound (yellow solid, 2.5 g, yield 52%).

### Step B: 3-Fluoro-4-(1,4-dioxa-8-azaspiro[4.5]decan-8-yl)phenol

At rt, to a solution of 8-(4-(benzyloxy)-2-fluorophenyl)-1,4-dioxa-8-azaspiro[4.5]decane (2.5 g, 7.28 mmol, 1.0 eq) in MeOH (35 mL) was added 10% Pd/C (0.5 g). The mixture was stirred under H₂ at rt overnight. Upon completion, the mixture was filtered, and the filtrate was concentrated to dryness to afford the title compound (yellow solid, 1.7 g, yield 92%).

### Step C: 3-(3-Fluoro-4-(1,4-dioxa-8-azaspiro[4.5]decan-8-yl)phenoxy)piperidine-2,6-dione

At 0°C, to a solution of 3-fluoro-4-(1,4-dioxa-8-azaspiro[4.5]decan-8-yl)phenol (1.3 g, 5.13 mmol, 1.0 eq) in DMF (25 mL) was added sodium hydride (310 mg, 7.7 mmol, 1.5 eq, 60% dispersion in mineral oil). After 0.5 h, 3-bromopiperidine-2,6-dione (1.08 g, 5.65 mmol, 1.1 eq) was added to the mixture at 0°C. The mixture was stirred at rt for 3 h. The reaction was quenched with saturated aqueous NH₄Cl and extracted with EtOAc. The organic phase was washed sequentially with water and saturated aqueous NaCl, dried over anhydrous Na₂SO₄, and concentrated under reduced pressure. The residue was purified by column chromatography to afford the title compound (white solid, 70 mg, yield 37%).

### Step D: 3-(3-Fluoro-4-(4-oxopiperidin-1-yl)phenoxy)piperidine-2,6-dione

A solution of 3-(3-fluoro-4-(1,4-dioxa-8-azaspiro[4.5]decan-8-yl)phenoxy)piperidine-2,6-dione (300 mg, 0.82 mmol, 1.0 eq) in water (3 mL) and formic acid (3 mL) was stirred at 50°C for 6 h. The mixture was neutralized with saturated aqueous NaHCO₃ and extracted with EtOAc. The organic phase was washed sequentially with water and saturated aqueous NaCl, dried over anhydrous Na₂SO₄, and concentrated under reduced pressure. The residue was purified by column chromatography to afford the title compound (yellow oil, 150 mg, yield 57%).

### Step E: N-(3-Carbamoyl-1-(4-(4-(1-(4-((2,6-dioxopiperidin-3-yl)oxy)-2-fluorophenyl)piperidin-4-yl)piperazin-1-yl)phenyl)-1H-pyrazol-4-yl)-6-(trifluoromethyl)picolinamide

At rt, to a solution of N-(3-carbamoyl-1-(4-(piperazin-1-yl)phenyl)-1H-pyrazol-4-yl)-6-(trifluoromethyl)picolinamide (300 mg, 0.65 mmol, 2.2 eq) and 3-(3-fluoro-4-(4-oxopiperidin-1-yl)phenoxy)piperidine-2,6-dione (100 mg, 0.22 mmol, 1.0 eq) in DMF (3 mL) was added one drop of HOAc, and the mixture was stirred for 16 h. Then sodium triacetoxyborohydride (231 mg, 1.1 mmol, 5.0 eq) was added, and the mixture was stirred at 25°C for 2 h. The reaction solution was diluted in EtOAc, washed sequentially with water and saturated aqueous NaCl, dried over anhydrous Na₂SO₄, and concentrated under reduced pressure. The residue was purified by preparative HPLC to afford the title compound (white solid, 7 mg, yield 3%). LCMS (ESI): m/z 764 [M+H]⁺. **¹H NMR** (400 MHz, DMSO-*d*6) δ 11.79 (s, 1H), 10.93 (s, 1H), 8.89 (s, 1H), 8.48-8.36 (m, 2H), 8.24-8.17 (m, 1H), 7.95 (s, 1H), 7.85-7.76 (m, 2H), 7.71 (s, 1H), 7.26-7.08 (m, 2H), 7.04-6.96 (m, 1H), 6.95-6.87 (m, 1H), 6.81-6.73 (m, 1H), 5.24-5.01 (m, 1H), 3.20-3.10 (m, 2H), 3.06-2.77 (m, 4H), 2.74-2.62 (m, 4H), 2.60-2.52 (m, 2H), 2.47-2.31 (m, 1H), 2.25-1.91 (m, 5H), 1.88-1.34 (m, 3H).

### Preparation Example 437. Synthesis of Compound A431

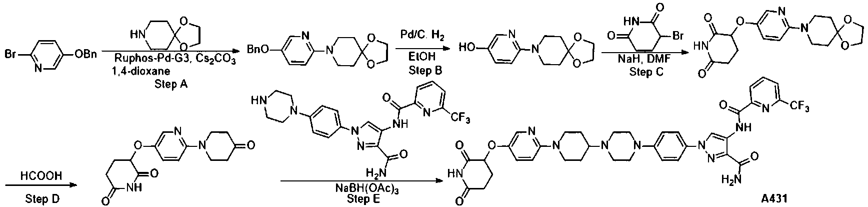

### Step A: 8-(5-(Benzyloxy)pyridin-2-yl)-1,4-dioxa-8-azaspiro[4.5]decane

To a solution of 5-(benzyloxy)-2-bromopyridine (2.0 g, 7.60 mmol, 1.0 eq), 1,4-dioxa-8-azaspiro[4.5]decane (2.17 g, 15.1 mmol, 2 eq) in 1,4-dioxane (20 mL) were added Ruphos-Pd-G₃ (0.90 g, 1.1 mmol, 0.15 eq) and Cs₂CO₃ (7.43 g, 21.4 mmol, 3.0 eq). The mixture was purged (×3) under N₂ and stirred at 100°C for 2 h. The reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 750 mg, yield 30%).

### Step B: 6-(1,4-Dioxa-8-azaspiro[4.5]decan-8-yl)pyridin-3-ol

To a solution of 8-(5-(benzyloxy)pyridin-2-yl)-1,4-dioxa-8-azaspiro[4.5]decane (750 mg, 2.30 mmol, 1.0 eq) in ethanol (10 mL) was added 10% wet palladium on carbon (0.27 g). The mixture was stirred under H₂ at rt overnight. The mixture was filtered, and the filtrate was concentrated to dryness to afford the title compound (yellow solid, 200 mg, yield 37%).

### Step C: 3-((6-(1,4-Dioxa-8-azaspiro[4.5]decan-8-yl)pyridin-3-yl)oxy)piperidine-2,6-dione

At 0°C, to a solution of 6-(1,4-dioxa-8-azaspiro[4.5]decan-8-yl)pyridin-3-ol (200 mg, 0.84 mmol, 1.0 eq) in DMF (3 mL) was added NaH (50 mg, 1.26 mmol, 1.5 eq, 60% dispersion in mineral oil). After stirring at 0°C for 10 min, 3-bromopiperidine-2,6-dione (193 mg, 1.01 mmol, 1.2 eq) was added to the mixture. The mixture was stirred at rt for 3 h. The mixture was quenched with saturated aqueous NH₄Cl and extracted with EtOAc. The organic phase was washed sequentially with water and saturated aqueous NaCl, dried over anhydrous Na₂SO₄, and concentrated under reduced pressure. The residue was purified by column chromatography to afford the title compound (white solid, 200 mg, yield 69%).

### Step D: 3-((6-(4-Oxopiperidin-1-yl)pyridin-3-yl)oxy)piperidine-2,6-dione

A solution of 3-((6-(1,4-dioxa-8-azaspiro[4.5]decan-8-yl)pyridin-3-yl)oxy)piperidine-2,6-dione (200 mg, 0.58 mmol, 1.0 eq) in formic acid (3 mL) was stirred at 50°C for 6 h. The reaction was quenched with saturated aqueous NaHCO₃ and extracted with EtOAc. The combined organic layers were washed sequentially with water and saturated aqueous NaCl, dried over anhydrous Na₂SO₄, and concentrated under reduced pressure to dryness to afford the title compound (yellow oil, 100 mg, yield 57%).

### Step E: N-(3-Carbamoyl-1-(4-(4-(1-(5-((2,6-Dioxopiperidin-3-yl)oxy)pyridin-2-yl)piperidin-4-yl)piperazin-1-yl)phenyl)-1H-pyrazol-4-yl)-6-(trifluoromethyl)picolinamide

To a solution of N-(3-carbamoyl-1-(4-(piperazin-1-yl)phenyl)-1H-pyrazol-4-yl)-6-(trifluoromethyl)picolinamide (303 mg, 0.66 mmol, 2.0 eq) and 3-((6-(4-oxopiperidin-1-yl)pyridin-3-yl)oxy)piperidine-2,6-dione (100 mg, 0.33 mmol, 1.0 eq) in DMF (3 mL) was added AcOH (0.2 mL). After stirring at rt for 2 h, sodium triacetoxyborohydride (231 mg, 1.11 mmol, 5.0 eq) was then added. The reaction solution was diluted in EtOAc, washed sequentially with water and saturated aqueous NaCl, dried over anhydrous Na₂SO₄, and concentrated under reduced pressure. The residue was purified by preparative HPLC to afford the title compound (white solid, 1.7 mg, yield 1%). LCMS (ESI): m/z 747 [M+H]⁺. **¹H NMR** (400 MHz, DMSO-*d*₆) δ 11.79 (s, 1H), 10.92 (s, 1H), 8.92 (s, 1H), 8.92 (s, 1H), 8.46-8.38 (m, 2H), 8.21 (d, *J* = 7.5 Hz, 1H), 7.95 (d, *J =* 2.6 Hz, 2H), 7.87 (d, *J* = 8.9 Hz, 2H), 7.74 (s, 1H), 7.38 (d, *J* = 6.4 Hz, 1H), 7.25 (s, 1H), 7.13 (s, 1H), 6.92 (d, *J* = 9.2 Hz, 1H), 5.15-4.94 (m, 1H), 4.39-4.32 (m, 2H), 3.99-3.93 (m, 2H), 3.65-3.62 (m, 2H), 3.15-3.11 (m, 2H), 2.81-2.75 (m, 2H), 2.67-2.62 (m, 2H), 2.22-2.12 (m, 4H), 2.01-1.96 (m, 1H), 1.72-1.50 (m, 3H).

### Preparation Example 438. Synthesis of Compound A432

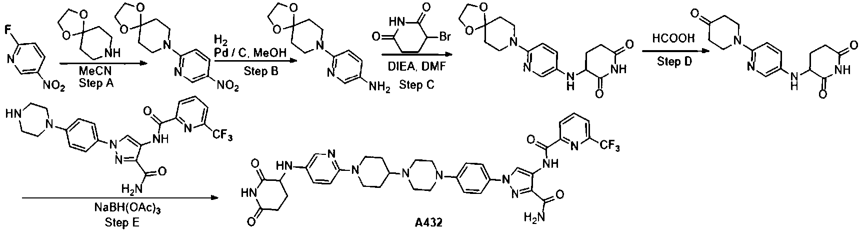

### Step A: 8-(5-Nitropyridin-2-yl)-1,4-dioxa-8-azaspiro[4.5]decane

To a solution of 2-fluoro-5-nitropyridine (10 g, 70.1 mmol, 1.0 eq) in acetonitrile (200 mL) were added sodium carbonate (13.6 g, 128 mmol, 1.8 eq) and 1,4-dioxa-8-azaspiro[4.5]decane (5.5 g, 48.3 mmol, 1.5 eq). The reaction solution was stirred at 70°C under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 10 g, yield 53%).

### Step B: 6-(1,4-Dioxa-8-azaspiro[4.5]decan-8-yl)pyridin-3-amine

To a solution of 8-(5-nitropyridin-2-yl)-1,4-dioxa-8-azaspiro[4.5]decane (10.0 g, 37.7 mmol, 1.0 eq) in MeOH (100 mL) was added Pd/C (1.0 g, w.t.=10 %). The reaction solution was purged with H₂ (×3) and stirred under H₂ at rt overnight. Upon completion, the reaction solution was filtered and concentrated under reduced pressure to afford the title compound (yellow solid, 6 g, crude), which was used directly without purification.

### Step C: 3-((6-(1,4-Dioxa-8-azaspiro[4.5]decan-8-yl)pyridin-3-yl)amino)piperidine-2,6-dione

To a solution of 6-(1,4-dioxa-8-azaspiro[4.5]decan-8-yl)pyridin-3-amine (3.0 g, 12.7 mmol, 1.0 eq) in DMF (25 mL) were added 3-bromopiperidine-2,6-dione (2.4 g, 12.7 mmol, 1.0 eq) and DIEA (4.91 g, 38.1 mmol, 3.0 eq). The reaction solution was stirred at 45°C overnight under N₂. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 3 g, yield 68%).

### Step D: 3-((6-(4-Oxopiperidin-1-yl)pyridin-3-yl)amino)piperidine-2,6-dione

To a solution of 3-((6-(1,4-dioxa-8-azaspiro[4.5]decan-8-yl)pyridin-3-yl)amino)piperidine-2,6-dione (2.0 g, 5.78 mmol, 1.0 eq) in water (1 mL) was added formic acid (10 mL). The reaction solution was stirred at 25°C for 6 h. Upon completion, the reaction solution was neutralized with saturated aqueous NaHCO₃, diluted in EtOAc, washed sequentially with water and saturated aqueous NaCl, dried over anhydrous Na₂SO₄, and concentrated under reduced pressure. The residue was purified by column chromatography to afford the title compound (yellow solid, 310 mg, yield 18%).

### Step E: N-(3-Carbamoyl-1-(4-(4-(1-(5-((2,6-Dioxopiperidin-3-yl)amino)pyridin-2-yl)piperidin-4-yl)piperazin-1-yl)phenyl)-1H-pyrazol-4-yl)-6-(trifluoromethyl)picolinamide

At rt, to a solution of 3-((6-(4-oxopiperidin-1-yl)pyridin-3-yl)amino)piperidine-2,6-dione (302 mg, 1.0 mmol, 2.0 eq), and N-(3-carbamoyl-1-(4-(piperazin-1-yl)phenyl)-1H-pyrazol-4-yl)-6-(trifluoromethyl)picolinamide (230 mg, 0.5 mmol, 1.0 eq) in DMF (3 mL) and AcOH (0.1 mL) was added NaBH₃CN (199 mg, 3.0 mmol, 6.0 eq). The reaction solution was stirred at rt under N₂ overnight. Upon completion, the reaction solution was concentrated under reduced pressure. The residue was purified by HPLC to afford the title compound (white solid, 20 mg, yield 3%). LCMS (ESI): m/z 746 [M+H]⁺. **¹H NMR** (400 MHz, DMSO-*d*6) δ 11.79 (s, 1H), 11.35 (s, 1H), 10.86 (s, 1H), 8.93 (s, 1H), 8.46-8.38 (m, 2H), 8.23-8.19 (m, 1H), 7.96 (s, 1H), 7.89-7.85 (m, 2H), 7.77-7.69 (m, 2H), 7.43-7.36 (m, 2H), 7.18 (d, J = 9.1 Hz, 2H), 4.46-4.26 (m, 4H), 3.97-3.93 (m, 4H), 3.70-3.53 (m, 4H), 3.32-3.19 (m, 4H), 3.10 (t, J = 12.1 Hz, 2H), 2.71-2.63 (m, 1H), 2.13-2.07 (m, 1H), 1.93-1.85 (m, 2H).

### Preparation Example 439. Synthesis of Compound A433

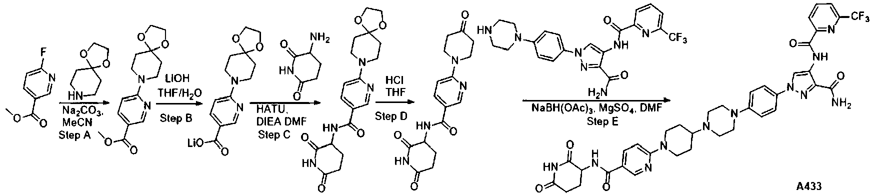

### Step A: Methyl 6-(1,4-dioxa-8-azaspiro[4.5]decan-8-yl)nicotinate

Under N₂ at rt, to a solution of methyl 6-fluoronicotinate (5 g, 32.2 mmol, 1.0 eq) in acetonitrile (200 mL) were added sodium carbonate (6.83 g, 64.4 mmol, 2.0 eq) and 1,4-dioxa-8-azaspiro[4.5]decane (5.5 g, 48.3 mmol, 1.5 eq). The reaction solution was stirred at 90°C under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by column chromatography (eluent: petroleum ether/ethyl acetate, gradient: 0%-30% ethyl acetate) to afford the title compound (white solid, 6.1 g, yield 68%).

### Step B: Lithium 6-(1,4-dioxa-8-azaspiro[4.5]decan-8-yl)nicotinate

Under N₂ at rt, to a solution of methyl 6-(1,4-dioxa-8-azaspiro[4.5]decan-8-yl)nicotinate (3 g, 10.8 mmol, 1.0 eq) in THF (30 mL) and water (6 mL) was added lithium hydroxide (517 mg, 21.56 mmol, 2.0 eq). The reaction solution was stirred at rt under N₂ for 1 h. Upon completion, the reaction solution was concentrated under reduced pressure to afford the title compound (white solid, 2.71 g, crude), which was used directly without purification.

### Step C: N-(2,6-Dioxopiperidin-3-yl)-6-(1,4-dioxa-8-azaspiro[4.5]decan-8-yl)nicotinamide

Under N₂ at rt, to a solution of lithium 6-(1,4-dioxa-8-azaspiro[4.5]decan-8-yl)nicotinate (1.6 g, 5.9 mmol, 1.2 eq) in DMF (15 mL) were added HATU (1.93 g, 5.9 mmol, 1.2 eq), 3-aminopiperidine-2,6-dione (632 mg, 4.9 mmol, 1.0 eq), and DIEA (1.89 g, 14.7 mmol, 3.0 eq). The reaction solution was stirred at rt under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by column chromatography (eluent: DCM/MeOH, gradient: 0%-5% MeOH) to afford the title compound (yellow solid, 1.6 g, yield 87%).

### Step D: N-(2,6-Dioxopiperidin-3-yl)-6-(4-oxopiperidin-1-yl)nicotinamide

Under N₂ at rt, to a solution of N-(2,6-dioxopiperidin-3-yl)-6-(1,4-dioxa-8-azaspiro[4.5]decan-8-yl)nicotinamide (800 mg, 2.1 mmol, 1.0 eq) in THF (2 mL) was added dilute HCl (4 mL, 2 N). The reaction solution was stirred at 50°C under N₂ for 1 h. Upon completion, the reaction solution was neutralized with saturated aqueous NaHCO₃, diluted in EtOAc, washed sequentially with water and saturated aqueous NaCl, dried over anhydrous Na₂SO₄, and concentrated under reduced pressure. The residue was purified by column chromatography (eluent: DCM/MeOH, gradient: 0%-5% MeOH) to afford the title compound (yellow solid, 400 mg, yield 58%).

### Step E: N-(3-Carbamoyl-1-(4-(4-(1-(5-((2,6-dioxopiperidin-3-yl)carbamoyl)pyridin-2-yl)piperidin-4-yl)piperazin-1-yl)phenyl)-1H-pyrazol-4-yl)-6-(trifluoromethyl)picolinamide

At rt, to a solution of N-(2,6-dioxopiperidin-3-yl)-6-(4-oxopiperidin-1-yl)nicotinamide (216 mg, 0.66 mmol, 3.0 eq) and N-(3-carbamoyl-1-(4-(piperazin-1-yl)phenyl)-1H-pyrazol-4-yl)-6-(trifluoromethyl)picolinamide (100 mg, 0.22 mmol, 1.0 eq) in DMF (3 mL) were added NaBH(OAc)₃ (231 mg, 1.1 mmol, 5.0 eq) and MgSO₄ (100 mg, 0.83 mmol, 3.7 eq). The reaction solution was stirred at rt under N₂ overnight. Upon completion, the reaction solution was concentrated under reduced pressure. The residue was purified by HPLC (column: YMC-Actus Triart C18; eluent: 0.1% NH₃·H₂O in deionized water/acetonitrile, gradient: 10%-95% acetonitrile) to afford the title compound (white solid, 12.5 mg, yield 7%). LCMS (ESI): m/z 774 [M+H]⁺. **¹H NMR** (400 MHz, DMSO-d6) δ 11.78 (s, 1H), 10.84 (s, 1H), 8.87 (s, 1H), 8.61 (d, *J* = 2.4 Hz, 1H), 8.53 (d, *J =* 8.3 Hz, 1H), 8.44 (d, *J =* 7.6 Hz, 1H), 8.41-8.36 (m, 1H), 8.23-8.19 (m, 1H), 7.99-7.88 (m, 2H), 7.77 (d, *J* = 9.0 Hz, 2H), 7.69 (s, 1H), 7.06 (d, *J* = 9.1 Hz, 2H), 6.90 (d, *J* = 9.2 Hz, 1H), 4.81-4.69 (m, 1H), 4.51-4.42 (m, 2H), 3.22-3.18 (m, 4H), 2.96-2.89 (m, 2H), 2.82-2.75 (m, 1H), 2.70-2.64 (m, 4H), 2.61-2.54 (m, 2H), 2.14-2.05 (m, 1H), 1.99-1.94 (m, 1H), 1.92-1.86 (m, 2H), 1.46-1.37 (m, 2H).

### Preparation Example 440. Synthesis of Compound A434-Int

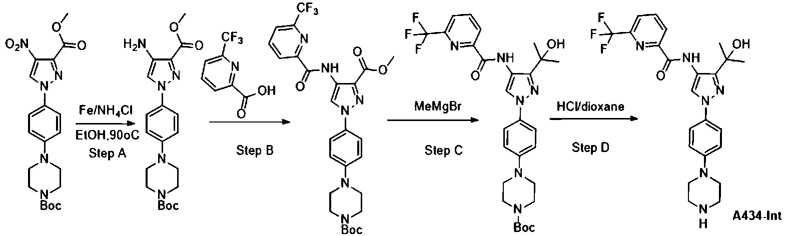

### Step A: Tert-butyl 4-(4-(4-amino-3-(methoxycarbonyl)-1H-pyrazol-1-yl)phenyl)piperazine-1-carboxylate

At rt, to a mixture of tert-butyl 4-(4-(3-(methoxycarbonyl)-4-nitro-1H-pyrazol-1-yl)phenyl)piperazine-1-carboxylate (10 g, 23.18 mmol, 1.0 eq) and ammonium chloride (6.2 g, 115.89 mmol, 5.0 eq) in anhydrous ethanol (100 mL) and water (30 mL) was added iron powder (6.47 g, 115.89 mmol, 5.0 eq). The mixture was stirred at 90°C for 1.5 h. The mixture was filtered hot over diatomaceous earth. The filtrate was diluted in water and extracted with EtOAc. The organic layer was washed with saturated aqueous NaCl, dried over anhydrous Na₂SO₄, and concentrated under reduced pressure to afford the title compound (pale yellow solid, 9.2 g, crude).

### Step B: Tert-butyl 4-(4-(3-(methoxycarbonyl)-4-(6-(trifluoromethyl)pyridinylamino)-1H-pyrazol-1-yl)phenyl)piperazine-1-carboxylate

At 0°C, tert-butyl 4-(4-(4-amino-3-(methoxycarbonyl)-1H-pyrazol-1-yl)phenyl)piperazine-1-carboxylate (900 mg, crude) and DIEA (869 mg, 6.73 mmol, 3.0 eq) were added to DMF (15 mL), and then 6-(trifluoromethyl)picolinic acid (471 mg, 2.47 mmol, 1.1 eq) and HATU (1.3 g, 3.36 mmol, 1.5 eq) were added. The mixture was stirred at rt for 1 h. The mixture was poured into cold water to precipitate a solid, which was filtered to afford the title compound (pale yellow solid, 1.05 g, yield 82%).

### Step C: Tert-butyl 4-(4-(3-(2-hydroxypropan-2-yl)-4-(6-(trifluoromethyl)pyridinylamino)-1H-pyrazol-1-yl)phenyl)piperazine-1-carboxylate

At rt, to a solution of tert-butyl 4-(4-(3-(methoxycarbonyl)-4-(6-(trifluoromethyl)pyridinylamino)-1H-pyrazol-1-yl)phenyl)piperazine-1-carboxylate (300 mg, 0.52 mmol, 1.0 eq) in THF (5 mL) and DCM (5 mL) was added methylmagnesium bromide (2.6 mL, 7.83 mmol, 15.0 eq). The mixture was stirred at rt overnight. Upon completion, the reaction was quenched with cold saturated aqueous NH₄Cl and extracted with DCM twice. The organic layer was washed with saturated aqueous NaCl, dried over anhydrous Na₂SO₄, and concentrated under reduced pressure. The residue was purified by column chromatography to afford the title compound (yellow solid, 180 mg, yield 60%).

### Step D: N-(3-(2-Hydroxypropan-2-yl)-1-(4-(piperazin-1-yl)phenyl)-1H-pyrazol-4-yl)-6-(trifluoromethyl)picolinamide

At rt, to a solution of tert-butyl 4-(4-(3-(2-hydroxypropan-2-yl)-4-(6-(trifluoromethyl)picolinamido)-1H-pyrazol-1-yl)phenyl)piperazine-1-carboxylate (300 mg, 0.52 mmol, 1.0 eq) in DCM (5 mL) was added 4 M hydrogen chloride-dioxane (5 mL). The reaction solution was stirred at rt for 1 h. Upon completion, the reaction solution was adjusted to pH 6 with 1 N NaOH solution, and extracted with DCM. The organic layer was washed with saturated aqueous NaCl, dried over anhydrous Na₂SO₄, and concentrated. The residue was purified by column chromatography to afford the title compound (yellow solid, 130 mg, yield 52%). **¹H NMR** (400 MHz, DMSO-d6) δ 11.20 (s, 1H), 8.74 (s, 1H), 8.41 (dd, J = 16.5, 7.5 Hz, 2H), 8.18 (d, J = 6.7 Hz, 1H), 7.60 (d, J = 9.0 Hz, 2H), 7.01 (d, J = 9.1 Hz, 2H), 5.93 (s, 1H), 3.11-3.03 (m, 4H), 2.88-2.80 (m, 4H), 1.58 (s, 6H), 1.23 (s, 1H).

### Preparation Example 441. Synthesis of Compound A434

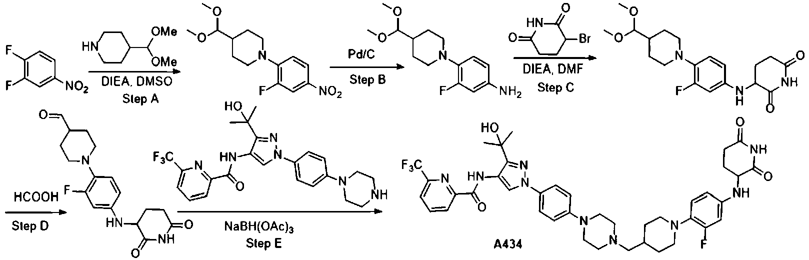

### Step A: 4-(Dimethoxymethyl)-1-(2-fluoro-4-nitrophenyl)piperidine

At rt, to a solution of 1,2-difluoro-4-nitrobenzene (6 g, 37.72 mmol, 1.0 eq) in dimethyl sulfoxide (60 mL) were added 4-(dimethoxymethyl)piperidine (6.6 g, 41.49 mmol, 1.1 eq) and DIEA (20.0 mL, 113.15 mmol, 3.0 eq). The mixture was stirred at 100°C under N₂ for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 11.2 g, yield 99%).

### Step B: 4-(4-(Dimethoxymethyl)piperidin-1-yl)-3-fluoroaniline

At rt, to a solution of 4-(dimethoxymethyl)-1-(2-fluoro-4-nitrophenyl)piperidine (5 g, 16.76 mmol, 1.0 eq) in MeOH (100 mL) was added Pd/C (1 g, w.t.=10%). The reaction solution was degassed (×3) under N₂ and then stirred under N₂ at rt for 3 h. Upon completion, the reaction solution was filtered through Celite and concentrated to afford the title compound (yellow solid, 4.4 g, yield 99%).

### Step C: 3-((4-(4-(Dimethoxymethyl)piperidin-1-yl)-3-fluorophenyl)amino)piperidine-2,6-dione

At rt, to a solution of 4-(4-(dimethoxymethyl)piperidin-1-yl)-3-fluoroaniline (500 mg, 1.87 mmol, 1.0 eq) in dioxane (5 mL) were added 3-bromopiperidine-2,6-dione (718 mg, 3.74 mmol, 2.0 eq) and DIEA (0.66 mL, 3.74 mmol, 2.0 eq). The mixture was stirred at 80°C under N₂ for 18 h. Upon completion, the mixture was diluted in EtOAc, washed sequentially with water and saturated aqueous NaCl, dried over anhydrous Na₂SO₄, and concentrated under reduced pressure. The residue was purified by column chromatography to afford the title compound (yellow solid, 480 mg, yield 68%).

### Step D: 1-(4-((2,6-Dioxopiperidin-3-yl)amino)-2-fluorophenyl)piperidine-4-carbaldehyde

At rt, 3-((4-(4-(dimethoxymethyl)piperidin-1-yl)-3-fluorophenyl)amino)piperidine-2,6-dione (100 mg, 0.264 mmol, 1.0 eq) was added to formic acid (5 mL), and the reaction solution was stirred at rt under N₂ for 2 h. Upon completion, the reaction mixture was concentrated under reduced pressure. The residue was used directly in the next step.

### Step E: N-(1-(4-(4-((1-(4-((2,6-Dioxopiperidin-3-yl)amino)-2-fluorophenyl)piperidin-4-yl)methyl)piperazin-1-yl)phenyl)-3-(2-hydroxypropan-2-yl)-1H-pyrazol-4-yl)-6-(trifluoromethyl)picolinamide

At rt, to a solution of 1-(4-((2,6-dioxopiperidin-3-yl)amino)-2-fluorophenyl)piperidine-4-carbaldehyde (93 mg, crude) in DMF (10 mL) were added N-(3-(2-hydroxypropan-2-yl)-1-(4-(piperazin-1-yl)phenyl)-1H-pyrazol-4-yl)-6-(trifluoromethyl)picolinamide (140 mg, 0.30 mmol, 1.0 eq) and a few drops of AcOH. After stirring for 2 h, NaBH(OAc)₃ (249 mg, 1.18 mmol, 4.0 eq) was added. The reaction solution was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 38.8 mg, yield 17%). **¹H NMR** (400 MHz, DMSO-d6) δ 11.20 (s, 1H), 10.78 (s, 1H), 8.75 (s, 1H), 8.46-8.35 (m, 2H), 8.18 (d, *J* = 7.6 Hz, 1H), 7.61 (d, *J* = 8.9 Hz, 2H), 7.03 (d, *J=* 9.0 Hz, 2H), 6.83 (t, *J* = 9.3 Hz, 1H), 6.60-6.29 (m, 2H), 5.93 (s, 1H), 5.78 (d, *J=* 7.6 Hz, 1H), 4.36-4.10 (m, 1H), 3.21-3.06 (m, 6H), 2.79-2.68 (m, 1H), 2.68-2.51 (m, 7H), 2.29-2.18 (m, 2H), 2.14-2.05 (m, 1H), 1.91-1.73 (m, 3H), 1.66-1.55 (m, 7H), 1.33-1.21 (m, 2H). LCMS (ESI): m/z 791.6 [M+H]⁺.

### Preparation Example 442. Synthesis of Compound A435

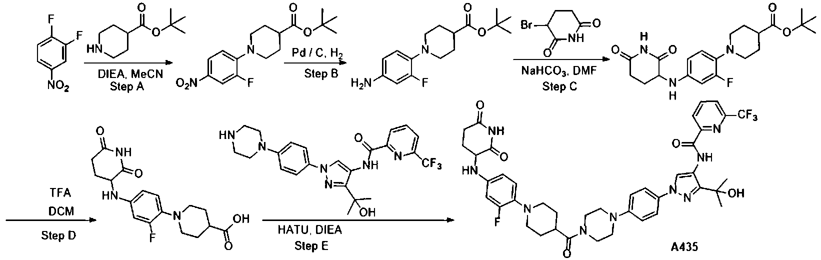

### Step A: Tert-butyl 1-(2-fluoro-4-nitrophenyl)piperidine-4-carboxylate

At rt, to a solution of 1,2-difluoro-4-nitrobenzene (2 g, 12.58 mmol, 1.0 eq) and tert-butyl piperidine-4-carboxylate (2.6 g, 13.84 mmol, 1.1 eq) in acetonitrile (35 mL) was added DIEA (5.5 mL, 31.45 mmol, 2.5 eq). The mixture was stirred at 80°C under N₂ for 8 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 1.5 g, yield 37%).

### Step B: Tert-butyl 1-(4-amino-2-fluorophenyl)piperidine-4-carboxylate

At rt, to a solution of tert-butyl 1-(2-fluoro-4-nitrophenyl)piperidine-4-carboxylate (1.5 g, 4.63 mmol, 1.0 eq) in EtOAc (40 mL) was added Pd/C (300 mg, w.t.=10%). The reaction solution was degassed (×3) under H₂ and stirred under H₂ at rt for 6 h. Upon completion, the reaction solution was filtered through Celite and concentrated to afford the title compound (brown oil, 1.3 g, crude).

### Step C: Tert-butyl 1-(4-((2,6-dioxopiperidin-3-yl)amino)-2-fluorophenyl)piperidine-4-carboxylate

At rt, tert-butyl 1-(4-amino-2-fluorophenyl)piperidine-4-carboxylate (1.3 g, crude), 3-bromopiperidine-2,6-dione (1.0 g, 5.75 mmol, 1.3 eq), and NaHCO₃ (930 mg, 11.05 mmol, 2.5 eq) were added to DMF (30 mL). The reaction solution was stirred at 60°C under N₂ for 6 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 800 mg, yield 45% over two steps).

### Step D: 1-(4-((2,6-Dioxopiperidin-3-yl)amino)-2-fluorophenyl)piperidine-4-carboxylic acid

At rt, to a solution of tert-butyl 1-(4-((2,6-dioxopiperidin-3-yl)amino)-2-fluorophenyl)piperidine-4-carboxylate (200 mg, 0.49 mmol, 1.0 eq) in DCM (10 mL) was added TFA (2 mL). The reaction solution was stirred at rt for 3 h. Upon completion, the reaction solution was concentrated under reduced pressure to afford the title compound (brown solid, 170 mg, crude),

### Step E: N-(1-(4-(4-(1-(4-((2,6-Dioxopiperidin-3-yl)amino)-2-fluorophenyl)piperidine-4-carbonyl)piperazin-1-yl)phenyl)-3-(2-hydroxypropan-2-yl)-1H-pyrazol-4-yl)-6-(trifluoromethyl)picolinamide

At 0°C, to a solution of 1-(4-((2,6-dioxopiperidin-3-yl)amino)-2-fluorophenyl)piperidine-4-carboxylic acid (150 mg, crude) and N-(3-(2-hydroxypropan-2-yl)-1-(4-(piperazin-1-yl)phenyl)-1H-pyrazol-4-yl)-6-(trifluoromethyl)picolinamide (204 mg, 0.43 mmol, 1.0 eq) in DMF (5 mL) were added HATU (245 mg, 0.64 mmol, 1.5 eq) and DIEA (111 mg, 0.86 mmol, 2.0 eq). The mixture was stirred at a constant temperature for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (yellow solid, 28.9 mg, yield 8%). **¹H NMR** (400 MHz, DMSO) δ 11.20 (s, 1H), 10.79 (s, 1H), 8.77 (s, 1H), 8.46-8.34 (m, 2H), 8.18 (d, *J =* 7.6 Hz, 1H), 7.65 (d, *J =* 9.0 Hz, 2H), 7.08 (d, *J =* 9.1 Hz, 2H), 6.99-6.82 (m, 1H), 6.63-6.40 (m, 2H), 6.06-5.59 (m, 2H), 4.43-4.12 (m, 1H), 3.75-3.61 (m, 4H), 3.26-3.10 (m, 6H), 2.87-2.66 (m, 3H), 2.61-2.51 (m, 2H), 2.12-2.05 (m, 1H), 1.93-1.65 (m, 5H), 1.58 (s, 6H). LCMS (ESI): m/z 806 [M+H]⁺.

### Preparation Example 443. Synthesis of Compound A436

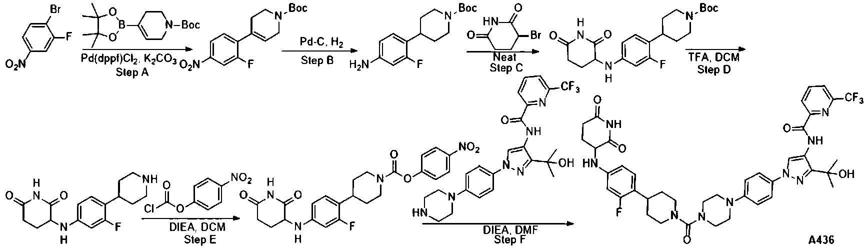

### Step A: Tert-butyl 4-(2-fluoro-4-nitrophenyl)-3,6-dihydropyridine-1(2H)-carboxylate

At rt, to a solution of 1-bromo-2-fluoro-4-nitrobenzene (5.0 g, 22.73 mmol, 1.0 eq) in dioxane (50 mL) and water (5 mL) were added tert-butyl 4-(4,4,5,5-tetramethyl-1,3,2-dioxaborolan-2-yl)-3,6-dihydropyridine-1(2H)-carboxylate (8.4 g, 27.27 mmol, 1.2 eq), K₂CO₃ (9.4 g, 68.19 mmol, 3.0 eq) and Pd(dppf)Cl₂ (1.7 g, 2.27 mmol, 0.1 eq). The mixture was stirred at 90°C under N₂ for 3 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 3 g, yield 41%).

### Step B: Tert-butyl 4-(4-amino-2-fluorophenyl)piperidine-1-carboxylate

At rt, to a solution of tert-butyl 4-(2-fluoro-4-nitrophenyl)-3,6-dihydropyridine-1(2H)-carboxylate (3.0 g, 9.32 mmol, 1.0 eq) in ethanol (30 mL) was added Pd/C (1 g, w.t.= 10%). The reaction mixture was degassed with H₂ (×3) and then stirred under H₂ at rt for 16 h. Upon completion, the reaction solution was filtered through Celite and concentrated under reduced pressure to afford the title compound (yellow solid, 2.0 g, crude).

### Step C: Tert-butyl 4-(4-((2,6-dioxopiperidin-3-yl)amino)-2-fluorophenyl)piperidine-1-carboxylate

At rt, to a solution of tert-butyl 4-(4-amino-2-fluorophenyl)piperidine-1-carboxylate (2.0 g, crude) in dioxane (20 mL) were added 3-bromopiperidine-2,6-dione (3.9 g, 20.41 mmol, 3.0 eq) and DIEA (2.4 mL, 13.6 mmol, 2.0 eq). The reaction solution was stirred at 90°C under N₂ for 16 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 3.0 g, yield 41% over two steps).

### Step D: 3-((3-Fluoro-4-(piperidin-4-yl)phenyl)amino)piperidine-2,6-dione

At rt, to a solution of tert-butyl 4-(4-((2,6-dioxopiperidin-3-yl)amino)-2-fluorophenyl)piperidine-1-carboxylate (900 mg, 2.2 mmol, 1.0 eq) in DCM (6 mL) was added TFA (1.5 mL). The reaction solution was stirred at rt under N₂ for 1 h. Upon completion, the reaction solution was concentrated under reduced pressure to afford the title compound (yellow solid, 0.6 g, crude).

### Step E: 4-Nitrophenyl 4-(4-((2,6-dioxopiperidin-3-yl)amino)-2-fluorophenyl)piperidine-1-carboxylate

At rt, to a solution of 3-((3-fluoro-4-(piperidin-4-yl)phenyl)amino)piperidine-2,6-dione (600 mg, crude) in DCM (15 mL) were added 4-nitrophenyl carbonochloridate (594 mg, 2.9 mmol, 1.5 eq) and DIEA (508 mg, 3.9 mmol, 2.0 eq). The reaction solution was stirred at rt under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 200 mg, yield 22% over two steps).

### Step F: N-(1-(4-(4-(4-((2,6-Dioxopiperidin-3-yl)amino)-2-fluorophenyl)piperidine-1-carbonyl)piperazin-1-yl)phenyl)-3-(2-hydroxypropan-2-yl)-1H-pyrazol-4-yl)-6-(trifluoromethyl)picolinamide

At rt, to a solution of 4-nitrophenyl 4-(4-((2,6-dioxopiperidin-3-yl)amino)-2-fluorophenyl)piperidine-1-carboxylate (200 mg, 0.4 mmol, 1.0 eq) in DMF (10 mL) were added DIEA (164.8 mg, 1.2 mmol, 3.0 eq) and N-(3-(2-hydroxypropan-2-yl)-1-(4-(piperazin-1-yl)phenyl)-1H-pyrazol-4-yl)-6-(trifluoromethyl)picolinamide (303 mg, 0.6 mmol, 1.5 eq). The reaction solution was stirred at 100°C under N₂ for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 26.1 mg, yield 8%). **¹H NMR** (400 MHz, DMSO) δ 11.21 (s, 1H), 10.79 (s, 1H), 8.77 (s, 1H), 8.46-8.32 (m, 2H), 8.18 (d, J = 7.6 Hz, 1H), 7.64 (d, J = 8.9 Hz, 2H), 7.10-6.97 (m, 3H), 6.51-6.42 (m, 2H), 6.03 (d, J = 7.7 Hz, 1H), 5.95 (s, 1H), 4.40-4.27 (m, 1H), 3.81-3.72 (m, 2H), 3.33-3.28 (m, 4H), 3.21-3.13 (m, 4H), 2.90-2.72 (m, 4H), 2.61-2.55 (m, 1H), 2.13-2.05 (m, 1H), 1.92-1.80 (m, 1H), 1.75-1.66 (m, 2H), 1.66-1.59 (m, 2H), 1.58 (s, 6H). LCMS (ESI): m/z 803 [M+H]⁺.

### Preparation Example 444. Synthesis of Compound A437

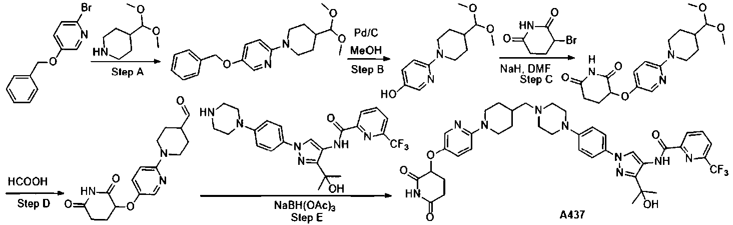

### Step A: 5-Benzyloxy-2-(4-(dimethoxymethyl)piperidin-1-yl)pyridine

To a stirred mixture of 5-(benzyloxy)-2-bromopyridine (5.0 g, 18.9 mmol, 1.0 eq) and 4-(dimethoxymethyl)piperidine (4.5 g, 28.4 mmol, 1.5 eq) in 1,4-dioxane (60 mL) were added Ruphos (1.8 g, 3.83 mmol, 0.2 eq), Ruphos Pd G1 (0.6 g, 2.72 mmol, 0.14 eq) and cesium carbonate (5.5 g, 56.8 mmol, 3.0 eq). The mixture was stirred at 90°C under N₂ for 16 h. The mixture was cooled with ice and quenched with saturated aqueous NH₄Cl. The mixture was extracted with EtOAc (×3). The combined organic phases were washed with water and saturated aqueous NaCl, dried over anhydrous Na₂SO₄, and concentrated under reduced pressure. The residue was purified by column chromatography to afford the title compound (yellow oil, 2.7 g, yield 42%).

### Step B: 6-(4-(Dimethoxymethyl)piperidin-1-yl)pyridin-3-ol

A mixture of 5-benzyloxy-2-(4-(dimethoxymethyl)piperidin-1-yl)pyridine (2.7 g, 7.91 mmol, 1.0 eq) in MeOH (30 mL) was stirred. Pd/C (10%, 0.3 g, 11% by weight) was added at rt. The mixture was degassed with H₂ (×3) and then stirred under a hydrogen balloon at rt for 16 h. The mixture was filtered and concentrated under reduced pressure. The residue was purified by column chromatography to afford the title compound (white solid, 1.8 g, yield 91%).

### Step C: 3-((6-(4-(Dimethoxymethyl)piperidin-1-yl)pyridin-3-yl)oxy)piperidine-2,6-dione

To a stirred solution of 6-(4-(dimethoxymethyl)piperidin-1-yl)pyridin-3-ol (800 mg, 3.21 mmol, 1.0 eq) in DMF (15 mL) was added NaH (60% in mineral oil, 254 mg, 6.42 mmol, 2.0 eq) at 0°C. The mixture was stirred at 0°C under N₂ for 30 min. Then 3-bromopiperidine-2,6-dione (913 mg, 4.81 mmol, 1.5 eq) was added at 0°C, and the mixture was stirred at rt for 2 h. The reaction solution was quenched with aqueous NH₄Cl at 0°C, and the mixture was extracted with EtOAc (×3). The combined organic phases were washed with water and saturated aqueous NaCl, dried over anhydrous Na₂SO₄, and concentrated under reduced pressure. The residue was purified by column chromatography to afford the title compound (white solid, 200 mg, yield 17%).

### Step D: 1-(5-((2,6-Dioxopiperidin-3-yl)oxy)pyridin-2-yl)piperidine-4-carbaldehyde

3-((6-(4-(dimethoxymethyl)piperidin-1-yl)pyridin-3-yl)oxy)piperidine-2,6-dione (200 mg, 0.62 mmol, 1.0 eq) and formic acid (5 mL) were mixed, and the mixture was stirred at 50°C for 30 min. The reaction solution was concentrated under reduced pressure to afford the title compound (yellow oil, 160 mg, yield 92%).

### Step E: N-(1-(4-(4-(1-(5-(2,6-Dioxopiperidin-3-yl)oxy)pyridin-2-yl)piperidin-4-yl)methyl)piperidin-1-yl)phenyl)-3-(2-hydroxypropyl)-1H-pyrazol-4-yl)-6-(trifluoromethyl)picolinamide

A mixture of 1-(5-((2,6-dioxopiperidin-3-yl)oxy)pyridin-2-yl)piperidine-4-carbaldehyde (50 mg, 0.16 mmol, 1.0 eq), 1 drop ofHOAc, and N-(3-(2-hydroxypropyl)-1-(4-(piperidine-1-carbonyl)phenyl)-1H-pyrazol-4-yl)-6-(trifluoromethyl)picolinamide (75 mg, 0.16 mmol, 1.0 eq) in DMF (6 mL) was stirred at rt for 2 h. Then, sodium triacetoxyborohydride (133 mg, 0.63 mmol, 4.0 eq) was added, and the reaction solution was stirred at rt for 1 additional hour. The reaction solution was quenched with aqueous NH₄Cl at 0°C. The mixture was extracted with EtOAc (×3). The combined organic phases were washed with water and saturated aqueous NaCl, dried over anhydrous Na₂SO₄, and concentrated under reduced pressure. The residue was purified by column chromatography to give the crude. The crude was further purified by preparative HPLC to afford the title compound (white solid, 28 mg, yield 23%). LCMS (ESI): m/z 776 [M+H]⁺. **¹H NMR** (400 MHz, DMSO-*d6*) δ 11.21 (s, 1H), 10.92 (s, 1H), 8.75 (s, 1H), 8.47-8.35 (m, 2H), 8.18 (d, *J =* 7.6 Hz, 1H), 7.93 (d, *J* = 3.0 Hz, 1H), 7.61 (d, *J* = 8.9 Hz, 2H), 7.32 (dd, *J* = 9.2, 3.0 Hz, 1H), 7.03 (d, *J* = 9.1 Hz, 2H), 6.79 (d, *J =* 9.3 Hz, 1H), 5.94 (s, 1H), 4.99 (dd, *J* = 10.6, 5.0 Hz, 1H), 4.14 (d, *J* = 12.5 Hz, 2H), 3.49-3.29 (m, 4H), 3.17 (s, 4H), 2.75-2.55 (m, 4H), 2.25-2.16 (m, 3H), 2.15-2.08 (m, 1H), 1.88-1.68 (m, 3H), 1.58 (s, 6H), 1.17-1.06 (m, 2H).

### Preparation Example 445. Synthesis of Compound A438

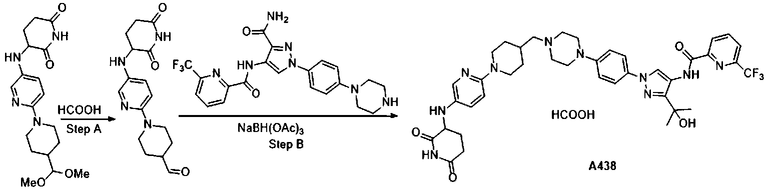

### Step A: 1-(5-((2,6-Dioxopiperidin-3-yl)amino)pyridin-2-yl)piperidine-4-carbaldehyde

3-((6-(4-(Dimethoxymethyl)piperidin-1-yl)pyridin-3-yl)amino)piperidine-2,6-dione (170 mg, 0.6 mmol, 1.0 eq) was added to a solution of formic acid (5 mL) at rt. The mixture was stirred at rt for 1 h. Upon completion, the reaction solution was concentrated under reduced pressure to afford the title compound (green oil, 140 mg, crude).

### Step B: N-(1-(4-(4-((1-(5-((2,6-Dioxopiperidin-3-yl)amino)pyridin-2-yl)piperidin-4-yl)methyl)piperazin-1-yl)phenyl)-3-(2-hydroxypropan-2-yl)-1H-pyrazol-4-yl)-6-(trifluoromethyl)picolinamide formate

To a solution of 1-(5-((2,6-dioxopiperidin-3-yl)amino)pyridin-2-yl)piperidine-4-carbaldehyde (140 mg, crude) in DMF (5 mL) was added N-(3-carbamoyl-1-(4-(piperazin-1-yl)phenyl)-1H-pyrazol-4-yl)-6-(trifluoromethyl)picolinamide (210 mg, 0.5 mmol, 1.0 eq) at rt. The mixture was stirred at rt under N₂ for 30 min. Then NaBH(OAc)₃ (456 mg, 2.1 mmol, 4.0 eq) was added, and the mixture was stirred for 1 additional hour. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 38.8 mg, yield 17%). **¹H NMR** (400 MHz, DMSO) δ 11.21 (s, 1H), 10.77 (s, 1H), 8.75 (s, 1H), 8.47-8.42 (m, 1H), 8.41-8.34 (m, 1H), 8.18 (dd, J = 7.6, 0.9 Hz, 1H), 8.15 (s, 1H), 7.70 (d, J = 2.9 Hz, 1H), 7.61 (d, J = 9.0 Hz, 2H), 7.06-7.00 (m, 3H), 6.68 (d, J = 9.0 Hz, 1H), 5.93 (s, 1H), 5.36 (s, 1H), 4.20 (dd, J = 10.9, 4.2 Hz, 1H), 4.01 (d, J = 12.5 Hz, 2H), 3.17 (s, 4H), 2.76-2.71 (m, 1H), 2.69-2.64 (m, 1H), 2.62-2.58 (m, 2H), 2.56-2.51 (m, 4H), 2.23 (d, J = 6.7 Hz, 2H), 2.15-2.08 (m, 1H), 1.91-1.83 (m, 1H), 1.80-1.68 (m, 3H), 1.58 (s, 6H), 1.19-1.09 (m, 2H). LCMS (ESI): m/z 774 [M+H]⁺.

### Preparation Example 446. Synthesis of Compound A439

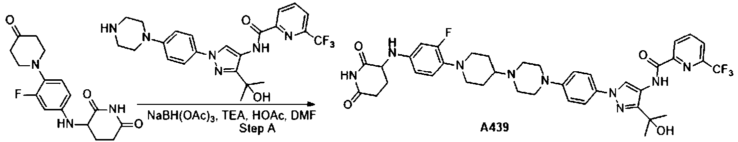

### Step A: N-(1-(4-(4-(1-(-4-((2,6-Dioxopiperidin-3-yl)amino)-2-fluorophenyl)piperidin-4-yl)piperazin-1-yl)phenyl)-3-(2-hydroxypropan-2-yl)-1H-pyrazol-4-yl)-6-(trifluoromethyl)picolinamide

At rt, to a solution of N-(3-(2-hydroxypropan-2-yl)-1-(4-(piperazin-1-yl)phenyl)-1H-pyrazol-4-yl)-6-(trifluoromethyl)picolinamide (100 mg, 0.21 mmol, 1.0 eq) and 3-(3-fluoro-4-(4-oxopiperidin-1-yl)phenyl)amino)piperidine-2,6-dione (102 mg, 0.32 mmol, 1.5 eq) in DMF (3 mL) was added TEA (0.1 mL, 0.63 mmol, 3.0 eq). The reaction solution was stirred at rt for 10 min, and then AcOH (63 mg, 1.05 mmol, 5.0 eq) was added. The reaction solution was stirred at rt overnight, and then sodium triacetoxyborohydride (134 mg, 1.63 mmol, 3.0 eq) was added. The reaction solution was stirred at 25°C for 2 h. Upon completion, the mixture was purified by HPLC to afford the title compound (yellow solid, 71.4 mg, yield 44%). LCMS (ESI): m/z 778 [M+H]⁺. **¹H NMR** (400 MHz, DMSO-*d6*) δ 11.20 (s, 1H), 10.77 (s, 1H), 8.75 (s, 1H), 8.46-8.31 (m, 2H), 8.18 (dd, *J=* 7.6, 1.0 Hz, 1H), 7.66-7.55 (m, 2H), 7.07-6.98 (m, 2H), 6.89-6.79 (m, 1H), 6.54-6.47 (m, 1H), 6.45-6.36 (m, 1H), 5.93 (s, 1H), 5.79 (d, *J* = 7.6 Hz, 1H), 4.31-4.20 (m, 1H), 3.22-3.13 (m, 6H), 2.79-2.71 (m, 1H), 2.70-2.64 (m, 4H), 2.62-2.54 (m, 3H), 2.37-2.30 (m, 1H), 2.12-2.04 (m, 1H), 1.90-1.81 (m, 3H), 1.64-1.54 (m, 8H).

### Preparation Example 447. Synthesis of Compound A440

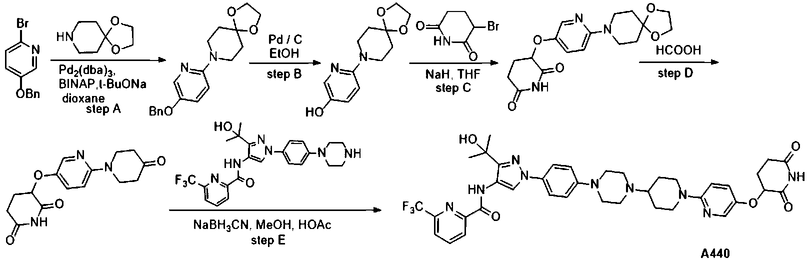

### Step A: 8-(5-(Benzyloxy)pyridin-2-yl)-1,4-dioxa-8-azaspiro[4.5]decane

To a solution of 5-(benzyloxy)-2-bromopyridine (1 g, 3.8 mmol, 1.0 eq) and 1,4-dioxa-8-azaspiro[4.5]decane (651 mg, 4.5 mmol, 1.2 eq) in dioxane (50 mL) were added Pd₂(dba)₃ (347 mg, 0.4 mmol, 0.1 eq), BINAP (472 mg, 0.8 mmol, 0.2 eq), and sodium tert-butoxide (728 mg, 7.6 mmol, 2.0 eq). The reaction mixture was degassed (×3) under N₂ and then heated to 120°C and stirred for 18 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 800 mg, yield 65%).

### Step B: 6-(1,4-Dioxa-8-azaspiro[4.5]decan-8-yl)pyridin-3-ol

To a solution of 8-(5-(benzyloxy)pyridin-2-yl)-1,4-dioxa-8-azaspiro[4.5]decane (800 mg, 2.5 mmol, 1.0 eq) in ethanol (50 mL) was added Pd/C (150 mg, w.t.= 10%). The reaction mixture was degassed with H₂ (×3) and then stirred under H₂ at rt for 4 h. Upon completion, the reaction mixture was filtered through a pad of Celite and concentrated under reduced pressure to afford the title compound (yellow solid, 520 mg, yield 90%).

### Step C: 3-((6-(1,4-Dioxa-8-azaspiro[4.5]decan-8-yl)pyridin-3-yl)oxy)piperidine-2,6-dione

To a solution of 6-(1,4-dioxa-8-azaspiro[4.5]decan-8-yl)pyridin-3-ol (520 mg, 2.2 mmol, 1.0 eq) in THF (50 mL) was added NaH (176 mg, 4.4 mmol, 2.0 eq, 60%) at 0°C. The mixture was stirred at 0°C under N₂ for 10 min. Then 3-bromopiperidine-2,6-dione (845 mg, 4.4 mmol, 2.0 eq) was added at 0°C. The mixture was stirred at 60°C under N₂ for 2 h. Upon completion, the reaction solution was quenched with saturated aqueous NH₄Cl and extracted with EtOAc. The combined organic phases were washed sequentially with water and saturated aqueous NaCl, dried over anhydrous Na₂SO₄, and concentrated under reduced pressure. The residue was purified by column chromatography to afford the title compound (white solid, 500 mg, yield 65%).

### Step D: 3-((6-(4-Oxopiperidin-1-yl)pyridin-3-yl)oxy)piperidine-2,6-dione

3-((6-(1,4-dioxa-8-azaspiro[4.5]decan-8-yl)pyridin-3-yl)oxy)piperidine-2,6-dione (150 mg, 0.4 mmol, 1.0 eq) was added to a solution of formic acid (3 mL). The mixture was stirred at 70°C for 16 h. Upon completion, the mixture was concentrated under reduced pressure to afford the title compound (yellow oil, 130 mg, crude).

### Step E: N-(1-(4-(4-(1-(5-((2,6-Dioxopiperidin-3-yl)oxy)pyridin-2-yl)piperidin-4-yl)piperazin-1-yl)phenyl)-3-(2-hydroxypropan-2-yl)-1H-pyrazol-4-yl)-6-(trifluoromethyl)picolinamide

To a solution of N-(3-(2-hydroxypropan-2-yl)-1-(4-(piperazin-1-yl)phenyl)-1H-pyrazol-4-yl)-6-(trifluoromethyl)picolinamide (204 mg, 0.4 mmol, 1.0 eq) in DMF (3 mL) were added 3-((6-(4-oxopiperidin-1-yl)pyridin-3-yl)oxy)piperidine-2,6-dione (130 mg, crude) and AcOH (0.2 mL). The mixture was stirred at rt under N₂ for 16 h, and then sodium triacetoxyborohydride (181 mg, 0.8 mmol, 2.0 eq) was added. The mixture was stirred at rt for 1 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (pale yellow solid, 37.5 mg, yield 11%). LCMS (ESI): m/z 762 [M+H]⁺. **¹H NMR** (400 MHz, DMSO) δ 11.19 (s, 1H), 10.91 (s, 1H), 8.74 (s, 1H), 8.46-8.35 (m, 2H), 8.20-8.15 (m, 1H), 7.93 (d, *J =* 3.1 Hz, 1H), 7.60 (d, *J* = 9.1 Hz, 2H), 7.33 (dd, *J* = 9.2, 3.1 Hz, 1H), 7.02 (d, *J* = 9.2 Hz, 2H), 6.82 (d, *J* = 9.3 Hz, 1H), 5.93 (s, 1H), 5.00 (dd, *J* = 10.6, 5.0 Hz, 1H), 4.19 (d, *J* = 12.9 Hz, 2H), 3.16 (s, 4H), 2.74 (t, *J* = 11.5 Hz, 2H), 2.67 (s, 4H), 2.64-2.55 (m, 2H), 2.45 (s, 1H), 2.23-2.07 (m, 2H), 1.87 (d, *J =* 10.6 Hz, 2H), 1.57 (s, 6H), 1.43 (dd, *J =* 22.2, 10.4 Hz, 2H).

### Preparation Example 448. Synthesis of Compound A441

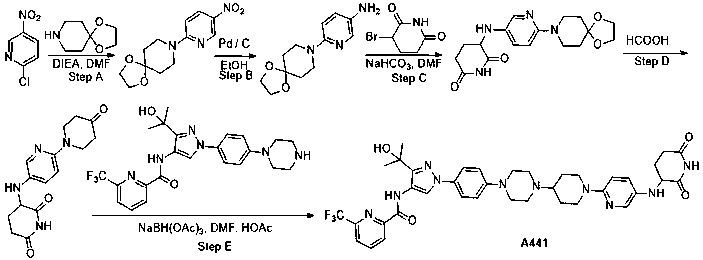

### Step A: 8-(5-Nitropyridin-2-yl)-1,4-dioxa-8-azaspiro[4.5]decane

To a solution of 8-aza-1,4-dioxaspiro[4.5]decane (1 g, 6.98 mmol, 1.0 eq) in DMF (15 mL) were added 2-chloro-5-nitropyridine (1.33 g, 8.38 mmol, 1.2 eq) and DIEA (3.2 mL, 20.95 mmol, 3.0 eq) at rt. The mixture was then stirred at 80°C for 2 h. Upon completion, the mixture was diluted in EtOAc, washed sequentially with water and saturated aqueous NaCl, dried over anhydrous Na₂SO₄, and concentrated under reduced pressure. The residue was purified by column chromatography to afford the title compound (yellow solid, 1.2 g, yield 65%).

### Step B: 6-(1,4-Dioxa-8-azaspiro[4.5]decan-8-yl)pyridin-3-amine

To a solution of 8-(5-nitropyridin-2-yl)-1,4-dioxa-8-azaspiro[4.5]decane (1.2 g, 4.52 mmol, 1.0 eq) in ethanol (40 mL) was added palladium on carbon (10%, 0.12 g, 1.13 mmol, 0.25 eq) at rt. The reaction solution was stirred under H₂ (1 atm) at rt for 18 h. Upon completion, the reaction solution was filtered through Celite and concentrated under reduced pressure to afford the title compound (yellow solid, 900 mg, crude), which was used directly without purification.

### Step C: 3-((6-(1,4-Dioxa-8-azaspiro[4.5]decan-8-yl)pyridin-3-yl)amino)piperidine-2,6-dione

To a solution of 6-(1,4-dioxa-8-azaspiro[4.5]decan-8-yl)pyridin-3-amine (900 mg, 3.83 mmol, 1.0 eq) in DMF (15 mL) were added 3-bromopiperidine-2,6-dione (881 mg, 4.59 mmol, 1.2 eq) and NaHCO₃ (964 mg, 11.48 mmol, 3.0 eq) at rt. The reaction mixture was then stirred at 90°C for 18 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (brown solid, 500 mg, yield 38%).

### Step D: 3-((6-(4-Oxopiperidin-1-yl)pyridin-3-yl)amino)piperidine-2,6-dione

3-((6-(1,4-dioxa-8-azaspiro[4.5]decan-8-yl)pyridin-3-yl)amino)piperidine-2,6-dione (200 mg, 0.58 mmol, 1.0 eq) was dissolved in formic acid (5 mL) at rt. The reaction solution was stirred at 70°C for 3 h. Upon completion, the reaction solution was concentrated under reduced pressure to afford the title compound (brown solid, 150 mg, crude), which was used directly without purification.

### Step E: N-(1-(4-(4-(1-(5-(2,6-Dioxopiperidin-3-yl)amino)pyridin-2-yl)piperidin-4-yl)piperazin-1-yl)phenyl)-3-(2-hydroxypropan-2-yl)-1H-pyrazol-4-yl)-6-(trifluoromethyl)picolinamide

To a solution of 3-((6-(4-oxopiperidin-1-yl)pyridin-3-yl)amino)piperidine-2,6-dione (150 mg, 0.5 mmol, 1.0 eq) in DMF (3 mL) were added N-(3-(2-hydroxypropan-2-yl)-1-(4-(piperazin-1-yl)phenyl)-1H-pyrazol-4-yl)-6-(trifluoromethyl)picolinamide (235 mg, 0.5 mmol, 1.0 eq) and AcOH (0.1 mL, 3.5 eq) at rt. The reaction solution was stirred at rt under N₂ for 16 h. Then sodium triacetoxyborohydride (523 mg, 2.48 mmol, 5.0 eq) was added. The mixture was stirred for an additional 2 h. Upon completion, the reaction solution was diluted in EtOAc, washed sequentially with water and saturated aqueous NaCl, dried over anhydrous Na₂SO₄, and concentrated under reduced pressure. The residue was purified by preparative chromatography to afford the title compound (brown solid, 5.8 mg, yield 2%). **¹H NMR** (400 MHz, MeOD-d4, formate) δ 8.71 (s, 1H), 8.45-8.42 (m, 2H), 8.28 (t, *J* = 7.8 Hz, 1H), 8.02 (d, *J* = 7.8 Hz, 1H), 7.74 (d, *J* = 2.9 Hz, 1H), 7.62 (d, *J* = 8.9 Hz, 2H), 7.19 (dd, *J* = 9.0, 2.8 Hz, 1H), 7.10 (d, *J* = 8.9 Hz, 2H), 6.82 (d, *J* = 8.9 Hz, 1H), 4.20 (dd, *J =* 11.7, 4.8 Hz, 1H), 4.12 (dd, *J =* 18.3, 6.1 Hz, 2H), 3.45-3.25 (m, 5H), 3.10-3.02 (m, 4H), 2.87-2.67 (m, 5H), 2.39-2.19 (m, 1H), 2.11 (dd, *J* = 16.8, 6.1 Hz, 2H), 2.05-1.85 (m, 2H), 1.67 (s, 6H). LCMS (ESI): m/z 761.5 [M+H]⁺.

### Preparation Example 449. Synthesis of Compound A442

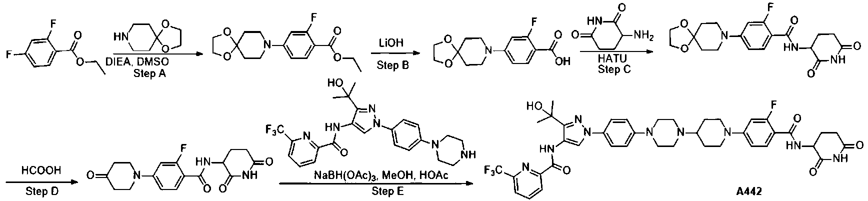

### Step A: Ethyl 2-fluoro-4-(1,4-dioxa-8-azaspiro[4.5]decan-8-yl)benzoate

A mixture of ethyl 2,4-difluorobenzoate (1 g, 5.37 mmol, 1.0 eq), 1,4-dioxa-8-azaspiro[4.5]decane (0.85 g, 5.91 mmol, 1.1 eq), DIEA (2.1 g, 16.12 mmol, 3.0 eq), and DMSO (10 mL) was heated to 80°C and reacted for 16 h. The reaction mixture was diluted in water and extracted with EtOAc (×3). The combined organic phases were washed with water and saturated aqueous NaCl, dried over anhydrous Na₂SO₄, filtered, and concentrated to obtain a crude, which was purified by column chromatography to afford the title compound (white solid, 1 g, yield 57%).

### Step B: 2-Fluoro-4-(1,4-dioxa-8-azaspiro[4.5]decan-8-yl)benzoic acid

Ethyl 2-fluoro-4-(1,4-dioxa-8-azaspiro[4.5]decan-8-yl)benzoate (1 g, 3.23 mmol, 1.0 eq) was dissolved in MeOH (10 mL). The mixture was cooled to 0°C, and a NaOH solution (2 N, 5 mL) was added. The mixture was reacted at rt for 2 h. The reaction mixture was adjusted to pH 3 with 1 N HCl and extracted with EtOAc (×3). The combined organic phases were washed with water and saturated aqueous NaCl, dried over anhydrous Na₂SO₄, filtered, and concentrated to afford the title compound (white solid, 900 mg, yield 94%).

### Step C: N-(2,6-Dioxopiperidin-3-yl)-2-fluoro-4-(1,4-dioxa-8-azaspiro[4.5]decan-8-yl)benzamide

To a solution of 2-fluoro-4-(1,4-dioxa-8-azaspiro[4.5]decan-8-yl)benzoic acid (900 mg, 3.2 mmol, 1.0 eq) in DMF (15 mL) were added 3-aminopiperidine-2,6-dione (492 mg, 3.84 mmol, 1.2 eq), DIEA (1.24 g, 9.6 mmol, 3.0 eq), and HATU (1.8 g, 4.80 mmol, 1.5 eq) in an ice bath. The mixture was reacted at rt for 2 h. The reaction mixture was diluted in water and extracted with EtOAc (×3). The combined organic phases were washed with water and saturated aqueous NaCl, dried over anhydrous Na₂SO₄, filtered, and concentrated to obtain a crude, which was purified by column chromatography to afford the title compound (white solid, 1 g, yield 76%).

### Step D: N-(2,6-Dioxopiperidin-3-yl)-2-fluoro-4-(4-oxopiperidin-1-yl)benzamide

N-(2,6-Dioxopiperidin-3-yl)-2-fluoro-4-(1,4-dioxa-8-azaspiro[4.5]decan-8-yl)benzamide (1 g, 2.55 mmol) was dissolved in formic acid (5 mL). The mixture was heated to 80°C and reacted for 2 h. The reaction mixture was concentrated to afford the title compound (yellow solid, 700 mg, crude).

### Step E: N-(1-(4-(4-(1-(4-((2,6-dioxopiperidin-3-yl)carbamoyl)-3-fluorophenyl)piperidin-4-yl)piperazin-1-yl)phenyl)-3-(2-hydroxypropan-2-yl)-1H-pyrazol-4-yl)-6-(trifluoromethyl)picolinamide

N-(2,6-Dioxopiperidin-3-yl)-2-fluoro-4-(4-oxopiperidin-1-yl)benzamide (100 mg, 0.29 mmol, 1.0 eq) was dissolved in DMF (5 mL). AcOH (0.1 mL) and N-(3-(2-hydroxypropan-2-yl)-1-(4-(piperazin-1-yl)phenyl)-1H-pyrazol-4-yl)-6-(trifluoromethyl)picolinamide (205 mg, 0.43 mmol, 1.5 eq) were added. The mixture was stirred at rt for 1 h. Then sodium triacetoxyborohydride (16 mg, 0.43 mmol, 1.5 eq) was added, and the mixture was stirred at rt for 2 h. The reaction mixture was diluted in water and extracted with EtOAc (×3). The combined organic phases were washed with water and saturated aqueous NaCl, dried over anhydrous Na₂SO₄, filtered, and concentrated to obtain a crude, which was purified by HPLC to afford the title compound (white solid, 42 mg, yield 17%). LCMS (ESI): m/z 806 [M+H]⁺. **¹H NMR** (400 MHz, DMSO-*d₆*) δ 11.21 (s, 1H), 10.85 (s, 1H), 8.79 (s, 1H), 8.41 (dt, *J =* 15.5, 7.6 Hz, 2H), 8.19 (d, *J =* 7.5 Hz, 1H), 8.09-8.04 (m, 1H), 7.67 (dd, *J =* 16.5, 8.9 Hz, 3H), 7.13 (d, *J* = 9.3 Hz, 2H), 6.92-6.85 (m, 2H), 5.96 (s, 1H), 4.78-4.69 (m, 1H), 4.11 (d, *J=* 12.1 Hz, 2H), 3.91 (d, *J* = 12.2 Hz, 2H), 3.66 (d, *J* = 11.1 Hz, 2H), 3.24 (d, *J =* 9.6 Hz, 3H), 3.01 (t, *J =* 12.2 Hz, 2H), 2.90 (t, *J=* 12.3 Hz, 2H), 2.77 (dd, *J =* 21.7, 8.9 Hz, 1H), 2.54 (s, 1H), 2.15 (t, *J* = 13.2 Hz, 3H), 2.01 (d, *J* = 5.7 Hz, 1H), 1.69 (d, *J=* 9.4 Hz, 2H), 1.58 (s, 6H).

### Preparation Example 450. Synthesis of Compound A443

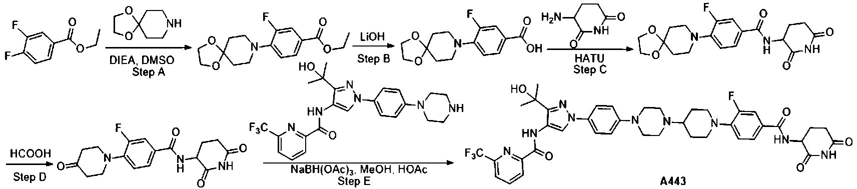

### Step A: Ethyl 3-fluoro-4-(1,4-dioxa-8-azaspiro[4.5]decan-8-yl)benzoate

Ethyl 3,4-difluorobenzoate (1 g, 5.37 mmol, 1.0 eq) was added to a solution of DMSO (10 mL), followed by the addition of 1,4-dioxa-8-azaspiro[4.5]decane (0.92 g, 6.45 mmol, 1.2 eq) and DIEA (2.08 g, 16.12 mmol, 3 eq). The mixture was stirred at 60°C overnight. After cooling to rt, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 1.4 g, yield 84%).

### Step B: 3-Fluoro-4-(1,4-dioxa-8-azaspiro[4.5]decan-8-yl)benzoic acid

To a solution of ethyl 3-fluoro-4-(1,4-dioxa-8-azaspiro[4.5]decan-8-yl)benzoate (1.4 g, 4.53 mmol, 1.0 eq) in MeOH (15 mL) and water (5 mL) was added LiOH (1.9 g, 45.26 mmol, 10.0 eq) at rt. The mixture was stirred at 50°C overnight. After cooling to rt, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 1.2 g, yield 94%).

### Step C: N-(2,6-Dioxopiperidin-3-yl)-3-fluoro-4-(1,4-dioxa-8-azaspiro[4.5]decan-8-yl)benzamide

To a solution of 3-fluoro-4-(1,4-dioxa-8-azaspiro[4.5]decan-8-yl)benzoic acid (1.2 g, 4.27 mmol, 1.0 eq) in DMF (10 mL) were added 3-aminopiperidine-2,6-dione (0.66 g, 5.12 mmol, 1.2 eq), HATU (1.95 g, 5.12 mmol, 1.2 eq), and DIEA (1.65 g, 12.80 mmol, 3.0 eq) at rt. The mixture was stirred at rt for 2 h. The reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 1 g, yield 60%).

### Step D: N-(2,6-Dioxopiperidin-3-yl)-3-fluoro-4-(4-oxopiperidin-1-yl)benzamide

A solution of N-(2,6-dioxopiperidin-3-yl)-3-fluoro-4-(1,4-dioxa-8-azaspiro[4.5]decan-8-yl)benzamide (500 mg, 1.28 mmol) in formic acid (5 mL) was stirred at 50°C for 3 h. The reaction solution was then concentrated in vacuo to afford the title compound (yellow solid, 300 mg, yield 68%).

### Step E: N-(1-(4-(4-(1-(4-((2,6-dioxopiperidin-3-yl)carbamoyl)-2-fluorophenyl)piperidin-4-yl)piperazin-1-yl)phenyl)-3-(2-hydroxypropan-2-yl)-1H-pyrazol-4-yl)-6-(trifluoromethyl)picolinamide

N-(2,6-Dioxopiperidin-3-yl)-3-fluoro-4-(4-oxopiperidin-1-yl)benzamide (200 mg, 0.58 mmol, 1.0 eq) and N-(3-(2-hydroxypropan-2-yl)-1-(4-(piperazin-1-yl)phenyl)-1H-pyrazol-4-yl)-6-(trifluoromethyl)picolinamide (410 mg, 0.86 mmol, 1.48 eq) were added to MeOH (5 mL). HOAc (21 mg, 0.12 mmol, 0.2 eq) was added, and the mixture was stirred for 16 h. Then sodium triacetoxyborohydride (181 mg, 2.88 mmol, 4.96 eq) was added, and the mixture was stirred at 25°C for 2 h. The reaction solution was diluted in EtOAc, washed sequentially with water and saturated aqueous NaCl, dried over anhydrous Na₂SO₄, and concentrated under reduced pressure. The residue was purified by preparative HPLC to afford the title compound (white solid, 44 mg, yield 9%). LCMS (ESI): m/z 806 [M+H]⁺. **¹H NMR** (400 MHz, DMSO-d6) δ 11.20 (s, 1H), 10.85 (s, 1H), 8.74 (s, 1H), 8.66 (d, *J =* 8.3 Hz, 1H), 8.44-8.36 (m, 2H), 8.18 (d, *J=* 7.9 Hz, 1H), 7.67-7.59 (m, 4H), 7.11 (t, *J* = 8.8 Hz, 1H), 7.03 (d, *J* = 9.1 Hz, 2H), 5.93 (s, 1H), 4.79-4.72 (m, 1H), 3.56 (d, *J* = 11.2 Hz, 2H), 3.21-3.14 (m, 5H), 2.83-2.74 (m, 3H), 2.69 (s, 4H), 2.58-2.42 (m, 2H), 2.14-2.06 (m, 1H), 1.99-1.89 (m, 3H), 1.66-1.59 (m, 1H), 1.58 (s, 6H).

### Preparation Example 451. Synthesis of Compound A444

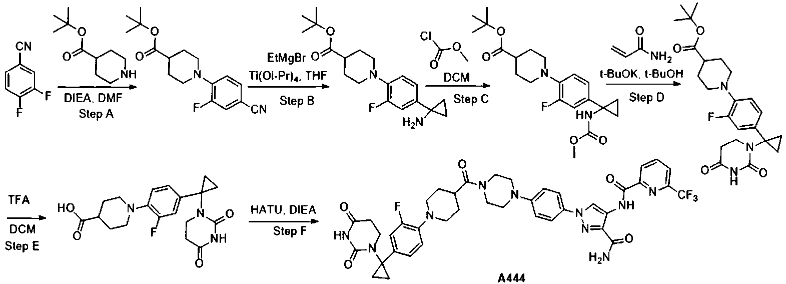

### Step A: Tert-butyl 1-(4-cyano-2-fluorophenyl)piperidine-4-carboxylate

To a solution of 3,4-difluorobenzonitrile (5 g, 35.9 mmol, 1.0 eq) and tert-butyl piperidine-4-carboxylate (6.66 g, 35.9 mmol, 1.0 eq) in DMF (75 mL) was added DIEA (19 mL, 108 mmol, 3.0 eq) at rt. The mixture was reacted at 120°C for 16 h. The mixture was diluted in EtOAc, washed sequentially with water and saturated aqueous NaCl, dried over anhydrous Na₂SO₄, and concentrated under reduced pressure. The residue was purified by column chromatography to afford the title compound (yellow solid, 4.5 g, yield 41%).

### Step B: Tert-butyl 1-(4-(1-aminocyclopropyl)-2-fluorophenyl)piperidine-4-carboxylate

At -25°C, to a solution of tert-butyl 1-(4-cyano-2-fluorophenyl)piperidine-4-carboxylate (2 g, 6.57 mmol, 1.0 eq) in THF (30.0 mL) was added tetraisopropyl titanate (2.05 g, 7.23 mmol, 1.1 eq), followed by the addition of ethylmagnesium bromide solution (14.5 mL, 14.5 mmol, 2.2 eq, 1 M in THF). After stirring for 1 h, BF₃·OEt₂ (1.87 g, 13.1 mmol, 2.0 eq) was added, and the mixture was stirred for an additional 0.5 h. Upon completion, the reaction was quenched with 3 N HCl, and the pH was adjusted to 9 with 3 N NaOH. The mixture was diluted in DCM and washed with aqueous NaCl. The organic layer was separated. The aqueous layer was extracted with DCM. The organic layer was dried over anhydrous Na₂SO₄, and concentrated under reduced pressure. The residue was purified by column chromatography to afford the title compound (yellow solid, 500 mg, yield 23%).

### Step C: Tert-butyl 1-(2-fluoro-4-(1-((methoxycarbonyl)amino)cyclopropyl)phenyl)piperidine-4-carboxylate

To a solution of tert-butyl 1-(4-(1-aminocyclopropyl)-2-fluorophenyl)piperidine-4-carboxylate (400 mg, 1.2 mmol, 1.0 eq) and TEA (4.8 mL, 3.0 eq) in DCM (10 mL) was added methyl chloroformate (135 mg, 1.44 mmol, 1.2 eq) at 0°C. The reaction solution was stirred at rt under N₂ for 2 h. Upon completion, the reaction solution was diluted in DCM, washed sequentially with water and saturated aqueous NaCl, dried over anhydrous Na₂SO₄, and concentrated under reduced pressure. The residue was purified by column chromatography to afford the title compound (white solid, 120 mg, yield 26%).

### Step D: Tert-butyl 1-(4-(1-(2,4-dioxotetrahydropyrimidin-1(2H)-yl)cyclopropyl)-2-fluorophenyl)piperidine-4-carboxylate

To a solution of tert-butyl 1-(2-fluoro-4-(1-((methoxycarbonyl)amino)cyclopropyl)phenyl)piperidine-4-carboxylate (100 mg, 0.25 mmol, 1.0 eq) in tert-butanol (10 mL) were added acrylamide (36 mg, 0.5 mmol, 2.0 eq) and potassium tert-butoxide (43 mg, 0.38 mmol, 1.5 eq) at rt. The mixture was stirred at 60°C under N₂ for 16 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 30 mg, yield 28%).

### Step E: 1-(4-(1-(2,4-Dioxotetrahydropyrimidin-1(2H)-yl)cyclopropyl)-2-fluorophenyl)piperidine-4-carboxylic acid

To a solution of tert-butyl 1-(4-(1-(2,4-dioxotetrahydropyrimidin-1(2H)-yl)cyclopropyl)-2-fluorophenyl)piperidine-4-carboxylate (30 mg, 0.07 mmol, 1.0 eq) in DCM (5 mL) was added TFA (1 mL) at 0°C. The reaction solution was stirred at rt for 0.5 h under N₂. Upon completion, the mixture was concentrated under reduced pressure to afford the title compound (white solid, 25 mg, yield 96%).

### Step F: N-(3-Carbamoyl-1-(4-(4-(1-(4-(1-(2,4-dioxotetrahydropyrimidin-1(2H)-yl)cyclopropyl)-2-fluorophenyl)piperidine-4-carbonyl)piperazin-1-yl)phenyl)-1H-pyrazol-4-yl)-6-(trifluoromethyl)picolinamide

To a solution of 1-(4-(1-(2,4-dioxotetrahydropyrimidin-1(2H)-yl)cyclopropyl)-2-fluorophenyl)piperidine-4-carboxylic acid (25 mg, 0.07 mmol, 1.0 eq) in DMF (6 mL) were added HATU (53 mg, 0.14 mmol, 2.0 eq), N-(3-carbamoyl-1-(4-(piperazin-1-yl)phenyl)-1H-pyrazol-4-yl)-6-(trifluoromethyl)picolinamide (39 mg, 0.08 mmol, 1.2 eq), and DIEA (36 mg, 0.28 mmol, 4.0 eq) at rt. The mixture was stirred at rt for 0.5 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (white solid, 2.0 mg, yield 4%). LCMS (ESI): m/z 817 [M+H]⁺. **¹H NMR** (400 MHz, DMSO-*d6*) δ 11.79 (s, 1H), 10.09 (s, 1H), 8.90 (s, 1H), 8.48-8.35 (m, 2H), 8.25-8.15 (m, 1H), 7.96 (s, 1H), 7.83 (d, *J=* 9.1 Hz, 2H), 7.72 (s, 1H), 7.12 (d, *J=* 9.2 Hz, 2H), 7.00-6.91 (m, 3H), 3.76-3.60 (m, 4H), 3.44 (t, *J=* 6.6 Hz, 2H), 3.36 (m, 1H), 3.28-3.16 (m, 4H), 2.92-2.78 (m, 1H), 2.78-2.69 (m, 2H), 2.59-2.53 (m, 3H), 1.79-1.70 (m, 4H), 1.34-1.29 (m, 2H), 1.26-1.19 (m, 2H).

### Preparation Example 452. Synthesis of Compound A445

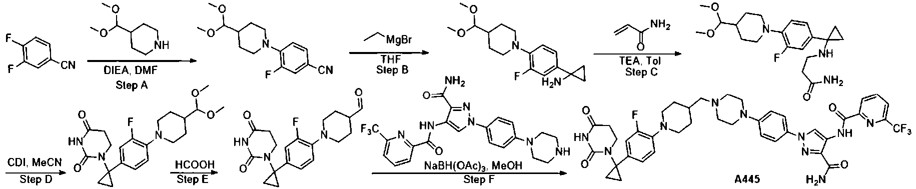

### Step A: 4-(4-(Dimethoxymethyl)piperidin-1-yl)-3-fluorobenzonitrile

A solution of 3,4-difluorobenzonitrile (5.0 g, 35.9 mmol, 1.0 eq), 4-(dimethoxymethyl)piperidine (6.3 g, 39.5 mmol, 1.1 eq), and DIEA (13.9 g, 108 mmol, 3.0 eq) in DMF (75 mL) was stirred at 120°C under N₂ for 16 h. After cooling to rt, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 7.5 g, yield 75%).

### Step B: 1-(4-(4-(Dimethoxymethyl)piperidin-1-yl)-3-fluorophenyl)cyclopropan-1-amine

To a solution of 4-(4-(dimethoxymethyl)piperidin-1-yl)-3-fluorobenzonitrile (2.0 g, 7.19 mmol, 1.0 eq) in THF (30 mL) was added tetraisopropyl titanate (2.25 g, 7.90 mmol, 1.1 eq) at -25°C, followed by the addition of ethylmagnesium bromide solution (2 M, 7.9 mL, 15.8 mmol, 2.2 eq) in tetrahydrofuran. After stirring for 1 h, boron trifluoride diethyl etherate (1.8 mL, 14.4 mmol, 2 eq) was added, and the mixture was stirred for an additional 0.5 h. The reaction was quenched with 3 N HCl at -25°C. The pH was adjusted to approximately 9 with 3 N NaOH. The mixture was diluted in DCM, washed sequentially with water and saturated aqueous NaCl, dried over anhydrous Na₂SO₄, and concentrated under reduced pressure. The residue was purified by column chromatography to afford the title compound (yellow solid, 500 mg, yield 22%).

### Step C: 3-((1-(4-(Dimethoxymethyl)piperidin-1-yl)-3-fluorophenyl)cyclopropyl)amino)propanamide

To a solution of 1-(4-(4-(dimethoxymethyl)piperidin-1-yl)-3-fluorophenyl)cyclopropan-1-amine (1.1 g, 2.14 mmol, 1.0 eq) in ethanol (40 mL) were added acrylamide (1.5 g, 21.4 mmol, 10.0 eq) and TEA (0.6 mL, 4.28 mmol, 2.0 eq). The mixture was stirred at 80°C overnight. Upon completion, the reaction mixture was diluted in EtOAc and washed with saturated aqueous NaCl. The organic layer was separated. The aqueous layer was extracted with EtOAc. The organic layer was dried over anhydrous Na₂SO₄, filtered, and concentrated. The crude was purified by column chromatography to afford the title compound (white solid, 275 mg, yield 34%).

### Step D: 1-(1-(4-(4-(Dimethoxymethyl)piperidin-1-yl)-3-fluorophenyl)cyclopropyl)dihydropyrimidine-2,4(1H,3H)-dione

To a solution of 3-((1-(4-(dimethoxymethyl)piperidin-1-yl)-3-fluorophenyl)cyclopropyl)amino)propanamide (220 mg, 0.58 mmol, 1.0 eq) in acetonitrile (10 mL) were added N,N'-carbonyldiimidazole (470 mg, 2.89 mmol, 5.0 eq) and cesium carbonate (566 mg, 1.74 mmol, 3.0 eq). The mixture was stirred at 90°C overnight. The reaction mixture was diluted in EtOAc and washed with saturated aqueous NaCl. The organic layer was separated. The aqueous layer was extracted with EtOAc. The organic phase was dried over anhydrous Na₂SO₄, filtered, and concentrated. The crude was purified by column chromatography to afford the title compound (white solid, 170 mg, yield 72%).

### Step E: 1-(4-(1-(2,4-Dioxotetrahydropyrimidin-1(2H)-yl)cyclopropyl)-2-fluorophenyl)piperidine-4-carbaldehyde

Formic acid (3 mL) was added to a flask containing 1-(1-(4-(4-(dimethoxymethyl)piperidin-1-yl)-3-fluorophenyl)cyclopropyl)dihydropyrimidine-2,4(1H,3H)-dione (170 mg, 0.42 mmol, 1.0 eq). The mixture was stirred at rt for 2 h and then concentrated to afford the crude compound (white solid, 150 mg, crude), which was used directly in the next step without further purification.

### Step F: N-(3-Carbamoyl-1-(4-(4-((1-(4-(2,4-dioxotetrahydropyrimidin-1(2H)-yl)cyclopropyl)-2-fluorophenyl)piperidin-4-yl)methyl)piperazin-1-yl)phenyl)-1H-pyrazol-4-yl)-6-(trifluoromethyl)picolinamide

To a solution of 1-(4-(1-(2,4-dioxotetrahydropyrimidin-1(2H)-yl)cyclopropyl)-2-fluorophenyl)piperidine-4-carbaldehyde (150 mg, 0.42 mmol, 1.0 eq) in anhydrous MeOH (5 mL) were added TEA (0.12 mL, 0.83 mmol, 2.0 eq) and N-(3-carbamoyl-1-(4-(piperazin-1-yl)phenyl)-1H-pyrazol-4-yl)-6-(trifluoromethyl)picolinamide (192 mg, 0.42 mmol, 1.0 eq) at rt. The mixture was stirred at rt for 10 min, and then AcOH (0.12 mL, 2.09 mmol, 5.0 eq) was added. The mixture was stirred for 30 min. Sodium triacetoxyborohydride (264 mg, 1.25 mmol, 3.0 eq) was added to the mixture at rt, and the mixture was stirred overnight. After concentration, the residue was purified by HPLC to afford the title compound (white solid, 65 mg, yield 19%). LCMS (ESI): m/z 803 [M+H]⁺. **¹H NMR** (400 MHz, DMSO-*d*₆) δ 11.80 (s, 1H), 10.10 (s, 1H), 8.93 (s, 1H), 8.49-8.36 (m, 2H), 8.22 (d, *J* = 7.5 Hz, 1H), 7.98-7.73 (m, 4H), 7.18 (d, *J =* 9.1 Hz, 2H), 7.00-6.93 (m, 3H), 3.93 (d, *J* = 10.6 Hz, 2H), 3.65 (d, *J* = 9.9 Hz, 2H), 3.45 (t, *J* = 6.7 Hz, 2H), 3.35 (d, *J =* 12.1 Hz, 2H), 3.17 (d, *J =* 8.0 Hz, 6H), 2.69 (t, *J =* 11.2 Hz, 2H), 2.55 (t, *J* = 6.6 Hz, 2H), 2.01 (s, 1H), 1.88 (d, *J =* 11.2 Hz, 2H), 1.50-1.37 (m, 2H), 1.32 (s, 2H), 1.21 (s, 2H).

### Preparation Example 453. Synthesis of Compound A446

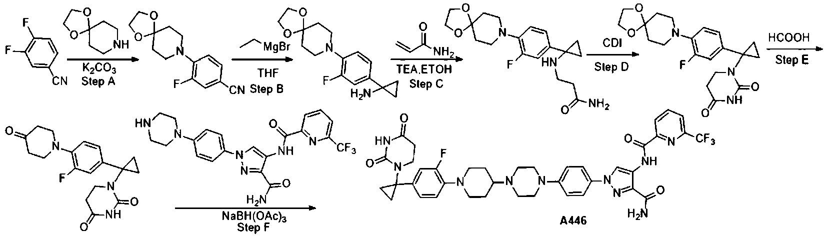

### Step A: 3-Fluoro-4-(1,4-dioxa-8-azaspiro[4.5]decan-8-yl)benzonitrile

To a solution of 3,4-difluorobenzonitrile (5 g, 35.9 mmol, 1.0 eq) and 1,4-dioxa-8-azaspiro[4.5]decane (4.9 g, 34.2 mmol, 1.0 eq) in DMF (50 mL) was added potassium carbonate (18.9 g, 136.9 mmol, 4.0 eq). The mixture was stirred at 120°C for 16 h. Upon completion, the mixture was diluted in EtOAc, washed sequentially with water and saturated aqueous NaCl, dried over anhydrous Na₂SO₄, and concentrated under reduced pressure. The residue was purified by column chromatography to afford the title compound (yellow solid, 8.4 g, yield 87%).

### Step B: 1-(3-Fluoro-4-(1,4-dioxa-8-azaspiro[4.5]decan-8-yl)phenyl)cyclopropan-1-amine

To a solution of 3-fluoro-4-(1,4-dioxa-8-azaspiro[4.5]decan-8-yl)benzonitrile (8.4 g, 32 mmol, 1.0 eq) in THF (100 mL) was added tetraisopropyl titanate (10 g, 35.2 mmol, 1.1 eq). The mixture was stirred under N₂ at rt for 10 min. Then ethylmagnesium bromide (3 M in tetrahydrofuran, 23.5 mL, 70.5 mmol, 2.2 eq) was added at -78°C. After stirring at -78°C for 1 h, boron trifluoride diethyl etherate solution (9.1 g, 64.1 mmol, 2.0 eq) was added. The mixture was warmed to rt and stirred for 30 min. The reaction solution was poured into saturated ammonium bicarbonate solution and extracted with EtOAc. The organic phases were combined, washed sequentially with water and saturated aqueous NaCl, dried over anhydrous Na₂SO₄, and concentrated under reduced pressure. The residue was purified by column chromatography to afford the title compound (yellow solid, 3 g, yield 30%).

### Step C: 3-((1-(3-Fluoro-4-(1,4-dioxa-8-azaspiro[4.5]decan-8-yl)phenyl)cyclopropyl)amino)propanamide

To a solution of 1-(3-fluoro-4-(1,4-dioxa-8-azaspiro[4.5]decan-8-yl)phenyl)cyclopropan-1-amine (1.5 g, 5.1 mmol, 1.0 eq) and TEA (1.4 mL, 10.3 mmol, 2.0 eq) in ethanol (20 mL) was added acrylamide (3.6 g, 51.3 mmol, 10.0 eq). The mixture was stirred at 80°C for 16 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 1 g, yield 54%).

### Step D: 1-(1-(3-Fluoro-4-(1,4-dioxa-8-azaspiro[4.5]decan-8-yl)phenyl)cyclopropyl)dihydropyrimidine-2,4(1H,3H)-dione

To a solution of 3-((1-(3-fluoro-4-(1,4-dioxa-8-azaspiro[4.5]decan-8-yl)phenyl)cyclopropyl)amino)propanamide (500 mg, 1.4 mmol, 1.0 eq) in acetonitrile (10 mL) were added N,N'-carbonyldiimidazole (1.1 g, 6.9 mmol, 5.0 eq) and cesium carbonate (2.2 g, 6.9 mmol, 5.0 eq). The mixture was stirred at 90°C for 2 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 200 mg, yield 37%).

### Step E: 1-(1-(3-Fluoro-4-(4-oxopiperidin-1-yl)phenyl)cyclopropyl)dihydropyrimidine-2,4(1H,3H)-dione

1-(1-(3-Fluoro-4-(1,4-dioxa-8-azaspiro[4.5]decan-8-yl)phenyl)cyclopropyl)dihydropyrimidine-2,4(1H,3H)-dione (200 mg, 0.5 mmol, 1.0 eq) was added to formic acid (10 mL). The mixture was stirred at 80°C for 16 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 100 mg, yield 56%).

### Step F: N-(3-Carbamoyl-1-(4-(4-(1-(4-(1-(2,4-dioxotetrahydropyrimidin-1(2H)-yl)cyclopropyl)-2-fluorophenyl)piperidin-4-yl)piperazin-1-yl)phenyl)-1H-pyrazol-4-yl)-6-(trifluoromethyl)picolinamide

To a solution of 1-(1-(3-fluoro-4-(4-oxopiperidin-1-yl)phenyl)cyclopropyl)dihydropyrimidine-2,4(1H,3H)-dione (100 mg, 0.3 mmol, 1.0 eq) and N-(3-carbamoyl-1-(4-(piperazin-1-yl)phenyl)-1H-pyrazol-4-yl)-6-(trifluoromethyl)picolinamide (133 mg, 0.3 mmol, 1.0 eq) in DMF (10 mL) was added AcOH (0.02 mL, 0.3 mmol, 1.0 eq). The mixture was stirred at 25°C for 4 h. Then sodium triacetoxyborohydride (122 mg, 0.6 mmol, 2.0 eq) was added, and the mixture was stirred for 1 h. The reaction solution was poured into water and extracted with EtOAc. The combined organic layers were washed with aqueous NaCl, dried over anhydrous Na₂SO₄, and concentrated under reduced pressure. The residue was purified by preparative chromatography to afford the title compound (white solid, 30 mg, yield 13%). LCMS (ESI): m/z 789 [M+H]⁺. **¹H NMR** (400 MHz, DMSO-*d*6) *δ* 11.80 (s, 1H), 10.10 (s, 1H), 8.94 (s, 1H), 8.42 (dt, *J* = 15.5, 7.7 Hz, 2H), 8.22 (d, *J=* 7.5 Hz, 1H), 7.96 (s, 1H), 7.88 (d, *J=* 8.8 Hz, 2H), 7.75 (s, 1H), 7.20 (d, *J* = 8.8 Hz, 2H), 7.09-6.76 (m, 3H), 4.00 (d, *J* = 12.8 Hz, 2H), 3.68 (d, *J* = 11.8 Hz, 2H), 3.51-3.43 (m, 6H), 3.28-3.25 (m, 1H), 3.06 (t, *J* = 12.3 Hz, 2H), 2.71 (t, *J* = 11.8 Hz, 2H), 2.57-2.53 (m, 2H), 2.20 (d, *J* = 11.7 Hz, 2H), 1.88-1.77 (m, 2H), 1.36-1.31 (m, 2H), 1.24-1.21 (m, 2H).

### Preparation Example 454. Synthesis of Compound A447

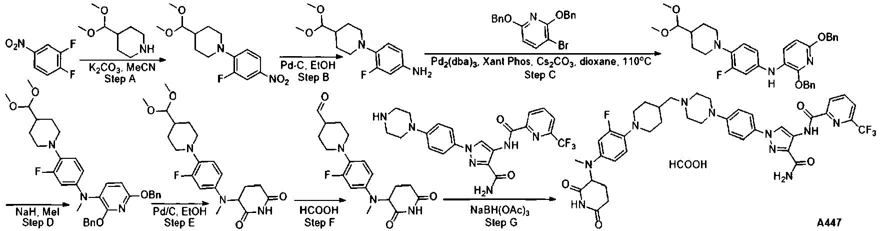

### Step A: 4-(Dimethoxymethyl)-1-(2-fluoro-4-nitrophenyl)piperidine

To a solution of 4-(dimethoxymethyl)piperidine (5 g, 31.40 mmol, 1.0 eq) and 1,2-difluoro-4-nitrobenzene (5.0 g, 31.40 mmol, 1.0 eq) in acetonitrile (50 mL) was added potassium carbonate (8.7 g, 62.80 mmol, 2.0 eq) at rt. The reaction solution was reacted at 80°C for 3 h. The reaction solution was filtered through Celite and subjected to rotary evaporation to dryness. The residue was purified by column chromatography to afford the title compound (yellow solid, 8 g, yield 85%).

### Step B: 4-(4-(Dimethoxymethyl)piperidin-1-yl)-3-fluoroaniline

To a solution of 4-(dimethoxymethyl)-1-(2-fluoro-4-nitrophenyl)piperidine (8 g, 26.82 mmol, 1.0 eq) in MeOH (100 mL) was added palladium on carbon (10%, 0.8 g) at rt. The reaction solution was reacted at rt overnight. The reaction solution was filtered through Celite and subjected to rotary evaporation to dryness to afford 4-(4-(dimethoxymethyl)piperidin-1-yl)-3-fluoroaniline (brown solid, 7 g, crude), which was used directly in the next reaction.

### Step C: 2,6-Bis(benzyloxy)-N-(4-(4-(dimethoxymethyl)piperidin-1-yl)-3-fluorophenyl)pyridin-3-amine

To a solution of 4-(4-(dimethoxymethyl)piperidin-1-yl)-3-fluoroaniline (2 g, crude), 2,6-bis(benzyloxy)-3-bromopyridine (3.3 g, 8.94 mmol, 1.2 eq), Xant Phos (0.9 g, 1.491 mmol, 0.2 eq), and cesium carbonate (7.3 g, 22.36 mmol, 3.0 eq) in dioxane (30 mL) was added tris(dibenzylideneacetone)dipalladium (0.7 g, 0.74 mmol, 0.1 eq) at rt. The reaction solution was stirred at 110°C overnight. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 2.5 g, yield 60% over two steps).

### Step D: 2,6-Bis(benzyloxy)-N-(4-(4-(dimethoxymethyl)piperidin-1-yl)-3-fluorophenyl)-N-methylpyridin-3-amine

To a solution of 2,6-bis(benzyloxy)-N-(4-(4-(dimethoxymethyl)piperidin-1-yl)-3-fluorophenyl)pyridin-3-amine (2.5 g, 4.48 mmol, 1.0 eq) in DMF (30 mL) was added sodium hydride (0.1 g, 5.38 mmol, 60%, 1.2 eq) at 0°C. After 30 min, iodomethane (0.6 mL, 6.72 mmol, 1.5 eq) was added at 0°C. The reaction solution was stirred at rt for 3 h. The reaction solution was quenched with saturated aqueous NH₄Cl and extracted with EtOAc. The organic phase was washed with saturated aqueous NaCl, dried over Na₂SO₄, and concentrated. The residue was purified by column chromatography to afford the title compound (white solid, 2 g, yield 78%).

### Step E: 3-((4-(4-(Dimethoxymethyl)piperidin-1-yl)-3-fluorophenyl)(methyl)amino)piperidine-2,6-dione

To a solution of 2,6-bis(benzyloxy)-N-(4-(4-(dimethoxymethyl)piperidin-1-yl)-3-fluorophenyl)-N-methylpyridin-3-amine (2 g, 3.49 mmol, 1.0 eq) in ethanol (50 mL) were added palladium on carbon (10%, 0.5 g) and palladium hydroxide on carbon (0.5 g) at rt. The reaction solution was stirred under H₂ at 50°C overnight. The reaction solution was filtered through Celite and subjected to rotary evaporation to dryness. The residue was purified by column chromatography to afford the title compound (gray solid, 600 mg, yield 44%).

### Step F: 1-(4-((2,6-Dioxopiperidin-3-yl)(methyl)amino)-2-fluorophenyl)piperidine-4-carbaldehyde

3-((4-(4-(Dimethoxymethyl)piperidin-1-yl)-3-fluorophenyl)(methyl)amino)piperidine-2,6-dione (200 mg, 0.51 mmol, 1.0 eq) was added to formic acid (5 mL) at rt. The mixture was reacted at rt overnight. The reaction solution was concentrated and dried to afford the title compound (yellow solid, 176 mg, crude), which was used directly in the next step.

### Step G: N-(3-Carbamoyl-1-(4-(4-((1-(4-((2,6-Dioxopiperidin-3-yl)(methyl)amino)-2-fluorophenyl)piperidin-4-yl)methyl)piperazin-1-yl)phenyl)-1H-pyrazol-4-yl)-6-(trifluoromethyl)picolinamide formate

To a solution of 1-(4-((2,6-dioxopiperidin-3-yl)(methyl)amino)-2-fluorophenyl)piperidine-4-carbaldehyde (176 mg, crude) and N-(3-carbamoyl-1-(4-(piperazin-1-yl)phenyl)-1H-pyrazol-4-yl)-6-(trifluoromethyl)picolinamide (279 mg, 0.61 mmol, 1.2 eq) in DMF (5 mL) was added TEA (0.2 mL, 1.01 mmol, 2.0 eq) at rt. After stirring at rt for 5 min, glacial AcOH (0.1 mL, 0.51 mmol, 1.0 eq) was added. After stirring at rt for 1 h, sodium triacetoxyborohydride (213 mg, 1.01 mmol, 2.0 eq) was added. The reaction solution was stirred at rt for 3 h. Upon completion, the reaction solution was purified by HPLC to afford the title compound (white solid, 38.8 mg, yield 10% over two steps). LCMS (ESI): m/z 791 [M+H]⁺. **¹H NMR** (400 MHz, DMSO-d6) δ 11.80 (s, 1H), 10.78 (s, 1H), 8.88 (s, 1H), 8.49-8.37 (m, 2H), 8.33 (s, 1H), 8.22 (d, *J* = 6.9 Hz, 1H), 7.95 (s, 1H), 7.79 (d, *J* = 9.1 Hz, 2H), 7.71 (s, 1H), 7.09 (d, *J* = 9.2 Hz, 2H), 6.96-6.86 (m, 1H), 6.67 (dd, *J =* 16.1, 2.6 Hz, 1H), 6.58-6.49 (m, 1H), 4.80 (dd, *J =* 12.5, 4.8 Hz, 1H), 3.25-3.14 (m, 9H), 2.81 (dd, *J* = 13.5, 5.5 Hz, 1H), 2.69 (s, 3H), 2.62-2.54 (m, 4H), 2.35-2.29 (m, 1H), 2.25 (d, *J* = 7.4 Hz, 2H), 1.84 (dd, *J* = 25.0, 8.2 Hz, 3H), 1.66 (s, 1H), 1.29 (d, *J* = 9.6 Hz, 2H).

### Preparation Example 455. Synthesis of Compound A448

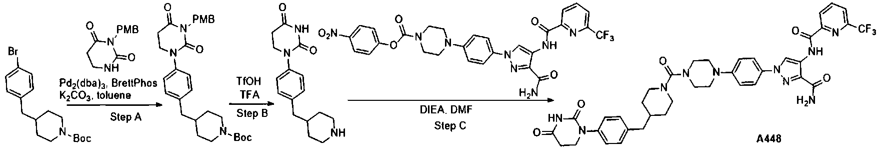

### Step A: Tert-butyl 4-(4-(3-(4-methoxybenzyl)-2,4-dioxotetrahydropyrimidin-1(2H)-yl)benzyl)piperidine-1-carboxylate

To a solution of tert-butyl 4-(4-bromobenzyl)piperidine-1-carboxylate (500 mg, 1.42 mmol, 1.0 eq) in toluene (10 mL) were added 3-(4-methoxybenzyl)dihydropyrimidine-2,4(1H,3H)-dione (398 mg, 1.70 mmol, 1.2 eq), Pd₂(dba)₃ (128 mg, 0.14 mmol, 0.1 eq), BrettPhos (80 mg, 0.14 mmol, 0.1 eq), and K₂CO₃ (588 mg, 4.26 mmol, 3.0 eq) at rt. The reaction solution was degassed (×3) under N₂ and stirred at 100°C for 18 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 280 mg, yield 39%).

### Step B: 1-(4-(Piperidin-4-ylmethyl)phenyl)dihydropyrimidine-2,4(1H,3H)-dione

To a solution of tert-butyl 4-(4-(3-(4-methoxybenzyl)-2,4-dioxotetrahydropyrimidin-1(2H)-yl)benzyl)piperidine-1-carboxylate (280 mg, 0.55 mmol, 1.0 eq) in TFA (5 mL) was added TfOH (1 mL). The mixture was stirred at 80°C for 18 h. Upon completion, the mixture was concentrated under reduced pressure. The residue was purified by column chromatography to afford the title compound (yellow solid, 110 mg, yield 70%).

### Step C: N-(3-Carbamoyl-1-(4-(4-(4-(2,4-dioxotetrahydropyrimidin-1(2H)-yl)benzyl)piperidine-1-carbonyl)piperazin-1-yl)phenyl)-1H-pyrazol-4-yl)-6-(trifluoromethyl)picolinamide

To a solution of 1-(4-(piperidin-4-ylmethyl)phenyl)dihydropyrimidine-2,4(1H,3H)-dione (70 mg, 0.24 mmol, 1.0 eq) in DMF (2 mL) were added 4-nitrophenyl 4-(4-(3-carbamoyl-4-(6-(trifluoromethyl)pyridinylamino)-1H-pyrazol-1-yl)phenyl)piperazine-1-carboxylate (152 mg, 0.24 mmol, 1.0 eq) and DIEA (93 mg, 0.72 mmol, 3.0 eq). The mixture was stirred at 100°C under N₂ for 18 h. Upon completion, the reaction solution was diluted in EtOAc, washed sequentially with water and saturated aqueous NaCl, dried over anhydrous Na₂SO₄, and concentrated under reduced pressure. The residue was purified by HPLC to afford the title compound (yellow solid, 30 mg, yield 16%). LCMS (ESI): m/z 773 [M+H]⁺. **¹H** NMR (400 MHz, DMSO-d6) δ 11.79 (s, 1H), 10.33 (s, 1H), 8.89 (s, 1H), 8.46-8.38 (m, 2H), 8.21 (d, *J =* 7.1 Hz, 1H), 7.95 (s, 1H), 7.81 (d, *J=* 9.0 Hz, 2H), 7.71 (s, 1H), 7.25-7.18 (m, 4H), 7.10 (d,*J* = 9.1 Hz, 2H), 3.77 (t, *J* = 6.6 Hz, 2H), 3.66-3.61 (m, 2H), 3.29-3.25 (m, 6H), 3.23-3.20 (m, 4H), 2.72-2.67 (m, 4H), 1.71-1.65 (m, 1H), 1.61-1.56 (m, 2H), 1.20-1.12 (m, 2H).

### Preparation Example 456. Synthesis of Compound A449

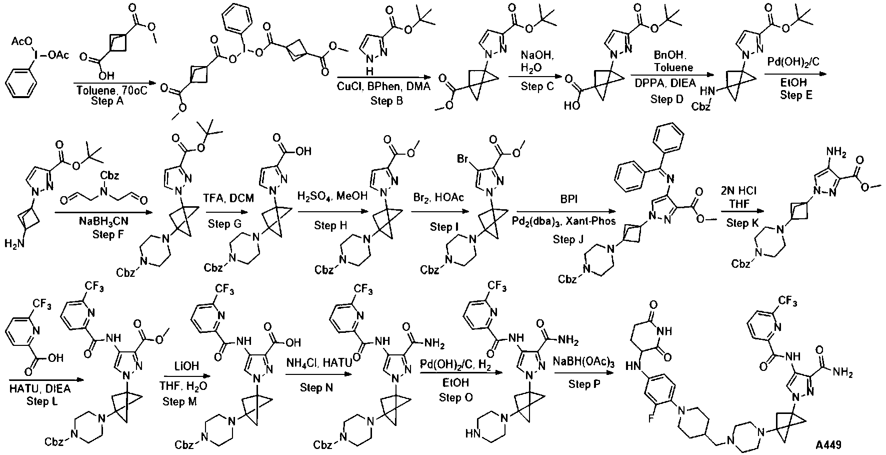

### Step A: 3,3'-Dimethyl O'1,O1-(phenyl-λ³-iodanediyl)bis(bicyclo[1.1.1]pentane-1,3-dicarboxylate)

To a solution of (diacetoxyiodo)benzene (10 g, 30.8 mmol, 1.0 eq) in toluene (100 mL) was added 3-(methoxycarbonyl)bicyclo[1.1.1]pentane-1-carboxylic acid (5.25 g, 30.8 mmol, 1.0 eq). The mixture was stirred at 70°C under N₂ for 5 h. The resulting mixture was concentrated to dryness. The residue was slurried with toluene to remove excess AcOH to afford the title compound (white solid, 16.5 g, yield 98%).

### Step B: Tert-butyl 1-(3-(methoxycarbonyl)bicyclo[1.1.1]pentan-1-yl)-1H-pyrazole-3-carboxylate

To a solution of 3,3'-dimethyl O'1,O1-(phenyl-λ³-iodanediyl)bis(bicyclo[1.1.1]pentane-1,3-dicarboxylate) (16.5 g, 30.3 mmol, 1.0 eq) and tert-butyl 1H-pyrazole-3-carboxylate (5.1 g, 30.3 mmol, 1.0 eq) in N,N-dimethylacetamide (160 mL) were added cuprous chloride (1.5 g, 15.2 mmol, 0.5 eq) and BPhen (7.1 g, 21.2 mmol, 0.7 eq). The mixture was stirred at rt under N₂ for 1 h. Upon completion, the reaction was quenched with water, diluted in EtOAc, washed sequentially with water and saturated aqueous NaCl, dried over anhydrous Na₂SO₄, and concentrated under reduced pressure. The residue was purified by column chromatography to afford the title compound (white solid, 6.4 g, yield 72%).

### Step C: 3-(3-(tert-Butoxycarbonyl)-1H-pyrazol-1-yl)bicyclo[1.1.1]pentane-1-carboxylic acid

To a mixed solution of tert-butyl 1-(3-(methoxycarbonyl)bicyclo[1.1.1]pentan-1-yl)-1H-pyrazole-3-carboxylate (6.4 g, 21.9 mmol, 1.0 eq) in MeOH (50 mL) and water (10 mL) was added NaOH (2.2 g, 54.7 mmol, 2.5 eq). The reaction was stirred at rt for 1 h. Upon completion, the reaction solution was neutralized to pH=2 with saturated citric acid solution and extracted with DCM twice. The organic phase was washed with water and saturated aqueous NaCl, dried over anhydrous Na₂SO₄, and concentrated under reduced pressure to afford the title compound (white solid, 4.7 g, yield 77%).

### Step D: Tert-butyl 1-(3-(((benzyloxy)carbonyl)amino)bicyclo[1.1.1]pentan-1-yl)-1H-pyrazole-3-carboxylate

To a mixed solution of 3-(3-(tert-butoxycarbonyl)-1H-pyrazol-1-yl)bicyclo[1.1.1]pentane-1-carboxylic acid (4.7 g, 16.9 mmol, 1.0 eq) in toluene (50 mL) and benzyl alcohol (5 mL) were added DIEA (5.9 mL, 33.8 mmol, 2.0 eq) and diphenylphosphoryl azide (4.7 mL, 22 mmol, 1.3 eq). The reaction solution was stirred at 100°C for 5 h. Upon completion, the reaction solution was quenched with water, diluted in EtOAc, washed sequentially with water and saturated aqueous NaCl, dried over anhydrous Na₂SO₄, and concentrated under reduced pressure. The residue was purified by column chromatography to afford the title compound (white solid, 4.6 g, yield 71%).

### Step E: Tert-butyl 1-(3-aminobicyclo[1.1.1]pentan-1-yl)-1H-pyrazole-3-carboxylate

To a solution of tert-butyl 1-(3-(((benzyloxy)carbonyl)amino)bicyclo[1.1.1]pentan-1-yl)-1H-pyrazole-3-carboxylate (4.6 g, 12.0 mmol, 1.0 eq) in ethanol (50 mL) was added wet palladium hydroxide on carbon (10%, 2 g). The reaction solution was stirred under H₂ at rt for 3 h. Upon completion, the reaction solution was filtered through Celite and concentrated under reduced pressure to afford the title compound (colorless oil, 3 g, crude), which was used directly without purification.

### Step F: Benzyl 4-(3-(3-(tert-butoxycarbonyl)-1H-pyrazol-1-yl)bicyclo[1.1.1]pentan-1-yl)piperazine-1-carboxylate

To a mixed solution of tert-butyl 1-(3-aminobicyclo[1.1.1]pentan-1-yl)-1H-pyrazole-3-carboxylate (3 g, 12.0 mmol, 1.0 eq) and benzyl bis(2-oxoethyl)carbamate (7.08 g, 30.1 mmol, 2.5 eq) in DCM (30 mL) and MeOH (10 mL) was added AcOH (6.7 g, 36.1 mmol, 3.0 eq) at rt. After stirring for 2 h, sodium cyanoborohydride (2.3 g, 36.1 mmol, 3.0 eq) was added in one portion, and the mixture was stirred for 16 h. Upon completion, the reaction solution was quenched with water, diluted in EtOAc, washed sequentially with water and saturated aqueous NaCl, dried over anhydrous Na₂SO₄, and concentrated under reduced pressure. The residue was purified by column chromatography to afford the title compound (white solid, 3.3 g, yield 61%).

### Step G: 1-(3-(4-((Benzyloxy)carbonyl)piperazin-1-yl)bicyclo[1.1.1]pentan-1-yl)-1H-pyrazole-3-carboxylic acid

To a solution of benzyl 4-(3-(3-(tert-butoxycarbonyl)-1H-pyrazol-1-yl)bicyclo[1.1.1]pentan-1-yl)piperazine-1-carboxylate (3.3 g, 7.3 mmol, 1.0 eq) in DCM (30 mL) was added TFA (10 mL). The mixture was stirred at 25°C for 2 h. Upon completion, the mixture was concentrated under reduced pressure to afford the title compound (colorless oil, 2.5 g, crude), which was used directly without purification.

### Step H: Benzyl 4-(3-(3-(methoxycarbonyl)-1H-pyrazol-1-yl)bicyclo[1.1.1]pentan-1-yl)piperazine-1-carboxylate

To a solution of 1-(3-(4-((benzyloxy)carbonyl)piperazin-1-yl)bicyclo[1.1.1]pentan-1-yl)-1H-pyrazole-3-carboxylic acid (2.5 g, 6.3 mmol, 1.0 eq) in MeOH (30 mL) was added concentrated sulfuric acid (0.34 mL, 6.3 mmol, 1.0 eq). The reaction solution was stirred at 80°C under N₂ for 2 h. Upon completion, the reaction solution was slowly poured into saturated aqueous NaHCO₃ to quench the reaction. The mixture was extracted with EtOAc. The organic phase was washed sequentially with water and saturated aqueous NaCl, dried over anhydrous Na₂SO₄, and concentrated under reduced pressure. The residue was purified by column chromatography to afford the title compound (yellow solid, 1.5 g, yield 58%).

### Step I: Benzyl 4-(3-(4-bromo-3-(methoxycarbonyl)-1H-pyrazol-1-yl)bicyclo[1.1.1]pentan-1-yl)piperazine-1-carboxylate

To a solution of benzyl 4-(3-(3-(methoxycarbonyl)-1H-pyrazol-1-yl)bicyclo[1.1.1]pentan-1-yl)piperazine-1-carboxylate (1.5 g, 3.7 mmol, 1.0 eq) in AcOH (20 mL) was added bromine (5.8 g, 36.5 mmol, 10.0 eq). The reaction solution was stirred at rt overnight. Upon completion, the reaction solution was quenched with saturated aqueous sodium thiosulfate solution, and extracted with EtOAc. The organic phase was washed sequentially with water and saturated aqueous NaCl, dried over anhydrous Na₂SO₄, and concentrated under reduced pressure. The residue was purified by column chromatography to afford the title compound (yellow solid, 400 mg, yield 22%).

### Step J: Benzyl 4-(3-(4-((diphenylmethylene)amino)-3-(methoxycarbonyl)-1H-pyrazol-1-yl)bicyclo[1.1.1]pentan-1-yl)piperazine-1-carboxylate

To a solution of benzyl 4-(3-(4-bromo-3-(methoxycarbonyl)-1H-pyrazol-1-yl)bicyclo[1.1.1]pentan-1-yl)piperazine-1-carboxylate (400 mg, 0.8 mmol, 1.0 eq) and benzophenone imine (296 mg, 1.6 mmol, 2.0 eq) in 1,4-dioxane (10 mL) were added Pd₂(dba)₃ (75 mg, 0.08 mmol, 0.1 eq), XantPhos (95 mg, 0.16 mmol, 0.2 eq) and Cs₂CO₃ (799 mg, 2.5 mmol, 3.0 eq) at 25°C. The reaction solution was stirred at 100°C under N₂ for 8 h. Upon completion, the reaction solution was quenched with water, and extracted with EtOAc. The organic phase was washed sequentially with water and saturated aqueous NaCl, dried over anhydrous Na₂SO₄, and concentrated under reduced pressure. The residue was purified by column chromatography to afford the title compound (yellow solid, 300 mg, yield 62%).

### Step K: Benzyl 4-(3-(4-amino-3-(methoxycarbonyl)-1H-pyrazol-1-yl)bicyclo[1.1.1]pentan-1-yl)piperazine-1-carboxylate

To a solution of benzyl 4-(3-(4-((diphenylmethylene)amino)-3-(methoxycarbonyl)-1H-pyrazol-1-yl)bicyclo[1.1.1]pentan-1-yl)piperazine-1-carboxylate (300 mg, 0.51 mmol, 1.0 eq) in THF (8 mL) was added 2 N HCl (2 mL). The reaction solution was stirred at 25°C for 1 h. Upon completion, the reaction solution was neutralized with saturated NaHCO₃ solution, and extracted with EtOAc. The organic phase was washed sequentially with water and saturated aqueous NaCl, dried over anhydrous Na₂SO₄, and concentrated under reduced pressure. The residue was purified by column chromatography to afford the title compound (yellow solid, 200 mg, yield 93%).

### Step L: Benzyl 4-(3-(3-(methoxycarbonyl)-4-(6-(trifluoromethyl)picolinamido)-1H-pyrazol-1-yl)bicyclo[1.1.1]pentan-1-yl)piperazine-1-carboxylate

To a solution of benzyl 4-(3-(4-amino-3-(methoxycarbonyl)-1H-pyrazol-1-yl)bicyclo[1.1.1]pentan-1-yl)piperazine-1-carboxylate (200 mg, 0.5 mmol, 1.0 eq), 6-(trifluoromethyl)picolinic acid (180 mg, 0.9 mmol, 2.0 eq) and HATU (357 mg, 0.9 mmol, 2.0 eq) in DMF (10 mL) was added DIEA (0.2 mL, 1.4 mmol, 3.0 eq) at rt. The reaction solution was stirred at 25°C for 1 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (yellow solid, 150 mg, yield 53%).

### Step M: 1-(3-(4-((Benzyloxy)carbonyl)piperazin-1-yl)bicyclo[1.1.1]pentan-1-yl)-4-(6-(trifluoromethyl)picolinamido)-1H-pyrazole-3-carboxylic acid

To a mixed solution of benzyl 4-(3-(3-(methoxycarbonyl)-4-(6-(trifluoromethyl)picolinamido)-1H-pyrazol-1-yl)bicyclo[1.1.1]pentan-1-yl)piperazine-1-carboxylate (150 mg, 0.25 mmol, 1.0 eq) in THF (4 mL) and water (2 mL) was added lithium hydroxide (53 mg, 1.25 mmol, 5.0 eq). The reaction solution was stirred at 25°C for 1 h. Upon completion, the reaction solution was concentrated under reduced pressure to afford the title lithium salt compound (yellow solid, 130 mg, crude), which was used directly without purification.

### Step N: Benzyl 4-(3-(3-carbamoyl-4-(6-(trifluoromethyl)picolinamido)-1H-pyrazol-1-yl)bicyclo[1.1.1]pentan-1-yl)piperazine-1-carboxylate

To a solution of 1-(3-(4-((benzyloxy)carbonyl)piperazin-1-yl)bicyclo[1.1.1]pentan-1-yl)-4-(6-(trifluoromethyl)picolinamido)-1H-pyrazole-3-carboxylic acid (130 mg, 0.2 mmol, 1.0 eq), ammonium chloride (59 mg, 1.1 mmol, 5.0 eq) and HATU (169 mg, 0.44 mmol, 2.0 eq) in DMF (5 mL) was added DIEA (0.1 mL, 0.7 mmol, 3.0 eq) at rt. The reaction solution was stirred at 25°C for 1 h. Upon completion, the reaction solution was diluted in EtOAc, washed sequentially with water and saturated aqueous NaCl, dried over anhydrous Na₂SO₄, and concentrated under reduced pressure. The residue was purified by column chromatography to afford the title compound (white solid, 100 mg, yield 77%).

### Step O: N-(3-Carbamoyl-1-(3-(piperazin-1-yl)bicyclo[1.1.1]pentan-1-yl)-1H-pyrazol-4-yl)-6-(trifluoromethyl)picolinamide

To a solution of benzyl 4-(3-(3-carbamoyl-4-(6-(trifluoromethyl)picolinamido)-1H-pyrazol-1-yl)bicyclo[1.1.1]pentan-1-yl)piperazine-1-carboxylate (100 mg, 0.17 mmol, 1.0 eq) in ethanol (10 mL) was added wet palladium hydroxide on carbon (10%, 50 mg). The reaction solution was stirred under H₂ at rt for 3 h. Upon completion, the reaction solution was filtered through Celite and concentrated under reduced pressure to afford the title compound (white solid, 60 mg, crude), which was used directly without purification.

### Step P: N-(3-Carbamoyl-1-(3-(4-((1-(4-((2,6-dioxopiperidin-3-yl)amino)-2-fluorophenyl)piperidin-4-yl)methyl)piperazin-1-yl)bicyclo[1.1.1]pentan-1-yl)-1H-pyrazol-4-yl)-6-(trifluoromethyl)picolinamide

At rt, to a solution of N-(3-carbamoyl-1-(3-(piperazin-1-yl)bicyclo[1.1.1]pentan-1-yl)-1H-pyrazol-4-yl)-6-(trifluoromethyl)picolinamide (20 mg, 0.04 mmol, 1.0 eq) and 1-(4-((2,6-dioxopiperidin-3-yl)amino)-2-fluorophenyl)piperidine-4-carbaldehyde (30 mg, 0.09 mmol, 2.0 eq) in DMF (5 mL) were added 3 drops of AcOH, and the mixture was stirred for 2 h. Then sodium triacetoxyborohydride (47 mg, 0.2 mmol, 5.0 eq) was added. The reaction solution was stirred at rt for 1 h. Upon completion, the reaction solution was diluted in EtOAc, washed sequentially with water and saturated aqueous NaCl, dried over anhydrous Na₂SO₄, and concentrated under reduced pressure. The crude was purified by HPLC to afford the title compound (white solid, 7.5 mg, yield 22%). LCMS (ESI): m/z 767 [M+H]⁺. **¹H NMR** (400 MHz, DMSO-*d*₆) δ 11.75 (s, 1H), 10.77 (s, 1H), 8.44-8.36 (m, 3H), 8.19 (dd, *J=* 7.3, 1.3 Hz, 1H), 7.67 (d, *J* = 38.6 Hz, 2H), 6.83 (t, *J*= 9.3 Hz, 1H), 6.50 (dd, *J=* 15.0, 2.4 Hz, 1H), 6.41 (dd, *J =* 8.7, 2.2 Hz, 1H), 5.78 (d, *J=* 7.7 Hz, 1H), 4.31-4.19 (m, 1H), 3.14-3.06 (m, 2H), 2.77-2.68 (m, 1H), 2.62-2.51 (m, 4H), 2.48-2.43 (m, 4H), 2.42-2.37 (m, 2H), 2.36-2.32 (m, 1H), 2.32-2.26 (m, 6H), 2.23-2.14 (m, 2H), 2.13-2.06 (m, 1H), 1.90-1.81 (m, 1H), 1.80-1.72 (m, 2H), 1.66-1.52 (m, 1H), 1.29-1.19 (m, 2H).

### Preparation Example 457. Synthesis of Compound A450

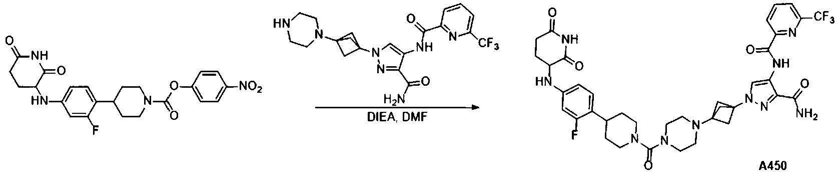

### N-(3-Carbamoyl-1-(3-(4-(4-((2,6-dioxopiperidin-3-yl)amino)-2-fluorophenyl)piperidine-1-carbonyl)piperazin-1-yl)bicyclo[1.1.1]pentan-1-yl)-1H-pyrazol-4-yl)-6-(trifluoromethyl)picolinamide

To a solution of N-(3-carbamoyl-1-(3-(piperazin-1-yl)bicyclo[1.1.1]pentan-1-yl)-1H-pyrazol-4-yl)-6-(trifluoromethyl)picolinamide (20 mg, 0.04 mmol, 1.0 eq) and 4-nitrophenyl 4-(4-((2,6-dioxopiperidin-3-yl)amino)-2-fluorophenyl)piperidine-1-carboxylate (25 mg, 0.05 mmol, 1.2 eq) in DMF (3 mL) was added DIEA (20 µL, 0.09 mmol, 2.0 eq) at 25°C. The mixture was stirred at 100°C under N₂ for 16 h. Upon completion, the reaction solution was quenched with water, diluted in EtOAc, washed sequentially with water and saturated aqueous NaCl, dried over anhydrous Na₂SO₄, and concentrated under reduced pressure. The crude was purified by HPLC to afford the title compound (white solid, 2 mg, yield 6%). LCMS (ESI): m/z 781 [M+H]⁺. **¹H NMR** (400 MHz, DMSO-d6) *δ* 11.75 (s, 1H), 10.78 (s, 1H), 8.45-8.36 (m, 3H), 8.22-8.17 (m, 1H), 7.73 (s, 1H), 7.62 (s, 1H), 6.99 (t, J = 8.8 Hz, 1H), 6.53-6.37 (m, 2H), 6.02 (d, J = 7.7 Hz, 1H), 4.35-4.28 (m, 1H), 3.72-3.66 (m, 2H), 3.22-3.16 (m, 4H), 2.85-2.80 (m, 2H), 2.74-2.69 (m, 1H), 2.58-2.55 (m, 1H), 2.48-2.44 (m, 4H), 2.36-2.33 (m, 1H), 2.33-2.28 (m, 6H), 2.14-2.08 (m, 1H), 1.91-1.84 (m, 1H), 1.70-1.64 (m, 2H), 1.61-1.52 (m, 2H).

### Preparation Example 458. Synthesis of Compound A451

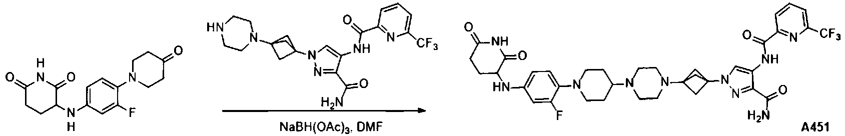

### N-(3-Carbamoyl-1-(3-(4-(1-(4-((2,6-Dioxopiperidin-3-yl)amino)-2-fluorophenyl)piperidin-4-yl)piperazin-1-yl)bicyclo[1.1.1]pentan-1-yl)-1H-pyrazol-4-yl)-6-(trifluoromethyl)picolinamide

To a solution of N-(3-carbamoyl-1-(3-(piperazin-1-yl)bicyclo[1.1.1]pentan-1-yl)-1H-pyrazol-4-yl)-6-(trifluoromethyl)picolinamide (20 mg, 0.04 mmol, 1.0 eq) and 3-((3-fluoro-4-(4-oxopiperidin-1-yl)phenyl)amino)piperidine-2,6-dione (20 mg, 0.06 mmol, 1.5 eq) in DMF (5 mL) were added 3 drops of AcOH. The mixture was stirred for 2 h at rt. Then sodium triacetoxyborohydride (47 mg, 0.2 mmol, 5.0 eq) was added. The reaction solution was stirred at rt for 1 h. Upon completion, the reaction solution was quenched with water, diluted in EtOAc, washed sequentially with water and saturated aqueous NaCl, dried over anhydrous Na₂SO₄, and concentrated under reduced pressure. The crude was purified by HPLC to afford the title compound (white solid, 2 mg, yield 7%). LCMS (ESI): m/z 753 [M+H]⁺. **¹H NMR** (400 MHz, DMSO-*d₆*) *δ* 11.75 (s, 1H), 10.78 (s, 1H), 8.40 (dd, *J* = 7.0, 4.8 Hz, 3H), 8.19 (dd, *J* = 7.4, 1.3 Hz, 1H), 7.72 (s, 1H), 7.62 (s, 1H), 6.83 (t, *J* = 9.4 Hz, 1H), 6.50 (dd, *J* = 15.0, 2.4 Hz, 1H), 6.41 (dd, *J* = 8.7, 2.0 Hz, 1H), 5.79 (d, *J* = 7.6 Hz, 1H), 4.30-4.19 (m, 1H), 3.21-3.13 (m, 2H), 2.76-2.70 (m, 1H), 2.62-2.53 (m, 7H), 2.49-2.43 (m, 4H), 2.34-2.31 (m, 1H), 2.31-2.28 (m, 6H), 2.11-2.06 (m, 1H), 1.90-1.77 (m, 3H), 1.62-1.49 (m, 2H).

### Preparation Example 459. Synthesis of Compound A452

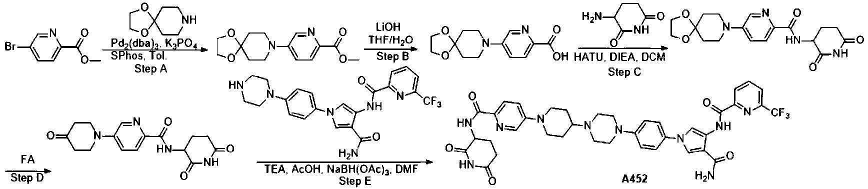

### Step A: Methyl 5-(1,4-dioxa-8-azaspiro[4.5]decan-8-yl)picolinate

To a solution of methyl 5-bromopicolinate (5.0 g, 23.1 mmol, 1.0 eq) and 1,4-dioxa-8-azaspiro[4.5]decane (5.0 g, 35.0 mmol, 1.5 eq) in anhydrous toluene (80 mL) were added Pd₂(dba)₃ (1.9 g, 2 mmol, 0.1 eq), 2-dicyclohexylphosphino-2',6'-dimethoxybiphenyl (2.7 g, 4 mmol, 0.2 eq), and potassium phosphate (14.6 g, 69 mmol, 3.0 eq). The mixture was stirred at 90°C under N₂ for 15 h. The mixture was filtered through Celite, and the filtrate was concentrated in vacuo to obtain a residue. The residue was purified by column chromatography to afford the title compound (yellow solid, 4.7 g, yield 73%).

### Step B: 5-(1,4-dioxa-8-azaspiro[4.5]decan-8-yl)picolinic acid

Compound methyl 5-(1,4-dioxa-8-azaspiro[4.5]decan-8-yl)picolinate (4.0 g, 14.3 mmol, 1.0 eq) was dissolved in a mixed solvent of THF (40 mL) and water (4 mL). Then lithium hydroxide (0.9 g, 21.4 mmol, 1.5 eq) was added. After stirring for 2 h, the reaction solution was concentrated in vacuo to afford the title compound (yellow solid, 3.8 g, crude).

### Step C: N-(2,6-Dioxopiperidin-3-yl)-5-(1,4-dioxa-8-azaspiro[4.5]decan-8-yl)picolinamide

Compound 5-(1,4-dioxa-8-azaspiro[4.5]decan-8-yl)picolinic acid (1.0 g, 4.0 mmol, 1.0 eq) and 3-aminopiperidine-2,6-dione hydrochloride (1.0 g, 6.0 mmol, 1.5 eq) were dissolved in DCM (20 mL). Then HATU (2.3 g, 6.0 mmol, 1.5 eq) and DIEA (2 mL) were added. After stirring at rt for 5 h, the reaction solution was poured into water and extracted with DCM. The combined organic layers were washed with saturated aqueous NaCl, dried over anhydrous Na₂SO₄, and concentrated in vacuo to obtain a residue. The residue was purified by column chromatography to afford the title compound (yellow solid, 620 mg, yield 50%).

### Step D: N-(2,6-Dioxopiperidin-3-yl)-5-(4-oxopiperidin-1-yl)picolinamide

Compound N-(2,6-dioxopiperidin-3-yl)-5-(1,4-dioxa-8-azaspiro[4.5]decan-8-yl)picolinamide (600 mg, 1.8 mmol, 1.0 eq) was dissolved in formic acid (10 mL). The mixture was stirred at 80°C for 15 h. The reaction solution was concentrated in vacuo to afford the title compound (yellow solid, 460 mg, crude).

### Step E: N-(4-Carbamoyl-1-(4-(4-(1-(6-((2,6-dioxopiperidin-3-yl)carbamoyl)pyridin-3-yl)piperidin-4-yl)piperazin-1-yl)phenyl)-1H-pyrrol-3-yl)-6-(trifluoromethyl)picolinamide

At rt, to a solution of N-(4-carbamoyl-1-(4-(piperazin-1-yl)phenyl)-1H-pyrrol-3-yl)-6-(trifluoromethyl)picolinamide (73 mg, 0.16 mmol, 1.0 eq) in DMF (5 mL) was added TEA to adjust the pH to 10. After stirring for 10 min, AcOH was added to adjust the pH to 3. After stirring for 5 min, N-(2,6-dioxopiperidin-3-yl)-5-(4-oxopiperidin-1-yl)picolinamide (53 mg, 0.16 mmol, 1.0 eq) was then added, and the mixture was stirred for 1 h. Then sodium triacetoxyborohydride (66 mg, 0.31 mmol, 2.0 eq) was added. After stirring for 18 h, the reaction solution was poured into water and extracted with EtOAc. The organic phase was washed with aqueous NaCl, dried over anhydrous Na₂SO₄, and concentrated under reduced pressure. The residue was purified by HPLC to afford the title compound (yellow solid, 4.8 mg, yield 4%). LCMS (ESI): m/z 773.3 [M+H]⁺. **¹H NMR** (400 MHz, DMSO-*d*₆) δ 12.24 (s, 1H), 10.86 (s, 1H), 8.74 (d, *J* = 8.2 Hz, 1H), 8.46-8.35 (m, 3H), 8.17 (dd, *J* = 7.4, 1.1 Hz, 1H), 7.98 (d, *J* = 2.4 Hz, 1H), 7.92-7.86 (m, 2H), 7.67 (s, 1H), 7.50 (dd, *J* = 12.7, 5.9 Hz, 3H), 7.29 (s, 1H), 7.18 *(d, J=* 9.0 Hz, 2H), 4.84-4.69 (m, 1H), 4.27-4.08 (m, 2H), 4.04-3.89 (m, 2H), 3.72-3.62 (m, 2H), 3.60-3.54 (m, 1H), 3.28-3.22 (m, 2H), 3.09-2.99 (m, 2H), 2.98-2.89 (m, 2H), 2.85-2.76 (m, 1H), 2.57-2.52 (m, 1H), 2.26-2.15 (m, 3H), 2.06-1.99 (m, 1H), 1.81-1.70 (m, 2H).

### Preparation Example 460. Synthesis of Compound A453

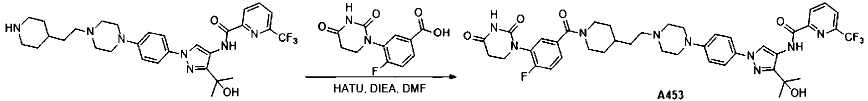

### N-(1-(4-(4-(2-(1-(3-(2,4-Dioxotetrahydropyrimidin-1(2H)-yl)-4-fluorobenzoyl)piperidin-4-yl)ethyl)piperazin-1-yl)phenyl)-3-(2-hydroxypropan-2-yl)-1H-pyrazol-4-yl)-6-(trifluoromethyl)picolinamide

To a solution of N-(3-(2-hydroxypropan-2-yl)-1-(4-(4-(2-(piperidin-4-yl)ethyl)piperazin-1-yl)phenyl)-1H-pyrazol-4-yl)-6-(trifluoromethyl)picolinamide (200 mg, 0.34 mmol, 1.0 eq) and 3-(2,4-dioxotetrahydropyrimidin-1(2H)-yl)-4-fluorobenzoic acid (86 mg, 0.34 mmol, 1.0 eq) in DMF (4 mL) were added HATU (259 mg, 0.68 mmol, 2.0 eq) and DIEA (88 mg, 0.68 mmol, 2.0 eq). The mixture was stirred at 25°C for 1 h. The reaction solution was diluted in EtOAc, washed sequentially with water and saturated aqueous NaCl, dried over anhydrous Na₂SO₄, and concentrated under reduced pressure. The residue was purified by preparative HPLC to afford the title compound (white solid, 20 mg, yield 7%). LCMS (ESI) m/z: 820 [M+H]⁺. **¹H NMR** (400 MHz, DMSO-d6) δ 11.19 (s, 1H), 10.53 (s, 1H), 8.73 (s, 1H), 8.48-8.38 (m, 2H), 8.17 (d, *J* = 7.4 Hz, 1H), 7.60 (d, *J* = 6.8 Hz, 2H), 7.49 (d, *J* = 6.7 Hz, 1H), 7.37 (d, *J* = 6.1 Hz, 2H), 7.02 (d, *J* = 6.8 Hz, 2H), 5.93 (s, 1H), 4.43 (s, 1H), 3.75 (s, 3H), 3.55-3.45 (m, 4H), 3.15 (s, 4H), 2.72 (s, 3H), 2.36 (s, 2H), 1.81-1.71 (m, 2H), 1.57 (s, 6H), 1.44 (s, 2H), 1.26-1.13 (m, 4H).

### Preparation Example 461. Synthesis of Compound A454

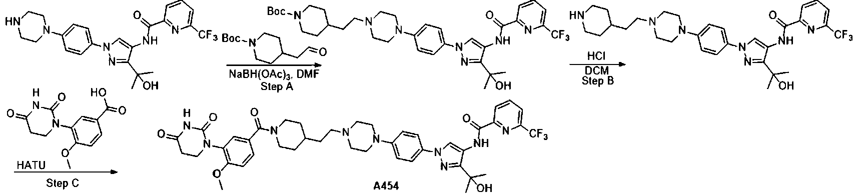

### Step A: Tert-butyl 4-(2-(4-(4-(3-(2-hydroxypropan-2-yl)-4-(6-(trifluoromethyl)picolinamido)-1H-pyrazol-1-yl)phenyl)piperazin-1-yl)ethyl)piperidine-1-carboxylate

To a solution of N-(3-(2-hydroxypropan-2-yl)-1-(4-(piperazin-1-yl)phenyl)-1H-pyrazol-4-yl)-6-(trifluoromethyl)picolinamide (500 mg, 1.05 mmol, 1.0 eq) and tert-butyl 4-(2-oxoethyl)piperidine-1-carboxylate (370 mg, 1.26 mmol, 1.2 eq) in DMF (5 mL) was added sodium triacetoxyborohydride (120 mg, 3.16 mmol, 3.0 eq). The solution was stirred at rt for 3 h. The crude was purified by reverse-phase column chromatography to afford the title compound (white solid, 400 mg, yield 55%).

### Step B: N-(3-(2-Hydroxypropan-2-yl)-1-(4-(4-(2-(piperidin-4-yl)ethyl)piperazin-1-yl)phenyl)-1H-pyrazol-4-yl)-6-(trifluoromethyl)picolinamide

To a solution of tert-butyl 4-(2-(4-(4-(3-(2-hydroxypropan-2-yl)-4-(6-(trifluoromethyl)picolinamido)-1H-pyrazol-1-yl)phenyl)piperazin-1-yl)ethyl)piperidine-1-carboxylate (350 mg, 0.51 mmol, 1 eq) in DCM (30 mL) was added dropwise 4 M hydrogen chloride in dioxane (15 mL) at 0°C. The mixture was stirred at rt for 2 h, and then the reaction solution was adjusted to pH ~8 with saturated NaHCO₃ solution and extracted with DCM. The organic phase was washed with aqueous NaCl, dried over anhydrous Na₂SO₄, and concentrated under reduced pressure to afford the title compound (yellow solid, 290 mg, yield 97%).

### Step C: N-(1-(4-(4-(2-(1-(3-(2,4-Dioxotetrahydropyrimidin-1(2H)-yl)-4-methoxybenzoyl)piperidin-4-yl)ethyl)piperazin-1-yl)phenyl)-3-(2-hydroxypropan-2-yl)-1H-pyrazol-4-yl)-6-(trifluoromethyl)picolinamide

To a solution of N-(3-(2-hydroxypropan-2-yl)-1-(4-(4-(2-(piperidin-4-yl)ethyl)piperazin-1-yl)phenyl)-1H-pyrazol-4-yl)-6-(trifluoromethyl)picolinamide (290 mg, 0.50 mmol, 1 eq) and 3-(2,4-dioxotetrahydropyrimidin-1(2H)-yl)-4-methoxybenzoic acid (131 mg, 0.50 mmol, 1 eq) in DMF (10 mL) were added DIEA (192 mg, 1.50 mmol, 3 eq) and HATU (226 mg, 0.59 mmol, 1.2 eq). The mixture was stirred at rt for 1 h. The mixture was diluted in water and extracted with EtOAc. The organic layer was washed with aqueous NaCl, dried over anhydrous Na₂SO₄, and concentrated under reduced pressure. The residue was purified by HPLC to afford the title compound (yellow solid, 51.4 mg, yield 12%). LCMS (ESI): m/z 832 [M+H]⁺. **¹H NMR** (400 MHz, MeOD-*d₄*) δ 8.70 (s, 1H), 8.44 (d, *J* = 7.8 Hz, 1H), 8.28 (t, *J* = 7.9 Hz, 1H), 8.02 (d, *J =* 7.6 Hz, 1H), 7.59 (d, *J=* 9.0 Hz, 2H), 7.45 (dd, *J =* 8.5, 2.1 Hz, 1H), 7.39 (d, *J* = 2.1 Hz, 1H), 7.19 (d, *J* = 8.6 Hz, 1H), 7.08 (d, *J* = 9.1 Hz, 2H), 4.58 (s, 1H), 3.93 (s, 4H), 3.77-3.66 (m, 2H), 3.28-3.21 (m, 4H), 3.13 (brs, 1H), 2.95-2.77 (m, 3H), 2.68 (d, *J* = 4.6 Hz, 4H), 2.56-2.46 (m, 2H), 1.96-1.73 (m, 2H), 1.67 (brs, 7H), 1.61-1.49 (m, 2H), 1.36-1.14 (m, 2H).

### Example 1: Kinase activity assay

### Assay for inhibitory effect of compound on IRAK4 kinase activity

The inhibitory effect of the compound on IRAK4 kinase activity was measured using the KinEASE-STK S1 serine/threonine kinase kit (Cisbio). The specific method was as follows: the compound was dissolved in dimethyl sulfoxide and then subjected to 3-fold serial dilution in dimethyl sulfoxide, yielding final test compound concentrations in the reaction system ranging from 5,000 nM to 0.0254 nM. The kinase at a final concentration of 8 nM was added, followed by incubation at rt for 15 minutes. Subsequently, biotinylated STK1 substrate at a final concentration of 400 nM and adenosine triphosphate (ATP) at a final concentration of 80 µM were sequentially added, and the mixture was incubated at 37°C for 90 minutes. Then, the reaction was terminated by adding an anti-phosphoserine/threonine antibody conjugated with a europium cryptate and streptavidin-conjugated XL665 to the reaction system. After incubation at rt for 1 hour, the fluorescence intensity at emission wavelengths of 620 nm and 665 nm for each well was measured using an EnVision microplate reader (PerkinElmer) in HTRF mode. The ratio value was calculated using the formula: Ratio = (665 nm/620 nm)×10⁴ The inhibition rate of the compound at each concentration was calculated using the formula: Inhibition% = [1-(Signal sample-Signal min)/(Signal max-Signal min)]×100. Subsequently, the dose-response curve was fitted by nonlinear curve fitting of log concentration versus inhibition rate using Fit Modela (205) in XLfit to give the IC₅₀ value of the compound for inhibition of protein activity.

**Table 1**

| Compound | IRAK4 HTRF IC₅₀ (µM) | Compound | IRAK4 HTRF IC₅₀ (µM) |
|---|---|---|---|
| A1 | B | A2 | C |
| A3 | B | A4 | C |
| A5 | A | A6 | A |
| A7 | B | A9 | B |
| A10 | B | A12 | A |
| A13 | B | A14 | A |
| A15 | A | A16 | A |
| A17 | B | A18 | B |
| A19 | A | A20 | A |
| A21 | A | A22 | A |
| A23 | A | A24 | A |
| A25 | A | A26 | A |
| A27 | A | A28 | A |
| A29 | B | A31 | B |
| A32 | A | A33 | B |
| A35 | B | A36 | B |
| A39 | B | A42 | A |
| A44 | C | A45 | B |
| A46 | B | A47 | A |
| A48 | A | A49 | A |
| A50 | C | A51 | C |
| A53 | C | A54 | C |
| A55 | C | A56 | C |
| A57 | C | A58 | C |
| A59 | C | A60 | C |
| A61 | C | A62 | B |
| A64 | C | A65 | C |
| A71 | C | A72 | C |
| A74 | B | A75 | B |
| A86 | B | A88 | A |
| A89 | A | A90 | A |
| A91 | B | A92 | A |
| A93 | A | A94 | A |
| A95 | A | A96 | B |
| A97 | A | A98 | B |
| A106 | A | A107 | C |
| A108 | C | A109 | B |
| A110 | A | A229 | A |
| A230 | A | A231 | A |
| A232 | A | A233 | A |
| A234 | A | A235 | A |
| A236 | B | A238 | A |
| A239 | A | A240 | A |
| A241 | A | A242 | A |
| A243 | A | A244 | A |
| A245 | C | A246 | A |
| A247 | B | A248 | A |
| A249 | B | A250 | A |
| A251 | A | A252 | A |
| A253 | A | A254 | B |
| A255 | B | A256 | A |
| A257 | A | A258 | B |
| A259 | B | A260 | A |
| A261 | A | A262 | A |
| A263 | B | A264 | B |
| A265 | B | A266 | B |
| A278 | A | A279 | B |
| A280 | B | A281 | B |
| A286 | B | A287 | B |
| A288 | A | A289 | A |
| A290 | B | A291 | A |
| A292 | A | A293 | A |
| A456 | A | A457 | B |

A represents IC₅₀ < 0.1 µM, B represents 0.1 µM < IC₅₀ < 1.0 µM, C represents IC₅₀ > 1.0 µM).

### Example 2. Degradation of IRAK4 in THP1 by compound

0.6 mL of THP-1 cells (ATCC) was seeded into 12-well culture plates (Greiner, 665180) at a density of 6×10⁵ cells/well. The plates were incubated at 37°C in a 5% carbon dioxide incubator. 0.6 mL of test compound solution was added to achieve final concentrations ranging from 0.15 to 10,000 nM. After 24 hours of incubation, the cells were collected into 1.5 mL centrifuge tubes and centrifuged at 1,000 rpm for 5 minutes. The cell pellets were washed once with 1×PBS, resuspended in 60 µL of lysis buffer (the cell lysis buffer was RIPA Lysis Buffer Strong (GenStar), supplemented with protease inhibitor cocktail tablets, Roche), and left to stand on ice for 30 minutes, followed by centrifugation at 12,000 g, 4°C for 15 minutes. The supernatants were collected, and the IRAK4 protein levels were respectively detected by Western blot (WB).

The total protein concentration in the cell lysate supernatant was determined using a BCA protein quantification kit (NCM). Based on the total protein concentration detected by BCA, the samples were adjusted to 2 µg/µL using cell lysis buffer and 5×SDS-PAGE protein loading buffer (Beyotime), heated at 95°C for 5 minutes in a metal bath, and then placed on ice for an ice bath for 5 minutes before being used as the loading samples for Western blot analysis. Protein electrophoresis was performed using a 10% SDS-PAGE gel with a loading volume of 20 µL (total protein 40 µg) and Tris-SDS electrophoresis at a constant voltage of 80 V. After electrophoresis, the proteins on the gel were transferred to a 0.2 µm PVDF membrane (Immobilon) using a semi-dry transfer method at 0.5 A and 20 V for 60 minutes. After transfer, the membrane was placed in 5% skimmed milk and incubated at rt for 2 hours. After blocking, the PVDF membrane was incubated with primary antibody against IRAK4 (Abcam) at 4°C overnight. The membrane was washed with TBST buffer (20 mM Tris-HCl pH 8.0, 150 mM NaCl, 0.05% Tween-20) for 30 minutes, incubated with secondary antibody (Absin) at rt for 2 hours, and finally incubated with a hypersensitive ECL chemiluminescence kit (NCM Biotech) for 1 minute for color development. The color development and protein band imaging were performed using a chemiluminescence imaging system (SinSage Technology Co., Ltd.). The protein bands were analyzed for grayscale values using Image J analysis software. The corrected grayscale values for each sample were calculated and then compared with those of the control group treated with DMSO alone to calculate the degradation rate.

**Table 2**

| Compound | DC₅₀ (µM) | Dₘₐₓ (%) | Compound | DC₅₀ (µM) | Dₘₐₓ (%) |
|---|---|---|---|---|---|
| A122 | A | A | A341 | A | A |
| A135 | A | A | A343 | A | A |
| A137 | A | A | A348 | A | A |
| A140 | A | A | A349 | C | C |
| A143 | A | A | A350 | A | A |
| A144 | A | A | A351 | B | B |
| A148 | B | A | A352 | A | A |
| A164 | B | A | A353 | C | C |
| A168 | A | A | A354 | A | A |
| A169 | A | A | A355 | A | A |
| A170 | A | A | A356 | A | A |
| A171 | B | A | A357 | A | A |
| A173 | A | A | A361 | B | B |
| A175 | A | B | A364 | A | A |
| A177 | A | A | A365 | A | A |
| A178 | A | A | A368 | A | A |
| A180 | A | A | A369 | A | A |
| A182 | A | A | A372 | A | A |
| A183 | A | A | A373 | A | A |
| A185 | A | B | A374 | A | A |
| A187 | A | A | A377 | A | A |
| A189 | B | A | A378 | A | A |
| A192 | A | A | A379 | A | A |
| A193 | A | B | A380 | A | A |
| A194 | A | B | A384 | C | C |
| A195 | A | A | A385 | C | C |
| A196 | A | A | A386 | C | C |
| A197 | A | A | A387 | C | C |
| A224 | B | A | A388 | C | C |
| A227 | A | A | A389 | C | C |
| A228 | A | A | A391 | A | A |
| A298 | A | A | A392 | B | B |
| A299 | A | A | A394 | B | B |
| A300 | A | A | A396 | A | A |
| A303 | A | A | A402 | A | A |
| A304 | A | B | A403 | A | B |
| A305 | B | B | A409 | A | A |
| A307 | A | A | A411 | A | A |
| A308 | A | A | A414 | A | A |
| A313 | B | A | A415 | A | A |
| A315 | B | A | A421 | A | A |
| A316 | A | A | A422 | A | A |
| A317 | A | A | A423 | B | A |
| A318 | A | A | A425 | A | A |
| A319 | B | B | A426 | A | A |
| A320 | A | A | A427 | A | A |
| A321 | A | A | A428 | A | A |
| A322 | A | A | A430 | A | A |
| A324 | A | A | A432 | B | B |
| A325 | A | A | A433 | B | B |
| A326 | A | A | A434 | A | A |
| A327 | A | A | A436 | A | A |
| A328 | A | A | A437 | A | A |
| A329 | A | A | A438 | A | A |
| A330 | A | A | A439 | A | A |
| A331 | A | A | A440 | A | A |
| A332 | A | A | A441 | A | A |
| A333 | A | A | A442 | A | A |
| A334 | A | A | A443 | A | A |
| A335 | A | A | A447 | A | A |
| A336 | A | A | A448 | B | B |
| A337 | A | A | A451 | A | A |
| A338 | A | A | A452 | A | A |
| A339 | A | A | A453 | A | A |
| A340 | A | A | A454 | A | A |

For DC₅₀, A represents DC₅₀ < 0.1 µM, B represents 0.1 µM < DC₅₀ < 1.0 µM, C represents DC₅₀ > 1.0 µM. For the maximum degradation rate Dmax: A represents Dmax > 60%, B represents 40% < Dmax < 60%, C represents Dmax < 40%.

### Example 3: Detection of inhibition of R848-induced IL-6 release

Cryopreserved human PBMCs (Milestone^{®} Biotechnologies, PB010C-W) were thawed and resuspended in RPMI-1640 medium (Gibco, 22400-105) supplemented with 10% fetal bovine serum (Gibco, 10099-141), 100 U/mL penicillin, and 100 µg/mL streptomycin (Gibco, 15140122). Subsequently, 190 µL of the PBMC suspension was added to each well of 96-well clear cell culture plates (Corning, 3599) to achieve a density of 1×10⁵ cells/well. The cell plates were incubated at 37°C in a 5% CO₂ incubator for 30 minutes. Then, 5 µL of diluted compound solution was added to the corresponding cell-seeded wells to achieve final concentrations ranging from 0.046 to 3,000 nM. The final concentration of DMSO was 0.1%. After the compound-treated cell plate was incubated at 37°C in a 5% CO₂ incubator for 24 hours, 5 µL of R848 (MCE, HY-13740) was added to achieve a final concentration of 10 µg/mL. The cell plates were incubated again at 37°C in a 5% CO₂ incubator for 5 hours, then centrifuged at 1,000 rpm for 1 minute. 100 µL of the cell supernatant from each well was transferred to a new 96-well clear cell culture plate for IL-6 concentration testing.

### Interleukin-6 (IL-6) ELISA assay

The IL-6 concentration in the supernatant of R848-induced human PBMCs was determined using a Human IL-6 (interleukin-6) ELISA KIT (4Bio, CHE0009-048). IL-6 standard solutions at different concentrations, ranging from 3.9 to 250 pg/mL, were prepared according to the manufacturer's instructions. 100 µL of each concentration of the IL-6 standard solution and the pre-diluted test cell supernatant samples were added separately to anti-IL-6 antibody-coated and blocked clear 96-well plates, followed by incubation at 37°C for 1.5 hours. According to the product instructions, the plates were washed, 100 µL of biotinylated antibody was added, and the plates were incubated at 37°C for 1 hour. The plates were washed, 100 µL of enzyme conjugate working solution was added. The plates were incubated at 37°C for 30 minutes, and then washed. 100 µL of chromogenic substrate was added, and the plates were incubated at 37°C for 10-20 minutes. The reaction was terminated by adding 100 µL of stop solution. After the reaction, the OD value at 450 nm was measured in absorbance mode using an Envision microplate reader (PerkinElmer). A standard curve was plotted using the corrected OD values of the various concentrations of IL-6 standard solutions. The IL-6 concentration in each cell supernatant was then determined on the basis of the corresponding concentration for the corrected OD value of the test sample on the standard curve. The inhibition rate of the compound at each concentration was calculated by comparison with the IL-6 concentration of the control group treated with 0.1% DMSO alone. Subsequently, the IC₅₀ value of the compound was calculated by performing non-linear curve fitting of concentration versus inhibition rate using GraphPad Prism 9.

**Table 3**

| Compound | IC₅₀ (µM) | Compound | IC₅₀ (µM) |
|---|---|---|---|
| A49 | C | A178 | A |
| A122 | A | A180 | A |
| A135 | A | A182 | A |
| A137 | A | A183 | A |
| A140 | A | A187 | A |
| A143 | A | A189 | B |
| A144 | A | A192 | B |
| A148 | B | A194 | A |
| A164 | C | A195 | A |
| A168 | A | A196 | A |
| A169 | A | A197 | B |
| A170 | A | A222 | B |
| A171 | B | A224 | B |
| A173 | A | A227 | A |
| A175 | A | A228 | A |
| A177 | A | | |

A represents IC₅₀ < 0.01 µM, B represents 0.01 µM < IC₅₀ < 0.03 µM, C represents 0.03 µM < IC₅₀ < 0.1 µM.

### Example 4. Pharmacokinetic detection

This study was designed to investigate the pharmacokinetic properties of the compounds in rats following intravenous injection and oral administration.

Experimental protocol: Six healthy male Sprague-Dawley (SD) rats were used for each compound, randomly divided into two groups of three animals each, with body weights ranging from 180-220 g. The rats were fasted overnight (10-14 hours) prior to administration, with free access to water, and food was restored 4 hours after administration. The compounds were formulated in a vehicle consisting of 5% DMSO + 10% solutol + 85% physiological saline (w/v). For control compound 1, A169, A173, A178, and A195, the intravenous injection group received a dose of 2 mg/kg with an administration volume of 2 mL/kg, and the oral administration group received a dose of 10 mg/kg with an administration volume of 10 mL/kg. For A170, A177, and A180, the intravenous injection group received a dose of 1 mg/kg with an administration volume of 1 mL/kg, and the oral administration group received a dose of 5 mg/kg with an administration volume of 5 mL/kg. Blank blood samples were collected prior to administration. After administration, 0.2 mL of blood was collected from the jugular vein or other suitable methods at predetermined time points (0.083 h, 0.25 h, 0.5 h, 1 h, 2 h, 4 h, 8 h, 24 h) into EDTA-K2 anticoagulant tubes, and plasma was separated by centrifugation at 6800 g for 6 minutes. After the experiment, the concentration of the compound in plasma was quantitatively detected using liquid chromatography-tandem mass spectrometry (LCMS/MS). Pharmacokinetic parameters were calculated on the basis of the plasma concentration data at different time points using Phoenix WinNonlin version 8.3.5.

**Table 4**

| Compound No. | Oral pharmacokinetic parameter | | | | |
|---|---|---|---|---|---|
| | Dose (mg/kg) | Cₘₐₓ (ng/mL) | T_{1/2} (hr) | AUC (ng.hr/mL) | F(%) |
| Control compound 1 | 10 | 98 | 4 | 428 | 8 |
| A169 | 10 | 408 | 5 | 3520 | 16 |
| A173 | 10 | 107 | 21 | 1134 | 18 |
| A178 | 10 | 593 | 15 | 6556 | 30 |
| A195 | 10 | 1252 | 15 | 19274 | 52 |
| A170 | 5 | 285 | 7 | 2158 | 28 |
| A177 | 5 | 120 | 8 | 1229 | 8 |

Control compound 1:

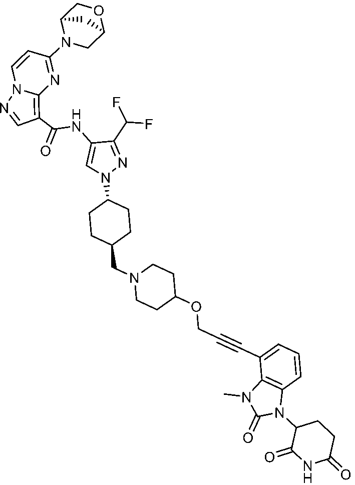

## Claims

1. A compound of formula (III) or a pharmaceutically acceptable salt, ester, solvate, or stereoisomer thereof: 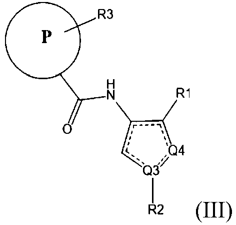 wherein:
the ring structure where Q3 and Q4 are present is selected from the following structures: 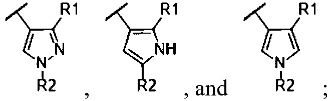
ring P is pyridinyl, pyrazolopyrimidinyl, or pyrrolopyridazinyl;
R1 is selected from -OR^{1a}, -COR^{1a}, -CONR^{1a}R¹⁶, -S(O)(NR^{1a})R^{1b}, -SO₂NR^{1a}R^{1b}, and optionally substituted alkyl, wherein R^{1a} and R^{1b} are independently selected from hydrogen, deuterium, optionally substituted alkyl, and optionally substituted cycloalkyl;
R2 is selected from optionally substituted cycloalkyl, optionally substituted heterocyclyl, and optionally substituted phenyl, pyridinyl, and pyrimidinyl;
R3 is selected from cyano, optionally substituted alkyl, and optionally substituted heteroaryl.

2. A compound of formula (II) or a pharmaceutically acceptable salt, ester, solvate, or stereoisomer thereof:
A-L-E (II)
wherein moiety A has the following structure: 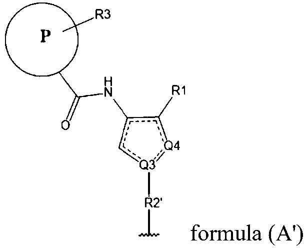
wherein the ring structure where Q3 and Q4 are present is selected from the following structures: 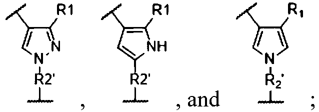
ring P is pyridinyl, pyrazolopyrimidinyl, or pyrrolopyridazinyl;
R1 is selected from -OR^{1a}, -COR^{1a}, -CONR^{1a}R^{1b}, -S(O)(NR^{1a})R^{1b}, -SO₂NR^{1a}R^{1b}, and optionally substituted alkyl, wherein R^{1a} and R^{1b} are independently selected from hydrogen, deuterium, optionally substituted alkyl, and optionally substituted cycloalkyl;
R2' is selected from optionally substituted cycloalkylene, optionally substituted heterocyclylene, optionally substituted phenylene, and pyridinylene;
R3 is selected from cyano, optionally substituted alkyl, and optionally substituted heteroaryl; and moiety A is attached to moiety L via a ring atom of R2';
moiety L has the following structure:
-L1-L2-L3-L4-L5- formula (L)
wherein:
L1, L3, and L5 are independently absent or selected from -Rm-, -Rm-Rn-, -Rm-O-Rn-, -Rm-C(O)-Rn-, - Rm-C(O)O-Rn-, -Rm-OC(O)-Rn-, -Rm-NRx-Rn-, -Rm-C(O)NRx-Rn-, -Rm-NRxC(O)-Rn-, -Rm-S(O)-Rn-, -Rm-S(O)NRx-Rn-, -Rm-NRxS(O)-Rn-, -Rm-S(O)₂-Rn-, -Rm-S(O)₂NRx-Rn-, -Rm-NRxS(O)₂-Rn-, - Rm-NRxC(O)NRy-Rn-, -Rm-OC(O)NRx-Rn-, -Rm-NRxC(O)O-Rn-, 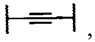 -(CH₂CH₂O)_{g}-, or - (OCH₂CH₂)_{g}-, wherein Rm and Rn are independently selected from a bond, optionally substituted alkylene, optionally substituted alkyleneoxy, optionally substituted cycloalkylene, or optionally substituted heterocyclylene; Rx and Ry are independently selected from hydrogen, deuterium, optionally substituted alkyl, optionally substituted cycloalkyl, or optionally substituted heterocyclyl; and g is an integer from 1 to 20;
L2 and L4 are independently absent or selected from optionally substituted cycloalkylene, optionally substituted heterocyclylene, optionally substituted arylene, or optionally substituted heteroarylene; and moiety E has the following structure: 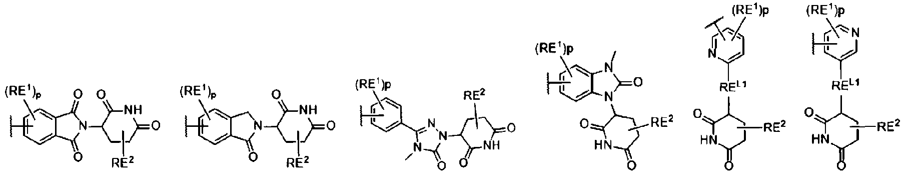 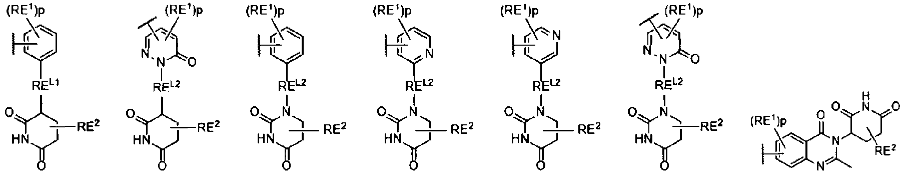 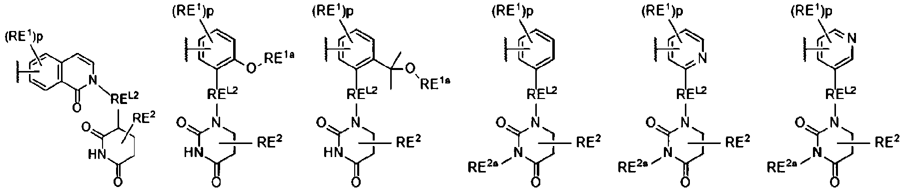
wherein RE¹ is selected from hydrogen, deuterium, halogen, hydroxyl, sulfhydryl, amino, nitro, cyano, optionally substituted alkyl, optionally substituted alkoxy, optionally substituted cycloalkyl, or optionally substituted heterocyclyl; RE² is selected from hydrogen, deuterium, or halogen; RE^{L1} is selected from a bond, -CH₂-, -NH-, -NCH₃-, -O-, -CONH-, or -CONCH₃-; RE^{L2} is selected from a bond or -CH₂-; and p is 1, 2, 3, 4, or 5;
RE^{1a} is selected from -COCH(CH₃)OH, -COCH₃, -CON(CH₃)₂, -CO(CH₂)₂CH(NH₂)COOH, or - COCH(NH₂)(CH₂)₂COOH, and RE^{2a} is selected from -CH₂OCOOC(CH₃)₃ or -CH₂OCOC(CH₃)₃.

3. The compound according to claim 1 or 2, wherein R1 is selected from alkyl (e.g., methyl, ethyl, or propyl (e.g., isopropyl)) substituted with one or more substituents independently selected from halogen and hydroxyl.

4. The compound according to claim 1 or 2, wherein R^{1a} and R^{1b} are independently selected from: hydrogen; deuterium; alkyl (e.g., methyl, ethyl, or propyl (e.g., isopropyl)); cycloalkyl (e.g., cyclopropyl); and alkyl (e.g., methyl, ethyl, or propyl (e.g., isopropyl)) and cycloalkyl (e.g., cyclopropyl) substituted with one or more substituents independently selected from: deuterium, halogen (e.g., fluorine), hydroxyl, sulfhydryl, amino, cyano, and nitro.

5. The compound according to claim 1, wherein R2 is selected from optionally substituted cyclobutyl, cyclopentyl, cyclohexyl, bicyclo[1.1.1]pentyl, or bicyclo[2.2.2]octyl; optionally substituted piperidinyl, azetidinyl, azacyclopentyl, 1-oxa-8-azaspiro[4.5]decyl, or 7-azaspiro[3.5]nonyl; and optionally substituted phenyl, pyridinyl, or pyrimidinyl.

6. The compound according to claim 1, wherein R2 is selected from unsubstituted cycloalkyl, heterocyclyl, phenyl, and pyridinyl; and cycloalkyl, heterocyclyl, phenyl, and pyridinyl substituted with one or more substituents independently selected from: deuterium, halogen (e.g., fluorine or bromine), hydroxyl, sulfhydryl, amino, cyano, nitro, -OR^{2a}, -NR^{2a}R^{2b}, -COR^{2a}, -COOR^{2a}, -CONR^{2a}R^{2b}, -NR^{2a}COR^{2b}, -OCOR^{2a}, optionally substituted alkyl, and optionally substituted heterocyclyl, wherein R^{2a} and R^{2b} are independently selected from hydrogen, deuterium, optionally substituted alkyl, and optionally substituted heterocyclyl.

7. The compound according to claim 1, wherein R^{2a} and R^{2b} are independently selected from hydrogen, deuterium, alkyl (e.g., methyl), halogen-substituted alkyl, heterocyclyl (e.g., piperazinyl), and alkylsubstituted heterocyclyl.

8. The compound according to claim 1, wherein R2 is selected from cycloalkyl, heterocyclyl, phenyl, and pyridinyl substituted with one or more substituents independently selected from:
alkyl;
heterocyclyl;
alkyl (e.g., methyl) or heterocyclyl (e.g., piperazinyl) substituted with one or more substituents selected from -OR^{2c}, -NR^{2c}R^{2d}, -COR^{2c}, -COOR^{2c}, -CONR^{2c}R^{2d}, and/or -NR^{2c}COR^{2d}, wherein R^{2c} and R^{2d} are independently selected from hydrogen, deuterium, alkyl (e.g., methyl), and phenyl; and heterocyclyl (e.g., piperazinyl) substituted with one or more substituents selected from alkyl (e.g., methyl).

9. The compound according to claim 1, wherein R2 is selected from phenyl; and phenyl substituted with one or more substituents independently selected from: deuterium; halogen (e.g., fluorine or bromine); -OR^{2a}, -NR^{2a}R^{2b}, -COR^{2a}, -COOR^{2a}, -CONR^{2a}R^{2b}, -NR²aCOR^{2b}, and -OCOR^{2a}, wherein R^{2a} and R^{2b} are independently selected from hydrogen, deuterium, alkyl (e.g., methyl), and heterocyclyl (e.g., piperazinyl); and alkyl (e.g., methyl), heterocyclyl (e.g., piperazinyl), and alkyl (e.g., methyl) or heterocyclyl (e.g., piperazinyl) substituted with one or more substituents independently selected from: -OR^{2c}, -NR^{2c}R^{2d}, - COR^{2c}, -COOR^{2c}, -CONR^{2c}R^{2d}, and/or -NR^{2c}COR^{2d}, wherein R^{2c} and R^{2d} are independently selected from hydrogen, deuterium, alkyl (e.g., methyl), and phenyl.

10. The compound according to claim 1 or 2, wherein R3 is selected from halogen (e.g., fluorine)-substituted alkyl (e.g., methyl), such as trifluoromethyl.

11. The compound according to claim 1 or 2, wherein R3 is selected from 5-membered heteroaryl comprising one or more nitrogen ring atoms.

12. The compound according to claim 2, wherein R2' is selected from cycloalkylene, heterocyclylene, phenylene, and pyridinylene optionally substituted with one or more substituents selected from: deuterium, halogen, hydroxyl, sulfhydryl, amino, cyano, nitro, an aldehyde group, carboxyl, and alkyl.

13. The compound according to claim 2, wherein R2' is selected from optionally substituted cyclobutylene, cyclopentylene, cyclohexylene, bicyclo[1.1.1]pentylene, or bicyclo[2.2.2]octylene; optionally substituted piperidinylene, azetidinylene, azacyclopentylene, 1-oxa-8-azaspiro[4.5]decylene, or 7-azaspiro[3.5]nonylene; and optionally substituted phenylene or pyridinylene.

14. The compound according to claim 2, wherein L1, L3, and L5 are independently absent or selected from -Rm-, -Rm-O-Rn-, -Rm-C(O)-Rn-, -Rm-C(O)O-Rn-, -Rm-OC(O)-Rn-, -Rm-NRx-Rn-, -Rm-C(O)NRx-Rn-, -Rm-NRxC(O)-Rn-, or 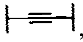 wherein Rm and Rn are independently selected from a bond or optionally substituted alkylene, and Rx is independently selected from hydrogen, deuterium, or optionally substituted alkyl.

15. The compound according to claim 2, wherein Rm and Rn are independently selected from a bond or alkylene (e.g., C₁₋₄ alkylene, such as methylene or ethylene) optionally substituted with halogen (e.g., fluorine).

16. The compound according to claim 2, wherein Rx is independently selected from hydrogen, deuterium, or alkyl (e.g., C₁₋₄ alkylene, such as methylene and ethylene).

17. The compound according to claim 2, wherein L2 is selected from optionally substituted heterocyclylene.

18. The compound according to claim 2, wherein L2 and L4 are independently selected from 3-12 membered heterocyclylene that comprises one or two nitrogen heteroatoms and is optionally substituted with one or more substituents selected from halogen (e.g., fluorine) and/or alkyl (e.g., methyl).

19. The compound according to claim 2, wherein L2 and L4 are independently selected from:

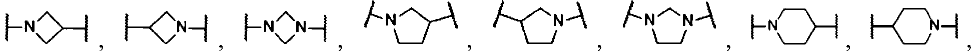

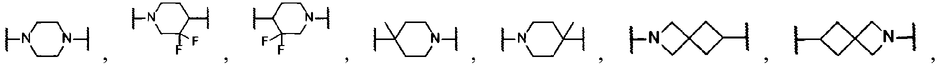

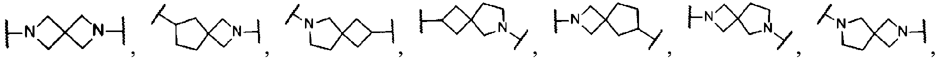

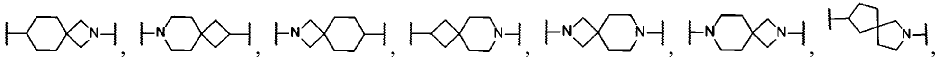

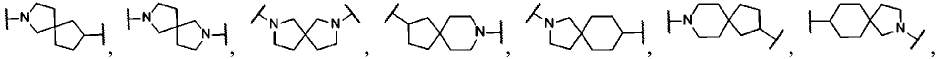

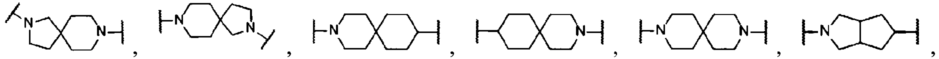

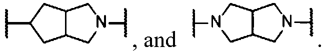

20. The compound according to claim 2, wherein L1 is absent or selected from -Rm-, -Rm-O-Rn-, -Rm-C(O)-Rn-, -Rm-C(O)O-Rn-, -Rm-OC(O)-Rn-, -Rm-NRx-Rn-, -Rm-C(O)NRx-Rn-, or -Rm-NRxC(O)-Rn-, wherein Rm and Rn are independently selected from a bond or alkylene (e.g., methylene or ethylene), and Rx is independently selected from hydrogen, deuterium, or alkyl (e.g., methyl).

21. The compound according to claim 2, wherein L1 is absent or selected from -CH₂-, -CH₂CH₂-, -CH₂O-, -C(O)-, -CH₂C(O)-, -CH₂NH-, -NHCH₂-, -CH₂N(CH₃)-, and -C(O)NH-.

22. The compound according to claim 2, wherein L3 is absent or selected from -Rm-, -Rm-O-Rn-, -Rm-C(O)-Rn-, -Rm-C(O)O-Rn-, -Rm-OC(O)-Rn-, -Rm-NRx-Rn-, -Rm-C(O)NRx-Rn-, or -Rm-NRxC(O)-Rn-, wherein Rm and Rn are independently selected from a bond or alkylene (e.g., methylene or ethylene), and Rx is independently selected from hydrogen, deuterium, or alkyl (e.g., methyl).

23. The compound according to claim 2, wherein L3 is absent or selected from -CH₂-, -CH₂CH₂-, -OCH₂-, -C(O)-, -C(O)CH₂-, -C(O)O-, -NH-, -NHCH₂-, and -N(CH₃)-.

24. The compound according to claim 2, wherein L5 is absent or selected from -Rm-, -Rm-C(O)-Rn-, -Rm-NRx-Rn-, or 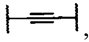 wherein Rm and Rn are independently selected from a bond or alkylene (e.g., methylene or ethylene), and Rx is independently selected from hydrogen, deuterium, or alkyl (e.g., methyl).

25. The compound according to claim 2, wherein L5 is absent or selected from -CH₂-, -C(O)-, -NH-, and

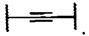

26. The compound according to claim 2, wherein moiety E has the following structure:

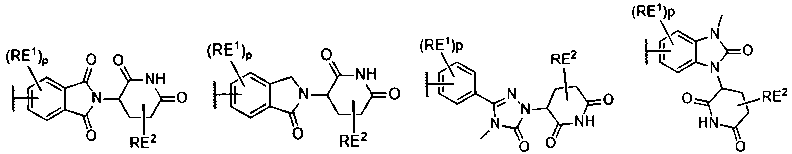

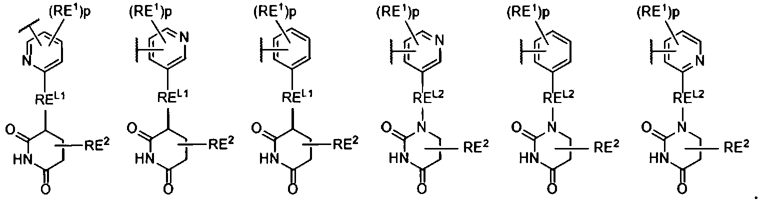

27. The compound according to claim 2, wherein RE¹ is selected from hydrogen, deuterium, halogen, hydroxyl, sulfhydryl, amino, nitro, cyano, alkyl, and alkoxy; RE² is selected from hydrogen, deuterium, or halogen; RE^{L1} is selected from a bond, -CH₂-, -NH-, -NCH₃-, -O-, -CONH-, or -CONCH₃-; RE^{L2} is selected from a bond, -CH₂-, or cyclopropylene; and p is 1 or 2.

28. The compound according to claim 2, wherein RE¹ is selected from hydrogen, deuterium, halogen (e.g., fluorine), and methoxy.

29. The compound according to claim 2, wherein RE² is selected from hydrogen or deuterium.

30. A pharmaceutical composition, comprising the compound or the pharmaceutically acceptable salt, ester, solvate, or stereoisomer thereof according to any one of claims 1-29, and a pharmaceutically acceptable excipient.

31. A method for treating or preventing an IRAK protein-mediated disease, comprising administering to a patient in need thereof the compound according to any one of claims 1-29 or the composition according to claim 30.
